# EUROPEAN PATENT APPLICATION

(11) **EP 4 800 015 A1**
(43) Date of publication of application: **02.09.2026**
(21) Application number: 24881638.1
(22) Date of filing: 23.10.2024
(51) Int. Cl.: C07D 487/04, A61K 31/519, A61P 35/00

(54) **AZABICYCLO DERIVATIVE AND PREPARATION METHOD THEREFOR, AND USE**

(30) Priority: 27.10.2023 CN 202311406430; 01.02.2024 CN 202410141106; 30.04.2024 CN 202410535328; 10.07.2024 CN 202410918626; 21.08.2024 CN 202411151360
(71) Applicant: Zhejiang Hisun Pharmaceutical Co., Ltd., Taizhou, Zhejiang 318000 (CN); Shanghai Aryl Pharmtech Co., Ltd., Songjiang, Shanghai 201612 (CN)
(72) Inventor: YU, Mingfeng, Shanghai 201612 (CN); CAO, Qi, Shanghai 201612 (CN); WANG, Xin, Shanghai 201612 (CN); MENG, Lichen, Shanghai 201612 (CN); ZHANG, Panpan, Taizhou, Zhejiang 318000 (CN); YE, Cheng, Shanghai 201612 (CN); WU, Chengfei, Shanghai 201612 (CN); MAO, Lifei, Taizhou, Zhejiang 318000 (CN); LUO, Yi, Taizhou, Zhejiang 318000 (CN); ZHU, Mingjiang, Taizhou, Zhejiang 318000 (CN); XU, Daiwang, Shanghai 201612 (CN); XU, Xiaojie, Taizhou, Zhejiang 318000 (CN); ZHENG, Xiaohe, Taizhou, Zhejiang 318000 (CN); ZHOU, Houjiang, Taizhou, Zhejiang 318000 (CN)
(74) Representative: HGF
(86) International application number: PCT/CN2024/126680
(87) International publication number: WO 2025/087274

(57) **Abstract**

The present invention relates to an azabicyclo derivative and a preparation method therefor, and a use of a pharmaceutical composition containing the derivative in medicine. Specifically, the present invention relates to an azabicyclo derivative represented by general formula (I), and a preparation method therefor and a pharmaceutically acceptable salt thereof, as well as a use of the azabicyclo derivative and the pharmaceutically acceptable salt as therapeutic agents, especially as WRN inhibitors, wherein the definition of each substituent in the general formula (I) is the same as that in the description.

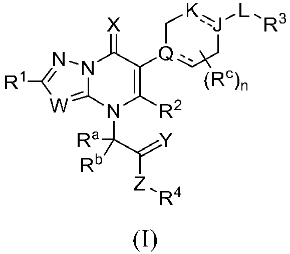

## Description

### FIELD

The present disclosure relates to a azabicyclic derivative, preparation methods therefor, pharmaceutical compositions comprising the derivative, and uses thereof as therapeutic agents, particularly as a WRN inhibitor

### BACKGROUND

Human WRN consists of 1432 amino acid residues and comprises five important components from the N-terminus to the C-terminus: an exonuclease domain, an ATPase domain, a RecQ C-terminal domain, a helicase/RNaseD C-terminal domain, and a nuclear localization signal. Among the five RecQ helicases in humans, WRN is the only one having 3' to 5' exonuclease activity, which is achieved by specific activation of the exonuclease domain located at the N-terminus by a Ku70/80 complex bound to DNA ends. The ATPase domain is the largest and most conserved component of the RecQ helicase family and serves as an ATP-dependent DNA translocation module by binding and hydrolyzing ATP. The RecQ C-terminal domain is the primary site for DNA binding and catalyzes unwinding of DNA double strands. Therefore, the ATPase domain and the RecQ C-terminal domain together constitute the core of the WRN helicase. WRN is a DNA helicase having multiple enzymatic activities that can bind both DNA and other proteins. This enables the enzyme to play an important role in maintaining genomic integrity and stability, including participating in DNA damage repair, replication, and transcription, as well as maintaining stability of telomeres and heterochromatin.

Synthetic lethality refers to a phenomenon in which simultaneous inhibition of two non-lethal genes (with forms of inhibition including genetic defects such as gene mutation and gene silencing, and/or molecular perturbations such as gene expression knockdown and pharmacological inhibition) leads to cell death. By utilizing this mechanism to identify a specific mutation in cancer, and then locating and inhibiting its synthetic lethal partner, cancer cells harboring the mutation can be selectively killed.

Research indicates that WRN is a synthetic lethal partner of high microsatellite instability (MSI-H), a genomic damage. High microsatellite instability (MSI-H) is a hypervariable state caused by defects in DNA mismatch repair (MMR) leading to frequent insertions and/or deletions in nucleotide repeat regions, commonly found in cancers such as endometrial cancer (31%), colorectal cancer (25%), and gastric cancer (19%). In MSI-H cancer cells, thymine/adenine dinucleotide (TA) repeats are highly unstable and undergo large-scale amplification to form non-canonical right-handed double-helix (non-B) DNA secondary structures (e.g., cruciform and G-quadruplex), which require WRN-specific unwinding to complete replication. In the absence of WRN, these DNA secondary structures are cleaved by a MUS81-EME1-SLX4 endonuclease complex, leading to extensive DNA end resection, depletion of replication protein A (RPA), chromosomal fragmentation, and cell death. Additionally, in tumor models having MMR deficiency, loss of WRN leads to activation of multiple DNA damage signaling markers, inducing cell cycle arrest and apoptosis, thereby inhibiting proliferation of tumor cells. Recent studies show that small molecule inhibitors of WRN can specifically induce tumor regression in MSI-H (high microsatellite instability) tumor models, while having no effect in microsatellite stable (MSS) tumor models. Therefore, small molecule chemical drugs capable of inhibiting WRN helicase activity are expected to become a new method for effectively treating MSI-H cancers.

Currently, there is a lack of effective inhibitors targeting the WRN target, and no such drugs have been approved for marketing, leaving a huge unmet clinical need.

### SUMMARY

In view of the foregoing, the present disclosure provides a compound represented by formula (I): 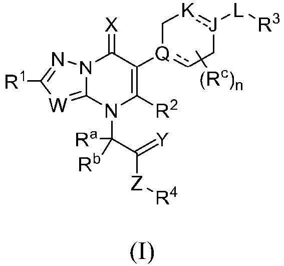
or a stereoisomer, a tautomer, or a pharmaceutically acceptable salt thereof,
wherein:
W is selected from the group consisting of N and CH;
X is selected from the group consisting of O and S;
Y is selected from the group consisting of CH₂, O, and S;
Z is selected from the group consisting of NH, CH₂, and CD₂;
L is selected from the group consisting of -C(=O)-, -C(=S)-, -S(=O)-, -S(=O)₂-, 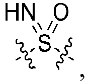 and -NR^{g}C(=O)-;
R^{g} is selected from the group consisting of hydrogen and C₁₋₆ alkyl;
- - - - - in Q - - - - - is selected from the group consisting of a single bond and a double bond;
Q is selected from the group consisting of C, N, and CR^{d};
R^{d} is selected from the group consisting of hydrogen, hydroxy, cyano, halogen, and methoxy;
- - - - - in K - - - - - is selected from the group consisting of a single bond and a double bond, or K - - - - - is absent, wherein when K - - - - - is absent, a 5-membered ring 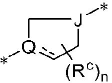 is formed;
provided that:
   when in K - - - - - is a single bond, K is selected from the group consisting of -(CH₂)ₚ- and -NH-, and J is N; p is selected from the group consisting of 1 and 2;
   when in K - - - - - is a double bond, K is CH, and J is C;
   R^{a} and R^{b} are each independently selected from the group consisting of hydrogen, deuterium, halogen, C₁₋₆ alkyl, and C₁₋₆ alkoxy, wherein the halogen is preferably fluorine; alternatively, R^{a} and R^{d}, together with the carbon atom to which they are attached, form a C₃₋₆ cycloalkyl, wherein the C₃₋₆ cycloalkyl is optionally further substituted with one or more substituents selected from the group consisting of halogen, hydroxy, cyano, carbonyl, and -NR⁶R⁷;
   R^{c}, being the same or different, is each independently selected from the group consisting of hydrogen, deuterium, C₁₋₆ alkyl, and C₃₋₆ cycloalkyl, wherein the C₁₋₆ alkyl or C₃₋₆ cycloalkyl is optionally further substituted with one or more substituents selected from the group consisting of halogen, hydroxy, cyano, carbonyl, and -NR⁶R⁷;
   alternatively, two R^{c}, together with the same carbon atom to which they are attached, form a -C(=O)-;
   alternatively, two R^{c}, together with the same carbon atom to which they are attached, form a C₃₋₆ cycloalkyl or a 3- to 8- membered heterocyclyl, wherein the 3- to 8- membered heterocyclyl contains one or more N, O, or S(=O)ᵣ;
   alternatively, two R^{c}, together with the different carbon atoms to which they are respectively attached, form a C₄₋₆ cycloalkyl or a 4- to 8- membered heterocyclyl, wherein the 4-to 8- membered heterocyclyl contains one or more N, O, or S(=O)ᵣ;
   R¹ is selected from the group consisting of alkyl, alkenyl, alkynyl, -NR¹⁵R¹⁶, alkoxy, alkylthio, 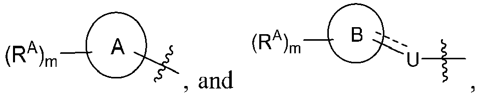 wherein the alkyl, alkoxy, or alkylthio is optionally further substituted with one or more substituents selected from R^{A}, and the alkenyl or alkynyl is substituted with one or more substituents selected from R^{AA};
   R^{AA} is selected from the group consisting of halogen, alkoxy, aryl, heteroaryl, cycloalkyl, heterocyclyl, fused ring, and -C(=O)NR⁶R⁷, wherein the aryl, heteroaryl, cycloalkyl, heterocyclyl, or fused ring is optionally further substituted with one or more substituents selected from the group consisting of C₁₋₆ alkyl, C₁₋₆ haloalkyl, C₁₋₆ alkoxy, C₁₋₆ haloalkoxy, halogen, hydroxy, and cyano, provided that the heterocyclyl is not morpholinyl;
   R¹⁵ and R¹⁶ are each independently selected from the group consisting of hydrogen, deuterium, C₁₋₆ alkyl, and aryl, wherein the C₁₋₆ alkyl or aryl is optionally further substituted with one or more substituents selected from the group consisting of halogen, hydroxy, and cyano;
   ring A is selected from the group consisting of:
      1) C₃₋₈ saturated monocyclic cycloalkyl, C₇₋₈ partially unsaturated monocyclic cycloalkyl, spirocycloalkyl, fused cycloalkyl, or bridged cycloalkyl, wherein the spirocycloalkyl, fused cycloalkyl, or bridged cycloalkyl comprises 0 or 1 double bond;
      2) 4-membered monocyclic heterocyclyl, 7- to 8- membered monocyclic heterocyclyl, spiroheterocyclyl, or fused heterocyclyl, wherein the monocyclic heterocyclyl, spiroheterocyclyl, or fused heterocyclyl comprises 0 or 1 double bond, provided that any ring constituting the spiroheterocyclyl is not cyclopropane, wherein the monocyclic heterocyclyl is optionally further formed into a bridged heterocyclyl by connecting two non-directly connected atoms on the ring via C₁₋₂ alkylene; and
      3) bicyclic aryl, bicyclic heteroaryl, or bicyclic fused ring;
   - - - - - in - - - - -U is selected from the group consisting of a single bond and a double bond;
   provided that: when - - - - - in - - - - - U is selected from a double bond, U is CR^{bb}, and ring B is selected from the group consisting of cycloalkyl, heterocyclyl, and fused cyclyl;
   when - - - - - in - - - - -U is selected from a single bond, U is selected from the group consisting of -O-, -N(R^{e})-, and -S-, and ring B is selected from the group consisting of cycloalkyl, heterocyclyl, aryl, heteroaryl, and fused cyclyl;
   R^{bb} is selected from the group consisting of hydrogen and C₁₋₆ alkyl;
   R^{e} is selected from the group consisting of hydrogen and C₁₋₆ alkyl;
   R^{A}, being the same or different, is each independently selected from the group consisting of deuterium, hydroxy, halogen, nitro, cyano, alkyl, alkenyl, alkynyl, cycloalkyl, heterocyclyl, aryl, heteroaryl, -SF₅, -OR⁵, -OC(=O)R⁵, -C(=O)R⁵, -C(=O)OR⁵, -N(R⁶)C(=O)R⁷, -N(R⁶)C(=O)OR⁷, -NR⁶R⁷, -C(=O)NR⁶R⁷, -S(=O)ᵣNR⁶R⁷, and -S(=O)ᵣR⁵, wherein the alkyl, alkenyl, alkynyl, cycloalkyl, heterocyclyl, aryl, or heteroaryl is optionally further substituted with one or more substituents selected from the group consisting of deuterium, hydroxy, halogen, nitro, cyano, alkyl, alkoxy, haloalkyl, haloalkoxy, hydroxyalkyl, cycloalkyl, heterocyclyl, aryl, heteroaryl, -OR⁸, =O, -C(=O)R⁸, -C(=O)OR⁸, -OC(=O)R⁸, -NR⁹R¹⁰, -C(=O)NR⁹R¹⁰, -S(=O)₂NR⁹R¹⁰, -N(R⁹)C(=O)R¹⁰, and -N(R⁹)C(=O)OR¹⁰;
   alternatively, two R^{A}, together with the same carbon atom to which they are attached, form a -C(=O), -C(=S), or -C(=NR^{cc});
   R^{cc} is selected from the group consisting of hydrogen, hydroxy, and C₁₋₆ alkoxy;
   R² is selected from the group consisting of hydrogen, deuterium, alkyl, hydroxyalkyl, haloalkyl, cycloalkyl, -OR⁵, -S(=O)ᵣR⁵, and -NR⁶R⁷;
   R³ is selected from the group consisting of hydrogen, C₁₋₆ alkyl, amino, hydroxylamino, aryl, heteroaryl, 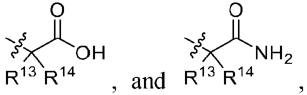 wherein the C₁₋₆ alkyl, aryl, or heteroaryl is optionally further substituted with one or more R^{B};
   R^{B}, being the same or different, is each independently selected from the group consisting of deuterium, alkyl, hydroxy, halogen, nitro, cyano, alkenyl, alkynyl, cycloalkyl, heterocyclyl, aryl, heteroaryl, -SF₅, -OR⁵, -OC(=O)R⁵, -C(=O)R⁵, -C(=O)OR⁵, -N(R⁶)C(=O)R⁷, -N(R⁶)C(=O)OR⁷, -NR⁶R⁷, -C(=O)NR⁶R⁷, -S(=O)ᵣNR⁶R⁷, and -S(=O)ᵣR⁵, wherein the alkenyl, alkynyl, cycloalkyl, heterocyclyl, aryl, or heteroaryl is optionally further substituted with one or more substituents selected from the group consisting of hydroxy, halogen, nitro, cyano, alkyl, alkoxy, haloalkyl, haloalkoxy, hydroxyalkyl, cycloalkyl, heterocyclyl, aryl, heteroaryl, -OR⁸, =O, -C(=O)R⁸, -C(=O)OR⁸, -OC(=O)R⁸, -NR⁹R¹⁰, -C(=O)NR⁹R¹⁰, -S(=O)₂NR⁹R¹⁰, -N(R⁹)C(=O)R¹⁰, and -N(R⁹)C(=O)OR¹⁰;
   R¹³ and R¹⁴ are each independently selected from the group consisting of hydrogen, deuterium, C₁₋₆ alkyl, and C₁₋₆ alkoxy; alternatively, R¹³ and R¹⁴, together with the carbon atom to which they are commonly attached, form a C₃₋₆ cycloalkyl, wherein the C₃₋₆ cycloalkyl is optionally further substituted with one or more substituents selected from the group consisting of halogen, hydroxy, and cyano;
   R⁴ is selected from the group consisting of aryl, heteroaryl, and bicyclic fused ring, wherein the bicyclic fused ring is preferably a fused ring formed from a monocyclic aryl or monocyclic heteroaryl and a monocyclic heterocyclyl or monocyclic cycloalkyl, wherein the aryl, heteroaryl, or bicyclic fused ring is optionally further substituted with one or more substituents selected from the group consisting of halogen, hydroxy, cyano, -SF₅, C₁₋₆ alkyl, C₁₋₆ cycloalkyl, C₁₋₆ haloalkyl, C₁₋₆ halocycloalkyl, C₁₋₆ hydroxyalkyl, C₁₋₆ alkoxy, C₁₋₆ haloalkoxy, C₁₋₆ alkenyl, C₁₋₆ alkynyl, -NR⁶R⁷, -C(=O)R⁵, and -S(=O)ᵣR⁵;
   R⁵ is each independently selected from the group consisting of hydrogen, alkyl, cycloalkyl, heterocyclyl, aryl, and heteroaryl, wherein the alkyl, cycloalkyl, heterocyclyl, aryl, or heteroaryl is optionally further substituted with one or more substituents selected from the group consisting of deuterium, hydroxy, halogen, nitro, cyano, alkyl, alkoxy, haloalkyl, haloalkoxy, cycloalkyl, heterocyclyl, aryl, heteroaryl, =O, -C(=O)R⁸, -C(=O)OR⁸, -OC(=O)R³, -NR⁹R¹⁰, -C(=O)NR⁹R¹⁰, -S(=O)₂NR⁹R¹⁰, and -N(R⁹)C(=O)R¹⁰;
   R⁶ and R⁷ are each independently selected from the group consisting of hydrogen, hydroxy, alkyl, cycloalkyl, heterocyclyl, aryl, and heteroaryl, wherein the alkyl, cycloalkyl, heterocyclyl, aryl, or heteroaryl may be optionally further substituted with one or more substituents selected from the group consisting of hydroxy, halogen, nitro, cyano, alkyl, alkoxy, cycloalkyl, heterocyclyl, aryl, heteroaryl, =O, -C(=O)R³, -C(=O)OR⁸, -OC(=O)R³, -NR⁹R¹⁰, -C(=O)NR⁹R¹⁰, -S(=O)₂NR⁹R¹⁰, and -N(R⁹)C(=O)R¹⁰;
   alternatively, R⁶ and R⁷, together with the atom to which they are attached, form a 4- to 8- membered heterocyclyl containing one or more N, O, or S(=O)ᵣ, wherein the 4- to 8-membered heterocyclyl is optionally further substituted with one or more substituents selected from the group consisting of hydroxy, halogen, nitro, cyano, alkyl, alkoxy, cycloalkyl, heterocyclyl, aryl, heteroaryl, =O, -C(=O)R³, -C(=O)OR⁸, -OC(=O)R⁸, -NR⁹R¹⁰, -C(=O)NR⁹R¹⁰, -S(=O)₂NR⁹R¹⁰, and -N(R⁹)C(=O)R¹⁰;
   R⁸, R⁹, and R¹⁰ are each independently selected from the group consisting of hydrogen, alkyl, amino, cycloalkyl, heterocyclyl, aryl, and heteroaryl, wherein the alkyl, cycloalkyl, heterocyclyl, aryl, or heteroaryl may be optionally further substituted with one or more substituents selected from the group consisting of hydroxy, halogen, nitro, amino, cyano, alkyl, alkoxy, cycloalkyl, heterocyclyl, aryl, heteroaryl, carboxy, and carboxylate;
   n is 0, 1, 2, 3, 4, 5, or 6;
   m is 0, 1, 2, 3, 4, or 5; and
   r is each independently 0, 1, or 2.

In a preferred embodiment, the present disclosure provides a compound of formula (I) or a stereoisomer, tautomer, or pharmaceutically acceptable salt thereof, wherein W is N.

In a preferred embodiment, the present disclosure provides a compound of formula (I) or a stereoisomer, tautomer, or pharmaceutically acceptable salt thereof, wherein W is CH.

In a preferred embodiment, the present disclosure provides a compound of formula (I) or a stereoisomer, tautomer, or pharmaceutically acceptable salt thereof, wherein X and Y are O.

In a preferred embodiment, the present disclosure provides a compound of formula (I) or a stereoisomer, tautomer, or pharmaceutically acceptable salt thereof, wherein Z is NH and L is -C(=O)-.

In a preferred embodiment, the present disclosure provides a compound of formula (I) or a stereoisomer, tautomer, or pharmaceutically acceptable salt thereof, wherein - - - - - in Q - - - - - is a single bond; Q is selected from the group consisting of N and CH;
- - - - - in K - - - - - is a single bond, K is selected from the group consisting of CH₂ and CH₂CH₂, and J is N; and
R^{c} is hydrogen.

In a preferred embodiment, the present disclosure provides a compound of formula (I) or a stereoisomer, tautomer, or pharmaceutically acceptable salt thereof, wherein R^{a} and R^{b} are hydrogen.

In a preferred embodiment, the present disclosure provides a compound of formula (I) or a stereoisomer, tautomer, or pharmaceutically acceptable salt thereof, which is a compound of formula (II) or a stereoisomer, tautomer, or pharmaceutically acceptable salt thereof: 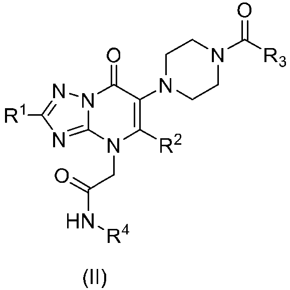 wherein: R¹, R², R³, and R⁴ are defined as in formula (I).

In a preferred embodiment, the present disclosure provides a compound of formula (I) or a stereoisomer, tautomer, or pharmaceutically acceptable salt thereof, which is a compound of formula (III) or a stereoisomer, tautomer, or pharmaceutically acceptable salt thereof: 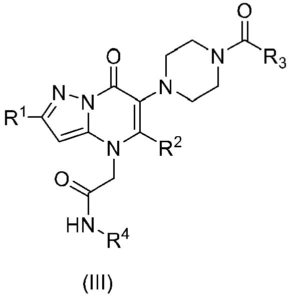 wherein: R¹, R², R³, and R⁴ are defined as in formula (I).

In a preferred embodiment, the present disclosure provides a compound of formula (I) or a stereoisomer, tautomer, or pharmaceutically acceptable salt thereof, wherein - - - - - in Q - - - - - is a single bond; Q is selected from the group consisting of N;
- - - - - in K - - - - - is a single bond, K is CH₂, and J is CH;
R¹ is hydrogen; and
L is -NHC(=O)-.

In a preferred embodiment, the present disclosure provides a compound of formula (I), (II), or (III) or a stereoisomer, tautomer, or pharmaceutically acceptable salt thereof, wherein R¹ is selected from the group consisting of C₁₋₆ alkyl, -NR¹⁵R¹⁶, C₂₋₆ alkenyl, and C₂₋₆ alkynyl, wherein the C₁₋₆ alkyl is optionally further substituted with one or more halogens, wherein the C₂₋₆ alkenyl or C₂₋₆ alkynyl is substituted with at least one or more substituents selected from R^{AA};
R^{AA} is selected from the group consisting of halogen, aryl, heteroaryl, cycloalkyl, heterocyclyl, fused ring, and -C(=O)NR⁶R⁷; wherein the aryl, heteroaryl, cycloalkyl, heterocyclyl, or fused ring is optionally further substituted with one or more substituents selected from the group consisting of methyl, trifluoromethyl, methoxy, trifluoromethoxy, halogen, hydroxy, and cyano; provided that the heterocyclyl is not morpholinyl;
R⁶ and R⁷ are each independently selected from the group consisting of hydrogen and alkyl, or, R⁶ and R⁷, together with the N atom to which they are attached, form a 4- to 8-membered heterocyclyl;
R¹⁵ is selected from the group consisting of hydrogen;
R¹⁶ is selected from the group consisting of C₁₋₆ alkyl and aryl, wherein the C₁₋₆ alkyl or aryl is optionally further substituted with one or more halogens.

In a preferred embodiment, the present disclosure provides a compound of formula (I), (II), or (III) or a stereoisomer, tautomer, or pharmaceutically acceptable salt thereof, wherein R¹ is selected from 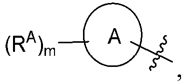 wherein ring A is selected from the group consisting of C₃₋₈ saturated monocyclic cycloalkyl, C₇₋₈ partially unsaturated monocyclic cycloalkyl, spirocycloalkyl, fused cycloalkyl, bridged cycloalkyl, 4-membered monocyclic heterocyclyl, 7-to 8- membered monocyclic heterocyclyl, spiroheterocyclyl, fused heterocyclyl, bicyclic heteroaryl, and bicyclic fused ring, wherein the spirocycloalkyl, fused cycloalkyl, bridged cycloalkyl, 4-membered monocyclic heterocyclyl, 7- to 8- membered monocyclic heterocyclyl, or spiroheterocyclyl contains 0 or 1 double bond, provided that any ring constituting the spiroheterocyclyl is not cyclopropane;
R^{A} is selected from the group consisting of deuterium, halogen, hydroxy, methoxy, -NR⁶R⁷, -C(=O)NR⁶R⁷, C₁₋₆ alkyl, C₃₋₈ cycloalkyl, and 5- to 6- membered heterocyclyl, wherein the C₁₋₆ alkyl or 5- to 6- membered heterocyclyl is optionally further substituted with one or more substituents selected from the group consisting of deuterium, hydroxy, halogen, cyano, alkyl, alkoxy, haloalkyl, and haloalkoxy, alternatively, two R^{A}, together with the same carbon atom to which they are attached, form -C(=O) or -C(=NR^{cc});
R⁶ and R⁷ are selected from the group consisting of hydrogen;
R^{cc} is selected from the group consisting of hydroxy and methoxy; and
m is 0, 1, 2, 3, 4, or 5.

In a preferred embodiment, the present disclosure provides a compound of formula (I), (II), or (III) or a stereoisomer, tautomer, or pharmaceutically acceptable salt thereof, wherein R¹ is selected from 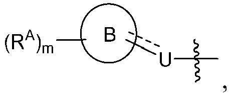 wherein - - - - - in - - - - - U is selected from a double bond, U is CR^{bb}, and ring B is selected from the group consisting of cycloalkyl and heterocyclyl;
R^{bb} is selected from the group consisting of hydrogen and methyl;
R^{A} is selected from the group consisting of deuterium, halogen, hydroxy, methyl, methoxy, -NR⁶R⁷, and -C(=O)NR⁶R⁷;
R⁶ and R⁷ are each independently hydrogen; and
m is 0, 1, 2, 3, 4, or 5.

In a preferred embodiment, the present disclosure provides a compound of formula (I), (II), or (III) or a stereoisomer, tautomer, or pharmaceutically acceptable salt thereof, wherein R¹ is selected from 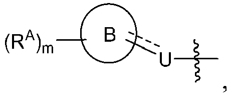 wherein - - - - - in - - - - - U is selected from a single bond, U is O, and ring B is selected from the group consisting of aryl and heteroaryl; and
m is 0.

In a preferred embodiment, the present disclosure provides a compound of formula (1), (II), or (III) or a stereoisomer, tautomer, or pharmaceutically acceptable salt thereof, wherein R¹ is selected from the group consisting of:

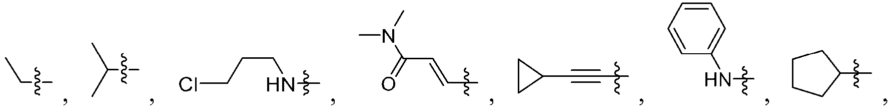

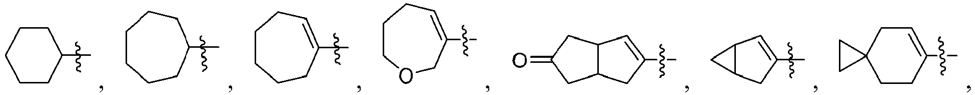

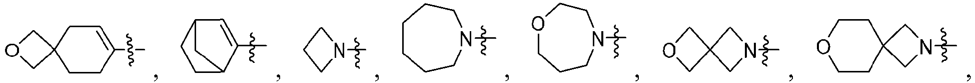

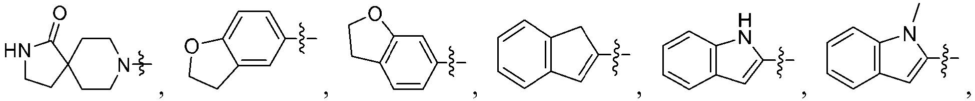

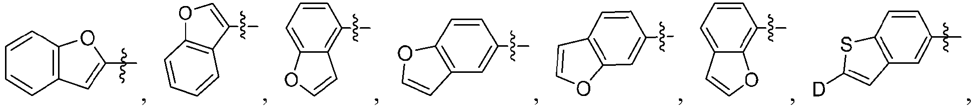

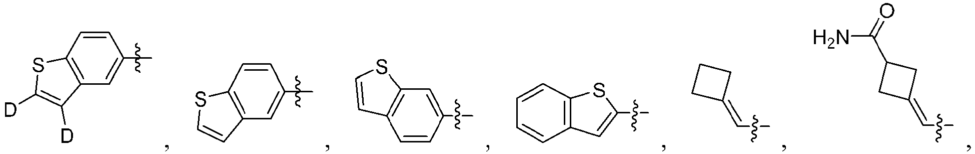

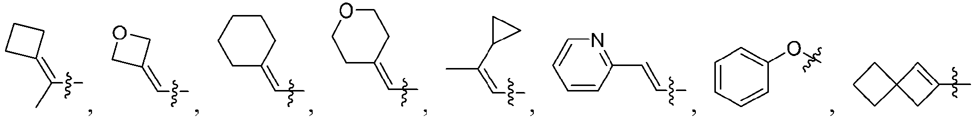

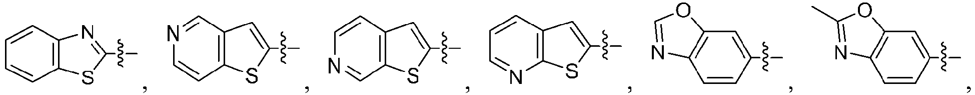

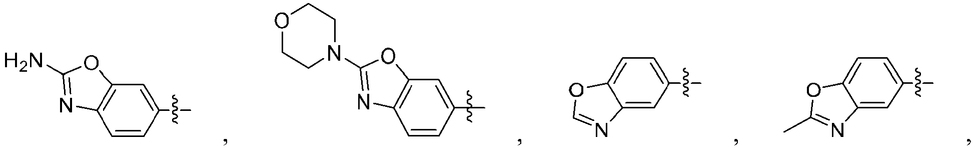

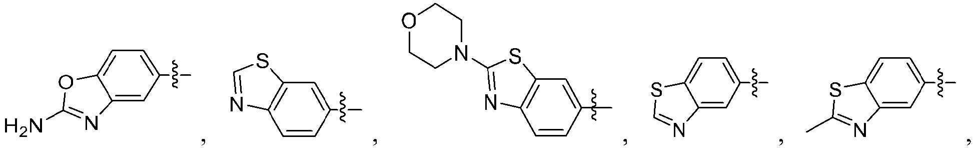

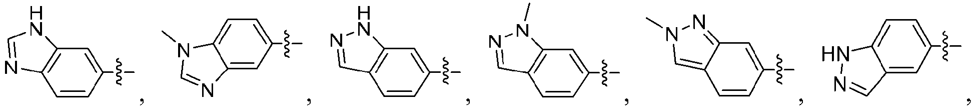

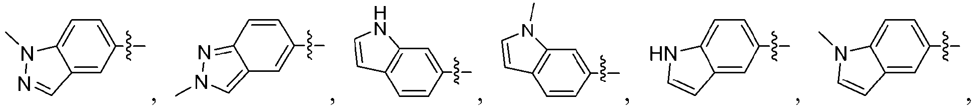

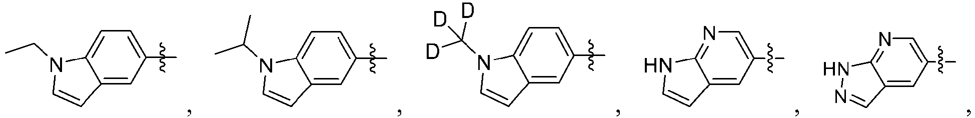

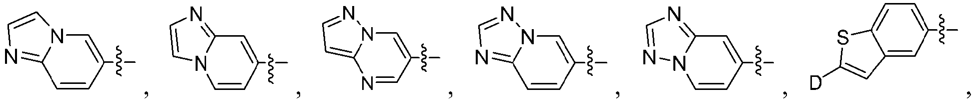

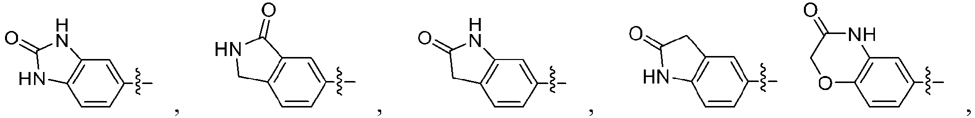

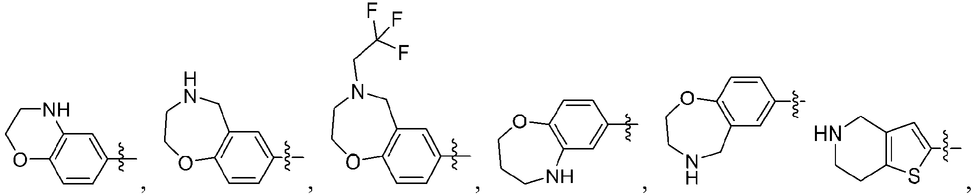

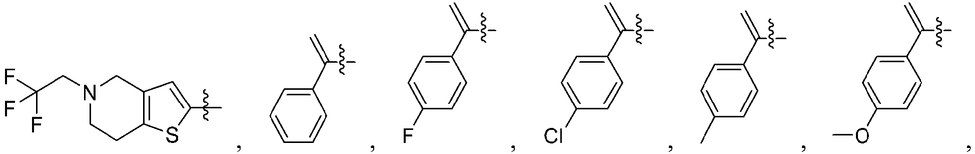

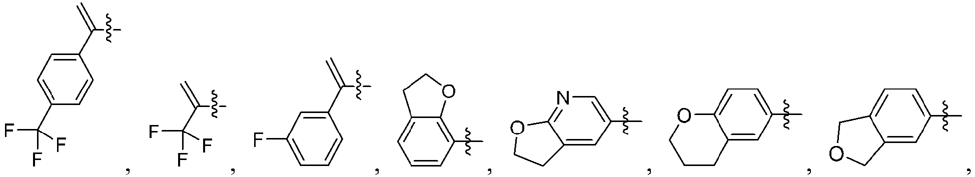

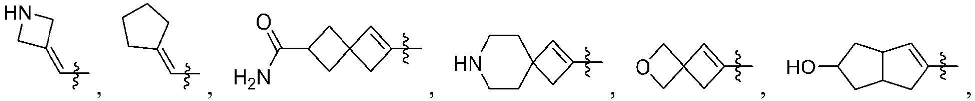

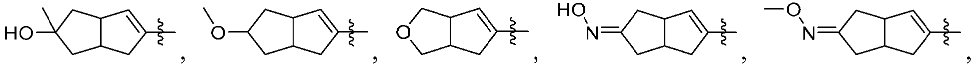

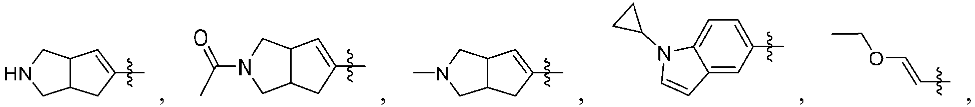

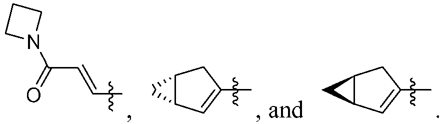

In a preferred embodiment, the present disclosure provides a compound of formula (I), (II), or (III), or a stereoisomer, tautomer, or pharmaceutically acceptable salt thereof, wherein R² is selected from the group consisting of hydrogen, deuterium, C₁₋₃ alkyl, 3- to 6- membered cycloalkyl, C₁₋₃ hydroxyalkyl, and C₁₋₃ haloalkyl, preferably methyl, ethyl, isopropyl, cyclopropyl, or cyclobutyl.

In a preferred embodiment, the present disclosure provides a compound of formula (I), (II), or (III), or a stereoisomer, tautomer, or pharmaceutically acceptable salt thereof, wherein R³ is selected from the group consisting of 5- or 6- membered heteroaryl and phenyl, wherein the 5- or 6- membered heteroaryl or phenyl is optionally further substituted with one or more substituents selected from the group consisting of deuterium, hydroxy, halogen, alkyl, alkylthio, alkoxy, haloalkyl, hydroxyalkyl, and haloalkoxy.

In a preferred embodiment, the present disclosure provides a compound of formula (1), (II), or (III), or a stereoisomer, tautomer, or pharmaceutically acceptable salt thereof, wherein R³ is selected from the group consisting of:

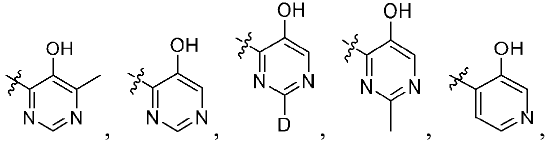

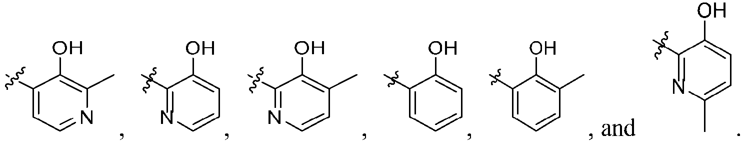

In a preferred embodiment, the present disclosure provides a compound of formula (I), (II), or (III), or a stereoisomer, tautomer, or pharmaceutically acceptable salt thereof,wherein R⁴ is selected from the group consisting of phenyl and heteroaryl, wherein the phenyl or heteroaryl is optionally further substituted with one or more substituents selected from the group consisting of halogen, hydroxy, cyano, -SF₅, C₁₋₆ alkyl, C₁₋₆ haloalkyl, C₁₋₆ alkoxy, C₁₋₆ haloalkoxy, C₁₋₆ alkenyl, C₁₋₆ alkynyl, -NR⁶R⁷, -C(=O)R⁵, and -S(=O)ᵣR⁵;
R⁵, R⁶, and R⁷ are each independently selected from the group consisting of hydrogen and C₁₋₆ alkyl, wherein the C₁₋₆ alkyl is optionally further substituted with one or more deuterium or halogen;
alternatively, R⁶ and R⁷, together with the atom to which they are attached, form a 4- to 8- membered heterocyclyl containing one or more N, O, or S(=O)ᵣ, wherein the 4- to 8-membered heterocyclyl is optionally further substituted with one or more substituents selected from the group consisting of hydroxy, halogen, C₁₋₆ alkyl, C₁₋₆ alkoxy, amino, and =O;
r is 0, 1, or 2.

In a preferred embodiment, the present disclosure provides a compound of formula (I), (II), or (III), or a stereoisomer, tautomer, or pharmaceutically acceptable salt thereof, wherein R⁴ is selected from the group consisting of:

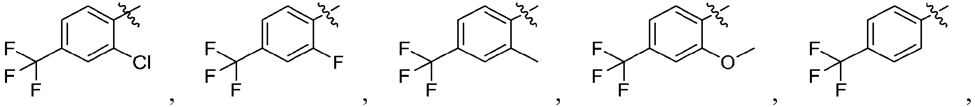

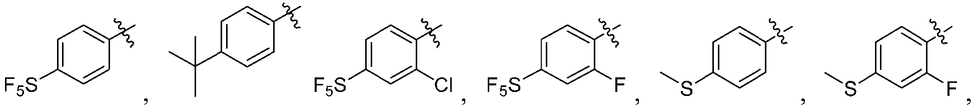

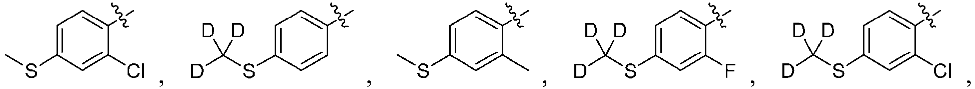

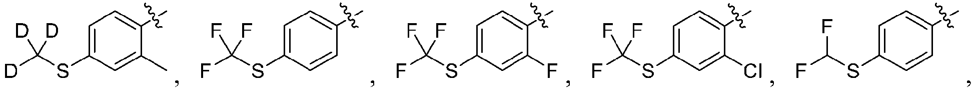

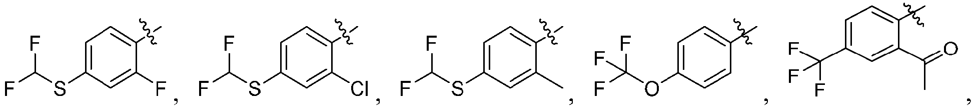

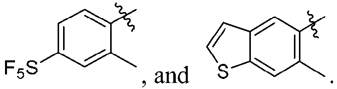

In a preferred embodiment of the present disclosure, the above-mentioned compound is selected from the group consisting of:

| **Compound No.** | **Structure** | **Name** |
|---|---|---|
| Example 1 | 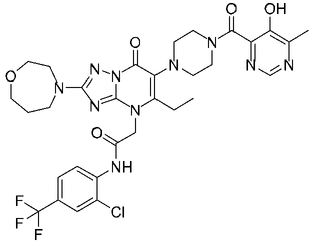 | N-(2-chloro-4-(trifluoromethyl)phenyl)-2-( 5-ethyl-6-(4-(5-hydroxy-6-methylpyrimidi ne-4-carbonyl)piperazin-1-yl)-2-(1,4-oxaze pan-4-yl)-7-oxo-[1,2,4]triazolo[1,5-a]pyri midin-4(7H)-yl)acetamide |
| Example 2 | 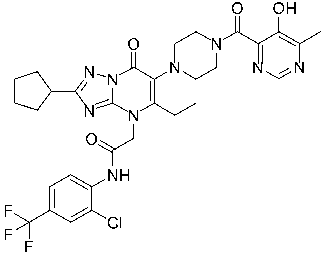 | N-(2-chloro-4-(trifluoromethyl)phenyl)-2-( 2-cyclopentyl-5-ethyl-6-(4-(5-hydroxy-6-methylpyrimidine-4-carbonyl)piperazin-1-yl)-7-oxo-[1,2,4]triazolo[1,5-a]pyrimidin-4 (7H)-yl)acetamide |
| Example 3 | 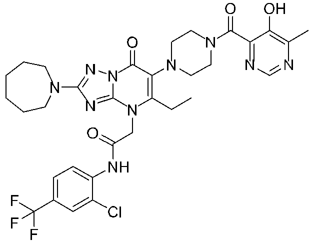 | 2-(2-(azepan-1-yl)-5-ethyl-6-(4-(5-hydroxy -6-methylpyrimidine-4-carbonyl)piperazin-1-yl)-7-oxo-[1,2,4]triazolo[1,5-a]pyrimidin -4(7H)-yl)-N-(2-chloro-4-(trifluoromethyl) phenyl)acetamide |
| Example 4 | 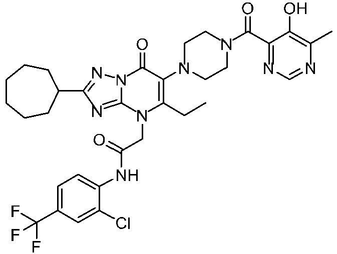 | N-(2-chloro-4-(trifluoromethyl)phenyl)-2-( 2-cycloheptyl-5-ethyl-6-(4-(5-hydroxy-6-methylpyrimidine-4-carbonyl)piperazin-1-yl)-7-oxo-[1,2,4]triazolo[1,5-a]pyrimidin-4 (7H)-yl)acetamide |
| Example 5 | 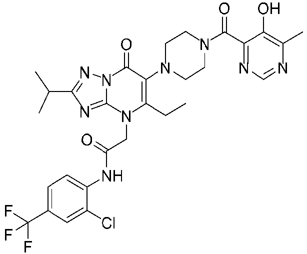 | N-(2-chloro-4-(trifluoromethyl)phenyl)-2-( 5-ethyl-6-(4-(5-hydroxy-6-methylpyrimidi ne-4-carbonyl)piperazin-1-yl)-2-isopropyl-7-oxo-[1,2,4]triazolo[1,5-a]pyrimidin-4(7H )-yl)acetamide |
| Example 6 | 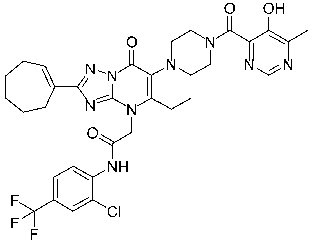 | N-(2-chloro-4-(trifluoromethyl)phenyl)-2-( 2-(cyclohept-1-en-1-yl)-5-ethyl-6-(4-(5-hy droxy-6-methylpyrimidine-4-carbonyl)pipe razin-1-yl)-7-oxo-[1,2,4]triazolo[1,5-a]pyri midin-4(7H)-yl)acetamide |
| Example 7 | 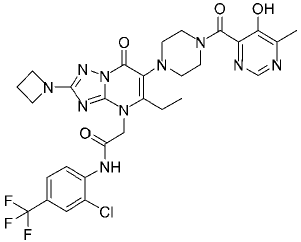 | 2-(2-(azetidin-1-yl)-5-ethyl-6-(4-(5-hydrox y-6-methylpyrimidine-4-carbonyl)piperazi n-1-yl)-7-oxo-[1,2,4]triazolo[1,5-a]pyrimid in-4(7H)-yl)-N-(2-chloro-4-(trifluoromethy l)phenyl)acetamide |
| Example 8 | 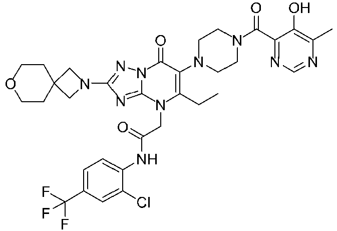 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-(7-oxa-2-azaspiro[3.5]nonan-2-yl)-[1,2,4]triaz olo[1,5-a]pyrimidin-4(7H)-yl)acetamide |
| Example 9 | 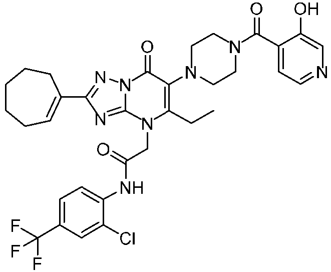 | N-(2-chloro-4-(trifluoromethyl)phenyl)-2-( 2-(cyclohept-1-en-1-yl)-5-ethyl-6-(4-(3-hy droxyisonicotinoyl)piperazin-1-yl)-7-oxo-[ 1,2,4]triazolo[1,5-a]pyrimidin-4(7H)-yl)ac etamide |
| Example 10 | 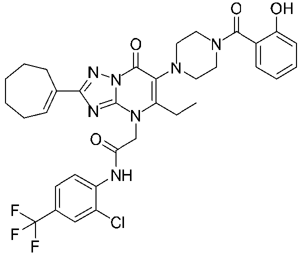 | N-(2-chloro-4-(trifluoromethyl)phenyl)-2-( 2-(cyclohept-1-en-1-yl)-5-ethyl-6-(4-(2-hy droxybenzoyl)piperazin-1-yl)-7-oxo-[1,2,4 ]triazolo[1,5-a]pyrimidin-4(7H)-yl)acetami de |
| Example 11 | 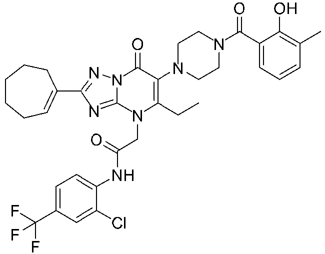 | N-(2-chloro-4-(trifluoromethyl)phenyl)-2-( 2-(cyclohept-1-en-1-yl)-5-ethyl-6-(4-(2-hy droxy-3-methylbenzoyl)piperazin-1-yl)-7-oxo-[1,2,4]triazolo[1,5-a]pyrimidin-4(7H)-yl)acetamide |
| Example 12 | 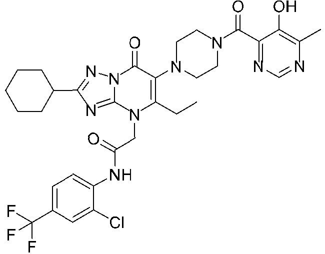 | N-(2-chloro-4-(trifluoromethyl)phenyl)-2-( 2-cyclohexyl-5-ethyl-6-(4-(5-hydroxy-6-m ethylpyrimidine-4-carbonyl)piperazin-1-yl) -7-oxo-[1,2,4]triazolo[1,5-a]pyrimidin-4(7 H)-yl)acetamide |
| Example 13 | 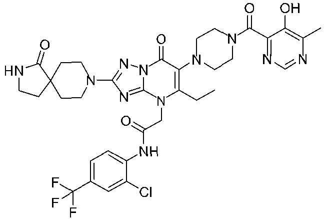 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-(1-oxo-2,8-diazaspiro[4.5]decan-8-yl)-[1,2,4]t riazolo[1,5-a]pyrimidin-4(7H)-yl)acetamid e |
| Example 14 | 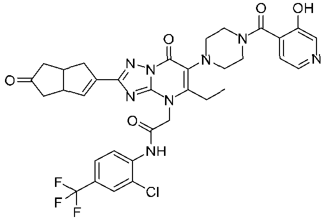 | N-(2-chloro-4-(trifluoromethyl)phenyl)-2-( 5-ethyl-6-(4-(3-hydroxyisonicotinoyl)piper azin-1-yl)-7-oxo-2-(5-oxo-1,3a,4,5,6,6a-he xahydropentalen-2-yl)-[1,2,4]triazolo[1,5-a ]pyrimidin-4(7H)-yl)acetamide |
| Example 15 | 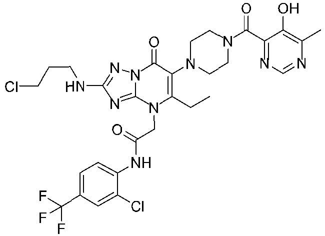 | N-(2-chloro-4-(trifluoromethyl)phenyl)-2-( 2-((3-chloropropyl)amino)-5-ethyl-6-(4-(5-hydroxy-6-methylpyrimidine-4-carbonyl)p iperazin-1-yl)-7-oxo-[1,2,4]triazolo[1,5-a]p yrimidin-4(7H)-yl)acetamide |
| Example 16 | 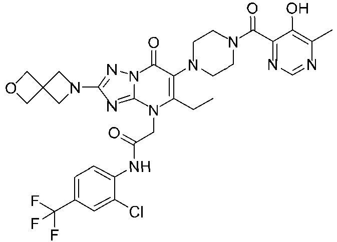 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-(2-oxa-6-azaspiro[3.3]heptan-6-yl)-[1,2,4]tria zolo[1,5-a]pyrimidin-4(7H)-yl)acetamide |
| Example 17 | 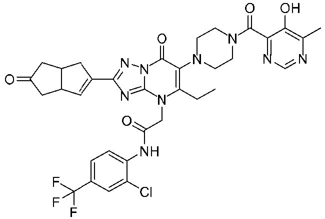 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-(5-oxo-1,3a,4,5,6,6a-hexahydropentalen-2-yl) -[1,2,4]triazolo[1,5-a]pyrimidin-4(7H)-yl)a cetamide |
| Example 18 | 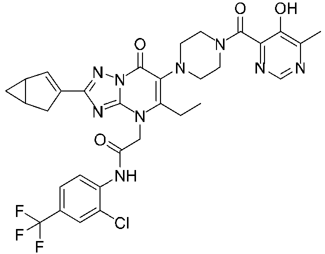 | 2-(2-(bicyclo[3.1.0]hex-2-en-3-yl)-5-ethyl-6-(4-(5-hydroxy-6-methylpyrimidine-4-car bonyl)piperazin-1-yl)-7-oxo-[1,2,4]triazolo [1,5-a]pyrimidin-4(7H)-yl)-N-(2-chloro-4-( trifluoromethyl)phenyl)acetamide |
| Example 19 | 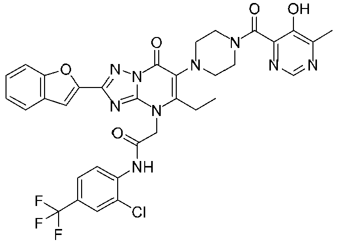 | 2-(2-(benzofuran-2-yl)-5-ethyl-6-(4-(5-hyd roxy-6-methylpyrimidine-4-carbonyl)piper azin-1-yl)-7-oxo-[1,2,4]triazolo[1,5-a]pyri midin-4(7H)-yl)-N-(2-chloro-4-(trifluorom ethyl)phenyl)acetamide |
| Example 20 | 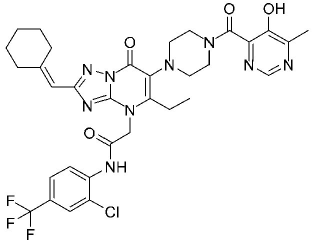 | N-(2-chloro-4-(trifluoromethyl)phenyl)-2-( 2-(cyclohexylidenemethyl)-5-ethyl-6-(4-(5 -hydroxy-6-methylpyrimidine-4-carbonyl) piperazin-1-yl)-7-oxo-[1,2,4]triazolo[1,5-a] pyrimidin-4(7H)-yl)acetamide |
| Example 21 | 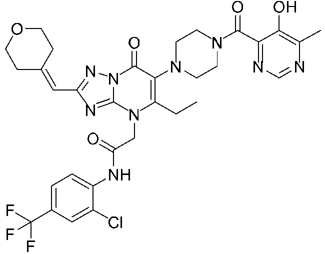 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-((te trahydro-4H-pyran-4-ylidene)methyl)-[1,2, 4]triazolo[1,5-a]pyrimidin-4(7H)-yl)aceta mide |
| Example 22 | 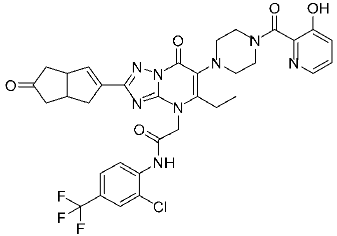 | N-(2-chloro-4-(trifluoromethyl)phenyl)-2-( 5-ethyl-6-(4-(3-hydroxypicolinoyl)piperazi n-1-yl)-7-oxo-2-(5-oxo-1,3a,4,5,6,6a-hexa hydropentalen-2-yl)-[1,2,4]triazolo[1,5-a]p yrimidin-4(7H)-yl)acetamide |
| Example 23 | 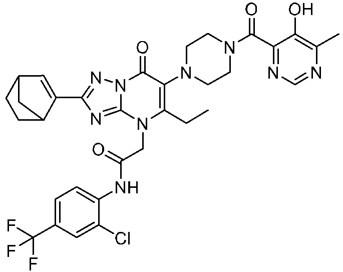 | 2-(2-(bicyclo[2.2.1]hept-2-en-2-yl)-5-ethyl -6-(4-(5-hydroxy-6-methylpyrimidine-4-ca rbonyl)piperazin-1-yl)-7-oxo-[1,2,4]triazol o[1,5-a]pyrimidin-4(7H)-yl)-N-(2-chloro-4 -(trifluoromethyl)phenyl)acetamide |
| Example 24 | 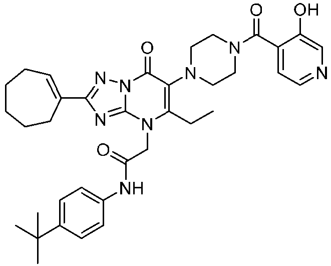 | N-(4-(tert-Butyl)phenyl)-2-(2-(cyclohept-1 -en-1-yl)-5-ethyl-6-(4-(3-hydroxyisonicoti noyl)piperazin-1-yl)-7-oxo-[1,2,4]triazolo[ 1,5-a]pyrimidin-4(7H)-yl)acetamide |
| Example 25 | 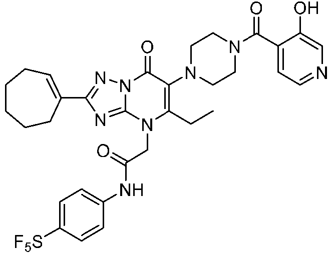 | 2-(2-(cyclohept-1-en-1-yl)-5-ethyl-6-(4-(3-hydroxyisonicotinoyl)piperazin-1-yl)-7-ox o-[1,2,4]triazolo[1,5-a]pyrimidin-4(7H)-yl) -N-(4-(pentafluoro-λ⁶-sulfaneyl)phenyl)ace tamide |
| Example 26 | 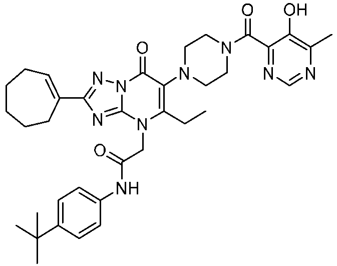 | N-(4-(tert-Butyl)phenyl)-2-(2-(cyclohept-1 -en-1-yl)-5-ethyl-6-(4-(5-hydroxy-6-methy lpyrimidine-4-carbonyl)piperazin-1-yl)-7-o xo-[1,2,4]triazolo[1,5-a]pyrimidin-4(7H)-y l)acetamide |
| Example 27 | 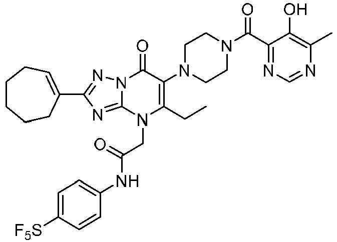 | 2-(2-(cyclohept-1-en-1-yl)-5-ethyl-6-(4-(5-hydroxy-6-methylpyrimidine-4-carbonyl)p iperazin-1-yl)-7-oxo-[1,2,4]triazolo[1,5-a]p yrimidin-4(7H)-yl)-N-(4-(pentafluoro-λ⁶-s ulfaneyl)phenyl)acetamide |
| Example 28 | 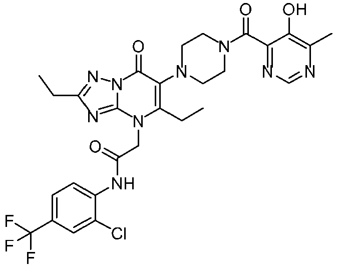 | N-(2-chloro-4-(trifluoromethyl)phenyl)-2-( 2,5-diethyl-6-(4-(5-hydroxy-6-methylpyri midine-4-carbonyl)piperazin-1-yl)-7-oxo-[ 1,2,4]triazolo[1,5-a]pyrimidin-4(7H)-yl)ac etamide |
| Example 29 | 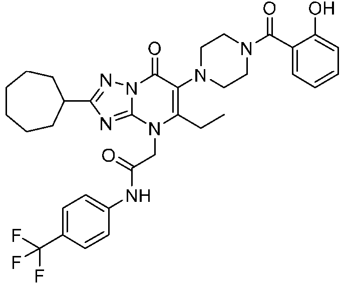 | 2-(2-cycloheptyl-5-ethyl-6-(4-(2-hydroxyb enzoyl)piperazin-1-yl)-7-oxo-[1,2,4]triazol o[1,5-a]pyrimidin-4(7H)-yl)-N-(4-(trifluor omethyl)phenyl)acetamide |
| Example 30 | 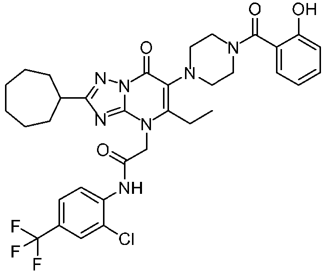 | N-(2-chloro-4-(trifluoromethyl)phenyl)-2-( 2-cycloheptyl-5-ethyl-6-(4-(2-hydroxybenz oyl)piperazin-1-yl)-7-oxo-[1,2,4]triazolo[1, 5-a]pyrimidin-4(7H)-yl)acetamide |
| Example 31 | 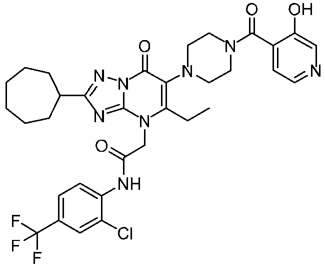 | N-(2-chloro-4-(trifluoromethyl)phenyl)-2-( 2-cycloheptyl-5-ethyl-6-(4-(3-hydroxyison icotinoyl)piperazin-1-yl)-7-oxo-[1,2,4]triaz olo[1,5-a]pyrimidin-4(7H)-yl)acetamide |
| Example 32 | 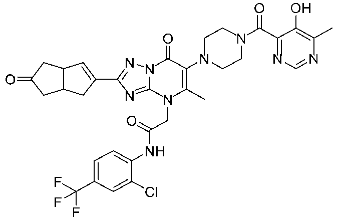 | N-(2-chloro-4-(trifluoromethyl)phenyl)-2-( 6-(4-(5-hydroxy-6-methylpyrimidine-4-car bonyl)piperazin-1-yl)-5-methyl-7-oxo-2-(5 -oxo-1,3a,4,5,6,6a-hexahydropentalen-2-yl )-[1,2,4]triazolo[1,5-a]pyrimidin-4(7H)-yl) acetamide |
| Example 33 | 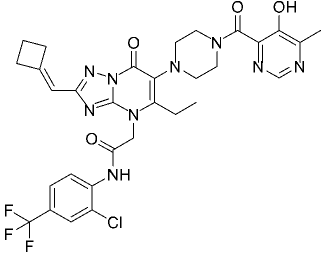 | N-(2-chloro-4-(trifluoromethyl)phenyl)-2-( 2-(cyclobutylidenemethyl)-5-ethyl-6-(4-(5-hydroxy-6-methylpyrimidine-4-carbonyl)p iperazin-1-yl)-7-oxo-[1,2,4]triazolo[1,5-a]p yrimidin-4(7H)-yl)acetamide |
| Example 34 | 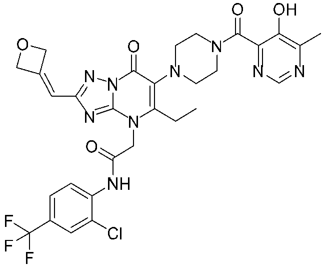 | N-(2-chloro-4-(trifluoromethyl)phenyl)-2-( 5-ethyl-6-(4-(5-hydroxy-6-methylpyrimidi ne-4-carbonyl)piperazin-1-yl)-2-(oxetan-3-ylidenemethyl)-7-oxo-[1,2,4]triazolo[1,5-a ]pyrimidin-4(7H)-yl)acetamide |
| Example 35 | 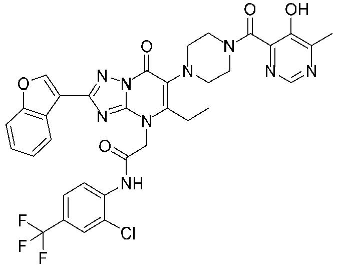 | 2-(2-(benzofuran-3-yl)-5-ethyl-6-(4-(5-hyd roxy-6-methylpyrimidine-4-carbonyl)piper azin-1-yl)-7-oxo-[1,2,4]triazolo[1,5-a]pyri midin-4(7H)-yl)-N-(2-chloro-4-(trifluorom ethyl)phenyl)acetamide |
| Example 36 | 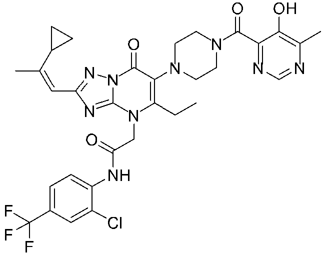 | (E)-N-(2-chloro-4-(trifluoromethyl)phenyl) -2-(2-(2-cyclopropylprop-1-en-1-yl)-5-ethy 1-6-(4-(5-hydroxy-6-methylpyrimidine-4-c arbonyl)piperazin-1-yl)-7-oxo-[1,2,4]triazo lo[1,5-a]pyrimidin-4(7H)-yl)acetamide |
| Example 37 | 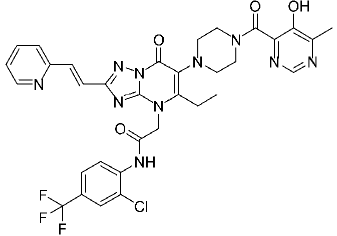 | (E)-N-(2-chloro-4-(trifluoromethyl)phenyl) -2-(5-ethyl-6-(4-(5-hydroxy-6-methylpyri midine-4-carbonyl)piperazin-1-yl)-7-oxo-2 -(2-(pyridin-2-yl)vinyl)-[1,2,4]triazolo[1,5-a]pyrimidin-4(7H)-yl)acetamide |
| Example 38 | 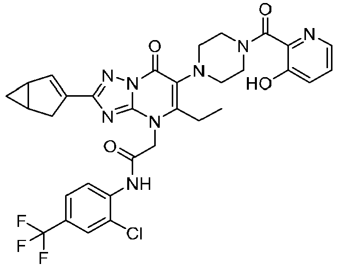 | 2-(2-(bicyclo[3.1.0]hex-2-en-3-yl)-5-ethyl-6-(4-(3-hydroxypicolinoyl)piperazin-1-yl)-7-oxo-[1,2,4]triazolo[1,5-a]pyrimidin-4(7H )-yl)-N-(2-chloro-4-(trifluoromethyl)pheny l)acetamide |
| Example 39 | 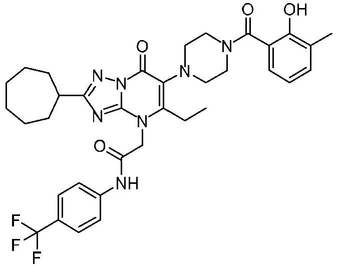 | 2-(2-cycloheptyl-5-ethyl-6-(4-(2-hydroxy-3-methylbenzoyl)piperazin-1-yl)-7-oxo-[1, 2,4]triazolo[1,5-a]pyrimidin-4(7H)-yl)-N-( 4-(trifluoromethyl)phenyl)acetamide |
| Example 40 | 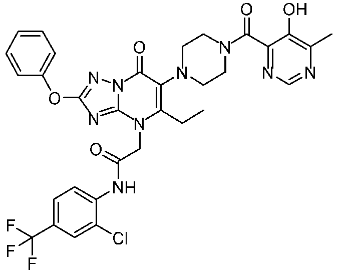 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-phe noxy-[1,2,4]triazolo[1,5-a]pyrimidin-4(7H) -yl)acetamide |
| Example 41 | 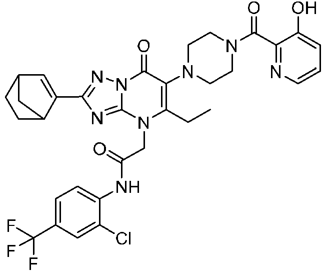 | 2-(2-(bicyclo[2.2.1]hept-2-en-2-yl)-5-ethyl -6-(4-(3-hydroxypicolinoyl)piperazin-1-yl) -7-oxo-[1,2,4]triazolo[1,5-a]pyrimidin-4(7 H)-yl)-N-(2-chloro-4-(trifluoromethyl)phe nyl)acetamide |
| Example 42 | 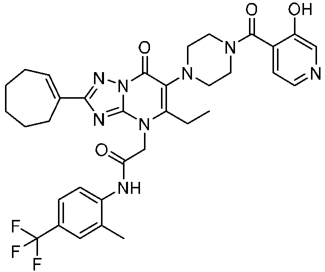 | 2-(2-(cyclohept-1-en-1-yl)-5-ethyl-6-(4-(3-hydroxyisonicotinoyl)piperazin-1-yl)-7-ox o-[1,2,4]triazolo[1,5-a]pyrimidin-4(7H)-yl) -N-(2-methyl-4-(trifluoromethyl)phenyl)ac etamide |
| Example 43 | 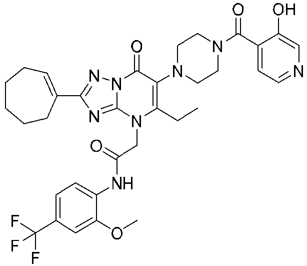 | 2-(2-(cyclohept-1-en-1-yl)-5-ethyl-6-(4-(3-hydroxyisonicotinoyl)piperazin-1-yl)-7-ox o-[1,2,4]triazolo[1,5-a]pyrimidin-4(7H)-yl) -N-(2-methoxy-4-(trifluoromethyl)phenyl) acetamide |
| Example 44 | 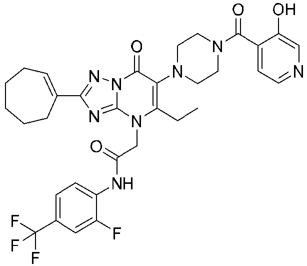 | 2-(2-(cyclohept-1-en-1-yl)-5-ethyl-6-(4-(3-hydroxyisonicotinoyl)piperazin-1-yl)-7-ox o-[1,2,4]triazolo[1,5-a]pyrimidin-4(7H)-yl) -N-(2-fluoro-4-(trifluoromethyl)phenyl)ace tamide |
| Example 45 | 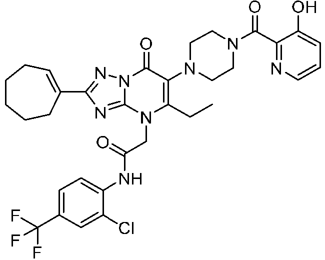 | N-(2-chloro-4-(trifluoromethyl)phenyl)-2-( 2-(cyclohept-1-en-1-yl)-5-ethyl-6-(4-(3-hy droxypicolinoyl)piperazin-1-y1)-7-oxo-[1,2 ,4]triazolo[1,5-a]pyrimidin-4(7H)-yl)aceta mide |
| Example 46 | 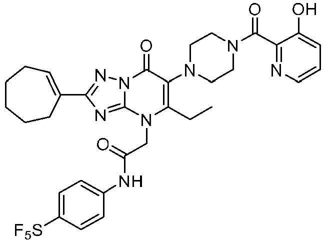 | 2-(2-(cyclohept-1-en-1-yl)-5-ethyl-6-(4-(3-hydroxypicolinoyl)piperazin-1-yl)-7-oxo-[ 1,2,4]triazolo[1,5-a]pyrimidin-4(7H)-yl)-N -(4-(pentafluoro-λ⁶ -sulfaneyl)phenyl)aceta mide |
| Example 47 | 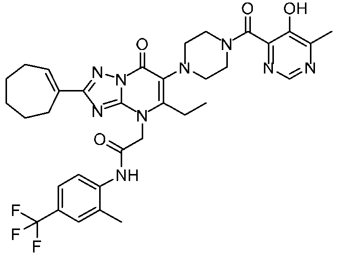 | 2-(2-(cyclohept-1-en-1-yl)-5-ethyl-6-(4-(5-hydroxy-6-methylpyrimidine-4-carbonyl)p iperazin-1-yl)-7-oxo-[1,2,4]triazolo[1,5-a]p yrimidin-4(7H)-yl)-N-(2-methyl-4-(trifluor omethyl)phenyl)acetamide |
| Example 48 | 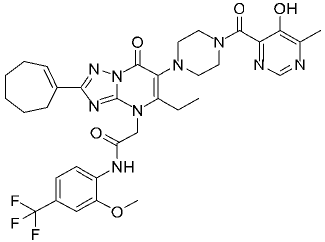 | 2-(2-(cyclohept-1-en-1-yl)-5-ethyl-6-(4-(5-hydroxy-6-methylpyrimidine-4-carbonyl)p iperazin-1-yl)-7-oxo-[1,2,4]triazolo[1,5-a]p yrimidin-4(7H)-yl)-N-(2-methoxy-4-(triflu oromethyl)phenyl)acetamide |
| Example 49 | 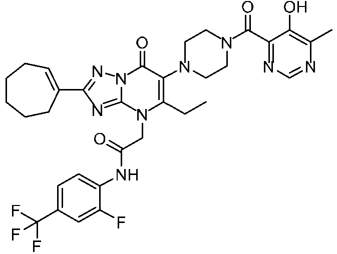 | 2-(2-(cyclohept-1-en-1-yl)-5-ethyl-6-(4-(5-hydroxy-6-methylpyrimidine-4-carbonyl)p iperazin-1-yl)-7-oxo-[1,2,4]triazolo[1,5-a]p yrimidin-4(7H)-yl)-N-(2-fluoro-4-(trifluor omethyl)phenyl)acetamide |
| Example 50 | 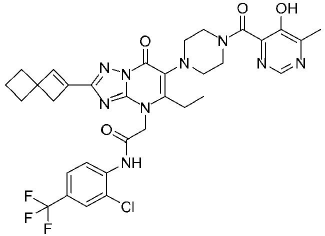 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-(spi ro[3.3]hept-1-en-2-yl)-[1,2,4]triazolo[1,5-a ]pyrimidin-4(7H)-yl)acetamide |
| Example 51 | 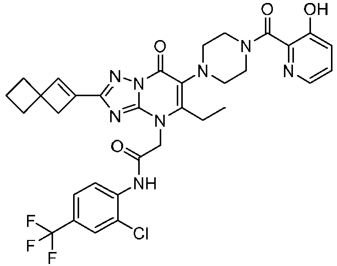 | N-(2-chloro-4-(trifluoromethyl)phenyl)-2-( 5-ethyl-6-(4-(3-hydroxypicolinoyl)piperazi n-1-yl)-7-oxo-2-(spiro[3.3]hept-1-en-2-yl)-[1,2,4]triazolo[1,5-a]pyrimidin-4(7H)-yl)ac etamide |
| Example 52 | 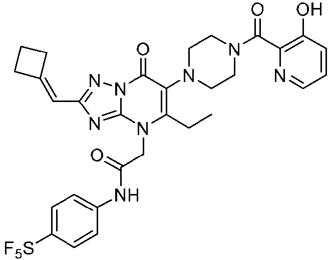 | 2-(2-(cyclobutylidenemethyl)-5-ethyl-6-(4-(3-hydroxypicolinoyl)piperazin-1-yl)-7-ox o-[1,2,4]triazolo[1,5-a]pyrimidin-4(7H)-yl) -N-(4-(pentafluoro-λ⁶-sulfaneyl)phenyl)ace tamide |
| Example 53 | 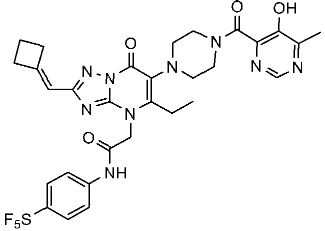 | 2-(2-(cyclobutylidenemethyl)-5-ethyl-6-(4-(5-hydroxy-6-methylpyrimidine-4-carbony 1)piperazin-1-yl)-7-oxo-[1,2,4]triazolo[1,5-a]pyrimidin-4(7H)-yl)-N-(4-(pentafluoro-λ ⁶-sulfaneyl)phenyl)acetamide |
| Example 54 | 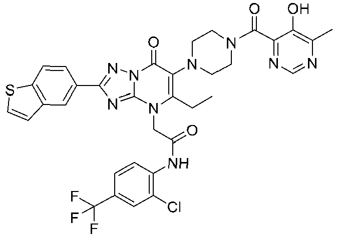 | 2-(2-(benzo[b]thiophen-5-yl)-5-ethyl-6-(4-(5-hydroxy-6-methylpyrimidine-4-carbony 1)piperazin-1-yl)-7-oxo-[1,2,4]triazolo[1,5-a]pyrimidin-4(7H)-yl)-N-(2-chloro-4-(trifl uoromethyl)phenyl)acetamide |
| Example 55 | 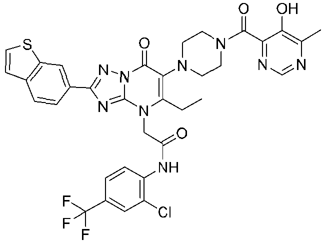 | 2-(2-(benzo[b]thiophen-6-yl)-5-ethyl-6-(4-(5-hydroxy-6-methylpyrimidine-4-carbony 1)piperazin-1-yl)-7-oxo-[1,2,4]triazolo[1,5-a]pyrimidin-4(7H)-yl)-N-(2-chloro-4-(trifl uoromethyl)phenyl)acetamide |
| Example 56 | 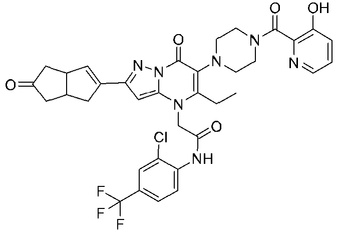 | N-(2-chloro-4-(trifluoromethyl)phenyl)-2-( 5-ethyl-6-(4-(3-hydroxypicolinoyl)piperazi n-1-yl)-7-oxo-2-(5-oxo-1,3a,4,5,6,6a-hexa hydropentalen-2-yl)pyrazolo[1,5-a]pyrimid in-4(7H)-yl)acetamide |
| Example 57 | 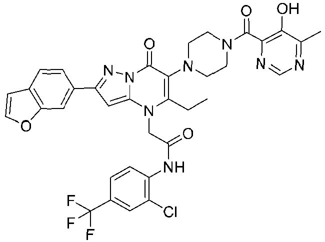 | 2-(2-(benzofuran-6-yl)-5-ethyl-6-(4-(5-hyd roxy-6-methylpyrimidine-4-carbonyl)piper azin-1-yl)-7-oxopyrazolo[1,5-a]pyrimidin-4(7H)-yl)-N-(2-chloro-4-(trifluoromethyl)p henyl)acetamide |
| Example 58 | 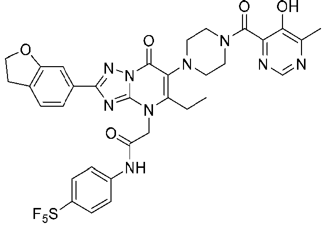 | 2-(2-(2,3-dihydrobenzofuran-6-yl)-5-ethyl-6-(4-(5-hydroxy-6-methylpyrimidine-4-car bonyl)piperazin-1-yl)-7-oxo-[1,2,4]triazolo [1,5-a]pyrimidin-4(7H)-yl)-N-(4-(pentaflu oro-À⁶ -sulfaneyl)phenyl)acetamide |
| Example 59 | 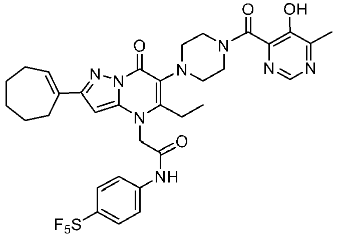 | 2-(2-(cyclohept-1-en-1-yl)-5-ethyl-6-(4-(5-hydroxy-6-methylpyrimidine-4-carbonyl)p iperazin-1-yl)-7-oxopyrazolo[1,5-a]pyrimi din-4(7H)-y1)-N-(4-(pentafluoro-λ⁶-sulfane yl)phenyl)acetamide |
| Example 60 | 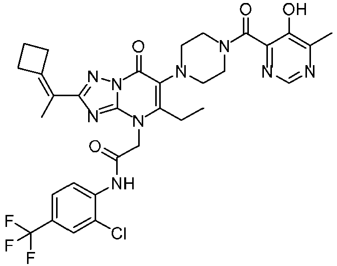 | N-(2-chloro-4-(trifluoromethyl)phenyl)-2-( 2-(1-cyclobutylideneethyl)-5-ethyl-6-(4-(5-hydroxy-6-methylpyrimidine-4-carbonyl)p iperazin-1-yl)-7-oxo-[1,2,4]triazolo[1,5-a]p yrimidin-4(7H)-yl)acetamide |
| Example 61 | 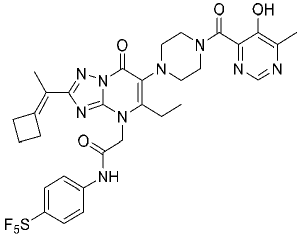 | 2-(2-(1-cyclobutylideneethyl)-5-ethyl-6-(4-(5-hydroxy-6-methylpyrimidine-4-carbony 1)piperazin-1-yl)-7-oxo-[1,2,4]triazolo[1,5-a]pyrimidin-4(7H)-yl)-N-(4-(pentafluoro-1 ⁶-sulfaneyl)phenyl)acetamide |
| Example 62 | 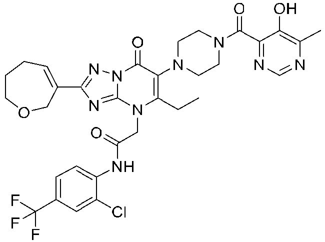 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-(2,5 ,6,7-tetrahydrooxepin-3-yl)-[1,2,4]triazolo[ 1,5-a]pyrimidin-4(7H)-yl)acetamide |
| Example 63 | 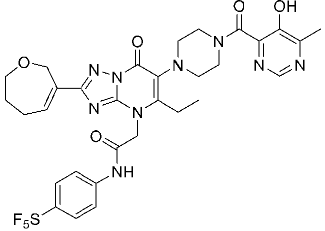 | 2-(5-ethyl-6-(4-(5-hydroxy-6-methylpyrim idine-4-carbonyl)piperazin-1-yl)-7-oxo-2-( 2,5,6,7-tetrahydrooxepin-3-yl)-[1,2,4]triaz olo[1,5-a]pyrimidin-4(7H)-yl)-N-(4-(penta fluoro-λ⁶ -sulfaneyl)phenyl)acetamide |
| Example 64 | 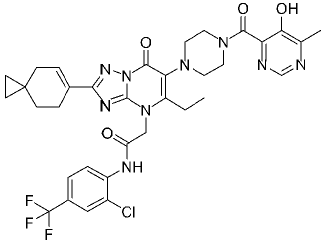 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-(spi ro[2.5]oct-5-en-6-yl)-[1,2,4]triazolo[1,5-a] pyrimidin-4(7H)-yl)acetamide |
| Example 65 | 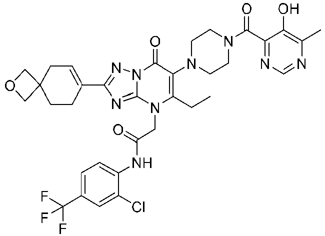 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-(2-oxaspiro[3.5]non-6-en-7-yl)-[1,2,4]triazolo [1,5-a]pyrimidin-4(7H)-yl)acetamide |
| Example 66 | 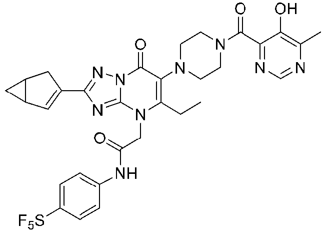 | 2-(2-(bicyclo[3.1.0]hex-2-en-3-yl)-5-ethyl-6-(4-(5-hydroxy-6-methylpyrimidine-4-car bonyl)piperazin-1-yl)-7-oxo-[1,2,4]triazolo [1,5-a]pyrimidin-4(7H)-yl)-N-(4-(pentaflu oro-λ⁶-sulfaneyl)phenyl)acetamide |
| Example 67 | 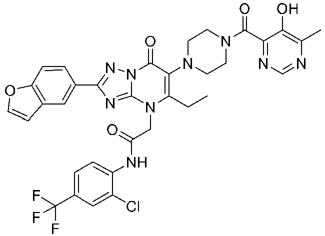 | 2-(2-(benzofuran-5-yl)-5-ethyl-6-(4-(5-hyd roxy-6-methylpyrimidine-4-carbonyl)piper azin-1-yl)-7-oxo-[1,2,4]triazolo[1,5-a]pyri midin-4(7H)-yl)-N-(2-chloro-4-(trifluorom ethyl)phenyl)acetamide |
| Example 68 | 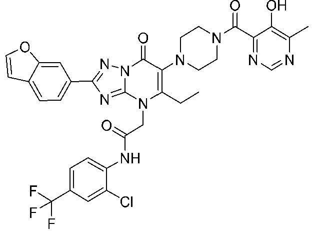 | 2-(2-(benzofuran-6-yl)-5-ethyl-6-(4-(5-hyd roxy-6-methylpyrimidine-4-carbonyl)piper azin-1-yl)-7-oxo-[1,2,4]triazolo[1,5-a]pyri midin-4(7H)-yl)-N-(2-chloro-4-(trifluorom ethyl)phenyl)acetamide |
| Example 69 | 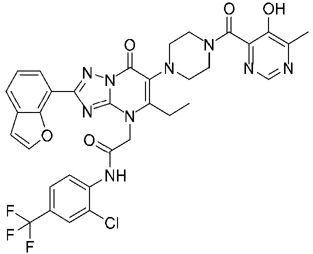 | 2-(2-(benzofuran-7-yl)-5-ethyl-6-(4-(5-hyd roxy-6-methylpyrimidine-4-carbonyl)piper azin-1-yl)-7-oxo-[1,2,4]triazolo[1,5-a]pyri midin-4(7H)-yl)-N-(2-chloro-4-(trifluorom ethyl)phenyl)acetamide |
| Example 70 | 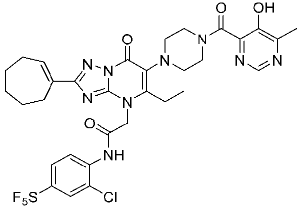 | N-(2-chloro-4-(pentafluoro-λ⁶ -sulfaneyl)ph enyl)-2-(2-(cyclohept-1-en-1-yl)-5-ethyl-6-(4-(5-hydroxy-6-methylpyrimidine-4-carbo nyl)piperazin-1-yl)-7-oxo-[1,2,4]triazolo[1, 5-a]pyrimidin-4(7H)-yl)acetamide |
| Example 71 | 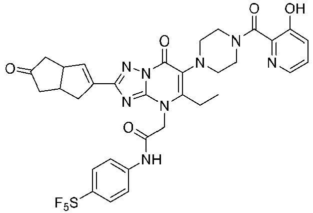 | 2-(5-ethyl-6-(4-(3-hydroxypicolinoyl)piper azin-1-yl)-7-oxo-2-(5-oxo-1,3a,4,5,6,6a-he xahydropentalen-2-yl)-[1,2,4]triazolo[1,5-a ]pyrimidin-4(7H)-yl)-N-(4-(pentafluoro-1⁶ -sulfaneyl)phenyl)acetamide |
| Example 72 | 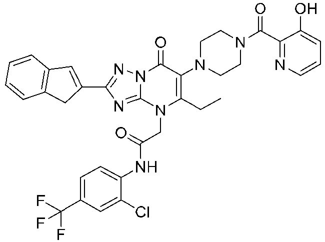 | N-(2-chloro-4-(trifluoromethyl)phenyl)-2-( 5-ethyl-6-(4-(3-hydroxypicolinoyl)piperazi n-1-yl)-2-(1H-inden-2-yl)-7-oxo-[1,2,4]tria zolo[1,5-a]pyrimidin-4(7H)-yl)acetamide |
| Example 73 | 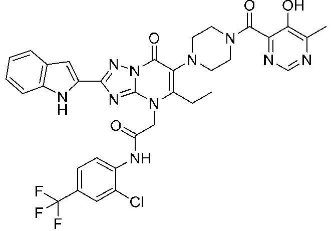 | N-(2-chloro-4-(trifluoromethyl)phenyl)-2-( 5-ethyl-6-(4-(5-hydroxy-6-methylpyrimidi ne-4-carbonyl)piperazin-1-yl)-2-(1H-indol-2-yl)-7-oxo-[1,2,4]triazolo[1,5-a]pyrimidin -4(7H)-yl)acetamide |
| Example 74 | 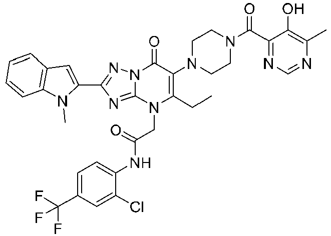 | N-(2-chloro-4-(trifluoromethyl)phenyl)-2-( 5-ethyl-6-(4-(5-hydroxy-6-methylpyrimidi ne-4-carbonyl)piperazin-1-yl)-2-(1-methyl-1H-indol-2-yl)-7-oxo-[1,2,4]triazolo[1,5-a] pyrimidin-4(7H)-yl)acetamide |
| Example 75 | 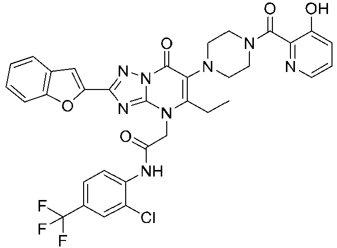 | 2-(2-(benzofuran-2-yl)-5-ethyl-6-(4-(3-hyd roxypicolinoyl)piperazin-1-yl)-7-oxo-[1,2, 4]triazolo[1,5-a]pyrimidin-4(7H)-yl)-N-(2-chloro-4-(trifluoromethyl)phenyl)acetamid e |
| Example 76 | 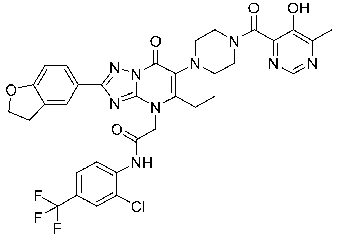 | N-(2-chloro-4-(trifluoromethyl)phenyl)-2-( 2-(2,3-dihydrobenzofuran-5-yl)-5-ethyl-6-( 4-(5-hydroxy-6-methylpyrimidine-4-carbo nyl)piperazin-1-yl)-7-oxo-[1,2,4]triazolo[1, 5-a]pyrimidin-4(7H)-yl)acetamide |
| Example 77 | 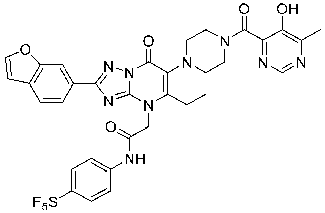 | 2-(2-(benzofuran-6-yl)-5-ethyl-6-(4-(5-hyd roxy-6-methylpyrimidine-4-carbonyl)piper azin-1-yl)-7-oxo-[1,2,4]triazolo[1,5-a]pyri midin-4(7H)-yl)-N-(4-(pentafluoro-λ⁶ -sulf aneyl)phenyl)acetamide |
| Example 78 | 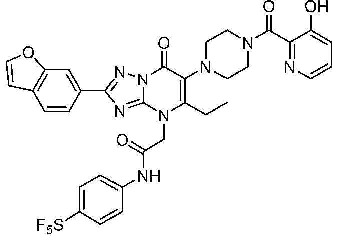 | 2-(2-(benzofuran-6-yl)-5-ethyl-6-(4-(3-hyd roxypicolinoyl)piperazin-1-yl)-7-oxo-[1,2, 4]triazolo[1,5-a]pyrimidin-4(7H)-yl)-N-(4-(pentafluoro-λ⁶-sulfaneyl)phenyl)acetamid e |
| Example 79 | 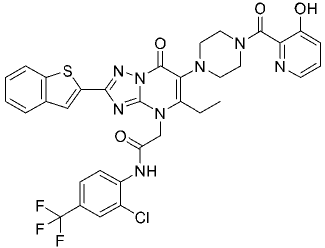 | 2-(2-(benzo[b]thiophen-2-yl)-5-ethyl-6-(4-(3-hydroxypicolinoyl)piperazin-1-yl)-7-ox o-[1,2,4]triazolo[1,5-a]pyrimidin-4(7H)-yl) -N-(2-chloro-4-(trifluoromethyl)phenyl)ac etamide |
| Example 80 | 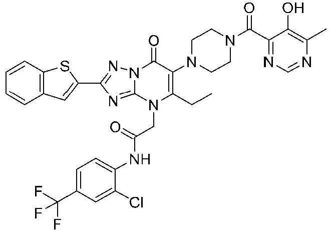 | 2-(2-(benzo[b]thiophen-2-yl)-5-ethyl-6-(4-(5-hydroxy-6-methylpyrimidine-4-carbony l)piperazin-1-yl)-7-oxo-[1,2,4]triazolo[1,5-a]pyrimidin-4(7H)-yl)-N-(2-chloro-4-(trifl uoromethyl)phenyl)acetamide |
| Example 81 | 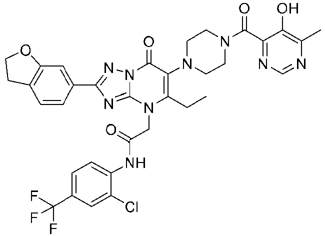 | N-(2-chloro-4-(trifluoromethyl)phenyl)-2-( 2-(2,3-dihydrobenzofuran-6-yl)-5-ethyl-6-( 4-(5-hydroxy-6-methylpyrimidine-4-carbo nyl)piperazin-1-yl)-7-oxo-[1,2,4]triazolo[1, 5-a]pyrimidin-4(7H)-yl)acetamide |
| Example 82 | 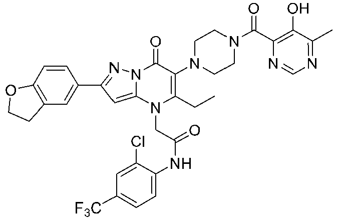 | N-(2-chloro-4-(trifluoromethyl)phenyl)-2-( 2-(2,3-dihydrobenzofuran-5-yl)-5-ethyl-6-( 4-(5-hydroxy-6-methylpyrimidine-4-carbo nyl)piperazin-1-yl)-7-oxopyrazolo[1,5-a]p yrimidin-4(7H)-yl)acetamide |
| Example 83 | 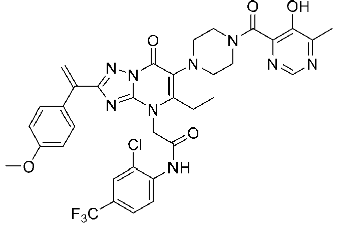 | N-(2-chloro-4-(trifluoromethyl)phenyl)-2-( 5-ethyl-6-(4-(5-hydroxy-6-methylpyrimidi ne-4-carbonyl)piperazin-1-yl)-2-(1-(4-met hoxyphenyl)vinyl)-7-oxo-[1,2,4]triazolo[1, 5-a]pyrimidin-4(7H)-yl)acetamide |
| Example 84 | 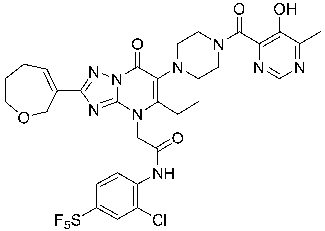 | N-(2-chloro-4-(pentafluoro-λ⁶-sulfaneyl)ph enyl)-2-(5-ethyl-6-(4-(5-hydroxy-6-methyl pyrimidine-4-carbonyl)piperazin-1-yl)-7-o xo-2-(2,5,6,7-tetrahydrooxepin-3-yl)-[1,2,4 ]triazolo[1,5-a]pyrimidin-4(7H)-yl)acetami de |
| Example 85 | 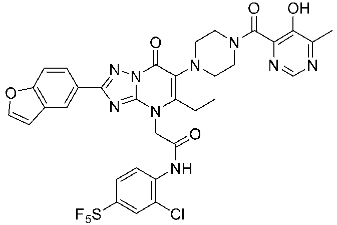 | 2-(2-(benzofuran-5-yl)-5-ethyl-6-(4-(5-hyd roxy-6-methylpyrimidine-4-carbonyl)piper azin-1-yl)-7-oxo-[1,2,4]triazolo[1,5-a]pyri midin-4(7H)-yl)-N-(2-chloro-4-(pentafluor o-λ⁶-sulfaneyl)phenyl)acetamide |
| Example 86 | 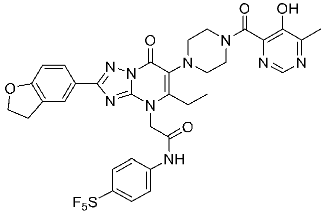 | 2-(2-(2,3-dihydrobenzofuran-5-yl)-5-ethyl-6-(4-(5-hydroxy-6-methylpyrimidine-4-car bonyl)piperazin-1-yl)-7-oxo-[1,2,4]triazolo [1,5-a]pyrimidin-4(7H)-yl)-N-(4-(pentaflu oro-λ⁶-sulfaneyl)phenyl)acetamide |
| Example 87 | 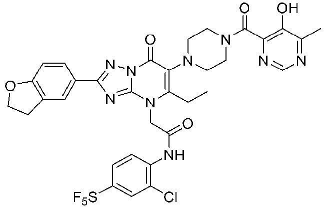 | N-(2-chloro-4-(pentafluoro-λ⁶-sulfaneyl)ph enyl)-2-(2-(2,3-dihydrobenzofuran-5-yl)-5-ethyl-6-(4-(5-hydroxy-6-methylpyrimidine -4-carbonyl)piperazin-1-yl)-7-oxo-[1,2,4]tr iazolo[1,5-a]pyrimidin-4(7H)-yl)acetamide |
| Example 88 | 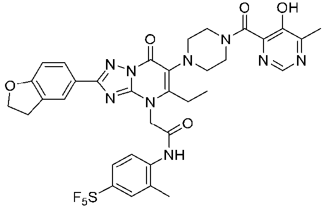 | 2-(2-(2,3-dihydrobenzofuran-5-yl)-5-ethyl-6-(4-(5-hydroxy-6-methylpyrimidine-4-car bonyl)piperazin-1-yl)-7-oxo-[1,2,4]triazolo [1,5-a]pyrimidin-4(7H)-yl)-N-(2-methyl-4-(pentafluoro-λ⁶-sulfaneyl)phenyl)acetamid e |
| Example 89 | 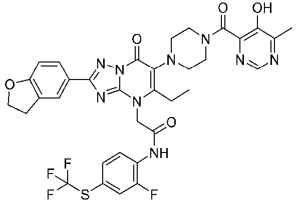 | 2-(2-(2,3-dihydrobenzofuran-5-yl)-5-ethyl-6-(4-(5-hydroxy-6-methylpyrimidine-4-car bonyl)piperazin-1-yl)-7-oxo-[1,2,4]triazolo [1,5-a]pyrimidin-4(7H)-yl)-N-(2-fluoro-4-( (trifluoromethyl)thio)phenyl)acetamide |
| Example 90 | 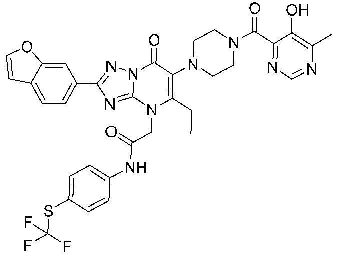 | 2-(2-(benzofuran-6-yl)-5-ethyl-6-(4-(5-hyd roxy-6-methylpyrimidine-4-carbonyl)piper azin-1-yl)-7-oxo-[1,2,4]triazolo[1,5-a]pyri midin-4(7H)-yl)-N-(4-((trifluoromethyl)thi o)phenyl)acetamide |
| Example 91 | 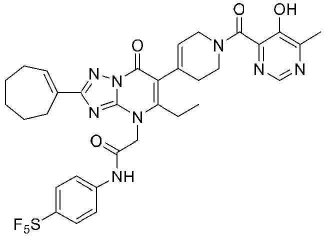 | 2-(2-(cyclohept-1-en-1-yl)-5-ethyl-6-(1-(5-hydroxy-6-methylpyrimidine-4-carbonyl)-1,2,3,6-tetrahydropyridin-4-yl)-7-oxo-[1,2, 4]triazolo[1,5-a]pyrimidin-4(7H)-yl)-N-(4-(pentafluoro-λ⁶-sulfaneyl)phenyl)acetamid e |
| Example 92 | 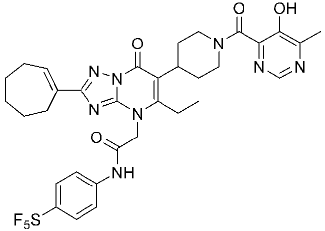 | 2-(2-(cyclohept-1-en-1-yl)-5-ethyl-6-(1-(5-hydroxy-6-methylpyrimidine-4-carbonyl)p iperidin-4-yl)-7-oxo-[1,2,4]triazolo[1,5-a]p yrimidin-4(7H)-yl)-N-(4-(pentafluoro-λ⁶-s ulfaneyl)phenyl)acetamide |
| Example 93 | 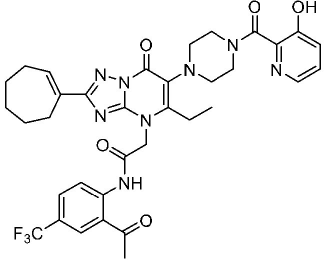 | N-(2-acetyl-4-(trifluoromethyl)phenyl)-2-( 2-(cyclohept-1-en-1-yl)-5-ethyl-6-(4-(3-hy droxypicolinoyl)piperazin-1-yl)-7-oxo-[1,2 ,4]triazolo[1,5-a]pyrimidin-4(7H)-yl)aceta mide |
| Example 94 | 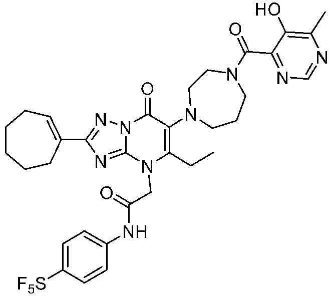 | 2-(2-(cyclohept-1-en-1-yl)-5-ethyl-6-(4-(5-hydroxy-6-methylpyrimidine-4-carbonyl)-1,4-diazepan-1-yl)-7-oxo-[1,2,4]triazolo[1, 5-a]pyrimidin-4(7H)-yl)-N-(4-(pentafluoro -λ⁶ -sulfaneyl)phenyl)acetamide |
| Example 95 | 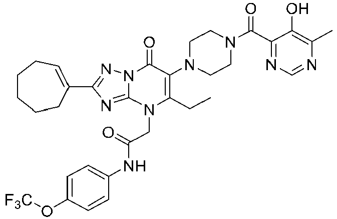 | 2-(2-(cyclohept-1-en-1-yl)-5-ethyl-6-(4-(5-hydroxy-6-methylpyrimidine-4-carbonyl)p iperazin-1-yl)-7-oxo-[1,2,4]triazolo[1,5-a]p yrimidin-4(7H)-yl)-N-(4-(trifluoromethoxy )phenyl)acetamide |
| Example 96 | 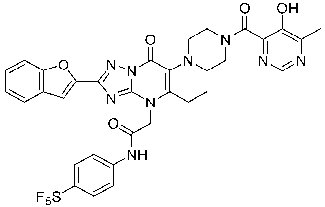 | 2-(2-(benzofuran-2-yl)-5-ethyl-6-(4-(5-hyd roxy-6-methylpyrimidine-4-carbonyl)piper azin-1-yl)-7-oxo-[1,2,4]triazolo[1,2,4]pyri midin-4(7H)-yl)-N-(4-(pentafluoro-λ⁶-sulf aneyl)phenyl)acetamide |
| Example 97 | 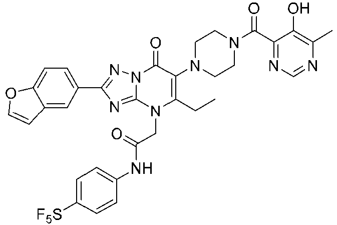 | 2-(2-(benzofuran-5-yl)-5-ethyl-6-(4-(5-hyd roxy-6-methylpyrimidine-4-carbonyl)piper azin-1-yl)-7-oxo-[1,2,4]triazolo[1,5-a]pyri midin-4(7H)-yl)-N-(4-(pentafluoro-λ⁶-sulf aneyl)phenyl)acetamide |
| Example 98 | 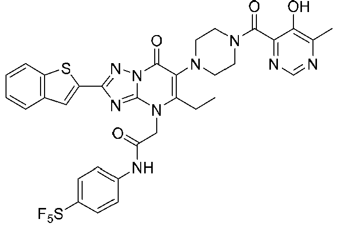 | 2-(2-(benzo[b]thiophen-2-yl)-5-ethyl-6-(4-(5-hydroxy-6-methylpyrimidine-4-carbony 1)piperazin-1-yl)-7-oxo-[1,2,4]triazolo[1,5-a]pyrimidin-4(7H)-yl)-N-(4-(pentafluoro-λ ⁶-sulfaneyl)phenyl)acetamide |
| Example 99 | 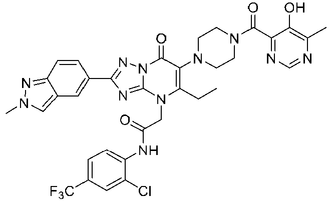 | N-(2-chloro-4-(trifluoromethyl)phenyl)-2-( 5-ethyl-6-(4-(5-hydroxy-6-methylpyrimidi ne-4-carbonyl)piperazin-1-yl)-2-(2-methyl-2H-indazol-5-yl)-7-oxo-[1,2,4]triazolo[1,5-a]pyrimidin-4(7H)-yl)acetamide |
| Example 100 | 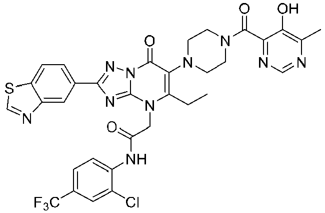 | 2-(2-(benzo[d]thiazol-5-yl)-5-ethyl-6-(4-(5 -hydroxy-6-methylpyrimidine-4-carbonyl) piperazin-1-yl)-7-oxo-[1,2,4]triazolo[1,5-a] pyrimidin-4(7H)-yl)-N-(2-chloro-4-(trifluo romethyl)phenyl)acetamide |
| Example 101 | 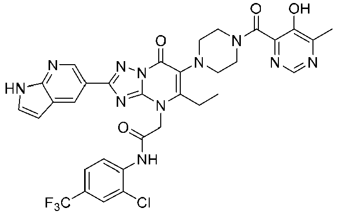 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-(1H -pyrrolo[2,3-b]pyridin-5-yl)-[1,2,4]triazolo [1,5-a]pyrimidin-4(7H)-yl)acetamide |
| Example 102 | 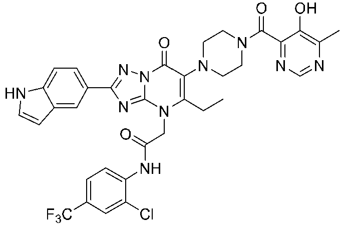 | N-(2-chloro-4-(trifluoromethyl)phenyl)-2-( 5-ethyl-6-(4-(5-hydroxy-6-methylpyrimidi ne-4-carbonyl)piperazin-1-yl)-2-(1H-indol-5-yl)-7-oxo-[1,2,4]triazolo[1,5-a]pyrimidin -4(7H)-yl)acetamide |
| Example 103 | 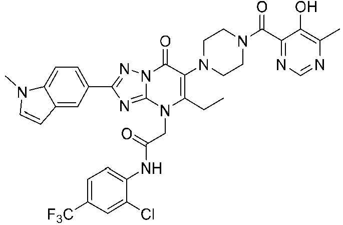 | N-(2-chloro-4-(trifluoromethyl)phenyl)-2-( 5-ethyl-6-(4-(5-hydroxy-6-methylpyrimidi ne-4-carbonyl)piperazin-1-yl)-2-(1-methyl-1H-indol-5-yl)-7-oxo-[1,2,4]triazolo[1,5-a] pyrimidin-4(7H)-yl)acetamide |
| Example 104 | 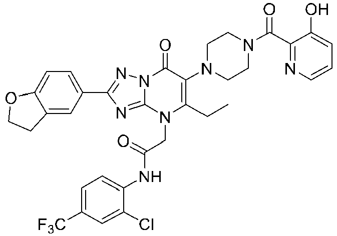 | N-(2-chloro-4-(trifluoromethyl)phenyl)-2-( 2-(2,3-dihydrobenzofuran-5-yl)-5-ethyl-6-( 4-(3-hydroxypicolinoyl)piperazin-1-yl)-7-o xo-[1,2,4]triazolo[1,5-a]pyrimidin-4(7H)-y l)acetamide |
| Example 105 | 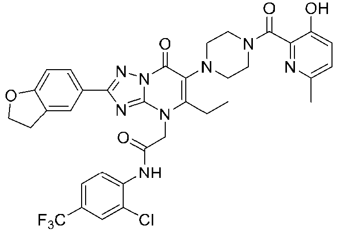 | N-(2-chloro-4-(trifluoromethyl)phenyl)-2-( 2-(2,3-dihydrobenzofuran-5-yl)-5-ethyl-6-( 4-(3-hydroxy-6-methylpicolinoyl)piperazin -1-yl)-7-oxo-[1,2,4]triazolo[1,5-a]pyrimidi n-4(7H)-yl)acetamide |
| Example 106 | 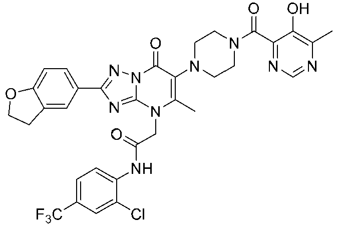 | N-(2-chloro-4-(trifluoromethyl)phenyl)-2-( 2-(2,3-dihydrobenzofuran-5-yl)-6-(4-(5-hy droxy-6-methylpyrimidine-4-carbonyl)pipe razin-1-yl)-5-methyl-7-oxo-[1,2,4]triazolo[ 1,5-a]pyrimidin-4(7H)-yl)acetamide |
| Example 107 | 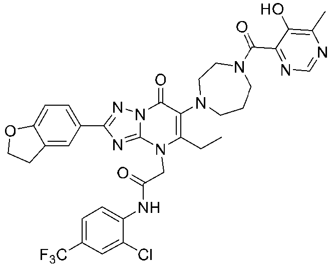 | N-(2-chloro-4-(trifluoromethyl)phenyl)-2-( 2-(2,3-dihydrobenzofuran-5-yl)-5-ethyl-6-( 4-(5-hydroxy-6-methylpyrimidine-4-carbo nyl)-1,4-diazepan-1-yl)-7-oxo-[1,2,4]triazo lo[1,5-a]pyrimidin-4(7H)-yl)acetamide |
| Example 108 | 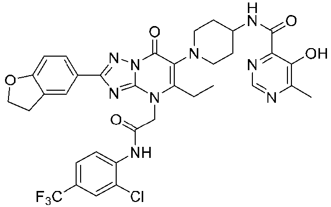 | N-(1-(4-(2-((2-chloro-4-(trifluoromethyl)p henyl)amino)-2-oxoethyl)-2-(2,3-dihydrob enzofuran-5-yl)-5-ethyl-7-oxo-4,7-dihydro -[1,2,4]triazolo[1,5-a]pyrimidin-6-yl)piperi din-4-yl)-5-hydroxy-6-methylpyrimidine-4 -carboxamide |
| Example 109 | 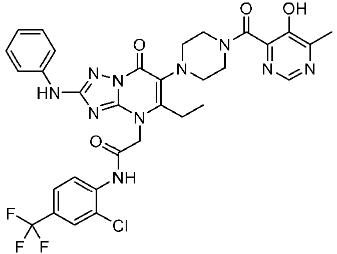 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-(ph enylamino)-[1,2,4]triazolo[1,5-a]pyrimidin -4(7H)-yl)acetamide |
| Example 110 | 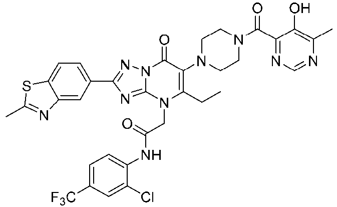 | N-(2-chloro-4-(trifluoromethyl)phenyl)-2-( 5-ethyl-6-(4-(5-hydroxy-6-methylpyrimidi ne-4-carbonyl)piperazin-1-yl)-2-(2-methyl benzo[d]thiazol-5-yl)-7-oxo-[1,2,4]triazolo [1,5-a]pyrimidin-4(7H)-yl)acetamide |
| Example 111 | 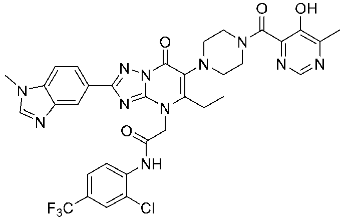 | N-(2-chloro-4-(trifluoromethyl)phenyl)-2-( 5-ethyl-6-(4-(5-hydroxy-6-methylpyrimidi ne-4-carbonyl)piperazin-1-yl)-2-(1-methyl-1H-benzo[d]imidazol-5-yl)-7-oxo-[1,2,4]tr iazolo[1,5-a]pyrimidin-4(7H)-yl)acetamide |
| Example 112 | 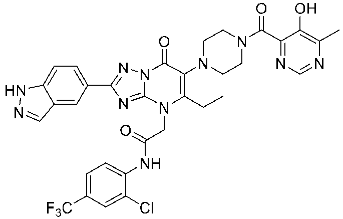 | N-(2-chloro-4-(trifluoromethyl)phenyl)-2-( 5-ethyl-6-(4-(5-hydroxy-6-methylpyrimidi ne-4-carbonyl)piperazin-1-yl)-2-(1H-indaz ol-5-yl)-7-oxo-[1,2,4]triazolo[1,5-a]pyrimi din-4(7H)-yl)acetamide |
| Example 113 | 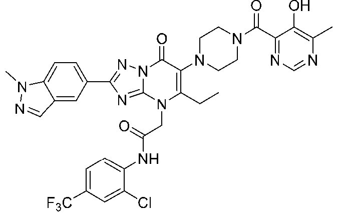 | N-(2-chloro-4-(trifluoromethyl)phenyl)-2-( 5-ethyl-6-(4-(5-hydroxy-6-methylpyrimidi ne-4-carbonyl)piperazin-1-yl)-2-(1-methyl-1H-indazol-5-yl)-7-oxo-[1,2,4]triazolo[1,5-a]pyrimidin-4(7H)-yl)acetamide |
| Example 114 | 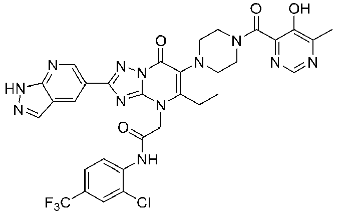 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-(1H -pyrazolo[3,4-b]pyridin-5-yl)-[1,2,4]triazol o[1,5-a]pyrimidin-4(7H)-yl)acetamide |
| Example 115 | 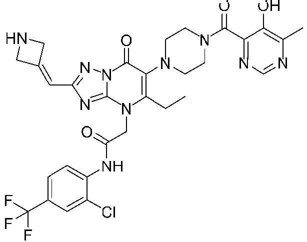 | 2-(2-(azetidin-3-ylidenemethyl)-5-ethyl-6-( 4-(5-hydroxy-6-methylpyrimidine-4-carbo nyl)piperazin-1-yl)-7-oxo-[1,2,4]triazolo[1, 5-a]pyrimidin-4(7H)-yl)-N-(2-chloro-4-(tri fluoromethyl)phenyl)acetamide |
| Example 116 | 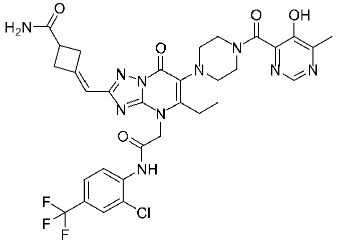 | 3-((4-(2-((2-chloro-4-(trifluoromethyl)phe nyl)amino)-2-oxoethyl)-5-ethyl-6-(4-(5-hy droxy-6-methylpyrimidine-4-carbonyl)pipe razin-1-yl)-7-oxo-4,7-dihydro-[1,2,4]triazo lo[1,5-a]pyrimidin-2-yl)methylene)cyclobu tane-1-carboxamide |
| Example 117 | 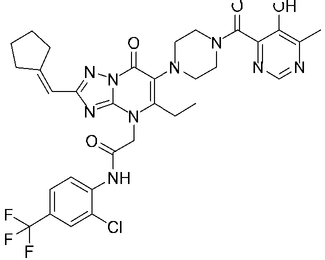 | N-(2-chloro-4-(trifluoromethyl)phenyl)-2-( 2-(cyclopentylidenemethyl)-5-ethyl-6-(4-( 5-hydroxy-6-methylpyrimidine-4-carbonyl )piperazin-1-yl)-7-oxo-[1,2,4]triazolo[1,5-a ]pyrimidin-4(7H)-yl)acetamide |
| Example 118 | 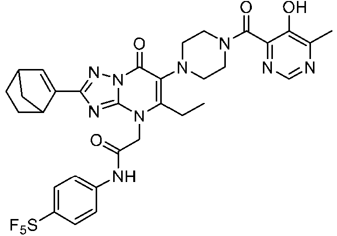 | 2-(2-(bicyclo[2.2.1]hept-2-en-2-yl)-5-ethyl -6-(4-(5-hydroxy-6-methylpyrimidine-4-ca rbonyl)piperazin-1-yl)-7-oxo-[1,2,4]triazol o[1,5-a]pyrimidin-4(7H)-yl)-N-(4-(pentafl uoro-λ⁶-sulfaneyl)phenyl)acetamide |
| Example 119 | 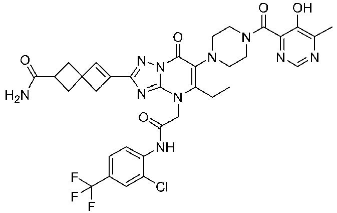 | 6-(4-(2-((2-chloro-4-(trifluoromethyl)phen yl)amino)-2-oxoethyl)-5-ethyl-6-(4-(5-hyd roxy-6-methylpyrimidine-4-carbonyl)piper azin-1-yl)-7-oxo-4,7-dihydro-[1,2,4]triazol o[1,5-a]pyrimidin-2-yl)spiro[3.3]hept-5-en e-2-carboxamide |
| Example 120 | 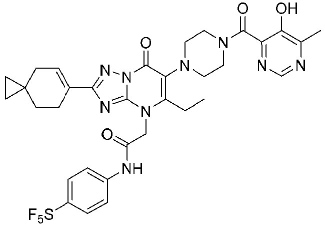 | 2-(5-ethyl-6-(4-(5-hydroxy-6-methylpyrim idine-4-carbonyl)piperazin-1-yl)-7-oxo-2-( spiro[2.5]oct-5-en-6-yl)-[1,2,4]triazolo[1,5 -a]pyrimidin-4(7H)-yl)-N-(4-(pentafluoro-λ⁶-sulfaneyl)phenyl)acetamide |
| Example 121 | 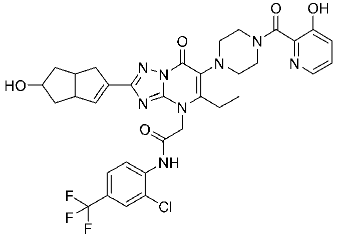 | N-(2-chloro-4-(trifluoromethyl)phenyl)-2-( 5-ethyl-2-(5-hydroxy-1,3a,4,5,6,6a-hexahy dropentalen-2-yl)-6-(4-(3-hydroxypicolino yl)piperazin-1-yl)-7-oxo-[1,2,4]triazolo[1,5 -a]pyrimidin-4(7H)-yl)acetamide |
| Example 122 | 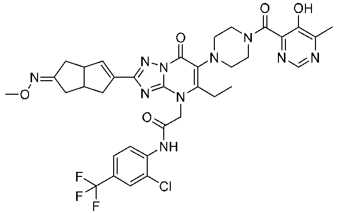 | (E)-N-(2-chloro-4-(trifluoromethyl)phenyl) -2-(5-ethyl-6-(4-(5-hydroxy-6-methylpyri midine-4-carbonyl)piperazin-1-yl)-2-(5-(m ethoxyimino)-1,3a,4,5,6,6a-hexahydropent alen-2-yl)-7-oxo-[1,2,4]triazolo[1,5-a]pyri midin-4(7H)-yl)acetamide |
| Example 123 | 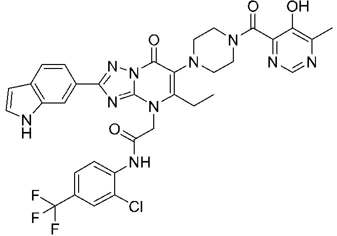 | N-(2-chloro-4-(trifluoromethyl)phenyl)-2-( 5-ethyl-6-(4-(5-hydroxy-6-methylpyrimidi ne-4-carbonyl)piperazin-1-yl)-2-(1H-indol-6-yl)-7-oxo-[1,2,4]triazolo[1,5-a]pyrimidin -4(7H)-yl)acetamide |
| Example 124 | 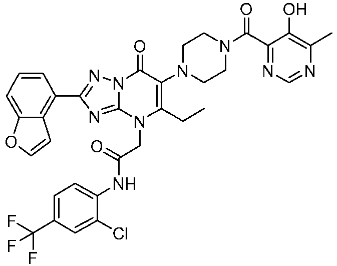 | 2-(2-(benzofuran-4-yl)-5-ethyl-6-(4-(5-hyd roxy-6-methylpyrimidine-4-carbonyl)piper azin-1-yl)-7-oxo-[1,2,4]triazolo[1,5-a]pyri midin-4(7H)-yl)-N-(2-chloro-4-(trifluorom ethyl)phenyl)acetamide |
| Example 125 | 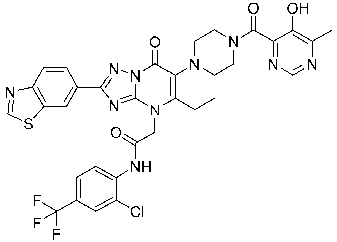 | 2-(2-(benzo[d]thiazol-6-yl)-5-ethyl-6-(4-(5 -hydroxy-6-methylpyrimidine-4-carbonyl) piperazin-1-yl)-7-oxo-[1,2,4]triazolo[1,5-a] pyrimidin-4(7H)-yl)-N-(2-chloro-4-(trifluo romethyl)phenyl)acetamide |
| Example 126 | 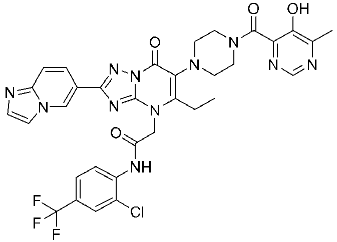 | N-(2-chloro-4-(trifluoromethyl)phenyl)-2-( 5-ethyl-6-(4-(5-hydroxy-6-methylpyrimidi ne-4-carbonyl)piperazin-1-yl)-2-(imidazo[ 1,2-a]pyridin-6-yl)-7-oxo-[1,2,4]triazolo[1, 5-a]pyrimidin-4(7H)-yl)acetamide |
| Example 127 | 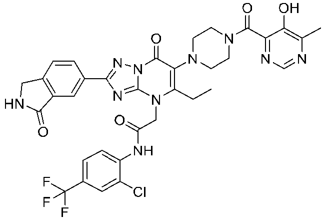 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-(3-oxoisoindolin-5-yl)-[1,2,4]triazolo[1,5-a]p yrimidin-4(7H)-yl)acetamide |
| Example 128 | 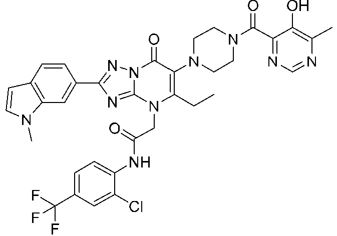 | N-(2-chloro-4-(trifluoromethyl)phenyl)-2-( 5-ethyl-6-(4-(5-hydroxy-6-methylpyrimidi ne-4-carbonyl)piperazin-1-yl)-2-(1-methyl-1H-indol-6-yl)-7-oxo-[1,2,4]triazolo[1,5-a] pyrimidin-4(7H)-yl)acetamide |
| Example 129 | 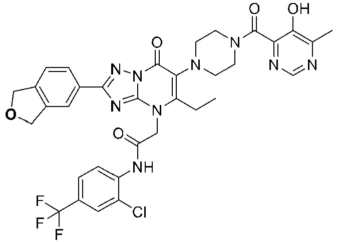 | N-(2-chloro-4-(trifluoromethyl)phenyl)-2-( 2-(1,3-dihydroisobenzofuran-5-yl)-5-ethyl-6-(4-(5-hydroxy-6-methylpyrimidine-4-car bonyl)piperazin-1-yl)-7-oxo-[1,2,4]triazolo [1,5-a]pyrimidin-4(7H)-yl)acetamide |
| Example 130 | 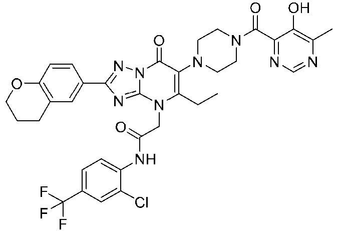 | N-(2-chloro-4-(trifluoromethyl)phenyl)-2-( 2-(chroman-6-yl)-5-ethyl-6-(4-(5-hydroxy-6-methylpyrimidine-4-carbonyl)piperazin-1-yl)-7-oxo-[1,2,4]triazolo[1,5-a]pyrimidin -4(7H)-yl)acetamide |
| Example 131 | 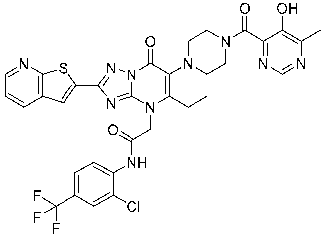 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-(thi eno[2,3-b]pyridin-2-yl)-[1,2,4]triazolo[1,5-a]pyrimidin-4(7H)-yl)acetamide |
| Example 132 | 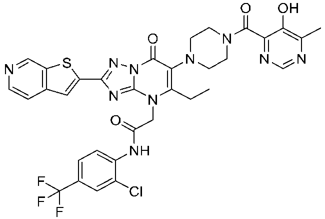 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-(thi eno[2,3-c]pyridin-2-yl)-[1,2,4]triazolo[1,5-a]pyrimidin-4(7H)-yl)acetamide |
| Example 133 | 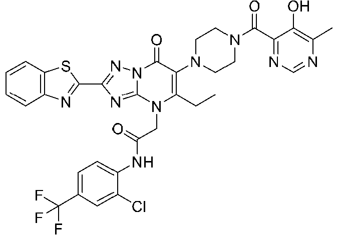 | 2-(2-(benzo[d]thiazol-2-yl)-5-ethyl-6-(4-(5 -hydroxy-6-methylpyrimidine-4-carbonyl) piperazin-1-yl)-7-oxo-[1,2,4]triazolo[1,5-a] pyrimidin-4(7H)-yl)-N-(2-chloro-4-(trifluo romethyl)phenyl)acetamide |
| Example 134 | 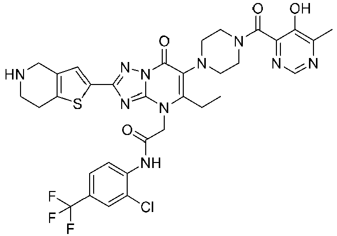 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-(4,5 ,6,7-tetrahydrothieno[3,2-c]pyridin-2-yl)-[ 1,2,4]triazolo[1,5-a]pyrimidin-4(7H)-yl)ac etamide |
| Example 135 | 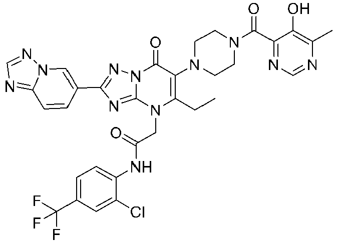 | 2-(2-([1,2,4]triazolo[1,5-a]pyridin-6-yl)-5-ethyl-6-(4-(5-hydroxy-6-methylpyrimidine -4-carbonyl)piperazin-1-yl)-7-oxo-[1,2,4]tr iazolo[1,5-a]pyrimidin-4(7H)-yl)-N-(2-chl oro-4-(trifluoromethyl)phenyl)acetamide |
| Example 136 | 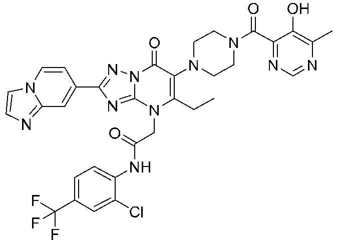 | N-(2-chloro-4-(trifluoromethyl)phenyl)-2-( 5-ethyl-6-(4-(5-hydroxy-6-methylpyrimidi ne-4-carbonyl)piperazin-1-yl)-2-(imidazo[ 1,2-a]pyridin-7-yl)-7-oxo-[1,2,4]triazolo[1, 5-a]pyrimidin-4(7H)-yl)acetamide |
| Example 137 | 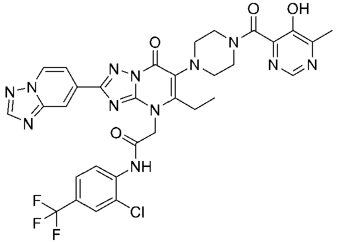 | 2-(2-([1,2,4]triazolo[1,5-a]pyridin-7-yl)-5-ethyl-6-(4-(5-hydroxy-6-methylpyrimidine -4-carbonyl)piperazin-1-yl)-7-oxo-[1,2,4]tr iazolo[1,5-a]pyrimidin-4(7H)-yl)-N-(2-chl oro-4-(trifluoromethyl)phenyl)acetamide |
| Example 138 | 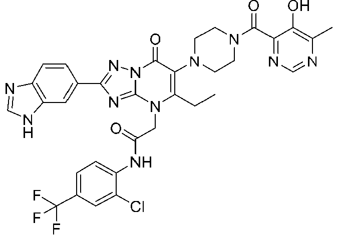 | 2-(2-(1H-benzo[d]imidazol-6-yl)-5-ethyl-6 -(4-(5-hydroxy-6-methylpyrimidine-4-carb onyl)piperazin-1-yl)-7-oxo-[1,2,4]triazolo[ 1,5-a]pyrimidin-4(7H)-yl)-N-(2-chloro-4-(t rifluoromethyl)phenyl)acetamide |
| Example 139 | 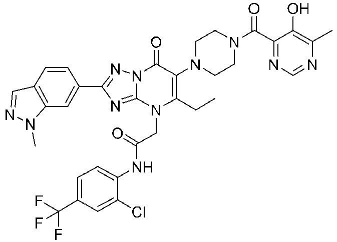 | N-(2-chloro-4-(trifluoromethyl)phenyl)-2-( 5-ethyl-6-(4-(5-hydroxy-6-methylpyrimidi ne-4-carbonyl)piperazin-1-yl)-2-(1-methyl-1H-indazol-6-yl)-7-oxo-[1,2,4]triazolo[1,5-a]pyrimidin-4(7H)-yl)acetamide |
| Example 140 | 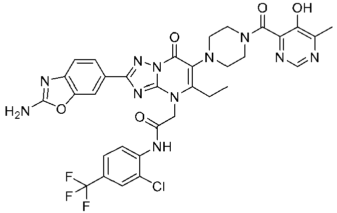 | 2-(2-(2-aminobenzo[d]oxazol-6-yl)-5-ethyl -6-(4-(5-hydroxy-6-methylpyrimidine-4-ca rbonyl)piperazin-1-yl)-7-oxo-[1,2,4]triazol o[1,5-a]pyrimidin-4(7H)-yl)-N-(2-chloro-4 -(trifluoromethyl)phenyl)acetamide |
| Example 141 | 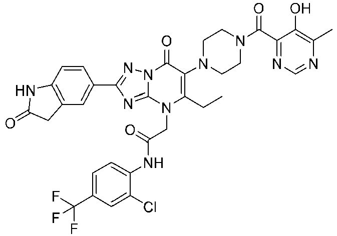 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-(2-oxoindolin-5-yl)-[1,2,4]triazolo[1,5-a]pyri midin-4(7H)-yl)acetamide |
| Example 142 | 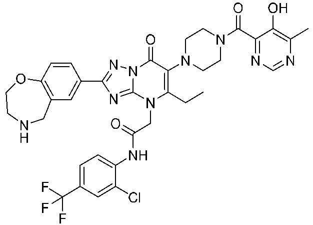 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-(2,3 ,4,5-tetrahydrobenzo[f][1,4]oxazepin-7-yl) -[1,2,4]triazolo[1,5-a]pyrimidin-4(7H)-yl)a cetamide |
| Example 143 | 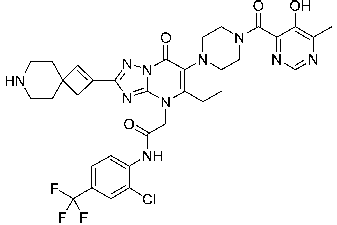 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-(7-a zaspiro[3.5]non-1-en-2-yl)-[1,2,4]triazolo[ 1,5-a]pyrimidin-4(7H)-yl)acetamide |
| Example 144 | 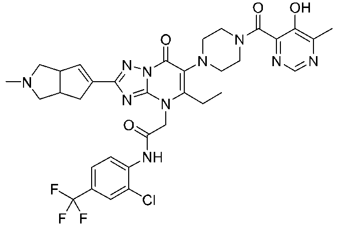 | N-(2-chloro-4-(trifluoromethyl)phenyl)-2-( 5-ethyl-6-(4-(5-hydroxy-6-methylpyrimidi ne-4-carbonyl)piperazin-1-yl)-2-(2-methyl-1,2,3,3a,4,6a-hexahydrocyclopenta[c]pyrro 1-5-yl)-7-oxo-[1,2,4]triazolo[1,5-a]pyrimidi n-4(7H)-yl)acetamide |
| Example 145 | 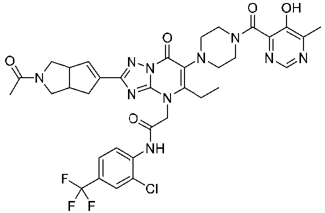 | 2-(2-(2-acetyl-1,2,3,3a,4,6a-hexahydrocycl openta[c]pyrrol-5-yl)-5-ethyl-6-(4-(5-hydr oxy-6-methylpyrimidine-4-carbonyl)pipera zin-1-yl)-7-oxo-[1,2,4]triazolo[1,5-a]pyrim idin-4(7H)-yl)-N-(2-chloro-4-(trifluoromet hyl)phenyl)acetamide |
| Example 146 | 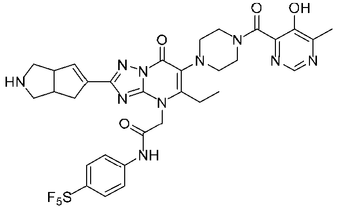 | 2-(5-ethyl-2-(1,2,3,3a,4,6a-hexahydrocyclo penta[c]pyrrol-5-yl)-6-(4-(5-hydroxy-6-me thylpyrimidine-4-carbonyl)piperazin-1-yl)-7-oxo-[1,2,4]triazolo[1,5-a]pyrimidin-4(7H )-yl)-N-(4-(pentafluoro-λ⁶-sulfaneyl)pheny l)acetamide |
| Example 147 | 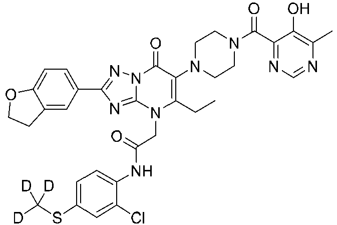 | N-(2-chloro-4-((methyl-d3)thio)phenyl)-2-(2-(2,3-dihydrobenzofuran-5-yl)-5-ethyl-6-(4-(5-hydroxy-6-methylpyrimidine-4-carbo nyl)piperazin-1-yl)-7-oxo-[1,2,4]triazolo[1, 5-a]pyrimidin-4(7H)-yl)acetamide |
| Example 148 | 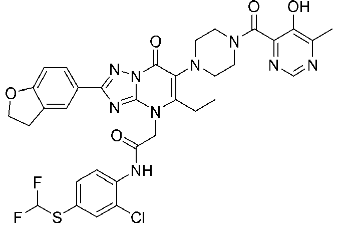 | N-(2-chloro-4-((difluoromethyl)thio)pheny l)-2-(2-(2,3-dihydrobenzofuran-5-yl)-5-eth yl-6-(4-(5-hydroxy-6-methylpyrimidine-4-carbonyl)piperazin-1-yl)-7-oxo-[1,2,4]triaz olo[1,5-a]pyrimidin-4(7H)-yl)acetamide |
| Example 149 | 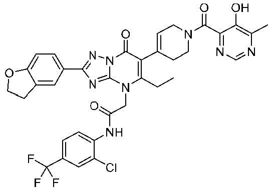 | N-(2-chloro-4-(trifluoromethyl)phenyl)-2-( 2-(2,3-dihydrobenzofuran-5-yl)-5-ethyl-6-( 1-(5-hydroxy-6-methylpyrimidine-4-carbo nyl)-1,2,3,6-tetrahydropyridin-4-yl)-7-oxo-[1,2,4]triazolo[1,5-a]pyrimidin-4(7H)-yl)ac etamide |
| Example 150 | 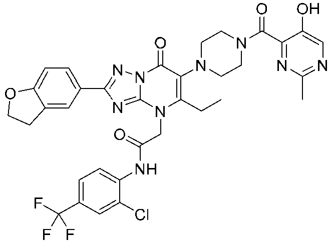 | N-(2-chloro-4-(trifluoromethyl)phenyl)-2-( 2-(2,3-dihydrobenzofuran-5-yl)-5-ethyl-6-( 4-(5-hydroxy-2-methylpyrimidine-4-carbo nyl)piperazin-1-yl)-7-oxo-[1,2,4]triazolo[1, 5-a]pyrimidin-4(7H)-yl)acetamide |
| Example 151 | 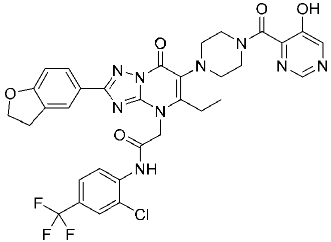 | N-(2-chloro-4-(trifluoromethyl)phenyl)-2-( 2-(2,3-dihydrobenzofuran-5-yl)-5-ethyl-6-( 4-(5-hydroxypyrimidine-4-carbonyl)pipera zin-1-yl)-7-oxo-[1,2,4]triazolo[1,5-a]pyrim idin-4(7H)-yl)acetamide |
| Example 152 | 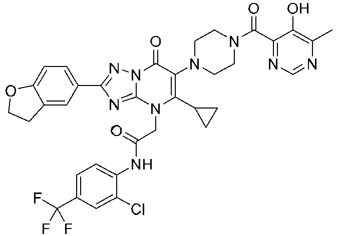 | N-(2-chloro-4-(trifluoromethyl)phenyl)-2-( 5-cyclopropyl-2-(2,3-dihydrobenzofuran-5 -yl)-6-(4-(5-hydroxy-6-methylpyrimidine-4-carbonyl)piperazin-1-yl)-7-oxo-[1,2,4]tri azolo[1,5-a]pyrimidin-4(7H)-yl)acetamide |
| Example 153 | 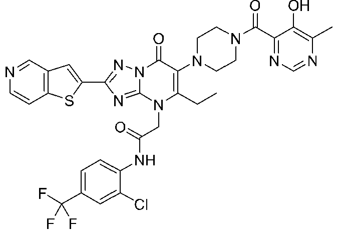 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-(thi eno[3,2-c]pyridin-2-yl)-[1,2,4]triazolo[1,5-a]pyrimidin-4(7H)-yl)acetamide |
| Example 154 | 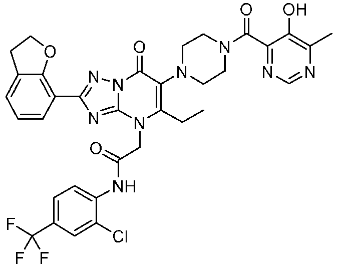 | N-(2-chloro-4-(trifluoromethyl)phenyl)-2-( 2-(2,3-dihydrobenzofuran-7-yl)-5-ethyl-6-( 4-(5-hydroxy-6-methylpyrimidine-4-carbo nyl)piperazin-1-yl)-7-oxo-[1,2,4]triazolo[1, 5-a]pyrimidin-4(7H)-yl)acetamide |
| Example 155 | 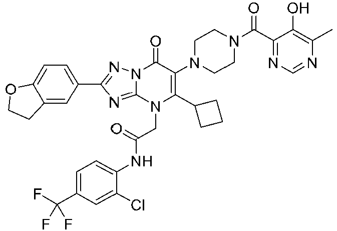 | N-(2-chloro-4-(trifluoromethyl)phenyl)-2-( 5-cyclobutyl-2-(2,3-dihydrobenzofuran-5-y l)-6-(4-(5-hydroxy-6-methylpyrimidine-4-c arbonyl)piperazin-1-yl)-7-oxo-[1,2,4]triazo lo[1,5-a]pyrimidin-4(7H)-yl)acetamide |
| Example 156 | 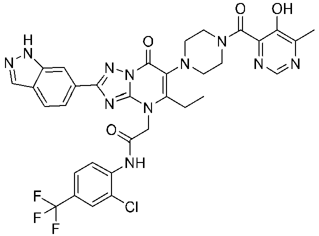 | N-(2-chloro-4-(trifluoromethyl)phenyl)-2-( 5-ethyl-6-(4-(5-hydroxy-6-methylpyrimidi ne-4-carbonyl)piperazin-1-yl)-2-(1H-indaz ol-6-yl)-7-oxo-[1,2,4]triazolo[1,5-a]pyrimi din-4(7H)-yl)acetamide |
| Example 157 | 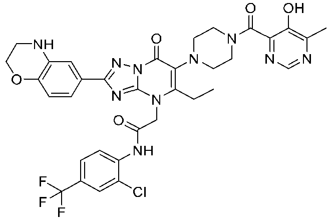 | N-(2-chloro-4-(trifluoromethyl)phenyl)-2-( 2-(3,4-dihydro-2H-benzo[b][1,4]oxazin-6-yl)-5-ethyl-6-(4-(5-hydroxy-6-methylpyri midine-4-carbonyl)piperazin-1-yl)-7-oxo-[ 1,2,4]triazolo[1,5-a]pyrimidin-4(7H)-yl)ac etamide |
| Example 158 | 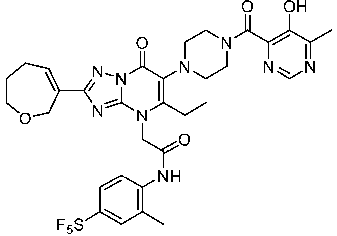 | 2-(5-ethyl-6-(4-(5-hydroxy-6-methylpyrim idine-4-carbonyl)piperazin-1-yl)-7-oxo-2-( 2,5,6,7-tetrahydrooxepin-3-yl)-[1,2,4]triaz olo[1,5-a]pyrimidin-4(7H)-yl)-N-(2-methyl -4-(pentafluoro-λ⁶-sulfaneyl)phenyl)aceta mide |
| Example 159 | 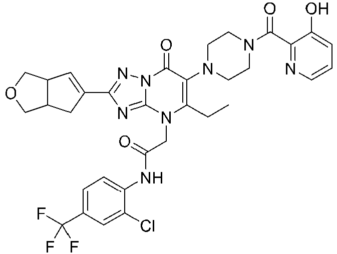 | N-(2-chloro-4-(trifluoromethyl)phenyl)-2-( 5-ethyl-6-(4-(3-hydroxypicolinoyl)piperazi n-1-yl)-7-oxo-2-(3,3a,4,6a-tetrahydro-1H-c yclopenta[c]furan-5-yl)-[1,2,4]triazolo[1,5-a]pyrimidin-4(7H)-yl)acetamide |
| Example 160 | 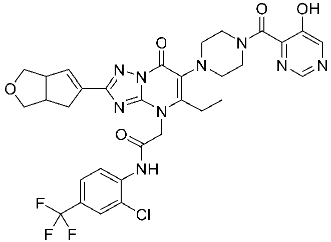 | N-(2-chloro-4-(trifluoromethyl)phenyl)-2-( 5-ethyl-6-(4-(5-hydroxypyrimidine-4-carb onyl)piperazin-1-yl)-7-oxo-2-(3,3a,4,6a-tet rahydro-1H-cyclopenta[c]furan-5-yl)-[1,2, 4]triazolo[1,5-a]pyrimidin-4(7H)-yl)aceta mide |
| Example 161 | 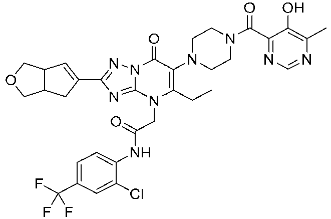 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-(3,3 a,4,6a-tetrahydro-1H-cyclopenta[c]furan-5 -yl)-[1,2,4]triazolo[1,5-a]pyrimidin-4(7H)-yl)acetamide |
| Example 162 | 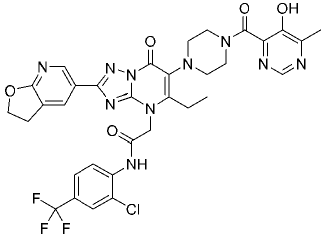 | N-(2-chloro-4-(trifluoromethyl)phenyl)-2-( 2-(2,3-dihydrofuro[2,3-b]pyridin-5-yl)-5-et hyl-6-(4-(5-hydroxy-6-methylpyrimidine-4 -carbonyl)piperazin-1-yl)-7-oxo-[1,2,4]tria zolo[1,5-a]pyrimidin-4(7H)-yl)acetamide |
| Example 163 | 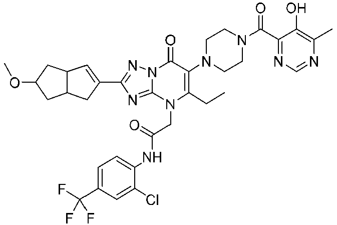 | N-(2-chloro-4-(trifluoromethyl)phenyl)-2-( 5-ethyl-6-(4-(5-hydroxy-6-methylpyrimidi ne-4-carbonyl)piperazin-1-yl)-2-(5-methox y-1,3a,4,5,6,6a-hexahydropentalen-2-yl)-7-oxo-[1,2,4]triazolo[1,5-a]pyrimidin-4(7H)-yl)acetamide |
| Example 164 | 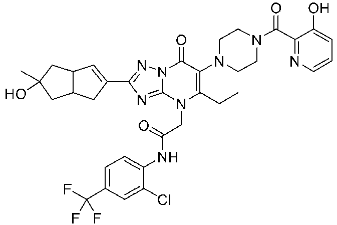 | N-(2-chloro-4-(trifluoromethyl)phenyl)-2-( 5-ethyl-2-(5-hydroxy-5-methyl-1,3a,4,5,6, 6a-hexahydropentalen-2-yl)-6-(4-(3-hydro xypicolinoyl)piperazin-1-yl)-7-oxo-[1,2,4]t riazolo[1,5-a]pyrimidin-4(7H)-yl)acetamid e |
| Example 165 | 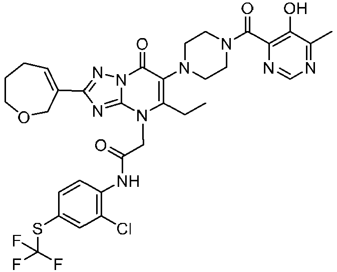 | N-(2-chloro-4-((trifluoromethyl)thio)pheny l)-2-(5-ethyl-6-(4-(5-hydroxy-6-methylpyri midine-4-carbonyl)piperazin-1-yl)-7-oxo-2 -(2,5,6,7-tetrahydrooxepin-3-yl)-[1,2,4]tria zolo[1,5-a]pyrimidin-4(7H)-yl)acetamide |
| Example 166 | 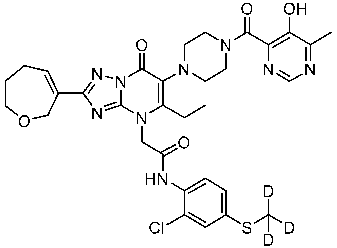 | N-(2-chloro-4-((methyl-d3)thio)phenyl)-2-(5-ethyl-6-(4-(5-hydroxy-6-methylpyrimidi ne-4-carbonyl)piperazin-1-yl)-7-oxo-2-(2,5 ,6,7-tetrahydrooxepin-3-yl)-[1,2,4]triazolo[ 1,5-a]pyrimidin-4(7H)-yl)acetamide |
| Example 167 | 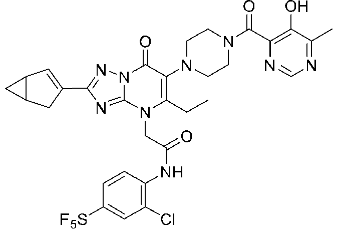 | 2-(2-(bicyclo[3.1.0]hex-2-en-3-yl)-5-ethyl-6-(4-(5-hydroxy-6-methylpyrimidine-4-car bonyl)piperazin-1-yl)-7-oxo-[1,2,4]triazolo [1,5-a]pyrimidin-4(7H)-yl)-N-(2-chloro-4-( pentafluoro-λ⁶-sulfaneyl)phenyl)acetamide |
| Example 168 | 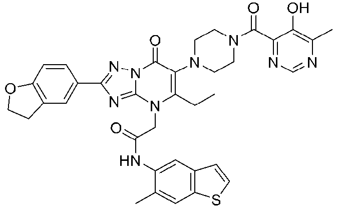 | 2-(2-(2,3-dihydrobenzofuran-5-yl)-5-ethyl-6-(4-(5-hydroxy-6-methylpyrimidine-4-car bonyl)piperazin-1-yl)-7-oxo-[1,2,4]triazolo [1,5-a]pyrimidin-4(7H)-yl)-N-(6-methylbe nzo[b]thiophen-5-yl)acetamide |
| Example 169 | 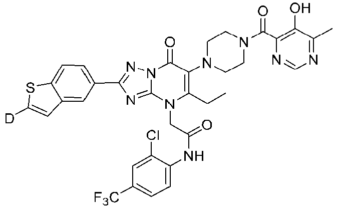 | 2-(2-(benzo[b]thiophen-5-yl-2-d)-5-ethyl-6 -(4-(5-hydroxy-6-methylpyrimidine-4-carb onyl)piperazin-1-yl)-7-oxo-[1,2,4]triazolo[ 1,5-a]pyrimidin-4(7H)-yl)-N-(2-chloro-4-(t rifluoromethyl)phenyl)acetamide |
| Example 170 | 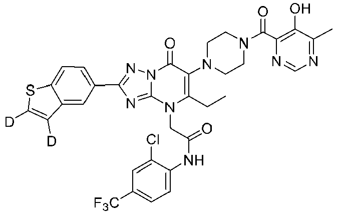 | 2-(2-(benzo[b]thiophen-5-yl-2,3-d2)-5-eth yl-6-(4-(5-hydroxy-6-methylpyrimidine-4-carbonyl)piperazin-1-yl)-7-oxo-[1,2,4]triaz olo[1,5-a]pyrimidin-4(7H)-yl)-N-(2-chloro -4-(trifluoromethyl)phenyl)acetamide |
| Example 171 | 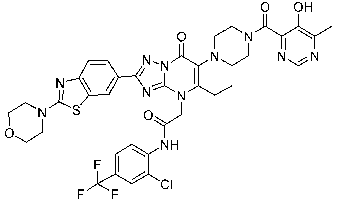 | N-(2-chloro-4-(trifluoromethyl)phenyl)-2-( 5-ethyl-6-(4-(5-hydroxy-6-methylpyrimidi ne-4-carbonyl)piperazin-1-yl)-2-(2-morpho linobenzo[d]thiazol-6-yl)-7-oxo-[1,2,4]tria zolo[1,5-a]pyrimidin-4(7H)-yl)acetamide |
| Example 172 | 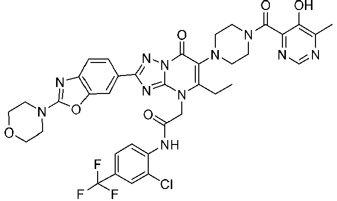 | N-(2-chloro-4-(trifluoromethyl)phenyl)-2-( 5-ethyl-6-(4-(5-hydroxy-6-methylpyrimidi ne-4-carbonyl)piperazin-1-yl)-2-(2-morpho linobenzo[d]oxazol-6-yl)-7-oxo-[1,2,4]tria zolo[1,5-a]pyrimidin-4(7H)-yl)acetamide |
| Example 173 | 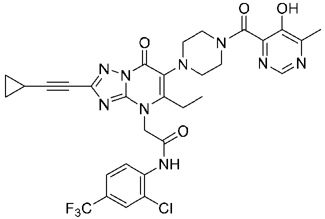 | N-(2-chloro-4-(trifluoromethyl)phenyl)-2-( 2-(cyclopropylethynyl)-5-ethyl-6-(4-(5-hy droxy-6-methylpyrimidine-4-carbonyl)pipe razin-1-yl)-7-oxo-[1,2,4]triazolo[1,5-a]pyri midin-4(7H)-yl)acetamide |
| Example 174 | 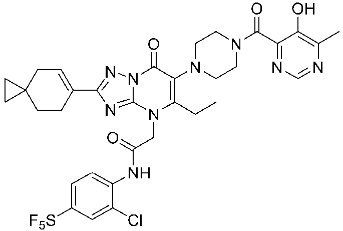 | N-(2-chloro-4-(pentafluoro-λ⁶-sulfaneyl)ph enyl)-2-(5-ethyl-6-(4-(5-hydroxy-6-methyl pyrimidine-4-carbonyl)piperazin-1-yl)-7-o xo-2-(spiro[2.5]oct-5-en-6-yl)-[1,2,4]triazo lo[1,5-a]pyrimidin-4(7H)-yl)acetamide |
| Example 175 | 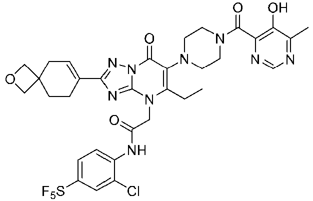 | N-(2-chloro-4-(pentafluoro-λ⁶ -sulfaneyl)ph enyl)-2-(5-ethyl-6-(4-(5-hydroxy-6-methyl pyrimidine-4-carbonyl)piperazin-1-yl)-7-o xo-2-(2-oxaspiro[3.5]non-6-en-7-yl)-[1,2,4 ]triazolo[1,5-a]pyrimidin-4(7H)-yl)acetami de |
| Example 176 | 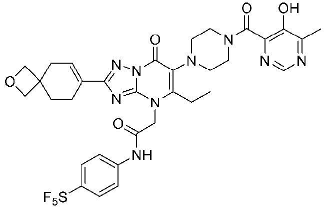 | 2-(5-ethyl-6-(4-(5-hydroxy-6-methylpyrim idine-4-carbonyl)piperazin-1-yl)-7-oxo-2-( 2-oxaspiro[3.5]non-6-en-7-yl)-[1,2,4]triazo lo[1,5-a]pyrimidin-4(7H)-yl)-N-(4-(pentafl uoro-λ⁶ -sulfaneyl)phenyl)acetamide |
| Example 177 | 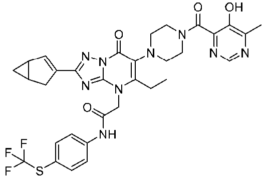 | 2-(2-(bicyclo[3.1.0]hex-2-en-3-yl)-5-ethyl-6-(4-(5-hydroxy-6-methylpyrimidine-4-car bonyl)piperazin-1-yl)-7-oxo-[1,2,4]triazolo [1,5-a]pyrimidin-4(7H)-yl)-N-(4-((trifluoro methyl)thio)phenyl)acetamide |
| Example 178 | 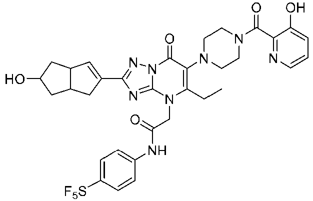 | 2-(5-ethyl-2-(5-hydroxy-1,3a,4,5,6,6a-hexa hydropentalen-2-yl)-6-(4-(3-hydroxypicoli noyl)piperazin-1-yl)-7-oxo-[1,2,4]triazolo[ 1,5-a]pyrimidin-4(7H)-yl)-N-(4-(pentafluo ro-λ⁶ -sulfaneyl)phenyl)acetamide |
| Example 179 | 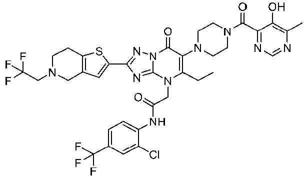 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-(5-( 2,2,2-trifluoroethyl)-4,5,6,7-tetrahydrothie no[3,2-c]pyridin-2-yl)-[1,2,4]triazolo[1,5-a ]pyrimidin-4(7H)-yl)acetamide |
| Example 180 | 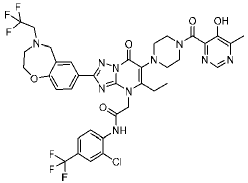 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-(4-( 2,2,2-trifluoroethyl)-2,3,4,5-tetrahydrobenz o[f][1,4]oxazepin-7-yl)-[1,2,4]triazolo[1,5-a]pyrimidin-4(7H)-yl)acetamide |
| Example 181 | 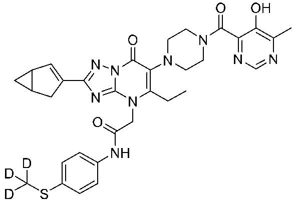 | 2-(2-(bicyclo[3.1.0]hex-2-en-3-yl)-5-ethyl-6-(4-(5-hydroxy-6-methylpyrimidine-4-car bonyl)piperazin-1-yl)-7-oxo-[1,2,4]triazolo [1,5-a]pyrimidin-4(7H)-yl)-N-(4-((methyl-d3)thio)phenyl)acetamide |
| Example 182 | 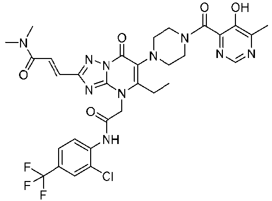 | (E)-3-(4-(2-((2-chloro-4-(trifluoromethyl)p henyl)amino)-2-oxoethyl)-5-ethyl-6-(4-(5-hydroxy-6-methylpyrimidine-4-carbonyl)p iperazin-1-yl)-7-oxo-4,7-dihydro-[1,2,4]tri azolo[1,5-a]pyrimidin-2-yl)-N,N-dimethyl acrylamide |
| Example 183 | 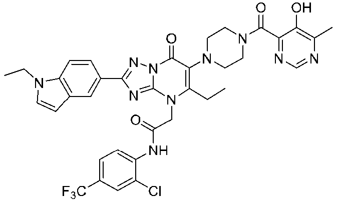 | N-(2-chloro-4-(trifluoromethyl)phenyl)-2-( 5-ethyl-2-(1-ethyl-1H-indol-5-yl)-6-(4-(5-h ydroxy-6-methylpyrimidine-4-carbonyl)pi perazin-1-yl)-7-oxo-[1,2,4]triazolo[1,5-a]p yrimidin-4(7H)-yl)acetamide |
| Example 184 | 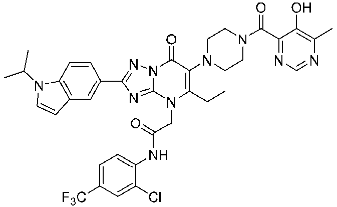 | N-(2-chloro-4-(trifluoromethyl)phenyl)-2-( 5-ethyl-6-(4-(5-hydroxy-6-methylpyrimidi ne-4-carbonyl)piperazin-1-yl)-2-(1-isoprop yl-1H-indol-5-yl)-7-oxo-[1,2,4]triazolo[1,5 -a]pyrimidin-4(7H)-yl)acetamide |
| Example 185 | 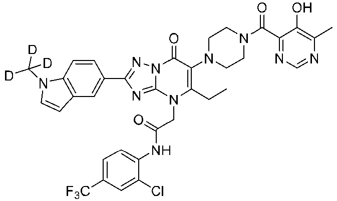 | N-(2-chloro-4-(trifluoromethyl)phenyl)-2-( 5-ethyl-6-(4-(5-hydroxy-6-methylpyrimidi ne-4-carbonyl)piperazin-1-yl)-2-(1-(methyl -d3)-1H-indol-5-yl)-7-oxo-[1,2,4]triazolo[ 1,5-a]pyrimidin-4(7H)-yl)acetamide |
| Example 186 | 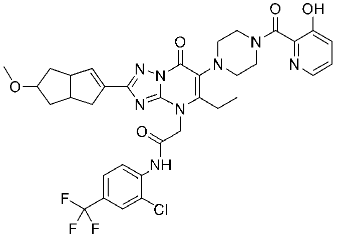 | N-(2-chloro-4-(trifluoromethyl)phenyl)-2-( 5-ethyl-6-(4-(3-hydroxypicolinoyl)piperazi n-1-yl)-2-(5-methoxy-1,3a,4,5,6,6a-hexahy dropentalen-2-yl)-7-oxo-[1,2,4]triazolo[1,5 -a]pyrimidin-4(7H)-yl)acetamide |
| Example 187 | 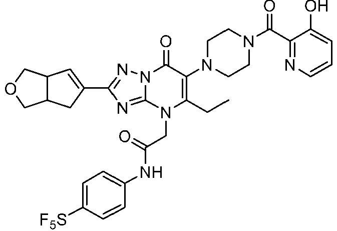 | 2-(5-ethyl-6-(4-(3-hydroxypicolinoyl)piper azin-1-yl)-7-oxo-2-(3,3a,4,6a-tetrahydro-1 H-cyclopenta[c]furan-5-yl)-[1,2,4]triazolo[ 1,5-a]pyrimidin-4(7H)-yl)-N-(4-(pentafluo ro-λ⁶ -sulfaneyl)phenyl)acetamide |
| Example 188 | 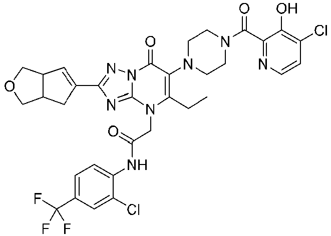 | 2-(6-(4-(4-chloro-3-hydroxypicolinoyl)pip erazin-1-yl)-5-ethyl-7-oxo-2-(3,3a,4,6a-tetr ahydro-1H-cyclopenta[c]furan-5-yl)-[1,2,4 ]triazolo[1,5-a]pyrimidin-4(7H)-yl)-N-(2-c hloro-4-(trifluoromethyl)phenyl)acetamide |
| Example 189 | 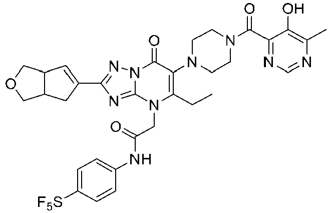 | 2-(5-ethyl-6-(4-(5-hydroxy-6-methylpyrim idine-4-carbonyl)piperazin-1-yl)-7-oxo-2-( 3,3a,4,6a-tetrahydro-1H-cyclopenta[c]fura n-5-yl)-[1,2,4]triazolo[1,5-a]pyrimidin-4(7 H)-yl)-N-(4-(pentafluoro-λ⁶-sulfaneyl)phe nyl)acetamide |
| Example 190 | 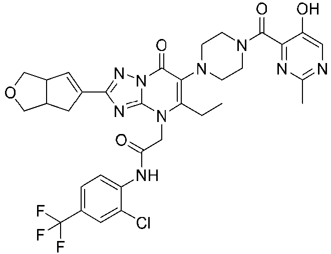 | N-(2-chloro-4-(trifluoromethyl)phenyl)-2-( 5-ethyl-6-(4-(5-hydroxy-2-methylpyrimidi ne-4-carbonyl)piperazin-1-yl)-7-oxo-2-(3,3 a,4,6a-tetrahydro-1H-cyclopenta[c]furan-5 -yl)-[1,2,4]triazolo[1,5-a]pyrimidin-4(7H)-yl)acetamide |
| Example 191 | 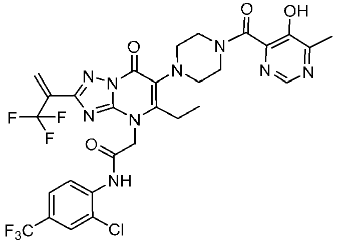 | N-(2-chloro-4-(trifluoromethyl)phenyl)-2-( 5-ethyl-6-(4-(5-hydroxy-6-methylpyrimidi ne-4-carbonyl)piperazin-1-yl)-7-oxo-2-(3,3 ,3-trifluoroprop-1-en-2-yl)-[1,2,4]triazolo[ 1,5-a]pyrimidin-4(7H)-yl)acetamide |
| Example 192 | 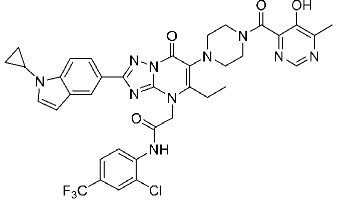 | N-(2-chloro-4-(trifluoromethyl)phenyl)-2-( 2-(1-cyclopropyl-1H-indol-5-yl)-5-ethyl-6-(4-(5-hydroxy-6-methylpyrimidine-4-carbo nyl)piperazin-1-yl)-7-oxo-[1,2,4]triazolo[1, 5-a]pyrimidin-4(7H)-yl)acetamide |
| Example 193 | 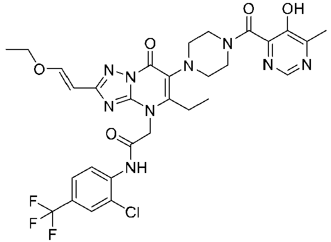 | (E)-N-(2-chloro-4-(trifluoromethyl)phenyl) -2-(2-(2-ethoxyvinyl)-5-ethyl-6-(4-(5-hydr oxy-6-methylpyrimidine-4-carbonyl)pipera zin-1-yl)-7-oxo-[1,2,4]triazolo[1,5-a]pyrim idin-4(7H)-yl)acetamide |
| Example 194 | 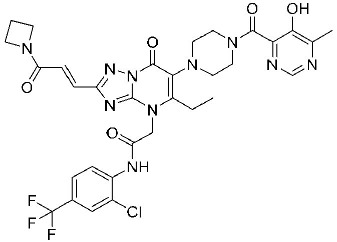 | (E)-2-(2-(3-(azetidin-1-yl)-3-oxoprop-1-en -1-yl)-5-ethyl-6-(4-(5-hydroxy-6-methylpy rimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acet amide |
| Example 195 | 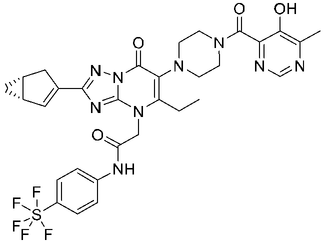 | 2-(2-((1R,5R)-bicyclo[3.1.0]hex-2-en-3-yl) -5-ethyl-6-(4-(5-hydroxy-6-methylpyrimidi ne-4-carbonyl)piperazin-1-yl)-7-oxo-[1,2,4 ]triazolo[1,5-a]pyrimidin-4(7H)-yl)-N-(4-( pentafluoro-λ6-sulfaneyl)phenyl)acetamide |
| Example 196 | 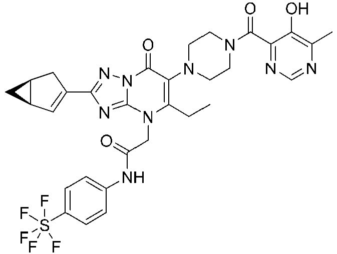 | 2-(2-((1S,5S)-bicyclo[3.1.0]hex-2-en-3-yl) -5-ethyl-6-(4-(5-hydroxy-6-methylpyrimidi ne-4-carbonyl)piperazin-1-yl)-7-oxo-[1,2,4 ]triazolo[1,5-a]pyrimidin-4(7H)-yl)-N-(4-( pentafluoro-λ6-sulfaneyl)phenyl)acetamide |

or a stereoisomer, tautomer, or pharmaceutically acceptable salt thereof.

Note: If there is a discrepancy between the drawn structure and the name given for the structure, the drawn structure shall prevail.

Further, the present disclosure provides a pharmaceutical composition, wherein the pharmaceutical composition comprises a compound of formula (I), (II) or (III), or a stereoisomer, tautomer, or pharmaceutically acceptable salt thereof, and a pharmaceutically acceptable carrier.

The present disclosure provides a use of the compound described in formula (I), (II) or (III), or a stereoisomer, tautomer, or pharmaceutically acceptable salt thereof, or the pharmaceutical composition thereof, in the manufacture of a WRN inhibitor.

The present disclosure further provides a use of the compound of formula (I), (II) or (III), or a stereoisomer, tautomer, or pharmaceutically acceptable salt thereof, or the pharmaceutical composition thereof, in the manufacture of a medicament for treating a WRN-mediated disease, wherein the WRN-mediated disease is preferably a high microsatellite instability (MSI-H) cancer; wherein the WRN-mediated disease is selected from the group consisting of colorectal cancer, gastric cancer, endometrial cancer, rectal adenocarcinoma, adrenocortical carcinoma, uterine sarcoma, cervical cancer, nephroblastoma, mesothelioma, esophageal cancer, breast cancer, renal clear cell carcinoma, ovarian serous cystadenocarcinoma, cholangiocarcinoma, thymoma, liver cancer, head and neck squamous cell carcinoma, sarcoma, cutaneous melanoma, lung squamous cell carcinoma, prostate cancer, lung adenocarcinoma, bladder transitional cell carcinoma, pediatric neuroblastoma, chronic lymphocytic leukemia, and glioma, preferably colorectal cancer, gastric cancer, or endometrial cancer.

The present disclosure further provides a use of the compound of formula (I), (II) or (III), or a stereoisomer, tautomer, or pharmaceutically acceptable salt thereof, or the pharmaceutical composition thereof, in the manufacture of a medicament for treating a high microsatellite instability (MSI-H) cancer.

The present disclosure provides a use of the compound of formula (I), (II) or (III), or a stereoisomer, tautomer, or pharmaceutically acceptable salt thereof, or the pharmaceutical composition thereof, in the manufacture of a medicament for treating colorectal cancer, gastric cancer, endometrial cancer, rectal adenocarcinoma, adrenocortical carcinoma, uterine sarcoma, cervical cancer, nephroblastoma, mesothelioma, esophageal cancer, breast cancer, renal clear cell carcinoma, ovarian serous cystadenocarcinoma, cholangiocarcinoma, thymoma, liver cancer, head and neck squamous cell carcinoma, sarcoma, cutaneous melanoma, lung squamous cell carcinoma, prostate cancer, lung adenocarcinoma, bladder transitional cell carcinoma, pediatric neuroblastoma, chronic lymphocytic leukemia, or glioma, preferably a use in the manufacture of a medicament for treating colorectal cancer, gastric cancer, or endometrial cancer.

### DETAILED DESCRIPTION

Unless there is a contrary statement, certain terms used in the specification and claims of the present disclosure are defined as follows:

In the present disclosure, "one or more" means 1, 2, 3, 4, or more.

In the present disclosure, when denoting the number of members of a group, C₁-C₁₀ alkyl and C₁₋₁₀ alkyl have the same meaning. Similarly, C₆-C₁₀ aryl and C₆-₁₀ aryl have the same meaning.

"Alkyl," when referred to as a group or as part of a group, refers to C₁-C₂₀ straight-chain or branched aliphatic hydrocarbon groups. Preferably C₁-C₁₀ alkyl, more preferably C₁-C₆ alkyl, and even more preferably C₁-C₄ alkyl. Examples of alkyl groups include, but are not limited to, methyl, ethyl, n-propyl, isopropyl, n-butyl, isobutyl, tert-Butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, and 2,3-dimethylbutyl. Alkyl may be substituted or unsubstituted.

"Alkenyl" refers to alkyl as defined above consisting of at least two carbon atoms and at least one carbon-carbon double bond, and representative examples include, but are not limited to, ethenyl, 1-propenyl, 2-propenyl, 1-, 2-, and 3-butenyl. Preferably C₂-C₄ alkenyl. Alkenyl may be optionally substituted or unsubstituted.

"Alkynyl" refers to an aliphatic hydrocarbon group containing one carbon-carbon triple bond, which may be straight-chain or branched. Preferably C₂-C₁₀ alkynyl, more preferably C₂-C₆ alkynyl, and most preferably C₂-C₄ alkynyl. Examples of alkynyl groups include, but are not limited to, ethynyl, 1-propynyl, 2-propynyl, 1-, 2-, and 3-butynyl. Alkynyl may be substituted or unsubstituted.

"Cycloalkyl" refers to non-aromatic cyclic alkyl, wherein one or more ring-forming atoms are carbon atoms, and the ring contains 0, 1, or more double bonds, including monocyclic, polycyclic, fused, bridged, and spiro rings, preferably monocyclic cycloalkyl having 3 to 8, 3 to 7, or 3 to 6 members, or bicyclic or tricyclic cycloalkyl having 7 to 10 members.

Examples of "monocyclic cycloalkyl" include, but are not limited to, cyclopropyl, cyclobutyl,

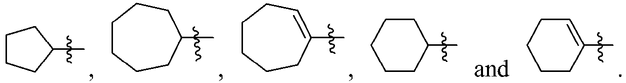

Monocyclic cycloalkyl may be substituted or unsubstituted.

"Spirocycloalkyl" refers to a polycyclic group having 5 to 18 members, two or more ring structures, and wherein monocyclic rings share one carbon atom (called a spiro atom) with each other, and the ring contains 0, 1, or more double bonds, but no ring has a fully conjugated π-electron aromatic system, preferably 6- to 14- membered, more preferably 7- to 10- membered. Spironcycloalkyl is classified into monospiro, dispiro, or polypiro cycloalkyl based on the number of spiro atoms shared between rings, preferably monospiro and dispiro cycloalkyl, preferably 4-membered/5-membered, 4-membered/4-membered, 4-membered/6-membered, 3-membered/6-membered, 5-membered/5-membered, or 5-membered/6-membered. Non-limiting examples of "spirocycloalkyl" include, but are not limited to: spiro[4.5]decyl, spiro[4.4]nonyl, spiro[3.5]nonyl, spiro[2.4]heptyl, 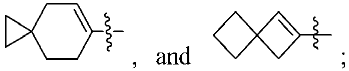 spirocycloalkyl may be substituted or unsubstituted.

"Fused cycloalkyl" refers to an all-carbon polycyclic group having 5 to 18 members, containing two or more ring structures sharing a pair of carbon atoms with each other, wherein one or more rings may contain 0, 1, or more double bonds, but no ring has a fully conjugated π-electron aromatic system, preferably 6- to 14- membered, more preferably 6- to 10- membered. According to the number of constituent rings, it can be classified into bicyclic, tricyclic, tetracyclic, or polycyclic fused cycloalkyl, preferably bicyclic or tricyclic, more preferably 3-membered/5-membered, 5-membered/5-membered, or 5-membered/6-membered bicyclic fused cycloalkyl. Non-limiting examples of "fused cycloalkyl" include, but are not limited to: bicyclo[3.1.0]hexyl, bicyclo[3.2.0]hept-1-enyl, bicyclo[3.2.0]heptyl, decahydronaphthyl, tetradecahydrophenanthryl, 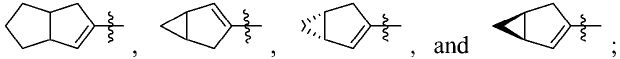 fused cycloalkyl may be substituted or unsubstituted.

"Bridged cycloalkyl" refers to an all-carbon polycyclic group having 5 to 18 members, containing two or more ring structures sharing two non-directly connected carbon atoms with each other, wherein one or more rings may contain 0, 1, or more double bonds, but no ring has a fully conjugated π-electron aromatic system, preferably 6- to 14- membered, more preferably 7-to 10- membered. Preferably 6- to 14- membered, more preferably 7- to 10- membered. According to the number of constituent rings, it can be classified into bicyclic, tricyclic, tetracyclic, or polycyclic bridged cycloalkyl, preferably bicyclic, tricyclic, or tetracyclic, more preferably bicyclic or tricyclic. Non-limiting examples of "bridged cycloalkyl" include, but are not limited to: (ls,4s)-bicyclo[2.2.1]heptyl, bicyclo[3.2.1]octyl, (1s,5s)-bicyclo[3.3.1]nonyl, bicyclo[2.2.2]octyl, (1r,5r)-bicyclo[3.3.2]decyl, and 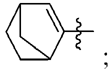 bridged cycloalkyl may be substituted or unsubstituted.

"Heterocyclyl," "heterocycloalkyl," "heterocycle," or "heterocyclic" are used interchangeably in the present application and all refer to non-aromatic heterocyclyl, wherein 1, 2, 3, or 4 ring-forming atoms are heteroatoms selected from the group consisting of nitrogen, oxygen, and S(O)ᵣ (wherein r is selected from the group consisting of 0, 1, and 2); the ring contains 0, 1, or more double bonds; including monocyclic, polycyclic, fused, bridged, and spiro rings; preferably having 5- to 7-membered monocycles or 7- to 10-membered bicyclic or tricyclic rings, which may contain 1, 2, or 3 atoms selected from nitrogen, oxygen, and/or sulfur. Preferably 3- to 8- membered heterocyclyl, 4- to 8- membered heterocyclyl, 5- to 7- membered heterocyclyl, or 7- to 10- membered heterocyclyl.

Heterocyclyl may be substituted or unsubstituted.

Examples of "monocyclic heterocyclyl" include, but are not limited to, morpholinyl, oxetanyl, azetidinyl, thiamorpholinyl, tetrahydrofuranyl, tetrahydropyranyl, 1,1-dioxido-thiamorpholinyl, piperidinyl, 2-oxo-piperidinyl, pyrrolidinyl, 2-oxo-pyrrolidinyl, piperazin-2-one, 8-oxa-3-aza-bicyclo[3.2.1]octyl, piperazinyl, hexahydropyrimidine,

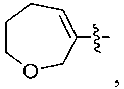

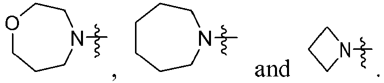

"Spiroheterocyclyl" refers to a polycyclic group having 5 to 18 members, two or more ring structures, and wherein monocyclic rings share one atom with each other, and the ring contains 0, 1, or more double bonds, but no ring has a fully conjugated π-electron aromatic system, wherein one or more ring atoms are heteroatoms selected from the group consisting of nitrogen, oxygen, and S(O)ᵣ (wherein r is selected from the group consisting of 0, 1, and 2), and the remaining ring atoms are carbon. Preferably 6- to 14- membered, more preferably 7- to 10-membered. According to the number of spiro atoms shared between rings, spiroheterocyclyl is classified into monospiroheterocyclyl, dispiroheterocyclyl, or polypiroheterocyclyl, preferably monospiroheterocyclyl and dispiroheterocyclyl. More preferably 3-membered/6-membered, 4-membered/4-membered, 4-membered/5-membered, 4-membered/6-membered, 5-membered/5-membered, 5-membered/6-membered, or 6-membered/6-membered monospiroheterocyclyl. Non-limiting examples of "spiroheterocyclyl" include, but are not limited to: 1,7-dioxaspiro[4.5]decyl, 2-oxa-7-azaspiro[4.4]nonyl, 7-oxaspiro[3.5]nonyl, 5-oxaspiro [2.4]heptyl,

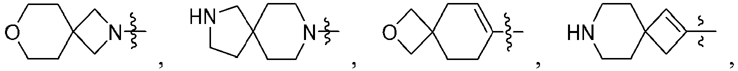

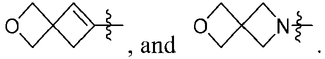

"Fused heterocyclyl" refers to a polycyclic group containing two or more ring structures sharing a pair of atoms with each other, wherein one or more rings may contain 0, 1, or more double bonds, but no ring has a fully conjugated π-electron aromatic system, wherein one or more ring atoms are heteroatoms selected from the group consisting of nitrogen, oxygen, and S(O)ᵣ (wherein r is selected from the group consisting of 0, 1, and 2), and the remaining ring atoms are carbon. Preferably 6 to 14 members, more preferably 7 to 10 members. According to the number of constituent rings, it can be classified into bicyclic, tricyclic, tetracyclic, or polycyclic fused heterocyclyl, preferably bicyclic or tricyclic, more preferably 5-membered/5-membered or 5-membered/6-membered bicyclic fused heterocyclyl. Non-limiting examples of "fused heterocyclyl" include, but are not limited to: octahydropyrrolo[3,4-c]pyrrolyl, octahydro-1H-isoindolyl, 3-azabicyclo[3.1.0]hexyl, octahydrobenzo[b][1,4]dioxine,

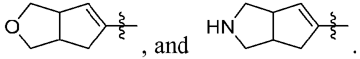

"Bridged heterocyclyl" refers to a polycyclic group having 5 to 14 members, 5 to 18 members, containing two or more ring structures sharing two non-directly connected atoms with each other, wherein one or more rings may contain 0, 1, or more double bonds, but no ring has a fully conjugated π-electron aromatic system, wherein one or more ring atoms are heteroatoms selected from the group consisting of nitrogen, oxygen, and S(O)ᵣ (wherein r is selected from the group consisting of 0, 1, and 2), and the remaining ring atoms are carbon. Preferably 6 to 14 members, more preferably 7 to 10 members. According to the number of constituent rings, it can be classified into bicyclic, tricyclic, tetracyclic, or polycyclic bridged heterocyclyl, preferably bicyclic, tricyclic, or tetracyclic, more preferably bicyclic or tricyclic. Non-limiting examples of "bridged heterocyclyl" include, but are not limited to: 2-azabicyclo[2.2.1]heptyl, 2-azabicyclo[2.2.2]octyl, and 2-azabicyclo[3.3.2]decyl.

"Aryl" refers to a carbocyclic aromatic system containing one or two rings, wherein the rings may be connected together in a fused manner. The term "aryl" includes monocyclic or bicyclic aryl groups, such as aromatic groups including phenyl, naphthyl, or tetrahydronaphthyl. Aryl is preferably C₆-C₁₀ aryl, more preferably phenyl and naphthyl, and most preferably naphthyl. Aryl may be substituted or unsubstituted.

"Heteroaryl" refers to an aromatic 5- to 6-membered monocyclic or 8- to 10-membered bicyclic ring, which may contain 1 to 4 heteroatoms selected from the group consisting of nitrogen, oxygen, and/or sulfur; preferably C5-10 heteroaryl. Examples of "heteroaryl" include, but are not limited to, furanyl, pyridyl, 2-oxo-1,2-dihydropyridyl, pyridazinyl, pyrimidyl, pyrazyl, thienyl, isoxazolyl, oxazolyl, oxadiazolyl, imidazolyl, pyrrolyl, pyrazolyl, triazolyl, tetrazolyl, thiazolyl, isothiazolyl, 1,2,3-thiadiazolyl, benzodioxolyl, benzothienyl, benzimidazolyl, indolyl, isoindolyl, 1,3-dioxo-isoindolyl, quinolyl, indazolyl, benzisothiazolyl, benzoxazolyl, benzisoxazolyl, isothiazolyl, 1H-1,2,4-triazolyl, 4H-1,2,4-triazolyl, pyridyl, pyridin-2(1H)-onyl, pyrimidyl, pyrazin-2(1H)-onyl, pyrimidin-4(3H)-onyl, pyrimidin-2(1H)-onyl, pyridazin-3(2H)-onyl, 1H-indolyl, 1H-benzo[d]imidazolyl, 1H-pyrrolo[2,3-c]pyridyl, 3H-imidazo[4,5-c]pyridyl, isoquinolyl, quinazolyl, 2H-isoindolyl, furan[3,2-b]pyridyl, furan[2,3-c]pyridyl, thieno[2,3-c]pyridyl, benzofuranyl, benzothiophenyl, 1H-pyrrolo[3,2-b]pyridyl, 2H-pyrrolo[3,4-c]pyridyl,

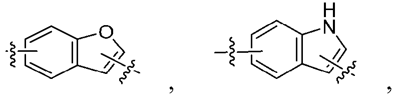

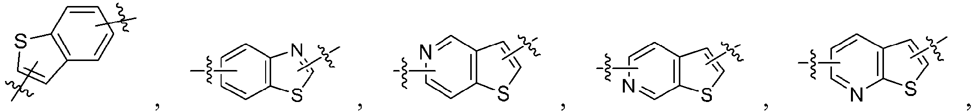

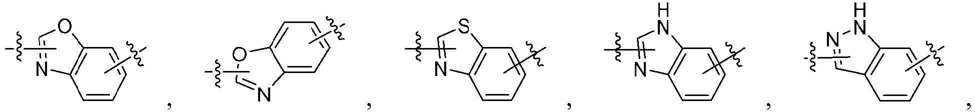

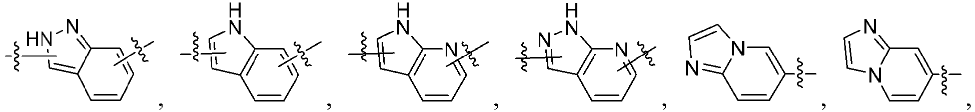

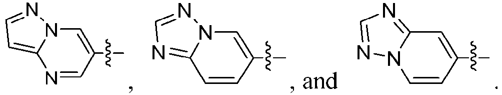

The heteroaryl may be substituted or unsubstituted.

"Fused ring" refers to a polycyclic group in which two or more ring structures share a pair of atoms, wherein at least one ring has an aromatic system with fully conjugated π electrons, and one or more rings may contain 0, 1, or more double bonds, but at least one ring does not have an aromatic system with fully conjugated π electrons, wherein the ring atoms are selected from 0, 1, or more heteroatoms selected from the group consisting of nitrogen, oxygen, and S(O)ᵣ (where r is selected from the group consisting of 0, 1, and 2), and the remaining ring atoms are carbon. The fused ring preferably includes bicyclic or tricyclic fused rings, wherein the bicyclic fused ring is preferably a fused ring of aryl or heteroaryl with monocyclic heterocyclyl or monocyclic cycloalkyl. Preferably 6- to 14- membered, more preferably 8- to 10- membered. Examples of "fused ring" include, but are not limited to:

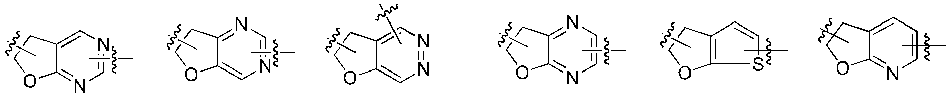

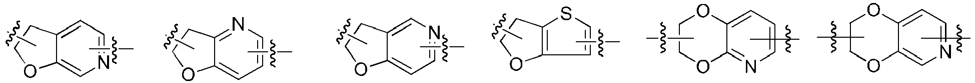

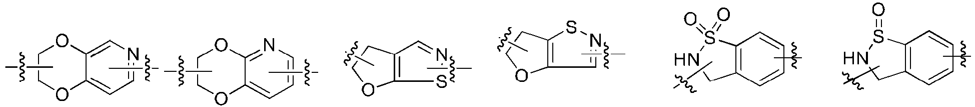

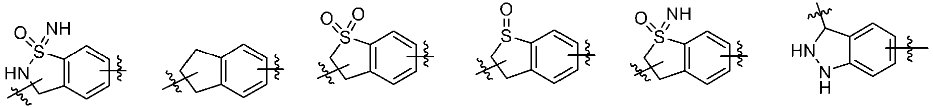

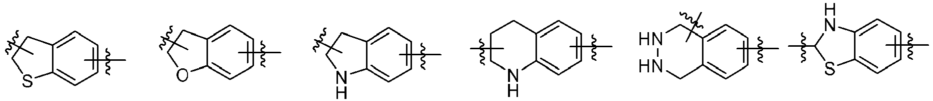

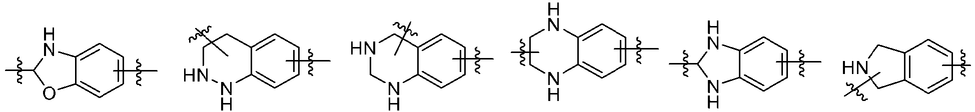

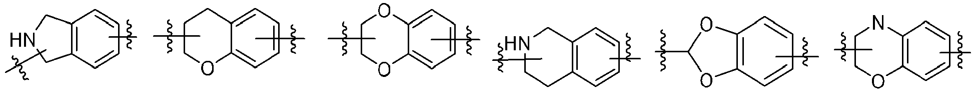

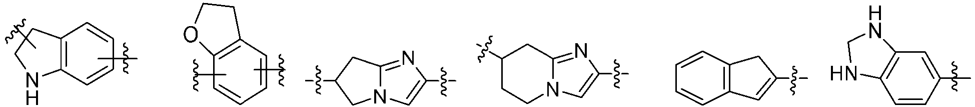

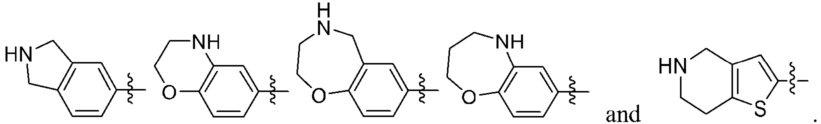

"Alkoxy" refers to a group of (alkyl-O-). wherein the alkyl is as defined herein. C₁-C₆ or C₁-C₄ alkoxy is preferred. Examples thereof include, but are not limited to: methoxy, ethoxy, n-propoxy, isopropoxy, n-butoxy, isobutoxy, and tert-butoxy.

"Alkylthio" refers to a group of (alkyl-S-). wherein the alkyl is as defined herein. C₁-C₆ or C₁-C₄ alkylthio is preferred. Examples thereof include, but are not limited to: methylthio, ethylthio, n-propylthio, isopropylthio, n-butylthio, isobutylthio, and tert-Butylthio.

"Nitro" refers to a -NO₂ group.

"Hydroxy" refers to a -OH group.

"Halogen" refers to fluorine, chlorine, bromine, and iodine; preferably fluorine, chlorine, and bromine.

"Amino" refers to -NH₂.

"Hydroxylamino" refers to -NHOH.

"Cyano" refers to -CN.

"Benzyl" refers to -CH₂-phenyl.

"Carboxy" refers to -C(=O)OH.

"Carboxylate" refers to -C(=O)O-alkyl or -C(=O)O-cycloalkyl, wherein the alkyl and cycloalkyl are as defined above.

"Hydroxyalkyl" refers to an alkyl substituted by hydroxy, wherein the alkyl is as defined above.

"Aminoalkyl" refers to an alkyl substituted by amino, wherein the alkyl is as defined above.

"Haloalkyl" refers to an alkyl substituted by halogen, wherein the alkyl is as defined above.

"Haloalkoxy" refers to an alkoxy substituted by halogen, wherein the alkoxy is as defined above.

"DMSO" refers to dimethyl sulfoxide.

"BOC" refers to tert-butoxycarbonyl.

"Bn" refers to benzyl.

"THP" refers to 2-tetrahydropyranyl.

"TFA" refers to trifluoroacetic acid.

"Ts" refers to tosyl.

"Bn" refers to benzyl.

"SEM" refers to (trimethylsilyl)ethoxymethyl.

"Leaving group" refers to an atom or functional group that dissociates from a larger molecule in a chemical reaction, and is a term used in nucleophilic substitution reactions and elimination reactions. In a nucleophilic substitution reaction, the reactant attacked by a nucleophile is called a substrate, and the atom or atomic group that breaks away from the substrate molecule with a pair of electrons is called a leaving group. Groups that readily accept electrons and have a strong ability to bear a negative charge are good leaving groups. The smaller the pKa of the conjugate acid of the leaving group, the easier it is for the leaving group to dissociate from other molecules. This is because when the pKa of its conjugate acid is smaller, the corresponding leaving group has a stronger tendency to exist in the form of an anion (or an electrically neutral leaving group) without binding to other atoms. Common leaving groups include, but are not limited to, halogen, mesyl, -OTs, or -OH.

"Substituted" refers to one or more hydrogen atoms, preferably up to 5, more preferably 1 to 3 hydrogen atoms in a group being independently replaced by a corresponding number of substituents. It is understood that the substituents are only in their possible chemical positions, and a person skilled in the art can determine possible or impossible substitutions without undue effort (through experimentation or theory). For example, an amino or hydroxy group having free hydrogen may be unstable when bonded to a carbon atom having an unsaturated (e.g., alkenyl) bond.

"Substituted" or "substituted" as described herein, unless otherwise specified, refers to a group being substituted by one or more substituents selected from the group consisting of: alkyl, alkenyl, alkynyl, alkoxy, alkylthio, alkylamino, halogen, mercapto, hydroxy, nitro, cyano, cycloalkyl, heterocyclyl, aryl, heteroaryl, cycloalkoxy, heterocycloalkoxy, cycloalkylthio, heterocycloalkylthio, amino, haloalkyl, hydroxyalkyl, carboxy, carboxylate, =O, OR⁵, -C(=O)R⁵, -C(=O)OR⁵, -NHC(=O)R⁵, -NHC(=O)OR⁵, -NR⁶R⁷, -C(=O)NR⁶R⁷, -CH₂NHC(=O)OR⁵, -CH₂NR⁶R⁷, and -S(O)ᵣR⁵.

As used in the present disclosure, when referring to being substituted by one or more substituents, the "one or more" includes 1, 2, 3, 4, 5, or more, preferably 1, 2, or 3.

As used in the present disclosure, the definitions of the above groups apply not only to when the groups appear alone, but also to when they appear as substituents.

Each R⁵ is independently selected from the group consisting of hydrogen atom, alkyl, cycloalkyl, heterocyclyl, aryl, and heteroaryl, wherein the alkyl, cycloalkyl, heterocyclyl, aryl, or heteroaryl is optionally further substituted by one or more substituents selected from the group consisting of hydroxy, halogen, nitro, cyano, alkyl, alkoxy, haloalkyl, haloalkoxy, cycloalkyl, heterocyclyl, aryl, heteroaryl, =O, -C(=O)R⁸, -C(=O)OR⁸, -OC(=O)R⁸, -NR⁹R¹⁰, -C(=O)NR⁹R¹⁰, -SO₂NR⁹R¹⁰, and -NR⁹C(=O)R¹⁰;
R⁶ and R⁷ are each independently selected from the group consisting of hydrogen atom, hydroxy, halogen, alkyl, alkoxy, cycloalkyl, heterocyclyl, aryl, and heteroaryl, wherein the alkyl, alkoxy, cycloalkyl, heterocyclyl, aryl, or heteroaryl is optionally further substituted by one or more substituents selected from the group consisting of hydroxy, halogen, nitro, cyano, alkyl, alkoxy, cycloalkyl, heterocyclyl, aryl, heteroaryl, =O, -C(=O)R⁸, -C(=O)OR⁸, -OC(=O)R⁸, -NR⁹R¹⁰, -C(=O)NR⁹R¹⁰, -SO₂NR⁹R¹⁰, and -NR⁹C(=O)R¹⁰;
alternatively, R⁶ and R⁷ together with the atoms to which they are attached form a 4- to 8- membered heterocyclyl, wherein the 4- to 8- membered heterocyclyl contains one or more N, O, or S(O)r, and the 4- to 8- membered heterocyclyl is optionally further substituted by one or more substituents selected from the group consisting of hydroxy, halogen, nitro, cyano, alkyl, alkoxy, cycloalkyl, heterocyclyl, aryl, heteroaryl, =O, -C(=O)R⁸, -C(=O)OR⁸, -OC(=O)R⁸, -NR⁹R¹⁰, -C(=O)NR⁹R¹⁰, -SO₂NR⁹R¹⁰, and -NR⁹C(=O)R¹⁰;
each of R⁸, R⁹, and R¹⁰ is independently selected from the group consisting of hydrogen atom, alkyl, amino, cycloalkyl, heterocyclyl, aryl, and heteroaryl, wherein the alkyl, cycloalkyl, heterocyclyl, aryl, or heteroaryl is optionally further substituted by one or more substituents selected from the group consisting of hydroxy, halogen, nitro, amino, cyano, alkyl, alkoxy, cycloalkyl, heterocyclyl, aryl, heteroaryl, carboxy, and carboxylate; and
each r is independently 0, 1, or 2.

The compounds of the present disclosure may contain an asymmetric center or chiral center, and thus exist in different stereoisomeric forms. It is intended that all stereoisomeric forms of the compounds of the present disclosure, including but not limited to diastereomers, enantiomers, atropisomers, and geometric (conformational) isomers, and mixtures thereof, such as racemic mixtures, are all within the scope of the present disclosure.

Unless otherwise specified, the structures described in the present disclosure also include all isomers of such structures (e.g., diastereomers, enantiomers, atropisomers, and geometric (conformational) isomer forms; for example, R and S configurations at each asymmetric center, (Z) and (E) double bond isomers, and (Z) and (E) conformational isomers. Accordingly, individual stereoisomers of the compounds of the present disclosure, as well as enantiomer mixtures, diastereomer mixtures, and geometric (conformational) isomer mixtures, are all within the scope of the present disclosure.

"Pharmaceutically acceptable salt" refers to certain salts of the above-mentioned compounds that retain the original biological activity and are suitable for pharmaceutical use. The pharmaceutically acceptable salt of the compound represented by formula (I) may be a metal salt or an amine salt formed with a suitable acid.

"Pharmaceutical composition" refers to a mixture containing one or more compounds described in the present disclosure, or physiologically pharmaceutically acceptable salts or prodrugs thereof, with other chemical components, as well as other components such as a physiologically pharmaceutically acceptable carrier. The purpose of the pharmaceutical composition is to facilitate administration to an organism and promote absorption of the active ingredient to exert biological activity.

### Synthetic Methods for Compounds of the Present Disclosure

To achieve the objectives of the present disclosure, the following technical solution is adopted in the present disclosure:
The present disclosure provides a method for producing a compound of formula (I), or a stereoisomer, tautomer, or pharmaceutically acceptable salt thereof, wherein the method comprises:

### Method 1:

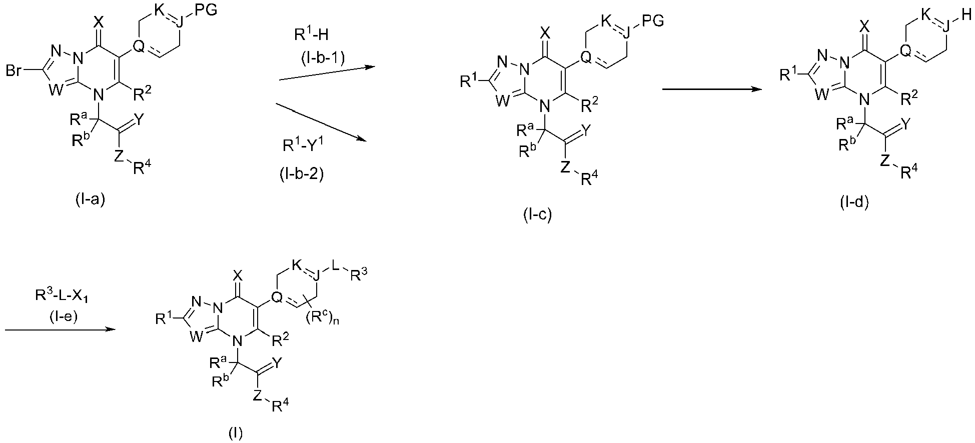

A compound of formula (I-a) undergoes SN₂ nucleophilic substitution reaction with a compound of formula (I-b-1) in the presence of a base, or undergoes a coupling reaction with a compound of formula (I-b-2) in the presence of a catalyst, to obtain a compound of formula (I-c); the compound of formula (I-c) further undergoes a deprotection reaction to obtain a compound of formula (I-d); the compound of formula (I-d) undergoes a condensation reaction with a compound of formula (I-e), and optionally further undergoes a deprotection reaction to obtain a compound of formula (I);
wherein:
K - - - - J is a single bond, and J is N;
PG is an amino protecting group, preferably tert-butoxycarbonyl;
Y₁ is selected from the group consisting of boronic acid and boronate ester;
L is selected from the group consisting of -(C=O)-;
X₁ is selected from the group consisting of hydroxy and halogen;
W, K, X, Y, Z, Q, R¹-R⁴, R^{a}, R^{b}, R^{c}, and n are as defined in claim 1.

### Method 2:

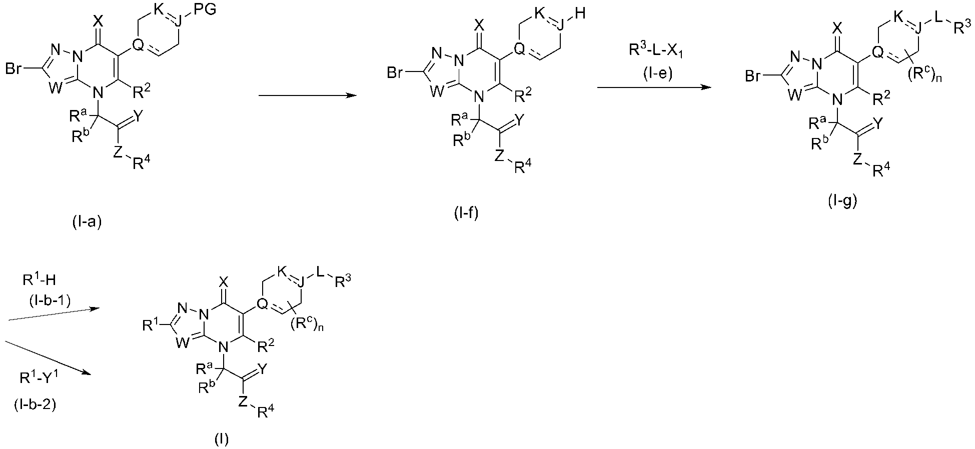

A compound of formula (I-a) undergoes a deprotection reaction to obtain a compound of formula (I-f); the compound of formula (I-f) undergoes a condensation reaction with a compound of formula (I-e) to obtain a compound of formula (I-g); the compound of formula (I-g) undergoes SN₂ nucleophilic substitution reaction with a compound of formula (I-b-1) in the presence of a base, or undergoes a coupling reaction with a compound of formula (I-b-2) in the presence of a catalyst, and optionally further undergoes a deprotection reaction, to obtain a compound of formula (I);
wherein:
K - - - - J is a single bond, and J is N;
PG is an amino protecting group, preferably tert-butoxycarbonyl;
Y₁ is selected from the group consisting of boronic acid and boronate ester;
L is selected from the group consisting of -(C=O)-;
X₁ is selected from the group consisting of hydroxy and halogen;
W, K, X, Y, Z, Q, R¹-R⁴, R^{a}, R^{b}, R^{c}, and n are as defined in claim 1.

### Detailed Description of Embodiments

The present disclosure is further described below in conjunction with examples, however, these examples are not intented to limit the scope of the present disclosure.

### Examples

The examples provide the production for representative compounds of formula (I) and related structural characterization data. It should be noted that the following examples are for illustrating the present disclosure and not for limiting the present disclosure. ¹H NMR spectra were measured using a Bruker instrument (400 MHz), and chemical shifts are reported in ppm. Tetramethylsilane was used as an internal standard (0.00 ppm). The representation of ¹H NMR: s = singlet, d = doublet, t = triplet, m = multiplet, br = broadened, dd = doublet of doublets, dt = doublet of triplets. When coupling constants are provided, the unit is Hz.

Mass spectra were measured using an LC/MS instrument, and the ionization mode may be ESI or APCI.

For thin layer chromatography (TLC), Yantai Huanghai HSGF254 or Qingdao GF254 silica gel plates were used. The specification of silica gel plates used for thin layer chromatography (TLC) was 0.15 mm to 0.2 mm, and the specification of those used for separation and purification by thin layer chromatography was 0.4 mm to 0.5 mm.

For column chromatography, Yantai Huanghai silica gel of 200 to 300 mesh was generally used as a carrier.

In the following examples, unless otherwise specified, all temperatures are presented in degrees Celsius. Unless otherwise specified, various starting materials and reagents are commercially available or are synthesized according to known methods. Commercially available starting materials and reagents are used directly without further purification. Unless otherwise specified, commercial manufacturers include, but are not limited to, Aldrich Chemical Company, ABCR GmbH & Co. KG, Acros Organics, Guangzan Chemical Technology Co., Ltd., and Jingyan Chemical Technology Co., Ltd.

CD₃OD: deuterated methanol.

CDCl₃: deuterated chloroform.

DMSO-*d*₆: deuterated dimethyl sulfoxide.

Argon atmosphere refers to a reaction flask connected to an argon balloon with a volume of about 1 L.

In the examples, unless otherwise specified, solutions in reactions refer to aqueous solutions.

Compounds were purified using silica gel column chromatography and reversed-phase column chromatography. The eluent systems were selected from the group consisting of: A: petroleum ether and ethyl acetate system; B: dichloromethane and methanol system; C: dichloromethane: ethyl acetate; and D: trifluoroacetic acid aqueous solution and acetonitrile system. The volume ratio of solvents varied depending on the polarity of the compound, and small amounts of acidic or basic reagents may be added for adjustment, such as acetic acid or triethylamine.

### Example 1

### N-(2-chloro-4-(trifluoromethyl)phenyl)-2-(5-ethyl-6-(4-(5-hydroxy-6-methylpyrimidine-4-carbon yl)piperazin-1-yl)-2-(1,4-oxazepan-4-yl)-7-oxo-[1,2,4]triazolo[1,5-a]pyrimidin-4(7H)-yl)acetami de

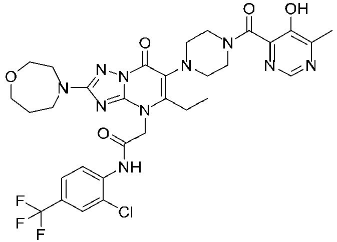

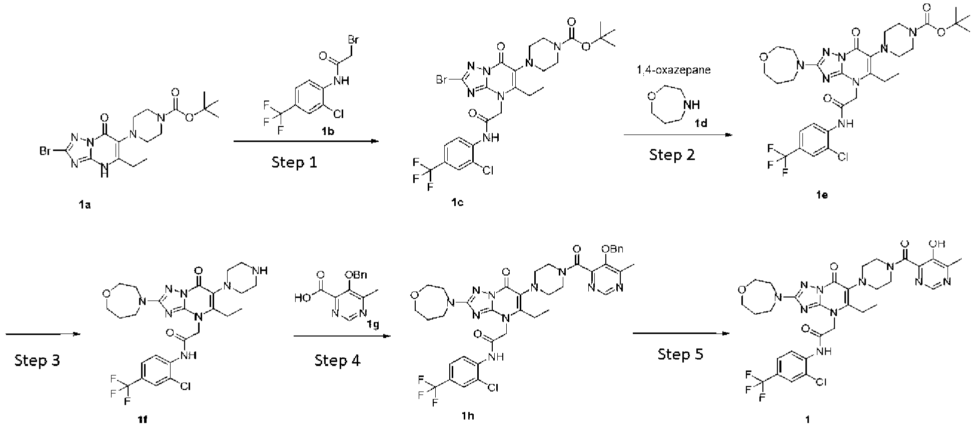

### Step 1

### tert-butyl 4-(2-bromo-4-(2-((2-chloro-4-(trifluoromethyl)phenyl)amino)-2-oxoethyl)-5-ethyl-7-oxo-4,7-dihydro-[1,2,4]triazolo[1,5-a]pyrimidin-6-yl)piperazine-1-carboxylate

tert-butyl 4-(2-bromo-5-ethyl-7-oxo-4,7-dihydro-[1,2,4]triazolo[1,5-a]pyrimidin-6-yl) piperazine-1-carboxylate **1a** (2 g, 4.68 mmol, prepared according to published patent WO2022249060 A1) and 2-bromo-N-(2-chloro-4-(trifluoromethyl)phenyl)acetamide **1b** (1.93 g, 6.08 mmol, prepared according to published patent WO2022249060 A1) were added to N,N-dimethylformamide (20 mL). N,N-diisopropylethylamine (1.81 g, 14.04 mmol) was added, the temperature was raised to 50 °C, and the reaction was performed for 4 hours. The reaction mixture was extracted with ethyl acetate (30 mL × 2). The combined organic phase was washed with saturated sodium chloride solution (30 mL × 2), dried over anhydrous sodium sulfate, filtered, and concentrated under reduced pressure. The resulting residue was purified by silica gel column chromatography (eluent: System B) to obtain tert-butyl 4-(2-bromo-4-(2-((2-chloro-4-(trifluoromethyl)phenyl)amino)-2-oxoethyl)-5-ethyl-7-oxo-4,7-dih ydro-[1,2,4]triazolo[1,5-a]pyrimidin-6-yl)piperazine-1-carboxylate **1c** (2.9 g) in a yield of 93.47%.

MS m/z (ESI): 563.7 [M-100+1]

### Step 2

### tert-butyl 4-(4-(2-((2-chloro-4-(trifluoromethyl)phenyl)amino)-2-oxoethyl)-5-ethyl-2-(1,4-oxazepan-4-yl)-7-oxo-4,7-dihydro-[1,2,4]triazolo[1,5-a]pyrimidin-6-yl)piperazine-1-carboxylate

tert-butyl 4-(2-bromo-4-(2-((2-chloro-4-(trifluoromethyl)phenyl)amino)-2-oxoethyl) -5-ethyl-7-oxo-4,7-dihydro-[1,2,4]triazolo[1,5-a]pyrimidin-6-yl)piperazine-1-carboxylate **1c** (70 mg, 105.60 µmol), 1,4-oxazepane **1d** (53.40 mg, 527.99 µmol, commercially available), and N,N-diisopropylethylamine (68.24 mg, 527.99 µmol) were added to a microwave tube. The temperature was raised to 100 °C, and the reaction was performed overnight. The reaction mixture was extracted with ethyl acetate (30 mL × 2). The combined organic phase was washed with saturated sodium chloride solution (30 mL × 2), dried over anhydrous sodium sulfate, filtered, and concentrated under reduced pressure. The resulting residue was purified by silica gel column chromatography (eluent: System A) to obtain tert-butyl 4-(4-(2-((2-chloro-4-(trifluoromethyl)phenyl)amino)-2-oxoethyl)-5-ethyl-2-(1,4-oxazepan-4-yl)-7-oxo-4,7-dihydro-[ 1,2,4]triazolo[1,5-a]pyrimidin-6-yl)piperazine-1-carboxylate **1e** (60 mg) in a yield of 83.18%.

MS m/z (ESI): 626.8 [M-56+1]

### Step 3

### N-(2-chloro-4-(trifluoromethyl)phenyl)-2-(5-ethyl-2-(1,4-oxazepan-4-yl)-7-oxo-6-(piperazin-1-yl)-[1,2,4]triazolo[1,5-a]pyrimidin-4(7H)-yl)acetamide

tert-butyl 4-(4-(2-((2-chloro-4-(trifluoromethyl)phenyl)amino)-2-oxoethyl)-5-ethyl-2-(1,4-oxazepan-4-yl)-7-oxo-4,7-dihydro-[1,2,4]triazolo[1,5-a]pyrimidin-6-yl)piperazine-1-carbo xylate **1e** (60 mg, 87.83 µmol) was added to dichloromethane (2.5 mL). Trifluoroacetic acid (0.5 mL) was added, and the reaction was performed at room temperature for 30 minutes. The reaction mixture was concentrated under reduced pressure to obtain N-(2-chloro-4-(trifluoromethyl)phenyl)-2-(5-ethyl-2-(1,4-oxazepan-4-yl)-7-oxo-6-(piperazin-1-yl )-[1,2,4]triazolo[1,5-a]pyrimidin-4(7H)-yl)acetamide **1f** (50 mg) in a yield of 97.64%.

MS m/z (ESI): 583.2 [M+1]

### Step 4

### 2-(6-(4-(5-(benzyloxy)-6-methylpyrimidine-4-carbonyl)piperazin-1-yl)-5-ethyl-2-(1,4-oxazepan-4-yl)-7-oxo-[1,2,4]triazolo[1,5-a]pyrimidin-4(7H)-yl)-N-(2-chloro-4-(trifluoromethyl)phenyl)acet amide

N-(2-Chloro-4-(trifluoromethyl)phenyl)-2-(5-ethyl-2-(1,4-oxazepan-4-yl)-7-oxo-6-(pipe razin-1-yl)-[1,2,4]triazolo[1,5-a]pyrimidin-4(7H)-yl)acetamide **1f** (50 mg, 85.76 µmol), 5-(benzyloxy)-6-methylpyrimidine-4-carboxylic acid **1g** (27.23 mg, 111.49 µmol, prepared according to published patent WO2022249060 A1), 1-(3-(dimethylamino)propyl)-3-ethylcarbodiimide hydrochloride (24.66 mg, 128.64 µmol), 1-hydroxybenzotriazole (17.38 mg, 128.64 µmol), and N,N-diisopropylethylamine (55.42 mg, 428.81 µmol) were added to N,N-dimethylformamide (2 mL), and the reaction was performed at room temperature for 4 h. The reaction mixture was extracted with ethyl acetate (30 mL ×2), the combined organic phases were washed with saturated sodium chloride solution (30 mL×2), dried over anhydrous sodium sulfate, filtered, and concentrated under reduced pressure, and the resulting residue was purified by silica gel column chromatography (eluent: System B) to obtain 2-(6-(4-(5-(benzyloxy)-6-methylpyrimidine-4-carbonyl)piperazin-1-yl)-5-ethyl-2-(1,4-oxazepan-4-yl)-7-oxo-[1,2,4]triazolo[1,5-a]pyrimidin-4(7H)-yl)-N-(2-chloro-4-(trifluoromethyl)phenyl)acet amide **1h** (50 mg), yield: 72.04%.

MS m/z (ESI): 790.3[M+1]

### Step 5

### N-(2-Chloro-4-(trifluoromethyl)phenyl)-2-(5-ethyl-6-(4-(5-hydroxy-6-methylpyrimidine-4-carbo nyl)piperazin-1-yl)-2-(1,4-oxazepan-4-yl)-7-oxo-[1,2,4]triazolo[1,5-a]pyrimidin-4(7H)-yl)acetam ide

2-(6-(4-(5-(Benzyloxy)-6-methylpyrimidine-4-carbonyl)piperazin-1-yl)-5-ethyl-2-(1,4-o xazepan-4-yl)-7-oxo-[1,2,4]triazolo[1,5-a]pyrimidin-4(7H)-yl)-N-(2-chloro-4-(trifluoromethyl)ph enyl)acetamide **1h** (50 mg, 61.79 µmol) was added to dichloromethane (2 mL), boron trichloride (1 mL) was added at 0 °C, and the reaction was performed at 0 °C for 1 h. Methanol (1 mL) was added to the reaction mixture to quench the reaction, the mixture was concentrated under reduced pressure, and the resulting residue was separated by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-2-(1,4-oxazepan-4-yl)-7-oxo-[1,2,4]triazolo[1,5-a]pyrimidin-4(7H)-yl)acetami de **1** (2.67 mg), yield: 4.95%.

MS m/z (ESI): 718.8[M+1]
¹H NMR (400 MHz, DMSO-*d₆*) δ 10.36 (s, 1H), 10.25 (s, 1H), 8.58 (s, 1H), 8.05 - 7.91 (m, 2H), 7.73 (d, *J* = 8.7 Hz, 1H), 5.21 (s, 2H), 4.51 (d, *J* = 12.5 Hz, 1H), 3.65 (td, *J* = 14.7, 5.5 Hz, 11H), 3.22 (d, *J* = 13.1 Hz, 1H), 3.02 - 2.84 (m, 3H), 2.78 (d, *J* = 11.4 Hz, 1H), 2.59 (s, 1H), 2.44 (s, 3H), 1.84 (t, *J* = 5.9 Hz, 2H), 1.26 - 1.01 (m, 4H).

### Example 2

### N-(2-Chloro-4-(trifluoromethyl)phenyl)-2-(2-cyclopentyl-5-ethyl-6-(4-(5-hydroxy-6-methylpyri midine-4-carbonyl)piperazin-1-yl)-7-oxo-[1,2,4]triazolo[1,5-a]pyrimidin-4(7H)-yl)acetamide

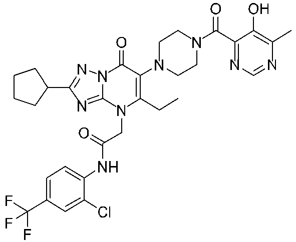

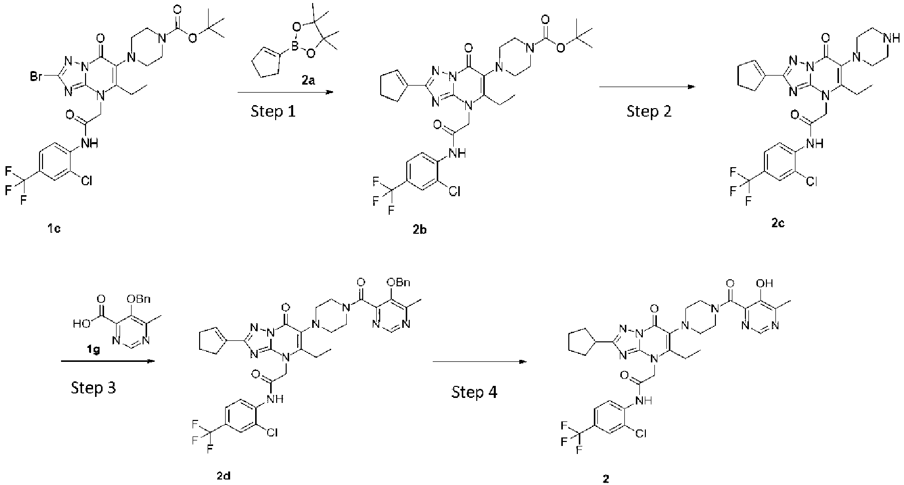

### Step 1

### tert-butyl 4-(4-(2-((2-chloro-4-(trifluoromethyl)phenyl)amino)-2-oxoethyl)-2-(cyclopent-1-en -1-yl)-5-ethyl-7-oxo-4,7-dihydro-[1,2,4]triazolo[1,5-a]pyrimidin-6-yl)piperazine-1-carboxylate

At room temperature, tert-butyl 4-(2-bromo-4-(2-((2-chloro-4-(trifluoromethyl)phenyl) amino)-2-oxoethyl)-5-ethyl-7-oxo-4,7-dihydro-[1,2,4]triazolo[1,5-a]pyrimidin-6-yl)piperazine-1-carboxylate **1c** (150 mg, 226.28 µmol), 2-(cyclopent-1-en-1-yl)-4,4,5,5-tetramethyl -1,3,2-dioxaborolane **2a** (52.70 mg, 271.54 µmol), [1,1'-bis(diphenylphosphino)ferrocene] palladium(II) dichloride dichloromethane complex (18.34 mg, 22.63 µmol), sodium carbonate (71.95 mg, 678.85 µmol), and 1,4-dioxane (1 mL) were added to a 100 mL two-necked flask, argon was exchanged four times, the temperature was raised to 80 °C, and the reaction was performed for 1.5 h. Water (20 mL) was added to the reaction mixture, the mixture was extracted with ethyl acetate (40 mL ×3), the organic phases were combined, washed with saturated sodium chloride solution (20 mL), dried over anhydrous sodium sulfate, filtered, and concentrated under reduced pressure, and the resulting residue was purified by silica gel column chromatography (eluent: System B) to obtain tert-butyl 4-(4-(2-((2-chloro-4-(trifluoromethyl)phenyl)amino)-2-oxoethyl)-2-(cyclopent-1-en-1-yl)-5-ethyl-7-oxo-4,7-dihydro-[1,2,4]triazolo[1,5-a]pyrimidin-6-yl )piperazine-1-carboxylate **2b** (110 mg), yield: 74.78%.

MS m/z (ESI): 650.3[M-56+1]

### Step 2

### N-(2-Chloro-4-(trifluoromethyl)phenyl)-2-(2-(cyclopent-1-en-1-yl)-5-ethyl-7-oxo-6-(piperazin-1-yl)-[1,2,4]triazolo[1,5-a]pyrimidin-4(7H)-yl)acetamide

tert-butyl 4-(4-(2-((2-chloro-4-(trifluoromethyl)phenyl)amino)-2-oxoethyl)-2-(cyclopent-1-en-1-yl)-5-ethyl-7-oxo-4,7-dihydro-[1,2,4]triazolo[1,5-a]pyrimidin-6-yl)piperazine-1-carboxylate **2b** (110 mg, 169.21 µmol) was added to dichloromethane (2.5 mL), trifluoroacetic acid (0.5 mL) was added, and the reaction was performed at room temperature for 1 h. The reaction mixture was concentrated under reduced pressure to obtain N-(2-chloro-4-(trifluoromethyl)phenyl)-2-(2-(cyclopent-1-en-1-yl)-5-ethyl-7-oxo-6-(piperazin-1-yl)-[1,2,4]triazolo[1,5-a]pyrimidin-4(7H)-yl)acetamide **2c** (90 mg), yield: 96.71%.

MS m/z (ESI): 559.8 [M+1]

### Step 3

### 2-(6-(4-(5-(Benzyloxy)-6-methylpyrimidine-4-carbonyl)piperazin-1-yl)-2-(cyclopent-1-en-1-yl)-5-ethyl-7-oxo-[1,2,4]triazolo[1,5-a]pyrimidin-4(7H)-yl)-N-(2-chloro-4-(trifluoromethyl)phenyl)a cetamide

N-(2-Chloro-4-(trifluoromethyl)phenyl)-2-(2-(cyclopent-1-en-1-yl)-5-ethyl-7-oxo-6-(pi perazin-1-yl)-[1,2,4]triazolo[1,5-a]pyrimidin-4(7H)-yl)acetamide **2c** (90 mg, 163.64 µmol), 5-(benzyloxy)-6-methylpyrimidine-4-carboxylic acid **1g** (51.96 mg, 212.74 µmol), 1-(3-(dimethylamino)propyl)-3-ethylcarbodiimide hydrochloride (47.06 mg, 245.47 µmol), 1-hydroxybenzotriazole (33.17 mg, 245.47 µmol), and N,N-diisopropylethylamine (105.75 mg, 818.22 µmol) were added to N,N-dimethylformamide (2 mL), and the reaction was performed at room temperature for 4 h. The reaction mixture was extracted with ethyl acetate (30 mL ×2), the combined organic phases were washed with saturated sodium chloride solution (30 mL ×2), dried over anhydrous sodium sulfate, filtered, and concentrated under reduced pressure, and the obtained residue was purified by silica gel column chromatography (eluent: System B) to give 2-(6-(4-(5-(benzyloxy)-6-methylpyrimidine-4-carbonyl)piperazin-1-yl)-2-(cyclopent-1-en-1-yl)-5 -ethyl-7-oxo-[1,2,4]triazolo[1,5-a]pyrimidin-4(7H)-yl)-N-(2-chloro-4-(trifluoromethyl)phenyl)ac etamide **2d** (80 mg), yield: 62.98%.

MS m/z (ESI): 775.8 [M+1]

### Step 4

### N-(2-Chloro-4-(trifluoromethyl)phenyl)-2-(2-cyclopentyl-5-ethyl-6-(4-(5-hydroxy-6-methylpyri midine-4-carbonyl)piperazin-1-yl)-7-oxo-[1,2,4]triazolo[1,5-a]pyrimidin-4(7H)-yl)acetamide

2-(6-(4-(5-(Benzyloxy)-6-methylpyrimidine-4-carbonyl)piperazin-1-yl)-2-(cyclopent-1-en-1-yl)-5-ethyl-7-oxo-[1,2,4]triazolo[1,5-a]pyrimidin-4(7H)-yl)-N-(2-chloro-4-(trifluoromethyl) phenyl)acetamide **2d** (50 mg, 64.42 µmol) was added to methanol (1 mL), 10% palladium on carbon (7.82 mg) was added, and the reaction was performed at room temperature overnight. The reaction mixture was filtered, concentrated under reduced pressure, and purified by preparative liquid chromatography (separation column: AKZONOBEL Kromasil; 250 ×21.2 mm I.D.; 5 µm, 20 mL/min; mobile phase A: 0.05% TFA+H₂O, mobile phase B: CH₃CN) to give N-(2-chloro-4-(trifluoromethyl)phenyl)-2-(2-cyclopentyl-5-ethyl-6-(4-(5-hydroxy-6-methylpyrim idine-4-carbonyl)piperazin-1-yl)-7-oxo-[1,2,4]triazolo[1,5-a]pyrimidin-4(7H)-yl)acetamide **2** (7.12 mg), yield: 4.68%.

MS m/z (ESI): 688.3 [M+1]
¹H NMR (400 MHz, DMSO-*d₆*) δ 10.34 (s, 1H), 10.23 (s, 1H), 8.58 (s, 1H), 8.03 (d, *J* = 8.6 Hz, 1H), 7.97 (d, *J* = 2.1 Hz, 1H), 7.72 (dd, *J* = 8.7, 2.1 Hz, 1H), 5.29 (s, 2H), 4.52 (d, *J* = 12.5 Hz, 1H), 3.49 (d, *J* = 11.3 Hz, 3H), 3.28 - 3.11 (m, 2H), 2.97 (d, *J* = 10.3 Hz, 3H), 2.80 (d, *J* = 11.3 Hz, 1H), 2.62 (d, *J* = 10.7 Hz, 1H), 2.44 (s, 3H), 2.02 - 1.90 (m, 2H), 1.86 - 1.58 (m, 6H), 1.17 (t, *J* = 7.4 Hz, 3H).

### Example 3

### 2-(2-(Azepan-1-yl)-5-ethyl-6-(4-(5-hydroxy-6-methylpyrimidine-4-carbonyl)piperazin-1-yl)-7-ox o-[1,2,4]triazolo[1,5-a]pyrimidin-4(7H)-yl)-N-(2-chloro-4-(trifluoromethyl)phenyl)acetamide

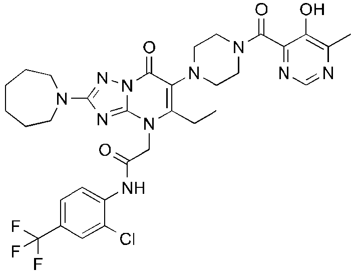

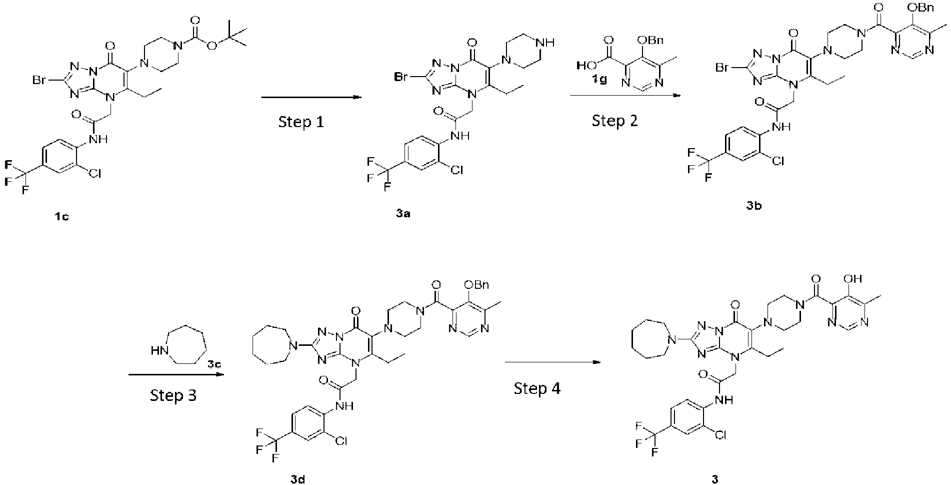

### Step 1

### 2-(2-Bromo-5-ethyl-7-oxo-6-(piperazin-1-yl)-[1,2,4]triazolo[1,5-a]pyrimidin-4(7H)-yl)-N-(2-chl oro-4-(trifluoromethyl)phenyl)acetamide

tert-butyl 4-(2-bromo-4-(2-((2-chloro-4-(trifluoromethyl)phenyl)amino)-2-oxoethyl) -5-ethyl-7-oxo-4,7-dihydro-[1,2,4]triazolo[1,5-a]pyrimidin-6-yl)piperazine-1-carboxylate **1c** (1 g, 1.51 mmol) was added to dichloromethane (5 mL), trifluoroacetic acid (2 mL) was added, and the reaction was performed at room temperature for 1 h. The reaction mixture was concentrated under reduced pressure to give 2-(2-bromo-5-ethyl-7-oxo-6-(piperazin-1-yl)-[1,2,4]triazolo [1,5-a]pyrimidin-4(7H)-yl)-N-(2-chloro-4-(trifluoromethyl)phenyl)acetamide **3a** (840 mg), yield: 98.94%.

MS m/z (ESI): 561.7 [M+1]

### Step 2

### 2-(6-(4-(5-(Benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide

2-(2-Bromo-5-ethyl-7-oxo-6-(piperazin-1-yl)-[1,2,4]triazolo[1,5-a]pyrimidin-4(7H)-yl)-N-(2-chloro-4-(trifluoromethyl)phenyl)acetamide **3a** (840 mg, 1.49 mmol), 5-(benzyloxy)-6-methylpyrimidine-4-carboxylic acid **1g** (473.93 mg, 1.94 mmol), 1-(3-(dimethylamino)propyl)-3-ethylcarbodiimide hydrochloride (429.20 mg, 2.24 mmol), 1-hydroxybenzotriazole (302.52 mg, 2.24 mmol), and N,N-diisopropylethylamine (964.53 mg, 7.46 mmol) were added to N,N-dimethylformamide (2 mL), and the reaction was performed at room temperature for 4 h. The reaction mixture was extracted with ethyl acetate (30 mL ×2), the combined organic phases were washed with saturated sodium chloride solution (30 mL×2), dried over anhydrous sodium sulfate, filtered, and concentrated under reduced pressure, and the obtained residue was purified by silica gel column chromatography (eluent: System B) to give 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (700 mg), yield: 59.44%.

MS m/z (ESI): 788.6 [M+1]

### Step 3

### 2-(2-(Azepan-1-yl)-6-(4-(5-(benzyloxy)-6-methylpyrimidine-4-carbonyl)piperazin-1-yl)-5-ethyl-7-oxo-[1,2,4]triazolo[1,5-a]pyrimidin-4(7H)-yl)-N-(2-chloro-4-(trifluoromethyl)phenyl)acetamid e

2-(6-(4-(5-(Benzyloxy)-6-methylpyrimidine-4-carbonyl)piperazin-1-yl)-2-bromo-5-ethy 1-7-oxo-[1,2,4]triazolo[1,5-a]pyrimidin-4(7H)-yl)-N-(2-chloro-4-(trifluoromethyl)phenyl)acetami de **3b** (50 mg, 63.37 µmol), azepane **3c** (62.85 mg, 633.71 µmol) and N,N-dimethylformamide (1 mL) were added to a microwave tube, the temperature was raised to 80 °C, and the reaction was performed for 1 h. The reaction mixture was extracted with ethyl acetate (30 mL ×2), the organic phases were combined, washed with saturated sodium chloride solution (30 mL×2), dried over anhydrous sodium sulfate, filtered, and concentrated under reduced pressure. The resulting residue was purified by silica gel column chromatography (eluent: System A) to obtain 2-(2-(azepan-1-yl)-6-(4-(5-(benzyloxy)-6-methylpyrimidine-4-carbonyl)piperazin-1-yl)-5-ethyl-7 -oxo-[1,2,4]triazolo[1,5-a]pyrimidin-4(7H)-yl)-N-(2-chloro-4-(trifluoromethyl)phenyl)acetamide **3d** (40 mg), yield: 78.19%.

MS m/z (ESI): 806.8 [M+1]

### Step 4

### 2-(2-(azepan-1-yl)-5-ethyl-6-(4-(5-hydroxy-6-methylpyrimidine-4-carbonyl)piperazin-1-yl)-7-ox o-[1,2,4]triazolo[1,5-a]pyrimidin-4(7H)-yl)-N-(2-chloro-4-(trifluoromethyl)phenyl)acetamide

2-(2-(azepan-1-yl)-6-(4-(5-(benzyloxy)-6-methylpyrimidine-4-carbonyl)piperazin-1-yl)-5-ethyl-7-oxo-[1,2,4]triazolo[1,5-a]pyrimidin-4(7H)-yl)-N-(2-chloro-4-(trifluoromethyl)phenyl)a cetamide **3d** (40 mg, 49.55 µmol) was added to dichloromethane (2.5 mL), boron trichloride (1 mL) was added at 0 °C, and the reaction was performed at 0 °C for 1 h. The reaction mixture was quenched by adding methanol (1 mL), concentrated under reduced pressure, and the resulting residue was purified by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain 2-(2-(azepan-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluor omethyl)phenyl)acetamide **3** (2.28 mg), yield: 6.36%.

MS m/z (ESI): 716.8 [M+1]
¹H NMR (400 MHz, DMSO-*d₆*) δ 10.35 (s, 1H), 10.23 (s, 1H), 8.58 (s, 1H), 8.03 - 7.94 (m, 2H), 7.72 (dd, *J* = 8.9, 2.1 Hz, 1H), 5.20 (s, 2H), 4.51 (d, *J* = 12.6 Hz, 1H), 3.53 (d, *J* = 5.9 Hz, 6H), 3.23 (t, *J* = 12.5 Hz, 1H), 3.00 - 2.88 (m, 3H), 2.77 (d, *J* = 11.3 Hz, 1H), 2.64 - 2.55 (m, 2H), 2.44 (s, 3H), 1.67 (q, *J* = 5.3 Hz, 4H), 1.47 (p, *J* = 2.6 Hz, 4H), 1.16 (t, *J* = 7.4 Hz, 3H).

### Example 4

### N-(2-chloro-4-(trifluoromethyl)phenyl)-2-(2-cycloheptyl-5-ethyl-6-(4-(5-hydroxy-6-methylpyrim idine-4-carbonyl)piperazin-1-yl)-7-oxo-[1,2,4]triazolo[1,5-a]pyrimidin-4(7H)-yl)acetamide

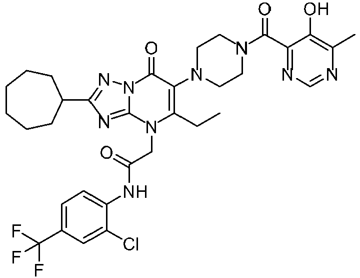

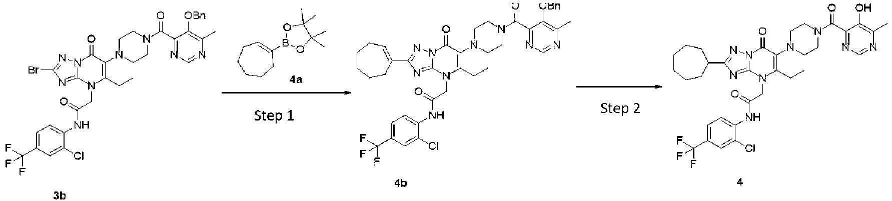

### Step 1

### 2-(6-(4-(5-(benzyloxy)-6-methylpyrimidine-4-carbonyl)piperazin-1-yl)-2-(cyclohept-1-en-1-yl)-5 -ethyl-7-oxo-[1,2,4]triazolo[1,5-a]pyrimidin-4(7H)-yl)-N-(2-chloro-4-(trifluoromethyl)phenyl)ac etamide

At room temperature, 2-(6-(4-(5-(benzyloxy)-6-methylpyrimidine-4-carbonyl)piperazin -1-yl)-2-bromo-5-ethyl-7-oxo-[1,2,4]triazolo[1,5-a]pyrimidin-4(7H)-yl)-N-(2-chloro-4-(trifluoro methyl)phenyl)acetamide **3b** (110 mg, 139.42 µmol), 2-(cyclohept-1-en-1-yl)-4,4,5,5-tetramethyl-1,3,2-dioxaborolane **4a** (37.16 mg, 167.30 µmol), [1,1'-bis(diphenylphosphino)ferrocene]palladium(II) dichloride dichloromethane complex (11.30 mg, 13.94 µmol), sodium carbonate (44.33 mg, 418.25 µmol) and 1,4-dioxane (1 mL) were added to a 100 mL two-necked flask. The air was replaced with argon four times, the temperature was raised to 80 °C, and the reaction was performed for 1.5 h. Water (20 mL) was added to the reaction mixture, the mixture was extracted with ethyl acetate (40 mL ×3), the organic phases were combined, washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-2-(cyclohept-1-en-1-yl)-5 -ethyl-7-oxo-[1,2,4]triazolo[1,5-a]pyrimidin-4(7H)-yl)-N-(2-chloro-4-(trifluoromethyl)phenyl)ac etamide **4b** (100 mg), yield: 89.18%.

MS m/z (ESI): 804.2 [M+1]

### Step 2

### N-(2-chloro-4-(trifluoromethyl)phenyl)-2-(2-cycloheptyl-5-ethyl-6-(4-(5-hydroxy-6-methylpyrim idine-4-carbonyl)piperazin-1-yl)-7-oxo-[1,2,4]triazolo[1,5-a]pyrimidin-4(7H)-yl)acetamide

2-(6-(4-(5-(benzyloxy)-6-methylpyrimidine-4-carbonyl)piperazin-1-yl)-2-(cyclohept-1-en-1-yl)-5-ethyl-7-oxo-[1,2,4]triazolo[1,5-a]pyrimidin-4(7H)-yl)-N-(2-chloro-4-(trifluoromethyl) phenyl)acetamide **4b** (50 mg, 62.17 µmol) was added to methanol (1 mL), 10% palladium on carbon (7.55 mg) was added at room temperature, and the reaction was performed at room temperature overnight. The reaction mixture was filtered and concentrated under reduced pressure. The resulting residue was purified by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(2-cycloheptyl-5-ethyl-6-(4-(5-hydroxy-6-methylpyrim idine-4-carbonyl)piperazin-1-yl)-7-oxo-[1,2,4]triazolo[1,5-a]pyrimidin-4(7H)-yl)acetamide 4 (1.25 mg), yield: 2.76%.

MS m/z (ESI): 715.8 [M+1]
¹H NMR (400 MHz, MeOD-*d₄*) δ 8.57 (s, 1H), 8.14 (d, *J* = 8.6 Hz, 1H), 7.81 (d, *J* = 2.0 Hz, 1H), 7.61 (dd, *J* = 8.9, 2.1 Hz, 1H), 5.35 (s, 2H), 4.70 (d, *J* = 12.8 Hz, 1H), 4.09 (d, *J* = 13.0 Hz, 1H), 3.72 (d, *J* = 12.6 Hz, 2H), 3.43 (t, *J* = 12.3 Hz, 2H), 3.06 (dd, *J* = 18.3, 10.8 Hz, 3H), 3.02 - 2.85 (m, 2H), 2.77 (d, *J* = 11.7 Hz, 1H), 2.52 (s, 3H), 2.13 - 1.98 (m, 2H), 1.92 - 1.74 (m, 4H), 1.70 - 1.50 (m, 6H), 1.30 (t, *J* = 7.5 Hz, 3H).

### Example 5

### N-(2-chloro-4-(trifluoromethyl)phenyl)-2-(5-ethyl-6-(4-(5-hydroxy-6-methylpyrimidine-4-carbon yl)piperazin-1-yl)-2-isopropyl-7-oxo-[1,2,4]triazolo[1,5-a]pyrimidin-4(7H)-yl)acetamide

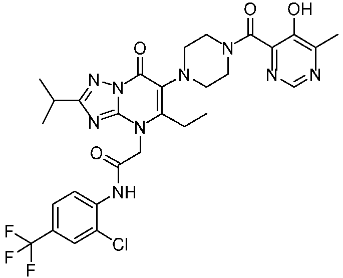

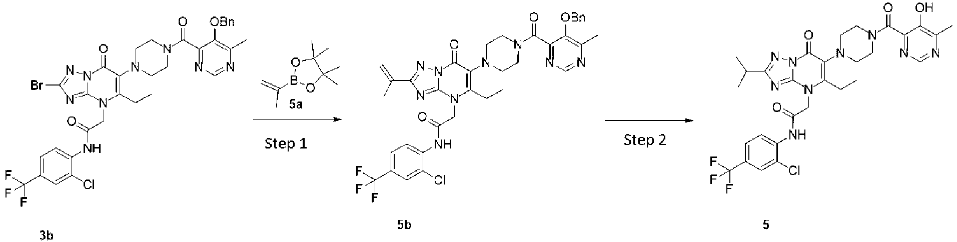

### Step 1

### 2-(6-(4-(5-(benzyloxy)-6-methylpyrimidine-4-carbonyl)piperazin-1-yl)-5-ethyl-7-oxo-2-(prop-1-en-2-yl)-[1,2,4]triazolo[1,5-a]pyrimidin-4(7H)-yl)-N-(2-chloro-4-(trifluoromethyl)phenyl)acetam ide

At room temperature, 2-(6-(4-(5-(benzyloxy)-6-methylpyrimidine-4-carbonyl)piperazin -1-yl)-2-bromo-5-ethyl-7-oxo-[1,2,4]triazolo[1,5-a]pyrimidin-4(7H)-yl)-N-(2-chloro-4-(trifluoro methyl)phenyl)acetamide **3b** (80 mg, 101.39 µmol), 4,4,5,5-tetramethyl-2-(prop-1-en-2-yl)-1,3,2-dioxaborolane **5a** (20.45 mg, 121.67 µmol), [1,1'-bis(diphenylphosphino)ferrocene]palladium(II) dichloride dichloromethane complex (8.22 mg, 10.14 µmol), sodium carbonate (32.24 mg, 304.18 µmol), and 1,4-dioxane (1 mL) were added to a 100 mL two-necked flask, followed by argon exchanging four times. The mixture was heated to 80 °C, and the reaction was performed for 1.5 hours. Water (20 mL) was added to the reaction mixture, which was extracted with ethyl acetate (40 mL × 3). The organic phases were combined, washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-5-ethyl-7-oxo-2-(prop-1-en-2-yl)-[1,2,4]triazolo[1,5-a]pyrimidin-4(7H)-yl)-N-(2-chloro-4-(trifluoromethyl)phenyl)acetam ide **5b** (50 mg) in a yield of 65.74%.

MS m/z (ESI): 750.6 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-2-isopropyl-7-oxo-[1,2,4]triazolo[1,5-a]pyrimidin-4(7H)-yl)acetamide

2-(6-(4-(5-(benzyloxy)-6-methylpyrimidine-4-carbonyl)piperazin-1-yl)-5-ethyl-7-oxo-2 -(prop-1-en-2-yl)-[1,2,4]triazolo[1,5-a]pyrimidin-4(7H)-yl)-N-(2-chloro-4-(trifluoromethyl)phen yl)acetamide **5b** (50 mg, 66.65 µmol) was added to methanol (1 mL), followed by addition of 10% palladium on carbon (8.09 mg). The reaction was performed at room temperature overnight. The reaction mixture was filtered and concentrated under reduced pressure. The resulting residue was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbonyl)piperazin-1-yl)-2-isopropyl-7-oxo-[1,2,4]triazolo[1,5-a]pyrimidin-4(7H)-yl)acetamide **5** (2.6 mg) in a yield of 5.70%.

MS m/z (ESI): 661.8 [M+1]
¹H NMR (400 MHz, DMSO-*d₆*) δ 10.35 (s, 1H), 10.23 (s, 1H), 8.58 (s, 1H), 8.03 (d, *J* = 8.6 Hz, 1H), 7.97 (d, *J* = 2.1 Hz, 1H), 7.72 (dd, *J* = 8.7, 2.2 Hz, 1H), 5.30 (s, 2H), 4.52 (d, *J* = 12.5 Hz, 1H), 3.48 (t, *J* = 10.9 Hz, 3H), 3.24 (t, *J* = 12.2 Hz, 1H), 3.05 - 2.89 (m, 4H), 2.80 (d, *J* = 11.4 Hz, 1H), 2.62 (d, *J* = 10.6 Hz, 1H), 2.44 (s, 3H), 1.26 (d, *J* = 6.9 Hz, 6H), 1.17 (d, *J* = 7.6 Hz, 3H).

### Example 6

### N-(2-chloro-4-(trifluoromethyl)phenyl)-2-(2-(cyclohept-1-en-1-yl)-5-ethyl-6-(4-(5-hydroxy-6-me thylpyrimidine-4-carbonyl)piperazin-1-yl)-7-oxo-[1,2,4]triazolo[1,5-a]pyrimidin-4(7H)-yl)aceta mide

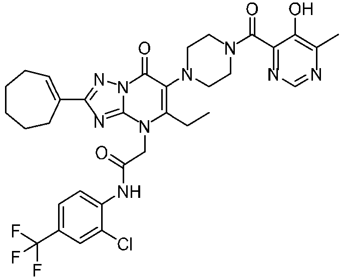

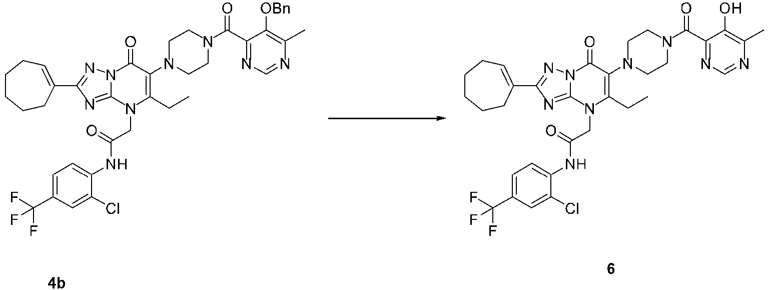

To 2-(6-(4-(5-(benzyloxy)-6-methylpyrimidine-4-carbonyl)piperazin-1-yl)-2-(cyclohept -1-en-1-yl)-5-ethyl-7-oxo-[1,2,4]triazolo[1,5-a]pyrimidin-4(7H)-yl)-N-(2-chloro-4-(trifluorometh yl)phenyl)acetamide **4b** (50 mg, 62.17 µmol) in (2.5 mL), boron trichloride (1 mL) was added at 0 °C, and the reaction was performed at 0 C for 1 hour. Methanol (1 mL) was added to quench the reaction, followed by concentration under reduced pressure. The resulting residue was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(2-(cyclohept-1-en-1-yl)-5-ethyl-6-(4-(5-hydroxy-6-me thylpyrimidine-4-carbonyl)piperazin-1-yl)-7-oxo-[1,2,4]triazolo[1,5-a]pyrimidin-4(7H)-yl)aceta mide **6** (3.07 mg) in a yield of 6.90%.

MS m/z (ESI): 713.8 [M+1]
¹H NMR (400 MHz, DMSO-*d₆*) δ 10.36 (s, 1H), 10.24 (s, 1H), 8.58 (s, 1H), 8.06 (d, *J* = 8.6 Hz, 1H), 7.97 (d, *J* = 2.1 Hz, 1H), 7.72 (dd, *J* = 8.7, 2.2 Hz, 1H), 7.10 (t, *J* = 6.7 Hz, 1H), 5.30 (s, 2H), 4.52 (d, *J* = 12.5 Hz, 1H), 3.56 - 3.44 (m, 3H), 3.24 (t, *J* = 12.6 Hz, 1H), 3.05 - 2.94 (m, 3H), 2.86 - 2.71 (m, 3H), 2.63 (d, *J* = 11.2 Hz, 1H), 2.44 (s, 3H), 2.36 - 2.25 (m, 2H), 1.79 (p, *J=* 5.9 Hz, 2H), 1.52 (q, *J* = 5.6 Hz, 4H), 1.18 (t, *J* = 7.4 Hz, 3H).

### Example 7

### 2-(2-(azetidin-1-yl)-5-ethyl-6-(4-(5-hydroxy-6-methylpyrimidine-4-carbonyl)piperazin-1-yl)-7-o xo-[1,2,4]triazolo[1,5-a]pyrimidin-4(7H)-yl)-N-(2-chloro-4-(trifluoromethyl)phenyl)acetamide

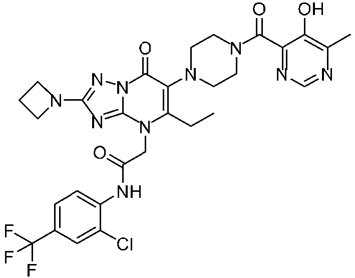

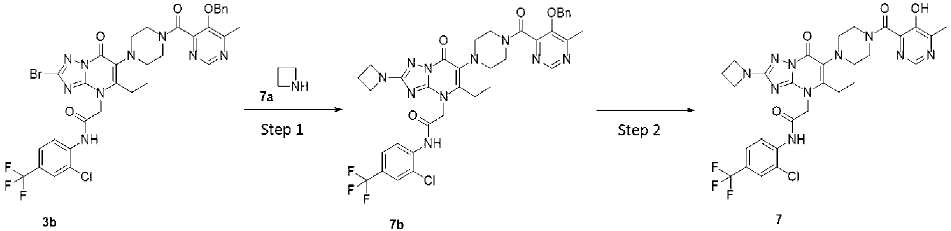

### Step 1

### 2-(2-(azetidin-1-yl)-6-(4-(5-(benzyloxy)-6-methylpyrimidine-4-carbonyl)piperazin-1-yl)-5-ethyl-7-oxo-[1,2,4]triazolo[1,5-a]pyrimidin-4(7H)-yl)-N-(2-chloro-4-(trifluoromethyl)phenyl)acetamid e

2-(6-(4-(5-(benzyloxy)-6-methylpyrimidine-4-carbonyl)piperazin-1-yl)-2-bromo-5-ethy 1-7-oxo-[1,2,4]triazolo[1,5-a]pyrimidin-4(7H)-yl)-N-(2-chloro-4-(trifluoromethyl)phenyl)acetami de **3b** (50 mg, 63.37 µmol) and aziridine **7a** (36.18 mg, 633.71 µmol) were added to a microwave tube, and the mixture was heated to 80 C and reacted for 1.5 h. The reaction mixture was extracted with ethyl acetate (30 mL×2), the organic phases were combined, washed with saturated sodium chloride solution (30 mL ×2), dried over anhydrous sodium sulfate, filtered, and concentrated under reduced pressure. The resulting residue was purified by silica gel column chromatography (eluent: System A) to give 2-(2-(azetidin-1-yl)-6-(4-(5-(benzyloxy)-6-methylpyrimidine-4-carbonyl)piperazin-1-yl)-5-ethyl-7-oxo-[1,2,4]triazolo[1,5-a]pyrimidin-4(7H)-yl)-N-(2-chloro-4-(trifluoromethyl)phenyl)acetamid e **7b** (30 mg), yield: 61.87%.

MS m/z (ESI): 765.3 [M+1]

### Step 2

### 2-(2-(azetidin-1-yl)-5-ethyl-6-(4-(5-hydroxy-6-methylpyrimidine-4-carbonyl)piperazin-1-yl)-7-o xo-[1,2,4]triazolo[1,5-a]pyrimidin-4(7H)-yl)-N-(2-chloro-4-(trifluoromethyl)phenyl)acetamide

2-(2-(azetidin-1-yl)-6-(4-(5-(benzyloxy)-6-methylpyrimidine-4-carbonyl)piperazin-1-yl )-5-ethyl-7-oxo-[1,2,4]triazolo[1,5-a]pyrimidin-4(7H)-yl)-N-(2-chloro-4-(trifluoromethyl)phenyl) acetamide **7b** (30 mg, 39.21 µmol) was added to methanol (1 mL), 10% palladium on carbon (5.71 mg) was added, and the mixture was heated to room temperature and reacted overnight. The mixture was filtered and concentrated under reduced pressure. The resulting residue was purified by preparative liquid phase separation (separation column AKZONOBEL Kromasil; 250×21.2 mm I.D.; 5 µm, 20 mL/min; mobile phase A: 0.05% TFA+H₂O, mobile phase B: CH₃CN) to give 2-(2-(azetidin-1-yl)-5-ethyl-6-(4-(5-hydroxy-6-methylpyrimidine-4-carbonyl)piperazin-1-yl)-7-o xo-[1,2,4]triazolo[1,5-a]pyrimidin-4(7H)-yl)-N-(2-chloro-4-(trifluoromethyl)phenyl)acetamide **7** (2.06 mg), yield: 7.63%.

MS m/z (ESI): 675.1 [M+1]
¹H NMR (400 MHz, MeOD-*d₄*) δ 8.56 (s, 1H), 8.16 (d, *J* = 8.6 Hz, 1H), 7.81 (d, *J* = 2.1 Hz, 1H), 7.62 (dd, *J* = 8.8, 2.1 Hz, 1H), 5.27 (s, 2H), 4.68 (d, *J* = 12.8 Hz, 1H), 4.08 (t, *J* = 7.5 Hz, 5H), 3.72 (s, 2H), 3.41 (t, *J* = 12.2 Hz, 1H), 3.14 (d, *J* = 12.4 Hz, 1H), 3.04 (d, *J* = 7.5 Hz, 2H), 2.90 (d, *J* = 11.6 Hz, 1H), 2.75 (d, *J* = 11.7 Hz, 1H), 2.52 (s, 3H), 2.40 (p, *J* = 7.5 Hz, 2H), 1.28 (d, *J* = 7.6 Hz, 3H).

### Example 8

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(7-oxa-2-azaspiro[3.5]nonan-2-yl)-[1,2,4]triazolo[1,5-a]pyrimidin-4(7 H)-yl)acetamide

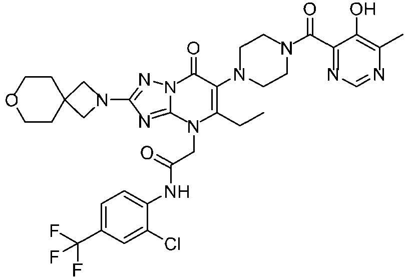

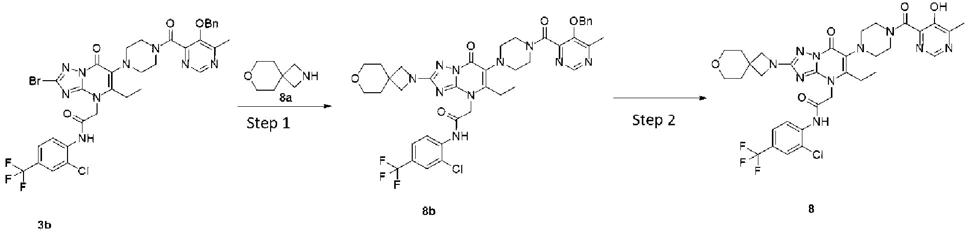

### Step 1

### 2-(6-(4-(5-(benzyloxy)-6-methylpyrimidine-4-carbonyl)piperazin-1-yl)-5-ethyl-7-oxo-2-(7-oxa-2 -azaspiro[3.5]nonan-2-yl)-[1,2,4]triazolo[1,5-a]pyrimidin-4(7H)-yl)-N-(2-chloro-4-(trifluorometh yl)phenyl)acetamide

2-(6-(4-(5-(benzyloxy)-6-methylpyrimidine-4-carbonyl)piperazin-1-yl)-2-bromo-5-ethy 1-7-oxo-[1,2,4]triazolo[1,5-a]pyrimidin-4(7H)-yl)-N-(2-chloro-4-(trifluoromethyl)phenyl)acetami de **3b** (50 mg, 63.37 µmol) and 7-oxa-2-azaspiro[3.5]nonane **8a** (80.60 mg, 633.71 µmol) were added to a microwave tube, and the mixture was heated to 80 C and reacted for 1.5 h. The reaction mixture was extracted with ethyl acetate (30 mL×2), the organic phases were combined, washed with saturated sodium chloride solution (30 mL×2), dried over anhydrous sodium sulfate, filtered, and concentrated under reduced pressure. The resulting residue was purified by silica gel column chromatography (eluent: System A) to give 2-(6-(4-(5-(benzyloxy)-6-methylpyrimidine -4-carbonyl)piperazin-1-yl)-5-ethyl-7-oxo-2-(7-oxa-2-azaspiro[3.5]nonan-2-yl)-[1,2,4]triazolo[1, 5-a]pyrimidin-4(7H)-yl)-N-(2-chloro-4-(trifluoromethyl)phenyl)acetamide **8b** (40 mg), yield: 75.57%.

MS m/z (ESI): 835.2 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(7-oxa-2-azaspiro[3.5]nonan-2-yl)-[1,2,4]triazolo[1,5-a]pyrimidin-4(7 H)-yl)acetamide

2-(6-(4-(5-(benzyloxy)-6-methylpyrimidine-4-carbonyl)piperazin-1-yl)-5-ethyl-7-oxo-2 -(7-oxa-2-azaspiro[3.5]nonan-2-yl)-[1,2,4]triazolo[1,5-a]pyrimidin-4(7H)-yl)-N-(2-chloro-4-(trifl uoromethyl)phenyl)acetamide **8b** (40 mg, 47.89 µmol) was added to dichloromethane (2.5 mL), boron trichloride (1 mL) was added at 0 °C, and the mixture was reacted at 0 °C for 1 h. Methanol (1 mL) was added to quench the reaction, and the mixture was concentrated under reduced pressure. The resulting residue was purified by preparative liquid phase separation (separation column AKZONOBEL Kromasil; 250×21.2 mm I.D.; 5 µm, 20 mL/min; mobile phase A: 0.05% TFA+H₂O, mobile phase B: CH₃CN) to give N-(2-chloro-4-(trifluoromethyl)phenyl)-2-(5-ethyl-6-(4-(5-hydroxy-6-methylpyrimidine-4-carbonyl)piperazin-1-yl)-7-oxo-2-(7-oxa-2-aza spiro[3.5]nonan-2-yl)-[1,2,4]triazolo[1,5-a]pyrimidin-4(7H)-yl)acetamide **8** (3.73 mg), yield: 9.96%.

MS m/z (ESI): 745.1 [M+1]
¹H NMR (400 MHz, DMSO-*d₆*) δ 10.33 (s, 1H), 10.23 (s, 1H), 8.57 (s, 1H), 8.07 (d, *J* = 8.5 Hz, 1H), 7.97 (d, *J* = 2.1 Hz, 1H), 7.72 (dd, *J* = 8.8, 2.1 Hz, 1H), 5.21 (s, 2H), 4.51 (d, *J* = 12.4 Hz, 1H), 3.54 - 3.45 (m, 7H), 3.23 (d, *J* = 24.7 Hz, 1H), 3.01 - 2.88 (m, 3H), 2.77 (d, *J* = 11.3 Hz, 1H), 2.59 (d, *J* = 10.6 Hz, 1H), 2.44 (s, 3H), 1.71 (t, *J* = 5.1 Hz, 4H), 1.15 (t, *J* = 7.4 Hz, 3H).

### Example 9

### N-(2-chloro-4-(trifluoromethyl)phenyl)-2-(2-(cyclohept-1-en-1-yl)-5-ethyl-6-(4-(3-hydroxyisonic otinoyl)piperazin-1-yl)-7-oxo-[1,2,4]triazolo[1,5-a]pyrimidin-4(7H)-yl)acetamide

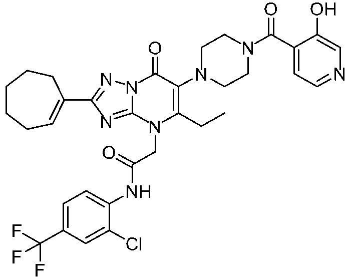

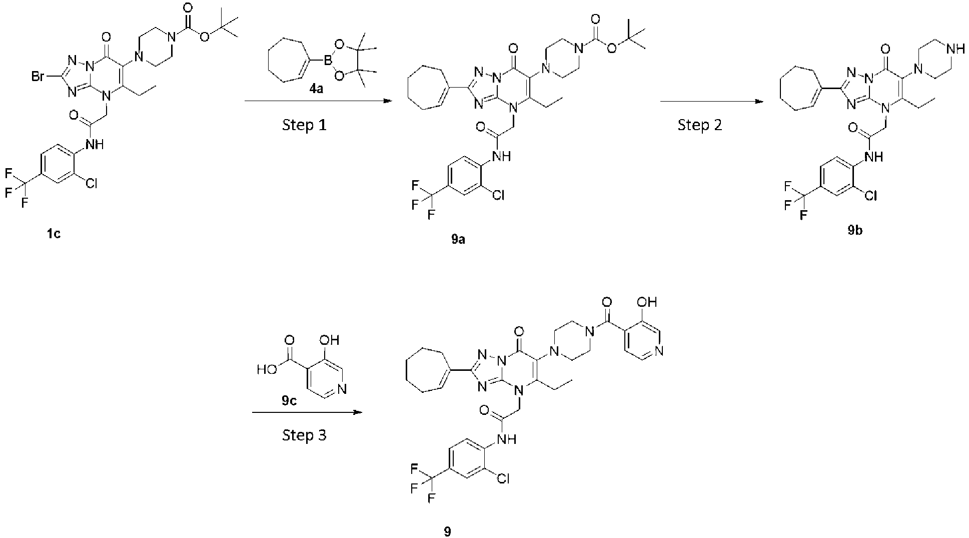

### Step 1

### tert-butyl 4-(4-(2-((2-chloro-4-(trifluoromethyl)phenyl)amino)-2-oxoethyl)-2-(cyclohept-1-en-1-yl)-5-ethyl -7-oxo-4,7-dihydro-[1,2,4]triazolo[1,5-a]pyrimidin-6-yl)piperazine-1-carboxylate

At room temperature, tert-butyl 4-(2-bromo-4-(2-((2-chloro-4-(trifluoromethyl)phenyl)amino)-2-oxoethyl)-5-ethyl-7-oxo-4,7-dih ydro-[1,2,4]triazolo[1,5-a]pyrimidin-6-yl)piperazine-1-carboxylate **1c** (700 mg, 1.06 mmol), 2-(cyclohept-1-en-1-yl)-4,4,5,5-tetramethyl-1,3,2-dioxaborolane **4a** (281.48 mg, 1.27 mmol), [1,1'-bis(diphenylphosphino)ferrocene]palladium(II) dichloride dichloromethane complex (85.60 mg, 105.60 µmol), sodium carbonate (335.77 mg, 3.17 mmol), and 1,4-dioxane (3 mL) were added to a 100 mL two-necked flask. The flask was subject to argon replacement four times, and the reaction was performed at 80 °C for 1.5 hours. Water (20 mL) was added to the reaction mixture, and the mixture was extracted with ethyl acetate (40 mL × 3). The organic phases were combined, washed with saturated sodium chloride solution (20 mL), dried over anhydrous sodium sulfate, filtered, and concentrated under reduced pressure. The residue was purified by silica gel column chromatography (eluent: System B) to obtain tert-butyl 4-(4-(2-((2-chloro-4-(trifluoromethyl)phenyl)amino)-2-oxoethyl)-2-(cyclohept-1-en-1-yl)-5-ethyl -7-oxo-4,7-dihydro-[1,2,4]triazolo[1,5-a]pyrimidin-6-yl)piperazine-1-carboxylate **9a** (650 mg). Yield: 90.77%.

MS m/z (ESI): 622.2 [M+1]

### Step 2

### N-(2-chloro-4-(trifluoromethyl)phenyl)-2-(2-(cyclohept-1-en-1-yl)-5-ethyl-7-oxo-6-(piperazin-1-yl)-[1,2,4]triazolo[1,5-a]pyrimidin-4(7H)-yl)acetamide

tert-butyl 4-(4-(2-((2-chloro-4-(trifluoromethyl)phenyl)amino)-2-oxoethyl)-2-(cyclohept -1-en-1-yl)-5-ethyl-7-oxo-4,7-dihydro-[1,2,4]triazolo[1,5-a]pyrimidin-6-yl)piperazine-1-carboxyl ate **9a** (40 mg, 58.98 µmol) was added to dichloromethane (1 mL), followed by addition of trifluoroacetic acid (0.3 mL). The reaction was performed at room temperature for 1 hour. The mixture was concentrated under reduced pressure to obtain N-(2-chloro-4-(trifluoromethyl) phenyl)-2-(2-(cyclohept-1-en-1-yl)-5-ethyl-7-oxo-6-(piperazin-1-yl)-[1,2,4]triazolo[1,5-a]pyrimi din-4(7H)-yl)acetamide **9b** (34 mg). Yield: 99.72%.

MS m/z (ESI): 578.2 [M+1]

### Step 3

### N-(2-chloro-4-(trifluoromethyl)phenyl)-2-(2-(cyclohept-1-en-1-yl)-5-ethyl-6-(4-(3-hydroxyisonic otinoyl)piperazin-1-yl)-7-oxo-[1,2,4]triazolo[1,5-a]pyrimidin-4(7H)-yl)acetamide

N-(2-chloro-4-(trifluoromethyl)phenyl)-2-(2-(cyclohept-1-en-1-yl)-5-ethyl-7-oxo-6-(pip erazin-1-yl)-[1,2,4]triazolo[1,5-a]pyrimidin-4(7H)-yl)acetamide **9b** (34 mg, 58.82 µmol), 3-hydroxyisonicotinic acid **9c** (10.64 mg, 76.47 µmol), 1-(3-(dimethylaminopropyl)-3-ethylcarbodiimide hydrochloride (16.91 mg, 88.23 µmol), 1-hydroxybenzotriazole (11.92 mg, 88.23 µmol), and N,N-diisopropylethylamine (38.01 mg, 294.10 µmol) were added to N,N-dimethylformamide (1 mL). The reaction was performed at room temperature for 4 hours. The mixture was filtered and concentrated under reduced pressure. The residue was purified by preparative liquid phase separation (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(2-(cyclohept-1-en-1-yl)-5-ethyl-6-(4-(3-hydroxyisonic otinoyl)piperazin-1-yl)-7-oxo-[1,2,4]triazolo[1,5-a]pyrimidin-4(7H)-yl)acetamide **9** (2.93 mg). Yield: 6.91%.

MS m/z (ESI): 699.3 [M+1]
¹H NMR (400 MHz, DMSO-*d₆*) δ 10.89 (s, 1H), 10.35 (s, 1H), 8.29 (s, 1H), 8.21 (dd, *J* = 5.1, 1.7 Hz, 1H), 8.05 (d, *J* = 8.5 Hz, 1H), 7.97 (d, *J* = 2.1 Hz, 1H), 7.72 (dd, *J* = 8.8, 2.2 Hz, 1H), 7.41 (d, *J* = 4.8 Hz, 1H), 7.10 (t, *J* = 6.8 Hz, 1H), 5.30 (s, 2H), 4.51 (s, 1H), 3.46 (q, *J* = 10.5 Hz, 2H), 3.34 - 3.20 (m, 2H), 3.01 - 2.88 (m, 3H), 2.83 - 2.68 (m, 3H), 2.63 (d, *J* = 11.2 Hz, 1H), 2.31 (t, *J* = 6.1 Hz, 2H), 1.79 (s, 2H), 1.57 - 1.44 (m, 4H), 1.18 (t, *J* = 7.4 Hz, 3H).

### Example 10

### N-(2-chloro-4-(trifluoromethyl)phenyl)-2-(2-(cyclohept-1-en-1-yl)-5-ethyl-6-(4-(2-hydroxybenzo yl)piperazin-1-yl)-7-oxo-[1,2,4]triazolo[1,5-a]pyrimidin-4(7H)-yl)acetamide

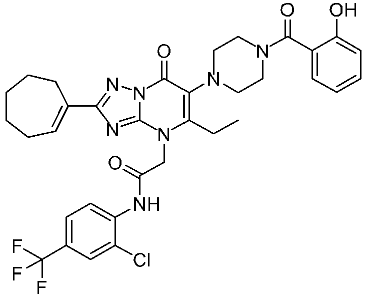

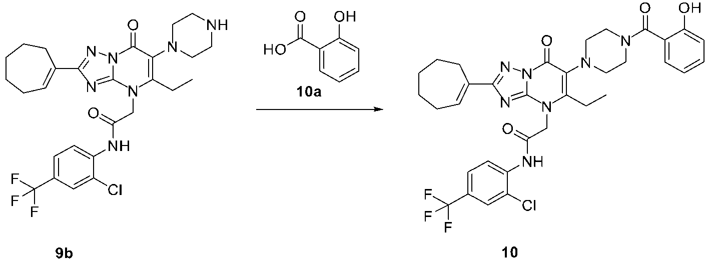

N-(2-Chloro-4-(trifluoromethyl)phenyl)-2-(2-(cyclohept-1-en-1-yl)-5-ethyl-7-oxo-6-(pi perazin-1-yl)-[1,2,4]triazolo[1,5-a]pyrimidin-4(7H)-yl)acetamide **9b** (100 mg, 173.00 µmol), 2-hydroxybenzoic acid **10a** (31.06 mg, 224.90 µmol), 1-(3-dimethylaminopropyl)-3-ethylcarbodiimide hydrochloride (49.75 mg, 259.50 µmol), 1-hydroxybenzotriazole (35.06 mg, 259.50 µmol), and N,N-diisopropylethylamine (111.79 mg, 865.01 µmol) were added to N,N-dimethylformamide (2 mL), and the reaction was performed at room temperature for 4 h. The mixture was filtered and concentrated under reduced pressure, and the resulting residue was purified by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(2-(cyclohept-1-en-1-yl)-5-ethyl-6-(4-(2-hydroxybenzo yl)piperazin-1-yl)-7-oxo-[1,2,4]triazolo[1,5-a]pyrimidin-4(7H)-yl)acetamide **10** (2.6 mg) in a yield of 2.12%.

MS m/z (ESI): 698.3 [M+1]
¹H NMR (400 MHz, DMSO-*d₆*) δ 10.35 (s, 1H), 9.84 (s, 1H), 8.06 (d, *J* = 8.6 Hz, 1H), 7.97 (d, *J* = 2.2 Hz, 1H), 7.72 (dd, *J* = 8.7, 2.2 Hz, 1H), 7.26 - 7.19 (m, 1H), 7.15 (dd, *J* = 7.6, 1.7 Hz, 1H), 7.10 (t, *J* = 6.8 Hz, 1H), 6.90 - 6.81 (m, 2H), 5.30 (s, 2H), 4.53 (s, 1H), 3.46 (d, *J* = 10.2 Hz, 2H), 2.97 (q, *J* = 7.3 Hz, 3H), 2.72 (t, *J* = 14.8 Hz, 4H), 2.31 (q, *J* = 6.1 Hz, 2H), 1.78 (q, *J* = 5.9 Hz, 2H), 1.52 (dq, *J* = 16.2, 7.1 Hz, 4H), 1.18 (t, *J* = 7.4 Hz, 3H).

### Example 11

### N-(2-Chloro-4-(trifluoromethyl)phenyl)-2-(2-(cyclohept-1-en-1-yl)-5-ethyl-6-(4-(2-hydroxy-3-m ethylbenzoyl)piperazin-1-yl)-7-oxo-[1,2,4]triazolo[1,5-a]pyrimidin-4(7H)-yl)acetamide

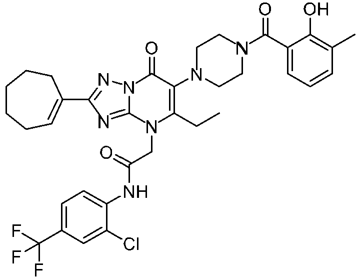

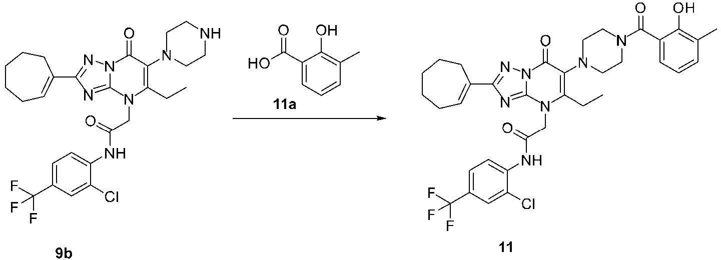

N-(2-Chloro-4-(trifluoromethyl)phenyl)-2-(2-(cyclohept-1-en-1-yl)-5-ethyl-7-oxo-6-(pi perazin-1-yl)-[1,2,4]triazolo[1,5-a]pyrimidin-4(7H)-yl)acetamide **9b** (100 mg, 173.00 µmol), 2-hydroxy-3-methylbenzoic acid **11a** (34.22 mg, 224.90 µmol), 1-(3-dimethylaminopropyl)-3-ethylcarbodiimide hydrochloride (49.75 mg, 259.50 µmol), 1-hydroxybenzotriazole (35.06 mg, 259.50 µmol), and N,N-diisopropylethylamine (111.79 mg, 865.01 µmol) were added to N,N-dimethylformamide (2 mL), and the reaction was performed at room temperature for 4 h. The mixture was filtered and concentrated under reduced pressure, and the resulting residue was purified by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(2-(cyclohept-1-en-1-yl)-5-ethyl-6-(4-(2-hydroxy-3-me thylbenzoyl)piperazin-1-yl)-7-oxo-[1,2,4]triazolo[1,5-a]pyrimidin-4(7H)-yl)acetamide **11** (5.66 mg) in a yield of 4.55%.

MS m/z (ESI): 712.2 [M+1]
¹H NMR (400 MHz, DMSO-*d₆*) δ 10.35 (s, 1H), 9.03 (s, 1H), 8.06 (d, *J* = 8.5 Hz, 1H), 7.97 (d, *J* = 2.1 Hz, 1H), 7.72 (dd, *J* = 8.7, 2.1 Hz, 1H), 7.15 (d, *J* = 7.1 Hz, 1H), 7.09 (t, *J* = 6.6 Hz, 1H), 7.01 (dd, *J* = 7.6, 1.7 Hz, 1H), 6.81 (t, *J* = 7.5 Hz, 1H), 5.30 (s, 2H), 3.45 (t, *J* = 11.4 Hz, 2H), 3.09 (s, 2H), 2.97 (d, *J* = 7.5 Hz, 2H), 2.72 (t, *J* = 12.2 Hz, 4H), 2.33 (d, *J* = 7.4 Hz, 2H), 2.20 (s, 3H), 1.79 (s, 2H), 1.52 (d, *J* = 6.6 Hz, 4H), 1.18 (t, *J* = 7.4 Hz, 3H).

### Example 12

### N-(2-Chloro-4-(trifluoromethyl)phenyl)-2-(2-cyclohexyl-5-ethyl-6-(4-(5-hydroxy-6-methylpyrim idine-4-carbonyl)piperazin-1-yl)-7-oxo-[1,2,4]triazolo[1,5-a]pyrimidin-4(7H)-yl)acetamide

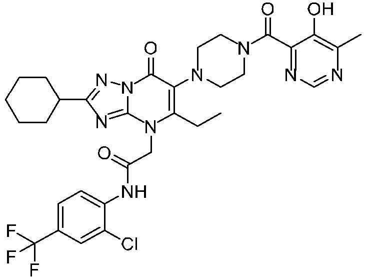

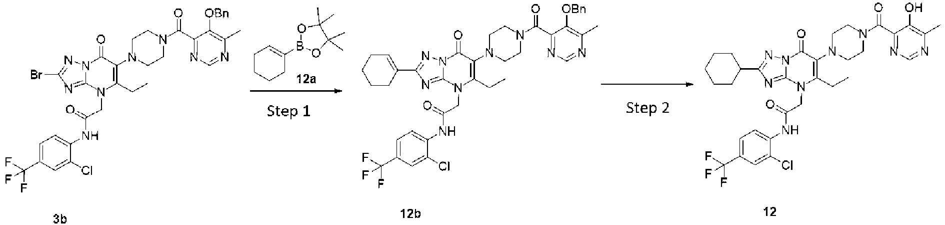

### Step 1

### 2-(6-(4-(5-(Benzyloxy)-6-methylpyrimidine-4-carbonyl)piperazin-1-yl)-2-(cyclohex-1-en-1-yl)-5 -ethyl-7-oxo-[1,2,4]triazolo[1,5-a]pyrimidin-4(7H)-yl)-N-(2-chloro-4-(trifluoromethyl)phenyl)ac etamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (60 mg, 76.05 µmol), 2-(cyclohex-1-en-1-yl)-4,4,5,5-tetramethyl-1,3,2-dioxaborolane **12a** (18.99 mg, 91.25 µmol), [1,1'-bis(diphenylphosphino)ferrocene]palladium(II) dichloride dichloromethane complex (6.16 mg, 7.60 µmol), sodium carbonate (24.18 mg, 228.14 µmol), and 1,4-dioxane (1 mL) were added to a 100 mL two-necked flask, and The mixture was subject to argon replacement four times. The mixture was heated to 80 °C, and the reaction was performed for 1.5 h. Water (20 mL) was added to the reaction mixture, and the mixture was extracted with ethyl acetate (40 mL ×3). The organic phases were combined, washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl) -2-(cyclohept-1-en-1-yl)-5-ethyl-7-oxo-[1,2,4]triazolo[1,5-a]pyrimidin-4(7H)-yl)-N-(2-chloro-4-( trifluoromethyl)phenyl)acetamide **12b** (50 mg) in a yield of 83.20%.

MS m/z (ESI): 790.2 [M+1]

### Step 2

### N-(2-Chloro-4-(trifluoromethyl)phenyl)-2-(2-cyclohexyl-5-ethyl-6-(4-(5-hydroxy-6-methylpyrim idine-4-carbonyl)piperazin-1-yl)-7-oxo-[1,2,4]triazolo[1,5-a]pyrimidin-4(7H)-yl)acetamide

2-(6-(4-(5-(Benzyloxy)-6-methylpyrimidine-4-carbonyl)piperazin-1-yl)-2-(cyclohept-1-en-1-yl)-5-ethyl-7-oxo-[1,2,4]triazolo[1,5-a]pyrimidin-4(7H)-yl)-N-(2-chloro-4-(trifluoromethyl) phenyl)acetamide **12b** (50 mg, 63.27 µmol) was added to methanol (1.5 mL), and 10% palladium on carbon (7.68 mg) was added. The reaction was performed at room temperature overnight. The mixture was filtered and concentrated under reduced pressure. The resulting residue was purified by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(2-cyclohexyl-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)ace tamide **12** (1.84 mg). Yield: 3.99%.

MS m/z (ESI): 702.1 [M+1]
¹H NMR (400 MHz, DMSO-*d₆*) δ 10.35 (d, *J* = 6.5 Hz, 1H), 10.24 (s, 1H), 8.58 (s, 1H), 8.03 (d, *J* = 8.7 Hz, 1H), 7.97 (d, *J* = 2.1 Hz, 1H), 7.72 (dd, *J* = 8.7, 2.2 Hz, 1H), 5.30 (d, *J* = 6.2 Hz, 2H), 4.52 (d, *J* = 12.7 Hz, 1H), 3.49 (d, *J* = 11.6 Hz, 3H), 3.03 - 2.91 (m, 3H), 2.80 (d, *J* = 11.2 Hz, 1H), 2.71 (ddt, *J* = 11.3, 7.3, 3.6 Hz, 1H), 2.65 - 2.58 (m, 1H), 2.44 (s, 3H), 1.99 - 1.88 (m, 2H), 1.81 - 1.60 (m, 4H), 1.51 (qd, *J* = 12.5, 3.2 Hz, 2H), 1.40 - 1.29 (m, 2H), 1.29 - 1.22 (m, 1H), 1.17 (t, *J =* 7.4 Hz, 3H).

### Example 13

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(1-oxo-2,8-diazaspiro[4.5]decan-8-yl)-[1,2,4]triazolo[1,5-a]pyrimidin-4(7H)-yl)acetamide

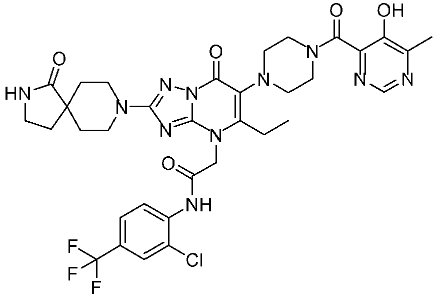

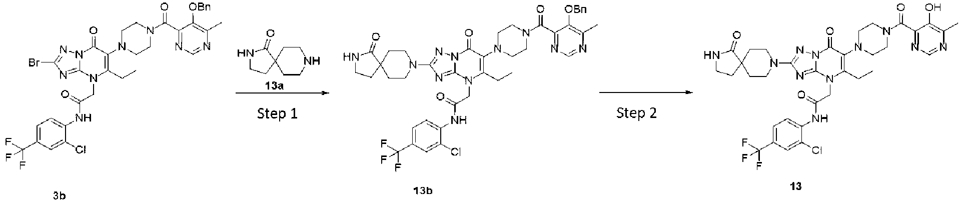

### Step 1

### 2-(6-(4-(5-(Benzyloxy)-6-methylpyrimidine-4-carbonyl)piperazin-1-yl)-5-ethyl-7-oxo-2-(1-oxo-2 ,8-diazaspiro[4.5]decan-8-yl)-[1,2,4]triazolo[1,5-a]pyrimidin-4(7H)-yl)-N-(2-chloro-4-(trifluoro methyl)phenyl)acetamide

2-(6-(4-(5-(Benzyloxy)-6-methylpyrimidine-4-carbonyl)piperazin-1-yl)-2-bromo-5-ethy 1-7-oxo-[1,2,4]triazolo[1,5-a]pyrimidin-4(7H)-yl)-N-(2-chloro-4-(trifluoromethyl)phenyl)acetami de **3b** (50 mg, 63.37 µmol), 2,8-diazaspiro[4.5]decan-1-one **13a** (24.17 mg, 126.74 µmol), cesium fluoride (28.88 mg, 190.11 µmol), and N,N-diisopropylethylamine (24.57 mg, 190.11 µmol) were added to a microwave tube. The reaction was performed at 100°C overnight. The reaction mixture was extracted with ethyl acetate (30 mL ×2). The combined organic phases were washed with saturated sodium chloride solution (30 mL ×2), dried over anhydrous sodium sulfate, filtered, and concentrated under reduced pressure. The resulting residue was purified by silica gel column chromatography (eluent: System A) to obtain 2-(6-(4-(5-(benzyloxy)-6-methylpyrimidine -4-carbonyl)piperazin-1-yl)-5-ethyl-7-oxo-2-(1-oxo-2,8-diazaspiro[4.5]decan-8-yl)-[1,2,4]triazol o[1,5-a]pyrimidin-4(7H)-yl)-N-(2-chloro-4-(trifluoromethyl)phenyl)acetamide **13b** (35 mg). Yield: 64.05%.

MS m/z (ESI): 862.1 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(1-oxo-2,8-diazaspiro[4.5]decan-8-yl)-[1,2,4]triazolo[1,5-a]pyrimidin-4(7H)-yl)acetamide

2-(6-(4-(5-(Benzyloxy)-6-methylpyrimidine-4-carbonyl)piperazin-1-yl)-5-ethyl-7-oxo-2 -(1-oxo-2,8-diazaspiro[4.5]decan-8-yl)-[1,2,4]triazolo[1,5-a]pyrimidin-4(7H)-yl)-N-(2-chloro-4-( trifluoromethyl)phenyl)acetamide **13b** (35 mg, 40.59 µmol) was added to dichloromethane (3 mL). BCl₃ (1 mL) was added at 0 °C, and the reaction was performed at 0 C for 1 h. Methanol was added to quench the reaction. The mixture was concentrated under reduced pressure. The resulting residue was purified by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(1-oxo-2,8-diazaspiro[4.5]decan-8-yl)-[1,2,4]triazolo[1,5-a]pyrimidin-4(7H)-yl)acetamide **13** (3.13 mg). Yield: 9.69%.

MS m/z (ESI): 772.1 [M+1]
¹H NMR (400 MHz, DMSO-*d₆*) δ 10.34 (s, 1H), 10.22 (s, 1H), 8.57 (s, 1H), 8.08 - 7.94 (m, 2H), 7.72 (d, *J* = 9.1 Hz, 1H), 7.58 (s, 1H), 5.21 (s, 2H), 4.51 (d, *J* = 12.2 Hz, 1H), 3.96 (d, *J* = 13.1 Hz, 2H), 3.23 (s, 1H), 3.18 (t, *J* = 6.7 Hz, 2H), 3.07 (t, *J* = 12.3 Hz, 2H), 2.96 (d, *J* = 10.6 Hz, 3H), 2.78 (d, *J* = 11.4 Hz, 1H), 2.60 (d, *J* = 10.3 Hz, 1H), 2.44 (s, 3H), 2.00 (t, *J* = 6.8 Hz, 2H), 1.68 - 1.60 (m, 2H), 1.36 (d, *J* = 13.3 Hz, 2H), 1.16 (t, *J* = 7.4 Hz, 3H).

### Example 14

### N-(2-chloro-4-(trifluoromethyl)phenyl)-2-(5-ethyl-6-(4-(3-hydroxyisonicotinoyl)piperazin-1-yl)-7-oxo-2-(5-oxo-1,3a,4,5,6,6a-hexahydropentalen-2-yl)-[1,2,4]triazolo[1,5-a]pyrimidin-4(7H)-yl)a cetamide

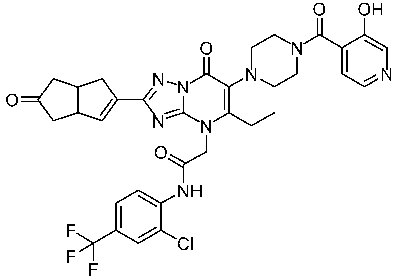

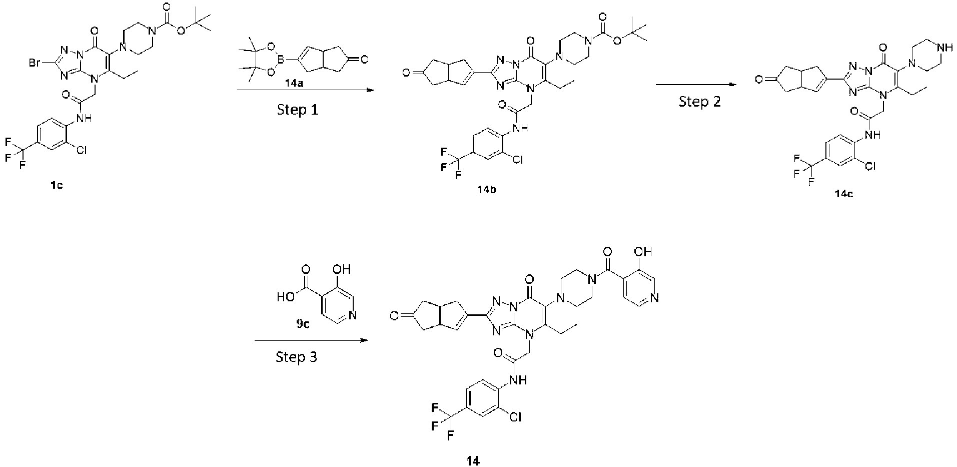

### Step 1

### tert-butyl 4-(4-(2-((2-chloro-4-(trifluoromethyl)phenyl)amino)-2-oxoethyl)-5-ethyl-7-oxo-2-(5-oxo-1,3a,4,5,6,6a-hexahydropentalen-2-yl)-4,7-dihydro-[1,2,4]triazolo[1,5-a]pyrimidin-6-yl)pipe razine-1-carboxylate

At room temperature, tert-butyl 4-(2-bromo-4-(2-((2-chloro-4-(trifluoromethyl)phenyl) amino)-2-oxoethyl)-5-ethyl-7-oxo-4,7-dihydro-[1,2,4]triazolo[1,5-a]pyrimidin-6-yl)piperazine-1-carboxylate **1c** (60 mg, 90.51 µmol), 5-(4,4,5,5-tetramethyl-1,3,2-dioxaborolan-2-yl)-3,3a,4,6a-tetrahydro-1H-pentalene-2-one **14a** (26.95 mg, 108.62 µmol, prepared according to published patent WO2020086533 A1), [1,1'-bis(diphenylphosphino)ferrocene]palladium(II) dichloride dichloromethane complex (7.34 mg, 9.05 µmol), sodium carbonate (28.78 mg, 271.54 µmol), and 1,4-dioxane (1 mL) were added to a 100 mL two-necked flask. The flask was subject to argon replacement four times and then heated to 80 °C. The reaction was performed for 1.5 h. Water (20 mL) was added to the reaction mixture, and the mixture was extracted with ethyl acetate (40 mL ×3). The organic phases were combined, washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(4-(2-((2-chloro-4-(trifluoromethyl)phenyl)amino)-2-oxoethyl) -5-ethyl-7-oxo-2-(5-oxo-1,3a,4,5,6,6a-hexahydropentalen-2-yl)-4,7-dihydro-[1,2,4]triazolo[1,5-a] pyrimidin-6-yl)piperazine-1-carboxylate 14b (45 mg) in a yield of 70.61%.

MS m/z (ESI): 648.0 [M-56+1]

### Step 2

### N-(2-chloro-4-(trifluoromethyl)phenyl)-2-(5-ethyl-7-oxo-2-(5-oxo-1,3a,4,5,6,6a-hexahydropental en-2-yl)-6-(piperazin-1-yl)-[1,2,4]triazolo[1,5-a]pyrimidin-4(7H)-yl)acetamide

tert-butyl 4-(4-(2-((2-chloro-4-(trifluoromethyl)phenyl)amino)-2-oxoethyl)-5-ethyl-7-oxo-2-(5-oxo-1,3a,4,5,6,6a-hexahydropentalen-2-yl)-4,7-dihydro-[1,2,4]triazolo[1,5-a]pyrimidin-6-yl)piperazine-1-carboxylate 14b (45 mg, 63.91 µmol) was added to dichloromethane (1 mL). Trifluoroacetic acid (0.3 mL) was added, and the reaction was performed at room temperature for 1 h. The mixture was concentrated under reduced pressure to obtain N-(2-chloro-4-(trifluoromethyl)phenyl)-2-(5-ethyl-7-oxo-2-(5-oxo-1,3a,4,5,6,6a-hexahydropental en-2-yl)-6-(piperazin-1-yl)-[1,2,4]triazolo[1,5-a]pyrimidin-4(7H)-yl)acetamide **14c** (35 mg) in a yield of 90.67%.

MS m/z (ESI): 604.1 [M+1]

### Step 3

### N-(2-chloro-4-(trifluoromethyl)phenyl)-2-(5-ethyl-6-(4-(3-hydroxyisonicotinoyl)piperazin-1-yl)-7-oxo-2-(5-oxo-1,3a,4,5,6,6a-hexahydropentalen-2-yl)-[1,2,4]triazolo[1,5-a]pyrimidin-4(7H)-yl)a cetamide

N-(2-Chloro-4-(trifluoromethyl)phenyl)-2-(5-ethyl-7-oxo-2-(5-oxo-1,3a,4,5,6,6a-hexah ydropentalen-2-yl)-6-(piperazin-1-yl)-[1,2,4]triazolo[1,5-a]pyrimidin-4(7H)-yl)acetamide **14c** (35 mg, 57.94 µmol), 3-hydroxyisonicotinic acid **9c** (10.48 mg, 75.33 µmol), 1-(3-(dimethylaminopropyl)-3-ethylcarbodiimide hydrochloride (16.66 mg, 86.92 µmol), 1-hydroxybenzotriazole (11.74 mg, 86.92 µmol), and N,N-diisopropylethylamine (37.44 mg, 289.72 µmol) were added to N,N-dimethylformamide (1 mL). The reaction was performed at room temperature for 4 h. The mixture was filtered and concentrated under reduced pressure. The resulting residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(5-ethyl-6-(4-(3-hydroxyisonicotinoyl)piperazin-1-yl)-7-oxo-2-(5-oxo-1,3a,4,5,6,6a-hexahydropentalen-2-yl)-[1,2,4]triazolo[1,5-a]pyrimidin-4(7H)-yl)a cetamide **14** (3.41 mg) in a yield of 7.85%.

MS m/z (ESI): 725.2 [M+1]
¹H NMR (400 MHz, DMSO-*d₆*) δ 10.68 (s, 1H), 10.33 (s, 1H), 8.27 (s, 1H), 8.17 (d, *J=* 5.0 Hz, 1H), 8.06 (d, *J=* 8.5 Hz, 1H), 7.95 (d, *J=* 2.1 Hz, 1H), 7.71 (dd, *J=* 8.8, 2.2 Hz, 1H), 7.33 (s, 1H), 6.52 (s, 1H), 5.31 (s, 2H), 4.52 (d, *J=* 12.6 Hz, 1H), 3.28 (d, *J =* 22.0 Hz, 3H), 3.15 - 2.91 (m, 6H), 2.80 (d, *J =* 11.6 Hz, 1H), 2.64 (d, *J=* 15.6 Hz, 2H), 2.59 - 2.52 (m, 1H), 2.25 (d, *J= 18.9* Hz, 2H), 1.95 (dd, *J=* 18.8, 6.9 Hz, 1H), 1.18 (t, *J=* 7.4 Hz, 3H).

### Example 15

### N-(2-chloro-4-(trifluoromethyl)phenyl)-2-(2-((3-chloropropyl)amino)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)ace tamide

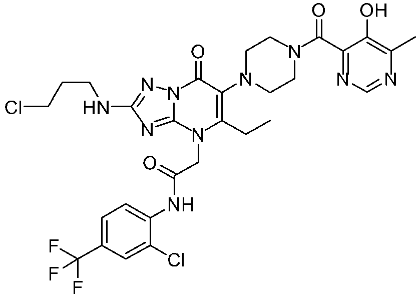

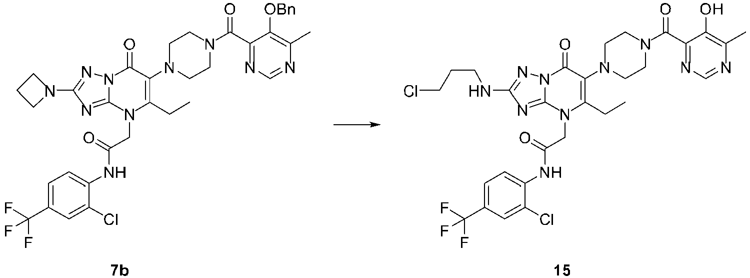

2-(2-(Azetidin-1-yl)-6-(4-(5-(benzyloxy)-6-methylpyrimidine-4-carbonyl)piperazin-1-yl )-5-ethyl-7-oxo-[1,2,4]triazolo[1,5-a]pyrimidin-4(7H)-yl)-N-(2-chloro-4-(trifluoromethyl)phenyl) acetamide **7b** (40 mg, 52.28 µmol) was added to dichloromethane (2.5 mL). Boron trichloride (1 mL) was added at 0 °C, and the reaction was performed at 0 °C for 1 h. Methanol was added to quench the reaction. The mixture was concentrated under reduced pressure at room temperature. The resulting residue was purified by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(2-((3-chloropropyl)amino)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)ace tamide **15** (1.36 mg) in a yield of 3.52%.

MS m/z (ESI): 711.0 [M+1]
¹H NMR (400 MHz, DMSO-*d₆*) δ 10.31 (s, 1H), 10.22 (s, 1H), 8.57 (s, 1H), 8.08 (d,*J* = 8.6 Hz, 1H), 7.96 (d, *J =* 2.1 Hz, 1H), 7.74-7.67 (m, 1H), 6.79 (t, *J =* 5.8 Hz, 1H), 5.17 (s, 2H), 4.51 (d, *J= 12.8* Hz, 1H), 3.68 (t, *J =* 6.5 Hz, 2H), 3.50 (d, *J=* 12.3 Hz, 3H), 3.25 (dd, *J =* 13.3, 6.9 Hz, 2H), 2.95 (d, *J=* 17.6 Hz, 3H), 2.77 (d, *J=* 11.3 Hz, 1H), 2.59 (d, *J=* 11.0 Hz, 2H), 2.44 (s, 3H), 1.99 (q, *J =* 6.6 Hz, 2H), 1.15 (t, *J =* 7.4 Hz, 3H).

### Example 16

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(2-oxa-6-azaspiro[3.3]heptan-6-yl)-[1,2,4]triazolo[1,5-a]pyrimidin-4( 7H)-yl)acetamide

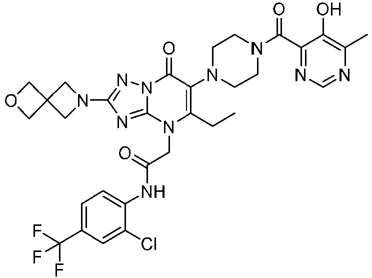

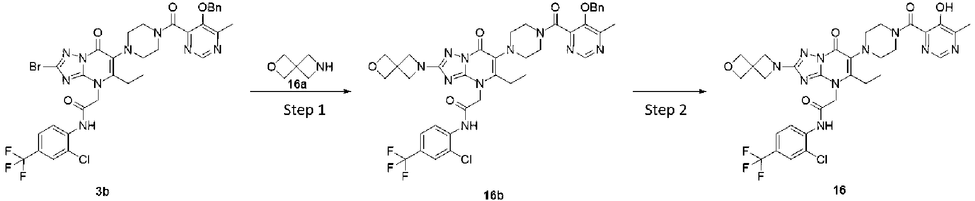

### Step 1

### 2-(6-(4-(5-(benzyloxy)-6-methylpyrimidine-4-carbonyl)piperazin-1-yl)-5-ethyl-7-oxo-2-(2-oxa-6 -azaspiro[3.3]heptan-6-yl)-[1,2,4]triazolo[1,5-a]pyrimidin-4(7H)-yl)-N-(2-chloro-4-(trifluoromet hyl)phenyl)acetamide

2-(6-(4-(5-(benzyloxy)-6-methylpyrimidine-4-carbonyl)piperazin-1-yl)-2-bromo-5-ethy 1-7-oxo-[1,2,4]triazolo[1,5-a]pyrimidin-4(7H)-yl)-N-(2-chloro-4-(trifluoromethyl)phenyl)acetami **de 3b** (50 mg, 63.37 µmol) and 2-oxa-6-azaspiro[3.3]heptane 16a (8.17 mg, 82.38 µmol, commercially available) were added to a microwave tube. The reaction was performed at 80 °C for 2 hours. The reaction mixture was extracted with ethyl acetate (30 mL × 2), the aqueous layer was separated, and the combined organic phase was washed with saturated sodium chloride solution (30 mL × 2), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl) -5-ethyl-7-oxo-2-(2-oxa-6-azaspiro[3.3]heptan-6-yl)-[1,2,4]triazolo[1,5-a]pyrimidin-4(7H)-yl)-N -(2-chloro-4-(trifluoromethyl)phenyl)acetamide 16b (40 mg) in a yield of 78.19%.

MS m/z (ESI): 807.1 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(2-oxa-6-azaspiro[3.3]heptan-6-yl)-[1,2,4]triazolo[1,5-a]pyrimidin-4( 7H)-yl)acetamide

2-(6-(4-(5-(benzyloxy)-6-methylpyrimidine-4-carbonyl)piperazin-1-yl)-5-ethyl-7-oxo-2 -(2-oxa-6-azaspiro[3.3]heptan-6-yl)-[1,2,4]triazolo[1,5-a]pyrimidin-4(7H)-yl)-N-(2-chloro-4-(trif luoromethyl)phenyl)acetamide 16b (30 mg, 37.16 µmol) was added to methanol (1.5 mL), and palladium on carbon (4.51 mg, 37.16 µmol) was added. The reaction was performed at room temperature for 16 hours. The mixture was filtered and concentrated under reduced pressure. The resulting residue was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(2-oxa-6-azaspiro[3.3]heptan-6-yl)-[1,2,4]triazolo[1,5-a]pyrimidin-4( 7H)-yl)acetamide 16 (8.23 mg) in a yield of 10.53%.

MS m/z (ESI): 717.1 [M+1]
¹H NMR (400 MHz, DMSO-*d₆*) δ 10.32 (d, J= 2.4 Hz, 1H), 10.23 (s, 1H), 8.57 (s, 1H), 8.06 (d, *J* = 8.5 Hz, 1H), 7.97 (t, *J =* 2.6 Hz, 1H), 7.72 (d, *J =* 8.8 Hz, 1H), 5.21 (d, *J* = 2.7 Hz, 2H), 4.69 (s, 2H), 4.51 (d, *J =* 12.7 Hz, 1H), 4.13 (s, 2H), 3.70 (s, 2H), 3.22 (t, *J =* 12.8 Hz, 2H), 2.98 (d, *J=* 12.0 Hz, 4H), 2.77 (d, *J =* 11.4 Hz, 1H), 2.59 (d, *J=* 10.6 Hz, 1H), 2.44 (s, 3H), 1.15 (td, *J= 7.5,* 2.4 Hz, 3H).

### Example 17

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(5-oxo-1,3a,4,5,6,6a-hexahydropentalen-2-yl)-[1,2,4]triazolo[1,5-a]py rimidin-4(7H)-yl)acetamide

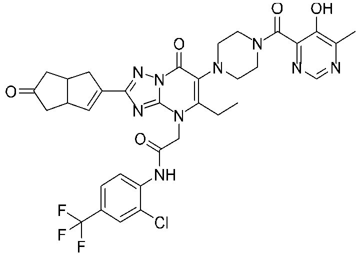

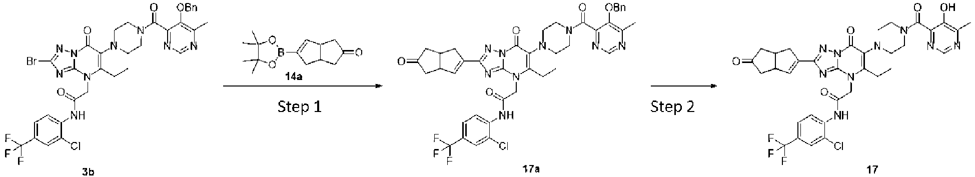

### Step 1

### 2-(6-(4-(5-(benzyloxy)-6-methylpyrimidine-4-carbonyl)piperazin-1-yl)-5-ethyl-7-oxo-2-(5-oxo-1 ,3a,4,5,6,6a-hexahydropentalen-2-yl)-[1,2,4]triazolo[1,5-a]pyrimidin-4(7H)-yl)-N-(2-chloro-4-(tr ifluoromethyl)phenyl)acetamide

At room temperature, 2-(6-(4-(5-(benzyloxy)-6-methylpyrimidine-4-carbonyl) piperazin-1-yl)-2-bromo-5-ethyl-7-oxo-[1,2,4]triazolo[1,5-a]pyrimidin-4(7H)-yl)-N-(2-chloro-4-( trifluoromethyl)phenyl)acetamide 3b (60 mg, 76.05 µmol), 5-(4,4,5,5-tetramethyl-1,3,2-dioxaborolan-2-yl)-3,3a,4,6a-tetrahydro-1H-cyclopenta[c]furan-2-on e 14a (22.64 mg, 91.25 µmol, commercially available), [1,1'-bis(diphenylphosphino)ferrocene] palladium(II) dichloride dichloromethane complex (6.16 mg, 7.60 µmol), and sodium carbonate (24.18 mg, 228.14 µmol) were added to 1,4-dioxane (1 mL). The mixture was subject to argon replacement four times, then heated to 80 °C and the reaction was performed for 1.5 hours. Water (20 mL) was added to the reaction mixture, and the mixture was extracted with ethyl acetate (40 mL×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-5-ethyl-7-oxo-2-(5-oxo-1 ,3a,4,5,6,6a-hexahydropentalen-2-yl)-[1,2,4]triazolo[1,5-a]pyrimidin-4(7H)-yl)-N-(2-chloro-4-(tr ifluoromethyl)phenyl)acetamide 17a (30 mg). Yield: 47.52%.

MS m/z (ESI): 830.3 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(5-oxo-1,3a,4,5,6,6a-hexahydropentalen-2-yl)-[1,2,4]triazolo[1,5-a]py rimidin-4(7H)-yl)acetamide

2-(6-(4-(5-(benzyloxy)-6-methylpyrimidine-4-carbonyl)piperazin-1-yl)-5-ethyl-7-oxo-2 -(5-oxo-1,3a,4,5,6,6a-hexahydropentalen-2-yl)-[1,2,4]triazolo[1,5-a]pyrimidin-4(7H)-yl)-N-(2-ch loro-4-(trifluoromethyl)phenyl)acetamide 17a (30 mg, 36.13 µmol) was added to dichloromethane (2 mL). Boron trichloride (0.5 mL) was added at 0 °C, and the reaction was performed at 0 °C for 1 hour. Methanol was added to quench the reaction, and the mixture was concentrated under reduced pressure. The resulting residue was purified by preparative liquid chromatography (separation column AKZONOBEL Kromasil; 250×21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(5-oxo-1,3a,4,5,6,6a-hexahydropentalen-2-yl)-[1,2,4]triazolo[1,5-a]py rimidin-4(7H)-yl)acetamide 17 (3.16 mg). Yield: 11.77%.

MS m/z (ESI): 740.1 [M+1]
¹H NMR (400 MHz, DMSO-*d₆*) δ 10.36 (s, 1H), 10.23 (s, 1H), 8.58 (s, 1H), 8.06 (d, *J =* 8.5 Hz, 1H), 7.97 (d, *J =* 2.1 Hz, 1H), 7.71 (dd, *J =* 8.7, 2.1 Hz, 1H), 6.52 (q, *J =* 1.9 Hz, 1H), 5.32 (s, 2H), 4.52 (d, *J =* 12.5 Hz, 1H), 3.25 (t, *J =* 12.4 Hz, 1H), 3.10 (dd, *J =* 16.6, 8.6 Hz, 2H), 3.01 (d, *J =* 14.1 Hz, 4H), 2.81 (d, *J =* 11.3 Hz, 1H), 2.64 (d, *J =* 14.9 Hz, 2H), 2.59 - 2.53 (m, 1H), 2.44 (s, 3H), 2.29 -2.22 (m, 1H), 1.94 (dd, J= 18.8, 6.9 Hz, 1H), 1.18 (t, *J=* 7.4 Hz, 3H).

### Example 18

### 2-(2-(bicyclo[3.1.0]hex-2-en-3-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(2-chloro-4-(trifluoromethyl)pheny l)acetamide

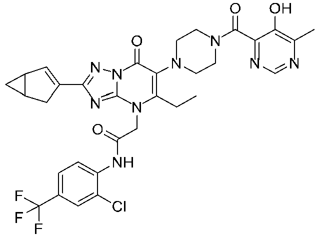

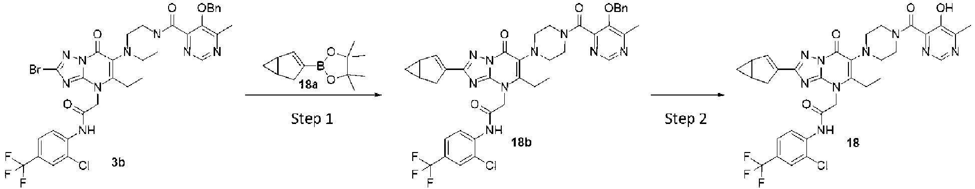

### Step 1

### 2-(6-(4-(5-(benzyloxy)-6-methylpyrimidine-4-carbonyl)piperazin-1-yl)-2-(bicyclo[3.1.0]hex-2-en -3-yl)-5-ethyl-7-oxo-[1,2,4]triazolo[1,5-a]pyrimidin-4(7H)-yl)-N-(2-chloro-4-(trifluoromethyl)ph enyl)acetamide

At room temperature, 2-(6-(4-(5-(benzyloxy)-6-methylpyrimidine-4-carbonyl)piperazin -1-yl)-2-bromo-5-ethyl-7-oxo-[1,2,4]triazolo[1,5-a]pyrimidin-4(7H)-yl)-N-(2-chloro-4-(trifluoro methyl)phenyl)acetamide 3b (50 mg, 63.37 µmol), 2-(bicyclo[3.1.0]hex-2-en-3-yl)-4,4,5,5-tetramethyl-1,3,2-dioxaborolane 18a (15.67 mg, 76.05 µmol, commercially available), [1,1'-bis(diphenylphosphino)ferrocene]palladium(II) dichloride dichloromethane complex (5.14 mg, 6.34 µmol), and sodium carbonate (20.15 mg, 190.11 µmol) were added to 1,4-dioxane (1 mL). The mixture was subject to argon replacement four times, then heated to 80 °C and the reaction was performed for 1.5 hours. Water (10 mL) was slowly added dropwise to the reaction mixture, and the mixture was extracted with ethyl acetate (40 mL ×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-2-(bicyclo[3.1.0]hex-2-en -3-yl)-5-ethyl-7-oxo-[1,2,4]triazolo[1,5-a]pyrimidin-4(7H)-yl)-N-(2-chloro-4-(trifluoromethyl)ph enyl)acetamide 18b (40 mg). Yield: 80.08%.

MS m/z (ESI): 788.1 [M+1]

### Step 2

### 2-(2-(bicyclo[3.1.0]hex-2-en-3-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(2-chloro-4-(trifluoromethyl)pheny l)acetamide

2-(6-(4-(5-(benzyloxy)-6-methylpyrimidine-4-carbonyl)piperazin-1-yl)-2-(bicyclo[3.1.0 ]hex-2-en-3-yl)-5-ethyl-7-oxo-[1,2,4]triazolo[1,5-a]pyrimidin-4(7H)-yl)-N-(2-chloro-4-(trifluoro methyl)phenyl)acetamide 18b (40 mg, 50.75 µmol) was added to dichloromethane (2 mL). Boron trichloride (0.5 mL) was added at 0 °C, and the reaction was performed at 0 °C for 1 hour. Methanol was added to quench the reaction, and the mixture was concentrated under reduced pressure. The resulting residue was purified by preparative liquid chromatography (separation column AKZONOBEL Kromasil; 250 ×21.2 mm I.D.; 5 µm, 20 mL/min; mobile phase A: 0.05% TFA + H₂O, mobile phase B: CH₃CN) to obtain 2-(2-(bicyclo[3.1.0]hex-2-en-3-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(2-chloro-4-(trifluoromethyl)pheny l)acetamide 18 (11.52 mg). Yield: 8.54%.

MS m/z (ESI): 698.2 [M+1]
¹H NMR (400 MHz, DMSO-*d₆*) δ 10.36 (s, 1H), 10.23 (s, 1H), 8.58 (s, 1H), 8.05 (d, *J =* 8.6 Hz, 1H), 7.97 (d, *J =* 2.1 Hz, 1H), 7.72 (dd, *J =* 8.7, 2.1 Hz, 1H), 6.81 (q, *J =* 2.0 Hz, 1H), 5.30 (s, 2H), 4.52 (d, *J=* 12.4 Hz, 1H), 3.49 (d, *J =* 12.2 Hz, 3H), 3.26 (d, *J =* 12.1 Hz, 1H), 3.04 - 2.89 (m, 5H), 2.83 - 2.56 (m, 4H), 2.44 (s, 3H), 2.03 - 1.91 (m, 1H), 1.83 - 1.72 (m, 1H), 1.17 (t, *J =* 7.4 Hz, 3H), 1.01 (td, *J =* 7.8, 3.8 Hz, 1H).

### Example 19

### 2-(2-(benzofuran-2-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamid e

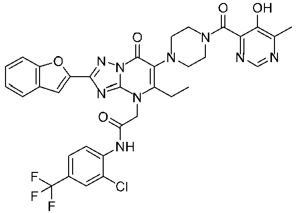

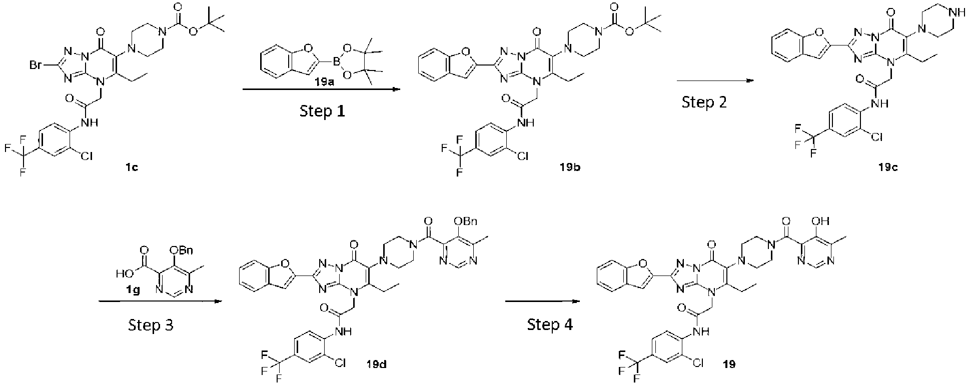

### Step 1

### tert-butyl 4-(2-(benzofuran-2-yl)-4-(2-((2-chloro-4-(trifluoromethyl)phenyl)amino)-2-oxoethyl) -5-ethyl-7-oxo-4,7-dihydro-[1,2,4]triazolo[1,5-a]pyrimidin-6-yl)piperazine-1-carboxylate

At room temperature, tert-butyl 4-(2-bromo-4-(2-((2-chloro-4-(trifluoromethyl)phenyl) amino)-2-oxoethyl)-5-ethyl-7-oxo-4,7-dihydro-[1,2,4]triazolo[1,5-a]pyrimidin-6-yl)piperazine-1-carboxylate 1c (60 mg, 90.51 µmol), 2-(benzofuran-2-yl)-4,4,5,5-tetramethyl-1,3,2-dioxaborolane 19a (26.51 mg, 108.62 µmol, commercially available), [1,1'-bis(diphenylphosphino)ferrocene]palladium(II) dichloride dichloromethane complex (7.34 mg, 9.05 µmol), and sodium carbonate (28.78 mg, 271.54 µmol) were added to 1,4-dioxane (2 mL). The mixture was subject to argon replacement four times, then heated to 80 °C and the reaction was performed for 1.5 h. Water (10 mL) was slowly added dropwise to the reaction mixture, and the mixture was extracted with ethyl acetate (40 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(2-(benzofuran-2-yl)-4-(2-((2-chloro-4-(trifluoromethyl)phenyl)amino)-2-oxoethyl)-5-ethyl-7-o xo-4,7-dihydro-[1,2,4]triazolo[1,5-a]pyrimidin-6-yl)piperazine-1-carboxylate 19b (40 mg). Yield: 63.12%.

MS m/z (ESI): 644.2 [M-56+1]

### Step 2

### 2-(2-(benzofuran-2-yl)-5-ethyl-7-oxo-6-(piperazin-1-yl)-[1,2,4]triazolo[1,5-a]pyrimidin -4(7H)-yl)-N-(2-chloro-4-(trifluoromethyl)phenyl)acetamide

tert-butyl 4-(2-(benzofuran-2-yl)-4-(2-((2-chloro-4-(trifluoromethyl)phenyl)amino)-2-oxoethyl)-5-ethyl-7-oxo-4,7-dihydro-[1,2,4]triazolo[1,5-a]pyrimidin-6-yl)piperazine-1-carboxyla te 19b (40 mg, 57.13 µmol) was added to dichloromethane (3 mL), followed by addition of trifluoroacetic acid (0.5 mL). The reaction was performed at room temperature for 1 h. The mixture was concentrated under reduced pressure to obtain 2-(2-(benzofuran-2-yl)-5-ethyl-7-oxo-6-(piperazin-1-yl)-[1,2,4]triazolo[1,5-a]pyrimidin-4(7H)-yl )-N-(2-chloro-4-(trifluoromethyl)phenyl)acetamide 19c (30 mg). Yield: 87.51%.

MS m/z (ESI): 600.1 [M+1]

### Step 3

### 2-(2-(benzofuran-2-yl)-6-(4-(5-(benzyloxy)-6-methylpyrimidine-4-carbonyl)piperazin-1-yl)-5-eth yl-7-oxo-[1,2,4]triazolo[1,5-a]pyrimidin-4(7H)-yl)-N-(2-chloro-4-(trifluoromethyl)phenyl)aceta mide

2-(2-(benzofuran-2-yl)-5-ethyl-7-oxo-6-(piperazin-1-yl)-[1,2,4]triazolo[1,5-a]pyrimidin -4(7H)-yl)-N-(2-chloro-4-(trifluoromethyl)phenyl)acetamide 19c (30 mg, 50.00 µmol), 5-(benzyloxy)-6-methylpyrimidine-4-carboxylic acid 1g (15.88 mg, 65.00 µmol), 1-(3-dimethylaminopropyl)-3-ethylcarbodiimide hydrochloride (14.38 mg, 75.00 µmol), 1-hydroxybenzotriazole (10.13 mg, 75.00 µmol), and N,N-diisopropylethylamine (32.31 mg, 250.00 µmol) were added to N,N-dimethylformamide (1 mL). The reaction was performed at room temperature for 4 h. The reaction mixture was extracted with ethyl acetate (30 mL × 2), and the aqueous layer was separated. The combined organic phases were washed with saturated sodium chloride solution (30 mL × 2), dried over anhydrous sodium sulfate, filtered, and concentrated under reduced pressure. The resulting residue was purified by silica gel column chromatography (eluent: System B) to obtain 2-(2-(benzofuran-2-yl)-6-(4-(5-(benzyloxy)-6-methylpyrimidine-4-carbonyl)piperazin-1-yl)-5-ethyl-7-oxo-[1,2,4]triazolo[1,5-a]pyrimidin-4(7 H)-yl)-N-(2-chloro-4-(trifluoromethyl)phenyl)acetamide 19d (30 mg). Yield: 72.62%.

MS m/z (ESI): 826.2 [M+1]

### Step 4

### 2-(2-(benzofuran-2-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamid e

2-(2-(benzofuran-2-yl)-6-(4-(5-(benzyloxy)-6-methylpyrimidine-4-carbonyl)piperazin-1 -yl)-5-ethyl-7-oxo-[1,2,4]triazolo[1,5-a]pyrimidin-4(7H)-yl)-N-(2-chloro-4-(trifluoromethyl)phen yl)acetamide 19d (30 mg, 36.31 µmol) was added to dichloromethane (2.5 mL). Boron trichloride (0.5 mL) was added at 0 °C, and the reaction was performed at 0 C for 1 h. Methanol was added to quench the reaction, and the mixture was concentrated under reduced pressure. The resulting residue was purified by preparative liquid chromatography (column: AKZONOBEL Kromasil; 250 × 21.2 mm I.D.; 5 µm, 20 mL/min; mobile phase A: 0.05% TFA + H₂O, mobile phase B: CH₃CN) to obtain 2-(2-(benzofuran-2-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(triflu oromethyl)phenyl)acetamide 19 (4.3 mg). Yield: 15.61%.

MS m/z (ESI): 736.0 [M+1]
¹H NMR (400 MHz, DMSO-*d₆*) δ 10.42 (s, 1H), 10.25 (s, 1H), 8.59 (s, 1H), 8.10 (d, *J =* 8.7 Hz, 1H), 7.98 (d, *J =* 2.1 Hz, 1H), 7.75 (dd, *J =* 17.2, 7.9 Hz, 3H), 7.62 (s, 1H), 7.44 (t, *J =* 7.7 Hz, 1H), 7.34 (t, *J=* 7.5 Hz, 1H), 5.42 (s, 2H), 4.55 (d, *J=* 12.5 Hz, 1H), 3.51 (d, *J=* 12.2 Hz, 4H), 3.03 (d, *J=* 10.2 Hz, 3H), 2.86 (d, *J=* 11.2 Hz, 1H), 2.68 (d, *J=* 10.7 Hz, 1H), 2.45 (s, 3H), 1.22 (t, *J=* 7.4 Hz, 3H).

### Example 20

### N-(2-chloro-4-(trifluoromethyl)phenyl)-2-(2-(cyclohexylidenemethyl)-5-ethyl-6-(4-(5-hydroxy-6 -methylpyrimidine-4-carbonyl)piperazin-1-yl)-7-oxo-[1,2,4]triazolo[1,5-a]pyrimidin-4(7H)-yl)ac etamide

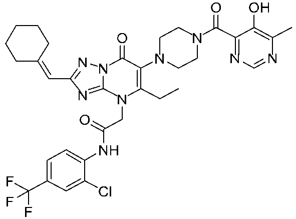

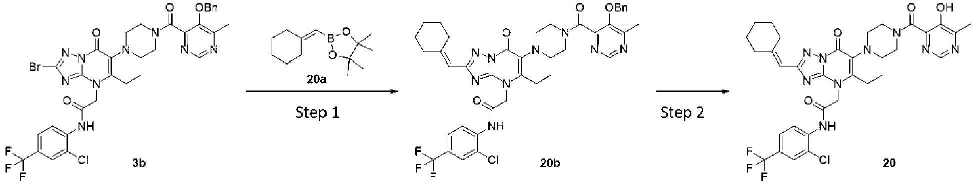

### Step 1

### 2-(6-(4-(5-(benzyloxy)-6-methylpyrimidine-4-carbonyl)piperazin-1-yl)-2-(cyclohexylidenemethy l)-5-ethyl-7-oxo-[1,2,4]triazolo[1,5-a]pyrimidin-4(7H)-yl)-N-(2-chloro-4-(trifluoromethyl)phenyl )acetamide

At room temperature, 2-(6-(4-(5-(benzyloxy)-6-methylpyrimidine-4-carbonyl) piperazin-1-yl)-2-bromo-5-ethyl-7-oxo-[1,2,4]triazolo[1,5-a]pyrimidin-4(7H)-yl)-N-(2-chloro-4-( trifluoromethyl)phenyl)acetamide 3b (50 mg, 63.37 µmol), 2-(cyclohexylidenemethyl)-4,4,5,5-tetramethyl-1,3,2-dioxaborolane 20a (16.89 mg, 76.05 µmol, prepared by a known method "European Journal of Organic Chemistry (2019), 2019(33), 5624-5635"), [1,1'-bis(diphenylphosphino)ferrocene]palladium(II) dichloride dichloromethane complex (5.14 mg, 6.34 µmol), and sodium carbonate (20.15 mg, 190.11 µmol) were added to 1,4-dioxane (1 mL). The mixture was subject to argon replacement four times, then heated to 80 °C, and the reaction was performed for 2 hours. Water (20 mL) was added to the reaction mixture, which was extracted with ethyl acetate (40 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-2-(cyclohexylidenemethy l)-5-ethyl-7-oxo-[1,2,4]triazolo[1,5-a]pyrimidin-4(7H)-yl)-N-(2-chloro-4-(trifluoromethyl)phenyl )acetamide 20b (40 mg) in a yield of 78.48%.

MS m/z (ESI): 804.2 [M+1]

### Step 2

### N-(2-chloro-4-(trifluoromethyl)phenyl)-2-(2-(cyclohexylidenemethyl)-5-ethyl-6-(4-(5-hydroxy-6 -methylpyrimidine-4-carbonyl)piperazin-1-yl)-7-oxo-[1,2,4]triazolo[1,5-a]pyrimidin-4(7H)-yl)ac etamide

2-(6-(4-(5-(benzyloxy)-6-methylpyrimidine-4-carbonyl)piperazin-1-yl)-2-(cyclohexylid enemethyl)-5-ethyl-7-oxo-[1,2,4]triazolo[1,5-a]pyrimidin-4(7H)-yl)-N-(2-chloro-4-(trifluorometh yl)phenyl)acetamide 20b (40 mg, 49.74 µmol) was added to dichloromethane (3 mL). Boron trichloride (1 mL) was added at 0 °C, and the reaction was performed at 0 C for 1 hour. Methanol was added to quench the reaction, and the mixture was concentrated under reduced pressure. The resulting residue was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(2-(cyclohexylidenemethyl)-5-ethyl-6-(4-(5-hydroxy-6-methylpyrimidine-4-carbonyl)piperazin-1-yl )-7-oxo-[1,2,4]triazolo[1,5-a]pyrimidin-4(7H)-yl)acetamide 20 (13.0 mg) in a yield of 9.79%.

MS m/z (ESI): 714.4 [M+1]
¹H NMR (400 MHz, DMSO-*d₆*) δ 10.37 (s, 1H), 10.23 (s, 1H), 8.58 (s, 1H), 8.04 (d, *J =* 8.6 Hz, 1H), 7.97 (d, *J =* 2.1 Hz, 1H), 7.72 (dd, *J =* 8.7, 2.1 Hz, 1H), 6.09 (s, 1H), 5.29 (s, 2H), 4.52 (d, *J =* 12.5 Hz, 1H), 3.30 - 3.19 (m, 1H), 3.00 (d, *J =* 9.2 Hz, 3H), 2.90 (t, *J =* 6.0 Hz, 2H), 2.81 (d, *J=* 11.4 Hz, 1H), 2.63 (d, *J =* 10.8 Hz, 1H), 2.44 (s, 3H), 2.26 (t, *J =* 5.6 Hz, 2H), 1.57 (t, *J=* 7.1 Hz, 3H), 1.49 (d, *J= 5.5* Hz, 2H), 1.18 (t, *J=* 7.4 Hz, 3H).

### Example 21

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-((tetrahydro-4H-pyran-4-ylidene)methyl)-[1,2,4]triazolo[1,5-a]pyrimi din-4(7H)-yl)acetamide

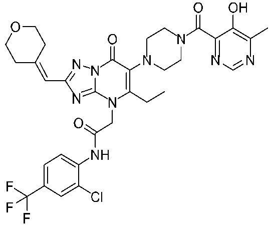

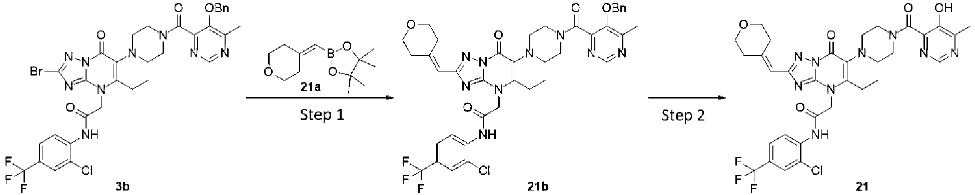

### Step 1

### 2-(6-(4-(5-(benzyloxy)-6-methylpyrimidine-4-carbonyl)piperazin-1-yl)-5-ethyl-7-oxo-2-((tetrahy dro-4H-pyran-4-ylidene)methyl)-[1,2,4]triazolo[1,5-a]pyrimidin-4(7H)-yl)-N-(2-chloro-4-(trifluo romethyl)phenyl)acetamide

At room temperature, 2-(6-(4-(5-(benzyloxy)-6-methylpyrimidine-4-carbonyl) piperazin-1-yl)-2-bromo-5-ethyl-7-oxo-[1,2,4]triazolo[1,5-a]pyrimidin-4(7H)-yl)-N-(2-chloro-4-( trifluoromethyl)phenyl)acetamide 3b (50 mg, 63.37 µmol), 4,4,5,5-tetramethyl-2-(tetrahydropyran-4-ylidene)methyl-1,3,2-dioxaborolane 21a (17.04 mg, 76.05 µmol, prepared according to published patent WO2021210586), [1,1'-bis(diphenylphosphino)ferrocene]palladium(II) dichloride dichloromethane complex (5.14 mg, 6.34 µmol), and sodium carbonate (20.15 mg, 190.11 µmol) were added to 1,4-dioxane (1 mL). The mixture was subject to argon replacement four times, the temperature was raised to 80 °C, and the reaction was performed for 2 hours. Water (20 mL) was added to the reaction mixture, and the mixture was extracted with ethyl acetate (40 mL×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-5-ethyl-7-oxo-2-((tetrahydro-4H-pyran-4-ylidene) methyl)-[1,2,4]triazolo[1,5-a]pyrimidin-4(7H)-yl)-N-(2-chloro-4-(trifluoromethyl)phenyl)acetami de 21b (30 mg). Yield: 58.72%.

MS m/z (ESI): 806.1 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-((tetrahydro-4H-pyran-4-ylidene)methyl)-[1,2,4]triazolo[1,5-a]pyrimi din-4(7H)-yl)acetamide

2-(6-(4-(5-(benzyloxy)-6-methylpyrimidine-4-carbonyl)piperazin-1-yl)-5-ethyl-7-oxo-2 -((tetrahydro-4H-pyran-4-ylidene)methyl)-[1,2,4]triazolo[1,5-a]pyrimidin-4(7H)-yl)-N-(2-chloro-4-(trifluoromethyl)phenyl)acetamide **21b** (30 mg, 37.21 µmol) was added to dichloromethane (3 mL). Boron trichloride (1 mL) was added at 0 °C, and the reaction was performed at 0 C for 1 hour. Methanol was added to quench the reaction, and the mixture was concentrated under reduced pressure. The resulting residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-7-oxo-2-((tetrahydro-4H-pyran-4-ylidene)methyl)-[1,2,4]triazolo[1,5-a]pyrimi din-4(7H)-yl)acetamide **21** (4.93 mg). Yield: 8.61%.

MS m/z (ESI): 716.1 [M+1]
¹H NMR (400 MHz, DMSO-*d₆*) δ 10.37 (s, 1H), 10.23 (s, 1H), 8.58 (s, 1H), 8.06 - 7.92 (m, 2H), 7.72 (dd, *J =* 8.8, 2.1 Hz, 1H), 6.21 (s, 1H), 5.29 (s, 2H), 4.52 (d, *J=* 12.2 Hz, 1H), 3.68 (t, *J =* 5.4 Hz, 2H), 3.56 (t, *J =* 5.5 Hz, 2H), 3.31 - 3.20 (m, 2H), 3.02 (t, *J =* 6.9 Hz, 6H), 2.81 (d, *J= 11.4* Hz, 1H), 2.63 (d, *J=* 10.9 Hz, 1H), 2.44 (s, 3H), 2.37 (t, *J= 5.3* Hz, 2H), 1.18 (t, *J=* 7.4 Hz, 3H).

### Example 22

### N-(2-chloro-4-(trifluoromethyl)phenyl)-2-(5-ethyl-6-(4-(3-hydroxypicolinoyl)piperazin-1-yl)-7-o xo-2-(5-oxo-1,3a,4,5,6,6a-hexahydropentalen-2-yl)-[1,2,4]triazolo[1,5-a]pyrimidin-4(7H)-yl)acet amide

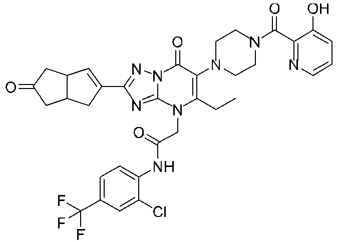

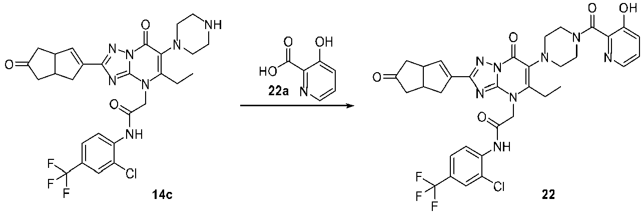

N-(2-chloro-4-(trifluoromethyl)phenyl)-2-(5-ethyl-7-oxo-2-(5-oxo-1,3a,4,5,6,6a-hexahy dropentalen-2-yl)-6-(piperazin-1-yl)-[1,2,4]triazolo[1,5-a]pyrimidin-4(7H)-yl)acetamide 14c (60 mg, 99.33 µmol), 3-hydroxypicolinic acid 22a (17.96 mg, 129.13 µmol, commercially available), 1-(3-dimethylaminopropyl)-3-ethylcarbodiimide hydrochloride (28.56 mg, 149.00 µmol), 1-hydroxybenzotriazole (20.13 mg, 149.00 µmol), and N,N-diisopropylethylamine (64.19 mg, 496.67 µmol) were added to N,N-dimethylformamide (1 mL). The reaction was performed at room temperature for 16 hours. The reaction mixture was extracted with ethyl acetate (30 mL×2). The aqueous layer was separated, and the combined organic phases were washed with saturated sodium chloride solution (20 mL ×2), dried over anhydrous sodium sulfate, and concentrated under reduced pressure. The resulting residue was purified by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(5-ethyl-6-(4-(3-hydroxypicolinoyl)piperazin-1-yl)-7-o xo-2-(5-oxo-1,3a,4,5,6,6a-hexahydropentalen-2-yl)-[1,2,4]triazolo[1,5-a]pyrimidin-4(7H)-yl)acet amide **22** (3.74 mg). Yield: 5.00%.

MS m/z (ESI): 725.2 [M+1]
¹H NMR (400 MHz, DMSO-*d₆*) δ 10.43 (s, 1H), 10.35 (s, 1H), 8.11 - 8.01 (m, 2H), 7.96 (d, *J =* 2.1 Hz, 1H), 7.71 (dd, *J=* 8.9, 2.1 Hz, 1H), 7.34 - 7.27 (m, 2H), 6.52 (d, *J =* 2.1 Hz, 1H), 5.36 - 5.27 (m, 2H), 4.54 (d, *J =* 12.5 Hz, 1H), 3.48 - 3.37 (m, 3H), 3.24 (d, *J =* 12.1 Hz, 1H), 3.16 - 2.88 (m, 7H), 2.79 (d, *J =* 11.4 Hz, 1H), 2.62 (s, 2H), 2.59 - 2.53 (m, 1H), 2.25 (d, *J* = 18.7 Hz, 2H), 1.94 (dd, *J =* 19.0, 7.0 Hz, 1H), 1.18 (t, *J =* 7.4 Hz, 3H).

### Example 23

### 2-(2-(bicyclo[2.2.1]hept-2-en-2-yl)-5-ethyl-6-(4-(5-hydroxy-6-methylpyrimidine-4-carbonyl)pipe razin-1-yl)-7-oxo-[1,2,4]triazolo[1,5-a]pyrimidin-4(7H)-yl)-N-(2-chloro-4-(trifluoromethyl)phen yl)acetamide

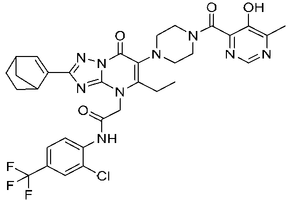

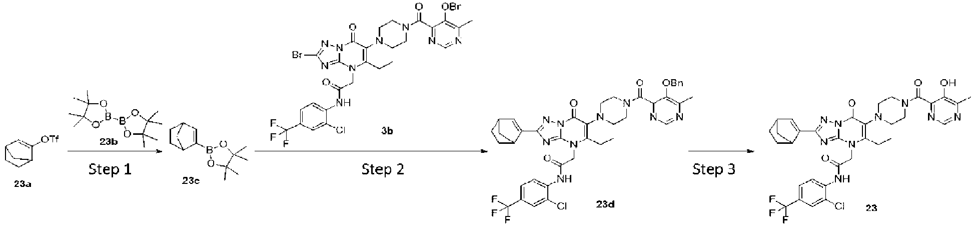

### Step 1

### 2-(bicyclo[2.2.1]hept-2-en-2-yl)-4,4,5,5-tetramethyl-1,3,2-dioxaborolane

2-Bicyclo[2.2.1]hept-2-en-2-yl trifluoromethanesulfonate **23a** (90 mg, 371.57 µmol, prepared according to a known method "Organic Letters (2019), 21(6), 1555-1558"), 4,4,5,5-tetramethyl-2-(4,4,5,5-tetramethyl-1,3,2-dioxaborolane-2-yl)-1,3,2-dioxaborolane **23b** (141.53 mg, 557.36 µmol, commercially available), potassium acetate (109.40 mg, 1.11 mmol), and bis(triphenylphosphine)palladium(II) dichloride (26.08 mg, 37.16 µmol) were added to 1,4-dioxane (1 mL), and the reaction was performed at 80 °C for 16 h. The reaction mixture was extracted with ethyl acetate (30 mL ×2), the aqueous layer was separated, and the combined organic phase was washed with saturated sodium chloride solution (20 mL×2), dried over anhydrous sodium sulfate, and concentrated under reduced pressure. The resulting residue was purified by silica gel column chromatography (eluent: System A) to obtain 2-(bicyclo[2.2.1]hept-2-en-2-yl)-4,4,5,5-tetramethyl-1,3,2-dioxaborolane **23c** (20 mg) in a yield of 24.45%.

### Step 2

### 2-(6-(4-(5-(benzyloxy)-6-methylpyrimidine-4-carbonyl)piperazin-1-yl)-2-(bicyclo[2.2.1]hept-2-e n-2-yl)-5-ethyl-7-oxo-[1,2,4]triazolo[1,5-a]pyrimidin-4(7H)-yl)-N-(2-chloro-4-(trifluoromethyl)p henyl)acetamide

2-(6-(4-(5-(Benzyloxy)-6-methylpyrimidine-4-carbonyl)piperazin-1-yl)-2-bromo-5-ethy l-7-oxo-[1,2,4]triazolo[1,5-a]pyrimidin-4(7H)-yl)-N-(2-chloro-4-(trifluoromethyl)phenyl)acetami de **3b** (50 mg, 63.37 µmol), 2-(bicyclo[2.2.1]hept-2-en-2-yl)-4,4,5,5-tetramethyl-1,3,2-dioxaborolane **23c** (16.74 mg, 76.05 µmol), [1,1'-bis(diphenylphosphino)ferrocene]palladium(II) dichloride dichloromethane complex (5.14 mg, 6.34 µmol), and sodium carbonate (20.15 mg, 190.11 µmol) were added to 1,4-dioxane (1 mL). The mixture was subject to argon replacement four times, and the mixture was heated to 80 C and the reaction was performed for 2 h. Water (20 mL) was added to the reaction mixture, which was extracted with ethyl acetate (30 mL×2). The aqueous layer was separated, and the combined organic phase was washed with saturated sodium chloride solution (20 mL ×2), dried over anhydrous sodium sulfate, and concentrated under reduced pressure. The resulting residue was purified by silica gel column chromatography (eluent: System A) to obtain 2-(6-(4-(5-(benzyloxy)-6-methylpyrimidine-4-carbonyl)piperazin-1-yl)-2-(bicyclo[2.2.1]hept-2-e n-2-yl)-5-ethyl-7-oxo-[1,2,4]triazolo[1,5-a]pyrimidin-4(7H)-yl)-N-(2-chloro-4-(trifluoromethyl)p henyl)acetamide **23d** (30 mg) in a yield of 59.01%.

MS m/z (ESI): 802.3 [M+1]

### Step 3

### 2-(2-(bicyclo[2.2.1]hept-2-en-2-yl)-5-ethyl-6-(4-(5-hydroxy-6-methylpyrimidine-4-carbonyl)pipe razin-1-yl)-7-oxo-[1,2,4]triazolo[1,5-a]pyrimidin-4(7H)-yl)-N-(2-chloro-4-(trifluoromethyl)phen yl)acetamide

2-(6-(4-(5-(Benzyloxy)-6-methylpyrimidine-4-carbonyl)piperazin-1-yl)-2-(bicyclo[2.2. 1]hept-2-en-2-yl)-5-ethyl-7-oxo-[1,2,4]triazolo[1,5-a]pyrimidin-4(7H)-yl)-N-(2-chloro-4-(trifluor omethyl)phenyl)acetamide **23d** (30 mg, 37.40 µmol) was added to dichloromethane (2 mL). Boron trichloride (0.8 mL) was added at 0 °C, and the reaction was performed at 0 C for 1 h. Methanol was added to quench the reaction, and the mixture was concentrated under reduced pressure. The resulting residue was purified by preparative liquid chromatography (separation column: AKZONOBEL Kromasil; 250 ×21.2 mm I.D.; 5 µm, 20 mL/min; mobile phase A: 0.05% TFA+H₂O, mobile phase B: CH₃CN) to obtain 2-(2-(bicyclo[2.2.1]hept-2-en-2-yl)-5-ethyl-6-(4-(5-hydroxy-6-methylpyrimidine-4-carbonyl)piperazin-1-yl)-7-oxo-[1,2,4]triazolo[1,5-a]pyrimi din-4(7H)-yl)-N-(2-chloro-4-(trifluoromethyl)phenyl)acetamide **23** (2.6 mg) in a yield of 9.41%.

MS m/z (ESI): 711.7 [M+1]
¹H NMR (400 MHz, DMSO-*d₆*) δ 10.36 (s, 1H), 10.24 (s, 1H), 8.58 (s, 1H), 8.06 (d, *J=* 8.5 Hz, 1H), 7.97 *(d, J=* 2.0 Hz, 1H), 7.77 - 7.68 (m, 1H), 6.75 (d, J= 3.1 Hz, 1H), 5.30 (s, 2H), 4.52 (d, *J=* 12.8 Hz, 1H), 3.50 (d, *J=* 12.4 Hz, 4H), 3.26 (d, *J=* 12.8 Hz, 2H), 3.01 (d, *J= 22.4* Hz, 4H), 2.81 (d, *J=* 11.4 Hz, 1H), 2.64 (s, 1H), 2.44 (s, 3H), 1.78 (d, *J=* 5.9 Hz, 2H), 1.47 (d, *J* = 8.0 Hz, 1H), 1.30 - 1.20 (m, 2H), 1.18 (t, *J= 7.4* Hz, 3H), 1.03 (d, *J=* 27.2 Hz, 2H).

### Example 24

### N-(4-(tert-butyl)phenyl)-2-(2-(cyclohept-1-en-1-yl)-5-ethyl-6-(4-(3-hydroxyisonicotinoyl)piperaz in-1-yl)-7-oxo-[1,2,4]triazolo[1,5-a]pyrimidin-4(7H)-yl)acetamide

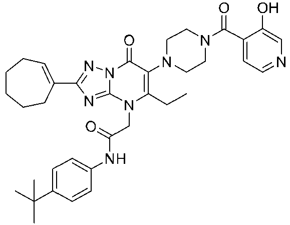

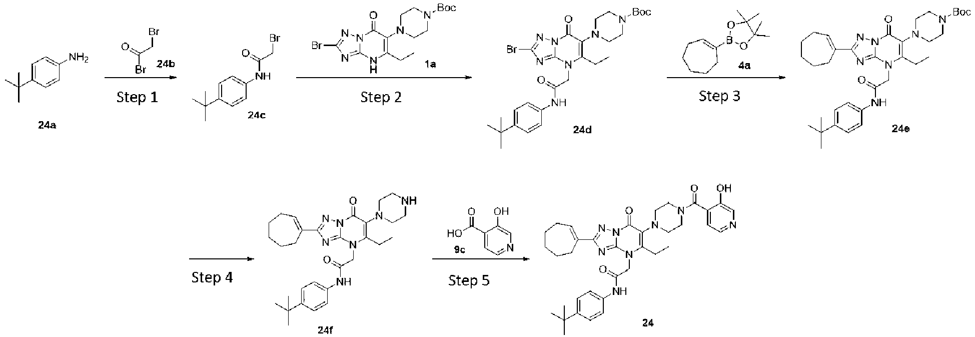

### Step 1

### 2-Bromo-N-(4-(tert-Butyl)phenyl)acetamide

4-tert-butyl aniline **24a** (1 g, 6.70 mmol, commercially available) was dissolved in dichloromethane (10 mL). 4-Dimethylaminopyridine (818.66 mg, 6.70 mmol) was added under ice bath, and the mixture was stirred for 10 min. 2-Bromoacetyl bromide **24b** (1.62 g, 8.04 mmol, commercially available) was slowly added, and the reaction was performed at room temperature for 4 h. Water (20 mL) was added to the reaction mixture, which was extracted with dichloromethane (20 mL ×3). The combined organic phase was washed with saturated sodium chloride solution (20 mL), dried over anhydrous sodium sulfate, filtered, and concentrated under reduced pressure. The resulting residue was purified by silica gel column chromatography (eluent: System A) to obtain 2-bromo-N-(4-(tert-Butyl)phenyl)acetamide **24c** (1.5 g) in a yield of 82.86%.
MS m/z (ESI): 270.0 [M+1]

### Step 2

### tert-butyl 4-(2-bromo-4-(2-((4-(tert-Butyl)phenyl)amino)-2-oxoethyl)-5-ethyl-7-oxo-4,7 -dihydro-[1,2,4]triazolo[1,5-a]pyrimidin-6-yl)piperazine-1-carboxylate

2-Bromo-N-(4-(tert-Butyl)phenyl)acetamide **24c** (303.49 mg, 1.12 mmol), tert-butyl 4-(2-bromo-5-ethyl-7-oxo-4,7-dihydro-[1,2,4]triazolo[1,5-a]pyrimidin-6-yl)piperazine-1-carboxy late **1a** (400 mg, 936.12 µmol), and N,N-diisopropylethylamine (362.95 mg, 2.81 mmol) were added to N,N-dimethylformamide (7 mL) at room temperature. The mixture was heated to 70 °C and the reaction was performed for 2 h. Water (20 mL) was added to the reaction mixture, which was extracted with ethyl acetate (30 mL×3). The combined organic phase was washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(2-bromo-4-(2-((4-(tert-Butyl)phenyl) amino)-2-oxoethyl)-5-ethyl-7-oxo-4,7-dihydro-[1,2,4]triazolo[1,5-a]pyrimidin-6-yl)piperazine-1-carboxylate **24d** (290 mg) in a yield of 50.25%.

MS m/z (ESI): 560.2 [M-56+H]

### Step 3

### tert-butyl 4-(4-(2-((4-(tert-Butyl)phenyl)amino)-2-oxoethyl)-2-(cyclohept-1-en-1-yl)-5-ethyl-7-oxo-4,7-dihydro-[1,2,4]triazolo[1,5-a]pyrimidin-6-yl)piperazine-1-carboxylate

At room temperature, tert-butyl 4-(2-bromo-4-(2-((4-(tert-Butyl)phenyl)amino)-2 -oxoethyl)-5-ethyl-7-oxo-4,7-dihydro-[1,2,4]triazolo[1,5-a]pyrimidin-6-yl)piperazine-1-carboxyl ate **24d** (290 mg, 470.36 µmol), 2-(cyclohept-1-en-1-yl)-4,4,5,5-tetramethyl-1,3,2-dioxaborolane **4a** (135.83 mg, 611.47 µmol), [1,1'-bis(diphenylphosphino)ferrocene]palladium(II) dichloride dichloromethane complex (38.13 mg, 47.04 µmol), and sodium carbonate (149.56 mg, 1.41 mmol) were added to a mixed solution of 1,4-dioxane (5 mL) and water (1 mL). The mixture was subject to argon replacement three times, the temperature was raised to 80 °C, and the reaction was performed for 2 hours. Water (20 mL) was added to the reaction mixture, and the mixture was extracted with ethyl acetate (30 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(4-(2-((4-(tert-Butyl)phenyl)amino)-2-oxoethyl)-2-(cyclohept-1-en-1-yl)-5-ethyl-7-oxo-4,7-dihydro-[1,2,4]triazolo[1,5-a]pyrimidin-6-yl)piperazine-1-carboxylate **24e** (260 mg) with a yield of 87.49%.

MS m/z (ESI): 576.3 [M-56+H]

### Step 4

### N-(4-(tert-Butyl)phenyl)-2-(2-(cyclohept-1-en-1-yl)-5-ethyl-7-oxo-6-(piperazin-1-yl)-[1,2,4] triazolo[1,5-a]pyrimidin-4(7H)-yl)acetamide

At room temperature, trifluoroacetic acid (1.5 mL) was added to a solution of 4-(4-(2-((4-(tert-Butyl)phenyl)amino)-2-oxoethyl)-2-(cyclohept-1-en-1-yl)-5-ethyl-7-oxo-4,7-dih ydro-[1,2,4]triazolo[1,5-a]pyrimidin-6-yl)piperazine-1-carboxylate **24e** (140 mg, 221.59 µmol) in dichloromethane (6 mL), and the reaction was performed at room temperature for 40 minutes. The mixture was concentrated under reduced pressure to obtain N-(4-(tert-Butyl)phenyl)-2-(2-(cyclohept-1-en-1-yl)-5-ethyl-7-oxo-6-(piperazin-1-yl)-[1,2,4]triaz olo[1,5-a]pyrimidin-4(7H)-yl)acetamide **24f** (117 mg) with a yield of 99.31%, which was used directly in the next step.

MS m/z (ESI): 532.3 [M+1]

### Step 5

### N-(4-(tert-butyl)phenyl)-2-(2-(cyclohept-1-en-1-yl)-5-ethyl-6-(4-(3-hydroxyisonicotinoyl) piperazin-1-yl)-7-oxo-[1,2,4]triazolo[1,5-a]pyrimidin-4(7H)-yl)acetamide

At room temperature, 3-hydroxyisonicotinic acid **9c** (20.93 mg, 150.46 µmol), 1-(3-dimethylaminopropyl)-3-ethylcarbodiimide hydrochloride (28.84 mg, 150.46 µmol), 1-hydroxybenzotriazole (20.33 mg, 150.46 µmol), and N,N-diisopropylethylamine (48.61 mg, 376.16 µmol) were added to N,N-dimethylformamide (1.5 mL), and the reaction was performed at room temperature for 30 minutes. Then N-(4-(tert-butyl)phenyl)-2-(2-(cyclohept-1-en-1-yl)-5-ethyl-7-oxo-6-(piperazin-1-yl)-[1,2,4]triazolo[1,5-a]pyrimidin-4(7H)-yl)acetamide 24f (40 mg, 75.23 µmol) was added, and the reaction was performed at room temperature for 16 hours. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(4-(tert-Butyl)phenyl)-2-(2-(cyclohept-1-en-1-yl)-5-ethyl-6-(4-(3-hydroxyisonicotinoyl)piperazin-1-yl)-7-oxo-[1,2,4]triazolo [1,5-a]pyrimidin-4(7H)-yl)acetamide **24** (19.42 mg) with a yield of 39.11%.

MS m/z (ESI): 653.3 [M+1]
¹H NMR (400 MHz, DMSO-*d₆*) δ 11.12 (s, 1H), 10.43 (s, 1H), 8.44 - 8.17 (m, 2H), 7.63 - 7.42 (m, 3H), 7.34 (d, *J =* 8.5 Hz, 2H), 7.06 (t, *J =* 6.7 Hz, 1H), 5.13 (s, 2H), 4.53 (d, *J =* 12.5 Hz, 1H), 3.48 (d, J= 11.2 Hz, 2H), 3.36 - 3.23 (m, 2H), 2.96 (dt, *J =* 16.0, 9.0 Hz, 3H), 2.81 (d, *J=* 11.2 Hz, 1H), 2.79 - 2.70 (m, 2H), 2.64 (d, *J=* 11.2 Hz, 1H), 2.32 (d, *J =* 8.8 Hz, 2H), 1.78 (s, 2H), 1.64 - 1.42 (m, 4H), 1.25 (s, 9H), 1.16 (t, J= 7.4 Hz, 3H).

### Example 25

### 2-(2-(cyclohept-1-en-1-yl)-5-ethyl-6-(4-(3-hydroxyisonicotinoyl)piperazin-1-yl)-7-oxo-[1,2,4] triazolo[1,5-a]pyrimidin-4(7H)-yl)-N-(4-(pentafluoro-λ⁶-sulfaneyl)phenyl)acetamide

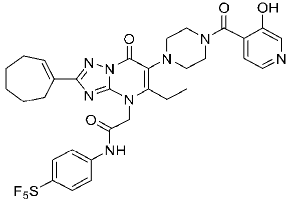

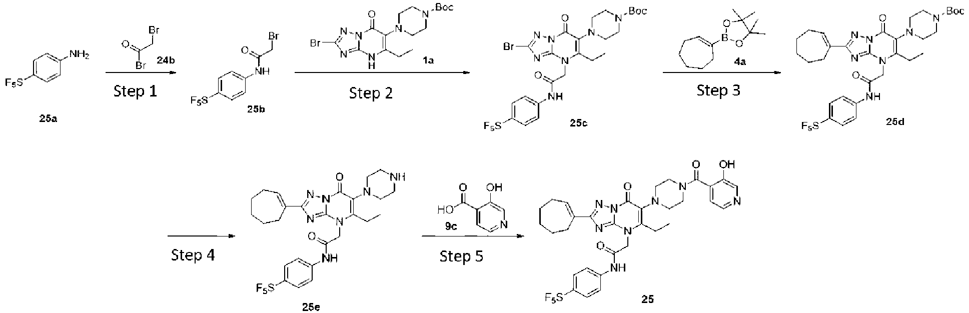

### Step 1

### 2-bromo-N-(4-(pentafluoro-λ⁶-sulfaneyl)phenyl)acetamide

Under an ice bath and argon atmosphere, a solution of 2-bromoacetyl bromide 24b (574.66 mg, 2.85 mmol) in dichloromethane (5 mL) was added to a solution of 4-(pentafluorosulfaneyl)aniline 25a (520 mg, 2.37 mmol, commercially available) in dichloromethane (7 mL), and the reaction was performed at room temperature for 16 hours. Water (20 mL) was added to the reaction mixture, and the mixture was extracted with dichloromethane (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System A) to obtain 2-bromo-N-(4-(pentafluoro-λ⁶-sulfaneyl)phenyl)acetamide 25b (720 mg) with a yield of 89.23%.

MS m/z (ESI): 339.9 [M+1]

### Step 2

### tert-butyl 4-(2-bromo-5-ethyl-7-oxo-4-(2-oxo-2-((4-(pentafluoro-λ⁶-sulfaneyl)phenyl)amino) ethyl)-4,7-dihydro-[1,2,4]triazolo[1,5-a]pyrimidin-6-yl)piperazine-1-carboxylate

2-Bromo-N-(4-(pentafluoro-λ⁶-sulfaneyl)phenyl)acetamide 25b (382.06 mg, 1.12 mmol), tert-butyl 4-(2-bromo-5-ethyl-7-oxo-4,7-dihydro-[1,2,4]triazolo[1,5-a]pyrimidin-6-yl) piperazine-1-carboxylate **1a** (400 mg, 936.12 µmol), and N,N-diisopropylethylamine (604.92 mg, 4.68 mmol) were added to N,N-dimethylformamide (7 mL), the temperature was raised to 50 °C, and the reaction was performed for 2 hours. Water (20 mL) was added to the reaction mixture, the mixture was extracted with ethyl acetate (20 mL ×3), the combined organic phase was washed with saturated sodium chloride solution (20 mL), dried over anhydrous sodium sulfate, filtered, and concentrated under reduced pressure, and the resulting residue was purified by silica gel column chromatography (eluent: System B) to obtain tert-butyl 4-(2-bromo-5-ethyl-7-oxo -4-(2-oxo-2-((4-(pentafluoro-λ⁶-sulfaneyl)phenyl)amino)ethyl)-4,7-dihydro-[1,2,4]triazolo[1,5-a] pyrimidin-6-yl)piperazine-1-carboxylate 25c (710 mg), yield: 77.34%.

MS m/z (ESI): 630.1 [M-56+H]

### Step 3

### tert-butyl 4-(2-(cyclohept-1-en-1-yl)-5-ethyl-7-oxo-4-(2-oxo-2-((4-(pentafluoro-λ⁶-sulfaneyl)phenyl)amino )ethyl)-4,7-dihydro-[1,2,4]triazolo[1,5-a]pyrimidin-6-yl)piperazine-1-carboxylate

At room temperature, tert-butyl 4-(2-bromo-5-ethyl-7-oxo-4-(2-oxo-2-((4-(pentafluoro -λ⁶-sulfaneyl)phenyl)amino)ethyl)-4,7-dihydro-[1,2,4]triazolo[1,5-a]pyrimidin-6-yl)piperazine-1-carboxylate 25c (710 mg, 1.03 mmol), 2-(cyclohept-1-en-1-yl)-4,4,5,5-tetramethyl-1,3,2 -dioxaborolane 4a (298.66 mg, 1.34 mmol), [1,1'-bis(diphenylphosphino)ferrocene]palladium(II) dichloride dichloromethane complex (83.83 mg, 103.42 µmol), and sodium carbonate (328.86 mg, 3.10 mmol) were added to a mixture of 1,4-dioxane (12 mL) and water (2 mL), argon replacement was performed 3 times, the temperature was raised to 90 °C, and the reaction was performed for 2 hours. Water (20 mL) was added to the reaction mixture, the mixture was extracted with ethyl acetate (20 mL×3), the combined organic phase was washed with saturated sodium chloride solution (20 mL), dried over anhydrous sodium sulfate, filtered, and concentrated under reduced pressure, and the resulting residue was purified by silica gel column chromatography (eluent: System B) to obtain tert-butyl 4-(2-(cyclohept-1-en-1-yl)-5-ethyl-7-oxo-4-(2-oxo-2-((4-(pentafluoro-λ⁶-sulfaneyl)phenyl)amino)ethyl)-4,7-dihydro-[1,2,4]tria zolo[1,5-a]pyrimidin-6-yl)piperazine-1-carboxylate 25d (450 mg), yield: 62.00%.

MS m/z (ESI): 646.2 [M-56+H]

### Step 4

### 2-(2-(cyclohept-1-en-1-yl)-5-ethyl-7-oxo-6-(piperazin-1-yl)-[1,2,4]triazolo[1,5-a]pyrimidin-4(7H )-yl)-N-(4-(pentafluoro-λ⁶ -sulfaneyl)phenyl)acetamide

At room temperature, trifluoroacetic acid (2 mL) was added to a solution of tert-butyl 4-(2-(cyclohept-1-en-1-yl)-5-ethyl-7-oxo-4-(2-oxo-2-((4-(pentafluoro-λ⁶-sulfaneyl)phenyl)amino )ethyl)-4,7-dihydro-[1,2,4]triazolo[1,5-a]pyrimidin-6-yl)piperazine-1-carboxylate 25d (450 mg, 641.25 µmol) in dichloromethane (8 mL), and the reaction was performed at room temperature for 40 minutes. The mixture was concentrated under reduced pressure, and the residue was purified by silica gel column chromatography (eluent: System B) to obtain 2-(2-(cyclohept-1-en-1-yl)-5-ethyl-7-oxo-6-(piperazin-1-yl)-[1,2,4]triazolo[1,5-a]pyrimidin-4(7H )-yl)-N-(4-(pentafluoro-λ⁶ -sulfaneyl)phenyl)acetamide 25e (340 mg), yield: 88.13%.

MS m/z (ESI): 602.3 [M+1]

### Step 5

### 2-(2-(cyclohept-1-en-1-yl)-5-ethyl-6-(4-(3-hydroxyisonicotinoyl)piperazin-1-yl)-7-oxo-[1,2,4] triazolo[1,5-a]pyrimidin-4(7H)-yl)-N-(4-(pentafluoro-λ⁶-sulfaneyl)phenyl)acetamide

At room temperature, 3-hydroxyisonicotinic acid 9c (27.75 mg, 199.46 µmol), 1-(3-dimethylaminopropyl)-3-ethylcarbodiimide hydrochloride (38.24 mg, 199.46 µmol), 1-hydroxybenzotriazole (26.95 mg, 199.46 µmol), and N,N-diisopropylethylamine (64.44 mg, 498.64 µmol) were added to N,N-dimethylformamide (1.5 mL), the reaction was performed at room temperature for 40 minutes, then 2-(2-(cyclohept-1-en-1-yl)-5-ethyl-7-oxo-6-(piperazin-1-yl)-[1,2,4]triazolo[1,5-a]pyrimidin-4(7H)-yl)-N-(4-(pentafluoro-λ⁶-sulfaneyl)phen yl)acetamide 25e (60 mg, 99.73 µmol) was added, and the reaction was performed at room temperature for 16 hours. Water (20 mL) was added to the reaction mixture, the mixture was extracted with ethyl acetate (20 mL×3), the combined organic phase was washed with saturated sodium chloride solution (20 mL), dried over anhydrous sodium sulfate, filtered, and concentrated under reduced pressure, and the resulting residue was purified by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain 2-(2-(cyclohept-1-en-1-yl)-5-ethyl-6-(4-(3-hydroxyisonicotinoyl)piperazin-1-yl)-7-oxo-[1,2,4]tria zolo[1,5-a]pyrimidin-4(7H)-yl)-N-(4-(pentafluoro-λ⁶-sulfaneyl)phenyl)acetamide 25 (24.53 mg), yield: 33.32%.

MS m/z (ESI): 723.3 [M+1]
¹H NMR (400 MHz, DMSO-*d₆*) δ 10.95 (s, 2H), 8.29 (s, 1H), 8.22 (d, *J=* 5.1 Hz, 1H), 7.95 - 7.84 (m, 2H), 7.77 (d, *J=* 8.9 Hz, 2H), 7.42 (d, *J=* 5.0 Hz, 1H), 7.04 (t, *J=* 6.7 Hz, 1H), 5.18 (s, 2H), 4.53 (d, *J =* 12.6 Hz, 1H), 3.47 (q, *J =* 11.1 Hz, 2H), 3.38 - 3.13 (m, 2H), 3.14 - 2.87 (m, 3H), 2.80 (d, *J=* 11.3 Hz, 1H), 2.76 - 2.70 (m, 2H), 2.64 (d, *J=* 11.3 Hz, 1H), 2.29 (t, *J* = 8.1 Hz, 2H), 1.77 (s, 2H), 1.51 (s, 4H), 1.16 (t, *J=* 7.4 Hz, 3H).

### Example 26

### N-(4-(tert-Butyl)phenyl)-2-(2-(cyclohept-1-en-1-yl)-5-ethyl-6-(4-(5-hydroxy-6-methylpyrimidine -4-carbonyl)piperazin-1-yl)-7-oxo-[1,2,4]triazolo[1,5-a]pyrimidin-4(7H)-yl)acetamide

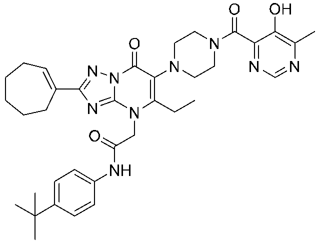

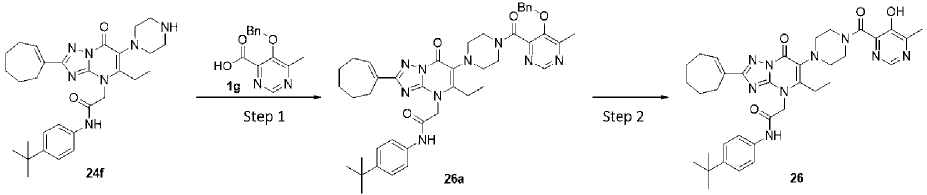

### Step 1

### 2-(6-(4-(5-(benzyloxy)-6-methylpyrimidine-4-carbonyl)piperazin-1-yl)-2-(cyclohept-1-en-1-yl)-5 -ethyl-7-oxo-[1,2,4]triazolo[1,5-a]pyrimidin-4(7H)-yl)-N-(4-(tert-Butyl)phenyl)acetamide

At room temperature, 5-(benzyloxy)-6-methylpyrimidine-4-carboxylic acid **1g** (68.91 mg, 282.12 µmol), 1-(3-dimethylaminopropyl)-3-ethylcarbodiimide hydrochloride (72.11 mg, 376.16 µmol), 1-hydroxybenzotriazole (50.83 mg, 376.16 µmol), and N,N-diisopropylethylamine (121.54 mg, 940.40 µmol) were added to N,N-dimethylformamide (1.5 mL), and the reaction was performed at room temperature for 40 min. Then a solution of N-(4-(tert-Butyl)phenyl)-2-(2-(cyclohept-1-en-1-yl)-5-ethyl-7-oxo-6-(piperazin-1-yl)-[1,2,4]triaz olo[1,5-a]pyrimidin-4(7H)-yl)acetamide 24f (100 mg, 188.08 µmol) in N,N-dimethylformamide (2 mL) was added, and the reaction was performed at room temperature for 16 h. Water (20 mL) was added to the reaction mixture, and the mixture was extracted with ethyl acetate (20 mL ×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-2-(cyclohept-1-en-1-yl)-5 -ethyl-7-oxo-[1,2,4]triazolo[1,5-a]pyrimidin-4(7H)-yl)-N-(4-(tert-Butyl)phenyl)acetamide 26a (65 mg) in a yield of 45.60%.

MS m/z (ESI): 758.5 [M+1]

### Step 2

### N-(4-(tert-Butyl)phenyl)-2-(2-(cyclohept-1-en-1-yl)-5-ethyl-6-(4-(5-hydroxy-6-methylpyrimidine -4-carbonyl)piperazin-1-yl)-7-oxo-[1,2,4]triazolo[1,5-a]pyrimidin-4(7H)-yl)acetamide

Under an ice bath and argon atmosphere, boron trichloride (1.5 mL) was added to a solution of 2-(6-(4-(5-(benzyloxy)-6-methylpyrimidine-4-carbonyl)piperazin-1-yl)-2-(cyclohept -1-en-1-yl)-5-ethyl-7-oxo-[1,2,4]triazolo[1,5-a]pyrimidin-4(7H)-yl)-N-(4-(tert-Butyl)phenyl)acet amide 26a (65 mg, 85.76 µmol) in dichloromethane (1 mL). The reaction was performed at room temperature for 16 h. The reaction was quenched by adding ice water. After dissolved in a small amount of methanol, the mixture was purified by preparative liquid chromatography (separation column: AKZONOBEL Kromasil; 250 ×21.2 mm I.D.; 5 µm, 20 mL/min; mobile phase A: 0.05% TFA+H₂O, mobile phase B: CH₃CN) to obtain N-(4-(tert-Butyl)phenyl)-2-(2-(cyclohept-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)acetamide 26 (12.35 mg) in a yield of 21.33%.

MS m/z (ESI): 668.4 [M+1]
¹H NMR (400 MHz, DMSO-*d₆*) δ 10.43 (d, *J =* 4.4 Hz, 1H), 10.25 (s, 1H), 8.58 (t, *J*= 3.2 Hz, 1H), 7.48 (dd, *J =* 8.7, 4.2 Hz, 2H), 7.38 -7.22 (m, 2H), 7.06 (q, *J =* 6.1 Hz, 1H), 5.13 (d, *J= 4.3* Hz, 2H), 4.52 (d, *J=* 12.6 Hz, 1H), 3.52 (s, 3H), 3.26 (d, *J=* 13.9 Hz, 1H), 2.97 (d, *J=* 17.0 Hz, 3H), 2.81 (d, *J =* 10.7 Hz, 1H), 2.78 - 2.69 (m, 2H), 2.64 (d, *J =* 11.4 Hz, 1H), 2.45 (dd, *J=* 4.6, 2.1 Hz, 3H), 2.31 (s, 2H), 1.88 - 1.72 (m, 2H), 1.52 (d, *J =* 12.0 Hz, 4H), 1.25 (dd, *J=* 4.8, 2.1 Hz, 9H), 1.20 - 1.10 (m, 3H).

### Example 27

### 2-(2-(cyclohept-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(4-(pentafluoro-λ⁶6-sulfaneyl)phenyl)acet amide

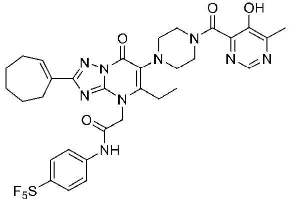

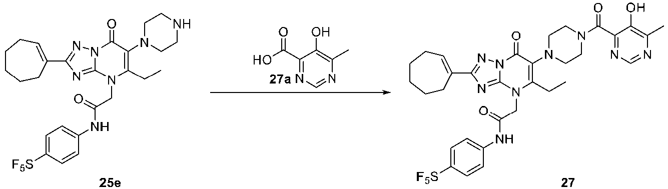

At room temperature, 5-hydroxy-6-methylpyrimidine-4-carboxylic acid 27a (30.74 mg, 199.46 µmol, commercially available), 1-(3-dimethylaminopropyl)-3-ethylcarbodiimide hydrochloride (38.24 mg, 199.46 µmol), 1-hydroxybenzotriazole (26.95 mg, 199.46 µmol), and N,N-diisopropylethylamine (64.44 mg, 498.64 µmol) were added to N,N-dimethylformamide (1.5 mL), and the reaction was performed at room temperature for 40 min. Then 2-(2-(cyclohept-1-en-1-yl)-5-ethyl-7-oxo-6-(piperazin-1-yl)-[1,2,4]triazolo[1,5-a]pyrimidin-4(7H )-yl)-N-(4-(pentafluoro-λ⁶-sulfaneyl)phenyl)acetamide 25e (60 mg, 99.73 µmol) was added, and the reaction was performed at room temperature for 16 h. Mass spectrometry monitoring showed product formation. Water (20 mL) was added to the reaction mixture, and the mixture was extracted with ethyl acetate (20 mL×3). The combined organic phases were washed with saturated sodium chloride solution (20 mL), dried over anhydrous sodium sulfate, filtered, and concentrated under reduced pressure. The residue was purified by preparative liquid chromatography (separation column: AKZONOBEL Kromasil; 250×21.2 mm I.D.; 5 µm, 20 mL/min; mobile phase A: 0.05% TFA+H₂O, mobile phase B: CH₃CN) to obtain 2-(2-(cyclohept-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(4-(pentafluoro-λ⁶-sulfaneyl)phenyl)aceta mide 27 (1.06 mg) in a yield of 1.35%.

MS m/z (ESI): 738.3 [M+1]
¹H NMR (400 MHz, DMSO-*d₆*) δ 10.97 (s, 1H), 10.24 (s, 1H), 8.58 (s, 1H), 7.93 - 7.85 (m, 2H), 7.77 (d, *J =* 9.0 Hz, 2H), 7.04 (t, *J =* 6.7 Hz, 1H), 5.18 (s, 2H), 4.52 (d, *J =* 12.7 Hz, 1H), 3.50 (q, *J =* 11.6, 10.4 Hz, 3H), 3.25 (t, *J =* 12.7 Hz, 1H), 2.98 (d, *J =* 12.1 Hz, 3H), 2.81 (d, *J =* 11.3 Hz, 1H), 2.73 (d, *J =* 9.6 Hz, 2H), 2.64 (d, *J =* 10.9 Hz, 1H), 2.45 (s, 3H), 2.30 (s, 2H), 1.77 (d, *J=* 7.6 Hz, 2H), 1.51 (s, 4H), 1.16 (t, J= 7.4 Hz, 3H).

### Example 28

N-(2-chloro-4-(trifluoromethyl)phenyl)-2-(2,5-diethyl-6-(4-(5-hydroxy-6-methylpyrimidine-4-carbonyl)piperazin-1-yl)-7-oxo-[1,2,4]triazolo[1,5-a]pyrimidin-4(7H)-yl)acetamide

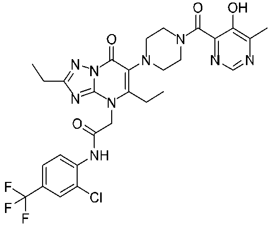

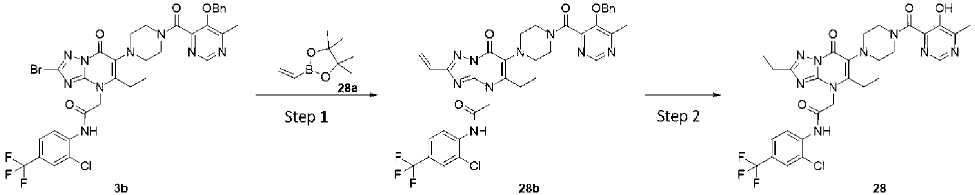

### Step 1

### 2-(6-(4-(5-(benzyloxy)-6-methylpyrimidine-4-carbonyl)piperazin-1-yl)-5-ethyl-7-oxo-2-vinyl-[1, 2,4]triazolo[1,5-a]pyrimidin-4(7H)-yl)-N-(2-chloro-4-(trifluoromethyl)phenyl)acetamide

At room temperature, 2-(6-(4-(5-(benzyloxy)-6-methylpyrimidine-4-carbonyl) piperazin-1-yl)-2-bromo-5-ethyl-7-oxo-[1,2,4]triazolo[1,5-a]pyrimidin-4(7H)-yl)-N-(2-chloro-4-( trifluoromethyl)phenyl)acetamide 3b (110 mg, 139.42 µmol), 4,4,5,5-tetramethyl-2-vinyl-1,3,2-dioxaborolane 28a (25.77 mg, 167.30 µmol, commercially available), [1,1'-bis(diphenylphosphino)ferrocene]palladium(II) dichloride dichloromethane complex (11.30 mg, 13.94 µmol), and sodium carbonate (44.33 mg, 418.25 µmol) were added to 1,4-dioxane (1 mL). The mixture was subject to argon replacement four times, then was heated to 80 °C and reacted for 1.5 hours. Water (20 mL) was added to the reaction mixture, and the mixture was extracted with ethyl acetate (40 mL×3). The combined organic phase was washed with saturated sodium chloride solution (20 mL), dried over anhydrous sodium sulfate, filtered, and concentrated under reduced pressure. The obtained residue was purified by silica gel column chromatography (eluent: System B) to obtain 2-(6-(4-(5-(benzyloxy)-6-methylpyrimidine-4-carbonyl)piperazin-1-yl)-5-ethyl-7-oxo-2-vinyl-[1,2,4]triazolo[1,5-a]pyrimidin-4(7H)-yl)-N-(2-chloro-4-(trifluoromethyl)phenyl)acetamide **28b** (90 mg) in a yield of 87.69%.

MS m/z (ESI): 736.2 [M+1]

### Step 2

### N-(2-chloro-4-(trifluoromethyl)phenyl)-2-(2,5-diethyl-6-(4-(5-hydroxy-6-methylpyrimidine-4-carbonyl)piperazin-1-yl)-7-oxo-[1,2,4]triazolo[1,5-a]pyrimidin-4(7H)-yl)acetamide

2-(6-(4-(5-(Benzyloxy)-6-methylpyrimidine-4-carbonyl)piperazin-1-yl)-5-ethyl-7-oxo-2 -vinyl-[1,2,4]triazolo[1,5-a]pyrimidin-4(7H)-yl)-N-(2-chloro-4-(trifluoromethyl)phenyl)acetamid e 28b (45 mg, 61.13 µmol) was added to methanol (1 mL), and palladium on carbon (7.42 mg, 61.13 µmol) was added. The reaction was performed at room temperature for 16 hours. The mixture was filtered and concentrated under reduced pressure. The residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(2,5-diethyl-6-(4-(5-hydroxy-6-methylpyrimidine-4-car bonyl)piperazin-1-yl)-7-oxo-[1,2,4]triazolo[1,5-a]pyrimidin-4(7H)-yl)acetamide 28 (2.61 mg) in a yield of 6.53%.

MS m/z (ESI): 648.0 [M+1]
¹H NMR (400 MHz, DMSO-*d₆*) δ 10.34 (s, 1H), 10.23 (s, 1H), 8.58 (s, 1H), 8.06 (d, *J=* 8.6 Hz, 1H), 7.96 (d, *J=* 2.1 Hz, 1H), 7.72 (dd, *J=* 8.7, 2.1 Hz, 1H), 5.31 (s, 2H), 4.52 (d, *J =* 12.6 Hz, 1H), 3.24 (t, *J =* 12.3 Hz, 1H), 2.97 (d, *J =* 10.5 Hz, 3H), 2.80 (d, *J =* 11.4 Hz, 1H), 2.70 (q, *J =* 7.6 Hz, 2H), 2.63 (d, *J=* 11.1 Hz, 1H), 2.44 (s, 3H), 1.24 (t, *J=* 7.6 Hz, 3H), 1.18 (t, *J=* 7.4 Hz, 3H).

### Example 29

### 2-(2-cycloheptyl-5-ethyl-6-(4-(2-hydroxybenzoyl)piperazin-1-yl)-7-oxo-[1,2,4]triazolo[1,5-a] pyrimidin-4(7H)-yl)-N-(4-(trifluoromethyl)phenyl)acetamide

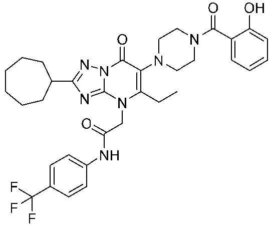

### Example 30

### N-(2-chloro-4-(trifluoromethyl)phenyl)-2-(2-cycloheptyl-5-ethyl-6-(4-(2-hydroxybenzoyl) piperazin-1-yl)-7-oxo-[1,2,4]triazolo[1,5-a]pyrimidin-4(7H)-yl)acetamide

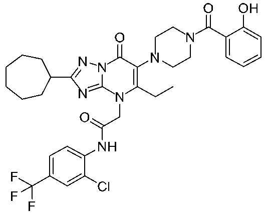

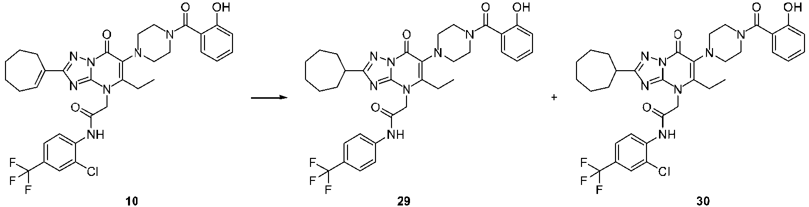

N-(2-chloro-4-(trifluoromethyl)phenyl)-2-(2-(cyclohept-1-en-1-yl)-5-ethyl-6-(4-(2-hydr oxybenzoyl)piperazin-1-yl)-7-oxo-[1,2,4]triazolo[1,5-a]pyrimidin-4(7H)-yl)acetamide 10 (50 mg, 71.62 µmol) was added to methanol (1.5 mL), followed by addition of palladium on carbon (8.70 mg, 71.62 µmol). The reaction was performed at room temperature for 16 hours. The mixture was filtered and concentrated under reduced pressure. The residue was purified by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain 2-(2-cycloheptyl-5-ethyl-6-(4-(2-hydroxybenzoyl)piperazin-1-yl)-7-oxo-[1,2,4]triazolo[1,5-a]pyri midin-4(7H)-yl)-N-(4-(trifluoromethyl)phenyl)acetamide 29 (2.93 mg) in a yield of 6.02% and N-(2-chloro-4-(trifluoromethyl)phenyl)-2-(2-cycloheptyl-5-ethyl-6-(4-(2-hydroxybenzoyl)pipera zin-1-yl)-7-oxo-[1,2,4]triazolo[1,5-a]pyrimidin-4(7H)-yl)acetamide 30 (2.59 mg) in a yield of 4.93%.

Example 29 MS m/z (ESI): 666.2 [M+1]
¹H NMR (400 MHz, DMSO-*d₆*) δ 10.86 (s, 1H), 9.85 (s, 1H), 7.82 - 7.67 (m, 4H), 7.30 - 7.12 (m, 2H), 6.94 - 6.81 (m, 2H), 5.17 (s, 2H), 4.53 (s, 1H), 3.49 (s, 9H), 2.91 (ddd, *J =* 13.8, 11.2, 6.2 Hz, 4H), 2.68 (s, 2H), 1.96 (ddt, J= 9.9, 6.4, 3.9 Hz, 2H), 1.71 (dtt, *J=* 15.4, 6.6, 3.1 Hz, 4H), 1.63 - 1.56 (m, 2H), 1.50 (tdd, *J =* 12.5, 8.4, 5.3 Hz, 4H), 1.16 (t, *J =* 7.4 Hz, 3H).

Example 30 MS m/z (ESI): 700.1 [M+1]
¹H NMR (400 MHz, DMSO-*d₆*) δ 10.34 (s, 1H), 9.84 (s, 1H), 8.10 - 7.90 (m, 2H), 7.72 (dd, *J =* 8.8, 2.1 Hz, 1H), 7.23 (dd, *J =* 7.7, 2.0 Hz, 1H), 7.15 (dd, *J =* 7.5, 1.7 Hz, 1H), 6.89 - 6.80 (m, 2H), 5.29 (s, 2H), 4.53 (s, 1H), 3.51 - 3.40 (m, 4H), 3.02 - 2.87 (m, 4H), 2.68 (s, 2H), 1.98 (ddt, *J=* 14.5, 7.5, 3.1 Hz, 2H), 1.75 (ddd, J= 20.8, 10.5, 6.2 Hz, 4H), 1.61 (d, *J=* 7.6 Hz, 2H), 1.52 (qd, *J =* 10.4, 7.0 Hz, 4H), 1.17 (t, *J=* 7.4 Hz, 3H).

### Example 31

### N-(2-chloro-4-(trifluoromethyl)phenyl)-2-(2-cycloheptyl-5-ethyl-6-(4-(3-hydroxyisonicotinoyl) piperazin-1-yl)-7-oxo-[1,2,4]triazolo[1,5-a]pyrimidin-4(7H)-yl)acetamide

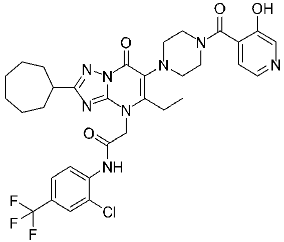

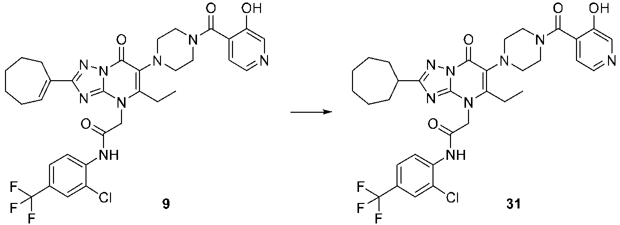

N-(2-chloro-4-(trifluoromethyl)phenyl)-2-(2-(cyclohept-1-en-1-yl)-5-ethyl-6-(4-(3-hydr oxyisonicotinoyl)piperazin-1-yl)-7-oxo-[1,2,4]triazolo[1,5-a]pyrimidin-4(7H)-yl)acetamide 9 (30 mg, 42.91 µmol) was added to methanol (1.5 mL), followed by addition of palladium on carbon (5.21 mg, 42.91 µmol), and the reaction was performed at room temperature for 16 h. The mixture was filtered and concentrated under reduced pressure, and the residue was purified by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(2-cycloheptyl-5-ethyl-6-(4-(3-hydroxyisonicotinoyl)pi perazin-1-yl)-7-oxo-[1,2,4]triazolo[1,5-a]pyrimidin-4(7H)-yl)acetamide **31** (3.49 mg) in a yield of 11.14%.

MS m/z (ESI): 701.1 [M+1]
¹H NMR (400 MHz, DMSO-*d₆*) δ 10.83 (s, 1H), 10.35 (d, *J =* 6.3 Hz, 1H), 8.27 (d, *J =* 2.5 Hz, 1H), 8.23 - 8.16 (m, 1H), 8.03 (d, J= 8.7 Hz, 1H), 7.97 (d, J= 2.2 Hz, 1H), 7.72 *(dd, J=* 8.8, 2.1 Hz, 1H), 7.38 (s, 1H), 5.29 (s, 2H), 4.52 (d, *J=* 12.5 Hz, 1H), 3.46 (d, *J=* 10.9 Hz, 2H), 3.34 - 3.22 (m, 2H), 2.92 (ddd, *J =* 13.9, 9.6, 4.7 Hz, 4H), 2.79 (d, *J=* 11.6 Hz, 1H), 2.62 (d, *J =* 11.2 Hz, 1H), 1.99 (dd, *J=* 14.1, 7.0 Hz, 2H), 1.74 (dt, *J=* 10.9, 7.8 Hz, 4H), 1.63 - 1.44 (m, 6H), 1.17 (t, *J =* 7.5 Hz, 3H).

### Example 32

### N-(2-chloro-4-(trifluoromethyl)phenyl)-2-(6-(4-(5-hydroxy-6-methylpyrimidine-4-carbonyl)piper azin-1-yl)-5-methyl-7-oxo-2-(5-oxo-1,3a,4,5,6,6a-hexahydropentalen-2-yl)-[1,2,4]triazolo[1,5-a] pyrimidin-4(7H)-yl)acetamide

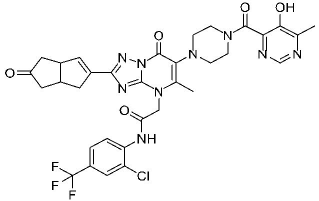

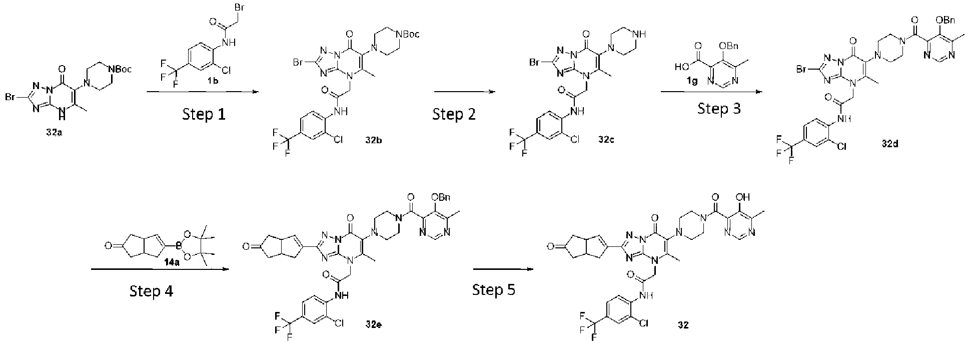

### Step 1

### tert-butyl 4-(2-bromo-4-(2-((2-chloro-4-(trifluoromethyl)phenyl)amino)-2-oxoethyl)-5-methyl -7-oxo-4,7-dihydro-[1,2,4]triazolo[1,5-a]pyrimidin-6-yl)piperazine-1-carboxylate

tert-butyl 4-(2-bromo-5-methyl-7-oxo-4,7-dihydro-[1,2,4]triazolo[1,5-a]pyrimidin-6-yl) piperazine-1-carboxylate **32a** (1 g, 2.42 mmol, prepared according to published patent WO2022249060 A1) and 2-bromo-N-(2-chloro-4-(trifluoromethyl)phenyl)acetamide 1b (995.61 mg, 3.15 mmol) were added to N,N-dimethylformamide (10 mL), followed by addition of N,N-diisopropylethylamine (938.18 mg, 7.26 mmol), the temperature was raised to 50 °C, and the reaction was performed for 4 h. The reaction mixture was extracted with ethyl acetate (30 mL ×2), the aqueous layer was separated, and the combined organic phase was washed with saturated sodium chloride solution (30 mL ×2), dried over anhydrous sodium sulfate, filtered, and concentrated under reduced pressure. The resulting residue was purified by silica gel column chromatography (eluent: System B) to obtain tert-butyl 4-(2-bromo-4-(2-((2-chloro-4-(trifluoromethyl)phenyl)amino)-2-oxoethyl)-5-methyl-7-oxo-4,7-dihydro-[1,2,4]triazolo[1,5-a] pyrimidin-6-yl)piperazine-1-carboxylate **32b** (1.1 g) in a yield of 70.06%.

MS m/z (ESI): 547.7 [M-100+1]

### Step 2

### 2-(2-bromo-5-methyl-7-oxo-6-(piperazin-1-yl)-[1,2,4]triazolo[1,5-a]pyrimidin-4(7H)-yl)-N-(2-chloro-4-(trifluoromethyl)phenyl)acetamide

tert-butyl 4-(2-bromo-4-(2-((2-chloro-4-(trifluoromethyl)phenyl)amino)-2-oxoethyl)-5-methyl-7-oxo-4,7-dihydro-[1,2,4]triazolo[1,5-a]pyrimidin-6-yl)piperazine-1-carboxylate 32b (260 mg, 400.70 µmol) was added to dichloromethane (5 mL), followed by addition of trifluoroacetic acid (1 mL), and the reaction was performed at room temperature for 1 h. The mixture was concentrated under reduced pressure to obtain 2-(2-bromo-5-methyl-7-oxo-6-(piperazin-1-yl)-[1,2,4]triazolo[1,5-a]pyrimidin-4(7H)-yl)-N-(2-chloro-4-(trifluoromethyl)phen yl)acetamide **32c** (210 mg) in a yield of 95.51%.

MS m/z (ESI): 548.0 [M+1]

### Step 3

### 2-(6-(4-(5-(benzyloxy)-6-methylpyrimidine-4-carbonyl)piperazin-1-yl)-2-bromo-5-methyl-7-oxo -[1,2,4]triazolo[1,5-a]pyrimidin-4(7H)-yl)-N-(2-chloro-4-(trifluoromethyl)phenyl)acetamide

2-(2-bromo-5-methyl-7-oxo-6-(piperazin-1-yl)-[1,2,4]triazolo[1,5-a]pyrimidin-4(7H)-yl )-N-(2-chloro-4-(trifluoromethyl)phenyl)acetamide **32c** (210 mg, 382.69 µmol), 5-(benzyloxy)-6-methylpyrimidine-4-carboxylic acid **1g** (121.51 mg, 497.50 µmol), 1-(3-dimethylaminopropyl)-3-ethylcarbodiimide hydrochloride (110.04 mg, 574.04 µmol), 1-hydroxybenzotriazole (77.56 mg, 574.04 µmol), and N,N-diisopropylethylamine (247.30 mg, 1.91 mmol) were added to N,N-dimethylformamide (2 mL), and the reaction was performed at room temperature for 4 h. Water (20 mL) was added to the reaction mixture, which was extracted with ethyl acetate (30 mL ×2), the aqueous layer was separated, and the combined organic phase was washed with saturated sodium chloride solution (30 mL ×2), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-2-bromo-5-methyl-7-oxo-[1,2,4]triazolo[1,5-a]pyri midin-4(7H)-yl)-N-(2-chloro-4-(trifluoromethyl)phenyl)acetamide **32d** (200 mg) in a yield of 67.44%.

MS m/z (ESI): 775.1 [M+1]

### Step 4

### 2-(6-(4-(5-(benzyloxy)-6-methylpyrimidine-4-carbonyl)piperazin-1-yl)-5-methyl-7-oxo-2-(5-oxo -1,3a,4,5,6,6a-hexahydropentalen-2-yl)-[1,2,4]triazolo[1,5-a]pyrimidin-4(7H)-yl)-N-(2-chloro-4-( trifluoromethyl)phenyl)acetamide

At room temperature, 2-(6-(4-(5-(benzyloxy)-6-methylpyrimidine-4-carbonyl) piperazin-1-yl)-2-bromo-5-methyl-7-oxo-[1,2,4]triazolo[1,5-a]pyrimidin-4(7H)-yl)-N-(2-chloro-4 -(trifluoromethyl)phenyl)acetamide **32d** (55 mg, 70.97 µmol), 5-(4,4,5,5-tetramethyl-1,3,2-dioxaborolan-2-yl)-3,3a,4,6a-tetrahydro-1H-cyclopenta[c]furan-2-on e **14a** (21.13 mg, 85.16 µmol), [1,1'-bis(diphenylphosphino)ferrocene]palladium(II) dichloride dichloromethane complex (5.75 mg, 7.10 µmol), and sodium carbonate (22.57 mg, 212.91 µmol) were added to 1,4-dioxane (1 mL). The mixture was subject to argon replacement four times, was heated to 80 °C, and the reaction was performed for 1.5 hours. Water (20 mL) was added to the reaction mixture, and the mixture was extracted with ethyl acetate (40 mL ×3). The combined organic phase was washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-5-methyl-7-oxo-2-(5-oxo -1,3a,4,5,6,6a-hexahydropentalen-2-yl)-[1,2,4]triazolo[1,5-a]pyrimidin-4(7H)-yl)-N-(2-chloro-4-( trifluoromethyl)phenyl)acetamide **32e** (36 mg) in a yield of 62.15%.

MS m/z (ESI): 815.7 [M+1]

### Step 5

### N-(2-chloro-4-(trifluoromethyl)phenyl)-2-(6-(4-(5-hydroxy-6-methylpyrimidine-4-carbonyl)piper azin-1-yl)-5-methyl-7-oxo-2-(5-oxo-1,3a,4,5,6,6a-hexahydropentalen-2-yl)-[1,2,4]triazolo[1,5-a] pyrimidin-4(7H)-yl)acetamide

2-(6-(4-(5-(benzyloxy)-6-methylpyrimidine-4-carbonyl)piperazin-1-yl)-5-methyl-7-oxo -2-(5-oxo-1,3a,4,5,6,6a-hexahydropentalen-2-yl)-[1,2,4]triazolo[1,5-a]pyrimidin-4(7H)-yl)-N-(2-chloro-4-(trifluoromethyl)phenyl)acetamide **32e** (36 mg, 44.11 µmol) was added to dichloromethane (1 mL). Boron trichloride (0.8 mL) was added at 0 °C, and the reaction was performed at 0 C for 1 hour. Methanol was added to quench the reaction. The mixture was concentrated under reduced pressure, and the residue was purified by preparative liquid chromatography (column: AKZONOBEL Kromasil; 250×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(6-(4-(5-hydroxy-6-methylpyrimidine-4-carbonyl)piper azin-1-yl)-5-methyl-7-oxo-2-(5-oxo-1,3a,4,5,6,6a-hexahydropentalen-2-yl)-[1,2,4]triazolo[1,5-a] pyrimidin-4(7H)-yl)acetamide 32 (3.00 mg) in a yield of 9.13%.

MS m/z (ESI): 725.7 [M+1]
¹H NMR (400 MHz, DMSO-*d₆*) δ 10.38 (s, 1H), 10.23 (s, 1H), 8.58 (s, 1H), 8.07 (d,*J* = 8.6 Hz, 1H), 7.97 (d, *J=* 2.1 Hz, 1H), 7.71 (dd, *J =* 8.7, 2.1 Hz, 1H), 6.53 (d, *J =* 2.0 Hz, 1H), 5.33 (s, 2H), 4.50 (d, *J =* 12.6 Hz, 1H), 3.59 (d, *J =* 7.3 Hz, 1H), 3.48 (d, *J =* 11.2 Hz, 3H), 3.27 (d, *J=* 12.1 Hz, 2H), 3.16 - 2.95 (m, 4H), 2.79 (d, *J=* 11.3 Hz, 1H), 2.65 (d, *J=* 14.3 Hz, 2H), 2.58 (s, 3H), 2.44 (s, 3H), 2.26 (dd, *J =* 18.3, 3.0 Hz, 1H), 1.95 (dd, *J =* 19.0, 6.9 Hz, 1H), 1.21 (d, *J=* 21.3 Hz, 1H).

### Example 33

### N-(2-chloro-4-(trifluoromethyl)phenyl)-2-(2-(cyclobutylidenemethyl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl) acetamide

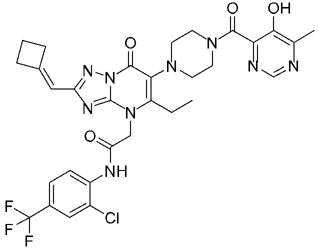

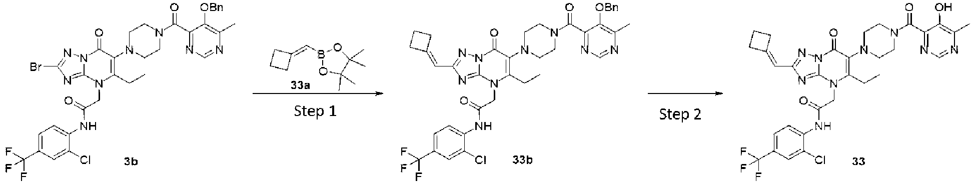

### Step 1

### 2-(6-(4-(5-(benzyloxy)-6-methylpyrimidine-4-carbonyl)piperazin-1-yl)-2-(cyclobutylidenemethyl )-5-ethyl-7-oxo-[1,2,4]triazolo[1,5-a]pyrimidin-4(7H)-yl)-N-(2-chloro-4-(trifluoromethyl)phenyl) acetamide

At room temperature, 2-(6-(4-(5-(benzyloxy)-6-methylpyrimidine-4-carbonyl) piperazin-1-yl)-2-bromo-5-ethyl-7-oxo-[1,2,4]triazolo[1,5-a]pyrimidin-4(7H)-yl)-N-(2-chloro-4-( trifluoromethyl)phenyl)acetamide 3b (50 mg, 63.37 µmol), 2-(cyclobutylidenemethyl)-4,4,5,5-tetramethyl-1,3,2-dioxaborolane 33a (14.76 mg, 76.05 µmol, prepared according to published patent WO2021007477 A1), [1,1'-bis(diphenylphosphino)ferrocene]palladium(II) dichloride dichloromethane complex (5.14 mg, 6.34 µmol), and sodium carbonate (20.15 mg, 190.11 µmol) were added to 1,4-dioxane (1 mL). The mixture was subject to argon replacement four times, was heated to 80 °C, and the reaction was performed for 1.5 hours. Water (20 mL) was added to the reaction mixture, and the mixture was extracted with ethyl acetate (40 mL×3). The combined organic phase was washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-2-(cyclobutylidenemethyl )-5-ethyl-7-oxo-[1,2,4]triazolo[1,5-a]pyrimidin-4(7H)-yl)-N-(2-chloro-4-(trifluoromethyl)phenyl) acetamide 33b (35 mg) in a yield of 71.15%.

MS m/z (ESI): 776.2 [M+1]

### Step 2

### N-(2-chloro-4-(trifluoromethyl)phenyl)-2-(2-(cyclobutylidenemethyl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl) acetamide

2-(6-(4-(5-(benzyloxy)-6-methylpyrimidine-4-carbonyl)piperazin-1-yl)-2-(cyclobutylid enemethyl)-5-ethyl-7-oxo-[1,2,4]triazolo[1,5-a]pyrimidin-4(7H)-yl)-N-(2-chloro-4-(trifluorometh yl)phenyl)acetamide 33b (35 mg, 45.09 µmol) was added to dichloromethane (2.5 mL). Boron trichloride (0.8 mL) was added at 0 °C, and the reaction was performed at 0 C for 1 hour. Methanol was added to quench the reaction. The mixture was concentrated under reduced pressure, and the residue was purified by preparative liquid chromatography (column: AKZONOBEL Kromasil; 250×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl) -2-(2-(cyclobutylidenemethyl)-5-ethyl-6-(4-(5-hydroxy-6-methylpyrimidine-4-carbonyl)piperazi n-1-yl)-7-oxo-[1,2,4]triazolo[1,5-a]pyrimidin-4(7H)-yl)acetamide 33 (2.75 mg) in a yield of 8.71%.

MS m/z (ESI): 686.2 [M+1]
¹H NMR (400 MHz, DMSO-*d₆*) δ 10.36 (s, 1H), 10.23 (s, 1H), 8.58 (s, 1H), 8.04 (d, *J =* 8.6 Hz, 1H), 7.96 (d, *J =* 2.1 Hz, 1H), 7.76 - 7.64 (m, 1H), 6.14 - 6.02 (m, 1H), 5.28 (s, 2H), 4.52 (d, *J=* 12.4 Hz, 1H), 3.24 (t, *J=* 12.4 Hz, 1H), 3.08 (d, *J =* 8.4 Hz, 2H), 2.99 (s, 3H), 2.91 - 2.78 (m, 3H), 2.63 (d, *J=* 10.7 Hz, 1H), 2.44 (s, 3H), 2.01 (p, *J=* 7.9 Hz, 2H), 1.18 (t, *J=* 7.4 Hz, 3H).

### Example 34

### N-(2-chloro-4-(trifluoromethyl)phenyl)-2-(5-ethyl-6-(4-(5-hydroxy-6-methylpyrimidine-4-carbon yl)piperazin-1-yl)-2-(oxetan-3-ylidenemethyl)-7-oxo-[1,2,4]triazolo[1,5-a]pyrimidin-4(7H)-yl) acetamide

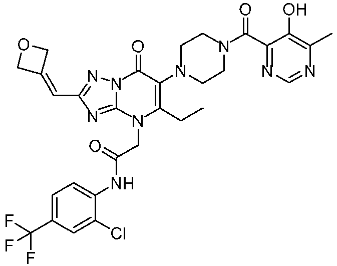

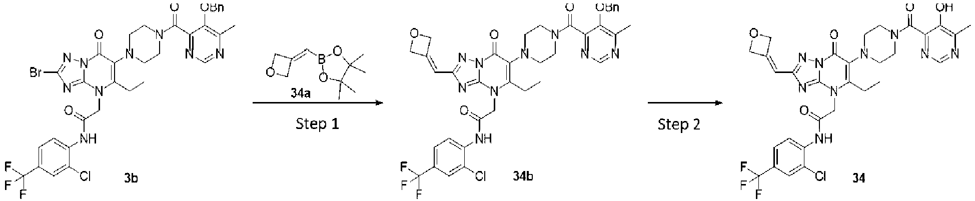

### Step 1

### 2-(6-(4-(5-(benzyloxy)-6-methylpyrimidine-4-carbonyl)piperazin-1-yl)-5-ethyl-2-(oxetan-3-ylide nemethyl)-7-oxo-[1,2,4]triazolo[1,5-a]pyrimidin-4(7H)-yl)-N-(2-chloro-4-(trifluoromethyl)pheny l)acetamide

At room temperature, 2-(6-(4-(5-(benzyloxy)-6-methylpyrimidine-4-carbonyl)piperazin -1-yl)-2-bromo-5-ethyl-7-oxo-[1,2,4]triazolo[1,5-a]pyrimidin-4(7H)-yl)-N-(2-chloro-4-(trifluoro methyl)phenyl)acetamide 3b (50 mg, 63.37 µmol), 4,4,5,5-tetramethyl-2-(oxetan-3-ylidenemethyl)-1,3,2-dioxaborolane 34a (14.91 mg, 76.05 µmol, prepared according to published patent WO2021076938), [1,1'-bis(diphenylphosphino)ferrocene]palladium(II) dichloride dichloromethane complex (5.14 mg, 6.34 µmol), and sodium carbonate (20.15 mg, 190.11 µmol) were added to 1,4-dioxane (1 mL). The mixture was subject to argon replacement four times, then was heated to 80 C and the reaction was performed for 1.5 hours. Water (20 mL) was added to the reaction mixture, and the mixture was extracted with ethyl acetate (40 mL × 3). The combined organic phases were washed with saturated sodium chloride solution (20 mL), dried over anhydrous sodium sulfate, filtered, and concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 2-(6-(4-(5-(benzyloxy)-6-methylpyrimidine-4-carbonyl)piperazin-1-yl)-5-ethyl-2-(oxetan-3-ylide nemethyl)-7-oxo-[1,2,4]triazolo[1,5-a]pyrimidin-4(7H)-yl)-N-(2-chloro-4-(trifluoromethyl)pheny l)acetamide 34b (40 mg). Yield: 81.11 %.

MS m/z (ESI): 778.2 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-2-(oxetan-3-ylidenemethyl)-7-oxo-[1,2,4]triazolo[1,5-a]pyrimidin-4(7H)-yl) acetamide

2-(6-(4-(5-(benzyloxy)-6-methylpyrimidine-4-carbonyl)piperazin-1-yl)-5-ethyl-2-(oxeta n-3-ylidenemethyl)-7-oxo-[1,2,4]triazolo[1,5-a]pyrimidin-4(7H)-yl)-N-(2-chloro-4-(trifluorometh yl)phenyl)acetamide 34b (35 mg, 44.98 µmol) was dissolved in dichloromethane (1.5 mL), followed by dropwise addition of trifluoroacetic acid (1.5 mL). The reaction was performed at room temperature for 48 hours. The mixture was concentrated under reduced pressure, and the residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbonyl)piperazin-1-yl)-2-(oxetan-3-ylidenemethy l)-7-oxo-[1,2,4]triazolo[1,5-a]pyrimidin-4(7H)-yl)acetamide 34 (4.07 mg). Yield: 13.15%.

MS m/z (ESI): 688.7 [M+1]
¹H NMR (400 MHz, DMSO-*d₆*) δ 10.66 (d, *J =* 2.4 Hz, 1H), 10.23 (s, 1H), 8.58 (s, 1H), 8.16 (d, *J=* 8.6 Hz, 1H), 8.02 (d, *J=* 2.1 Hz, 1H), 7.77 (dd, *J =* 8.7, 2.1 Hz, 1H), 6.98 (q, *J=* 1.8 Hz, 1H), 5.71 (s, 1H), 5.54 (s, 2H), 5.35 (s, 2H), 4.53 (d, *J =* 19.6 Hz, 3H), 3.29 (t, *J =* 11.8 Hz, 2H), 3.10 - 2.98 (m, 2H), 2.88 (d, *J =* 11.3 Hz, 1H), 2.70 (d, *J =* 11.1 Hz, 1H), 2.45 (s, 3H), 1.21 (t, *J =* 7.4 Hz, 3H).

### Example 35

### 2-(2-(benzofuran-3-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl) acetamide

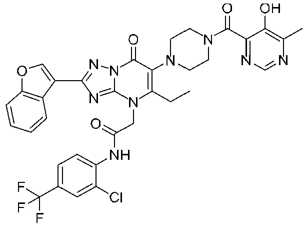

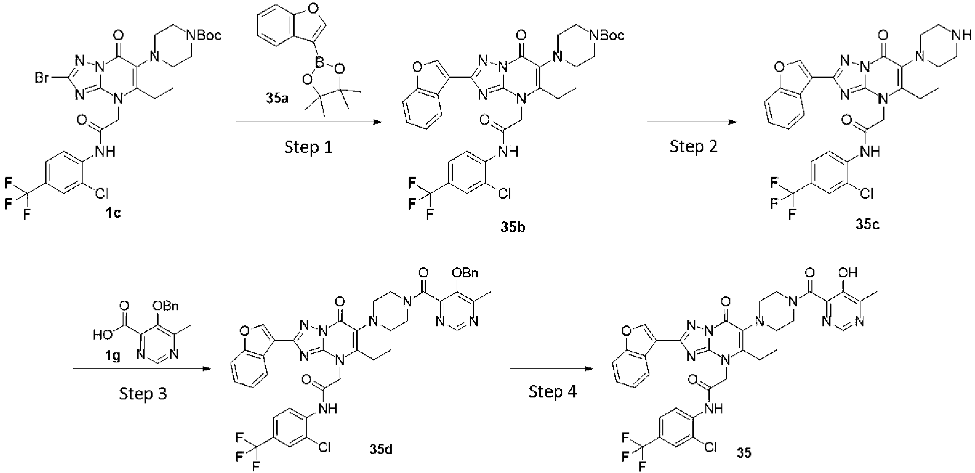

### Step 1

### tert-butyl 4-(2-(benzofuran-3-yl)-4-(2-((2-chloro-4-(trifluoromethyl)phenyl)amino)-2-oxoethyl) -5-ethyl-7-oxo-4,7-dihydro-[1,2,4]triazolo[1,5-a]pyrimidin-6-yl)piperazine-1-carboxylate

At room temperature, tert-butyl 4-(2-bromo-4-(2-((2-chloro-4-(trifluoromethyl) phenyl)amino)-2-oxoethyl)-5-ethyl-7-oxo-4,7-dihydro-[1,2,4]triazolo[1,5-a]pyrimidin-6-yl)piper azine-1-carboxylate 1c (70 mg, 105.60 µmol), 2-(benzofuran-3-yl)-4,4,5,5-tetramethyl-1,3,2-dioxaborolane 35a (25.78 mg, 105.60 µmol, commercially available), [1,1'-bis(diphenylphosphino)ferrocene]palladium(II) dichloride dichloromethane complex (8.56 mg, 10.56 µmol), and sodium carbonate (33.58 mg, 316.80 µmol) were added to 1,4-dioxane (1 mL). The mixture was subject to argon replacement four times, then heated to 80 °C and reacted for 1.5 h. Water (20 mL) was added to the reaction mixture, which was extracted with ethyl acetate (40 mL ×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(2-(benzofuran-3-yl)-4-(2-((2-chloro-4-(trifluoromethyl)phenyl)amino)-2-oxoethyl)-5-ethyl-7-oxo-4,7-dihydro-[1,2,4]triazolo[1,5-a]pyri midin-6-yl)piperazine-1-carboxylate 35b (60 mg). Yield: 81.16%.

MS m/z (ESI): 644.2 [M-56+1]

### Step 2

### 2-(2-(benzofuran-3-yl)-5-ethyl-7-oxo-6-(piperazin-1-yl)-[1,2,4]triazolo[1,5-a]pyrimidin-4(7H) -yl)-N-(2-chloro-4-(trifluoromethyl)phenyl)acetamide

Tert-Butyl 4-(2-(benzofuran-3-yl)-4-(2-((2-chloro-4-(trifluoromethyl)phenyl)amino)-2-oxoethyl)-5-ethyl-7-oxo-4,7-dihydro-[1,2,4]triazolo[1,5-a]pyrimidin-6-yl)piperazine-1-carboxyla te 35b (60 mg, 85.70 µmol) was added to dichloromethane (3 mL), followed by addition of trifluoroacetic acid (0.3 mL). The reaction was continued for 1 h, then concentrated under reduced pressure to obtain 2-(2-(benzofuran-3-yl)-5-ethyl-7-oxo-6-(piperazin-1-yl)-[1,2,4]triazolo[1,5-a]pyrimidin-4(7H)-yl)-N-(2-chloro-4-(trifluoromethyl)phenyl)acetamide 35c (50 mg). Yield: 97.24%. The product was used directly in the next step.

MS m/z (M+1): 600.1 [M+1]

### Step 3

### 2-(2-(benzofuran-3-yl)-6-(4-(5-(benzyloxy)-6-methylpyrimidine-4-carbonyl)piperazin-1-yl)-5-ethyl-7-oxo-[1,2,4]triazolo[1,5-a]pyrimidin-4(7H)-yl)-N-(2-chloro-4-(trifluoromethyl)phenyl)ace tamide

2-(2-(benzofuran-3-yl)-5-ethyl-7-oxo-6-(piperazin-1-yl)-[1,2,4]triazolo[1,5-a]pyrimidin -4(7H)-yl)-N-(2-chloro-4-(trifluoromethyl)phenyl)acetamide 35c (50 mg, 83.33 µmol), 5-(benzyloxy)-6-methylpyrimidine-4-carboxylic acid 1g (26.46 mg, 108.34 µmol), 1-(3-dimethylaminopropyl)-3-ethylcarbodiimide hydrochloride (23.96 mg, 125.00 µmol), 1-hydroxybenzotriazole (16.89 mg, 125.00 µmol), and N,N-diisopropylethylamine (53.85 mg, 416.67 µmol) were added to N,N-dimethylformamide (1.5 mL). The reaction was performed at room temperature for 16 h. Water (20 mL) was added to the reaction mixture, which was extracted with ethyl acetate (30 mL ×2). The aqueous layer was separated, and the combined organic phases were washed with saturated sodium chloride solution (30 mL×2), dried over anhydrous sodium sulfate, filtered, and concentrated under reduced pressure. The resulting residue was purified by silica gel column chromatography (eluent: System B) to obtain 2-(2-(benzofuran-3-yl)-6-(4-(5-(benzyloxy)-6-methylpyrimidine-4-carbonyl)piperazin-1-yl)-5-eth yl-7-oxo-[1,2,4]triazolo[1,5-a]pyrimidin-4(7H)-yl)-N-(2-chloro-4-(trifluoromethyl)phenyl)aceta mide 35d (40 mg). Yield: 58.09%.

MS m/z (ESI): 826.3 [M+1]

### Step 4

### 2-(2-(benzofuran-3-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl) acetamide

2-(2-(benzofuran-3-yl)-6-(4-(5-(benzyloxy)-6-methylpyrimidine-4-carbonyl)piperazin-1 -yl)-5-ethyl-7-oxo-[1,2,4]triazolo[1,5-a]pyrimidin-4(7H)-yl)-N-(2-chloro-4-(trifluoromethyl)phen yl)acetamide 35d (40 mg, 48.41 µmol) was added to dichloromethane (2.5 mL). Boron trichloride (1 mL) was added at 0 °C, and the reaction was performed at 0 C for 1 h. The reaction was quenched by addition of methanol, then concentrated under reduced pressure. The residue was purified by preparative liquid chromatography (column: AKZONOBEL Kromasil; 250 ×21.2 mm I.D.; 5 µm, 20 mL/min; mobile phase A: 0.05% TFA + H₂O, mobile phase B: CH₃CN) to obtain 2-(2-(benzofuran-3-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamid e 35 (2.83 mg). Yield: 7.60%.

MS m/z (ESI): 736.0 [M+1]
¹H NMR (400 MHz, DMSO-*d₆*) δ 10.46 (s, 1H), 10.24 (s, 1H), 8.71 (s, 1H), 8.59 (s, 1H), 8.34 - 8.26 (m, 1H), 8.08 (d, *J =* 8.5 Hz, 1H), 7.98 (d, *J=* 2.1 Hz, 1H), 7.75 - 7.68 (m, 2H), 7.42 (dtd, *J =* 19.0, 7.4, 1.4 Hz, 2H), 5.42 (s, 2H), 4.55 (d, *J =* 12.5 Hz, 1H), 3.53 (t, *J =* 11.1 Hz, 3H), 3.04 (d, *J= 9.7* Hz, 4H), 2.86 (d, *J=* 11.4 Hz, 1H), 2.70 (s, 1H), 2.45 (s, 3H), 1.23 (t, *J=* 7.4 Hz, 3H).

### Example 36

### (E)-N-(2-chloro-4-(trifluoromethyl)phenyl)-2-(2-(2-cyclopropylprop-1-en-1-yl)-5-ethyl-6-(4-(5-h ydroxy-6-methylpyrimidine-4-carbonyl)piperazin-1-yl)-7-oxo-[1,2,4]triazolo[1,5-a]pyrimidin -4(7H)-yl)acetamide

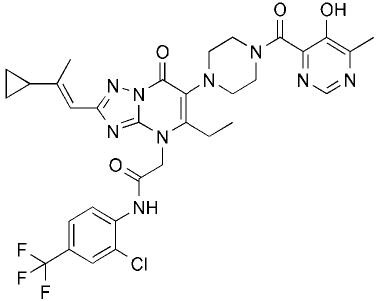

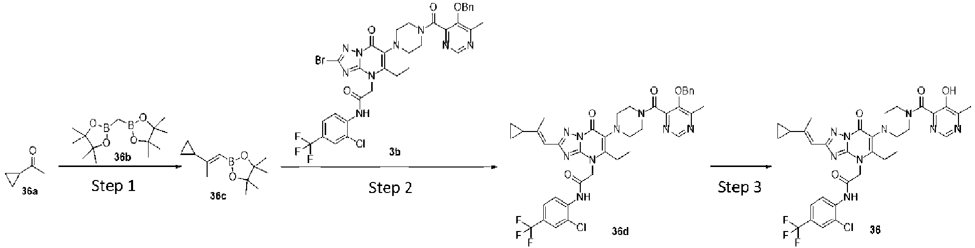

### Step 1

### (E)-2-(2-cyclopropylprop-1-en-1-yl)-4,4,5,5-tetramethyl-1,3,2-dioxaborolane

2,2,6,6-Tetramethylpiperidine (379.53 mg, 2.69 mmol) was added to tetrahydrofuran (4.5 mL), and n-butyllithium (2.5 M n-hexane solution) (2.5 M, 1.07 mL) was added dropwise at -30 °C. The reaction was continued at -30 °C for 30 minutes, then cooled to -78 °C, and a solution of bis(4,4,5,5-tetramethyl-1,3,2-dioxaborolan-2-yl)methane 36b (600 mg, 2.24 mmol, commercially available) in tetrahydrofuran (4.5 mL) was added dropwise. After 30 minutes, a solution of 1-cyclopropylethan-1-one 36a (188.34 mg, 2.24 mmol, commercially available) in tetrahydrofuran (4.5 mL) was added. The reaction was heated to room temperature and performed for 16 hours. Water (20 mL) was added to the reaction mixture, which was extracted with petroleum ether (30 mL ×2). The aqueous layer was separated, and the combined organic phase was washed with saturated sodium chloride solution (30 mL ×2), dried over anhydrous sodium sulfate, filtered, and concentrated under reduced pressure. The resulting residue was purified by silica gel column chromatography (eluent: System A) to obtain (E)-2-(2-cyclopropylprop-1-en-1-yl)-4,4,5,5-tetramethyl-1,3,2-dioxaborolane 36c (160 mg) in a yield of 34.34%.

### Step 2

### (E)-2-(6-(4-(5-(benzyloxy)-6-methylpyrimidine-4-carbonyl)piperazin-1-yl)-2-(2-cyclopropylprop -1-en-1-yl)-5-ethyl-7-oxo-[1,2,4]triazolo[1,5-a]pyrimidin-4(7H)-yl)-N-(2-chloro-4-(trifluoromethyl)phenyl)acetamide

At room temperature, 2-(6-(4-(5-(benzyloxy)-6-methylpyrimidine-4-carbonyl) piperazin-1-yl)-2-bromo-5-ethyl-7-oxo-[1,2,4]triazolo[1,5-a]pyrimidin-4(7H)-yl)-N-(2-chloro-4-( trifluoromethyl)phenyl)acetamide 3b (50 mg, 63.37 µmol), (E)-2-(2-cyclopropylprop-1-en-1-yl)-4,4,5,5-tetramethyl-1,3,2-dioxaborolane 36c (15.83 mg, 76.05 µmol), [1,1'-bis(diphenylphosphino)ferrocene]palladium(II) dichloride dichloromethane complex (5.14 mg, 6.34 µmol), and sodium carbonate (20.15 mg, 190.11 µmol) were added to 1,4-dioxane (1 mL). The mixture was subject to argon replacement four times, and the reaction was heated to 80 °C and performed for 1.5 hours. Water (20 mL) was added to the reaction mixture, which was extracted with ethyl acetate (40 mL×3). The combined organic phase was washed with saturated sodium chloride solution (20 mL), dried over anhydrous sodium sulfate, filtered, and concentrated under reduced pressure. The resulting residue was purified by silica gel column chromatography (eluent: System B) to obtain (E)-2-(6-(4-(5-(benzyloxy) -6-methylpyrimidine-4-carbonyl)piperazin-1-yl)-2-(2-cyclopropylprop-1-en-1-yl)-5-ethyl-7-oxo-[ 1,2,4]triazolo[1,5-a]pyrimidin-4(7H)-yl)-N-(2-chloro-4-(trifluoromethyl)phenyl)acetamide 36d (30 mg) in a yield of 59.91%.

MS m/z (ESI): 790.3 [M+1]

### Step 3

### (E)-N-(2-chloro-4-(trifluoromethyl)phenyl)-2-(2-(2-cyclopropylprop-1-en-1-yl)-5-ethyl-6-(4-(5-h ydroxy-6-methylpyrimidine-4-carbonyl)piperazin-1-yl)-7-oxo-[1,2,4]triazolo[1,5-a]pyrimidin-4(7H)-yl)acetamide

(E)-2-(6-(4-(5-(benzyloxy)-6-methylpyrimidine-4-carbonyl)piperazin-1-yl)-2-(2-cyclop ropylprop-1-en-1-yl)-5-ethyl-7-oxo-[1,2,4]triazolo[1,5-a]pyrimidin-4(7H)-yl)-N-(2-chloro-4-(trifl uoromethyl)phenyl)acetamide 36d (30 mg, 37.96 µmol) was added to dichloromethane (3 mL), and boron trichloride (1 mL) was added at 0 °C. The reaction was performed at 0 °C for 1 hour. Methanol was added to quench the reaction, and the mixture was concentrated under reduced pressure. The residue was purified by preparative liquid chromatography (separation column: AKZONOBEL Kromasil; 250×21.2 mm I.D.; 5 µm, 20 mL/min; mobile phase A: 0.05% TFA+H₂O, mobile phase B: CH₃CN) to obtain (E)-N-(2-chloro-4-(trifluoromethyl)phenyl)-2-(2-(2-cyclopropylprop-1-en-1-yl)-5-ethyl-6-(4-(5-hydroxy-6-methylp yrimidine-4-carbonyl)piperazin-1-yl)-7-oxo-[1,2,4]triazolo[1,5-a]pyrimidin-4(7H)-yl)acetamide 36 (3.46 mg) in a yield of 12.24%.

MS m/z (ESI): 699.7 [M+1]
¹H NMR (400 MHz, DMSO-*d₆*) δ 10.38 - 10.29 (m, 1H), 10.24 (s, 1H), 8.58 (s, 1H), 8.05 (t, *J =* 8.5 Hz, 1H), 7.96 (d, *J =* 2.2 Hz, 1H), 7.73 - 7.65 (m, 1H), 6.21 (d, *J =* 13.3 Hz, 1H), 5.30 (s, 2H), 4.52 (d, *J=* 12.4 Hz, 1H), 3.50 (d, *J=* 10.7 Hz, 3H), 3.40 (s, 1H), 3.33 - 3.18 (m, 2H), 2.98 (d, *J =* 14.1 Hz, 3H), 2.82 (d, *J=* 11.4 Hz, 1H), 2.64 (d, *J =* 10.9 Hz, 1H), 2.45 (s, 3H), 2.00 *(d, J=* 1.2 Hz, 1H), 1.68 (ddd, *J=* 13.3, 8.9, 5.9 Hz, 1H), 1.63 (s, 1H), 1.56 (d, *J=* 1.4 Hz, 1H), 1.18 (td, *J =* 7.6, 3.6 Hz, 3H), 0.77 - 0.58 (m, 3H).

### Example 37

### (E)-N-(2-chloro-4-(trifluoromethyl)phenyl)-2-(5-ethyl-6-(4-(5-hydroxy-6-methylpyrimidine-4-ca rbonyl)piperazin-1-yl)-7-oxo-2-(2-(pyridin-2-yl)vinyl)-[1,2,4]triazolo[1,5-a]pyrimidin-4(7H)-yl)a cetamide

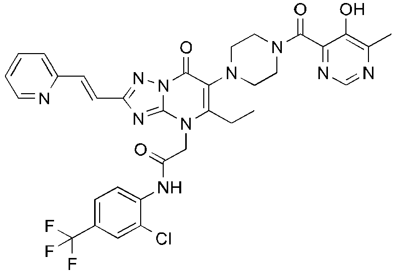

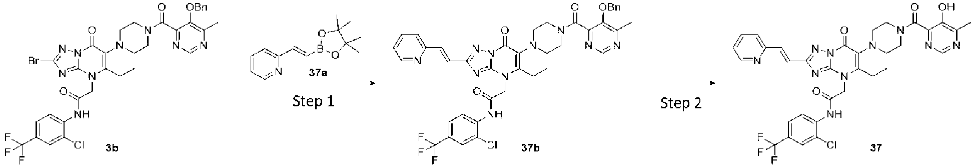

### Step 1

### (E)-2-(6-(4-(5-(benzyloxy)-6-methylpyrimidine-4-carbonyl)piperazin-1-yl)-5-ethyl-7-oxo-2-(2-(p yridin-2-yl)vinyl)-[1,2,4]triazolo[1,5-a]pyrimidin-4(7H)-yl)-N-(2-chloro-4-(trifluoromethyl) phenyl)acetamide

At room temperature, 2-(6-(4-(5-(benzyloxy)-6-methylpyrimidine-4-carbonyl) piperazin-1-yl)-2-bromo-5-ethyl-7-oxo-[1,2,4]triazolo[1,5-a]pyrimidin-4(7H)-yl)-N-(2-chloro-4-( trifluoromethyl)phenyl)acetamide 3b (50 mg, 63.37 µmol), (E)-2-(2-(4,4,5,5-tetramethyl-1,3,2-dioxaborolan-2-yl)vinyl)pyridine 37a (17.57 mg, 76.05 µmol, prepared according to a known method "European Journal of Organic Chemistry (2019), 2019(33), 5624-5635"), [1,1'-bis(diphenylphosphino)ferrocene]palladium(II) dichloride dichloromethane complex (5.14 mg, 6.34 µmol), and sodium carbonate (20.15 mg, 190.11 µmol) were added to 1,4-dioxane (1 mL). The mixture was subject to argon replacement four times, heated to 80 °C, and the reaction was performed for 1.5 hours. Water (20 mL) was added to the reaction mixture, and the mixture was extracted with ethyl acetate (40 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain (E)-2-(6-(4-(5-(benzyloxy)-6-methylpyrimidine-4-carbonyl)piperazin-1-yl)-5-ethyl-7-oxo-2-(2-(p yridin-2-yl)vinyl)-[1,2,4]triazolo[1,5-a]pyrimidin-4(7H)-yl)-N-(2-chloro-4-(trifluoromethyl)phen yl)acetamide 37b (40 mg). Yield: 77.62%.

MS m/z (ESI): 813.3 [M+1]

### Step 2

### (E)-N-(2-chloro-4-(trifluoromethyl)phenyl)-2-(5-ethyl-6-(4-(5-hydroxy-6-methylpyrimidine-4-ca rbonyl)piperazin-1-yl)-7-oxo-2-(2-(pyridin-2-yl)vinyl)-[1,2,4]triazolo[1,5-a]pyrimidin-4(7H)-yl) acetamide

(E)-2-(6-(4-(5-(benzyloxy)-6-methylpyrimidine-4-carbonyl)piperazin-1-yl)-5-ethyl-7-o xo-2-(2-(pyridin-2-yl)vinyl)-[1,2,4]triazolo[1,5-a]pyrimidin-4(7H)-yl)-N-(2-chloro-4-(trifluorom ethyl)phenyl)acetamide 37b (40 mg, 49.19 µmol) was added to dichloromethane (3 mL). Boron trichloride (0.8 mL) was added at 0 °C, and the reaction was performed at 0 C for 1 hour. Methanol was added to quench the reaction, and the mixture was concentrated under reduced pressure. The residue was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain (E)-N-(2-chloro-4-(trifluoromethyl)phenyl)-2-(5-ethyl-6-(4-(5-hydroxy-6-methylpyrimidine-4-ca rbonyl)piperazin-1-yl)-7-oxo-2-(2-(pyridin-2-yl)vinyl)-[1,2,4]triazolo[1,5-a]pyrimidin-4(7H)-yl)a cetamide 37 (2.95 mg). Yield: 8.15%.

MS m/z (ESI): 722.7 [M+1]
¹H NMR (400 MHz, DMSO-*d*₆) δ 10.41 (s, 1H), 10.24 (s, 1H), 8.65 (d, *J* = 4.6 Hz, 1H), 8.58 (s, 1H), 8.08 (d, *J* = 8.5 Hz, 1H), 7.98 (d, *J* = 2.1 Hz, 1H), 7.90 (s, 1H), 7.78 (s, 1H), 7.72 (dd, *J =* 11.1, 2.6 Hz, 1H), 7.66 (s, 1H), 7.58 (d, *J =* 16.2 Hz, 1H), 7.41 (s, 1H), 5.38 (s, 2H), 4.53 (d, *J =* 12.5 Hz, 1H), 3.51 (d, *J =* 12.0 Hz, 3H), 3.26 (t, *J =* 12.8 Hz, 2H), 2.84 (d, *J =* 11.5 Hz, 1H), 2.65 (s, 1H), 2.45 (s, 3H), 1.21 (t, *J* = 7.5 Hz, 3H).

### Example 38

### 2-(2-(bicyclo[3.1.0]hex-2-en-3-yl)-5-ethyl-6-(4-(3-hydroxypicolinoyl)piperazin-1-yl)-7-oxo-[1,2, 4]triazolo[1,5-a]pyrimidin-4(7H)-yl)-N-(2-chloro-4-(trifluoromethyl)phenyl)acetamide

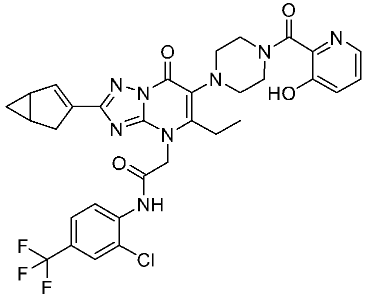

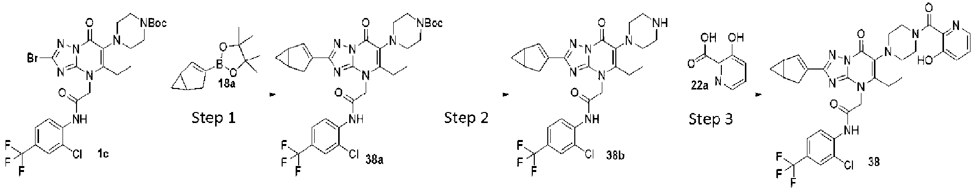

### Step 1

### tert-butyl 4-(2-(bicyclo[3.1.0]hex-2-en-3-yl)-4-(2-((2-chloro-4-(trifluoromethyl)phenyl)amino)-2-oxoethyl) -5-ethyl-7-oxo-4,7-dihydro-[1,2,4]triazolo[1,5-a]pyrimidin-6-yl)piperazine-1-carboxylate

At room temperature, tert-butyl 4-(2-bromo-4-(2-((2-chloro-4-(trifluoromethyl)phenyl) amino)-2-oxoethyl)-5-ethyl-7-oxo-4,7-dihydro-[1,2,4]triazolo[1,5-a]pyrimidin-6-yl)piperazine-1-carboxylate **1c** (50 mg, 75.43 µmol), 2-(bicyclo[3.1.0]hex-2-en-3-yl)-4,4,5,5-tetramethyl -1,3,2-dioxaborolane **18a** (18.65 mg, 90.51 µmol), [1,1'-bis(diphenylphosphino)ferrocene] palladium(II) dichloride dichloromethane complex (6.11 mg, 7.54 µmol), and sodium carbonate (23.98 mg, 226.28 µmol) were added to 1,4-dioxane (1 mL). The mixture was subject to argon replacement four times, heated to 80 °C, and the reaction was performed for 1.5 hours. Water (20 mL) was added to the reaction mixture, and the mixture was extracted with ethyl acetate (40 mL ×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(2-(bicyclo[3.1.0]hex-2-en-3-yl)-4-(2-((2-chloro-4-(trifluoromethyl)phenyl)amino) -2-oxoethyl)-5-ethyl-7-oxo-4,7-dihydro-[1,2,4]triazolo[1,5-a]pyrimidin-6-yl)piperazine-1-carbox ylate **38a** (42 mg). Yield: 84.10%.

MS m/z (ESI): 605.7 [M-56+1]

### Step 2

### 2-(2-(bicyclo[3.1.0]hex-2-en-3-yl)-5-ethyl-7-oxo-6-(piperazin-1-yl)-[1,2,4]triazolo[1,5-a] pyrimidin-4(7H)-yl)-N-(2-chloro-4-(trifluoromethyl)phenyl)acetamide

Tert-butyl 4-(2-(bicyclo[3.1.0]hex-2-en-3-yl)-4-(2-((2-chloro-4-(trifluoromethyl)phenyl) amino)-2-oxoethyl)-5-ethyl-7-oxo-4,7-dihydro-[1,2,4]triazolo[1,5-a]pyrimidin-6-yl)piperazine-1-carboxylate **38a** (42 mg, 63.43 µmol) was added to dichloromethane (3 mL), followed by addition of trifluoroacetic acid (1 mL). The reaction was performed at room temperature for 1 hour. The mixture was concentrated under reduced pressure to obtain 2-(2-(bicyclo[3.1.0]hex-2-en-3-yl)-5-ethyl-7-oxo-6-(piperazin-1-yl)-[1,2,4]triazolo[1,5-a]pyrimid in-4(7H)-yl)-N-(2-chloro-4-(trifluoromethyl)phenyl)acetamide **38b** (35 mg). Yield: 98.18%, which was used directly in the next step.

MS m/z (ESI): 561.8 [M+1]

### Step 3

### 2-(2-(bicyclo[3.1.0]hex-2-en-3-yl)-5-ethyl-6-(4-(3-hydroxypicolinoyl)piperazin-1-yl)-7-oxo-[1,2, 4]triazolo[1,5-a]pyrimidin-4(7H)-yl)-N-(2-chloro-4-(trifluoromethyl)phenyl)acetamide

2-(2-(Bicyclo[3.1.0]hex-2-en-3-yl)-5-ethyl-7-oxo-6-(piperazin-1-yl)-[1,2,4]triazolo[1,5-a]pyrimidin-4(7H)-yl)-N-(2-chloro-4-(trifluoromethyl)phenyl)acetamide **38b** (35 mg, 62.28 µmol), 3-hydroxypicolinic acid **22a** (11.26 mg, 80.96 µmol), 1-(3-dimethylaminopropyl)-3-ethylcarbodiimide hydrochloride (17.91 mg, 93.42 µmol), 1-hydroxybenzotriazole (12.62 mg, 93.42 µmol), and N,N-diisopropylethylamine (40.24 mg, 311.40 µmol) were added to N,N-dimethylformamide (2 mL), and the reaction was performed at room temperature for 4 h. The reaction mixture was added to water (20 mL) and extracted with ethyl acetate (30 mL ×2). The aqueous layer was separated, and the combined organic phase was washed with saturated sodium chloride solution (30 mL ×2), dried over anhydrous sodium sulfate, filtered, and concentrated under reduced pressure. The residue was purified by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain 2-(2-(bicyclo[3.1.0]hex-2-en-3-yl)-5-ethyl-6-(4-(3-hydroxypicolinoyl)piperazin-1-yl)-7-oxo-[1,2, 4]triazolo[1,5-a]pyrimidin-4(7H)-yl)-N-(2-chloro-4-(trifluoromethyl)phenyl)acetamide **38** (2.96 mg). Yield: 6.74%.

MS m/z (ESI): 683.2 [M+1]
¹H NMR (400 MHz, DMSO-*d*₆) δ 10.43 (s, 1H), 10.36 (s, 1H), 8.06 (d, *J* = 9.9 Hz, 2H), 7.97 (s, 1H), 7.71 (d, *J* = 8.7 Hz, 1H), 7.30 (d, *J* = 2.9 Hz, 2H), 6.80 (s, 1H), 5.29 (s, 2H), 4.54 (d, *J= 12.6* Hz, 1H), 3.43 (dd, *J* = 23.8, 12.4 Hz, 4H), 2.96 (s, 4H), 2.82 - 2.58 (m, 4H), 1.98 (s, 1H), 1.79 (s, 1H), 1.18 (q, *J* = 8.4 Hz, 3H), 1.01 (s, 1H).

### Example 39

### 2-(2-cycloheptyl-5-ethyl-6-(4-(2-hydroxy-3-methylbenzoyl)piperazin-1-yl)-7-oxo-[1,2,4]triazolo[ 1,5-a]pyrimidin-4(7H)-yl)-N-(4-(trifluoromethyl)phenyl)acetamide

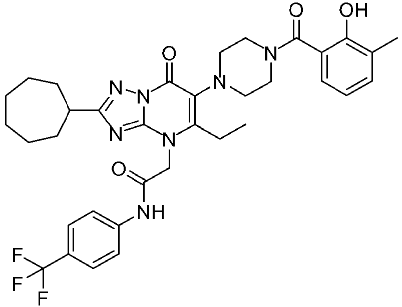

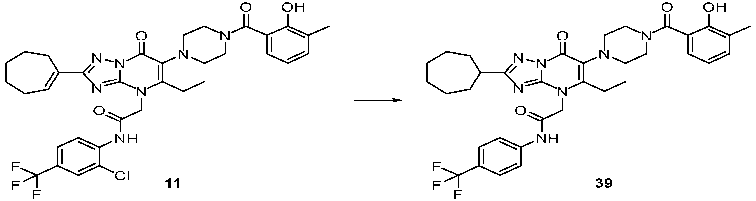

N-(2-Chloro-4-(trifluoromethyl)phenyl)-2-(2-(cyclohept-1-en-1-yl)-5-ethyl-6-(4-(2-hyd roxy-3-methylbenzoyl)piperazin-1-yl)-7-oxo-[1,2,4]triazolo[1,5-a]pyrimidin-4(7H)-yl)acetamide **11** (40 mg, 56.17 µmol) was added to methanol (1.5 mL), followed by addition of palladium on carbon (6.82 mg, 56.17 µmol), and the reaction was performed at room temperature for 16 h. The mixture was filtered and concentrated under reduced pressure. The residue was purified by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain 2-(2-cycloheptyl-5-ethyl-6-(4-(2-hydroxy-3-methylbenzoyl)piperazin-1-yl)-7-oxo-[1,2,4]triazolo[ 1,5-a]pyrimidin-4(7H)-yl)-N-(4-(trifluoromethyl)phenyl)acetamide **39** (2.9 mg). Yield: 7.34%.

MS m/z (ESI): 680.2 [M+1]
¹H NMR (400 MHz, DMSO-*d*₆) δ 10.84 (s, 1H), 9.04 (s, 1H), 7.82 - 7.67 (m, 4H), 7.19 - 7.09 (m, 1H), 7.01 (dd, *J* = 7.7, 1.7 Hz, 1H), 6.81 (t, *J* = 7.5 Hz, 1H), 5.17 (s, 2H), 3.98 (s, 1H), 3.47 (dd, *J* = 11.7, 3.0 Hz, 2H), 3.09 (s, 2H), 2.99 - 2.85 (m, 3H), 2.70 (d, *J* = 10.7 Hz, 2H), 2.20 (s, 3H), 2.01 - 1.91 (m, 2H), 1.71 (dtt, *J =* 15.3, 6.5, 3.0 Hz, 4H), 1.58 (dd, *J* = 7.7, 5.2 Hz, 2H), 1.54 - 1.41 (m, 4H), 1.16 (t, *J =* 7.4 Hz, 3H).

### Example 40

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-phenoxy-[1,2,4]triazolo[1,5-a]pyrimidin-4(7H)-yl)acetamide

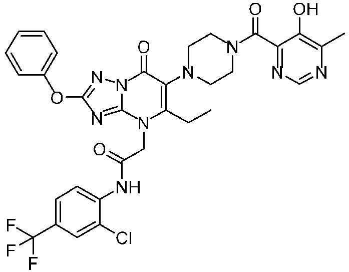

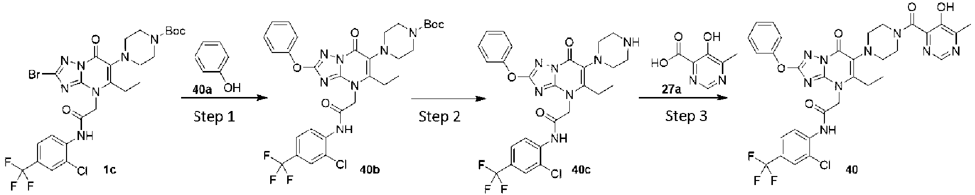

### Step 1

### tert-butyl 4-(4-(2-((2-chloro-4-(trifluoromethyl)phenyl)amino)-2-oxoethyl)-5-ethyl-7-oxo-2-phenoxy-4,7-dihydro-[1,2,4]triazolo[1,5-a]pyrimidin-6-yl)piperazine-1-carboxylate

Sodium methoxide (40.75 mg, 754.28 µmol) was added to a solution of phenol **40a** (56.79 mg, 603.42 µmol, commercially available) in tetrahydrofuran (5 mL) at 0 °C, and the reaction was performed at 0 °C for 1 h. Then tert-butyl 4-(2-bromo-4-(2-((2-chloro-4-(trifluoromethyl)phenyl)amino)-2-oxoethyl)-5-ethyl-7-oxo-4,7-dihydro-[1,2,4]triazolo[1 ,5-a]pyrimidin-6-yl)piperazine-1-carboxylate **1c** (200 mg, 301.71 µmol) was added to the reaction mixture, and the reaction mixture was heated to 70 °C and stirred for 16 h. Upon completion of the reaction, the reaction mixture was diluted with water (5 mL) and extracted with ethyl acetate (10 mL ×3). The combined organic phase was washed with saturated sodium chloride solution (10 mL ×2), dried over anhydrous sodium sulfate, filtered, and concentrated under reduced pressure. The resulting residue was purified by column chromatography (eluent: System B) to obtain tert-butyl 4-(4-(2-((2-chloro-4-(trifluoromethyl) phenyl)amino)-2-oxoethyl)-5-ethyl-7-oxo-2-phenoxy-4,7-dihydro-[1,2,4]triazolo[1,5-a]pyrimidin -6-yl)piperazine-1-carboxylate **40b** (100 mg). Yield: 49.02%.

MS m/z (ESI): 620.2 [M-55]

### Step 2

### N-(2-chloro-4-(trifluoromethyl)phenyl)-2-(5-ethyl-7-oxo-2-phenoxy-6-(piperazin-1-yl)-[1,2,4] triazolo[1,5-a]pyrimidin-4(7H)-yl)acetamide

tert-butyl 4-(4-(2-((2-chloro-4-(trifluoromethyl)phenyl)amino)-2-oxoethyl)-5-ethyl-7-oxo-2-phenoxy-4,7-dihydro-[1,2,4]triazolo[1,5-a]pyrimidin-6-yl)piperazine-1-carboxylate **40b** (100 mg, 147.91 µmol) was dissolved in dichloromethane (2 mL), trifluoroacetic acid (0.5 mL) was added, and the reaction mixture was stirred at room temperature for 1 h. The reaction mixture was concentrated under reduced pressure to obtain N-(2-chloro-4-(trifluoromethyl)phenyl)-2-(5-ethyl-7-oxo-2-phenoxy-6-(piperazin-1-yl)-[1,2,4]triazolo[1,5-a]pyrimidin-4(7H)-yl)acetami de **40c** (90 mg). Yield: 54.26%.

MS m/z (ESI): 576.2 [M+1]

### Step 3

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-phenoxy-[1,2,4]triazolo[1,5-a]pyrimidin-4(7H)-yl)acetamide

At room temperature, to a solution of N-(2-chloro-4-(trifluoromethyl)phenyl)-2-(5-ethyl-7-oxo-2-phenoxy-6-(piperazin-1-yl)-[1,2,4]triazolo[1,5-a]pyrimidin-4(7H)-yl)acetamide **40c** (90 mg, 156.26 µmol) in N,N-dimethylformamide (2 mL) were added 1-(3-dimethylaminopropyl)-3-ethylcarbodiimide hydrochloride (44.93 mg, 234.39 µmol), 5-hydroxy-6-methylpyrimidine-4-carboxylic acid **27a** (72.25 mg, 468.78 µmol), 1-hydroxybenzotriazole (31.67 mg, 234.39 µmol), and N,N-diisopropylethylamine (60.58 mg, 468.78 µmol), and the reaction was performed at room temperature for 16 hours. After completion of the reaction, the mixture was filtered, and the filtrate was purified by preparative liquid chromatography (separation column AKZONOBEL Kromasil; 250×19 mm I.D.; 10 µm, 20 mL/min; mobile phase A: 10 mmol NH₄HCO₃, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-7-oxo-2-phenoxy-[1,2,4]triazolo[1,5-a]pyrimidin-4(7H)-yl)acetamide **40** (2 mg) with a yield of 1.80%.

MS m/z (ESI): 712.3 [M+1]
¹H NMR (400 MHz, MeOD-*d*₄) δ 8.48 (s, 1H), 8.13 (d, *J* = 8.8 Hz, 1H), 7.84 - 7.76 (m, 1H), 7.64 - 7.59 (m, 1H), 7.40 - 7.32 (m, 2H), 7.26 (d, *J* = 7.6 Hz, 2H), 7.20 (t, *J* = 7.2 Hz, 1H), 5.29 (s, 2H), 4.75 - 4.63 (m, 1H), 4.13 - 3.92 (m, 1H), 3.70 (d, *J* = 9.6 Hz, 2H), 3.52 - 3.39 (m, 1H), 3.17 - 3.05 (m, 3H), 2.92 (d, *J* = 11.2 Hz, 1H), 2.77 (d, *J* = 10.8 Hz, 1H), 2.49 (s, 3H), 1.29 (t, 3H).

### Example 41

### 2-(2-(bicyclo[2.2.1]hept-2-en-2-yl)-5-ethyl-6-(4-(3-hydroxypicolinoyl)piperazin-1-yl)-7-oxo-[1,2,4]triazolo[1,5-a]pyrimidin-4(7H)-yl)-N-(2-chloro-4-(trifluoromethyl)phenyl)acetamide

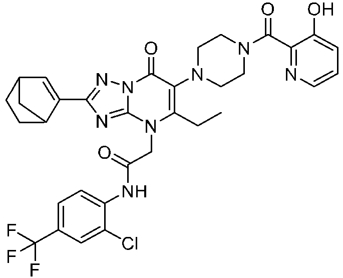

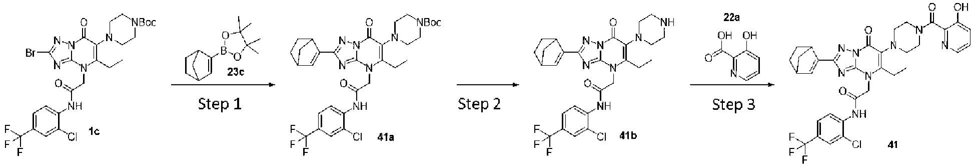

### Step 1

### tert-butyl 4-(2-(bicyclo[2.2.1]hept-2-en-2-yl)-4-(2-((2-chloro-4-(trifluoromethyl)phenyl)amino)-2-oxoethyl)-5-ethyl-7-oxo-4,7-dihydro-[1,2,4]triazolo[1,5-a]pyrimidin-6-yl)piperazine-1-carboxyla te

At room temperature, tert-butyl 4-(2-bromo-4-(2-((2-chloro-4-(trifluoromethyl)phenyl) amino)-2-oxoethyl)-5-ethyl-7-oxo-4,7-dihydro-[1,2,4]triazolo[1,5-a]pyrimidin-6-yl)piperazine-1-carboxylate **1c** (60 mg, 90.51 µmol), 2-(bicyclo[2.2.1]hept-2-en-2-yl)-4,4,5,5-tetramethyl-1,3,2-dioxaborolane **23c** (23.91 mg, 108.62 µmol), [1,1'-bis(diphenylphosphino)ferrocene]palladium(II) dichloride dichloromethane complex (7.34 mg, 9.05 µmol), and sodium carbonate (28.78 mg, 271.54 µmol) were added to 1,4-dioxane (1 mL), and the mixture was subject to argon replacement four times, then heated to 80 °C for 1.5 hours. Water (20 mL) was added to the reaction mixture, followed by extraction with ethyl acetate (40 mL ×3). The combined organic phase was washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(2-(bicyclo[2.2.1]hept-2-en-2-yl)-4-(2-((2-chloro-4-(trifluoromethyl)phenyl)amino)-2-oxoethyl)-5-ethyl-7-oxo-4,7-dihydro-[1,2,4]triazolo[1,5-a]pyri midin-6-yl)piperazine-1-carboxylate **41a** (60 mg) with a yield of 98.04%.

MS m/z (ESI): 675.8 [M+1]

### Step 2

### 2-(2-(bicyclo[2.2.1]hept-2-en-2-yl)-5-ethyl-7-oxo-6-(piperazin-1-yl)-[1,2,4]triazolo[1,5-a] pyrimidin-4(7H)-yl)-N-(2-chloro-4-(trifluoromethyl)phenyl)acetamide

tert-butyl 4-(2-(bicyclo[2.2.1]hept-2-en-2-yl)-4-(2-((2-chloro-4-(trifluoromethyl)phenyl) amino)-2-oxoethyl)-5-ethyl-7-oxo-4,7-dihydro-[1,2,4]triazolo[1,5-a]pyrimidin-6-yl)piperazine-1-carboxylate **41a** (60 mg, 88.74 µmol) was added to dichloromethane (3 mL), followed by addition of trifluoroacetic acid (1 mL), and the reaction was performed at room temperature for 1 hour. The mixture was concentrated under reduced pressure to obtain 2-(2-(bicyclo[2.2.1]hept-2-en-2-yl)-5-ethyl-7-oxo-6-(piperazin-1-yl)-[1,2,4]triazolo[1,5-a]pyrimi din-4(7H)-yl)-N-(2-chloro-4-(trifluoromethyl)phenyl)acetamide **41b** (50 mg) with a yield of 97.82%, which was used directly in the next step.

MS m/z (ESI): 575.8 [M+1]

### Step 3

### 2-(2-(bicyclo[2.2.1]hept-2-en-2-yl)-5-ethyl-6-(4-(3-hydroxypicolinoyl)piperazin-1-yl)-7-oxo-[1,2,4]triazolo[1,5-a]pyrimidin-4(7H)-yl)-N-(2-chloro-4-(trifluoromethyl)phenyl)acetamide

2-(2-(Bicyclo[2.2.1]hept-2-en-2-yl)-5-ethyl-7-oxo-6-(piperazin-1-yl)-[1,2,4]triazolo[1,5 -a]pyrimidin-4(7H)-yl)-N-(2-chloro-4-(trifluoromethyl)phenyl)acetamide **41b** (50 mg, 86.80 µmol), 3-hydroxypicolinic acid **22a** (15.70 mg, 112.84 µmol), 1-(3-dimethylaminopropyl)-3-ethylcarbodiimide hydrochloride (24.96 mg, 130.21 µmol), 1-hydroxybenzotriazole (17.59 mg, 130.21 µmol), and N,N-diisopropylethylamine (56.09 mg, 434.02 µmol) were added to N,N-dimethylformamide (2 mL), and the reaction was performed at room temperature for 4 hours. Water (20 mL) was added to the reaction mixture, followed by extraction with ethyl acetate (30 mL ×3). The aqueous layer was separated, and the combined organic phase was washed with saturated sodium chloride solution (30 mL ×2), dried over anhydrous sodium sulfate, filtered, and concentrated under reduced pressure. The resulting residue was purified by preparative liquid chromatography (separation column AKZONOBEL Kromasil; 250×19 mm I.D.; 10 µm, 20 mL/min; mobile phase A: 10 mmol NH₄HCO₃, mobile phase B: CH₃CN) to obtain 2-(2-(bicyclo[2.2.1]hept-2-en-2-yl)-5-ethyl-6-(4-(3-hydroxypicolinoyl)piperazin-1-yl)-7-oxo-[1,2,4]triazolo[1,5-a]pyrimidin-4(7H)-yl)-N-(2-chloro-4 -(trifluoromethyl)phenyl)acetamide **41** (3.28 mg) with a yield of 5.36%.

MS m/z (ESI): 697.2 [M+1]
¹H NMR (400 MHz, DMSO-*d*₆) δ 10.47 (s, 1H), 10.36 (s, 1H), 8.14 - 8.01 (m, 2H), 7.97 (s, 1H), 7.71 (d, *J =* 8.7 Hz, 1H), 7.32 (d, *J =* 3.1 Hz, 2H), 6.75 (d, *J =* 3.1 Hz, 1H), 5.30 (s, 2H), 4.55 (d, *J* = 12.8 Hz, 1H), 3.57 - 3.33 (m, 4H), 3.00 (d, *J* = 27.0 Hz, 4H), 2.79 (d, *J* = 11.3 Hz, 1H), 2.62 (d, *J =* 10.5 Hz, 1H), 1.78 (d, *J* = 7.2 Hz, 2H), 1.46 (s, 1H), 1.26 (d, *J =* 8.5 Hz, 1H), 1.18 (t, *J =* 7.4 Hz, 3H), 1.03 (d, *J =* 25.7 Hz, 2H).

### Example 42

### 2-(2-(cyclohept-1-en-1-yl)-5-ethyl-6-(4-(3-hydroxyisonicotinoyl)piperazin-1-yl)-7-oxo-[1,2,4] triazolo[1,5-a]pyrimidin-4(7H)-yl)-N-(2-methyl-4-(trifluoromethyl)phenyl)acetamide

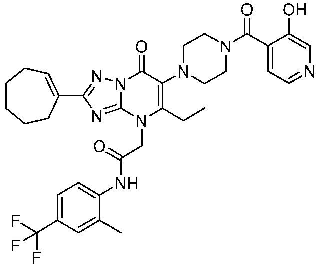

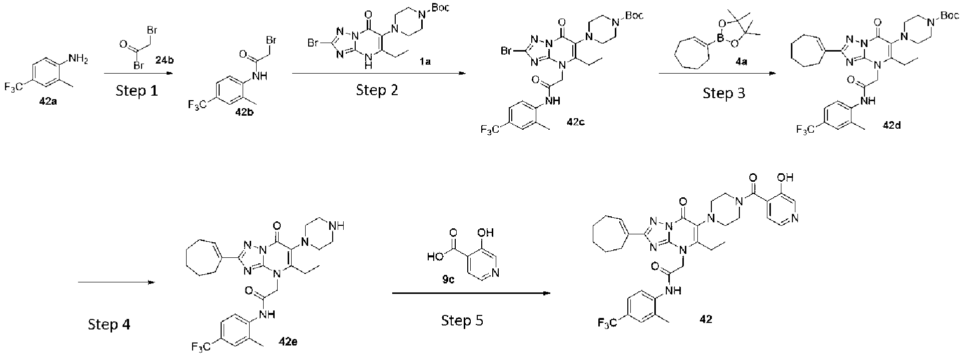

### Step 1

### 2-bromo-N-(2-methyl-4-(trifluoromethyl)phenyl)acetamide

Under an ice bath and argon atmosphere, a solution of 2-bromoacetyl bromide **24b** (605.01 mg, 3.00 mmol, 260.33 µL) in dichloromethane (7 mL) was added dropwise to a solution of 2-methyl-4-(trifluoromethyl)aniline **42a** (500 mg, 2.85 mmol, commercially available) and 4-dimethylaminopyridine (348.76 mg, 2.85 mmol) in dichloromethane (20 mL). The reaction was performed at room temperature for 16 h. The mixture was concentrated under reduced pressure, and the resulting residue was purified by silica gel column chromatography (eluent: System A) to obtain 2-bromo-N-(2-methyl-4-(trifluoromethyl)phenyl)acetamide **42b** (640 mg) with a yield of 75.72%.

MS m/z (ESI): 296.0 [M+1]

### Step 2

### tert-butyl 4-(2-bromo-5-ethyl-4-(2-((2-methyl-4-(trifluoromethyl)phenyl)amino)-2-oxoethyl)-7-oxo-4,7-dihydro-[1,2,4]triazolo[1,5-a]pyrimidin-6-yl)piperazine-1-carboxylate

At room temperature, 2-bromo-N-(2-methyl-4-(trifluoromethyl)phenyl)acetamide **42b** (270.24 mg, 912.72 µmol), tert-butyl 4-(2-bromo-5-ethyl-7-oxo-4,7-dihydro-[1,2,4]triazolo[1,5-a] pyrimidin-6-yl)piperazine-1-carboxylate **1a** (300 mg, 702.09 µmol), and N,N-diisopropylethylamine (453.69 mg, 3.51 mmol) were added to N,N-dimethylformamide (12 mL). The temperature was raised to 60 °C, and the reaction was performed for 1.5 h. Water (20 mL) was added to the reaction mixture, and the mixture was extracted with ethyl acetate (20 mL ×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(2-bromo-5-ethyl-4-(2-((2-methyl-4-(trifluoromethyl)phenyl)amino)-2-oxoethyl) -7-oxo-4,7-dihydro-[1,2,4]triazolo[1,5-a]pyrimidin-6-yl)piperazine-1-carboxylate **42c** (451 mg) with a yield of 99.98%.

MS m/z (ESI): 586.1 [M-56+H]

### Step 3

### tert-butyl 4-(2-(cyclohept-1-en-1-yl)-5-ethyl-4-(2-((2-methyl-4-(trifluoromethyl)phenyl)amino)-2-oxoethyl)-7-oxo-4,7-dihydro-[1,2,4]triazolo[1,5-a]pyrimidin-6-yl)piperazine-1-carboxylate

At room temperature, tert-butyl 4-(2-bromo-5-ethyl-4-(2-((2-methyl-4-(trifluoromethyl) phenyl)amino)-2-oxoethyl)-7-oxo-4,7-dihydro-[1,2,4]triazolo[1,5-a]pyrimidin-6-yl)piperazine-1-carboxylate **42c** (250 mg, 389.13 µmol), 2-(cyclohept-1-en-1-yl)-4,4,5,5-tetramethyl-1,3,2-dioxaborolane **4a** (103.72 mg, 466.95 µmol), [1,1'-bis(diphenylphosphino)ferrocene]palladium(II) dichloride dichloromethane complex (31.54 mg, 38.91 µmol), and sodium carbonate (123.73 mg, 1.17 mmol) were added to a mixed solvent of 1,4-dioxane (5 mL) and water (0.5 mL). The mixture was subject to argon replacement three times. The temperature was raised to 85 °C, and the reaction was performed for 2 h. Water (20 mL) was added to the reaction mixture, and the mixture was extracted with ethyl acetate (20 mL ×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(2-(cyclohept-1-en-1-yl)-5-ethyl-4-(2-((2-methyl-4-(trifluoromethyl)phenyl)amino)-2-oxoethyl)-7-oxo-4,7-dihy dro-[1,2,4]triazolo[1,5-a]pyrimidin-6-yl)piperazine-1-carboxylate **42d** (250 mg) with a yield of 97.68%.

MS m/z (ESI): 658.3 [M+1]

### Step 4

### 2-(2-(cyclohept-1-en-1-yl)-5-ethyl-7-oxo-6-(piperazin-1-yl)-[1,2,4]triazolo[1,5-a]pyrimidin-4 (7H)-yl)-N-(2-methyl-4-(trifluoromethyl)phenyl)acetamide

At room temperature, trifluoroacetic acid (1.5 mL) was added to a solution of tert-butyl 4-(2-(cyclohept-1-en-1-yl)-5-ethyl-4-(2-((2-methyl-4-(trifluoromethyl)phenyl)amino)-2-oxoethyl )-7-oxo-4,7-dihydro-[1,2,4]triazolo[1,5-a]pyrimidin-6-yl)piperazine-1-carboxylate **42d** (150 mg, 228.06 µmol) in dichloromethane (6 mL). The reaction was performed at room temperature for 40 min. The reaction mixture was concentrated under reduced pressure to obtain 2-(2-(cyclohept-1-en-1-yl)-5-ethyl-7-oxo-6-(piperazin-1-yl)-[1,2,4]triazolo[1,5-a]pyrimidin-4(7H )-yl)-N-(2-methyl-4-(trifluoromethyl)phenyl)acetamide **42e** (127.1 mg) with a yield of 99.95%, which was used directly in the next step.

MS m/z (ESI): 558.3 [M+1]

### Step 5

### 2-(2-(cyclohept-1-en-1-yl)-5-ethyl-6-(4-(3-hydroxyisonicotinoyl)piperazin-1-yl)-7-oxo-[1,2,4] triazolo[1,5-a]pyrimidin-4(7H)-yl)-N-(2-methyl-4-(trifluoromethyl)phenyl)acetamide

At room temperature, 3-hydroxyisonicotinic acid **9c** (15.72 mg, 112.98 µmol), 1-(3-dimethylaminopropyl)-3-ethylcarbodiimide hydrochloride (28.88 mg, 150.64 µmol), 1-hydroxybenzotriazole (20.35 mg, 150.64 µmol), and N,N-diisopropylethylamine (48.67 mg, 376.61 µmol) were added to N,N-dimethylformamide (1.5 mL), and the reaction was performed at room temperature for 40 min. Then, a solution of 2-(2-(cyclohept-1-en-1-yl)-5-ethyl-7-oxo-6-(piperazin-1-yl)-[1,2,4]triazolo[1,5-a]pyrimidin-4(7H )-yl)-N-(2-methyl-4-(trifluoromethyl)phenyl)acetamide **42e** (42 mg, 75.32 µmol) in N,N-dimethylformamide (2 mL) was added, and the reaction was performed at room temperature for 16 h. After the monitored reaction was completed, water (20 mL) was added to the reaction mixture, and the mixture was extracted with ethyl acetate (20 mL ×3). The combined organic phases were washed with saturated sodium chloride solution (20 mL), dried over anhydrous sodium sulfate, filtered, and concentrated under reduced pressure. The resulting residue was purified by preparative liquid chromatography (separation column AKZONOBEL Kromasil; 250×19 mm I.D.; 10 µm, 20 mL/min; mobile phase A: 10 mmol NH₄HCO₃, mobile phase B: CH₃CN) to obtain 2-(2-(cyclohept-1-en-1-yl)-5-ethyl-6-(4-(3-hydroxyisonicotinoyl)piperazin -1-yl)-7-oxo-[1,2,4]triazolo[1,5-a]pyrimidin-4(7H)-yl)-N-(2-methyl-4-(trifluoromethyl)phenyl)ac etamide **42** (18.84 mg) in a yield of 34.53%.

MS m/z (ESI): 679.3 [M+1]
¹H NMR (400 MHz, DMSO-*d*₆) δ 11.22 (s, 1H), 10.03 (s, 1H), 8.32 (s, 1H), 8.26 (d, *J* = 5.1 Hz, 1H), 7.72 (d, *J =* 8.4 Hz, 1H), 7.63 (d, *J =* 2.1 Hz, 1H), 7.60 - 7.47 (m, 2H), 7.10 (t, *J* = 6.8 Hz, 1H), 5.24 (s, 2H), 4.53 (d, *J =* 12.5 Hz, 1H), 3.47 (q, *J* = 10.8, 10.3 Hz, 2H), 3.30 (q, *J* = 12.1, 11.4 Hz, 2H), 3.14 - 2.89 (m, 3H), 2.80 (d, *J =* 11.2 Hz, 1H), 2.78 - 2.70 (m, 2H), 2.64 (d, *J* = 11.2 Hz, 1H), 2.36 (s, 3H), 2.32 (d, *J* = 6.2 Hz, 2H), 1.79 (q, *J* = 6.0 Hz, 2H), 1.53 (h, *J* = 9.0, 7.1 Hz, 4H), 1.19 (t, *J* = 7.4 Hz, 3H).

### Example 43

### 2-(2-(cyclohept-1-en-1-yl)-5-ethyl-6-(4-(3-hydroxyisonicotinoyl)piperazin-1-yl)-7-oxo-[1,2,4] triazolo[1,5-a]pyrimidin-4(7H)-yl)-N-(2-methoxy-4-(trifluoromethyl)phenyl)acetamide

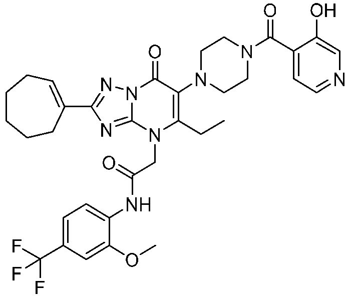

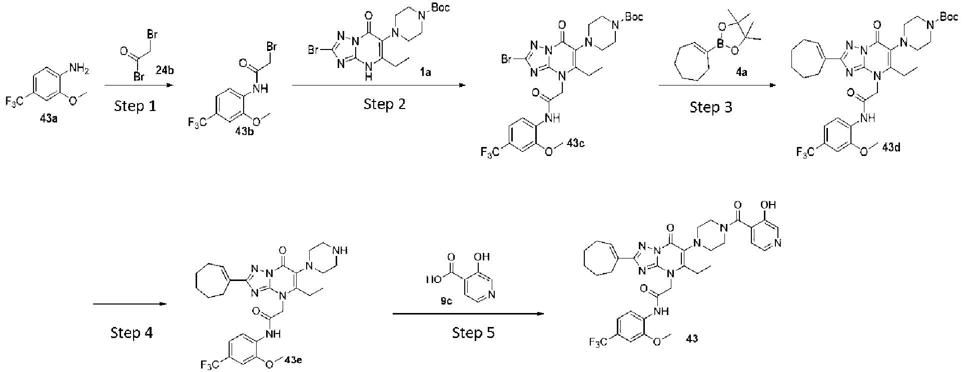

### Step 1

### 2-bromo-N-(2-methoxy-4-(trifluoromethyl)phenyl)acetamide

In an ice bath under argon atmosphere, a solution of 2-bromoacetyl bromide **24b** (554.37 mg, 2.75 mmol, 238.54 µL) in dichloromethane (7 mL) was added dropwise to a solution of 2-methoxy-4-(trifluoromethyl)aniline **43a** (500 mg, 2.62 mmol) and 4-dimethylaminopyridine (319.57 mg, 2.62 mmol) in dichloromethane (9.76 mL), and the reaction was performed at room temperature for 16 h. The mixture was concentrated under reduced pressure, and the resulting residue was purified by silica gel column chromatography (eluent: System A) to obtain 2-bromo-N-(2-methoxy-4-(trifluoromethyl)phenyl)acetamide **43b** (690 mg) in a yield of 84.52%.

MS m/z (ESI): 312.0 [M+1]

### Step 2

### tert-butyl 4-(2-bromo-5-ethyl-4-(2-((2-methoxy-4-(trifluoromethyl)phenyl)amino)-2-oxoethyl)-7-oxo-4,7-dihydro-[1,2,4]triazolo[1,5-a]pyrimidin-6-yl)piperazine-1-carboxylate

At room temperature, 2-bromo-N-(2-methoxy-4-(trifluoromethyl)phenyl)acetamide **43b** (350.58 mg, 1.12 mmol), tert-butyl 4-(2-bromo-5-ethyl-7-oxo-4,7-dihydro-[1,2,4]triazolo [1,5-a]pyrimidin-6-yl)piperazine-1-carboxylate **1a** (400 mg, 936.12 µmol), and N,N-diisopropylethylamine (362.95 mg, 2.81 mmol) were added to N,N-dimethylformamide (15 mL), and the temperature was raised to 60 °C. The reaction was performed for 1.5 h. Water (20 mL) was added to the reaction mixture, and the mixture was extracted with ethyl acetate (20 mL×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(2-bromo-5-ethyl-4-(2-((2-methoxy-4-(trifluoromethyl)phenyl)amino)-2-oxoethyl) -7-oxo-4,7-dihydro-[1,2,4]triazolo[1,5-a]pyrimidin-6-yl)piperazine-1-carboxylate **43c** (520 mg) in a yield of 84.36%.

MS m/z (ESI): 604.1 [M-56+H]

### Step 3

### tert-butyl 4-(2-(cyclohept-1-en-1-yl)-5-ethyl-4-(2-((2-methoxy-4-(trifluoromethyl)phenyl)amino) -2-oxoethyl)-7-oxo-4,7-dihydro-[1,2,4]triazolo[1,5-a]pyrimidin-6-yl)piperazine-1-carboxylate

At room temperature, tert-butyl 4-(2-bromo-5-ethyl-4-(2-((2-methoxy-4-(trifluoromethyl)phenyl)amino)-2-oxoethyl)-7-oxo-4,7-dihydro-[1,2,4]triazolo[1,5-a]pyrimidin-6 -yl)piperazine-1-carboxylate **43c** (250 mg, 379.67 µmol), 2-(cyclohept-1-en-1-yl)-4,4,5,5-tetramethyl-1,3,2-dioxaborolane **4a** (109.64 mg, 493.57 µmol), [1,1'-bis(diphenylphosphino) ferrocene]palladium(II) dichloride dichloromethane complex (30.77 mg, 37.97 µmol), and sodium carbonate (120.72 mg, 1.14 mmol) were added to a mixed solution of 1,4-dioxane (3 mL) and water (0.5 mL). The mixture was subject to argon replacement 3 times, and the temperature was raised to 85 °C. The reaction was performed for 2 h. Water (20 mL) was added to the reaction mixture, and the mixture was extracted with ethyl acetate (20 mL ×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(2-(cyclohept-1-en-1-yl)-5-ethyl-4-(2-((2-methoxy-4-(trifluoromethyl)phenyl)amino)-2-oxoeth yl)-7-oxo-4,7-dihydro-[1,2,4]triazolo[1,5-a]pyrimidin-6-yl)piperazine-1-carboxylate **43d** (255 mg) in a yield of 99.69%.

MS m/z (ESI): 618.3 [M-56+H]

### Step 4

### 2-(2-(cyclohept-1-en-1-yl)-5-ethyl-7-oxo-6-(piperazin-1-yl)-[1,2,4]triazolo[1,5-a]pyrimidin-4(7H)-yl)-N-(2-methoxy-4-(trifluoromethyl)phenyl)acetamide

At room temperature, trifluoroacetic acid (1.5 mL) was added to a solution of tert-butyl 4-(2-(cyclohept-1-en-1-yl)-5-ethyl-4-(2-((2-methoxy-4-(trifluoromethyl)phenyl)amino)-2-oxoeth yl)-7-oxo-4,7-dihydro-[1,2,4]triazolo[1,5-a]pyrimidin-6-yl)piperazine-1-carboxylate **43d** (255 mg, 378.49 µmol) in dichloromethane (6 mL), and the reaction was performed at room temperature for 40 min. The mixture was concentrated under reduced pressure to obtain 2-(2-(cyclohept-1-en-1-yl)-5-ethyl-7-oxo-6-(piperazin-1-yl)-[1,2,4]triazolo[1,5-a]pyrimidin-4(7H )-yl)-N-(2-methoxy-4-(trifluoromethyl)phenyl)acetamide **43e** (217 mg) in a yield of 99.95%, which was used directly in the next step.

MS m/z (ESI): 574.3 [M+1]

### Step 5

### 2-(2-(cyclohept-1-en-1-yl)-5-ethyl-6-(4-(3-hydroxyisonicotinoyl)piperazin-1-yl)-7-oxo-[1,2,4] triazolo[1,5-a]pyrimidin-4(7H)-yl)-N-(2-methoxy-4-(trifluoromethyl)phenyl)acetamide

At room temperature, 3-hydroxyisonicotinic acid **9c** (18.19 mg, 130.75 µmol), 1-(3-dimethylaminopropyl)-3-ethylcarbodiimide hydrochloride (33.42 mg, 174.33 µmol), 1-hydroxybenzotriazole (23.56 mg, 174.33 µmol), and N,N-diisopropylethylamine (56.33 mg, 435.84 µmol) were added to N,N-dimethylformamide (2 mL), and the reaction was performed at room temperature for 40 min. 2-(2-(cyclohept-1-en-1-yl)-5-ethyl-7-oxo-6-(piperazin-1-yl)-[1,2,4]triazolo[1,5-a]pyrimidin-4(7H)-yl)-N-(2-methoxy-4-(trifluoromethyl)phen yl)acetamide **43e** (50 mg, 87.17 µmol) was added, and the reaction was performed at room temperature for 16 h. Water (20 mL) was added to the reaction mixture, and the mixture was extracted with ethyl acetate (20 mL×3). The combined organic phases were washed with saturated sodium chloride solution (20 mL), dried over anhydrous sodium sulfate, filtered, and concentrated under reduced pressure. The resulting residue was purified by preparative liquid chromatography (separation column AKZONOBEL Kromasil; 250×19 mm I.D.; 10 µm, 20 mL/min; mobile phase A: 10 mmol NH₄HCO₃, mobile phase B: CH₃CN) to obtain 2-(2-(cyclohept-1-en-1-yl)-5-ethyl-6-(4-(3-hydroxyisonicotinoyl)piperazin-1-yl)-7-oxo-[1,2,4]tria zolo[1,5-a]pyrimidin-4(7H)-yl)-N-(2-methoxy-4-(trifluoromethyl)phenyl)acetamide **43** (21.52 mg) in a yield of 34.86%.

MS m/z (ESI): 695.3 [M+1]
¹H NMR (400 MHz, DMSO-*d*₆) δ 11.08 (s, 1H), 10.12 (s, 1H), 8.31 (s, 1H), 8.24 (d, *J* = 5.1 Hz, 1H), 8.20 (d, *J =* 8.5 Hz, 1H), 7.47 (d, *J =* 5.1 Hz, 1H), 7.37 (d, *J* = 1.9 Hz, 1H), 7.28 (d, *J =* 8.3 Hz, 1H), 7.06 (t, *J =* 6.7 Hz, 1H), 5.31 (s, 2H), 4.52 (d, *J* = 12.6 Hz, 1H), 3.99 (s, 3H), 3.47 (q, *J =* 10.2, 9.7 Hz, 2H), 3.28 (dt, *J =* 23.9, 12.5 Hz, 2H), 2.95 (q, *J* = 10.8, 7.8 Hz, 3H), 2.80 (d, *J =* 11.1 Hz, 1H), 2.73 (d, *J* = 8.7 Hz, 2H), 2.63 (d, *J =* 11.1 Hz, 1H), 2.30 (s, 2H), 1.78 (s, 2H), 1.70 - 1.43 (m, 4H), 1.15 (t, *J* = 7.4 Hz, 3H).

### Example 44

### 2-(2-(cyclohept-1-en-1-yl)-5-ethyl-6-(4-(3-hydroxyisonicotinoyl)piperazin-1-yl)-7-oxo-[1,2,4] triazolo[1,5-a]pyrimidin-4(7H)-yl)-N-(2-fluoro-4-(trifluoromethyl)phenyl)acetamide

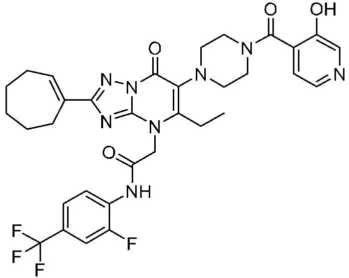

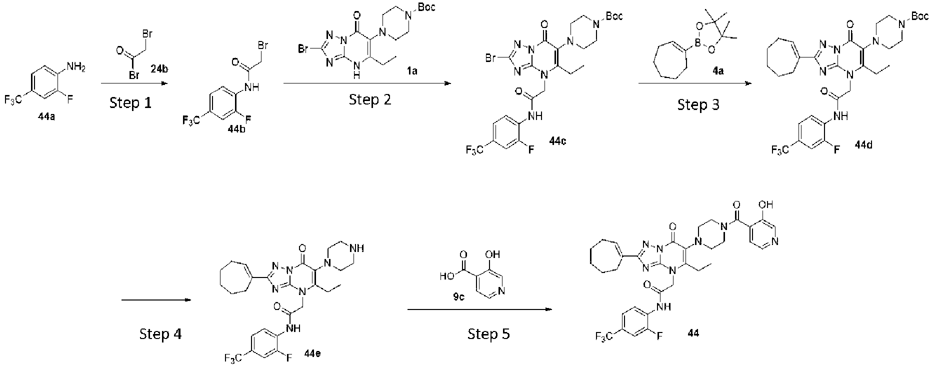

### Step 1

### 2-bromo-N-(2-fluoro-4-(trifluoromethyl)phenyl)acetamide

Under an ice bath and argon atmosphere, a solution of 2-bromoacetyl bromide **24b** (1.35 g, 6.70 mmol, 581.88 µL) in dichloromethane (5 mL) was added dropwise to a solution of 2-fluoro-4-(trifluoromethyl)aniline **44a** (1 g, 5.58 mmol, commercially available) and 4-dimethylaminopyridine (682.08 mg, 5.58 mmol) in dichloromethane (10 mL), and the reaction was performed at room temperature for 16 h. Water (20 mL) was added to the reaction mixture, and the mixture was extracted with dichloromethane (20 mL×3). The combined organic phases were washed with saturated sodium chloride solution (20 mL), dried over anhydrous sodium sulfate, filtered, and concentrated under reduced pressure. The resulting residue was purified by silica gel column chromatography (eluent: System A) to obtain 2-bromo-N-(2-fluoro-4-(trifluoromethyl)phenyl)acetamide **44b** (1.6 g) in a yield of 95.51%.

MS m/z (ESI): 300.0 [M+1]

### Step 2

### tert-butyl 4-(2-bromo-5-ethyl-4-(2-((2-fluoro-4-(trifluoromethyl)phenyl)amino)-2-oxoethyl)-7-oxo-4,7-dihydro-[1,2,4]triazolo[1,5-a]pyrimidin-6-yl)piperazine-1-carboxylate

At room temperature, 2-bromo-N-(2-fluoro-4-(trifluoromethyl)phenyl)acetamide **44b** (421.32 mg, 1.40 mmol), tert-butyl 4-(2-bromo-5-ethyl-7-oxo-4,7-dihydro-[1,2,4] triazolo[1,5-a]pyrimidin-6-yl)piperazine-1-carboxylate **1a** (500 mg, 1.17 mmol), and N,N-diisopropylethylamine (756.15 mg, 5.85 mmol) were added to N,N-dimethylformamide (7 mL), the temperature was raised to 50 °C, and the reaction was performed for 1.5 h. Water (20 mL) was added to the reaction mixture, and the mixture was extracted with ethyl acetate (20 mL ×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(2-bromo-5-ethyl-4-(2-((2-fluoro-4-(trifluoromethyl)phenyl)amino)-2-oxoethyl)-7-oxo-4,7-dihydro-[1,2,4]triazolo[1,5-a]pyrimidin-6-yl)piperazine-1-carboxylate **44c** (710 mg) in a yield of 93.86%.

MS m/z (ESI): 590.2 [M-56+H]

### Step 3

### tert-butyl 4-(2-(cyclohept-1-en-1-yl)-5-ethyl-4-(2-((2-fluoro-4-(trifluoromethyl)phenyl)amino) -2-oxoethyl)-7-oxo-4,7-dihydro-[1,2,4]triazolo[1,5-a]pyrimidin-6-yl)piperazine-1-carboxylate

At room temperature, tert-butyl 4-(2-bromo-5-ethyl-4-(2-((2-fluoro-4-(trifluoromethyl) phenyl)amino)-2-oxoethyl)-7-oxo-4,7-dihydro-[1,2,4]triazolo[1,5-a]pyrimidin-6-yl)piperazine-1-carboxylate) **44c** (300 mg, 464.09 µmol, 2-(cyclohept-1-en-1-yl)-4,4,5,5-tetramethyl -1,3,2-dioxaborolane **4a** (134.01 mg, 603.31 µmol), [1,1'-bis(diphenylphosphino)ferrocene] palladium(II) dichloride dichloromethane complex (37.62 mg, 46.41 µmol), and sodium carbonate (147.57 mg, 1.39 mmol) were added to a mixed solution of 1,4-dioxane/water (10/1 mL). The mixture was subject to argon replacement three times, the temperature was raised to 85 °C, and the reaction was performed for 3 h. Water (20 mL) was added to the reaction mixture, and the mixture was extracted with ethyl acetate (20 mL ×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(2-(cyclohept-1-en-1-yl)-5-ethyl-4-(2-((2-fluoro-4-(trifluoromethyl)phenyl)amino)-2-oxoethyl) -7-oxo-4,7-dihydro-[1,2,4]triazolo[1,5-a]pyrimidin-6-yl)piperazine-1-carboxylate **44d** (200 mg) in a yield of 65.13%.

MS m/z (ESI): 606.3 [M-56+H]

### Step 4

### 2-(2-(cyclohept-1-en-1-yl)-5-ethyl-7-oxo-6-(piperazin-1-yl)-[1,2,4]triazolo[1,5-a]pyrimidin-4(7H)-yl)-N-(2-fluoro-4-(trifluoromethyl)phenyl)acetamide

At room temperature, trifluoroacetic acid (1.5 mL) was added to a solution of tert-butyl 4-(2-(cyclohept-1-en-1-yl)-5-ethyl-4-(2-((2-fluoro-4-(trifluoromethyl)phenyl)amino)-2-oxoethyl) -7-oxo-4,7-dihydro-[1,2,4]triazolo[1,5-a]pyrimidin-6-yl)piperazine-1-carboxylate **44d** (200 mg, 302.26 µmol) in dichloromethane (6 mL), and the reaction was performed at room temperature for 40 min. The mixture was concentrated under reduced pressure to obtain 2-(2-(cyclohept-1-en-1-yl)-5-ethyl-7-oxo-6-(piperazin-1-yl)-[1,2,4]triazolo[1,5-a]pyrimidin-4(7H )-yl)-N-(2-fluoro-4-(trifluoromethyl)phenyl)acetamide **44e** (169 mg) with a yield of 99.56%, which was used directly in the next step.

MS m/z (ESI): 562.3 [M+1]

### Step 5

### 2-(2-(cyclohept-1-en-1-yl)-5-ethyl-6-(4-(3-hydroxyisonicotinoyl)piperazin-1-yl)-7-oxo-[1,2,4] triazolo[1,5-a]pyrimidin-4(7H)-yl)-N-(2-fluoro-4-(trifluoromethyl)phenyl)acetamide

At room temperature, 3-hydroxyisonicotinic acid **9c** (15.61 mg, 112.18 µmol), 1-(3-dimethylaminopropyl)-3-ethylcarbodiimide hydrochloride (28.67 mg, 149.58 µmol), 1-hydroxybenzotriazole (20.21 mg, 149.58 µmol), and N,N-diisopropylethylamine (48.33 mg, 373.95 µmol) were added to N,N-dimethylformamide (1.5 mL), and the reaction was performed at room temperature for 30 min. 2-(2-(cyclohept-1-en-1-yl)-5-ethyl-7-oxo-6 -(piperazin-1-yl)-[1,2,4]triazolo[1,5-a]pyrimidin-4(7H)-yl)-N-(2-fluoro-4-(trifluoromethyl)phenyl )acetamide **44e** (42 mg, 74.79 µmol) was added, and the reaction was performed at room temperature for 16 h. Water (20 mL) was added to the reaction mixture, and the mixture was extracted with ethyl acetate (20 mL ×3). The combined organic phases were washed with saturated sodium chloride solution (20 mL), dried over anhydrous sodium sulfate, filtered, and concentrated under reduced pressure. The resulting residue was purified by preparative liquid chromatography (separation column AKZONOBEL Kromasil; 250×19 mm I.D.; 10 µm, 20 mL/min; mobile phase A: 10 mmol NH₄HCO₃, mobile phase B: CH₃CN) to obtain 2-(2-(cyclohept-1-en-1-yl)-5-ethyl-6-(4-(3-hydroxyisonicotinoyl)piperazin-1-yl)-7-oxo-[1,2,4]tria zolo[1,5-a]pyrimidin-4(7H)-yl)-N-(2-fluoro-4-(trifluoromethyl)phenyl)acetamide **44** (14.02 mg) with a yield of 26.88%.

MS m/z (ESI): 683.2 [M+1]
¹H NMR (400 MHz, DMSO-*d*₆) δ 11.32 (s, 1H), 10.71 (s, 1H), 8.59 - 8.15 (m, 3H), 7.88 - 7.73 (m, 1H), 7.55 (dd, *J =* 13.3, 6.9 Hz, 2H), 7.07 (t, *J =* 6.7 Hz, 1H), 5.30 (s, 2H), 4.55 (s, 1H), 3.48 (q, *J* = 10.6, 9.9 Hz, 2H), 3.31 (q, *J* = 12.2 Hz, 2H), 2.97 (dq, *J* = 14.1, 8.5, 7.6 Hz, 3H), 2.81 (d, *J* = 11.1 Hz, 1H), 2.78 - 2.70 (m, 2H), 2.64 (d, *J* = 11.2 Hz, 1H), 2.31 (q, *J* = 6.2 Hz, 2H), 1.78 (q, *J* = 5.8, 5.3 Hz, 2H), 1.51 (dt, *J* = 12.0, 6.0 Hz, 4H), 1.17 (t, *J* = 7.4 Hz, 3H).

### Example 45

### N-(2-chloro-4-(trifluoromethyl)phenyl)-2-(2-(cyclohept-1-en-1-yl)-5-ethyl-6-(4-(3-hydroxypicolinoyl)piperazin-1-yl)-7-oxo-[1,2,4]triazolo[1,5-a]pyrimidin-4(7H)-yl)acetamide

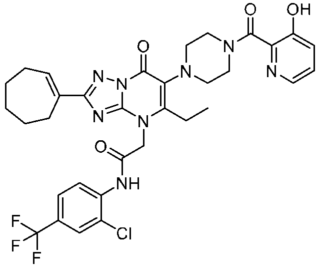

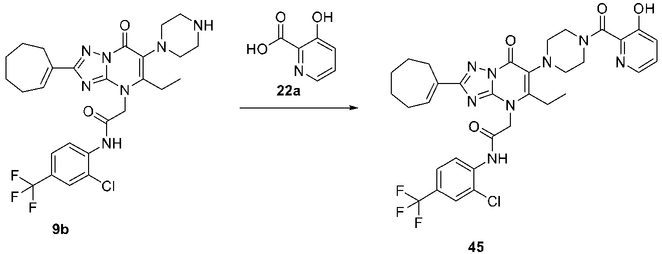

At room temperature, 3-hydroxypicolinic acid **22a** (21.30 mg, 153.11 µmol), 1-(3-dimethylaminopropyl)-3-ethylcarbodiimide hydrochloride (39.13 mg, 204.14 µmol), 1-hydroxybenzotriazole (27.58 mg, 204.14 µmol), and N,N-diisopropylethylamine (65.96 mg, 510.36 µmol) were added to N,N-dimethylformamide (1.5 mL), and the reaction was performed at room temperature for 40 min. A solution of N-(2-chloro-4-(trifluoromethyl) phenyl)-2-(2-(cyclohept-1-en-1-yl)-5-ethyl-7-oxo-6-(piperazin-1-yl)-[1,2,4]triazolo[1,5-a]pyrimi din-4(7H)-yl)acetamide **9b** (59 mg, 102.07 µmol) in N,N-dimethylformamide (2 mL) was added, and the reaction was performed at room temperature for 4 h. Water (20 mL) was added to the reaction mixture, and the mixture was extracted with ethyl acetate (20 mL ×3). The combined organic phases were washed with saturated sodium chloride solution (20 mL), dried over anhydrous sodium sulfate, filtered, and concentrated under reduced pressure. The resulting residue was purified by preparative liquid chromatography (separation column AKZONOBEL Kromasil; 250×19 mm I.D.; 10 µm, 20 mL/min; mobile phase A: 10 mmol NH₄HCO₃, mobile phase B: CH₃CN) to obtain N-(2-chloro-4-(trifluoromethyl)phenyl)-2-(2-(cyclohept -1-en-1-yl)-5-ethyl-6-(4-(3-hydroxypicolinoyl)piperazin-1-yl)-7-oxo-[1,2,4]triazolo[1,5-a]pyrimi din-4(7H)-yl)acetamide **45** (24.45 mg) with a yield of 33.92%.

MS m/z (ESI): 699.3 [M+1]
¹H NMR (400 MHz, DMSO-*d*₆) δ 10.46 (s, 1H), 10.37 (s, 1H), 8.19 - 8.03 (m, 2H), 7.97 (d, *J* = 2.0 Hz, 1H), 7.72 (dd, *J* = 8.7, 2.1 Hz, 1H), 7.43 - 7.25 (m, 2H), 7.10 (t, *J* = 6.8 Hz, 1H), 5.31 (s, 2H), 4.55 (d, *J* = 12.4 Hz, 1H), 3.59 - 3.34 (m, 3H), 3.22 (t, *J* = 13.0 Hz, 1H), 2.97 (h, *J* = 8.4, 7.8 Hz, 3H), 2.85 - 2.67 (m, 3H), 2.62 (d, *J* = 10.9 Hz, 1H), 2.31 (q, *J* = 6.3, 5.7 Hz, 2H), 1.78 (q, *J* = 5.9 Hz, 2H), 1.52 (h, *J* = 8.7, 7.0 Hz, 4H), 1.18 (t, *J* = 7.4 Hz, 3H).

### Example 46

### 2-(2-(cyclohept-1-en-1-yl)-5-ethyl-6-(4-(3-hydroxypicolinoyl)piperazin-1-yl)-7-oxo-[1,2,4] triazolo[1,5-a]pyrimidin-4(7H)-yl)-N-(4-(pentafluoro-λ⁶-sulfaneyl)phenyl)acetamide

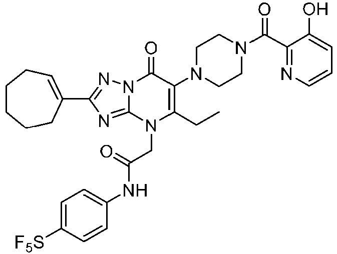

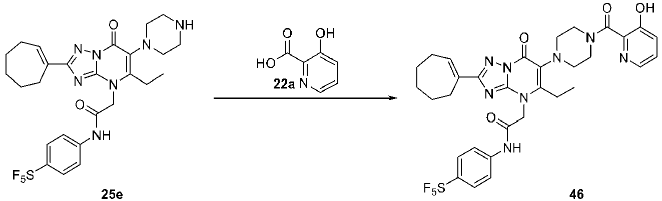

At room temperature, 3-hydroxypicolinic acid **22a** (17.34 mg, 124.66 µmol), 1-(3-dimethylaminopropyl)-3-ethylcarbodiimide hydrochloride (31.86 mg, 166.21 µmol), 1-hydroxybenzotriazole (22.46 mg, 166.21 µmol), and N,N-diisopropylethylamine (53.70 mg, 415.54 µmol) were added to N,N-dimethylformamide (2 mL), and the reaction was performed at room temperature for 40 minutes. Then, 2-(2-(cyclohept-1-en-1-yl)-5-ethyl-7-oxo-6-(piperazin-1-yl)-[1,2,4]triazolo[1,5-a]pyrimidin-4(7H)-yl)-N-(4-(pentafluoro-λ⁶-sulfaneyl)phenyl )acetamide **25e** (50 mg, 83.11 µmol) was added, and the reaction was performed at room temperature for 16 hours. Water (20 mL) was added to the reaction mixture, and the mixture was extracted with ethyl acetate (20 mL ×3). The combined organic phase was washed with saturated sodium chloride solution (20 mL), dried over anhydrous sodium sulfate, filtered, and concentrated under reduced pressure. The resulting residue was purified by preparative liquid chromatography (separation column AKZONOBEL Kromasil; 250×19 mm I.D.; 10 µm, 20 mL/min; mobile phase A: 10 mmol NH₄HCO₃, mobile phase B: CH₃CN) to obtain 2-(2-(cyclohept-1-en-1-yl)-5-ethyl-6-(4-(3-hydroxypicolinoyl)piperazin-1-yl)-7-oxo-[1,2,4]triazo lo[1,5-a]pyrimidin-4(7H)-yl)-N-(4-(pentafluoro-λ⁶-sulfaneyl)phenyl)acetamide **46** (16 mg) in a yield of 26.32%.

MS m/z (ESI): 723.3 [M+1]
¹H NMR (400 MHz, DMSO-*d*₆) δ 10.97 (s, 1H), 10.47 (s, 1H), 8.08 (dd, *J* = 3.8, 2.1 Hz, 1H), 7.88 (d, *J* = 9.0 Hz, 2H), 7.77 (d, *J* = 8.9 Hz, 2H), 7.32 (d, *J* = 3.7 Hz, 2H), 7.04 (t, *J* = 6.8 Hz, 1H), 5.19 (s, 2H), 4.55 (d, *J =* 11.8 Hz, 1H), 3.42 (d, *J =* 15.1 Hz, 2H), 3.23 (t, *J =* 12.0 Hz, 1H), 2.96 (p, *J* = 7.2, 6.5 Hz, 3H), 2.80 (d, *J* = 11.3 Hz, 1H), 2.77 - 2.68 (m, 2H), 2.62 (d, *J* = 11.1 Hz, 1H), 2.30 (q, *J =* 6.3 Hz, 2H), 2.08 (s, 1H), 1.78 (p, *J =* 5.6 Hz, 2H), 1.51 (dp, *J =* 17.2, 6.1, 5.6 Hz, 4H), 1.16 (t, *J* = 7.4 Hz, 3H).

### Example 47

### 2-(2-(cyclohept-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-methyl-4-(trifluoromethyl)phenyl) acetamide

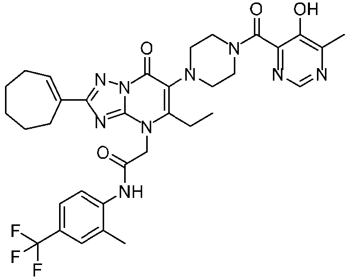

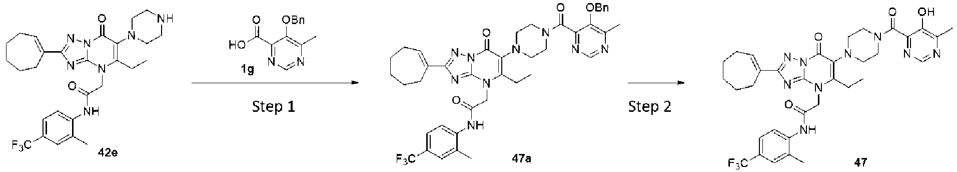

### Step 1

### 2-(6-(4-(5-(benzyloxy)-6-methylpyrimidine-4-carbonyl)piperazin-1-yl)-2-(cyclohept-1-en-1-yl)-5 -ethyl-7-oxo-[1,2,4]triazolo[1,5-a]pyrimidin-4(7H)-yl)-N-(2-methyl-4-(trifluoromethyl)phenyl) acetamide

At room temperature, 5-(benzyloxy)-6-methylpyrimidine-4-carboxylic acid **1g** (55.19 mg, 225.96 µmol), 1-(3-dimethylaminopropyl)-3-ethylcarbodiimide hydrochloride (57.76 mg, 301.29 µmol), 1-hydroxybenzotriazole (40.71 mg, 301.29 µmol), and N,N-diisopropylethylamine (97.35 mg, 753.22 µmol) were added to N,N-dimethylformamide (1.5 mL), and the reaction was performed at room temperature for 40 minutes. Then, a solution of 2-(2-(cyclohept-1-en-1-yl)-5-ethyl-7-oxo-6-(piperazin-1-yl)-[1,2,4]triazolo[1,5-a]pyrimidin-4(7H )-yl)-N-(2-methyl-4-(trifluoromethyl)phenyl)acetamide **42e** (84 mg, 150.64 µmol) in N,N-dimethylformamide (2 mL) was added, and the reaction was performed at room temperature for 16 hours. Water (20 mL) was added to the reaction mixture, and the mixture was extracted with ethyl acetate (20 mL × 3). The combined organic phase was washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-2-(cyclohept-1-en-1-yl)-5 -ethyl-7-oxo-[1,2,4]triazolo[1,5-a]pyrimidin-4(7H)-yl)-N-(2-methyl-4-(trifluoromethyl)phenyl)ac etamide **47a** (118 mg) in a yield of 99.93%.

MS m/z (ESI): 784.3 [M+1]

### Step 2

### 2-(2-(cyclohept-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-methyl-4-(trifluoromethyl)phenyl)aceta mide

Under an ice bath and argon atmosphere, boron trichloride (1.5 mL) was added to a solution of 2-(6-(4-(5-(benzyloxy)-6-methylpyrimidine-4-carbonyl)piperazin-1-yl)-2-(cyclohept-1-en-1-yl)-5 -ethyl-7-oxo-[1,2,4]triazolo[1,5-a]pyrimidin-4(7H)-yl)-N-(2-methyl-4-(trifluoromethyl)phenyl)ac etamide **47a** (118 mg, 150.54 µmol) in dichloromethane (1 mL), and the reaction was performed at room temperature for 16 hours. The reaction was quenched with methanol, concentrated, dissolved in methanol, and purified by preparative liquid chromatography (separation column AKZONOBEL Kromasil; 250×19 mm I.D.; 10 µm, 20 mL/min; mobile phase A: 10 mmol NH₄HCO₃, mobile phase B: CH₃CN) to obtain 2-(2-(cyclohept-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-methyl-4-(trifluoromethyl)phenyl)aceta mide **47** (36.41 mg) in 34.31%.

MS m/z (ESI): 694.3 [M+1]
¹H NMR (400 MHz, DMSO-*d*₆) δ 10.25 (s, 1H), 10.02 (s, 1H), 8.58 (s, 1H), 7.72 (d, *J* = 8.4 Hz, 1H), 7.63 (d, *J* = 2.1 Hz, 1H), 7.53 (dd, *J* = 8.5, 2.2 Hz, 1H), 7.10 (t, *J* = 6.8 Hz, 1H), 5.24 (s, 2H), 4.53 (d, *J* = 12.4 Hz, 1H), 3.57 - 3.40 (m, 3H), 3.25 (t, *J* = 12.1 Hz, 1H), 2.99 (q, *J* = 9.1, 8.6 Hz, 3H), 2.81 (d, *J =* 11.3 Hz, 1H), 2.75 (d, *J =* 8.6 Hz, 2H), 2.63 (d, *J =* 10.6 Hz, 1H), 2.45 (s, 3H), 2.36 (s, 3H), 2.35 - 2.27 (m, 2H), 1.79 (d, *J =* 7.0 Hz, 2H), 1.53 (dt, *J* = 11.4, 5.5 Hz, 4H), 1.19 (t, *J* = 7.4 Hz, 3H).

### Example 48

### 2-(2-(cyclohept-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-methoxy-4-(trifluoromethyl)phenyl)ace tamide

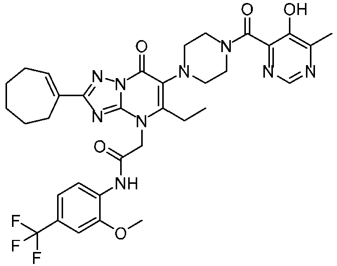

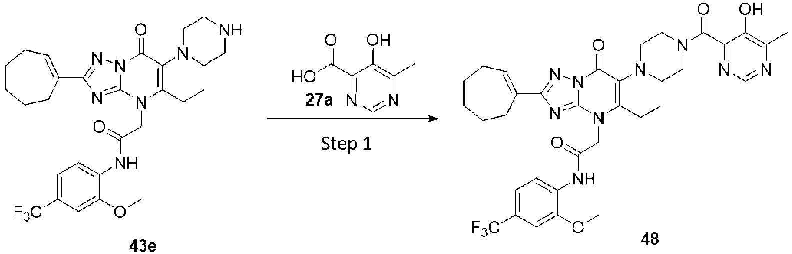

At room temperature, 5-hydroxy-6-methylpyrimidine-4-carboxylic acid **27a** (28.21 mg, 183.05 µmol), 1-(3-dimethylaminopropyl)-3-ethylcarbodiimide hydrochloride (46.79 mg, 244.07 µmol), 1-hydroxybenzotriazole (32.98 mg, 244.07 µmol), and N,N-diisopropylethylamine (78.86 mg, 610.17 µmol) were added to N,N-dimethylformamide (2 mL), and the reaction was performed at room temperature for 40 minutes. 2-(2-(Cyclohept-1-en-1-yl)-5-ethyl-7-oxo-6-(piperazin-1-yl)-[1,2,4]triazolo[1,5-a]pyrimidin-4(7 H)-yl)-N-(2-methoxy-4-(trifluoromethyl)phenyl)acetamide **43e** (70 mg, 122.03 µmol) was added, and the reaction was performed at room temperature for 48 hours. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by preparative liquid chromatography (separation column: AKZONOBEL Kromasil; 250×19 mm I.D.; 10 µm, 20 mL/min; mobile phase A: 10 mmol NH₄HCO₃, mobile phase B: CH₃CN) to obtain 2-(2-(cyclohept-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-methoxy-4-(trifluoromethyl)phenyl)ace tamide **48** (4.3 mg) in a yield of 4.91%.

MS m/z (ESI): 710.3 [M+1]
¹H NMR (400 MHz, DMSO-*d*₆) δ 10.24 (s, 1H), 10.12 (s, 1H), 8.59 (s, 1H), 8.20 (d, *J =* 8.4 Hz, 1H), 7.37 (d, *J* = 1.9 Hz, 1H), 7.28 (dd, *J =* 8.6, 1.9 Hz, 1H), 7.06 (t, *J =* 6.8 Hz, 1H), 5.31 (s, 2H), 4.52 (d, *J* = 12.5 Hz, 1H), 3.99 (s, 3H), 3.50 (q, *J* = 11.0, 10.1 Hz, 3H), 3.24 (t, *J* = 12.7 Hz, 1H), 3.06 - 2.87 (m, 3H), 2.81 (d, *J* = 11.5 Hz, 1H), 2.77 - 2.69 (m, 2H), 2.63 (d, *J* = 11.0 Hz, 1H), 2.45 (s, 3H), 2.35 - 2.24 (m, 2H), 1.86 - 1.72 (m, 2H), 1.53 (dt, *J* = 13.5, 6.0 Hz, 4H), 1.16 (t, *J* = 7.4 Hz, 3H).

### Example 49

### 2-(2-(cyclohept-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-fluoro-4-(trifluoromethyl)phenyl)aceta mide

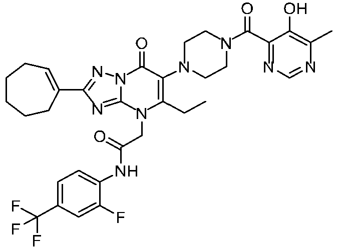

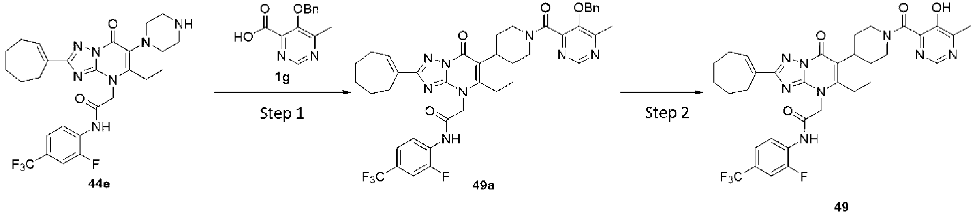

### Step 1

### 2-(6-(1-(5-(benzyloxy)-6-methylpyrimidine-4-carbonyl)piperidin-4-yl)-2-(cyclohept-1-en-1-yl)-5 -ethyl-7-oxo-[1,2,4]triazolo[1,5-a]pyrimidin-4(7H)-yl)-N-(2-fluoro-4-(trifluoromethyl)phenyl)ace tamide

At room temperature, 5-(benzyloxy)-6-methylpyrimidine-4-carboxylic acid **1g** (82.85 mg, 339.23 µmol), 1-(3-dimethylaminopropyl)-3-ethylcarbodiimide hydrochloride (86.71 mg, 452.30 µmol), 1-hydroxybenzotriazole (61.11 mg, 452.30 µmol), and N,N-diisopropylethylamine (146.14 mg, 1.13 mmol) were added to N,N-dimethylformamide (2 mL), and the reaction was performed at room temperature for 30 minutes. 2-(2-(Cyclohept-1-en-1-yl)-5-ethyl-7-oxo-6-(piperazin-1-yl)-[1,2,4]triazolo[1,5-a]pyrimidin-4(7 H)-yl)-N-(2-fluoro-4-(trifluoromethyl)phenyl)acetamide **44e** (127 mg, 226.15 µmol) was added, and the reaction was performed at room temperature for 16 hours. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(1-(5-(benzyloxy)-6-methylpyrimidine-4-carbonyl)piperidin-4-yl)-2-(cyclohept-1-en-1-yl)-5 -ethyl-7-oxo-[1,2,4]triazolo[1,5-a]pyrimidin-4(7H)-yl)-N-(2-fluoro-4-(trifluoromethyl)phenyl)ace tamide **49a** (178 mg) in a yield of 99.91%.

MS m/z (ESI): 788.3 [M+1]

### Step 2

### 2-(2-(cyclohept-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-fluoro-4-(trifluoromethyl)phenyl)aceta mide

Under an ice bath and argon atmosphere, boron trichloride (2 mL) was added to a solution of 2-(6-(1-(5-(benzyloxy)-6-methylpyrimidine-4-carbonyl)piperidin-4-yl)-2-(cyclohept-1-en-1-yl)-5 -ethyl-7-oxo-[1,2,4]triazolo[1,5-a]pyrimidin-4(7H)-yl)-N-(2-fluoro-4-trifluoromethyl)phenyl) acetamide **49a** (80 mg, 101.55 µmol) in dichloromethane (2 mL), and the reaction was performed at room temperature for 3 hours. The reaction was quenched with ice water. After concentration, the residue was dissolved in methanol and purified by preparative liquid chromatography (separation column: AKZONOBEL Kromasil; 250×19 mm I.D.; 10 µm, 20 mL/min; mobile phase A: 10 mmol NH₄HCO₃, mobile phase B: CH₃CN) to obtain 2-(2-(cyclohept-1-en-1-yl)-5-ethyl-6-(4-(5-hydroxy-6-methylpyrimidine-4-carbonyl)piperazin-1-yl)-7-oxo-[1,2,4]triazolo[1,5-] pyrimidin-4(7H)-yl)-N-(2-fluoro-4-(trifluoromethyl)phenyl)acetamide **49** (9.51 mg) in a yield of 13.29%.

MS m/z (ESI): 698.3 [M+1]
¹H NMR (400 MHz, DMSO-*d*₆) δ 10.70 (s, 1H), 10.27 (s, 1H), 8.59 (s, 1H), 8.23 (t,*J* = 8.2 Hz, 1H), 7.80 (dd, *J* = 11.0, 2.0 Hz, 1H), 7.57 (dd, *J =* 8.7, 2.0 Hz, 1H), 7.07 (t, *J* = 6.8 Hz, 1H), 5.29 (s, 2H), 4.52 (d, *J* = 12.5 Hz, 1H), 3.52 (s, 1H), 3.48 (d, *J* = 9.9 Hz, 2H), 3.34 - 3.17 (m, 1H), 3.07 - 2.89 (m, 3H), 2.81 (d, *J* = 11.4 Hz, 1H), 2.76 - 2.71 (m, 2H), 2.64 (d, *J* = 11.0 Hz, 1H), 2.45 (s, 3H), 2.31 (q, *J* = 6.4 Hz, 2H), 1.78 (q, *J* = 5.0, 3.7 Hz, 2H), 1.53 (dt, *J* = 13.4, 6.0 Hz, 4H), 1.17 (t, *J* = 7.4 Hz, 3H).

### Example 50

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(spiro[3.3]hept-1-en-2-yl)-[1,2,4]triazolo[1,5-a]pyrimidin-4(7H)-yl)ac etamide

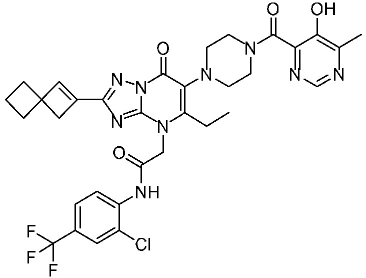

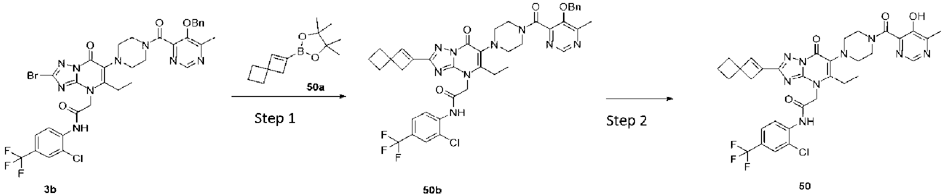

### Step 1

### 2-(6-(4-(5-(benzyloxy)-6-methylpyrimidine-4-carbonyl)piperazin-1-yl)-5-ethyl-7-oxo-2-(spiro[3. 3]hept-1-en-2-yl)-[1,2,4]triazolo[1,5-a]pyrimidin-4(7H)-yl)-N-(2-chloro-4-(trifluoromethyl)phen yl)acetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (50 mg, 63.37 µmol), 4,4,5,5-tetramethyl-2-(spiro[3.3]hept-1-en-2-yl)-1,3,2-dioxaborolane **50a** (16.74 mg, 76.05 µmol, prepared according to published patent WO2020128768), [1,1'-bis(diphenylphosphino)ferrocene]palladium(II) dichloride dichloromethane complex (5.14 mg, 6.34 µmol), sodium carbonate (20.15 mg, 190.11 µmol), and 1,4-dioxane (1 mL) were added to a 10 mL two-necked flask. The mixture was subject to argon replacement four times, and the temperature was raised to 80 °C. The reaction was performed for 1.5 hours. Water (20 mL) was added to the reaction mixture, and the mixture was extracted with ethyl acetate (40 mL × 3). The organic phases were combined, washed with saturated sodium chloride solution (20 mL), dried over anhydrous sodium sulfate, filtered, and concentrated under reduced pressure. The obtained residue was purified by silica gel column chromatography (eluent: System B) to obtain 2-(6-(4-(5-(benzyloxy)-6-methylpyrimidine-4-carbonyl)piperazin-1-yl)-5-ethyl-7-oxo-2-(spiro[3. 3]hept-1-en-2-yl)-[1,2,4]triazolo[1,5-a]pyrimidin-4(7H)-yl)-N-(2-chloro-4-(trifluoromethyl)phen yl)acetamide **50b** (39 mg). Yield: 76.71%.

MS m/z (ESI): 801.7 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(spiro[3.3]hept-1-en-2-yl)-[1,2,4]triazolo[1,5-a]pyrimidin-4(7H)-yl)ac etamide

2-(6-(4-(5-(benzyloxy)-6-methylpyrimidine-4-carbonyl)piperazin-1-yl)-5-ethyl-7-oxo-2 -(spiro[3.3]hept-1-en-2-yl)-[1,2,4]triazolo[1,5-a]pyrimidin-4(7H)-yl)-N-(2-chloro-4-(trifluoromet hyl)phenyl)acetamide **50b** (39 mg, 48.61 µmol) was added to dichloromethane (2.5 mL). Boron trichloride (0.8 mL) was added at 0 °C, and the reaction was performed at 0 °C for 1 hour. Methanol (2 mL) was added to quench the reaction, and the mixture was concentrated under reduced pressure. The obtained residue was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(spiro[3.3]hept-1-en-2-yl)-[1,2,4]triazolo[1,5-a]pyrimidin-4(7H)-yl)ac etamide **50** (2.87 mg). Yield: 8.12%.

MS m/z (ESI): 711.7 [M+1]
¹H NMR (400 MHz, DMSO-*d*₆) δ 10.35 (s, 1H), 10.24 (s, 1H), 8.58 (s, 1H), 8.08 (d,*J* = 8.6 Hz, 1H), 7.97 (d, *J =* 2.1 Hz, 1H), 7.71 (dd, *J* = 8.9, 2.1 Hz, 1H), 6.75 (s, 1H), 5.32 (s, 2H), 4.52 (d, *J =* 12.6 Hz, 1H), 3.48 (t, *J =* 11.7 Hz, 3H), 3.23 (d, *J* = 12.5 Hz, 1H), 3.04 - 2.93 (m, 3H), 2.81 (s, 3H), 2.63 (d, *J* = 10.8 Hz, 1H), 2.44 (s, 3H), 2.26 - 2.12 (m, 4H), 1.94 - 1.79 (m, 2H), 1.18 (t, *J* = 7.4 Hz, 3H).

### Example 51

### N-(2-chloro-4-(trifluoromethyl)phenyl)-2-(5-ethyl-6-(4-(3-hydroxypicolinoyl)piperazin-1-yl)-7-o xo-2-(spiro[3.3]hept-1-en-2-yl)-[1,2,4]triazolo[1,5-a]pyrimidin-4(7H)-yl)acetamide

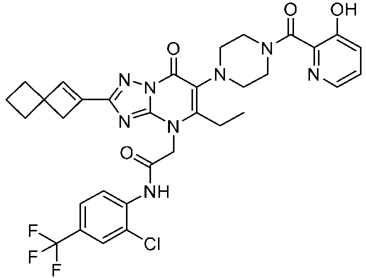

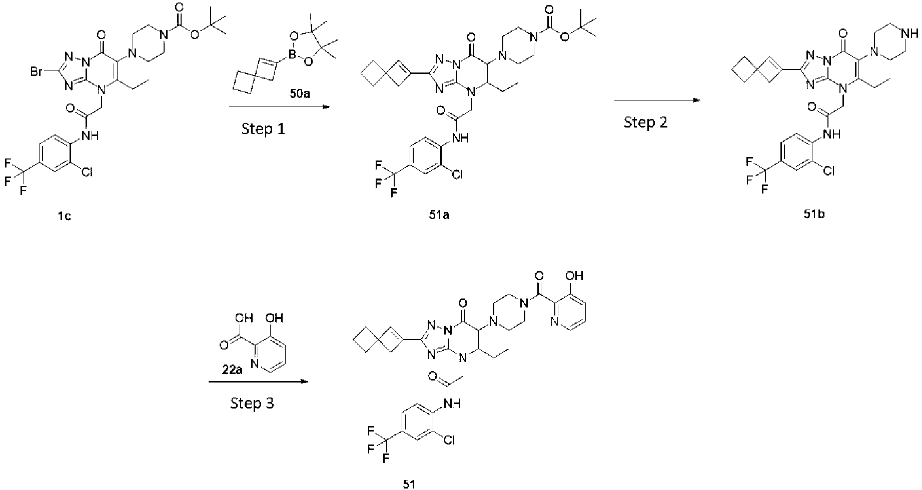

### Step 1

### tert-butyl 4-(4-(2-((2-chloro-4-(trifluoromethyl)phenyl)amino)-2-oxoethyl)-5-ethyl-7-oxo-2-(spiro[3.3]hept -1-en-2-yl)-4,7-dihydro-[1,2,4]triazolo[1,5-a]pyrimidin-6-yl)piperazine-1-carboxylate

At room temperature, tert-butyl 4-(2-bromo-4-(2-((2-chloro-4-(trifluoromethyl)phenyl) amino)-2-oxoethyl)-5-ethyl-7-oxo-4,7-dihydro-[1,2,4]triazolo[1,5-a]pyrimidin-6-yl)piperazine-1-carboxylate **1c** (50 mg, 75.43 µmol), 4,4,5,5-tetramethyl-2-(spiro[3.3]hept-1-en-2-yl)-1,3,2-dioxaborolane **50a** (19.92 mg, 90.51 µmol), [1,1'-bis(diphenylphosphino)ferrocene]palladium(II) dichloride dichloromethane complex (6.11 mg, 7.54 µmol), sodium carbonate (23.98 mg, 226.28 µmol), and 1,4-dioxane (1 mL) were added to a 10 mL two-necked flask. The mixture was subject to argon replacement four times, and the temperature was raised to 80 °C. The reaction was performed for 1.5 hours. Water (20 mL) was added to the reaction mixture, and the mixture was extracted with ethyl acetate (40 mL × 3). The organic phases were combined, washed with saturated sodium chloride solution (20 mL), dried over anhydrous sodium sulfate, filtered, and concentrated under reduced pressure. The obtained residue was purified by silica gel column chromatography (eluent: System B) to obtain tert-butyl 4-(4-(2-((2-chloro-4-(trifluoromethyl)phenyl)amino)-2-oxoethyl)-5-ethyl-7-oxo-2-(spiro[3.3]hept -1-en-2-yl)-4,7-dihydro-[1,2,4]triazolo[1,5-a]pyrimidin -6-yl)piperazine-1-carboxylate **51a** (47 mg). Yield: 92.16%.

MS m/z (ESI): 676.2 [M+1]

### Step 2

### N-(2-chloro-4-(trifluoromethyl)phenyl)-2-(5-ethyl-7-oxo-6-(piperazin-1-yl)-2-(spiro[3.3]hept-1-e n-2-yl)-[1,2,4]triazolo[1,5-a]pyrimidin-4(7H)-yl)acetamide

tert-butyl 4-(4-(2-((2-chloro-4-(trifluoromethyl)phenyl)amino)-2-oxoethyl)-5-ethyl-7-oxo-2-(spiro[3.3]hept -1-en-2-yl)-4,7-dihydro-[1,2,4]triazolo[1,5-a]pyrimidin-6-yl)piperazine-1-carboxylate **51a** (47 mg, 69.51 µmol) was added to dichloromethane (2.5 mL). Trifluoroacetic acid (0.8 mL) was added, and the reaction was performed at room temperature for 1 hour. The mixture was concentrated under reduced pressure to obtain N-(2-chloro-4-(trifluoromethyl)phenyl)-2-(5-ethyl-7-oxo-6-(piperazin-1-yl)-2-(spiro[3.3]hept-1-e n-2-yl)-[1,2,4]triazolo[1,5-a]pyrimidin-4(7H) -yl)acetamide **51b** (40 mg), which was used directly in the next step.

MS m/z (ESI): 576.2 [M+1]

### Step 3

### N-(2-chloro-4-(trifluoromethyl)phenyl)-2-(5-ethyl-6-(4-(3-hydroxypicolinoyl)piperazin-1-yl)-7-o xo-2-(spiro[3.3]hept-1-en-2-yl)-[1,2,4]triazolo[1,5-a]pyrimidin-4(7H)-yl)acetamide

N-(2-chloro-4-(trifluoromethyl)phenyl)-2-(5-ethyl-7-oxo-6-(piperazin-1-yl)-2-(spiro [3.3]hept-1-en-2-yl)-[1,2,4]triazolo[1,5-a]pyrimidin-4(7H)-yl)acetamide **51b** (40 mg, 69.44 µmol), 3-hydroxypicolinic acid **22a** (12.56 mg, 90.28 µmol), 1-(3-dimethylaminopropyl)-3-ethylcarbodiimide hydrochloride (19.97 mg, 104.16 µmol), 1-hydroxybenzotriazole (14.07 mg, 104.16 µmol), and N,N-diisopropylethylamine (44.87 mg, 347.21 µmol) were added to N,N-dimethylformamide (2 mL), and the reaction was performed at room temperature for 4 h. The reaction mixture was extracted with ethyl acetate (30 mL ×3), the aqueous layer was separated, and the combined organic phase was washed with saturated sodium chloride solution (30 mL ×2), dried over anhydrous sodium sulfate, filtered, and concentrated under reduced pressure. The obtained residue was purified by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(5-ethyl-6-(4-(3-hydroxypicolinoyl)piperazin-1-yl)-7-o xo-2-(spiro[3.3]hept-1-en-2-yl)-[1,2,4]triazolo[1,5-a]pyrimidin-4(7H)-yl)acetamide **51** (3.30 mg), yield: 6.77%.

MS m/z (ESI): 696.7 [M+1]
¹H NMR (400 MHz, DMSO-*d*₆) δ 10.46 (s, 1H), 10.34 (s, 1H), 8.11-8.03 (m, 2H), 7.97 (d, *J* = 2.1 Hz, 1H), 7.71 (dd, *J* = 8.8, 2.1 Hz, 1H), 7.38-7.28 (m, 2H), 6.75 (s, 1H), 5.32 (s, 2H), 4.54 (d, *J* = 12.7 Hz, 1H), 3.44 (dd, *J =* 21.8, 11.7 Hz, 3H), 3.22 (t, *J =* 12.2 Hz, 1H), 2.99 (d, *J* = 5.6 Hz, 3H), 2.81 (s, 3H), 2.69-2.53 (m, 1H), 2.46-2.43 (m, 1H), 2.19 (q, *J* = 6.4 Hz, 4H), 1.93-1.81 (m, 2H), 1.18 (t, *J =* 7.4 Hz, 3H).

### Example 52

### 2-(2-(cyclobutylidenemethyl)-5-ethyl-6-(4-(3-hydroxypicolinoyl)piperazin-1-yl)-7-oxo-[1,2,4]tri azolo[1,5-a]pyrimidin-4(7H)-yl)-N-(4-(pentafluoro-λ⁶-sulfaneyl)phenyl)acetamide

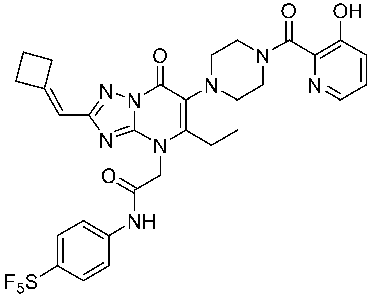

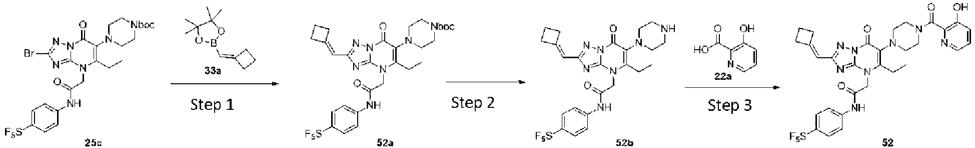

### Step 1

### tert-butyl 4-(2-(cyclobutylidenemethyl)-5-ethyl-7-oxo-4-(2-oxo-2-((4-(pentafluoro-λ⁶-sulfaneyl)phenyl)ami no)ethyl)-4,7-dihydro-[1,2,4]triazolo[1,5-a]pyrimidin-6-yl)piperazine-1-carboxylate

At room temperature, tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)piperazine-1-carboxylate **25c** (85 mg, 123.82 µmol), 2-(cyclobutylidenemethyl)-4,4,5,5-tetramethyl-1,3,2-dioxaborolane **33a** (48.06 mg, 247.64 µmol), [1,1'-bis(diphenylphosphino)ferrocene]palladium dichloride (10.03 mg, 12.38 µmol), and sodium carbonate (26.25 mg, 247.64 µmol) were added to a mixture of water (0.5 mL) and 1,4-dioxane (2 mL). After replacing with argon 3 times, the temperature was raised to 80 °C, and the mixture was stirred for 3 h. Water (50 mL) was added to the reaction mixture, which was extracted with dichloromethane (50 mL ×3). The combined organic phase was washed with saturated sodium chloride solution, dried over anhydrous sodium sulfate, filtered, and concentrated. The residue was purified by silica gel column chromatography (eluent: System B) to obtain tert-butyl 4-(2-(cyclobutylidenemethyl)-5-ethyl-7-oxo-4-(2-oxo-2-((4-(pentafluoro-λ⁶-sulfaneyl)phenyl)ami no)ethyl)-4,7-dihydro-[1,2,4]triazolo[1,5-a]pyrimidin-6-yl)piperazine-1-carboxylate **52a** (60 mg), yield 71.93%.

MS m/z (ESI): 617.8 [M+H-56]

### Step 2

### 2-(2-(cyclobutylidenemethyl)-5-ethyl-7-oxo-6-(piperazin-1-yl)-[1,2,4]triazolo[1,5-a]pyrimidin-4( 7H)-yl)-N-(4-(pentafluoro-λ⁶-sulfaneyl)phenyl)acetamide

tert-butyl 4-(2-(cyclobutylidenemethyl)-5-ethyl-7-oxo-4-(2-oxo-2-((4-(pentafluoro-λ⁶-sulfaneyl)phenyl)ami no)ethyl)-4,7-dihydro-[1,2,4]triazolo[1,5-a]pyrimidin-6-yl)piperazine-1-carboxylate **52a** (65 mg, 96.48 µmol) was dissolved in dichloromethane (2 mL), then trifluoroacetic acid (0.5 mL) was added dropwise, and the reaction was performed at room temperature for 2 h. The mixture was concentrated under reduced pressure to obtain 2-(2-(cyclobutylidenemethyl)-5-ethyl-7-oxo-6-(piperazin-1-yl)-[1,2,4]triazolo[1,5-a]pyrimidin-4( 7H)-yl)-N-(4-(pentafluoro-λ⁶-sulfaneyl)phenyl)acetamide **52b** (50 mg), yield 90.35%, which was used directly in the next step.

MS m/z (ESI): 573.8 [M+H]

### Step 3

### 2-(2-(cyclobutylidenemethyl)-5-ethyl-6-(4-(3-hydroxypicolinoyl)piperazin-1-yl)-7-oxo-[1,2,4]tri azolo[1,5-a]pyrimidin-4(7H)-yl)-N-(4-(pentafluoro-λ⁶-sulfaneyl)phenyl)acetamide

3-Hydroxypicolinic acid **22a** (21.83 mg, 156.91 µmol), 1-(3-dimethylaminopropyl)-3-ethylcarbodiimide hydrochloride (33.42 mg, 174.34 µmol), 1-hydroxybenzotriazole (23.56 mg, 174.34 µmol), and N,N-diisopropylethylamine (56.33 mg, 435.86 µmol) were dissolved in N,N-dimethylformamide (1 mL), and the reaction was performed at room temperature for 10 min. Then 2-(2-(cyclobutylidenemethyl)-5-ethyl-7-oxo-6-(piperazin-1-yl)-[1,2,4]triazolo[1,5-a]pyrimidin-4( 7H)-yl)-N-(4-(pentafluoro-λ⁶-sulfaneyl)phenyl)acetamide **52b** (50 mg, 87.17 µmol) was added, and the reaction was performed at room temperature for 16 h. The reaction mixture was extracted with ethyl acetate (30 mL ×2), the aqueous layer was separated, and the combined organic phase was washed with saturated sodium chloride solution (20 mL ×2), dried over anhydrous sodium sulfate, and concentrated under reduced pressure. The obtained residue was purified by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain 2-(2-(cyclobutylidenemethyl)-5-ethyl-6-(4-(3-hydroxypicolinoyl)piperazin-1-yl)-7-oxo-[1,2,4]tri azolo[1,5-a]pyrimidin-4(7H)-yl)-N-(4-(pentafluoro-λ⁶-sulfaneyl)phenyl)acetamide **52** (15 mg), yield 23.53%.

MS m/z (ESI): 694.7 [M+H]
1H NMR (400 MHz, DMSO-d6) δ 10.95 (s, 1H), 10.45 (s, 1H), 8.08 (dd, J = 3.8, 2.2 Hz, 1H), 7.91 - 7.84 (m, 2H), 7.76 (d, J = 8.9 Hz, 2H), 7.35 - 7.26 (m, 2H), 6.07 (p, J = 2.4 Hz, 1H), 5.16 (s, 2H), 4.55 (d, J = 12.5 Hz, 1H), 3.57 - 3.36 (m, 4H), 3.22 (t, J = 12.2 Hz, 1H), 3.06 - 2.91 (m, 5H), 2.82 (q, J = 13.9, 10.9 Hz, 3H), 2.62 (d, J = 11.0 Hz, 1H), 1.97 (p, J = 7.9 Hz, 2H), 1.17 (t, J = 7.4 Hz, 3H).

### Example 53

### 2-(2-(cyclobutylidenemethyl)-5-ethyl-6-(4-(5-hydroxy-6-methylpyrimidine-4-carbonyl)piperazin -1-yl)-7-oxo-[1,2,4]triazolo[1,5-a]pyrimidin-4(7H)-yl)-N-(4-(pentafluoro-λ⁶-sulfaneyl)phenyl)ace tamide

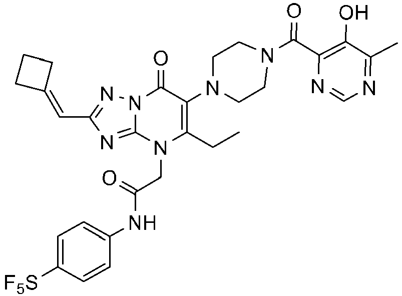

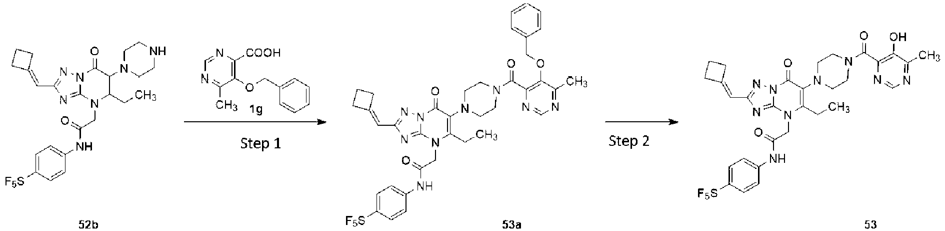

### Step 1

### 2-(6-(4-(5-(benzyloxy)-6-methylpyrimidine-4-carbonyl)piperazin-1-yl)-2-(cyclobutylidenemethyl )-5-ethyl-7-oxo-[1,2,4]triazolo[1,5-a]pyrimidin-4(7H)-yl)-N-(4-(pentafluoro-λ⁶-sulfaneyl)phenyl) acetamide

5-(Benzyloxy)-6-methylpyrimidine-4-carboxylic acid **1g** (29.81 mg, 122.04 µmol), 1-(3-dimethylaminopropyl)-3-ethylcarbodiimide hydrochloride (33.42 mg, 174.34 µmol), 1-hydroxybenzotriazole (23.56 mg, 174.34 µmol), and N,N-diisopropylethylamine (56.33 mg, 435.86 µmol) were sequentially added to N,N-dimethylformamide (1.5 mL), and the reaction was performed at room temperature for 10 minutes, followed by addition of 2-(2-(cyclobutylidenemethyl)-5-ethyl-7-oxo-6-(piperazin-1-yl)-[1,2,4]triazolo[1,5-a]pyrimidin-4( 7H)-yl)-N-(4-(pentafluoro-λ⁶-sulfaneyl)phenyl)acetamide **52b** (50 mg, 87.17 µmol), and the reaction was performed at room temperature for 16 hours. Water (50 mL) was added to the reaction mixture, and the mixture was extracted with dichloromethane (50 mL × 3). The combined organic phases were washed with saturated sodium chloride solution, dried over anhydrous sodium sulfate, filtered, and concentrated. The residue was purified by silica gel column chromatography (eluent: System B) to obtain 2-(6-(4-(5-(benzyloxy)-6-methylpyrimidine-4-carbonyl)piperazin-1-yl)-2-(cyclobutylidenemethyl )-5-ethyl-7-oxo-[1,2,4]triazolo[1,5-a]pyrimidin-4(7H)-yl)-N-(4-(pentafluoro-λ⁶-sulfaneyl)phenyl) acetamide **53a** (40 mg) in a yield of 57.37%.

MS m/z (ESI): 799.7 [M+H]

### Step 2

### 2-(2-(cyclobutylidenemethyl)-5-ethyl-6-(4-(5-hydroxy-6-methylpyrimidine-4-carbonyl)piperazin -1-yl)-7-oxo-[1,2,4]triazolo[1,5-a]pyrimidin-4(7H)-yl)-N-(4-(pentafluoro-λ⁶-sulfaneyl)phenyl)ace tamide

2-(6-(4-(5-(Benzyloxy)-6-methylpyrimidine-4-carbonyl)piperazin-1-yl)-2-(cyclobutylid enemethyl)-5-ethyl-7-oxo-[1,2,4]triazolo[1,5-a]pyrimidin-4(7H)-yl)-N-(4-(pentafluoro-λ⁶-sulfane yl)phenyl)acetamide **53a** (40 mg, 50.01 µmol) was dissolved in dichloromethane (2 mL), followed by dropwise addition of trifluoroacetic acid (1 mL), and the reaction was performed at room temperature for 2 hours. The resulting residue was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain 2-(2-(cyclobutylidenemethyl)-5-ethyl-6-(4-(5-hydroxy-6-methylpyrimidine-4-carbonyl)piperazin -1-yl)-7-oxo-[1,2,4]triazolo[1,5-a]pyrimidin-4(7H)-yl)-N-(4-(pentafluoro-λ⁶-sulfaneyl)phenyl) acetamide **53** (15 mg) in a yield of 40.15%.

MS m/z (ESI): 709.7 [M+H]
1H NMR (400 MHz, DMSO-d6) δ 10.95 (s, 1H), 10.22 (s, 1H), 8.58 (s, 1H), 7.88 (d, J = 9.3 Hz, 2H), 7.76 (d, J = 8.9 Hz, 2H), 6.10 - 6.05 (m, 1H), 5.16 (s, 2H), 4.52 (d, J = 12.6 Hz, 1H), 3.57 -3.44 (m, 3H), 3.01 (d, J = 9.9 Hz, 5H), 2.82 (d, J = 15.0 Hz, 3H), 2.63 (d, J = 11.3 Hz, 1H), 2.44 (s, 3H), 1.97 (p, J = 7.9 Hz, 2H), 1.17 (t, J = 7.4 Hz, 3H).

### Example 54

### 2-(2-(benzo[b]thiophen-5-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acet amide

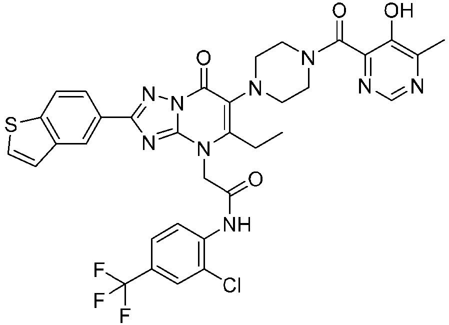

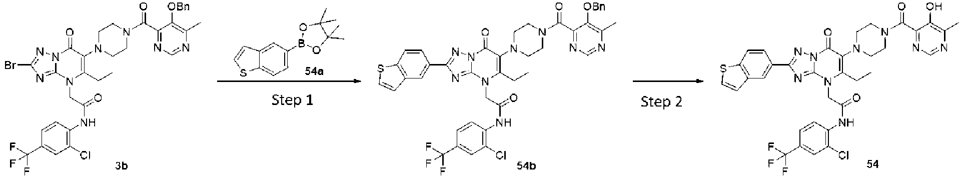

### Step 1

### 2-(2-(benzo[b]thiophen-5-yl)-6-(4-(5-(benzyloxy)-6-methylpyrimidine-4-carbonyl)piperazin-1-yl )-5-ethyl-7-oxo-[1,2,4]triazolo[1,5-a]pyrimidin-4(7H)-yl)-N-(2-chloro-4-(trifluoromethyl)phenyl) acetamide

2-(6-(4-(5-(Benzyloxy)-6-methylpyrimidine-4-carbonyl)piperazin-1-yl)-2-bromo-5-ethy 1-7-oxo-[1,2,4]triazolo[1,5-a]pyrimidin-4(7H)-yl)-N-(2-chloro-4-(trifluoromethyl)phenyl) acetamide **3b** (60 mg, 76.05 µmol), 2-(benzo[b]thiophen-5-yl)-4,4,5,5-tetramethyl-1,3,2-dioxaborolane **54a** (25.72 mg, 98.86 µmol, commercially available), [1,1'-bis(diphenylphosphino)ferrocene]palladium(II) dichloride (6.16 mg, 7.60 µmol), and sodium carbonate (16.12 mg, 152.09 µmol) were added to 1,4-dioxane (1.5 mL), The mixture was subject to argon replacement, and the temperature was raised to 80 °C, and the reaction was performed for 2 hours. Water (50 mL) was added, and the mixture was extracted with dichloromethane (50 mL × 3). The combined organic phases were washed with saturated sodium chloride solution, dried over anhydrous sodium sulfate, filtered, and concentrated. The residue was purified by silica gel column chromatography (eluent: System B) to obtain 2-(2-(benzo[b]thiophen-5-yl)-6-(4-(5-(benzyloxy)-6-methylpyrimidine-4-carbonyl)piperazin-1-yl )-5-ethyl-7-oxo-[1,2,4]triazolo[1,5-a]pyrimidin-4(7H)-yl)-N-(2-chloro-4-(trifluoromethyl)phenyl) acetamide **54b** (50 mg) in a yield of 78.06%.

MS m/z (ESI): 843.2 [M+H]

### Step 2

### 2-(2-(benzo[b]thiophen-5-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acet amide

2-(2-(benzo[b]thiophen-5-yl)-6-(4-(5-(benzyloxy)-6-methylpyrimidine-4-carbonyl) piperazin-1-yl)-5-ethyl-7-oxo-[1,2,4]triazolo[1,5-a]pyrimidin-4(7H)-yl)-N-(2-chloro-4-(trifluoro methyl)phenyl)acetamide **54b** (50 mg, 59.36 µmol) was dissolved in trifluoroacetic acid (3 mL), heated to 40 °C, and stirred for 4 h. The mixture was concentrated under reduced pressure, and the resulting residue was purified by preparative liquid chromatography (separation column AKZONOBEL Kromasil; 250 ×21.2 mm I.D.; 5 µm, 20 mL/min; mobile phase A: 0.05% TFA + H₂O, mobile phase B: CH₃CN) to obtain 2-(2-(benzo[b]thiophen-5-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acet amide **54** (15 mg) in a yield of 32.99%.

MS m/z (ESI): 753.1 [M+H]
¹H NMR (400 MHz, DMSO-d6) δ 10.46 (s, 1H), 10.26 (s, 1H), 8.65 (s, 1H), 8.59 (s, 1H), 8.19 - 8.05 (m, 3H), 7.99 (d, J = 2.0 Hz, 1H), 7.87 (d, J = 5.4 Hz, 1H), 7.72 (dd, J = 8.7, 2.1 Hz, 1H), 7.61 (d, J = 5.4 Hz, 1H), 5.43 (s, 2H), 4.55 (d, J = 12.5 Hz, 1H), 3.55 (q, J = 12.1 Hz, 3H), 3.26 (d, J = 12.0 Hz, 1H), 3.03 (q, J = 12.6, 9.9 Hz, 3H), 2.86 (d, J = 11.3 Hz, 1H), 2.75 - 2.65 (m, 1H), 2.45 (s, 3H), 1.22 (t, J = 7.4 Hz, 3H).

### Example 55

### 2-(2-(benzo[b]thiophen-6-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acet amide

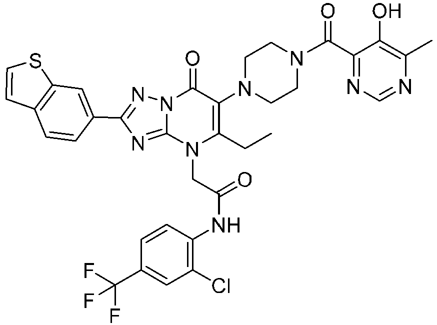

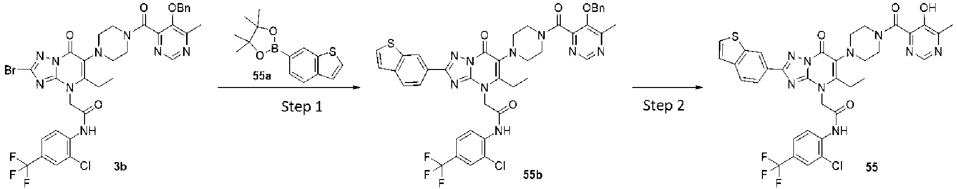

### Step 1

### 2-(2-(benzo[b]thiophen-6-yl)-6-(4-(5-(benzyloxy)-6-methylpyrimidine-4-carbonyl)piperazin-1-yl )-5-ethyl-7-oxo-[1,2,4]triazolo[1,5-a]pyrimidin-4(7H)-yl)-N-(2-chloro-4-(trifluoromethyl)phenyl) acetamide

2-(6-(4-(5-(benzyloxy)-6-methylpyrimidine-4-carbonyl)piperazin-1-yl)-2-bromo-5-ethy 1-7-oxo-[1,2,4]triazolo[1,5-a]pyrimidin-4(7H)-yl)-N-(2-chloro-4-(trifluoromethyl) phenyl)acetamide **3b** (60 mg, 76.05 µmol), 2-(benzo[b]thiophen-6-yl)-4,4,5,5-tetramethyl-1,3,2-dioxaborolane **55a** (25.72 mg, 98.86 µmol, commercially available), [1,1'-bis(diphenylphosphino)ferrocene]palladium(II) dichloride (6.16 mg, 7.60 µmol), and sodium carbonate (16.12 mg, 152.09 µmol) were added to 1,4-dioxane (1.5 mL). The mixture was subject to argon replacement, then heated to 80 °C and stirred for 2 h. Water (50 mL) was added, and the mixture was extracted with dichloromethane (50 mL × 3). The combined organic phases were washed with saturated sodium chloride solution, dried over anhydrous sodium sulfate, filtered, and concentrated. The residue was purified by silica gel column chromatography (eluent: System B) to obtain 2-(2-(benzo[b]thiophen-6-yl)-6-(4-(5-(benzyloxy)-6-methylpyrimidine-4-carbonyl)piperazin-1-yl )-5-ethyl-7-oxo-[1,2,4]triazolo[1,5-a]pyrimidin-4(7H)-yl)-N-(2-chloro-4-(trifluoromethyl)phenyl) acetamide **55b** (40 mg) in a yield of 62.45%.

MS m/z (ESI): 842.1 [M+H]

### Step 2

### 2-(2-(benzo[b]thiophen-6-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acet amide

2-(2-(benzo[b]thiophen-6-yl)-6-(4-(5-(benzyloxy)-6-methylpyrimidine-4-carbonyl) piperazin-1-yl)-5-ethyl-7-oxo-[1,2,4]triazolo[1,5-a]pyrimidin-4(7H)-yl)-N-(2-chloro-4-(trifluoro methyl)phenyl)acetamide **55b** (40 mg, 47.49 µmol) was dissolved in trifluoroacetic acid (5 mL), heated to 40 °C, and stirred for 4 h. The mixture was concentrated under reduced pressure, and the resulting residue was purified by preparative liquid chromatography (separation column AKZONOBEL Kromasil; 250×21.2 mm I.D.; 5 µm, 20 mL/min; mobile phase A: 0.05% TFA + H₂O, mobile phase B: CH₃CN) to obtain 2-(2-(benzo[b]thiophen-6-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acet amide **55** (10 mg) in a yield of 27.72%.

MS m/z (ESI): 752.1 [M+H]
¹H NMR (400 MHz, DMSO-d6) δ 10.46 (s, 1H), 10.26 (s, 1H), 8.75 (s, 1H), 8.59 (s, 1H), 8.17 - 7.86 (m, 5H), 7.72 (dd, J = 8.7, 2.1 Hz, 1H), 7.54 (d, J = 5.4 Hz, 1H), 5.43 (s, 2H), 4.55 (d, J = 12.5 Hz, 1H), 3.61 - 3.44 (m, 3H), 3.26 (d, J = 12.0 Hz, 1H), 3.02 (t, J = 11.4 Hz, 3H), 2.89 - 2.82 (m, 1H), 2.68 (d, J = 10.8 Hz, 1H), 2.45 (s, 3H), 1.23 (t, J = 7.4 Hz, 3H).

### Example 56

### N-(2-chloro-4-(trifluoromethyl)phenyl)-2-(5-ethyl-6-(4-(3-hydroxypicolinoyl)piperazin-1-yl)-7-o xo-2-(5-oxo-1,3a,4,5,6,6a-hexahydropentalen-2-yl)pyrazolo[1,5-a]pyrimidin-4(7H)-yl)acetamide

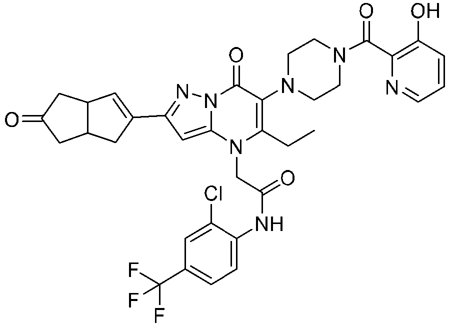

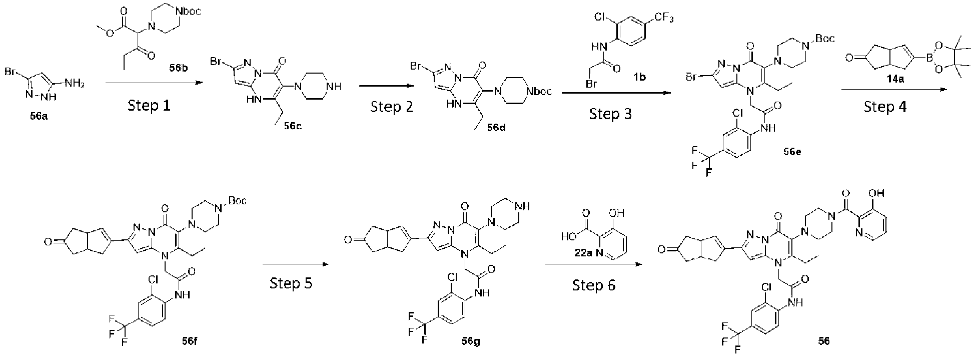

### Step 1

### 2-bromo-5-ethyl-6-(piperazin-1-yl)pyrazolo[1,5-a]pyrimidin-7(4H)-one

3-Bromo-1H-pyrazol-5-amine **56a** (0.5 g, 3.09 mmol, commercially available) was dissolved in ethanol (8 mL), followed by addition of tert-butyl 4-(1-methoxycarbonyl-2-oxobutyl)piperazine-1-carboxylate **56b** (1.46 g, 4.63 mmol, commercially available) and polyphosphoric acid (1.04 g, 3.09 mmol). The mixture was heated to 85 °C and the reaction was performed for 12 h. The product was used directly in the next step.

MS m/z (ESI): 325.8 [M+H]

### Step 2

### tert-butyl 4-(2-bromo-5-ethyl-7-oxo-4,7-dihydropyrazolo[1,5-a]pyrimidin-6-yl)piperazine-1-carboxylate

Water (5 mL) and sodium bicarbonate (412.08 mg, 4.91 mmol) were added to the reaction solution from the previous step, and the mixture was stirred until no more gas was produced. Di-tert-butyl dicarbonate (802.90 mg, 3.68 mmol) was added, and the reaction was performed at room temperature for 16 h. Water (50 mL) was added, and the mixture was extracted with dichloromethane (50 mL × 3). The combined organic phases were washed with saturated sodium chloride solution, dried over anhydrous sodium sulfate, filtered, and concentrated. The residue was purified by silica gel column chromatography (eluent: System B) to obtain tert-butyl 4-(2-bromo-5-ethyl-7-oxo-4,7-dihydropyrazolo[1,5-a]pyrimidin-6-yl)piperazine-1-carboxylate **56d** (0.4 g) with a yield of 76.52%.

MS m/z (ESI): 425.8 [M+H]

### Step 3

### tert-butyl 4-(2-bromo-4-(2-((2-chloro-4-(trifluoromethyl)phenyl)amino)-2-oxoethyl)-5-ethyl-7-oxo-4,7-dih ydropyrazolo[1,5-a]pyrimidin-6-yl)piperazine-1-carboxylate

Tert-butyl 4-(2-bromo-5-ethyl-7-oxo-4,7-dihydropyrazolo[1,5-a]pyrimidin-6-yl)piperazine-1-carboxylate **56d** (0.15 g, 351.86 µmol) and 2-bromo-N-(2-chloro-4-(trifluoromethyl)phenyl)acetamide **1b** (133.64 mg, 422.23 µmol) were sequentially added to N,N-dimethylformamide (2 mL), followed by addition of N,N-diisopropylethylamine (227.37 mg, 1.76 mmol). The mixture was heated to 50 °C and the reaction was performed for 5 h. After the reaction was substantially complete, water (50 mL) was added, and the mixture was extracted with dichloromethane (50 mL × 3). The combined organic phases were washed with saturated sodium chloride solution, dried over anhydrous sodium sulfate, filtered, and concentrated. The residue was purified by silica gel column chromatography (eluent: System B) to obtain tert-butyl 4-(2-bromo-4-(2-((2-chloro-4-(trifluoromethyl)phenyl)amino)-2-oxoethyl)-5-ethyl-7-oxo-4,7-dih ydropyrazolo[1,5-a]pyrimidin-6-yl)piperazine-1-carboxylate **56e** (140 mg) with a yield of 60.11%.

MS m/z (ESI): 604.6 [M+H]

### Step 4

### tert-butyl 4-(4-(2-((2-chloro-4-(trifluoromethyl)phenyl)amino)-2-oxoethyl)-5-ethyl-7-oxo-2-(5-oxo-1,3a,4, 5,6,6a-hexahydropentalen-2-yl)-4,7-dihydropyrazolo[1,5-a]pyrimidin-6-yl)piperazine-1-carboxyl ate

5-(4,4,5,5-Tetramethyl-1,3,2-dioxaborolan-2-yl)-3,3a,4,6a-tetrahydro-1H-cyclopenta [c]furan-2-one **14a** (78.72 mg, 317.27 µmol), tert-butyl 4-(2-bromo-4-(2-((2-chloro-4-(trifluoromethyl)phenyl)amino)-2-oxoethyl)-5-ethyl-7-oxo-4,7-dih ydropyrazolo[1,5-a]pyrimidin-6-yl)piperazine-1-carboxylate **56e** (140 mg, 211.51 µmol), [1,1'-bis(diphenylphosphino)ferrocene]palladium(II) dichloride (17.13 mg, 21.15 µmol), and sodium carbonate (44.84 mg, 423.03 µmol) were dissolved in a mixed solution of 1,4-dioxane (2 mL) and water (1 mL). The mixture was subject to argon replacement three times and was heated to 80 °C. The reaction was performed for 2 h. After the reaction was complete, water (50 mL) was added, and the mixture was extracted with dichloromethane (50 mL × 3). The combined organic phases were washed with saturated sodium chloride solution, dried over anhydrous sodium sulfate, filtered, and concentrated. The residue was purified by silica gel column chromatography (eluent: System B) to obtain tert-butyl 4-(4-(2-((2-chloro-4-(trifluoromethyl)phenyl)amino)-2-oxoethyl)-5-ethyl-7-oxo-2-(5-oxo-1,3a,4, 5,6,6a-hexahydropentalen-2-yl)-4,7-dihydropyrazolo[1,5-a]pyrimidin-6-yl)piperazine-1-carboxyl ate **56f** (100 mg) with a yield of 67.24%.

MS m/z (ESI): 646.7 [M+H-56]

### Step 5

### N-(2-Chloro-4-(trifluoromethyl)phenyl)-2-(5-ethyl-7-oxo-2-(5-oxo-1,3a,4,5,6,6a-hexahydropenta len-2-yl)-6-(piperazin-1-yl)pyrazolo[1,5-a]pyrimidin-4(7H)-yl)acetamide

Tert-butyl 4-(4-(2-((2-chloro-4-(trifluoromethyl)phenyl)amino)-2-oxoethyl)-5-ethyl-7-oxo-2-(5-oxo-1,3a,4, 5,6,6a-hexahydropentalen-2-yl)-4,7-dihydropyrazolo[1,5-a]pyrimidin-6-yl)piperazine-1-carboxyl ate **56f** (100 mg, 142.22 µmol) was dissolved in dichloromethane (3 mL), and trifluoroacetic acid (0.5 g, 4.39 mmol) was added dropwise. The reaction was performed at room temperature for 3 h. The mixture was concentrated under reduced pressure to obtain N-(2-chloro-4-(trifluoromethyl)phenyl)-2-(5-ethyl-7-oxo-2-(5-oxo-1,3a,4,5,6,6a-hexahydropental en-2-yl)-6-(piperazin-1-yl)pyrazolo[1,5-a]pyrimidin-4(7H)-yl)acetamide **56g** (70 mg), which was used directly in the next step.

MS m/z (ESI): [M+H-56]+=602.8

### Step 6

### N-(2-Chloro-4-(trifluoromethyl)phenyl)-2-(5-ethyl-6-(4-(3-hydroxypicolinoyl)piperazin-1-yl)-7-oxo-2-(5-oxo-1,3a,4,5,6,6a-hexahydropentalen-2-yl)pyrazolo[1,5-a]pyrimidin-4(7H)-yl)acetamid e

3-Hydroxypicolinic acid **22a** (32.30 mg, 232.16 µmol), 1-(3-dimethylaminopropyl)-3-ethylcarbodiimide hydrochloride (66.76 mg, 348.24 µmol), 1-hydroxybenzotriazole (47.05 mg, 348.24 µmol), and N,N-diisopropylethylamine (75.01 mg, 580.40 µmol) were sequentially added to N,N-dimethylformamide (2 mL), and the reaction was performed at room temperature for 10 min. N-(2-Chloro-4-(trifluoromethyl)phenyl)-2-(5-ethyl-7-oxo-2-(5-oxo-1,3 a,4,5, 6,6a-hexahydropenta len-2-yl)-6-(piperazin-1-yl)pyrazolo[1,5-a]pyrimidin-4(7H)-yl)acetamide **56g** (70 mg, 116.08 µmol) was added, and the reaction was performed at room temperature for 16 h. After the reaction was complete, water (50 mL) was added, and the mixture was extracted with dichloromethane (50 mL ×3). The combined organic phases were washed with saturated sodium chloride solution, dried over anhydrous sodium sulfate, filtered, and concentrated. The residue was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(3-hydroxypicolinoyl)piperazin-1-yl)-7-o xo-2-(5-oxo-1,3a,4,5,6,6a-hexahydropentalen-2-yl)pyrazolo[1,5-a]pyrimidin-4(7H)-yl)acetamide **56** (30 mg) with a yield of 34.91%.

MS m/z (ESI): 723.7 [M+H-56]
¹H NMR (400 MHz, DMSO-d6) δ 10.45 (s, 1H), 10.34 (s, 1H), 8.11 (d, J = 8.6 Hz, 1H), 8.06 (d, J = 4.3 Hz, 1H), 7.97 (d, J = 2.1 Hz, 1H), 7.71 (dd, J = 8.8, 2.2 Hz, 1H), 7.35 - 7.24 (m, 2H), 6.55 (s, 1H), 6.23 (s, 1H), 5.20 (s, 2H), 4.54 (d, J = 12.5 Hz, 1H), 3.53 (dd, J = 20.4, 8.2 Hz, 4H), 3.22 (d, J = 12.3 Hz, 2H), 3.07 (dd, J = 16.4, 8.4 Hz, 2H), 2.99 - 2.89 (m, 3H), 2.77 (d, J = 11.7 Hz, 1H), 2.68 - 2.52 (m, 4H), 2.24 (s, 1H), 1.92 (dd, J = 18.9, 6.8 Hz, 1H), 1.17 (t, J = 7.4 Hz, 3H).

### Example 57

### 2-(2-(benzofuran-6-yl)-5-ethyl-6-(4-(5-hydroxy-6-methylpyrimidine-4-carbonyl)piperazin-1-yl)-7-oxopyrazolo[1,5-a]pyrimidin-4(7H)-yl)-N-(2-chloro-4-(trifluoromethyl)phenyl)acetamide

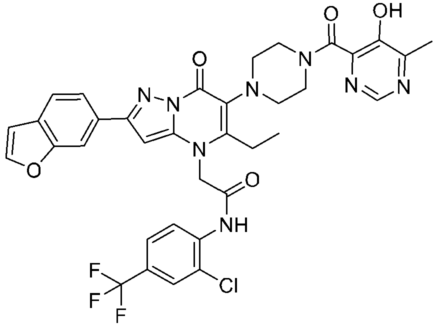

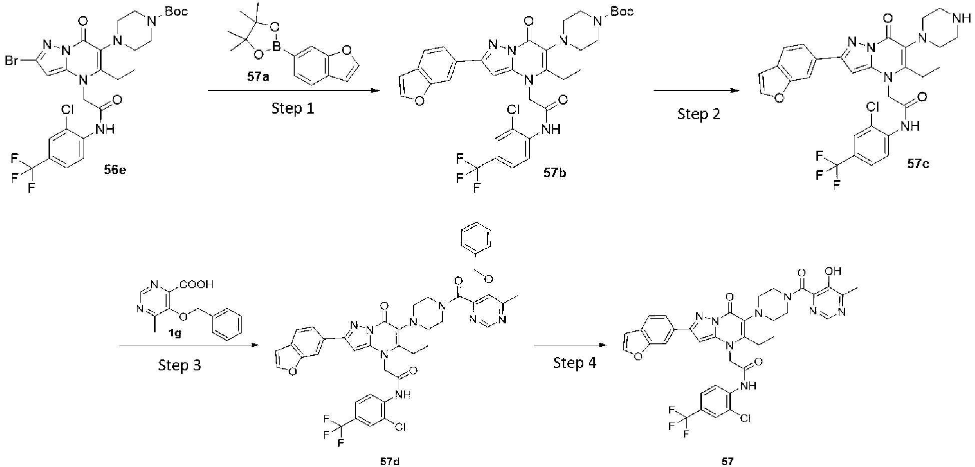

### Step 1

### tert-butyl 4-(2-(benzofuran-6-yl)-4-(2-((2-chloro-4-(trifluoromethyl)phenyl)amino)-2-oxoethyl)-5-ethyl-7-o xo-4,7-dihydropyrazolo[1,5-a]pyrimidin-6-yl)piperazine-1-carboxylate

2-(Benzofuran-6-yl)-4,4,5,5-tetramethyl-1,3,2-dioxaborolane **57a** (44.25 mg, 181.30 umol), tert-butyl 4-(2-bromo-4-(2-((2-chloro-4-(trifluoromethyl)phenyl)amino)-2-oxoethyl)-5-ethyl-7-oxo-4,7-dih ydropyrazolo[1,5-a]pyrimidin-6-yl)piperazine-1-carboxylate **56e** (60 mg, 90.65 µmol), [1,1'-bis(diphenylphosphino)ferrocene]palladium dichloride (7.34 mg, 9.06 µmol), and sodium carbonate (19.22 mg, 181.30 µmol) were dissolved in a mixture solution of 1,4-dioxane (2 mL) and water (1 mL). The mixture was subject to argon replacement three times, heated to 80 °C, and the reaction was performed for 16 h. Water (50 mL) was added, and the mixture was extracted with dichloromethane (50 mL×3). The combined organic phases were washed with saturated sodium chloride solution, dried over anhydrous sodium sulfate, filtered, and concentrated. The residue was purified by silica gel column chromatography (eluent: System B) to obtain tert-butyl 4-(2-(benzofuran-6-yl)-4-(2-((2-chloro-4-(trifluoromethyl)phenyl)amino)-2-oxoethyl)-5-ethyl-7-o xo-4,7-dihydropyrazolo[1,5-a]pyrimidin-6-yl)piperazine-1-carboxylate **57b** (40 mg) with a yield of 63.12%.

MS m/z (ESI): 642.7 [M+H-56]

### Step 2

### 2-(2-(benzofuran-6-yl)-5-ethyl-7-oxo-6-(piperazin-1-yl)pyrazolo[1,5-a]pyrimidin-4(7H)-yl)-N-(2 -chloro-4-(trifluoromethyl)phenyl)acetamide

tert-butyl 4-(2-(benzofuran-6-yl)-4-(2-((2-chloro-4-(trifluoromethyl)phenyl)amino)-2-oxoethyl)-5-ethyl-7-o xo-4,7-dihydropyrazolo[1,5-a]pyrimidin-6-yl)piperazine-1-carboxylate **57b** (40 mg, 57.21 µmol) was dissolved in dichloromethane (4 mL), followed by dropwise addition of trifluoroacetic acid (1 g, 8.77 mmol). The reaction was performed at room temperature for 3 h. After the reaction was completed, the mixture was concentrated under reduced pressure. The resulting residue was purified by preparative liquid chromatography (separation column: AKZONOBEL Kromasil; 250 ×21.2 mm I.D.; 5 µm, 20 mL/min; mobile phase A: 0.05% TFA+H₂O, mobile phase B: CH₃CN) to obtain 2-(2-(benzofuran-6-yl)-5-ethyl-7-oxo-6-(piperazin-1-yl)pyrazolo[1,5-a]pyrimidin-4(7H)-yl)-N-(2 -chloro-4-(trifluoromethyl)phenyl)acetamide **57c** (20 mg) with a yield of 58.36%.

MS m/z (ESI): 598.7 [M+H]

### Step 3

### 2-(2-(benzofuran-6-yl)-6-(4-(5-(benzyloxy)-6-methylpyrimidine-4-carbonyl)piperazin-1-yl)-5-eth yl-7-oxopyrazolo[1,5-a]pyrimidin-4(7H)-yl)-N-(2-chloro-4-(trifluoromethyl)phenyl)acetamide

5-(Benzyloxy)-6-methylpyrimidine-4-carboxylic acid **1g** (16.31 mg, 66.78 µmol), 1-(3-dimethylaminopropyl)-3-ethylcarbodiimide hydrochloride (12.80 mg, 66.78 µmol), 1-hydroxybenzotriazole (9.02 mg, 66.78 µmol), and N,N-diisopropylethylamine (21.58 mg, 166.94 µmol) were dissolved in N,N-dimethylformamide (1 mL) and reacted at room temperature for 15 min. Then 2-(2-(benzofuran-6-yl)-5-ethyl-7-oxo-6-(piperazin-1-yl)pyrazolo[1,5-a]pyrimidin-4(7H)-yl)-N-(2 -chloro-4-(trifluoromethyl)phenyl)acetamide **57c** (20 mg, 33.39 µmol) was added, and the reaction was performed at room temperature for 16 h. Water (50 mL) was added, and the mixture was extracted with dichloromethane (50 mL ×3). The combined organic phases were washed with saturated sodium chloride solution, dried over anhydrous sodium sulfate, filtered, and concentrated. The residue was purified by silica gel column chromatography (eluent: System B) to obtain 2-(2-(benzofuran-6-yl)-6-(4-(5-(benzyloxy)-6-methylpyrimidine-4-carbonyl)piperazin-1-yl)-5-eth yl-7-oxopyrazolo[1,5-a]pyrimidin-4(7H)-yl)-N-(2-chloro-4-(trifluoromethyl)phenyl)acetamide **57d** (12 mg) with a yield of 43.55%.

MS m/z (ESI): 824.6 [M+H]

### Step 4

### 2-(2-(benzofuran-6-yl)-5-ethyl-6-(4-(5-hydroxy-6-methylpyrimidine-4-carbonyl)piperazin-1-yl)-7-oxopyrazolo[1,5-a]pyrimidin-4(7H)-yl)-N-(2-chloro-4-(trifluoromethyl)phenyl)acetamide

2-(2-(Benzofuran-6-yl)-6-(4-(5-(benzyloxy)-6-methylpyrimidine-4-carbonyl)piperazin-1-yl)-5-ethyl-7-oxopyrazolo[1,5-a]pyrimidin-4(7H)-yl)-N-(2-chloro-4-(trifluoromethyl)phenyl) acetamide **57d** (12 mg, 14.54 µmol) was dissolved in dichloromethane (1 mL), followed by addition of trifluoroacetic acid (3 g, 26.31 mmol). The mixture was heated to 45 °C, and the reaction was performed for 6 h. The mixture was concentrated under reduced pressure. The residue was purified by preparative liquid chromatography (separation column: AKZONOBEL Kromasil; 250 ×21.2 mm I.D.; 5 µm, 20 mL/min; mobile phase A: 0.05% TFA+H₂O, mobile phase B: CH₃CN) to obtain 2-(2-(benzofuran-6-yl)-5-ethyl-6-(4-(5-hydroxy-6-methylpyrimidine-4-carbonyl) piperazin-1-yl)-7-oxopyrazolo[1,5-a]pyrimidin-4(7H)-yl)-N-(2-chloro-4-(trifluoromethyl)phenyl) acetamide **57** (8 mg) with a yield of 73.94%.

MS m/z (ESI): 734.6 [M+H]
¹H NMR (400 MHz, DMSO-d6) δ 10.31 (s, 1H), 10.25 (s, 1H), 8.59 (s, 1H), 8.14 (d, J = 8.6 Hz, 1H), 8.11 - 8.04 (m, 2H), 7.99 (d, J = 2.2 Hz, 1H), 7.89 - 7.83 (m, 1H), 7.79 - 7.67 (m, 2H), 7.05 - 6.95 (m, 2H), 5.25 (s, 2H), 4.53 (d, J = 12.5 Hz, 1H), 3.27 (d, J = 12.6 Hz, 1H), 2.98 (d, J = 11.3 Hz, 3H), 2.83 (d, J = 11.2 Hz, 1H), 2.64 (s, 1H), 2.58 - 2.52 (m, 1H), 2.45 (s, 3H), 1.21 (t, J = 7.5 Hz, 3H).

### Example 58

### 2-(2-(2,3-dihydrobenzofuran-6-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(4-(pentafluoro-λ⁶-sulfaneyl)phenyl )acetamide

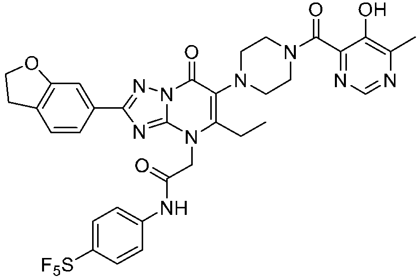

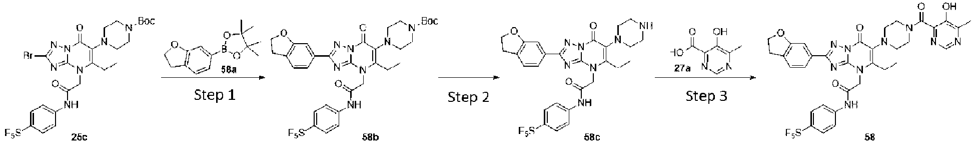

### Step 1

### tert-butyl 4-(2-(2,3-dihydrobenzofuran-6-yl)-5-ethyl-7-oxo-4-(2-oxo-2-((4-(pentafluoro-λ⁶-sulfaneyl)pheny 1)amino)ethyl)-4,7-dihydro-[1,2,4]triazolo[1,5-a]pyrimidin-6-yl)piperazine-1-carboxylate

At room temperature, tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)piperazine-1-carboxylate **25c** (150 mg, 218.50 µmol), 2-(2,3-dihydrobenzofuran-6-yl)-4,4,5,5-tetramethyl-1,3,2-dioxaborolane 58a (69.91 mg, 284.05 µmol), [1,1'-bis(diphenylphosphino)ferrocene]palladium(II) dichloride dichloromethane complex (17.71 mg, 21.85 µmol), and sodium carbonate (69.48 mg, 655.51 µmol) were added to a mixed solution of 1,4-dioxane (7 mL) and water (1 mL). The mixture was subject to argon replacement three times, then the temperature was raised to 85 °C and the reaction was performed for 2 h. Water (20 mL) was added to the reaction mixture, followed by extraction with ethyl acetate (20 mL×3). The combined organic phase was washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(2-(2,3-dihydrobenzofuran-6-yl)-5-ethyl-7-oxo-4-(2-oxo-2-((4-(pentafluoro-λ⁶-sulfaneyl)pheny l)amino) ethyl)-4,7-dihydro-[1,2,4]triazolo[1,5-a]pyrimidin-6-yl)piperazine-1-carboxylate 58b (110 mg) with a yield of 69.37%.

MS m/z (ESI): 670.2 [M-56+H]

### Step 2

### 2-(2-(2,3-dihydrobenzofuran-6-yl)-5-ethyl-7-oxo-6-(piperazin-l-yl)-[1,2,4]triazolo[1,5-a]pyrimid in-4(7H)-yl)-N-(4-(pentafluoro-λ⁶-sulfaneyl)phenyl)acetamide

At room temperature, trifluoroacetic acid (1.5 mL) was added to a solution of tert-butyl 4-(2-(2,3-dihydrobenzofuran-6-yl)-5-ethyl-7-oxo-4-(2-oxo-2-((4-(pentafluoro-λ⁶-sulfaneyl) phenyl)amino)ethyl)-4,7-dihydro-[1,2,4]triazolo[1,5-a]pyrimidin-6-yl)piperazine-1-carboxylate 58b (110 mg, 151.57 µmol) in dichloromethane (6 mL). The reaction was performed at room temperature for 40 min. The mixture was concentrated under reduced pressure to obtain 2-(2-(2,3-dihydrobenzofuran-6-yl)-5-ethyl-7-oxo-6-(piperazin-1-yl)-[1,2,4]triazolo[1,5-a]pyrimid in-4(7H)-yl)-N-(4-(pentafluoro-λ⁶-sulfaneyl)phenyl)acetamide 58c (94.8 mg) with a yield of 99.97%, which was used directly in the next step.

MS m/z (ESI): 626.2 [M+1]

### Step 3

### 2-(2-(2,3-dihydrobenzofuran-6-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(4-(pentafluoro-λ⁶-sulfaneyl)phenyl )acetamide

At room temperature, 5-hydroxy-6-methylpyrimidine-4-carboxylic acid 27a (34.74 mg, 225.38 µmol), 1-(3-dimethylaminopropyl)-3-ethylcarbodiimide hydrochloride (57.61 mg, 300.51 µmol), 1-hydroxybenzotriazole (40.60 mg, 300.51 µmol), and N,N-diisopropylethylamine (97.09 mg, 751.26 µmol) were added to N,N-dimethylformamide (3 mL). The reaction was performed at room temperature for 40 min, followed by addition of a solution of 2-(2-(2,3-dihydrobenzofuran-6-yl)-5-ethyl-7-oxo-6-(piperazin-1-yl)-[1,2,4]triazolo[1,5-a]pyrimid in-4(7H)-yl)-N-(4-(pentafluoro-λ⁶-sulfaneyl)phenyl)acetamide 58c (94 mg, 150.25 µmol) in N,N-dimethylformamide (3 mL). The reaction was performed at room temperature for 18 h. Water (20 mL) was added to the reaction mixture, followed by extraction with ethyl acetate (20 mL×3). The combined organic phase was washed with saturated sodium chloride solution (20 mL), dried over anhydrous sodium sulfate, filtered, and concentrated under reduced pressure. The residue was purified by preparative liquid chromatography (separation column: AKZONOBEL Kromasil; 250 ×21.2 mm I.D.; 5 µm, 20 mL/min; mobile phase A: 0.05% TFA+H₂O, mobile phase B: CH₃CN) to obtain 2-(2-(2,3-dihydrobenzofuran-6-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(4-(pentafluoro-λ⁶-sulfaneyl)phenyl )acetamide 58 (3.25 mg) with a yield of 2.67%.

MS m/z (EST):762.2 [M+1]
¹H NMR (400 MHz, DMSO-d6) δ 11.00 (s, 1H), 10.24 (s, 1H), 8.59 (s, 1H), 7.98 - 7.86 (m, 2H), 7.78 (d, J = 8.9 Hz, 2H), 7.59 (dd, J = 7.7, 1.4 Hz, 1H), 7.47 - 7.28 (m, 2H), 5.27 (s, 2H), 4.56 (q, J = 10.6, 9.7 Hz, 3H), 3.61 - 3.47 (m, 3H), 3.23 (q, J = 9.3, 8.8 Hz, 3H), 3.11 - 2.94 (m, 3H), 2.84 (d, J = 11.4 Hz, 1H), 2.67 (d, J = 10.7 Hz, 1H), 2.45 (s, 3H), 1.19 (t, J = 7.4 Hz, 3H).

### Example 59

### 2-(2-(cyclohept-1-en-1-yl)-5-ethyl-6-(4-(5-hydroxy-6-methylpyrimidine-4-carbonyl)piperazin-1-yl)-7-oxopyrazolo[1,5-a]pyrimidin-4(7H)-yl)-N-(4-(pentafluoro-λ⁶-sulfaneyl)phenyl)acetamide

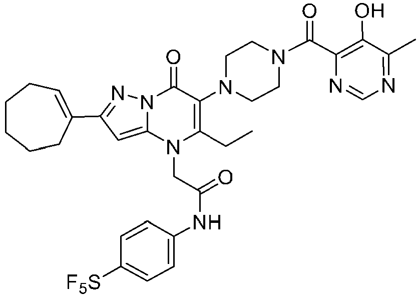

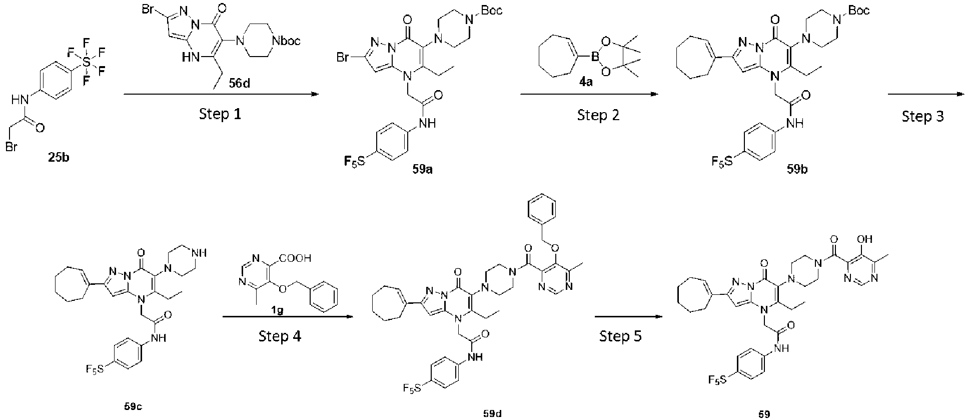

### Step 1

### tert-butyl 4-(2-bromo-5-ethyl-7-oxo-4-(2-oxo-2-((4-(pentafluoro-λ⁶-sulfaneyl)phenyl)amino)ethyl)-4,7-dih ydropyrazolo[1,5-a]pyrimidin-6-yl)piperazine-1-carboxylate

Tert-butyl 4-(1-methoxycarbonyl-2-oxobutyl)piperazine-1-carboxylate **56b** (0.1 g, 234.57 µmol) and 2-bromo-N-(4-(pentafluoro-λ⁶-sulfaneyl)phenyl)acetamide 25b (103.71 mg, 304.94 µmol) were sequentially added to N,N-dimethylformamide (4 mL), followed by addition of N,N-diisopropylethylamine (151.58 mg, 1.17 mmol). The mixture was heated to 60 °C and the reaction was performed for 5 hours. Water (50 mL) was added to the reaction mixture, followed by extraction with dichloromethane (50 mL×3). The combined organic phases were washed with saturated sodium chloride solution, dried over anhydrous sodium sulfate, filtered, and concentrated. The residue was purified by silica gel column chromatography (eluent: System B) to obtain tert-butyl 4-(2-bromo-5-ethyl-7-oxo-4-(2-oxo-2-((4-(pentafluoro-λ⁶-sulfaneyl)phenyl)amino)ethyl)-4,7-dih ydropyrazolo[1,5-a]pyrimidin-6-yl)piperazine-1-carboxylate 59a (85 mg) with a yield of 52.86%.

MS m/z (ESI): 628.6 [M+H]

### Step 2

### tert-butyl 4-(2-(cyclohept-1-en-1-yl)-5-ethyl-7-oxo-4-(2-oxo-2-((4-(pentafluoro-λ⁶-sulfaneyl)phenyl)amino )ethyl)-4,7-dihydropyrazolo[1,5-a]pyrimidin-6-yl)piperazine-1-carboxylate

2-(Cyclohept-1-en-1-yl)-4,4,5,5-tetramethyl-1,3,2-dioxaborolane 4a (33.05 mg, 148.80 µmol), tert-butyl 4-(2-bromo-5-ethyl-7-oxo-4-(2-oxo-2-((4-(pentafluoro-λ⁶-sulfaneyl)phenyl) amino)ethyl)-4,7-dihydropyrazolo[1,5-a]pyrimidin-6-yl)piperazine-1-carboxylate 59a (85 mg, 124.00 µmol), [1,1'-bis(diphenylphosphino)ferrocene]palladium(II) dichloride (10.04 mg, 12.40 µmol), and sodium carbonate (26.28 mg, 247.99 µmol) were sequentially added to a mixed solution of 1,4-dioxane (1.5 mL) and water (0.5 mL). After argon replacement, the mixture was heated to 80 °C and the reaction was performed for 3 hours. Upon completion of the reaction, water (50 mL) was added to the reaction mixture, followed by extraction with dichloromethane (50 mL×3). The combined organic phases were washed with saturated sodium chloride solution, dried over anhydrous sodium sulfate, filtered, and concentrated. The residue was purified by silica gel column chromatography (eluent: System B) to obtain tert-butyl 4-(2-(cyclohept-1-en-1-yl)-5-ethyl-7-oxo-4-(2-oxo-2-((4-(pentafluoro-λ⁶-sulfaneyl)phenyl)amino )ethyl)-4,7-dihydropyrazolo[1,5-a]pyrimidin-6-yl)piperazine-1-carboxylate 59b (70 mg) with a yield of 80.56%.

MS m/z (ESI): 644.8 [M+H-56]

### Step 3

### 2-(2-(cyclohept-1-en-1-yl)-5-ethyl-7-oxo-6-(piperazin-1-yl)pyrazolo[1,5-a]pyrimidin-4(7H)-yl)-N-(4-(pentafluoro-λ⁶-sulfaneyl)phenyl)acetamide

tert-butyl 4-(2-(cyclohept-1-en-1-yl)-5-ethyl-7-oxo-4-(2-oxo-2-((4-(pentafluoro-λ⁶-sulfaneyl)phenyl)amino )ethyl)-4,7-dihydropyrazolo[1,5-a]pyrimidin-6-yl)piperazine-1-carboxylate 59b (70 mg, 99.89 µmol) was dissolved in dichloromethane (3 mL), followed by dropwise addition of trifluoroacetic acid (1 g, 8.77 mmol). The reaction was performed at room temperature for 3 hours. The mixture was concentrated under reduced pressure to obtain 2-(2-(cyclohept-1-en-1-yl)-5-ethyl-7-oxo-6-(piperazin-1-yl)pyrazolo[1,5-a]pyrimidin-4(7H)-yl)-N-(4-(pentafluoro-λ⁶-sulfaneyl)phenyl)acetamide 59c (60 mg), which was used directly in the next step.

MS m/z (ESI): [M+H]+=600.8

### Step 4

### 2-(6-(4-(5-(benzyloxy)-6-methylpyrimidine-4-carbonyl)piperazin-1-yl)-2-(cyclohept-1-en-1-yl)-5 -ethyl-7-oxopyrazolo[1,5-a]pyrimidin-4(7H)-yl)-N-(4-(pentafluoro-λ⁶-sulfaneyl)phenyl)acetamid e

5-(benzyloxy)-6-methylpyrimidine-4-carboxylic acid 1g (43.92 mg, 179.81 µmol), N,N-dimethylformamide (2.5 mL), 1-(3-dimethylaminopropyl)-3-ethylcarbodiimide hydrochloride (57.45 mg, 299.68 µmol), 1-hydroxybenzotriazole (40.49 mg, 299.68 µmol), and N,N-diisopropylethylamine (64.55 mg, 499.46 µmol) were sequentially added. The reaction was performed at room temperature for 10 minutes, followed by addition of 2-(2-(cyclohept-1-en-1-yl)-5-ethyl-7-oxo-6-(piperazin-1-yl)pyrazolo[1,5-a]pyrimidin-4(7H)-yl)-N-(4-(pentafluoro-λ⁶-sulfaneyl)phenyl)acetamide 59c (60 mg, 99.89 µmol). Upon completion of the reaction, water (50 mL) was added to the reaction mixture, followed by extraction with dichloromethane (50 mL ×3). The combined organic phases were washed with saturated sodium chloride solution, dried over anhydrous sodium sulfate, filtered, and concentrated. The residue was purified by silica gel column chromatography (eluent: System B) to obtain 2-(6-(4-(5-(benzyloxy)-6-methylpyrimidine-4-carbonyl)piperazin-1-yl)-2-(cyclohept-1-en-1-yl)-5 -ethyl-7-oxopyrazolo[1,5-a]pyrimidin-4(7H)-yl)-N-(4-(pentafluoro-λ⁶-sulfaneyl)phenyl)acetamid e 59d (60 mg) with a yield of 72.64%.

MS m/z (ESI): 826.7 [M+H]

### Step 6

### 2-(2-(cyclohept-1-en-1-yl)-5-ethyl-6-(4-(5-hydroxy-6-methylpyrimidine-4-carbonyl)piperazin-1-yl)-7-oxopyrazolo[1,5-a]pyrimidin-4(7H)-yl)-N-(4-(pentafluoro-λ⁶-sulfaneyl)phenyl)acetamide

2-(6-(4-(5-(benzyloxy)-6-methylpyrimidine-4-carbonyl)piperazin-1-yl)-2-(cyclohept-1-en-1-yl)-5-ethyl-7-oxopyrazolo[1,5-a]pyrimidin-4(7H)-yl)-N-(4-(pentafluoro-λ⁶-sulfaneyl)phenyl )acetamide 59d (60 mg, 72.56 µmol) was dissolved in dichloromethane (1 mL), followed by addition of trifluoroacetic acid (4 g, 35.08 mmol). The mixture was heated to 45 C and the reaction was performed for 6 hours. Upon completion of the reaction, the mixture was concentrated under reduced pressure. The residue was purified by preparative liquid chromatography (separation column: AKZONOBEL Kromasil; 250×21.2 mm I.D.; 5 µm, 20 mL/min; mobile phase A: 0.05% TFA+H₂O, mobile phase B: CH₃CN) to obtain 2-(2-(cyclohept-1-en-1-yl)-5-ethyl-6-(4-(5-hydroxy-6-methylpyrimidine-4-carbonyl)piperazin-1-yl)-7-oxopyrazolo[1,5-a]pyrimidin-4(7H)-yl)-N-(4-(pentafluoro-λ⁶-sulfaneyl)phenyl)acetamide 59 (20 mg) with a yield of 34.87%.

MS m/z (ESI): 736.7 [M+H]
¹H NMR (400 MHz, DMSO-d6) δ 10.88 (s, 1H), 10.24 (s, 1H), 8.58 (s, 1H), 7.93 - 7.84 (m, 2H), 7.78 (d, J = 8.9 Hz, 2H), 6.58 (t, J = 6.7 Hz, 1H), 6.46 (s, 1H), 4.98 (s, 2H), 4.51 (d, J = 12.6 Hz, 1H), 3.54 (dd, J = 14.6, 3.7 Hz, 3H), 3.29 - 3.18 (m, 1H), 3.05 - 2.83 (m, 3H), 2.79 (d, J = 11.3 Hz, 1H), 2.75 - 2.69 (m, 2H), 2.61 (d, J = 11.1 Hz, 1H), 2.45 (s, 3H), 2.28 (q, J = 6.4 Hz, 2H), 1.78 (p, J = 5.7 Hz, 2H), 1.51 (dt, J = 11.9, 6.0 Hz, 4H), 1.16 (t, J = 7.4 Hz, 3H).

### Example 60

### N-(2-chloro-4-(trifluoromethyl)phenyl)-2-(2-(1-cyclobutylideneethyl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)ace tamide

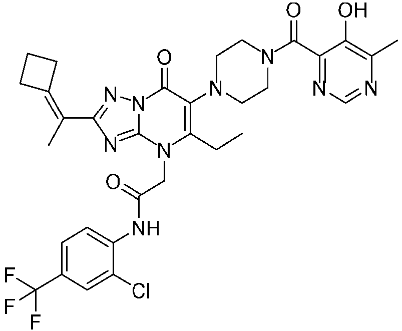

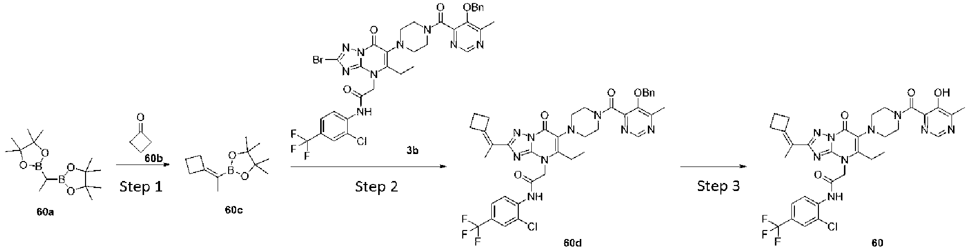

### Step 1

### 2-(1-cyclobutylideneethyl)-4,4,5,5-tetramethyl-1,3,2-dioxaborolane

2,2,6,6-Tetramethylpiperidine (241.83 mg, 1.71 mmol) was added to tetrahydrofuran (3 mL), and n-butyllithium (684.84 µL, 2.5 M) was added dropwise at -30 °C. The reaction was continued at -30 °C for 30 minutes, then cooled to -78 °C. A solution of 2,2'-(ethane-1,1-diyl)bis(4,4,5,5-tetramethyl-1,3,2-dioxaborolane) (402.33 mg, 1.43 mmol) in tetrahydrofuran (3 mL) was added dropwise. After 30 minutes, a solution of cyclobutanone 60b (100 mg, 1.43 mmol) in tetrahydrofuran (4.5 mL) was added dropwise. The mixture was slowly heated to room temperature and the reaction was performed for 2 hours. Water (30 mL) was added to the reaction mixture, which was extracted with petroleum ether (30 mL × 2). The combined organic phases were washed with saturated sodium chloride solution (30 mL × 2), dried over anhydrous sodium sulfate, filtered, and concentrated under reduced pressure. The resulting residue was purified by silica gel column chromatography (eluent: System A) to obtain 2-(1-cyclobutylideneethyl)-4,4,5,5-tetramethyl-1,3,2-dioxaborolane 60c (150 mg) with a yield of 50.52%.

### Step 2

### 2-(6-(4-(5-(benzyloxy)-6-methylpyrimidine-4-carbonyl)piperazin-1-yl)-2-(1-cyclobutylideneethy l)-5-ethyl-7-oxo-[1,2,4]triazolo[1,5-a]pyrimidin-4(7H)-yl)-N-(2-chloro-4-(trifluoromethyl)phenyl )acetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide 3b (50 mg, 63.37 µmol), 2-(1-cyclobutylideneethyl)-4,4,5,5-tetramethyl-1,3,2-dioxaborolane 60c (15.83 mg, 76.05 µmol), [1,1'-bis(diphenylphosphino)ferrocene]palladium(II) dichloride (5.14 mg, 6.34 µmol), and sodium carbonate (20.15 mg, 190.11 µmol) were added to 1,4-dioxane (1 mL) and water (0.3 mL). The mixture was subject to argon replacement four times, heated to 80 °C, and the reaction was performed for 1.5 hours. Water (20 mL) was added to the reaction mixture, which was extracted with ethyl acetate (40 mL ×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-2-(1-cyclobutylideneethy l)-5-ethyl-7-oxo-[1,2,4]triazolo[1,5-a]pyrimidin-4(7H)-yl)-N-(2-chloro-4-(trifluoromethyl)phenyl )acetamide 60d (36 mg) with a yield of 71.89%.

MS m/z (ESI): 790.25 [M+1]

### Step 3

### N-(2-chloro-4-(trifluoromethyl)phenyl)-2-(2-(1-cyclobutylideneethyl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)ace tamide

2-(6-(4-(5-(Benzyloxy)-6-methylpyrimidine-4-carbonyl)piperazin-1-yl)-2-(1-cyclobutyl ideneethyl)-5-ethyl-7-oxo-[1,2,4]triazolo[1,5-a]pyrimidin-4(7H)-yl)-N-(2-chloro-4-(trifluorometh yl)phenyl)acetamide **60d** (36 mg, 45.56 µmol) was added to dichloromethane (2.5 mL). Boron trichloride (0.8 mL) was added at 0 °C, and the reaction was performed at 0 °C for 1 hour. Methanol was added to quench the reaction, and the mixture was concentrated under reduced pressure. The residue was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(2-(1-cyclobutylideneethyl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)ace tamide 60 (3.09 mg) with a yield of 9.50%.

MS m/z (ESI): 699.7 [M+1]
¹H NMR (400 MHz, DMSO) δ 10.36 (s, 1H), 10.23 (s, 1H), 8.58 (s, 1H), 8.02 (d, J = 8.6 Hz, 1H), 7.96 (d, J = 2.1 Hz, 1H), 7.72 (dd, J = 8.8, 2.2 Hz, 1H), 5.28 (s, 2H), 4.52 (d, J = 12.5 Hz, 1H), 3.50 (d, J = 12.6 Hz, 4H), 3.25 (t, J = 12.7 Hz, 1H), 3.08 (s, 2H), 3.01 (d, J = 9.4 Hz, 3H), 2.81 (d, J = 9.6 Hz, 3H), 2.63 (d, J = 10.9 Hz, 1H), 2.44 (s, 3H), 1.95 (p, J = 7.9 Hz, 2H), 1.89 - 1.82 (m, 3H), 1.18 (t, J = 7.4 Hz, 3H).

### Example 61

### 2-(2-(1-cyclobutylideneethyl)-5-ethyl-6-(4-(5-hydroxy-6-methylpyrimidine-4-carbonyl)piperazin -1-yl)-7-oxo-[1,2,4]triazolo[1,5-a]pyrimidin-4(7H)-yl)-N-(4-(pentafluoro-λ⁶-sulfaneyl)phenyl)ace tamide

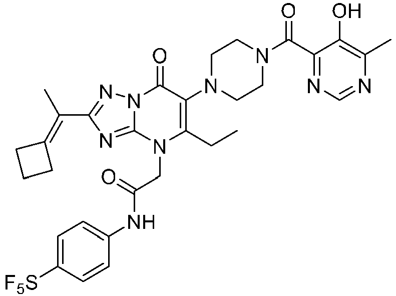

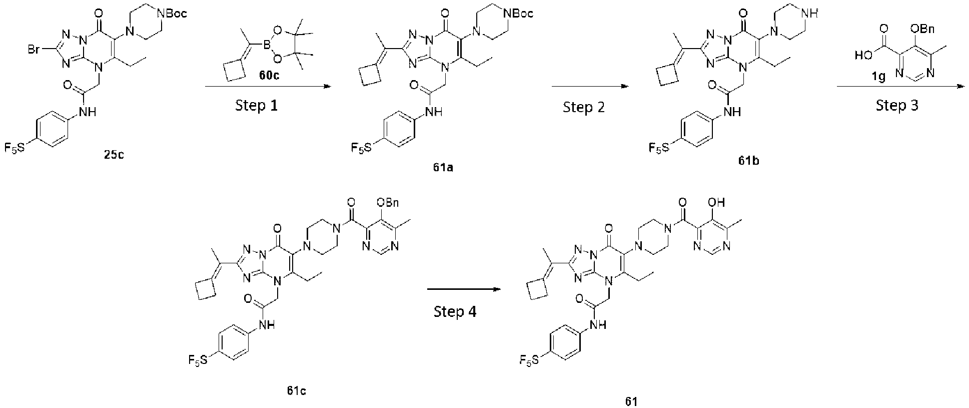

### Step 1

### tert-butyl 4-(2-(1-cyclobutylideneethyl)-5-ethyl-7-oxo-4-(2-oxo-2-((4-(pentafluoro-λ⁶-sulfaneyl)phenyl)am ino)ethyl)-4,7-dihydro-[1,2,4]triazolo[1,5-a]pyrimidin-6-yl)piperazine-1-carboxylate

At room temperature, tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)piperazine-1-carboxylate 25c (120 mg, 174.80 µmol), 2-(1-cyclobutylideneethyl)-4,4,5,5-tetramethyl-1,3,2-dioxaborolane 60c (43.65 mg, 209.76 µmol), [1,1'-bis(diphenylphosphino)ferrocene]palladium dichloride (14.17 mg, 17.48 µmol), and sodium carbonate (55.58 mg, 524.41 µmol) were added to a mixture of 1,4-dioxane (2 mL) and water (0.6 mL). The mixture was subject to argon replacement four times, heated to 80 °C, and the reaction was performed for 1.5 h. Water (20 mL) was added to the reaction mixture, which was extracted with ethyl acetate (40 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(2-(1-cyclobutylideneethyl)-5-ethyl-7-oxo-4-(2-oxo-2-((4-(pentafluoro-λ⁶-sulfaneyl)phenyl)am ino)ethyl)-4,7-dihydro-[1,2,4]triazolo[1,5-a]pyrimidin-6-yl)piperazine-1-carboxylate 61a (90 mg) with a yield of 74.87%.

MS m/z (ESI): 688.2 [M+1]

### Step 2

### 2-(2-(1-cyclobutylideneethyl)-5-ethyl-7-oxo-6-(piperazin-1-yl)-[1,2,4]triazolo[1,5-a]pyrimidin-4( 7H)-yl)-N-(4-(pentafluoro-λ⁶-sulfaneyl)phenyl)acetamide

Tert-Butyl 4-(2-(1-cyclobutylideneethyl)-5-ethyl-7-oxo-4-(2-oxo-2-((4-(pentafluoro-λ⁶-sulfaneyl)phenyl)am ino)ethyl)-4,7-dihydro-[1,2,4]triazolo[1,5-a]pyrimidin-6-yl)piperazine-1-carboxylate **61a** (43 mg, 62.53 µmol) was added to dichloromethane (2 mL), followed by addition of trifluoroacetic acid (0.8 mL). The reaction was performed at room temperature for 1 h. The mixture was concentrated under reduced pressure to obtain 2-(2-(1-cyclobutylideneethyl)-5-ethyl-7-oxo-6-(piperazin-1-yl)-[1,2,4]triazolo[1,5-a]pyrimidin-4( 7H)-yl)-N-(4-(pentafluoro-λ⁶-sulfaneyl)phenyl)acetamide **61b** (36 mg) with a yield of 97.99%.

MS m/z (ESI): 587.7 [M+1]

### Step 3

### 2-(6-(4-(5-(benzyloxy)-6-methylpyrimidine-4-carbonyl)piperazin-1-yl)-2-(1-cyclobutylideneethy l)-5-ethyl-7-oxo-[1,2,4]triazolo[1,5-a]pyrimidin-4(7H)-yl)-N-(4-(pentafluoro-λ⁶-sulfaneyl)phenyl )acetamide

2-(2-(1-cyclobutylideneethyl)-5-ethyl-7-oxo-6-(piperazin-1-yl)-[1,2,4]triazolo[1,5-a]pyr imidin-4(7H)-yl)-N-(4-(pentafluoro-λ⁶-sulfaneyl)phenyl)acetamide **61b** (30 mg, 51.05 µmol), 5-(benzyloxy)-6-methylpyrimidine-4-carboxylic acid **1g** (16.21 mg, 66.37 µmol), 1-(3-dimethylaminopropyl)-3-ethylcarbodiimide hydrochloride (14.68 mg, 76.58 µmol), 1-hydroxybenzotriazole (10.35 mg, 76.58 µmol), and N,N-diisopropylethylamine (32.99 mg, 255.27 µmol) were added to N,N-dimethylformamide (1 mL). The reaction was performed at room temperature for 4 h. Water (20 mL) was added to the reaction mixture, which was extracted with ethyl acetate (30 mL × 3). The combined organic phases were washed sequentially with saturated sodium chloride solution (30 mL × 2), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-2-(1-cyclobutylideneethy l)-5-ethyl-7-oxo-[1,2,4]triazolo[1,5-a]pyrimidin-4(7H)-yl)-N-(4-(pentafluoro-λ⁶-sulfaneyl)phenyl )acetamide 61c (30 mg) with a yield of 72.20%.

MS m/z (ESI): 813.5 [M+1]

### Step 4

### 2-(2-(1-cyclobutylideneethyl)-5-ethyl-6-(4-(5-hydroxy-6-methylpyrimidine-4-carbonyl)piperazin -1-yl)-7-oxo-[1,2,4]triazolo[1,5-a]pyrimidin-4(7H)-yl)-N-(4-(pentafluoro-λ⁶-sulfaneyl)phenyl)ace tamide

2-(6-(4-(5-(benzyloxy)-6-methylpyrimidine-4-carbonyl)piperazin-1-yl)-2-(1-cyclobutyli deneethyl)-5-ethyl-7-oxo-[1,2,4]triazolo[1,5-a]pyrimidin-4(7H)-yl)-N-(4-(pentafluoro-λ⁶-sulfane yl)phenyl)acetamide 61c (30 mg, 36.86 µmol) was added to dichloromethane (1 mL), followed by addition of trifluoroacetic acid (3 mL). The reaction was performed at room temperature for 16 h. The mixture was concentrated under reduced pressure, and the residue was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain 2-(2-(1-cyclobutylideneethyl)-5-ethyl-6-(4-(5-hydroxy-6-methylpyrimidine-4-carbonyl)piperazin -1-yl)-7-oxo-[1,2,4]triazolo[1,5-a]pyrimidin-4(7H)-yl)-N-(4-(pentafluoro-λ⁶-sulfaneyl)phenyl)ace tamide **61** (2.94 mg) with a yield of 10.40%. MS m/z (ESI): 724.3 [M+1]
¹H NMR (400 MHz, DMSO) δ 10.94 (s, 1H), 10.24 (s, 1H), 8.58 (s, 1H), 7.88 (d, J = 9.0 Hz, 2H), 7.75 (d, J = 8.7 Hz, 2H), 5.15 (s, 2H), 4.52 (d, J = 12.5 Hz, 1H), 3.57 - 3.43 (m, 4H), 3.28 (s, 1H), 2.99 (s, 4H), 2.80 (s, 3H), 2.63 (d, J = 10.8 Hz, 1H), 2.44 (s, 3H), 1.90 (t, J = 7.9 Hz, 2H), 1.84 (s, 3H), 1.17 (t, J = 7.4 Hz, 3H).

### Example 62

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(2,5,6,7-tetrahydrooxepin-3-yl)-[1,2,4]triazolo[1,5-a]pyrimidin-4(7H) -yl)acetamide

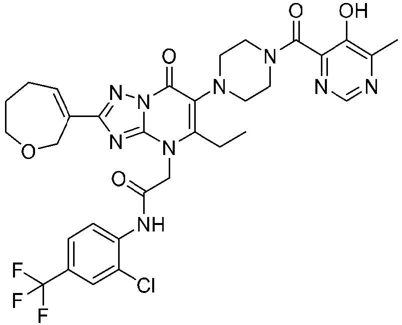

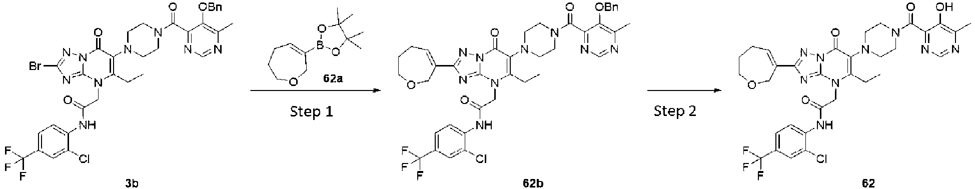

### Step 1

### 2-(6-(4-(5-(benzyloxy)-6-methylpyrimidine-4-carbonyl)piperazin-1-yl)-5-ethyl-7-oxo-2-(2,5,6,7-t etrahydrooxepin-3-yl)-[1,2,4]triazolo[1,5-a]pyrimidin-4(7H)-yl)-N-(2-chloro-4-(trifluoromethyl) phenyl)acetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (50 mg, 63.37 µmol), 4,4,5,5-tetramethyl-2-(2,5,6,7-tetrahydrooxepin-3-yl)-1,3,2-dioxaborolane **62a** (17.04 mg, 76.05 µmol, commercially available), [1,1'-bis(diphenylphosphino)ferrocene]palladium(II) dichloride (5.14 mg, 6.34 µmol), and sodium carbonate (20.15 mg, 190.11 µmol) were added to a mixed solution of 1,4-dioxane (1 mL) and water (0.3 mL). The mixture was subject to argon replacement four times, heated to 80 °C, and the reaction was performed for 1.5 hours. Water (20 mL) was added to the reaction mixture, the mixture was extracted with ethyl acetate (40 mL × 3), the combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-5-ethyl-7-oxo-2-(2,5,6,7-t etrahydrooxepin-3-yl)-[1,2,4]triazolo[1,5-a]pyrimidin-4(7H)-yl)-N-(2-chloro-4-(trifluoromethyl) phenyl)acetamide **62b** (33 mg) in a yield of 64.59%.

MS m/z (ESI): 806.3 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(2,5,6,7-tetrahydrooxepin-3-yl)-[1,2,4]triazolo[1,5-a]pyrimidin-4(7H) -yl)acetamide

2-(6-(4-(5-(benzyloxy)-6-methylpyrimidine-4-carbonyl)piperazin-1-yl)-5-ethyl-7-oxo-2 -(2,5,6,7-tetrahydrooxepin-3-yl)-[1,2,4]triazolo[1,5-a]pyrimidin-4(7H)-yl)-N-(2-chloro-4-(trifluor omethyl)phenyl)acetamide **62b** (33 mg, 40.93 µmol) was added to dichloromethane (2.5 mL), boron trichloride (0.8 mL, 1 M) was added at 0 °C, and the reaction was performed at 0 C for 1 hour. Methanol was added to quench the reaction, the mixture was concentrated under reduced pressure, and the residue was purified by preparative liquid chromatography (separation column: AKZONOBEL Kromasil; 250×21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(2,5,6,7-tetrahydrooxepin-3-yl)-[1,2,4]triazolo[1,5-a]pyrimidin-4(7H) -yl)acetamide (3.46 mg) in a yield of 11.59%.

MS m/z (ESI): 715.7 [M+1]
¹H NMR (400 MHz, DMSO) δ 10.36 (s, 1H), 10.23 (s, 1H), 8.58 (s, 1H), 8.06 (d, J = 8.6 Hz, 1H), 7.97 (s, 1H), 7.76 - 7.68 (m, 1H), 7.05 (t, J = 5.8 Hz, 1H), 5.30 (s, 2H), 4.63 (s, 2H), 4.52 (d, J = 12.5 Hz, 1H), 3.84 (t, J = 5.8 Hz, 4H), 3.49 (d, J = 12.1 Hz, 3H), 3.24 (t, J = 12.3 Hz, 1H), 2.81 (d, J = 11.4 Hz, 1H), 2.63 (d, J = 10.5 Hz, 1H), 2.44 (s, 3H), 1.87 - 1.77 (m, 2H), 1.18 (t, J = 7.5 Hz, 3H).

### Example 63

### 2-(5-ethyl-6-(4-(5-hydroxy-6-methylpyrimidine-4-carbonyl)piperazin-1-yl)-7-oxo-2-(2,5,6,7-tetra hydrooxepin-3-yl)-[1,2,4]triazolo[1,5-a]pyrimidin-4(7H)-yl)-N-(4-(pentafluoro-λ⁶-sulfaneyl)phen yl)acetamide

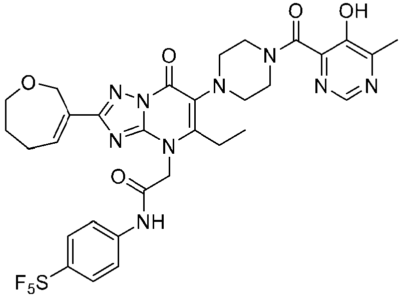

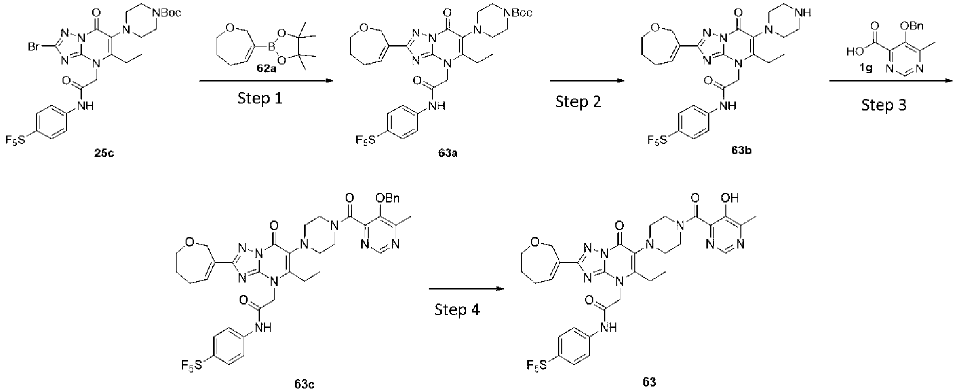

### Step 1

### tert-butyl 4-(5-ethyl-7-oxo-4-(2-oxo-2-((4-(pentafluoro-λ⁶-sulfaneyl)phenyl)amino)ethyl)-2-(2,5,6,7-tetrahy drooxepin-3-yl)-4,7-dihydro-[1,2,4]triazolo[1,5-a]pyrimidin-6-yl)piperazine-1-carboxylate

At room temperature, tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)piperazine-1-carboxylate 25c (50 mg, 72.83 µmol), 4,4,5,5-tetramethyl-2-(2,5,6,7-tetrahydrooxepin-3-yl)-1,3,2-dioxaborolane 62a (19.59 mg, 87.40 µmol), [1,1'-bis(diphenylphosphino)ferrocene]palladium(II) dichloride (5.90 mg, 7.28 µmol), and sodium carbonate (23.16 mg, 218.50 µmol) were added to a mixed solution of 1,4-dioxane (2 mL) and water (0.6 mL). The mixture was subject to argon replacement four times, heated to 80 °C, and the reaction was performed for 1.5 hours. Water (20 mL) was added to the reaction mixture, the mixture was extracted with ethyl acetate (40 mL × 3), the combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(5-ethyl-7-oxo-4-(2-oxo-2-((4-(pentafluoro-λ⁶-sulfaneyl)phenyl)amino)ethyl)-2-(2,5,6,7-tetrahy drooxepin-3-yl)-4,7-dihydro-[1,2,4]triazolo[1,5-a]pyrimidin-6-yl)piperazine-1-carboxylate 63a (32 mg) in a yield of 62.43%.

MS m/z (ESI): 648.3 [M-56+1]

### Step 2

### 2-(5-ethyl-7-oxo-6-(piperazin-1-yl)-2-(2,5,6,7-tetrahydrooxepin-3-yl)-[1,2,4]triazolo[1 ,5-a]pyrim idin-4(7H)-yl)-N-(4-(pentafluoro-λ⁶-sulfaneyl)phenyl)acetamide

Tert-butyl 4-(5-ethyl-7-oxo-4-(2-oxo-2-((4-(pentafluoro-λ⁶-sulfaneyl)phenyl)amino) ethyl)-2-(2,5,6,7-tetrahydrooxepin-3-yl)-4,7-dihydro-[1,2,4]triazolo[1,5-a]pyrimidin-6-yl) piperazine-1-carboxylate 63a (32 mg, 45.47 µmol) was added to dichloromethane (0.8 mL), followed by addition of trifluoroacetic acid (0.8 mL), and the reaction was performed at room temperature for 1 hour. The mixture was concentrated under reduced pressure to obtain 2-(5-ethyl-7-oxo-6-(piperazin-1-yl)-2-(2,5,6,7-tetrahydrooxepin-3-yl)-[1,2,4]triazolo[1,5-a]pyrim idin-4(7H)-yl)-N-(4-(pentafluoro-λ⁶-sulfaneyl)phenyl)acetamide 63b (27 mg) in a yield of 98.37%, which was used directly in the next step.

MS m/z (ESI): 604.2 [M+1]

### Step 3

### 2-(6-(4-(5-(benzyloxy)-6-methylpyrimidine-4-carbonyl)piperazin-1-yl)-5-ethyl-7-oxo-2-(2,5,6,7-t etrahydrooxepin-3-yl)-[1,2,4]triazolo[1,5-a]pyrimidin-4(7H)-yl)-N-(4-(pentafluoro-λ⁶-sulfaneyl)p henyl)acetamide

2-(5-Ethyl-7-oxo-6-(piperazin-1-yl)-2-(2,5,6,7-tetrahydrooxepin-3-yl)-[1,2,4]triazolo [1,5-a]pyrimidin-4(7H)-yl)-N-(4-(pentafluoro-λ⁶ -sulfaneyl)phenyl)acetamide 63b (27 mg, 44.73 µmol), 5-(benzyloxy)-6-methylpyrimidine-4-carboxylic acid 1g (14.20 mg, 58.15 µmol), 1-(3-dimethylaminopropyl)-3-ethylcarbodiimide hydrochloride (12.86 mg, 67.10 µmol), 1-hydroxybenzotriazole (9.07 mg, 67.10 µmol), and N,N-diisopropylethylamine (28.91 mg, 223.66 µmol) were added to N,N-dimethylformamide (2 mL), and the reaction was performed at room temperature for 4 h. Water (20 mL) was added to the reaction mixture, and the mixture was extracted with ethyl acetate (30 mL × 2). The combined organic phases were washed with saturated sodium chloride solution (30 mL × 2), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-5-ethyl-7-oxo-2-(2,5,6,7-t etrahydrooxepin-3-yl)-[1,2,4]triazolo[1,5-a]pyrimidin-4(7H)-yl)-N-(4-(pentafluoro-λ⁶-sulfaneyl)p henyl)acetamide 63c (33 mg) with a yield of 88.90%.

MS m/z (ESI): 830.2 [M+1]

### Step 4

### 2-(5-Ethyl-6-(4-(5-hydroxy-6-methylpyrimidine-4-carbonyl)piperazin-1-yl)-7-oxo-2-(2,5,6,7-tetr ahydrooxepin-3-yl)-[1,2,4]triazolo[1,5-a]pyrimidin-4(7H)-yl)-N-(4-(pentafluoro-λ⁶-sulfaneyl)phe nyl)acetamide

2-(6-(4-(5-(Benzyloxy)-6-Methylpyrimidine-4-carbonyl)piperazin-1-yl)-5-ethyl-7-oxo-2-(2,5,6,7-tetrahydrooxepin-3-yl)-[1,2,4]triazolo[1,5-a]pyrimidin-4(7H)-yl)-N-(4-(pentafluoro-λ⁶ -sulfaneyl)phenyl)acetamide 63c (33 mg, 39.77 µmol) was added to dichloromethane (1 mL), followed by addition of trifluoroacetic acid (3 mL), and the reaction was performed at room temperature for 16 h. The mixture was concentrated under reduced pressure, and the residue was purified by preparative liquid chromatography (separation column: AKZONOBEL Kromasil; 250 ×21.2 mm I.D.; 5 µm, 20 mL/min; mobile phase A: 0.05% TFA + H₂O, mobile phase B: CH₃CN) to obtain 2-(5-ethyl-6-(4-(5-hydroxy-6-methylpyrimidine-4-carbonyl)piperazin-1-yl)-7-oxo-2-(2,5,6,7-tetra hydrooxepin-3-yl)-[1,2,4]triazolo[1,5-a]pyrimidin-4(7H)-yl)-N-(4-(pentafluoro-λ⁶-sulfaneyl)phen yl)acetamide 63 (2.96 mg) with a yield of 9.85%.

MS m/z (ESI): 740.1 [M+1]
¹H NMR (400 MHz, DMSO) δ 10.96 (s, 1H), 10.24 (s, 1H), 8.58 (s, 1H), 7.90 - 7.82 (m, 2H), 7.77 (d, J = 8.9 Hz, 2H), 7.00 (t, J = 5.8 Hz, 1H), 5.18 (s, 2H), 4.62 (s, 2H), 4.52 (d, J = 12.8 Hz, 2H), 3.82 (t, J = 5.7 Hz, 2H), 3.49 (d, J = 12.6 Hz, 3H), 3.24 (d, J = 25.6 Hz, 1H), 3.03 - 2.91 (m, 3H), 2.81 (d, J = 11.2 Hz, 1H), 2.63 (d, J = 10.7 Hz, 1H), 2.44 (s, 3H), 2.43 (s, 1H), 1.84 - 1.76 (m, 2H), 1.16 (t, J = 7.4 Hz, 3H).

### Example 64

### N-(2-Chloro-4-(trifluoromethyl)phenyl)-2-(5-ethyl-6-(4-(5-hydroxy-6-methylpyrimidine-4-carbo nyl)piperazin-1-yl)-7-oxo-2-(spiro[2.5]oct-5-en-6-yl)-[1,2,4]triazolo[1,5-a]pyrimidin-4(7H)-yl)ac etamide

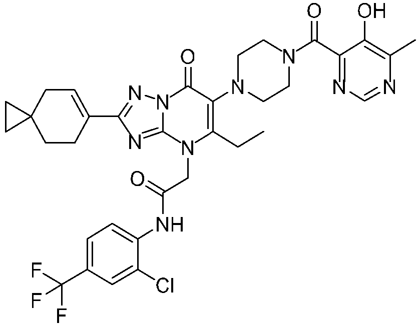

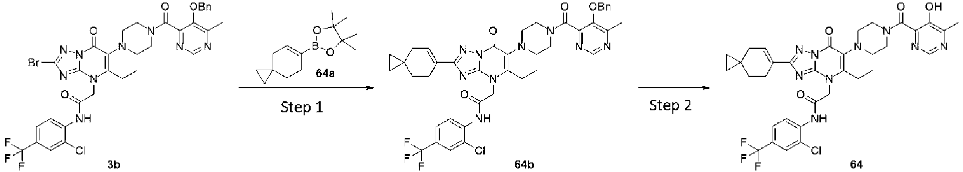

### Step 1

### 2-(6-(4-(5-(Benzyloxy)-6-methylpyrimidine-4-carbonyl)piperazin-1-yl)-5-ethyl-7-oxo-2-(spiro[2. 5]oct-5-en-6-yl)-[1,2,4]triazolo[1,5-a]pyrimidin-4(7H)-yl)-N-(2-chloro-4-(trifluoromethyl)phenyl )acetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide 3b (50 mg, 63.37 µmol), 4,4,5,5-tetramethyl-2-(spiro[2.5]oct-5-en-6-yl)-1,3,2-dioxaborolane 64a (17.81 mg, 76.05 µmol, prepared according to published patent WO2020128768), [1,1'-bis(diphenylphosphino)ferrocene]palladium(II) dichloride (5.14 mg, 6.34 µmol), and sodium carbonate (20.15 mg, 190.11 µmol) were added to a mixed solution of 1,4-dioxane (1 mL) and water (0.3 mL). The mixture was subject to argon gas replacement four times, heated to 80 °C, and the reaction was performed for 1.5 h. Water (20 mL) was added to the reaction mixture, and the mixture was extracted with ethyl acetate (30 mL × 2). The combined organic phases were washed with saturated sodium chloride solution (30 mL × 2), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-5-ethyl-7-oxo-2-(spiro[2. 5]oct-5-en-6-yl)-[1,2,4]triazolo[1,5-a]pyrimidin-4(7H)-yl)-N-(2-chloro-4-(trifluoromethyl)phenyl )acetamide 64b (37 mg) with a yield of 71.53%.

MS m/z (ESI): 816.2 [M+1]

### Step 2

### N-(2-Chloro-4-(trifluoromethyl)phenyl)-2-(5-ethyl-6-(4-(5-hydroxy-6-methylpyrimidine-4-carbo nyl)piperazin-1-yl)-7-oxo-2-(spiro[2.5]oct-5-en-6-yl)-[1,2,4]triazolo[1,5-a]pyrimidin-4(7H)-yl)ac etamide

2-(6-(4-(5-(Benzyloxy)-6-methylpyrimidine-4-carbonyl)piperazin-1-yl)-5-ethyl-7-oxo-2 -(spiro[2.5]oct-5-en-6-yl)-[1,2,4]triazolo[1,5-a]pyrimidin-4(7H)-yl)-N-(2-chloro-4-(trifluorometh yl)phenyl)acetamide **64b** (37 mg, 45.33 µmol) was added to dichloromethane (2.5 mL), and boron trichloride (0.8 mL) was added at 0 °C. The reaction was performed at 0 C for 1 h. Methanol was added to quench the reaction, and the mixture was concentrated under reduced pressure. The residue was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(spiro[2.5]oct-5-en-6-yl)-[1,2,4]triazolo[1,5-a]pyrimidin-4(7H)-yl)ace tamide 64 (2.81 mg) with a yield of 8.51%.

MS m/z (ESI): 726.3 [M+1]
¹H NMR (400 MHz, DMSO) δ 10.36 (s, 1H), 10.23 (s, 1H), 8.58 (s, 1H), 8.06 (d, J = 8.5 Hz, 1H), 7.96 (d, J = 2.2 Hz, 1H), 7.74 - 7.67 (m, 1H), 6.89 (s, 1H), 5.31 (s, 2H), 4.52 (d, J = 12.6 Hz, 1H), 3.50 (d, J = 12.2 Hz, 4H), 3.25 (t, J = 12.2 Hz, 2H), 2.98 (d, J = 7.2 Hz, 4H), 2.82 (d, J = 11.3 Hz, 1H), 2.64 (d, J = 11.9 Hz, 2H), 2.44 (s, 3H), 2.10 (s, 2H), 1.49 (t, J = 6.2 Hz, 2H), 1.19 (t, J = 7.4 Hz, 3H), 0.41 - 0.26 (m, 4H).

### Example 65

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(2-oxaspiro[3.5]non-6-en-7-yl)-[1,2,4]triazolo[1,5-a]pyrimidin-4(7H)-yl)acetamide

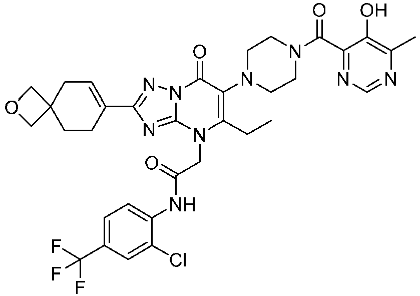

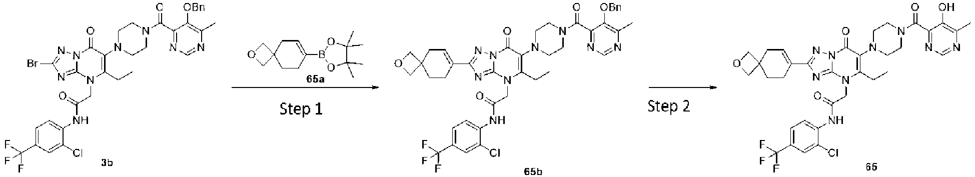

### Step 1

### 2-(6-(4-(5-(benzyloxy)-6-methylpyrimidine-4-carbonyl)piperazin-1-yl)-5-ethyl-7-oxo-2-(2-oxasp iro[3.5]non-6-en-7-yl)-[1,2,4]triazolo[1,5-a]pyrimidin-4(7H)-yl)-N-(2-chloro-4-(trifluoromethyl) phenyl)acetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide 3b (50 mg, 63.37 µmol), 4,4,5,5-tetramethyl-2-(2-oxaspiro[3.5]non-6-en-7-yl)-1,3,2-dioxaborolane 65a (19.02 mg, 76.05 µmol, prepared according to published patent WO2021055744), [1,1-bis(diphenylphosphino)ferrocene]palladium(II) dichloride (5.14 mg, 6.34 µmol), and sodium carbonate (20.15 mg, 190.11 µmol) were dissolved in a mixed solution of 1,4-dioxane (1 mL) and water (0.3 mL). The mixture was subject to argon replacement four times, then heated to 80 °C, and the reaction was performed for 1.5 hours. Water (20 mL) was added to the reaction mixture, and the mixture was extracted with ethyl acetate (30 mL × 2). The combined organic phases were washed with saturated sodium chloride solution (30 mL × 2), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-5-ethyl-7-oxo-2-(2-oxasp iro[3.5]non-6-en-7-yl)-[1,2,4]triazolo[1,5-a]pyrimidin-4(7H)-yl)-N-(2-chloro-4-(trifluoromethyl) phenyl)acetamide 65b (40 mg) with a yield of 75.84%.

MS m/z (ESI): 831.7 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(2-oxaspiro[3.5]non-6-en-7-yl)-[1,2,4]triazolo[1,5-a]pyrimidin-4(7H)-yl)acetamide

2-(6-(4-(5-(Benzyloxy)-6-methylpyrimidine-4-carbonyl)piperazin-1-yl)-5-ethyl-7-oxo-2 -(2-oxaspiro[3.5]non-6-en-7-yl)-[1,2,4]triazolo[1,5-a]pyrimidin-4(7H)-yl)-N-(2-chloro-4-(trifluor omethyl)phenyl)acetamide 65b (40 mg, 48.06 µmol) was added to dichloromethane (1 mL), followed by addition of trifluoroacetic acid (3 mL), and the reaction was performed at room temperature for 16 hours. The mixture was concentrated under reduced pressure, and the residue was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(2-oxaspiro[3.5]non-6-en-7-yl)-[1,2,4]triazolo[1,5-a]pyrimidin-4(7H)-yl)acetamide 65 (2.87 mg) with a yield of 7.92%.

MS m/z (ESI): 741.7 [M+1]
¹H NMR (400 MHz, DMSO) δ 10.36 (s, 1H), 10.24 (s, 1H), 8.58 (s, 1H), 8.06 (d, J = 8.6 Hz, 1H), 7.98 (d, J = 2.1 Hz, 1H), 7.75 - 7.69 (m, 1H), 6.77 (s, 1H), 5.31 (s, 2H), 4.52 (d, J = 13.0 Hz, 1H), 4.37 (d, J = 5.7 Hz, 2H), 4.29 (d, J = 5.7 Hz, 2H), 3.48 (t, J = 10.9 Hz, 4H), 3.24 (t, J = 12.8 Hz, 1H), 2.96 (s, 3H), 2.81 (d, J = 11.3 Hz, 1H), 2.63 (d, J = 10.9 Hz, 2H), 2.54 (s, 4H), 2.44 (s, 3H), 1.96 (t, J = 6.1 Hz, 2H), 1.18 (t, J = 7.4 Hz, 3H).

### Example 66

### 2-(2-(bicyclo[3.1.0]hex-2-en-3-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(4-(pentafluoro-λ⁶-sulfaneyl)phenyl )acetamide

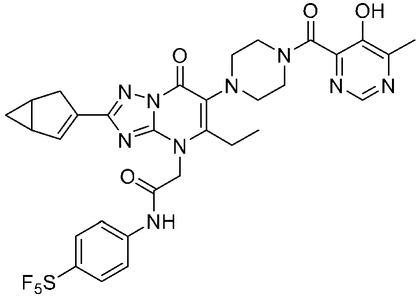

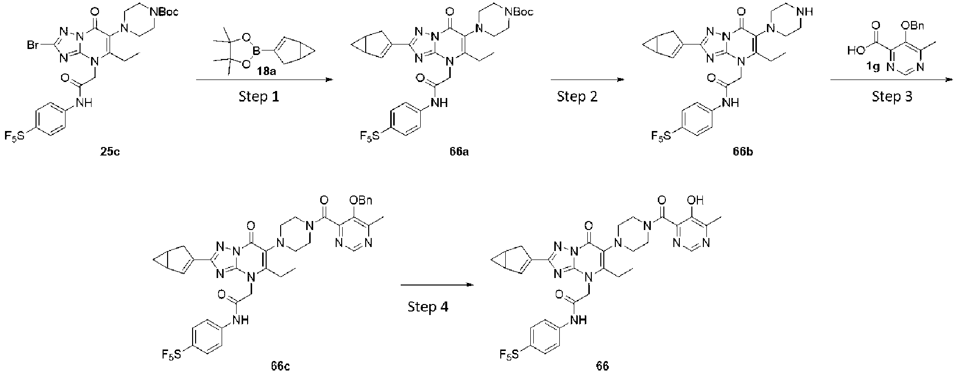

### Step 1

### tert-butyl 4-(2-(bicyclo[3.1.0]hex-2-en-3-yl)-5-ethyl-7-oxo-4-(2-oxo-2-((4-(pentafluoro-λ⁶-sulfaneyl)pheny l)amino)ethyl)-4,7-dihydro-[1,2,4]triazolo[1,5-a]pyrimidin-6-yl)piperazine-1-carboxylate

At room temperature, tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)piperazine-1-carboxylate 25c (50 mg, 72.83 µmol), 2-(bicyclo[3.1.0]hex-2-en-3-yl)-4,4,5,5-tetramethyl-1,3,2-dioxaborolane 18a (18.01 mg, 87.40 µmol), [1,1'-bis(diphenylphosphino)ferrocene]palladium(II) dichloride (5.90 mg, 7.28 µmol), and sodium carbonate (23.16 mg, 218.50 µmol) were dissolved in a mixed solution of 1,4-dioxane (2 mL) and water (0.6 mL). The mixture was subject to argon replacement four times, heated to 80 °C, and the reaction was performed for 1.5 hours. Water (20 mL) was added to the reaction mixture, which was extracted with ethyl acetate (40 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(2-(bicyclo[3.1.0]hex-2-en-3-yl)-5-ethyl-7-oxo-4-(2-oxo-2-((4-(pentafluoro-λ⁶-sulfaneyl)pheny l)amino)ethyl)-4,7-dihydro-[1,2,4]triazolo[1,5-a]pyrimidin-6-yl)piperazine-1-carboxylate 66a (35 mg) with a yield of 70.08%.

MS m/z (ESI): 630.3 [M-56+1]

### Step 2

### 2-(2-(bicyclo[3.1.0]hex-2-en-3-yl)-5-ethyl-7-oxo-6-(piperazin-1-yl)-[1,2,4]triazolo[1,5-a]pyrimid in-4(7H)-yl)-N-(4-(pentafluoro-λ⁶-sulfaneyl)phenyl)acetamide

Tert-Butyl 4-(2-(bicyclo[3.1.0]hex-2-en-3-yl)-5-ethyl-7-oxo-4-(2-oxo-2-((4-(pentafluoro-λ⁶-sulfaneyl)pheny 1)amino)ethyl)-4,7-dihydro-[1,2,4]triazolo[1,5-a]pyrimidin-6-yl)piperazine-1-carboxylate **66a** (35 mg, 51.04 µmol) was added to dichloromethane (2 mL), followed by addition of trifluoroacetic acid (0.8 mL). The reaction was performed at room temperature for 1 hour. The mixture was concentrated under reduced pressure to obtain 2-(2-(bicyclo[3.1.0]hex-2-en-3-yl)-5-ethyl-7-oxo-6-(piperazin-1-yl)-[1,2,4]triazolo[1,5-a]pyrimid in-4(7H)-yl)-N-(4-(pentafluoro-λ⁶-sulfaneyl)phenyl)acetamide **66b** (29 mg) with a yield of 97.02%.

MS m/z (ESI): 586.2 [M+1]

### Step 3

### 2-(6-(4-(5-(benzyloxy)-6-methylpyrimidine-4-carbonyl)piperazin-1-yl)-2-(bicyclo[3.1.0]hex-2-en -3-yl)-5-ethyl-7-oxo-[1,2,4]triazolo[1,5-a]pyrimidin-4(7H)-yl)-N-(4-(pentafluoro-λ⁶-sulfaneyl)ph enyl)acetamide

2-(2-(Bicyclo[3.1.0]hex-2-en-3-yl)-5-ethyl-7-oxo-6-(piperazin-1-yl)-[1,2,4]triazolo[1,5-a]pyrimidin-4(7H)-yl)-N-(4-(pentafluoro-λ⁶-sulfaneyl)phenyl)acetamide **66b** (29 mg, 49.52 µmol), 5-(benzyloxy)-6-methylpyrimidine-4-carboxylic acid **1g** (15.72 mg, 64.38 µmol), 1-(3-dimethylaminopropyl)-3-ethylcarbodiimide hydrochloride (14.24 mg, 74.28 µmol), 1-hydroxybenzotriazole (10.04 mg, 74.28 µmol), and N,N-diisopropylethylamine (32.00 mg, 247.61 µmol) were added to N,N-dimethylformamide (2 mL). The reaction was performed at room temperature for 4 hours. Water (20 mL) was added to the reaction mixture, which was extracted with ethyl acetate (30 mL × 2). The combined organic phases were washed sequentially with saturated sodium chloride solution (30 mL × 2), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-2-(bicyclo[3.1.0]hex-2-en -3-yl)-5-ethyl-7-oxo-[1,2,4]triazolo[1,5-a]pyrimidin-4(7H)-yl)-N-(4-(pentafluoro-λ⁶-sulfaneyl)ph enyl)acetamide 66c (30 mg) with a yield of 74.62%.

MS m/z (ESI): 812.3 [M+1]

### Step 4

### 2-(2-(bicyclo[3.1.0]hex-2-en-3-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(4-(pentafluoro-λ⁶-sulfaneyl)phenyl )acetamide

2-(6-(4-(5-(Benzyloxy)-6-methylpyrimidine-4-carbonyl)piperazin-1-yl)-2-(bicyclo[3.1. 0]hex-2-en-3-yl)-5-ethyl-7-oxo-[1,2,4]triazolo[1,5-a]pyrimidin-4(7H)-yl)-N-(4-(pentafluoro-λ⁶-s ulfaneyl)phenyl)acetamide **66c** (30 mg, 36.95 µmol) was added to dichloromethane (1 mL), followed by addition of trifluoroacetic acid (3 mL). The reaction was performed at room temperature for 18 hours. The mixture was concentrated under reduced pressure, and the residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 2-(2-(bicyclo[3.1.0]hex-2-en-3-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(4-(pentafluoro-λ⁶-sulfaneyl)phenyl )acetamide **66** (3.73 mg) with a yield of 11.19%.

MS m/z (ESI): 722.1 [M+1]
¹H NMR (400 MHz, DMSO) δ 10.96 (s, 1H), 10.24 (s, 1H), 8.58 (d, J = 4.3 Hz, 1H), 7.88 (d, J = 9.1 Hz, 2H), 7.77 (d, J = 8.9 Hz, 2H), 6.77 (d, J = 2.1 Hz, 1H), 5.18 (s, 2H), 4.58 - 4.46 (m, 2H), 3.49 (d, J = 12.5 Hz, 3H), 3.24 (s, 1H), 3.04 - 2.88 (m, 4H), 2.84 - 2.58 (m, 4H), 2.44 (s, 3H), 1.96 (s, 1H), 1.78 (s, 1H), 1.16 (q, J = 8.0 Hz, 3H), 1.01 (dt, J = 8.0, 4.1 Hz, 1H).

### Example 67

### 2-(2-(benzofuran-5-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamid e

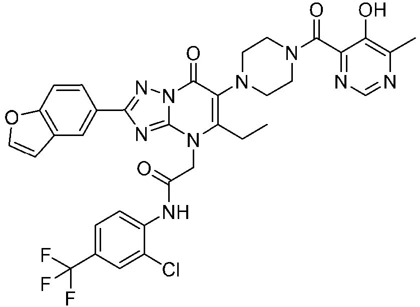

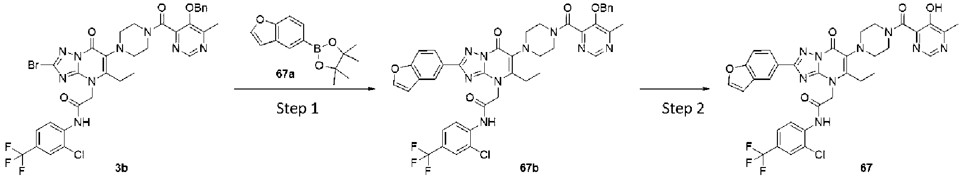

### Step 1

### 2-(2-(benzofuran-5-yl)-6-(4-(5-(benzyloxy)-6-methylpyrimidine-4-carbonyl)piperazin-1-yl)-5-eth yl-7-oxo-[1,2,4]triazolo[1,5-a]pyrimidin-4(7H)-yl)-N-(2-chloro-4-(trifluoromethyl)phenyl)aceta mide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide 3b (200 mg, 253.49 µmol), 2-(benzofuran-5-yl)-4,4,5,5-tetramethyl-1,3,2-dioxaborolane 67a (74.25 mg, 304.18 µmol, commercially available), [1,1'-bis(diphenylphosphino)ferrocene]palladium(II) dichloride (20.55 mg, 25.35 µmol), and sodium carbonate (80.60 mg, 760.46 µmol) were dissolved in a mixture solution of 1,4-dioxane (1 mL) and water (0.3 mL). The mixture was subject to argon replacement four times, heated to 80 °C, and the reaction was performed for 1.5 hours. Water (20 mL) was added to the reaction solution, and the mixture was extracted with ethyl acetate (40 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(2-(benzofuran-5-yl)-6-(4-(5-(benzyloxy)-6-methylpyrimidine-4-carbonyl)piperazin-1-yl)-5-eth yl-7-oxo-[1,2,4]triazolo[1,5-a]pyrimidin-4(7H)-yl)-N-(2-chloro-4-(trifluoromethyl)phenyl)aceta mide 67b (150 mg) in a yield of 71.62%.

MS m/z (ESI): 825.6 [M+1]

### Step 2

### 2-(2-(benzofuran-5-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamid e

2-(2-(Benzofuran-5-yl)-6-(4-(5-(benzyloxy)-6-methylpyrimidine-4-carbonyl)piperazin-1-yl)-5-ethyl-7-oxo-[1,2,4]triazolo[1,5-a]pyrimidin-4(7H)-yl)-N-(2-chloro-4-(trifluoromethyl) phenyl)acetamide **67b** (150 mg, 181.55 µmol) was added to dichloromethane (6 mL), and boron trichloride (3 mL) was added at 0 °C. The reaction was performed at 0 °C for 1 hour. Methanol was added to quench the reaction, and the mixture was concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 2-(2-(benzofuran-5-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamid e 67 (15.33 mg) in a yield of 11.39%.

MS m/z (ESI): 735.7 [M+1]
¹H NMR (400 MHz, DMSO) δ 10.43 (s, 1H), 10.24 (s, 1H), 8.59 (s, 1H), 8.43 (d, J = 1.7 Hz, 1H), 8.14 - 8.03 (m, 3H), 7.98 (d, J = 2.1 Hz, 1H), 7.72 (t, J = 7.8 Hz, 2H), 7.08 (d, J = 2.3 Hz, 1H), 5.42 (s, 2H), 4.54 (d, J = 12.5 Hz, 1H), 3.54 (q, J = 11.0 Hz, 3H), 3.27 (t, J = 12.5 Hz, 1H), 3.02 (s, 3H), 2.85 (d, J = 11.3 Hz, 1H), 2.68 (d, J = 10.0 Hz, 1H), 2.45 (s, 3H), 1.22 (t, J = 7.4 Hz, 3H).

### Example 68

### 2-(2-(benzofuran-6-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamid e

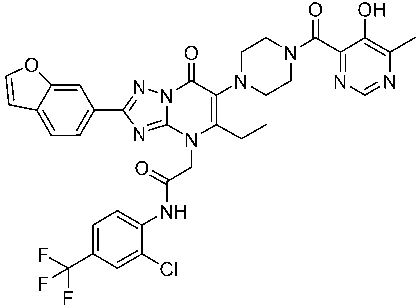

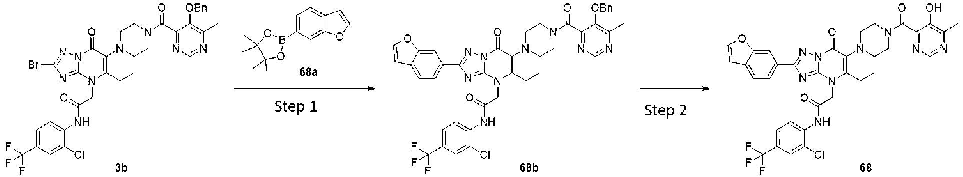

### Step 1

### 2-(2-(benzofuran-6-yl)-6-(4-(5-(benzyloxy)-6-methylpyrimidine-4-carbonyl)piperazin-1-yl)-5-eth yl-7-oxo-[1,2,4]triazolo[1,5-a]pyrimidin-4(7H)-yl)-N-(2-chloro-4-(trifluoromethyl)phenyl)aceta mide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide 3b (150 mg, 190.11 µmol), 2-(benzofuran-6-yl)-4,4,5,5-tetramethyl-1,3,2-dioxaborolane 68a (55.69 mg, 228.14 µmol, commercially available), [1,1'-bis(diphenylphosphino)ferrocene]palladium(II) dichloride (15.41 mg, 19.01 µmol), and sodium carbonate (60.45 mg, 570.34 µmol) were added to a mixture solution of 1,4-dioxane (1 mL) and water (0.3 mL). The mixture was subject to argon replacement four times, heated to 80 °C, and the reaction was performed for 1.5 hours. Water (20 mL) was added to the reaction solution, and the mixture was extracted with ethyl acetate (40 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(2-(benzofuran-6-yl)-6-(4-(5-(benzyloxy)-6-methylpyrimidine-4-carbonyl)piperazin-1-yl)-5-eth yl-7-oxo-[1,2,4]triazolo[1,5-a]pyrimidin-4(7H)-yl)-N-(2-chloro-4-(trifluoromethyl)phenyl)aceta mide 68b (120 mg) in a yield of 76.40%.

MS m/z (ESI): 825.6 [M+1]

### Step 2

### 2-(2-(benzofuran-6-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamid e

2-(2-(benzofuran-6-yl)-6-(4-(5-(benzyloxy)-6-methylpyrimidine-4-carbonyl)piperazin-1 -yl)-5-ethyl-7-oxo-[1,2,4]triazolo[1,5-a]pyrimidin-4(7H)-yl)-N-(2-chloro-4-(trifluoromethyl) phenyl)acetamide 68b (120 mg, 145.24 µmol) was added to dichloromethane (6 mL), and boron trichloride (2 mL) was added at 0 °C. The reaction was performed at 0 °C for 1 hour. Methanol was added to quench the reaction, and the mixture was concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 2-(2-(benzofuran-6-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamid e 68 (15.33 mg) in a yield of 14.03%.

MS m/z (ESI): 735.7 [M+1]
¹H NMR (400 MHz, DMSO) δ 10.45 (s, 1H), 10.24 (s, 1H), 8.59 (s, 1H), 8.26 (s, 1H), 8.13 (d, J = 2.2 Hz, 1H), 8.08 - 8.02 (m, 2H), 7.98 (d, J = 2.1 Hz, 1H), 7.80 (d, J = 8.1 Hz, 1H), 7.72 (dd, J = 8.8, 2.1 Hz, 1H), 7.06 (d, J = 2.2 Hz, 1H), 5.42 (s, 2H), 4.55 (d, J = 12.5 Hz, 1H), 3.60 - 3.49 (m, 3H), 3.27 (t, J = 12.1 Hz, 1H), 3.09 - 2.97 (m, 3H), 2.90 - 2.81 (m, 1H), 2.68 (d, J = 10.8 Hz, 1H), 2.45 (s, 3H), 1.22 (t, J = 7.5 Hz, 3H).

### Example 69

### 2-(2-(benzofuran-7-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamid

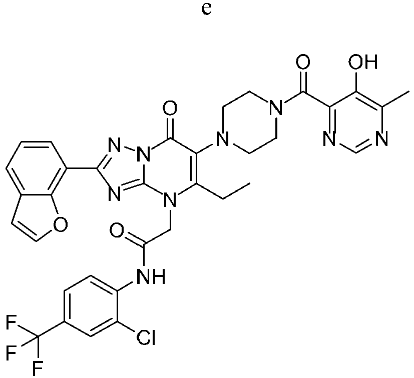

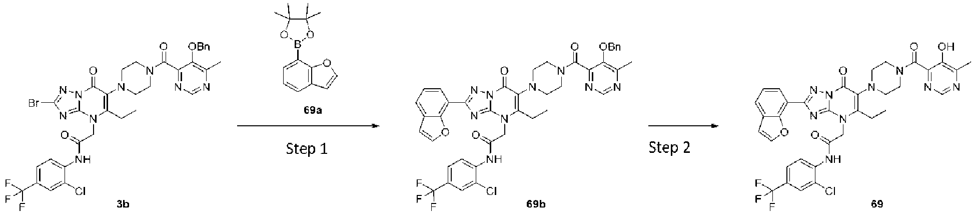

### Step 1

### 2-(2-(benzofuran-7-yl)-6-(4-(5-(benzyloxy)-6-methylpyrimidine-4-carbonyl)piperazin-1-yl)-5-eth yl-7-oxo-[1,2,4]triazolo[1,5-a]pyrimidin-4(7H)-yl)-N-(2-chloro-4-(trifluoromethyl)phenyl)aceta mide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide 3b (200 mg, 253.49 µmol), 2-(benzofuran-7-yl)-4,4,5,5-tetramethyl-1,3,2-dioxaborolane 69a (53.37 mg, 329.53 µmol, commercially available), [1,1'-bis(diphenylphosphino)ferrocene]palladium(II) dichloride (20.55 mg, 25.35 µmol), and sodium carbonate (80.60 mg, 760.46 µmol) were dissolved in a mixed solution of 1,4-dioxane (1 mL) and water (0.3 mL). The mixture was subject to argon replacement four times, heated to 80 °C, and the reaction was performed for 1.5 hours. Water (20 mL) was added to the reaction mixture, followed by extraction with ethyl acetate (40 mL × 3). The combined organic phases were washed with saturated sodium chloride solution (20 mL), dried over anhydrous sodium sulfate, filtered, and concentrated under reduced pressure. The obtained residue was purified by silica gel column chromatography (eluent: System B) to obtain 2-(2-(benzofuran-7-yl)-6-(4-(5-(benzyloxy)-6-methylpyrimidine-4-carbonyl)piperazin-1-yl)-5-eth yl-7-oxo-[1,2,4]triazolo[1,5-a]pyrimidin-4(7H)-yl)-N-(2-chloro-4-(trifluoromethyl)phenyl)aceta mide 69b (150 mg) with a yield of 71.62%.

MS m/z (ESI): 825.6 [M+1]

### Step 2

### 2-(2-(benzofuran-7-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamid e

2-(2-(benzofuran-7-yl)-6-(4-(5-(benzyloxy)-6-methylpyrimidine-4-carbonyl)piperazin-1 -yl)-5-ethyl-7-oxo-[1,2,4]triazolo[1,5-a]pyrimidin-4(7H)-yl)-N-(2-chloro-4-(trifluoromethyl) phenyl)acetamide 69b (150 mg, 181.55 µmol) was added to dichloromethane (4.5 mL), and boron trichloride (1.5 mL) was added at 0 °C. The reaction was performed at 0 °C for 1 hour. Methanol was added to quench the reaction, and the mixture was concentrated under reduced pressure. The residue was purified by preparative liquid chromatography (separation column: AKZONOBEL Kromasil; 250×21.2 mm I.D.; 5 µm, 20 mL/min; mobile phase A: 0.05% TFA + H₂O, mobile phase B: CH₃CN) to obtain 2-(2-(benzofuran-7-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamid e 69 (15.04 mg) with a yield of 10.94%.

MS m/z (ESI): 735.6 [M+1]
¹H NMR (400 MHz, DMSO) δ 10.44 (s, 1H), 10.25 (s, 1H), 8.59 (s, 1H), 8.17 (d, J = 2.2 Hz, 1H), 8.10 - 7.93 (m, 3H), 7.86 - 7.79 (m, 1H), 7.71 (dd, J = 8.6, 2.1 Hz, 1H), 7.40 (t, J = 7.7 Hz, 1H), 7.09 (d, J = 2.2 Hz, 1H), 5.43 (s, 2H), 4.55 (d, J = 12.4 Hz, 1H), 3.55 (q, J = 12.2 Hz, 3H), 3.28 (t, J = 13.5 Hz, 1H), 3.03 (t, J = 6.9 Hz, 3H), 2.89 - 2.80 (m, 1H), 2.70 (s, 1H), 2.45 (s, 3H), 1.23 (t, J = 7.4 Hz, 3H).

### Example 70

### N-(2-chloro-4-(pentafluoro-λ⁶-sulfaneyl)phenyl)-2-(2-(cyclohept-1-en-1-yl)-5-ethyl-6-(4-(5-hydr oxy-6-methylpyrimidine-4-carbonyl)piperazin-1-yl)-7-oxo-[1,2,4]triazolo[1,5-a]pyrimidin-4(7H) -yl)acetamide

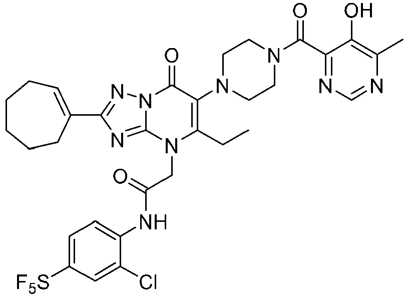

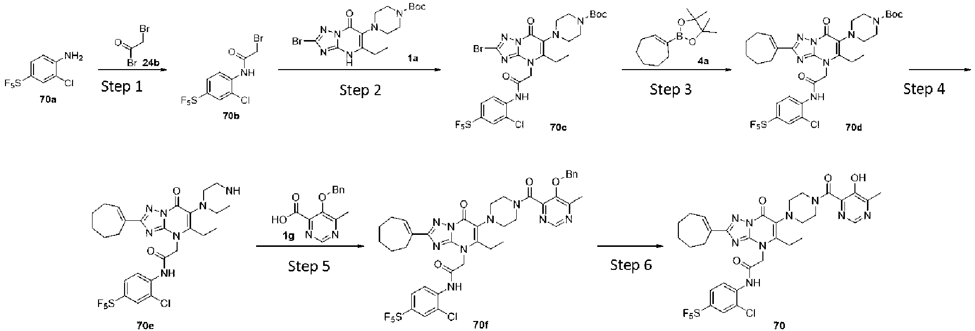

### Step 1

### 2-bromo-N-(2-chloro-4-(pentafluoro-λ⁶-sulfaneyl)phenyl)acetamide

Under ice bath conditions, a solution of 2-bromoacetyl bromide 24b (437.72 mg, 2.17 mmol, 188.35 µL) in dichloromethane (10 mL) was slowly added dropwise to a solution of 2-chloro-4-(pentafluoro-λ⁶-sulfaneyl)aniline 70a (500 mg, 1.97 mmol, commercially available) in dichloromethane (10 mL). The reaction was performed at room temperature overnight. Water (20 mL) was added to the reaction mixture, followed by extraction with ethyl acetate (40 mL × 3). The combined organic phases were washed with saturated sodium chloride solution (20 mL), dried over anhydrous sodium sulfate, filtered, and concentrated under reduced pressure. The obtained residue was purified by silica gel column chromatography (eluent: System A) to obtain 2-bromo-N-(2-chloro-4-(pentafluoro-λ⁶-sulfaneyl)phenyl)acetamide **70b** (600 mg) with a yield of 81.26%.

MS m/z (ESI): 373.9 [M+1]

### Step 2

### tert-butyl 4-(2-bromo-4-(2-((2-chloro-4-(pentafluoro-λ⁶-sulfaneyl)phenyl)amino)-2-oxoethyl)-5-ethyl-7-ox o-4,7-dihydro-[1,2,4]triazolo[1,5-a]pyrimidin-6-yl)piperazine-1-carboxylate

At room temperature, tert-butyl 4-(2-bromo-5-ethyl-7-oxo-4,7-dihydro-[1,2,4]triazolo[1,5-a]pyrimidin-6-yl)piperazine-1-carboxy late 1a (280 mg, 655.28 µmol), 2-bromo-N-(2-chloro-4-(pentafluoro-λ⁶-sulfaneyl)phenyl)acetamide 70b (294.53 mg, 786.34 µmol), and N,N-diisopropylethylamine (423.44 mg, 3.28 mmol) were added to N,N-dimethylformamide (10 mL), the temperature was raised to 50 °C, and the reaction was performed for 2 hours. Water (20 mL) was added to the reaction solution, the mixture was extracted with ethyl acetate (40 mL×3), the combined organic phases were washed with saturated sodium chloride solution (20 mL), dried over anhydrous sodium sulfate, filtered, and concentrated under reduced pressure, and the obtained residue was purified by silica gel column chromatography (eluent: System B) to obtain tert-butyl 4-(2-bromo-4-(2-((2-chloro-4-(pentafluoro-λ⁶-sulfaneyl)phenyl)amino)-2-oxoethyl)-5-ethyl-7-ox o-4,7-dihydro-[1,2,4]triazolo[1,5-a]pyrimidin-6-yl)piperazine-1-carboxylate70c (420 mg) with a yield of 88.90%.

MS m/z (ESI): 664.1 [M-56+H]

### Step 3

### tert-butyl 4-(4-(2-((2-chloro-4-(pentafluoro-λ⁶-sulfaneyl)phenyl)amino)-2-oxoethyl)-2-(cyclohept-1-en-1-yl )-5-ethyl-7-oxo-4,7-dihydro-[1,2,4]triazolo[1,5-a]pyrimidin-6-yl)piperazine-1-carboxylate

At room temperature, tert-butyl 4-(2-bromo-4-(2-((2-chloro-4-(pentafluoro-λ⁶-sulfaneyl)phenyl)amino)-2-oxoethyl)-5-ethyl-7-ox o-4,7-dihydro-[1,2,4]triazolo[1,5-a]pyrimidin-6-yl)piperazine-1-carboxylate**70c** (220 mg, 305.16 µmol), 2-(cyclohept-1-en-1-yl)-4,4,5,5-tetramethyl-1,3,2-dioxaborolane 4a (81.34 mg, 366.19 µmol), [1,1'-bis(diphenylphosphino)ferrocene]palladium(II) dichloride (24.73 mg, 30.52 µmol), and sodium carbonate (97.03 mg, 915.48 µmol) were dissolved in a mixed solution of 1,4-dioxane (5 mL) and water (0.5 mL), The mixture was subject to argon replacement 3 times, the temperature was raised to 85 °C, and the reaction was performed for 2 hours. Water (20 mL) was added to the reaction solution, the mixture was extracted with ethyl acetate (40 mL ×3), the combined organic phases were washed with saturated sodium chloride solution (20 mL), dried over anhydrous sodium sulfate, filtered, and concentrated under reduced pressure, and the obtained residue was purified by silica gel column chromatography (eluent: System B) to obtain tert-butyl 4-(4-(2-((2-chloro-4-(pentafluoro-λ⁶-sulfaneyl)phenyl)amino)-2-oxoethyl)-2-(cyclohept-1-en-1-yl )-5-ethyl-7-oxo-4,7-dihydro-[1,2,4]triazolo[1,5-a]pyrimidin-6-yl)piperazine-1-carboxylate 70d (170 mg) with a yield of 75.67%.

MS m/z (ESI): 680.2 [M-56+H]

### Step 4

### N-(2-chloro-4-(pentafluoro-λ⁶-sulfaneyl)phenyl)-2-(2-(cyclohept-1-en-1-yl)-5-ethyl-7-oxo-6-(pip erazin-1-yl)-[1,2,4]triazolo[1,5-a]pyrimidin-4(7H)-yl)acetamide

At room temperature, trifluoroacetic acid (1.5 mL) was added to a solution of tert-butyl 4-(4-(2-((2-chloro-4-(pentafluoro-λ⁶-sulfaneyl)phenyl)amino)-2-oxoethyl)-2-(cyclohept-1-en-1-yl )-5-ethyl-7-oxo-4,7-dihydro-[1,2,4]triazolo[1,5-a]pyrimidin-6-yl)piperazine-1-carboxylate **70d** (170 mg, 230.92 µmol) in dichloromethane (6 mL), and the reaction was performed at room temperature for 40 minutes. The mixture was concentrated under reduced pressure to obtain N-(2-chloro-4-(pentafluoro-λ⁶-sulfaneyl)phenyl)-2-(2-(cyclohept-1-en-1-yl)-5-ethyl-7-oxo-6-(pip erazin-1-yl)-[1,2,4]triazolo[1,5-a]pyrimidin-4(7H)-yl)acetamide **70e** (146.8 mg) with a yield of 99.94%.

MS m/z (ESI): 636.2 [M+1]

### Step 5

### 2-(6-(4-(5-(benzyloxy)-6-methylpyrimidine-4-carbonyl)piperazin-1-yl)-2-(cyclohept-1-en-1-yl)-5 -ethyl-7-oxo-[1,2,4]triazolo[1,5-a]pyrimidin-4(7H)-yl)-N-(2-chloro-4-(pentafluoro-λ⁶-sulfaneyl)p henyl)acetamide

At room temperature, 5-(benzyloxy)-6-methylpyrimidine-4-carboxylic acid 1g (42.05 mg, 172.15 µmol), 1-(3-dimethylaminopropyl)-3-ethylcarbodiimide hydrochloride (44.00 mg, 229.53 µmol), 1-hydroxybenzotriazole (31.01 mg, 229.53 µmol), and N,N-diisopropylethylamine (74.16 mg, 573.83 µmol) were added to N,N-dimethylformamide (3 mL) and reacted at room temperature for 30 minutes, then a solution of N-(2-chloro-4-(pentafluoro-λ⁶-sulfaneyl)phenyl)-2-(2-(cyclohept-1-en-1-yl)-5-ethyl-7-oxo-6-(pip erazin-1-yl)-[1,2,4]triazolo[1,5-a]pyrimidin-4(7H)-yl)acetamide **70e** (73 mg, 114.77 µmol) in N,N-dimethylformamide (3 mL) was added, and the reaction was performed at room temperature for 16 hours. Water (20 mL) was added to the reaction solution, the mixture was extracted with ethyl acetate (40 mL×3), the combined organic phases were washed with saturated sodium chloride solution (20 mL), dried over anhydrous sodium sulfate, filtered, and concentrated under reduced pressure, and the obtained residue was purified by silica gel column chromatography (eluent: System B) to obtain 2-(6-(4-(5-(benzyloxy)-6-methylpyrimidine-4-carbonyl)piperazin-1-yl)-2-(cyclohept-1-en-1-yl)-5 -ethyl-7-oxo-[1,2,4] triazolo[1,5-a]pyrimidin-4(7H)-yl)-N-(2-chloro-4-(pentafluoro-λ⁶-sulfaneyl)phenyl)acetamide 70f (98 mg) with a yield of 99.03%.

MS m/z (ESI): 862.3 [M+1]

### Step 6

### N-(2-chloro-4-(pentafluoro-λ⁶-sulfaneyl)phenyl)-2-(2-(cyclohept-1-en-1-yl)-5-ethyl-6-(4-(5-hydr oxy-6-methylpyrimidine-4-carbonyl)piperazin-1-yl)-7-oxo-[1,2,4]triazolo[1,5-a]pyrimidin-4(7H) -yl)acetamide

At room temperature, trifluoroacetic acid (4 mL) was added to a solution of 2-(6-(4-(5-(benzyloxy)-6-methylpyrimidine-4-carbonyl)piperazin-1-yl)-2-(cyclohept-1-en-1-yl)-5 -ethyl-7-oxo-[1,2,4]triazolo[1,5-a]pyrimidin-4(7H)-yl)-N-(2-chloro-4-(pentafluoro-λ⁶-sulfaneyl)p henyl) acetamide 70f (98 mg, 113.65 µmol) in dichloromethane (1 mL), and the reaction was performed at room temperature for 18 hours. Methanol was added to quench the reaction, the mixture was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain N-(2-chloro-4-(pentafluoro-λ⁶-sulfaneyl)phenyl)-2-(2-(cyclohept-1-en-1-yl)-5-ethyl-6-(4-(5-hydr oxy-6-methylpyrimidine-4-carbonyl)piperazin-1-yl)-7-oxo-[1,2,4]triazolo[1,5-a]pyrimidin-4(7H) -yl)acetamide 70 (23.25 mg) with a yield of 26.23%.

MS m/z (ESI): 772.2 [M+1]
¹H NMR (400 MHz, DMSO-d6) δ 10.42 (s, 1H), 10.40 - 9.89 (m, 1H), 8.59 (s, 1H), 8.16 (d, J = 2.6 Hz, 1H), 8.11 (d, J = 9.1 Hz, 1H), 7.91 (dd, J = 9.2, 2.6 Hz, 1H), 7.10 (t, J = 6.8 Hz, 1H), 5.33 (s, 2H), 4.53 (d, J = 12.5 Hz, 1H), 3.58 - 3.43 (m, 3H), 3.25 (t, J = 12.7 Hz, 1H), 3.08 - 2.88 (m, 3H), 2.81 (d, J = 11.2 Hz, 1H), 2.77 - 2.70 (m, 2H), 2.64 (d, J = 11.0 Hz, 1H), 2.45 (s, 3H), 2.31 (q, J = 6.3 Hz, 2H), 1.79 (p, J = 5.8 Hz, 2H), 1.52 (dq, J = 15.5, 9.0, 7.0 Hz, 4H), 1.18 (t, J = 7.4 Hz, 3H).

### Example 71

### 2-(5-ethyl-6-(4-(3-hydroxypicolinoyl)piperazin-1-yl)-7-oxo-2-(5-oxo-1,3a,4,5,6,6a-hexahydropen talen-2-yl)-[1,2,4]triazolo[1,5-a]pyrimidin-4(7H)-yl)-N-(4-(pentafluoro-λ⁶-sulfaneyl)phenyl)aceta mide

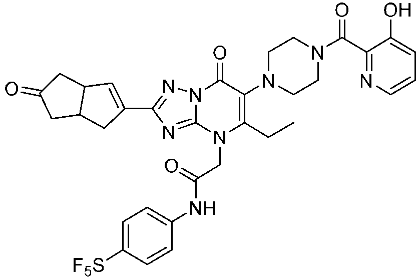

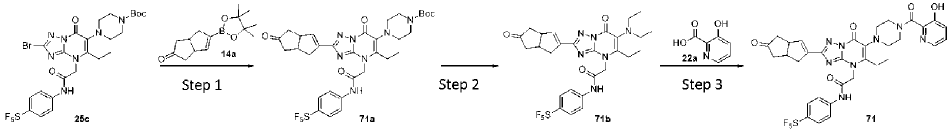

### Step 1

### tert-butyl 4-(5-ethyl-7-oxo-2-(5-oxo-1,3a,4,5,6,6a-hexahydropentalen-2-yl)-4-(2-oxo-2-((4-(pentafluoro-λ⁶-sulfaneyl)phenyl)amino)ethyl)-4,7-dihydro-[1,2,4]triazolo[1,5-a]pyrimidin-6-yl)piperazine-1-car boxylate

At room temperature, tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)piperazine-1-carboxylate 25c (80 mg, 116.53 µmol), 5-(4,4,5,5-tetramethyl-1,3,2-dioxaborolan-2-yl)-3,3a,4,6a-tetrahydro-1H-cyclopenta[c]furan-2-on e 14a (34.70 mg, 139.84 µmol), [1,1'-bis(diphenylphosphino)ferrocene]palladium(II) dichloride (9.45 mg, 11.65 µmol), and sodium carbonate (37.05 mg, 349.60 µmol) were dissolved in a mixed solution of 1,4-dioxane (3 mL) and water (0.3 mL). The mixture was subject to argon replacement three times, then heated to 80 °C, and the reaction was performed for 2 hours. The mixture was concentrated under reduced pressure to obtain tert-butyl 4-(5-ethyl-7-oxo-2-(5-oxo-1,3a,4,5,6,6a-hexahydropentalen-2-yl)-4-(2-oxo-2-((4-(pentafluoro-λ⁶-sulfaneyl)phenyl)amino)ethyl)-4,7-dihydro-[1,2,4]triazolo[1,5-a]pyrimidin-6-yl)piperazine-1-car boxylate 71a (70 mg) with a yield of 82.54%.

MS m/z (ESI): 728.2 [M+1]

### Step 2

### 2-(6-(diethylamino)-5-ethyl-7-oxo-2-(5-oxo-1,3a,4,5,6,6a-hexahydropentalen-2-yl)-[1,2,4]triazol o[1,5-a]pyrimidin-4(7H)-yl)-N-(4-(pentafluoro-λ⁶-sulfaneyl)phenyl)acetamide

At room temperature, trifluoroacetic acid (1 mL) was added to a solution of tert-butyl 4-(5-ethyl-7-oxo-2-(5-oxo-1,3a,4,5,6,6a-hexahydropentalen-2-yl)-4-(2-oxo-2-((4-(pentafluoro-λ⁶-sulfaneyl)phenyl)amino)ethyl)-4,7-dihydro-[1,2,4]triazolo[1,5-a]pyrimidin-6-yl)piperazine-1-car boxylate 71a (70 mg, 96.19 µmol) in dichloromethane (4 mL), and the reaction was performed at room temperature for 40 minutes. The mixture was concentrated under reduced pressure to obtain 2-(6-(diethylamino)-5-ethyl-7-oxo-2-(5-oxo-1,3a,4,5,6,6a-hexahydropentalen-2-yl)-[1,2,4] triazolo[1,5-a]pyrimidin-4(7H)-yl)-N-(4-(pentafluoro-λ⁶-sulfaneyl)phenyl)acetamide 71b (60 mg) with a yield of 99.39%, which was used directly in the next step.

MS m/z (ESI): 628.2 [M+1]

### Step 3

### 2-(5-ethyl-6-(4-(3-hydroxypicolinoyl)piperazin-1-yl)-7-oxo-2-(5-oxo-1,3a,4,5,6,6a-hexahydropen talen-2-yl)-[1,2,4]triazolo[1,5-a]pyrimidin-4(7H)-yl)-N-(4-(pentafluoro-λ⁶-sulfaneyl)phenyl)aceta mide

At room temperature, 3-hydroxypicolinic acid 22a (19.95 mg, 143.40 µmol), 1-(3-dimethylaminopropyl)-3-ethylcarbodiimide hydrochloride (36.65 mg, 191.20 µmol), 1-hydroxybenzotriazole (25.83 mg, 191.20 µmol), and N,N-diisopropylethylamine (61.78 mg, 477.99 µmol) were dissolved in N,N-dimethylformamide (2 mL), and the reaction was performed at room temperature for 40 minutes. A solution of 2-(6-(diethylamino)-5-ethyl-7-oxo-2-(5-oxo-1,3a,4,5,6,6a-hexahydropentalen-2-yl)-[1,2,4]triazol o[1,5-a]pyrimidin-4(7H)-yl)-N-(4-(pentafluoro-λ⁶-sulfaneyl)phenyl)acetamide 71b (60 mg, 95.60 µmol) in N,N-dimethylformamide (2 mL) was added, and the reaction was performed at room temperature for 16 hours. Water (20 mL) was added to the reaction mixture, which was extracted with ethyl acetate (40 mL ×3). The combined organic phases were washed with saturated sodium chloride solution (20 mL), dried over anhydrous sodium sulfate, filtered, and concentrated under reduced pressure. The residue was purified by preparative liquid chromatography (column: AKZONOBEL Kromasil; 250 × 21.2 mm I.D.; 5 µm, 20 mL/min; mobile phase A: 0.05% TFA + H₂O, mobile phase B: CH₃CN) to obtain 2-(5-ethyl-6-(4-(3-hydroxypicolinoyl)piperazin-1-yl)-7-oxo-2-(5-oxo-1,3a,4,5,6,6a-hexahydropen talen-2-yl)-[1,2,4]triazolo[1,5-a]pyrimidin-4(7H)-yl)-N-(4-(pentafluoro-λ⁶-sulfaneyl)phenyl)aceta mide 71 (27.43 mg) with a yield of 37.79%.

MS m/z (ESI): 749.2 [M+1]
¹H NMR (400 MHz, DMSO-d6) δ 10.96 (s, 1H), 10.48 (s, 1H), 8.08 (dd, J = 3.7, 2.2 Hz, 1H), 7.98 - 7.83 (m, 2H), 7.77 (d, J = 8.9 Hz, 2H), 7.37 - 7.27 (m, 2H), 6.48 (d, J = 2.1 Hz, 1H), 5.20 (s, 2H), 4.55 (d, J = 12.8 Hz, 1H), 3.62 - 3.37 (m, 4H), 3.23 (t, J = 12.3 Hz, 1H), 3.08 (dd, J = 16.2, 8.1 Hz, 2H), 3.01 - 2.86 (m, 3H), 2.80 (d, J = 11.2 Hz, 1H), 2.63 (d, J = 15.7 Hz, 2H), 2.56 (d, J = 9.9 Hz, 1H), 2.47 (s, 1H), 2.29 - 2.21 (m, 1H), 1.94 (dd, J = 18.8, 6.9 Hz, 1H), 1.15 (q, J = 7.5 Hz, 3H).

### Example 72

### N-(2-chloro-4-(trifluoromethyl)phenyl)-2-(5-ethyl-6-(4-(3-hydroxypicolinoyl)piperazin-1-yl)-2-( 1H-inden-2-yl)-7-oxo-[1,2,4]triazolo[1,5-a]pyrimidin-4(7H)-yl)acetamide

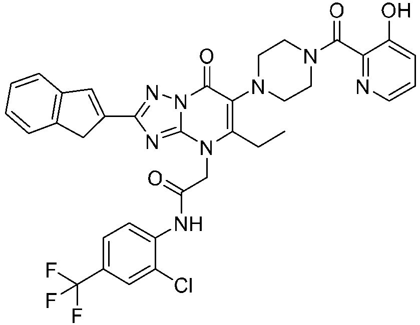

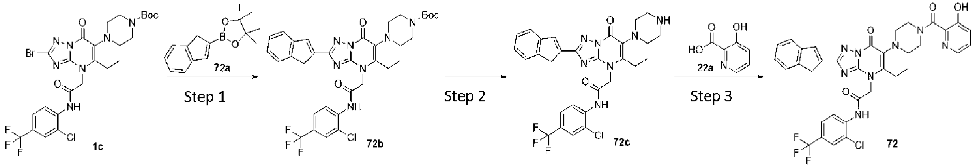

### Step 1

### tert-butyl 4-(4-(2-((2-chloro-4-(trifluoromethyl)phenyl)amino)-2-oxoethyl)-5-ethyl-2-(1H-inden-2-yl)-7-ox o-4,7-dihydro-[1,2,4]triazolo[1,5-a]pyrimidin-6-yl)piperazine-1-carboxylate

At room temperature, tert-butyl 4-(2-bromo-4-(2-((2-chloro-4-(trifluoromethyl)phenyl)amino)-2-oxoethyl)-5-ethyl-7-oxo-4,7-dih ydro-[1,2,4]triazolo[1,5-a]pyrimidin-6-yl)piperazine-1-carboxylate 1c (150 mg, 226.28 µmol), 2-(1H-inden-2-yl)-4,4,5,5-tetramethyl-1,3,2-dioxaborolane 72a (71.22 mg, 294.17 µmol), [1,1'-bis(diphenylphosphino)ferrocene]palladium(II) dichloride (18.34 mg, 22.63 µmol), and sodium carbonate (71.95 mg, 678.85 µmol) were dissolved in a mixed solution of 1,4-dioxane (5 mL) and water (0.5 mL). The mixture was subject to argon replacement three times, was heated to 85 °C, and was reacted for 2 h. Water (20 mL) was added to the reaction mixture, and the mixture was extracted with ethyl acetate (40 mL × 3). The combined organic phase was washed with saturated sodium chloride solution (20 mL), dried over anhydrous sodium sulfate, filtered, and concentrated under reduced pressure. The obtained residue was purified by silica gel column chromatography (eluent: System B) to obtain tert-butyl 4-(4-(2-((2-chloro-4-(trifluoromethyl)phenyl)amino)-2-oxoethyl)-5-ethyl-2-(1H-inden-2-yl)-7-ox o-4,7-dihydro-[1,2,4]triazolo[1,5-a]pyrimidin-6-yl)piperazine-1-carboxylate72b (150 mg) with a yield of 94.95%.

MS m/z (ESI): 642.2 [M-56+H]

### Step 2

### N-(2-Chloro-4-(trifluoromethyl)phenyl)-2-(5-ethyl-2-(1H-inden-2-yl)-7-oxo-6-(piperazin-1-yl)-[ 1,2,4]triazolo[1,5-a]pyrimidin-4(7H)-yl)acetamide

At room temperature, trifluoroacetic acid (1.5 mL) was added to a solution of tert-butyl 4-(4-(2-((2-chloro-4-(trifluoromethyl)phenyl)amino)-2-oxoethyl)-5-ethyl-2-(1H-inden-2-yl)-7-ox o-4,7-dihydro-[1,2,4]triazolo[1,5-a]pyrimidin-6-yl)piperazine-1-carboxylate72b (150 mg, 214.86 µmol) in dichloromethane (6 mL). The reaction mixture was reacted at room temperature for 40 min. The mixture was concentrated under reduced pressure to obtain N-(2-chloro-4-(trifluoromethyl)phenyl)-2-(5-ethyl-2-(1H-inden-2-yl)-7-oxo-6-(piperazin-1-yl)-[1 ,2,4]triazolo [1,5-a]pyrimidin-4(7H)-yl)acetamide 72c (128 mg) with a yield of 99.62%, which was used directly in the next step.

MS m/z (ESI): 598.2 [M+1]

### Step 3

### N-(2-Chloro-4-(trifluoromethyl)phenyl)-2-(5-ethyl-6-(4-(3-hydroxypicolinoyl)piperazin-1-yl)-2-( 1H-inden-2-yl)-7-oxo-[1,2,4]triazolo[1,5-a]pyrimidin-4(7H)-yl)acetamide

At room temperature, 3-hydroxypicolinic acid 22a (44.66 mg, 321.06 µmol), 1-(3-dimethylaminopropyl)-3-ethylcarbodiimide hydrochloride (82.06 mg, 428.08 µmol), 1-hydroxybenzotriazole (57.84 mg, 428.08 µmol), and N,N-diisopropylethylamine (138.31 mg, 1.07 mmol) were added to N,N-dimethylformamide (2 mL). The mixture was reacted at room temperature for 40 min, then N-(2-chloro-4-(trifluoromethyl)phenyl)-2-(5-ethyl-2-(1H-inden-2-yl)-7-oxo-6-(piperazin-1-yl)-[1 ,2,4]triazolo[1,5-a]pyrimidin-4(7H)-yl)acetamide 72c (128 mg, 214.04 µmol) was added, and the reaction mixture was reacted at room temperature for 18 h. Water (20 mL) was added to the reaction mixture, and the mixture was extracted with ethyl acetate (40 mL × 3). The combined organic phase was washed with saturated sodium chloride solution (20 mL), dried over anhydrous sodium sulfate, filtered, and concentrated under reduced pressure. The residue was separated and purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(3-hydroxypicolinoyl)piperazin-1-yl)-2-( 1H-inden-2-yl)-7-oxo-[1,2,4]triazolo[1,5-a]pyrimidin-4(7H)-yl)acetamide 72 (34.56 mg) with a yield of 21.20%.

MS m/z (ESI): 719.2 [M+1]
¹H NMR (400 MHz, DMSO-d6) δ 10.42 (s, 2H), 8.18 - 8.02 (m, 2H), 7.98 (d, J = 2.1 Hz, 1H), 7.72 (dd, J = 8.8, 2.1 Hz, 1H), 7.65 (d, J = 1.8 Hz, 1H), 7.55 (dd, J = 8.4, 6.9 Hz, 2H), 7.38 - 7.21 (m, 4H), 5.37 (s, 2H), 4.56 (d, J = 12.7 Hz, 1H), 3.90 (s, 2H), 3.42 (d, J = 12.7 Hz, 2H), 3.24 (t, J = 12.1 Hz, 1H), 3.14 - 2.91 (m, 3H), 2.82 (d, J = 11.4 Hz, 1H), 2.64 (d, J = 11.7 Hz, 1H), 2.08 (s, 1H), 1.21 (t, J = 7.4 Hz, 3H).

### Example 73

### N-(2-Chloro-4-(trifluoromethyl)phenyl)-2-(5-ethyl-6-(4-(5-hydroxy-6-methylpyrimidine-4-carbo nyl)piperazin-1-yl)-2-(1H-indol-2-yl)-7-oxo-[1,2,4]triazolo[1,5-a]pyrimidin-4(7H)-yl)acetamide

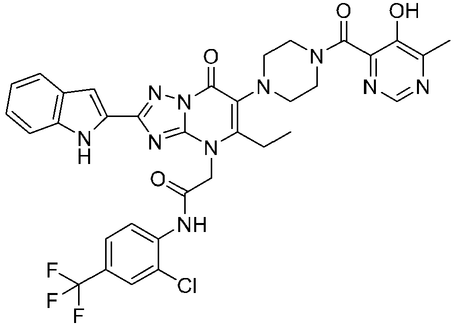

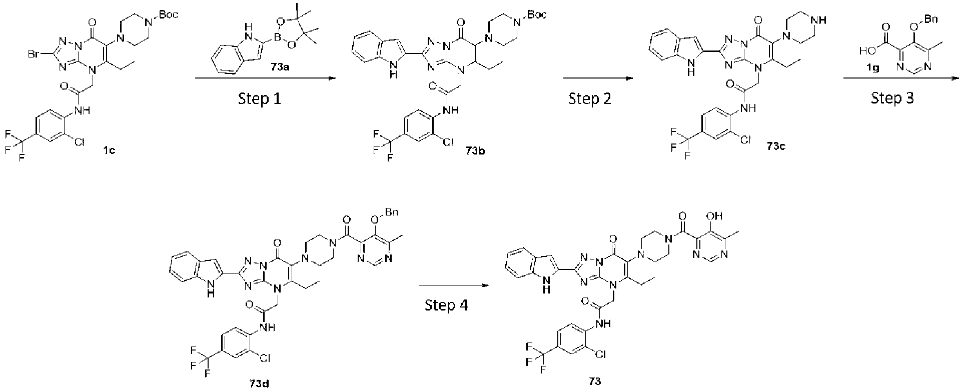

### Step 1

### tert-butyl 4-(4-(2-((2-chloro-4-(trifluoromethyl)phenyl)amino)-2-oxoethyl)-5-ethyl-2-(1H-indol-2-yl)-7-ox o-4,7-dihydro-[1,2,4]triazolo[1,5-a]pyrimidin-6-yl)piperazine-1-carboxylate

At room temperature, tert-butyl 4-(2-bromo-4-(2-((2-chloro-4-(trifluoromethyl)phenyl)amino)-2-oxoethyl)-5-ethyl-7-oxo-4,7-dih ydro-[1,2,4]triazolo[1,5-a]pyrimidin-6-yl)piperazine-1-carboxylate 1c (150 mg, 226.28 µmol), 2-(4,4,5,5-tetramethyl-1,3,2-dioxaborolan-2-yl)-1H-indole 73a (66.01 mg, 271.54 µmol), [1,1'-bis(diphenylphosphino)ferrocene]palladium(II) dichloride (18.34 mg, 22.63 µmol), and sodium carbonate (71.95 mg, 678.85 µmol) were dissolved in a mixed solution of 1,4-dioxane (3 mL) and water (0.3 mL). The mixture was subject to argon replacement 3 times, then heated to 90 C and the reaction was performed for 1.5 h. Water (20 mL) was added to the reaction mixture, and the mixture was extracted with ethyl acetate (40 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(4-(2-((2-chloro-4-(trifluoromethyl)phenyl)amino)-2-oxoethyl)-5-ethyl-2-(1H-indol-2-yl)-7-ox o-4,7-dihydro-[1,2,4]triazolo[1,5-a]pyrimidin-6-yl)piperazine-1-carboxylate73b (158 mg) with a yield of 99.87%.

MS m/z (ESI): 643.2 [M-56+H]

### Step 2

### N-(2-Chloro-4-(trifluoromethyl)phenyl)-2-(5-ethyl-2-(1H-indol-2-yl)-7-oxo-6-(piperazin-1-yl)-[1 ,2,4]triazolo[1,5-a]pyrimidin-4(7H)-yl)acetamide

At room temperature, trifluoroacetic acid (1.5 mL) was added to a solution of tert-butyl 4-(4-(2-((2-chloro-4-(trifluoromethyl)phenyl)amino)-2-oxoethyl)-5-ethyl-2-(1H-indol-2-yl)-7-ox o-4,7-dihydro-[1,2,4]triazolo[1,5-a]pyrimidin-6-yl)piperazine-1-carboxylate73b (158 mg, 226.00 µmol) in dichloromethane (6 mL), and the reaction was performed at room temperature for 40 min. The reaction mixture was concentrated under reduced pressure to obtain N-(2-chloro-4-(trifluoromethyl)phenyl)-2-(5-ethyl-2-(1H-indol-2-yl)-7-oxo-6-(piperazin-1-yl)-[1, 2,4]triazolo[1,5-a]pyrimidin-4(7H)-yl)acetamide 73c (135 mg) with a yield of 99.72%.

MS m/z (ESI): 599.2 [M+1]

### Step 3

### 2-(6-(4-(5-(Benzyloxy)-6-methylpyrimidine-4-carbonyl)piperazin-1-yl)-5-ethyl-2-(1H-indol-2-yl )-7-oxo-[1,2,4]triazolo[1,5-a]pyrimidin-4(7H)-yl)-N-(2-chloro-4-(trifluoromethyl)phenyl)acetami de

At room temperature, 5-(benzyloxy)-6-methylpyrimidine-4-carboxylic acid 1g (55.05 mg, 225.37 µmol), 1-(3-dimethylaminopropyl)-3-ethylcarbodiimide hydrochloride (57.61 mg, 300.50 µmol), 1-hydroxybenzotriazole (40.60 mg, 300.50 µmol), and N,N-diisopropylethylamine (97.09 mg, 751.25 µmol) were added to N,N-dimethylformamide (3 mL), and the reaction was performed at room temperature for 40 min. Then a solution of N-(2-chloro-4-(trifluoromethyl)phenyl)-2-(5-ethyl-2-(1H-indol-2-yl)-7-oxo-6-(piperazin-1-yl)-[1, 2,4]triazolo[1,5-a]pyrimidin-4(7H)-yl)acetamide 73c (90 mg, 150.25 µmol) in N,N-dimethylformamide (2 mL) was added, and the reaction was performed at room temperature for 18 h. Water (20 mL) was added to the reaction mixture, and the mixture was extracted with ethyl acetate (40 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-5-ethyl-2-(1H-indol-2-yl) -7-oxo-[1,2,4]triazolo[1,5-a]pyrimidin-4(7H)-yl)-N-(2-chloro-4-(trifluoromethyl)phenyl)acetami de 73d (80 mg) with a yield of 64.52%.

MS m/z (ESI): 825.2 [M+1]

### Step 4

### N-(2-Chloro-4-(trifluoromethyl)phenyl)-2-(5-ethyl-6-(4-(5-hydroxy-6-methylpyrimidine-4-carbo nyl)piperazin-1-yl)-2-(1H-indol-2-yl)-7-oxo-[1,2,4]triazolo[1,5-a]pyrimidin-4(7H)-yl)acetamide

Under ice bath, boron trichloride (1.5 mL) was added to a solution of 2-(6-(4-(5-(benzyloxy)-6-methylpyrimidine-4-carbonyl)piperazin-1-yl)-5-ethyl-2-(1H-indol-2-yl) -7-oxo-[1,2,4]triazolo[1,5-a]pyrimidin-4(7H)-yl)-N-(2-chloro-4-(trifluoromethyl)phenyl)acetami de 73d (80 mg, 96.94 µmol) in dichloromethane (1.5 mL), and the reaction was performed at room temperature for 18 h. The reaction was quenched with methanol, and the concentrated residue was separated and purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-2-(1H-indol-2-yl)-7-oxo-[1,2,4]triazolo[1,5-a]pyrimidin-4(7H)-yl)acetamide 73 (1.36 mg) with a yield of 1.85%.

MS m/z (ESI): 735.2 [M+1]
¹H NMR (400 MHz, DMSO-d6) δ 12.03 (d, J = 2.2 Hz, 1H), 10.44 (s, 1H), 10.26 (s, 1H), 8.59 (s, 1H), 8.09 (d, J = 8.6 Hz, 1H), 7.99 (d, J = 2.1 Hz, 1H), 7.73 (dd, J = 8.8, 2.0 Hz, 1H), 7.61 (d, J = 7.9 Hz, 1H), 7.45 (d, J = 8.2 Hz, 1H), 7.18 (t, J = 7.6 Hz, 1H), 7.10 (d, J = 2.0 Hz, 1H), 7.04 (t, J = 7.4 Hz, 1H), 5.41 (s, 2H), 4.55 (d, J = 12.6 Hz, 1H), 3.55 (q, J = 11.6, 10.8 Hz, 3H), 3.26 (d, J = 13.0 Hz, 1H), 3.01 (d, J = 13.4 Hz, 3H), 2.87 (d, J = 11.4 Hz, 1H), 2.69 (d, J = 11.0 Hz, 1H), 2.46 (s, 3H), 1.23 (t, J = 7.4 Hz, 3H).

### Example 74

### N-(2-Chloro-4-(trifluoromethyl)phenyl)-2-(5-ethyl-6-(4-(5-hydroxy-6-methylpyrimidine-4-carbo nyl)piperazin-1-yl)-2-(1-methyl-1H-indol-2-yl)-7-oxo-[1,2,4]triazolo[1,5-a]pyrimidin-4(7H)-yl)a

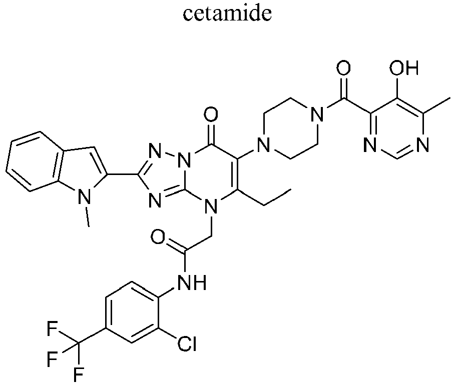

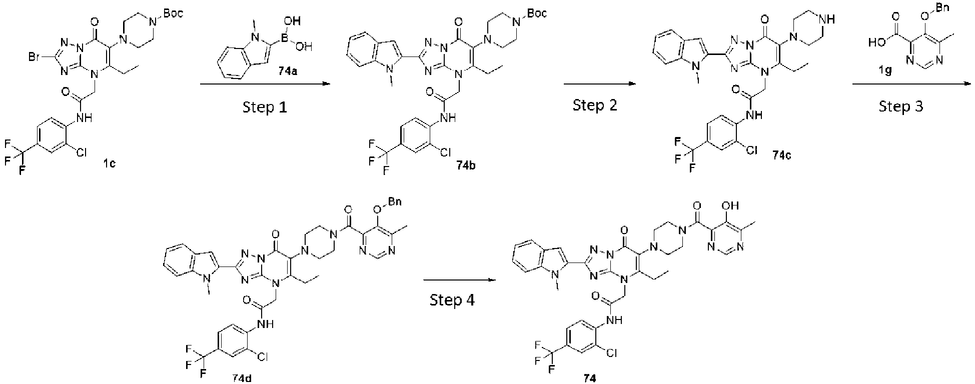

### Step 1

### tert-butyl 4-(4-(2-((2-chloro-4-(trifluoromethyl)phenyl)amino)-2-oxoethyl)-5-ethyl-2-(1-methyl-1H-indol-2 -yl)-7-oxo-4,7-dihydro-[1,2,4]triazolo[1,5-a]pyrimidin-6-yl)piperazine-1-carboxylate

At room temperature, tert-butyl 4-(2-bromo-4-(2-((2-chloro-4-(trifluoromethyl)phenyl)amino)-2-oxoethyl)-5-ethyl-7-oxo-4,7-dih ydro-[1,2,4]triazolo[1,5-a]pyrimidin-6-yl)piperazine-1-carboxylate 1c (160 mg, 241.37 µmol), (1-methyl-1H-indol-2-yl)boronic acid 74a (50.69 mg, 289.64 µmol, commercially available), [1,1'-bis(diphenylphosphino)ferrocene]palladium dichloride (19.56 mg, 24.14 µmol), and sodium carbonate (76.75 mg, 724.11 µmol) were dissolved in a mixed solution of 1,4-dioxane (5 mL) and water (0.5 mL). The mixture was subject to argon replacement three times, then heated to 85 °C and reacted for 4 h. Water (20 mL) was added to the reaction mixture, and the mixture was extracted with ethyl acetate (40 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(4-(2-((2-chloro-4-(trifluoromethyl)phenyl)amino)-2-oxoethyl)-5-ethyl-2-(1-methyl-1H-indol-2 -yl)-7-oxo-4,7-dihydro-[1,2,4]triazolo[1,5-a]pyrimidin-6-yl)piperazine-1-carboxylate 74b (170 mg) with a yield of 98.76%.

MS m/z (ESI): 658.2 [M-56+H]

### Step 2

### N-(2-chloro-4-(trifluoromethyl)phenyl)-2-(5-ethyl-2-(1-methyl-1H-indol-2-yl)-7-oxo-6-(piperazi n-1-yl)-[1,2,4]triazolo[1,5-a]pyrimidin-4(7H)-yl)acetamide

At room temperature, trifluoroacetic acid (1.5 mL) was added to a solution of tert-butyl 4-(4-(2-((2-chloro-4-(trifluoromethyl)phenyl)amino)-2-oxoethyl)-5-ethyl-2-(1-methyl-1H-indol-2 -yl)-7-oxo-4,7-dihydro-[1,2,4]triazolo[11,5-a]pyrimidin-6-yl)piperazine-1-carboxylate 74b (170 mg, 238.38 µmol) in dichloromethane (6 mL). The reaction mixture was stirred at room temperature for 40 min. The mixture was concentrated under reduced pressure, and the resulting residue was purified by silica gel column chromatography (eluent: System B) to obtain N-(2-chloro-4-(trifluoromethyl)phenyl)-2-(5-ethyl-2-(1-methyl-1H-indol-2-yl)-7-oxo-6-(piperazi n-1-yl)-[1,2,4]triazolo[1,5-a]pyrimidin-4(7H)-yl)acetamide 74c (140 mg) with a yield of 95.80%.

MS m/z (ESI): 613.2 [M+1]

### Step 3

### 2-(6-(4-(5-(benzyloxy)-6-methylpyrimidine-4-carbonyl)piperazin-1-yl)-5-ethyl-2-(1-methyl-1H-i ndol-2-yl)-7-oxo-[1,2,4]triazolo[1,5-a]pyrimidin-4(7H)-yl)-N-(2-chloro-4-(trifluoromethyl)pheny )acetamide

At room temperature, 5-(benzyloxy)-6-methylpyrimidine-4-carboxylic acid 1g (47.81 mg, 195.75 µmol), 1-(3-dimethylaminopropyl)-3-ethylcarbodiimide hydrochloride (50.03 mg, 261.00 µmol), 1-hydroxybenzotriazole (35.27 mg, 261.00 µmol), and N,N-diisopropylethylamine (84.33 mg, 652.49 µmol) were added to N,N-dimethylformamide (3 mL). The mixture was stirred at room temperature for 40 min, then a solution of N-(2-chloro-4-(trifluoromethyl)phenyl)-2-(5-ethyl-2-(1-methyl-1H-indol-2-yl)-7-oxo-6-(piperazi n-1-yl)-[1,2,4]triazolo[1,5-a]pyrimidin-4(7H)-yl)acetamide 74c (80 mg, 130.50 µmol) in N,N-dimethylformamide (2 mL) was added. The reaction was performed at room temperature for 18 h. Water (20 mL) was added to the reaction mixture, and the mixture was extracted with ethyl acetate (40 mL × 3). The combined organic phases were washed with saturated sodium chloride solution (20 mL), dried over anhydrous sodium sulfate, filtered, and concentrated under reduced pressure. The residue was purified by preparative liquid chromatography (column: AKZONOBEL Kromasil; 250×21.2 mm I.D.; 5 µm, 20 mL/min; mobile phase A: 0.05% TFA + H₂O, mobile phase B: CH₃CN) to obtain 2-(6-(4-(5-(benzyloxy)-6-methylpyrimidine-4-carbonyl)piperazin-1-yl)-5-ethyl-2-(1-methyl-1H-i ndol-2-yl)-7-oxo-[1,2,4]triazolo[1,5-a]pyrimidin-4(7H)-yl)-N-(2-chloro-4-(trifluoromethyl)pheny )acetamide 74d (80 mg) with a yield of 73.04%.

MS m/z (ESI): 839.3 [M+1]

### Step 4

### N-(2-chloro-4-(trifluoromethyl)phenyl)-2-(5-ethyl-6-(4-(5-hydroxy-6-methylpyrimidine-4-carbon yl)piperazin-1-yl)-2-(1-methyl-1H-indol-2-yl)-7-oxo-[1,2,4]triazolo[1,5-a]pyrimidin-4(7H)-yl)ac etamide

Under an ice bath and argon atmosphere, boron trichloride (1 mL) was added to a solution of 2-(6-(4-(5-(benzyloxy)-6-methylpyrimidine-4-carbonyl)piperazin-1-yl)-5-ethyl-2-(1-methyl-1H-i ndol-2-yl)-7-oxo-[1,2,4]triazolo[1,5-a]pyrimidin-4(7H)-yl)-N-(2-chloro-4-(trifluoromethyl) phenyl)acetamide 74d (80 mg, 95.32 µmol) in dichloromethane (1.5 mL). The reaction mixture was stirred at room temperature for 18 h. The reaction was quenched with methanol, and the concentrated residue was purified by preparative liquid chromatography (column: AKZONOBEL Kromasil; 250 ×21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-2-(1-methyl-1H-indol-2-yl)-7-oxo-[1,2,4]triazolo[1,5-a]pyrimidin-4(7H)-yl)ac etamide 74 (7.03 mg) with a yield of 9.35%.

MS m/z (ESI): 749.2 [M+1]
¹H NMR (400 MHz, DMSO-d6) δ 10.45 (s, 1H), 10.25 (s, 1H), 8.59 (s, 1H), 8.06 (d, J = 8.6 Hz, 1H), 7.98 (d, J = 2.1 Hz, 1H), 7.72 (dd, J = 8.8, 2.1 Hz, 1H), 7.64 (d, J = 7.9 Hz, 1H), 7.56 (d, J = 8.4 Hz, 1H), 7.27 (t, J = 7.7 Hz, 1H), 7.20 (s, 1H), 7.10 (t, J = 7.5 Hz, 1H), 5.41 (s, 2H), 4.55 (d, J = 12.6 Hz, 1H), 4.17 (s, 3H), 3.66 - 3.46 (m, 3H), 3.27 (d, J = 13.4 Hz, 1H), 3.03 (dt, J = 16.1, 6.3 Hz, 3H), 2.86 (d, J = 11.4 Hz, 1H), 2.68 (d, J = 10.8 Hz, 1H), 2.46 (s, 3H), 1.23 (t, J = 7.4 Hz, 3H).

### Example 75

### 2-(2-(benzofuran-2-yl)-5-ethyl-6-(4-(3-hydroxypicolinoyl)piperazin-1-yl)-7-oxo-[1,2,4]triazolo[1 ,5-a]pyrimidin-4(7H)-yl)-N-(2-chloro-4-(trifluoromethyl)phenyl)acetamide

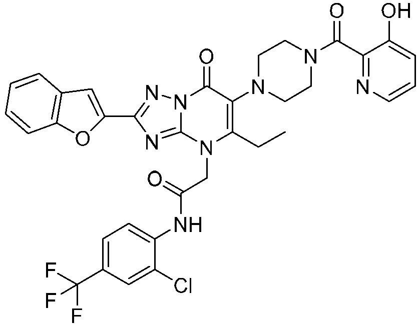

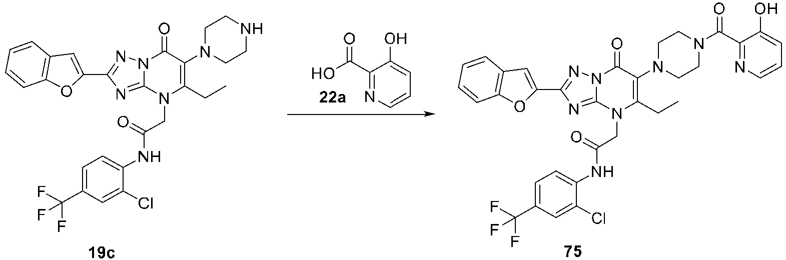

At room temperature, 3-hydroxypicolinic acid 22a (15.65 mg, 112.50 µmol), 1-(3-dimethylaminopropyl)-3-ethylcarbodiimide hydrochloride (28.76 mg, 150.00 µmol), 1-hydroxybenzotriazole (20.27 mg, 150.00 µmol), and N,N-diisopropylethylamine (48.47 mg, 375.01 µmol) were added to N,N-dimethylformamide (2 mL), and the reaction was performed at room temperature for 40 minutes. Then a solution of 2-(2-(benzofuran-2-yl)-5-ethyl-7-oxo-6-(piperazin-1-yl)-[1,2,4]triazolo[1,5-a]pyrimidin-4(7H)-yl )-N-(2-chloro-4-(trifluoromethyl)phenyl) acetamide 19c (45 mg, 75.00 µmol) in N,N-dimethylformamide (1.5 mL) was added, and the reaction was performed at room temperature for 18 hours. Water (20 mL) was added to the reaction mixture, and the mixture was extracted with ethyl acetate (40 mL ×3). The combined organic phases were washed with saturated sodium chloride solution (20 mL), dried over anhydrous sodium sulfate, filtered, and concentrated under reduced pressure. The residue was purified by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain 2-(2-(benzofuran-2-yl)-5-ethyl-6-(4-(3-hydroxypicolinoyl)piperazin-1-yl)-7-oxo-[1,2,4]triazolo[1 ,5-a]pyrimidin-4(7H)-yl)-N-(2-chloro-4-(trifluoromethyl)phenyl)acetamide 75 (6.67 mg) with a yield of 11.57%.

MS m/z (ESI): 721.2 [M+1]
¹H NMR (400 MHz, DMSO-d6) δ 10.45 (s, 1H), 10.41 (s, 1H), 8.21 - 8.05 (m, 2H), 7.98 (d, J = 2.1 Hz, 1H), 7.77 (d, J = 7.7 Hz, 1H), 7.72 (td, J = 5.4, 2.5 Hz, 2H), 7.62 (s, 1H), 7.50 - 7.42 (m, 1H), 7.39 - 7.29 (m, 3H), 5.42 (s, 2H), 4.57 (d, J = 12.5 Hz, 1H), 3.54 - 3.41 (m, 3H), 3.25 (t, J = 12.0 Hz, 1H), 3.16 - 2.91 (m, 3H), 2.84 (d, J = 11.3 Hz, 1H), 2.67 (d, J = 10.8 Hz, 1H), 1.22 (td, J = 8.1, 7.6, 2.7 Hz, 3H).

### Example 76

### N-(2-chloro-4-(trifluoromethyl)phenyl)-2-(2-(2,3-dihydrobenzofuran-5-yl)-5-ethyl-6-(4-(5-hydro xy-6-methylpyrimidine-4-carbonyl)piperazin-1-yl)-7-oxo-[1,2,4]triazolo[1,5-a]pyrimidin-4(7H)-yl)acetamide

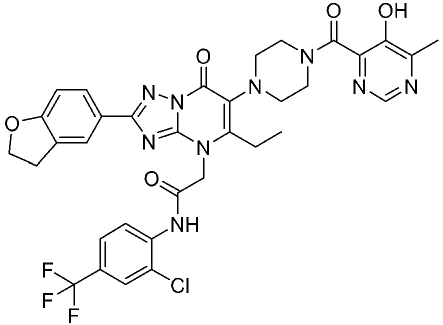

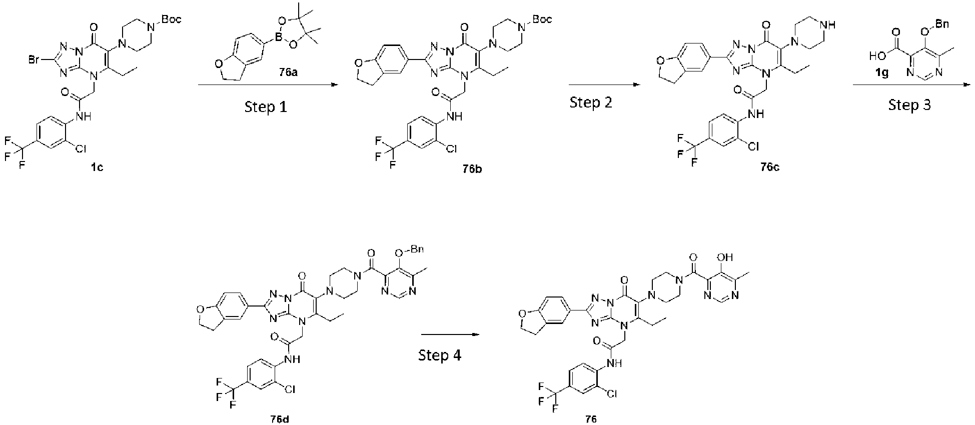

### Step 1

### tert-butyl 4-(4-(2-((2-chloro-4-(trifluoromethyl)phenyl)amino)-2-oxoethyl)-2-(2,3-dihydrobenzofuran-5-yl) -5-ethyl-7-oxo-4,7-dihydro-[1,2,4]triazolo[1,5-a]pyrimidin-6-yl)piperazine-1-carboxylate

At room temperature, tert-butyl 4-(2-bromo-4-(2-((2-chloro-4-(trifluoromethyl) phenyl)amino)-2-oxoethyl)-5-ethyl-7-oxo-4,7-dihydro-[1,2,4]triazolo[1,5-a]pyrimidin-6-yl)piper azine-1-carboxylate 1c (150 mg, 226.28 µmol), 2-(2,3-dihydrobenzofuran-5-yl)-4,4,5,5-tetramethyl-1,3,2-dioxaborolane 76a (48.23 mg, 294.17 µmol), [1,1'-bis(diphenylphosphino)ferrocene]palladium dichloride (18.34 mg, 22.63 µmol), and sodium carbonate (71.95 mg, 678.85 µmol) were dissolved in a mixed solution of 1,4-dioxane (10 mL) and water (1 mL). The mixture was subject to argon replacement three times, heated to 85 °C, and the reaction was performed for 2 hours. Water (20 mL) was added to the reaction mixture, and the mixture was extracted with ethyl acetate (40 mL×3). The combined organic phases were washed with saturated sodium chloride solution (20 mL), dried over anhydrous sodium sulfate, filtered, and concentrated under reduced pressure. The obtained residue was purified by silica gel column chromatography (eluent: System B) to obtain tert-butyl 4-(4-(2-((2-chloro-4-(trifluoromethyl)phenyl)amino)-2-oxoethyl)-2-(2,3-dihydrobenzofuran-5-yl) -5-ethyl-7-oxo-4,7-dihydro-[1,2,4]triazolo[1,5-a]pyrimidin-6-yl)piperazine-1-carboxylate 76b (150 mg) with a yield of 94.41%.

MS m/z (ESI): 646.3 [M-56+H]

### Step 2

### N-(2-chloro-4-(trifluoromethyl)phenyl)-2-(2-(2,3-dihydrobenzofuran-5-yl)-5-ethyl-7-oxo-6-(pipe razin-1-yl)-[1,2,4]triazolo[1,5-a]pyrimidin-4(7H)-yl)acetamide

At room temperature, trifluoroacetic acid (1.5 mL) was added to a solution of tert-butyl 4-(4-(2-((2-chloro-4-(trifluoromethyl)phenyl)amino)-2-oxoethyl)-2-(2,3-dihydrobenzofuran-5-yl) -5-ethyl-7-oxo-4,7-dihydro-[1,2,4]triazolo[1,5-a]pyrimidin-6-yl)piperazine-1-carboxylate 76b (150 mg, 213.64 µmol) in dichloromethane (6 mL), and the reaction was performed at room temperature for 40 minutes. The reaction mixture was concentrated under reduced pressure to obtain N-(2-chloro-4-(trifluoromethyl)phenyl)-2-(2-(2,3-dihydrobenzofuran-5-yl)-5-ethyl-7-oxo-6-(pipe razin-1-yl)-[1,2,4]triazolo[1,5-a]pyrimidin-4(7H)-yl)acetamide 76c (128 mg) with a yield of 99.52%.

MS m/z (ESI): 602.2 [M+1]

### Step 3

### 2-(6-(4-(5-(benzyloxy)-6-methylpyrimidine-4-carbonyl)piperazin-1-yl)-2-(2,3-dihydrobenzofuran -5-yl)-5-ethyl-7-oxo-[1,2,4]triazolo[1,5-a]pyrimidin-4(7H)-yl)-N-(2-chloro-4-(trifluoromethyl)ph enyl)acetamide

At room temperature, 5-(benzyloxy)-6-methylpyrimidine-4-carboxylic acid **1g** (77.90 mg, 318.93 µmol), 1-(3-dimethylaminopropyl)-3-ethylcarbodiimide hydrochloride (81.52 mg, 425.24 µmol), 1-hydroxybenzotriazole (57.46 mg, 425.24 µmol), and N,N-diisopropylethylamine (137.40 mg, 1.06 mmol) were dissolved in N,N-dimethylformamide (3 mL), and the reaction was performed at room temperature for 40 minutes. Then a solution of N-(2-chloro-4-(trifluoromethyl)phenyl)-2-(2-(2,3-dihydrobenzofuran-5-yl)-5-ethyl-7-oxo-6-(pipe razin-1-yl)-[1,2,4]triazolo[1,5-a]pyrimidin-4(7H)-yl)acetamide 76c (128 mg, 212.62 µmol) in N,N-dimethylformamide (3 mL) was added, and the reaction was performed at room temperature for 18 hours. Water (20 mL) was added to the reaction mixture, and the mixture was extracted with ethyl acetate (40 mL×3). The combined organic phases were washed with saturated sodium chloride solution (20 mL), dried over anhydrous sodium sulfate, filtered, and concentrated under reduced pressure. The obtained residue was purified by silica gel column chromatography (eluent: System B) to obtain 2-(6-(4-(5-(benzyloxy)-6-methylpyrimidine-4-carbonyl)piperazin-1-yl)-2-(2,3-dihydrobenzofuran -5-yl)-5-ethyl-7-oxo-[1,2,4]triazolo[1,5-a]pyrimidin-4(7H)-yl)-N-(2-chloro-4-(trifluoromethyl)ph enyl)acetamide 76d (150 mg) with a yield of 85.18%.

MS m/z (ESI): 828.3 [M+1]

### Step 4

### N-(2-chloro-4-(trifluoromethyl)phenyl)-2-(2-(2,3-dihydrobenzofuran-5-yl)-5-ethyl-6-(4-(5-hydro xy-6-methylpyrimidine-4-carbonyl)piperazin-1-yl)-7-oxo-[1,2,4]triazolo[1,5-a]pyrimidin-4(7H)-yl)acetamide

At room temperature, trifluoroacetic acid (4.5 mL) was added to a solution of 2-(6-(4-(5-(benzyloxy)-6-methylpyrimidine-4-carbonyl)piperazin-1-yl)-2-(2,3-dihydrobenzofuran -5-yl)-5-ethyl-7-oxo-[1,2,4]triazolo[1,5-a]pyrimidin-4(7H)-yl)-N-(2-chloro-4-(trifluoromethyl)ph enyl) acetamide 76d (150 mg, 181.11 µmol) in dichloromethane (0.5 mL), and the reaction was performed at room temperature for 48 h. The mixture was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(2-(2,3-dihydrobenzofuran-5-yl)-5-ethyl-6-(4-(5-hydro xy-6-methylpyrimidine-4-carbonyl)piperazin-1-yl)-7-oxo-[1,2,4]triazolo[1,5-a]pyrimidin-4(7H)-yl)acetamide 76 (24.73 mg) with a yield of 17.02%.

MS m/z (ESI): 738.3 [M+1]
¹H NMR (400 MHz, DMSO-d6) δ 10.43 (s, 1H), 10.26 (s, 1H), 8.59 (s, 1H), 8.07 (d, J = 8.6 Hz, 1H), 7.98 (d, J = 1.9 Hz, 2H), 7.88 (dd, J = 8.3, 1.8 Hz, 1H), 7.72 (dd, J = 8.8, 2.1 Hz, 1H), 6.88 (d, J = 8.3 Hz, 1H), 5.39 (s, 2H), 4.61 (t, J = 8.7 Hz, 2H), 4.54 (d, J = 12.4 Hz, 1H), 3.59 - 3.48 (m, 3H), 3.27 (q, J = 9.8, 8.7 Hz, 3H), 3.02 (q, J = 10.3, 8.8 Hz, 3H), 2.84 (d, J = 11.2 Hz, 1H), 2.67 (d, J = 10.8 Hz, 1H), 2.46 (s, 3H), 1.21 (t, J = 7.4 Hz, 3H).

### Example 77

### 2-(2-(benzofuran-6-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(4-(pentafluoro-λ⁶-sulfaneyl)phenyl)acetamid e

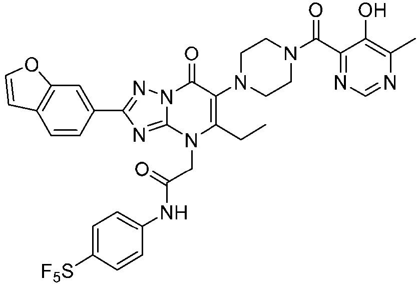

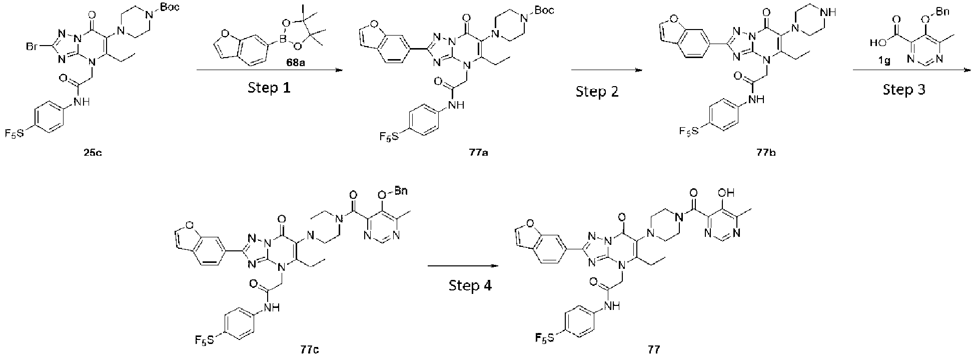

### Step 1

### tert-butyl 4-(2-(benzofuran-6-yl)-5-ethyl-7-oxo-4-(2-oxo-2-((4-(pentafluoro-λ⁶-sulfaneyl)phenyl)amino)eth yl)-4,7-dihydro-[1,2,4]triazolo[1,5-a]pyrimidin-6-yl)piperazine-1-carboxylate

At room temperature, tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)piperazine-1-carboxylate 25c (140 mg, 203.94 µmol), 2-(benzofuran-6-yl)-4,4,5,5-tetramethyl-1,3,2-dioxaborolane 68a (59.74 mg, 244.72 µmol), [1,1'-bis(diphenylphosphino)ferrocene]palladium(II) dichloride (16.53 mg, 20.39 µmol), and sodium carbonate (64.85 mg, 611.81 µmol) were dissolved in a mixed solution of 1,4-dioxane (12 mL) and water (2 mL). The mixture was subject to argon replacement 3 times, heated to 80 °C, and the reaction was performed for 2 h. Water (20 mL) was added to the reaction mixture, which was extracted with ethyl acetate (20 mL×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(2-(benzofuran-6-yl)-5-ethyl-7-oxo-4-(2-oxo-2-((4-(pentafluoro-λ⁶-sulfaneyl)phenyl)amino)eth yl)-4,7-dihydro-[1,2,4]triazolo[1,5-a]pyrimidin-6-yl)piperazine-1-carboxylate 77a (100 mg) with a yield of 67.76%.

MS m/z (ESI): 668.2 [M-56+H]

### Step 2

### 2-(2-(benzofuran-6-yl)-5-ethyl-7-oxo-6-(piperazin-1-yl)-[1,2,4]triazolo[1,5-a]pyrimidin-4(7H)-yl )-N-(4-(pentafluoro-λ⁶-sulfaneyl)phenyl)acetamide

At room temperature, trifluoroacetic acid (1.5 mL) was added to a solution of tert-butyl 4-(2-(benzofuran-6-yl)-5-ethyl-7-oxo-4-(2-oxo-2-((4-(pentafluoro-λ⁶-sulfaneyl)phenyl)amino)eth yl)-4,7-dihydro-[1,2,4]triazolo[1,5-a]pyrimidin-6-yl)piperazine-1-carboxylate 77a (100 mg, 138.18 µmol) in dichloromethane (6 mL), and the reaction was performed at room temperature for 40 min. The mixture was concentrated under reduced pressure to obtain 2-(2-(benzofuran-6-yl)-5-ethyl-7-oxo-6-(piperazin-1-yl)-[1,2,4]triazolo[1,5-a]pyrimidin-4(7H)-yl )-N-(4-(pentafluoro-λ⁶-sulfaneyl)phenyl)acetamide 77b (86 mg) with a yield of 99.81%, which was used directly in the next step.

MS m/z (ESI): 624.2 [M+1]

### Step 3

### 2-(2-(benzofuran-6-yl)-6-(4-(5-(benzyloxy)-6-methylpyrimidine-4-carbonyl)piperazin-1 -yl)-5-ethyl-7-oxo-[1,2,4]triazolo[1,5-a]pyrimidin-4(7H)-yl)-N-(4-(pentafluoro-λ⁶-sulfaneyl)phen yl)acetamide

At room temperature, 5-(benzyloxy)-6-methylpyrimidine-4-carboxylic acid **1g** (28.20 mg, 115.46 µmol), 1-(3-dimethylaminopropyl)-3-ethylcarbodiimide hydrochloride (29.51 mg, 153.95 µmol), 1-hydroxybenzotriazole (20.80 mg, 153.95 µmol), and N,N-diisopropylethylamine (49.74 mg, 384.86 µmol) were dissolved in N,N-dimethylformamide (2 mL), and the reaction was performed at room temperature for 40 min. Then 2-(2-(benzofuran-6-yl)-5-ethyl-7-oxo-6-(piperazin-1-yl)-[1,2,4]triazolo[1,5-a]pyrimidin-4(7H)-yl )-N-(4-(pentafluoro-λ⁶-sulfaneyl) phenyl)acetamide **77b** (48 mg, 76.97 µmol) was added, and the reaction was performed at room temperature for 18 h. Water (20 mL) was added to the reaction mixture, which was extracted with ethyl acetate (20 mL ×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(2-(benzofuran-6-yl)-6-(4-(5-(benzyloxy)-6-methylpyrimidine-4-carbonyl)piperazin-1-yl)-5-eth yl-7-oxo-[1,2,4]triazolo[1,5-a]pyrimidin-4(7H)-yl)-N-(4-(pentafluoro-λ⁶-sulfaneyl)phenyl)acetam ide **77c** (50 mg) with a yield of 76.44%.

MS m/z (ESI): 850.3 [M+1]

### Step 4

### 2-(2-(benzofuran-6-yl)-5-ethyl-6-(4-(5-hydroxy-6-methylpyrimidine-4-carbonyl)piperazin- 1-yl)-7-oxo-[1,2,4]triazolo[1,5-a]pyrimidin-4(7H)-yl)-N-(4-(pentafluoro-λ⁶-sulfaneyl)phenyl)acetamid e

To a solution of 2-(2-(benzofuran-6-yl)-6-(4-(5-(benzyloxy)-6-methylpyrimidine-4-carbonyl)piperazin-1-yl)-5-eth yl-7-oxo-[1,2,4]triazolo[1,5-a]pyrimidin-4(7H)-yl)-N-(4-(pentafluoro-λ⁶-sulfaneyl)phenyl)acetam ide **77c** (50 mg, 58.84 µmol) in dichloromethane (0.5 mL) was added trifluoroacetic acid (2 mL) at room temperature, and the reaction was performed at room temperature for 48 h. The mixture was concentrated under reduced pressure, and the residue was purified by preparative liquid chromatography (column AKZONOBEL Kromasil; 250×21.2 mm I.D.; 5 µm, 20 mL/min; mobile phase A: 0.05% TFA+H₂O, mobile phase B: CH₃CN) to obtain 2-(2-(benzofuran-6-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(4-(pentafluoro-λ⁶-sulfaneyl)phenyl)acetamid e **77** (10.96 mg) with a yield of 24.28%.

MS m/z (ESI): 760.2 [M+1]
¹H NMR (400 MHz, DMSO-d6) δ 11.05 (s, 1H), 10.28 (s, 1H), 8.60 (s, 1H), 8.23 (s, 1H), 8.12 (d, J = 2.2 Hz, 1H), 8.03 (dd, J = 8.1, 1.4 Hz, 1H), 7.89 (d, J = 9.3 Hz, 2H), 7.87 - 7.71 (m, 3H), 7.05 (d, J = 2.1 Hz, 1H), 5.31 (s, 2H), 4.56 (d, J = 12.5 Hz, 1H), 3.65 - 3.43 (m, 3H), 3.38 - 3.19 (m, 1H), 3.02 (q, J = 8.9, 7.1 Hz, 3H), 2.87 (d, J = 11.2 Hz, 1H), 2.69 (d, J = 10.8 Hz, 1H), 2.46 (s, 3H), 1.21 (t, J = 7.5 Hz, 3H).

### Example 78

### 2-(2-(benzofuran-6-yl)-5-ethyl-6-(4-(3-hydroxypicolinoyl)piperazin-1-yl)-7-oxo-[1,2,4]triazolo[1 ,5-a]pyrimidin-4(7H)-yl)-N-(4-(pentafluoro-λ⁶-sulfaneyl)phenyl)acetamide

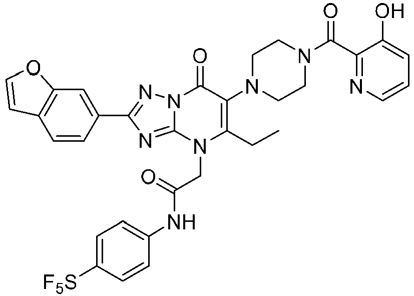

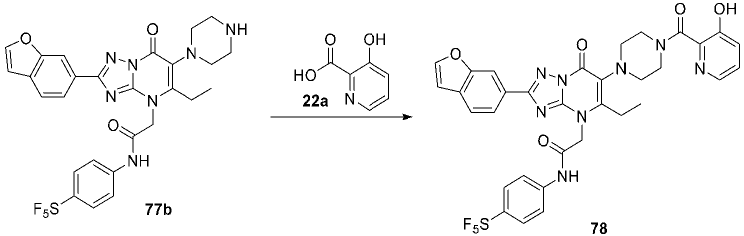

3-Hydroxypicolinic acid 22a (13.38 mg, 96.22 µmol), 1-(3-dimethylaminopropyl)-3-ethylcarbodiimide hydrochloride (24.59 mg, 128.29 µmol), 1-hydroxybenzotriazole (17.33 mg, 128.29 µmol), and N,N-diisopropylethylamine (41.45 mg, 320.72 µmol) were added to N,N-dimethylformamide (2 mL) at room temperature, and the reaction was performed at room temperature for 40 min. Then, 2-(2-(benzofuran-6-yl)-5-ethyl-7-oxo-6-(piperazin-1-yl)-[1,2,4]triazolo[1,5-a]pyrimidin-4(7H)-yl )-N-(4-(pentafluoro-λ⁶-sulfaneyl)phenyl)acetamide **77b** (40 mg, 64.14 µmol) was added, and the reaction was performed at room temperature overnight. Water (20 mL) was added to the reaction mixture, and the mixture was extracted with ethyl acetate (20 mL×3). The combined organic phases were washed with saturated sodium chloride solution (20 mL), dried over anhydrous sodium sulfate, filtered, and concentrated under reduced pressure. The residue was purified by preparative liquid chromatography (column AKZONOBEL Kromasil; 250×21.2 mm I.D.; 5 µm, 20 mL/min; mobile phase A: 0.05% TFA+H₂O, mobile phase B: CH₃CN) to obtain 2-(2-(benzofuran-6-yl)-5-ethyl-6-(4-(3-hydroxypicolinoyl)piperazin-1-yl)-7-oxo-[1,2,4]triazolo[1 ,5-a]pyrimidin-4(7H)-yl)-N-(4-(pentafluoro-λ⁶-sulfaneyl)phenyl)acetamide **78** (8.46 mg) with a yield of 17.00%.

MS m/z (ESI): 745.2 [M+1]
¹H NMR (400 MHz, DMSO-d6) δ 11.03 (s, 1H), 10.48 (s, 1H), 8.23 (s, 1H), 8.17 - 8.05 (m, 2H), 8.02 (dd, J = 8.2, 1.4 Hz, 1H), 7.89 (d, J = 9.1 Hz, 2H), 7.79 (dd, J = 8.6, 6.1 Hz, 3H), 7.44 - 7.24 (m, 2H), 7.05 (d, J = 2.1 Hz, 1H), 5.31 (s, 2H), 4.58 (d, J = 12.4 Hz, 1H), 3.55 - 3.47 (m, 2H), 3.44 (d, J = 12.6 Hz, 1H), 3.26 (t, J = 12.1 Hz, 1H), 3.01 (h, J = 9.2, 8.3 Hz, 3H), 2.85 (d, J = 11.2 Hz, 1H), 2.67 (d, J = 10.8 Hz, 1H), 1.20 (t, J = 7.5 Hz, 3H).

### Example 79

### 2-(2-(benzo[b]thiophen-2-yl)-5-ethyl-6-(4-(3-hydroxypicolinoyl)piperazin-1-yl)-7-oxo-[1,2,4]tria zolo[1,5-a]pyrimidin-4(7H)-yl)-N-(2-chloro-4-(trifluoromethyl)phenyl)acetamide

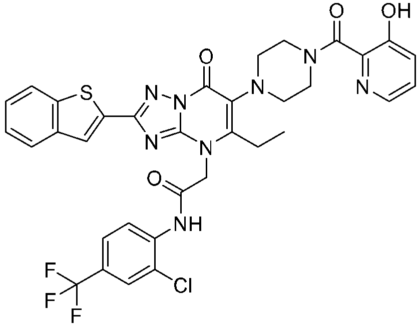

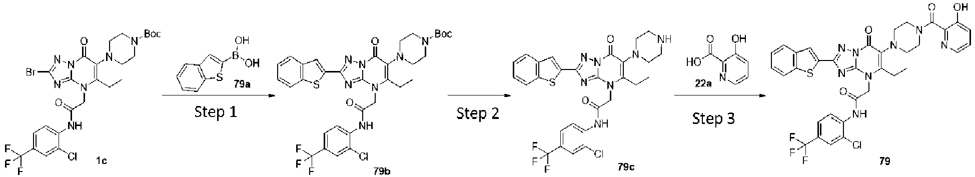

### Step 1

### tert-butyl 4-(2-(benzo[b]thiophen-2-yl)-4-(2-((2-chloro-4-(trifluoromethyl)phenyl)amino)-2-oxoethyl)-5-et hyl-7-oxo-4,7-dihydro-[1,2,4]triazolo[1,5-a]pyrimidin-6-yl)piperazine-1-carboxylate

Tert-butyl 4-(2-bromo-4-(2-((2-chloro-4-(trifluoromethyl)phenyl)amino)-2-oxoethyl)-5-ethyl-7-oxo-4,7-dih ydro-[1,2,4]triazolo[1,5-a]pyrimidin-6-yl)piperazine-1-carboxylate **1c** (51.56 mg, 289.64 µmol), [1,1'-bis(diphenylphosphino)ferrocene]palladium(II) dichloride dichloromethane complex (19.56 mg, 24.14 µmol), and sodium carbonate (76.75 mg, 724.11 µmol) were dissolved in a mixed solution of 1,4-dioxane (5 mL) and water (0.5 mL). The mixture was subject to argon replacement three times, heated to 80 °C, and the reaction was performed for 2 h. Water (20 mL) was added to the reaction mixture, and the mixture was extracted with ethyl acetate (20 mL ×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(2-(benzo[b]thiophen-2-yl)-4-(2-((2-chloro-4-(trifluoromethyl)phenyl)amino)-2-oxoethyl)-5-et hyl-7-oxo-4,7-dihydro-[1,2,4]triazolo[1,5-a]pyrimidin-6-yl)piperazine-1-carboxylate **79b** (170 mg) with a yield of 98.34%.

MS m/z (ESI): 660.2 [M-56+H]

### Step 2

### 2-(2-(benzo[b]thiophen-2-yl)-5-ethyl-7-oxo-6-(piperazin-1-yl)-[1,2,4]triazolo[1,5-a]pyrimidin-4( 7H)-yl)-N-(2-chloro-4-(trifluoromethyl)phenyl)acetamide

At room temperature, trifluoroacetic acid (1.5 mL) was added to a solution of tert-butyl 4-(2-(benzo[b]thiophen-2-yl)-4-(2-((2-chloro-4-(trifluoromethyl)phenyl)amino)-2-oxoethyl)-5-et hyl-7-oxo-4,7-dihydro-[1,2,4]triazolo[1,5-a]pyrimidin-6-yl)piperazine-1-carboxylate **79b** (170 mg, 237.37 µmol) in dichloromethane (6 mL), and the reaction was performed at room temperature for 40 min. The mixture was concentrated under reduced pressure, and the resulting residue was purified by silica gel column chromatography (eluent: System B) to obtain 2-(2-(benzo[b]thiophen-2-yl)-5-ethyl-7-oxo-6-(piperazin-1-yl)-[1,2,4]triazolo[1,5-a]pyrimidin-4( 7H)-yl)-N-(2-chloro-4-(trifluoromethyl)phenyl)acetamide **79c** (100 mg) with a yield of 68.38%.

MS m/z (ESI): 616.2 [M+1]

### Step 3

### 2-(2-(benzo[b]thiophen-2-yl)-5-ethyl-6-(4-(3-hydroxypicolinoyl)piperazin-1-yl)-7-oxo-[1,2,4]tria zolo[1,5-a]pyrimidin-4(7H)-yl)-N-(2-chloro-4-(trifluoromethyl)phenyl)acetamide

At room temperature, 3-hydroxypicolinic acid **22a** (13.55 mg, 97.39 µmol), 1-(3-dimethylaminopropyl)-3-ethylcarbodiimide hydrochloride (24.89 mg, 129.86 µmol), 1-hydroxybenzotriazole (17.55 mg, 129.86 µmol), and N,N-diisopropylethylamine (41.96 mg, 324.65 µmol) were added to N,N-dimethylformamide (2 mL), and the mixture was stirred at room temperature for 40 min. Then, 2-(2-(benzo[b]thiophen-2-yl)-5-ethyl-7-oxo-6-(piperazin-1-yl)-[1,2,4]triazolo[1,5-a]pyrimidin-4( 7H)-yl)-N-(2-chloro-4-(trifluoromethyl)phenyl)acetamide **79c** (40 mg, 64.93 µmol) was added, and the reaction was performed at room temperature for 18 h. Water (20 mL) was added to the reaction mixture, which was extracted with ethyl acetate (20 mL×3). The combined organic phases were washed with saturated sodium chloride solution (20 mL), dried over anhydrous sodium sulfate, filtered, and concentrated under reduced pressure. The residue was purified by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain 2-(2-(benzo[b]thiophen-2-yl)-5-ethyl-6-(4-(3-hydroxypicolinoyl)piperazin-1-yl)-7-oxo-[1,2,4]tria zolo[1,5-a]pyrimidin-4(7H)-yl)-N-(2-chloro-4-(trifluoromethyl)phenyl)acetamide (9.95 mg) with a yield of 19.44%.

MS m/z (ESI): 737.2 [M+1]
¹H NMR (400 MHz, DMSO-d6) δ 10.43 (s, 2H), 8.13 (s, 1H), 8.11 - 8.02 (m, 3H), 7.98 (q, J = 3.5 Hz, 2H), 7.72 (dd, J = 8.8, 2.1 Hz, 1H), 7.52 - 7.41 (m, 2H), 7.36 - 7.27 (m, 2H), 5.40 (s, 2H), 4.57 (d, J = 12.5 Hz, 1H), 3.47 (dd, J = 26.3, 12.6 Hz, 3H), 3.25 (t, J = 12.1 Hz, 1H), 3.12 - 2.93 (m, 3H), 2.84 (d, J = 11.6 Hz, 1H), 2.66 (d, J = 11.0 Hz, 1H), 1.22 (t, J = 7.4 Hz, 3H).

### Example 80

### 2-(2-(benzo[b]thiophen-2-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acet amide

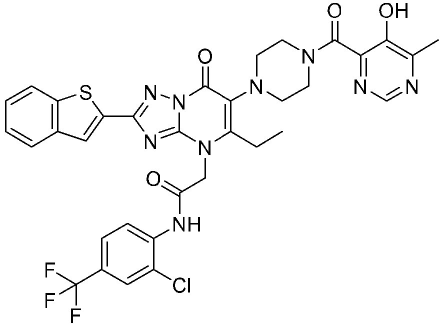

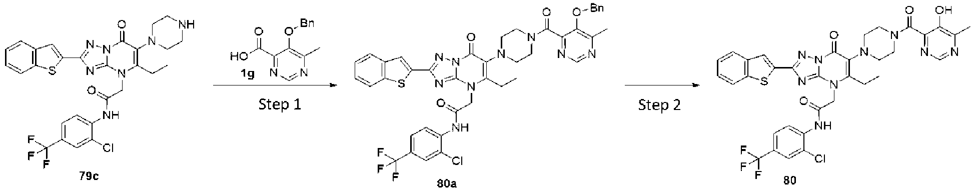

### Step 1

### 2-(2-(benzo[b]thiophen-2-yl)-6-(4-(5-(benzyloxy)-6-methylpyrimidine-4-carbonyl)piperazin-1-yl )-5-ethyl-7-oxo-[1,2,4]triazolo[1,5-a]pyrimidin-4(7H)-yl)-N-(2-chloro-4-(trifluoromethyl)phenyl) acetamide

At room temperature, 5-(benzyloxy)-6-methylpyrimidine-4-carboxylic acid **1g** (23.79 mg, 97.39 µmol),1-(3-dimethylaminopropyl)-3-ethylcarbodiimide hydrochloride (37.34 mg, 194.79 µmol), 1-hydroxybenzotriazole (26.32 mg, 194.79 µmol), and N,N-diisopropylethylamine (62.94 mg, 486.97 µmol) were dissolved in N,N-dimethylformamide (2 mL), and the reaction was performed at room temperature for 40 min. Then, 2-(2-(benzo[b]thiophen-2-yl)-5-ethyl-7-oxo-6-(piperazin-1-yl)-[1,2,4]triazolo[1,5-a]pyrimidin-4( 7H)-yl)-N-(2-chloro-4-(trifluoromethyl)phenyl) acetamide 79c (60 mg, 97.39 µmol) was added, and the reaction was performed at room temperature for 18 h. Water (20 mL) was added to the reaction mixture, which was extracted with ethyl acetate (20 mL×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(2-(benzo[b]thiophen-2-yl)-6-(4-(5-(benzyloxy)-6-methylpyrimidine-4-carbonyl)piperazin-1-yl )-5-ethyl-7-oxo-[1,2,4]triazolo[1,5-a]pyrimidin-4(7H)-yl)-N-(2-chloro-4-(trifluoromethyl)phenyl) acetamide **80a** (60 mg) with a yield of 73.14%.

MS m/z (ESI): 842.2 [M+1]

### Step 2

### 2-(2-(benzo[b]thiophen-2-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acet amide

In an ice bath under argon atmosphere, boron trichloride (1 mL) was added to a solution of 2-(2-(benzo[b]thiophen-2-yl)-6-(4-(5-(benzyloxy)-6-methylpyrimidine-4-carbonyl)piperazin-1-yl )-5-ethyl-7-oxo-[1,2,4]triazolo[1,5-a]pyrimidin-4(7H)-yl)-N-(2-chloro-4-(trifluoromethyl) phenyl)acetamide **80a** (60 mg, 71.23 µmol) in dichloromethane (1 mL), and then the reaction was performed at room temperature for 2 h. The reaction was quenched with methanol, and the mixture was concentrated. The residue was dissolved in methanol and purified by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain 2-(2-(benzo[b]thiophen-2-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl) acetamide **80** (0.87 mg) with a yield of 1.58%.

MS m/z (ESI): 752.2 [M+1]
¹H NMR (400 MHz, DMSO-d6) δ 10.43 (s, 1H), 10.25 (s, 1H), 8.59 (s, 1H), 8.13 (s, 1H), 8.06 (t, J = 9.2 Hz, 2H), 7.98 (q, J = 4.2, 3.5 Hz, 2H), 7.72 (dd, J = 8.8, 2.1 Hz, 1H), 7.56 - 7.34 (m, 2H), 5.40 (s, 2H), 4.54 (d, J = 12.4 Hz, 1H), 3.52 (d, J = 12.2 Hz, 3H), 3.27 (d, J = 2.7 Hz, 1H), 3.03 (d, J = 10.3 Hz, 3H), 2.85 (d, J = 11.3 Hz, 1H), 2.68 (d, J = 10.1 Hz, 1H), 2.45 (s, 3H), 1.27 - 1.19 (m, 3H).

### Example 81

### N-(2-chloro-4-(trifluoromethyl)phenyl)-2-(2-(2,3-dihydrobenzofuran-6-yl)-5-ethyl-6-(4-(5-hydro xy-6-methylpyrimidine-4-carbonyl)piperazin-1-yl)-7-oxo-[1,2,4]triazolo[1,5-a]pyrimidin-4(7H)-yl)acetamide

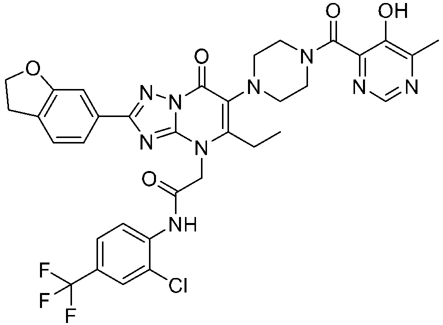

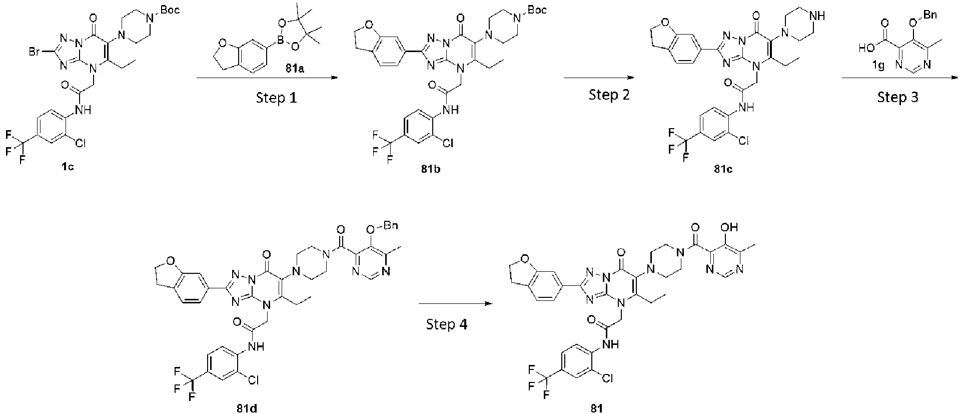

### Step 1

### tert-butyl 4-(4-(2-((2-chloro-4-(trifluoromethyl)phenyl)amino)-2-oxoethyl)-2-(2,3-dihydrobenzofuran-6-yl) -5-ethyl-7-oxo-4,7-dihydro-[1,2,4]triazolo[1,5-a]pyrimidin-6-yl)piperazine-1-carboxylate

At room temperature, tert-butyl 4-(2-bromo-4-(2-((2-chloro-4-(trifluoromethyl) phenyl)amino)-2-oxoethyl)-5-ethyl-7-oxo-4,7-dihydro-[1,2,4]triazolo[1,5-a]pyrimidin-6-yl)piper azine-1-carboxylate **1c** (160 mg, 241.37 µmol), 2-(2,3-dihydrobenzofuran-6-yl)-4,4,5,5-tetramethyl-1,3,2-dioxaborolane 81a (77.22 mg, 313.78 µmol, commercially available), [1,1'-bis(diphenylphosphino)ferrocene]palladium(II) dichloride dichloromethane complex (19.57 mg, 24.14 µmol), and sodium carbonate (76.75 mg, 724.11 µmol) were dissolved in a mixed solution of 1,4-dioxane (10 mL) and water (1 mL). The mixture was subject to argon replacement 3 times, heated to 85 °C, and the reaction was performed for 2 hours. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(4-(2-((2-chloro-4-(trifluoromethyl)phenyl)amino)-2-oxoethyl)-2-(2,3-dihydrobenzofuran-6-yl) -5-ethyl-7-oxo-4,7-dihydro-[1,2,4]triazolo[1,5-a]pyrimidin-6-yl)piperazine-1-carboxylate 81b (160 mg) with a yield of 94.41%.

MS m/z (ESI): 646.2 [M-56+H]

### Step 2

### N-(2-chloro-4-(trifluoromethyl)phenyl)-2-(2-(2,3-dihydrobenzofuran-6-yl)-5-ethyl-7-oxo-6-(pipe razin-1-yl)-[1,2,4]triazolo[1,5-a]pyrimidin-4(7H)-yl)acetamide

At room temperature, trifluoroacetic acid (1 mL) was added to a solution of tert-butyl 4-(4-(2-((2-chloro-4-(trifluoromethyl)phenyl)amino)-2-oxoethyl)-2-(2,3-dihydrobenzofuran-6-yl) -5-ethyl-7-oxo-4,7-dihydro-[1,2,4]triazolo[1,5-a]pyrimidin-6-yl)piperazine-1-carboxylate **81b** (80 mg, 113.94 µmol) in dichloromethane (4 mL), and the reaction was performed at room temperature for 40 minutes. The mixture was concentrated under reduced pressure to obtain N-(2-chloro-4-(trifluoromethyl)phenyl)-2-(2-(2,3-dihydrobenzofuran-6-yl)-5-ethyl-7-oxo-6-(pipe razin-1-yl)-[1,2,4]triazolo[1,5-a]pyrimidin-4(7H)-yl)acetamide 81c (68.5 mg) with a yield of 99.86%, which was used directly in the next step.

MS m/z (ESI): 602.2 [M+1]

### Step 3

### 2-(6-(4-(5-(benzyloxy)-6-methylpyrimidine-4-carbonyl)piperazin-1-yl)-2-(2,3-dihydrobenzofuran -6-yl)-5-ethyl-7-oxo-[1,2,4]triazolo[1,5-a]pyrimidin-4(7H)-yl)-N-(2-chloro-4-(trifluoromethyl)ph enyl)acetamide

At room temperature, 5-(benzyloxy)-6-methylpyrimidine-4-carboxylic acid **1g** (41.69 mg, 170.68 µmol), 1-(3-dimethylaminopropyl)-3-ethylcarbodiimide hydrochloride (43.63 mg, 227.57 µmol), 1-hydroxybenzotriazole (30.75 mg, 227.57 µmol), and N,N-diisopropylethylamine (73.53 mg, 568.93 µmol) were dissolved in N,N-dimethylformamide (3 mL), and the reaction was performed at room temperature for 40 minutes. Subsequently, a solution of N-(2-chloro-4-(trifluoromethyl)phenyl)-2-(2-(2,3-dihydrobenzofuran-6-yl)-5-ethyl-7-oxo-6-(pipe razin-1-yl)-[1,2,4]triazolo[1,5-a]pyrimidin-4(7H)-yl)acetamide 81c (68.5 mg, 113.79 µmol) in N,N-dimethylformamide (3 mL) was added, and the reaction was performed at room temperature for 48 hours. Water (20 mL) was added to the reaction mixture, and the mixture was extracted with ethyl acetate (20 mL ×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-2-(2,3-dihydrobenzofuran -6-yl)-5-ethyl-7-oxo-[1,2,4]triazolo[1,5-a]pyrimidin-4(7H)-yl)-N-(2-chloro-4-(trifluoromethyl)ph enyl)acetamide 81d (60 mg) with a yield of 63.67%.

MS m/z (ESI): 828.3 [M+1]

### Step 4

### N-(2-chloro-4-(trifluoromethyl)phenyl)-2-(2-(2,3-dihydrobenzofuran-6-yl)-5-ethyl-6-(4-(5-hydro xy-6-methylpyrimidine-4-carbonyl)piperazin-1-yl)-7-oxo-[1,2,4]triazolo[1,5-a]pyrimidin-4(7H)-yl)acetamide

At room temperature, trifluoroacetic acid (2 mL) was added to a solution of 2-(6-(4-(5-(benzyloxy)-6-methylpyrimidine-4-carbonyl)piperazin-1-yl)-2-(2,3-dihydrobenzofuran -6-yl)-5-ethyl-7-oxo-[1,2,4]triazolo[1,5-a]pyrimidin-4(7H)-yl)-N-(2-chloro-4-(trifluoromethyl)ph enyl) acetamide 81d (60 mg, 72.44 µmol) in dichloromethane (0.5 mL), and the reaction was performed at room temperature for 48 hours. The mixture was concentrated under reduced pressure, and the residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(2-(2,3-dihydrobenzofuran-6-yl)-5-ethyl-6-(4-(5-hydro xy-6-methylpyrimidine-4-carbonyl)piperazin-1-yl)-7-oxo-[1,2,4]triazolo[1,5-a]pyrimidin-4(7H)-yl)acetamide 81 (20.09 mg) with a yield of 36.82%.

MS m/z (ESI): 738.2 [M+1]
¹H NMR (400 MHz, DMSO-d6) δ 10.42 (s, 1H), 10.25 (s, 1H), 8.59 (s, 1H), 8.04 (d, J = 8.6 Hz, 1H), 7.98 (d, J = 2.0 Hz, 1H), 7.72 (dd, J = 8.7, 2.1 Hz, 1H), 7.62 (dd, J = 7.7, 1.4 Hz, 1H), 7.42 (d, J = 1.3 Hz, 1H), 7.36 (d, J = 7.7 Hz, 1H), 5.38 (s, 2H), 4.58 (t, J = 8.8 Hz, 2H), 4.51 (d, J = 12.2 Hz, 1H), 3.56 - 3.46 (m, 3H), 3.23 (t, J = 8.9 Hz, 3H), 3.02 (d, J = 10.4 Hz, 3H), 2.84 (d, J = 11.2 Hz, 1H), 2.66 (d, J = 10.8 Hz, 1H), 2.45 (s, 3H), 1.21 (t, J = 7.4 Hz, 3H).

### Example 82

### N-(2-chloro-4-(trifluoromethyl)phenyl)-2-(2-(2,3-dihydrobenzofuran-5-yl)-5-ethyl-6-(4-(5-hydro xy-6-methylpyrimidine-4-carbonyl)piperazin-1-yl)-7-oxopyrazolo[1,5-a]pyrimidin-4(7H)-yl)acet amide

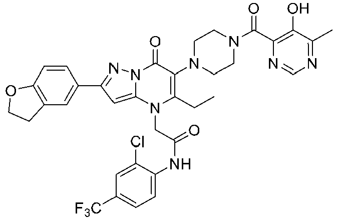

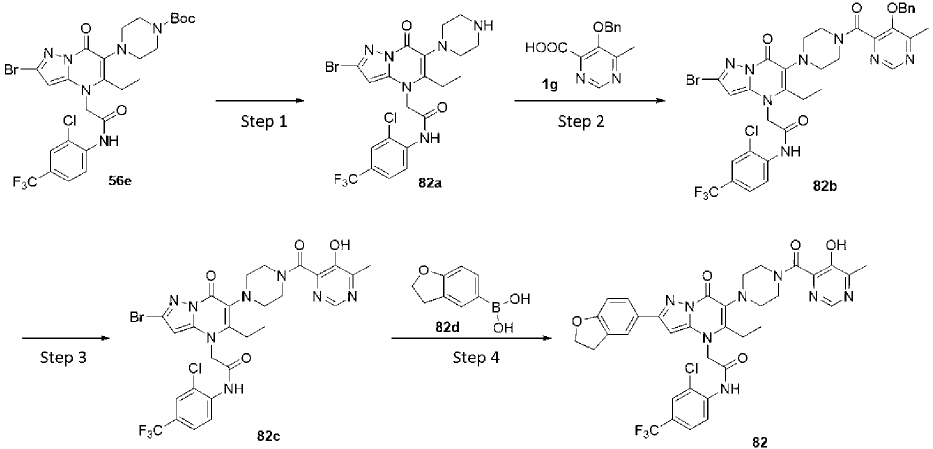

### Step 1

### 2-(2-bromo-5-ethyl-7-oxo-6-(piperazin-1-yl)pyrazolo[1,5-a]pyrimidin-4(7H)-yl)-N-(2-chloro-4-(t rifluoromethyl)phenyl)acetamide

Tert-butyl 4-(2-bromo-4-(2-((2-chloro-4-(trifluoromethyl)phenyl)amino)-2-oxoethyl)-5-ethyl-7-oxo-4,7-dih ydropyrazolo[1,5-a]pyrimidin-6-yl)piperazine-1-carboxylate 56e (100 mg, 151.08 µmol) was dissolved in dichloromethane (3 mL), followed by dropwise addition of trifluoroacetic acid (1 g, 8.77 mmol), and the reaction was performed at room temperature for 3 h. The mixture was concentrated under reduced pressure to obtain 2-(2-bromo-5-ethyl-7-oxo-6-(piperazin-1-yl)pyrazolo[1,5-a]pyrimidin-4(7H)-yl)-N-(2-chloro-4-(t rifluoromethyl)phenyl) acetamide 82a (80 mg), and the crude product was used directly in the next step.

MS m/z (ESI): 560.9 [M+H]

### Step 2

### 2-(6-(4-(5-(benzyloxy)-6-methylpyrimidine-4-carbonyl)piperazin-1-yl)-2-bromo-5-ethyl-7-oxopy razolo[1,5-a]pyrimidin-4(7H)-yl)-N-(2-chloro-4-(trifluoromethyl)phenyl)acetamide

5-(Benzyloxy)-6-methylpyrimidine-4-carboxylic acid **1g** (52.17 mg, 213.61 µmol) was dissolved in N,N-dimethylformamide (2 mL), followed by addition of 1-(3-dimethylaminopropyl)-3-ethylcarbodiimide hydrochloride (54.60 mg, 284.81 µmol), 1-hydroxybenzotriazole (38.48 mg, 284.81 µmol) and N,N-diisopropylethylamine (92.02 mg, 712.02 µmol), and the reaction was performed at room temperature for 15 min, then 2-(2-bromo-5-ethyl-7-oxo-6-(piperazin-1-yl)pyrazolo[1,5-a]pyrimidin-4(7H)-yl)-N-(2-chloro-4-(t rifluoromethyl)phenyl)acetamide 82a (80 mg, 142.4 µmol) was added, and the reaction was performed at room temperature for 18 h. Water (50 mL) was added to the reaction mixture, which was extracted with dichloromethane (50 mL × 3), the combined organic phase was washed with saturated sodium chloride solution (30 mL × 2), dried over anhydrous sodium sulfate, filtered, and concentrated under reduced pressure, and the resulting residue was purified by silica gel column chromatography (eluent: System B) to obtain 2-(6-(4-(5-(benzyloxy)-6-methylpyrimidine-4-carbonyl)piperazin-1-yl)-2-bromo-5-ethyl-7-oxopy razolo[1,5-a]pyrimidin-4(7H)-yl)-N-(2-chloro-4-(trifluoromethyl)phenyl)acetamide 82b (80 mg) with a yield of 71.29%.

MS m/z (ESI): 788.9 [M+H]

### Step 3

### 2-(2-bromo-5-ethyl-6-(4-(5-hydroxy-6-methylpyrimidine-4-carbonyl)piperazin-1-yl)-7-oxopyraz olo[1,5-a]pyrimidin-4(7H)-yl)-N-(2-chloro-4-(trifluoromethyl)phenyl)acetamide

2-(6-(4-(5-(Benzyloxy)-6-methylpyrimidine-4-carbonyl)piperazin-1-yl)-2-bromo-5-ethy 1-7-oxopyrazolo[1,5-a]pyrimidin-4(7H)-yl)-N-(2-chloro-4-(trifluoromethyl)phenyl)acetamide 82b (65 mg, 82.49 µmol) was dissolved in trifluoroacetic acid (3 mL), and the reaction was performed at 40 °C for 3 h. The mixture was concentrated under reduced pressure to obtain 2-(2-bromo-5-ethyl-6-(4-(5-hydroxy-6-methylpyrimidine-4-carbonyl)piperazin-1-yl)-7-oxopyraz olo[1,5-a]pyrimidin-4(7H)-yl)-N-(2-chloro-4-(trifluoromethyl)phenyl)acetamide 82c (50 mg), and the crude product was used directly in the next step.

MS m/z (ESI): 698.9 [M+H]

### Step 4

### N-(2-chloro-4-(trifluoromethyl)phenyl)-2-(2-(2,3-dihydrobenzofuran-5-yl)-5-ethyl-6-(4-(5-hydro xy-6-methylpyrimidine-4-carbonyl)piperazin-1-yl)-7-oxopyrazolo[1,5-a]pyrimidin-4(7H)-yl)acet amide

At room temperature, (2,3-dihydrobenzofuran-5-yl)boronic acid 82d (23.49 mg, 143.29 µmol), 2-(2-bromo-5-ethyl-6-(4-(5-hydroxy-6-methylpyrimidine-4-carbonyl)piperazin-1-yl)-7-oxopyraz olo[1,5-a]pyrimidin-4(7H)-yl)-N-(2-chloro-4-(trifluoromethyl)phenyl)acetamide **82c** (50 mg, 71.64 µmol), [1,1'-bis(diphenylphosphino)ferrocene]palladium(II) dichloride (5.31 mg, 7.16 µmol) and sodium carbonate (75.94 mg, 716.45 µmol) were dissolved in 1,4-dioxane (1 mL) and water (0.5 mL), followed by argon replacement, and the reaction was performed at 80 °C for 3 h. Water (20 mL) was added, the mixture was extracted with dichloromethane (30 mL × 3), the combined organic phase was washed with saturated sodium chloride solution (30 mL × 2), dried over anhydrous sodium sulfate, filtered, and concentrated under reduced pressure, and the resulting residue was separated by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(2,3-dihydrobenzofuran-5-yl)-5-ethyl-6-(4-(5-hydroxy-6-methylpyrimidine-4-carbonyl)piperazin-1-yl)-7-oxopyrazolo[1,5-a]pyrimidin-4(7H)-yl)acetami de 82 (20 mg) with a yield of 35.98%.

MS m/z (ESI): 737.0 [M+H]
¹H NMR (400 MHz, DMSO-d6) δ 10.29 (s, 1H), 10.24 (s, 1H), 8.58 (s, 1H), 8.13 (d, J = 8.6 Hz, 1H), 7.98 (d, J = 2.1 Hz, 1H), 7.79 (d, J = 1.8 Hz, 1H), 7.72 (dd, J = 8.8, 2.1 Hz, 1H), 7.64 (dd, J = 8.3, 1.9 Hz, 1H), 6.85 (d, J = 8.3 Hz, 1H), 6.78 (s, 1H), 5.22 (s, 2H), 4.62 - 4.49 (m, 3H), 3.24 (t, J = 8.8 Hz, 3H), 2.96 (t, J = 8.5 Hz, 3H), 2.81 (d, J = 11.4 Hz, 1H), 2.64 (d, J = 11.1 Hz, 1H), 2.45 (s, 3H), 1.19 (t, J = 7.4 Hz, 3H).

### Example 83

### N-(2-chloro-4-(trifluoromethyl)phenyl)-2-(5-ethyl-6-(4-(5-hydroxy-6-methylpyrimidine-4-carbon yl)piperazin-1-yl)-2-(1-(4-methoxyphenyl)vinyl)-7-oxo-[1,2,4]triazolo[1,5-a]pyrimidin-4(7H)-yl) acetamide

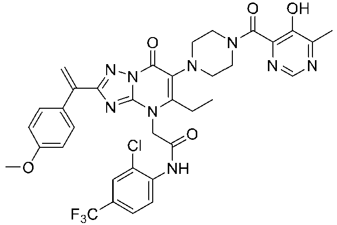

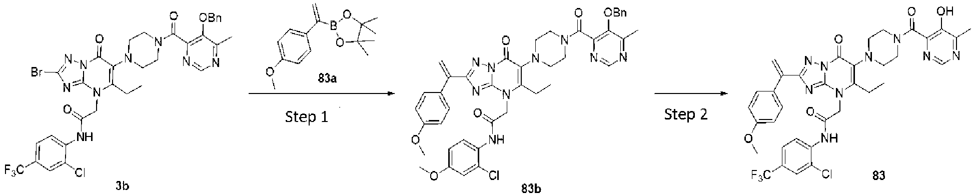

### Step 1

### 2-(6-(4-(5-(benzyloxy)-6-methylpyrimidine-4-carbonyl)piperazin-1-yl)-5-ethyl-2-(1-(4-methoxyp henyl)vinyl)-7-oxo-[1,2,4]triazolo[1,5-a]pyrimidin-4(7H)-yl)-N-(2-chloro-4-methoxyphenyl)acet amide

2-(6-(4-(5-(Benzyloxy)-6-methylpyrimidine-4-carbonyl)piperazin-1-yl)-2-bromo-5-ethy 1-7-oxo-[1,2,4]triazolo[1,5-a]pyrimidin-4(7H)-yl)-N-(2-chloro-4-(trifluoromethyl)phenyl) acetamide **3b** (60 mg, 76.05 µmol), 2-(1-(4-methoxyphenyl)vinyl)-4,4,5,5-tetramethyl-1,3,2-dioxaborolane **83a** (27.70 mg, 106.46 µmol, commercially available), [1,1'-bis(diphenylphosphino)ferrocene]palladium(II) dichloride (6.16 mg, 7.60 µmol),and sodium carbonate (16.12 mg, 152.09 µmol) were dissolved in 1,4-dioxane (2 mL) and water (0.5 mL). After argon replacement, the reaction was performed at 80 °C for 2 hours. Water (20 mL) was added to the reaction mixture, and the mixture was extracted with dichloromethane (20 mL × 3). The combined organic phase was washed with saturated sodium chloride solution (20 mL), dried over anhydrous sodium sulfate, filtered, and concentrated under reduced pressure. The resulting residue was purified by silica gel column chromatography (eluent: System **B)** to obtain 2-(6-(4-(5-(benzyloxy)-6-methylpyrimidine-4-carbonyl)piperazin-1-yl)-5-ethyl-2-(1-(4-methoxyp henyl) vinyl)-7-oxo-[1,2,4]triazolo[1,5-a]pyrimidin-4(7H)-yl)-N-(2-chloro-4-methoxyphenyl)acetamide **83b** (45 mg) with a yield of 70.26%.

MS m/z (ESI): 842.3 [M+H]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-2-(1-(4-methoxyphenyl)vinyl)-7-oxo-[1,2,4]triazolo[1,5-a]pyrimidin-4(7H)-yl) acetamide

2-(6-(4-(5-(Benzyloxy)-6-methylpyrimidine-4-carbonyl)piperazin-1-yl)-5-ethyl-2-(1-(4-methoxyphenyl)vinyl)-7-oxo-[1,2,4]triazolo[1,5-a]pyrimidin-4(7H)-yl)-N-(2-chloro-4-methoxyp henyl)acetamide **83b** (45 mg, 53.43 µmol) was dissolved in trifluoroacetic acid (3 mL), and the reaction was performed at 45 °C for 3 hours. After the reaction was completed, the mixture was concentrated under reduced pressure, and then separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(2-chloro-4-(trifluoromethyl)phenyl)-2-(5-ethyl-6-(4-(5-hydroxy-6-methylpyrimidine-4-carbon yl)piperazin-1-yl)-2-(1-(4-methoxyphenyl)vinyl)-7-oxo-[1,2,4]triazolo[1,5-a]pyrimidin-4(7H)-yl) acetamide **83** (10 mg) with a yield of 23.64%.

MS m/z (ESI): 752.1 [M+H]
¹H NMR (400 MHz, DMSO-d6) δ 10.36 (s, 1H), 10.23 (s, 1H), 8.58 (s, 1H), 8.07 (d, J = 8.6 Hz, 1H), 7.96 (d, J = 2.1 Hz, 1H), 7.73 (dd, J = 8.7, 2.1 Hz, 1H), 7.46 (d, J = 8.7 Hz, 1H), 7.14 - 6.97 (m, 1H), 6.91 (d, J = 8.8 Hz, 1H), 6.12 (d, J = 1.5 Hz, 1H), 5.74 (d, J = 1.5 Hz, 1H), 5.34 (s, 1H), 4.53 (d, J = 12.6 Hz, 1H), 3.88 (s, 9H), 3.76 (s, 2H), 3.50 (d, J = 10.7 Hz, 2H), 3.31 - 3.13 (m, 1H), 3.02 (d, J = 9.4 Hz, 2H), 2.83 (d, J = 11.2 Hz, 1H), 2.75 - 2.57 (m, 1H), 2.44 (s, 2H), 1.26 - 1.16 (m, 3H).

### Example 84

### N-(2-chloro-4-(pentafluoro-λ⁶-sulfaneyl)phenyl)-2-(5-ethyl-6-(4-(5-hydroxy-6-methylpyrimidine -4-carbonyl)piperazin-1-yl)-7-oxo-2-(2,5,6,7-tetrahydrooxepin-3-yl)-[1,2,4]triazolo[1,5-a]pyrimi din-4(7H)-yl)acetamide

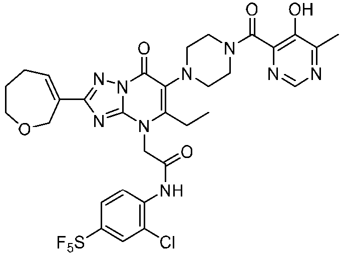

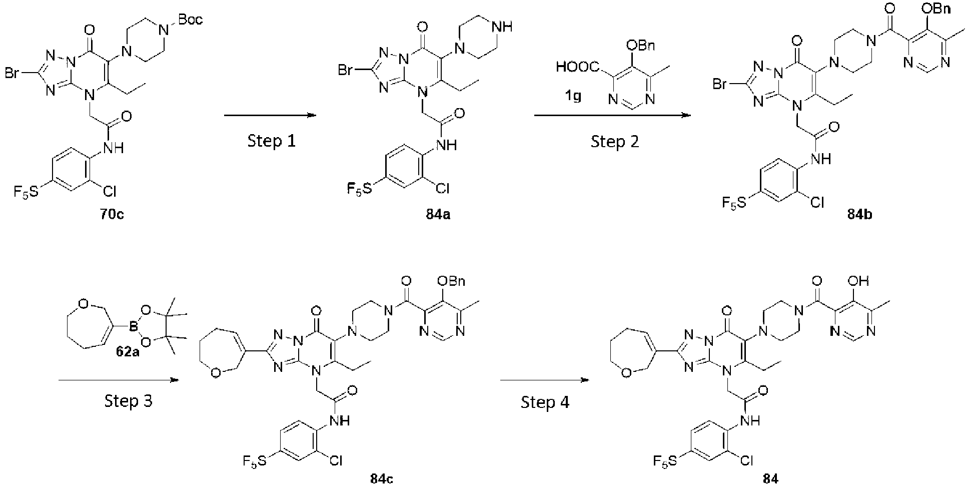

### Step 1

### 2-(2-bromo-5-ethyl-7-oxo-6-(piperazin-1-yl)-[1,2,4]triazolo[1,5-a]pyrimidin-4(7H)-yl)-N-(2-chlo ro-4-(pentafluoro-λ⁶-sulfaneyl)phenyl)acetamide

Tert-butyl 4-(2-bromo-4-(2-((2-chloro-4-(pentafluoro-λ⁶-sulfaneyl)phenyl)amino)-2-oxoethyl)-5-ethyl-7-ox 0-4,7-dihydro-[1,2,4]triazolo[1,5-a]pyrimidin-6-yl)piperazine-1-carboxylate **70c** (0.5 g, 693.54 µmol) was dissolved in dichloromethane (8 mL), and trifluoroacetic acid (2 g, 17.54 mmol) was added dropwise. The reaction was performed at room temperature for 3 hours. The mixture was concentrated under reduced pressure to obtain 2-(2-bromo-5-ethyl-7-oxo-6-(piperazin-1-yl)-[1,2,4]triazolo[1,5-a]pyrimidin-4(7H)-yl)-N-(2-chlo ro-4-(pentafluoro-λ⁶-sulfaneyl)phenyl)acetamide **84a** (0.4 g), which was directly used in the next step as a crude product.

MS m/z (ESI): 621.9 [M+H]

### Step 2

### 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(pentafluoro-λ⁶-sulfaneyl)phenyl)acetami de

5-(Benzyloxy)-6-methylpyrimidine-4-carboxylic acid **1g** (236.05 mg, 966.46 µmol) was added to N,N-dimethylformamide (5 mL), followed by addition of 1-(3-dimethylaminopropyl)-3-ethylcarbodiimide hydrochloride (247.03 mg, 1.29 mmol), 1-hydroxybenzotriazole (174.12 mg, 1.29 mmol) and N,N-diisopropylethylamine (416.35 mg, 3.22 mmol), and the reaction was performed at room temperature for 15 minutes. Subsequently, 2-(2-bromo-5-ethyl-7-oxo-6-(piperazin-1-yl)-[1,2,4]triazolo[1,5-a]pyrimidin-4(7H)-yl)-N-(2-chlo ro-4-(pentafluoro-λ⁶-sulfaneyl)phenyl)acetamide **84a** (0.4 g, 644.31 µmol) was added, and the reaction was performed at room temperature overnight. Water (50 mL) was added to the reaction mixture, followed by extraction with dichloromethane (50 mL×3). The combined organic phases were washed with saturated sodium chloride solution (30 mL×2), dried over anhydrous sodium sulfate, filtered, and concentrated under reduced pressure. The obtained residue was purified by silica gel column chromatography (eluent: System B) to obtain 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(pentafluoro-λ⁶-sulfaneyl)phenyl)acetami de **84b** (0.45 g) with a yield of 82.45%.

MS m/z (ESI): 847.8 [M+H]

### Step 3

### 2-(6-(4-(5-(Benzyloxy)-6-methylpyrimidine-4-carbonyl)piperazin-1-yl)-5-ethyl-7-oxo-2-(2,5,6,7-tetrahydrooxepin-3-yl)-[1,2,4]triazolo[1,5-a]pyrimidin-4(7H)-yl)-N-(2-chloro-4-(pentafluoro-λ⁶-s ulfaneyl)phenyl)acetamide

4,4,5,5-Tetramethyl-2-(2,5,6,7-tetrahydrooxepin-3-yl)-1,3,2-dioxaborolane **62a** (31.75 mg, 141.67 µmol), 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(pentafluoro-λ⁶-sulfaneyl)phenyl)acetami de **84b** (60 mg, 70.83 µmol),[1,1'-bis(diphenylphosphino)ferrocene]palladium(II) dichloride (5.25 mg, 7.08 µmol) and sodium carbonate (15.02 mg, 141.67 µmol) were dissolved in 1,4-dioxane (2 mL) and water (0.5 mL). The mixture was subject to argon replacement, and the mixture was heated to 80 °C, and the reaction was performed for 2 hours. Water (50 mL) was added to the reaction mixture, followed by extraction with dichloromethane (50 mL ×3). The combined organic phases were washed with saturated sodium chloride solution (30 mL×2), dried over anhydrous sodium sulfate, filtered, and concentrated under reduced pressure. The residue was purified by silica gel column chromatography (eluent: System B) to obtain 2-(6-(4-(5-(benzyloxy)-6-methylpyrimidine-4-carbonyl)piperazin-1-yl)-5-ethyl-7-oxo-2-(2,5,6,7-t etrahydrooxepin-3-yl)-[1,2,4]triazolo[1,5-a]pyrimidin-4(7H)-yl)-N-(2-chloro-4-(pentafluoro-λ⁶-s ulfaneyl)phenyl)acetamide **84c** (30 mg) with a yield of 49.00%.

MS m/z (ESI): 865.3 [M+H]

### Step 4

### N-(2-chloro-4-(pentafluoro-λ⁶-sulfaneyl)phenyl)-2-(5-ethyl-6-(4-(5-hydroxy-6-methylpyrimidine -4-carbonyl)piperazin-1-yl)-7-oxo-2-(2,5,6,7-tetrahydrooxepin-3-yl)-[1,2,4]triazolo[1,5-a]pyrimi din-4(7H)-yl)acetamide

2-(6-(4-(5-(Benzyloxy)-6-methylpyrimidine-4-carbonyl)piperazin-1-yl)-5-ethyl-7-oxo-2 -(2,5,6,7-tetrahydrooxepin-3-yl)-[1,2,4]triazolo[1,5-a]pyrimidin-4(7H)-yl)-N-(2-chloro-4-(pentafl uoro-λ⁶-sulfaneyl)phenyl)acetamide **84c** (30 mg, 34.71 µmol) was dissolved in trifluoroacetic acid (3 mL), and the mixture was heated to 45 °C, and the reaction was performed for 3 hours. The mixture was concentrated under reduced pressure, and the obtained residue was separated by preparative liquid chromatography (separation column AKZONOBEL Kromasil; 250×21.2 mm I.D.; 5 µm, 20 mL/min; mobile phase A: 0.05% TFA + H₂O, mobile phase B: CH₃CN) to obtain N-(2-chloro-4-(pentafluoro-λ⁶-sulfaneyl)phenyl)-2-(5-ethyl-6-(4-(5-hydroxy-6-methylpyrimidine -4-carbonyl)piperazin-1-yl)-7-oxo-2-(2,5,6,7-tetrahydrooxepin-3-yl)-[1,2,4]triazolo[1,5-a]pyrimi din-4(7H)-yl)acetamide **84** (10 mg) with a yield of 35.73%.

MS m/z (ESI): 773.9 [M+H]
¹H NMR (400 MHz, DMSO-d6) δ 10.40 (s, 1H), 10.23 (s, 1H), 8.58 (s, 1H), 8.15 (d, J = 2.6 Hz, 1H), 8.10 (d, J = 9.1 Hz, 1H), 7.90 (dd, J = 9.2, 2.6 Hz, 1H), 7.05 (t, J = 5.8 Hz, 1H), 5.32 (s, 2H), 4.63 (s, 2H), 4.52 (d, J = 12.5 Hz, 1H), 4.17 (s, 9H), 3.84 (t, J = 5.7 Hz, 2H), 3.49 (h, J = 9.3 Hz, 3H), 3.30 - 3.22 (m, 1H), 3.04 - 2.93 (m, 3H), 2.81 (d, J = 11.3 Hz, 1H), 2.63 (d, J = 11.2 Hz, 1H), 2.44 (d, J = 11.2 Hz, 2H), 2.44 (s, 3H), 1.82 (p, J = 5.6 Hz, 2H), 1.18 (t, J = 7.4 Hz, 3H).

### Example 85

### 2-(2-(Benzofuran-5-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(pentafluoro-λ⁶-sulfaneyl)phenyl) acetamide

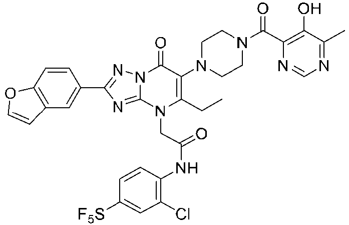

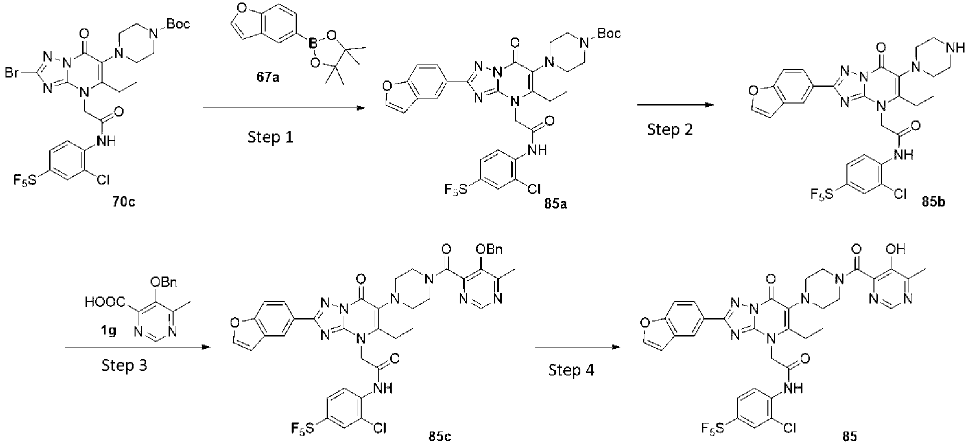

### Step 1

### tert-butyl 4-(2-(benzofuran-5-yl)-4-(2-((2-chloro-4-(pentafluoro-λ⁶-sulfaneyl)phenyl)amino)-2-oxoethyl)-5-ethyl-7-oxo-4,7-dihydro-[1,2,4]triazolo[1,5-a]pyrimidin-6-yl)piperazine-1-carboxylate

2-(Benzofuran-5-yl)-4,4,5,5-tetramethyl-1,3,2-dioxaborolane 67a (40.63 mg, 166.45 µmol), tert-butyl 4-(2-bromo-4-(2-((2-chloro-4-(pentafluoro-λ⁶-sulfaneyl)phenyl)amino)-2-oxoethyl)-5-ethyl-7-ox 0-4,7-dihydro-[1,2,4]triazolo[1,5-a]pyrimidin-6-yl)piperazine-1-carboxylate **70c** (80 mg, 110.97 µmol), [1,1'-bis(diphenylphosphino)ferrocene]palladium(II) dichloride (8.23 mg, 11.10 µmol) and sodium carbonate (23.52 mg, 221.93 µmol) were dissolved in 1,4-dioxane (2 mL) and water (0.5 mL). After The mixture was subject to argon replacement, the reaction was performed at 80 °C for 2 hours. Water (50 mL) was added, followed by extraction with dichloromethane (50 mL×3). The combined organic phases were washed with saturated sodium chloride solution (30 mL×2), dried over anhydrous sodium sulfate, filtered, and concentrated under reduced pressure. The residue was purified by silica gel column chromatography (eluent: System B) to obtain tert-butyl 4-(2-(benzofuran-5-yl)-4-(2-((2-chloro-4-(pentafluoro-λ⁶-sulfaneyl)phenyl)amino)-2-oxoethyl)-5-ethyl-7-oxo-4,7-dihydro-[1,2,4]triazolo[1,5-a]pyrimidin-6-yl)piperazine-1-carboxylate **85a** (60 mg, 79.14 µmol, 71.32% yield).

MS m/z (ESI): 702.0 [M+H-56]

### Step 2

### 2-(2-(benzofuran-5-yl)-5-ethyl-7-oxo-6-(piperazin-1-yl)-[1,2,4]triazolo[1,5-a]pyrimidin-4(7H)-yl )-N-(2-chloro-4-(pentafluoro-λ⁶-sulfaneyl)phenyl)acetamide

Tert-butyl 4-(2-(benzofuran-5-yl)-4-(2-((2-chloro-4-(pentafluoro-λ⁶-sulfaneyl)phenyl) amino)-2-oxoethyl)-5-ethyl-7-oxo-4,7-dihydro-[1,2,4]triazolo[1,5-a]pyrimidin-6-yl)piperazine-1-carboxylate 85a (60 mg, 79.14 µmol) was dissolved in dichloromethane (3 mL), trifluoroacetic acid (1 g, 8.77 mmol) was added dropwise, and the reaction was performed at room temperature for 3 hours. The mixture was concentrated under reduced pressure to obtain 2-(2-(benzofuran-5-yl)-5-ethyl-7-oxo-6-(piperazin-1-yl)-[1,2,4]triazolo[1,5-a]pyrimidin-4(7H)-yl )-N-(2-chloro-4-(pentafluoro-λ⁶-sulfaneyl)phenyl)acetamide **85b** (40 mg), and the crude product was directly used in the next step.

MS m/z (ESI): 658.2 [M+H]

### Step 3

### 2-(2-(benzofuran-5-yl)-6-(4-(5-(benzyloxy)-6-methylpyrimidine-4-carbonyl)piperazin-1-yl)-5-eth yl-7-oxo-[1,2,4]triazolo[1,5-a]pyrimidin-4(7H)-yl)-N-(2-chloro-4-(pentafluoro-λ⁶-sulfaneyl)phen yl)acetamide

5-(Benzyloxy)-6-methylpyrimidine-4-carboxylic acid **1g** (26.72 mg, 109.42 µmol) was dissolved in N,N-dimethylformamide (2 mL), then 1-(3-dimethylaminopropyl)-3-ethylcarbodiimide hydrochloride (34.96 mg, 182.36 µmol), 1-hydroxybenzotriazole (24.64 mg, 182.36 µmol) and N,N-diisopropylethylamine (39.28 mg, 303.93 µmol) were added, and the reaction was performed for 15 minutes, followed by addition of 2-(2-(benzofuran-5-yl)-5-ethyl-7-oxo-6-(piperazin-1-yl)-[1,2,4]triazolo[1,5-a]pyrimidin-4(7H)-yl )-N-(2-chloro-4-(pentafluoro-λ⁶-sulfaneyl)phenyl)acetamide **85b** (40 mg, 60.79 µmol), and the reaction was performed at room temperature for 18 hours. Water (50 mL) was added, extraction was performed with dichloromethane (50 mL × 3), the combined organic phase was washed with saturated sodium chloride solution (30 mL × 2), dried over anhydrous sodium sulfate, filtered, and concentrated under reduced pressure, and the residue was purified by silica gel column chromatography (eluent: System B) to obtain 2-(2-(benzofuran-5-yl)-6-(4-(5-(benzyloxy)-6-methylpyrimidine-4-carbonyl)piperazin-1-yl)-5-eth yl-7-oxo-[1,2,4]triazolo[1,5-a]pyrimidin-4(7H)-yl)-N-(2-chloro-4-(pentafluoro-λ⁶-sulfaneyl)phen yl)acetamide **85c** (40 mg) with a yield of 74.42%.

MS m/z (ESI): 883.9 [M+H]

### Step 4

### 2-(2-(benzofuran-5-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(pentafluoro-λ⁶-sulfaneyl)phenyl) acetamide

2-(2-(Benzofuran-5-yl)-6-(4-(5-(benzyloxy)-6-methylpyrimidine-4-carbonyl)piperazin-1-yl)-5-ethyl-7-oxo-[1,2,4]triazolo[1,5-a]pyrimidin-4(7H)-yl)-N-(2-chloro-4-(pentafluoro-λ⁶-sulf aneyl)phenyl)acetamide **85c** (40 mg, 45.23 µmol) was dissolved in trifluoroacetic acid (3 mL), and the reaction was performed at 45 °C for 3 hours. The mixture was concentrated under reduced pressure, and the obtained residue was separated by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 2-(2-(benzofuran-5-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(pentafluoro-λ⁶-sulfaneyl)phenyl) acetamide 85 (10 mg) with a yield of 27.00%.

MS m/z (ESI): 793.9 [M+H]
¹H NMR (400 MHz, DMSO-d6) δ 10.48 (s, 1H), 10.24 (s, 1H), 8.59 (s, 1H), 8.42 (d, J = 1.7 Hz, 1H), 8.16 (d, J = 2.6 Hz, 1H), 8.14 - 8.06 (m, 2H), 8.08 (d, J = 2.4 Hz, 1H), 7.90 (dd, J = 9.2, 2.6 Hz, 1H), 7.73 (d, J = 8.7 Hz, 1H), 7.08 (d, J = 2.3 Hz, 1H), 5.43 (s, 2H), 4.54 (d, J = 12.4 Hz, 1H), 3.61 - 3.48 (m, 3H), 3.26 (d, J = 12.1 Hz, 1H), 3.06 - 2.99 (m, 3H), 2.85 (d, J = 11.3 Hz, 1H), 2.67 (d, J = 10.7 Hz, 1H), 2.45 (s, 3H), 2.07 (s, 1H), 1.22 (t, J = 7.5 Hz, 3H).

### Example 86

### 2-(2-(2,3-dihydrobenzofuran-5-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(4-(pentafluoro-λ⁶-sulfaneyl)phenyl )acetamide

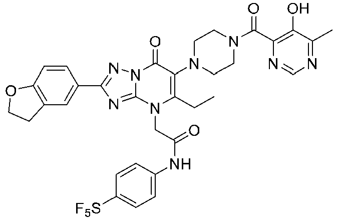

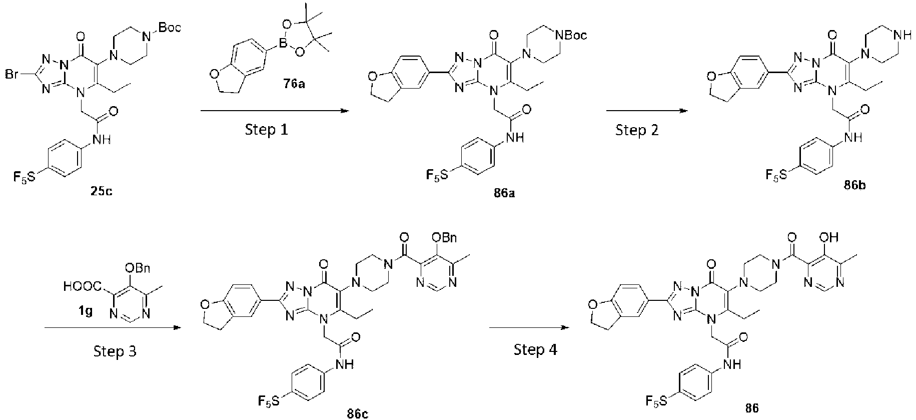

### Step 1

### tert-butyl 4-(2-(2,3-dihydrobenzofuran-5-yl)-5-ethyl-7-oxo-4-(2-oxo-2-((4-(pentafluoro-λ⁶-sulfaneyl)pheny l)amino)ethyl)-4,7-dihydro-[1,2,4]triazolo[1,5-a]pyrimidin-6-yl)piperazine-1-carboxylate

2-(2,3-Dihydrobenzofuran-5-yl)-4,4,5,5-tetramethyl-1,3,2-dioxaborolane **76a** (28.66 mg, 174.80 µmol), tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)piperazine-1-carboxylate **25c** (80 mg, 116.53 µmol), [1,1'-bis(diphenylphosphino)ferrocene]palladium dichloride (8.64 mg, 11.65 µmol) and sodium carbonate (24.70 mg, 233.07 µmol) were dissolved in 1,4-dioxane (2 mL) and water (0.5 mL), The mixture was subject to argon replacement three times, the temperature was raised to 80 °C, and the reaction was performed for 2 hours. Water (50 mL) was added to the reaction mixture, extraction was performed with dichloromethane (50 mL × 3), the combined organic phase was washed with saturated sodium chloride solution (30 mL × 2), dried over anhydrous sodium sulfate, filtered, and concentrated under reduced pressure, and the residue was purified by silica gel column chromatography (eluent: System B) to obtain tert-butyl 4-(2-(2,3-dihydrobenzofuran-5-yl)-5-ethyl-7-oxo-4-(2-oxo-2-((4-(pentafluoro-λ⁶-sulfaneyl)pheny l)amino)ethyl)-4,7-dihydro-[1,2,4]triazolo[1,5-a]pyrimidin-6-yl)piperazine-1-carboxylate 86a (35 mg) with a yield of 41.38%.

MS m/z (ESI): 670 [M+H-56]

### Step 2

### 2-(2-(2,3-dihydrobenzofuran-5-yl)-5-ethyl-7-oxo-6-(piperazin-1-yl)-[1,2,4]triazolo[1,5-a]pyrimid in-4(7H)-yl)-N-(4-(pentafluoro-λ⁶-sulfaneyl)phenyl)acetamide

Tert-butyl 4-(4-(2-((4-(pentafluoro-λ⁶-sulfaneyl)phenyl)amino)-2-oxoethyl)-2-(2,3-dihydrobenzofuran-5-yl) -5-ethyl-7-oxo-4,7-dihydro-[1,2,4]triazolo[1,5-a]pyrimidin-6-yl)piperazine-1-carboxylate **86a** (35 mg, 48.23 µmol) was dissolved in dichloromethane (3 mL), and trifluoroacetic acid (1 g, 8.77 mmol) was added dropwise. The reaction was performed at room temperature for 2 hours. After the reaction was completed, the mixture was concentrated under reduced pressure to obtain 2-(2-(2,3-dihydrobenzofuran-5-yl)-5-ethyl-7-oxo-6-(piperazin-1-yl)-[1,2,4]triazolo[1,5-a]pyrimid in-4(7H)-yl)-N-(4-(pentafluoro-λ⁶-sulfaneyl)phenyl)acetamide **86b** (25 mg), and the crude product was directly used in the next step.

MS m/z (ESI): 626.0 [M+H]

### Step 3

### 2-(6-(4-(5-(benzyloxy)-6-methylpyrimidine-4-carbonyl)piperazin-1-yl)-2-(2,3-dihydrobenzofuran -5-yl)-5-ethyl-7-oxo-[1,2,4]triazolo[1,5-a]pyrimidin-4(7H)-yl)-N-(4-(pentafluoro-λ⁶-sulfaneyl)ph enyl)acetamide

5-(Benzyloxy)-6-methylpyrimidine-4-carboxylic acid 1g (17.57 mg, 71.93 µmol) was dissolved in N,N-dimethylformamide (1 mL), followed by addition of 1-(3-dimethylaminopropyl)-3-ethylcarbodiimide hydrochloride (15.32 mg, 79.92 µmol), 1-hydroxybenzotriazole (10.80 mg, 79.92 µmol) and N,N-diisopropylethylamine (25.82 mg, 199.80 µmol). The reaction was performed at room temperature for 15 minutes, then 2-(2-(2,3-dihydrobenzofuran-5-yl)-5-ethyl-7-oxo-6-(piperazin-1-yl)-[1,2,4]triazolo[1,5-a]pyrimid in-4(7H)-yl)-N-(4-(pentafluoro-λ⁶-sulfaneyl) phenyl)acetamide **86b** (25 mg, 39.96 µmol) was added. The reaction was performed at room temperature for 18 hours. H₂O (50 mL) was added, and the mixture was extracted with dichloromethane (50 mL × 3). The combined organic phases were washed with saturated sodium chloride solution (30 mL × 2), dried over anhydrous sodium sulfate, filtered, and concentrated under reduced pressure. The residue was purified by silica gel column chromatography (eluent: System B) to obtain 2-(6-(4-(5-(benzyloxy)-6-methylpyrimidine-4-carbonyl)piperazin-1-yl)-2-(2,3-dihydrobenzofuran -5-yl)-5-ethyl-7-oxo-[1,2,4]triazolo[1,5-a]pyrimidin-4(7H)-yl)-N-(4-(pentafluoro-λ⁶-sulfaneyl)ph enyl)acetamide **86c** (25 mg) with a yield of 73.44%.

MS m/z (ESI): 852.0 [M+H]

### Step 4

### 2-(2-(2,3-dihydrobenzofuran-5-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(4-(pentafluoro-λ⁶-sulfaneyl)phenyl )acetamide

2-(6-(4-(5-(Benzyloxy)-6-methylpyrimidine-4-carbonyl)piperazin-1-yl)-2-(2,3-dihydrob enzofuran-5-yl)-5-ethyl-7-oxo-[1,2,4]triazolo[1,5-a]pyrimidin-4(7H)-yl)-N-(4-(pentafluoro-λ⁶-sul faneyl)phenyl)acetamide **86c** (25 mg, 29.35 µmol) was dissolved in trifluoroacetic acid (2 mL), and the temperature was raised to 40 °C. The reaction was performed for 2.5 hours. The mixture was concentrated under reduced pressure, and the resulting residue was separated by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 2-(2-(2,3-dihydrobenzofuran-5-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(4-(pentafluoro-λ⁶-sulfaneyl)phenyl )acetamide **86** (10 mg) with a yield of 44.29%.

MS m/z (ESI): 762.0 [M+H]
¹H NMR (400 MHz, DMSO-d6) δ 11.02 (s, 1H), 10.24 (s, 1H), 8.59 (s, 1H), 7.94 (d, J = 1.7 Hz, 1H), 7.92 - 7.76 (m, 5H), 6.86 (d, J = 8.3 Hz, 1H), 5.26 (s, 2H), 4.57 (dt, J = 18.6, 10.5 Hz, 3H), 3.54 (q, J = 10.6, 9.8 Hz, 3H), 3.24 (t, J = 8.9 Hz, 3H), 3.02 (d, J = 13.2 Hz, 2H), 2.98 (s, 1H), 2.84 (d, J = 11.3 Hz, 1H), 2.67 (d, J = 10.7 Hz, 1H), 2.45 (s, 3H), 1.19 (t, J = 7.4 Hz, 3H).

### Example 87

### N-(2-chloro-4-(pentafluoro-λ⁶-sulfaneyl)phenyl)-2-(2-(2,3-dihydrobenzofuran-5-yl)-5-ethyl-6-(4-(5-hydroxy-6-methylpyrimidine-4-carbonyl)piperazin-1-yl)-7-oxo-[1,2,4]triazolo[1,5-a]pyrimidin -4(7H)-yl)acetamide

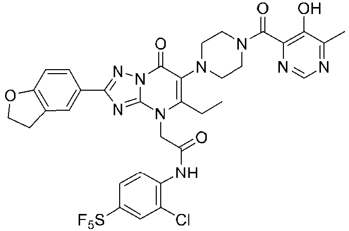

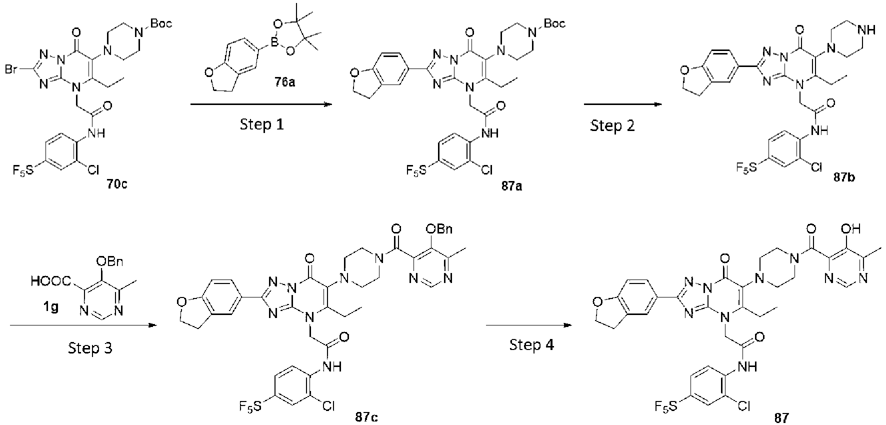

### Step 1

### tert-butyl 4-(4-(2-((2-chloro-4-(pentafluoro-λ⁶-sulfaneyl)phenyl)amino)-2-oxoethyl)-2-(2,3-dihydrobenzofu ran-5-yl)-5-ethyl-7-oxo-4,7-dihydro-[1,2,4]triazolo[1,5-a]pyrimidin-6-yl)piperazine-l-carboxylat e

2-(2,3-Dihydrobenzofuran-5-yl)-4,4,5,5-tetramethyl-1,3,2-dioxaborolane **76a** (28.66 mg, 174.77 µmol), tert-butyl 4-(2-bromo-4-(2-((2-chloro-4-(pentafluoro-λ⁶-sulfaneyl)phenyl)amino)-2-oxoethyl)-5-ethyl-7-ox 0-4,7-dihydro-[1,2,4]triazolo[1,5-a]pyrimidin-6-yl)piperazine-1-carboxylate **70c** (70 mg, 97.10 µmol), [1,1'-bis(diphenylphosphino)ferrocene]palladium(II) dichloride (7.20 mg, 9.71 µmol) and sodium carbonate (20.58 mg, 194.19 µmol) were dissolved in 1,4-dioxane (2 mL) and water (0.5 mL). The mixture was subject to argon replacement three times, then the temperature was raised to 85 °C. The reaction was performed for 2.5 hours. Water (50 mL) was added, and the mixture was extracted with dichloromethane (50 mL × 3). The combined organic phases were washed with saturated sodium chloride solution (30 mL × 2), dried over anhydrous sodium sulfate, filtered, and concentrated under reduced pressure. The residue was purified by silica gel column chromatography (eluent: System B) to obtain tert-butyl 4-(4-(2-((2-chloro-4-(pentafluoro-λ⁶-sulfaneyl)phenyl)amino)-2-oxoethyl)-2-(2,3-dihydrobenzofu ran-5-yl)-5-ethyl-7-oxo-4,7-dihydro-[1,2,4]triazolo[1,5-a]pyrimidin-6-yl)piperazine-1-carboxylat e **87a** (40 mg) with a yield of 54.19%.

MS m/z (ESI): 704.0 [M+H-56]

### Step 2

### N-(2-chloro-4-(pentafluoro-λ⁶-sulfaneyl)phenyl)-2-(2-(2,3-dihydrobenzofuran-5-yl)-5-ethyl-7-ox o-6-(piperazin-1-yl)-[1,2,4]triazolo[1,5-a]pyrimidin-4(7H)-yl)acetamide

Tert-butyl 4-(4-(2-((2-chloro-4-(pentafluoro-λ⁶-sulfaneyl)phenyl)amino)-2-oxoethyl)-2-(2,3-dihydrobenzofu ran-5-yl)-5-ethyl-7-oxo-4,7-dihydro-[1,2,4]triazolo[1,5-a]pyrimidin-6-yl)piperazine-1-carboxylat e 87a (40 mg, 52.62 µmol) was dissolved in dichloromethane (3 mL), and trifluoroacetic acid (1 g, 8.77 mmol) was added dropwise. The reaction was performed at room temperature for 3 h. The mixture was concentrated under reduced pressure to obtain N-(2-chloro-4-(pentafluoro-λ⁶-sulfaneyl)phenyl)-2-(2-(2,3-dihydrobenzofuran-5-yl)-5-ethyl-7-ox 0-6-(piperazin-1-yl)-[1,2,4]triazolo[1,5-a]pyrimidin-4(7H)-yl)acetamide **87b** (25 mg), and the crude product was directly used in the next step.

MS m/z (ESI): 660.0 [M+H]

### Step 3

### 2-(6-(4-(5-(benzyloxy)-6-methylpyrimidine-4-carbonyl)piperazin-1-yl)-2-(2,3-dihydrobenzofuran -5-yl)-5-ethyl-7-oxo-[1,2,4]triazolo[1,5-a]pyrimidin-4(7H)-yl)-N-(2-chloro-4-(pentafluoro-λ⁶-sulf aneyl)phenyl)acetamide

5-(Benzyloxy)-6-methylpyrimidine-4-carboxylic acid **1g** (18.50 mg, 75.75 µmol) was dissolved in N,N-dimethylformamide (1 mL), followed by addition of 1-(3-dimethylaminopropyl)-3-ethylcarbodiimide hydrochloride (14.52 mg, 75.75 µmol), 1-hydroxybenzotriazole (10.24 mg, 75.75 µmol), and N,N-diisopropylethylamine (14.69 mg, 113.63 µmol). The reaction was performed at room temperature for 10 min, then N-(2-chloro-4-(pentafluoro-1⁶-sulfaneyl)phenyl)-2-(2-(2,3-dihydrobenzofuran-5-yl)-5-ethyl-7-ox 0-6-(piperazin-1-yl)-[1,2,4]triazolo[1,5-a]pyrimidin-4(7H)-yl)acetamide **87b** (25 mg, 37.88 µmol) was added, and the reaction was performed at room temperature for 16 h. Water (50 mL) was added, and the mixture was extracted with dichloromethane (50 mL × 3). The combined organic phase was washed with saturated sodium chloride solution (30 mL × 2), dried over anhydrous sodium sulfate, filtered, and concentrated under reduced pressure. The residue was separated by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 2-(6-(4-(5-(benzyloxy)-6-methylpyrimidine-4-carbonyl)piperazin-1-yl)-2-(2,3-dihydrobenzofuran -5-yl)-5-ethyl-7-oxo-[1,2,4]triazolo[1,5-a]pyrimidin-4(7H)-yl)-N-(2-chloro-4-(pentafluoro-λ⁶-sulf aneyl)phenyl)acetamide **87c** (20 mg) with a yield of 59.58%.

MS m/z (ESI): 885.9 [M+H]

### Step 4

### N-(2-chloro-4-(pentafluoro-λ⁶-sulfaneyl)phenyl)-2-(2-(2,3-dihydrobenzofuran-5-yl)-5-ethyl-6-(4-(5-hydroxy-6-methylpyrimidine-4-carbonyl)piperazin-1-yl)-7-oxo-[1,2,4]triazolo[1,5-a]pyrimidin -4(7H)-yl)acetamide

2-(6-(4-(5-(Benzyloxy)-6-methylpyrimidine-4-carbonyl)piperazin-1-yl)-2-(2,3-dihydrob enzofuran-5-yl)-5-ethyl-7-oxo-[1,2,4]triazolo[1,5-a]pyrimidin-4(7H)-yl)-N-(2-chloro-4-(pentaflu oro-λ⁶ -sulfaneyl)phenyl)acetamide **87c** (20 mg, 22.57 µmol) was dissolved in trifluoroacetic acid (3 mL), and the temperature was raised to 45 °C. The reaction was performed for 3 h. After completion of the reaction, the mixture was concentrated under reduced pressure, and the resulting residue was separated by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(pentafluoro-λ⁶-sulfaneyl)phenyl)-2-(2-(2,3-dihydrobenzofuran-5-yl)-5-ethyl-6-(4-(5-hydroxy-6-methylpyrimidine-4-carbonyl)piperazin-1-yl)-7-oxo-[1,2,4]triazolo[1,5-a]pyrimidin -4(7H)-yl)acetamide **87** (10 mg) with a yield of 52.32%.

MS m/z (ESI): 795.9 [M+H]
¹H NMR (400 MHz, DMSO-d6) δ 10.48 (s, 1H), 10.25 (s, 1H), 8.58 (s, 1H), 8.16 (d, J = 2.6 Hz, 1H), 8.10 (d, J = 9.1 Hz, 1H), 7.97 (d, J = 1.7 Hz, 1H), 7.88 (ddd, J = 11.1, 8.9, 2.2 Hz, 2H), 6.88 (d, J = 8.4 Hz, 1H), 5.39 (s, 2H), 4.60 (t, J = 8.7 Hz, 2H), 4.54 (d, J = 12.7 Hz, 1H), 3.56 (d, J = 11.4 Hz, 1H), 3.51 (s, 3H), 3.25 (t, J = 8.6 Hz, 3H), 3.01 (s, 3H), 2.84 (d, J = 11.3 Hz, 1H), 2.66 (d, J = 10.7 Hz, 1H), 2.45 (s, 3H), 1.20 (t, J = 7.4 Hz, 3H).

### Example 88

### 2-(2-(2,3-dihydrobenzofuran-5-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(2-methyl-4-(pentafluoro-λ⁶-sulfan eyl)phenyl)acetamide

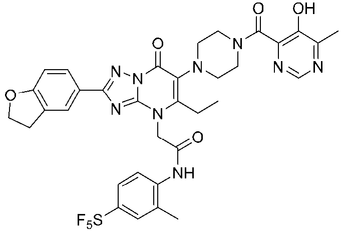

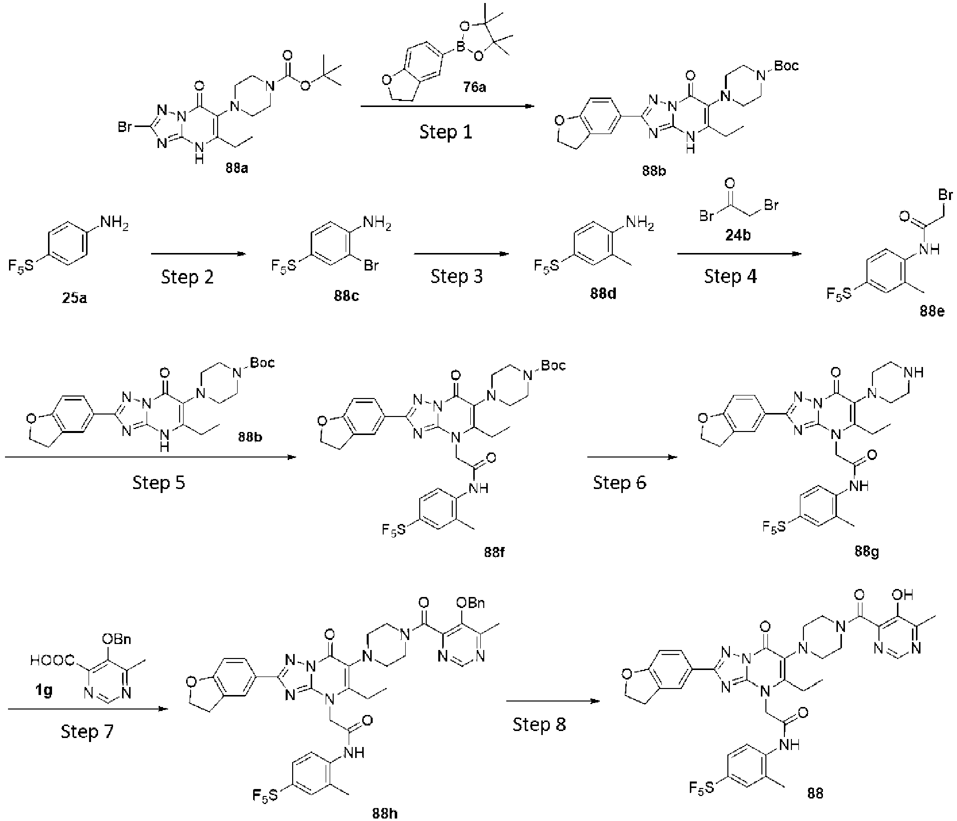

### Step 1

### tert-butyl 4-(2-(2,3-dihydrobenzofuran-5-yl)-5-ethyl-7-oxo-4,7-dihydro-[1,2,4]triazolo[1,5-a]pyrimidin-6-y 1)piperazine-1-carboxylate

2-(2,3-Dihydrobenzofuran-5-yl)-4,4,5,5-tetramethyl-1,3,2-dioxaborolane **76a** (1.07 g, 6.55 mmol), tert-butyl 4-(2-bromo-5-ethyl-7-oxo-4,7-dihydro-[1,2,4]triazolo[1,5-a]pyrimidin-6-yl)piperazine-1-carboxy late **88a** (2 g, 4.68 mmol, prepared according to published patent WO2022249060), [1,1'-bis(diphenylphosphino)ferrocene]palladium(II) dichloride (347.20 mg, 468.06 µmol), and sodium carbonate (992.19 mg, 9.36 mmol) were dissolved in 1,4-dioxane (20 mL). The reaction was heated to 110 °C with stirring for 18 h. Water (20 mL) was added to the reaction mixture, and the mixture was extracted with dichloromethane (50 mL × 3). The combined organic phase was washed with saturated sodium chloride solution (30 mL × 2), dried over anhydrous sodium sulfate, filtered, and concentrated under reduced pressure. The residue was purified by silica gel column chromatography (eluent: System B) to obtain tert-butyl 4-(2-(2,3-dihydrobenzofuran-5-yl)-5-ethyl-7-oxo-4,7-dihydro-[1,2,4]triazolo[1,5-a]pyrimidin-6-y 1) piperazine-1-carboxylate **88b** (1.4 g) with a yield of 64.11 %.

MS m/z (ESI): 466.4 [M+H]

### Step 2

### 2-bromo-4-(pentafluoro-λ⁶-sulfaneyl)aniline

4-(Pentafluorosulfaneyl)aniline **25a** (0.25 g, 1.14 mmol) was dissolved in dichloromethane (4 mL), and dibromohydantoin (179.37 mg, 627.35 µmol) was slowly added under an ice-water bath. The reaction was performed for 1 hour under a water bath. Saturated sodium bicarbonate solution (30 mL) was added, and the mixture was extracted with dichloromethane (50 mL×3). The combined organic phase was washed with saturated sodium chloride solution (30 mL×2), dried over anhydrous sodium sulfate, filtered, and concentrated under reduced pressure. The residue was purified by silica gel column chromatography (eluent: System B) to obtain 2-bromo-4-(pentafluoro-λ⁶-sulfaneyl)aniline **88c** (0.3 g) with a yield of 88.24%.

MS m/z (ESI): 297.8 [M+H]

### Step 3

### 2-methyl-4-(pentafluoro-λ⁶-sulfaneyl)aniline

2-Bromo-4-(pentafluoro-λ⁶-sulfaneyl)aniline **88c** (0.25 g, 838.73 µmol), tetrakis(triphenylphosphine)palladium (48.46 mg, 41.94 µmol), potassium carbonate (521.64 mg, 3.77 mmol), and trimethylboroxine (315.86 mg, 2.52 mmol) were dissolved in 1,4-dioxane (5 mL). After argon replacement, the temperature was raised to 90 °C and the reaction was performed for 8 hours. Water (20 mL) was added, and the mixture was extracted with dichloromethane (50 mL ×3). The combined organic phase was washed with saturated sodium chloride solution (30 mL×2), dried over anhydrous sodium sulfate, filtered, and concentrated under reduced pressure. The residue was purified by silica gel column chromatography (eluent: System B) to obtain 2-methyl-4-(pentafluoro-λ⁶-sulfaneyl)aniline **88d** (80 mg) with a yield of 40.90%.

MS m/z (ESI): 234.0 [M+H]

### Step 4

### 2-Bromo-N-(2-methyl-4-(pentafluoro-λ⁶-sulfaneyl)phenyl)acetamide

2-Methyl-4-(pentafluoro-λ⁶-sulfaneyl)aniline **88d** (0.1 g, 428.81 µmol) was dissolved in dichloromethane (3 mL), followed by addition of 4-dimethylaminopyridine (57.63 mg, 471.69 µmol). 2-Bromoacetyl bromide **24b** (129.83 mg, 643.22 µmol) was slowly added dropwise, and the reaction was performed for 4 hours at room temperature. The reaction mixture was concentrated under reduced pressure, and the residue was purified by silica gel column chromatography (eluent: System A) to obtain 2-bromo-N-(2-methyl-4-(pentafluoro-λ⁶-sulfaneyl)phenyl)acetamide **88e** (120 mg) with a yield of 79.02%.

MS m/z (ESI): 353.9 [M+H]

### Step 5

### tert-butyl 4-(2-(2,3-dihydrobenzofuran-5-yl)-5-ethyl-4-(2-((2-methyl-4-(pentafluoro-λ⁶-sulfaneyl)phenyl)a mino)-2-oxoethyl)-7-oxo-4,7-dihydro-[1,2,4]triazolo[1,5-a]pyrimidin-6-yl)piperazine-1-carboxyl ate

Tert-butyl 4-(2-(2,3-dihydrobenzofuran-5-yl)-5-ethyl-7-oxo-4,7-dihydro-[1,2,4]triazolo [1,5-a]pyrimidin-6-yl)piperazine-1-carboxylate **88b** (70 mg, 150.04 µmol) was dissolved in N,N-dimethylformamide (2 mL), followed by addition of N,N-diisopropylethylamine (77.57 mg, 600.17 µmol) and 2-bromo-N-(2-methyl-4-(pentafluoro-λ⁶-sulfaneyl)phenyl)acetamide **88e** (63.76 mg, 180.05 µmol). The temperature was raised to 50 °C and the reaction was performed for 2.5 hours. Water (20 mL) was added, and the mixture was extracted with dichloromethane (50 mL ×3).The combined organic phase was washed with saturated sodium chloride solution (30 mL ×2), dried over anhydrous sodium sulfate, filtered, and concentrated under reduced pressure. The residue was purified by silica gel column chromatography (eluent: System B) to obtain tert-butyl 4-(2-(2,3-dihydrobenzofuran-5-yl)-5-ethyl-4-(2-((2-methyl-4-(pentafluoro-λ⁶-sulfaneyl)phenyl)a mino)-2-oxoethyl)-7-oxo-4,7-dihydro-[1,2,4]triazolo[1,5-a]pyrimidin-6-yl)piperazine-1-carboxyl ate **88f** (80 mg) with a yield of 72.08%.

MS m/z (ESI): 740.0 [M+H]

### Step 6

### 2-(2-(2,3-Dihydrobenzofuran-5-yl)-5-ethyl-7-oxo-6-(piperazin-1-yl)-[1,2,4]triazolo[1,5-a]pyrimi din-4(7H)-yl)-N-(2-methyl-4-(pentafluoro-λ⁶-sulfaneyl)phenyl)acetamide

Tert-butyl 4-(2-(2,3-dihydrobenzofuran-5-yl)-5-ethyl-4-(2-((2-methyl-4-(pentafluoro-λ⁶-sulfaneyl)phenyl)a mino)-2-oxoethyl)-7-oxo-4,7-dihydro-[1,2,4]triazolo[1,5-a]pyrimidin-6-yl)piperazine-1-carboxyl ate **88f** (80 mg, 108.14 µmol) was dissolved in dichloromethane (3 mL). Trifluoroacetic acid (1 g, 8.77 mmol) was added dropwise, and the reaction was performed for 2 hours at room temperature. The mixture was concentrated under reduced pressure to obtain 2-(2-(2,3-dihydrobenzofuran-5-yl)-5-ethyl-7-oxo-6-(piperazin-1-yl)-[1,2,4]triazolo[1,5-a]pyrimid in-4(7H)-yl)-N-(2-methyl-4-(pentafluoro-λ⁶-sulfaneyl)phenyl)acetamide **88g** (50 mg), which was used directly in the next step as a crude product.

MS m/z (ESI): 640.0 [M+H]

### Step 7

### 2-(6-(4-(5-(Benzyloxy)-6-methylpyrimidine-4-carbonyl)piperazin-1-yl)-2-(2,3-dihydrobenzofura n-5-yl)-5-ethyl-7-oxo-[1,2,4]triazolo[1,5-a]pyrimidin-4(7H)-yl)-N-(2-methyl-4-(pentafluoro-λ⁶-s ulfaneyl)phenyl)acetamide

5-(Benzyloxy)-6-methylpyrimidine-4-carboxylic acid **1g** (34.37 mg, 140.70 µmol) was dissolved in N,N-dimethylformamide (2 mL), followed by addition of 1-(3-dimethylaminopropyl)-3-ethylcarbodiimide hydrochloride (44.96 mg, 234.51 µmol), 1-hydroxybenzotriazole (31.69 mg, 234.51 µmol), and N,N-diisopropylethylamine (50.51 mg, 390.85 µmol). The reaction was performed for 15 minutes at room temperature, followed by addition of 2-(2-(2,3-dihydrobenzofuran-5-yl)-5-ethyl-7-oxo-6-(piperazin-1-yl)-[1,2,4]triazolo[1,5-a]pyrimid in-4(7H)-yl)-N-(2-methyl-4-(pentafluoro-λ⁶-sulfaneyl)phenyl)acetamide **88g** (50 mg, 78.17 µmol). The reaction was performed for 18 hours at room temperature. Water (20 mL) was added, and the mixture was extracted with dichloromethane (50 mL ×3). The combined organic phase was washed with saturated sodium chloride solution (30 mL ×2), dried over anhydrous sodium sulfate, filtered, and concentrated under reduced pressure. The residue was purified by silica gel column chromatography (eluent: System B) to obtain 2-(6-(4-(5-(benzyloxy)-6-methylpyrimidine-4-carbonyl)piperazin-1-yl)-2-(2,3-dihydrobenzofuran -5-yl)-5-ethyl-7-oxo-[1,2,4]triazolo[1,5-a]pyrimidin-4(7H)-yl)-N-(2-methyl-4-(pentafluoro-λ⁶-sul faneyl)phenyl)acetamide **88h** (50 mg) with a yield of 73.87%.

MS m/z (ESI): 866.0 [M+H]

### Step 8

### 2-(2-(2,3-Dihydrobenzofuran-5-yl)-5-ethyl-6-(4-(5-hydroxy-6-methylpyrimidine-4-carbonyl)pipe razin-1-yl)-7-oxo-[1,2,4]triazolo[1,5-a]pyrimidin-4(7H)-yl)-N-(2-methyl-4-(pentafluoro-λ⁶-sulfan eyl)phenyl) acetamide

2-(6-(4-(5-(Benzyloxy)-6-methylpyrimidine-4-carbonyl)piperazin-1-yl)-2-(2,3-dihydrob enzofuran-5-yl)-5-ethyl-7-oxo-[1,2,4]triazolo[1,5-a]pyrimidin-4(7H)-yl)-N-(2-methyl-4-(pentaflu oro-λ⁶-sulfaneyl)phenyl)acetamide **88h** (50 mg, 57.75 µmol) was dissolved in trifluoroacetic acid (3 mL), and the reaction was performed at 45 °C for 3 h. The mixture was concentrated under reduced pressure, and the resulting residue was separated by preparative liquid chromatography (separation column AKZONOBEL Kromasil; 250 ×21.2 mm I.D.; 5 µm, 20 mL/min; mobile phase A: 0.05% TFA + H₂O, mobile phase B: CH₃CN) to obtain 2-(2-(2,3-dihydroxybenzofuran-5-yl)-5-ethyl-6-(4-(5-hydroxy-6-methylpyrimidine-4-carbonyl)pi perazin-1-yl)-7-oxo-[1,2,4]triazolo[1,5-a]pyrimidin-4(7H)-yl)-N-(2-methyl-4-(pentafluoro-λ⁶-sulf aneyl)phenyl)acetamide **88** (10 mg) with a yield of 21.21%.

MS m/z (ESI): 776.0 [M+H]
¹H NMR (400 MHz, DMSO-d6) δ 10.24 (s, 1H), 10.09 (s, 1H), 8.58 (s, 1H), 7.97 (d, J = 1.7 Hz, 1H), 7.87 (dd, J = 8.3, 1.8 Hz, 1H), 7.82 (d, J = 2.6 Hz, 1H), 7.76 (d, J = 9.0 Hz, 1H), 7.70 (dd, J = 9.0, 2.7 Hz, 1H), 6.88 (d, J = 8.4 Hz, 1H), 5.32 (s, 2H), 4.61 (t, J = 8.7 Hz, 2H), 4.54 (d, J = 12.4 Hz, 1H), 3.56 (d, J = 12.1 Hz, 3H), 3.29 (d, J = 9.0 Hz, 1H), 3.24 (d, J = 8.9 Hz, 2H), 3.02 (dd, J = 11.3, 4.8 Hz, 3H), 2.84 (d, J = 11.3 Hz, 1H), 2.66 (d, J = 11.5 Hz, 1H), 2.45 (s, 3H), 2.40 (s, 3H), 1.21 (t, J = 7.4 Hz, 3H).

### Example 89

### 2-(2-(2,3-Dihydrobenzofuran-5-yl)-5-ethyl-6-(4-(5-hydroxy-6-methylpyrimidine-4-carbonyl)pipe razin-1-yl)-7-oxo-[1,2,4]triazolo[1,5-a]pyrimidin-4(7H)-yl)-N-(2-fluoro-4-((trifluoromethyl)thio) phenyl)acetamide

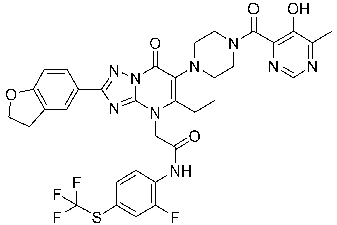

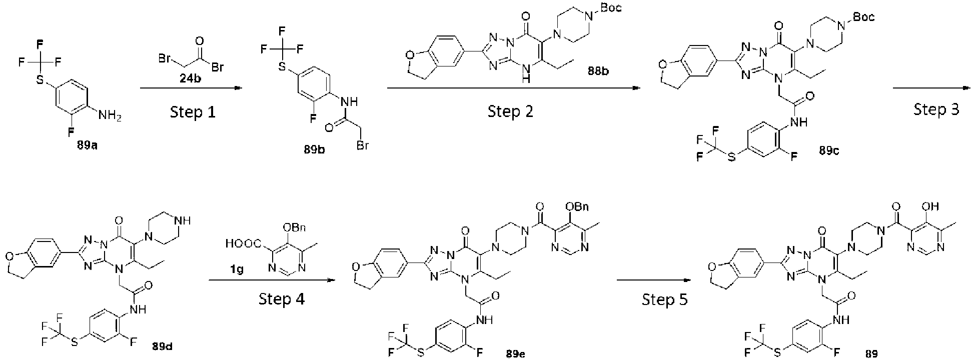

### Step 1

### 2-Bromo-N-(2-fluoro-4-((trifluoromethyl)thio)phenyl)acetamide

2-Fluoro-4-((trifluoromethyl)thio)aniline **89a** (0.2 g, 947.06 µmol, commercially available) was dissolved in dichloromethane (5 mL). 4-Dimethylaminopyridine (127.27 mg, 1.04 mmol) was added under ice bath, and the mixture was stirred for 10 min. 2-Bromoacetyl bromide **24b** (229.39 mg, 1.14 mmol, 98.71 µL) was slowly added, and the reaction was performed at room temperature for 18 h. The reaction mixture was concentrated under reduced pressure, and the residue was purified by silica gel column chromatography (eluent: System A) to obtain 2-bromo-N-(2-fluoro-4-((trifluoromethyl)thio)phenyl)acetamide **89b** (0.2 g) with a yield of 63.59%.

MS m/z (ESI): 331.8 [M+H]

### Step 2

### tert-butyl 4-(2-(2,3-dihydrobenzofuran-5-yl)-5-ethyl-4-(2-((2-fluoro-4-((trifluoromethyl)thio)phenyl)amino )-2-oxoethyl)-7-oxo-4,7-dihydro-[1,2,4]triazolo[1,5-a]pyrimidin-6-yl)piperazine-1-carboxylate

Tert-butyl 4-(2-(2,3-dihydrobenzofuran-5-yl)-5-ethyl-7-oxo-4,7-dihydro-[1,2,4] triazolo[1,5-a]pyrimidin-6-yl)piperazine-1-carboxylate **88b** (100 mg, 214.35 µmol) was dissolved in N,N-dimethylformamide (2 mL). N,N-Diisopropylethylamine (110.81 mg, 857.39 µmol) and 2-bromo-N-(2-fluoro-4-((trifluoromethyl)thio)phenyl)acetamide **89b** (92.54 mg, 278.65 µmol) were added, and the reaction was performed at 50 °C for 2.5 h. Water (20 mL) was added, and the mixture was extracted with dichloromethane (50 mL × 3). The combined organic phases were washed with saturated sodium chloride solution (30 mL × 2), dried over anhydrous sodium sulfate, filtered, and concentrated under reduced pressure. The residue was purified by silica gel column chromatography (eluent: System B) to obtain tert-butyl 4-(2-(2,3-dihydrobenzofuran-5-yl)-5-ethyl-4-(2-((2-fluoro-4-((trifluoromethyl)thio)phenyl)amino )-2-oxoethyl)-7-oxo-4,7-dihydro-[1,2,4]triazolo[1,5-a]pyrimidin-6-yl)piperazine-1-carboxylate 89c (70 mg) with a yield of 45.50%.

MS m/z (ESI): 662.0 [M+H]

### Step 3

### 2-(2-(2,3-Dihydrobenzofuran-5-yl)-5-ethyl-7-oxo-6-(piperazin-1-yl)-[1,2,4]triazolo[1,5-a]pyrimi din-4(7H)-yl)-N-(2-fluoro-4-((trifluoromethyl)thio)phenyl)acetamide

Tert-butyl 4-(2-(2,3-dihydrobenzofuran-5-yl)-5-ethyl-4-(2-((2-fluoro-4-((trifluoromethyl) thio)phenyl)amino)-2-oxoethyl)-7-oxo-4,7-dihydro-[1,2,4]triazolo[1,5-a]pyrimidin-6-yl) piperazine-1-carboxylate **89c** (70 mg, 97.53 µmol) was dissolved in dichloromethane (3 mL). Trifluoroacetic acid (3 g, 26.31 mmol) was added dropwise, and the reaction was performed at room temperature for 2 h. After completion of the reaction, the mixture was concentrated under reduced pressure to obtain 2-(2-(2,3-dihydrobenzofuran-5-yl)-5-ethyl-7-oxo-6-(piperazin-1-yl)-[1,2,4]triazolo[1,5-a]pyrimid in-4(7H)-yl)-N-(2-fluoro-4-((trifluoromethyl)thio)phenyl)acetamide **89d** (50 mg). The crude product was used directly in the next step.

MS m/z (ESI): 618.0 [M+H]

### Step 4

### 2-(6-(4-(5-(Benzyloxy)-6-methylpyrimidine-4-carbonyl)piperazin-1-yl)-2-(2,3-dihydrobenzofura n-5-yl)-5-ethyl-7-oxo-[1,2,4]triazolo[1,5-a]pyrimidin-4(7H)-yl)-N-(2-fluoro-4-((trifluoromethyl)t hio)phenyl)acetamide

5-(Benzyloxy)-6-methylpyrimidine-4-carboxylic acid **1g** (35.59 mg, 145.72 µmol) was dissolved in N,N-dimethylformamide (2 mL). 1-(3-Dimethylaminopropyl)-3-ethylcarbodiimide hydrochloride (46.56 mg, 242.87 µmol), 1-hydroxybenzotriazole (32.82 mg, 242.87 µmol), and N,N-diisopropylethylamine (52.31 mg, 404.78 µmol) were added, and the reaction was performed for 15 min. 2-(2-(2,3-Dihydrobenzofuran-5-yl)-5-ethyl-7-oxo-6-(piperazin-1-yl)-[1,2,4]triazolo[1,5-a]pyrimi din-4(7H)-yl)-N-(2-fluoro-4-((trifluoromethyl)thio)phenyl)acetamide **89d** (50 mg, 80.96 µmol) was added, and the reaction was performed at room temperature for 18 h. Water (20 mL) was added, and the mixture was extracted with dichloromethane (50 mL × 3). The combined organic phases were washed with saturated sodium chloride solution (30 mL × 2), dried over anhydrous sodium sulfate, filtered, and concentrated under reduced pressure. The residue was purified by silica gel column chromatography (eluent: System B) to obtain 2-(6-(4-(5-(benzyloxy)-6-methylpyrimidine-4-carbonyl)piperazin-1-yl)-2-(2,3-dihydrobenzofuran -5-yl)-5-ethyl-7-oxo-[1,2,4]triazolo[1,5-a]pyrimidin-4(7H)-yl)-N-(2-fluoro-4-((trifluoromethyl)th io) phenyl)acetamide **89e** (60 mg) with a yield of 87.83%.

MS m/z (ESI): 844.0 [M+H]

### Step 5

### 2-(2-(2,3-dihydrobenzofuran-5-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(2-fluoro-4-

2-(6-(4-(5-(Benzyloxy)-6-methylpyrimidine-4-carbonyl)piperazin-1-yl)-2-(2,3-dihydrob enzofuran-5-yl)-5-ethyl-7-oxo-[1,2,4]triazolo[1,5-a]pyrimidin-4(7H)-yl)-N-(2-fluoro-4-((trifluoro methyl)thio)phenyl)acetamide **89e** (30 mg, 35.55 µmol) was dissolved in trifluoroacetic acid (3 mL), the temperature was raised to 45 °C and the reaction was performed for 3 hours. The mixture was concentrated under reduced pressure, and the resulting residue was separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain 2-(2-(2,3-dihydroxybenzofuran-5-yl)-5-ethyl-6-(4-(5-hydroxy-6-methylpyrimidine-4-carbonyl) piperazin-1-yl)-7-oxo-[1,2,4]triazolo[1,5-a]pyrimidin-4(7H)-yl)-N-(2-fluoro-4-((trifluoromethyl) thio)phenyl)acetamide **89** (8 mg) in a yield of 26.87%.

MS m/z (ESI): 754.0 [M+H]
¹H NMR (400 MHz, DMSO-d6) δ 10.69 (s, 1H), 10.24 (s, 1H), 8.59 (s, 1H), 8.16 (t, J = 8.4 Hz, 1H), 7.95 (d, J = 1.7 Hz, 1H), 7.86 (dd, J = 8.4, 1.9 Hz, 1H), 7.77 (dd, J = 10.5, 2.1 Hz, 1H), 7.54 (dd, J = 8.6, 2.1 Hz, 1H), 6.87 (d, J = 8.3 Hz, 1H), 5.35 (s, 2H), 4.60 (t, J = 8.7 Hz, 2H), 4.54 (d, J = 12.4 Hz, 1H), 3.53 (td, J = 12.3, 11.4, 6.8 Hz, 3H), 3.26 (q, J = 8.9 Hz, 3H), 3.06 - 2.93 (m, 3H), 2.84 (d, J = 11.1 Hz, 1H), 2.70 - 2.63 (m, 1H), 2.45 (s, 3H), 1.19 (t, J = 7.5 Hz, 3H).

### Example 90

### 2-(2-(benzofuran-6-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(4-((trifluoromethyl)thio)phenyl)acetamide

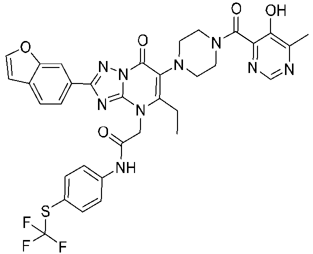

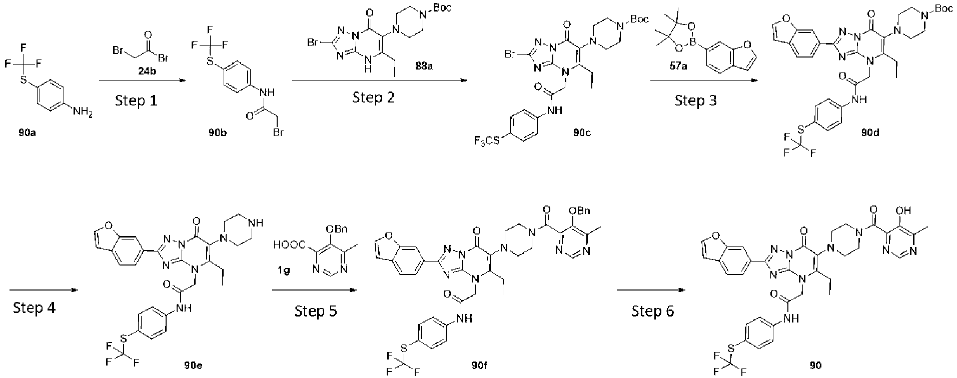

### Step 1

### 2-bromo-N-(4-((trifluoromethyl)thio)phenyl)acetamide

2-Bromoacetyl bromide **24b** (626.88 mg, 3.11 mmol, 269.74 µL) was dissolved in dichloromethane (8 mL), 4-dimethylaminopyridine (347.81 mg, 2.85 mmol) was added under an ice bath, and the mixture was stirred for 10 minutes, followed by slow addition of 4-((trifluoromethyl)thio)aniline **90a** (0.5 g, 2.59 mmol, commercially available). The reaction was performed at room temperature for 12 hours. The mixture was concentrated under reduced pressure, and the residue was purified by silica gel column chromatography (eluent: System A) to obtain 2-bromo-N-(4-((trifluoromethyl)thio)phenyl)acetamide **90b** (0.5 g) in a yield of 61.50%.

MS m/z (ESI): 315.9 [M+H]

### Step 2

### tert-butyl 4-(2-bromo-5-ethyl-7-oxo-4-(2-oxo-2-((4-((trifluoromethyl)thio)phenyl)amino)ethyl)-4,7-dihydro -[1,2,4]triazolo[1,5-a]pyrimidin-6-yl)piperazine-1-carboxylate

Tert-butyl 4-(2-bromo-5-ethyl-7-oxo-4,7-dihydro-[1,2,4]triazolo[1,5-a]pyrimidin-6-yl)piperazine-l-carboxy late **88a** (501.16 mg, 1.17 mmol) was dissolved in N,N-dimethylformamide (6 mL), followed by addition of N,N-diisopropylethylamine (454.74 mg, 3.52 mmol), then 2-bromo-N-(4-((trifluoromethyl)thio)phenyl)acetamide **90b** (405.26 mg, 1.29 mmol) was added, the temperature was raised to 50 °C and the mixture was stirred for 5 hours. Water (20 mL) was added, the mixture was extracted with dichloromethane (50 mL×3), the combined organic phases were washed with saturated sodium chloride solution (30 mL×2), dried over anhydrous sodium sulfate, filtered, and concentrated under reduced pressure, and the residue was purified by silica gel column chromatography (eluent: System B) to obtain tert-butyl 4-(2-bromo-5-ethyl-7-oxo-4-(2-oxo-2-((4-((trifluoromethyl)thio)phenyl)amino)ethyl)-4,7-dihydro -[1,2,4]triazolo[1,5-a]pyrimidin-6-yl)piperazine-1-carboxylate **90c** (0.5 g) in a yield of 64.54%.

MS m/z (ESI): 604.0 [M+H-56]

### Step 3

### tert-butyl 4-(2-(benzofuran-6-yl)-5-ethyl-7-oxo-4-(2-oxo-2-((4-((trifluoromethyl)thio)phenyl)amino)ethyl)-4,7-dihydro-[1,2,4]triazolo[1,5-a]pyrimidin-6-yl)piperazine-1-carboxylate

2-(Benzofuran-6-yl)-4,4,5,5-tetramethyl-1,3,2-dioxaborolane **57a** (83.15 mg, 340.65 µmol), tert-butyl 4-(2-bromo-5-ethyl-7-oxo-4-(2-oxo-2-((4-((trifluoromethyl)thio) phenyl)amino)ethyl)-4,7-dihydro-[1,2,4]triazolo[1,5-a]pyrimidin-6-yl)piperazine-1-carboxylate **90c** (0.15 g, 227.10 µmol),[1,1'-bis(diphenylphosphino)ferrocene]palladium(II) dichloride dichloromethane complex (18.40 mg, 22.71 µmol), and sodium carbonate (48.14 mg, 454.20 µmol) were dissolved in 1,4-dioxane (2 mL) and water (0.5 mL), after three cycles of argon replacement, the temperature was raised to 80 °C and the reaction was performed for 3 hours. After completion of the reaction, water (20 mL) was added, the mixture was extracted with dichloromethane (50 mL ×3), the combined organic phases were washed with saturated sodium chloride solution (30 mL×2), dried over anhydrous sodium sulfate, filtered, and concentrated under reduced pressure, and the residue was purified by silica gel column chromatography (eluent: System B) to obtain tert-butyl 4-(2-(benzofuran-6-yl)-5-ethyl-7-oxo-4-(2-oxo-2-((4-((trifluoromethyl)thio)phenyl)amino) ethyl)-4,7-dihydro-[1,2,4]triazolo[1,5-a]pyrimidin-6-yl)piperazine-1-carboxylate **90d** (80 mg) in a yield of 58.95%.

MS m/z (ESI): 642.1 [M+H-56]

### Step 4

### 2-(2-(benzofuran-6-yl)-5-ethyl-7-oxo-6-(piperazin-1-yl)-[1,2,4]triazolo[1,5-a]pyrimidin-4(7H)-yl )-N-(4-((trifluoromethyl)thio)phenyl)acetamide

Tert-butyl 4-(2-(benzofuran-6-yl)-5-ethyl-7-oxo-4-(2-oxo-2-((4-((trifluoromethyl)thio) phenyl)amino)ethyl)-4,7-dihydro-[1,2,4]triazolo[1,5-a]pyrimidin-6-yl)piperazine-1-carboxylate 90d (80 mg, 114.66 µmol) was dissolved in dichloromethane (4 mL), trifluoroacetic acid (1 g, 8.77 mmol) was added dropwise, and the reaction was performed at room temperature for 4 h. The mixture was concentrated under reduced pressure, and the resulting residue was separated by preparative liquid chromatography (separation column AKZONOBEL Kromasil; 250×21.2 mm I.D.; 5µm, 20 mL/min; mobile phase A: 0.05% TFA+H₂O, mobile phase B: CH3CN) to obtain 2-(2-(benzofuran-6-yl)-5-ethyl-7-oxo-6-(piperazin-1-yl)-[1,2,4]triazolo[1,5-a]pyrimidin-4(7H)-yl )-N-(4-((trifluoromethyl)thio)phenyl)acetamide **90e** (50 mg) in a yield of 72.97%.

MS m/z (ESI): 598.1 [M+H]

### Step 5

### 2-(2-(benzofuran-6-yl)-6-(4-(5-(benzyloxy)-6-methylpyrimidine-4-carbonyl)piperazin-1-yl)-5-eth yl-7-oxo-[1,2,4]triazolo[1,5-a]pyrimidin-4(7H)-yl)-N-(4-((trifluoromethyl)thio)phenyl)acetamide

5-(Benzyloxy)-6-methylpyrimidine-4-carboxylic acid **1g** (30.65 mg, 125.50 µmol) was dissolved in N,N-dimethylformamide (2 mL), then 1-(3-dimethylaminopropyl)-3-ethylcarbodiimide hydrochloride (32.08 mg, 167.33 µmol), 1-hydroxybenzotriazole (22.61 mg, 167.33 µmol) and N,N-diisopropylethylamine (54.07 mg, 418.33 µmol) were added, and the reaction was performed at room temperature for 10 min. Subsequently, 2-(2-(benzofuran-6-yl)-5-ethyl-7-oxo-6-(piperazin-1-yl)-[1,2,4]triazolo[1,5-a]pyrimidin-4(7H)-yl )-N-(4-((trifluoromethyl) thio)phenyl)acetamide **90e** (50 mg, 83.67 µmol) was added, and the reaction was performed at room temperature for 18 h. Water (20 mL) was added, the mixture was extracted with dichloromethane (50 mL×3), the combined organic phases were washed with saturated sodium chloride solution (30 mL ×2), dried over anhydrous sodium sulfate, filtered, and concentrated under reduced pressure. The residue was purified by silica gel column chromatography (eluent: System B) to obtain 2-(2-(benzofuran-6-yl)-6-(4-(5-(benzyloxy)-6-methylpyrimidine-4-carbonyl) piperazin-1-yl)-5-ethyl-7-oxo-[1,2,4]triazolo[1,5-a]pyrimidin-4(7H)-yl)-N-(4-((trifluoromethyl) thio)phenyl)acetamide **90f** (40 mg) in a yield of 58.03%.

MS m/z (ESI): 824.0 [M+H]

### Step 6

### 2-(2-(benzofuran-6-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(4-((trifluoromethyl)thio)phenyl)acetamide

2-(2-(Benzofuran-6-yl)-6-(4-(5-(benzyloxy)-6-methylpyrimidine-4-carbonyl)piperazin-1-yl)-5-ethyl-7-oxo-[1,2,4]triazolo[1,5-a]pyrimidin-4(7H)-yl)-N-(4-((trifluoromethyl)thio)phenyl) acetamide **90f** (40 mg, 48.55 µmol) was dissolved in trifluoroacetic acid (3 mL), the temperature was raised to 45 °C, and the reaction was performed for 3 h. After completion of the reaction, the mixture was concentrated under reduced pressure, and the resulting residue was separated by preparative liquid chromatography (separation column AKZONOBEL Kromasil; 250×21.2 mm I.D.; 5µm, 20 mL/min; mobile phase A: 0.05% TFA+H₂O, mobile phase B: CH₃CN) to obtain 2-(2-(benzo furan-6-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(4-((trifluoromethyl)thio)phenyl)acetamide **90** (25 mg) in a yield of 66.67%.

MS m/z (ESI): [M+H] 734.1
¹H NMR (400 MHz, DMSO-d6) δ 10.93 (s, 1H), 10.24 (s, 1H), 8.59 (s, 1H), 8.23 (s, 1H), 8.12 (d, J = 2.2 Hz, 1H), 8.02 (dd, J = 8.2, 1.4 Hz, 1H), 7.82 - 7.72 (m, 3H), 7.69 (d, J = 8.7 Hz, 2H), 7.05 (dd, J = 2.2, 1.0 Hz, 1H), 5.30 (s, 2H), 4.55 (d, J = 12.5 Hz, 1H), 3.55 (q, J = 11.3, 10.7 Hz, 3H), 3.26 (d, J = 12.7 Hz, 1H), 3.05 - 2.97 (m, 3H), 2.86 (d, J = 11.2 Hz, 1H), 2.68 (d, J = 10.7 Hz, 1H), 2.45 (s, 3H), 1.20 (t, J = 7.4 Hz, 3H).

### Example 91

### 2-(2-(cyclohept-1-en-1-yl)-5-ethyl-6-(1-(5-hydroxy-6-methylpyrimidine-4-carbonyl)-1,2,3,6-tetra hydropyridin-4-yl)-7-oxo-[1,2,4]triazolo[1,5-a]pyrimidin-4(7H)-yl)-N-(4-(pentafluoro-λ⁶-sulfane yl)phenyl)acetamide

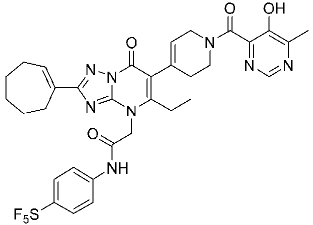

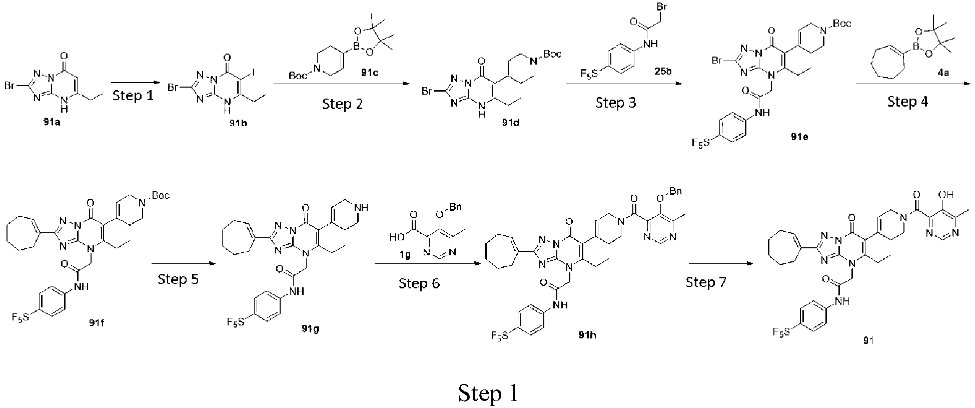

### 2-bromo-5-ethyl-6-iodo-[1,2,4]triazolo[1,5-a]pyrimidin-7(4H)-one

2-Bromo-5-ethyl-[1,2,4]triazolo[1,5-a]pyrimidin-7(4H)-one **91a** (300 mg, 1.23 mmol, prepared according to published patent WO2022249060) and N-iodosuccinimide (305.45 mg, 1.36 mmol) were dissolved in acetic acid (5 mL), the temperature was raised to 60 °C, and the mixture was stirred for 3 h. The mixture was cooled with an ice-water bath, the solid was collected by filtration, washed with ethanol, and the filter cake was dried to obtain 2-bromo-5-ethyl-6-iodo-[1,2,4]triazolo[1,5-a]pyrimidin-7(4H)-one **91b** (380 mg) in a yield of 83.45%.

MS m/z (ESI): [M+1] 369.0

### Step 2

### tert-butyl 4-(2-bromo-5-ethyl-7-oxo-4,7-dihydro-[1,2,4]triazolo[1,5-a]pyrimidin-6-yl)-3,6-dihydropyridine-1(2H)-carboxylate

At room temperature, 2-bromo-5-ethyl-6-iodo-[1,2,4]triazolo[1,5-a]pyrimidin-7(4H)-one **91b** (1.5 g, 4.07 mmol), tert-butyl 4-(4,4,5,5-tetramethyl-1,3,2-dioxaborolan-2-yl)-3,6-dihydropyridine-1(2H)-carboxylate **91c** (1.51 g, 4.88 mmol, commercially available), palladium(II) alkylsulfonato(2-aminobiphenyl-2-yl)tricyclohexylphosphine (264.32 mg, 406.55 µmol), potassium phosphate (2.59 g, 12.20 mmol), and n-butanol (0.2 mL) were added to a mixture of water (3 mL) and 1,4-dioxane (20 mL). The mixture was subject to argon replacement 4 times, the temperature was raised to 75 °C, and the reaction was performed for 18 h. The mixture was concentrated under reduced pressure, and the residue was purified by silica gel column chromatography (eluent: System B) to obtain tert-butyl 4-(2-bromo-5-ethyl-7-oxo-4,7-dihydro-[1,2,4]triazolo[1,5-a]pyrimidin-6-yl)-3,6-dihydropyridine-1(2H)-carboxylate **91d** (760 mg) in a yield of 44.06%.

MS m/z (ESI): 368.1 [M+H-56]

### Step 3

### tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)-3,6-dihydropyridine-1(2H)-carboxylate

At room temperature, tert-butyl 4-(2-bromo-5-ethyl-7-oxo-4,7-dihydro-[1,2,4]triazolo[1,5-a]pyrimidin-6-yl)-3,6-dihydropyridine-1(2H)-carboxylate **91d** (200 mg, 471.37 µmol), 2-bromo-N-(4-(pentafluoro-λ6-sulfaneyl)phenyl)acetamide **25b** (176.35 mg, 518.51 µmol), and N,N-diisopropylethylamine (182.76 mg, 1.41 mmol) were added to N,N-dimethylformamide (7 mL), and the temperature was raised to 70 °C. The reaction was performed for 3 h. Water (20 mL) was added to the reaction mixture, and the mixture was extracted with ethyl acetate (20 mL ×3). The combined organic phase was washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)-3,6-dihydropyridine-1(2H)-carboxylate **91e** (120 mg) with a yield of 37.25%.

MS m/z (EST): 627.1 [M+H-56]

### Step 4

### tert-butyl 4-(2-(cyclohept-1-en-1-yl)-5-ethyl-7-oxo-4-(2-oxo-2-((4-(pentafluoro-λ⁶-sulfaneyl)phenyl)amino )ethyl)-4,7-dihydro-[1,2,4]triazolo[1,5-a]pyrimidin-6-yl)-3,6-dihydropyridine-1(2H)-carboxylate

At room temperature, tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)-3,6-dihydropyridine-1(2H)-carboxylate 91e (60 mg, 87.79 µmol), 2-(cyclohept-1-en-1-yl)-4,4,5,5-tetramethyl-1,3,2-dioxaborolane 4a (25.35 mg, 114.12 µmol),[1,1'-bis(diphenylphosphino)ferrocene]palladium(II) dichloride dichloromethane complex (7.12 mg, 8.78 µmol), and sodium carbonate (27.91 mg, 263.36 µmol) were dissolved in a mixed solution of 1,4-dioxane (5 mL) and water (0.5 mL). The mixture was subject to argon replacement 3 times, and the temperature was raised to 85 °C. The reaction was performed for 2 h. Water (20 mL) was added to the reaction mixture, and the mixture was extracted with ethyl acetate (20 mL ×3). The combined organic phase was washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(2-(cyclohept-1-en-1-yl)-5-ethyl-7-oxo-4-(2-oxo-2-((4-(pentafluoro-λ⁶-sulfaneyl)phenyl)amino) ethyl)-4,7-dihydro-[1,2,4]triazolo[1,5-a]pyrimidin-6-yl)-3,6-dihydropyridine-1(2H)-carboxylate 91f (50 mg) with a yield of 81.51%.

MS m/z (ESI): 643.1 [M+H-56]

### Step 5

### 2-(2-(cyclohept-1-en-1-yl)-5-ethyl-7-oxo-6-(1,2,3,6-tetrahydropyridin-4-yl)-[1,2,4]triazolo[1,5-a] pyrimidin-4(7H)-yl)-N-(4-(pentafluoro-λ⁶-sulfaneyl)phenyl)acetamide

At room temperature, trifluoroacetic acid (1 mL) was added to a solution of tert-butyl 4-(2-(cyclohept-1-en-1-yl)-5-ethyl-7-oxo-4-(2-oxo-2-((4-(pentafluoro-λ⁶-sulfaneyl)phenyl)amino) ethyl)-4,7-dihydro-[1,2,4]triazolo[1,5-a]pyrimidin-6-yl)-3,6-dihydropyridine-1(2H)-carboxylate **91f** (50 mg, 71.56 µmol) in dichloromethane (4 mL). The reaction was performed at room temperature for 40 min. The mixture was concentrated under reduced pressure to obtain 2-(2-(cyclohept-1-en-1-yl)-5-ethyl-7-oxo-6-(1,2,3,6-tetrahydropyridin-4-yl)-[1,2,4]triazolo[1,5-a] pyrimidin-4(7H)-yl)-N-(4-(pentafluoro-λ⁶-sulfaneyl)phenyl)acetamide **91g** (42.8 mg) as a crude product, which was used directly in the next step.

MS m/z (ESI): 599.3 [M+1]

### Step 6

### 2-(6-(1-(5-(benzyloxy)-6-methylpyrimidine-4-carbonyl)-1,2,3,6-tetrahydropyridin-4-yl)-2-(cyclo hept-1-en-1-yl)-5-ethyl-7-oxo-[1,2,4]triazolo[1,5-a]pyrimidin-4(7H)-yl)-N-(4-(pentafluoro-λ⁶-sul faneyl)phenyl)acetamide

At room temperature, 5-(benzyloxy)-6-methylpyrimidine-4-carboxylic acid **1g** (26.19 mg, 107.24 µmol), 1-(3-dimethylaminopropyl)-3-ethylcarbodiimide hydrochloride (27.41 mg, 142.99 µmol), 1-hydroxybenzotriazole (19.32 mg, 142.99 µmol), and N,N-dimethylformamide (46.20 mg, 357.48 µmol) were dissolved in N,N-dimethylformamide (3 mL). The reaction was performed at room temperature for 40 min, followed by addition of a solution of 2-(2-(cyclohept-1-en-1-yl)-5-ethyl-7-oxo-6-(1,2,3,6-tetrahydropyridin-4-yl)-[1,2,4]triazolo[1,5-a] pyrimidin-4(7H)-yl)-N-(4-(pentafluoro-λ⁶-sulfaneyl)phenyl)acetamide **91g** (42.8 mg, 71.50 µmol) in N,N-dimethylformamide (3 mL). The reaction was performed at room temperature for 18 h. Water (20 mL) was added to the reaction mixture, and the mixture was extracted with ethyl acetate (20 mL ×3). The combined organic phase was washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(1-(5-(benzyloxy)-6-methylpyrimidine-4-carbonyl)-1,2,3,6-tetrahydropyridin-4-yl)-2-(cyclo hept-1-en-1-yl)-5-ethyl-7-oxo-[1,2,4]triazolo[1,5-a]pyrimidin-4(7H)-yl)-N-(4-(pentafluoro-λ⁶-sul faneyl) phenyl)acetamide **91h** (49 mg) with a yield of 83.09%.

MS m/z (ESI): 825.4 [M+1]

### Step 7

### 2-(2-(cyclohept-1-en-1-yl)-5-ethyl-6-(1-(5-hydroxy-6-methylpyrimidine-4-carbonyl)-1,2,3,6-tetra hydropyridin-4-yl)-7-oxo-[1,2,4]triazolo[1,5-a]pyrimidin-4(7H)-yl)-N-(4-(pentafluoro-λ⁶-sulfane yl)phenyl)acetamide

At room temperature, trifluoroacetic acid (4 mL) was added to a solution of 2-(6-(1-(5-(benzyloxy)-6-methylpyrimidine-4-carbonyl)-1,2,3,6-tetrahydropyridin-4-yl)-2-(cyclo hept-1-en-1-yl)-5-ethyl-7-oxo-[1,2,4]triazolo[1,5-a]pyrimidin-4(7H)-yl)-N-(4-(pentafluoro-λ⁶-sul faneyl) phenyl)acetamide **91h** (49 mg, 59.40 µmol) in dichloromethane (1 mL). The reaction was performed at room temperature for 48 h. Upon completion of the reaction, the mixture was concentrated under reduced pressure. The resulting residue was separated by preparative liquid chromatography (separation column: AKZONOBEL Kromasil; 250 ×21.2 mm I.D.; 5 µm, 20 mL/min; mobile phase A: 0.05% TFA+H₂O, mobile phase B: CH₃CN) to obtain 2-(2-(cyclohept-1-en-1-yl)-5-ethyl-6-(1-(5-hydroxy-6-methylpyrimidine-4-carbonyl)-1,2,3,6-tetra hydropyridin-4-yl)-7-oxo-[1,2,4]triazolo[1,5-a]pyrimidin-4(7H)-yl)-N-(4-(pentafluoro-λ⁶-sulfane yl)phenyl)acetamide **91** (13.36 mg) with a yield of 30.30%.

MS m/z (ESI): 735.2 [M+1]
¹H NMR (400 MHz, DMSO-d6) δ 10.97 (s, 1H), 10.29 (s, 1H), 8.59 (s, 1H), 7.88 (d, J = 9.2 Hz, 2H), 7.78 (d, J = 8.9 Hz, 2H), 7.05 (t, J = 6.7 Hz, 1H), 5.74 (d, J = 68.7 Hz, 1H), 5.18 (s, 2H), 4.34 (s, 1H), 4.09 - 3.99 (m, 3H), 3.50 (s, 1H), 2.86 - 2.59 (m, 4H), 2.45 (s, 3H), 2.30 (q, J = 6.3 Hz, 2H), 2.18 (s, 1H), 1.77 (q, J = 6.0 Hz, 2H), 1.52 (dt, J = 14.7, 6.4 Hz, 4H), 1.16 (dt, J = 16.3, 7.4 Hz, 3H).

### Example 92

### 2-(2-(cyclohept-1-en-1-yl)-5-ethyl-6-(1-(5-hydroxy-6-methylpyrimidine-4-carbonyl)piperidin-4-yl)-7-oxo-[1,2,4]triazolo[1,5-a]pyrimidin-4(7H)-yl)-N-(4-(pentafluoro-λ⁶-sulfaneyl)phenyl)aceta mide

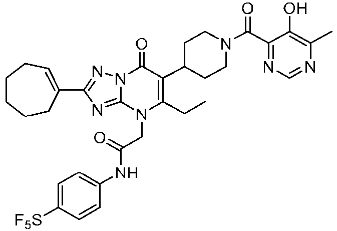

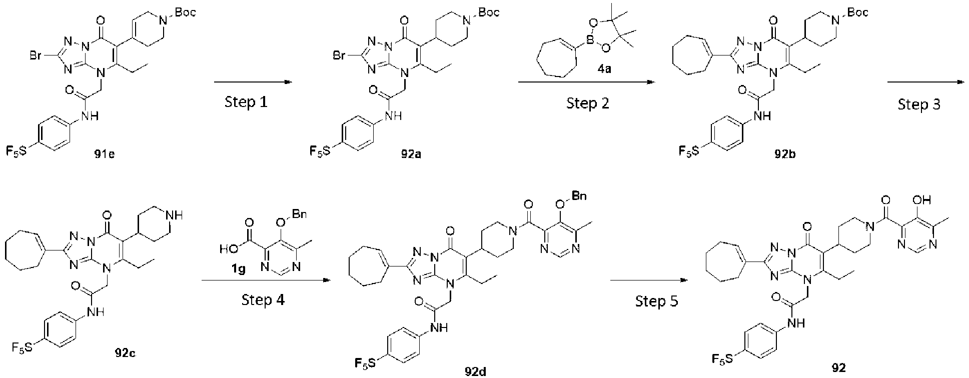

### Step 1

### tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)piperidine-1-carboxylate

At room temperature, platinum dioxide (19.93 mg, 87.79 µmol) was added to a solution of tert-butyl 4-(2-bromo-5-ethyl-7-oxo-4-(2-oxo-2-((4-(pentafluoro-λ⁶-sulfaneyl)phenyl) amino)ethyl)-4,7-dihydro-[1,2,4]triazolo[1,5-a]pyrimidin-6-yl)-3,6-dihydropyridine-1(2H)-carbo xylate **91e** (60 mg, 87.79 µmol) in ethyl acetate (1.5 mL), 2 drops of acetic acid were added dropwise, hydrogen replacement was performed 3 times, and the reaction was performed at 30 °C for 18 h. The mixture was filtered and concentrated, and the resulting residue was separated by preparative liquid chromatography (separation column AKZONOBEL Kromasil; 250×21.2 mm I.D.; 5 µm, 20 mL/min; mobile phase A: 0.05% TFA + H₂O, mobile phase B: CH₃CN) to obtain tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)piperidine-1-carboxylate **92a** (22 mg) with a yield of 36.56%.

MS m/z (ESI): 629.1 [M+H-56]

### Step 2

### tert-butyl 4-(2-(cyclohept-1-en-1-yl)-5-ethyl-7-oxo-4-(2-oxo-2-((4-(pentafluoro-λ⁶-sulfaneyl)phenyl)amino )ethyl)-4,7-dihydro-[1,2,4]triazolo[1,5-a]pyrimidin-6-yl)piperidine-1-carboxylate

At room temperature, tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)piperidine-1-carboxylate **92a** (30 mg, 43.76 µmol), 2-(cyclohept-1-en-1-yl)-4,4,5,5-tetramethyl-1,3,2-dioxaborolane **4a** (12.64 mg, 56.89 µmol), [1,1'-bis(diphenylphosphino)ferrocene]palladium(II) dichloride dichloromethane complex (3.55 mg, 4.38 µmol), and sodium carbonate (13.92 mg, 131.29 µmol) were added to a mixed solution of 1,4-dioxane (5 mL) and water (1 mL), argon replacement was performed three times, the temperature was raised to 85 °C, and the reaction was performed for 2 h. Water (20 mL) was added to the reaction mixture, the mixture was extracted with ethyl acetate (20 mL × 3), the combined organic phases were washed with saturated sodium chloride solution (20 mL), dried over anhydrous sodium sulfate, filtered, and concentrated under reduced pressure, and the resulting residue was purified by silica gel column chromatography (eluent: System B) to obtain tert-butyl 4-(2-(cyclohept-1-en-1-yl)-5-ethyl-7-oxo-4-(2-oxo-2-((4-(pentafluoro-λ⁶-sulfaneyl)phenyl)amino )ethyl)-4,7-dihydro-[1,2,4]triazolo[1,5-a]pyrimidin-6-yl)piperidine-1-carboxylate **92b** (30 mg) with a yield of 97.82%.

MS m/z (ESI): 645.3 [M+H-56]

### Step 3

### 2-(2-(cyclohept-1-en-1-yl)-5-ethyl-7-oxo-6-(piperidin-4-yl)-[1,2,4]triazolo[1,5-a]pyrimidin-4(7H )-yl)-N-(4-(pentafluoro-λ⁶-sulfaneyl)phenyl)acetamide

At room temperature, trifluoroacetic acid (1 mL) was added to a solution of tert-butyl 4-(2-(cyclohept-1-en-1-yl)-5-ethyl-7-oxo-4-(2-oxo-2-((4-(pentafluoro-λ⁶-sulfaneyl)phenyl)amino) ethyl)-4,7-dihydro-[1,2,4]triazolo[1,5-a]pyrimidin-6-yl)piperidine-1-carboxylate **92b** (30 mg, 42.81 µmol) in dichloromethane (4 mL), and the reaction was performed at room temperature for 40 min. The mixture was concentrated under reduced pressure to obtain 2-(2-(cyclohept-1-en-1-yl)-5-ethyl-7-oxo-6-(piperidin-4-yl)-[1,2,4]triazolo[1,5-a]pyrimidin-4(7H )-yl)-N-(4-(pentafluoro-λ⁶-sulfaneyl)phenyl)acetamide **92c** (25.7 mg) as a crude product, which was used directly in the next step.

MS m/z (ESI): 601.3 [M+1]

### Step 4

### 2-(6-(1-(5-(benzyloxy)-6-methylpyrimidine-4-carbonyl)piperidin-4-yl)-2-(cyclohept-1-en-1-yl)-5 -ethyl-7-oxo-[1,2,4]triazolo[1,5-a]pyrimidin-4(7H)-yl)-N-(4-(pentafluoro-λ⁶-sulfaneyl)phenyl)ac etamide

At room temperature, 5-(benzyloxy)-6-methylpyrimidine-4-carboxylic acid **1g** (15.25 mg, 62.43 µmol), 1-(3-dimethylaminopropyl)-3-ethylcarbodiimide hydrochloride (15.96 mg, 83.24 µmol), 1-hydroxybenzotriazole (11.25 mg, 83.24 µmol), and N,N-diisopropylethylamine (26.90 mg, 208.11 µmol) were added to N,N-dimethylformamide (2 mL). The reaction was performed at room temperature for 40 min, then a solution of 2-(2-(cyclohept-1-en-1-yl)-5-ethyl-7-oxo-6-(piperidin-4-yl)-[1,2,4]triazolo[1,5-a]pyrimidin-4(7H )-yl)-N-(4-(pentafluoro-λ⁶-sulfaneyl)phenyl) acetamide **92c** (25 mg, 41.62 µmol) in N,N-dimethylformamide (2 mL) was added, and the reaction was performed at room temperature for 24 h. Water (20 mL) was added, the mixture was extracted with ethyl acetate (20 mL × 3), the combined organic phases were washed with saturated sodium chloride solution (20 mL), dried over anhydrous sodium sulfate, filtered, and concentrated under reduced pressure, and the residue was purified by silica gel column chromatography (eluent: System B) to obtain 2-(6-(1-(5-(benzyloxy)-6-methylpyrimidine-4-carbonyl)piperidin-4-yl)-2-(cyclohept-1-en-1-yl)-5 -ethyl-7-oxo-[1,2,4]triazolo[1,5-a]pyrimidin-4(7H)-yl)-N-(4-(pentafluoro-λ⁶-sulfaneyl)phenyl)ac etamide **92d** (25 mg) with a yield of 72.64%.

MS m/z (ESI): 827.3 [M+1]

### Step 5

### 2-(2-(cyclohept-1-en-1-yl)-5-ethyl-6-(1-(5-hydroxy-6-methylpyrimidine-4-carbonyl)piperidin-4-yl)-7-oxo-[1,2,4]triazolo[1,5-a]pyrimidin-4(7H)-yl)-N-(4-(pentafluoro-λ⁶-sulfaneyl)phenyl)aceta mide

At room temperature, trifluoroacetic acid (4 mL) was added to a solution of 2-(6-(1-(5-(benzyloxy)-6-methylpyrimidine-4-carbonyl)piperidin-4-yl)-2-(cyclohept-1-en-1-yl)-5 -ethyl-7-oxo-[1,2,4]triazolo[1,5-a]pyrimidin-4(7H)-yl)-N-(4-(pentafluoro-λ⁶-sulfaneyl)phenyl)ac etamide **92d** (25 mg, 30.23 µmol) in dichloromethane (1 mL), and the reaction was performed at room temperature for 24 h. The mixture was concentrated under reduced pressure, and the resulting residue was separated by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain 2-(2-(cyclohept-1-en-1-yl)-5-ethyl-6-(1-(5-hydroxy-6-methylpyrimidine-4-carbonyl)piperidin-4-yl)-7-oxo-[1,2,4]triazolo[1,5-a]pyrimidin-4(7H)-yl)-N-(4-(pentafluoro-λ⁶-sulfaneyl)phenyl)aceta mide **92** (2.29 mg) with a yield of 9.77%.

MS m/z (ESI): 737.3 [M+1]
¹H NMR (400 MHz, DMSO-d6) δ 10.99 (s, 1H), 10.24 (s, 1H), 8.58 (s, 1H), 7.89 (d, J = 9.0 Hz, 2H), 7.78 (d, J = 8.9 Hz, 2H), 7.05 (t, J = 6.8 Hz, 1H), 5.22 (s, 2H), 4.64 (d, J = 12.5 Hz, 1H), 3.58 (d, J = 13.3 Hz, 1H), 3.18 (t, J = 12.7 Hz, 1H), 3.00 (s, 1H), 2.94 - 2.78 (m, 3H), 2.73 (d, J = 8.6 Hz, 2H), 2.45 (s, 3H), 2.40 (s, 1H), 2.30 (q, J = 6.5, 6.0 Hz, 2H), 1.77 (d, J = 6.9 Hz, 2H), 1.61 (d, J = 12.3 Hz, 1H), 1.58 - 1.38 (m, 5H), 1.23 (s, 1H), 1.17 (t, J = 7.4 Hz, 3H)

### Example 93

### N-(2-acetyl-4-(trifluoromethyl)phenyl)-2-(2-(cyclohept-1-en-1-yl)-5-ethyl-6-(4-(3-hydroxypicoli noyl)piperazin-1-yl)-7-oxo-[1,2,4]triazolo[1,5-a]pyrimidin-4(7H)-yl)acetamide

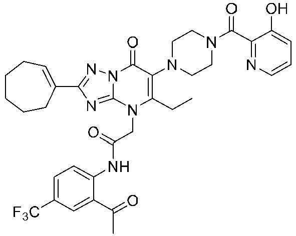

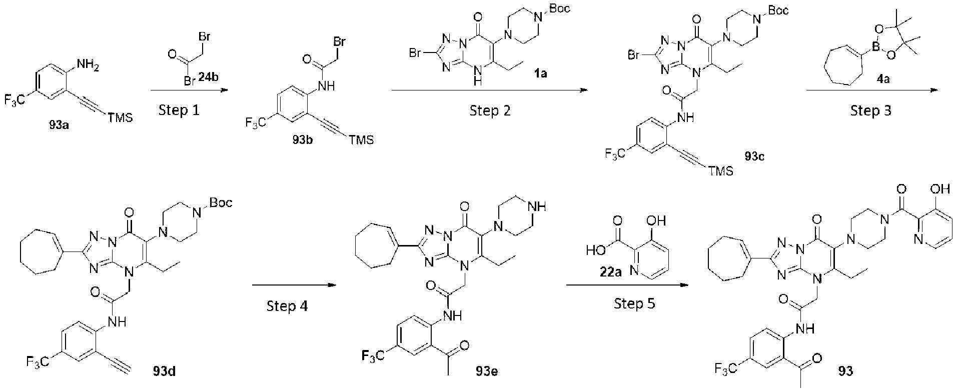

### Step 1

### 2-bromo-N-(4-(trifluoromethyl)-2-((trimethylsilyl)ethynyl)phenyl)acetamide

Under ice bath, a solution of 2-bromoacetyl bromide **24b** (267.48 mg, 1.33 mmol, 115.09 µL) in dichloromethane (5 mL) was slowly added dropwise to a solution of 4-(trifluoromethyl)-2-((trimethylsilyl)ethynyl)aniline **93a** (310 mg, 1.20 mmol, prepared according to published patent WO2011112731) in dichloromethane (5 mL), and the reaction was performed at room temperature for 18 h. Water (20 mL) was added, and the mixture was extracted with ethyl acetate (20 mL×3). The combined organic phases were washed with saturated sodium chloride solution (20 mL), dried over anhydrous sodium sulfate, filtered, and concentrated under reduced pressure. The residue was purified by silica gel column chromatography (eluent: System A) to obtain 2-bromo-N-(4-(trifluoromethyl)-2-((trimethylsilyl)ethynyl)phenyl)acetamide **93b** (250 mg) with a yield of 54.86%.

MS m/z (ESI): 378.0 [M+1]

### Step 2

### tert-butyl 4-(2-bromo-5-ethyl-7-oxo-4-(2-oxo-2-((4-(trifluoromethyl)-2-((trimethylsilyl)ethynyl)phenyl)ami no)ethyl)-4,7-dihydro-[1,2,4]triazolo[1,5-a]pyrimidin-6-yl)piperazine-1-carboxylate

At room temperature, N,N-diisopropylethylamine (347.83 mg, 2.69 mmol) was added to a solution of 2-bromo-N-(4-(trifluoromethyl)-2-((trimethylsilyl)ethynyl)phenyl)acetamide **93b** (244.33 mg, 645.92 µmol) and tert-butyl 4-(2-bromo-5-ethyl-7-oxo-4,7-dihydro-[1,2,4]triazolo[1,5-a]pyrimidin-6-yl)piperazine-1-carboxy late **1a** (230 mg, 538.27 µmol) in N,N-dimethylformamide (5 mL). The temperature was raised to 50 °C, and the reaction was performed for 2 h. Water (20 mL) was added, and the mixture was extracted with ethyl acetate (20 mL×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(2-bromo-5-ethyl-7-oxo-4-(2-oxo-2-((4-(trifluoromethyl)-2-((trimethylsilyl)ethynyl)phenyl) amino)ethyl)-4,7-dihydro-[1,2,4]triazolo[1,5-a]pyrimidin-6-yl)piperazine-1-carboxylate **93c** (170 mg) with a yield of 43.58%.

MS m/z (ESI): 668.2 [M+H-56]

### Step 3

### tert-butyl 4-(2-(cyclohept-1-en-1-yl)-5-ethyl-4-(2-((2-ethynyl-4-(trifluoromethyl)phenyl)amino)-2-oxoethyl )-7-oxo-4,7-dihydro-[1,2,4]triazolo[1,5-a]pyrimidin-6-yl)piperazine-1-carboxylate

At room temperature, tert-butyl 4-(2-bromo-5-ethyl-7-oxo-4-(2-oxo-2-((4-(trifluoromethyl)-2-((trimethylsilyl)ethynyl)phenyl)ami no)ethyl)-4,7-dihydro-[1,2,4]triazolo[1,5-a]pyrimidin-6-yl)piperazine-1-carboxylate **93c** (170 mg, 234.60 µmol), 2-(cyclohept-1-en-1-yl)-4,4,5,5-tetramethyl-1,3,2-dioxaborolane **4a** (67.74 mg, 304.98 µmol), [1,1'-bis(diphenylphosphino)ferrocene]palladium(II) dichloride dichloromethane complex (19.02 mg, 23.46 µmol), and sodium carbonate (74.60 mg, 703.80 µmol) were dissolved in a mixed solution of 1,4-dioxane (5 mL) and water (0.5 mL). The mixture was subject to argon replacement three times, then heated to 80 °C, and the reaction was performed for 2 h. Water (20 mL) was added, and the mixture was extracted with ethyl acetate (20 mL×3). The combined organic phases were washed with saturated sodium chloride solution (20 mL), dried over anhydrous sodium sulfate, filtered, and concentrated under reduced pressure. The residue was purified by silica gel column chromatography (eluent: System B) to obtain tert-butyl 4-(2-(cyclohept-1-en-1-yl)-5-ethyl-4-(2-((2-ethynyl-4-(trifluoromethyl)phenyl)amino)-2-oxoethyl )-7-oxo-4,7-dihydro-[1,2,4]triazolo[1,5-a]pyrimidin-6-yl)piperazine-1-carboxylate **93d** (130 mg) with a yield of 82.99%.

MS m/z (ESI): 612.3 [M+H-56]

### Step 4

### N-(2-acetyl-4-(trifluoromethyl)phenyl)-2-(2-(cyclohept-1-en-1-yl)-5-ethyl-7-oxo-6-(piperazin-1-yl)-[1,2,4]triazolo[1,5-a]pyrimidin-4(7H)-yl)acetamide

At room temperature, trifluoroacetic acid (0.5 mL) was added to a solution of tert-butyl 4-(2-(cyclohept-1-en-1-yl)-5-ethyl-4-(2-((2-ethynyl-4-(trifluoromethyl)phenyl)amino)-2-oxoethyl) -7-oxo-4,7-dihydro-[1,2,4]triazolo[1,5-a]pyrimidin-6-yl)piperazine-1-carboxylate **93d** (130 mg, 194.69 µmol) in dichloromethane (2 mL), and the reaction was performed at room temperature for 40 minutes. The mixture was concentrated under reduced pressure, and the residue was purified by silica gel column chromatography (eluent: System B) to obtain N-(2-acetyl-4-(trifluoromethyl)phenyl)-2-(2-(cyclohept-1-en-1-yl)-5-ethyl-7-oxo-6-(piperazin-1-yl)-[1,2,4] triazolo[1,5-a]pyrimidin-4(7H)-yl)acetamide **93e** (40 mg) in a yield of 35.08%.

MS m/z (ESI): 586.3 [M+1]

### Step 5

### N-(2-acetyl-4-(trifluoromethyl)phenyl)-2-(2-(cyclohept-1-en-1-yl)-5-ethyl-6-(4-(3-hydroxypicoli noyl)piperazin-1-yl)-7-oxo-[1,2,4]triazolo[1,5-a]pyrimidin-4(7H)-yl)acetamide

At room temperature, 3-hydroxypicolinic acid **22a** (14.25 mg, 102.46 µmol), 1-(3-dimethylaminopropyl)-3-ethylcarbodiimide hydrochloride (26.19 mg, 136.61 µmol), 1-hydroxybenzotriazole (18.46 mg, 136.61 µmol), and N,N-diisopropylethylamine (44.14 mg, 341.52 µmol) were added to N,N-dimethylformamide (2 mL), and the reaction was performed at room temperature for 40 minutes. A solution of N-(2-acetyl-4-(trifluoromethyl)phenyl)-2-(2-(cyclohept-1-en-1-yl)-5-ethyl-7-oxo-6-(piperazin-1-yl)-[1,2,4]triazolo[1,5-a]pyrimidin-4(7H)-yl)acetamide **93e** (40 mg, 68.30 µmol) in N,N-dimethylformamide (2 mL) was added, and the reaction was performed at room temperature for 16 hours. The mixture was concentrated under reduced pressure, and the resulting residue was separated by preparative liquid chromatography (separation column: AKZONOBEL Kromasil; 250×21.2 mm I.D.; 5 µm, 20 mL/min; mobile phase A: 0.05% TFA + H₂O, mobile phase B: CH₃CN) to obtain N-(2-acetyl-4-(trifluoromethyl)phenyl)-2-(2-(cyclohept-1-en-1-yl)-5-ethyl-6-(4-(3-hydroxypicoli noyl)piperazin-1-yl)-7-oxo-[1,2,4] triazolo[1,5-a]pyrimidin-4(7H)-yl)acetamide **93** (4.32 mg) in a yield of 8.70%.

MS m/z (ESI): 707.3 [M+1]
¹H NMR (400 MHz, DMSO-d6) δ 11.57 (s, 1H), 10.59 (s, 1H), 8.31 (d, J = 8.7 Hz, 1H), 8.19 (d, J = 2.1 Hz, 1H), 8.10 (dd, J = 4.3, 1.6 Hz, 1H), 7.97 (dd, J = 8.9, 2.2 Hz, 1H), 7.47-7.32 (m, 2H), 7.05 (t, J = 6.7 Hz, 1H), 5.25 (s, 2H), 4.55 (d, J = 12.6 Hz, 1H), 3.45 (dd, J = 26.7, 13.0 Hz, 3H), 3.23 (t, J = 12.3 Hz, 1H), 2.97 (d, J = 10.2 Hz, 3H), 2.81 (d, J = 10.9 Hz, 1H), 2.78-2.69 (m, 2H), 2.64 (s, 4H), 2.29 (s, 2H), 1.77 (d, J = 7.6 Hz, 2H), 1.50 (s, 4H), 1.16 (t, J = 7.4 Hz, 3H).

### Example 94

### 2-(2-(cyclohept-1-en-1-yl)-5-ethyl-6-(4-(5-hydroxy-6-methylpyrimidine-4-carbonyl)-1,4-diazepa n-1-yl)-7-oxo-[1,2,4]triazolo[1,5-a]pyrimidin-4(7H)-yl)-N-(4-(pentafluoro-λ⁶-sulfaneyl)phenyl)a cetamide

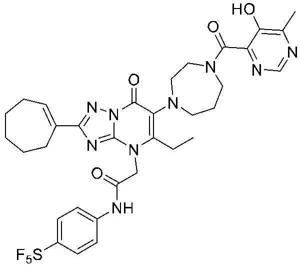

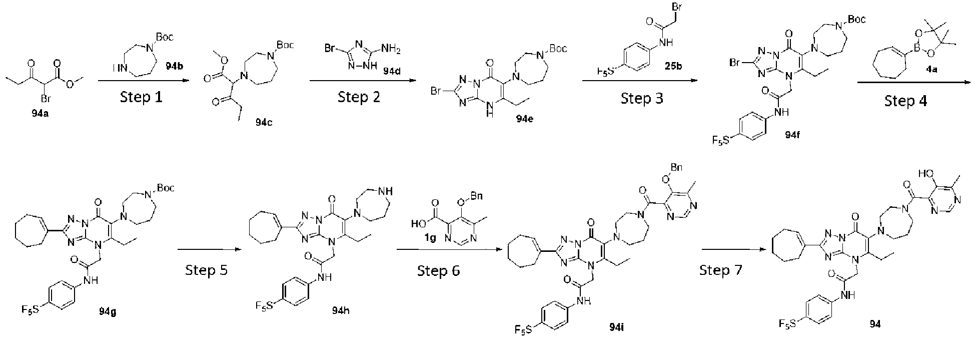

### Step 1

### tert-butyl 4-(1-methoxy-1,3-dioxopentan-2-yl)-1,4-diazepane-1-carboxylate

At room temperature, methyl 2-bromo-3-oxopentanoate **94a** (5 g, 23.92 mmol, prepared according to published patent WO2017216283), tert-butyl 1,4-diazepane-1-carboxylate **94b** (5.27 g, 26.31 mmol), and potassium carbonate (9.92 g, 71.76 mmol) were added to acetonitrile (25 mL), and the reaction was performed at room temperature for 18 hours. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The residue was purified by silica gel column chromatography (eluent: System A) to obtain tert-butyl 4-(1-methoxy-1,3-dioxopentan-2-yl)-1,4-diazepane-1-carboxylate **94c** (3.61 g) in a yield of 45.96%.

MS m/z (ESI): 329.2 [M+1]

### Step 2

### tert-butyl 4-(2-bromo-5-ethyl-7-oxo-4,7-dihydro-[1,2,4]triazolo[1,5-a]pyrimidin-6-yl)-1,4-diazepane-1-car boxylate

At room temperature, 3-bromo-1H-1,2,4-triazol-5-amine **94d** (1.6 g, 9.82 mmol, commercially available), tert-butyl 4-(1-methoxy-1,3-dioxopentan-2-yl)-1,4-diazepane-1-carboxylate **94c** (3.22 g, 9.82 mmol), polyphosphoric acid (3.32 g, 9.82 mmol), and ethanol (12.07 mL) were added to a 50 mL sealed tube. The temperature was raised to 90 °C, and the reaction was performed for 6 hours; monitoring showed that the major product was the deprotected product. Saturated sodium bicarbonate solution (with minimal water addition, followed by direct concentration under reduced pressure) and a solution of sodium hydroxide (785.39 mg, 19.63 mmol) were added to the reaction mixture, and the pH was adjusted to 5-6. Di-tert-butyl dicarbonate (2.79 g, 12.76 mmol, 2.93 mL) was added, and the reaction was performed at room temperature for 18 hours. The mixture was concentrated under reduced pressure, and the resulting residue was purified by silica gel column chromatography (eluent: System B) to obtain tert-butyl 4-(2-bromo-5-ethyl-7-oxo-4,7-dihydro-[1,2,4]triazolo[1,5-a]pyrimidin-6-yl)-1,4-diazepane-1-car boxylate **94e** (1.7 g) in a yield of 39.24%.

MS m/z (ESI): 441.1 [M+1]

### Step 3

### tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)-1,4-diazepane-1-carboxylate

At room temperature, tert-butyl 4-(2-bromo-5-ethyl-7-oxo-4,7-dihydro-[1,2,4]triazolo[1,5-a]pyrimidin-6-yl)-1,4-diazepane-1-car boxylate **94e** (200 mg, 453.18 µmol), 2-bromo-N-(4-(pentafluoro-λ⁶-sulfaneyl)phenyl)acetamide **25b** (184.96 mg, 543.82 µmol), and N,N-diisopropylethylamine (175.71 mg, 1.36 mmol) were added to N,N-dimethylformamide (7 mL). The temperature was raised to 50 °C, and the reaction was performed for 2 hours. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The residue was purified by silica gel column chromatography (eluent: System B) to obtain tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)-1,4-diazepane-1-carboxylate **94f** (170 mg) in a yield of 53.55%.

MS m/z (ESI): 664.1 [M+H-56]

### Step 4

### tert-butyl 4-(2-(cyclohept-1-en-1-yl)-5-ethyl-7-oxo-4-(2-oxo-2-((4-(pentafluoro-λ⁶-sulfaneyl)phenyl)amino )ethyl)-4,7-dihydro-[1,2,4]triazolo[1,5-a]pyrimidin-6-yl)-1,4-diazepane-1-carboxylate

At room temperature, tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)-1,4-diazepane-1-carboxylate **94f** (170 mg, 242.68 µmol), 2-(cyclohept-1-en-1-yl)-4,4,5,5-tetramethyl-1,3,2-dioxaborolane **4a** (70.08 mg, 315.48 µmol), [1,1'-bis(diphenylphosphino)ferrocene]palladium(II) dichloride dichloromethane complex (19.67 mg, 24.27 µmol), and sodium carbonate (77.16 mg, 728.03 µmol) were added to a mixed solution of 1,4-dioxane (10 mL) and water (1 mL). The mixture was subject to argon replacement three times, then was heated to 85 °C, and the reaction was performed for 2 h. Water (20 mL) was added to the reaction mixture, followed by extraction with ethyl acetate (20 mL×3). The combined organic phases were washed with saturated sodium chloride solution (20 mL), dried over anhydrous sodium sulfate, filtered, and concentrated under reduced pressure. The residue was purified by silica gel column chromatography (eluent: System B) to obtain tert-butyl 4-(2-(cyclohept-1-en-1-yl)-5-ethyl-7-oxo-4-(2-oxo-2-((4-(pentafluoro-λ⁶-sulfaneyl)phenyl)amino )ethyl)-4,7-dihydro-[1,2,4]triazolo[1,5-a]pyrimidin-6-yl)-1,4-diazepane-1-carboxylate **94g** (160 mg) with a yield of 92.11%.

MS m/z (ESI): 660.3 [M+H-56]

### Step 5

### 2-(2-(cyclohept-1-en-1-yl)-6-(1,4-diazepan-1-yl)-5-ethyl-7-oxo-[1,2,4]triazolo[1,5-a]pyrimidin-4 (7H)-yl)-N-(4-(pentafluoro-λ⁶-sulfaneyl)phenyl)acetamide

At room temperature, trifluoroacetic acid (1.5 mL) was added to a solution of tert-butyl 4-(2-(cyclohept-1-en-1-yl)-5-ethyl-7-oxo-4-(2-oxo-2-((4-(pentafluoro-λ⁶-sulfaneyl)phenyl)amino) ethyl)-4,7-dihydro-[1,2,4]triazolo[1,5-a]pyrimidin-6-yl)-1,4-diazepane-1-carboxylate **94g** (160 mg, 223.53 µmol) in dichloromethane (6 mL). The reaction was performed at room temperature for 40 min. The mixture was concentrated under reduced pressure to obtain 2-(2-(cyclohept-1-en-1-yl)-6-(1,4-diazepan-1-yl)-5-ethyl-7-oxo-[1,2,4]triazolo[1,5-a]pyrimidin-4 (7H)-yl)-N-(4-(pentafluoro-λ⁶-sulfaneyl)phenyl)acetamide **94h** (137 mg) as a crude product, which was used directly in the next step.

MS m/z (ESI): 616.3 [M+1]

### Step 6

### 2-(6-(4-(5-(benzyloxy)-6-methylpyrimidine-4-carbonyl)-1,4-diazepan-1-yl)-2-(cyclohept-1-en-1-yl)-5-ethyl-7-oxo-[1,2,4]triazolo[1,5-a]pyrimidin-4(7H)-yl)-N-(4-(pentafluoro-λ⁶-sulfaneyl)phen yl)acetamide

At room temperature, 5-(benzyloxy)-6-methylpyrimidine-4-carboxylic acid **1g** (81.53 mg, 333.79 µmol), 1-(3-dimethylaminopropyl)-3-ethylcarbodiimide hydrochloride (85.32 mg, 445.05 µmol), 1-hydroxybenzotriazole (60.14 mg, 445.05 µmol), and N,N-diisopropylethylamine (143.80 mg, 1.11 mmol) were added to N,N-dimethylformamide (3 mL). The reaction was performed at room temperature for 40 min, then a solution of 2-(2-(cyclohept-1-en-1-yl)-6-(1,4-diazepan-1-yl)-5-ethyl-7-oxo-[1,2,4]triazolo[1,5-a]pyrimidin-4 (7H)-yl)-N-(4-(pentafluoro-λ⁶-sulfaneyl)phenyl) acetamide **94h** (137 mg, 222.53 µmol) in N,N-dimethylformamide (3 mL) was added. The reaction was performed at room temperature for 48 h. Water (20 mL) was added to the reaction mixture, followed by extraction with ethyl acetate (20 mL ×3). The combined organic phases were washed with saturated sodium chloride solution (20 mL), dried over anhydrous sodium sulfate, filtered, and concentrated under reduced pressure. The residue was purified by silica gel column chromatography (eluent: System B) to obtain 2-(6-(4-(5-(benzyloxy)-6-methylpyrimidine-4-carbonyl)-1,4-diazepan-1-yl)-2-(cyclohept-1-en-1-yl)-5-ethyl-7-oxo-[1,2,4]triazolo[1,5-a]pyrimidin-4(7H)-yl)-N-(4-(pentafluoro-λ⁶-sulfaneyl)phen yl)acetamide **94i** (120 mg) with a yield of 64.05%.

MS m/z (ESI): 842.4 [M+1]

### Step 7

### 2-(2-(cyclohept-1-en-1-yl)-5-ethyl-6-(4-(5-hydroxy-6-methylpyrimidine-4-carbonyl)-1,4-diazepa n-1-yl)-7-oxo-[1,2,4]triazolo[1,5-a]pyrimidin-4(7H)-yl)-N-(4-(pentafluoro-λ⁶-sulfaneyl)phenyl)a cetamide

At room temperature, trifluoroacetic acid (4 mL) was added to a solution of 2-(6-(4-(5-(benzyloxy)-6-methylpyrimidine-4-carbonyl)-1,4-diazepan-1-yl)-2-(cyclohept-1-en-1-yl)-5-ethyl-7-oxo-[1,2,4]triazolo[1,5-a]pyrimidin-4(7H)-yl)-N-(4-(pentafluoro-λ⁶-sulfaneyl)phen yl) acetamide **94i** (120 mg, 142.54 µmol) in dichloromethane (1 mL). The reaction was performed at 30 °C for 48 h. The mixture was concentrated under reduced pressure, and the resulting residue was separated by preparative liquid chromatography (separation column: AKZONOBEL Kromasil; 250×21.2 mm I.D.; 5 µm, 20 mL/min; mobile phase A: 0.05% TFA + H₂O, mobile phase B: CH₃CN) to obtain 2-(2-(cyclohept-1-en-1-yl)-5-ethyl-6-(4-(5-hydroxy-6-methylpyrimidine-4-carbonyl)-1,4-diazepa n-1-yl)-7-oxo-[1,2,4]triazolo[1,5-a]pyrimidin-4(7H)-yl)-N-(4-(pentafluoro-λ⁶-sulfaneyl)phenyl)a cetamide **94** (35.41 mg) with a yield of 32.72%.

MS m/z (ESI): 752.3 [M+1]
¹H NMR (400 MHz, DMSO-d6) δ 10.96 (d, J = 5.5 Hz, 1H), 10.34 (s, 1H), 8.58 (d, J = 11.7 Hz, 1H), 7.88 (d, J = 8.7 Hz, 2H), 7.78 (dd, J = 8.9, 5.0 Hz, 2H), 7.04 (q, J = 6.7 Hz, 1H), 5.16 (d, J = 9.6 Hz, 2H), 4.00 - 3.58 (m, 4H), 3.47 - 3.32 (m, 2H), 3.01 (dq, J = 12.9, 6.4 Hz, 2H), 2.88 (ddd, J = 17.3, 13.0, 5.0 Hz, 2H), 2.73 (q, J = 5.7 Hz, 2H), 2.45 (d, J = 7.8 Hz, 3H), 2.29 (qd, J = 6.7, 4.7, 3.8 Hz, 2H), 1.89 (p, J = 6.6 Hz, 1H), 1.78 (dp, J = 10.9, 5.1 Hz, 2H), 1.64 (dt, J = 13.9, 4.1 Hz, 1H), 1.51 (dp, J = 16.5, 6.0, 5.5 Hz, 4H), 1.13 (dt, J = 25.8, 7.4 Hz, 3H).

### Example 95

### 2-(2-(cyclohept-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(4-(trifluoromethoxy)phenyl)acetamide

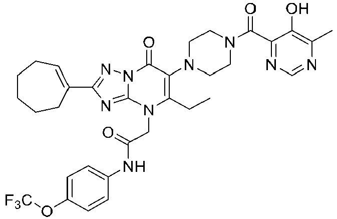

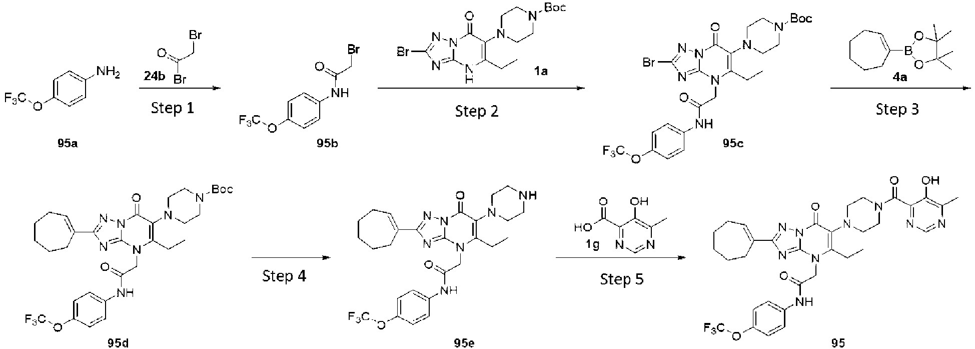

### Step 1

### 2-bromo-N-(4-(trifluoromethoxy)phenyl)acetamide

Under an ice bath and argon atmosphere, a solution of 2-bromoacetyl bromide **24b** (1.37 g, 6.77 mmol) in dichloromethane (5 mL) was added dropwise to a solution of 4-(trifluoromethoxy)aniline (1 g, 5.65 mmol) and 4-dimethylaminopyridine (689.74 mg, 5.65 mmol) in dichloromethane (10 mL). The reaction was performed at room temperature for 18 h. Water (20 mL) was added to the reaction mixture, and the mixture was extracted with dichloromethane (20 mL × 3). The combined organic phases were washed with saturated sodium chloride solution (20 mL), dried over anhydrous sodium sulfate, filtered, and concentrated under reduced pressure. The residue was purified by silica gel column chromatography (eluent: System A) to obtain 2-bromo-N-(4-(trifluoromethoxy)phenyl)acetamide **95b** (1.59 g) with a yield of 94.49%.

MS m/z (ESI): 298.0 [M+1]

### Step 2

### tert-butyl 4-(2-bromo-5-ethyl-7-oxo-4-(2-oxo-2-((4-(trifluoromethoxy)phenyl)amino)ethyl)-4,7-dihydro-[1, 2,4]triazolo[1,5-a]pyrimidin-6-yl)piperazine-1-carboxylate

At room temperature, 2-bromo-N-(4-(trifluoromethoxy)phenyl)acetamide **95b** (272.04 mg, 912.72 µmol), tert-butyl 4-(2-bromo-5-ethyl-7-oxo-4,7-dihydro-[1,2,4]triazolo[1,5-a]pyrimidin-6-yl)piperazine-1-carboxy late **1a** (300 mg, 702.09 µmol), and N,N-diisopropylethylamine (272.21 mg, 2.11 mmol) were added to N,N-dimethylformamide (7 mL). The temperature was raised to 70 °C, and the reaction was performed for 3 h. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The residue was purified by silica gel column chromatography (eluent: System B) to obtain tert-butyl 4-(2-bromo-5-ethyl-7-oxo-4-(2-oxo-2-((4-(trifluoromethoxy)phenyl)amino)ethyl)-4,7-dihydro-[1, 2,4]triazolo[1,5-a]pyrimidin-6-yl)piperazine-1-carboxylate **95c** (450 mg) with a yield of 99.46%.

MS m/z (ESI): 588.1 [M+H-56]

### Step 3

### tert-butyl 4-(2-(cyclohept-1-en-1-yl)-5-ethyl-7-oxo-4-(2-oxo-2-((4-(trifluoromethoxy)phenyl)amino)ethyl)-4,7-dihydro-[1,2,4]triazolo[1,5-a]pyrimidin-6-yl)piperazine-1-carboxylate

At room temperature, tert-butyl 4-(2-bromo-5-ethyl-7-oxo-4-(2-oxo-2-((4-(trifluoromethoxy)phenyl)amino)ethyl)-4,7-dihydro-[1, 2,4]triazolo[1,5-a]pyrimidin-6-yl)piperazine-1-carboxylate **95c** (250 mg, 387.93 µmol), 2-(cyclohept-1-en-1-yl)-4,4,5,5-tetramethyl-1,3,2-dioxaborolane **4a** (103.41 mg, 465.52 µmol), [1,1'-bis(diphenylphosphino)ferrocene]palladium(II) dichloride dichloromethane complex (31.44 mg, 38.79 µmol), and sodium carbonate (123.35 mg, 1.16 mmol) were dissolved in 1,4-dioxane (5 mL) and water (0.5 mL). The mixture was subject to argon replacement 3 times. The temperature was raised to 85 °C, and the reaction was performed for 1.5 h. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The residue was purified by silica gel column chromatography (eluent: System B) to obtain tert-butyl 4-(2-(cyclohept-1-en-1-yl)-5-ethyl-7-oxo-4-(2-oxo-2-((4-(trifluoromethoxy)phenyl)amino)ethyl)-4,7-dihydro-[1,2,4]triazolo[1,5-a] pyrimidin-6-yl)piperazine-1-carboxylate **95d** (170 mg) with a yield of 66.43%.

MS m/z (ESI): 604.3 [M+H-56]

### Step 4

### 2-(2-(cyclohept-1-en-1-yl)-5-ethyl-7-oxo-6-(piperazin-1-yl)-[1,2,4]triazolo[1,5-a]pyrimidin-4(7H )-yl)-N-(4-(trifluoromethoxy)phenyl)acetamide

At room temperature, trifluoroacetic acid (1 mL) was added to a solution of tert-butyl 4-(2-(cyclohept-1-en-1-yl)-5-ethyl-7-oxo-4-(2-oxo-2-((4-(trifluoromethoxy)phenyl)amino)ethyl)-4,7-dihydro-[1,2,4]triazolo[1,5-a]pyrimidin-6-yl)piperazine-1-carboxylate **95d** (70 mg, 106.11 µmol) in dichloromethane (4 mL). The reaction was performed at room temperature for 40 min. The mixture was concentrated under reduced pressure to obtain 2-(2-(cyclohept-1-en-1-yl)-5-ethyl-7-oxo-6-(piperazin-1-yl)-[1,2,4]triazolo[1,5-a]pyrimidin-4(7H )-yl)-N-(4-(trifluoromethoxy) phenyl)acetamide **95e** (59 mg) as a crude product, which was used directly in the next step.

MS m/z (ESI): 560.3 [M+1]

### Step 5

### 2-(2-(cyclohept-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(4-(trifluoromethoxy)phenyl)acetamide

At room temperature, 5-hydroxy-6-methylpyrimidine-4-carboxylic acid **27a** (24.38 mg, 158.15 µmol), 1-(3-dimethylaminopropyl)-3-ethylcarbodiimide hydrochloride (38.24 mg, 199.46 µmol), 1-hydroxybenzotriazole (26.95 mg, 199.46 µmol), and N,N-diisopropylethylamine (64.44 mg, 498.64 µmol) were added to N,N-dimethylformamide (1.5 mL). The reaction was performed at room temperature for 30 min. 2-(2-(cyclohept-1-en-1-yl)-5-ethyl-7-oxo-6-(piperazin-1-yl)-[1,2,4]triazolo[1,5-a]pyrimidin-4(7H )-yl)-N-(4-(trifluoromethoxy)phenyl)acetamide **95e** (59 mg, 105.44 µmol) was added, and the reaction was performed at room temperature for 18 h. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain 2-(2-(cyclohept-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(4-(trifluoromethoxy)phenyl)acetamide 95 (8.16 mg) with a yield of 10.61%.

MS m/z (ESI): 696.3 [M+1]
¹H NMR (400 MHz, DMSO-d6) δ 10.69 (s, 1H), 10.28 (s, 1H), 8.59 (s, 1H), 7.79 - 7.58 (m, 2H), 7.34 (d, J = 8.6 Hz, 2H), 7.05 (t, J = 6.8 Hz, 1H), 5.15 (s, 2H), 4.52 (d, J = 12.6 Hz, 1H), 3.50 (q, J = 11.5, 10.2 Hz, 3H), 3.35 - 3.19 (m, 1H), 3.08 - 2.87 (m, 3H), 2.85 - 2.77 (m, 1H), 2.77 - 2.69 (m, 2H), 2.63 (d, J= 11.4 Hz, 1H), 2.45 (s, 3H), 2.31 (d, J = 9.4 Hz, 2H), 1.77 (d, J = 8.7 Hz, 2H), 1.51 (d, J = 11.5 Hz, 4H), 1.16 (t, J = 7.4 Hz, 3H).

### Example 96

### 2-(2-(benzofuran-2-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(4-(pentafluoro-λ⁶-sulfaneyl)phenyl)acetamid e

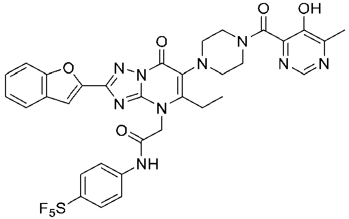

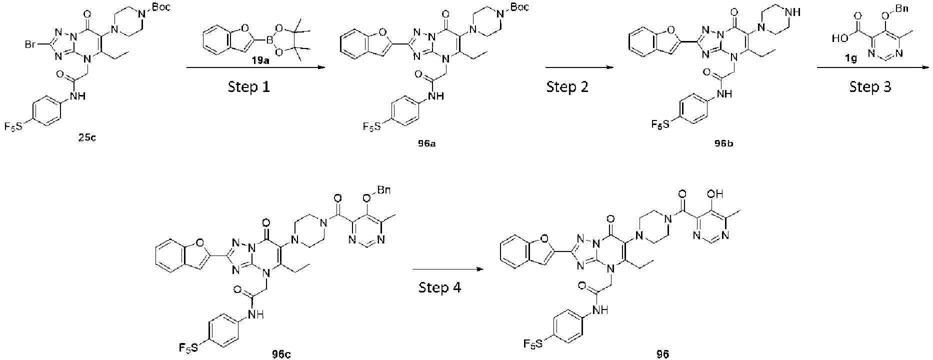

### Step 1

### tert-butyl 4-(2-(benzofuran-2-yl)-5-ethyl-7-oxo-4-(2-oxo-2-((4-(pentafluoro-λ⁶-sulfaneyl)phenyl)amino)eth yl)-4,7-dihydro-[1,2,4]triazolo[1,5-a]pyrimidin-6-yl)piperazine-1-carboxylate

At room temperature, tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)piperazine-1-carboxylate **25c** (80 mg, 116.53 µmol), 2-(benzofuran-2-yl)-4,4,5,5-tetramethyl-1,3,2-dioxaborolane **19a** (36.98 mg, 151.49 µmol), [1,1'-bis(diphenylphosphino)ferrocene]palladium(II) dichloride dichloromethane complex (9.45 mg, 11.65 µmol), and sodium carbonate (37.05 mg, 349.60 µmol) were dissolved in a mixed solution of 1,4-dioxane (7 mL) and water (1 mL). The mixture was subject to argon replacement three times, then heated to 85 °C, and the reaction was performed for 2 h. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The residue was purified by silica gel column chromatography (eluent: System B) to obtain tert-butyl 4-(2-(benzofuran-2-yl)-5-ethyl-7-oxo-4-(2-oxo-2-((4-(pentafluoro-λ⁶-sulfaneyl)phenyl)amino)eth yl)-4,7-dihydro-[1,2,4]triazolo[1,5-a]pyrimidin-6-yl)piperazine-1-carboxylate **96a** (84 mg) with a yield of 99.60%.

MS m/z (ESI): 668.2 [M-56+H]

### Step 2

### 2-(2-(benzofuran-2-yl)-5-ethyl-7-oxo-6-(piperazin-1-yl)-[1,2,4]triazolo[1,5-a]pyrimidin-4(7H)-yl )-N-(4-(pentafluoro-λ⁶-sulfaneyl)phenyl)acetamide

At room temperature, trifluoroacetic acid (1 mL) was added to a solution of tert-butyl 4-(2-(benzofuran-2-yl)-5-ethyl-7-oxo-4-(2-oxo-2-((4-(pentafluoro-λ⁶-sulfaneyl)phenyl)amino)eth yl)-4,7-dihydro-[1,2,4]triazolo[1,5-a]pyrimidin-6-yl)piperazine-1-carboxylate **96a** (84 mg, 116.07 µmol) in dichloromethane (4 mL), and the reaction was performed at room temperature for 40 min. The mixture was concentrated under reduced pressure to obtain 2-(2-(benzofuran-2-yl)-5-ethyl-7-oxo-6-(piperazin-1-yl)-[1,2,4]triazolo[1,5-a]pyrimidin-4(7H)-yl )-N-(4-(pentafluoro-λ⁶-sulfaneyl)phenyl)acetamide **96b** (72 mg) as a crude product, which was used directly in the next step.

MS m/z (ESI): 624.3 [M+1]

### Step 3

### 2-(2-(benzofuran-2-yl)-6-(4-(5-(benzyloxy)-6-methylpyrimidine-4-carbonyl)piperazin-1-yl)-5-eth yl-7-oxo-[1,2,4]triazolo[1,5-a]pyrimidin-4(7H)-yl)-N-(4-(pentafluoro-λ⁶-sulfaneyl)phenyl)acetam ide

At room temperature, 5-(benzyloxy)-6-methylpyrimidine-4-carboxylic acid **1g** (42.30 mg, 173.19 µmol), 1-(3-dimethylaminopropyl)-3-ethylcarbodiimide hydrochloride (44.27 mg, 230.92 µmol), 1-hydroxybenzotriazole (31.20 mg, 230.92 µmol), and N,N-diisopropylethylamine (74.61 mg, 577.30 µmol) were dissolved in N,N-dimethylformamide (3 mL), and the reaction was performed at room temperature for 40 min. A solution of 2-(2-(benzofuran-2-yl)-5-ethyl-7-oxo-6-(piperazin-1-yl)-[1,2,4]triazolo[1,5-a]pyrimidin-4(7H)-yl )-N-(4-(pentafluoro-λ⁶-sulfaneyl)phenyl) acetamide **96b** (72 mg, 115.46 µmol) in N,N-dimethylformamide (3 mL) was added, and the reaction was performed at room temperature overnight. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The residue was purified by silica gel column chromatography (eluent: System B) to obtain 2-(2-(benzofuran-2-yl)-6-(4-(5-(benzyloxy)-6-methylpyrimidine-4-carbonyl)piperazin-1-yl)-5-eth yl-7-oxo-[1,2,4]triazolo[1,5-a]pyrimidin-4(7H)-yl)-N-(4-(pentafluoro-λ⁶-sulfaneyl)phenyl)acetam ide **96c** (60 mg) with a yield of 61.15%.

MS m/z (ESI): 850.3 [M+1]

### Step 4

### 2-(2-(benzofuran-2-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(4-(pentafluoro-λ⁶-sulfaneyl)phenyl)acetamid e

At room temperature, trifluoroacetic acid (4 mL) was added to a solution of 2-(2-(benzofuran-2-yl)-6-(4-(5-(benzyloxy)-6-methylpyrimidine-4-carbonyl)piperazin-1-yl)-5-eth yl-7-oxo-[1,2,4]triazolo[1,5-a]pyrimidin-4(7H)-yl)-N-(4-(pentafluoro-λ⁶-sulfaneyl)phenyl)acetam ide **96c** (60 mg, 70.60 µmol) in dichloromethane (1 mL), and the reaction was performed at 30 °C for 18 h. The mixture was concentrated under reduced pressure, and the resulting residue was separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain 2-(2-(benzofuran-2-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(4-(pentafluoro-λ⁶-sulfaneyl)phenyl)acetamid e 96 (9.97 mg) with a yield of 17.84%.

MS m/z (ESI): 760.3 [M+1]
¹H NMR (400 MHz, DMSO-d6) δ 11.04 (s, 1H), 10.27 (s, 1H), 8.60 (s, 1H), 7.90 (d, J = 9.0 Hz, 2H), 7.81 (d, J = 9.0 Hz, 2H), 7.74 (dd, J = 12.8, 8.0 Hz, 2H), 7.62 (s, 1H), 7.43 (t, J = 8.1 Hz, 1H), 7.33 (t, J = 7.5 Hz, 1H), 5.30 (s, 2H), 4.56 (d, J = 12.5 Hz, 1H), 3.55 (td, J = 12.2, 11.7, 6.0 Hz, 3H), 3.29 (t, J = 12.4 Hz, 1H), 3.03 (q, J = 11.4, 9.4 Hz, 3H), 2.87 (d, J = 11.2 Hz, 1H), 2.70 (d, J = 11.0 Hz, 1H), 2.46 (s, 3H), 1.21 (t, J = 7.5 Hz, 3H).

### Example 97

### 2-(2-(benzofuran-5-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(4-(pentafluoro-λ⁶-sulfaneyl)phenyl)acetamid e

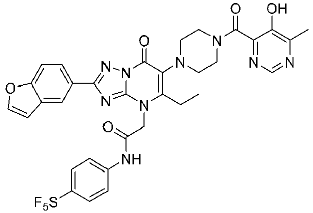

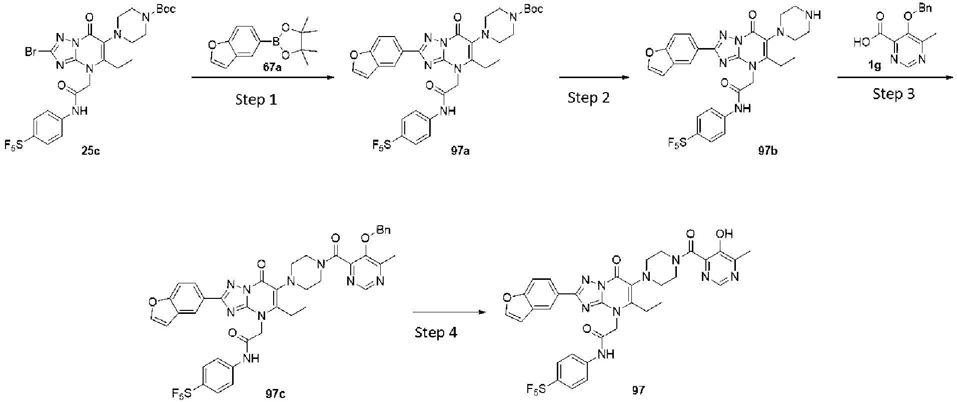

### Step 1

### tert-butyl 4-(2-(benzofuran-5-yl)-5-ethyl-7-oxo-4-(2-oxo-2-((4-(pentafluoro-λ⁶-sulfaneyl)phenyl)amino)eth yl)-4,7-dihydro-[1,2,4]triazolo[1,5-a]pyrimidin-6-yl)piperazine-1-carboxylate

At room temperature, tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)piperazine-1-carboxylate 25c (80 mg, 116.53 µmol), 2-(benzofuran-5-yl)-4,4,5,5-tetramethyl-1,3,2-dioxaborolane 67a (36.98 mg, 151.49 µmol), [1,1'-bis(diphenylphosphino)ferrocene]palladium(II) dichloride (9.45 mg, 11.65 µmol), and sodium carbonate (37.05 mg, 349.60 µmol) were dissolved in a mixed solution of 1,4-dioxane (7 mL) and water (1 mL). The mixture was subject to argon replacement three times, then heated to 85 °C, and the reaction was performed for 2 hours. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The residue was purified by silica gel column chromatography (eluent: System B) to obtain tert-butyl 4-(2-(benzofuran-5-yl)-5-ethyl-7-oxo-4-(2-oxo-2-((4-(pentafluoro-λ⁶-sulfaneyl)phenyl)amino)eth yl)-4,7-dihydro-[1,2,4]triazolo[1,5-a]pyrimidin-6-yl)piperazine-1-carboxylate **97a** (50 mg) in a yield of 59.29%.

MS m/z (ESI): 668.3 [M+H-56]

### Step 2

### 2-(2-(benzofuran-5-yl)-5-ethyl-7-oxo-6-(piperazin-1-yl)-[1,2,4]triazolo[1,5-a]pyrimidin-4(7H)-yl )-N-(4-(pentafluoro-λ⁶-sulfaneyl)phenyl)acetamide

Trifluoroacetic acid (1 mL) was added to a solution of tert-butyl 4-(2-(benzofuran-5-yl)-5-ethyl-7-oxo-4-(2-oxo-2-((4-(pentafluoro-λ⁶-sulfaneyl)phenyl)amino)eth yl)-4,7-dihydro-[1,2,4] triazolo[1,5-a]pyrimidin-6-yl)piperazine-1-carboxylate **97a** (50 mg, 69.09 µmol) in dichloromethane (4 mL) at room temperature, and the reaction was performed at room temperature for 40 minutes. The mixture was concentrated under reduced pressure to obtain 2-(2-(benzofuran-5-yl)-5-ethyl-7-oxo-6-(piperazin-1-yl)-[1,2,4]triazolo[1,5-a]pyrimidin-4(7H)-yl )-N-(4-(pentafluoro-λ⁶-sulfaneyl)phenyl)acetamide **97b** (43 mg) as a crude product, which was used directly in the next step.

MS m/z (ESI): 624.3 [M+1]

### Step 3

### 2-(2-(benzofuran-5-yl)-6-(4-(5-(benzyloxy)-6-methylpyrimidine-4-carbonyl)piperazin-1-yl)-5-eth yl-7-oxo-[1,2,4]triazolo[1,5-a]pyrimidin-4(7H)-yl)-N-(4-(pentafluoro-λ⁶-sulfaneyl)phenyl)acetam ide

At room temperature, 5-(benzyloxy)-6-methylpyrimidine-4-carboxylic acid **1g** (25.26 mg, 103.43 µmol), 1-(3-dimethylaminopropyl)-3-ethylcarbodiimide hydrochloride (26.44 mg, 137.91 µmol), 1-hydroxybenzotriazole (18.63 mg, 137.91 µmol), and N,N-diisopropylethylamine (44.56 mg, 344.77 µmol) were added to N,N-dimethylformamide (3 mL), and the reaction was performed at room temperature for 40 minutes. A solution of 2-(2-(benzofuran-5-yl)-5-ethyl-7-oxo-6-(piperazin-1-yl)-[1,2,4]triazolo[1,5-a]pyrimidin-4(7H)-yl )-N-(4-(pentafluoro-λ⁶-sulfaneyl)phenyl) acetamide **97b** (43 mg, 68.95 µmol) in N,N-dimethylformamide (3 mL) was then added. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The residue was purified by silica gel column chromatography (eluent: System B) to obtain 2-(2-(benzofuran-5-yl)-6-(4-(5-(benzyloxy)-6-methylpyrimidine-4-carbonyl)piperazin-1-yl)-5-eth yl-7-oxo-[1,2,4]triazolo[1,5-a]pyrimidin-4(7H)-yl)-N-(4-(pentafluoro-λ⁶-sulfaneyl)phenyl)acetam ide **97c** (50 mg) in a yield of 85.32%.

MS m/z (ESI): 850.3 [M+1]

### Step 4

### 2-(2-(benzofuran-5-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(4-(pentafluoro-λ⁶-sulfaneyl)phenyl)acetamid e

Trifluoroacetic acid (4 mL) was added to a solution of 2-(2-(benzofuran-5-yl)-6-(4-(5-(benzyloxy)-6-methylpyrimidine-4-carbonyl)piperazin-1-yl)-5-eth yl-7-oxo-[1,2,4]triazolo[1,5-a]pyrimidin-4(7H)-yl)-N-(4-(pentafluoro-λ⁶-sulfaneyl)phenyl)acetam ide **97c** (50 mg, 58.84 µmol) in dichloromethane (1 mL) at room temperature, and the reaction was performed at 30 °C for 48 hours. The mixture was concentrated under reduced pressure, and the resulting residue was separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain 2-(2-(benzofuran-5-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(4-(pentafluoro-λ⁶-sulfaneyl)phenyl)acetamid e 97 (17.56 mg) in a yield of 38.03%.

MS m/z (ESI): 760.3 [M+1]
¹H NMR (400 MHz, DMSO-d6) δ 11.04 (s, 1H), 10.27 (s, 1H), 8.60 (s, 1H), 8.40 (d, J = 1.6 Hz, 1H), 8.18 - 8.02 (m, 2H), 7.90 (d, J = 9.3 Hz, 2H), 7.80 (d, J= 8.9 Hz, 2H), 7.72 (d, J = 8.7 Hz, 1H), 7.08 (d, J = 2.1 Hz, 1H), 5.30 (s, 2H), 4.55 (d, J = 12.4 Hz, 1H), 3.61 - 3.48 (m, 3H), 3.35 - 3.21 (m, 1H), 3.01 (q, J= 8.5, 5.4 Hz, 3H), 2.86 (d, J = 11.3 Hz, 1H), 2.69 (d, J = 10.9 Hz, 1H), 2.46 (s, 3H), 1.21 (q, J = 7.5, 6.0 Hz, 3H).

### Example 98

### 2-(2-(benzo[b]thiophen-2-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(4-(pentafluoro-λ⁶-sulfaneyl)phenyl)acet amide

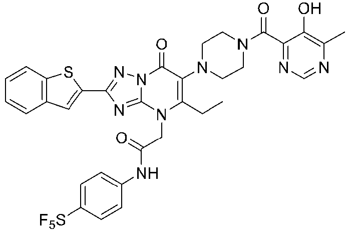

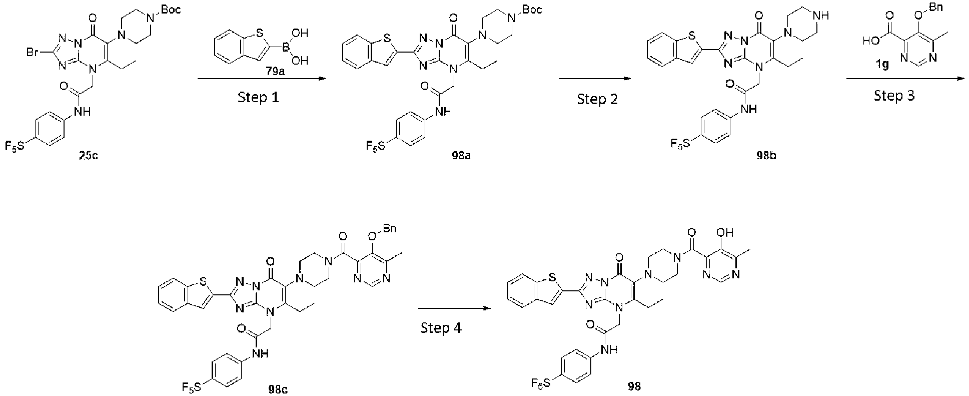

### Step 1

### tert-butyl 4-(2-(benzo[b]thiophen-2-yl)-5-ethyl-7-oxo-4-(2-oxo-2-((4-(pentafluoro-λ⁶-sulfaneyl)phenyl)ami no)ethyl)-4,7-dihydro-[1,2,4]triazolo[1,5-a]pyrimidin-6-yl)piperazine-1-carboxylate

At room temperature, tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)piperazine-1-carboxylate **25c** (90 mg, 131.10 µmol), benzo[b]thiophen-2-ylboronic acid **79a** (30.34 mg, 170.43 µmol), [1,1'-bis(diphenylphosphino)ferrocene]palladium(II) dichloride dichloromethane complex (10.63 mg, 13.11 µmol), and sodium carbonate (41.69 mg, 393.30 µmol) were dissolved in a mixed solution of 1,4-dioxane (7 mL) and water (1 mL). The mixture was subject to argon replacement three times, then heated to 85 °C and reacted for 2 h. Water (20 mL) was added to the reaction mixture, and the mixture was extracted with ethyl acetate (20 mL×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 4-(2-(benzo[b]thiophen-2-yl)-5-ethyl-7-oxo-4-(2-oxo-2-((4-(pentafluoro-λ⁶-sulfaneyl)phenyl)ami no)ethyl)-4,7-dihydro-[1,2,4]triazolo[1,5-a]pyrimidin-6-yl)piperazine-1-carboxylate **98a** (75 mg) with a yield of 77.12%.

MS m/z (ESI): 684.2 [M+1-56]

### Step 2

### 2-(2-(Benzo[b]thiophen-2-yl)-5-ethyl-7-oxo-6-(piperazin-1-yl)-[1,2,4]triazolo[1,5-a]pyrimidin-4( 7H)-yl)-N-(4-(pentafluoro-λ⁶-sulfaneyl)phenyl)acetamide

At room temperature, trifluoroacetic acid (1 mL) was added to a solution of tert-butyl 4-(2-(benzo[b]thiophen-2-yl)-5-ethyl-7-oxo-4-(2-oxo-2-((4-(pentafluoro-λ⁶-sulfaneyl)phenyl)ami no) ethyl)-4,7-dihydro-1,2,4]triazolo[1,5-a]pyrimidin-6-yl)piperazine-1-carboxylate **98a** (75 mg, 101.38 µmol) in dichloromethane (4 mL). The reaction was performed at room temperature for 40 min. The mixture was concentrated under reduced pressure to obtain 2-(2-(benzo[b]thiophen-2-yl)-5-ethyl-7-oxo-6-(piperazin-1-yl)-[1,2,4]triazolo[1,5-a]pyrimidin-4( 7H)-yl)-N-(4-(pentafluoro-λ⁶-sulfaneyl)phenyl)acetamide **98b** (64 mg) as a crude product, which was used directly in the next step.

MS m/z (ESI): 640.2 [M+1]

### Step 3

### 2-(2-(Benzo[b]thiophen-2-yl)-6-(4-(5-(benzyloxy)-6-methylpyrimidine-4-carbonyl)piperazin-1-yl )-5-ethyl-7-oxo-[1,2,4]triazolo[1,5-a]pyrimidin-4(7H)-yl)-N-(4-(pentafluoro-λ⁶-sulfaneyl)phenyl) acetamide

At room temperature, 5-(benzyloxy)-6-methylpyrimidine-4-carboxylic acid **1g** (36.66 mg, 150.08 µmol), 1-(3-dimethylaminopropyl)-3-ethylcarbodiimide hydrochloride (38.36 mg, 200.11 µmol), 1-hydroxybenzotriazole (27.04 mg, 200.11 µmol), and N,N-diisopropylethylamine (64.65 mg, 500.26 µmol) were dissolved in N,N-dimethylformamide (3 mL). The reaction was performed at room temperature for 40 min. Then a solution of 2-(2-(benzo[b]thiophen-2-yl)-5-ethyl-7-oxo-6-(piperazin-1-yl)-[1,2,4]triazolo[1,5-a]pyrimidin-4( 7H)-yl)-N-(4-(pentafluoro-λ⁶-sulfaneyl)phenyl) acetamide **98b** (64 mg, 100.05 µmol) in N,N-dimethylformamide (3 mL) was added, and the reaction was performed at room temperature for 18 h. Water (20 mL) was added to the reaction mixture, and the mixture was extracted with ethyl acetate (20 mL ×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(2-(benzo[b]thiophen-2-yl)-6-(4-(5-(benzyloxy)-6-methylpyrimidine-4-carbonyl)piperazin-1-yl )-5-ethyl-7-oxo-[1,2,4]triazolo[1,5-a]pyrimidin-4(7H)-yl)-N-(4-(pentafluoro-1⁶-sulfaneyl)phenyl) acetamide **98c** (50 mg) with a yield of 57.71%.

MS m/z (ESI): 866.2 [M+1]

### Step 4

### 2-(2-(Benzo[b]thiophen-2-yl)-5-ethyl-6-(4-(5-hydroxy-6-methylpyrimidine-4-carbonyl)piperazin -1-yl)-7-oxo-[1,2,4]triazolo[1,5-a]pyrimidin-4(7H)-yl)-N-(4-(pentafluoro-λ⁶-sulfaneyl)phenyl)ace tamide

At room temperature, trifluoroacetic acid (4 mL) was added to a solution of 2-(2-(benzo[b]thiophen-2-yl)-6-(4-(5-(benzyloxy)-6-methylpyrimidine-4-carbonyl)piperazin-1-yl )-5-ethyl-7-oxo-[1,2,4]triazolo[1,5-a]pyrimidin-4(7H)-yl)-N-(4-(pentafluoro-λ⁶-sulfaneyl)phenyl) acetamide **98c** (50 mg, 57.74 µmol) in dichloromethane (1 mL). The reaction was performed at room temperature for 48 h. The mixture was concentrated under reduced pressure, and the resulting residue was separated by preparative liquid chromatography (separation column AKZONOBEL Kromasil; 250 ×21.2 mm I.D.; 5 µm, 20 mL/min; mobile phase A: 0.05% TFA + H₂O, mobile phase B: CH₃CN) to obtain 2-(2-(benzo[b]thiophen-2-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(4-(pentafluoro-λ⁶-sulfaneyl)phenyl)acet amide **98** (22.43 mg) with a yield of 49.07%.

MS m/z (ESI): 776.2 [M+1]
¹H NMR (400 MHz, DMSO-d6) δ 11.04 (s, 1H), 10.27 (s, 1H), 8.60 (s, 1H), 8.11 (s, 1H), 8.08-8.02 (m, 1H), 8.01-7.94 (m, 1H), 7.90 (d, J = 9.0 Hz, 2H), 7.81 (d, J = 8.9 Hz, 2H), 7.44 (qd, J = 7.6, 3.7 Hz, 2H), 5.29 (s, 2H), 4.55 (d, J = 12.5 Hz, 1H), 3.54 (td, J = 12.7, 12.0, 6.8 Hz, 3H), 3.34-3.20 (m, 1H), 3.02 (td, J = 10.0, 6.0 Hz, 3H), 2.86 (d, J = 11.3 Hz, 1H), 2.69 (d, J = 10.7 Hz, 1H), 2.46 (s, 3H), 1.20 (t, J = 7.4 Hz, 3H).

### Example 99

### N-(2-Chloro-4-(trifluoromethyl)phenyl)-2-(5-ethyl-6-(4-(5-hydroxy-6-methylpyrimidine-4-carbo nyl)piperazin-1-yl)-2-(2-methyl-2H-indazol-5-yl)-7-oxo-[1,2,4]triazolo[1,5-a]pyrimidin-4(7H)-yl )acetamide

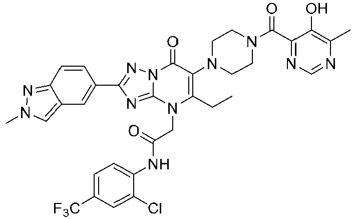

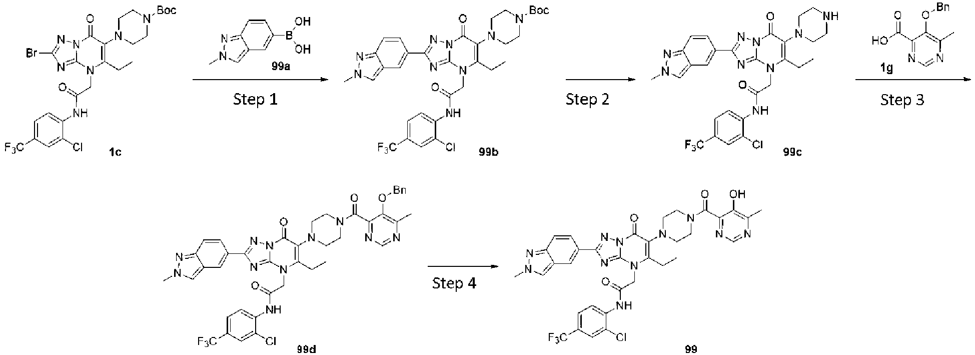

### Step 1

### tert-butyl 4-(4-(2-((2-chloro-4-(trifluoromethyl)phenyl)amino)-2-oxoethyl)-5-ethyl-2-(2-methyl-2H-indazol -5-yl)-7-oxo-4,7-dihydro-[1,2,4]triazolo[1,5-a]pyrimidin-6-yl)piperazine-1-carboxylate

At room temperature, tert-butyl 4-(2-bromo-4-(2-((2-chloro-4-(trifluoromethyl) phenyl)amino)-2-oxoethyl)-5-ethyl-7-oxo-4,7-dihydro-[1,2,4]triazolo[1,5-a]pyrimidin-6-yl)piper azine-1-carboxylate **1c** (100 mg, 150.86 µmol), (2-methyl-2H-indazol-5-yl)boronic acid (31.86 mg, 181.03 µmol), [1,1'-bis(diphenylphosphino)ferrocene]palladium(II) dichloride dichloromethane complex (12.23 mg, 15.09 µmol), and sodium carbonate (47.97 mg, 452.57 µmol) were dissolved in 1,4-dioxane (5 mL) and water (1 mL). The mixture was subject to argon replacement three times, then heated to 85 °C and reacted for 2 h. Water (20 mL) was added to the reaction mixture, followed by extraction with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(4-(2-((2-chloro-4-(trifluoromethyl)phenyl)amino)-2-oxoethyl)-5-ethyl-2-(2-methyl-2H-indazol -5-yl)-7-oxo-4,7-dihydro-[1,2,4]triazolo[1,5-a]pyrimidin-6-yl)piperazine-1-carboxylate **99b** (70 mg) with a yield of 64.98%.

MS m/z (ESI): 714.3 [M+1]

### Step 2

### N-(2-chloro-4-(trifluoromethyl)phenyl)-2-(5-ethyl-2-(2-methyl-2H-indazol-5-yl)-7-oxo-6-(pipera zin-1-yl)-[1,2,4]triazolo[1,5-a]pyrimidin-4(7H)-yl)acetamide

At room temperature, trifluoroacetic acid (1 mL) was added to a solution of tert-butyl 4-(4-(2-((2-chloro-4-(trifluoromethyl)phenyl)amino)-2-oxoethyl)-5-ethyl-2-(2-methyl-2H-indazol -5-yl)-7-oxo-4,7-dihydro-[1,2,4]triazolo[1,5-a]pyrimidin-6-yl)piperazine-1-carboxylate **99b** (70 mg, 98.02 µmol) in dichloromethane (4 mL), and the reaction was performed at room temperature for 40 min. The mixture was concentrated under reduced pressure to obtain N-(2-chloro-4-(trifluoromethyl)phenyl)-2-(5-ethyl-2-(2-methyl-2H-indazol-5-yl)-7-oxo-6-(pipera zin-1-yl)-[1,2,4]triazolo[1,5-a]pyrimidin-4(7H)-yl)acetamide **99c** (60 mg), which was used directly in the next step without further purification.

MS m/z (ESI): 614.3 [M+1]

### Step 3

### 2-(6-(4-(5-(benzyloxy)-6-methylpyrimidine-4-carbonyl)piperazin-1-yl)-5-ethyl-2-(2-methyl-2H-i ndazol-5-yl)-7-oxo-[1,2,4]triazolo[1,5-a]pyrimidin-4(7H)-yl)-N-(2-chloro-4-(trifluoromethyl)phe nyl)acetamide

At room temperature, 5-(benzyloxy)-6-methylpyrimidine-4-carboxylic acid **1g** (35.80 mg, 146.57 µmol), 1-(3-dimethylaminopropyl)-3-ethylcarbodiimide hydrochloride (37.46 mg, 195.43 µmol), 1-hydroxybenzotriazole (26.41 mg, 195.43 µmol), and N,N-diisopropylethylamine (63.14 mg, 488.58 µmol) were dissolved in N,N-dimethylformamide (3 mL) and reacted at room temperature for 40 min. Then a solution of N-(2-chloro-4-(trifluoromethyl)phenyl)-2-(5-ethyl-2-(2-methyl-2H-indazol-5-yl)-7-oxo-6-(pipera zin-1-yl)-[1,2,4]triazolo[1,5-a]pyrimidin-4(7H)-yl)acetamide **99c** (60 mg, 97.72 µmol) in N,N-dimethylformamide (3 mL) was added, and the reaction was performed at room temperature for 18 h. Water (20 mL) was added to the reaction mixture, followed by extraction with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-5-ethyl-2-(2-methyl-2H-i ndazol-5-yl)-7-oxo-[1,2,4]triazolo[1,5-a]pyrimidin-4(7H)-yl)-N-(2-chloro-4-(trifluoromethyl)phe nyl)acetamide **99d** (82 mg) with a yield of 99.87%.

MS m/z (ESI): 840.3 [M+1]

### Step 4

### N-(2-chloro-4-(trifluoromethyl)phenyl)-2-(5-ethyl-6-(4-(5-hydroxy-6-methylpyrimidine-4-carbon yl)piperazin-1-yl)-2-(2-methyl-2H-indazol-5-yl)-7-oxo-[1,2,4]triazolo[1,5-a]pyrimidin-4(7H)-yl) acetamide

At room temperature, trifluoroacetic acid (4 mL) was added to a solution of 2-(6-(4-(5-(benzyloxy)-6-methylpyrimidine-4-carbonyl)piperazin-1-yl)-5-ethyl-2-(2-methyl-2H-i ndazol-5-yl)-7-oxo-[1,2,4]triazolo[1,5-a]pyrimidin-4(7H)-yl)-N-(2-chloro-4-(trifluoromethyl)phe nyl) acetamide **99d** (82 mg, 97.59 µmol) in dichloromethane (1 mL), and the reaction was performed at 30 °C for 24 h. The mixture was concentrated under reduced pressure, and the resulting residue was separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-2-(2-methyl-2H-indazol-5-yl)-7-oxo-[1,2,4] triazolo[1,5-a]pyrimidin-4(7H)-yl)acetamide **99** (43.65 mg) with a yield of 58.43%.

MS m/z (ESI): 750.3 [M+1]
¹H NMR (400 MHz, DMSO-d6) δ 10.43 (s, 1H), 10.26 (s, 1H), 8.59 (s, 1H), 8.51 (d, J = 8.9 Hz, 2H), 8.08 (d, J = 8.6 Hz, 1H), 8.03 - 7.93 (m, 2H), 7.79 - 7.59 (m, 2H), 5.42 (s, 2H), 4.55 (d, J = 12.5 Hz, 1H), 4.20 (s, 3H), 3.53 (dd, J = 14.4, 6.0 Hz, 3H), 3.28 (t, J = 12.4 Hz, 1H), 3.03 (d, J = 9.2 Hz, 3H), 2.86 (d, J = 11.2 Hz, 1H), 2.68 (d, J = 10.7 Hz, 1H), 2.46 (s, 3H), 1.23 (t, J = 7.4 Hz, 3H).

### Example 100

### 2-(2-(benzo[d]thiazol-5-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)aceta mide

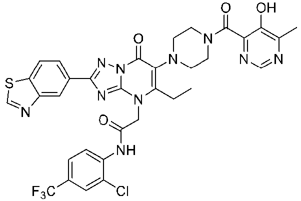

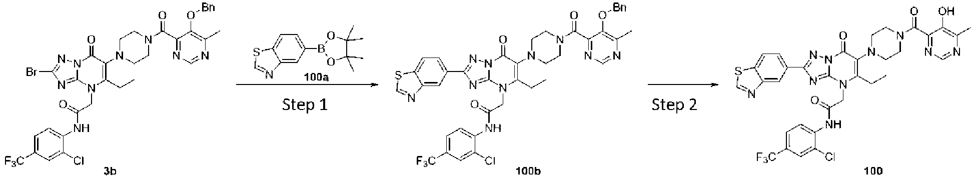

### Step 1

### 2-(2-(benzo[d]thiazol-5-yl)-6-(4-(5-(benzyloxy)-6-methylpyrimidine-4-carbonyl)piperazin-1-yl)-5-ethyl-7-oxo-[1,2,4]triazolo[1,5-a]pyrimidin-4(7H)-yl)-N-(2-chloro-4-(trifluoromethyl)phenyl)a cetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (60 mg, 76.05 µmol), 5-(4,4,5,5-tetramethyl-1,3,2-dioxaborolan-2-yl)benzo[d]thiazole **100a** (23.83 mg, 91.25 µmol, commercially available), [1,1'-bis(diphenylphosphino)ferrocene]palladium(II) dichloride dichloromethane complex (6.16 mg, 7.60 µmol), and sodium carbonate (24.18 mg, 228.14 µmol) were dissolved in a mixed solution of 1,4-dioxane (7 mL) and water (1 mL). The mixture was subject to argon replacement three times, then heated to 85 °C, and the reaction was performed for 2 h. Water (20 mL) was added to the reaction mixture, which was extracted with ethyl acetate (20 mL × 3). The combined organic phase was washed with saturated sodium chloride solution (20 mL), dried over anhydrous sodium sulfate, filtered, and concentrated under reduced pressure. The resulting residue was purified by silica gel column chromatography (eluent: System B) to give 2-(2-(benzo[d]thiazol-5-yl)-6-(4-(5-(benzyloxy)-6-methylpyrimidine-4-carbonyl)piperazin-1-yl)-5-ethyl-7-oxo-[1,2,4]triazolo[1,5-a]pyrimidin-4(7H)-yl)-N-(2-chloro-4-(trifluoromethyl)phenyl)a cetamide **100b** (40 mg) with a yield of 62.38%.

MS m/z (ESI): 843.3 [M+1]

### Step 2

### 2-(2-(benzo[d]thiazol-5-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)aceta mide

At room temperature, trifluoroacetic acid (4 mL) was added to a solution of 2-(2-(benzo[d]thiazol-5-yl)-6-(4-(5-(benzyloxy)-6-methylpyrimidine-4-carbonyl)piperazin-1-yl)-5-ethyl-7-oxo-[1,2,4]triazolo[1,5-a]pyrimidin-4(7H)-yl)-N-(2-chloro-4-(trifluoromethyl) phenyl)acetamide **100b** (40 mg, 47.43 µmol) in dichloromethane (1 mL). The reaction was performed at 30 °C for 48 h. The mixture was concentrated under reduced pressure, and the resulting residue was separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to give 2-(2-(benzo[d]thiazol-5-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)aceta mide **100** (8.92 mg) with a yield of 24.47%.

MS m/z (EST): 753.2 [M+1]
¹H NMR (400 MHz, DMSO-d6) δ 10.46 (s, 1H), 10.25 (s, 1H), 9.50 (s, 1H), 8.76 (d, J = 1.5 Hz, 1H), 8.59 (s, 1H), 8.33 (d, J = 8.4 Hz, 1H), 8.23 (dd, J = 8.4, 1.6 Hz, 1H), 8.06 (d, J = 8.5 Hz, 1H), 7.98 (d, J = 2.1 Hz, 1H), 7.71 (dd, J = 8.8, 2.1 Hz, 1H), 5.44 (s, 2H), 4.55 (d, J = 12.4 Hz, 1H), 3.60 - 3.50 (m, 3H), 3.28 (t, J = 12.5 Hz, 1H), 3.04 (tt, J = 12.8, 8.1 Hz, 3H), 2.86 (d, J = 11.4 Hz, 1H), 2.69 (d, J = 11.0 Hz, 1H), 2.46 (s, 3H), 1.23 (t, J = 7.4 Hz, 3H).

### Example 101

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(1H-pyrrolo[2,3-b]pyridin-5-yl)-[1,2,4]triazolo[1,5-a]pyrimidin-4(7H) -yl)acetamide

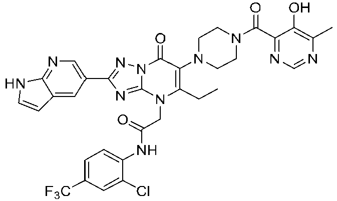

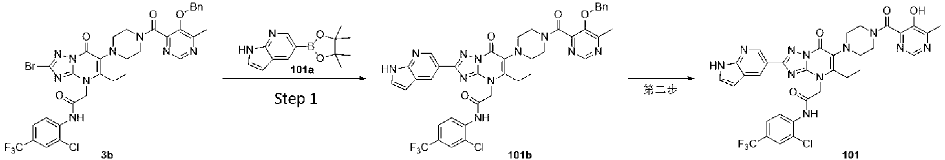

### Step 1

### 2-(6-(4-(5-(benzyloxy)-6-methylpyrimidine-4-carbonyl)piperazin-1-yl)-5-ethyl-7-oxo-2-(1H-pyrr olo[2,3-b]pyridin-5-yl)-[1,2,4]triazolo[1,5-a]pyrimidin-4(7H)-yl)-N-(2-chloro-4-(trifluoromethyl) phenyl)acetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (80 mg, 101.39 µmol), 5-(4,4,5,5-tetramethyl-1,3,2-dioxaborolan-2-yl)-1H-pyrrolo[2,3-b]pyridine **101a** (29.70 mg, 121.67 µmol), [1,1'-bis(diphenylphosphino)ferrocene]palladium(II) dichloride dichloromethane complex (8.22 mg, 10.14 µmol), and sodium carbonate (32.24 mg, 304.18 µmol) were added to a mixture of 1,4-dioxane (7 mL) and water (1 mL). The mixture was subject to argon replacement three times, then heated to 85 °C, and the reaction was performed for 2 h. Water (20 mL) was added to the reaction mixture, which was extracted with ethyl acetate (20 mL × 3). The combined organic phase was washed with saturated sodium chloride solution (20 mL), dried over anhydrous sodium sulfate, filtered, and concentrated under reduced pressure. The resulting residue was purified by silica gel column chromatography (eluent: System B) to give 2-(6-(4-(5-(benzyloxy)-6-methylpyrimidine-4-carbonyl)piperazin-1-yl)-5-ethyl-7-oxo-2-(1H-pyrr olo[2,3-b]pyridin-5-yl)-[1,2,4]triazolo[1,5-a]pyrimidin-4(7H)-yl)-N-(2-chloro-4-(trifluoromethyl) phenyl)acetamide **101b** (70 mg) with a yield of 83.56%.

MS m/z (ESI): 826.3 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(1H-pyrrolo[2,3-b]pyridin-5-yl)-[1,2,4]triazolo[1,5-a]pyrimidin-4(7H) -yl)acetamide

At room temperature, trifluoroacetic acid (4 mL) was added to a solution of 2-(6-(4-(5-(benzyloxy)-6-methylpyrimidine-4-carbonyl)piperazin-1-yl)-5-ethyl-7-oxo-2-(1H-pyrr olo[2,3-b]pyridin-5-yl)-[1,2,4]triazolo[1,5-a]pyrimidin-4(7H)-yl)-N-(2-chloro-4-(trifluoromethyl) phenyl) acetamide **101b** (70 mg, 84.72 µmol) in dichloromethane (1 mL), and the reaction was performed at 30 °C for 24 h. The mixture was concentrated under reduced pressure, and the resulting residue was separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(1H-pyrrolo[2,3-b]pyridin-5-yl)-[1,2,4]triazolo[1,5-a]pyrimidin-4(7H) -yl)acetamide **101** (19.77 mg) with a yield of 31.07%.

MS m/z (EST): 736.2 [M+1]
¹H NMR (400 MHz, DMSO-d6) δ 11.91 (d, J = 2.7 Hz, 1H), 10.44 (s, 1H), 10.25 (s, 1H), 8.96 (d, J = 2.0 Hz, 1H), 8.64 (d, J = 2.0 Hz, 1H), 8.59 (s, 1H), 8.07 (d, J = 8.6 Hz, 1H), 7.98 (d, J = 2.0 Hz, 1H), 7.71 (dd, J = 8.6, 2.1 Hz, 1H), 7.64 - 7.48 (m, 1H), 6.59 (dd, J = 3.5, 1.9 Hz, 1H), 5.42 (s, 2H), 4.55 (d, J = 12.5 Hz, 1H), 3.63 - 3.42 (m, 3H), 3.28 (t, J = 12.4 Hz, 1H), 3.03 (d, J = 10.4 Hz, 3H), 2.85 (d, J = 11.2 Hz, 1H), 2.68 (d, J = 10.2 Hz, 1H), 2.45 (s, 3H), 1.22 (t, J= 7.4 Hz, 3H).

### Example 102

### N-(2-chloro-4-(trifluoromethyl)phenyl)-2-(5-ethyl-6-(4-(5-hydroxy-6-methylpyrimidine-4-carbon yl)piperazin-1-yl)-2-(1H-indol-5-yl)-7-oxo-[1,2,4]triazolo[1,5-a]pyrimidin-4(7H)-yl)acetamide

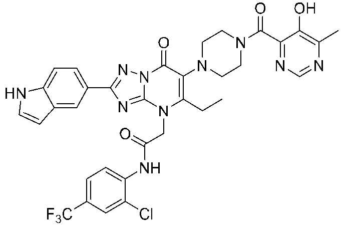

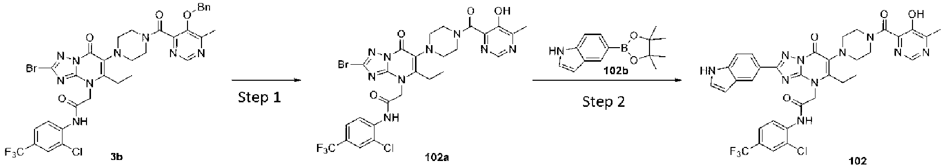

### Step 1

### 2-(2-bromo-5-ethyl-6-(4-(5-hydroxy-6-methylpyrimidine-4-carbonyl)piperazin-1-yl)-7-oxo-[1,2, 4]triazolo[1,5-a]pyrimidin-4(7H)-yl)-N-(2-chloro-4-(trifluoromethyl)phenyl)acetamide

At room temperature, trifluoroacetic acid (10 mL) was added to a solution of 2-(6-(4-(5-(benzyloxy)-6-methylpyrimidine-4-carbonyl)piperazin-1-yl)-2-bromo-5-ethyl-7-oxo-[ 1,2,4] triazolo[1,5-a]pyrimidin-4(7H)-yl)-N-(2-chloro-4-(trifluoromethyl)phenyl)acetamide **3b** (600 mg, 760.46 µmol) in dichloromethane (2 mL), and the reaction was performed at 30°C for 48 h. Water (20 mL) was added to the reaction mixture, and the mixture was extracted with ethyl acetate (20 mL ×3). The combined organic phase was washed with saturated sodium chloride solution (20 mL), dried over anhydrous sodium sulfate, filtered, and concentrated under reduced pressure. The resulting residue was purified by silica gel column chromatography (eluent: System B) to obtain 2-(2-bromo-5-ethyl-6-(4-(5-hydroxy-6-methylpyrimidine-4-carbonyl)piperazin-1-yl)-7-oxo-[1,2, 4]triazolo[1,5-a]pyrimidin-4(7H)-yl)-N-(2-chloro-4-(trifluoromethyl)phenyl)acetamide **102a** (280 mg) with a yield of 52.68%.

MS m/z (ESI): 698.1 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-2-(1H-indol-5-yl)-7-oxo-[1,2,4]triazolo[1,5-a]pyrimidin-4(7H)-yl)acetamide

At room temperature, 2-(2-bromo-5-ethyl-6-(4-(5-hydroxy-6-methylpyrimidine-4-carbonyl)piperazin-1-yl)-7-oxo-[1,2, 4]triazolo[1,5-a]pyrimidin-4(7H)-yl)-N-(2-chloro-4-(trifluoromethyl)phenyl)acetamide **102a** (60 mg, 85.85 µmol), 5-(4,4,5,5-tetramethyl-1,3,2-dioxaborolan-2-yl)-1H-indole **102b** (25.05 mg, 103.02 µmol), [1,1'-bis(diphenylphosphino)ferrocene]palladium(II) dichloride dichloromethane complex (6.96 mg, 8.59 µmol), and sodium carbonate (27.30 mg, 257.56 µmol) were added to a mixed solution of 1,4-dioxane (4 mL) and water (0.5 mL). The mixture was subject to argon replacement three times, then heated to 85 °C, and the reaction was performed for 2 h. Water (20 mL) was added to the reaction mixture, and the mixture was extracted with ethyl acetate (20 mL ×3). The combined organic phase was washed with saturated sodium chloride solution (20 mL), dried over anhydrous sodium sulfate, filtered, and concentrated under reduced pressure. The resulting residue was purified by silica gel column chromatography (eluent: System B), concentrated under reduced pressure, and then separated by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-2-(1H-indol-5-yl)-7-oxo-[1,2,4]triazolo[1,5-a]pyrimidin-4(7H)-yl)acetamide 102 (21.11 mg) with a yield of 32.11%.

MS m/z (EST): 735.3 [M+1]
¹H NMR (400 MHz, DMSO-d6) δ 11.33 (s, 1H), 10.43 (s, 1H), 10.25 (s, 1H), 8.59 (s, 1H), 8.35 (d, J = 1.5 Hz, 1H), 8.08 (d, J = 8.6 Hz, 1H), 7.98 (d, J = 2.0 Hz, 1H), 7.88 (dd, J = 8.5, 1.6 Hz, 1H), 7.71 (dd, J = 8.9, 2.1 Hz, 1H), 7.49 (d, J = 8.5 Hz, 1H), 7.42 (t, J = 2.7 Hz, 1H), 6.54 (t, J = 2.4 Hz, 1H), 5.41 (s, 2H), 4.55 (d, J = 12.5 Hz, 1H), 3.55 (d, J = 10.7 Hz, 3H), 3.27 (t, J = 12.5 Hz, 1H), 3.01 (t, J = 9.5 Hz, 3H), 2.85 (d, J = 11.3 Hz, 1H), 2.67 (d, J = 10.6 Hz, 1H), 2.45 (s, 3H), 1.22 (t, J = 7.4 Hz, 3H).

### Example 103

### N-(2-chloro-4-(trifluoromethyl)phenyl)-2-(5-ethyl-6-(4-(5-hydroxy-6-methylpyrimidine-4-carbon yl)piperazin-1-yl)-2-(1-methyl-1H-indol-5-yl)-7-oxo-[1,2,4]triazolo[1,5-a]pyrimidin-4(7H)-yl)ac etamide

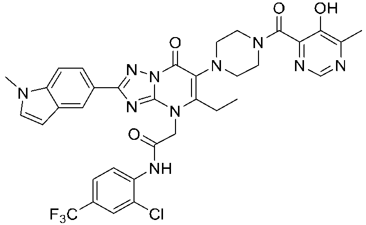

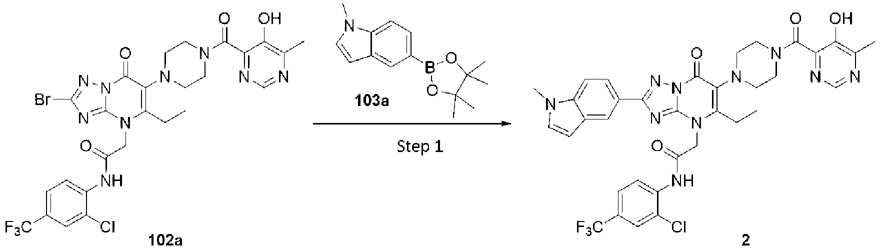

At room temperature, 2-(2-bromo-5-ethyl-6-(4-(5-hydroxy-6-methylpyrimidine-4-carbonyl)piperazin-1-yl)-7-oxo-[1,2, 4]triazolo[1,5-a]pyrimidin-4(7H)-yl)-N-(2-chloro-4-(trifluoromethyl)phenyl)acetamide **102a** (60 mg, 85.85 µmol), 1-methyl-5-(4,4,5,5-tetramethyl-1,3,2-dioxaborolan-2-yl)-1H-indole **103a** (26.49 mg, 103.02 µmol), [1,1'-bis(diphenylphosphino)ferrocene]palladium(II) dichloride dichloromethane complex (6.96 mg, 8.59 µmol), and sodium carbonate (27.30 mg, 257.56 µmol) were dissolved in a mixture of 1,4-dioxane (4 mL) and water (0.5 mL). The mixture was subject to argon replacement three times, then was heated to 85 °C and the reaction was performed for 2 hours. Water (20 mL) was added to the reaction mixture, and the mixture was extracted with ethyl acetate (20 mL × 3). The combined organic phase was washed with saturated sodium chloride solution (20 mL), dried over anhydrous sodium sulfate, filtered, and concentrated under reduced pressure. The resulting residue was purified by silica gel column chromatography (eluent: System B), and after concentration under reduced pressure, was separated by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to give N-(2-chloro-4-(trifluoromethyl)phenyl)-2-(5-ethyl-6-(4-(5-hydroxy-6-methylpyrimidine-4-carbon yl)piperazin-1-yl)-2-(1-methyl-1H-indol-5-yl)-7-oxo-[1,2,4]triazolo[1,5-a]pyrimidin-4(7H)-yl)ac etamide **103** (1.36 mg) with a yield of 2.09%.

MS m/z (ESI): 749.2 [M+1]
¹H NMR (400 MHz, DMSO-d6) δ 10.44 (s, 1H), 10.25 (s, 1H), 8.59 (s, 1H), 8.35 (d, J = 1.5 Hz, 1H), 8.08 (d, J = 8.6 Hz, 1H), 8.03 - 7.89 (m, 2H), 7.71 (dd, J = 8.8, 2.0 Hz, 1H), 7.55 (d, J = 8.7 Hz, 1H), 7.41 (d, J = 3.1 Hz, 1H), 6.55 (d, J = 3.1 Hz, 1H), 5.42 (s, 2H), 4.55 (d, J = 12.5 Hz, 1H), 3.83 (s, 3H), 3.62 - 3.53 (m, 3H), 3.27 (t, J = 12.6 Hz, 1H), 3.10 - 2.94 (m, 3H), 2.85 (d, J = 11.2 Hz, 1H), 2.67 (d, J = 10.5 Hz, 1H), 2.45 (s, 3H), 1.22 (t, J = 7.3 Hz, 3H).

### Example 104

### N-(2-chloro-4-(trifluoromethyl)phenyl)-2-(2-(2,3-dihydrobenzofuran-5-yl)-5-ethyl-6-(4-(3-hydro xypicolinoyl)piperazin-1-yl)-7-oxo-[1,2,4]triazolo[1,5-a]pyrimidin-4(7H)-yl)acetamide

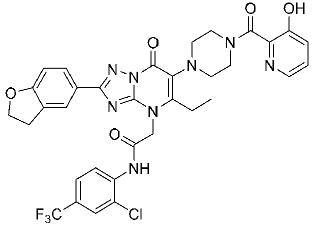

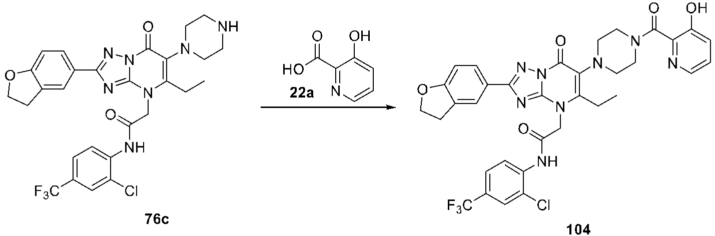

At room temperature, 3-hydroxypicolinic acid **22a** (20.80 mg, 149.50 µmol), 1-(3-dimethylaminopropyl)-3-ethylcarbodiimide hydrochloride (38.21 mg, 199.33 µmol), 1-hydroxybenzotriazole (26.93 mg, 199.33 µmol), and N,N-diisopropylethylamine (64.40 mg, 498.33 µmol) were dissolved in N,N-dimethylformamide (2.5 mL) and reacted at room temperature for 40 minutes. To the mixture was added a solution of N-(2-chloro-4-(trifluoromethyl)phenyl)-2-(2-(2,3-dihydrobenzofuran-5-yl)-5-ethyl-7-oxo-6-(pipe razin-1-yl)-[1,2,4]triazolo[1,5-a]pyrimidin -4(7H)-yl)acetamide **76c** (60 mg, 99.67 µmol) in N,N-dimethylformamide (2.5 mL), and the reaction was performed at 30 °C for 18 hours. Water (20 mL) was added to the reaction mixture, and the mixture was extracted with ethyl acetate (20 mL × 3). The combined organic phase was washed with saturated sodium chloride solution (20 mL), dried over anhydrous sodium sulfate, filtered, and concentrated under reduced pressure. The resulting residue was purified by silica gel column chromatography (eluent: System B), and after concentration under reduced pressure, was separated by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to give N-(2-chloro-4-(trifluoromethyl)phenyl)-2-(2-(2,3-dihydrobenzofuran-5-yl)-5-ethyl-6-(4-(3-hydro xypicolinoyl)piperazin-1-yl)-7-oxo-[1,2,4]triazolo[1,5-a]pyrimidin-4(7H)-yl)acetamide **104** (10.21 mg) with a yield of 13.60%.

MS m/z (ESI): 723.2 [M+1]
¹H NMR (400 MHz, DMSO-d6) δ 10.51 (s, 1H), 10.42 (s, 1H), 8.17 - 8.03 (m, 2H), 7.97 (d, J = 2.3 Hz, 2H), 7.88 (dd, J = 8.3, 1.8 Hz, 1H), 7.72 (dd, J = 8.8, 2.1 Hz, 1H), 7.33 (d, J = 3.8 Hz, 2H), 6.88 (d, J = 8.4 Hz, 1H), 5.38 (s, 2H), 4.59 (q, J = 12.7, 10.7 Hz, 3H), 3.56 - 3.48 (m, 2H), 3.43 (d, J = 12.5 Hz, 1H), 3.25 (t, J = 8.9 Hz, 3H), 3.00 (dq, J = 17.4, 10.6, 8.5 Hz, 3H), 2.83 (d, J = 11.1 Hz, 1H), 2.65 (d, J = 11.1 Hz, 1H), 1.21 (t, J = 7.4 Hz, 3H).

### Example 105

### N-(2-chloro-4-(trifluoromethyl)phenyl)-2-(2-(2,3-dihydrobenzofuran-5-yl)-5-ethyl-6-(4-(3-hydroxy-6-methylpicolinoyl)piperazin-1-yl)-7-oxo-[1,2,4]triazolo[1,5-a]pyrimidin-4(7H)-yl)a cetamide

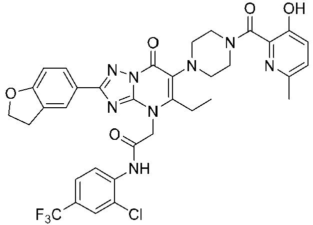

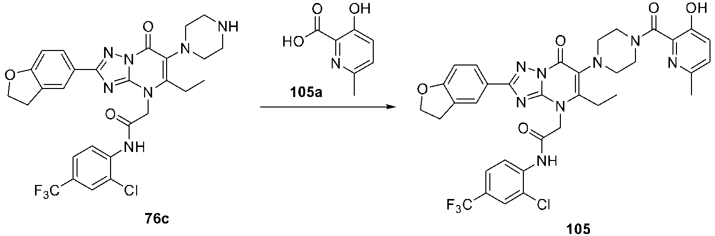

At room temperature, 3-hydroxy-6-methylpicolinic acid **105a** (22.89 mg, 149.50 µmol, commercially available), 1-(3-dimethylaminopropyl)-3-ethylcarbodiimide hydrochloride (38.21 mg, 199.33 µmol), 1-hydroxybenzotriazole (26.93 mg, 199.33 µmol), and N,N-diisopropylethylamine (64.40 mg, 498.33 µmol) were dissolved in N,N-dimethylformamide (3 mL) and reacted at room temperature for 40 minutes. To the mixture was added a solution of N-(2-chloro-4-(trifluoromethyl)phenyl)-2-(2-(2,3-dihydrobenzofuran-5-yl)-5-ethyl-7-oxo-6-(pipe razin-1-yl)-[1,2,4]triazolo[1,5-a]pyrimidin-4(7H)-yl)acetamide **76c** (60 mg, 99.67 µmol) in N,N-dimethylformamide (3 mL), and the reaction was performed at room temperature overnight. Water (20 mL) was added to the reaction mixture, and the mixture was extracted with ethyl acetate (20 mL × 3). The combined organic phase was washed with saturated sodium chloride solution (20 mL), dried over anhydrous sodium sulfate, filtered, and concentrated under reduced pressure. The residue was separated by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to give N-(2-chloro-4-(trifluoromethyl)phenyl)-2-(2-(2,3-dihydrobenzofuran-5-yl)-5-ethyl-6-(4-(3-hydro xy-6-methylpicolinoyl)piperazin-1-yl)-7-oxo-[1,2,4]triazolo[1,5-a]pyrimidin-4(7H)-yl)acetamide 105 (13.37 mg) with a yield of 17.29%.

MS m/z (ESI): 737.3 [M+1]
¹H NMR (400 MHz, DMSO-d6) δ 10.51 (s, 1H), 10.41 (s, 1H), 8.06 (d, J = 8.6 Hz, 1H), 7.97 (d, J = 2.3 Hz, 2H), 7.87 (dd, J = 8.3, 1.8 Hz, 1H), 7.72 (dd, J = 8.8, 2.1 Hz, 1H), 7.35 (d, J = 8.5 Hz, 1H), 7.26 (d, J = 8.6 Hz, 1H), 6.88 (d, J = 8.3 Hz, 1H), 5.38 (s, 2H), 4.60 (t, J = 8.7 Hz, 3H), 3.49 (dd, J = 18.7, 10.8 Hz, 3H), 3.25 (t, J = 8.5 Hz, 3H), 3.13 - 2.92 (m, 3H), 2.83 (d, J = 11.3 Hz, 1H), 2.66 (d, J = 11.2 Hz, 1H), 2.43 (s, 3H), 1.21 (t, J = 7.4 Hz, 3H).

### Example 106

### N-(2-chloro-4-(trifluoromethyl)phenyl)-2-(2-(2,3-dihydrobenzofuran-5-yl)-6-(4-(5-hydroxy-6-me thylpyrimidine-4-carbonyl)piperazin-1-yl)-5-methyl-7-oxo-[1,2,4]triazolo[1,5-a]pyrimidin-4(7H) -yl)acetamide

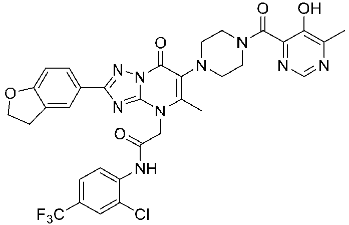

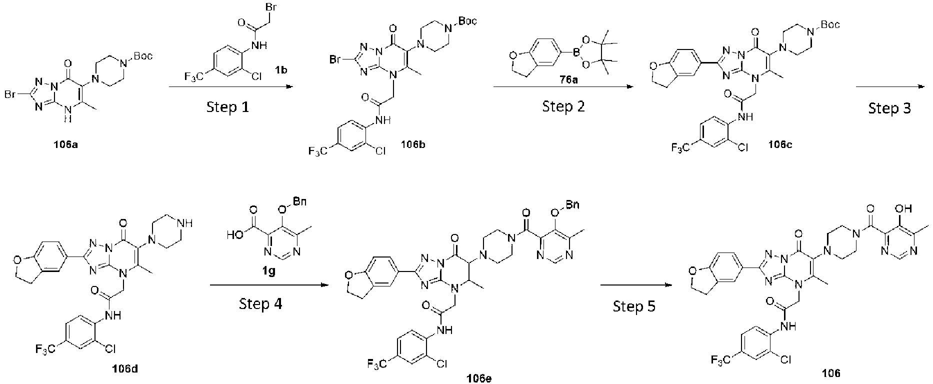

### Step 1

### tert-butyl 4-(2-bromo-4-(2-((2-chloro-4-(trifluoromethyl)phenyl)amino)-2-oxoethyl)-5-methyl-7-oxo-4,7-di hydro-[1,2,4]triazolo[1,5-a]pyrimidin-6-yl)piperazine-1-carboxylate

At room temperature, tert-butyl 4-(2-bromo-5-methyl-7-oxo-4,7-dihydro-[1,2,4]triazolo[1,5-a]pyrimidin-6-yl)piperazine-1-carbo xylate **106a** (200 mg, 483.95 µmol, prepared according to published patent WO2022249060), 2-bromo-N-(2-chloro-4-(trifluoromethyl)phenyl)acetamide **1b** (168.49 mg, 532.34 µmol), and N,N-diisopropylethylamine (312.73 mg, 2.42 mmol) were dissolved in N,N-dimethylformamide (10 mL), the temperature was raised to 50 °C, and the reaction was performed for 2 h. Water (20 mL) was added to the reaction mixture, the mixture was extracted with ethyl acetate (20 mL ×3), the combined organic phases were washed with saturated sodium chloride solution (20 mL), dried over anhydrous sodium sulfate, filtered, and concentrated under reduced pressure, and the obtained residue was purified by silica gel column chromatography (eluent: System B) to obtain tert-butyl 4-(2-bromo-4-(2-((2-chloro-4-(trifluoromethyl)phenyl)amino)-2-oxoethyl)-5-methyl-7-oxo-4,7-di hydro-[1,2,4]triazolo[1,5-a]pyrimidin-6-yl)piperazine-1-carboxylate **106b** (240 mg) with a yield of 76.43%.

MS m/z (ESI): 592.1 [M+H-56]

### Step 2

### tert-butyl 4-(4-(2-((2-chloro-4-(trifluoromethyl)phenyl)amino)-2-oxoethyl)-2-(2,3-dihydrobenzofuran-5-yl) -5-methyl-7-oxo-4,7-dihydro-[1,2,4]triazolo[1,5-a]pyrimidin-6-yl)piperazine-1-carboxylate

At room temperature, tert-butyl 4-(2-bromo-4-(2-((2-chloro-4-(trifluoromethyl)phenyl)amino)-2-oxoethyl)-5-methyl-7-oxo-4,7-di hydro-[1,2,4]triazolo[1,5-a]pyrimidin-6-yl)piperazine-1-carboxylate **106b** (240 mg, 369.88 µmol), 2-(2,3-dihydrobenzofuran-5-yl)-4,4,5,5-tetramethyl-1,3,2-dioxaborolane **76a** (66.71 mg, 406.87 µmol), [1,1'-bis(diphenylphosphino)ferrocene]palladium(II) dichloride dichloromethane complex (29.98 mg, 36.99 µmol), and sodium carbonate (117.61 mg, 1.11 mmol) were dissolved in a mixed solution of 1,4-dioxane (10 mL) and water (1 mL), The mixture was subject to argon replacement three times, the temperature was raised to 80 °C, and the reaction was performed for 2 h. Water (20 mL) was added to the reaction mixture, the mixture was extracted with ethyl acetate (20 mL×3), the combined organic phases were washed with saturated sodium chloride solution (20 mL), dried over anhydrous sodium sulfate, filtered, and concentrated under reduced pressure, and the obtained residue was purified by silica gel column chromatography (eluent: System B) to obtain tert-butyl 4-(4-(2-((2-chloro-4-(trifluoromethyl)phenyl)amino)-2-oxoethyl)-2-(2,3-dihydrobenzofuran-5-yl) -5-methyl-7-oxo-4,7-dihydro-[1,2,4]triazolo[1,5-a]pyrimidin-6-yl)piperazine-1-carboxylate **106c** (150 mg) with a yield of 58.94%.

MS m/z (ESI): 632.2 [M+H-56]

### Step 3

### N-(2-chloro-4-(trifluoromethyl)phenyl)-2-(2-(2,3-dihydrobenzofuran-5-yl)-5-methyl-7-oxo-6-(pi perazin-1-yl)-[1,2,4]triazolo[1,5-a]pyrimidin-4(7H)-yl)acetamide

At room temperature, trifluoroacetic acid (1.5 mL) was added to a solution of tert-butyl 4-(4-(2-((2-chloro-4-(trifluoromethyl)phenyl)amino)-2-oxoethyl)-2-(2,3-dihydrobenzofuran-5-yl) -5-methyl-7-oxo-4,7-dihydro-[1,2,4]triazolo[1,5-a]pyrimidin-6-yl)piperazine-1-carboxylate **106c** (150 mg, 217.99 µmol) in dichloromethane (6 mL), and the reaction was performed at room temperature for 40 min. The mixture was concentrated under reduced pressure to obtain N-(2-chloro-4-(trifluoromethyl)phenyl)-2-(2-(2,3-dihydrobenzofuran-5-yl)-5-methyl-7-oxo-6-(pi perazin-1-yl)-[1,2,4]triazolo[1,5-a]pyrimidin-4(7H)-yl)acetamide **106d** (128 mg), and the crude product was used directly in the next step.

MS m/z (ESI): 588.2 [M+1]

### Step 4

### 2-(6-(4-(5-(benzyloxy)-6-methylpyrimidine-4-carbonyl)piperazin-1-yl)-2-(2,3-dihydrobenzofuran -5-yl)-5-methyl-7-oxo-[1,2,4]triazolo[1,5-a]pyrimidin-4(7H)-yl)-N-(2-chloro-4-(trifluoromethyl) phenyl)acetamide

At room temperature, 5-(benzyloxy)-6-methylpyrimidine-4-carboxylic acid **1g** (79.76 mg, 326.54 µmol), 1-(3-dimethylaminopropyl)-3-ethylcarbodiimide hydrochloride (83.46 mg, 435.39 µmol), 1-hydroxybenzotriazole (58.83 mg, 435.39 µmol), and N,N-diisopropylethylamine (140.67 mg, 1.09 mmol) were dissolved in N,N-dimethylformamide (2.5 mL), the reaction was performed at room temperature for 40 min, then a solution of N-(2-chloro-4-(trifluoromethyl)phenyl)-2-(2-(2,3-dihydrobenzofuran-5-yl)-5-methyl-7-oxo-6-(pi perazin-1-yl)-[1,2,4]triazolo[1,5-a]pyrimidin-4(7H)-yl)acetamide **106d** (128 mg, 217.69 µmol) in N,N-dimethylformamide (2.5 mL) was added, and the reaction was performed at room temperature for 18 h. Water (20 mL) was added to the reaction mixture, the mixture was extracted with ethyl acetate (20 mL×3), the combined organic phases were washed with saturated sodium chloride solution (20 mL), dried over anhydrous sodium sulfate, filtered, and concentrated under reduced pressure, and the obtained residue was purified by silica gel column chromatography (eluent: System B) to obtain 2-(6-(4-(5-(benzyloxy)-6-methylpyrimidine-4-carbonyl)piperazin-1-yl)-2-(2,3-dihydrobenzofuran -5-yl)-5-methyl-7-oxo-[1,2,4]triazolo[1,5-a]pyrimidin-4(7H)-yl)-N-(2-chloro-4-(trifluoromethyl) phenyl)acetamide **106e** (150 mg) with a yield of 84.63%.

MS m/z (ESI): 814.3 [M+1]

### Step 5

### N-(2-chloro-4-(trifluoromethyl)phenyl)-2-(2-(2,3-dihydrobenzofuran-5-yl)-6-(4-(5-hydroxy-6-me thylpyrimidine-4-carbonyl)plperazin-1-yl)-5-methyl-7-oxo-[1,2,4]triazolo[1,5-a]pyrimidin-4(7H) -yl)acetamide

At room temperature, trifluoroacetic acid (4 mL) was added to a solution of 2-(6-(4-(5-(benzyloxy)-6-methylpyrimidine-4-carbonyl)piperazin-1-yl)-2-(2,3-dihydrobenzofuran -5-yl)-5-methyl-7-oxo-[1,2,4]triazolo[1,5-a]pyrimidin-4(7H)-yl)-N-(2-chloro-4-(trifluoromethyl) phenyl)acetamide **106e** (150 mg, 184.23 µmol) in dichloromethane (1 mL), and the reaction was performed at 30 °C for 18 h. The reaction mixture was concentrated under reduced pressure and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(2-(2,3-dihydrobenzofuran-5-yl)-6-(4-(5-hydroxy-6-me thylpyrimidine-4-carbonyl)piperazin-1-yl)-5-methyl-7-oxo-[1,2,4]triazolo[1,5-a]pyrimidin-4(7H) -yl)acetamide 106 (53.41 mg) with a yield of 39.24%.

MS m/z (ESI): 724.2 [M+1]
¹H NMR (400 MHz, DMSO-d6) δ 10.43 (s, 1H), 10.25 (s, 1H), 8.59 (s, 1H), 8.07 (d, J = 8.6 Hz, 1H), 7.98 (s, 2H), 7.88 (d, J = 8.4 Hz, 1H), 7.72 (d, J = 8.6 Hz, 1H), 6.88 (d, J = 8.4 Hz, 1H), 5.40 (s, 2H), 4.61 (t, J = 8.7 Hz, 2H), 4.52 (d, J = 12.7 Hz, 1H), 3.50 (t, J = 10.8 Hz, 3H), 3.27 (q, J = 10.3, 8.5 Hz, 3H), 3.03 (t, J = 11.8 Hz, 1H), 2.82 (d, J = 11.3 Hz, 1H), 2.65 (d, J = 10.6 Hz, 1H), 2.61 (s, 3H), 2.45 (s, 3H).

### Example 107

### N-(2-chloro-4-(trifluoromethyl)phenyl)-2-(2-(2,3-dihydrobenzofuran-5-yl)-5-ethyl-6-(4-(5-hydro xy-6-methylpyrimidine-4-carbonyl)-1,4-diazepan-1-yl)-7-oxo-[1,2,4]triazolo[1,5-a]pyrimidin-4(7 H)-yl)acetamide

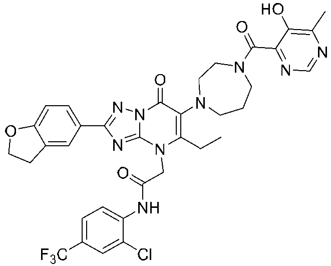

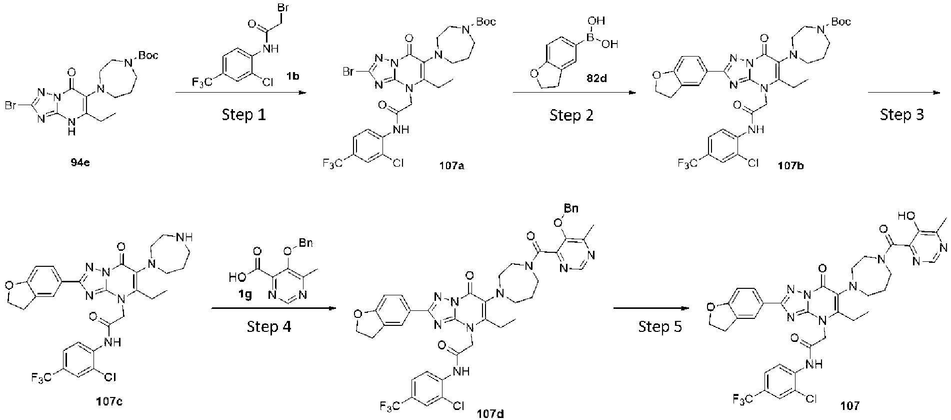

### Step 1

### tert-butyl 4-(2-bromo-4-(2-((2-chloro-4-(trifluoromethyl)phenyl)amino)-2-oxoethyl)-5-ethyl-7-oxo-4,7-dih ydro-[1,2,4]triazolo[1,5-a]pyrimidin-6-yl)-1,4-diazepane-1-carboxylate

At room temperature, tert-butyl 4-(2-bromo-5-ethyl-7-oxo-4,7-dihydro-[1,2,4]triazolo[1,5-a]pyrimidin-6-yl)-1,4-diazepane-1-car boxylate 94e (300 mg, 679.78 µmol), 2-bromo-N-(2-chloro-4-(trifluoromethyl)phenyl)acetamide 1b (236.67 mg, 747.75 µmol), and N,N-diisopropylethylamine (351.42 mg, 2.72 mmol) were added to N,N-dimethylformamide (15 mL), and the temperature was raised to 50 °C. The reaction was performed for 2 h. Water (20 mL) was added to the reaction mixture, which was extracted with ethyl acetate (20 mL×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(2-bromo-4-(2-((2-chloro-4-(trifluoromethyl)phenyl)amino)-2-oxoethyl)-5-ethyl-7-oxo-4,7-dih ydro-[1,2,4]triazolo[1,5-a]pyrimidin-6-yl)-1,4-diazepane-1-carboxylate **107a** (240 mg) with a yield of 52.16%.

MS m/z (ESI): 620.1 [M+H-56]

### Step 2

### tert-butyl 4-(4-(2-((2-chloro-4-(trifluoromethyl)phenyl)amino)-2-oxoethyl)-2-(2,3-dihydrobenzofuran-5-yl) -5-ethyl-7-oxo-4,7-dihydro-[1,2,4]triazolo[1,5-a]pyrimidin-6-yl)-1,4-diazepane-1-carboxylate

At room temperature, tert-butyl 4-(2-bromo-4-(2-((2-chloro-4-(trifluoromethyl)phenyl)amino)-2-oxoethyl)-5-ethyl-7-oxo-4,7-dih ydro-[1,2,4]triazolo[1,5-a]pyrimidin-6-yl)-1,4-diazepane-1-carboxylate **107a** (120 mg, 177.28 µmol), (2,3-dihydrobenzofuran-5-yl)boronic acid **82d** (31.97 mg, 195.00 µmol), [1,1'-bis(diphenylphosphino)ferrocene]palladium(II) dichloride dichloromethane complex (14.37 mg, 17.73 µmol), and sodium carbonate (56.37 mg, 531.83 µmol) were dissolved in a mixed solution of 1,4-dioxane (7 mL) and water (1 mL). The mixture was subject to argon replacement three times, and the temperature was raised to 85 °C. The reaction was performed for 2 h. After completion of the reaction was monitored, water (20 mL) was added to the reaction mixture, which was extracted with ethyl acetate (20 mL ×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(4-(2-((2-chloro-4-(trifluoromethyl)phenyl)amino)-2-oxoethyl)-2-(2,3-dihydrobenzofuran-5-yl) -5-ethyl-7-oxo-4,7-dihydro-[1,2,4]triazolo[1,5-a]pyrimidin-6-yl)-1,4-diazepane-1-carboxylate **107b** (120 mg) with a yield of 94.52%.

MS m/z (ESI): 660.3 [M+H-56]

### Step 3

### 2-(6-(1,4-diazepan-1-yl)-2-(2,3-dihydrobenzofuran-5-yl)-5-ethyl-7-oxo-[1,2,4]triazolo[1,5-a]pyri midin-4(7H)-yl)-N-(2-chloro-4-(trifluoromethyl)phenyl)acetamide

At room temperature, trifluoroacetic acid (1 mL) was added to a solution of tert-butyl 4-(4-(2-((2-chloro-4-(trifluoromethyl)phenyl)amino)-2-oxoethyl)-2-(2,3-dihydrobenzofuran-5-yl) -5-ethyl-7-oxo-4,7-dihydro-[1,2,4]triazolo[1,5-a]pyrimidin-6-yl)-1,4-diazepane-1-carboxylate **107b** (120 mg, 167.56 µmol) in dichloromethane (4 mL), and the reaction was performed at room temperature for 40 min. The mixture was concentrated under reduced pressure to obtain 2-(6-(1,4-diazepan-1-yl)-2-(2,3-dihydrobenzofuran-5-yl)-5-ethyl-7-oxo-[1,2,4]triazolo[1,5-a]pyri midin-4(7H)-yl)-N-(2-chloro-4-(trifluoromethyl)phenyl)acetamide **107c** (103 mg) as a crude product, which was used directly in the next step.

MS m/z (ESI): 616.3 [M+1]

### Step 4

### 2-(6-(4-(5-(benzyloxy)-6-methylpyrimidine-4-carbonyl)-1,4-diazepan-1-yl)-2-(2,3-dihydrobenzof uran-5-yl)-5-ethyl-7-oxo-[1,2,4]triazolo[1,5-a]pyrimidin-4(7H)-yl)-N-(2-chloro-4-(trifluoromethy l)phenyl)acetamide

At room temperature, 5-(benzyloxy)-6-methylpyrimidine-4-carboxylic acid 1g (61.26 mg, 250.80 µmol), 1-(3-dimethylaminopropyl)-3-ethylcarbodiimide hydrochloride (64.10 mg, 334.40 µmol), 1-hydroxybenzotriazole (45.18 mg, 334.40 µmol), and N,N-diisopropylethylamine (108.04 mg, 835.99 µmol) were added to N,N-dimethylformamide (3 mL), and the reaction was performed at room temperature for 40 minutes. Then, a solution of 2-(6-(1,4-diazepan-1-yl)-2-(2,3-dihydrobenzofuran-5-yl)-5-ethyl-7-oxo-[1,2,4]triazolo[1,5-a]pyri midin-4(7H)-yl)-N-(2-chloro-4-(trifluoromethyl)phenyl)acetamide **107c** (103 mg, 167.20 µmol) in N,N-dimethylformamide (3 mL) was added, and the reaction was performed at room temperature for 18 hours. Water (20 mL) was added to the reaction mixture, and the mixture was extracted with ethyl acetate (20 mL×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)-1,4-diazepan-1-yl)-2-(2,3-dihydrobenzof uran-5-yl)-5-ethyl-7-oxo-[1,2,4]triazolo[1,5-a]pyrimidin-4(7H)-yl)-N-(2-chloro-4-(trifluoromethy 1) phenyl)acetamide **107d** (130 mg) in a yield of 92.31%.

MS m/z (ESI): 842.3 [M+1]

### Step 5

### N-(2-chloro-4-(trifluoromethyl)phenyl)-2-(2-(2,3-dihydrobenzofuran-5-yl)-5-ethyl-6-(4-(5-hydro xy-6-methylpyrimidine-4-carbonyl)-1,4-diazepan-1-yl)-7-oxo-[1,2,4]triazolo[1,5-a]pyrimidin-4(7 H)-yl)acetamide

At room temperature, trifluoroacetic acid (4 mL) was added to a solution of 2-(6-(4-(5-(benzyloxy)-6-methylpyrimidine-4-carbonyl)-1,4-diazepan-1-yl)-2-(2,3-dihydrobenzof uran-5-yl)-5-ethyl-7-oxo-[1,2,4]triazolo[1,5-a]pyrimidin-4(7H)-yl)-N-(2-chloro-4-(trifluoromethy 1) phenyl)acetamide **107d** (130 mg, 154.35 µmol) in dichloromethane (1 mL), and the reaction was performed at room temperature for 48 hours. The reaction mixture was concentrated under reduced pressure, and then separated by preparative liquid chromatography (separation column AKZONOBEL Kromasil; 250×21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(2-(2,3-dihydrobenzofuran-5-yl)-5-ethyl-6-(4-(5-hydro xy-6-methylpyrimidine-4-carbonyl)-1,4-diazepan-1-yl)-7-oxo-[1,2,4]triazolo[1,5-a]pyrimidin-4(7 H)-yl)acetamide **107** (33.06 mg) in a yield of 27.91%.

MS m/z (ESI): 752.3 [M+1]
¹H NMR (400 MHz, DMSO-d6) δ 10.39 (d, J = 6.0 Hz, 2H), 8.59 (d, J = 12.0 Hz, 1H), 8.06 (t, J = 8.8 Hz, 1H), 7.97 (d, J = 5.4 Hz, 2H), 7.87 (ddd, J = 8.3, 6.3, 1.8 Hz, 1H), 7.72 (d, J = 8.6 Hz, 1H), 6.88 (dd, J = 8.3, 3.3 Hz, 1H), 5.35 (d, J = 9.8 Hz, 2H), 4.60 (td, J = 8.7, 2.4 Hz, 2H), 3.84 - 3.67 (m, 2H), 3.64 - 3.35 (m, 4H), 3.25 (td, J = 8.7, 3.0 Hz, 2H), 3.04 (hept, J = 7.5, 6.5 Hz, 2H), 3.02 - 2.74 (m, 2H), 2.45 (d, J = 7.9 Hz, 3H), 2.01 - 1.58 (m, 2H), 1.18 (dt, J = 25.4, 7.5 Hz, 3H).

### Example 108

### N-(1-(4-(2-((2-chloro-4-(trifluoromethyl)phenyl)amino)-2-oxoethyl)-2-(2,3-dihydrobenzofuran-5 -yl)-5-ethyl-7-oxo-4,7-dihydro-[1,2,4]triazolo[1,5-a]pyrimidin-6-yl)piperidin-4-yl)-5-hydroxy-6-methylpyrimidine-4-carboxamide

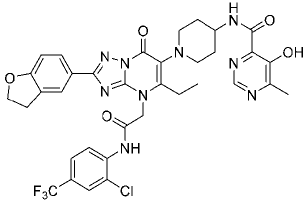

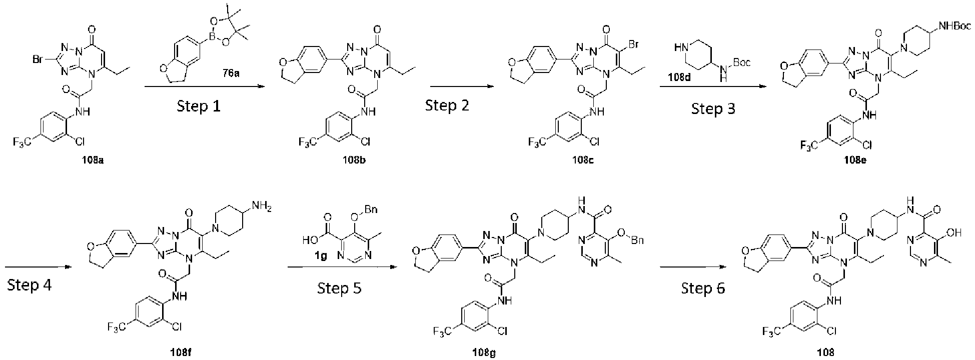

### Step 1

### N-(2-chloro-4-(trifluoromethyl)phenyl)-2-(2-(2,3-dihydrobenzofuran-5-yl)-5-ethyl-7-oxo-[1,2,4]t riazolo[1,5-a]pyrimidin-4(7H)-yl)acetamide

At room temperature, 2-(2-bromo-5-ethyl-7-oxo-[1,2,4]triazolo[1,5-a]pyrimidin-4(7H)-yl)-N-(2-chloro-4-(trifluoromet hyl)phenyl)acetamide **108a** (3.45 g, 7.21 mmol, prepared according to published patent WO2022249060), 2-(2,3-dihydrobenzofuran-5-yl)-4,4,5,5-tetramethyl-1,3,2-dioxaborolane 76a (1.36 g, 8.29 mmol), [1,1'-bis(diphenylphosphino)ferrocene]palladium(II) dichloride dichloromethane complex (584.25 mg, 720.78 µmol), and sodium carbonate (2.29 g, 21.62 mmol) were dissolved in a mixed solution of 1,4-dioxane (50 mL) and water (5 mL). The mixture was subject to argon replacement three times, heated to 75 °C, and the reaction was performed for 5 hours. Water (20 mL) was added to the reaction mixture, and the mixture was extracted with ethyl acetate (20 mL×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain N-(2-chloro-4-(trifluoromethyl)phenyl)-2-(2-(2,3-dihydrobenzofuran-5-yl)-5-ethyl-7-oxo-[1,2,4]t riazolo[1,5-a]pyrimidin-4(7H)-yl)acetamide **108b** (2.68 g) in a yield of 71.80%.

MS m/z (ESI): 518.1 [M+1]

### Step 2

### 2-(6-bromo-2-(2,3-dihydrobenzofuran-5-yl)-5-ethyl-7-oxo-[1,2,4]triazolo[1,5-a]pyrimidin-4(7H)-yl)-N-(2-chloro-4-(trifluoromethyl)phenyl)acetamide

At room temperature, N-bromosuccinimide (412.40 mg, 2.32 mmol) was added to a solution of N-(2-chloro-4-(trifluoromethyl)phenyl)-2-(2-(2,3-dihydrobenzofuran-5-yl)-5-ethyl-7-oxo-[1,2,4]t riazolo[1,5-a]pyrimidin-4(7H)-yl)acetamide **108b** (1 g, 1.93 mmol) in N,N-dimethylformamide (10 mL), and the reaction was performed at room temperature overnight. Water (20 mL) was added to the reaction mixture, and a solid precipitated. The mixture was extracted with ethyl acetate (20 mL×3). The combined organic phases and the precipitated solid were combined, concentrated under reduced pressure, and purified by silica gel column chromatography (eluent: System B) to obtain 2-(6-bromo-2-(2,3-dihydrobenzofuran-5-yl)-5-ethyl-7-oxo-[1,2,4]triazolo[1,5-a]pyrimidin-4(7H)-yl)-N-(2-chloro-4-(trifluoromethyl)phenyl)acetamide **108c** (1.1 g) in a yield of 95.46%.

MS m/z (ESI): 596.1 [M+1]

### Step 3

### tert-butyl (1-(4-(2-((2-chloro-4-(trifluoromethyl)phenyl)amino)-2-oxoethyl)-2-(2,3-dihydrobenzofuran-5-yl )-5-ethyl-7-oxo-4,7-dihydro-[1,2,4]triazolo[1,5-a]pyrimidin-6-yl)piperidin-4-yl)carbamate

At room temperature, 2-(6-bromo-2-(2,3-dihydrobenzofuran-5-yl)-5-ethyl-7-oxo-[1,2,4]triazolo[1,5-a]pyrimidin-4(7H)-yl)-N-(2-chloro-4-(trifluoromethyl)phenyl)acetamide **108c** (90 mg, 150.81 µmol), piperidin-4-yl carbamate **108d** (302.04 mg, 1.51 mmol, commercially available), and silver tetrafluoroborate (58.72 mg, 301.62 µmol) were dissolved in dimethyl sulfoxide (1.5 mL), and the reaction was heated to 130 °C and the reaction was performed for 6 h. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl (1-(4-(2-((2-chloro-4-(trifluoromethyl)phenyl)amino)-2-oxoethyl)-2-(2,3-dihydrobenzofuran-5-yl )-5-ethyl-7-oxo-4,7-dihydro-[1,2,4]triazolo[1,5-a]pyrimidin-6-yl)piperazin-4-yl)carbamate **108e** (100 mg) with a yield of 92.59%.

MS m/z (ESI): 660.3 [M+H-56]

### Step 4

### 2-(6-(4-aminopiperidin-1-yl)-2-(2,3-dihydrobenzofuran-5-yl)-5-ethyl-7-oxo-[1,2,4]triazolo[1,5-a] pyrimidin-4(7H)-yl)-N-(2-chloro-4-(trifluoromethyl)phenyl)acetamide

At room temperature, trifluoroacetic acid (1 mL) was added to a solution of tert-butyl (1-(4-(2-((2-chloro-4-(trifluoromethyl)phenyl)amino)-2-oxoethyl)-2-(2,3-dihydrobenzofuran-5-yl )-5-ethyl-7-oxo-4,7-dihydro-[1,2,4]triazolo[1,5-a]pyrimidin-6-yl)piperazin-4-yl)carbamate **108e** (100 mg, 139.64 µmol) in dichloromethane (4 mL), and the reaction was performed at room temperature for 40 min. After the reaction was found to be completed through monitoring, the mixture was concentrated under reduced pressure to obtain 2-(6-(4-aminopiperidin-1-yl)-2-(2,3-dihydrobenzofuran-5-yl)-5-ethyl-7-oxo-[1,2,4]triazolo[1,5-a] pyrimidin-4(7H)-yl)-N-(2-chloro-4-(trifluoromethyl)phenyl)acetamide **108f** (86 mg), which was directly used in the next step as a crude product.

MS m/z (ESI): 616.3 [M+1]

### Step 5

### 5-(benzyloxy)-N-(1-(4-(2-((2-chloro-4-(trifluoromethyl)phenyl)amino)-2-oxoethyl)-2-(2,3-dihydr obenzofuran-5-yl)-5-ethyl-7-oxo-4,7-dihydro-[1,2,4]triazolo[1,5-a]pyrimidin-6-yl)piperidin-4-yl) -6-methylpyrimidine-4-carboxamide

At room temperature, 5-(benzyloxy)-6-methylpyrimidine-4-carboxylic acid **1g** (51.15 mg, 209.40 µmol), 1-(3-dimethylaminopropyl)-3-ethylcarbodiimide hydrochloride (53.52 mg, 279.21 µmol), 1-hydroxybenzotriazole (37.73 mg, 279.21 µmol), and N,N-diisopropylethylamine (90.21 mg, 698.01 µmol) were dissolved in N,N-dimethylformamide (3 mL), and the reaction was performed at room temperature for 40 min. Then, a solution of 2-(6-(4-aminopiperidin-1-yl)-2-(2,3-dihydrobenzofuran-5-yl)-5-ethyl-7-oxo-[1,2,4]triazolo[1,5-a] pyrimidin-4(7H)-yl)-N-(2-chloro-4-(trifluoromethyl)phenyl)acetamide **108f** (86 mg, 139.60 µmol) in N,N-dimethylformamide (3 mL) was added, and the reaction was performed at room temperature for 18 h. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 5-(benzyloxy)-N-(1-(4-(2-((2-chloro-4-(trifluoromethyl)phenyl)amino)-2-oxoethyl)-2-(2,3-dihydr obenzofuran-5-yl)-5-ethyl-7-oxo-4,7-dihydro-[1,2,4]triazolo[1,5-a]pyrimidin-6-yl)piperidin-4-yl) -6-methylpyrimidine-4-carboxamide **108g** (90 mg) with a yield of 76.54%.

MS m/z (ESI): 842.3 [M+1]

### Step 6

### N-(1-(4-(2-((2-chloro-4-(trifluoromethyl)phenyl)amino)-2-oxoethyl)-2-(2,3-dihydrobenzofuran-5 -yl)-5-ethyl-7-oxo-4,7-dihydro-[1,2,4]triazolo[1,5-a]pyrimidin-6-yl)piperidin-4-yl)-5-hydroxy-6-methylpyrimidine-4-carboxamide

At room temperature, trifluoroacetic acid (4 mL) was added to a solution of 5-(benzyloxy)-N-(1-(4-(2-((2-chloro-4-(trifluoromethyl)phenyl)amino)-2-oxoethyl)-2-(2,3-dihydr obenzofuran-5-yl)-5-ethyl-7-oxo-4,7-dihydro-[1,2,4]triazolo[1,5-a]pyrimidin-6-yl) piperidin-4-yl)-6-methylpyrimidine-4-carboxamide **108g** (90 mg, 106.85 µmol) in dichloromethane (1 mL), and the reaction was performed at 30 °C for 18 h. The reaction mixture was concentrated under reduced pressure, and then separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(1-(4-(2-((2-chloro-4-(trifluoromethyl)phenyl)amino)-2-oxoethyl)-2-(2,3-dihydrobenzofuran-5 -yl)-5-ethyl-7-oxo-4,7-dihydro-[1,2,4]triazolo[1,5-a]pyrimidin-6-yl)piperidin-4-yl)-5-hydroxy-6-methylpyrimidine-4-carboxamide 1**08** (46.17 mg) with a yield of 56.87%.

MS m/z (ESI): 752.3 [M+1]
¹H NMR (400 MHz, DMSO-d6) δ 12.54 (s, 1H), 10.41 (s, 1H), 9.36 (d, J = 8.4 Hz, 1H), 8.66 (s, 1H), 8.08 (d, J = 8.6 Hz, 1H), 7.97 (s, 2H), 7.92 - 7.79 (m, 1H), 7.72 (d, J = 8.7 Hz, 1H), 6.88 (d, J = 8.3 Hz, 1H), 5.38 (s, 2H), 4.61 (t, J = 8.7 Hz, 2H), 3.99 - 3.87 (m, 1H), 3.53 (t, J = 11.6 Hz, 2H), 3.25 (t, J = 8.7 Hz, 2H), 2.99 (q, J = 7.4 Hz, 2H), 2.81 (d, J = 11.1 Hz, 2H), 2.46 (s, 3H), 1.93 (dt, J = 11.8, 6.6 Hz, 2H), 1.84 (d, J = 12.5 Hz, 2H), 1.22 (t, J = 7.3 Hz, 3H).

### Example 109

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(phenylamino)-[1,2,4]triazolo[1,5-a]pyrimidin-4(7H)-yl)acetamide

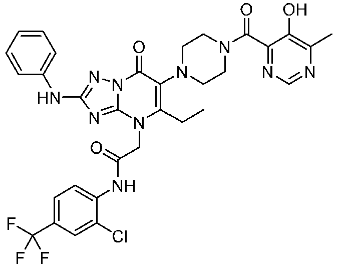

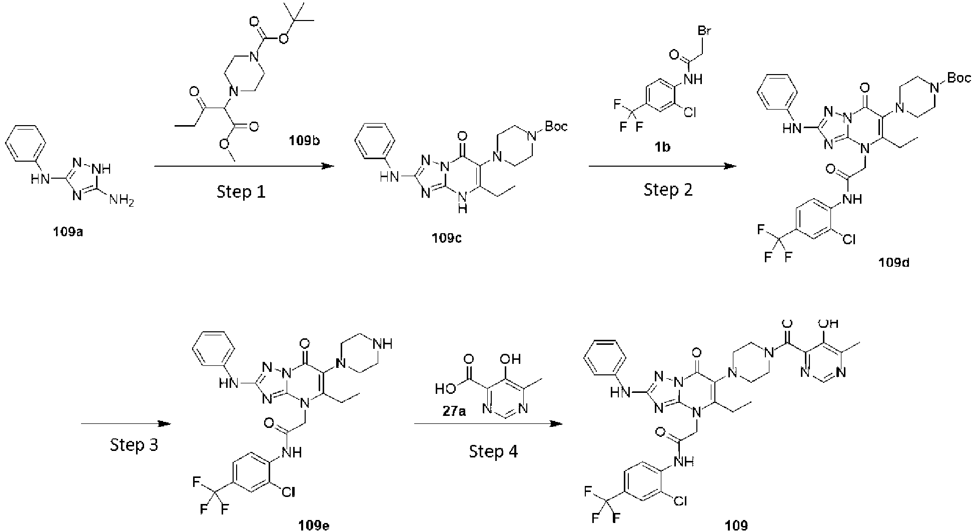

### Step 1

### tert-butyl 4-(5-ethyl-7-oxo-2-(phenylamino)-4,7-dihydro-[1,2,4]triazolo[1,5-a]pyrimidin-6-yl)piperazine-1-carboxylate

N³-Phenyl-1H-1,2,4-triazole-3,5-diamine **109a** (350 mg, 2.00 mmol, commercially available) and tert-butyl 4-(1-methoxy-1,3-dioxolan-2-yl)piperazine-1-carboxylate **109b** (628.07 mg, 2.00 mmol, prepared according to published patent WO2022249060) were dissolved in ethanol (3 mL), then polyphosphoric acid (0.6 g) was slowly added. The reaction mixture was stirred at 100 °C for 16 hours. The solvent was removed under reduced pressure, saturated sodium bicarbonate solution (10 mL) was added to the reaction mixture, the mixture was extracted with dichloromethane (10 mL × 3), the combined organic phases were washed with saturated sodium chloride solution (10 mL × 3), dried over anhydrous sodium sulfate, filtered, and concentrated under reduced pressure, and the resulting residue was purified by column chromatography (eluent: System B) to obtain tert-butyl 4-(5-ethyl-7-oxo-2-(phenylamino)-4,7-dihydro-[1,2,4]triazolo[1,5-a]pyrimidin-6-yl)piperazine-1-carboxylate **109c** (200 mg) in a yield of 22.79%.

MS m/z (ESI): 440.3 [M+1]

### Step 2

### tert-butyl 4-(4-(2-((2-chloro-4-(trifluoromethyl)phenyl)amino)-2-oxoethyl)-5-ethyl-7-oxo-2-(phenylamino) -4,7-dihydro-[1,2,4]triazolo[1,5-a]pyrimidin-6-yl)piperazine-1-carboxylate

Tert-butyl 4-(5-ethyl-7-oxo-2-(phenylamino)-4,7-dihydro-[1,2,4]triazolo[1,5-a]pyrimidin-6-yl)piperazine-1-carboxylate **109c** (200 mg, 455.05 µmol) and 2-bromo-N-(2-chloro-4-(trifluoromethyl)phenyl)acetamide **1b** (144.02 mg, 455.05 µmol) were dissolved in N,N-dimethylformamide (3 mL), then N,N-diisopropylethane-1,2-diamine (147.03 mg, 1.14 mmol) was added, and the reaction mixture was stirred at 50 °C for 18 hours. Water (20 mL) was added to the reaction mixture, the mixture was extracted with ethyl acetate (20 mL × 3), the combined organic phases were washed with saturated sodium chloride solution (20 mL), dried over anhydrous sodium sulfate, filtered, and concentrated under reduced pressure, and the resulting residue was purified by silica gel column chromatography (eluent: System A) to obtain tert-butyl 4-(4-(2-((2-chloro-4-(trifluoromethyl)phenyl)amino)-2-oxoethyl)-5-ethyl-7-oxo-2-(phenylamino) -4,7-dihydro-[1,2,4]triazolo[1,5-a]pyrimidin-6-yl)piperazine-1-carboxylate **109d** (130 mg) in a yield of 42.32%.

MS m/z (ESI): 675.2 [M+1]

### Step 3

### N-(2-chloro-4-(trifluoromethyl)phenyl)-2-(5-ethyl-7-oxo-2-(phenylamino)-6-(piperazin-1-yl)-[1, 2,4]triazolo[1,5-a]pyrimidin-4(7H)-yl)acetamide

Tert-butyl 4-(4-(2-((2-chloro-4-(trifluoromethyl)phenyl)amino)-2-oxoethyl)-5-ethyl-7-oxo-2-(phenylamino) -4,7-dihydro-[1,2,4]triazolo[1,5-a]pyrimidin-6-yl)piperazine-1-carboxylate **109d** (100 mg, 148.13 µmol) was dissolved in trifluoroacetic acid (0.3 mL) and dichloromethane (3 mL), and the reaction mixture was stirred at room temperature for 1 hour. The solvent was removed under reduced pressure to obtain N-(2-chloro-4-(trifluoromethyl)phenyl)-2-(5-ethyl-7-oxo-2-(phenylamino)-6-(piperazin-1-yl)-[1, 2,4]triazolo[1,5-a]pyrimidin-4(7H)-yl)acetamide **109e** (80 mg), and the crude product was directly used in the next step.

MS m/z (ESI): 575.2 [M+1]

### Step 4

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(phenylamino)-[1,2,4]triazolo[1,5-a]pyrimidin-4(7H)-yl)acetamide

N-(2-chloro-4-(trifluoromethyl)phenyl)-2-(5-ethyl-7-oxo-2-(phenylamino)-6-(piperazin-1-yl)-[1,2,4]triazolo[1,5-a]pyrimidin-4(7H)-yl)acetamide **109e** (100 mg, 173.92 µmol) and 5-hydroxy-6-methylpyrimidine-4-carboxylic acid 27a (80.41 mg, 521.75 µmol) were dissolved in N,N-dimethylformamide (1 mL), then 1-hydroxybenzotriazole (35.25 mg, 260.88 µmol), 1-(3-dimethylaminopropyl)-3-ethylcarbodiimide hydrochloride (50.01 mg, 260.88 µmol), and N,N-diisopropylethylamine (67.43 mg, 521.75 µmol) were added, and the mixture was stirred at 25 °C for 16 hours. Water (10 mL) was added to the reaction mixture, the mixture was extracted with ethyl acetate (10 mL × 3), the combined organic phases were washed with saturated sodium chloride solution (30 mL × 3), dried over anhydrous sodium sulfate, filtered, and concentrated under reduced pressure, and the resulting residue was purified by preparative liquid chromatography (Waters 3767/QDA column: SunFire Sunfire C₁₈, 19 × 250 mm, 10 µm; mobile phase A: 0.05% TFA/H₂O, B: ACN; flow rate: 20 mL/min) to obtain N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(phenylamino)-[1,2,4]triazolo[1,5-a]pyrimidin-4(7H)-yl)acetamide 109 (18 mg) in a yield of 14.55%.

MS m/z (ESI): 711.2 [M+1]
¹H NMR (400 MHz, MeOD-d4) δ 8.53 (s, 1H), 8.20 (d, J = 8.8 Hz, 1H), 7.81 (s, 1H), 7.63 - 7.57 (d, J = 8.4 Hz, 3H), 7.29 - 7.20 (m, 2H), 6.94 - 6.87 (m, 1H), 5.32 (s, 2H), 4.76 - 4.69 (m, 1H), 4.11 -4.03 (m, 1H), 3.79 - 3.71 (m, 2H), 3.51 - 3.38 (m, 1H), 3.17 - 3.04 (m, 3H), 2.96 - 2.89 (m, 1H), 2.81 - 2.73 (m, 1H), 2.52 (s, 3H), 1.38 - 1.26 (m, 3H).

### Example 110

### N-(2-chloro-4-(trifluoromethyl)phenyl)-2-(5-ethyl-6-(4-(5-hydroxy-6-methylpyrimidine-4-carbon yl)piperazin-1-yl)-2-(2-methylbenzo[d]thiazol-5-yl)-7-oxo-[1,2,4]triazolo[1,5-a]pyrimidin-4(7H)-yl)acetamide

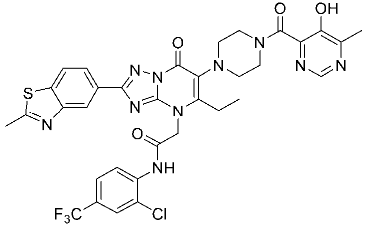

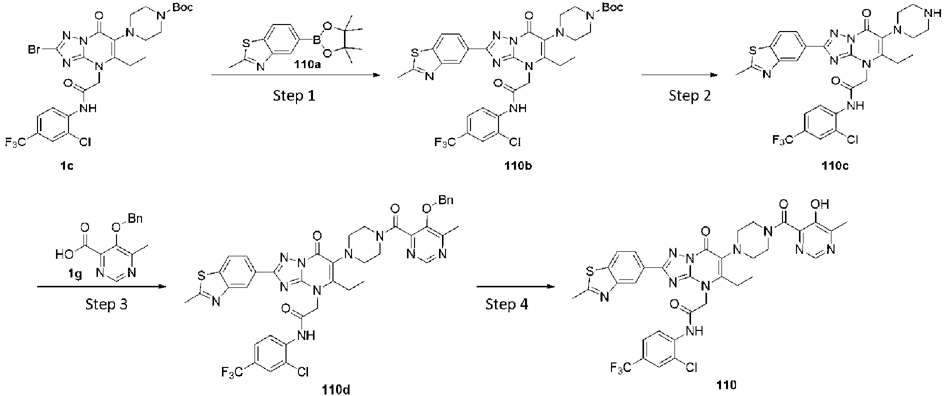

### Step 1

### tert-butyl 4-(4-(2-((2-chloro-4-(trifluoromethyl)phenyl)amino)-2-oxoethyl)-5-ethyl-2-(2-methylbenzo[d]thi azol-5-yl)-7-oxo-4,7-dihydro-[1,2,4]triazolo[1,5-a]pyrimidin-6-yl)piperazine-1-carboxylate

At room temperature, tert-butyl 4-(2-bromo-4-(2-((2-chloro-4-(trifluoromethyl)phenyl)amino)-2-oxoethyl)-5-ethyl-7-oxo-4,7-dih ydro-[1,2,4]triazolo[1,5-a]pyrimidin-6-yl)piperazine-1-carboxylate **1c** (90 mg, 135.77 µmol), 2-methyl-5-(4,4,5,5-tetramethyl-1,3,2-dioxaborolan-2-yl)benzo[d]thiazole **110a** (44.83 mg, 162.92 µmol), [1,1'-bis(diphenylphosphino)ferrocene]palladium(II) dichloride dichloromethane complex (11.01 mg, 13.58 µmol), and sodium carbonate (43.17 mg, 407.31 µmol) were dissolved in a mixture solution of 1,4-dioxane (20 mL) and water (4 mL). The mixture was subject to argon replacement three times, then heated to 78 °C, and the reaction was performed for 2 hours. Water (20 mL) was added to the reaction mixture, followed by extraction with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(4-(2-((2-chloro-4-(trifluoromethyl)phenyl)amino)-2-oxoethyl)-5-ethyl-2-(2-methylbenzo[d]thi azol-5-yl)-7-oxo-4,7-dihydro-[1,2,4]triazolo[1,5-a]pyrimidin-6-yl)piperazine-1-carboxylate **110b** (90 mg) with a yield of 90.66%.

MS m/z (ESI): 675.3 [M+H-56]

### Step 2

### N-(2-Chloro-4-(trifluoromethyl)phenyl)-2-(5-ethyl-2-(2-methylbenzo[d]thiazol-5-yl)-7-oxo-6-(pi perazin-1-yl)-[1,2,4]triazolo[1,5-a]pyrimidin-4(7H)-yl)acetamide

At room temperature, trifluoroacetic acid (1 mL) was added to a solution of tert-butyl 4-(4-(2-((2-chloro-4-(trifluoromethyl)phenyl)amino)-2-oxoethyl)-5-ethyl-2-(2-methylbenzo[d]thi azol-5-yl)-7-oxo-4,7-dihydro-[1,2,4]triazolo[1,5-a]pyrimidin-6-yl)piperazine-1-carboxylate **110b** (90 mg, 123.09 µmol) in dichloromethane (4 mL), and the reaction was performed at room temperature for 40 minutes. The mixture was concentrated under reduced pressure to obtain N-(2-chloro-4-(trifluoromethyl)phenyl)-2-(5-ethyl-2-(2-methylbenzo[d]thiazol-5-yl)-7-oxo-6-(pi perazin-1-yl)-[1,2,4]triazolo[1,5-a]pyrimidin-4(7H)-yl)acetamide **110c** (77 mg), and the crude product was directly used in the next step.

MS m/z (ESI): 631.2 [M+1]

### Step 3

### 2-(6-(4-(5-(Benzyloxy)-6-methylpyrimidine-4-carbonyl)piperazin-1-yl)-5-ethyl-2-(2-methylbenz o[d]thiazol-5-yl)-7-oxo-[1,2,4]triazolo[1,5-a]pyrimidin-4(7H)-yl)-N-(2-chloro-4-(trifluoromethyl )phenyl)acetamide

At room temperature, 5-(benzyloxy)-6-methylpyrimidine-4-carboxylic acid **1g** (44.70 mg, 183.02 µmol), 1-(3-dimethylaminopropyl)-3-ethylcarbodiimide hydrochloride (46.78 mg, 244.03 µmol), 1-hydroxybenzotriazole (32.97 mg, 244.03 µmol), and N,N-diisopropylethylamine (78.85 mg, 610.07 µmol) were added to N,N-dimethylformamide (3 mL), and the reaction was performed at room temperature for 30 minutes. Then, a solution of N-(2-chloro-4-(trifluoromethyl)phenyl)-2-(5-ethyl-2-(2-methylbenzo[d]thiazol-5-yl)-7-oxo-6-(pi perazin-1-yl)-[1,2,4]triazolo[1,5-a]pyrimidin-4(7H)-yl)acetamide **110c** (77 mg, 122.01 µmol) in N,N-dimethylformamide (3 mL) was added, and the reaction was performed at room temperature for 18 hours. Water (20 mL) was added to the reaction mixture, followed by extraction with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-5-ethyl-2-(2-methylbenzo [d]thiazol-5-yl)-7-oxo-[1,2,4]triazolo[1,5-a]pyrimidin-4(7H)-yl)-N-(2-chloro-4-(trifluoromethyl) phenyl)acetamide **110d** (100 mg) with a yield of 95.60%.

MS m/z (ESI): 857.2 [M+1]

### Step 4

### N-(2-chloro-4-(trifluoromethyl)phenyl)-2-(5-ethyl-6-(4-(5-hydroxy-6-methylpyrimidine-4-carbon yl)piperazin-1-yl)-2-(2-methylbenzo[d]thiazol-5-yl)-7-oxo-[1,2,4]triazolo[1,5-a]pyrimidin-4(7H)-yl)acetamide

At room temperature, trifluoroacetic acid (4 mL) was added to a solution of 2-(6-(4-(5-(benzyloxy)-6-methylpyrimidine-4-carbonyl)piperazin-1-yl)-5-ethyl-2-(2-methylbenzo [d]thiazol-5-yl)-7-oxo-[1,2,4]triazolo[1,5-a]pyrimidin-4(7H)-yl)-N-(2-chloro-4-(trifluoromethyl) phenyl) acetamide **110d** (100 mg, 116.65 µmol) in dichloromethane (1 mL), and the reaction was performed at 30 °C for 18 hours. The reaction mixture was concentrated under reduced pressure, then separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-2-(2-methylbenzo[d]thiazol-5-yl)-7-oxo-[1,2,4]triazolo[1,5-a]pyrimidin-4(7H)-yl)acetamide **110** (7.97 mg) with a yield of 8.82%.

MS m/z (ESI): 767.2 [M+1]
¹H NMR (400 MHz, DMSO-d6) δ 10.47 (s, 1H), 10.28 (s, 1H), 8.72 - 8.53 (m, 2H), 8.25 - 8.10 (m, 2H), 8.07 (d, J = 8.5 Hz, 1H), 7.98 (d, J = 2.0 Hz, 1H), 7.72 (dd, J = 8.7, 2.1 Hz, 1H), 5.44 (s, 2H), 4.56 (d, J = 12.4 Hz, 1H), 3.60 - 3.48 (m, 3H), 3.29 (t, J = 12.5 Hz, 1H), 3.05 (q, J = 6.7, 5.9 Hz, 3H), 2.88 (s, 1H), 2.84 (s, 3H), 2.69 (d, J = 10.9 Hz, 1H), 2.46 (s, 3H), 1.24 (t, J = 7.4 Hz, 3H).

### Example 111

### N-(2-chloro-4-(trifluoromethyl)phenyl)-2-(5-ethyl-6-(4-(5-hydroxy-6-methylpyrimidine-4-carbon yl)piperazin-1-yl)-2-(1-methyl-1H-benzo[d]imidazol-5-yl)-7-oxo-[1,2,4]triazolo[1,5-a]pyrimidin-4(7H)-yl)acetamide

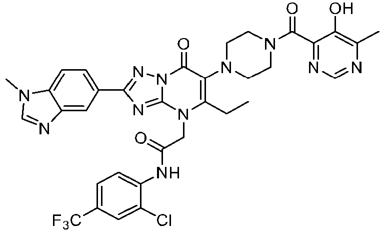

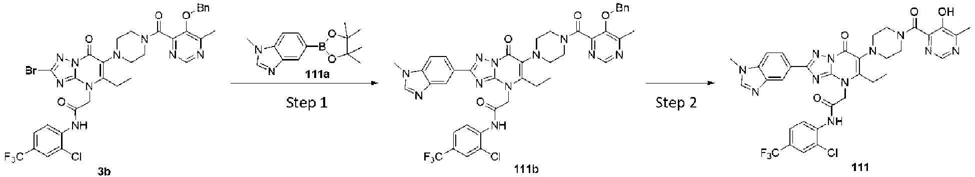

### Step 1

### 2-(6-(4-(5-(Benzyloxy)-6-methylpyrimidine-4-carbonyl)piperazin-1-yl)-5-ethyl-2-(1-methyl-1H-benzo[d]imidazol-5-yl)-7-oxo-[1,2,4]triazolo[1,5-a]pyrimidin-4(7H)-yl)-N-(2-chloro-4-(trifluoro methyl)phenyl)acetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (80 mg, 101.39 µmol), 1-methyl-5-(4,4,5,5-tetramethyl-1,3,2-dioxaborolan-2-yl)-1H-benzo[d]imidazole **111a** (31.41 mg, 121.67 µmol, commercially available), [1,1'-bis(diphenylphosphino)ferrocene]palladium(II) dichloride dichloromethane complex (8.22 mg, 10.14 µmol), and sodium carbonate (32.24 mg, 304.18 µmol) were dissolved in 1,4-dioxane (7 mL) and water (1 mL). The mixture was subject to argon replacement 3 times, then heated to 85 °C and the reaction was performed for 2 hours. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-5-ethyl-2-(1-methyl-1H-b enzo[d]imidazol-5-yl)-7-oxo-[1,2,4]triazolo[1,5-a]pyrimidin-4(7H)-yl)-N-(2-chloro-4-(trifluorom ethyl)phenyl)acetamide **111b** (70 mg) in a yield of 82.16%.

MS m/z (ESI): 840.4 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-2-(1-methyl-1H-benzo[d]imidazol-5-yl)-7-oxo-[1,2,4]triazolo[1,5-a]pyrimidin-4(7H)-yl)acetamide

At room temperature, trifluoroacetic acid (4 mL) was added to a solution of 2-(6-(4-(5-(benzyloxy)-6-methylpyrimidine-4-carbonyl)piperazin-1-yl)-5-ethyl-2-(1-methyl-1H-b enzo[d]imidazol-5-yl)-7-oxo-[1,2,4]triazolo[1,5-a]pyrimidin-4(7H)-yl)-N-(2-chloro-4-(trifluorom ethyl)phenyl)acetamide **111b** (70 mg, 83.31 µmol) in dichloromethane (1 mL), and the reaction was performed at room temperature for 18 hours. The mixture was concentrated under reduced pressure, then separated by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-2-(1-methyl-1H-benzo[d]imidazol-5-yl)-7-oxo-[1,2,4]triazolo[1,5-a]pyrimidin-4(7H)-yl)acetamide **111** (12.86 mg) in a yield of 20.37%.

MS m/z (ESI): 750.3 [M+1]
¹H NMR (400 MHz, DMSO-d6) δ 10.47 (s, 1H), 10.26 (s, 1H), 8.97 (s, 1H), 8.59 (s, 1H), 8.44 (d, J = 1.4 Hz, 1H), 8.23 (dd, J = 8.6, 1.5 Hz, 1H), 8.06 (d, J = 8.6 Hz, 1H), 7.97 (d, J = 2.1 Hz, 1H), 7.92 (d, J = 8.6 Hz, 1H), 7.71 (dd, J = 8.9, 2.1 Hz, 1H), 5.43 (s, 2H), 4.55 (d, J = 12.5 Hz, 1H), 4.00 (s, 3H), 3.54 (d, J= 10.5 Hz, 3H), 3.28 (t, J = 12.8 Hz, 1H), 3.04 (d, J = 7.0 Hz, 3H), 2.88 - 2.79 (m, 1H), 2.68 (d, J = 10.5 Hz, 1H), 2.45 (s, 3H), 1.23 (t, J = 7.7 Hz, 3H).

### Example 112

### N-(2-chloro-4-(trifluoromethyl)phenyl)-2-(5-ethyl-6-(4-(5-hydroxy-6-methyl pyrimidine-4-carbonyl)piperazin-1-yl)-2-(1H-indazol-5-yl)-7-oxo-[1,2,4]triazolo[1,5-a]pyrimidin -4(7H)-yl)acetamide

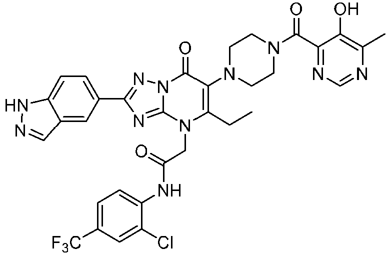

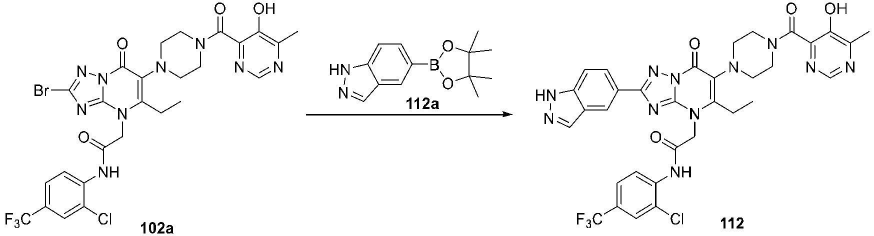

At room temperature, 2-(2-bromo-5-ethyl-6-(4-(5-hydroxy-6-methylpyrimidine-4-carbonyl)piperazin-1-yl)-7-oxo-[1,2, 4]triazolo[1,5-a]pyrimidin-4(7H)-yl)-N-(2-chloro-4-(trifluoromethyl)phenyl)acetamide **102a** (80 mg, 114.47 µmol), 5-(4,4,5,5-tetramethyl-1,3,2-dioxaborolan-2-yl)-1H-indazole **112a** (33.53 mg, 137.36 µmol, commercially available), [1,1'-bis(diphenylphosphino)ferrocene]palladium(II) dichloride dichloromethane complex (9.28 mg, 11.45 µmol), and sodium carbonate (36.40 mg, 343.41 µmol) were dissolved in 1,4-dioxane (5 mL) and water (0.5 mL). The mixture was subject to argon replacement three times, then heated to 95 °C and the reaction was performed for 4 hours. The mixture was concentrated under reduced pressure, then separated by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-2-(1H-indazol-5-yl)-7-oxo-[1,2,4]triazolo[1,5-a]pyrimidin-4(7H)-yl)acetamide **112** (1.05 mg) in a yield of 1.22%.

MS m/z (ESI): 736.3 [M+1]
¹H NMR (400 MHz, DMSO-d6) δ 13.29 (s, 1H), 10.44 (s, 1H), 10.25 (s, 1H), 8.57 (d, J = 16.2 Hz, 2H), 8.21 (s, 1H), 8.20 - 8.06 (m, 2H), 7.98 (d, J = 2.0 Hz, 1H), 7.82 - 7.55 (m, 2H), 5.43 (s, 2H), 4.55 (d, J = 12.5 Hz, 1H), 3.67 - 3.49 (m, 3H), 3.31 - 3.21 (m, 1H), 3.12 - 2.93 (m, 3H), 2.86 (d, J = 11.2 Hz, 1H), 2.68 (d, J = 10.7 Hz, 1H), 2.46 (s, 3H), 1.23 (t, J = 7.3 Hz, 3H).

### Example 113

### N-(2-chloro-4-(trifluoromethyl)phenyl)-2-(5-ethyl-6-(4-(5-hydroxy-6-methylpyrimidine-4-carbon yl)piperazin-1-yl)-2-(1-methyl-1H-indazol-5-yl)-7-oxo-[1,2,4]triazolo[1,5-a]pyrimidin-4(7H)-yl) acetamide

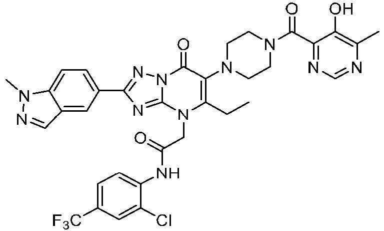

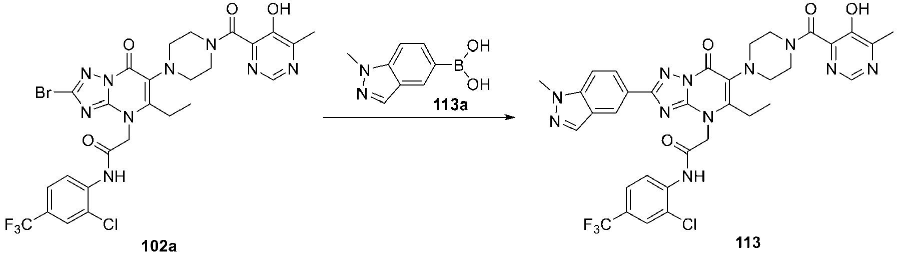

At room temperature, 2-(2-bromo-5-ethyl-6-(4-(5-hydroxy-6-methylpyrimidine-4-carbonyl)piperazin-1-yl)-7-oxo-[1,2, 4]triazolo[1,5-a]pyrimidin-4(7H)-yl)-N-(2-chloro-4-(trifluoromethyl)phenyl)acetamide **102a** (80 mg, 114.47 µmol), (1-methyl-1H-indazol-5-yl)boronic acid **113a** (24.17 mg, 137.36 µmol, commercially available), [1,1'-bis(diphenylphosphino)ferrocene]palladium(II) dichloride dichloromethane complex (9.28 mg, 11.45 µmol), and sodium carbonate (36.40 mg, 343.41 µmol) were dissolved in 1,4-dioxane (5 mL) and water (0.5 mL). The mixture was subject to argon replacement three times, then heated to 85 °C, and the reaction was performed for 2 hours. The mixture was concentrated under reduced pressure and separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-2-(1-methyl-1H-indazol-5-yl)-7-oxo-[1,2,4]triazolo[1,5-a]pyrimidin-4(7H)-yl) acetamide **113** (31.49 mg) in a yield of 36.31%.

MS m/z (ESI): 750.2 [M+1]
¹H NMR (400 MHz, DMSO-d6) δ 10.44 (s, 1H), 10.31 (d, J = 23.1 Hz, 1H), 8.59 (s, 1H), 8.56 - 8.49 (m, 1H), 8.29 - 8.11 (m, 2H), 8.08 (d, J = 8.5 Hz, 1H), 7.98 (d, J = 2.1 Hz, 1H), 7.77 (d, J = 8.9 Hz, 1H), 7.71 (dd, J = 8.7, 2.1 Hz, 1H), 5.42 (s, 2H), 4.55 (d, J = 12.5 Hz, 1H), 4.09 (s, 3H), 3.55 (td, J = 11.8, 10.8, 6.3 Hz, 3H), 3.28 (t, J = 12.5 Hz, 1H), 3.03 (d, J = 10.0 Hz, 3H), 2.86 (d, J = 11.2 Hz, 1H), 2.68 (d, J = 10.2 Hz, 1H), 2.45 (s, 3H), 1.22 (t, J = 7.4 Hz, 3H).

### Example 114

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(1H-pyrazolo[3,4-b]pyridin-5-yl)-[1,2,4]triazolo[1,5-a]pyrimidin-4(7 H)-yl)acetamide

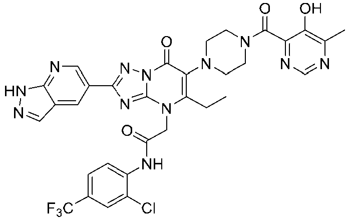

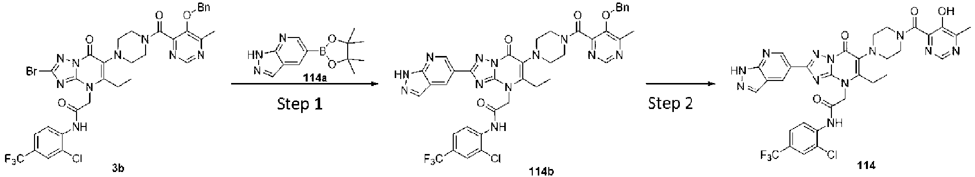

### Step 1

### 2-(6-(4-(5-(benzyloxy)-6-methylpyrimidine-4-carbonyl)piperazin-1-yl)-5-ethyl-7-oxo-2-(1H-pyra zolo[3,4-b]pyridin-5-yl)-[1,2,4]triazolo[1,5-a]pyrimidin-4(7H)-yl)-N-(2-chloro-4-(trifluoromethy l)phenyl)acetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide 3b (110 mg, 139.42 µmol), 5-(4,4,5,5-tetramethyl-1,3,2-dioxaborolan-2-yl)-1H-pyrazolo[3,4-b]pyridine **114a** (41.00 mg, 167.30 µmol), [1,1'-bis(diphenylphosphino)ferrocene]palladium(II) dichloride dichloromethane complex (11.30 mg, 13.94 µmol), and sodium carbonate (44.33 mg, 418.25 µmol) were dissolved in 1,4-dioxane (7 mL) and water (1 mL). The mixture was subject to argon replacement 3 times, then heated to 85 °C, and the reaction was performed for 2 hours. Water (20 mL) was added to the reaction mixture, and the mixture was extracted with ethyl acetate (20 mL ×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-5-ethyl-7-oxo-2-(1H-pyra zolo[3,4-b]pyridin-5-yl)-[1,2,4]triazolo[1,5-a]pyrimidin-4(7H)-yl)-N-(2-chloro-4-(trifluoromethy l)phenyl)acetamide **114b** (60 mg) in a yield of 52.03%.

MS m/z (ESI): 827.3 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(1H-pyrazolo[3,4-b]pyridin-5-yl)-[1,2,4]triazolo[1,5-a]pyrimidin-4(7 H)-yl)acetamide

At room temperature, trifluoroacetic acid (4 mL) was added to a solution of 2-(6-(4-(5-(benzyloxy)-6-methylpyrimidine-4-carbonyl)piperazin-1-yl)-5-ethyl-7-oxo-2-(1H-pyra zolo[3,4-b]pyridin-5-yl)-[1,2,4]triazolo[1,5-a]pyrimidin-4(7H)-yl)-N-(2-chloro-4-(trifluoromethy l)phenyl) acetamide **114b** (60 mg, 72.53 µmol) in dichloromethane (1 mL). The reaction was performed at 30 °C for 18 hours. The mixture was concentrated under reduced pressure and separated by preparative liquid chromatography (separation column AKZONOBEL Kromasil; 250 ×21.2 mm I.D.; 5 µm, 20 mL/min; mobile phase A: 0.05% TFA+H₂O, mobile phase B: CH3CN) to obtain N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(1H-pyrazolo[3,4-b]pyridin-5-yl)-[1,2,4]triazolo[1,5-a]pyrimidin-4(7 H)-yl)acetamide **114** (19.08 mg) in a yield of 35.33%.

MS m/z (ESI): 737.3 [M+1]
¹H NMR (400 MHz, DMSO-d6) δ 13.90 (s, 1H), 10.45 (s, 1H), 10.26 (s, 1H), 9.24 (d, J = 2.0 Hz, 1H), 8.90 (d, J = 2.0 Hz, 1H), 8.60 (s, 1H), 8.29 (s, 1H), 8.08 (d, J = 8.6 Hz, 1H), 7.98 (d, J = 2.1 Hz, 1H), 7.72 (dd, J = 8.8, 2.1 Hz, 1H), 5.44 (s, 2H), 4.55 (d, J = 12.4 Hz, 1H), 3.53 (d, J = 7.2 Hz, 3H), 3.32 - 3.19 (m, 1H), 3.04 (q, J = 13.6, 10.2 Hz, 3H), 2.87 (d, J = 11.3 Hz, 1H), 2.69 (d, J = 10.7 Hz, 1H), 2.46 (s, 3H), 1.23 (t, J = 7.4 Hz, 3H).

### Example 115

### 2-(2-(azetidin-3-ylidenemethyl)-5-ethyl-6-(4-(5-hydroxy-6-methylpyrimidine-4-carbonyl)piperaz in-1-yl)-7-oxo-[1,2,4]triazolo[1,5-a]pyrimidin-4(7H)-yl)-N-(2-chloro-4-(trifluoromethyl)phenyl)a cetamide

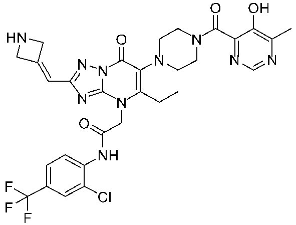

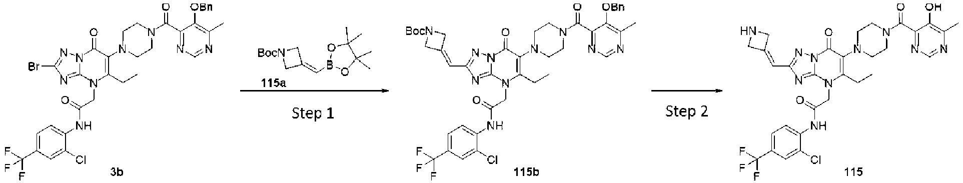

### Step 1

### tert-butyl 3-((6-(4-(5-(benzyloxy)-6-methylpyrimidine-4-carbonyl)piperazin-1-yl)-4-(2-((2-chloro-4-(triflu oromethyl)phenyl)amino)-2-oxoethyl)-5-ethyl-7-oxo-4,7-dihydro-[1,2,4]triazolo[1,5-a]pyrimidin -2-yl)methylene)azetidine-1-carboxylate

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (50 mg, 63.37 µmol), tert-butyl 3-((4,4,5,5-tetramethyl-1,3,2-dioxaborolan-2-yl)methylene)azetidine-1-carboxylate **115a** (22.45 mg, 76.05 µmol, commercially available), [1,1'-bis(diphenylphosphino)ferrocene]palladium(II) dichloride dichloromethane complex (5.14 mg, 6.34 µmol), and sodium carbonate (20.15 mg, 190.11 µmol) were dissolved in 1,4-dioxane (1 mL) and water (0.3 mL). The mixture was subject to argon replacement four times, then heated to 80 °C, and the reaction was performed for 1.5 hours. Water (20 mL) was added to the reaction mixture, and the mixture was extracted with ethyl acetate (20 mL × 3). The combined organic phase was washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 3-((6-(4-(5-(benzyloxy)-6-methylpyrimidine-4-carbonyl)piperazin-1-yl)-4-(2-((2-chloro-4-(triflu oromethyl)phenyl)amino)-2-oxoethyl)-5-ethyl-7-oxo-4,7-dihydro-[1,2,4]triazolo[1,5-a]pyrimidin -2-yl)methylene)azetidine-1-carboxylate **115b** (35 mg) in a yield of 62.95%.

MS m/z (ESI): 877.5 [M+1]

### Step 2

### 2-(2-(azetidin-3-ylidenemethyl)-5-ethyl-6-(4-(5-hydroxy-6-methylpyrimidine-4-carbonyl)piperaz in-1-yl)-7-oxo-[1,2,4]triazolo[1,5-a]pyrimidin-4(7H)-yl)-N-(2-chloro-4-(trifluoromethyl)phenyl)a cetamide

To a solution of tert-butyl 3-((6-(4-(5-(benzyloxy)-6-methylpyrimidine-4-carbonyl)piperazin-1-yl)-4-(2-((2-chloro-4-(triflu oromethyl)phenyl)amino)-2-oxoethyl)-5-ethyl-7-oxo-4,7-dihydro-[1,2,4]triazolo[1,5-a]pyrimidin -2-yl)methylene)azetidine-1-carboxylate **115b** (35 mg, 39.89 µmol) in dichloromethane (1 mL) was added trifluoroacetic acid (3 mL), and the reaction was performed at room temperature for 18 hours. The mixture was concentrated under reduced pressure, then separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain 2-(2-(azetidin-3-ylidenemethyl)-5-ethyl-6-(4-(5-hydroxy-6-methylpyrimidine-4-carbonyl)piperaz in-1-yl)-7-oxo-[1,2,4]triazolo[1,5-a]pyrimidin-4(7H)-yl)-N-(2-chloro-4-(trifluoromethyl)phenyl)a cetamide **115** (15.82 mg) in a yield of 9.79%.

MS m/z (ESI): 687.2 [M+1]
¹H NMR (400 MHz, DMSO) δ 10.37 (s, 1H), 10.23 (s, 1H), 8.93 (s, 2H), 8.58 (s, 1H), 8.05 (d, J = 8.6 Hz, 1H), 7.97 (d, J = 2.1 Hz, 1H), 7.76 - 7.66 (m, 1H), 6.56 - 6.46 (m, 1H), 5.31 (s, 2H), 4.85 (d, J = 28.6 Hz, 4H), 4.52 (d, J = 12.7 Hz, 1H), 3.50 (d, J = 2.7 Hz, 2H), 3.25 (t, J = 12.6 Hz, 2H), 3.02 (d, J = 12.5 Hz, 3H), 2.81 (d, J = 11.2 Hz, 1H), 2.63 (d, J = 10.5 Hz, 1H), 2.44 (s, 3H), 1.19 (t, J = 7.5 Hz, 3H).

### Example 116

### 3-((4-(2-((2-chloro-4-(trifluoromethyl)phenyl)amino)-2-oxoethyl)-5-ethyl-6-(4-(5-hydroxy-6-met hylpyrimidine-4-carbonyl)piperazin-1-yl)-7-oxo-4,7-dihydro-[1,2,4]triazolo[1,5-a]pyrimidin-2-yl )methylene)cyclobutane-1-carboxamide

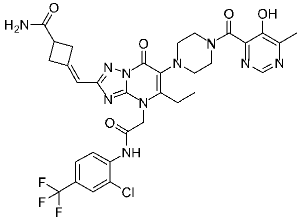

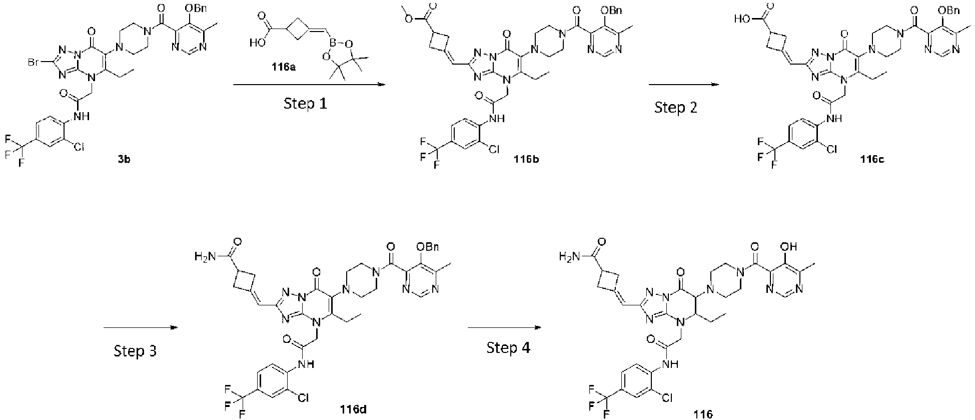

### Step 1

### methyl 3-((6-(4-(5-(benzyloxy)-6-methylpyrimidine-4-carbonyl)piperazin-1-yl)-4-(2-((2-chloro-4-(triflu oromethyl)phenyl)amino)-2-oxoethyl)-5-ethyl-7-oxo-4,7-dihydro-[1,2,4]triazolo[1,5-a]pyrimidin -2-yl)methylene)cyclobutane-1-carboxylate

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (200 mg, 253.49 µmol), 3-((4,4,5,5-tetramethyl-1,3,2-dioxaborolan-2-yl)methylene)cyclobutane-1-carboxylic acid **116a** (76.69 mg, 304.18 µmol, commercially available), [1,1'-bis(diphenylphosphino)ferrocene]palladium(II) dichloride dichloromethane complex (20.55 mg, 25.35 µmol), and sodium carbonate (80.60 mg, 760.46 µmol) were dissolved in 1,4-dioxane (3 mL) and water (0.9 mL). The mixture was subject to argon replacement four times, then heated to 80 °C, and the reaction was performed for 1.5 hours. Water (20 mL) was added to the reaction mixture, and the mixture was extracted with ethyl acetate (20 mL × 3). The combined organic phase was washed with saturated sodium chloride solution (20 mL), dried over anhydrous sodium sulfate, filtered, and concentrated under reduced pressure. The resulting residue was purified by silica gel column chromatography (eluent: System B) to obtain methyl 3-((6-(4-(5-(benzyloxy)-6-methylpyrimidine-4-carbonyl)piperazin-1-yl)-4-(2-((2-chloro-4-(triflu oromethyl)phenyl)amino)-2-oxoethyl)-5-ethyl-7-oxo-4,7-dihydro-[1,2,4]triazolo[1,5-a]pyrimidin -2-yl)methylene)cyclobutane-1-carboxylate **116b** (130 mg) in a yield of 61.48%.

MS m/z (ESI): [M+1] 834.2

### Step 2

### 3-((6-(4-(5-(benzyloxy)-6-methylpyrimidine-4-carbonyl)piperazin-1-yl)-4-(2-((2-chloro-4-(triflu oromethyl)phenyl)amino)-2-oxoethyl)-5-ethyl-7-oxo-4,7-dihydro-[1,2,4]triazolo[1,5-a]pyrimidin -2-yl)methylene)cyclobutane-1-carboxylic acid

Methyl 3-((6-(4-(5-(benzyloxy)-6-methylpyrimidine-4-carbonyl)piperazin-1-yl)-4-(2-((2-chloro-4-(triflu oromethyl)phenyl)amino)-2-oxoethyl)-5-ethyl-7-oxo-4,7-dihydro-[1,2,4]triazolo[1,5-a]pyrimidin -2-yl)methylene)cyclobutane-1-carboxylate **116b** (130 mg, 155.83 µmol) was added to ethanol (3 mL) and water (0.6 mL), and lithium hydroxide monohydrate (32.69 mg, 779.15 µmol) was added. The reaction was performed at room temperature for 18 h. The pH was adjusted to neutral by dropwise addition of 1N aqueous hydrochloric acid solution at 0 °C. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 3-((6-(4-(5-(benzyloxy)-6-methylpyrimidine-4-carbonyl)piperazin-1-yl)-4-(2-((2-chloro-4-(triflu oromethyl)phenyl)amino)-2-oxoethyl)-5-ethyl-7-oxo-4,7-dihydro-[1,2,4]triazolo[1,5-a]pyrimidin -2-yl)methylene)cyclobutane-1-carboxylic acid **116c** (100 mg) in a yield of 78.24%.

MS m/z (ESI): 820.3 [M+1]

### Step 3

### 3-((6-(4-(5-(benzyloxy)-6-methylpyrimidine-4-carbonyl)piperazin-1-yl)-4-(2-((2-chloro-4-(triflu oromethyl)phenyl)amino)-2-oxoethyl)-5-ethyl-7-oxo-4,7-dihydro-[1,2,4]triazolo[1,5-a]pyrimidin -2-yl)methylene)cyclobutane-1-carboxamide

3-((6-(4-(5-(Benzyloxy)-6-methylpyrimidine-4-carbonyl)piperazin-1-yl)-4-(2-((2-chloro -4-(trifluoromethyl)phenyl)amino)-2-oxoethyl)-5-ethyl-7-oxo-4,7-dihydro-[1,2,4]triazolo [1,5-a]pyrimidin-2-yl)methylene)cyclobutane-1-carboxylic acid **116c** (35 mg, 42.67 µmol) was added to dichloromethane (1 mL), and N,N'-carbonyldiimidazole (8.99 mg, 55.47 µmol) was added. The reaction was performed at room temperature for 2 h, followed by addition of aqueous ammonia (0.3 mL). The reaction was continued at room temperature for 2 h. Water (20 mL) was added to the reaction mixture, and the mixture was extracted with dichloromethane (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System A) to obtain 3-((6-(4-(5-(benzyloxy)-6-methylpyrimidine-4-carbonyl)piperazin-1-yl)-4-(2-((2-chloro-4-(triflu oromethyl) phenyl)amino)-2-oxoethyl)-5-ethyl-7-oxo-4,7-dihydro-[1,2,4]triazolo[1,5-a]pyrimidin-2-yl)meth ylene)cyclobutane-1-carboxamide **116d** (26 mg) in a yield of 74.38%.

MS m/z (ESI): 819.32 [M+1]

### Step 4

### 3-((4-(2-((2-chloro-4-(trifluoromethyl)phenyl)amino)-2-oxoethyl)-5-ethyl-6-(4-(5-hydroxy-6-met hylpyrimidine-4-carbonyl)piperazin-1-yl)-7-oxo-4,7-dihydro-[1,2,4]triazolo[1,5-a]pyrimidin-2-yl )methylene)cyclobutane-1-carboxamide

3-((6-(4-(5-(Benzyloxy)-6-methylpyrimidine-4-carbonyl)piperazin-1-yl)-4-(2-((2-chloro -4-(trifluoromethyl)phenyl)amino)-2-oxoethyl)-5-ethyl-7-oxo-4,7-dihydro-[1,2,4]triazolo [1,5-a]pyrimidin-2-yl)methylene)cyclobutane-1-carboxamide **116d** (26 mg, 31.74 µmol) was added to dichloromethane (2.5 mL), and boron trichloride (0.8 mL, 1M in hexane) was added at 0 °C. The reaction was performed at 0 °C for 1 h. The reaction was quenched with methanol and concentrated under reduced pressure. The residue was separated by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 3-((4-(2-((2-chloro-4-(trifluoromethyl)phenyl)amino)-2-oxoethyl)-5-ethyl-6-(4-(5-hydroxy-6-met hylpyrimidine-4-carbonyl)piperazin-1-yl)-7-oxo-4,7-dihydro-[1,2,4]triazolo[1,5-a]pyrimidin-2-yl )methylene)cyclobutane-1-carboxamide **116** (3.94 mg) in a yield of 8.64%.

MS m/z (ESI): 729.2 [M+1]
¹H NMR (400 MHz, DMSO) δ 10.35 (s, 1H), 10.22 (s, 1H), 8.58 (s, 1H), 8.02 (d, J = 8.6 Hz, 1H), 7.94 (d, J = 2.1 Hz, 1H), 7.71 (dd, J = 8.9, 2.1 Hz, 1H), 7.30 (s, 1H), 6.82 (s, 1H), 6.14 (t, J = 2.3 Hz, 1H), 5.28 (s, 2H), 4.52 (d, J = 12.4 Hz, 1H), 3.50 (d, J = 12.2 Hz, 3H), 3.31 - 3.15 (m, 3H), 3.08 (t, J = 7.7 Hz, 6H), 2.81 (d, J = 11.2 Hz, 1H), 2.63 (d, J = 10.8 Hz, 1H), 2.44 (s, 3H), 1.18 (t, J = 7.4 Hz, 3H).

### Example 117

### N-(2-chloro-4-(trifluoromethyl)phenyl)-2-(2-(cyclopentylidenemethyl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)a cetamide

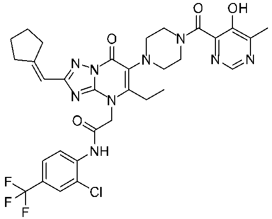

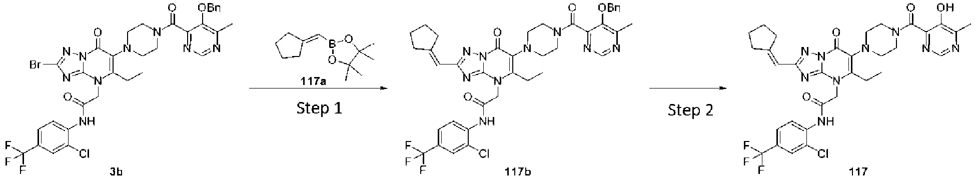

### Step 1

### 2-(6-(4-(5-(benzyloxy)-6-methylpyrimidine-4-carbonyl)piperazin-1-yl)-2-(cyclopentylidenemeth yl)-5-ethyl-7-oxo-[1,2,4]triazolo[1,5-a]pyrimidin-4(7H)-yl)-N-(2-chloro-4-(trifluoromethyl)phen yl)acetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (50 mg, 63.37 µmol), 2-(cyclopentylidenemethyl)-4,4,5,5-tetramethyl-1,3,2-dioxaborolane **117a** (15.83 mg, 76.05 µmol), [1,1'-bis(diphenylphosphino)ferrocene]palladium(II) dichloride dichloromethane complex (5.14 mg, 6.34 µmol), and sodium carbonate (20.15 mg, 190.11 µmol) were dissolved in 1,4-dioxane (1 mL) and water (0.3 mL). The mixture was subject to argon replacement four times and heated to 80 °C. The reaction was performed for 1.5 h. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-2-(cyclopentylidenemeth yl)-5-ethyl-7-oxo-[1,2,4]triazolo[1,5-a]pyrimidin-4(7H)-yl)-N-(2-chloro-4-(trifluoromethyl)phen yl)acetamide **117b** (37 mg) in a yield of 73.88%.

MS m/z (ESI): [M+1] 790.3

### Step 2

### N-(2-chloro-4-(trifluoromethyl)phenyl)-2-(2-(cyclopentylidenemethyl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)a cetamide

2-(6-(4-(5-(Benzyloxy)-6-methylpyrimidine-4-carbonyl)piperazin-1-yl)-2-(cyclopentyli denemethyl)-5-ethyl-7-oxo-[1,2,4]triazolo[1,5-a]pyrimidin-4(7H)-yl)-N-(2-chloro-4-(trifluoromet hyl)phenyl)acetamide **117b** (37 mg, 46.82 µmol) was added to dichloromethane (2.5 mL), and boron trichloride (0.8 mL) was added at 0 °C. The reaction was performed at 0 °C for 1 hour. Methanol was added to quench the reaction, and the mixture was concentrated under reduced pressure, followed by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(2-(cyclopentylidenemethyl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)a cetamide **117** (7.63 mg) with a yield of 12.38%.

MS m/z (ESI): 699.7 [M+1]
¹H NMR (400 MHz, DMSO) δ 10.38 (s, 1H), 10.24 (s, 1H), 8.58 (s, 1H), 8.05 (d, J = 8.6 Hz, 1H), 7.96 (d, J = 2.1 Hz, 1H), 7.72 (dd, J = 8.7, 2.1 Hz, 1H), 6.33 - 6.24 (m, 1H), 5.31 (s, 2H), 4.52 (d, J = 12.6 Hz, 1H), 3.53 (d, J = 12.0 Hz, 3H), 3.25 (t, J = 12.3 Hz, 1H), 3.01 (d, J = 9.9 Hz, 3H), 2.82 (d, J = 11.3 Hz, 1H), 2.74 (s, 2H), 2.64 (d, J = 10.8 Hz, 1H), 2.45 (s, 3H), 1.75 - 1.53 (m, 4H), 1.19 (t, J = 7.4 Hz, 3H).

### Example 118

### 2-(2-(bicyclo[2.2.1]hept-2-en-2-yl)-5-ethyl-6-(4-(5-hydroxy-6-methylpyrimidine-4-carbonyl)pipe razin-1-yl)-7-oxo-[1,2,4]triazolo[1,5-a]pyrimidin-4(7H)-yl)-N-(4-(pentafluoro-λ⁶-sulfaneyl)phen yl)acetamide

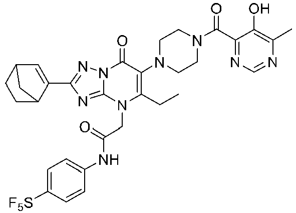

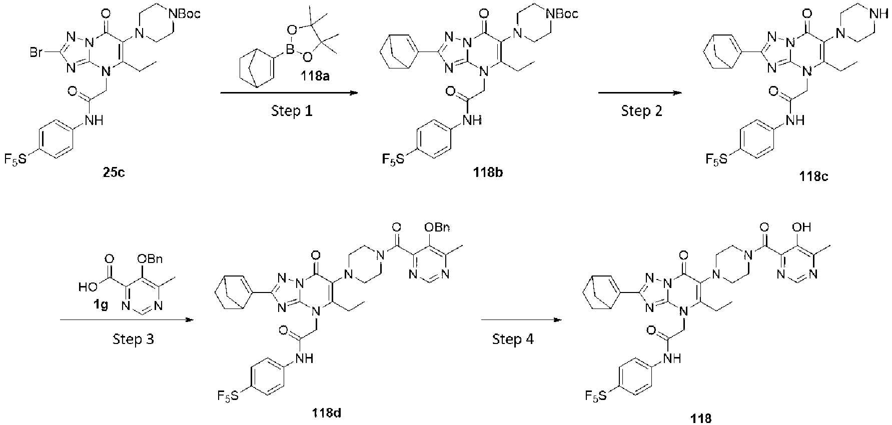

### Step 1

### tert-butyl 4-(2-(bicyclo[2.2.1]hept-2-en-2-yl)-5-ethyl-7-oxo-4-(2-oxo-2-((4-(pentafluoro-λ⁶-sulfaneyl)phen yl)amino)ethyl)-4,7-dihydro-[1,2,4]triazolo[1,5-a]pyrimidin-6-yl)piperazine-1-carboxylate

At room temperature, tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)piperazine-1-carboxylate **25c** (60 mg, 87.40 µmol), 2-(bicyclo[2.2.1]hept-2-en-2-yl)-4,4,5,5-tetramethyl-1,3,2-dioxaborolane **118a** (23.09 mg, 104.88 µmol), [1,1'-bis(diphenylphosphino)ferrocene]palladium(II) dichloride dichloromethane complex (7.08 mg, 8.74 µmol), and sodium carbonate (27.79 mg, 262.20 µmol) were dissolved in a mixed solution of 1,4-dioxane (2 mL) and water (0.6 mL). The mixture was subject to argon replacement four times, then heated to 80 °C and reacted for 1.5 hours. Water (20 mL) was added to the reaction mixture, which was extracted with ethyl acetate (20 mL ×3). The combined organic phase was washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(2-(bicyclo[2.2.1]hept-2-en-2-yl)-5-ethyl-7-oxo-4-(2-oxo-2-((4-(pentafluoro-λ⁶-sulfaneyl)phen yl)amino)ethyl)-4,7-dihydro-[1,2,4]triazolo[1,5-a]pyrimidin-6-yl)piperazine-1-carboxylate **118b** (45 mg) with a yield of 73.58%.

MS m/z (ESI): 700.2 [M+1]

### Step 2

### 2-(2-(bicyclo[2.2.1]hept-2-en-2-yl)-5-ethyl-7-oxo-6-(piperazin-1-yl)-[1,2,4]triazolo[1,5-a]pyrimi din-4(7H)-yl)-N-(4-(pentafluoro-λ⁶-sulfaneyl)phenyl)acetamide

Trifluoroacetic acid (0.8 mL) was added to a solution of tert-butyl 4-(2-(bicyclo[2.2.1]hept-2-en-2-yl)-5-ethyl-7-oxo-4-(2-oxo-2-((4-(pentafluoro-λ⁶-sulfaneyl)phen yl) amino)ethyl)-4,7-dihydro-[1,2,4]triazolo[1,5-a]pyrimidin-6-yl)piperazine-1-carboxylate **118b** (45 mg, 64.31 µmol) in dichloromethane (3 mL), and the reaction was performed at room temperature for 1 hour. The mixture was concentrated under reduced pressure to obtain 2-(2-(bicyclo[2.2.1]hept-2-en-2-yl)-5-ethyl-7-oxo-6-(piperazin-1-yl)-[1,2,4]triazolo[1,5-a]pyrimi din-4(7H)-yl)-N-(4-(pentafluoro-λ⁶-sulfaneyl)phenyl)acetamide **118c** (38 mg), which was used directly in the next step without further purification.

MS m/z (ESI): 600.2 [M+1]

### Step 3

### 2-(6-(4-(5-(benzyloxy)-6-methylpyrimidine-4-carbonyl)piperazin-1-yl)-2-(bicyclo[2.2.1]hept-2-e n-2-yl)-5-ethyl-7-oxo-[1,2,4]triazolo[1,5-a]pyrimidin-4(7H)-yl)-N-(4-(pentafluoro-λ⁶-sulfaneyl)p henyl)acetamide

2-(2-(Bicyclo[2.2.1]hept-2-en-2-yl)-5-ethyl-7-oxo-6-(piperazin-1-yl)-[1,2,4]triazolo[1,5 -a]pyrimidin-4(7H)-yl)-N-(4-(pentafluoro-λ⁶-sulfaneyl)phenyl)acetamide **118c** (38 mg, 63.37 µmol), 5-(benzyloxy)-6-methylpyrimidine-4-carboxylic acid **1g** (20.12 mg, 82.39 µmol), 1-(3-dimethylaminopropyl)-3-ethylcarbodiimide hydrochloride (18.22 mg, 95.06 µmol), 1-hydroxybenzotriazole (12.84 mg, 95.06 µmol), and N,N-diisopropylethylamine (40.95 mg, 316.87 µmol) were added to N,N-dimethylformamide (2 mL), and the reaction was performed at room temperature for 4 hours. Water (20 mL) was added to the reaction mixture, which was extracted with ethyl acetate (20 mL ×3). The combined organic phase was washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-2-(bicyclo[2.2.1]hept-2-e n-2-yl)-5-ethyl-7-oxo-[1,2,4]triazolo[1,5-a]pyrimidin-4(7H)-yl)-N-(4-(pentafluoro-λ⁶-sulfaneyl)p henyl)acetamide **118d** (30 mg) with a yield of 57.32%.

MS m/z (ESI): 826.2 [M+1]

### Step 4

### 2-(2-(bicyclo[2.2.1]hept-2-en-2-yl)-5-ethyl-6-(4-(5-hydroxy-6-methylpyrimidine-4-carbonyl)pipe razin-1-yl)-7-oxo-[1,2,4]triazolo[1,5-a]pyrimidin-4(7H)-yl)-N-(4-(pentafluoro-λ⁶-sulfaneyl)phen yl)acetamide

2-(6-(4-(5-(benzyloxy)-6-methylpyrimidine-4-carbonyl)piperazin-1-yl)-2-(bicyclo[2.2.1] hept-2-en-2-yl)-5-ethyl-7-oxo-[1,2,4]triazolo[1,5-a]pyrimidin-4(7H)-yl)-N-(4-(pentafluoro-λ⁶-sul faneyl)phenyl)acetamide **118d** (30 mg, 36.33 µmol) was added to dichloromethane (0.8 mL), followed by addition of trifluoroacetic acid (3 mL), and the reaction was performed at room temperature for 18 h. The mixture was concentrated under reduced pressure, and then separated by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain 2-(2-(bicyclo[2.2.1]hept-2-en-2-yl)-5-ethyl-6-(4-(5-hydroxy-6-methylpyrimidine-4-carbonyl) piperazin-1-yl)-7-oxo-[1,2,4]triazolo[1,5-a]pyrimidin-4(7H)-yl)-N-(4-(pentafluoro-λ⁶-sulfaneyl) phenyl)acetamide **118** (6.51 mg) with a yield of 12.88%.

MS m/z (ESI): 736.1 [M+1]
¹H NMR (400 MHz, DMSO) δ 10.96 (s, 1H), 10.24 (s, 1H), 8.58 (s, 1H), 7.93 - 7.84 (m, 2H), 7.77 (d, J = 8.9 Hz, 2H), 6.72 (d, J = 3.1 Hz, 1H), 5.18 (s, 2H), 4.52 (d, J = 12.6 Hz, 1H), 3.50 (d, J = 12.8 Hz, 4H), 3.25 (t, J = 12.7 Hz, 1H), 3.07 - 2.90 (m, 4H), 2.81 (d, J = 11.3 Hz, 1H), 2.64 (d, J = 11.0 Hz, 1H), 2.45 (s, 3H), 1.77 (d, J = 7.0 Hz, 2H), 1.45 (d, J = 8.2 Hz, 1H), 1.25 (d, J = 8.5 Hz, 1H), 1.16 (t, J = 7.4 Hz, 3H), 1.09 - 0.96 (m, 2H).

### Example 119

### 6-(4-(2-((2-chloro-4-(trifluoromethyl)phenyl)amino)-2-oxoethyl)-5-ethyl-6-(4-(5-hydroxy-6-met hylpyrimidine-4-carbonyl)piperazin-1-yl)-7-oxo-4,7-dihydro-[1,2,4]triazolo[1,5-a]pyrimidin-2-yl )spiro[3.3]hept-5-ene-2-carboxamide

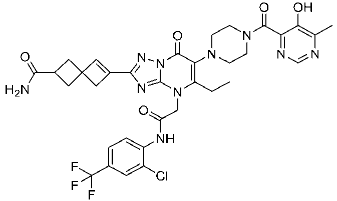

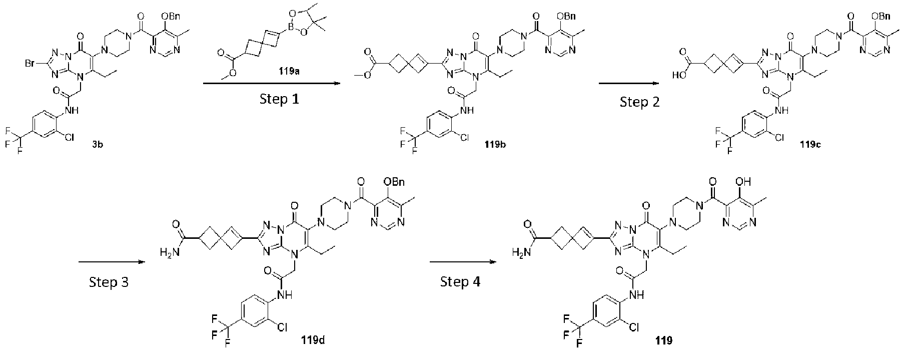

### Step 1

### methyl 6-(6-(4-(5-(benzyloxy)-6-methylpyrimidine-4-carbonyl)piperazin-1-yl)-4-(2-((2-chloro-4-(trifluo romethyl)phenyl)amino)-2-oxoethyl)-5-ethyl-7-oxo-4,7-dihydro-[1,2,4]triazolo[1,5-a]pyrimidin-2-yl)spiro[3.3]hept-5-ene-2-carboxylate

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (70 mg, 88.72 µmol), methyl 6-(4,4,5,5-tetramethyl-1,3,2-dioxaborolan-2-yl)spiro[3.3]hept-5-ene-2-carboxylate **119a** (29.61 mg, 106.46 µmol, prepared according to published patent WO2020168143), [1,1'-bis(diphenylphosphino) ferrocene]palladium(II) dichloride dichloromethane complex (7.19 mg, 8.87 µmol), and sodium carbonate (28.21 mg, 266.16 µmol) were dissolved in 1,4-dioxane (2 mL) and water (0.6 mL). The mixture was subject to argon replacement four times, then heated to 80 °C and reacted for 1.5 h. Water (20 mL) was added to the reaction mixture, and the mixture was extracted with ethyl acetate (20 mL ×3). The combined organic phases were washed with saturated sodium chloride solution (20 mL), dried over anhydrous sodium sulfate, filtered, and concentrated under reduced pressure. The resulting residue was purified by silica gel column chromatography (eluent: system B) to obtain methyl 6-(6-(4-(5-(benzyloxy)-6-methylpyrimidine-4-carbonyl)piperazin-1-yl)-4-(2-((2-chloro-4-(trifluo romethyl)phenyl)amino)-2-oxoethyl)-5-ethyl-7-oxo-4,7-dihydro-[1,2,4]triazolo [1,5-a]pyrimidin-2-yl)spiro[3.3]hept-5-ene-2-carboxylate **119b** (55 mg) with a yield of 72.06%.

MS m/z (ESI): 860.3 [M+1]

### Step 2

### 6-(6-(4-(5-(benzyloxy)-6-methylpyrimidine-4-carbonyl)piperazin-1-yl)-4-(2-((2-chloro-4-(trifluo romethyl)phenyl)amino)-2-oxoethyl)-5-ethyl-7-oxo-4,7-dihydro-[1,2,4]triazolo[1,5-a]pyrimidin-2-yl)spiro[3.3]hept-5-ene-2-carboxylic acid

Methyl 6-(6-(4-(5-(benzyloxy)-6-methylpyrimidine-4-carbonyl)piperazin-1-yl)-4-(2-((2-chloro-4-(trifluo romethyl)phenyl)amino)-2-oxoethyl)-5-ethyl-7-oxo-4,7-dihydro-[1,2,4]triazolo [1,5-a]pyrimidin-2-yl)spiro[3.3]hept-5-ene-2-carboxylate **119b** (55 mg, 63.93 µmol) was added to acetonitrile (3 mL) and water (0.6 mL), followed by addition of lithium hydroxide monohydrate (13.41 mg, 319.66 µmol), and the reaction was performed at room temperature for 18 h. At 0 °C, 1 N aqueous hydrochloric acid solution was added dropwise to adjust the pH to neutral. The mixture was extracted with ethyl acetate (20 mL ×3). The combined organic phases were washed with saturated sodium chloride solution (20 mL), dried over anhydrous sodium sulfate, filtered, and concentrated under reduced pressure. The resulting residue was purified by silica gel column chromatography (eluent: system B) to obtain 6-(6-(4-(5-(benzyloxy)-6-methylpyrimidine-4-carbonyl)piperazin-1-yl)-4-(2-((2-chloro-4-(trifluo romethyl)phenyl)amino)-2-oxoethyl)-5-ethyl-7-oxo-4,7-dihydro-[1,2,4]triazolo[1,5-a]pyrimidin-2-yl)spiro[3.3]hept-5-ene-2-carboxylic acid **119c** (30 mg) with a yield of 55.45%.

MS m/z (ESI): 846.3 [M+1]

### Step 3

### 6-(6-(4-(5-(benzyloxy)-6-methylpyrimidine-4-carbonyl)piperazin-1-yl)-4-(2-((2-chloro-4-(trifluo romethyl)phenyl)amino)-2-oxoethyl)-5-ethyl-7-oxo-4,7-dihydro-[1,2,4]triazolo[1,5-a]pyrimidin-2-yl)spiro[3.3]hept-5-ene-2-carboxamide

6-(6-(4-(5-(Benzyloxy)-6-methylpyrimidine-4-carbonyl)piperazin-1-yl)-4-(2-((2-chloro-4-(trifluoromethyl)phenyl)amino)-2-oxoethyl)-5-ethyl-7-oxo-4,7-dihydro-[1,2,4]triazolo[1,5-a]py rimidin-2-yl)spiro[3.3]hept-5-ene-2-carboxylic acid **119c** (30 mg, 35.45 µmol) was added to dichloromethane (1 mL), and N,N'-carbonyldiimidazole (7.47 mg, 46.09 µmol) was added. The reaction was performed at room temperature for 2 hours, then aqueous ammonia (0.3 mL) was added, and the reaction was continued at room temperature for 2 hours. The reaction mixture was extracted with dichloromethane (20 mL × 3), and the combined organic phase was washed with saturated sodium chloride solution (20 mL), dried over anhydrous sodium sulfate, filtered, and concentrated under reduced pressure. The resulting residue was purified by silica gel column chromatography (eluent: System A) to obtain 6-(6-(4-(5-(benzyloxy)-6-methylpyrimidine-4-carbonyl)piperazin-1-yl)-4-(2-((2-chloro-4-(trifluo romethyl)phenyl)amino)-2-oxoethyl)-5-ethyl-7-oxo-4,7-dihydro-[1,2,4]triazolo[1,5-a]pyrimidin-2-yl)spiro[3.3]hept-5-ene-2-carboxamide **119d** (23 mg) in a yield of 76.76%.

MS m/z (ESI): 845.1 [M+1]

### Step 4

### 6-(4-(2-((2-chloro-4-(trifluoromethyl)phenyl)amino)-2-oxoethyl)-5-ethyl-6-(4-(5-hydroxy-6-met hylpyrimidine-4-carbonyl)piperazin-1-yl)-7-oxo-4,7-dihydro-[1,2,4]triazolo[1,5-a]pyrimidin-2-yl )spiro[3.3]hept-5-ene-2-carboxamide

6-(6-(4-(5-(Benzyloxy)-6-methylpyrimidine-4-carbonyl)piperazin-1-yl)-4-(2-((2-chloro-4-(trifluoromethyl)phenyl)amino)-2-oxoethyl)-5-ethyl-7-oxo-4,7-dihydro-[1,2,4]triazolo[1,5-a]py rimidin-2-yl)spiro[3.3]hept-5-ene-2-carboxamide **119d** (23 mg, 27.21 µmol) was added to dichloromethane (2.5 mL), and boron trichloride (0.8 mL) was added at 0 °C. The reaction was performed at 0 °C for 1 hour. Methanol was added to quench the reaction, and the mixture was concentrated under reduced pressure. The resulting product was separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain 6-(4-(2-((2-chloro-4-(trifluoromethyl)phenyl)amino)-2-oxoethyl)-5-ethyl-6-(4-(5-hydroxy-6-met hylpyrimidine-4-carbonyl)piperazin-1-yl)-7-oxo-4,7-dihydro-[1,2,4]triazolo[1,5-a]pyrimidin-2-yl )spiro[3.3]hept-5-ene-2-carboxamide **119** (4.95 mg) in a yield of 12.39%.

MS m/z (ESI): 755.1 [M+1]
¹H NMR (400 MHz, DMSO) δ 10.34 (s, 1H), 10.23 (s, 1H), 8.58 (s, 1H), 8.08 (d, J = 8.8 Hz, 1H), 7.97 (s, 1H), 7.71 (d, J = 8.8 Hz, 1H), 7.20 (s, 1H), 6.73 (s, 1H), 6.62 (s, 1H), 5.32 (s, 2H), 4.52 (d, J = 12.0 Hz, 1H), 3.49 (d, J = 11.8 Hz, 3H), 3.02 - 2.93 (m, 3H), 2.87 (s, 1H), 2.81 (d, J = 11.3 Hz, 1H), 2.72 (s, 1H), 2.62 (s, 1H), 2.44 (s, 3H), 2.42 - 2.31 (m, 3H), 2.24 (s, 2H), 1.18 (t, J = 7.4 Hz, 3H).

### Example 120

### 2-(5-ethyl-6-(4-(5-hydroxy-6-methylpyrimidine-4-carbonyl)piperazin-1-yl)-7-oxo-2-(spiro[2.5]oc t-5-en-6-yl)-[1,2,4]triazolo[1,5-a]pyrimidin-4(7H)-yl)-N-(4-(pentafluoro-λ6-sulfaneyl)phenyl)ace tamide

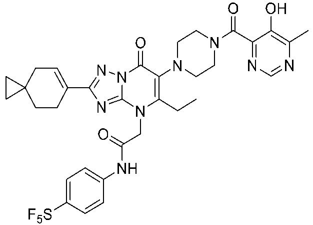

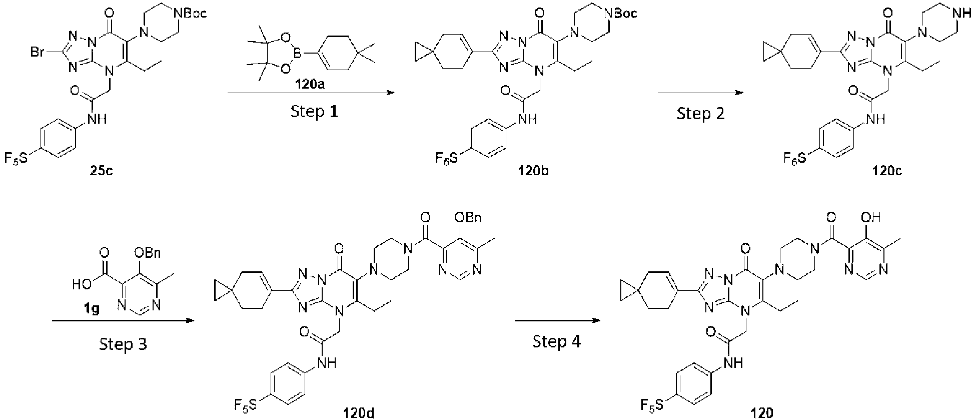

### Step 1

### tert-butyl 4-(5-ethyl-7-oxo-4-(2-oxo-2-((4-(pentafluoro-λ6-sulfaneyl)phenyl)amino)ethyl)-2-(spiro[2.5]oct-5-en-6-yl)-4,7-dihydro-[1,2,4]triazolo[1,5-a]pyrimidin-6-yl)piperazine-1-carboxylate

At room temperature, tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)piperazine-1-carboxylate **25c** (60 mg, 87.40 µmol), 4,4,5,5-tetramethyl-2-(spiro[2.5]oct-5-en-6-yl)-1,3,2-dioxaborolane **120a** (24.56 mg, 104.88 µmol, commercially available), [1,1'-bis(diphenylphosphino)ferrocene]palladium(II) dichloride dichloromethane complex (7.08 mg, 8.74 µmol), and sodium carbonate (27.79 mg, 262.20 µmol) were dissolved in 1,4-dioxane (2 mL) and water (0.6 mL). The mixture was subject to argon replacement four times, then heated to 80 °C and reacted for 1.5 hours. Water (20 mL) was added to the reaction mixture, which was extracted with ethyl acetate (20 mL × 3). The combined organic phase was washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(5-ethyl-7-oxo-4-(2-oxo-2-((4-(pentafluoro-λ6-sulfaneyl)phenyl)amino)ethyl)-2-(spiro[2.5]oct-5-en-6-yl)-4,7-dihydro-[1,2,4]triazolo[1,5-a]pyrimidin-6-yl)piperazine-1-carboxylate **120b** (45 mg) in a yield of 72.13%.

MS m/z (ESI): [M+1] 714.2

### Step 2

### 2-(5-ethyl-7-oxo-6-(piperazin-1-yl)-2-(spiro[2.5]oct-5-en-6-yl)-[1,2,4]triazolo[1,5-a]pyrimidin-4( 7H)-yl)-N-(4-(pentafluoro-λ6-sulfaneyl)phenyl)acetamide

Tert-butyl 4-(5-ethyl-7-oxo-4-(2-oxo-2-((4-(pentafluoro-λ6-sulfaneyl)phenyl)amino) ethyl)-2-(spiro[2.5]oct-5-en-6-yl)-4,7-dihydro-[1,2,4]triazolo[1,5-a]pyrimidin-6-yl)piperazine-1-c arboxylate **120b** (45 mg, 63.05 µmol) was added to dichloromethane (3 mL), followed by addition of trifluoroacetic acid (0.8 mL). The reaction was performed at room temperature for 1 hour. The mixture was concentrated under reduced pressure to obtain 2-(5-ethyl-7-oxo-6-(piperazin-1-yl)-2-(spiro[2.5]oct-5-en-6-yl)-[1,2,4]triazolo[1,5-a]pyrimidin-4( 7H)-yl)-N-(4-(pentafluoro-λ6-sulfaneyl)phenyl)acetamide **120c** (38 mg), which was used directly in the next step as a crude product.

MS m/z (ESI): 614.2 [M+1]

### Step 3

### 2-(6-(4-(5-(benzyloxy)-6-methylpyrimidine-4-carbonyl)piperazin-1-yl)-5-ethyl-7-oxo-2-(spiro[2. 5]oct-5-en-6-yl)-[1,2,4]triazolo[1,5-a]pyrimidin-4(7H)-yl)-N-(4-(pentafluoro-λ6-sulfaneyl)phenyl )acetamide

2-(5-Ethyl-7-oxo-6-(piperazin-1-yl)-2-(spiro[2.5]oct-5-en-6-yl)-[1,2,4]triazolo[1,5-a]py rimidin-4(7H)-yl)-N-(4-(pentafluoro-λ6-sulfaneyl)phenyl)acetamide **120c** (38 mg, 61.93 µmol), 5-(benzyloxy)-6-methylpyrimidine-4-carboxylic acid **1g** (19.66 mg, 80.50 µmol), 1-(3-dimethylaminopropyl)-3-ethylcarbodiimide hydrochloride (17.81 mg, 92.89 µmol), 1-hydroxybenzotriazole (12.55 mg, 92.89 µmol), and N,N-diisopropylethylamine (40.02 mg, 309.63 µmol) were added to N,N-dimethylformamide (2 mL), and the reaction was performed at room temperature for 4 h. Water (20 mL) was added to the reaction mixture, and the mixture was extracted with ethyl acetate (20 mL ×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-5-ethyl-7-oxo-2-(spiro[2. 5]oct-5-en-6-yl)-[1,2,4]triazolo[1,5-a]pyrimidin-4(7H)-yl)-N-(4-(pentafluoro-λ6-sulfaneyl)phenyl )acetamide **120d** (33 mg) with a yield of 63.45%.

MS m/z (ESI): 840.3 [M+1]

### Step 4

### 2-(5-Ethyl-6-(4-(5-hydroxy-6-methylpyrimidine-4-carbonyl)piperazin-1-yl)-7-oxo-2-(spiro[2.5]o ct-5-en-6-yl)-[1,2,4]triazolo[1,5-a]pyrimidin-4(7H)-yl)-N-(4-(pentafluoro-λ6-sulfaneyl)phenyl)ac etamide

2-(6-(4-(5-(Benzyloxy)-6-methylpyrimidine-4-carbonyl)piperazin-1-yl)-5-ethyl-7-oxo-2 -(spiro[2.5]oct-5-en-6-yl)-[1,2,4]triazolo[1,5-a]pyrimidin-4(7H)-yl)-N-(4-(pentafluoro-λ⁶-sulfane yl)phenyl)acetamide **120d** (33 mg, 39.29 µmol) was added to dichloromethane (1 mL), and trifluoroacetic acid (3 mL) was added. The reaction was performed at room temperature for 18 h. The mixture was concentrated under reduced pressure and then separated by preparative liquid chromatography (separation column: AKZONOBEL Kromasil; 250 ×21.2 mm I.D.; 5 µm, 20 mL/min; mobile phase A: 0.05% TFA+H₂O, mobile phase B: CH₃CN) to obtain 2-(5-ethyl-6-(4-(5-hydroxy-6-methylpyrimidine-4-carbonyl)piperazin-1-yl)-7-oxo-2-(spiro[2.5]oc t-5-en-6-yl)-[1,2,4]triazolo[1,5-a]pyrimidin-4(7H)-yl)-N-(4-(pentafluoro-λ6-sulfaneyl)phenyl)ace tamide **120** (1.44 mg) with a yield of 4.85%.

MS m/z (ESI): 750.2 [M+1]
¹H NMR (400 MHz, DMSO) δ 10.98 (s, 1H), 10.25 (s, 1H), 8.58 (s, 1H), 7.88 (d, J = 9.1 Hz, 2H), 7.77 (d, J = 8.9 Hz, 2H), 6.84 (s, 1H), 5.19 (s, 2H), 4.52 (d, J = 12.4 Hz, 1H), 2.98 (s, 4H), 2.82 (d, J = 10.5 Hz, 1H), 2.65 (d, J = 12.9 Hz, 2H), 2.55 (d, J = 3.4 Hz, 2H), 2.45 (s, 3H), 2.09 (s, 2H), 1.48 (t, J = 6.2 Hz, 2H), 1.17 (t, J = 7.4 Hz, 3H), 0.39 - 0.27 (m, 4H).

### Example 121

### N-(2-chloro-4-(trifluoromethyl)phenyl)-2-(5-ethyl-2-(5-hydroxy-1,3a,4,5,6,6a-hexahydropentalen -2-yl)-6-(4-(3-hydroxypicolinoyl)piperazin-1-yl)-7-oxo-[1,2,4]triazolo[1,5-a]pyrimidin-4(7H)-yl) acetamide

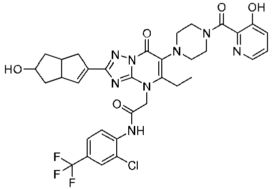

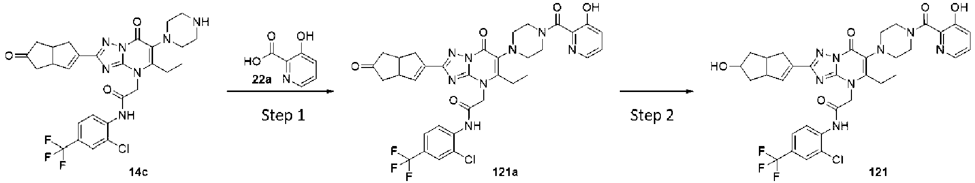

### Step 1

### N-(2-chloro-4-(trifluoromethyl)phenyl)-2-(5-ethyl-6-(4-(3-hydroxypicolinoyl)piperazin-1-yl)-7-o xo-2-(5-oxo-1,3a,4,5,6,6a-hexahydropentalen-2-yl)-[1,2,4]triazolo[1,5-a]pyrimidin-4(7H)-yl)acet amide

N-(2-chloro-4-(trifluoromethyl)phenyl)-2-(5-ethyl-7-oxo-2-(5-oxo-1,3a,4,5,6,6a-hexahy dropentalen-2-yl)-6-(piperazin-1-yl)-[1,2,4]triazolo[1,5-a]pyrimidin-4(7H)-yl)acetamide **14c** (428 mg, 708.58 µmol), 3-hydroxypicolinic acid **22a** (128.14 mg, 921.16 µmol), 1-(3-dimethylaminopropyl)-3-ethylcarbodiimide hydrochloride (203.75 mg, 1.06 mmol), 1-hydroxybenzotriazole (143.62 mg, 1.06 mmol), and N,N-diisopropylethylamine (457.89 mg, 3.54 mmol) were added to N,N-dimethylformamide (10 mL), and the reaction was performed at room temperature for 4 h. Water (20 mL) was added to the reaction mixture, and the mixture was extracted with ethyl acetate (20 mL ×3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain N-(2-chloro-4-(trifluoromethyl)phenyl)-2-(5-ethyl-6-(4-(3-hydroxypicolinoyl)piperazin-1-yl)-7-o xo-2-(5-oxo-1,3a,4,5,6,6a-hexahydropentalen-2-yl)-[1,2,4]triazolo[1,5-a]pyrimidin-4(7H)-yl)acet amide **121a** (280 mg) with a yield of 54.50%.

MS m/z (ESI): 725.2 [M+1]

### Step 2

### N-(2-chloro-4-(trifluoromethyl)phenyl)-2-(5-ethyl-2-(5-hydroxy-1,3a,4,5,6,6a-hexahydropentalen -2-yl)-6-(4-(3-hydroxypicolinoyl)piperazin-1-yl)-7-oxo-[1,2,4]triazolo[1,5-a]pyrimidin-4(7H)-yl) acetamide

N-(2-chloro-4-(trifluoromethyl)phenyl)-2-(5-ethyl-6-(4-(3-hydroxypicolinoyl)piperazin-1-yl)-7-oxo-2-(5-oxo-1,3a,4,5,6,6a-hexahydropentalen-2-yl)-[1,2,4]triazolo[1,5-a]pyrimidin-4(7 H)-yl)acetamide **121a** (70 mg, 96.54 µmol) was added to methanol (2 mL), and sodium borohydride (3.65 mg, 96.54 µmol) was added at 0°C. The temperature was raised to room temperature, and the reaction was performed for 1 h. Water (20 mL) was added at 0 °C, and the mixture was extracted with ethyl acetate (20 mL ×3). The combined organic phases were washed with saturated sodium chloride solution (20 mL), dried over anhydrous sodium sulfate, filtered, and concentrated under reduced pressure. The residue was then separated by preparative liquid chromatography (separation column: AKZONOBEL Kromasil; 250 ×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(5-ethyl-2-(5-hydroxy-1,3a,4,5,6,6a-hexahydropentalen -2-yl)-6-(4-(3-hydroxypicolinoyl)piperazin-1-yl)-7-oxo-[1,2,4]triazolo[1,5-a]pyrimidin-4(7H)-yl) acetamide **121** (11.83 mg) with a yield of 3.95%.

MS m/z (ESI): 727.2 [M+1]
¹H NMR (400 MHz, DMSO) δ 10.59 (s, 1H), 10.37 (s, 1H), 8.18 - 8.03 (m, 2H), 7.97 (s, 1H), 7.72 (d, J = 8.6 Hz, 1H), 7.35 (d, J = 4.5 Hz, 2H), 6.54 (s, 1H), 5.32 (s, 2H), 4.55 (d, J = 12.5 Hz, 1H), 3.98 (q, J = 7.0 Hz, 1H), 3.45 (dd, J = 24.6, 12.5 Hz, 3H), 3.22 (d, J = 14.2 Hz, 2H), 2.98 (d, J = 13.4 Hz, 4H), 2.83 - 2.52 (m, 5H), 2.06 (d, J = 10.4 Hz, 2H), 1.31 - 1.22 (m, 2H), 1.18 (t, J = 7.4 Hz, 3H).

### Example 122

### (E)-N-(2-chloro-4-(trifluoromethyl)phenyl)-2-(5-ethyl-6-(4-(5-hydroxy-6-methylpyrimidine-4-ca rbonyl)piperazin-1-yl)-2-(5-(methoxyimino)-1,3a,4,5,6,6a-hexahydropentalen-2-yl)-7-oxo-[1,2,4] triazolo[1,5-a]pyrimidin-4(7H)-yl)acetamide

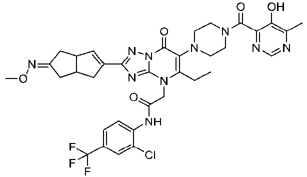

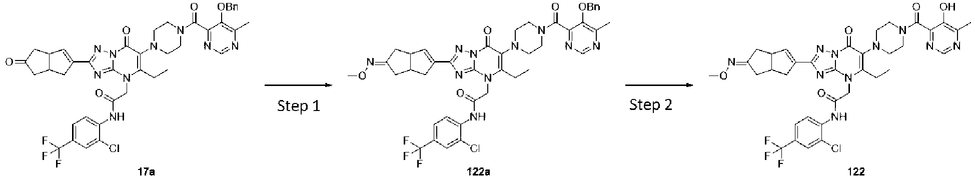

### Step 1

### (E)-2-(6-(4-(5-(benzyloxy)-6-methylpyrimidine-4-carbonyl)piperazin-1-yl)-5-ethyl-2-(5-(methox yimino)-1,3a,4,5,6,6a-hexahydropentalen-2-yl)-7-oxo-[1,2,4]triazolo[1,5-a]pyrimidin-4(7H)-yl)-N-(2-chloro-4-(trifluoromethyl)phenyl)acetamide

2-(6-(4-(5-(Benzyloxy)-6-methylpyrimidine-4-carbonyl)piperazin-1-yl)-5-ethyl-7-oxo-2 -(5-oxo-1,3a,4,5,6,6a-hexahydropentalen-2-yl)-[1,2,4]triazolo[1,5-a]pyrimidin-4(7H)-yl)-N-(2-ch loro-4-(trifluoromethyl)phenyl)acetamide **17a** (40 mg, 48.18 µmol) was added to ethanol (1.5 mL), followed by addition of methoxyamine hydrochloride (12.07 mg, 144.53 µmol) and pyridine (0.1 mL). The reaction was performed at room temperature for 18 hours. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System A) to obtain (E)-2-(6-(4-(5-(benzyloxy)-6-methylpyrimidine-4-carbonyl)piperazin-1-yl)-5-ethyl-2-(5-(methox yimino)-1,3a,4,5,6,6a-hexahydropentalen-2-yl)-7-oxo-[1,2,4]triazolo[1,5-a]pyrimidin-4(7H)-yl)-N-(2-chloro-4-(trifluoromethyl)phenyl)acetamide **122a** (30 mg) in a yield of 72.47%.

MS m/z (ESI): 859.2 [M+1]

### Step 2

### (E)-N-(2-chloro-4-(trifluoromethyl)phenyl)-2-(5-ethyl-6-(4-(5-hydroxy-6-methylpyrimidine-4-ca rbonyl)piperazin-1-yl)-2-(5-(methoxyimino)-1,3a,4,5,6,6a-hexahydropentalen-2-yl)-7-oxo-[1,2,4] triazolo[1,5-a]pyrimidin-4(7H)-yl)acetamide

(E)-2-(6-(4-(5-(benzyloxy)-6-methylpyrimidine-4-carbonyl)piperazin-1-yl)-5-ethyl-2-(5 -(methoxyimino)-1,3a,4,5,6,6a-hexahydropentalen-2-yl)-7-oxo-[1,2,4]triazolo[1,5-a]pyrimidin-4( 7H)-yl)-N-(2-chloro-4-(trifluoromethyl)phenyl)acetamide **122a** (30 mg, 34.91 µmol) was added to dichloromethane (2.5 mL), followed by addition of boron trichloride (0.8 mL) at 0 °C. The reaction was performed at 0 °C for 1 hour. The reaction was quenched by addition of methanol, and the mixture was concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain (E)-N-(2-chloro-4-(trifluoromethyl)phenyl)-2-(5-ethyl-6-(4-(5-hydroxy-6-methylpyrimidine-4-ca rbonyl) piperazin-1-yl)-2-(5-(methoxyimino)-1,3a,4,5,6,6a-hexahydropentalen-2-yl)-7-oxo-[1,2,4] triazolo[1,5-a]pyrimidin-4(7H)-yl)acetamide **122** (3.32 mg) in a yield of 12.24%.

MS m/z (ESI): 769.2 [M+1]
¹H NMR (400 MHz, DMSO) δ 10.36 (s, 1H), 10.24 (s, 1H), 8.58 (s, 1H), 8.07 (d, J = 8.6 Hz, 1H), 7.97 (d, J = 2.1 Hz, 1H), 7.71 (dd, J = 9.1, 2.1 Hz, 1H), 6.46 (d, J = 11.7 Hz, 1H), 5.32 (s, 2H), 4.52 (d, J = 12.5 Hz, 1H), 3.73 - 3.63 (m, 3H), 3.48 (t, J = 11.1 Hz, 4H), 3.24 (t, J = 12.5 Hz, 1H), 2.92 (s, 4H), 2.81 (d, J = 11.4 Hz, 1H), 2.77 - 2.55 (m, 4H), 2.44 (s, 3H), 2.14 - 2.03 (m, 1H), 1.18 (t, J = 7.4 Hz, 3H).

### Example 123

### N-(2-chloro-4-(trifluoromethyl)phenyl)-2-(5-ethyl-6-(4-(5-hydroxy-6-methylpyrimidine-4-carbon yl)piperazin-1-yl)-2-(1H-indo1-6-yl)-7-oxo-[1,2,4]triazolo[1,5-a]pyrimidin-4(7H)-yl)acetamide

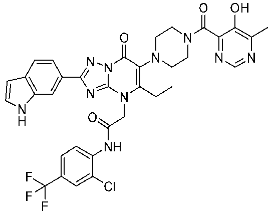

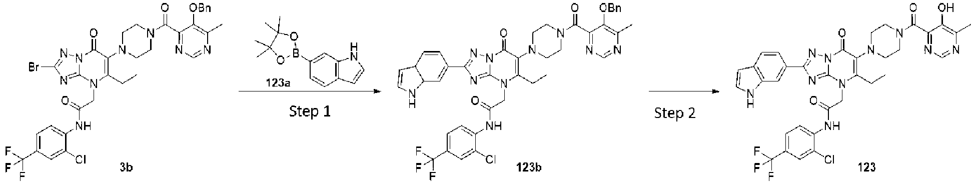

### Step 1

### 2-(6-(4-(5-(benzyloxy)-6-methylpyrimidine-4-carbonyl)piperazin-1-yl)-2-(3a,7a-dihydro-1H-ind ol-6-yl)-5-ethyl-7-oxo-[1,2,4]triazolo[1,5-a]pyrimidin-4(7H)-yl)-N-(2-chloro-4-(trifluoromethyl) phenyl)acetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (50 mg, 63.37 µmol), 6-(4,4,5,5-tetramethyl-1,3,2-dioxaborolan-2-yl)-1H-indole **123a** (18.49 mg, 76.05 µmol, commercially available), [1,1'-bis(diphenylphosphino)ferrocene]palladium(II) dichloride dichloromethane complex (5.14 mg, 6.34 µmol), and sodium carbonate (20.15 mg, 190.11 µmol) were dissolved in 1,4-dioxane (1 mL) and water (0.3 mL). The mixture was subject to argon replacement four times, then heated to 80 °C and reacted for 1.5 h. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-2-(3a,7a-dihydro-1H-ind ol-6-yl)-5-ethyl-7-oxo-[1,2,4]triazolo[1,5-a]pyrimidin-4(7H)-yl)-N-(2-chloro-4-(trifluoromethyl) phenyl)acetamide **123b** (36 mg) in a yield of 68.84%.

MS m/z (ESI): 827.2 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-2-(1H-indol-6-yl)-7-oxo-[1,2,4]triazolo[1,5-a]pyrimidin-4(7H)-yl)acetamide

2-(6-(4-(5-(Benzyloxy)-6-methylpyrimidine-4-carbonyl)piperazin-1-yl)-2-(3a,7a-dihydr o-1H-indol-6-yl)-5-ethyl-7-oxo-[1,2,4]triazolo[1,5-a]pyrimidin-4(7H)-yl)-N-(2-chloro-4-(trifluor omethyl)phenyl)acetamide **123b** (36 mg, 43.62 µmol) was added to dichloromethane (2.5 mL). Boron trichloride (0.8 mL) was added at 0 °C, and the reaction was performed at 0 °C for 1 h. Methanol was added to quench the reaction, and the mixture was concentrated under reduced pressure. The residue was separated by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-2-(1H-indol-6-yl)-7-oxo-[1,2,4] triazolo[1,5-a]pyrimidin-4(7H)-yl)acetamide **123** (10.47 mg) in a yield of 7.87%.

MS m/z (ESI): 735.2 [M+1]
¹H NMR (400 MHz, DMSO) δ 11.33 (d, J = 2.4 Hz, 1H), 10.42 (s, 1H), 10.24 (s, 1H), 8.59 (s, 1H), 8.18 (s, 1H), 8.07 (d, J = 8.5 Hz, 1H), 7.97 (d, J = 2.1 Hz, 1H), 7.79 (dd, J = 8.3, 1.5 Hz, 1H), 7.71 (dd, J = 8.7, 2.2 Hz, 1H), 7.64 (d, J = 8.3 Hz, 1H), 7.50 (dt, J = 5.5, 2.4 Hz, 1H), 6.50 (d, J = 2.6 Hz, 1H), 5.41 (s, 2H), 4.55 (d, J = 12.5 Hz, 1H), 3.61 - 3.47 (m, 3H), 3.27 (d, J = 24.7 Hz, 1H), 3.03 (d, J = 9.4 Hz, 3H), 2.85 (d, J = 11.4 Hz, 1H), 2.68 (d, J = 10.3 Hz, 1H), 2.45 (s, 3H), 1.22 (t, J = 7.4 Hz, 3H).

### Example 124

### 2-(2-(benzofuran-4-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamid e

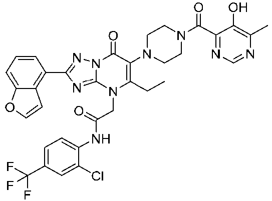

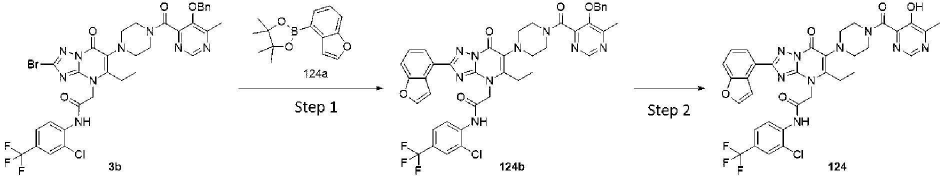

### Step 1

### 2-(2-(benzofuran-4-yl)-6-(4-(5-(benzyloxy)-6-methylpyrimidine-4-carbonyl)piperazin-1-yl)-5-eth yl-7-oxo-[1,2,4]triazolo[1,5-a]pyrimidin-4(7H)-yl)-N-(2-chloro-4-(trifluoromethyl)phenyl)aceta mide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (50 mg, 63.37 µmol), 2-(benzofuran-4-yl)-4,4,5,5-tetramethyl-1,3,2-dioxaborolane **124a** (18.56 mg, 76.05 µmol, commercially available), [1,1'-bis(diphenylphosphino)ferrocene]palladium(II) dichloride dichloromethane complex (5.14 mg, 6.34 µmol), and sodium carbonate (20.15 mg, 190.11 µmol) were dissolved in 1,4-dioxane (1 mL) and water (0.3 mL). The mixture was subject to argon replacement four times, then heated to 80 °C and reacted for 1.5 h. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(2-(benzofuran-4-yl)-6-(4-(5-(benzyloxy)-6-methylpyrimidine-4-carbonyl)piperazin-1-yl)-5-eth yl-7-oxo-[1,2,4]triazolo[1,5-a]pyrimidin-4(7H)-yl)-N-(2-chloro-4-(trifluoromethyl)phenyl)aceta mide **124b** (35 mg) in a yield of 66.85%.

MS m/z (ESI): 826.2 [M+1]

### Step 2

### 2-(2-(benzo furan-4-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamid e

2-(2-(Benzofuran-4-yl)-6-(4-(5-(benzyloxy)-6-methylpyrimidine-4-carbonyl)piperazin-1-yl)-5-ethyl-7-oxo-[1,2,4]triazolo[1,5-a]pyrimidin-4(7H)-yl)-N-(2-chloro-4-(trifluoromethyl) phenyl)acetamide **124b** (35 mg, 42.36 µmol) was added to dichloromethane (2.5 mL). Boron trichloride (0.8 mL) was added at 0 °C, and the reaction was performed at 0 °C for 1 h. Methanol was added to quench the reaction, and the mixture was concentrated under reduced pressure. The residue was separated by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 2-(2-(benzofuran-4-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamid e **124** (14.82 mg) in a yield of 11.34%.

MS m/z (ESI): 736.1 [M+1]
¹H NMR (400 MHz, DMSO) δ 10.48 (s, 1H), 10.25 (s, 1H), 8.59 (s, 1H), 8.14 (d, J = 2.2 Hz, 1H), 8.06 (t, J = 8.0 Hz, 2H), 7.97 (d, J = 2.1 Hz, 1H), 7.80 - 7.64 (m, 3H), 7.46 (t, J = 8.0 Hz, 1H), 5.46 (s, 2H), 4.55 (d, J = 12.6 Hz, 1H), 3.54 (t, J = 11.9 Hz, 4H), 3.28 (d, J = 24.9 Hz, 1H), 3.12 - 2.98 (m, 3H), 2.87 (d, J = 11.3 Hz, 1H), 2.69 (d, J = 11.2 Hz, 1H), 2.45 (s, 3H), 1.23 (t, J = 7.5 Hz, 3H).

### Example 125

### 2-(2-(benzo[d]thiazol-6-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)aceta mide

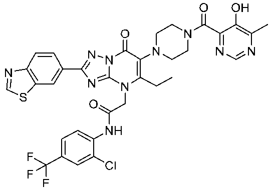

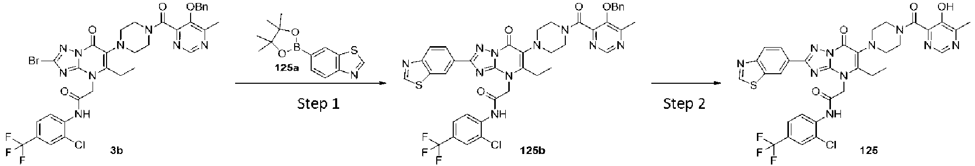

### Step 1

### 2-(2-(benzo[d]thiazol-6-yl)-6-(4-(5-(benzyloxy)-6-methylpyrimidine-4-carbonyl)piperazin-1-yl)-5-ethyl-7-oxo-[1,2,4]triazolo[1,5-a]pyrimidin-4(7H)-yl)-N-(2-chloro-4-(trifluoromethyl)phenyl)a cetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (50 mg, 63.37 µmol), 6-(4,4,5,5-tetramethyl-1,3,2-dioxaborolan-2-yl)-1,3-benzothiazole **125a** (19.86 mg, 76.05 µmol, commercially available), [1,1'-bis(diphenylphosphino)ferrocene]palladium(II) dichloride dichloromethane complex (5.14 mg, 6.34 µmol), and sodium carbonate (20.15 mg, 190.11 µmol) were added to 1,4-dioxane (1 mL) and water (0.3 mL). The mixture was subject to argon replacement four times, then heated to 80 °C and reacted for 1.5 hours. Water (20 mL) was added to the reaction mixture, which was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(2-(benzo[d]thiazol-6-yl)-6-(4-(5-(benzyloxy)-6-methylpyrimidine-4-carbonyl)piperazin-1-yl)-5-ethyl-7-oxo-[1,2,4]triazolo[1,5-a]pyrimidin-4(7H)-yl)-N-(2-chloro-4-(trifluoromethyl)phenyl)a cetamide **125b** (33 mg) in a yield of 61.75%.

MS m/z (ESI): 843.2 [M+1]

### Step 2

### 2-(2-(benzo[d]thiazol-6-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)aceta mide

2-(2-(Benzo[d]thiazol-6-yl)-6-(4-(5-(benzyloxy)-6-methylpyrimidine-4-carbonyl)pipera zin-1-yl)-5-ethyl-7-oxo-[1,2,4]triazolo[1,5-a]pyrimidin-4(7H)-yl)-N-(2-chloro-4-(trifluoromethyl )phenyl)acetamide **125b** (33 mg, 39.13 µmol) was added to dichloromethane (2.5 mL). Boron trichloride (0.8 mL) was added at 0 °C, and the reaction was performed at 0 °C for 1 hour. Methanol was added to quench the reaction, and the mixture was concentrated under reduced pressure. The residue was separated by preparative liquid chromatography (separation column: AKZONOBEL Kromasil; 250×21.2 mm I.D.; 5 µm, 20 mL/min; mobile phase A: 0.05% TFA + H₂O, mobile phase B: CH₃CN) to obtain 2-(2-(benzo[d]thiazol-6-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)aceta mide **125** (15.27 mg) in a yield of 10.31%.

MS m/z (ESI): 753.1 [M+1]
¹H NMR (400 MHz, DMSO) δ 10.44 (s, 1H), 10.24 (s, 1H), 9.50 (s, 1H), 8.93 (d, J = 1.7 Hz, 1H), 8.59 (s, 1H), 8.28 (dd, J = 8.6, 1.7 Hz, 1H), 8.21 (d, J = 8.6 Hz, 1H), 8.08 (d, J = 8.6 Hz, 1H), 7.98 (d, J = 2.1 Hz, 1H), 7.74 - 7.67 (m, 1H), 5.43 (s, 2H), 4.55 (d, J = 12.3 Hz, 1H), 3.59 - 3.45 (m, 3H), 3.28 (d, J = 25.2 Hz, 1H), 3.03 (d, J = 9.9 Hz, 3H), 2.86 (d, J = 11.3 Hz, 1H), 2.70 (s, 1H), 2.45 (s, 3H), 1.23 (t, J = 7.4 Hz, 3H).

### Example 126

### N-(2-chloro-4-(trifluoromethyl)phenyl)-2-(5-ethyl-6-(4-(5-hydroxy-6-methylpyrimidine-4-carbon yl)piperazin-1-yl)-2-(imidazo[1,2-a]pyridin-6-yl)-7-oxo-[1,2,4]triazolo[1,5-a]pyrimidin-4(7H)-yl )acetamide

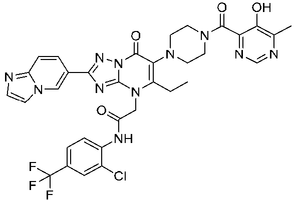

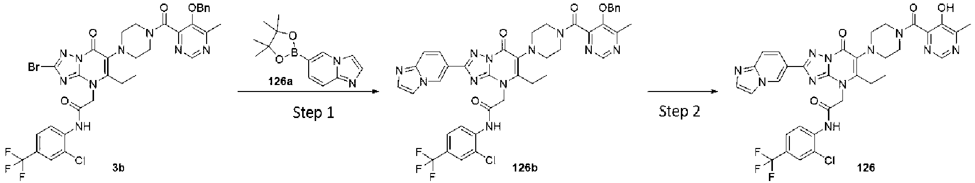

### Step 1

### 2-(6-(4-(5-(benzyloxy)-6-methylpyrimidine-4-carbonyl)piperazin-1-yl)-5-ethyl-2-(imidazo[1,2-a] pyridin-6-yl)-7-oxo-[1,2,4]triazolo[1,5-a]pyrimidin-4(7H)-yl)-N-(2-chloro-4-(trifluoromethyl)phe nyl)acetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (50 mg, 63.37 µmol), 6-(4,4,5,5-tetramethyl-1,3,2-dioxaborolan-2-yl)imidazo[1,2-a]pyridine **126a** (18.56 mg, 76.05 µmol, commercially available), [1,1'-bis(diphenylphosphino)ferrocene]palladium(II) dichloride dichloromethane complex (5.14 mg, 6.34 µmol), and sodium carbonate (20.15 mg, 190.11 µmol) were dissolved in 1,4-dioxane (1 mL) and water (0.3 mL). The mixture was subject to argon replacement four times and heated to 80 °C for 1.5 h. Water (20 mL) was added to the reaction mixture, followed by extraction with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-5-ethyl-2-(imidazo[1,2-a] pyridin-6-yl)-7-oxo-[1,2,4]triazolo[1,5-a]pyrimidin-4(7H)-yl)-N-(2-chloro-4-(trifluoromethyl)phe nyl)acetamide **126b** (36 mg) in a yield of 68.76%.

MS m/z (ESI): 826.2 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-2-(imidazo[1,2-a]pyridin-6-yl)-7-oxo-[1,2,4]triazolo[1,5-a]pyrimidin-4(7H)-yl )acetamide

2-(6-(4-(5-(Benzyloxy)-6-methylpyrimidine-4-carbonyl)piperazin-1-yl)-5-ethyl-2-(imid azo[1,2-a]pyridin-6-yl)-7-oxo-[1,2,4]triazolo[1,5-a]pyrimidin-4(7H)-yl)-N-(2-chloro-4-(trifluoro methyl)phenyl)acetamide **126b** (36 mg, 43.57 µmol) was added to dichloromethane (2.5 mL). Boron trichloride (0.8 mL) was added at 0 °C, and the reaction was performed at 0 °C for 1 h. The reaction was quenched by adding methanol and concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-2-(imidazo[1,2-a]pyridin-6-yl)-7-oxo-[1,2,4] riazolo[1,5-a]pyrimidin-4(7H)-yl)acetamide **126** (14.53 mg) in a yield of 9.88%.

MS m/z (ESI): 736.1 [M+1]
¹H NMR (400 MHz, DMSO) δ 10.43 (s, 1H), 10.24 (s, 1H), 9.65 (s, 1H), 8.59 (s, 1H), 8.50 - 8.40 (m, 2H), 8.19 (d, J = 2.0 Hz, 1H), 8.08 (t, J = 9.4 Hz, 2H), 7.98 (s, 1H), 7.72 (d, J = 8.5 Hz, 1H), 5.42 (s, 2H), 4.55 (d, J = 12.8 Hz, 1H), 3.53 (d, J = 12.1 Hz, 3H), 3.31 (s, 1H), 3.03 (s, 3H), 2.86 (d, J = 11.3 Hz, 1H), 2.69 (d, J = 11.4 Hz, 1H), 2.45 (s, 3H), 1.23 (t, J = 7.5 Hz, 3H).

### Example 127

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(3-oxoisoindolin-5-yl)-[1,2,4]triazolo[1,5-a]pyrimidin-4(7H)-yl)aceta mide

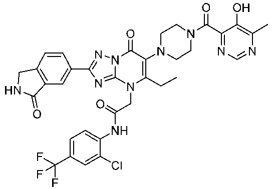

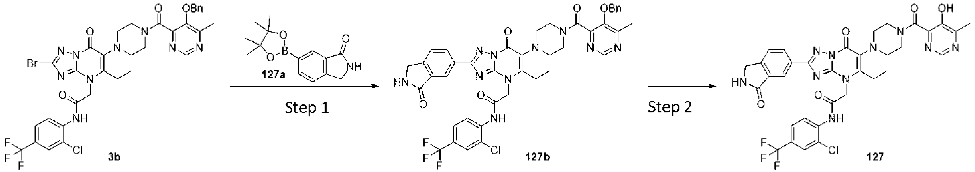

### Step 1

### 2-(6-(4-(5-(benzyloxy)-6-methylpyrimidine-4-carbonyl)piperazin-1-yl)-5-ethyl-7-oxo-2-(3-oxois oindolin-5-yl)-[1,2,4]triazolo[1,5-a]pyrimidin-4(7H)-yl)-N-(2-chloro-4-(trifluoromethyl)phenyl)a cetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (50 mg, 63.37 µmol), 6-(4,4,5,5-tetramethyl-1,3,2-dioxaborolan-2-yl)isoindolin-1-one **127a** (19.70 mg, 76.05 µmol, commercially available), [1,1'-bis(diphenylphosphino)ferrocene]palladium(II) dichloride dichloromethane complex (5.14 mg, 6.34 µmol), and sodium carbonate (20.15 mg, 190.11 µmol) were dissolved in 1,4-dioxane (1 mL) and water (0.3 mL). The mixture was subject to argon replacement four times and heated to 80 °C for 1.5 h. Water (20 mL) was added to the reaction mixture, followed by extraction with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-5-ethyl-7-oxo-2-(3-oxois oindolin-5-yl)-[1,2,4]triazolo[1,5-a]pyrimidin-4(7H)-yl)-N-(2-chloro-4-(trifluoromethyl)phenyl)a cetamide **127b** (37 mg) in a yield of 69.41%.

MS m/z (ESI): 841.1 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(3-oxoisoindolin-5-yl)-[1,2,4]triazolo[1,5-a]pyrimidin-4(7H)-yl)aceta mide

2-(6-(4-(5-(Benzyloxy)-6-methylpyrimidine-4-carbonyl)piperazin-1-yl)-5-ethyl-7-oxo-2 -(3-oxoisoindolin-5-yl)-[1,2,4]triazolo[1,5-a]pyrimidin-4(7H)-yl)-N-(2-chloro-4-(trifluoromethyl) phenyl)acetamide **127b** (37 mg, 43.98 µmol) was added to dichloromethane (1.5 mL). Boron trichloride (0.8 mL) was added at 0 °C, and the reaction was performed at 0 °C for 1 h. The reaction was quenched by adding methanol and concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(3-oxoisoindolin-5-yl)-[1,2,4]triazolo[1,5-a]pyrimidin-4(7H)-yl)aceta mide **127** (11.12 mg) in a yield of 8.60%.

MS m/z (ESI): 751.1 [M+1]
¹H NMR (400 MHz, DMSO) δ 10.44 (s, 1H), 10.24 (s, 1H), 8.69 (s, 1H), 8.59 (s, 1H), 8.37 (s, 1H), 8.34 (dd, J = 7.9, 1.6 Hz, 1H), 8.05 (d, J = 8.5 Hz, 1H), 7.96 (s, 1H), 7.72 (t, J = 9.5 Hz, 2H), 5.43 (s, 2H), 4.54 (d, J = 12.6 Hz, 1H), 4.46 (s, 2H), 3.27 (s, 1H), 3.04 (d, J = 10.4 Hz, 3H), 2.85 (d, J = 11.2 Hz, 1H), 2.45 (s, 3H), 1.22 (t, J = 7.4 Hz, 3H).

### Example 128

### N-(2-chloro-4-(trifluoromethyl)phenyl)-2-(5-ethyl-6-(4-(5-hydroxy-6-methylpyrimidine-4-carbon yl)piperazin-1-yl)-2-(1-methyl-1H-indol-6-yl)-7-oxo-[1,2,4]triazolo[1,5-a]pyrimidin-4(7H)-yl)ac etamide

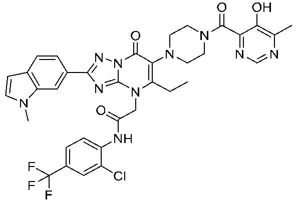

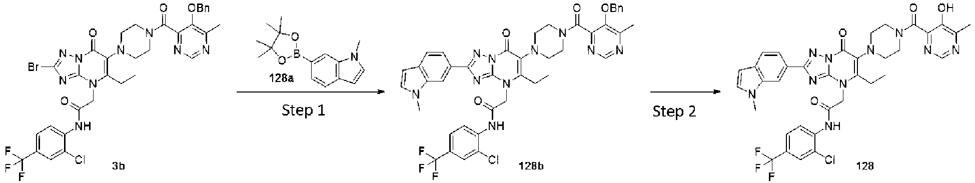

### Step 1

### 2-(6-(4-(5-(benzyloxy)-6-methylpyrimidine-4-carbonyl)piperazin-1-yl)-5-ethyl-2-(1-methyl-1H-i ndol-6-yl)-7-oxo-[1,2,4]triazolo[1,5-a]pyrimidin-4(7H)-yl)-N-(2-chloro-4-(trifluoromethyl)pheny 1)acetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (50 mg, 63.37 µmol), 1-methyl-6-(4,4,5,5-tetramethyl-1,3,2-dioxaborolan-2-yl)-1H-indole **128a** (19.55 mg, 76.05 µmol), [1,1'-bis(diphenylphosphino)ferrocene]palladium(II) dichloride dichloromethane complex (5.14 mg, 6.34 µmol), and sodium carbonate (20.15 mg, 190.11 µmol) were dissolved in 1,4-dioxane (1 mL) and water (0.3 mL). The mixture was subject to argon replacement four times, then heated to 80 °C and reacted for 1.5 hours. Water (20 mL) was added to the reaction mixture, which was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-5-ethyl-2-(1-methyl-1H-i ndol-6-yl)-7-oxo-[1,2,4]triazolo[1,5-a]pyrimidin-4(7H)-yl)-N-(2-chloro-4-(trifluoromethyl)pheny l)acetamide **128b** (33 mg) in a yield of 62.05%.

MS m/z (ESI): 839.2 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-2-(1-methyl-1H-indol-6-yl)-7-oxo-[1,2,4]triazolo[1,5-a]pyrimidin-4(7H)-yl)ac etamide

2-(6-(4-(5-(Benzyloxy)-6-methylpyrimidine-4-carbonyl)piperazin-1-yl)-5-ethyl-2-(1-me thyl-1H-indol-6-yl)-7-oxo-[1,2,4]triazolo[1,5-a]pyrimidin-4(7H)-yl)-N-(2-chloro-4-(trifluoromet hyl)phenyl)acetamide **128b** (33 mg, 39.32 µmol) was added to dichloromethane (2.5 mL). Boron trichloride (0.8 mL) was added at 0 °C, and the reaction was performed at 0 °C for 1 hour. Methanol was added to quench the reaction, and the mixture was concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-2-(1-methyl-1H-indol-6-yl)-7-oxo-[1,2,4]triazolo[1,5-a]pyrimidin-4(7H)-yl)ac etamide **128** (7.25 mg) in a yield of 9.81%.

MS m/z (ESI): 749.2 [M+1]
¹H NMR (400 MHz, DMSO) δ 10.46 (s, 1H), 10.24 (s, 1H), 8.59 (s, 1H), 8.15 (s, 1H), 8.08 (d, J = 8.6 Hz, 1H), 7.98 (d, J = 2.1 Hz, 1H), 7.84 (dd, J = 8.3, 1.4 Hz, 1H), 7.76 - 7.70 (m, 1H), 7.65 (d, J = 8.3 Hz, 1H), 7.47 (d, J = 3.1 Hz, 1H), 6.49 (d, J = 3.1 Hz, 1H), 5.43 (s, 2H), 4.54 (d, J = 12.7 Hz, 1H), 3.86 (s, 3H), 3.27 (t, J = 12.4 Hz, 2H), 3.03 (d, J = 9.4 Hz, 3H), 2.86 (d, J = 11.4 Hz, 1H), 2.69 (s, 2H), 2.45 (s, 3H), 1.23 (q, J = 6.9 Hz, 3H).

### Example 129

### N-(2-chloro-4-(trifluoromethyl)phenyl)-2-(2-(1,3-dihydroisobenzofuran-5-yl)-5-ethyl-6-(4-(5-hy droxy-6-methylpyrimidine-4-carbonyl)piperazin-1-yl)-7-oxo-[1,2,4]triazolo[1,5-a]pyrimidin-4(7 H)-yl)acetamide

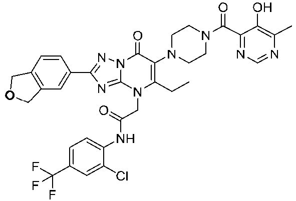

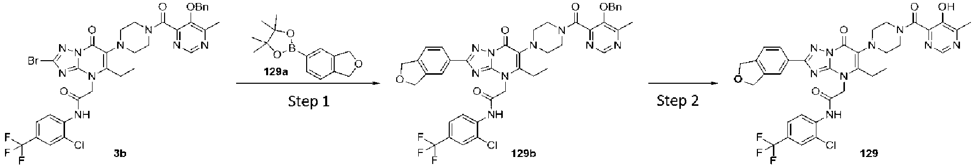

### Step 1

### 2-(6-(4-(5-(benzyloxy)-6-methylpyrimidine-4-carbonyl)piperazin-1-yl)-2-(1,3-dihydroisobenzofu ran-5-yl)-5-ethyl-7-oxo-[1,2,4]triazolo[1,5-a]pyrimidin-4(7H)-yl)-N-(2-chloro-4-(trifluoromethyl )phenyl)acetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide 3b (50 mg, 63.37 µmol), 2-(1,3-dihydroisobenzofuran-5-yl)-4,4,5,5-tetramethyl-1,3,2-dioxaborolane **129a** (18.72 mg, 76.05 µmol, commercially available), [1,1'-bis(diphenylphosphino)ferrocene]palladium(II) dichloride dichloromethane complex (5.14 mg, 6.34 µmol), and sodium carbonate (20.15 mg, 190.11 µmol) were dissolved in 1,4-dioxane (1 mL) and water (0.3 mL). The mixture was subject to argon replacement four times, then heated to 80 °C and reacted for 1.5 hours. Water (20 mL) was added to the reaction mixture, and the mixture was extracted with ethyl acetate (20 mL × 3). The combined organic phase was washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-2-(1,3-dihydroisobenzofu ran-5-yl)-5-ethyl-7-oxo-[1,2,4]triazolo[1,5-a]pyrimidin-4(7H)-yl)-N-(2-chloro-4-(trifluoromethyl )phenyl)acetamide **129b** (30 mg) with a yield of 57.16%.

MS m/z (ESI): 828.2 [M+1]

### Step 2

### N-(2-chloro-4-(trifluoromethyl)phenyl)-2-(2-(1,3-dihydroisobenzofuran-5-yl)-5-ethyl-6-(4-(5-hy droxy-6-methylpyrimidine-4-carbonyl)piperazin-1-yl)-7-oxo-[1,2,4]triazolo[1,5-a]pyrimidin-4(7 H)-yl)acetamide

2-(6-(4-(5-(Benzyloxy)-6-methylpyrimidine-4-carbonyl)piperazin-1-yl)-2-(1,3-dihydroi sobenzofuran-5-yl)-5-ethyl-7-oxo-[1,2,4]triazolo[1,5-a]pyrimidin-4(7H)-yl)-N-(2-chloro-4-(triflu oromethyl)phenyl)acetamide **129b** (30 mg, 36.22 µmol) was added to dichloromethane (2.5 mL). Boron trichloride (0.8 mL) was added at 0 °C, and the reaction was performed at 0 °C for 1 hour. Methanol was added to quench the reaction, and the mixture was concentrated under reduced pressure. The residue was separated by preparative liquid chromatography (separation column: AKZONOBEL Kromasil; 250 ×21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(2-(1,3-dihydroisobenzofuran-5-yl)-5-ethyl-6-(4-(5-hy droxy-6-methylpyrimidine-4-carbonyl)piperazin-1-yl)-7-oxo-[1,2,4]triazolo[1,5-a]pyrimidin-4(7 H)-yl)acetamide **129** (7.31 mg) with a yield of 7.07%.

MS m/z (ESI): 738.2 [M+1]
¹H NMR (400 MHz, Dioxane) δ 11.46 (s, 1H), 11.28 (s, 1H), 9.63 (s, 1H), 9.16 - 9.06 (m, 3H), 9.02 (d, J = 2.1 Hz, 1H), 8.76 (dd, J = 8.6, 2.1 Hz, 1H), 8.50 (d, J = 8.0 Hz, 1H), 6.44 (s, 2H), 6.10 (d, J = 5.0 Hz, 4H), 5.59 (d, J = 12.5 Hz, 1H), 4.58 (d, J = 9.0 Hz, 3H), 4.31 (t, J = 12.6 Hz, 1H), 4.07 (d, J = 10.4 Hz, 3H), 3.89 (d, J = 11.3 Hz, 1H), 3.72 (d, J = 10.2 Hz, 1H), 3.50 (s, 3H), 2.26 (t, J = 7.4 Hz, 3H).

### Example 130

### N-(2-chloro-4-(trifluoromethyl)phenyl)-2-(2-(chroman-6-yl)-5-ethyl-6-(4-(5-hydroxy-6-methylpy rimidine-4-carbonyl)piperazin-1-yl)-7-oxo-[1,2,4]triazolo[1,5-a]pyrimidin-4(7H)-yl)acetamide

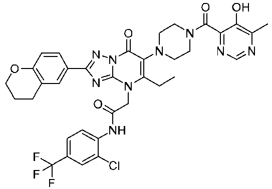

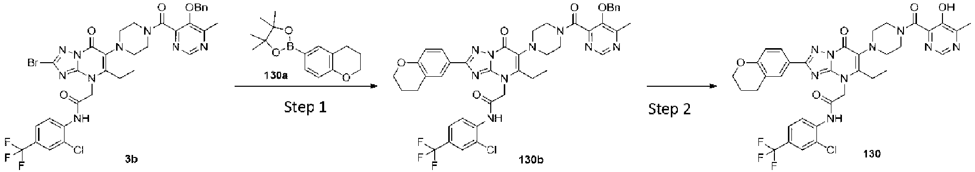

### Step 1

### 2-(6-(4-(5-(benzyloxy)-6-methylpyrimidine-4-carbonyl)piperazin-1-yl)-2-(chroman-6-yl)-5-ethyl -7-oxo-[1,2,4]triazolo[1,5-a]pyrimidin-4(7H)-yl)-N-(2-chloro-4-(trifluoromethyl)phenyl)acetami de

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (50 mg, 63.37 µmol), 2-chroman-6-yl-4,4,5,5-tetramethyl-1,3,2-dioxaborolane **130a** (19.78 mg, 76.05 µmol), [1,1'-bis(diphenylphosphino)ferrocene]palladium(II) dichloride dichloromethane complex (5.14 mg, 6.34 µmol), and sodium carbonate (20.15 mg, 190.11 µmol) were dissolved in 1,4-dioxane (1 mL) and water (0.3 mL). The mixture was subject to argon replacement four times, then heated to 80 °C and reacted for 1.5 hours. Water (20 mL) was added to the reaction mixture, and the mixture was extracted with ethyl acetate (20 mL × 3). The combined organic phase was washed with saturated sodium chloride solution (20 mL), dried over anhydrous sodium sulfate, filtered, and concentrated under reduced pressure. The resulting residue was purified by silica gel column chromatography (eluent: System **B)** to obtain 2-(6-(4-(5-(benzyloxy)-6-methylpyrimidine-4-carbonyl)piperazin-1-yl)-2-(chroman-6-yl)-5-ethyl -7-oxo-[1,2,4]triazolo[1,5-a]pyrimidin-4(7H)-yl)-N-(2-chloro-4-(trifluoromethyl)phenyl)acetami de **130b** (33 mg) with a yield of 61.83%.

MS m/z (ESI): 842.2 [M+1]

### Step 2

### N-(2-chloro-4-(trifluoromethyl)phenyl)-2-(2-(chroman-6-yl)-5-ethyl-6-(4-(5-hydroxy-6-methylpy rimidine-4-carbonyl)piperazin-1-yl)-7-oxo-[1,2,4]triazolo[1,5-a]pyrimidin-4(7H)-yl)acetamide

2-(6-(4-(5-(Benzyloxy)-6-methylpyrimidine-4-carbonyl)piperazin-1-yl)-2-(chroman-6-y l)-5-ethyl-7-oxo-[1,2,4]triazolo[1,5-a]pyrimidin-4(7H)-yl)-N-(2-chloro-4-(trifluoromethyl) phenyl)acetamide **130b** (33 mg, 39.18 µmol) was added to dichloromethane (2.5 mL). Boron trichloride (0.8 mL) was added at 0 °C, and the reaction was performed at 0 °C for 1 hour. Methanol was added to quench the reaction, and the mixture was concentrated under reduced pressure. The residue was separated by preparative liquid chromatography (separation column: AKZONOBEL Kromasil; 250×21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(2-(chroman-6-yl)-5-ethyl-6-(4-(5-hydroxy-6-methylpy rimidine-4-carbonyl)piperazin-1-yl)-7-oxo-[1,2,4]triazolo[1,5-a]pyrimidin -4(7H)-yl)acetamide 130 (6.85 mg) with a yield of 6.92%.

MS m/z (ESI): 752.0 [M+1]
¹H NMR (400 MHz, DMSO) δ 10.41 (s, 1H), 10.24 (s, 1H), 8.58 (s, 1H), 8.06 (d, J = 8.6 Hz, 1H), 7.98 (d, J = 2.1 Hz, 1H), 7.85 - 7.77 (m, 2H), 7.74 - 7.68 (m, 1H), 6.84 (d, J = 8.4 Hz, 1H), 5.38 (s, 2H), 4.53 (d, J = 12.6 Hz, 1H), 4.19 (t, J = 5.1 Hz, 2H), 3.28 (d, J = 13.1 Hz, 2H), 3.00 (s, 3H), 2.81 (d, J = 7.1 Hz, 3H), 2.65 (s, 1H), 2.45 (s, 3H), 1.99 - 1.89 (m, 2H), 1.21 (t, J = 7.5 Hz, 3H).

### Example 131

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(thieno[2,3-b]pyridin-2-yl)-[1,2,4]triazolo[1,5-a]pyrimidin-4(7H)-yl)a cetamide

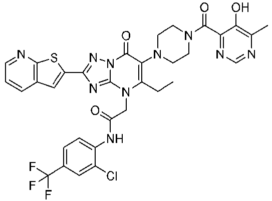

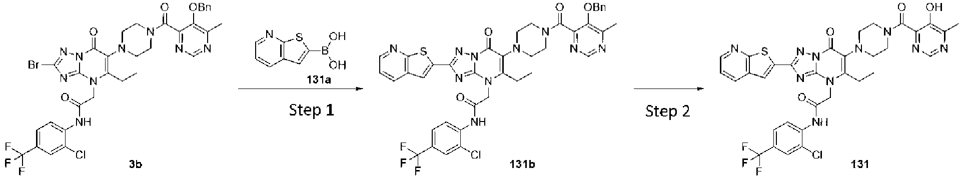

### Step 1

### 2-(6-(4-(5-(benzyloxy)-6-methylpyrimidine-4-carbonyl)piperazin-1-yl)-5-ethyl-7-oxo-2-(thieno[2 ,3-b]pyridin-2-yl)-[1,2,4]triazolo[1,5-a]pyrimidin-4(7H)-yl)-N-(2-chloro-4-(trifluoromethyl)phen yl)acetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide 3b (50 mg, 63.37 µmol), thieno[2,3-b]pyridin-2-ylboronic acid **131a** (13.61 mg, 76.05 µmol, commercially available), [1,1'-bis(diphenylphosphino)ferrocene]palladium(II) dichloride dichloromethane complex (5.14 mg, 6.34 µmol), and sodium carbonate (20.15 mg, 190.11 µmol) were dissolved in 1,4-dioxane (1 mL) and water (0.3 mL). The mixture was subject to argon replacement four times, then heated to 80 °C and reacted for 1.5 hours. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System **B)** to obtain 2-(6-(4-(5-(benzyloxy)-6-methylpyrimidine-4-carbonyl)piperazin-1-yl)-5-ethyl-7-oxo-2-(thieno[2 ,3-b]pyridin-2-yl)-[1,2,4]triazolo[1,5-a]pyrimidin-4(7H)-yl)-N-(2-chloro-4-(trifluoromethyl)phen yl)acetamide **131b** (36 mg) in a yield of 67.37%.

MS m/z (ESI): 843.2 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(thieno[2,3-b]pyridin-2-yl)-[1,2,4]triazolo[1,5-a]pyrimidin-4(7H)-yl)a cetamide

2-(6-(4-(5-(Benzyloxy)-6-methylpyrimidine-4-carbonyl)piperazin-1-yl)-5-ethyl-7-oxo-2 -(thieno[2,3-b]pyridin-2-yl)-[1,2,4]triazolo[1,5-a]pyrimidin-4(7H)-yl)-N-(2-chloro-4-(trifluorome thyl)phenyl)acetamide **131b** (36 mg, 42.69 µmol) was added to dichloromethane (1 mL), and trifluoroacetic acid (3 mL) was added. The mixture was reacted at 40 °C for 3 hours. The mixture was concentrated under reduced pressure, and then separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(thieno[2,3-b]pyridin-2-yl)-[1,2,4]triazolo[1,5-a]pyrimidin-4(7H)-yl)a cetamide **131** (6.6 mg) in a yield of 8.72%.

MS m/z (ESI): 753.0 [M+1]
¹H NMR (400 MHz, DMSO) δ 10.41 (s, 1H), 10.24 (s, 1H), 8.63 (dd, J = 4.6, 1.6 Hz, 1H), 8.59 (s, 1H), 8.37 (dd, J = 8.1, 1.7 Hz, 1H), 8.13 (s, 1H), 8.07 (d, J = 8.6 Hz, 1H), 7.97 (d, J = 2.1 Hz, 1H), 7.72 (dd, J = 8.6, 2.2 Hz, 1H), 7.51 (dd, J = 8.1, 4.6 Hz, 1H), 5.40 (s, 2H), 4.55 (d, J = 12.6 Hz, 1H), 3.53 (d, J = 11.4 Hz, 3H), 3.29 (d, J = 12.1 Hz, 1H), 3.04 (d, J = 9.5 Hz, 3H), 2.86 (d, J = 11.2 Hz, 1H), 2.68 (d, J = 10.6 Hz, 1H), 2.45 (s, 3H), 1.22 (t, J = 7.4 Hz, 3H).

### Example 132

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(thieno[2,3-c]pyridin-2-yl)-[1,2,4]triazolo[1,5-a]pyrimidin-4(7H)-yl)a cetamide

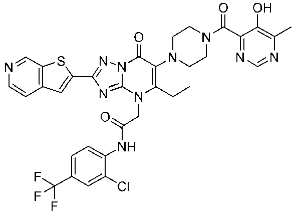

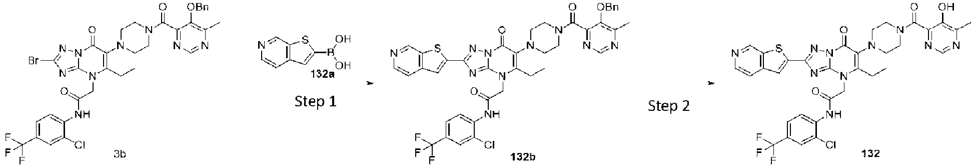

### Step 1

### 2-(6-(4-(5-(benzyloxy)-6-methylpyrimidine-4-carbonyl)piperazin-1-yl)-5-ethyl-7-oxo-2-(thieno[2 ,3-c]pyridin-2-yl)-[1,2,4]triazolo[1,5-a]pyrimidin-4(7H)-yl)-N-(2-chloro-4-(trifluoromethyl)phen yl)acetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (50 mg, 63.37 µmol), thieno[2,3-c]pyridin-2-ylboronic acid **132a** (13.61 mg, 76.05 µmol), [1,1'-bis(diphenylphosphino)ferrocene]palladium(II) dichloride dichloromethane complex (5.14 mg, 6.34 µmol), and sodium carbonate (20.15 mg, 190.11 µmol) were dissolved in 1,4-dioxane (1 mL) and water (0.3 mL). The mixture was subject to argon replacement four times, then heated to 80 °C and reacted for 1.5 hours. Water (20 mL) was added to the reaction mixture, followed by extraction with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System **B)** to obtain 2-(6-(4-(5-(benzyloxy)-6-methylpyrimidine-4-carbonyl)piperazin-1-yl)-5-ethyl-7-oxo-2-(thieno[2 ,3-c]pyridin-2-yl)-[1,2,4]triazolo[1,5-a]pyrimidin-4(7H)-yl)-N-(2-chloro-4-(trifluoromethyl)phen yl)acetamide **132b** (33 mg) in a yield of 61.75%.

MS m/z (ESI): 843.2 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(thieno[2,3-c]pyridin-2-yl)-[1,2,4]triazolo[1,5-a]pyrimidin-4(7H)-yl)a cetamide

2-(6-(4-(5-(benzyloxy)-6-methylpyrimidine-4-carbonyl)piperazin-1-yl)-5-ethyl-7-oxo-2 -(thieno[2,3-c]pyridin-2-yl)-[1,2,4]triazolo[1,5-a]pyrimidin-4(7H)-yl)-N-(2-chloro-4-(trifluorome thyl)phenyl)acetamide **132b** (33 mg, 39.13 µmol) was added to dichloromethane (1 mL), followed by addition of trifluoroacetic acid (3 mL). The reaction was performed at 40 °C for 3 hours. The reaction was quenched by addition of methanol and concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(thieno[2,3-c]pyridin-2-yl)-[1,2,4]triazolo[1,5-a]pyrimidin-4(7H)-yl)a cetamide 132 (12.81 mg) in a yield of 8.80%. MS m/z (ESI): 753.0 [M+1]
¹H NMR (400 MHz, DMSO) δ 10.43 (s, 1H), 10.24 (s, 1H), 9.49 (s, 1H), 8.65 - 8.55 (m, 2H), 8.30 (s, 1H), 8.10 (dd, J = 22.6, 7.2 Hz, 2H), 7.98 (d, J = 2.1 Hz, 1H), 7.72 (d, J = 8.9 Hz, 1H), 5.41 (s, 2H), 4.55 (d, J = 12.6 Hz, 1H), 3.53 (d, J = 11.7 Hz, 4H), 3.31 (s, 1H), 3.05 (d, J = 9.7 Hz, 3H), 2.86 (d, J = 11.4 Hz, 1H), 2.70 (s, 1H), 2.45 (s, 3H), 1.22 (t, J = 7.4 Hz, 3H).

### Example 133

### 2-(2-(benzo[d]thiazol-2-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)aceta mide

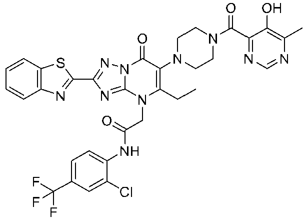

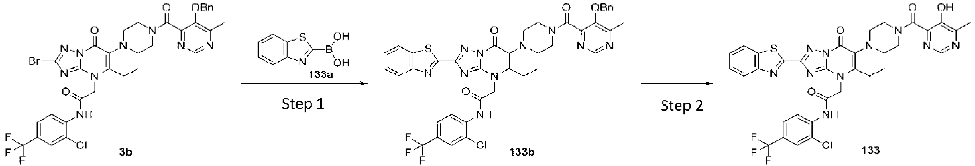

### Step 1

### 2-(2-(benzo[d]thiazol-2-yl)-6-(4-(5-(benzyloxy)-6-methylpyrimidine-4-carbonyl)piperazin-1-yl)-5-ethyl-7-oxo-[1,2,4]triazolo[1,5-a]pyrimidin-4(7H)-yl)-N-(2-chloro-4-(trifluoromethyl)phenyl)a cetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide 3b (50 mg, 63.37 µmol), 1,3-benzothiazol-2-ylboronic acid **133a** (13.61 mg, 76.05 µmol, commercially available), [1,1'-bis(diphenylphosphino)ferrocene]palladium(II) dichloride dichloromethane complex (5.14 mg, 6.34 µmol), and sodium carbonate (20.15 mg, 190.11 µmol) were dissolved in 1,4-dioxane (1 mL) and water (0.3 mL). The mixture was subject to argon replacement four times, then heated to 80 °C and reacted for 1.5 hours. Water (20 mL) was added to the reaction mixture, followed by extraction with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System **B)** to obtain 2-(2-(benzo[d]thiazol-2-yl)-6-(4-(5-(benzyloxy)-6-methylpyrimidine-4-carbonyl)piperazin-1-yl)-5-ethyl-7-oxo-[1,2,4]triazolo[1,5-a]pyrimidin-4(7H)-yl)-N-(2-chloro-4-(trifluoromethyl)phenyl)a cetamide **133b** (29 mg) in a yield of 54.27%.

MS m/z (ESI): 843.2 [M+1]

### Step 2

### 2-(2-(benzo[d]thiazol-2-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)aceta mide

2-(2-(Benzo[d]thiazol-2-yl)-6-(4-(5-(benzyloxy)-6-methylpyrimidine-4-carbonyl) piperazin-1-yl)-5-ethyl-7-oxo-[1,2,4]triazolo[1,5-a]pyrimidin-4(7H)-yl)-N-(2-chloro-4-(trifluoro methyl)phenyl)acetamide **133b** (29 mg, 34.39 µmol) was added to dichloromethane (2.5 mL), and boron trichloride (0.8 mL) was added at 0 °C. The reaction was performed at 0 °C for 1 hour. The reaction was quenched by addition of methanol and concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain 2-(2-(benzo[d]thiazol-2-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)aceta mide **133** (1.74 mg) in a yield of 6.58%.

MS m/z (ESI): 752.9 [M+1]
¹H NMR (400 MHz, DMSO) δ 10.49 (s, 1H), 10.24 (s, 1H), 9.52 (s, 1H), 8.59 (s, 1H), 8.34 (d, J = 7.5 Hz, 1H), 8.27 (dd, J = 8.1, 1.1 Hz, 1H), 8.06 (d, J = 8.5 Hz, 1H), 7.98 (d, J = 2.1 Hz, 1H), 7.78 - 7.69 (m, 2H), 5.45 (s, 2H), 4.55 (d, J = 12.4 Hz, 1H), 3.55 (q, J = 11.9 Hz, 3H), 3.04 (dd, J = 21.2, 10.6 Hz, 3H), 2.87 (d, J = 11.7 Hz, 1H), 2.71 (s, 1H), 2.45 (s, 3H), 1.23 (t, J = 7.4 Hz, 3H).

### Example 134

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(4,5,6,7-tetrahydrothieno[3,2-c]pyridin-2-yl)-[1,2,4]triazolo[1,5-a]pyri midin-4(7H)-yl)acetamide

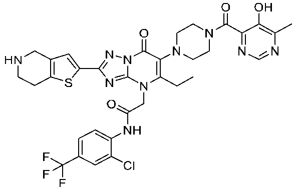

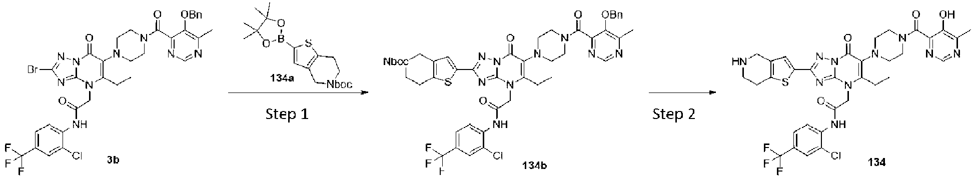

### Step 1

### tert-butyl 2-(6-(4-(5-(benzyloxy)-6-methylpyrimidine-4-carbonyl)piperazin-1-yl)-4-(2-((2-chloro-4-(trifluo romethyl)phenyl)amino)-2-oxoethyl)-5-ethyl-7-oxo-4,7-dihydro-[1,2,4]triazolo[1,5-a]pyrimidin-2-yl)-6,7-dihydrothieno[3,2-c]pyridine-5(4H)-carboxylate

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (50 mg, 63.37 µmol), tert-butyl 2-(4,4,5,5-tetramethyl-1,3,2-dioxaborolan-2-yl)-6,7-dihydro-4H-thieno[3,2-c]pyridine-5-carboxyl ate **134a** (27.78 mg, 76.05 µmol, commercially available), [1,1'-bis(diphenylphosphino)ferrocene]palladium(II) dichloride dichloromethane complex (5.14 mg, 6.34 µmol), and sodium carbonate (20.15 mg, 190.11 µmol) were dissolved in 1,4-dioxane (1 mL) and water (0.3 mL). The mixture was subject to argon replacement four times, then heated to 80 °C and reacted for 1.5 hours. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System **B)** to obtain tert-butyl 2-(6-(4-(5-(benzyloxy)-6-methylpyrimidine-4-carbonyl)piperazin-1-yl)-4-(2-((2-chloro-4-(trifluo romethyl)phenyl)amino)-2-oxoethyl)-5-ethyl-7-oxo-4,7-dihydro-[1,2,4]triazolo[1,5-a]pyrimidin-2-yl)-6,7-dihydrothieno[3,2-c]pyridine-5(4H)-carboxylate **134b** (36 mg) in a yield of 59.96%.

MS m/z (ESI): 947.3 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(4,5,6,7-tetrahydrothieno[3,2-c]pyridin-2-yl)-[1,2,4]triazolo[1,5-a]pyri midin-4(7H)-yl)acetamide

Tert-butyl 2-(6-(4-(5-(benzyloxy)-6-methylpyrimidine-4-carbonyl)piperazin-1-yl)-4-(2-((2-chloro-4-(trifluo romethyl)phenyl)amino)-2-oxoethyl)-5-ethyl-7-oxo-4,7-dihydro-[1,2,4] triazolo[1,5-a]pyrimidin-2-yl)-6,7-dihydrothieno[3,2-c]pyridine-5(4H)-carboxylate **134b** (36 mg, 38.00 µmol) was added to dichloromethane (1 mL). Trifluoroacetic acid (3 mL) was added, and the mixture was reacted at 40 °C for 3 hours. Methanol was added to quench the reaction, and the mixture was concentrated under reduced pressure. The residue was separated by preparative liquid chromatography (separation column: AKZONOBEL Kromasil; 250×21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(4,5,6,7-tetrahydrothieno[3,2-c]pyridin-2-yl)-[1,2,4]triazolo[1,5-a]pyri midin-4(7H)-yl)acetamide **134** (11.25 mg) in a yield of 6.55%.

MS m/z (ESI): 757.2 [M+1]
¹H NMR (400 MHz, DMSO) δ 10.38 (s, 1H), 10.23 (s, 1H), 9.02 (s, 2H), 8.58 (s, 1H), 8.06 (d, J = 8.6 Hz, 1H), 7.97 (d, J = 2.1 Hz, 1H), 7.72 (dd, J = 8.7, 2.2 Hz, 1H), 7.61 (s, 1H), 5.34 (s, 2H), 4.53 (d, J = 12.5 Hz, 1H), 4.24 (s, 2H), 3.28 (d, J = 12.1 Hz, 2H), 3.09 (s, 2H), 3.01 (s, 3H), 2.83 (d, J = 11.3 Hz, 1H), 2.64 (s, 1H), 2.45 (s, 3H), 1.20 (t, J = 7.4 Hz, 3H).

### Example 135

### 2-(2-([1,2,4]triazolo[1,5-a]pyridin-6-yl)-5-ethyl-6-(4-(5-hydroxy-6-methylpyrimidine-4-carbonyl )piperazin-1-yl)-7-oxo-[1,2,4]triazolo[1,5-a]pyrimidin-4(7H)-yl)-N-(2-chloro-4-(trifluoromethyl) phenyl)acetamide

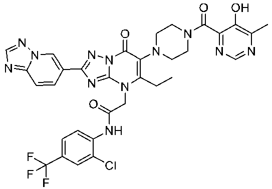

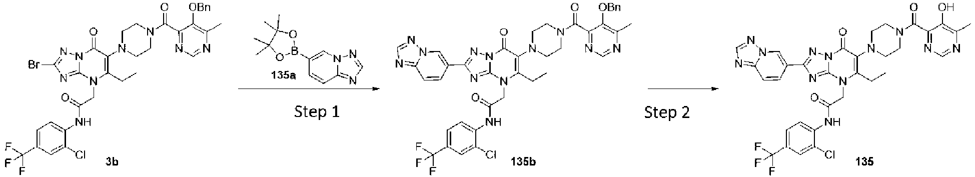

### Step 1

### 2-(2-([1,2,4]triazolo[1,5-a]pyridin-6-yl)-6-(4-(5-(benzyloxy)-6-methylpyrimidine-4-carbonyl)pip erazin-1-yl)-5-ethyl-7-oxo-[1,2,4]triazolo[1,5-a]pyrimidin-4(7H)-yl)-N-(2-chloro-4-(trifluoromet hyl)phenyl)acetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide 3b (50 mg, 63.37 µmol), 6-(4,4,5,5-tetramethyl-1,3,2-dioxaborolan-2-yl)-[1,2,4]triazolo[1,5-a]pyridine 135a (18.64 mg, 76.05 µmol, commercially available), [1,1'-bis(diphenylphosphino)ferrocene]palladium(II) dichloride dichloromethane complex (5.14 mg, 6.34 µmol), and sodium carbonate (20.15 mg, 190.11 µmol) were dissolved in 1,4-dioxane (1 mL) and water (0.3 mL). The mixture was subject to argon replacement four times, then heated to 80 °C and reacted for 1.5 hours. Water (20 mL) was added to the reaction mixture, which was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System **B)** to obtain 2-(2-([1,2,4]triazolo[1,5-a]pyridin-6-yl)-6-(4-(5-(benzyloxy)-6-methylpyrimidine-4-carbonyl)pip erazin-1-yl)-5-ethyl-7-oxo-[1,2,4]triazolo[1,5-a]pyrimidin-4(7H)-yl)-N-(2-chloro-4-(trifluoromet hyl)phenyl)acetamide **135b** (33 mg) in a yield of 62.95%.

MS m/z (ESI): 827.0 [M+1]

### Step 2

### 2-(2-([1,2,4]triazolo[1,5-a]pyridin-6-yl)-5-ethyl-6-(4-(5-hydroxy-6-methylpyrimidine-4-carbonyl )piperazin-1-yl)-7-oxo-[1,2,4]triazolo[1,5-a]pyrimidin-4(7H)-yl)-N-(2-chloro-4-(trifluoromethyl) phenyl)acetamide

2-(2-([1,2,4]Triazolo[1,5-a]pyridin-6-yl)-6-(4-(5-(benzyloxy)-6-methylpyrimidine-4-car bonyl)piperazin-1-yl)-5-ethyl-7-oxo-[1,2,4]triazolo[1,5-a]pyrimidin-4(7H)-yl)-N-(2-chloro-4-(trif luoromethyl)phenyl)acetamide **135b** (33 mg, 39.89 µmol) was added to dichloromethane (1 mL), followed by addition of trifluoroacetic acid (3 mL). The mixture was reacted at 40 °C for 3 hours. Methanol was added to quench the reaction, and the mixture was concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain 2-(2-([1,2,4]triazolo[1,5-a]pyridin-6-yl)-5-ethyl-6-(4-(5-hydroxy-6-methylpyrimidine-4-carbonyl )piperazin-1-yl)-7-oxo-[1,2,4]triazolo[1,5-a]pyrimidin-4(7H)-yl)-N-(2-chloro-4-(trifluoromethyl) phenyl)acetamide **135** (10.3 mg) in a yield of 7.98%.

MS m/z (ESI): 737.2 [M+1]
¹H NMR (400 MHz, DMSO) δ 10.46 (s, 1H), 10.25 (s, 1H), 9.45 (s, 1H), 8.61 (d, J = 15.7 Hz, 2H), 8.29 (dd, J = 9.2, 1.7 Hz, 1H), 8.09 - 7.97 (m, 3H), 7.72 (dd, J = 8.8, 2.1 Hz, 1H), 5.43 (s, 2H), 4.55 (d, J = 12.6 Hz, 1H), 3.53 (d, J = 11.7 Hz, 3H), 3.26 (d, J = 12.3 Hz, 1H), 3.03 (dt, J = 16.5, 6.4 Hz, 3H), 2.86 (d, J = 11.3 Hz, 1H), 2.68 (d, J = 10.3 Hz, 1H), 2.45 (s, 3H), 1.23 (t, J = 7.4 Hz, 3H).

### Example 136

### N-(2-chloro-4-(trifluoromethyl)phenyl)-2-(5-ethyl-6-(4-(5-hydroxy-6-methylpyrimidine-4-carbon yl)piperazin-1-yl)-2-(imidazo[1,2-a]pyridin-7-yl)-7-oxo-[1,2,4]triazolo[1,5-a]pyrimidin-4(7H)-yl )acetamide

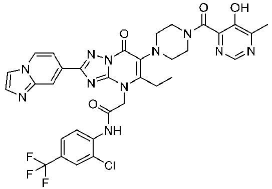

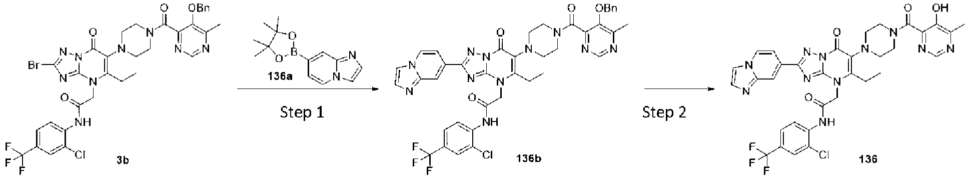

### Step 1

### 2-(6-(4-(5-(benzyloxy)-6-methylpyrimidine-4-carbonyl)piperazin-1-yl)-5-ethyl-2-(imidazo[1,2-a] pyridin-7-yl)-7-oxo-[1,2,4]triazolo[1,5-a]pyrimidin-4(7H)-yl)-N-(2-chloro-4-(trifluoromethyl)phe nyl)acetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide 3b (50 mg, 63.37 µmol), 7-(4,4,5,5-tetramethyl-1,3,2-dioxaborolan-2-yl)imidazo[1,2-a]pyridine **136a** (15.47 mg, 63.37 µmol, commercially available), [1,1'-bis(diphenylphosphino)ferrocene]palladium(II) dichloride dichloromethane complex (5.14 mg, 6.34 µmol), and sodium carbonate (20.15 mg, 190.11 µmol) were dissolved in 1,4-dioxane (1 mL) and water (0.3 mL). The mixture was subject to argon replacement four times, then heated to 80 °C and reacted for 1.5 hours. Water (20 mL) was added to the reaction mixture, which was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System **B)** to obtain 2-(6-(4-(5-(benzyloxy)-6-methylpyrimidine-4-carbonyl)piperazin-1-yl)-5-ethyl-2-(imidazo[1,2-a] pyridin-7-yl)-7-oxo-[1,2,4]triazolo[1,5-a]pyrimidin-4(7H)-yl)-N-(2-chloro-4-(trifluoromethyl)phe nyl)acetamide **136b** (29 mg) in a yield of 55.39%.

MS m/z (ESI): 826.2 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-2-(imidazo[1,2-a]pyridin-7-yl)-7-oxo-[1,2,4]triazolo[1,5-a]pyrimidin-4(7H)-yl )acetamide

2-(6-(4-(5-(Benzyloxy)-6-methylpyrimidine-4-carbonyl)piperazin-1-yl)-5-ethyl-2-(imid azo[1,2-a]pyridin-7-yl)-7-oxo-[1,2,4]triazolo[1,5-a]pyrimidin-4(7H)-yl)-N-(2-chloro-4-(trifluoro methyl)phenyl)acetamide **136b** (29 mg, 35.10 µmol) was added to dichloromethane (1 mL), followed by addition of trifluoroacetic acid (3 mL). The mixture was reacted at 40 °C for 3 hours. Methanol was added to quench the reaction, and the mixture was concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-2-(imidazo[1,2-a]pyridin-7-yl)-7-oxo-[1,2,4]triazolo[1,5-a]pyrimidin-4(7H)-yl )acetamide **136** (6.11 mg) in a yield of 11.92%.

MS m/z (ESI): 736.1 [M+1]
¹H NMR (400 MHz, DMSO) δ 10.44 (s, 1H), 10.23 (s, 1H), 8.97 (d, J = 7.1 Hz, 1H), 8.59 (s, 1H), 8.41 (d, J = 13.8 Hz, 2H), 8.19 (s, 1H), 8.09 - 7.94 (m, 3H), 7.75 - 7.68 (m, 1H), 5.44 (s, 2H), 4.55 (d, J = 12.4 Hz, 1H), 3.30 (d, J = 12.1 Hz, 2H), 3.05 (t, J = 8.3 Hz, 3H), 2.86 (d, J = 11.4 Hz, 1H), 2.70 (s, 1H), 2.45 (s, 3H), 1.23 (t, J = 7.5 Hz, 3H).

### Example 137

### 2-(2-([1,2,4]triazolo[1,5-a]pyridin-7-yl)-5-ethyl-6-(4-(5-hydroxy-6-methylpyrimidine-4-carbonyl )piperazin-1-yl)-7-oxo-[1,2,4]triazolo[1,5-a]pyrimidin-4(7H)-yl)-N-(2-chloro-4-(trifluoromethyl) phenyl)acetamide

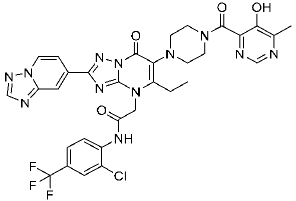

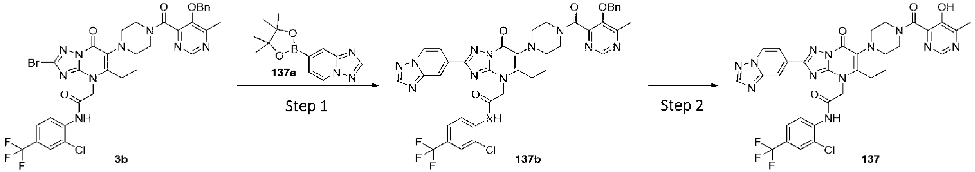

### Step 1

### 2-(2-([1,2,4]triazolo[1,5-a]pyridin-7-yl)-6-(4-(5-(benzyloxy)-6-methylpyrimidine-4-carbonyl)pip erazin-1-yl)-5-ethyl-7-oxo-[1,2,4]triazolo[1,5-a]pyrimidin-4(7H)-yl)-N-(2-chloro-4-(trifluoromet hyl)phenyl)acetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide 3b (50 mg, 63.37 µmol), 7-(4,4,5,5-tetramethyl-1,3,2-dioxaborolan-2-yl)-[1,2,4]triazolo[1,5-a]pyridine 137a (18.64 mg, 76.05 µmol, commercially available), [1,1'-bis(diphenylphosphino)ferrocene]palladium(II) dichloride dichloromethane complex (5.14 mg, 6.34 µmol), and sodium carbonate (20.15 mg, 190.11 µmol) were dissolved in 1,4-dioxane (1 mL) and water (0.3 mL). The mixture was subject to argon replacement four times, then heated to 80 °C and reacted for 1.5 hours. Water (20 mL) was added to the reaction mixture, and the mixture was extracted with ethyl acetate (20 mL × 3). The combined organic phase was washed with saturated sodium chloride solution (20 mL), dried over anhydrous sodium sulfate, filtered, and concentrated under reduced pressure. The resulting residue was purified by silica gel column chromatography (eluent: System B) to obtain 2-(2-([1,2,4]triazolo[1,5-a]pyridin-7-yl)-6-(4-(5-(benzyloxy)-6-methylpyrimidine-4-carbonyl)pip erazin-1-yl)-5-ethyl-7-oxo-[1,2,4]triazolo [1,5-a]pyrimidin-4(7H)-yl)-N-(2-chloro-4-(trifluoromethyl)phenyl)acetamide **137b** (33 mg) in a yield of 62.95%.

MS m/z (ESI): 827.3 [M+1]

### Step 2

### 2-(2-([1,2,4]triazolo[1,5-a]pyridin-7-yl)-5-ethyl-6-(4-(5-hydroxy-6-methylpyrimidine-4-carbonyl )piperazin-1-yl)-7-oxo-[1,2,4]triazolo[1,5-a]pyrimidin-4(7H)-yl)-N-(2-chloro-4-(trifluoromethyl) phenyl)acetamide

2-(2-([1,2,4]Triazolo[1,5-a]pyridin-7-yl)-6-(4-(5-(benzyloxy)-6-methylpyrimidine-4-car bonyl)piperazin-1-yl)-5-ethyl-7-oxo-[1,2,4]triazolo[1,5-a]pyrimidin-4(7H)-yl)-N-(2-chloro-4-(trif luoromethyl)phenyl)acetamide **137b** (33 mg, 39.89 µmol) was added to dichloromethane (1 mL), followed by addition of trifluoroacetic acid (3 mL). The reaction was performed at 40 °C for 3 hours. Methanol was added to quench the reaction, and the mixture was concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain 2-(2-([1,2,4]triazolo[1,5-a]pyridin-7-yl)-5-ethyl-6-(4-(5-hydroxy-6-methylpyrimidine-4-carbonyl )piperazin-1-yl)-7-oxo-[1,2,4]triazolo[1,5-a]pyrimidin-4(7H)-yl)-N-(2-chloro-4-(trifluoromethyl) phenyl)acetamide **137** (17.54 mg) in a yield of 8.18%.

MS m/z (ESI): 737.2 [M+1]
¹H NMR (400 MHz, DMSO) δ 10.45 (s, 1H), 10.24 (s, 1H), 9.09 (d, J = 7.1 Hz, 1H), 8.60 (d, J = 9.7 Hz, 2H), 8.43 (t, J = 1.3 Hz, 1H), 8.06 (d, J = 8.6 Hz, 1H), 7.98 (d, J = 2.1 Hz, 1H), 7.83 (dd, J = 7.1, 1.7 Hz, 1H), 7.71 (dd, J = 8.9, 2.1 Hz, 1H), 5.44 (s, 2H), 4.55 (d, J = 12.6 Hz, 1H), 3.28 (t, J = 12.7 Hz, 1H), 3.03 (dd, J = 18.8, 9.6 Hz, 3H), 2.86 (d, J = 11.0 Hz, 1H), 2.70 (s, 1H), 2.45 (s, 3H), 1.23 (t, J = 7.5 Hz, 3H).

### Example 138

### 2-(2-(1H-benzo[d]imidazol-6-yl)-5-ethyl-6-(4-(5-hydroxy-6-methylpyrimidine-4-carbonyl)pipera zin-1-yl)-7-oxo-[1,2,4]triazolo[1,5-a]pyrimidin-4(7H)-yl)-N-(2-chloro-4-(trifluoromethyl)phenyl) acetamide

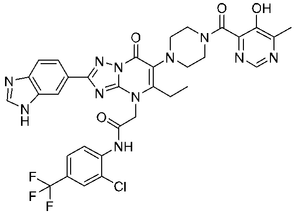

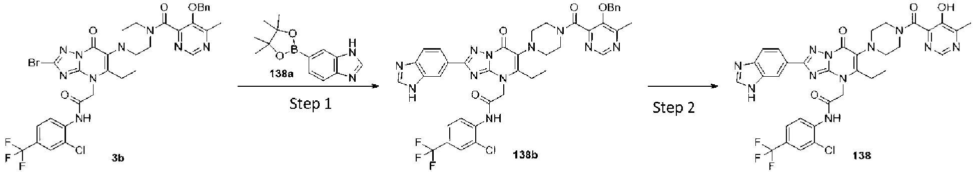

### Step 1

### 2-(2-(1H-benzo[d]imidazol-6-yl)-6-(4-(5-(benzyloxy)-6-methylpyrimidine-4-carbonyl)piperazin-1-yl)-5-ethyl-7-oxo-[1,2,4]triazolo[1,5-a]pyrimidin-4(7H)-yl)-N-(2-chloro-4-(trifluoromethyl)phe nyl)acetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (50 mg, 63.37 µmol), 6-(4,4,5,5-tetramethyl-1,3,2-dioxaborolan-2-yl)-1H-benzo[d]imidazole **138a** (18.56 mg, 76.05 µmol, commercially available), [1,1'-bis(diphenylphosphino)ferrocene]palladium(II) dichloride dichloromethane complex (5.14 mg, 6.34 µmol), and sodium carbonate (20.15 mg, 190.11 µmol) were dissolved in 1,4-dioxane (1 mL) and water (0.3 mL). The mixture was subject to argon replacement four times, then heated to 80 °C and reacted for 1.5 hours. Water (20 mL) was added to the reaction mixture, followed by extraction with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System **B)** to obtain 2-(2-(1H-benzo[d]imidazol-6-yl)-6-(4-(5-(benzyloxy)-6-methylpyrimidine-4-carbonyl)piperazin-1-yl)-5-ethyl-7-oxo-[1,2,4]triazolo[1,5-a]pyrimidin-4(7H)-yl)-N-(2-chloro-4-(trifluoromethyl)phe nyl)acetamide **138b** (30 mg) in a yield of 57.30%.

MS m/z (ESI): 826.1 [M+1]

### Step 2

### 2-(2-(1H-benzo[d]imidazol-6-yl)-5-ethyl-6-(4-(5-hydroxy-6-methylpyrimidine-4-carbonyl)pipera zin-1-yl)-7-oxo-[1,2,4]triazolo[1,5-a]pyrimidin-4(7H)-yl)-N-(2-chloro-4-(trifluoromethyl)phenyl) acetamide

2-(2-(1H-benzo[d]imidazol-6-yl)-6-(4-(5-(benzyloxy)-6-methylpyrimidine-4-carbonyl) piperazin-1-yl)-5-ethyl-7-oxo-[1,2,4]triazolo[1,5-a]pyrimidin-4(7H)-yl)-N-(2-chloro-4-(trifluoro methyl)phenyl)acetamide **138b** (30 mg, 36.31 µmol) was added to dichloromethane (1 mL), followed by addition of trifluoroacetic acid (3 mL). The mixture was reacted at 40 °C for 3 hours. Methanol was added to quench the reaction, and the mixture was concentrated under reduced pressure, then separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain 2-(2-(1H-benzo[d]imidazol-6-yl)-5-ethyl-6-(4-(5-hydroxy-6-methylpyrimidine-4-carbonyl)pipera zin-1-yl)-7-oxo-[1,2,4]triazolo[1,5-a]pyrimidin-4(7H)-yl)-N-(2-chloro-4-(trifluoromethyl)phenyl) acetamide **138** (2.07 mg) in a yield of 7.36%.

MS m/z (ESI): 736.2 [M+1]
¹H NMR (400 MHz, DMSO) δ 10.44 (s, 1H), 10.24 (s, 1H), 9.06 (s, 1H), 8.59 (s, 1H), 8.42 (s, 1H), 8.19 (d, J = 8.7 Hz, 1H), 8.07 (d, J = 8.6 Hz, 1H), 7.98 (d, J = 2.1 Hz, 1H), 7.87 (d, J = 8.5 Hz, 1H), 7.74 - 7.68 (m, 1H), 5.42 (s, 2H), 4.55 (d, J = 12.4 Hz, 1H), 3.29 (d, J = 12.5 Hz, 2H), 3.04 (d, J = 10.8 Hz, 3H), 2.86 (d, J = 11.2 Hz, 1H), 2.69 (s, 1H), 2.45 (s, 3H), 1.22 (t, J = 7.5 Hz, 3H).

### Example 139

### N-(2-chloro-4-(trifluoromethyl)phenyl)-2-(5-ethyl-6-(4-(5-hydroxy-6-methylpyrimidine-4-carbon yl)piperazin-1-yl)-2-(1-methyl-1H-indazol-6-yl)-7-oxo-[1,2,4]triazolo[1,5-a]pyrimidin-4(7H)-yl) acetamide

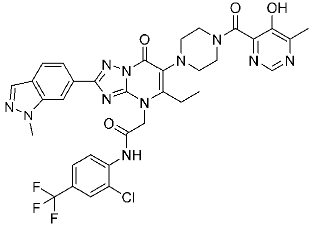

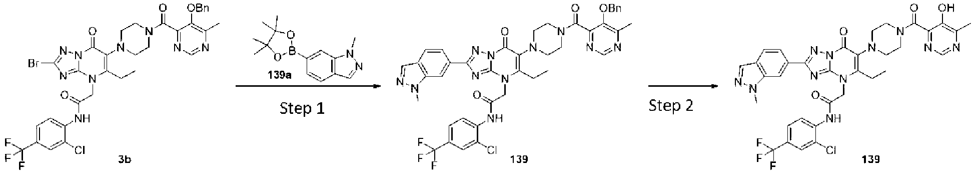

### Step 1

### 2-(6-(4-(5-(benzyloxy)-6-methylpyrimidine-4-carbonyl)piperazin-1-yl)-5-ethyl-2-(1-methyl-1H-i ndazol-6-yl)-7-oxo-[1,2,4]triazolo[1,5-a]pyrimidin-4(7H)-yl)-N-(2-chloro-4-(trifluoromethyl)phe nyl)acetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide 3b (50 mg, 63.37 µmol), 1-methyl-6-(4,4,5,5-tetramethyl-1,3,2-dioxaborolan-2-yl)-1H-indazole 139a (19.63 mg, 76.05 µmol), [1,1'-bis(diphenylphosphino)ferrocene]palladium(II) dichloride dichloromethane complex (5.14 mg, 6.34 µmol), and sodium carbonate (20.15 mg, 190.11 µmol) were dissolved in 1,4-dioxane (1 mL) and water (0.3 mL). The mixture was subject to argon replacement four times, then heated to 80 °C and reacted for 1.5 hours. Water (20 mL) was added to the reaction mixture, followed by extraction with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System **B)** to obtain 2-(6-(4-(5-(benzyloxy)-6-methylpyrimidine-4-carbonyl)piperazin-1-yl)-5-ethyl-2-(1-methyl-1H-i ndazol-6-yl)-7-oxo-[1,2,4]triazolo[1,5-a]pyrimidin-4(7H)-yl)-N-(2-chloro-4-(trifluoromethyl)phe nyl)acetamide **139b** (39 mg) in a yield of 73.24%.

MS m/z (ESI): 840.2 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-2-(1-methyl-1H-indazol-6-yl)-7-oxo-[1,2,4]triazolo[1,5-a]pyrimidin-4(7H)-yl) acetamide

2-(6-(4-(5-(Benzyloxy)-6-methylpyrimidine-4-carbonyl)piperazin-1-yl)-5-ethyl-2-(1-me thyl-1H-indazol-6-yl)-7-oxo-[1,2,4]triazolo[1,5-a]pyrimidin-4(7H)-yl)-N-(2-chloro-4-(trifluorom ethyl)phenyl)acetamide **139b** (39 mg, 46.41 µmol) was added to dichloromethane (1 mL), followed by addition of trifluoroacetic acid (3 mL). The mixture was reacted at 40 °C for 3 hours. Methanol was added to quench the reaction, and the mixture was concentrated under reduced pressure, then separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-2-(1-methyl-1H-indazol-6-yl)-7-oxo-[1,2,4]triazolo [1,5-a]pyrimidin-4(7H)-yl)acetamide **139** (5.62 mg) in a yield of 9.90%.

MS m/z (ESI): 750.2 [M+1]
¹H NMR (400 MHz, DMSO) δ 10.46 (s, 1H), 10.25 (s, 1H), 8.59 (s, 1H), 8.32 (s, 1H), 8.15 - 8.06 (m, 2H), 7.99 (d, J = 2.1 Hz, 1H), 7.94 - 7.87 (m, 2H), 7.72 (dd, J = 8.8, 2.1 Hz, 1H), 5.44 (s, 2H), 4.55 (d, J = 12.5 Hz, 1H), 4.12 (s, 3H), 3.53 (t, J = 10.2 Hz, 3H), 3.28 (t, J = 12.5 Hz, 1H), 3.02 (s, 3H), 2.86 (d, J = 11.2 Hz, 1H), 2.68 (d, J = 11.5 Hz, 1H), 2.45 (s, 3H), 1.23 (t, J = 7.4 Hz, 3H).

### Example 140

### 2-(2-(2-aminobenzo[d]oxazol-6-yl)-5-ethyl-6-(4-(5-hydroxy-6-methylpyrimidine-4-carbonyl)pip erazin-1-yl)-7-oxo-[1,2,4]triazolo[1,5-a]pyrimidin-4(7H)-yl)-N-(2-chloro-4-(trifluoromethyl)phe nyl)acetamide

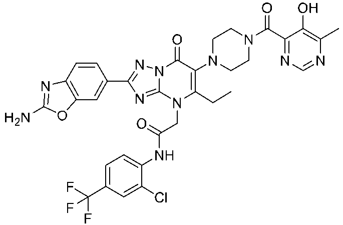

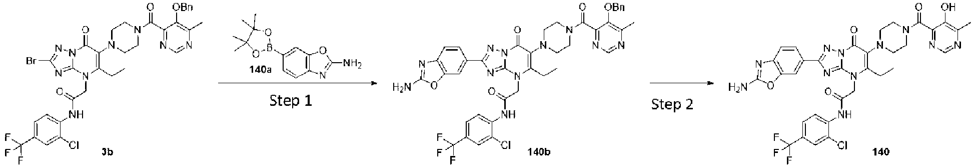

### Step 1

### 2-(2-(2-aminobenzo[d]oxazol-6-yl)-6-(4-(5-(benzyloxy)-6-methylpyrimidine-4-carbonyl)piperazi n-1-yl)-5-ethyl-7-oxo-[1,2,4]triazolo[1,5-a]pyrimidin-4(7H)-yl)-N-(2-chloro-4-(trifluoromethyl)p henyl)acetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (50 mg, 63.37 µmol), 6-(4,4,5,5-tetramethyl-1,3,2-dioxaborolan-2-yl)-1,3-benzoxazol-2-amine **140a** (19.78 mg, 76.05 µmol), [1,1'-bis(diphenylphosphino)ferrocene]palladium(II) dichloride dichloromethane complex (5.14 mg, 6.34 µmol), and sodium carbonate (20.15 mg, 190.11 µmol) were dissolved in 1,4-dioxane (1 mL) and water (0.3 mL). The mixture was subject to argon replacement four times, then heated to 80 °C and reacted for 1.5 hours. Water (20 mL) was added to the reaction mixture, followed by extraction with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System **B)** to obtain 2-(2-(2-aminobenzo[d]oxazol-6-yl)-6-(4-(5-(benzyloxy)-6-methylpyrimidine-4-carbonyl)piperazi n-1-yl)-5-ethyl-7-oxo-[1,2,4]triazolo[1,5-a]pyrimidin-4(7H)-yl)-N-(2-chloro-4-(trifluoromethyl)p henyl)acetamide **140b** (33 mg) in a yield of 61.83%.

MS m/z (ESI): 842.2 [M+1]

### Step 2

### 2-(2-(2-aminobenzo[d]oxazol-6-yl)-5-ethyl-6-(4-(5-hydroxy-6-methylpyrimidine-4-carbonyl)pip erazin-1-yl)-7-oxo-[1,2,4]triazolo[1,5-a]pyrimidin-4(7H)-yl)-N-(2-chloro-4-(trifluoromethyl)phe nyl)acetamide

2-(2-(2-Aminobenzo[d]oxazol-6-yl)-6-(4-(5-(benzyloxy)-6-methylpyrimidine-4-carbon yl)piperazin-1-yl)-5-ethyl-7-oxo-[1,2,4]triazolo[1,5-a]pyrimidin-4(7H)-yl)-N-(2-chloro-4-(trifluo romethyl)phenyl)acetamide **140b** (33 mg, 39.18 µmol) was added to dichloromethane (1 mL), followed by addition of trifluoroacetic acid (3 mL). The mixture was reacted at 40 C for 3 hours. Methanol was added to quench the reaction, and the mixture was concentrated under reduced pressure. The residue was separated by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 2-(2-(2-aminobenzo[d]oxazol-6-yl)-5-ethyl-6-(4-(5-hydroxy-6-methylpyrimidine-4-carbonyl)pip erazin-1-yl)-7-oxo-[1,2,4]triazolo[1,5-a]pyrimidin-4(7H)-yl)-N-(2-chloro-4-(trifluoromethyl)phe nyl)acetamide **140** (18.19 mg) in a yield of 9.88%.

MS m/z (ESI): 752.0 [M+1]
¹H NMR (400 MHz, DMSO) δ 10.42 (s, 1H), 10.24 (s, 1H), 8.59 (s, 1H), 8.06 (d, J = 8.6 Hz, 1H), 7.98 - 7.90 (m, 3H), 7.81 - 7.66 (m, 3H), 7.30 (d, J = 8.2 Hz, 1H), 5.39 (s, 2H), 4.54 (d, J = 12.5 Hz, 1H), 3.53 (d, J = 12.2 Hz, 3H), 3.27 (t, J = 12.6 Hz, 1H), 3.01 (s, 3H), 2.84 (d, J = 11.3 Hz, 1H), 2.67 (d, J = 9.3 Hz, 1H), 2.45 (s, 3H), 1.22 (t, J = 7.5 Hz, 3H).

### Example 141

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(2-oxoindolin-5-yl)-[1,2,4]triazolo[1,5-a]pyrimidin-4(7H)-yl)acetamid e

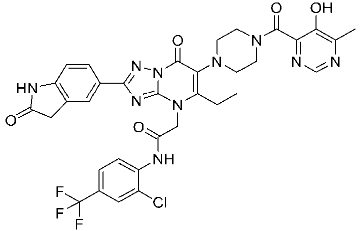

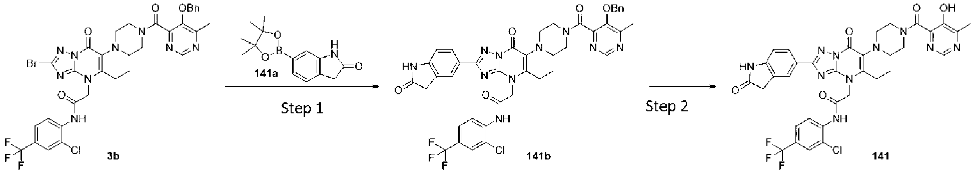

### Step 1

### 2-(6-(4-(5-(benzyloxy)-6-methylpyrimidine-4-carbonyl)piperazin-1-yl)-5-ethyl-7-oxo-2-(2-oxoin dolin-5-yl)-[1,2,4]triazolo[1,5-a]pyrimidin-4(7H)-yl)-N-(2-chloro-4-(trifluoromethyl)phenyl)acet amide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (50 mg, 63.37 µmol), 6-(4,4,5,5-tetramethyl-1,3,2-dioxaborolan-2-yl)indolin-2-one **141a** (19.70 mg, 76.05 µmol), [1,1'-bis(diphenylphosphino)ferrocene]palladium(II) dichloride dichloromethane complex (5.14 mg, 6.34 µmol), and sodium carbonate (20.15 mg, 190.11 µmol) were dissolved in 1,4-dioxane (1 mL) and water (0.3 mL). The mixture was subject to argon replacement four times, then heated to 80 °C and reacted for 1.5 hours. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System **B)** to obtain 2-(6-(4-(5-(benzyloxy)-6-methylpyrimidine-4-carbonyl)piperazin-1-yl)-5-ethyl-7-oxo-2-(2-oxoin dolin-5-yl)-[1,2,4]triazolo[1,5-a]pyrimidin-4(7H)-yl)-N-(2-chloro-4-(trifluoromethyl)phenyl)acet amide **141b** (30 mg) in a yield of 56.27%.

MS m/z (ESI): 841.1 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(2-oxoindolin-5-yl)-[1,2,4]triazolo[1,5-a]pyrimidin-4(7H)-yl)acetamid e

2-(6-(4-(5-(Benzyloxy)-6-methylpyrimidine-4-carbonyl)piperazin-1-yl)-5-ethyl-7-oxo-2 -(2-oxoindolin-5-yl)-[1,2,4]triazolo[1,5-a]pyrimidin-4(7H)-yl)-N-(2-chloro-4-(trifluoromethyl) phenyl)acetamide **141b** (30 mg, 35.66 µmol) was added to dichloromethane (1 mL). Trifluoroacetic acid (3 mL) was added at 0 °C, and the reaction was performed at 40 C for 3 hours. Methanol was added to quench the reaction, and the mixture was concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(2-oxoindolin-5-yl)-[1,2,4]triazolo[1,5-a]pyrimidin-4(7H)-yl)acetamid e **141** (2.19 mg) in a yield of 7.60%.

MS m/z (ESI): 751.0 [M+1]
¹H NMR (400 MHz, DMSO) δ 10.54 (s, 1H), 10.39 (s, 1H), 10.23 (s, 1H), 8.59 (s, 1H), 8.06 (d, J = 8.6 Hz, 1H), 7.97 (d, J = 2.1 Hz, 1H), 7.72 (dt, J = 7.1, 2.2 Hz, 2H), 7.54 (d, J = 1.5 Hz, 1H), 7.34 (d, J = 7.7 Hz, 1H), 5.39 (s, 2H), 4.54 (d, J = 12.4 Hz, 1H), 3.53 (d, J = 14.6 Hz, 5H), 3.02 (d, J = 9.8 Hz, 3H), 2.84 (d, J = 11.3 Hz, 1H), 2.67 (d, J = 10.5 Hz, 1H), 2.45 (s, 3H), 1.21 (t, J = 7.4 Hz, 3H).

### Example 142

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(2,3,4,5-tetrahydrobenzo[f][1,4]oxazepin-7-yl)-[1,2,4]triazolo[1,5-a]p yrimidin-4(7H)-yl)acetamide

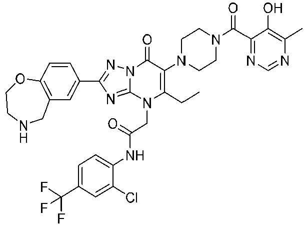

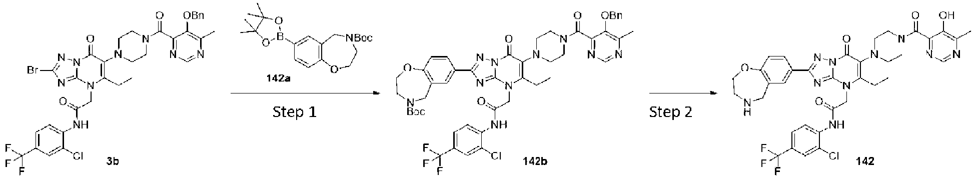

### Step 1

### tert-butyl 7-(6-(4-(5-(benzyloxy)-6-methylpyrimidine-4-carbonyl)piperazin-1-yl)-4-(2-((2-chloro-4-(trifluo romethyl)phenyl)amino)-2-oxoethyl)-5-ethyl-7-oxo-4,7-dihydro-[1,2,4]triazolo[1,5-a]pyrimidin-2-yl)-2,3-dihydrobenzo[f][1,4]oxazepine-4(5H)-carboxylate

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide 3b (50 mg, 63.37 µmol), tert-butyl 7-(4,4,5,5-tetramethyl-1,3,2-dioxaborolan-2-yl)-2,3-dihydrobenzo[f][1,4]oxazepine-4(5H)-carbox ylate **142a** (28.54 mg, 76.05 µmol, commercially available), [1,1'-bis(diphenylphosphino)ferrocene]palladium(II) dichloride dichloromethane complex (5.14 mg, 6.34 µmol), and sodium carbonate (20.15 mg, 190.11 µmol) were dissolved in 1,4-dioxane (1 mL) and water (0.3 mL). The mixture was subject to argon replacement four times, then heated to 80 °C and reacted for 1.5 hours. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System **B)** to obtain tert-butyl 7-(6-(4-(5-(benzyloxy)-6-methylpyrimidine-4-carbonyl)piperazin-1-yl)-4-(2-((2-chloro-4-(trifluo romethyl)phenyl)amino)-2-oxoethyl)-5-ethyl-7-oxo-4,7-dihydro-[1,2,4]triazolo[1,5-a]pyrimidin-2-yl)-2,3-dihydrobenzo[f][1,4]oxazepine-4(5H)-carboxylate **142b** (37 mg) in a yield of60.98%.

MS m/z (ESI): 957.3 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(2,3,4,5-tetrahydrobenzo[f][1,4]oxazepin-7-yl)-[1,2,4]triazolo[1,5-a]p yrimidin-4(7H)-yl)acetamide

Tert-Butyl 7-(6-(4-(5-(benzyloxy)-6-methylpyrimidine-4-carbonyl)piperazin-1-yl)-4-(2-((2-chloro-4-(trifluo romethyl)phenyl)amino)-2-oxoethyl)-5-ethyl-7-oxo-4,7-dihydro-[1,2,4] triazolo[1,5-a]pyrimidin-2-yl)-2,3-dihydrobenzo[f][1,4]oxazepine-4(5H)-carboxylate **142b** (37 mg, 38.65 µmol) was added to dichloromethane (1 mL). Trifluoroacetic acid (3 mL) was added, and the reaction was performed at 40 °C for 3 hours. Methanol was added to quench the reaction, and the mixture was concentrated under reduced pressure. The residue was separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin -1-yl)-7-oxo-2-(2,3,4,5-tetrahydrobenzo[f][1,4]oxazepin-7-yl)-[1,2,4]triazolo[1,5-a]pyrimidin-4(7 H)-yl)acetamide **142** (12.09 mg) in a yield of 10.29%.

MS m/z (ESI): 767.2 [M+1]
¹H NMR (400 MHz, DMSO) δ 10.42 (s, 1H), 10.23 (s, 1H), 9.16 (s, 2H), 8.59 (s, 1H), 8.22 (d, J = 2.2 Hz, 1H), 8.07 (dd, J = 8.3, 1.8 Hz, 2H), 7.98 (d, J = 2.1 Hz, 1H), 7.72 (dd, J = 8.7, 2.2 Hz, 1H), 7.23 (d, J = 8.4 Hz, 1H), 5.39 (s, 2H), 4.58 - 4.42 (m, 3H), 4.27 (d, J = 5.9 Hz, 2H), 3.29 (d, J = 11.9 Hz, 2H), 3.02 (s, 3H), 2.85 (d, J = 11.2 Hz, 1H), 2.45 (s, 3H), 1.21 (t, J = 7.4 Hz, 3H).

### Example 143

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(7-azaspiro[3.5]non-1-en-2-yl)-[1,2,4]triazolo[1,5-a]pyrimidin-4(7H)-yl)acetamide

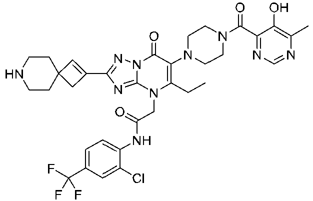

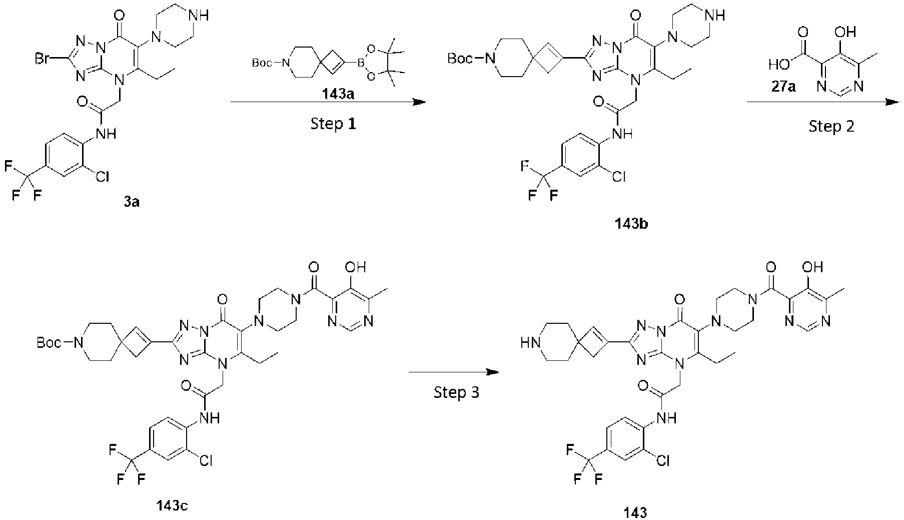

### Step 1

### tert-butyl 2-(4-(2-((2-chloro-4-(trifluoromethyl)phenyl)amino)-2-oxoethyl)-5-ethyl-7-oxo-6-(piperazin-1-yl )-4,7-dihydro-[1,2,4]triazolo[1,5-a]pyrimidin-2-yl)-7-azaspiro[3.5]non-1-ene-7-carboxylate

2-(2-Bromo-5-ethyl-7-oxo-6-(piperazin-1-yl)-[1,2,4]triazolo[1,5-a]pyrimidin-4(7H)-yl)-N-(2-chloro-4-(trifluoromethyl)phenyl)acetamide **3a** (483.0 mg, 0.86 mmol), tert-butyl 2-(4,4,5,5-tetramethyl-1,3,2-dioxaborolan-2-yl)-7-azaspiro[3.5]non-1-ene-7-carboxylate **143a** (300 mg, 0.86 mmol, commercially available), sodium carbonate (191.18 mg, 1.8 mmol), and [1,1'-bis(diphenylphosphino)ferrocene]palladium(II) dichloride dichloromethane complex (69.62 mg, 0.08 mmol) were dissolved in 1,4-dioxane (9 mL) and water (0.9 mL), and the reaction mixture was subject to nitrogen replacement three times. The reaction mixture was stirred at 90 °C for 2 hours. Water (30 mL) was added to the reaction mixture, and the mixture was extracted with ethyl acetate (20 mL × 3). The organic phases were combined, washed with saturated sodium chloride solution (20 mL × 3), dried over anhydrous sodium sulfate, filtered, and concentrated under reduced pressure. The resulting residue was purified by column chromatography (eluent: System B) to obtain tert-butyl 2-(4-(2-((2-chloro-4-(trifluoromethyl)phenyl)amino)-2-oxoethyl)-5-ethyl-7-oxo-6-(piperazin-1-yl )-4,7-dihydro-[1,2,4]triazolo[1,5-a]pyrimidin-2-yl)-7-azaspiro[3.5]non-1-ene-7-carboxylate **143b** (110 mg) in a yield of 18.16%.

MS m/z (ESI): 705.2 [M+1]

### Step 2

### tert-butyl 2-(4-(2-((2-chloro-4-(trifluoromethyl)phenyl)amino)-2-oxoethyl)-5-ethyl-6-(4-(5-hydroxy-6-met hylpyrimidine-4-carbonyl)piperazin-1-yl)-7-oxo-4,7-dihydro-[1,2,4]triazolo[1,5-a]pyrimidin-2-yl )-7-azaspiro[3.5]non-1-ene-7-carboxylate

5-Hydroxy-6-methylpyrimidine-4-carboxylic acid **27a** (36.06 mg, 0.23 mmol), N,N-diisopropylethylamine (60.48 mg, 0.47 mmol), 1-hydroxybenzotriazole (42.16 mg, 0.31 mmol), and 1-(3-dimethylaminopropyl)-3-ethylcarbodiimide hydrochloride (59.81 mg, 0.31 mmol) were dissolved in N,N-dimethylformamide (4 mL), and tert-butyl 2-(4-(2-((2-chloro-4-(trifluoromethyl)phenyl)amino)-2-oxoethyl)-5-ethyl-7-oxo-6-(piperazin-1-yl )-4,7-dihydro-[1,2,4]triazolo[1,5-a]pyrimidin-2-yl)-7-azaspiro[3.5]non-1-ene-7-carboxylate **143b** (110 mg, 0.15 mmol) was added to the reaction mixture. The reaction mixture was stirred at 30 °C for 16 h. The reaction mixture was purified by reverse-phase column chromatography (eluent: System **B)** to obtain tert-butyl 2-(4-(2-((2-chloro-4-(trifluoromethyl)phenyl)amino)-2-oxoethyl)-5-ethyl-6-(4-(5-hydroxy-6-met hylpyrimidine-4-carbonyl)piperazin-1-yl)-7-oxo-4,7-dihydro-[1,2,4]triazolo [1,5-a]pyrimidin-2-yl)-7-azaspiro[3.5]non-1-ene-7-carboxylate **143c** (35 mg) in a yield of 26.67%.

MS m/z (ESI): 841.2 [M+1]

### Step 3

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(7-azaspiro[3.5]non-1-en-2-yl)-[1,2,4]triazolo[1,5-a]pyrimidin-4(7H)-yl)acetamide

Tert-butyl 2-(4-(2-((2-chloro-4-(trifluoromethyl)phenyl)amino)-2-oxoethyl)-5-ethyl-6-(4-(5-hydroxy-6-met hylpyrimidine-4-carbonyl)piperazin-1-yl)-7-oxo-4,7-dihydro-[1,2,4]triazolo [1,5-a]pyrimidin-2-yl)-7-azaspiro[3.5]non-1-ene-7-carboxylate **143c** (35 mg, 0.04 mmol) was dissolved in dichloromethane (5 mL), and trifluoroacetic acid (4.74 mg, 0.04 mmol) was added to the reaction mixture at 0 °C. The reaction mixture was stirred at room temperature for 30 minutes. The solvent was removed under reduced pressure, and the residue was purified by preparative liquid chromatography (column: XBridge XBridge C₁₈; 19 × 250 mm, 10 µm, 20 mL/min; mobile phase A: 0.05% TFA/H₂O, mobile phase B: CH₃CN gradient: 33-43%; retention time: 6.3-7.2 min/17 min) to obtain N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(7-azaspiro[3.5]non-1-en-2-yl)-[1,2,4] triazolo[1,5-a]pyrimidin-4(7H)-yl)acetamide **143** (30.83 mg) in a yield of 79.75%.

MS m/z (ESI): 741.1 [M+1]
¹H NMR (400 MHz, DMSO-d6) δ 10.35 (s, 1H), 10.23 (s, 1H), 8.58 (s, 1H), 8.10 (d, J = 8.8 Hz, 1H), 7.98 (s, 1H), 7.72 (d, J = 8.8 Hz, 1H), 6.92 (s, 1H), 5.32 (s, 2H), 4.52 (d, J = 12.0 Hz, 1H), 3.51-3.48 (m, 4H), 3.29 - 3.24 (m, 1H), 3.20 - 3.13 (m, 2H), 3.07-3.00 (m 5H), 2.84 - 2.78 (m, 1H), 2.66 - 2.63 (m, 2H), 2.44 (s, 3H), 1.84 - 1.74 (m, 4H), 1.18 (t, J = 8.0 Hz, 3H).

### Example 144

### N-(2-chloro-4-(trifluoromethyl)phenyl)-2-(5-ethyl-6-(4-(5-hydroxy-6-methylpyrimidine-4-carbon yl)piperazin-1-yl)-2-(2-methyl-1,2,3,3a,4,6a-hexahydrocyclopenta[c]pyrrol-5-yl)-7-oxo-[1,2,4]tri azolo[1,5-a]pyrimidin-4(7H)-yl)acetamide

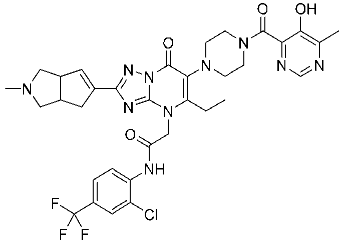

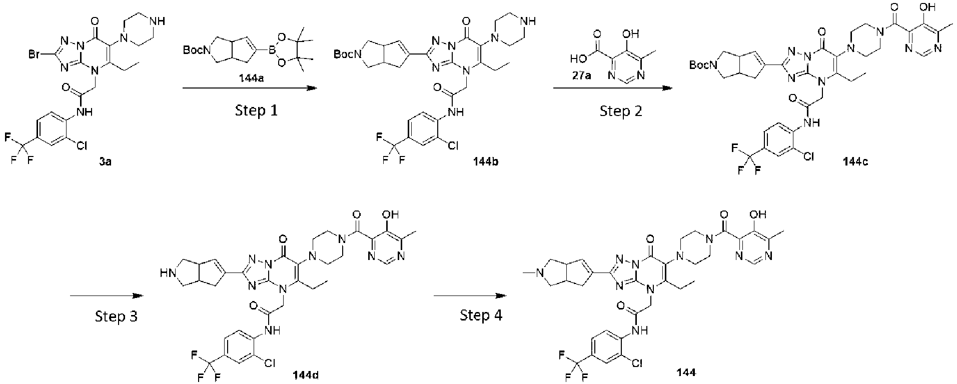

### Step 1

### tert-butyl 5-(4-(2-((2-chloro-4-(trifluoromethyl)phenyl)amino)-2-oxoethyl)-5-ethyl-7-oxo-6-(piperazin-1-yl )-4,7-dihydro-[1,2,4]triazolo[1,5-a]pyrimidin-2-yl)-3,3a,4,6a-tetrahydrocyclopenta[c]pyrrole-2(1 H)-carboxylate

2-(2-Bromo-5-ethyl-7-oxo-6-(piperazin-1-yl)-[1,2,4]triazolo[1,5-a]pyrimidin-4(7H)-yl)-N-(2-chloro-4-(trifluoromethyl)phenyl)acetamide **3a** (800.0 mg, 1.42 mmol), tert-butyl 5-(4,4,5,5-tetramethyl-1,3,2-dioxaborolan-2-yl)-3,3a,4,6a-tetrahydrocyclopenta[c]pyrrole-2(1H)-carboxylate **144a** (476.56 mg, 1.42 mmol, commercially available), sodium carbonate (316.40 mg, 2.99 mmol), and [1,1'-bis(diphenylphosphino)ferrocene]palladium(II) dichloride dichloromethane complex (115.23 mg, 0.14 mmol) were dissolved in 1,4-dioxane (24 mL) and water (2.4 mL), and the reaction mixture was subject to nitrogen replacement three times. The reaction mixture was stirred at 90 °C for 2 hours. Water (30 mL) was added to the reaction mixture, and the mixture was extracted with ethyl acetate (20 mL × 3). The organic phases were combined, washed with saturated sodium chloride solution (20 mL × 3), dried over anhydrous sodium sulfate, filtered, and concentrated under reduced pressure. The resulting residue was purified by column chromatography (eluent: System **B)** to obtain tert-butyl 5-(4-(2-((2-chloro-4-(trifluoromethyl)phenyl)amino)-2-oxoethyl)-5-ethyl-7-oxo-6-(piperazin-1-yl )-4,7-dihydro-[1,2,4]triazolo[1,5-a]pyrimidin-2-yl)-3,3a,4,6a-tetrahydrocyclopenta[c]pyrrole-2(1 H)-carboxylate **144b** (370 mg) with a yield of 37.66%.

MS m/z (ESI): 691.2 [M+1]

### Step 2

### tert-butyl 5-(4-(2-((2-chloro-4-(trifluoromethyl)phenyl)amino)-2-oxoethyl)-5-ethyl-6-(4-(5-hydroxy-6-met hylpyrimidine-4-carbonyl)piperazin-1-yl)-7-oxo-4,7-dihydro-[1,2,4]triazolo[1,5-a]pyrimidin-2-yl )-3,3a,4,6a-tetrahydrocyclopenta[c]pyrrole-2(1H)-carboxylate

5-Hydroxy-6-methylpyrimidine-4-carboxylic acid **27a** (120.42 mg, 0.78 mmol), N,N-diisopropylethylamine (201.95 mg, 1.56 mmol), 1-hydroxybenzotriazole (140.76 mg, 1.04 mmol), and 1-(3-dimethylaminopropyl)-3-ethylcarbodiimide hydrochloride (199.7 mg, 1.04 mmol) were dissolved in N,N-dimethylformamide (2 mL), and then tert-butyl 5-(4-(2-((2-chloro-4-(trifluoromethyl)phenyl)amino)-2-oxoethyl)-5-ethyl-7-oxo-6-(piperazin-1-yl )-4,7-dihydro-[1,2,4]triazolo[1,5-a]pyrimidin-2-yl)-3,3a,4,6a-tetrahydrocyclopenta[c]pyrrole-2(1 H)-carboxylate **144b** (360 mg, 0.52 mmol) was added to the reaction mixture. The reaction mixture was stirred at 30 °C for 16 hours. The reaction mixture was purified by reverse-phase column chromatography (eluent: System **B)** to obtain tert-butyl 5-(4-(2-((2-chloro-4-(trifluoromethyl)phenyl)amino)-2-oxoethyl)-5-ethyl-6-(4-(5-hydroxy-6-met hylpyrimidine-4-carbonyl)piperazin-1-yl)-7-oxo-4,7-dihydro-[1,2,4]triazolo[1,5-a]pyrimidin-2-yl )-3,3a,4,6a-tetrahydrocyclopenta[c]pyrrole-2(1H)-carboxylate **144c** (165 mg) with a yield of 38.29%.

MS m/z (ESI): 827.5 [M+1]

### Step 3

### N-(2-chloro-4-(trifluoromethyl)phenyl)-2-(5-ethyl-2-(1,2,3,3a,4,6a-hexahydrocyclopenta[c]pyrrol -5-yl)-6-(4-(5-hydroxy-6-methylpyrimidine-4-carbonyl)piperazin-1-yl)-7-oxo-[1,2,4]triazolo[1,5-a]pyrimidin-4(7H)-yl)acetamide

Tert-butyl 5-(4-(2-((2-chloro-4-(trifluoromethyl)phenyl)amino)-2-oxoethyl)-5-ethyl-6-(4-(5-hydroxy-6-met hylpyrimidine-4-carbonyl)piperazin-1-yl)-7-oxo-4,7-dihydro-[1,2,4] triazolo[1,5-a]pyrimidin-2-yl)-3,3a,4,6a-tetrahydrocyclopenta[c]pyrrole-2(1H)-carboxylate **144c** (165 mg, 0.2 mmol) was dissolved in dichloromethane (10 mL), and trifluoroacetic acid (22.74 mg, 0.2 mmol) was added to the reaction mixture at 0 °C. The reaction mixture was stirred at room temperature for 30 minutes. An aqueous sodium bicarbonate solution was added to the reaction mixture to adjust the pH to 8, and the mixture was extracted with dichloromethane (20 mL × 3). The organic phases were combined, washed with saturated sodium chloride solution (20 mL × 3), dried over anhydrous sodium sulfate, filtered, and concentrated under reduced pressure to obtain N-(2-chloro-4-(trifluoromethyl)phenyl)-2-(5-ethyl-2-(1,2,3,3a,4,6a-hexahydrocyclopenta[c]pyrrol -5-yl)-6-(4-(5-hydroxy-6-methylpyrimidine-4-carbonyl)piperazin-1-yl)-7-oxo-[1,2,4]triazolo[1,5-a]pyrimidin-4(7H)-yl)acetamide **144d** (140 mg) with a yield of 96.53%.

MS m/z (ESI): 727.2 [M+1]

### Step 4

### N-(2-chloro-4-(trifluoromethyl)phenyl)-2-(5-ethyl-6-(4-(5-hydroxy-6-methylpyrimidine-4-carbon yl)piperazin-1-yl)-2-(2-methyl-1,2,3,3a,4,6a-hexahydrocyclopenta[c]pyrrol-5-yl)-7-oxo-[1,2,4]tri azolo[1,5-a]pyrimidin-4(7H)-yl)acetamide

N-(2-chloro-4-(trifluoromethyl)phenyl)-2-(5-ethyl-2-(1,2,3a,4,6a-hexahydrocyclopenta [c]pyrrol-5-yl)-6-(4-(5-hydroxy-6-methylpyrimidine-4-carbonyl)piperazin-1-yl)-7-oxo-[1,2,4] triazolo[1,5-a]pyrimidin-4(7H)-yl)acetamide **144d** (30 mg, 0.04 mmol) was dissolved in methanol (4 mL), and formaldehyde (24.78 mg, 0.82 mmol) and sodium cyanoborohydride (7.78 mg, 0.12 mmol) were added to the reaction mixture, and the reaction mixture was stirred at room temperature for 16 hours. The solvent was removed by concentration under reduced pressure, and the residue was purified by preparative liquid chromatography (separation column XBridge XBridge C₁₈; 19 ×250 mm, 10 µm, 20 mL/min; mobile phase A: 0.1% FA/H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-2-(2-methyl-1,2,3,3a,4,6a-hexahydrocyclopenta[c]pyrrol-5-yl)-7-oxo-[1,2,4]tri azolo[1,5-a]pyrimidin-4(7H)-yl)acetamide **144** (4.11 mg) in a yield of 13.44%.

MS m/z (ESI): 741.1 [M+1]
¹H NMR (400 MHz, DMSO-d6) δ 10.36 (s, 1H), 8.55 (s, 1H), 8.07 (d, J = 8.0 Hz, 1H), 7.97 (s, 1H), 7.72 - 7.70 (m, 1H), 6.44 (s, 1H), 5.32 (s, 2H), 4.53-4.50 (m, 1H), 3.51 - 3.48 (m, 3H), 3.26 - 3.21 (m, 2H), 2.98-2.93 (m, 5H), 2.85 - 2.76 (m, 1H), 2.68 - 2.62 (m, 1H), 2.56 - 2.52 (m, 1H), 2.48 - 2.36 (m, 7H), 2.18 (s, 3H), 1.18 (t, J = 8.0 Hz, 3H).

### Example 145

### 2-(2-(2-Acetyl-1,2,3,3a,4,6a-hexahydrocyclopenta[c]pyrrol-5-yl)-5-ethyl-6-(4-(5-hydroxy-6-meth ylpyrimidine-4-carbonyl)piperazin-1-yl)-7-oxo-[1,2,4]triazolo[1,5-a]pyrimidin-4(7H)-yl)-N-(2-ch loro-4-(trifluoromethyl)phenyl)acetamide

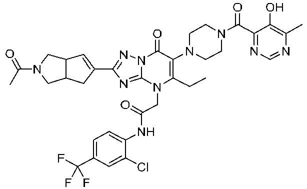

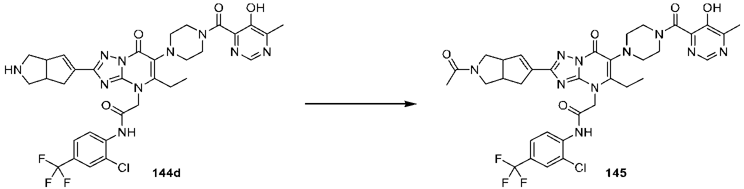

N-(2-Chloro-4-(trifluoromethyl)phenyl)-2-(5-ethyl-2-(1,2,3a,4,6a-hexahydrocyclopenta [c]pyrrol-5-yl)-6-(4-(5-hydroxy-6-methylpyrimidine-4-carbonyl)piperazin-1-yl)-7-oxo-[1,2,4] triazolo[1,5-a]pyrimidin-4(7H)-yl)acetamide **144d** (50 mg, 0.068 mmol) and N,N-diisopropylethylamine (15.11 mg, 0.11 mmol) were dissolved in N,N-dimethylformamide (2 mL), and acetyl chloride (5.4 mg, 0.068 mmol) was added to the reaction mixture at 0 °C, and the reaction mixture was stirred at 0 °C for 30 minutes. The solvent was removed by concentration under reduced pressure, and the residue was purified by preparative liquid chromatography (separation column XBridge XBridge C₁₈; 19×250 mm, 10 µm, 20 mL/min; mobile phase A: 0.1% FA/H₂O, mobile phase B: CH₃CN gradient: 43-53%; retention time: 9.1-10.1 min/17 min) to obtain 2-(2-(2-acetyl-1,2,3,3a,4,6a-hexahydrocyclopenta[c]pyrrol-5-yl)-5-ethyl-6-(4-(5-hydroxy-6-meth ylpyrimidine-4-carbonyl)piperazin-1-yl)-7-oxo-[1,2,4]triazolo[1,5-a]pyrimidin-4(7H)-yl)-N-(2-ch loro-4-(trifluoromethyl)phenyl)acetamide **145** (6.67 mg) in a yield of 12.61%.

MS m/z (ESI): 769.2 [M+1]
¹H NMR (400 MHz, DMSO-d6) δ 10.36 (s, 1H), 8.51 (s, 1H), 8.07 (dd, J = 8.8, 4.8 Hz, 1H), 7.96 (s, 1H), 7.71 (d, J = 8.8 Hz, 1H), 6.50 (d, J = 12.0 Hz, 1H), 5.32 (s, 2H), 4.52 (d, J = 16.0 Hz, 1H), 3.76 - 3.67 (m, 1H), 3.63 - 3.55 (m, 2H), 3.52 - 3.41 (m, 4H), 3.25 - 3.17 (m, 1H), 3.13 - 3.11 (m, 1H), 3.04 - 2.91 (m, 5H), 2.82 - 2.79 (m, 1H), 2.69 - 2.62 (m, 2H), 2.42 (s, 3H), 1.90-1.89 (m, 3H), 1.18 (t, J = 8.0 Hz, 3H).

### Example 146

### 2-(5-Ethyl-2-(1,2,3,3a,4,6a-hexahydrocyclopenta[c]pyrrol-5-yl)-6-(4-(5-hydroxy-6-methylpyrimi dine-4-carbonyl)piperazin-1-yl)-7-oxo-[1,2,4]triazolo[1,5-a]pyrimidin-4(7H)-yl)-N-(4-(pentafluo ro-λ⁶-sulfaneyl)phenyl)acetamide

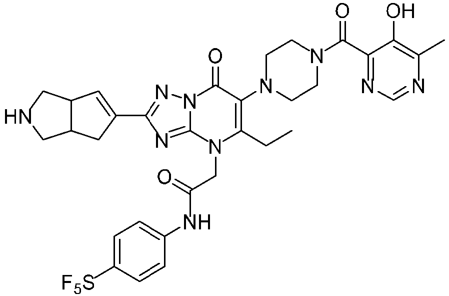

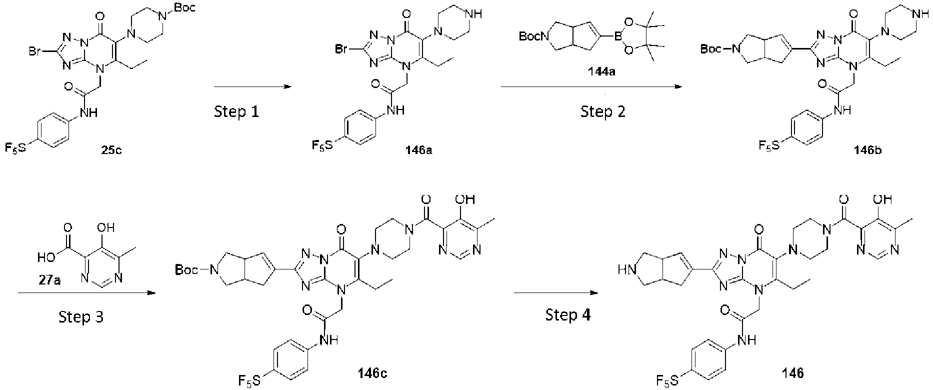

### Step 1

### 2-(2-Bromo-5-ethyl-7-oxo-6-(piperazin-1-yl)-[1,2,4]triazolo[1,5-a]pyrimidin-4(7H)-yl)-N-(4-(pe ntafluoro-λ⁶-sulfaneyl)phenyl)acetamide

Tert-butyl 4-(2-bromo-5-ethyl-7-oxo-4-(2-oxo-2-((4-(pentafluoro-λ⁶-sulfaneyl)phenyl) amino)ethyl)-4,7-dihydro-[1,2,4]triazolo[1,5-a]pyrimidin-6-yl)piperazine-1-carboxylate **25c** (420 mg, 611.81 µmol) was dissolved in 1,4-dioxane (5 mL), and 4N hydrochloric acid in dioxane solution (10 mL) was added at 0 °C, and the reaction mixture was stirred at room temperature for 3 hours. The mixture was concentrated under reduced pressure to obtain 2-(2-bromo-5-ethyl-7-oxo-6-(piperazin-1-yl)-[1,2,4]triazolo[1,5-a]pyrimidin-4(7H)-yl)-N-(4-(pen tafluoro-λ⁶-sulfaneyl) phenyl)acetamide **146a** (350 mg) in a yield of 97.56%.

MS m/z (ESI): 588.1 [M+1]

### Step 2

### tert-butyl 5-(5-ethyl-7-oxo-4-(2-oxo-2-((4-(pentafluoro-λ⁶-sulfaneyl)phenyl)amino)ethyl)-6-(piperazin-1-yl )-4,7-dihydro-[1,2,4]triazolo[1,5-a]pyrimidin-2-yl)-3,3a,4,6a-tetrahydrocyclopenta[c]pyrrole-2(1 H)-carboxylate

2-(2-Bromo-5-ethyl-7-oxo-6-(piperazin-1-yl)-[1,2,4]triazolo[1,5-a]pyrimidin-4(7H)-yl)-N-(4-(pentafluoro-λ⁶-sulfaneyl)phenyl)acetamide **146a** (350 mg, 596.89 µmol), tert-butyl 5-(4,4,5,5-tetramethyl-1,3,2-dioxaborolan-2-yl)-3,3a,4,6a-tetrahydrocyclopenta[c]pyrrole-2(1H)-carboxylate **144a** (200 mg, 596.89 µmol), sodium carbonate (132.85 mg, 1.25 mmol), and [1,1'-bis(diphenylphosphino)ferrocene]palladium dichloride (48 mg, 59.69 µmol) were dissolved in dioxane (10 mL) and water (1 mL), and the reaction mixture was stirred at 90 °C under nitrogen saturation for 2 hours. Water (30 mL) was added to the reaction mixture, and the mixture was extracted with ethyl acetate (50 mL × 3). The organic phases were combined, washed with saturated sodium chloride solution (50 mL × 3), dried over anhydrous sodium sulfate, filtered, and concentrated under reduced pressure. The resulting residue was purified by thin-layer silica gel plate separation (eluent: System B) to obtain tert-butyl 5-(5-ethyl-7-oxo-4-(2-oxo-2-((4-(pentafluoro-λ⁶-sulfaneyl)phenyl)amino)ethyl)-6-(piperazin-1-yl )-4,7-dihydro-[1,2,4]triazolo[1,5-a]pyrimidin-2-yl)-3,3a,4,6a-tetrahydrocyclopenta[c]pyrrole-2(1 H)-carboxylate **146b** (300 mg) with a yield of 70.32%.

MS m/z (ESI): 715.4 [M+1]

### Step 3

### tert-butyl 5-(5-ethyl-6-(4-(5-hydroxy-6-methylpyrimidine-4-carbonyl)piperazin-1-yl)-7-oxo-4-(2-oxo-2-((4 -(pentafluoro-λ⁶-sulfaneyl)phenyl)amino)ethyl)-4,7-dihydro-[1,2,4]triazolo[1,5-a]pyrimidin-2-yl) -3,3a,4,6a-tetrahydrocyclopenta[c]pyrrole-2(1H)-carboxylate

5-Hydroxy-6-methylpyrimidine-4-carboxylic acid 27a (64.69 mg, 419.73 µmol), 1-hydroxybenzotriazole (75.62 mg, 559.64 µmol), 1-(3-dimethylaminopropyl)-3-ethylcarbodiimide hydrochloride (107.28 mg, 559.64 µmol), and N,N-diisopropylethylamine (108.49 mg, 839.46 µmol) were dissolved in N,N-dimethylformamide (2 mL), and the mixture was stirred at 30 °C for 10 minutes. Then tert-butyl 5-(5-ethyl-7-oxo-4-(2-oxo-2-((4-(pentafluoro-λ⁶-sulfaneyl)phenyl)amino)ethyl)-6-(piperazin-1-yl )-4,7-dihydro-[1,2,4]triazolo[1,5-a]pyrimidin-2-yl)-3,3a,4,6a-tetrahydrocyclopenta[c]pyrrole-2(1 H)-carboxylate **146b** (200 mg, 279.82 µmol) was added, and the mixture was stirred at 30°C for 16 hours. Water (30 mL) was added to the reaction mixture, and the mixture was extracted with ethyl acetate (30 mL × 3). The organic phases were combined, washed with saturated sodium chloride solution (30 mL × 3), dried over anhydrous sodium sulfate, filtered, and concentrated under reduced pressure. The resulting residue was purified by reverse-phase column chromatography (eluent: System **D)** to obtain tert-butyl 5-(5-ethyl-6-(4-(5-hydroxy-6-methylpyrimidine-4-carbonylpiperazin-1-yl)-7-oxo-4-(2-oxo-2-((4-(pentafluoro-λ⁶-sulfaneyl)phenyl)amino)ethyl)-4,7-dihydro-[1,2,4]triazolo[1,5-a]pyrimidin-2-yl)-3,3a,4,6a-tetrahydrocyclopenta[c]pyrrole-2(1H)-carboxylate **146c** (60 mg) with a yield of 22.68%.

MS m/z (ESI): 795.3 [M-55]

### Step 4

### 2-(5-ethyl-2-(1,2,3,3a,4,6a-hexahydrocyclopenta[c]pyrrol-5-yl)-6-(4-(5-hydroxy-6-methylpyrimi dine-4-carbonyl)piperazin-1-yl)-7-oxo-[1,2,4]triazolo[1,5-a]pyrimidin-4(7H)-yl)-N-(4-(pentafluo ro-λ⁶-sulfaneyl)phenyl)acetamide

Tert-butyl 5-(5-ethyl-6-(4-(5-hydroxy-6-methylpyrimidine-4-carbonylpiperazin-l-yl)-7-oxo-4-(2-oxo-2-((4-(pentafluoro-λ⁶-sulfaneyl)phenyl)amino)ethyl)-4,7-dihydro-[1,2,4]triazolo [1,5-a]pyrimidin-2-yl)-3,3a,4,6a-tetrahydrocyclopenta[c]pyrrole-2(1H)-carboxylate **146c** (60 mg, 70.52 µmol) was dissolved in dichloromethane (2.5 mL) and trifluoroacetic acid (0.5 mL), and the reaction mixture was stirred at room temperature for 1 hour. The reaction solution was concentrated under reduced pressure, and the resulting residue was purified by preparative liquid chromatography (Waters 3767/QDA column: SunFire Sunfire C₁₈, 19 × 250 mm, 10 µm; mobile phase A: 10 mmol/L A: 0.05% TFA/H₂O, B: ACN; flow rate: 20 mL/min; gradient: 30-40% retention time: 8.8-10 min, total 17 min) to obtain 2-(5-ethyl-2-(1,2,3,3a,4,6a-hexahydrocyclopenta[c]pyrrol-5-yl)-6-(4-(5-hydroxy-6-methylpyrimi dine-4-carbonyl)piperazin-1-yl)-7-oxo-[1,2,4]triazolo[1,5-a]pyrimidin-4(7H)-yl)-N-(4-(pentafluo ro-λ⁶-sulfaneyl)phenyl) acetamide **146** (29.62 mg) with a yield of 55.95%.

**MS** m/z (ESI): 751.3 [M+1]
¹H NMR (400 MHz, MeOD-d4) δ 8.57 (s, 1H), 7.82 - 7.72 (m, 4H), 6.53 (d, J = 1.6 Hz, 1H), 5.32-5.21 (m, 2H), 4.70 (d, J = 11.6 Hz, 1H), 4.12 (d, J = 10.8 Hz, 1H), 3.85-3.80 (m, 1H), 3.79-3.70 (m, 2H), 3.53 - 3.33 (m, 6H), 3.21 - 3.02 (m, 5H), 2.94 (d, J = 10.8 Hz, 1H), 2.87-2.77 (m, 2H), 2.52 (s, 3H), 1.29 (t, J = 7.6 Hz, 3H).

### Example 147

### N-(2-chloro-4-((methyl-d3)thio)phenyl)-2-(2-(2,3-dihydrobenzofuran-5-yl)-5-ethyl-6-(4-(5-hydro xy-6-methylpyrimidine-4-carbonyl)piperazin-1-yl)-7-oxo-[1,2,4]triazolo[1,5-a]pyrimidin-4(7H)-yl)acetamide

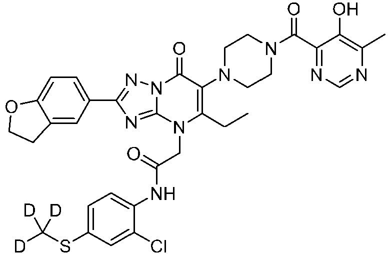

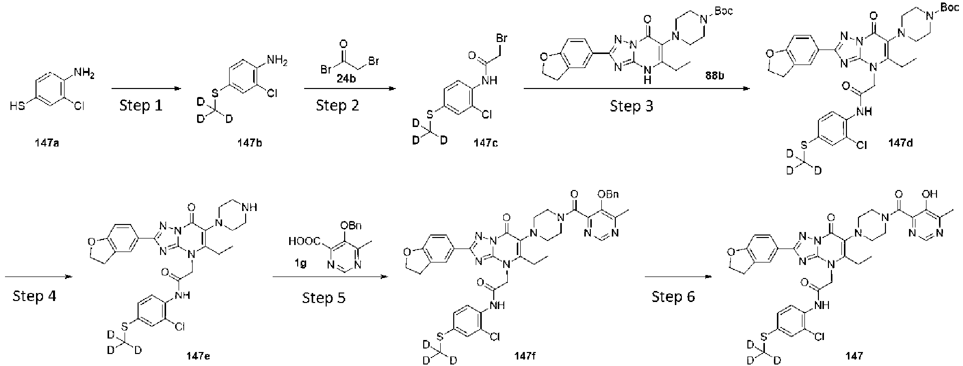

### Step 1

### 2-chloro-4-((methyl-d3)thio)aniline

4-Amino-3-chlorobenzenethiol **147a** (100 mg, 626.42 µmol) was dissolved in acetonitrile (2 mL), followed by addition of triethylamine (95.08 mg, 939.64 µmol) and deuterated iodomethane (136.21 mg, 939.64 µmol), and the reaction was allowed to proceed at room temperature for 30 minutes. Water (50 mL) was added to the reaction solution, and the mixture was extracted with ethyl acetate (30 mL × 3). The combined organic phases were washed with saturated sodium chloride solution (50 mL × 2), dried over anhydrous sodium sulfate, filtered, and the filtrate was concentrated under reduced pressure. The resulting residue was purified by thin-layer silica gel plate separation (eluent: System **B)** to obtain 2-chloro-4-((methyl-d3)thio)aniline **147b** (70 mg) with a yield of 63.25%.

### Step 2

### 2-bromo-N-(2-chloro-4-((methyl-d3)thio)phenyl)acetamide

2-Chloro-4-((methyl-d3)thio)aniline **147b** (0.1 g, 565.99 µmol) and 4-dimethylaminopyridine (69.15 mg, 565.99 µmol) were dissolved in dichloromethane (3 mL), and 2-bromoacetyl bromide **24b** (137.09 mg, 679.19 µmol) was slowly added under ice bath, followed by reaction at room temperature for 18 hours. After completion of the reaction, the residue was purified by silica gel column chromatography (developing solvent: System A) to obtain 2-bromo-N-(2-chloro-4-((methyl-d3)thio)phenyl)acetamide **147c** (0.14 g) with a yield of 83.11%.

MS m/z (ESI): 298.9 [M+1]

### Step 3

### tert-butyl 4-(4-(2-((2-chloro-4-((methyl-d3)thio)phenyl)amino)-2-oxoethyl)-2-(2,3-dihydrobenzofuran-5-yl )-5-ethyl-7-oxo-4,7-dihydro-[1,2,4]triazolo[1,5-a]pyrimidin-6-yl)piperazine-1-carboxylate

2-Bromo-N-(2-chloro-4-((methyl-d3)thio)phenyl)acetamide **147c** (76.55 mg, 257.22 µmol), N,N-dimethylformamide (2 mL), and N,N-diisopropylethylamine (83.11 mg, 643.04 µmol) were added sequentially, followed by tert-butyl 4-(2-(2,3-dihydrobenzofuran-5-yl)-5-ethyl-7-oxo-4,7-dihydro-[1,2,4]triazolo[1,5-a]pyrimidin-6-y l)piperazine-1-carboxylate **88b** (100 mg, 214.35 µmol), and the reaction was performed at 50°C for 2 hours. Water (50 mL) was added to the reaction mixture, and the mixture was extracted with ethyl acetate (30 mL×3). The combined organic phases were washed with saturated sodium chloride solution (20 mL ×2), dried over anhydrous sodium sulfate, filtered, and concentrated under reduced pressure. The residue was purified by silica gel column chromatography (eluent: system **B)** to obtain tert-butyl 4-(4-(2-((2-chloro-4-((methyl-d3)thio)phenyl)amino)-2-oxoethyl)-2-(2,3-dihydrobenzofuran-5-yl )-5-ethyl-7-oxo-4,7-dihydro-[1,2,4]triazolo[1,5-a]pyrimidin-6-yl)piperazine-1-carboxylate **147d** (100 mg) with a yield of 68.28%.

MS m/z (ESI): 683.1 [M+H]

### Step 4

### N-(2-chloro-4-((methyl-d3)thio)phenyl)-2-(2-(2,3-dihydrobenzofuran-5-yl)-5-ethyl-7-oxo-6-(pipe razin-1-yl)-[1,2,4]triazolo[1,5-a]pyrimidin-4(7H)-yl)acetamide

Tert-butyl 4-(4-(2-((2-chloro-4-((methyl-d3)thio)phenyl)amino)-2-oxoethyl)-2-(2,3-dihydrobenzofuran-5-yl )-5-ethyl-7-oxo-4,7-dihydro-[1,2,4]triazolo[1,5-a]pyrimidin-6-yl)piperazine-1-carboxylate **147d** (100 mg, 146.36 µmol) was dissolved in dichloromethane (4 mL), and trifluoroacetic acid (1 g, 8.77 mmol) was added dropwise. The reaction was performed at room temperature for 3 hours. Upon completion of the reaction, the mixture was concentrated to obtain N-(2-chloro-4-((methyl-d3)thio)phenyl)-2-(2-(2,3-dihydrobenzofuran-5-yl)-5-ethyl-7-oxo-6-(pipe razin-1-yl)-[1,2,4]triazolo[1,5-a]pyrimidin-4(7H)-yl)acetamide **147e** (60 mg) with a yield of 70.30%.

MS m/z (ESI): 583.0 [M+H]+

### Step 5

### 2-(6-(4-(5-(benzyloxy)-6-methylpyrimidine-4-carbonyl)piperazin-1-yl)-2-(2,3-dihydrobenzofuran -5-yl)-5-ethyl-7-oxo-[1,2,4]triazolo[1,5-a]pyrimidin-4(7H)-yl)-N-(2-chloro-4-((methyl-d3)thio)p henyl)acetamide

5-(Benzyloxy)-6-methylpyrimidine-4-carboxylic acid **1g** (50.26 mg, 205.79 µmol), 1-(3-dimethylaminopropyl)-3-ethylcarbodiimide hydrochloride (39.45 mg, 205.79 µmol), 1-hydroxybenzotriazole (27.81 mg, 205.79 µmol), and N,N-diisopropylethylamine (66.49 mg, 514.48 µmol) were added sequentially to N,N-dimethylformamide (2 mL), and the reaction was performed at room temperature for 15 minutes. N-(2-chloro-4-((methyl-d3)thio)phenyl)-2-(2-(2,3-dihydrobenzofuran-5-yl)-5-ethyl-7-oxo-6-(pipe razin-1-yl)-[1,2,4]triazolo[1,5-a]pyrimidin-4(7H)-yl)acetamide **147e** (60 mg, 102.90 µmol) was added, and the reaction was performed at room temperature for 16 hours. Water (30 mL) was added to the reaction mixture, and the mixture was extracted with dichloromethane (50 mL×3). The combined organic phases were washed with saturated sodium chloride solution (50 mL×2), dried over anhydrous sodium sulfate, filtered, and concentrated under reduced pressure. The resulting residue was purified by thin-layer silica gel plate separation (eluent: system **B)** to obtain 2-(6-(4-(5-(benzyloxy)-6-methylpyrimidine-4-carbonyl)piperazin-1-yl)-2-(2,3-dihydrobenzofuran -5-yl)-5-ethyl-7-oxo-[1,2,4]triazolo[1,5-a]pyrimidin-4(7H)-yl)-N-(2-chloro-4-((methyl-d3)thio)p henyl)acetamide **147f** (50 mg) with a yield of 60.04%.

MS m/z (ESI): 809.0 [M+H]

### Step 6

### N-(2-chloro-4-((methyl-d3)thio)phenyl)-2-(2-(2,3-dihydrobenzofuran-5-yl)-5-ethyl-6-(4-(5-hydro xy-6-methylpyrimidine-4-carbonyl)piperazin-1-yl)-7-oxo-[1,2,4]triazolo[1,5-a]pyrimidin-4(7H)-yl)acetamide

2-(6-(4-(5-(Benzyloxy)-6-methylpyrimidine-4-carbonyl)piperazin-1-yl)-2-(2,3-dihydrob enzofuran-5-yl)-5-ethyl-7-oxo-[1,2,4]triazolo[1,5-a]pyrimidin-4(7H)-yl)-N-(2-chloro-4-((methyl-d3)thio)phenyl)acetamide **147f** (50 mg, 61.78 µmol) was dissolved in trifluoroacetic acid (3 mL), and the reaction was performed at 45 °C for 3 hours. Methanol was added to quench the reaction, and the mixture was concentrated under reduced pressure. The residue was separated by preparative liquid chromatography (separation column: AKZONOBEL Kromasil; 250 ×21.2 mm I.D.; 5 µm, 20 mL/min; mobile phase A: 0.05% TFA+H₂O, mobile phase B: CH₃CN) to obtain N-(2-chloro-4-((methyl-d3)thio)phenyl)-2-(2-(2,3-dihydrobenzofuran-5-yl)-5-ethyl-6-(4-(5-hydro xy-6-methylpyrimidine-4-carbonyl)piperazin-1-yl)-7-oxo-[1,2,4]triazolo[1,5-a]pyrimidin-4(7H)-yl)acetamide **147** (15 mg) with a yield of 33.08%.

MS m/z (ESI): 719.1 [M+H]
¹H NMR (400 MHz, DMSO-d6) δ 10.24 (s, 1H), 10.12 (s, 1H), 8.58 (s, 1H), 7.98 (d, J = 1.8 Hz, 1H), 7.88 (dd, J = 8.3, 1.9 Hz, 1H), 7.59 (d, J = 8.6 Hz, 1H), 7.39 (d, J = 2.2 Hz, 1H), 7.21 (dd, J = 8.6, 2.2 Hz, 1H), 6.88 (d, J = 8.3 Hz, 1H), 5.28 (s, 2H), 4.61 (t, J = 8.7 Hz, 2H), 4.53 (d, J = 12.3 Hz, 1H), 3.53 (q, J = 10.7, 8.5 Hz, 3H), 3.26 (t, J = 8.8 Hz, 3H), 3.00 (q, J = 8.5, 7.7 Hz, 3H), 2.83 (d, J = 11.3 Hz, 1H), 2.70-2.62 (m, 1H), 2.45 (s, 3H), 1.21 (t, J = 7.4 Hz, 3H).

### Example 148

### N-(2-chloro-4-((difluoromethyl)thio)phenyl)-2-(2-(2,3-dihydrobenzofuran-5-yl)-5-ethyl-6-(4-(5-h ydroxy-6-methylpyrimidine-4-carbonyl)piperazin-1-yl)-7-oxo-[1,2,4]triazolo[1,5-a]pyrimidin-4(7 H)-yl)acetamide

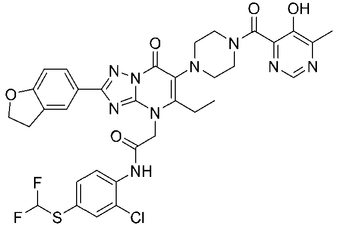

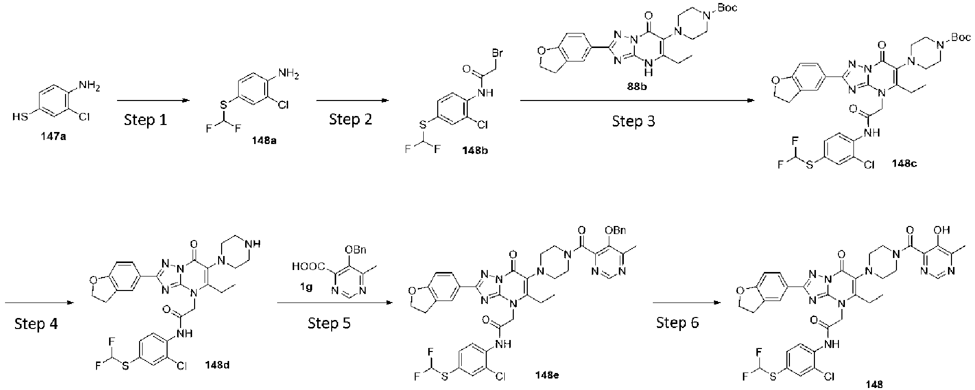

### Step 1

### 2-chloro-4-((difluoromethyl)thio)aniline

Sodium hydride (35.08 mg, 876.99 µmol, 60%) was dissolved in N,N-dimethylformamide (3 mL), then 4-amino-3-chlorobenzenethiol **147a** (70 mg, 438.50 µmol) and (difluoromethyl)triphenylphosphonium bromide (258.63 mg, 657.75 µmol) were slowly added, and the reaction was performed at room temperature for 16 hours. Water (30 mL) was added to the reaction mixture, the mixture was extracted with dichloromethane (50 mL×3), the organic phases were combined, washed with saturated sodium chloride solution (50 mL ×2), dried over anhydrous sodium sulfate, filtered, and concentrated under reduced pressure, and the resulting residue was purified by silica gel column chromatography (eluent: System **A)** to obtain 2-chloro-4-((difluoromethyl)thio)aniline **148a** (45 mg) with a yield of 48.95%.

MS m/z(ESI): 209.9 [M+H]

### Step 2

### 2-bromo-N-(2-chloro-4-((difluoromethyl)thio)phenyl)acetamide

2-Chloro-4-((difluoromethyl)thio)aniline **148a** (40 mg, 190.80 µmol) was dissolved in dichloromethane (4 mL), then 4-dimethylaminopyridine (23.31 mg, 190.80 µmol) was added, and then 2-bromoacetyl bromide (46.21 mg, 228.96 µmol) was slowly added, and the reaction was performed at room temperature for 18 hours. After completion of the reaction, water (30 mL) was added to the reaction mixture, the mixture was extracted with dichloromethane (50 mL×3), the organic phases were combined, washed with saturated sodium chloride solution (50 mL ×2), dried over anhydrous sodium sulfate, filtered, and concentrated under reduced pressure, and the resulting residue was purified by silica gel column chromatography (eluent: System A) to obtain 2-bromo-N-(2-chloro-4-((difluoromethyl)thio)phenyl)acetamide **148b** (50 mg) with a yield of 79.27%.

MS m/z(ESI): 331.9 [M+H]

### Step 3

### tert-butyl 4-(4-(2-((2-chloro-4-((difluoromethyl)thio)phenyl)amino)-2-oxoethyl)-2-(2,3-dihydrobenzofuran-5-yl)-5-ethyl-7-oxo-4,7-dihydro-[1,2,4]triazolo[1,5-a]pyrimidin-6-yl)piperazine-1-carboxylate

2-Bromo-N-(2-chloro-4-((difluoromethyl)thio)phenyl)acetamide **148b** (50.00 mg, 151.24 µmol), tert-butyl 4-(2-(2,3-dihydrobenzofuran-5-yl)-5-ethyl-7-oxo-4,7-dihydro-[1,2,4]triazolo[1,5-a]pyrimidin-6-y l)piperazine-1-carboxylate **88b** (80 mg, 171.48 µmol), and N,N-diisopropylethylamine (66.49 mg, 514.43 µmol) were sequentially added to N,N-dimethylformamide (3 mL), and the reaction was performed at 50 °C for 2.5 hours. After completion of the reaction, water (30 mL) was added to the reaction mixture, the mixture was extracted with dichloromethane (50 mL ×3), the organic phases were combined, washed with saturated sodium chloride solution (50 mL×2), dried over anhydrous sodium sulfate, filtered, and concentrated under reduced pressure, and the resulting residue was purified by silica gel column chromatography (eluent: System **B)** to obtain tert-butyl 4-(4-(2-((2-chloro-4-((difluoromethyl)thio)phenyl)amino)-2-oxoethyl)-2-(2,3-dihydrobenzofuran-5-yl)-5-ethyl-7-oxo-4,7-dihydro-[1,2,4]triazolo[1,5-a]pyrimidin-6-yl)piperazine-1-carboxylate **148c** (25 mg) with a yield of 20.36%.

MS m/z(ESI): 659.9 [M+H-56]

### Step 4

### N-(2-chloro-4-((difluoromethyl)thio)phenyl)-2-(2-(2,3-dihydrobenzofuran-5-yl)-5-ethyl-7-oxo-6-(piperazin-1-yl)-[1,2,4]triazolo[1,5-a]pyrimidin-4(7H)-yl)acetamide

Tert-butyl 4-(4-(2-((2-chloro-4-((difluoromethyl)thio)phenyl)amino)-2-oxoethyl)-2-(2,3-dihydrobenzofuran-5-yl)-5-ethyl-7-oxo-4,7-dihydro-[1,2,4]triazolo[1,5-a]pyrimidin-6-yl)piperazine-1-carboxylate **148c** (25 mg, 34.91 µmol) was dissolved in dichloromethane (3 mL), trifluoroacetic acid (1 g, 8.77 mmol) was added dropwise, and the reaction was performed at room temperature for 2 hours. After completion of the reaction, the mixture was concentrated under reduced pressure to obtain N-(2-chloro-4-((difluoromethyl)thio)phenyl)-2-(2-(2,3-dihydrobenzofuran-5-yl)-5-ethyl-7-oxo-6-(piperazin-1-yl)-[1,2,4]triazolo[1,5-a]pyrimidin-4(7H)-yl)acetamide **148d** (20 mg), and the crude product was directly used in the next step.

MS m/z(ESI): 616.2 [M+H]

### Step 5

### 2-(6-(4-(5-(benzyloxy)-6-methylpyrimidine-4-carbonyl)piperazin-1-yl)-2-(2,3-dihydrobenzofuran -5-yl)-5-ethyl-7-oxo-[1,2,4]triazolo[1,5-a]pyrimidin-4(7H)-yl)-N-(2-chloro-4-((difluoromethyl)th io)phenyl)acetamide

5-(Benzyloxy)-6-methylpyrimidine-4-carboxylic acid 1g (15.86 mg, 64.93 µmol), 1-(3-dimethylaminopropyl)-3-ethylcarbodiimide hydrochloride (12.45 mg, 64.93 µmol), 1-hydroxybenzotriazole (8.77 mg, 64.93 µmol), and N,N-diisopropylethylamine (20.98 mg, 162.32 µmol) were sequentially added to N,N-dimethylformamide (2 mL) and reacted at room temperature for 15 minutes, then N-(2-chloro-4-((difluoromethyl)thio)phenyl)-2-(2-(2,3-dihydrobenzofuran-5-yl)-5-ethyl-7-oxo-6-(piperazin-1-yl)-[1,2,4]triazolo[1,5-a]pyrimidin-4(7H)-yl)acetamide **148d** (20 mg, 32.46 µmol) was added, and the reaction was performed at room temperature for 18 hours. After completion of the reaction, water (30 mL) was added to the reaction mixture, the mixture was extracted with dichloromethane (50 mL ×3), the organic phases were combined, washed with saturated sodium chloride solution (50 mL ×2), dried over anhydrous sodium sulfate, filtered, and concentrated under reduced pressure, and the resulting residue was purified by silica gel column chromatography (eluent: System **B)** to obtain 2-(6-(4-(5-(benzyloxy)-6-methylpyrimidine-4-carbonyl)piperazin-1-yl)-2-(2,3-dihydrobenzofuran -5-yl)-5-ethyl-7-oxo-[1,2,4]triazolo[1,5-a]pyrimidin-4(7H)-yl)-N-(2-chloro-4-((difluoromethyl)th io)phenyl)acetamide **148e** (12 mg) with a yield of 43.89%.

MS m/z(ESI): 842.0 [M+H]

### Step 6

### N-(2-chloro-4-((difluoromethyl)thio)phenyl)-2-(2-(2,3-dihydrobenzofuran-5-yl)-5-ethyl-6-(4-(5-h ydroxy-6-methylpyrimidine-4-carbonyl)piperazin-1-yl)-7-oxo-[1,2,4]triazolo[1,5-a]pyrimidin-4(7 H)-yl)acetamide

2-(6-(4-(5-(Benzyloxy)-6-methylpyrimidine-4-carbonyl)piperazin-1-yl)-2-(2,3-dihydrob enzofuran-5-yl)-5-ethyl-7-oxo-[1,2,4]triazolo[1,5-a]pyrimidin-4(7H)-yl)-N-(2-chloro-4-((difluoro methyl)thio)phenyl)acetamide **148e** (12 mg, 14.25 µmol) was dissolved in trifluoroacetic acid (3 mL) and reacted at 45 °C for 3 hours. After completion of the reaction, methanol was added to quench the reaction, the mixture was concentrated under reduced pressure, and then separated by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain N-(2-chloro-4-((difluoromethyl)thio)phenyl)-2-(2-(2,3-dihydrobenzofuran-5-yl)-5-ethyl-6-(4-(5-h ydroxy-6-methylpyrimidine-4-carbonyl)piperazin-1-yl)-7-oxo-[1,2,4]triazolo[1,5-a]pyrimidin-4(7 H)-yl)acetamide **148** (4 mg) with a yield of 33.59%.

MS m/z(ESI): 752.2 [M+H]
¹H NMR (400 MHz, DMSO-d6) δ 10.32 (s, 1H), 8.58 (s, 1H), 7.97 (d, J = 1.7 Hz, 1H), 7.91 - 7.83 (m, 2H), 7.76 (d, J = 2.1 Hz, 1H), 7.54 (dd, J = 8.5, 2.1 Hz, 1H), 7.50 (t, 1H), 6.88 (d, J = 8.4 Hz, 1H), 5.34 (s, 2H), 4.62 (d, J = 8.6 Hz, 2H), 4.53 (d, J = 12.5 Hz, 1H), 3.52 (d, J = 11.9 Hz, 3H), 3.25 (s, 3H), 3.01 (s, 2H), 2.84 (d, J = 11.4 Hz, 1H), 2.45 (s, 3H), 1.21 (t, J = 7.5 Hz, 3H).

### Example 149

### N-(2-chloro-4-(trifluoromethyl)phenyl)-2-(2-(2,3-dihydrobenzofuran-5-yl)-5-ethyl-6-(1-(5-hydro xy-6-methylpyrimidine-4-carbonyl)-1,2,3,6-tetrahydropyridin-4-yl)-7-oxo-[1,2,4]triazolo[1,5-a]p yrimidin-4(7H)-yl)acetamide

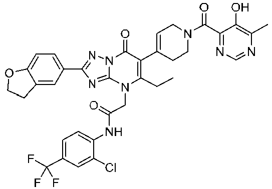

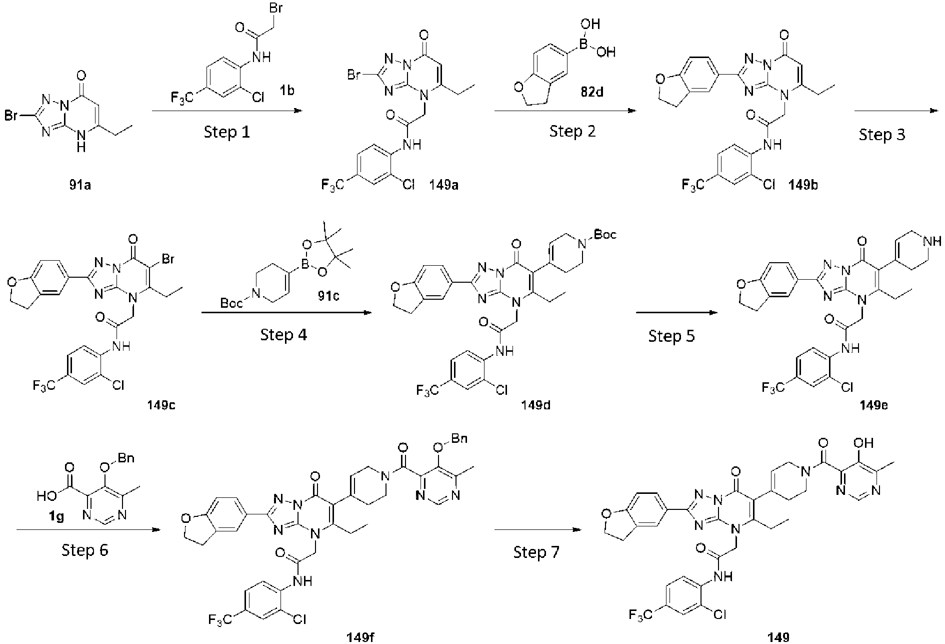

### Step 1

### 2-(2-bromo-5-ethyl-7-oxo-[1,2,4]triazolo[1,5-a]pyrimidin-4(7H)-yl)-N-(2-chloro-4-(trifluoromet hyl)phenyl)acetamide

2-Bromo-5-ethyl-[1,2,4]triazolo[1,5-a]pyrimidin-7(4H)-one **91a** (3 g, 12.34 mmol), 2-bromo-N-(2-chloro-4-(trifluoromethyl)phenyl)acetamide **1b** (4.10 g, 12.96 mmol), and N,N-diisopropylethylamine (6.38 g, 49.37 mmol) were added to N,N-dimethylformamide (20 mL), and the temperature was raised to 50 °C for reaction for 2 h. Water (30 mL) was added to the reaction mixture, and the mixture was extracted with ethyl acetate (50 mL ×3). The organic phases were combined, washed with saturated sodium chloride solution (50 mL ×2), dried over anhydrous sodium sulfate, filtered, and concentrated under reduced pressure. The obtained residue was purified by silica gel column chromatography (eluent: System **A)** to obtain 2-(2-bromo-5-ethyl-7-oxo-[1,2,4]triazolo[1,5-a]pyrimidin-4(7H)-yl)-N-(2-chloro-4-(trifluoromet hyl)phenyl)acetamide **149a** (3.45 g) with a yield of 58.40%.

MS m/z (ESI): 478.0 [M+1]

### Step 2

### N-(2-chloro-4-(trifluoromethyl)phenyl)-2-(2-(2,3-dihydrobenzofuran-5-yl)-5-ethyl-7-oxo-[1,2,4]t riazolo[1,5-a]pyrimidin-4(7H)-yl)acetamide

At room temperature, 2-(2-bromo-5-ethyl-7-oxo-[1,2,4]triazolo[1,5-a]pyrimidin-4(7H)-yl)-N-(2-chloro-4-(trifluoromet hyl)phenyl)acetamide **149a** (3.45 g, 7.21 mmol), (2,3-dihydrobenzofuran-5-yl)boronic acid **82d** (1.36 g, 8.29 mmol), [1,1'-bis(diphenylphosphino)ferrocene]palladium(II) dichloride (584.25 mg, 720.78 µmol), and sodium carbonate (2.29 g, 21.62 mmol) were dissolved in 1,4-dioxane (50 mL) and water (5 mL). The mixture was subject to argon replacement three times, and the temperature was raised to 75 °C for reaction for 6 h. Water (30 mL) was added to the reaction mixture, and the mixture was extracted with ethyl acetate (50 mL×3). The organic phases were combined, washed with saturated sodium chloride solution (50 mL ×2), dried over anhydrous sodium sulfate, filtered, and concentrated under reduced pressure. The obtained residue was purified by silica gel column chromatography (eluent: System **B)** to obtain N-(2-chloro-4-(trifluoromethyl)phenyl)-2-(2-(2,3-dihydrobenzofuran-5-yl)-5-ethyl-7-oxo-[1,2,4]t riazolo[1,5-a]pyrimidin-4(7H)-yl)acetamide **149b** (2.68 g) with a yield of 71.80%.

MS m/z (ESI): 518.1 [M+1]

### Step 3

### 2-(6-bromo-2-(2,3-dihydrobenzofuran-5-yl)-5-ethyl-7-oxo-[1,2,4]triazolo[1,5-a]pyrimidin-4(7H)-yl)-N-(2-chloro-4-(trifluoromethyl)phenyl)acetamide

At room temperature, N-bromosuccinimide (412.40 mg, 2.32 mmol) was added to N,N-dimethylformamide (10 mL) containing N-(2-chloro-4-(trifluoromethyl)phenyl)-2-(2-(2,3-dihydrobenzofuran-5-yl)-5-ethyl-7-oxo-[1,2,4]t riazolo[1,5-a]pyrimidin-4(7H)-yl)acetamide **149b** (1 g, 1.93 mmol), and the reaction was performed at room temperature for 18 h. Water (30 mL) was added to the reaction mixture, and the mixture was extracted with ethyl acetate (50 mL ×3). The organic phases were combined, washed with saturated sodium chloride solution (50 mL ×2), dried over anhydrous sodium sulfate, filtered, and concentrated under reduced pressure. The obtained residue was purified by silica gel column chromatography (eluent: System **B**) to obtain 2-(6-bromo-2-(2,3-dihydrobenzofuran-5-yl)-5-ethyl-7-oxo-[1,2,4]triazolo[1,5-a]pyrimidin-4(7H)-yl)-N-(2-chloro-4-(trifluoromethyl)phenyl)acetamide **149c** (1.1 g) with a yield of 95.46%.

MS m/z (ESI): 596.1 [M+1]

### Step 4

### tert-butyl 4-(4-(2-((2-chloro-4-(trifluoromethyl)phenyl)amino)-2-oxoethyl)-2-(2,3-dihydrobenzofuran-5-yl) -5-ethyl-7-oxo-4,7-dihydro-[1,2,4]triazolo[1,5-a]pyrimidin-6-yl)-3,6-dihydropyridine-1(2H)-carb oxylate

At room temperature, 2-(6-bromo-2-(2,3-dihydrobenzofuran-5-yl)-5-ethyl-7-oxo-[1,2,4]triazolo[1,5-a]pyrimidin-4(7H)-yl)-N-(2-chloro-4-(trifluoromethyl)phenyl)acetamide **149c** (150 mg, 251.35 µmol), tert-butyl 4-(4,4,5,5-tetramethyl-1,3,2-dioxaborolan-2-yl)-3,6-dihydropyridine-1(2H)-carboxylate **91c** (93.26 mg, 301.62 µmol), [1,1'-bis(diphenylphosphino)ferrocene]palladium(II) dichloride (20.37 mg, 25.13 µmol), and sodium carbonate (79.92 mg, 754.04 µmol) were dissolved in 1,4-dioxane (7 mL) and water (0.7 mL). The mixture was subject to argon replacement three times, and the temperature was raised to 100 °C for reaction for 6 h. Water (30 mL) was added to the reaction mixture, and the mixture was extracted with ethyl acetate (50 mL ×3). The organic phases were combined, washed with saturated sodium chloride solution (50 mL ×2), dried over anhydrous sodium sulfate, filtered, and concentrated under reduced pressure. The obtained residue was purified by silica gel column chromatography (eluent: System **B**) to obtain tert-butyl 4-(4-(2-((2-chloro-4-(trifluoromethyl)phenyl)amino)-2-oxoethyl)-2-(2,3-dihydrobenzofuran-5-yl) -5-ethyl-7-oxo-4,7-dihydro-[1,2,4]triazolo[1,5-a]pyrimidin-6-yl)-3,6-dihydropyridine-1(2H)-carb oxylate **149d** (140 mg) with a yield of 79.67%.

MS m/z (ESI): 643.2 [M-56+H]

### Step 5

### N-(2-chloro-4-(trifluoromethyl)phenyl)-2-(2-(2,3-dihydrobenzofuran-5-yl)-5-ethyl-7-oxo-6-(1,2, 3,6-tetrahydropyridin-4-yl)-[1,2,4]triazolo[1,5-a]pyrimidin-4(7H)-yl)acetamide

At room temperature, trifluoroacetic acid (1 mL) was added to a solution of tert-butyl 4-(4-(2-((2-chloro-4-(trifluoromethyl)phenyl)amino)-2-oxoethyl)-2-(2,3-dihydrobenzofuran-5-yl) -5-ethyl-7-oxo-4,7-dihydro-[1,2,4]triazolo[1,5-a]pyrimidin-6-yl)-3,6-dihydropyridine-1(2H)-carb oxylate **149d** (50 mg, 71.52 µmol) in dichloromethane (4 mL), and the reaction was performed at room temperature for 40 minutes. The mixture was concentrated under reduced pressure to obtain N-(2-chloro-4-(trifluoromethyl)phenyl)-2-(2-(2,3-dihydrobenzofuran-5-yl)-5-ethyl-7-oxo-6-(1,2, 3,6-tetrahydropyridin-4-yl)-[1,2,4]triazolo[1,5-a]pyrimidin-4(7H)-yl)acetamide **149e** (42 mg), which was used directly in the next step without purification.

MS m/z (ESI): 599.2 [M+1]

### Step 6

### 2-(6-(1-(5-(benzyloxy)-6-methylpyrimidine-4-carbonyl)-1,2,3,6-tetrahydropyridin-4-yl)-2-(2,3-di hydrobenzofuran-5-yl)-5-ethyl-7-oxo-[1,2,4]triazolo[1,5-a]pyrimidin-4(7H)-yl)-N-(2-chloro-4-(tr ifluoromethyl)phenyl)acetamide

At room temperature, 5-(benzyloxy)-6-methylpyrimidine-4-carboxylic acid **1g** (26.30 mg, 107.68 µmol), 1-(3-dimethylaminopropyl)-3-ethylcarbodiimide hydrochloride (27.52 mg, 143.57 µmol), 1-hydroxybenzotriazole (19.40 mg, 143.57 µmol), and N,N-diisopropylethylamine (46.39 mg, 358.93 µmol) were added to N,N-dimethylformamide (2.5 mL), and the reaction was performed at room temperature for 40 minutes. A solution of N-(2-chloro-4-(trifluoromethyl)phenyl)-2-(2-(2,3-dihydrobenzofuran-5-yl)-5-ethyl-7-oxo-6-(1,2, 3,6-tetrahydropyridin-4-yl)-[1,2,4]triazolo[1,5-a]pyrimidin-4(7H)-yl)acetamide **149e** (43 mg, 71.79 µmol) in N,N-dimethylformamide (2.5 mL) was added, and the reaction was performed at room temperature for 18 hours. Water (30 mL) was added to the reaction mixture, which was extracted with ethyl acetate (50 mL ×3). The organic phases were combined, washed with saturated sodium chloride solution (50 mL ×2), dried over anhydrous sodium sulfate, filtered, and concentrated under reduced pressure. The residue was purified by silica gel column chromatography (eluent: System **B**) to obtain 2-(6-(1-(5-(benzyloxy)-6-methylpyrimidine-4-carbonyl)-1,2,3,6-tetrahydropyridin-4-yl)-2-(2,3-di hydrobenzofuran-5-yl)-5-ethyl-7-oxo-[1,2,4]triazolo[1,5-a]pyrimidin-4(7H)-yl)-N-(2-chloro-4-(tr ifluoromethyl)phenyl)acetamide **149f** (20 mg) in a yield of 33.76%.

MS m/z (ESI): 825.2 [M+1]

### Step 7

### N-(2-chloro-4-(trifluoromethyl)phenyl)-2-(2-(2,3-dihydrobenzofuran-5-yl)-5-ethyl-6-(1-(5-hydro xy-6-methylpyrimidine-4-carbonyl)-1,2,3,6-tetrahydropyridin-4-yl)-7-oxo-[1,2,4]triazolo[1,5-a]p yrimidin-4(7H)-yl)acetamide

At room temperature, trifluoroacetic acid (3 mL) was added to a solution of 2-(6-(1-(5-(benzyloxy)-6-methylpyrimidine-4-carbonyl)-1,2,3,6-tetrahydropyridin-4-yl)-2-(2,3-di hydrobenzofuran-5-yl)-5-ethyl-7-oxo-[1,2,4]triazolo[1,5-a]pyrimidin-4(7H)-yl)-N-(2-chloro-4-(tr ifluoromethyl)phenyl)acetamide **149f** (20 mg, 24.24 µmol) in dichloromethane (1 mL), and the reaction was performed at room temperature for 48 hours. Methanol was added to quench the reaction, and the mixture was concentrated under reduced pressure. The residue was separated by preparative liquid chromatography (separation column: AKZONOBEL Kromasil; 250 ×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(2-(2,3-dihydrobenzofuran-5-yl)-5-ethyl-6-(1-(5-hydro xy-6-methylpyrimidine-4-carbonyl)-1,2,3,6-tetrahydropyridin-4-yl)-7-oxo-[1,2,4]triazolo[1,5-a]p yrimidin-4(7H)-yl)acetamide (8.37 mg) in a yield of 45.57%.

MS m/z (ESI): 735.2 [M+1]
¹H NMR (400 MHz, DMSO-d6) δ 10.41 (s, 1H), 10.27 (s, 1H), 8.59 (s, 1H), 8.06 (dd, J = 8.5, 2.6 Hz, 1H), 8.02 - 7.94 (m, 2H), 7.88 (dd, J = 8.4, 1.8 Hz, 1H), 7.72 (dd, J = 8.8, 2.1 Hz, 1H), 6.88 (d, J = 8.3 Hz, 1H), 5.77 (d, J = 68.0 Hz, 1H), 5.37 (s, 2H), 4.60 (t, J = 8.7 Hz, 2H), 4.34 (s, 2H), 4.02 (s, 1H), 3.52 (s, 1H), 3.25 (t, J = 8.7 Hz, 2H), 3.00 - 2.65 (m, 2H), 2.45 (s, 3H), 2.33 - 1.87 (m, 2H), 1.22 (q, J = 8.4, 7.8 Hz, 3H).

### Example 150

### N-(2-chloro-4-(trifluoromethyl)phenyl)-2-(2-(2,3-dihydrobenzofuran-5-yl)-5-ethyl-6-(4-(5-hydro xy-2-methylpyrimidine-4-carbonyl)piperazin-1-yl)-7-oxo-[1,2,4]triazolo[1,5-a]pyrimidin-4(7H)-yl)acetamide

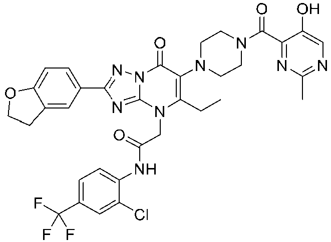

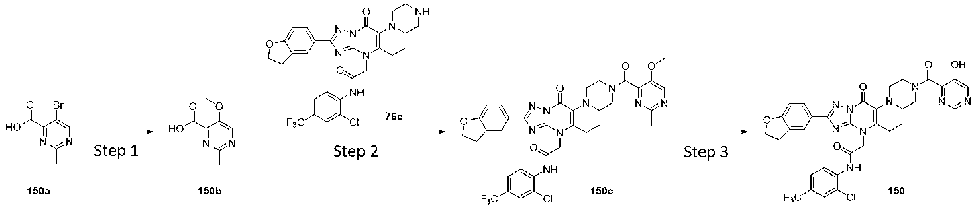

### Step 1

### 5-methoxy-2-methylpyrimidine-4-carboxylic acid

At room temperature, 5-bromo-2-methylpyrimidine-4-carboxylic acid **150a** (200 mg, 921.57 µmol, commercially available), sodium methoxide (109.52 mg, 2.03 mmol), copper oxide (21.99 mg, 276.47 µmol), and methanol (2.5 mL) were added to a 5 mL microwave tube. The tube was purged with argon for 1 minute, sealed, and heated to 110 °C for 7 hours. The pH was adjusted to 2-3 with concentrated hydrochloric acid, and the mixture was concentrated. The residue was purified by silica gel column chromatography (eluent: System **B**) to obtain 5-methoxy-2-methylpyrimidine-4-carboxylic acid **150b** (30 mg) in a yield of 19.48%.

MS m/z (ESI): 169.1 [M+1]

### Step 2

### N-(2-chloro-4-(trifluoromethyl)phenyl)-2-(2-(2,3-dihydrobenzofuran-5-yl)-5-ethyl-6-(4-(5-metho xy-2-methylpyrimidine-4-carbonyl)piperazin-1-yl)-7-oxo-[1,2,4]triazolo[1,5-a]pyrimidin-4(7H)-yl)acetamide

At room temperature, 5-methoxy-2-methylpyrimidine-4-carboxylic acid **150b** (29.33 mg, 174.42 µmol), 1-(3-dimethylaminopropyl)-3-ethylcarbodiimide hydrochloride (44.58 mg, 232.56 µmol), 1-hydroxybenzotriazole (31.42 mg, 232.56 µmol), and N,N-diisopropylethylamine (75.14 mg, 581.39 µmol) were dissolved in N,N-dimethylformamide (2 mL) and reacted at room temperature for 40 minutes, followed by addition of a solution of N-(2-chloro-4-(trifluoromethyl)phenyl)-2-(2-(2,3-dihydrobenzofuran-5-yl)-5-ethyl-7-oxo-6-(pipe razin-1-yl)-[1,2,4]triazolo[1,5-a]pyrimidin-4(7H)-yl)acetamide **76c** (70 mg, 116.28 µmol) in N,N-dimethylformamide (2 mL), and the reaction was performed at room temperature for 18 hours. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain N-(2-chloro-4-(trifluoromethyl)phenyl)-2-(2-(2,3-dihydrobenzofuran-5-yl)-5-ethyl-6-(4-(5-metho xy-2-methylpyrimidine-4-carbonyl)piperazin-1-yl)-7-oxo-[1,2,4]triazolo[1,5-a]pyrimidin-4(7H)-yl)acetamide **150c** (70 mg) with a yield of 80.04%.

MS m/z (ESI): 752.0 [M+1]

### Step 3

### N-(2-chloro-4-(trifluoromethyl)phenyl)-2-(2-(2,3-dihydrobenzofuran-5-yl)-5-ethyl-6-(4-(5-hydro xy-2-methylpyrimidine-4-carbonyl)piperazin-1-yl)-7-oxo-[1,2,4]triazolo[1,5-a]pyrimidin-4(7H)-yl)acetamide

At room temperature, N-(2-chloro-4-(trifluoromethyl)phenyl)-2-(2-(2,3-dihydrobenzofuran-5-yl)-5-ethyl-6-(4-(5-metho xy-2-methylpyrimidine-4-carbonyl)piperazin-1-yl)-7-oxo-[1,2,4]triazolo[1,5-a]pyrimidin-4(7H)-yl)acetamide **150c** (40 mg, 53.18 µmol) and anhydrous lithium chloride (11.27 mg, 265.91 µmol) were added to N,N-dimethylformamide (1 mL), and the temperature was raised to 157 °C for reaction for 8 hours. Methanol was added to quench the reaction, and the mixture was concentrated under reduced pressure, followed by separation using preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(2-(2,3-dihydrobenzofuran-5-yl)-5-ethyl-6-(4-(5-hydro xy-2-methylpyrimidine-4-carbonyl)piperazin-1-yl)-7-oxo-[1,2,4]triazolo[1,5-a]pyrimidin-4(7H)-yl)acetamide **150** (10.54 mg) with a yield of 26.58%.

MS m/z (ESI): 738.2 [M+1]
¹H NMR (400 MHz, DMSO-d6) δ 10.55 (s, 1H), 10.39 (s, 1H), 8.34 (s, 1H), 8.06 (d, J = 8.6 Hz, 1H), 7.97 (d, J = 2.2 Hz, 2H), 7.87 (dd, J = 8.4, 1.8 Hz, 1H), 7.71 (dd, J = 9.0, 2.1 Hz, 1H), 6.88 (d, J = 8.3 Hz, 1H), 5.37 (s, 2H), 4.60 (t, J = 8.7 Hz, 2H), 4.51 (d, J = 12.2 Hz, 1H), 3.56 - 3.43 (m, 2H), 3.28 (dt, J = 17.3, 5.5 Hz, 4H), 2.99 (dt, J = 13.2, 6.4 Hz, 3H), 2.83 (d, J = 11.2 Hz, 1H), 2.74 - 2.61 (m, 1H), 2.53 (s, 3H), 1.20 (t, J = 7.4 Hz, 3H).

### Example 151

### N-(2-chloro-4-(trifluoromethyl)phenyl)-2-(2-(2,3-dihydrobenzofuran-5-yl)-5-ethyl-6-(4-(5-hydro xypyrimidine-4-carbonyl)piperazin-1-yl)-7-oxo-[1,2,4]triazolo[1,5-a]pyrimidin-4(7H)-yl)acetami de

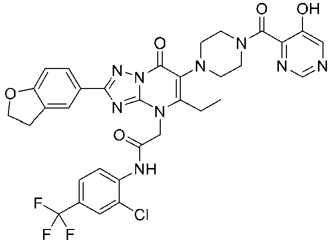

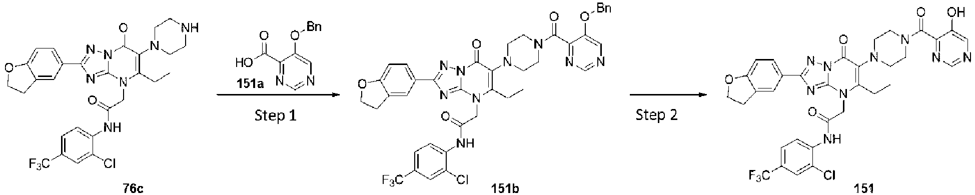

### Step 1

### 2-(6-(4-(5-(benzyloxy)pyrimidine-4-carbonyl)piperazin-1-yl)-2-(2,3-dihydrobenzofuran-5-yl)-5-e thyl-7-oxo-[1,2,4]triazolo[1,5-a]pyrimidin-4(7H)-yl)-N-(2-chloro-4-(trifluoromethyl)phenyl)acet amide

At room temperature, 5-(benzyloxy)pyrimidine-4-carboxylic acid **151a** (34.42 mg, 149.50 µmol, commercially available), 1-(3-dimethylaminopropyl)-3-ethylcarbodiimide hydrochloride (38.21 mg, 199.33 µmol), 1-hydroxybenzotriazole (26.93 mg, 199.33 µmol), and N,N-diisopropylethylamine (64.40 mg, 498.33 µmol) were dissolved in N,N-dimethylformamide (2 mL) and reacted at room temperature for 40 minutes. A solution of N-(2-chloro-4-(trifluoromethyl)phenyl)-2-(2-(2,3-dihydrobenzofuran-5-yl)-5-ethyl-7-oxo-6-(pipe razin-1-yl)-[1,2,4]triazolo[1,5-a]pyrimidin-4(7H)-yl)acetamide **76c** (60 mg, 99.67 µmol) in N,N-dimethylformamide (2 mL) was added, and the reaction was performed at room temperature for 3 hours. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)pyrimidine-4-carbonyl)piperazin-1-yl)-2-(2,3-dihydrobenzofuran-5-yl)-5-e thyl-7-oxo-[1,2,4]triazolo[1,5-a]pyrimidin-4(7H)-yl)-N-(2-chloro-4-(trifluoromethyl)phenyl)acet amide **151b** (40 mg) with a yield of 49.29%.

MS m/z (ESI): 814.2 [M+1]

### Step 2

### N-(2-chloro-4-(trifluoromethyl)phenyl)-2-(2-(2,3-dihydrobenzofuran-5-yl)-5-ethyl-6-(4-(5-hydro xypyrimidine-4-carbonyl)piperazin-1-yl)-7-oxo-[1,2,4]triazolo[1,5-a]pyrimidin-4(7H)-yl)acetami de

At room temperature, trifluoroacetic acid (4 mL) was added to a solution of 2-(6-(4-(5-(benzyloxy)pyrimidine-4-carbonyl)piperazin-1-yl)-2-(2,3-dihydrobenzofuran-5-yl)-5-e thyl-7-oxo-[1,2,4]triazolo[1,5-a]pyrimidin-4(7H)-yl)-N-(2-chloro-4-(trifluoromethyl)phenyl)acet amide **151b** (40 mg, 49.13 µmol) in dichloromethane (0.5 mL), and the reaction was performed at 40 °C for 72 hours. The mixture was concentrated under reduced pressure, followed by separation using preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(2-(2,3-dihydrobenzofuran-5-yl)-5-ethyl-6-(4-(5-hydro xypyrimidine-4-carbonyl)piperazin-1-yl)-7-oxo-[1,2,4]triazolo[1,5-a]pyrimidin-4(7H)-yl)acetami de 151 (2.12 mg) with a yield of 5.90%.

MS m/z (ESI): 724.2 [M+1]
¹H NMR (400 MHz, DMSO-d6) δ 10.93 (s, 1H), 10.40 (s, 1H), 8.72 (s, 1H), 8.47 (s, 1H), 8.06 (d, J = 8.6 Hz, 1H), 7.97 (t, J = 2.5 Hz, 2H), 7.88 (dd, J = 8.3, 1.8 Hz, 1H), 7.71 (dd, J = 8.8, 2.1 Hz, 1H), 6.88 (d, J = 8.4 Hz, 1H), 5.38 (s, 2H), 4.60 (t, J = 8.7 Hz, 2H), 4.53 (d, J = 12.4 Hz, 1H), 3.52 (ddd, J = 11.3, 7.2, 3.3 Hz, 2H), 3.35 - 3.22 (m, 4H), 3.13 - 2.88 (m, 3H), 2.84 (d, J = 11.2 Hz, 1H), 2.66 (d, J = 11.2 Hz, 1H), 1.21 (t, J = 7.5 Hz, 3H).

### Example 152

### N-(2-chloro-4-(trifluoromethyl)phenyl)-2-(5-cyclopropyl-2-(2,3-dihydrobenzofuran-5-yl)-6-(4-(5 -hydroxy-6-methylpyrimidine-4-carbonyl)piperazin-1-yl)-7-oxo-[1,2,4]triazolo[1,5-a]pyrimidin-4 (7H)-yl)acetamide

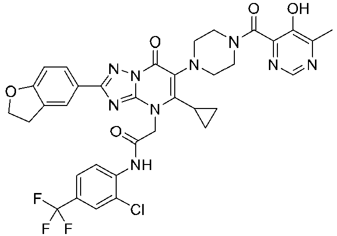

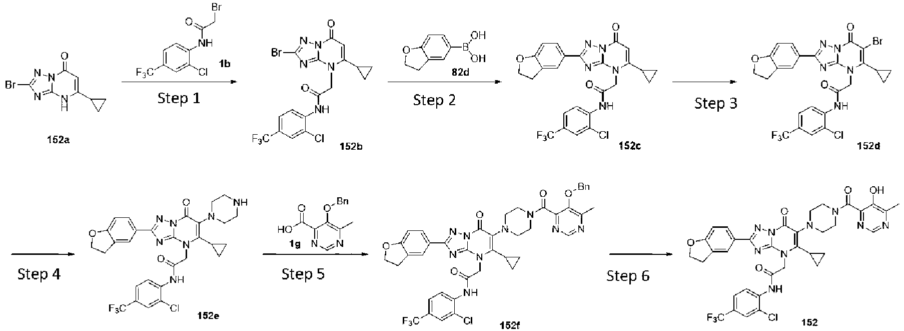

### Step 1

### 2-(2-bromo-5-cyclopropyl-7-oxo-[1,2,4]triazolo[1,5-a]pyrimidin-4(7H)-yl)-N-(2-chloro-4-(trifluo romethyl)phenyl)acetamide

At room temperature, 2-bromo-5-cyclopropyl-[1,2,4]triazolo[1,5-a]pyrimidin-7(4H)-one **152a** (547 mg, 2.14 mmol, prepared according to published patent WO2022249060), 2-bromo-N-(2-chloro-4-(trifluoromethyl)phenyl)acetamide **1b** (746.61 mg, 2.36 mmol), and N,N-diisopropylethylamine (1.39 g, 10.72 mmol) were added to N,N-dimethylformamide (10 mL), and the temperature was raised to 50 °C for reaction for 2 hours. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System **B**) to obtain 2-(2-bromo-5-cyclopropyl-7-oxo-[1,2,4]triazolo[1,5-a]pyrimidin-4(7H)-yl)-N-(2-chloro-4-(trifluo romethyl) phenyl)acetamide **152b** (360 mg) in a yield of 34.21%.

MS m/z (ESI): 490.0 [M+1]

### Step 2

### N-(2-chloro-4-(trifluoromethyl)phenyl)-2-(5-cyclopropyl-2-(2,3-dihydrobenzofuran-5-yl)-7-oxo-[ 1,2,4]triazolo[1,5-a]pyrimidin-4(7H)-yl)acetamide

At room temperature, 2-(2-bromo-5-cyclopropyl-7-oxo-[1,2,4]triazolo[1,5-a]pyrimidin-4(7H)-yl)-N-(2-chloro-4-(trifluo romethyl)phenyl)acetamide **152b** (360 mg, 733.70 µmol), (2,3-dihydrobenzofuran-5-yl)boronic acid **82d** (144.36 mg, 880.44 µmol), [1,1'-bis(diphenylphosphino)ferrocene]palladium(II) dichloride (59.47 mg, 73.37 µmol), and sodium carbonate (233.30 mg, 2.20 mmol) were dissolved in 1,4-dioxane (10 mL) and water (1 mL). The mixture was subject to argon replacement three times and was heated to 77 °C for reaction for 5 hours. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain N-(2-chloro-4-(trifluoromethyl)phenyl)-2-(5-cyclopropyl-2-(2,3-dihydrobenzofuran-5-yl)-7-oxo-[ 1,2,4]triazolo[1,5-a]pyrimidin-4(7H)-yl)acetamide **152c** (388 mg) in a yield of 99.80%.

MS m/z (ESI): 530.0 [M+1]

### Step 3

### 2-(6-bromo-5-cyclopropyl-2-(2,3-dihydrobenzofuran-5-yl)-7-oxo-[1,2,4]triazolo[1,5-a]pyrimidin -4(7H)-yl)-N-(2-chloro-4-(trifluoromethyl)phenyl)acetamide

At room temperature, N-bromosuccinimide (136.23 mg, 765.43 µmol) was added to a solution of N-(2-chloro-4-(trifluoromethyl)phenyl)-2-(5-cyclopropyl-2-(2,3-dihydrobenzofuran-5-yl)-7-oxo-[ 1,2,4]triazolo[1,5-a]pyrimidin-4(7H)-yl)acetamide **152c** (338 mg, 637.86 µmol) in N,N-dimethylformamide (10 mL), and the reaction was performed at room temperature for 18 hours. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System **B**) to obtain 2-(6-bromo-5-cyclopropyl-2-(2,3-dihydrobenzofuran-5-yl)-7-oxo-[1,2,4]triazolo[1,5-a]pyrimidin -4(7H)-yl)-N-(2-chloro-4-(trifluoromethyl)phenyl)acetamide **152d** (388 mg) in a yield of 99.92%.

MS m/z (ESI): 608.0 [M+1]

### Step 4

### N-(2-chloro-4-(trifluoromethyl)phenyl)-2-(5-cyclopropyl-2-(2,3-dihydrobenzofuran-5-yl)-7-oxo-6-(piperazin-1-yl)-[1,2,4]triazolo[1,5-a]pyrimidin-4(7H)-yl)acetamide

At room temperature, 2-(6-bromo-5-cyclopropyl-2-(2,3-dihydrobenzofuran-5-yl)-7-oxo-[1,2,4]triazolo[1,5-a]pyrimidin -4(7H)-yl)-N-(2-chloro-4-(trifluoromethyl)phenyl)acetamide **152d** (170 mg, 279.24 µmol), piperazine (288.63 mg, 3.35 mmol), silver tetrafluoroborate (59.80 mg, 307.16 µmol), and dimethyl sulfoxide (1.2 mL) were added to a 5 mL microwave tube. The tube was sealed and was heated to 140 °C for reaction for 6 hours. The mixture was concentrated under reduced pressure and then separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-cyclopropyl-2-(2,3-dihydrobenzofuran-5-yl)-7-oxo-6-(piperazin-1-yl)-[1,2,4]triazolo[1,5-a]pyrimidin-4(7H)-yl)acetamide **152e** (60 mg) in a yield of 34.99%.

MS m/z (ESI): 614.2 [M+1]

### Step 5

### 2-(6-(4-(5-(benzyloxy)-6-methylpyrimidine-4-carbonyl)piperazin-1-yl)-5-cyclopropyl-2-(2,3-dih ydrobenzofuran-5-yl)-7-oxo-[1,2,4]triazolo[1,5-a]pyrimidin-4(7H)-yl)-N-(2-chloro-4-(trifluorom ethyl)phenyl)acetamide

At room temperature, 5-(benzyloxy)-6-methylpyrimidine-4-carboxylic acid **1g** (35.80 mg, 146.58 µmol), 1-(3-dimethylaminopropyl)-3-ethylcarbodiimide hydrochloride (37.46 mg, 195.43 µmol), 1-hydroxybenzotriazole (26.41 mg, 195.43 µmol), and N,N-diisopropylethylamine (63.14 mg, 488.59 µmol) were dissolved in N,N-dimethylformamide (2 mL) and reacted at room temperature for 40 minutes, then a solution of N-(2-chloro-4-(trifluoromethyl)phenyl)-2-(5-cyclopropyl-2-(2,3-dihydrobenzofuran-5-yl)-7-oxo-6-(piperazin-1-yl)-[1,2,4]triazolo[1,5-a]pyrimidin-4(7H)-yl)acetamide **152e** (60 mg, 97.72 µmol) in N,N-dimethylformamide (2 mL) was added, and the reaction was performed at room temperature for 48 hours. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System **B**) to obtain 2-(6-(4-(5-(benzyloxy)-6-methylpyrimidine-4-carbonyl)piperazin-1-yl)-5-cyclopropyl-2-(2,3-dih ydrobenzofuran-5-yl)-7-oxo-[1,2,4]triazolo[1,5-a]pyrimidin-4(7H)-yl)-N-(2-chloro-4-(trifluorom ethyl)phenyl)acetamide **152f** (15 mg) in a yield of 18.27%.

MS m/z (ESI): 840.3 [M+1]

### Step 6

### N-(2-chloro-4-(trifluoromethyl)phenyl)-2-(5-cyclopropyl-2-(2,3-dihydrobenzofuran-5-yl)-6-(4-(5 -hydroxy-6-methylpyrimidine-4-carbonyl)piperazin-1-yl)-7-oxo-[1,2,4]triazolo[1,5-a]pyrimidin-4 (7H)-yl)acetamide

At room temperature, trifluoroacetic acid (2 mL) was added to a solution of 2-(6-(4-(5-(benzyloxy)-6-methylpyrimidine-4-carbonyl)piperazin-1-yl)-5-cyclopropyl-2-(2,3-dih ydrobenzofuran-5-yl)-7-oxo-[1,2,4]triazolo[1,5-a]pyrimidin-4(7H)-yl)-N-(2-chloro-4-(trifluorom ethyl)phenyl)acetamide **152f** (15 mg, 17.85 µmol) in dichloromethane (0.5 mL), and the reaction was performed at 30 °C for 24 hours. The mixture was concentrated under reduced pressure, then separated by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-cyclopropyl-2-(2,3-dihydrobenzofuran-5-yl)-6-(4-(5 -hydroxy-6-methylpyrimidine-4-carbonyl)piperazin-1-yl)-7-oxo-[1,2,4]triazolo[1,5-a]pyrimidin-4 (7H)-yl)acetamide **152** (1.16 mg) in a yield of 8.58%.

MS m/z (ESI): 750.2 [M+1]
¹H NMR (400 MHz, DMSO-d6) δ 10.42 (s, 1H), 10.38 - 9.83 (m, 1H), 8.59 (s, 1H), 8.03 (d, J = 8.6 Hz, 1H), 7.97 (t, J = 2.5 Hz, 2H), 7.87 (dd, J = 8.3, 1.8 Hz, 1H), 7.71 (dd, J = 8.7, 2.1 Hz, 1H), 6.87 (d, J = 8.3 Hz, 1H), 5.51 (s, 2H), 4.60 (t, J = 8.7 Hz, 2H), 4.38 (s, 1H), 3.46 (s, 3H), 3.27 (q, J = 12.8, 8.7 Hz, 4H), 2.90 (d, J = 10.0 Hz, 1H), 2.74 (d, J = 9.6 Hz, 1H), 2.45 (s, 3H), 1.80 (ddd, J = 8.5, 5.9, 2.6 Hz, 1H), 1.27 (tt, J = 10.7, 5.3 Hz, 2H), 1.01 (dd, J = 6.1, 2.3 Hz, 2H).

### Example 153

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(thieno[3,2-c]pyridin-2-yl)-[1,2,4]triazolo[1,5-a]pyrimidin-4(7H)-yl)a cetamide

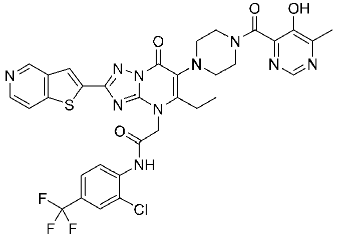

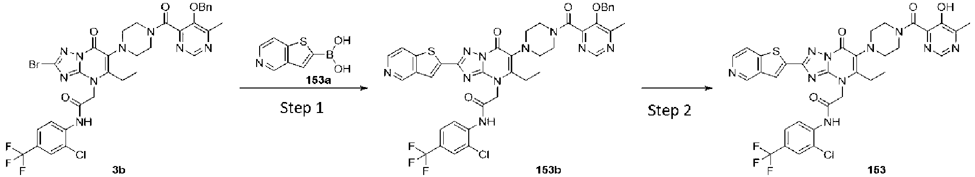

### Step 1

### 2-(6-(4-(5-(benzyloxy)-6-methylpyrimidine-4-carbonyl)piperazin-1-yl)-5-ethyl-7-oxo-2-(thieno[3 ,2-c]pyridin-2-yl)-[1,2,4]triazolo[1,5-a]pyrimidin-4(7H)-yl)-N-(2-chloro-4-(trifluoromethyl)phen yl)acetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (50 mg, 63.37 µmol), thieno[3,2-c]pyridin-2-ylboronic acid **153a** (13.61 mg, 76.05 µmol, commercially available), [1,1'-bis(diphenylphosphino)ferrocene]palladium(II) dichloride (5.14 mg, 6.34 µmol), and sodium carbonate (20.15 mg, 190.11 µmol) were dissolved in 1,4-dioxane (1 mL) and water (0.3 mL). The mixture was subject to argon replacement four times, then heated to 80 °C and reacted for 1.5 hours. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System **B**) to obtain 2-(6-(4-(5-(benzyloxy)-6-methylpyrimidine-4-carbonyl)piperazin-1-yl)-5-ethyl-7-oxo-2-(thieno[3 ,2-c]pyridin-2-yl)-[1,2,4]triazolo[1,5-a]pyrimidin-4(7H)-yl)-N-(2-chloro-4-(trifluoromethyl)phen yl)acetamide **153b** (40 mg) in a yield of 74.85%.

MS m/z (ESI): 843.0 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(thieno[3,2-c]pyridin-2-yl)-[1,2,4]triazolo[1,5-a]pyrimidin-4(7H)-yl)a cetamide

2-(6-(4-(5-(Benzyloxy)-6-methylpyrimidine-4-carbonyl)piperazin-1-yl)-5-ethyl-7-oxo-2 -(thieno[3,2-c]pyridin-2-yl)-[1,2,4]triazolo[1,5-a]pyrimidin-4(7H)-yl)-N-(2-chloro-4-(trifluorome thyl)henyl)acetamide **153b** (40 mg, 47.43 µmol) was added to dichloromethane (1.50 mL), trifluoroacetic acid (1.50 mL) was added, the temperature was raised to 40 °C, and the reaction was performed for 3 hours. The mixture was concentrated under reduced pressure, then separated by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(thieno[3,2-c]pyridin-2-yl)-[1,2,4]triazolo[1,5-a]pyrimidin-4(7H)-yl)a cetamide **153** (2.23 mg) in a yield of 6.18%.

MS m/z (ESI): 753.1 [M+1]
¹H NMR (400 MHz, DMSO) δ 10.42 (s, 1H), 10.23 (s, 1H), 9.32 (s, 1H), 8.57 (d, J = 13.9 Hz, 2H), 8.34 - 8.26 (m, 2H), 8.07 (d, J = 8.7 Hz, 1H), 7.98 (s, 1H), 7.72 (d, J = 8.3 Hz, 1H), 5.41 (s, 2H), 4.54 (d, J = 12.7 Hz, 1H), 3.53 (d, J = 11.9 Hz, 12H), 3.29 (d, J = 11.9 Hz, 3H), 3.04 (d, J = 10.8 Hz, 3H), 2.86 (d, J = 11.2 Hz, 1H), 2.69 (s, 1H), 2.45 (s, 3H), 1.22 (t, J = 7.4 Hz, 3H).

### Example 154

### N-(2-chloro-4-(trifluoromethyl)phenyl)-2-(2-(2,3-dihydrobenzofuran-7-yl)-5-ethyl-6-(4-(5-hydroxy-6-methylpyrimidine-4-carbonyl)piperazin-1-yl)-7-oxo-[1,2,4]triazolo[1,5-a]pyrimidin -4(7H)-yl)acetamide

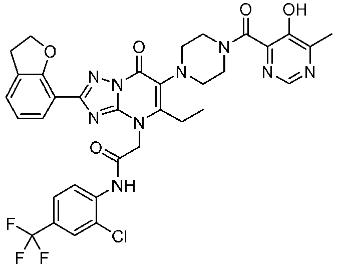

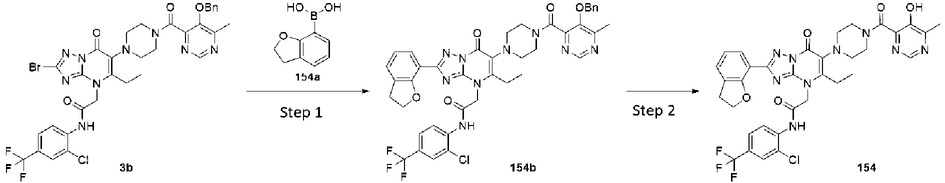

### Step 1

### 2-(6-(4-(5-(benzyloxy)-6-methylpyrimidine-4-carbonyl)piperazin-1-yl)-2-(2,3-dihydrobenzofuran -7-yl)-5-ethyl-7-oxo-[1,2,4]triazolo[1,5-a]pyrimidin-4(7H)-yl)-N-(2-chloro-4-(trifluoromethyl)ph enyl)acetamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (50 mg, 63.37 µmol), (2,3-dihydrobenzofuran-7-yl)boronic acid **154a** (12.47 mg, 76.05 µmol, commercially available), [1,1'-bis(diphenylphosphino)ferrocene]palladium(II) dichloride (5.14 mg, 6.34 µmol), and sodium carbonate (20.15 mg, 190.11 µmol) were dissolved in 1,4-dioxane (1 mL) and water (0.3 mL). The mixture was subject to argon replacement four times, then heated to 80 °C and reacted for 1.5 hours. Water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System **B**) to obtain 2-(6-(4-(5-(benzyloxy)-6-methylpyrimidine-4-carbonyl)piperazin-1-yl)-2-(2,3-dihydrobenzofuran -7-yl)-5-ethyl-7-oxo-[1,2,4]triazolo[1,5-a]pyrimidin-4(7H)-yl)-N-(2-chloro-4-(trifluoromethyl)ph enyl)acetamide **154b** (35 mg) with a yield of 66.68%.

MS m/z(ESI): 828.2 [M+1]

### Step 2

### N-(2-chloro-4-(trifluoromethyl)phenyl)-2-(2-(2,3-dihydrobenzofuran-7-yl)-5-ethyl-6-(4-(5-hydro xy-6-methylpyrimidine-4-carbonyl)piperazin-1-yl)-7-oxo-[1,2,4]triazolo[1,5-a]pyrimidin-4(7H)-yl)acetamide

2-(6-(4-(5-(benzyloxy)-6-methylpyrimidine-4-carbonyl)piperazin-1-yl)-2-(2,3-dihydrob enzofuran-7-yl)-5-ethyl-7-oxo-[1,2,4]triazolo[1,5-a]pyrimidin-4(7H)-yl)-N-(2-chloro-4-(trifluoro methyl)phenyl)acetamide **154b** (35 mg, 42.26 µmol) was added to methanol (1 mL). Palladium on carbon (899.43 µg, 8.45 µmol, 10%) was added, and the mixture was reacted at room temperature under a hydrogen atmosphere for 18 hours. The reaction mixture was filtered and concentrated under reduced pressure, then separated by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(2-(2,3-dihydrobenzofuran-7-yl)-5-ethyl-6-(4-(5-hydro xy-6-methylpyrimidine-4-carbonyl)piperazin-1-yl)-7-oxo-[1,2,4]triazolo[1,5-a]pyrimidin-4(7H)-yl)acetamide **154** (5.61 mg) with a yield of 16.91%.

MS m/z(ESI): 738.3 [M+1]
¹H NMR (400 MHz, DMSO) δ 10.39 (s, 1H), 10.23 (s, 1H), 8.58 (s, 1H), 8.06 (d, J = 8.6 Hz, 1H), 7.96 (d, J = 2.1 Hz, 1H), 7.79 - 7.68 (m, 2H), 7.39 - 7.32 (m, 1H), 6.93 (t, J = 7.6 Hz, 1H), 5.37 (s, 2H), 4.64 (t, J = 8.8 Hz, 2H), 4.54 (d, J = 12.3 Hz, 1H), 3.53 (q, J = 11.9 Hz, 3H), 3.26 (q, J = 10.0 Hz, 3H), 3.02 (d, J = 8.5 Hz, 3H), 2.84 (d, J = 11.2 Hz, 1H), 2.67 (s, 1H), 2.45 (s, 3H), 1.21 (t, J = 7.5 Hz, 3H).

### Example 155

### N-(2-chloro-4-(trifluoromethyl)phenyl)-2-(5-cyclobutyl-2-(2,3-dihydrobenzofuran-5-yl)-6-(4-(5-hydroxy-6-methylpyrimidine-4-carbonyl)piperazin-1-yl)-7-oxo-[1,2,4]triazolo[1,5-a]pyrimidin-4( 7H)-yl)acetamide

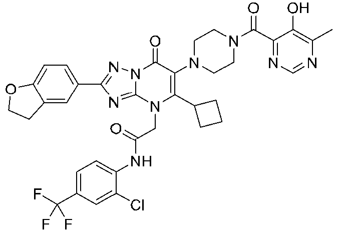

### Step 1

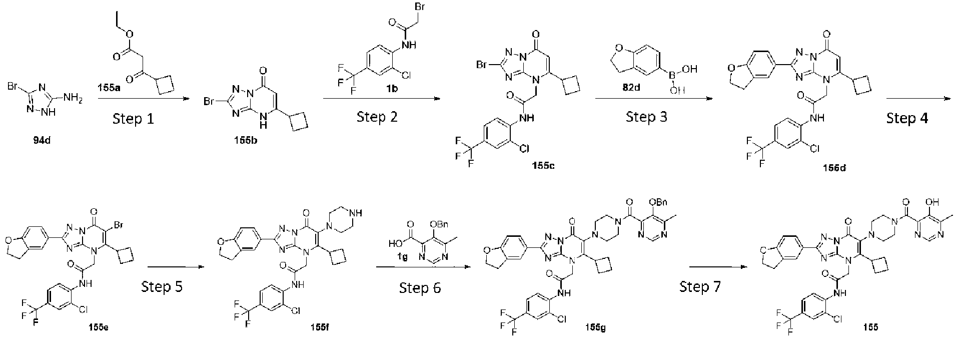

### 2-bromo-5-cyclobutyl-[1,2,4]triazolo[1,5-a]pyrimidin-7(4H)-one

At room temperature, 3-bromo-1H-1,2,4-triazol-5-amine **94d** (1.6 g, 9.82 mmol), ethyl 3-cyclobutyl-3-oxopropanoate **155a** (2.01 g, 11.78 mmol, commercially available), polyphosphoric acid (3.32 g, 9.82 mmol), and ethanol (20 mL) were added to a 15 mL microwave tube. The mixture was heated to 90 °C and reacted for 6 hours. The reaction mixture was concentrated under reduced pressure, and the resulting residue was purified by silica gel column chromatography (eluent: System **B)** to obtain 2-bromo-5-cyclobutyl-[1,2,4]triazolo[1,5-a]pyrimidin-7(4H)-one **155b** (1 g) with a yield of 37.85%.

MS m/z(ESI): 269.0 [M+1]

### Step 2

### 2-(2-bromo-5-cyclobutyl-7-oxo-[1,2,4]triazolo[1,5-a]pyrimidin-4(7H)-yl)-N-(2-chloro-4-(trifluor omethyl)phenyl)acetamide

2-Bromo-5-cyclobutyl-[1,2,4]triazolo[1,5-a]pyrimidin-7(4H)-one **155b** (1 g, 3.72 mmol) and 2-bromo-N-(2-chloro-4-(trifluoromethyl)phenyl)acetamide **1b** (1.41 g, 4.46 mmol) were added to N,N-dimethylformamide (15 mL), N,N-diisopropylethylamine (1.44 g, 11.15 mmol) was added, the temperature was raised to 50 °C, and the reaction was performed for 1.5 hours. Water (20 mL) was added to the reaction mixture, extraction was performed with ethyl acetate (20 mL ×3), the combined organic phase was washed with saturated sodium chloride solution (20 mL), dried over anhydrous sodium sulfate, filtered, and concentrated under reduced pressure, and the resulting residue was purified by silica gel column chromatography (eluent: System **B**) to obtain 2-(2-bromo-5-cyclobutyl-7-oxo-[1,2,4]triazolo[1,5-a]pyrimidin-4(7H)-yl)-N-(2-chloro-4-(trifluor omethyl) phenyl)acetamide **155c** (1.5 g) with a yield of 79.98%.

MS m/z (ESI): 504.0 [M+1]

### Step 3

### N-(2-chloro-4-(trifluoromethyl)phenyl)-2-(5-cyclobutyl-2-(2,3-dihydrobenzofuran-5-yl)-7-oxo-[1 ,2,4]triazolo[1,5-a]pyrimidin-4(7H)-yl)acetamide

At room temperature, 2-(2-bromo-5-cyclobutyl-7-oxo-[1,2,4]triazolo[1,5-a]pyrimidin-4(7H)-yl)-N-(2-chloro-4-(trifluor omethyl)phenyl)acetamide **155c** (1.5 g, 2.97 mmol), (2,3-dihydrobenzofuran-5-yl)boronic acid **82d** (560.43 mg, 3.42 mmol), [1,1'-bis(diphenylphosphino)ferrocene]palladium dichloride (240.92 mg, 297.21 µmol), and sodium carbonate (945.05 mg, 8.92 mmol) were added to 1,4-dioxane (5 mL) and water (1.5 mL). The mixture was subject to argon replacement four times. The temperature was raised to 80 °C, and the reaction was performed for 1.5 hours. Water (20 mL) was added to the reaction mixture, extraction was performed with ethyl acetate (20 mL ×3), the combined organic phase was washed with saturated sodium chloride solution (20 mL), dried over anhydrous sodium sulfate, filtered, and concentrated under reduced pressure, and the resulting residue was purified by silica gel column chromatography (eluent: System **B**) to obtain N-(2-chloro-4-(trifluoromethyl)phenyl)-2-(5-cyclobutyl-2-(2,3-dihydrobenzofuran-5-yl)-7-oxo-[1 ,2,4]triazolo[1,5-a]pyrimidin-4(7H)-yl)acetamide **155d** (1 g) with a yield of 61.86%.

MS m/z (ESI): 544.0 [M+1]

### Step 4

### 2-(6-Bromo-5-cyclobutyl-2-(2,3-dihydrobenzofuran-5-yl)-7-oxo-[1,2,4]triazolo[1,5-a]pyrimidin-4(7H)-yl)-N-(2-chloro-4-(trifluoromethyl)phenyl)acetamide

At room temperature, N-bromosuccinimide (392.66 mg, 2.21 mmol) was added to a solution of N-(2-chloro-4-(trifluoromethyl)phenyl)-2-(5-cyclobutyl-2-(2,3-dihydrobenzofuran-5-yl)-7-oxo-[1 ,2,4]triazolo[1,5-a]pyrimidin-4(7H)-yl)acetamide **155d** (1 g, 1.84 mmol) in N,N-dimethylformamide (10 mL), and the reaction was performed at room temperature for 18 hours. Water (20 mL) was added to the reaction mixture, extraction was performed with ethyl acetate (20 mL ×3), the combined organic phase was washed with saturated sodium chloride solution (20 mL), dried over anhydrous sodium sulfate, filtered, and concentrated under reduced pressure, and the resulting residue was purified by silica gel column chromatography (eluent: System **A**) to obtain 2-(6-bromo-5-cyclobutyl-2-(2,3-dihydrobenzofuran-5-yl)-7-oxo-[1,2,4]triazolo[1,5-a]pyrimidin-4 (7H)-yl)-N-(2-chloro-4-(trifluoromethyl)phenyl)acetamide **155e** (200 mg) with a yield of 17.47%.

MS m/z (ESI): 622.1 [M+1]

### Step 5

### N-(2-chloro-4-(trifluoromethyl)phenyl)-2-(5-cyclobutyl-2-(2,3-dihydrobenzofuran-5-yl)-7-oxo-6-(piperazin-1-yl)-[1,2,4]triazolo[1,5-a]pyrimidin-4(7H)-yl)acetamide

At room temperature, 2-(6-bromo-5-cyclobutyl-2-(2,3-dihydrobenzofuran-5-yl)-7-oxo-[1,2,4]triazolo[1,5-a]pyrimidin-4 (7H)-yl)-N-(2-chloro-4-(trifluoromethyl)phenyl)acetamide **155e** (200 mg, 321.12 µmol), piperazine (331.92 mg, 3.85 mmol), and silver tetrafluoroborate (68.76 mg, 353.23 µmol) were added to dimethyl sulfoxide (1.2 mL), the temperature was raised to 140 °C, and the reaction was performed for 6 hours. The reaction mixture was separated directly by preparative liquid chromatography (separation column AKZONOBEL Kromasil; 250×21.2 mm I.D.; 5 µm, 20 mL/min; mobile phase A: 0.05% TFA+H2O, mobile phase B: CH3CN) to obtain N-(2-chloro-4-(trifluoromethyl)phenyl)-2-(5-cyclobutyl-2-(2,3-dihydrobenzofuran-5-yl)-7-oxo-6-(piperazin-1-yl)-[1,2,4]triazolo[1,5-a]pyrimidin-4(7H)-yl)acetamide **155f** (30 mg) with a yield of 14.88%.

MS m/z (ESI): 628.3 [M+1]

### Step 6

### 2-(6-(4-(5-(Benzyloxy)-6-methylpyrimidine-4-carbonyl)piperazin-1-yl)-5-cyclobutyl-2-(2,3-dihy drobenzofuran-5-yl)-7-oxo-[1,2,4]triazolo[1,5-a]pyrimidin-4(7H)-yl)-N-(2-chloro-4-(trifluoromet hyl)phenyl)acetamide

At room temperature, N-(2-chloro-4-(trifluoromethyl)phenyl)-2-(5-cyclobutyl-2-(2,3-dihydrobenzofuran-5-yl)-7-oxo-6-(piperazin-1-yl)-[1,2,4]triazolo[1,5-a]pyrimidin-4(7H)-yl)acetamide **155f** (30 mg, 47.77 µmol), 5-(benzyloxy)-6-methylpyrimidine-4-carboxylic acid **1g** (15.17 mg, 62.10 µmol), 1-(3-dimethylaminopropyl)-3-ethylcarbodiimide hydrochloride (13.74 mg, 71.65 µmol), 1-hydroxybenzotriazole (9.68 mg, 71.65 µmol), and N,N-diisopropylethylamine (30.87 mg, 238.84 µmol) were dissolved in N,N-dimethylformamide (2 mL), and the reaction was performed at room temperature for 18 hours. Water (20 mL) was added to the reaction mixture, extraction was performed with ethyl acetate (20 mL ×3), the combined organic phase was washed with saturated sodium chloride solution (20 mL), dried over anhydrous sodium sulfate, filtered, and concentrated under reduced pressure, and the resulting residue was purified by silica gel column chromatography (eluent: System **B**) to obtain 2-(6-(4-(5-(benzyloxy)-6-methylpyrimidine-4-carbonyl)piperazin-1-yl)-5-cyclobutyl-2-(2,3-dihy drobenzofuran-5-yl)-7-oxo-[1,2,4]triazolo [1,5-a]pyrimidin-4(7H)-yl)-N-(2-chloro-4-(trifluoromethyl)phenyl)acetamide **155g** (30 mg) with a yield of 73.52%.

MS m/z (ESI): 854.2 [M+1]

### Step 7

### N-(2-chloro-4-(trifluoromethyl)phenyl)-2-(5-cyclobutyl-2-(2,3-dihydrobenzofuran-5-yl)-6-(4-(5-hydroxy-6-methylpyrimidine-4-carbonyl)piperazin-1-yl)-7-oxo-[1,2,4]triazolo[1,5-a]pyrimidin-4( 7H)-yl)acetamide

2-(6-(4-(5-(Benzyloxy)-6-methylpyrimidine-4-carbonyl)piperazin-1-yl)-5-cyclobutyl-2-(2,3-dihydrobenzofuran-5-yl)-7-oxo-[1,2,4]triazolo[1,5-a]pyrimidin-4(7H)-yl)-N-(2-chloro-4-(trif luoromethyl)phenyl)acetamide **155g** (30 mg, 35.70 µmol) was added to trifluoroacetic acid (2.5 mL) and reacted at room temperature for 18 hours. The reaction mixture was quenched with methanol, concentrated under reduced pressure, and then separated by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(5-cyclobutyl-2-(2,3-dihydrobenzofuran-5-yl)-6-(4-(5-hydroxy-6-methylpyrimidine-4-carbonyl)piperazin-1-yl)-7-oxo-[1,2,4]triazolo[1,5-a]pyrimidin-4( 7H)-yl)acetamide **155** (2.08 mg) in a yield of 7.60%.

MS m/z (ESI): 764.2 [M+1]
¹H NMR (400 MHz, DMSO) δ 10.35 (s, 1H), 10.23 (s, 1H), 8.58 (s, 1H), 8.04 (d, J = 8.5 Hz, 1H), 7.97 (s, 2H), 7.91 - 7.84 (m, 1H), 7.74 - 7.67 (m, 1H), 6.87 (d, J = 8.4 Hz, 1H), 5.37 (s, 2H), 4.60 (t, J = 8.7 Hz, 2H), 4.50 (d, J = 12.8 Hz, 1H), 3.80 (t, J = 8.8 Hz, 1H), 3.61 (d, J = 10.4 Hz, 3H), 3.25 (t, J = 8.8 Hz, 3H), 3.07 (t, J = 11.5 Hz, 2H), 2.88 (d, J = 11.2 Hz, 1H), 2.74 - 2.59 (m, 3H), 2.45 (s, 3H), 2.02 - 1.86 (m, 3H).

### Example 156

### N-(2-chloro-4-(trifluoromethyl)phenyl)-2-(5-ethyl-6-(4-(5-hydroxy-6-methylpyrimidine-4-carbon yl)piperazin-1-yl)-2-(1H-indazol-6-yl)-7-oxo-[1,2,4]triazolo[1,5-a]pyrimidin-4(7H)-yl)acetamide

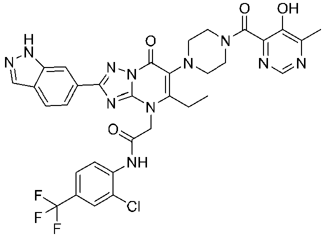

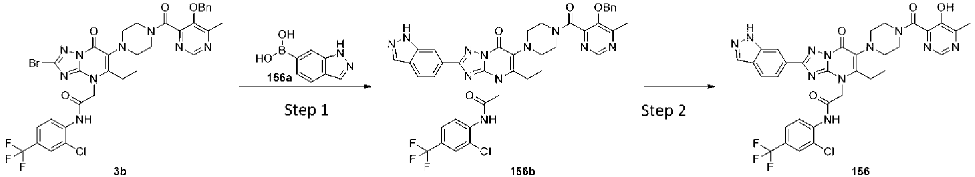

### Step 1

### 2-(6-(4-(5-(Benzyloxy)-6-methylpyrimidine-4-carbonyl)piperazin-1-yl)-5-ethyl-2-(1H-indazol-6-yl)-7-oxo-[1,2,4]triazolo[1,5-a]pyrimidin-4(7H)-yl)-N-(2-chloro-4-(trifluoromethyl)phenyl)aceta mide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (60 mg, 76.05 µmol), (1H-indazol-6-yl)boronic acid **156a** (14.78 mg, 91.25 µmol), [1,1'-bis(diphenylphosphino)ferrocene]palladium(II) dichloride dichloromethane complex (6.16 mg, 7.60 µmol), and sodium carbonate (24.18 mg, 228.14 µmol) were added to 1,4-dioxane (1 mL) and water (0.3 mL). The mixture was subject to argon replacement four times, heated to 80 °C, and reacted for 1.5 hours. The reaction mixture was extracted with ethyl acetate (30 mL ×2), the aqueous layer was separated, and the combined organic phase was washed with saturated sodium chloride solution (30 mL ×2), dried over anhydrous sodium sulfate, filtered, and concentrated under reduced pressure. The obtained residue was purified by silica gel column chromatography (eluent: System B) to obtain 2-(6-(4-(5-(benzyloxy)-6-methylpyrimidine-4-carbonyl)piperazin-1-yl)-5-ethyl-2-(1H-indazol-6-yl)-7-oxo-[1,2,4]triazolo [1,5-a]pyrimidin-4(7H)-yl)-N-(2-chloro-4-(trifluoromethyl)phenyl)acetamide **156b** (33 mg) in a yield of 52.52%.

MS m/z: [M+1] 826.3

### Step 2

### N-(2-chloro-4-(trifluoromethyl)phenyl)-2-(5-ethyl-6-(4-(5-hydroxy-6-methylpyrimidine-4-carbon yl)piperazin-1-yl)-2-(1H-indazol-6-yl)-7-oxo-[1,2,4]triazolo[1,5-a]pyrimidin-4(7H)-yl)acetamide

2-(6-(4-(5-(Benzyloxy)-6-methylpyrimidine-4-carbonyl)piperazin-1-yl)-5-ethyl-2-(1H-i ndazol-6-yl)-7-oxo-[1,2,4]triazolo[1,5-a]pyrimidin-4(7H)-yl)-N-(2-chloro-4-(trifluoromethyl) phenyl)acetamide **156b** (33 mg, 39.94 µmol) was added to dichloromethane (2.5 mL). Boron trichloride (0.8 mL, 1.0 M in hexane) was added at 0 °C, and the reaction was performed at 0 °C for 1 hour. After the reaction was complete, methanol was added to quench the reaction, and the mixture was concentrated under reduced pressure. The obtained residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-2-(1H-indazol-6-yl)-7-oxo-[1,2,4]triazolo[1,5-a]pyrimidin-4(7H)-yl)acetamide 156 (5.4 mg) in a yield of 6.17%.

MS m/z: 736.2 [M+1]
¹H NMR (400 MHz, DMSO) δ 13.28 (s, 1H), 10.42 (s, 1H), 10.23 (s, 1H), 8.59 (s, 1H), 8.27 (s, 1H), 8.15 (s, 1H), 8.07 (d, J = 8.6 Hz, 1H), 7.97 (d, J = 2.1 Hz, 1H), 7.89 (s, 2H), 7.74-7.66 (m, 1H), 5.43 (s, 2H), 4.55 (d, J = 12.5 Hz, 1H), 3.28 (t, J = 12.9 Hz, 1H), 3.04 (d, J = 10.2 Hz, 3H), 2.86 (d, J = 11.4 Hz, 1H), 2.69 (s, 1H), 2.45 (s, 3H), 1.22 (t, J = 7.4 Hz, 3H).

### Example 157

### N-(2-chloro-4-(trifluoromethyl)phenyl)-2-(2-(3,4-dihydro-2H-benzo[b][1,4]oxazin-6-yl)-5-ethyl-6-(4-(5-hydroxy-6-methylpyrimidine-4-carbonyl)piperazin-1-yl)-7-oxo-[1,2,4]triazolo[1,5-a]pyri midin-4(7H)-yl)acetamide

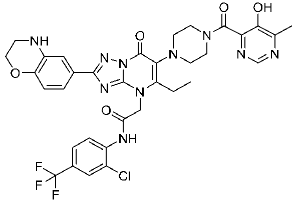

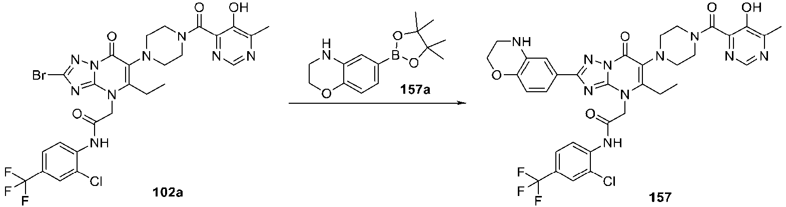

At room temperature, 2-(2-bromo-5-ethyl-6-(4-(5-hydroxy-6-methylpyrimidine-4-carbonyl)piperazin-1-yl)-7-oxo-[1,2, 4]triazolo[1,5-a]pyrimidin-4(7H)-yl)-N-(2-chloro-4-(trifluoromethyl)phenyl)acetamide **102a** (50 mg, 71.54 µmol), 6-(4,4,5,5-tetramethyl-1,3,2-dioxaborolan-2-yl)-3,4-dihydro-2H-benzo[b][1,4]oxazine **157a** (22.42 mg, 85.85 µmol, commercially available), [1,1'-bis(diphenylphosphino)ferrocene]palladium(II) dichloride dichloromethane complex (5.80 mg, 7.15 µmol), and sodium carbonate (22.75 mg, 214.63 µmol) were added to 1,4-dioxane (10 mL). The mixture was subject to argon replacement four times, then heated to 80 °C and reacted for 1.5 hours. The reaction mixture was extracted with ethyl acetate (40 mL × 3), and the organic phase was washed with saturated sodium chloride solution (20 mL), dried over anhydrous sodium sulfate, filtered, and concentrated under reduced pressure. The resulting residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(2-(3,4-dihydro-2H-benzo[b][1,4]oxazin-6-yl)-5-ethyl-6-(4-(5-hydroxy-6-methylpyrimidine-4-carbonyl)piperazin-1-yl)-7-oxo-[1,2,4]triazolo[1,5-a]pyri midin-4(7H)-yl)acetamide **157** (7.71 mg) with a yield of 6.97%.

MS m/z (ESI): 753.2 [M+1]
¹H NMR (400 MHz, DMSO) δ 10.35 (s, 1H), 10.21 (s, 1H), 8.58 (s, 1H), 8.06 (d, J = 8.6 Hz, 1H), 7.95 (s, 1H), 7.71 (d, J = 8.8 Hz, 1H), 7.38 (d, J = 2.1 Hz, 1H), 7.25 (dd, J = 8.2, 2.1 Hz, 1H), 6.73 (d, J = 8.3 Hz, 1H), 5.36 (s, 2H), 4.53 (d, J = 12.7 Hz, 1H), 4.17 (t, J = 4.2 Hz, 2H), 3.53 (d, J = 8.8 Hz, 3H), 3.31 (t, J = 4.4 Hz, 3H), 3.00 (d, J = 8.7 Hz, 3H), 2.84 (d, J = 11.3 Hz, 1H), 2.64 (s, 1H), 2.45 (s, 3H), 1.21 (t, J = 7.4 Hz, 3H).

### Example 158

### 2-(5-ethyl-6-(4-(5-hydroxy-6-methylpyrimidine-4-carbonyl)piperazin-1-yl)-7-oxo-2-(2,5,6,7-tetra hydrooxepin-3-yl)-[1,2,4]triazolo[1,5-a]pyrimidin-4(7H)-yl)-N-(2-methyl-4-(pentafluoro-λ⁶-sulfa neyl)phenyl)acetamide

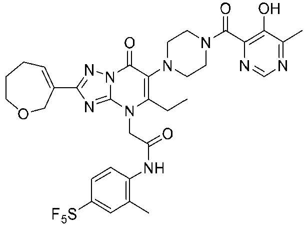

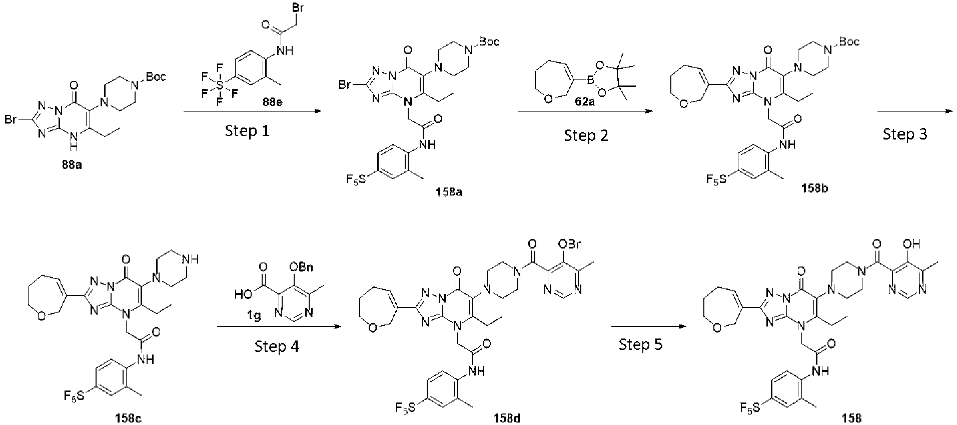

### Step 1

### tert-butyl 4-(2-bromo-5-ethyl-4-(2-((2-methyl-4-(pentafluoro-λ⁶-sulfaneyl)phenyl)amino)-2-oxoethyl)-7-ox o-4,7-dihydro-[1,2,4]triazolo[1,5-a]pyrimidin-6-yl)piperazine-1-carboxylate

Tert-butyl 4-(2-bromo-5-ethyl-7-oxo-4,7-dihydro-[1,2,4]triazolo[1,5-a]pyrimidin-6-yl)piperazine-1-carboxy late **88a** (0.3 g, 702.09 µmol) was dissolved in N,N-dimethylformamide (4 mL). Subsequently, 2-bromo-N-(2-methyl-4-(pentafluoro-λ⁶-sulfaneyl)phenyl)acetamide **88e** (298.36 mg, 842.51 µmol) and N,N-diisopropylethylamine (181.48 mg, 1.40 mmol) were added. The mixture was heated to 50 °C and reacted for 2 hours. Water was added for dilution (50 mL), and the mixture was extracted with dichloromethane (50 mL × 3). The combined organic phase was washed with saturated sodium chloride solution (25 mL × 3), dried over anhydrous sodium sulfate, filtered, and concentrated. The residue was purified by silica gel column chromatography (eluent: System A) to obtain tert-butyl 4-(2-bromo-5-ethyl-4-(2-((2-methyl-4-(pentafluoro-λ⁶-sulfaneyl)phenyl)amino)-2-oxoethyl)-7-ox o-4,7-dihydro-[1,2,4]triazolo[1,5-a]pyrimidin-6-yl)piperazine-1-carboxylate **158a** (0.35 g) with a yield of 71.16%.

MS m/z (ESI): 644.1 [M+H-56]

### Step 2

### tert-butyl 4-(5-ethyl-4-(2-((2-methyl-4-(pentafluoro-λ⁶-sulfaneyl)phenyl)amino)-2-oxoethyl)-7-oxo-2-(2,5, 6,7-tetrahydrooxepin-3-yl)-4,7-dihydro-[1,2,4]triazolo[1,5-a]pyrimidin-6-yl)piperazine-1-carbox ylate

4,4,5,5-Tetramethyl-2-(2,5,6,7-tetrahydrooxepin-3-yl)-1,3,2-dioxaborolane **62a** (71.98 mg, 321.19 µmol) and tert-butyl 4-(2-bromo-5-ethyl-4-(2-((2-methyl-4-(pentafluoro-λ⁶-sulfaneyl)phenyl)amino)-2-oxoethyl)-7-ox o-4,7-dihydro-[1,2,4]triazolo[1,5-a]pyrimidin-6-yl)piperazine-1-carboxylate **158a** (150 mg, 214.13 µmol) were dissolved in 1,4-dioxane (3 mL) and water (1 mL). Subsequently, [1,1'-bis(diphenylphosphino)ferrocene]palladium(II) dichloride (15.88 mg, 21.41 µmol) and sodium carbonate (45.39 mg, 428.25 µmol) were added. After argon replacement, the mixture was heated to 80 °C and reacted for 2 hours. Water (50 mL) was added for dilution, and the mixture was extracted with dichloromethane (50 mL × 3). The combined organic phase was washed with saturated sodium chloride solution (25 mL × 3), dried over anhydrous sodium sulfate, filtered, and concentrated. The residue was purified by silica gel column chromatography (eluent: System A) to obtain tert-butyl 4-(5-ethyl-4-(2-((2-methyl-4-(pentafluoro-λ⁶-sulfaneyl)phenyl)amino)-2-oxoethyl)-7-oxo-2-(2,5, 6,7-tetrahydrooxepin-3-yl)-4,7-dihydro-[1,2,4]triazolo[1,5-a]pyrimidin-6-yl)piperazine-1-carbox ylate **158b** (80 mg) with a yield of 52.05%.

MS m/z (ESI): 662.0 [M+H-56]

### Step 3

### 2-(5-ethyl-7-oxo-6-(piperazin-1-yl)-2-(2,5,6,7-tetrahydrooxepin-3-yl)-[1,2,4]triazolo[1,5-a]pyrim idin-4(7H)-yl)-N-(2-methyl-4-(pentafluoro-λ⁶-sulfaneyl)phenyl)acetamide

Tert-butyl 4-(5-ethyl-4-(2-((2-methyl-4-(pentafluoro-λ⁶-sulfaneyl)phenyl)amino)-2-oxoethyl)-7-oxo-2-(2,5, 6,7-tetrahydrooxepin-3-yl)-4,7-dihydro-[1,2,4]triazolo[1,5-a]pyrimidin-6-yl)piperazine-1-carbox ylate **158b** (80 mg, 111.46 µmol) was dissolved in dichloromethane (3 mL). Trifluoroacetic acid (0.5 g, 4.39 mmol) was added dropwise, and the reaction was performed at room temperature for 3 hours. After completion of the reaction, the mixture was concentrated under reduced pressure to obtain 2-(5-ethyl-7-oxo-6-(piperazin-1-yl)-2-(2,5,6,7-tetrahydrooxepin-3-yl)-[1,2,4]triazolo[1,5-a]pyrim idin-4(7H)-yl)-N-(2-methyl-4-(pentafluoro-λ⁶-sulfaneyl)phenyl) acetamide **158c** (60 mg) as a crude product, which was used directly in the next step.

MS m/z (ESI): 618.2 [M+H]

### Step 4

### 2-(6-(4-(5-(benzyloxy)-6-methylpyrimidine-4-carbonyl)piperazin-1-yl)-5-ethyl-7-oxo-2-(2,5,6,7-t etrahydrooxepin-3-yl)-[1,2,4]triazolo[1,5-a]pyrimidin-4(7H)-yl)-N-(2-methyl-4-(pentafluoro-λ⁶-s ulfaneyl)phenyl)acetamide

5-(Benzyloxy)-6-methylpyrimidine-4-carboxylic acid **1g** (37.96 mg, 155.43 umol) was dissolved in N,N-dimethylformamide (2 mL), followed by addition of 1-(3-dimethylaminopropyl)-3-ethylcarbodiimide hydrochloride (37.25 mg, 194.29 umol), 1-hydroxybenzotriazole (26.25 mg, 194.29 umol) and N,N-diisopropylethylamine (62.78 mg, 485.73 umol), and the reaction was performed at room temperature for 10 minutes, then 2-(5-ethyl-7-oxo-6-(piperazin-1-yl)-2-(2,5,6,7-tetrahydrooxepin-3-yl)-[1,2,4]triazolo[1,5-a]pyrim idin-4(7H)-yl)-N-(2-methyl-4-(pentafluoro-λ⁶ -sulfaneyl)phenyl)acetamide **158c** (60 mg, 97.15 umol) was added, and the reaction was performed at room temperature for 18 hours. Water (50 mL) was added to the reaction mixture to dilute, extracted with dichloromethane (50 mL ×3), the combined organic phase was washed with saturated sodium chloride solution, dried over anhydrous sodium sulfate, filtered and concentrated, and the residue was purified by silica gel column chromatography (eluent: System B) to obtain 2-(6-(4-(5-(benzyloxy)-6-methylpyrimidine-4-carbonyl)piperazin-1-yl)-5-ethyl-7-oxo-2-(2,5,6,7-t etrahydrooxepin-3-yl)-[1,2,4]triazolo[1,5-a]pyrimidin-4(7H)-yl)-N-(2-methyl-4-(pentafluoro-λ⁶-s ulfaneyl)phenyl)acetamide **158d** (50 mg) in a yield of 60.99%.

MS m/z (ESI): 844.3 [M+H]

### Step 5

### 2-(5-ethyl-6-(4-(5-hydroxy-6-methylpyrimidine-4-carbonyl)piperazin-1-yl)-7-oxo-2-(2,5,6,7-tetra hydrooxepin-3-yl)-[1,2,4]triazolo[1,5-a]pyrimidin-4(7H)-yl)-N-(2-methyl-4-(pentafluoro-λ⁶-sulfa neyl)phenyl)acetamide

2-(6-(4-(5-(Benzyloxy)-6-methylpyrimidine-4-carbonyl)piperazin-1-yl)-5-ethyl-7-oxo-2 -(2,5,6,7-tetrahydrooxepin-3-yl)-[1,2,4]triazolo[1,5-a]pyrimidin-4(7H)-yl)-N-(2-methyl-4-(pentaf luoro-λ⁶-sulfaneyl)phenyl)acetamide **158d** (50 mg, 59.25 umol) was dissolved in trifluoroacetic acid (3 mL), heated to 40 °C for 3 hours. After completion of the reaction, the mixture was concentrated under reduced pressure, and the resulting residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain 2-(5-ethyl-6-(4-(5-hydroxy-6-methylpyrimidine-4-carbonyl)piperazin-1-yl)-7-oxo-2-(2,5,6,7-tetra hydrooxepin-3-yl)-[1,2,4]triazolo[1,5-a]pyrimidin-4(7H)-yl)-N-(2-methyl-4-(pentafluoro-λ⁶-sulfa neyl)phenyl) acetamide **158** (15 mg) in a yield of 30.23%.

MS m/z (ESI): 754.3 [M+H]
¹H NMR (400 MHz, DMSO-d6) δ 10.03 (s, 1H), 8.58 (s, 1H), 7.82 (d, J = 2.7 Hz, 1H), 7.79 - 7.67 (m, 2H), 7.05 (t, J = 5.8 Hz, 1H), 5.24 (s, 2H), 4.63 (s, 2H), 4.52 (d, J = 12.6 Hz, 1H), 3.84 (t, J = 5.7 Hz, 2H), 3.50 (d, J = 11.8 Hz, 4H), 3.28 (s, 5H), 2.99 (d, J = 7.7 Hz, 2H), 2.81 (d, J = 11.3 Hz, 1H), 2.63 (d, J = 11.1 Hz, 1H), 2.44 (s, 3H), 2.38 (s, 3H), 1.82 (p, J = 5.7 Hz, 2H), 1.19 (t, J = 7.4 Hz, 3H).

### Example 159

### N-(2-chloro-4-(trifluoromethyl)phenyl)-2-(5-ethyl-6-(4-(3-hydroxypicolinoyl)piperazin-1-yl)-7-o xo-2-(3,3a,4,6a-tetrahydro-1H-cyclopenta[c]furan-5-yl)-[1,2,4]triazolo[1,5-a]pyrimidin-4(7H)-yl) acetamide

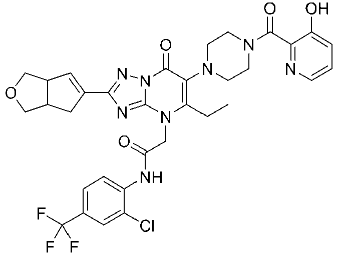

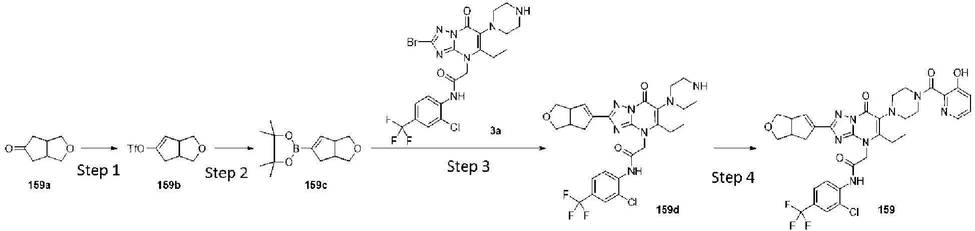

### Step 1

### 3,3a,4,6a-tetrahydro-1H-cyclopenta[c]furan-5-yl trifluoromethanesulfonate

Tetrahydro-1H-cyclopenta[c]furan-5(3H)-one **159a** (1.79 g, 14.19 mmol, prepared according to published patent CN115232057) was dissolved in tetrahydrofuran (30 mL), and lithium bis(trimethylsilyl)amide (1 M, 28.38 mL) was added dropwise at -78 C under nitrogen atmosphere, then stirred at -78 °C for 1 hour. N-phenylbis(trifluoromethanesulfonimide) (5.58 g, 15.61 mmol) was dissolved in tetrahydrofuran (50 mL), then added dropwise to the above reaction mixture at -78 °C. The reaction mixture was then stirred at 25 °C for 16 hours. After completion of the reaction, water (100 mL) was added to the reaction mixture, extracted with ethyl acetate (100 mL ×3), the combined organic phase was washed with saturated sodium chloride solution (200 mL ×3), dried over anhydrous sodium sulfate, filtered, concentrated under reduced pressure, and the resulting residue was purified by column chromatography (eluent: System A) to obtain 3,3a,4,6a-tetrahydro-1H-cyclopenta[c]furan-5-yl trifluoromethanesulfonate **159b** (3.2 g) in a yield of 87.34%.

¹H NMR (400 MHz, DMSO-d6) δ 5.75-5.70 (m, 1H), 3.73-3.68 (m, 1H), 3.63-3.57 (m, 1H), 3.54-3.49 (m, 3H), 2.97-2.77 (m, 2H), 2.41-2.32 (m, 1H).

### Step 2

### 4,4,5,5-tetramethyl-2-(3,3a,4,6a-tetrahydro-1H-cyclopenta[c]furan-5-yl)-1,3,2-dioxaborolane

3,3a,4,6a-Tetrahydro-1H-cyclopenta[c]furan-5-yl trifluoromethanesulfonate **159b** (500 mg, 1.94 mmol) was dissolved in 1,4-dioxane (15 mL), then potassium acetate (571.11 mg, 5.81 mmol), bis(pinacolato)diboron (737.58 mg, 2.90 mmol) and [1,1'-bis(diphenylphosphino)ferrocene]palladium(II) dichloride (143.64 mg, 193.64 umol) were added, and the reaction mixture was stirred at 90 °C for 2 hours under nitrogen atmosphere. The reaction mixture was used directly in the next step.

### Step 3

### N-(2-chloro-4-(trifluoromethyl)phenyl)-2-(5-ethyl-7-oxo-6-(piperazin-1-yl)-2-(3,3a,4,6a-tetrahyd ro-1H-cyclopenta[c]furan-5-yl)-[1,2,4]triazolo[1,5-a]pyrimidin-4(7H)-yl)acetamide

To the reaction mixture containing 4,4,5,5-tetramethyl-2-(3,3a,4,6a-tetrahydro-1H-cyclopenta[c]furan-5-yl)-1,3,2-dioxaborolane **159c** (1.94 mmol) prepared in the previous step was added 1,4-dioxane (9 mL) and water (0.9 mL), followed by addition of sodium carbonate (118.68 mg, 1.12 mmol), 2-(2-bromo-5-ethyl-7-oxo-6-(piperazin-1-yl)-[1,2,4]triazolo[1,5-a]pyrimidin-4(7H)-yl)-N-(2-chlo ro-4-(trifluoromethyl)phenyl)acetamide **3a** (300.32 mg, 533.64 µmol), and [1,1-bis(diphenylphosphino)ferrocene]palladium(II) dichloride (39.58 mg, 53.36 µmol). The mixture was stirred at 90 °C for 2 hours under nitrogen protection. After completion of the reaction, water (30 mL) was added to the reaction mixture, which was extracted with ethyl acetate (30 mL × 3). The combined organic phases were washed with saturated sodium chloride solution (30 mL × 3), dried over anhydrous sodium sulfate, filtered, and concentrated under reduced pressure. The obtained residue was purified by silica gel plate (eluent: System B) to obtain N-(2-chloro-4-(trifluoromethyl)phenyl)-2-(5-ethyl-7-oxo-6-(piperazin-1-yl)-2-(3,3a,4,6a-tetrahyd ro-1H-cyclopenta[c]furan-5-yl)-[1,2,4]triazolo[1,5-a]pyrimidin-4(7H)-yl)acetamide **159d** (80 mg) with a yield of 25.32%.

MS m/z (ESI): 592.2 [M+1]

### Step 4

### N-(2-chloro-4-(trifluoromethyl)phenyl)-2-(5-ethyl-6-(4-(3-hydroxypicolinoyl)piperazin-1-yl)-7-o xo-2-(3,3a,4,6a-tetrahydro-1H-cyclopenta[c]furan-5-yl)-[1,2,4]triazolo[1,5-a]pyrimidin-4(7H)-yl) acetamide

3-Hydroxypicolinic acid (10.57 mg, 76.01 µmol), 1-hydroxybenzotriazole (13.69 mg, 101.35 µmol), 1-(3-dimethylaminopropyl)-3-ethylcarbodiimide hydrochloride (19.43 mg, 101.35 µmol), and N,N-diisopropylethylamine (19.65 mg, 152.02 µmol) were dissolved in N,N-dimethylformamide (1.5 mL), and the mixture was stirred at 25 °C for 10 minutes. Then N-(2-chloro-4-(trifluoromethyl)phenyl)-2-(5-ethyl-7-oxo-6-(piperazin-1-yl)-2-(3,3a,4,6a-tetrahyd ro-1H-cyclopenta[c]furan-5-yl)-[1,2,4]triazolo[1,5-a]pyrimidin-4(7H)-yl)acetamide **159e** (30 mg, 50.67 µmol) was added, and the mixture was stirred at 30 °C for 16 hours. After completion of the reaction, the reaction mixture was purified by preparative liquid chromatography (Waters 3767/QDA column: SunFire Sunfire C₁₈, 19×250 mm, 10 µm; mobile phase A: 10 mmol/L, 0.1% FA/H₂O, B: acetonitrile; flow rate: 20 mL/min) to obtain N-(2-chloro-4-(trifluoromethyl)phenyl)-2-(5-ethyl-6-(4-(3-hydroxypicolinoyl)piperazin-1-yl)-7-o xo-2-(3,3a,4,6a-tetrahydro-1H-cyclopenta[c]furan-5-yl)-[1,2,4]triazolo[1,5-a]pyrimidin-4(7H)-yl) acetamide 159 (11.93 mg) with a yield of 33.01%.

MS m/z (ESI): 713.2 [M+1]
¹H NMR (400 MHz, DMSO-d6) δ 10.42 (s, 1H), 10.36 (s, 1H), 8.07-8.05 (m, 2H), 7.97-7.96 (m, 1H), 7.71 (d, J = 8.0 Hz, 1H), 7.30-7.29 (m, 2H), 6.45 (s, 1H), 5.31 (s, 2H), 4.54 (d, J = 12 Hz, 1H), 3.77-3.73 (m, 1H), 3.68-3.60 (m, 2H), 3.54-3.51 (m, 2H), 3.49-3.37 (m, 2H), 3.25-3.15 (m, 1H), 3.00-2.97 (m, 5H), 2.79 (d, J = 12.0 Hz, 1H), 2.63-2.57 (m, 3H), 1.18 (t, J = 8.0 Hz, 3H).

### Example 160

### N-(2-chloro-4-(trifluoromethyl)phenyl)-2-(5-ethyl-6-(4-(5-hydroxypyrimidine-4-carbonyl)pipera zin-1-yl)-7-oxo-2-(3,3a,4,6a-tetrahydro-1H-cyclopenta[c]furan-5-yl)-[1,2,4]triazolo[1,5-a]pyrimi din-4(7H)-yl)acetamide

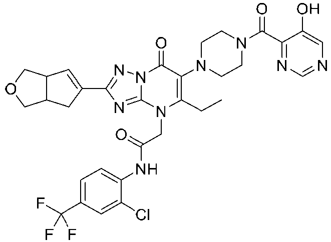

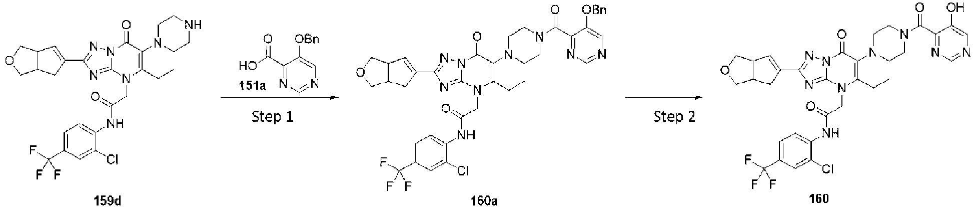

### Step 1

### 2-(6-(4-(5-(benzyloxy)pyrimidine-4-carbonyl)piperazin-1-yl)-5-ethyl-7-oxo-2-(3,3a,4,6a-tetrahyd ro-1H-cyclopenta[c]furan-5-yl)-[1,2,4]triazolo[1,5-a]pyrimidin-4(7H)-yl)-N-(2-chloro-4-(trifluor omethyl)phenyl)acetamide

5-(Benzyloxy)pyrimidine-4-carboxylic acid **151a** (23.33 mg, 101.35 µmol), 1-hydroxybenzotriazole (18.26 mg, 135.13 µmol), 1-(3-dimethylaminopropyl)-3-ethylcarbodiimide hydrochloride (25.90 mg, 135.13 µmol), and N,N-diisopropylethylamine (26.20 mg, 202.70 µmol) were dissolved in N,N-dimethylformamide (4 mL), and the mixture was stirred at 30 °C for 10 minutes under nitrogen protection. Then N-(2-chloro-4-(trifluoromethyl)phenyl)-2-(5-ethyl-7-oxo-6-(piperazin-1-yl)-2-(3,3a,4,6a-tetrahyd ro-1H-cyclopenta[c]furan-5-yl)-[1,2,4]triazolo[1,5-a]pyrimidin-4(7H)-yl)acetamide **159d** (40 mg, 67.57 µmol) was added, and the mixture was stirred at 30°C for 16 hours. After completion of the reaction, water (30 mL) was added to the reaction mixture, which was extracted with ethyl acetate (30 mL × 3). The combined organic phases were washed with saturated sodium chloride solution (30 mL × 3), dried over anhydrous sodium sulfate, filtered, and concentrated under reduced pressure. The obtained residue was purified by column chromatography (eluent: System B) to obtain 2-(6-(4-(5-(benzyloxy)pyrimidine-4-carbonyl)piperazin-1-yl)-5-ethyl-7-oxo-2-(3,3a,4,6a-tetrahyd ro-1H-cyclopenta[c]furan-5-yl)-[1,2,4]triazolo[1,5-a]pyrimidin-4(7H)-yl)-N-(2-chloro-4-(trifluor omethyl)phenyl)acetamide **160a** (40 mg) with a yield of 73.61%.

MS m/z (ESI): 804.5 [M+1]

### Step 2

### N-(2-chloro-4-(trifluoromethyl)phenyl)-2-(5-ethyl-6-(4-(5-hydroxypyrimidine-4-carbonyl)pipera zin-1-yl)-7-oxo-2-(3,3a,4,6a-tetrahydro-1H-cyclopenta[c]furan-5-yl)-[1,2,4]triazolo[1,5-a]pyrimi din-4(7H)-yl)acetamide

2-(6-(4-(5-(Benzyloxy)pyrimidine-4-carbonyl)piperazin-1-yl)-5-ethyl-7-oxo-2-(3,3a,4,6 a-tetrahydro-1H-cyclopenta[c]furan-5-yl)-[1,2,4]triazolo[1,5-a]pyrimidin-4(7H)-yl)-N-(2-chloro-4-(trifluoromethyl)phenyl)acetamide **160a** (40 mg, 49.74 µmol) was dissolved in trifluoroacetic acid (3 mL), and the reaction mixture was stirred at 45 °C for 2 days. After the reaction was complete, the reaction mixture was diluted with dichloromethane (30 mL) and concentrated under reduced pressure. The resulting residue was purified by preparative liquid chromatography (Waters 2767QDA column: Sunfire Sunfire C₁₈, 19 ×250 mm, 10 µm; mobile phase A: 0.05% TFA/H₂O, B: ACN; flow rate: 20 mL/min) to obtain N-(2-chloro-4-(trifluoromethyl)phenyl)-2-(5-ethyl-6-(4-(5-hydroxypyrimidine-4-carbonyl)pipera zin-1-yl)-7-oxo-2-(3,3a,4,6a-tetrahydro-1H-cyclopenta[c]furan-5-yl)-[1,2,4]triazolo[1,5-a]pyrimi din-4(7H)-yl)acetamide 160 (15.63 mg) with a yield of 44.01%.

MS m/z (ESI): 714.5 [M+1]
¹H NMR (400 MHz, DMSO) δ 10.94 (s, 1H), 10.36 (s, 1H), 8.72 (s, 1H), 8.46 (s, 1H), 8.07 (d, J = 8.8 Hz, 1H), 7.97 (d, J = 1.6 Hz, 1H), 7.72 (d, J = 8.8 Hz, 1H), 6.45 (s, 1H), 5.32 (s, 2H), 4.51 (d, J = 12.4 Hz, 1H), 3.78-3.73 (m, 1H), 3.66-3.63 (m, 2H), 3.55-3.51 (m, 2H), 3.50-3.43 (m, 2H), 3.35-3.21 (m, 2H), 3.08-2.91 (m, 5H), 2.85-2.78 (m, 1H), 2.66-2.55 (m, 2H), 1.18 (t, J = 7.2 Hz, 3H).

### Example 161

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(3,3a,4,6a-tetrahydro-1H-cyclopenta[c]furan-5-yl)-[1,2,4]triazolo[1,5-a]pyrimidin-4(7H)-yl)acetamide

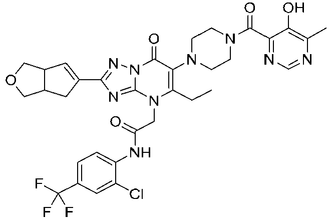

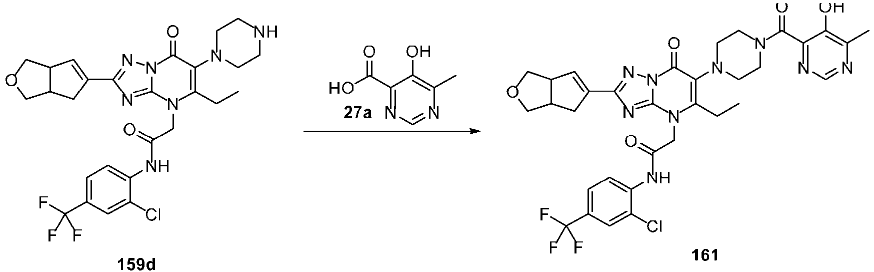

5-Hydroxy-6-methylpyrimidine-4-carboxylic acid **27a** (7.81 mg, 50.67 µmol), 1-hydroxybenzotriazole (9.13 mg, 67.57 µmol), 1-(3-dimethylaminopropyl)-3-ethylcarbodiimide hydrochloride (12.95 mg, 67.57 µmol), and N,N-diisopropylethylamine (13.10 mg, 101.35 µmol) were dissolved in N,N-dimethylformamide (1.5 mL), and the mixture was stirred at 25 C under nitrogen atmosphere for 10 minutes. Then N-(2-chloro-4-(trifluoromethyl)phenyl)-2-(5-ethyl-7-oxo-6-(piperazin-1-yl)-2-(3,3a,4,6a-tetrahyd ro-1H-cyclopenta[c]furan-5-yl)-[1,2,4]triazolo[1,5-a]pyrimidin-4(7H)-yl)acetamide **159d** (20 mg, 33.78 µmol) was added, and the mixture was stirred at 30°C for 16 hours. After the reaction was complete, the reaction mixture was purified by preparative liquid chromatography (Waters 3767/QDA column: SunFire Sunfire C₁₈, 19 ×250 mm, 10 µm; mobile phase A: 10 mmol/L, 0.1% FA/H₂O, B: acetonitrile; flow rate: 20 mL/min) to obtain N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(3,3a,4,6a-tetrahydro-1H-cyclopenta[c]furan-5-yl)-[1,2,4]triazolo[1,5-a]pyrimidin-4(7H)-yl)acetamide **161** (5.35 mg) with a yield of 21.75%.

MS m/z (ESI): 728.2 [M+1]
¹H NMR (400 MHz, DMSO-d6) δ 10.36 (s, 1H), 10.23 (s, 1H), 8.58 (s, 1H), 8.06 (d, J = 8.0 Hz, 1H), 7.97 (s, 1H), 7.71 (d, J = 12.0 Hz, 1H), 6.45 (s, 1H), 5.32 (s, 2H), 4.52 (d, J = 16.0 Hz, 1H), 3.7-3.73 (m, 1H), 3.66-3.62 (m, 2H), 3.53-3.45 (m, 4H), 3.25 (m, 1H), 2.99-2.97 (m, 5H), 2.85-2.78 (m, 1H), 2.65-2.57 (m, 3H), 2.44 (s, 3H), 1.18 (t, J = 8.0 Hz, 3H).

### Example 162

### N-(2-chloro-4-(trifluoromethyl)phenyl)-2-(2-(2,3-dihydrofuro[2,3-b]pyridin-5-yl)-5-ethyl-6-(4-(5 -hydroxy-6-methylpyrimidine-4-carbonyl)piperazin-1-yl)-7-oxo-[1,2,4]triazolo[1,5-a]pyrimidin-4 (7H)-yl)acetamide

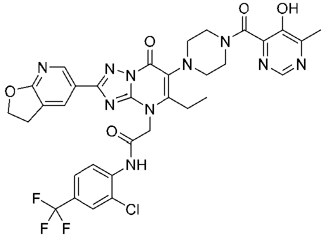

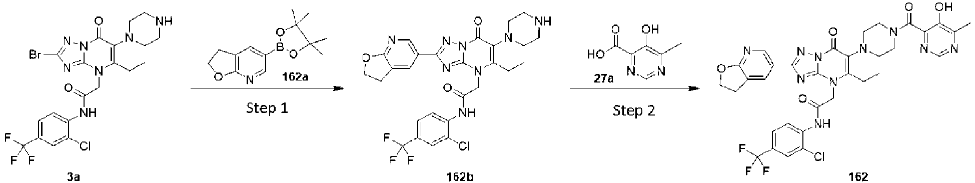

### Step 1

### N-(2-chloro-4-(trifluoromethyl)phenyl)-2-(2-(2,3-dihydrofuro[2,3-b]pyridin-5-yl)-5-ethyl-7-oxo-6-(piperazin-1-yl)-[1,2,4]triazolo[1,5-a]pyrimidin-4(7H)-yl)acetamide

5-(4,4,5,5-Tetramethyl-1,3,2-dioxaborolan-2-yl)-2,3-dihydrofuro[2,3-b]pyridine **162a** (160 mg, 686.49 µmol, prepared according to published patent WO2017112719), 2-(2-bromo-5-ethyl-7-oxo-6-(piperazin-1-yl)-[1,2,4]triazolo[1,5-a]pyrimidin-4(7H)-yl)-N-(2-chlo ro-4-(trifluoromethyl)phenyl)acetamide **3a** (240 mg, 426.46 µmol), sodium carbonate (160 mg, 858.11 µmol), and [1,1'-bis(diphenylphosphino)ferrocene]palladium dichloride (80 mg, 107.85 µmol) were added to 1,4-dioxane (16 mL) and water (3.2 mL). The reaction mixture was stirred at 100 °C under nitrogen atmosphere for 3 hours. After the reaction was complete, the reaction mixture was diluted with water (30 mL) and extracted with ethyl acetate (30 mL × 3). The combined organic phases were washed with saturated sodium chloride solution (30 mL), dried over anhydrous sodium sulfate, filtered, and concentrated under reduced pressure. The residue was purified by thin layer chromatography on silica gel (eluent: System B) to obtain N-(2-chloro-4-(trifluoromethyl)phenyl)-2-(2-(2,3-dihydrofuro[2,3-b]pyridin-5-yl)-5-ethyl-7-oxo-6-(piperazin-1-yl)-[1,2,4]triazolo[1,5-a]pyrimidin-4(7H)-yl)acetamide **162b** (100 mg) with a yield of 38.65%.

MS m/z (ESI): 603.5 [M+1]

### Step 2

### N-(2-chloro-4-(trifluoromethyl)phenyl)-2-(2-(2,3-dihydrofuro[2,3-b]pyridin-5-yl)-5-ethyl-6-(4-(5 -hydroxy-6-methylpyrimidine-4-carbonyl)piperazin-1-yl)-7-oxo-[1,2,4]triazolo[1,5-a]pyrimidin-4 (7H)-yl)acetamide

5-Hydroxy-6-methylpyrimidine-4-carboxylic acid **27a** (30.67 mg, 199.01 µmol), 1-hydroxybenzotriazole (35.85 mg, 265.34 µmol), 1-(3-dimethylaminopropyl)-3-ethylcarbodiimide hydrochloride (50.87 mg, 265.34 µmol), and N,N-diisopropylethylamine (51.44 mg, 398.01 µmol) were dissolved in N,N-dimethylformamide (5 mL), and the mixture was stirred at 30 °C under nitrogen for 10 minutes. Then N-(2-chloro-4-(trifluoromethyl)phenyl)-2-(2-(2,3-dihydrofuro[2,3-b]pyridin-5-yl)-5-ethyl-7-oxo-6-(piperazin-1-yl)-[1,2,4]triazolo[1,5-a]pyrimidin-4(7H)-yl)acetamide **162b** (100 mg, 132.67 µmol) was added, and the mixture was stirred at 30°C for 16 hours. After the reaction was completed, water (30 mL) was added to the reaction mixture, and the mixture was extracted with ethyl acetate (30 mL × 3). The combined organic phases were washed with saturated sodium chloride solution (30 mL × 3), dried over anhydrous sodium sulfate, filtered, and concentrated under reduced pressure. The resulting residue was purified by preparative liquid chromatography (Waters 2767/QDA, column: XBridge C₁₈, 19×250 mm, 10 µm; mobile phase A: 10 mmol NH₄HCO₃/H₂O, B: ACN; flow rate: 20 mL/min) to obtain N-(2-chloro-4-(trifluoromethyl)phenyl)-2-(2-(2,3-dihydrofuro[2,3-b]pyridin-5-yl)-5-ethyl-6-(4-(5 -hydroxy-6-methylpyrimidine-4-carbonyl)piperazin-1-yl)-7-oxo-[1,2,4]triazolo[1,5-a]pyrimidin-4 (7H)-yl)acetamide (2.5 mg) with a yield of 2.55%.

MS m/z (ESI): 739.2 [M+1]
¹H NMR (400 MHz, DMSO) δ 10.60-10.00 (m, 2H), 8.63 (s, 1H), 8.56 (s, 1H), 8.24 (s, 1H), 8.05 (d, J = 8.4 Hz, 1H), 7.97 (s, 1H), 7.72 (d, J = 8.8 Hz, 1H), 5.38 (s, 2H), 4.65 (t, J = 8.4 Hz, 2H), 4.53 (d, J = 12.0 Hz, 1H), 3.55-3.46 (m, 3H), 3.30-3.22 (m, 3H), 3.07-2.96 (m, 3H), 2.88-2.79 (m, 1H), 2.70-2.63 (m, 1H), 2.44 (s, 3H), 1.21 (t, J = 7.6 Hz, 3H).

### Example 163

### N-(2-chloro-4-(trifluoromethyl)phenyl)-2-(5-ethyl-6-(4-(5-hydroxy-6-methylpyrimidine-4-carbon yl)piperazin-1-yl)-2-(5-methoxy-1,3a,4,5,6,6a-hexahydropentalen-2-yl)-7-oxo-[1,2,4]triazolo[1,5 -a]pyrimidin-4(7H)-yl)acetamide

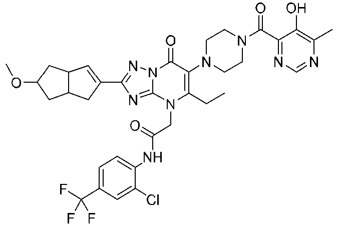

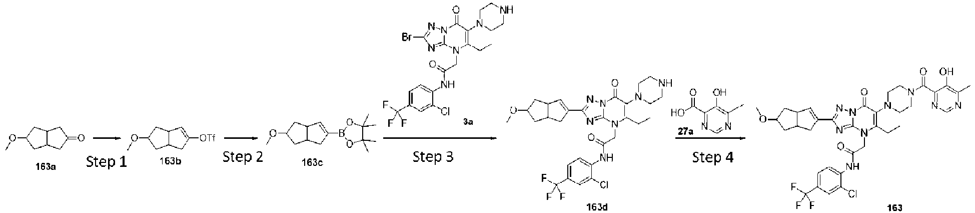

### Step 1

### 5-methoxy-1,3a,4,5,6,6a-hexahydropentalen-2-yl trifluoromethanesulfonate

5-Methoxyhexahydropentalen-2(1H)-one **163a** (247 mg, 1.60 mmol, prepared according to published patent WO2014000629) was dissolved in tetrahydrofuran (4 mL), and lithium bis(trimethylsilyl)amide (1 M, 3.2 mL) was added dropwise at -78 °C under nitrogen. The reaction mixture was stirred at -78 °C for 1 hour. Then N-phenylbis(trifluoromethanesulfonyl)imide (629.45 mg, 1.76 mmol) was dissolved in tetrahydrofuran (6 mL) and added dropwise to the reaction mixture at -78 °C. The reaction mixture was stirred at 25 °C for 16 hours. After the reaction was completed, water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System A) to obtain 5-methoxy-1,3a,4,5,6,6a-hexahydropentalen-2-yl trifluoromethanesulfonate **163b** (300 mg) with a yield of 65.43%.

¹H NMR (400 MHz, DMSO-d6) δ 5.77-5.73 (m, 1H), 3.74-3.67 (m, 1H), 3.14 (s, 3H), 3.13-3.05 (m, 1H), 2.90-2.64 (m, 2H), 2.39-2.27 (m, 1H), 2.08-1.86 (m, 2H), 1.52-1.39 (m, 2H).

### Step 2

### 2-(5-methoxy-1,3a,4,5,6,6a-hexahydropentalen-2-yl)-4,4,5,5-tetramethyl-1,3,2-dioxaborolane

5-Methoxy-1,3a,4,5,6,6a-hexahydropentalen-2-yl trifluoromethanesulfonate **163b** (300 mg, 1.05 mmol) was dissolved in 1,4-dioxane (3 mL), and potassium acetate (308.54 mg, 3.14 mmol), bis(pinacolato)diboron (397.70 mg, 1.57 mmol), and [1,1'-bis(diphenylphosphino)ferrocene]palladium(II) dichloride (77.74 mg, 104.80 µmol) were added. The mixture was stirred at 90 °C under nitrogen for 2 hours. After the reaction was completed, the reaction solution was used directly in the next step.

### Step 3

### N-(2-chloro-4-(trifluoromethyl)phenyl)-2-(5-ethyl-2-(5-methoxy-1,3a,4,5,6,6a-hexahydropentale n-2-yl)-7-oxo-6-(piperazin-1-yl)-[1,2,4]triazolo[1,5-a]pyrimidin-4(7H)-yl)acetamide

To the reaction solution of 2-(5-methoxy-1,3a,4,5,6,6a-hexahydropentalen-2-yl)-4,4,5,5-tetramethyl-1,3,2-dioxaborolane **163c** (1.05 mmol) from the previous step were added 1,4-dioxane (1 mL) and water (0.1 mL), sodium carbonate (51.40 mg, 484.92 µmol), 2-(2-bromo-5-ethyl-7-oxo-6-(piperazin-1-yl)-[1,2,4]triazolo[1,5-a]pyrimidin-4(7H)-yl)-N-(2-chlo ro-4-(trifluoromethyl)phenyl)acetamide **3a** (129.95 mg, 230.91 µmol), and [1,1'-bis(diphenylphosphino)ferrocene]palladium(II) dichloride (17.13 mg, 23.09 µmol). The mixture was stirred at 90 °C under nitrogen for 2 hours. After the reaction was completed, water (30 mL) was added to the reaction mixture, and the mixture was extracted with ethyl acetate (30 mL × 3). The combined organic phases were washed with saturated sodium chloride solution (30 mL), dried over anhydrous sodium sulfate, filtered, and concentrated under reduced pressure. The residue was purified by thin-layer chromatography silica gel plate (eluent: System B) to obtain N-(2-chloro-4-(trifluoromethyl)phenyl)-2-(5-ethyl-2-(5-methoxy-1,3a,4,5,6,6a-hexahydropentale n-2-yl)-7-oxo-6-(piperazin-1-yl)-[1,2,4]triazolo[1,5-a]pyrimidin-4(7H)-yl)acetamide **163d** (40 mg) with a yield of 27.94%.

MS m/z (ESI): 620.3 [M+1]

### Step 4

### N-(2-chloro-4-(trifluoromethyl)phenyl)-2-(5-ethyl-6-(4-(5-hydroxy-6-methylpyrimidine-4-carbon yl)piperazin-1-yl)-2-(5-methoxy-1,3a,4,5,6,6a-hexahydropentalen-2-yl)-7-oxo-[1,2,4]triazolo[1,5 -a]pyrimidin-4(7H)-yl)acetamide

5-Hydroxy-6-methylpyrimidine-4-carboxylic acid **27a** (13.05 mg, 84.67 umol), 1-hydroxybenzotriazole (15.25 mg, 112.89 umol), 1-(3-dimethylaminopropyl)-3-ethylcarbodiimide hydrochloride (21.64 mg, 112.89 umol), and N,N-diisopropylethylamine (21.89 mg, 169.34 umol) were dissolved in N,N-dimethylformamide (1 mL), and the mixture was stirred at 25 °C under nitrogen atmosphere for 10 minutes. Then N-(2-chloro-4-(trifluoromethyl)phenyl)-2-(5-ethyl-2-(5-methoxy-1,3a,4,5,6,6a-hexahydropentale n-2-yl)-7-oxo-6-(piperazin-1-yl)-[1,2,4]triazolo[1,5-a]pyrimidin-4(7H)-yl)acetamide **163d** (35 mg, 56.45 umol) was added, and the mixture was stirred at 30 °C for 16 hours. After the reaction was completed, the reaction mixture was purified by preparative liquid chromatography (Waters 3767/QDA column: SunFire Sunfire C₁₈, 19 ×250 mm, 10 um; mobile phase A: 10 mmol/L A: 0.1% FA/H₂O, B: acetonitrile; flow rate: 20 mL/min) to obtain N-(2-chloro-4-(trifluoromethyl)phenyl)-2-(5-ethyl-6-(4-(5-hydroxy-6-methylpyrimidine-4-carbon yl)piperazin-1-yl)-2-(5-methoxy-1,3a,4,5,6,6a-hexahydropentalen-2-yl)-7-oxo-[1,2,4] triazolo[1,5-a]pyrimidin-4(7H)-yl)acetamide **163** (17.24 mg) with a yield of 40.39%.

MS m/z (ESI): 756.2 [M+1]
¹H NMR (400 MHz, DMSO-d6) δ 10.36 (s, 1H), 10.23 (s, 1H), 8.58 (s, 1H), 8.06 (d, J = 8.0 Hz, 1H), 7.97 (m, 1H), 7.73-7.70 (m, 1H), 6.51 (s, 1H), 5.31 (s, 2H), 4.52 (d, J = 8.0 Hz, 1H), 3.77-3.70 (m, 1H), 3.51-3.48 (m, 3H), 3.27-3.24 (m, 2H), 3.13 (s, 3H), 2.98-2.90 (m, 4H), 2.82-2.77 (m, 2H), 2.64-2.58 (m, 2H), 2.44 (s, 3H), 2.11-2.06 (m, 2H), 1.45-1.38 (m, 2H), 1.18 (t, J = 8.0 Hz, 3H).

### Example 164

### N-(2-chloro-4-(trifluoromethyl)phenyl)-2-(5-ethyl-2-(5-hydroxy-5-methyl-1,3a,4,5,6,6a-hexahyd ropentalen-2-yl)-6-(4-(3-hydroxypicolinoyl)piperazin-1-yl)-7-oxo-[1,2,4]triazolo[1,5-a]pyrimidin -4(7H)-yl)acetamide

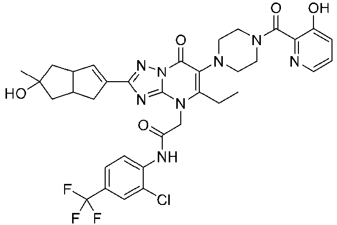

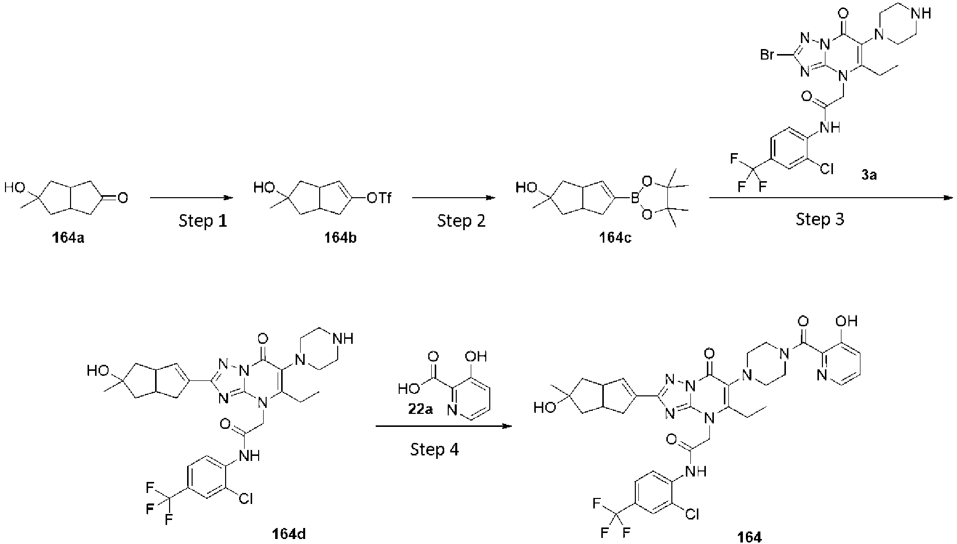

### Step 1

### 5-hydroxy-5-methyl-1,3a,4,5,6,6a-hexahydropentalen-2-yl trifluoromethanesulfonate

5-Hydroxy-5-methylhexahydropentalen-2(1H)-one **164a** (200 mg, 1.30 mmol, prepared according to published patent WO2014000629) was dissolved in tetrahydrofuran (2 mL), and lithium bis(trimethylsilyl)amide (1 M, 2.59 mL) was added dropwise at -78 °C under nitrogen atmosphere. The reaction mixture was stirred at -78 °C for 1 hour. N-phenylbis(trifluoromethanesulfonyl)imide) (509.68 mg, 1.43 mmol) was dissolved in tetrahydrofuran (4 mL) and added dropwise to the reaction mixture at -78 °C. The reaction mixture was stirred at 25 °C for 16 hours. After the reaction was completed, water (20 mL) was added to the reaction mixture, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by column chromatography (eluent: System A) to obtain 5-hydroxy-5-methyl-1,3a,4,5,6,6a-hexahydropentalen-2-yl trifluoromethanesulfonate **164b** (85 mg) with a yield of 22.89%.

¹H NMR (400 MHz, DMSO-d6) δ 5.80-5.74 (m, 1H), 4.42 (br s, 1H), 3.15-2.95 (m, 1H), 2.88-2.66 (m, 2H), 2.42-2.30 (m, 1H), 1.83-1.70 (m, 2H), 1.57-1.34 (m, 2H), 1.14 (s, 3H).

### Step 2

### 2-methyl-5-(4,4,5,5-tetramethyl-1,3,2-dioxaborolan-2-yl)-1,2,3,3a,4,6a-hexahydropentalen-2-ol

5-Hydroxy-5-methyl-1,3a,4,5,6,6a-hexahydropentalen-2-yl trifluoromethanesulfonate **164b** (85 mg, 296.93 umol) was dissolved in 1,4-dioxane (1 mL), and potassium acetate (87.42 mg, 890.78 umol), bis(pinacolato)diboron (112.68 mg, 445.39 umol), and [1,1'-bis(diphenylphosphino)ferrocene]palladium(II) dichloride (22.03 mg, 29.69 umol) were added. The mixture was stirred at 90 °C under nitrogen atmosphere for 2 hours. After the reaction was completed, the reaction solution was used directly in the next step.

### Step 3

### N-(2-chloro-4-(trifluoromethyl)phenyl)-2-(5-ethyl-2-(5-hydroxy-5-methyl-1,3a,4,5,6,6a-hexahyd ropentalen-2-yl)-7-oxo-6-(piperazin-1-yl)-[1,2,4]triazolo[1,5-a]pyrimidin-4(7H)-yl)acetamide

To the reaction solution of 2-methyl-5-(4,4,5,5-tetramethyl-1,3,2-dioxaborolan-2-yl)-1,2,3,3a,4,6a-hexahydropentalen-2-ol **164c** (296.93 µmol) from the previous step were added 1,4-dioxane (1 mL) and water (0.1 mL), followed by sodium carbonate (47.47 mg, 447.79 µmol), 2-(2-bromo-5-ethyl-7-oxo-6-(piperazin-1-yl)-[1,2,4]triazolo[1,5-a]pyrimidin-4(7H)-yl)-N-(2-chlo ro-4-(trifluoromethyl)phenyl)acetamide **3a** (120 mg, 213.23 µmol), and [1,1'-bis(diphenylphosphino)ferrocene]palladium(II) dichloride (15.82 mg, 21.32 µmol).The mixture was stirred at 90 °C under nitrogen atmosphere for 2 hours. After the reaction was completed, water (30 mL) was added to the reaction mixture, and the mixture was extracted with ethyl acetate (30 mL × 3). The combined organic phases were washed with saturated sodium chloride solution (30 mL), dried over anhydrous sodium sulfate, filtered, and concentrated under reduced pressure. The residue was purified by thin layer chromatography silica gel plate (eluent: System B) to obtain N-(2-chloro-4-(trifluoromethyl)phenyl)-2-(5-ethyl-2-(5-hydroxy-5-methyl-1,3a,4,5,6,6a-hexahyd ropentalen-2-yl)-7-oxo-6-(piperazin-1-yl)-[1,2,4]triazolo[1,5-a]pyrimidin-4(7H)-yl)acetamide **164d** (40 mg) with a yield of 21.18%.

MS m/z (ESI): 620.3 [M+1]

### Step 4

### N-(2-chloro-4-(trifluoromethyl)phenyl)-2-(5-ethyl-2-(5-hydroxy-5-methyl-1,3a,4,5,6,6a-hexahyd ropentalen-2-yl)-6-(4-(3-hydroxypicolinoyl)piperazin-1-yl)-7-oxo-[1,2,4]triazolo[1,5-a]pyrimidin -4(7H)-yl)acetamide

3-Hydroxypicolinic acid **22a** (11.78 mg, 84.67 µmol), 1-hydroxybenzotriazole (15.25 mg, 112.89 µmol), 1-(3-dimethylaminopropyl)-3-ethylcarbodiimide hydrochloride (21.64 mg, 112.89 µmol), and N,N-diisopropylethylamine (21.89 mg, 169.34 µmol) were dissolved in N,N-dimethylformamide (1 mL), and the mixture was stirred at 25 °C under nitrogen atmosphere for 10 minutes. Then N-(2-chloro-4-(trifluoromethyl)phenyl)-2-(5-ethyl-2-(5-hydroxy-5-methyl-1,3a,4,5,6,6a-hexahyd ropentalen-2-yl)-7-oxo-6-(piperazin-1-yl)-[1,2,4]triazolo[1,5-a]pyrimidin-4(7H)-yl)acetamide **164d** (35 mg, 56.45 µmol) was added, and the mixture was stirred at 30 °C for 16 hours. After the reaction was complete, the reaction mixture was purified by preparative liquid chromatography (Waters 3767/QDA column: SunFire Sunfire C₁₈, 19 ×250 mm, 10 µm; mobile phase A: 10 mmol/L A: 0.1% FA/H₂O, B: acetonitrile; flow rate: 20 mL/min) to obtain N-(2-chloro-4-(trifluoromethyl)phenyl)-2-(5-ethyl-2-(5-hydroxy-5-methyl-1,3a,4,5,6,6a-hexahyd ropentalen-2-yl)-6-(4-(3-hydroxypicolinoyl)piperazin-1-yl)-7-oxo-[1,2,4]triazolo[1,5-a]pyrimidin -4(7H)-yl) acetamide **164** (10.54 mg) with a yield of 25.19%.

MS m/z (ESI): 741.2 [M+1]
¹H NMR (400 MHz, DMSO) δ 10.60-10.20 (m, 2H), 8.10-8.01 (m, 2H), 7.96 (s, 1H), 7.70 (d, J = 12 Hz, 1H), 7.28 (m, 2H), 6.52-6.51 (m, 1H), 5.30 (s, 2H), 4.54 (d, J = 12.0 Hz, 1H), 4.28 (s, 1H), 3.48-3.39 (m, 3H), 3.25-3.19 (m, 2H), 2.99-2.88 (m, 4H), 2.80-2.78 (m, 2H), 2.67-2.53 (m, 2H), 1.85-1.78 (m, 2H), 1.50-1.45 (m, 2H), 1.20-1.16 (m, 6H).

### Example 165

### N-(2-chloro-4-((trifluoromethyl)thio)phenyl)-2-(5-ethyl-6-(4-(5-hydroxy-6-methylpyrimidine-4-c arbonyl)piperazin-1-yl)-7-oxo-2-(2,5,6,7-tetrahydrooxepin-3-yl)-[1,2,4]triazolo[1,5-a]pyrimidin-4(7H)-yl)acetamide

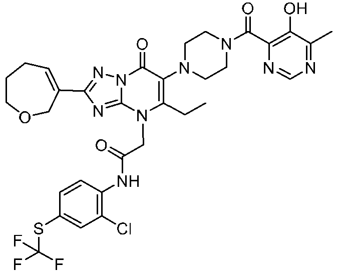

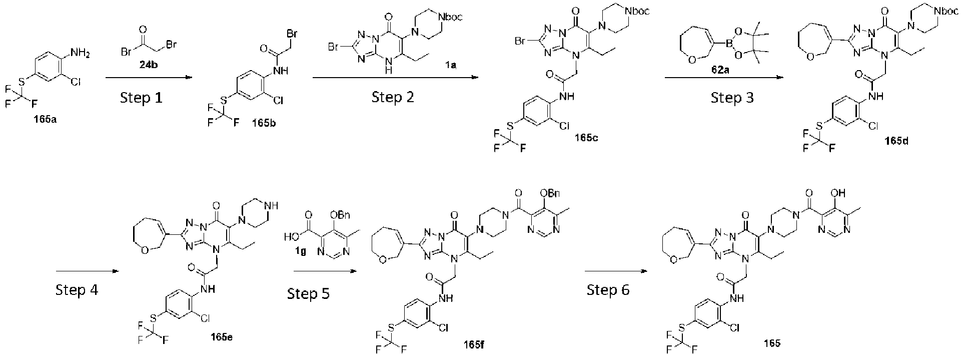

### Step 1

### 2-bromo-N-(2-chloro-4-((trifluoromethyl)thio)phenyl)acetamide

Under ice bath, 2-bromoacetyl bromide 24b (518.72 mg, 2.57 mmol) was slowly added to a solution of 2-chloro-4-((trifluoromethyl)thio)aniline **165a** (0.45 g, 1.98 mmol, prepared according to published patent WO2009008287) and 4-dimethylaminopyridine (313.97 mg, 2.57 mmol) in dichloromethane (4 mL), and the reaction was performed at room temperature for 18 hours. The reaction mixture was concentrated under reduced pressure, and the residue was purified by silica gel column chromatography (eluent: System A) to obtain 2-bromo-N-(2-chloro-4-((trifluoromethyl)thio)phenyl)acetamide **165b** (0.5 g) with a yield of 72.56%.

MS m/z (ESI): 348.9 [M+H]

### Step 2

### tert-butyl 4-(2-bromo-4-(2-((2-chloro-4-((trifluoromethyl)thio)phenyl)amino)-2-oxoethyl)-5-ethyl-7-oxo-4, 7-dihydro-[1,2,4]triazolo[1,5-a]pyrimidin-6-yl)piperazine-1-carboxylate

2-Bromo-N-(2-chloro-4-((trifluoromethyl)thio)phenyl)acetamide **165b** (244.73 mg, 702.09 µmol) and N,N-diisopropylethylamine (226.85 mg, 1.76 mmol) were added to a solution of tert-butyl 4-(2-bromo-5-ethyl-7-oxo-4,7-dihydro-[1,2,4]triazolo[1,5-a]pyrimidin-6-yl)piperazine-1-carboxy late **1a** (0.25 g, 585.08 µmol) in N,N-dimethylformamide (4 mL), and the mixture was heated to 50 °C and stirred for 3 hours. After the reaction was complete, the reaction mixture was directly separated by silica gel column chromatography (eluent: System A) to obtain tert-butyl 4-(2-bromo-4-(2-((2-chloro-4-((trifluoromethyl)thio)phenyl)amino)-2-oxoethyl)-5-ethyl-7-oxo-4, 7-dihydro-[1,2,4]triazolo[1,5-a]pyrimidin-6-yl)piperazine-1-carboxylate **165c** (0.3 g) with a yield of 73.78%.

MS m/z (ESI): 596.0 [M+ H-100]

### Step 3

### tert-butyl 4-(4-(2-((2-chloro-4-((trifluoromethyl)thio)phenyl)amino)-2-oxoethyl)-5-ethyl-7-oxo-2-(2,5,6,7-t etrahydrooxepin-3-yl)-4,7-dihydro-[1,2,4]triazolo[1,5-a]pyrimidin-6-yl)piperazine-1-carboxylate

4,4,5,5-Tetramethyl-2-(2,5,6,7-tetrahydrooxepin-3-yl)-1,3,2-dioxaborolane **62a** (145.11 mg, 647.53 µmol), tert-butyl 4-(2-bromo-4-(2-((2-chloro-4-((trifluoromethyl)thio)phenyl)amino)-2-oxoethyl)-5-ethyl-7-oxo-4, 7-dihydro-[1,2,4]triazolo[1,5-a]pyrimidin-6-yl)piperazine-1-carboxylate **165c** (0.3 g, 431.69 µmol), [1,1'-bis(diphenylphosphino)ferrocene]palladium(II) dichloride (32.02 mg, 43.17 µmol), and sodium carbonate (91.51 mg, 863.37 µmol) were added to a mixed solvent of 1,4-dioxane (3 mL) and water (1 mL), and the mixture was subject to argon replacement four times. The mixture was heated to 80 °C and reacted for 1.5 hours. Water (20 mL) was added to the reaction mixture, and the mixture was extracted with ethyl acetate (40 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(4-(2-((2-chloro-4-((trifluoromethyl)thio)phenyl)amino)-2-oxoethyl)-5-ethyl-7-oxo-2-(2,5,6,7-t etrahydrooxepin-3-yl)-4,7-dihydro-[1,2,4]triazolo[1,5-a]pyrimidin-6-yl)piperazine-1-carboxylate **165d** (160 mg) with a yield of 52.04%.

MS m/z (ESI): 656.2 [M+ H-56]

### Step 4

### N-(2-chloro-4-((trifluoromethyl)thio)phenyl)-2-(5-ethyl-7-oxo-6-(piperazin-1-yl)-2-(2,5,6,7-tetra hydrooxepin-3-yl)-[1,2,4]triazolo[1,5-a]pyrimidin-4(7H)-yl)acetamide

Tert-butyl 4-(4-(2-((2-chloro-4-((trifluoromethyl)thio)phenyl)amino)-2-oxoethyl)-5-ethyl-7-oxo-2-(2,5,6,7-t etrahydrooxepin-3-yl)-4,7-dihydro-[1,2,4]triazolo[1,5-a]pyrimidin-6-yl)piperazine-1-carboxylate **165d** (160 mg, 224.66 µmol) was dissolved in dichloromethane (5 mL), and trifluoroacetic acid (1 g, 8.77 mmol) was added dropwise. The reaction was performed at room temperature for 3 hours. The mixture was concentrated under reduced pressure to obtain N-(2-chloro-4-((trifluoromethyl)thio)phenyl)-2-(5-ethyl-7-oxo-6-(piperazin-1-yl)-2-(2,5,6,7-tetra hydrooxepin-3-yl)-[1,2,4]triazolo[1,5-a]pyrimidin-4(7H)-yl)acetamide **165e** (120 mg) as a crude product, which was directly used in the next step.

MS m/z (ESI): 612.2 [M+H]

### Step 5

### 2-(6-(4-(5-(benzyloxy)-6-methylpyrimidine-4-carbonyl)piperazin-1-yl)-5-ethyl-7-oxo-2-(2,5,6,7-t etrahydrooxepin-3-yl)-[1,2,4]triazolo[1,5-a]pyrimidin-4(7H)-yl)-N-(2-chloro-4-((trifluoromethyl) thio)phenyl)acetamide

5-(Benzyloxy)-6-methylpyrimidine-4-carboxylic acid **1g** (86.20 mg, 352.90 µmol), 1-(3-dimethylaminopropyl)-3-ethylcarbodiimide hydrochloride (93.96 mg, 490.14 µmol), 1-hydroxybenzotriazole (66.23 mg, 490.14 µmol), and N,N-diisopropylethylamine (126.69 mg, 980.29 µmol) were added to N,N-dimethylformamide (4 mL), and the reaction was performed at room temperature for 15 minutes. A solution of N-(2-chloro-4-((trifluoromethyl)thio)phenyl)-2-(5-ethyl-7-oxo-6-(piperazin-1-yl)-2-(2,5,6,7-tetra hydrooxepin-3-yl)-[1,2,4]triazolo[1,5-a]pyrimidin-4(7H)-yl)acetamide **165e** (120 mg, 196.06 µmol) in N,N-dimethylformamide (2 mL) was added, and the reaction was performed at room temperature for 18 hours. Water (20 mL) was added to the reaction mixture, and the mixture was extracted with dichloromethane (30 mL × 3). The combined organic phases were washed with saturated sodium chloride solution (30 mL × 2), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-5-ethyl-7-oxo-2-(2,5,6,7-t etrahydrooxepin-3-yl)-[1,2,4]triazolo[1,5-a]pyrimidin-4(7H)-yl)-N-(2-chloro-4-((trifluoromethyl) thio)phenyl)acetamide **165f** (100 mg) with a yield of 60.84%.

MS m/z (ESI): 838.2 [M+H]

### Step 6

### N-(2-chloro-4-((trifluoromethyl)thio)phenyl)-2-(5-ethyl-6-(4-(5-hydroxy-6-methylpyrimidine-4-c arbonyl)piperazin-1-yl)-7-oxo-2-(2,5,6,7-tetrahydrooxepin-3-yl)-[1,2,4]triazolo[1,5-a]pyrimidin-4(7H)-yl)acetamide

2-(6-(4-(5-(Benzyloxy)-6-methylpyrimidine-4-carbonyl)piperazin-1-yl)-5-ethyl-7-oxo-2 -(2,5,6,7-tetrahydrooxepin-3-yl)-[1,2,4]triazolo[1,5-a]pyrimidin-4(7H)-yl)-N-(2-chloro-4-((trifluo romethyl)thio)phenyl)acetamide **165f** (100 mg, 119.29 µmol) was dissolved in trifluoroacetic acid (5 mL), and the reaction was heated to 45 °C with stirring for 3 hours. The mixture was concentrated under reduced pressure, and the residue was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-((trifluoromethyl)thio)phenyl)-2-(5-ethyl-6-(4-(5-hydroxy-6-methylpyrimidine-4-c arbonyl) piperazin-1-yl)-7-oxo-2-(2,5,6,7-tetrahydrooxepin-3-yl)-[1,2,4]triazolo[1,5-a]pyrimidin-4(7H)-yl )acetamide **165** (20 mg) with a yield of 21.74%.

MS m/z (ESI): 748.2 [M+H]
¹H NMR (400 MHz, DMSO-d6) δ 10.29 (s, 1H), 10.22 (s, 1H), 8.58 (s, 1H), 7.99 (d, J = 8.6 Hz, 1H), 7.92 (d, J = 2.1 Hz, 1H), 7.68 (dd, J = 8.6, 2.1 Hz, 1H), 7.05 (t, J = 5.8 Hz, 1H), 5.30 (s, 2H), 4.63 (s, 2H), 4.52 (d, J = 12.6 Hz, 1H), 3.84 (t, J = 5.7 Hz, 2H), 3.52 (d, J = 11.1 Hz, 2H), 3.47 (d, J = 12.2 Hz, 1H), 3.31 - 3.19 (m, 1H), 2.99 (t, J = 8.2 Hz, 3H), 2.81 (d, J = 11.3 Hz, 1H), 2.63 (d, J = 11.1 Hz, 1H), 2.46 (s, 1H), 2.44 (s, 4H), 1.82 (p, J = 5.5 Hz, 2H), 1.19 (t, J = 7.4 Hz, 3H).

### Example 166

### N-(2-chloro-4-((methyl-d3)thio)phenyl)-2-(5-ethyl-6-(4-(5-hydroxy-6-methylpyrimidine-4-carbo nyl)piperazin-1-yl)-7-oxo-2-(2,5,6,7-tetrahydrooxepin-3-yl)-[1,2,4]triazolo[1,5-a]pyrimidin-4(7H )-yl)acetamide

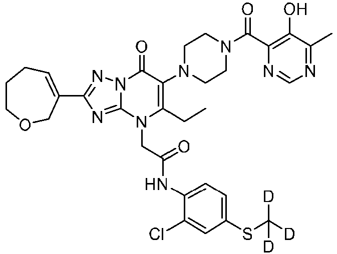

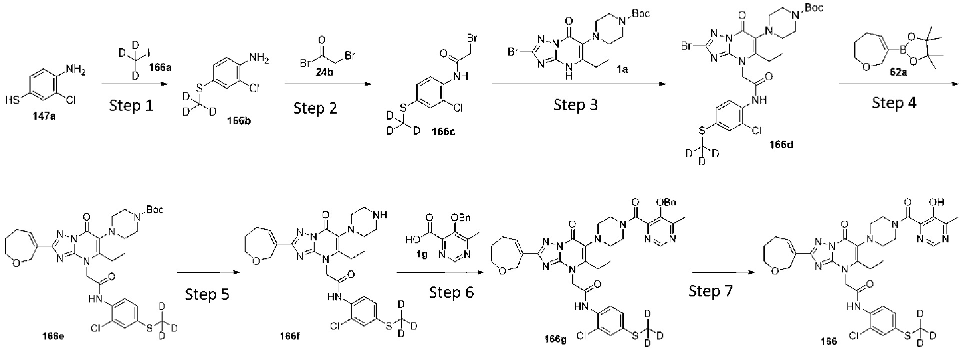

### Step 1

### 2-chloro-4-((methyl-d3)thio)aniline

Triethylamine (554.64 mg, 5.48 mmol) was added to a solution of 4-amino-3-chlorobenzenethiol **147a** (0.35 g, 2.19 mmol) in acetonitrile (6 mL). Under ice bath, deuterated iodomethane **166a** (476.72 mg, 3.29 mmol, commercially available) was added dropwise, and the reaction was performed at room temperature for 30 minutes. Water (50 mL) was added to the reaction mixture, and the mixture was extracted with ethyl acetate (30 mL × 3). The combined organic phases were washed with saturated sodium chloride solution (50 mL × 2), dried over anhydrous sodium sulfate, filtered, and the filtrate was concentrated under reduced pressure. The resulting residue was purified by thin-layer silica gel plate separation (eluent: System **B**) to obtain 2-chloro-4-((methyl-d3)thio)aniline **166b** (0.3 g) with a yield of 77.45%.

MS m/z (ESI): 177.0 [M+H]

### Step 2

### 2-bromo-N-(2-chloro-4-((methyl-d3)thio)phenyl)acetamide

4-Dimethylaminopyridine (228.19 mg, 1.87 mmol) was added to a solution of 2-chloro-4-((methyl-d3)thio)aniline **166b** (0.3 g, 1.70 mmol) in dichloromethane (6 mL). Under ice bath, 2-bromoacetyl bromide **24b** (514.08 mg, 2.55 mmol) was added dropwise, and then the reaction was performed at room temperature for 18 hours. The reaction mixture was concentrated under reduced pressure, and the residue was purified by silica gel column chromatography (eluent: System A) to obtain 2-bromo-N-(2-chloro-4-((methyl-d3)thio)phenyl)acetamide **166c** (0.4 g) with a yield of 79.15%.

MS m/z (ESI): 297.0 [M+H]

### Step 3

### tert-butyl 4-(2-bromo-4-(2-((2-chloro-4-((methyl-d3)thio)phenyl)amino)-2-oxoethyl)-5-ethyl-7-oxo-4,7-dih ydro-[1,2,4]triazolo[1,5-a]pyrimidin-6-yl)piperazine-1-carboxylate

Tert-butyl 4-(2-bromo-5-ethyl-7-oxo-4,7-dihydro-[1,2,4]triazolo[1,5-a]pyrimidin-6-yl)piperazine-1-carboxy late **1a** (344.58 mg, 806.41 µmol) and N,N-diisopropylethylamine (260.55 mg, 2.02 mmol) were added to a solution of 2-bromo-N-(2-chloro-4-((methyl-d3)thio)phenyl)acetamide **166c** (0.2 g, 672.01 µmol) in N,N-dimethylformamide (6 mL). The reaction was heated to 50 °C with stirring for 2.5 hours. After completion of the reaction, the reaction mixture was concentrated under reduced pressure and separated by silica gel column chromatography (eluent: System A) to obtain tert-butyl 4-(2-bromo-4-(2-((2-chloro-4-((methyl-d3)thio)phenyl)amino)-2-oxoethyl)-5-ethyl-7-oxo-4,7-dih ydro-[1,2,4]triazolo[1,5-a]pyrimidin-6-yl)piperazine-1-carboxylate **166d** (0.35 g) with a yield of 80.87%.

MS m/z (ESI): 543.1 [M+H-100]

### Step 4

### tert-butyl 4-(4-(2-((2-chloro-4-((methyl-d3)thio)phenyl)amino)-2-oxoethyl)-5-ethyl-7-oxo-2-(2,5,6,7-tetrah ydrooxepin-3-yl)-4,7-dihydro-[1,2,4]triazolo[1,5-a]pyrimidin-6-yl)piperazine-1-carboxylate

To a mixed solution of 4,4,5,5-tetramethyl-2-(2,5,6,7-tetrahydrooxepin-3-yl)-1,3,2-dioxaborolane **62a** (156.59 mg, 698.76 µmol) and tert-butyl 4-(2-bromo-4-(2-((2-chloro-4-((methyl-d3)thio)phenyl)amino)-2-oxoethyl)-5-ethyl-7-oxo-4,7-dih ydro-[1,2,4]triazolo[1,5-a]pyrimidin-6-yl)piperazine-1-carboxylate **166d** (300 mg, 465.84 µmol) in 1,4-dioxane (4 mL) and water (2 mL) were added [1,1'-bis(diphenylphosphino)ferrocene]palladium(II) dichloride (34.56 mg, 46.58 µmol) and sodium carbonate (98.75 mg, 931.68 µmol). The mixture was subject to argon replacement four times, heated to 80 C, and reacted for 1.5 hours. Water (20 mL) was added to the reaction mixture, and the mixture was extracted with ethyl acetate (40 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(4-(2-((2-chloro-4-((methyl-d3)thio)phenyl)amino)-2-oxoethyl)-5-ethyl-7-oxo-2-(2,5,6,7-tetrah ydrooxepin-3-yl)-4,7-dihydro-[1,2,4]triazolo[1,5-a]pyrimidin-6-yl)piperazine-1-carboxylate **166e** (120 mg) with a yield of 38.96%.

MS m/z (ESI): 605.2 [M+H-56]

### Step 5

### N-(2-chloro-4-((methyl-d3)thio)phenyl)-2-(5-ethyl-7-oxo-6-(piperazin-1-yl)-2-(2,5,6,7-tetrahydro oxepin-3-yl)-[1,2,4]triazolo[1,5-a]pyrimidin-4(7H)-yl)acetamide

Tert-butyl 4-(4-(2-((2-chloro-4-((methyl-d3)thio)phenyl)amino)-2-oxoethyl)-5-ethyl-7-oxo-2-(2,5,6,7-tetrah ydrooxepin-3-yl)-4,7-dihydro-[1,2,4]triazolo[1,5-a]pyrimidin-6-yl)piperazine-1-carboxylate **166e** (120 mg, 181.48 µmol) was dissolved in dichloromethane (5 mL), followed by dropwise addition of trifluoroacetic acid (1 g, 8.77 mmol). The reaction was performed at room temperature for 2 hours. After completion of the reaction, the mixture was concentrated under reduced pressure to obtain N-(2-chloro-4-((methyl-d3)thio)phenyl)-2-(5-ethyl-7-oxo-6-(piperazin-1-yl)-2-(2,5,6,7-tetrahydro oxepin-3-yl)-[1,2,4]triazolo[1,5-a]pyrimidin-4(7H)-yl)acetamide 166f (90 mg), which was used directly in the next step.

MS m/z (ESI): 561.2 [M+H]

### Step 6

### 2-(6-(4-(5-(benzyloxy)-6-methylpyrimidine-4-carbonyl)piperazin-1-yl)-5-ethyl-7-oxo-2-(2,5,6,7-t etrahydrooxepin-3-yl)-[1,2,4]triazolo[1,5-a]pyrimidin-4(7H)-yl)-N-(2-chloro-4-((methyl-d3)thio) phenyl)acetamide

To a solution of 5-(benzyloxy)-6-methylpyrimidine-4-carboxylic acid **1g** (62.68 mg, 256.63 µmol) in N,N-dimethylformamide (3 mL) were added N,N-diisopropylethylamine (103.65 mg, 801.98 µmol), 1-(3-dimethylaminopropyl)-3-ethylcarbodiimide hydrochloride (76.87 mg, 400.99 µmol), and 1-hydroxybenzotriazole (54.18 mg, 400.99 µmol). The reaction was performed at room temperature for 15 minutes, followed by addition of N-(2-chloro-4-((methyl-d3)thio)phenyl)-2-(5-ethyl-7-oxo-6-(piperazin-1-yl)-2-(2,5,6,7-tetrahydro oxepin-3-yl)-[1,2,4]triazolo[1,5-a]pyrimidin-4(7H)-yl)acetamide **166f** (90 mg, 160.40 µmol). The reaction was performed at room temperature for 18 hours. Water (20 mL) was added to the reaction mixture, and the mixture was extracted with dichloromethane (30 mL × 3). The combined organic phases were washed with saturated sodium chloride solution (30 mL × 2), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-5-ethyl-7-oxo-2-(2,5,6,7-t etrahydrooxepin-3-yl)-[1,2,4]triazolo[1,5-a]pyrimidin-4(7H)-yl)-N-(2-chloro-4-((methyl-d3)thio) phenyl)acetamide **166g** (70 mg) with a yield of 55.43%.

MS m/z (ESI): 787.3 [M+H]

### Step 7

### N-(2-chloro-4-((methyl-d3)thio)phenyl)-2-(5-ethyl-6-(4-(5-hydroxy-6-methylpyrimidine-4-carbo nyl)piperazin-1-yl)-7-ox0-2-(2,5,6,7-tetrahydrooxepin-3-yl)-[1,2,4]triazolo[1,5-a]pyrimidin-4(7H )-yl)acetamide

2-(6-(4-(5-(Benzyloxy)-6-methylpyrimidine-4-carbonyl)piperazin-1-yl)-5-ethyl-7-oxo-2 -(2,5,6,7-tetrahydrooxepin-3-yl)-[1,2,4]triazolo[1,5-a]pyrimidin-4(7H)-yl)-N-(2-chloro-4-((methy l-d3)thio)phenyl)acetamide **166g** (70 mg, 88.91 µmol) was dissolved in trifluoroacetic acid (5 mL), heated to 40 °C, and reacted for 3 hours. The mixture was concentrated under reduced pressure, and the residue was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-((methyl-d3)thio)phenyl)-2-(5-ethyl-6-(4-(5-hydroxy-6-methylpyrimidine-4-carbo nyl)piperazin-1-yl)-7-oxo-2-(2,5,6,7-tetrahydrooxepin-3-yl)-[1,2,4]triazolo[1,5-a]pyrimidin-4(7H )-yl)acetamide **166** (30 mg) with a yield of 45.98%.

MS m/z (ESI): 697.3 [M+H]
¹H NMR (400 MHz, DMSO-d6) δ 10.21 (s, 1H), 10.05 (s, 1H), 8.57 (s, 1H), 7.58 (d, J = 8.5 Hz, 1H), 7.39 (d, J = 2.2 Hz, 1H), 7.22 (dd, J = 8.5, 2.2 Hz, 1H), 7.07 (t, J = 5.8 Hz, 1H), 5.20 (s, 2H), 4.63 (s, 2H), 4.52 (d, J = 12.6 Hz, 1H), 3.84 (t, J = 5.8 Hz, 2H), 3.51 (t, J = 10.6 Hz, 3H), 3.23 (d, J = 11.4 Hz, 1H), 2.98 (p, J = 8.7, 7.6 Hz, 3H), 2.81 (d, J = 11.4 Hz, 1H), 2.63 (d, J = 11.2 Hz, 1H), 2.46 (d, J = 5.8 Hz, 2H), 2.44 (s, 3H), 1.82 (p, J = 5.6 Hz, 2H), 1.19 (t, J = 7.4 Hz, 3H).

### Example 167

### 2-(2-(bicyclo[3.1.0]hex-2-en-3-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(2-chloro-4-(pentafluoro-λ⁶-sulfane yl)phenyl)acetamide

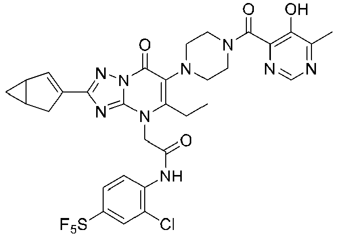

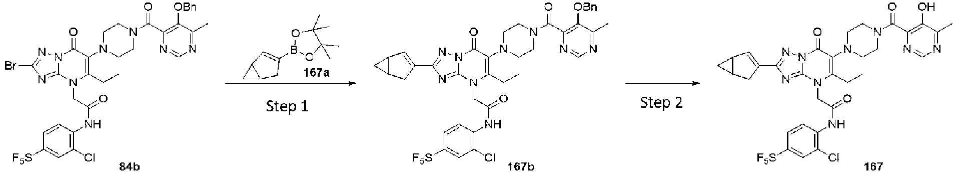

### Step 1

### 2-(6-(4-(5-(benzyloxy)-6-methylpyrimidine-4-carbonyl)piperazin-1-yl)-2-(bicyclo[3.1.0]hex-2-en -3-yl)-5-ethyl-7-oxo-[1,2,4]triazolo[1,5-a]pyrimidin-4(7H)-yl)-N-(2-chloro-4-(pentafluoro-λ⁶-sulf aneyl)phenyl)acetamide

2-(bicyclo[3.1.0]hex-2-en-3-yl)-4,4,5,5-tetramethyl-1,3,2-dioxaborolane **167a** (35.04 mg, 170.00 µmol, prepared according to published patent WO2021002986), 2-(2-bromo-5-ethyl-7-oxo-6-(piperazin-1-yl)-[1,2,4]triazolo[1,5-a]pyrimidin-4(7H)-yl)-N-(2-chlo ro-4-(pentafluoro-λ⁶-sulfaneyl)phenyl)acetamide **84a** (80 mg, 94.45 µmol), [1,1'-bis(diphenylphosphino)ferrocene]palladium(II) dichloride (7.01 mg, 9.44 µmol), and sodium carbonate (20.02 mg, 188.89 µmol) were added to a mixed solution of 1,4-dioxane (2 mL) and water (0.5 mL). The mixture was heated to 80 °C and stirred for 2 hours. Water (20 mL) was added to the reaction mixture, which was extracted with ethyl acetate (40 mL × 3). The combined organic phases were washed with saturated sodium chloride solution (20 mL), dried over anhydrous sodium sulfate, filtered, and concentrated under reduced pressure. The residue was purified by silica gel column chromatography (eluent: System B) to obtain 2-(6-(4-(5-(benzyloxy)-6-methylpyrimidine-4-carbonyl)piperazin-1-yl)-2-(bicyclo[3.1.0]hex-2-en -3-yl)-5-ethyl-7-oxo-[1,2,4]triazolo[1,5-a]pyrimidin-4(7H)-yl)-N-(2-chloro-4-(pentafluoro-λ⁶-sulf aneyl)phenyl)acetamide **167b** (45 mg) with a yield of 56.4%.

MS m/z (ESI): 846.2 [M+H]

### Step 2

### 2-(2-(bicyclo[3.1.0]hex-2-en-3-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(2-chloro-4-(pentafluoro-λ⁶-sulfane yl)phenyl)acetamide

2-(6-(4-(5-(Benzyloxy)-6-methylpyrimidine-4-carbonyl)piperazin-1-yl)-2-(bicyclo [3.1.0]hex-2-en-3-yl)-5-ethyl-7-oxo-[1,2,4]triazolo[1,5-a]pyrimidin-4(7H)-yl)-N-(2-chloro-4-(pe ntafluoro-λ⁶-sulfaneyl)phenyl)acetamide **167b** (45 mg, 53.17 µmol) was dissolved in trifluoroacetic acid (3 mL). The mixture was heated to 45 °C and reacted for 3 hours. The mixture was concentrated under reduced pressure, and the residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 2-(2-(bicyclo[3.1.0]hex-2-en-3-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(2-chloro-4-(pentafluoro-λ⁶-sulfane yl) phenyl)acetamide **167** (10 mg) with a yield of 23.63%.

MS m/z (ESI): 756.2 [M+H]

### Example 168

### 2-(2-(2,3-dihydrobenzofuran-5-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(6-methylbenzo[b]thiophen-5-yl)ac etamide

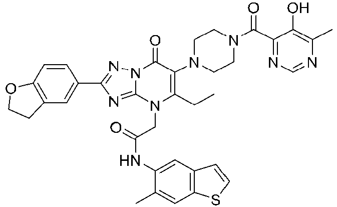

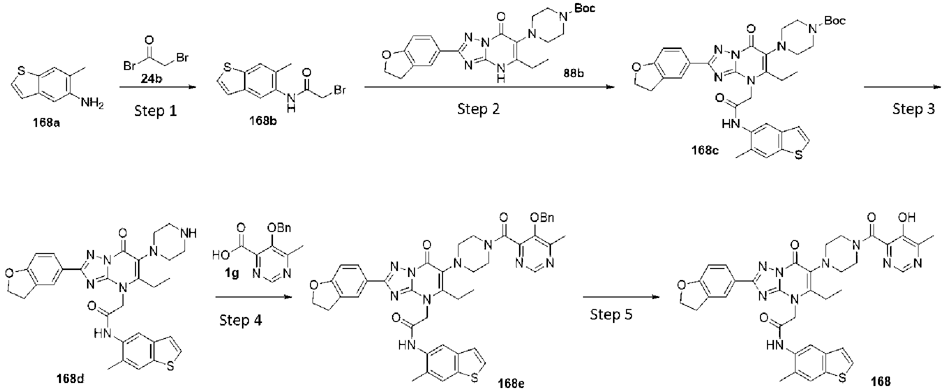

### Step 1

### 2-bromo-N-(6-methylbenzo[b]thiophen-5-yl)acetamide

Under ice bath, 4-dimethylaminopyridine (37.42 mg, 306.30 µmol) and 2-bromoacetyl bromide 24b (61.82 mg, 306.30 µmol) were added to a solution of 6-methylbenzo[b]thiophen-5-amine **168a** (50 mg, 306.30 µmol, commercially available) in dichloromethane (3 mL). The reaction mixture was stirred at room temperature for 18 hours. The reaction mixture was concentrated under reduced pressure, and the residue was purified by silica gel column chromatography (eluent: System A) to obtain 2-bromo-N-(6-methylbenzo[b]thiophen-5-yl)acetamide **168b** (60 mg) with a yield of 68.93%.

MS m/z (ESI): 283.9 [M+H]

### Step 2

### tert-butyl 4-(2-(2,3-dihydrobenzofuran-5-yl)-5-ethyl-4-(2-((6-methylbenzo[b]thiophen-5-yl)amino)-2-oxoet hyl)-7-oxo-4,7-dihydro-[1,2,4]triazolo[1,5-a]pyrimidin-6-yl)piperazine-1-carboxylate

2-Bromo-N-(6-methylbenzo[b]thiophen-5-yl)acetamide **168b** (60.91 mg, 214.35 µmol) and N,N-diisopropylethylamine (83.11 mg, 643.04 µmol) were added to a solution of tert-butyl 4-(2-(2,3-dihydrobenzofuran-5-yl)-5-ethyl-7-oxo-4,7-dihydro-[1,2,4]triazolo[1,5-a]pyrimidin-6-y l)piperazine-1-carboxylate **88b** (100 mg, 214.35 µmol) in N,N-dimethylformamide (3 mL). The mixture was heated to 50 °C and reacted for 2 hours. The reaction mixture was concentrated under reduced pressure and purified by silica gel column chromatography (eluent: System A) to obtain tert-butyl 4-(2-(2,3-dihydrobenzofuran-5-yl)-5-ethyl-4-(2-((6-methylbenzo[b]thiophen-5-yl)amino)-2-oxoet hyl)-7-oxo-4,7-dihydro-[1,2,4]triazolo[1,5-a]pyrimidin-6-yl)piperazine-1-carboxylate **168c** (70 mg) with a yield of 48.76%.

MS m/z (ESI): 670.3 [M+H]

### Step 3

### 2-(2-(2,3-dihydrobenzofuran-5-yl)-5-ethyl-7-oxo-6-(piperazin-1-yl)-[1,2,4]triazolo[1,5-a]pyrimid in-4(7H)-yl)-N-(6-methylbenzo[b]thiophen-5-yl)acetamide

Tert-butyl 4-(2-(2,3-dihydrobenzofuran-5-yl)-5-ethyl-4-(2-((6-methylbenzo[b]thiophen-5-yl)amino)-2-oxoet hyl)-7-oxo-4,7-dihydro-[1,2,4]triazolo[1,5-a]pyrimidin-6-yl)piperazine-1-carboxylate **168c** (70 mg, 104.51 µmol) was dissolved in dichloromethane (3 mL). Trifluoroacetic acid (1.40 g, 12.28 mmol) was added dropwise, and the mixture was reacted at room temperature for 3 hours. The mixture was concentrated under reduced pressure to obtain 2-(2-(2,3-dihydrobenzofuran-5-yl)-5-ethyl-7-oxo-6-(piperazin-1-yl)-[1,2,4]triazolo[1,5-a]pyrimid in-4(7H)-yl)-N-(6-methylbenzo[b]thiophen-5-yl)acetamide **168d** (40 mg), which was used directly in the next step.

MS m/z (ESI): 570.2 [M+H]

### Step 4

### 2-(6-(4-(5-(benzyloxy)-6-methylpyrimidine-4-carbonyl)piperazin-1-yl)-2-(2,3-dihydrobenzofuran -5-yl)-5-ethyl-7-oxo-[1,2,4]triazolo[1,5-a]pyrimidin-4(7H)-yl)-N-(6-methylbenzo[b]thiophen-5-y l)acetamide

To a solution of 5-(benzyloxy)-6-methylpyrimidine-4-carboxylic acid **1g** (27.44 mg, 112.34 µmol) in N,N-dimethylformamide (2 mL) were added 1-(3-dimethylaminopropyl)-3-ethylcarbodiimide hydrochloride (26.92 mg, 140.43 µmol), 1-hydroxybenzotriazole (18.97 mg, 140.43 µmol), and N,N-diisopropylethylamine (45.37 mg, 351.08 µmol), and the reaction was performed at room temperature for 10 minutes. Then 2-(2-(2,3-dihydrobenzofuran-5-yl)-5-ethyl-7-oxo-6-(piperazin-1-yl)-[1,2,4]triazolo[1,5-a]pyrimid in-4(7H)-yl)-N-(6-methylbenzo[b]thiophen -5-yl)acetamide **168d** (40 mg, 70.22 µmol) was added, and the reaction was performed at room temperature for 18 hours. Water (20 mL) was added to the reaction mixture, and the mixture was extracted with dichloromethane (30 mL × 3). The combined organic phases were washed with saturated sodium chloride solution (30 mL × 2), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-2-(2,3-dihydrobenzofuran -5-yl)-5-ethyl-7-oxo-[1,2,4]triazolo[1,5-a]pyrimidin-4(7H)-yl)-N-(6-methylbenzo[b]thiophen-5-y l)acetamide **168e** (45 mg) in a yield of 80.52%.

MS m/z (ESI): 796.3 [M+H]

### Step 5

### 2-(2-(2,3-dihydrobenzofuran-5-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(6-methylbenzo[b]thiophen-5-yl)ac etamide

2-(6-(4-(5-(Benzyloxy)-6-methylpyrimidine-4-carbonyl)piperazin-1-yl)-2-(2,3-dihydrob enzofuran-5-yl)-5-ethyl-7-oxo-[1,2,4]triazolo[1,5-a]pyrimidin-4(7H)-yl)-N-(6-methylbenzo[b]thi ophen-5-yl)acetamide **168e** (45 mg, 56.54 µmol) was dissolved in trifluoroacetic acid (5 mL), and the reaction was performed at 40 °C for 3 hours. The mixture was concentrated under reduced pressure, and the residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 2-(2-(2,3-dihydrobenzofuran-5-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(6-methylbenzo[b]thiophen-5-yl)ac etamide **168** (10 mg) in a yield of 22.55%.

MS m/z (ESI): 706.3 [M+H]
¹H NMR (400 MHz, DMSO-d6) δ 10.23 (s, 1H), 9.91 (s, 1H), 8.58 (s, 1H), 8.00 (d, J = 1.7 Hz, 1H), 7.94 - 7.85 (m, 2H), 7.84 (d, J = 6.7 Hz, 1H), 7.66 (d, J = 5.4 Hz, 1H), 7.35 (d, J = 5.5 Hz, 1H), 6.90 (d, J = 8.4 Hz, 1H), 5.27 (s, 2H), 4.61 (t, J = 8.7 Hz, 2H), 4.54 (d, J = 12.5 Hz, 1H), 3.60 - 3.47 (m, 4H), 3.26 (t, J = 9.4 Hz, 4H), 3.05 (s, 1H), 3.06 - 2.96 (m, 2H), 2.84 (d, J = 9.7 Hz, 2H), 2.66 (d, J = 11.6 Hz, 1H), 2.51 - 2.41 (m, 1H), 2.45 (s, 3H), 2.37 (s, 3H), 2.07 (s, 1H), 1.23 (dt, J = 12.8, 7.4 Hz, 3H).

### Example 169

### 2-(2-(benzo[b]thiophen-5-yl-2-d)-5-ethyl-6-(4-(5-hydroxy-6-methylpyrimidine-4-carbonyl)pipera zin-1-yl)-7-oxo-[1,2,4]triazolo[1,5-a]pyrimidin-4(7H)-yl)-N-(2-chloro-4-(trifluoromethyl)phenyl) acetamide

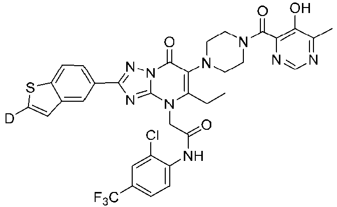

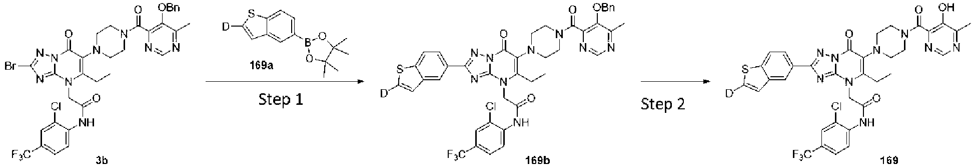

### Step 1

### 2-(2-(benzo[b]thiophen-5-yl-2-d)-6-(4-(5-(benzyloxy)-6-methylpyrimidine-4-carbonyl)piperazin-1-yl)-5-ethyl-7-oxo-[1,2,4]triazolo[1,5-a]pyrimidin-4(7H)-yl)-N-(2-chloro-4-(trifluoromethyl)phe nyl)acetamide

2-(6-(4-(5-(Benzyloxy)-6-methylpyrimidine-4-carbonyl)piperazin-1-yl)-2-bromo-5-ethy 1-7-oxo-[1,2,4]triazolo[1,5-a]pyrimidin-4(7H)-yl)-N-(2-chloro-4-(trifluoromethyl)phenyl)acetami de **3b** (50 mg, 63.37 µmol), 2-(benzo[b]thiophen-5-yl-2-d)-4,4,5,5-tetramethyl-1,3,2-dioxaborolane **169a** (21.52 mg, 82.38 µmol, prepared according to published patent CN116640127), [1,1'-bis(diphenylphosphino)ferrocene]palladium(II) dichloride (4.70 mg, 6.34 µmol), and sodium carbonate (13.43 mg, 126.74 µmol) were dissolved in a mixed solution of 1,4-dioxane (2 mL) and water (0.5 mL). After the mixture was subject to argon replacement three times, the temperature was raised to 80 °C, and the reaction was performed for 2 hours. Water (20 mL) was added to the reaction mixture, and the mixture was extracted with dichloromethane (30 mL × 3). The combined organic phases were washed with saturated sodium chloride solution (30 mL × 2), dried over anhydrous sodium sulfate, filtered, and concentrated under reduced pressure. The resulting residue was purified by silica gel column chromatography (eluent: System B) to obtain 2-(2-(benzo[b]thiophen-5-yl-2-d)-6-(4-(5-(benzyloxy)-6-methylpyrimidine-4-carbonyl)piperazin-l-yl)-5-ethyl-7-oxo-[1,2,4]triazolo[1,5-a]pyrimidin-4(7H)-yl)-N-(2-chloro-4-(trifluoromethyl)phe nyl)acetamide **169b** (30 mg) in a yield of 56.14%.

MS m/z (ESI): 843.2 [M+H]

### Step 2

### 2-(2-(benzo[b]thiophen-5-yl-2-d)-5-ethyl-6-(4-(5-hydroxy-6-methylpyrimidine-4-carbonyl)pipera zin-1-yl)-7-oxo-[1,2,4]triazolo[1,5-a]pyrimidin-4(7H)-yl)-N-(2-chloro-4-(trifluoromethyl)phenyl) acetamide

2-(2-(Benzo[b]thiophen-5-yl-2-d)-6-(4-(5-(benzyloxy)-6-methylpyrimidine-4-carbonyl) piperazin-1-yl)-5-ethyl-7-oxo-[1,2,4]triazolo[1,5-a]pyrimidin-4(7H)-yl)-N-(2-chloro-4-(trifluoro methyl)phenyl)acetamide **169b** (30 mg, 35.57 µmol) was dissolved in trifluoroacetic acid (3 mL), heated to 45 °C, and reacted for 3 h. The mixture was concentrated under reduced pressure, and the residue was purified by preparative liquid chromatography (column AKZONOBEL Kromasil; 250 ×21.2 mm I.D.; 5 µm, 20 mL/min; mobile phase A: 0.05% TFA+H₂O, mobile phase B: CH₃CN) to obtain 2-(2-(benzo[b]thiophen-5-yl-2-d)-5-ethyl-6-(4-(5-hydroxy-6-methylpyrimidine-4-carbonyl)pipera zin-1-yl)-7-oxo-[1,2,4]triazolo[1,5-a]pyrimidin-4(7H)-yl)-N-(2-chloro-4-(trifluoromethyl)phenyl) acetamide **169** (15 mg) in a yield of 54.30%.

MS m/z (ESI): 753.2 [M+H]
NMR showed 84% deuterium at position 2 and 10% at position 3.

¹H NMR (400 MHz, DMSO-d6) δ 10.44 (s, 1H), 10.25 (s, 1H), 8.65 (d, J = 1.5 Hz, 1H), 8.59 (s, 1H), 8.18 - 8.04 (m, 3H), 7.97 (d, J = 2.1 Hz, 1H), 7.71 (dd, J = 8.7, 2.1 Hz, 1H), 7.60 (s, 1H), 5.43 (s, 2H), 4.55 (d, J = 12.3 Hz, 1H), 3.72 (s, 6H), 3.62 - 3.49 (m, 3H), 3.34 - 3.23 (m, 1H), 3.04 (d, J = 9.2 Hz, 3H), 2.90 - 2.82 (m, 1H), 2.68 (d, J = 13.9 Hz, 1H), 2.46 (s, 3H), 1.23 (t, J = 7.4 Hz, 3H).

### Example 170

### 2-(2-(benzo[b]thiophen-5-yl-2,3-d2)-5-ethyl-6-(4-(5-hydroxy-6-methylpyrimidine-4-carbonyl)pip erazin-1-yl)-7-oxo-[1,2,4]triazolo9[1,5-a]pyrimidin-4(7H)-yl)-N-(2-chloro-4-(trifluoromethyl)phe nyl)acetamide

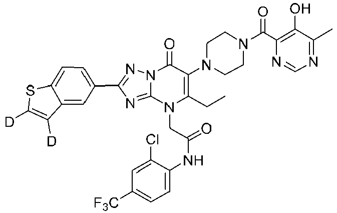

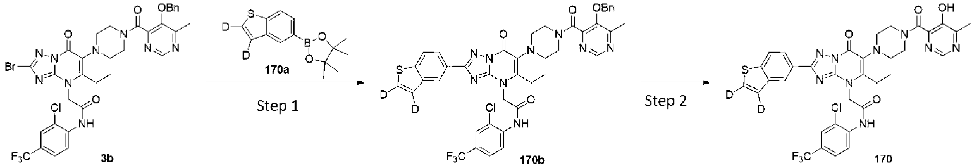

### Step 1

### 2-(2-(benzo[b]thiophen-5-yl-2,3-d2)-6-(4-(5-(benzyloxy)-6-methylpyrimidine-4-carbonyl)piperaz in-1-yl)-5-ethyl-7-oxo-[1,2,4]triazolo[1,5-a]pyrimidin-4(7H)-yl)-N-(2-chloro-4-(trifluoromethyl) phenyl)acetamide

2-(6-(4-(5-(Benzyloxy)-6-methylpyrimidine-4-carbonyl)piperazin-1-yl)-2-bromo-5-ethy 1-7-oxo-[1,2,4]triazolo[1,5-a]pyrimidin-4(7H)-yl)-N-(2-chloro-4-(trifluoromethyl)phenyl) acetamide **3b** (50 mg, 63.37 µmol), 2-(benzo[b]thiophen-5-yl-2,3-d2)-4,4,5,5-tetramethyl-1,3,2-dioxaborolane **170a** (21.60 mg, 82.38 µmol), [1,1'-bis(diphenylphosphino)ferrocene]palladium(II) dichloride (4.70 mg, 6.34 µmol), and sodium carbonate (13.43 mg, 126.74 µmol) were dissolved in 1,4-dioxane (2 mL) and water (0.5 mL). After the mixture was subject to argon replacement three times, the mixture was reacted at 80 °C for 2 h. Water (20 mL) was added to the reaction mixture, and the mixture was extracted with dichloromethane (30 mL × 3). The combined organic phases were washed with saturated sodium chloride solution (30 mL × 2), dried over anhydrous sodium sulfate, filtered, and concentrated under reduced pressure. The resulting residue was purified by silica gel column chromatography (eluent: System B) to obtain 2-(2-(benzo[b]thiophen-5-yl-2,3-d2)-6-(4-(5-(benzyloxy)-6-methylpyrimidine-4-carbonyl)piperaz in-1-yl)-5-ethyl-7-oxo-[1,2,4]triazolo[1,5-a]pyrimidin-4(7H)-yl)-N-(2-chloro-4-(trifluoromethyl) phenyl)acetamide **170b** (40 mg) in a yield of 74.76%.

MS m/z (ESI): 844.2 [M+H]

### Step 2

### 2-(2-(benzo[b]thiophen-5-yl-2,3-d2)-5-ethyl-6-(4-(5-hydroxy-6-methylpyrimidine-4-carbonyl)pip erazin-1-yl)-7-oxo-[1,2,4]triazolo[1,5-a]pyrimidin-4(7H)-yl)-N-(2-chloro-4-(trifluoromethyl)phe nyl)acetamide

2-(2-(Benzo[b]thiophen-5-yl-2,3-d2)-6-(4-(5-(benzyloxy)-6-methylpyrimidine-4-carbon yl)piperazin-1-yl)-5-ethyl-7-oxo-[1,2,4]triazolo[1,5-a]pyrimidin-4(7H)-yl)-N-(2-chloro-4-(trifluo romethyl)phenyl)acetamide **170b** (40 mg, 47.38 µmol) was dissolved in trifluoroacetic acid (3 mL) and reacted at 45 C for 3 h. The mixture was concentrated under reduced pressure, and the residue was purified by preparative liquid chromatography (column AKZONOBEL Kromasil; 250 ×21.2 mm I.D.; 5 µm, 20 mL/min; mobile phase A: 0.05% TFA+H₂O, mobile phase B: CH₃CN) to obtain 2-(2-(benzo[b]thiophen-5-yl-2,3-d2)-5-ethyl-6-(4-(5-hydroxy-6-methylpyrimidine-4-carbonyl)pip erazin-1-yl)-7-oxo-[1,2,4]triazolo[1,5-a]pyrimidin-4(7H)-yl)-N-(2-chloro-4-(trifluoromethyl)phe nyl)acetamide **170** (15 mg) in a yield of 39.88%.

MS m/z (ESI): 754.2 [M+H]
NMR: 93% deuterium at position 2 and 78% at position 3.

¹H NMR (400 MHz, DMSO-d6) δ 10.45 (s, 1H), 10.24 (s, 1H), 8.65 (d, J = 1.5 Hz, 1H), 8.59 (s, 1H), 8.15 (d, J = 8.5 Hz, 1H), 8.13 - 8.04 (m, 2H), 7.97 (d, J = 2.1 Hz, 1H), 7.71 (dd, J = 8.7, 2.1 Hz, 1H), 5.43 (s, 2H), 4.55 (d, J = 12.5 Hz, 1H), 3.62 - 3.49 (m, 3H), 3.31 - 3.23 (m, 1H), 3.04 (d, J = 9.3 Hz, 3H), 2.86 (d, J = 11.4 Hz, 1H), 2.68 (d, J = 14.2 Hz, 1H), 2.46 (s, 3H), 1.23 (t, J = 7.5 Hz, 3H).

### Example 171

### N-(2-chloro-4-(trifluoromethyl)phenyl)-2-(5-ethyl-6-(4-(5-hydroxy-6-methylpyrimidine-4-carbon yl)piperazin-1-yl)-2-(2-morpholinobenzo[d]thiazol-6-yl)-7-oxo-[1,2,4]triazolo[1,5-a]pyrimidin-4( 7H)-yl)acetamide

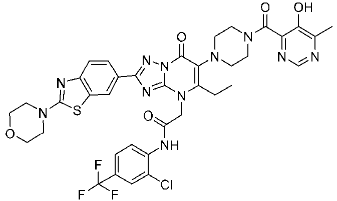

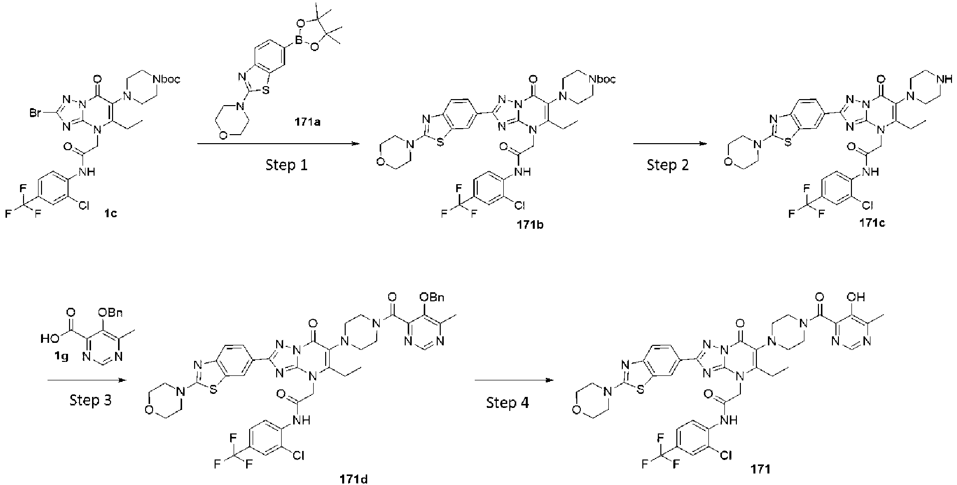

### Step 1

### tert-butyl 4-(4-(2-((2-chloro-4-(trifluoromethyl)phenyl)amino)-2-oxoethyl)-5-ethyl-2-(2-morpholinobenzo[ d]thiazol-6-yl)-7-oxo-4,7-dihydro-[1,2,4]triazolo[1,5-a]pyrimidin-6-yl)piperazine-l-carboxylate

Tert-butyl 4-(2-bromo-4-(2-((2-chloro-4-(trifluoromethyl)phenyl)amino)-2-oxoethyl)-5-ethyl-7-oxo-4,7-dih ydro-[1,2,4]triazolo[1,5-a]pyrimidin-6-yl)piperazine-1-carboxylate **1c** (150 mg, 226.28 µmol), 4-(6-(4,4,5,5-tetramethyl-1,3,2-dioxaborolan-2-yl)benzo[d]thiazol-2-yl)morpholine **171a** (101.86 mg, 294.17 µmol, prepared according to the published literature "Journal of the American Chemical Society (2017), 139(24), 8267-8276"), [1,1'-bis(diphenylphosphino)ferrocene]palladium(II) dichloride (16.79 mg, 22.63 µmol), and sodium carbonate (47.97 mg, 452.57 µmol) were added to 1,4-dioxane (3 mL) and water (1 mL). The mixture was subject to argon replacement three times, heated to 80 °C, and reacted for 2 hours. Water (20 mL) was added to the reaction mixture, and the mixture was extracted with dichloromethane (30 mL × 3). The combined organic phases were washed with saturated sodium chloride solution (30 mL × 2), dried over anhydrous sodium sulfate, filtered, and concentrated under reduced pressure. The resulting residue was purified by silica gel column chromatography (eluent: System B) to obtain tert-butyl 4-(4-(2-((2-chloro-4-(trifluoromethyl)phenyl)amino)-2-oxoethyl)-5-ethyl-2-(2-morpholinobenzo[ d]thiazol-6-yl)-7-oxo-4,7-dihydro-[1,2,4]triazolo[1,5-a]pyrimidin-6-yl)piperazine-1-carboxylate **171b** (50 mg) with a yield of 27.54%.

MS m/z (ESI): 802.3 [M+H]

### Step 2

### N-(2-Chloro-4-(trifluoromethyl)phenyl)-2-(5-ethyl-2-(2-morpholinobenzo[d]thiazo1-6-y1)-7-oxo-6-(piperazin-1-yl)-[1,2,4]triazolo[1,5-a]pyrimidin-4(7H)-yl)acetamide

Trifluoroacetic acid (1 g, 8.77 mmol) was added dropwise to a solution of tert-butyl 4-(4-(2-((2-chloro-4-(trifluoromethyl)phenyl)amino)-2-oxoethyl)-5-ethyl-2-(2-morpholinobenzo[ d] thiazol-6-yl)-7-oxo-4,7-dihydro-[1,2,4]triazolo[1,5-a]pyrimidin-6-yl)piperazine-1-carboxylate **171b** (50 mg, 62.32 µmol) in dichloromethane (5 mL). The reaction was performed at room temperature for 2 hours. The mixture was concentrated under reduced pressure to obtain N-(2-chloro-4-(trifluoromethyl)phenyl)-2-(5-ethyl-2-(2-morpholinobenzo[d]thiazol-6-yl)-7-oxo-6 -(piperazin-1-yl)-[1,2,4]triazolo[1,5-a]pyrimidin-4(7H)-yl)acetamide **171c** (30 mg), which was used directly in the next step.

MS m/z (ESI): [M+H]+ = 702.2

### Step 3

### 2-(6-(4-(5-(Benzyloxy)-6-methylpyrimidine-4-carbonyl)piperazin-1-yl)-5-ethyl-2-(2-morpholino benzo[d]thiazol-6-yl)-7-oxo-[1,2,4]triazolo[1,5-a]pyrimidin-4(7H)-yl)-N-(2-chloro-4-(trifluorom ethyl)phenyl) acetamide

1-(3-Dimethylaminopropyl)-3-ethylcarbodiimide hydrochloride (20.48 mg, 106.82 µmol), 1-hydroxybenzotriazole (14.43 mg, 106.82 µmol), and N,N-diisopropylethylamine (27.61 mg, 213.63 µmol) were added to a solution of 5-(benzyloxy)-6-methylpyrimidine-4-carboxylic acid **1g** (18.78 mg, 76.91 µmol) in N,N-dimethylformamide (2 mL). The reaction mixture was stirred at room temperature for 15 minutes, then a solution of N-(2-chloro-4-(trifluoromethyl)phenyl)-2-(5-ethyl-2-(2-morpholinobenzo[d]thiazol-6-yl)-7-oxo-6 -(piperazin-1-yl)-[1,2,4]triazolo[1,5-a]pyrimidin-4(7H)-yl)acetamide **171c** (30 mg, 42.73 µmol) in N,N-dimethylformamide (2 mL) was added. The reaction mixture was stirred at room temperature for 18 hours. Water (20 mL) was added to the reaction mixture, and the mixture was extracted with dichloromethane (30 mL × 3). The combined organic phases were washed with saturated sodium chloride solution (30 mL × 2), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-5-ethyl-2-(2-morpholinob enzo[d]thiazol-6-yl)-7-oxo-[1,2,4]triazolo[1,5-a]pyrimidin-4(7H)-yl)-N-(2-chloro-4-(trifluoromet hyl)phenyl)acetamide **171d** (25 mg) with a yield of 63.03%.

MS m/z (ESI): 928.3 [M+H]

### Step 4

### N-(2-chloro-4-(trifluoromethyl)phenyl)-2-(5-ethyl-6-(4-(5-hydroxy-6-methylpyrimidine-4-carbon yl)piperazin-1-yl)-2-(2-morpholinobenzo[d]thiazol-6-yl)-7-oxo-[1,2,4]triazolo[1,5-a]pyrimidin-4( 7H)-yl)acetamide

2-(6-(4-(5-(Benzyloxy)-6-methylpyrimidine-4-carbonyl)piperazin-1-yl)-5-ethyl-2-(2-m orpholinobenzo[d]thiazol-6-yl)-7-oxo-[1,2,4]triazolo[1,5-a]pyrimidin-4(7H)-yl)-N-(2-chloro-4-(tr ifluoromethyl)phenyl)acetamide **171d** (25 mg, 26.93 µmol) was dissolved in trifluoroacetic acid (3 mL). The mixture was heated to 45 °C and reacted for 3 hours. The mixture was concentrated under reduced pressure, and the residue was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-2-(2-morpholinobenzo[d]thiazol-6-yl)-7-oxo-[1,2,4]triazolo[1,5-a]pyrimidin-4( 7H)-yl)acetamide **171** (12 mg) with a yield of 50.50%.

MS m/z (ESI): 838.2 [M+H]
¹H NMR (400 MHz, DMSO-d6) δ 10.41 (s, 1H), 10.23 (s, 1H), 8.59 (s, 1H), 8.52 (d, J = 1.8 Hz, 1H), 8.10 - 8.00 (m, 2H), 7.97 (d, J = 2.1 Hz, 1H), 7.71 (dd, J = 8.7, 2.2 Hz, 1H), 7.56 (d, J = 8.5 Hz, 1H), 5.40 (s, 2H), 4.54 (d, J = 12.6 Hz, 1H), 3.74 (t, J = 4.8 Hz, 4H), 3.60 (t, J = 4.9 Hz, 4H), 3.57 - 3.50 (m, 2H), 3.27 (s, 1H), 3.07 -2.99 (m, 3H), 2.85 (d, J = 11.0 Hz, 1H), 2.67 (d, J = 9.5 Hz, 1H), 2.45 (s, 3H), 1.22 (t, J = 7.5 Hz, 3H).

### Example 172

### N-(2-chloro-4-(trifluoromethyl)phenyl)-2-(5-ethyl-6-(4-(5-hydroxy-6-methylpyrimidine-4-carbon yl)piperazin-1-yl)-2-(2-morpholinobenzo[d]oxazol-6-yl)-7-oxo-[1,2,4]triazolo[1,5-a]pyrimidin-4( 7H)-yl)acetamide

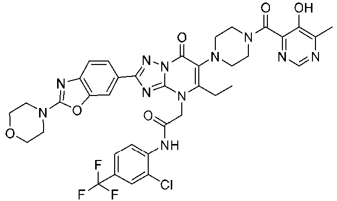

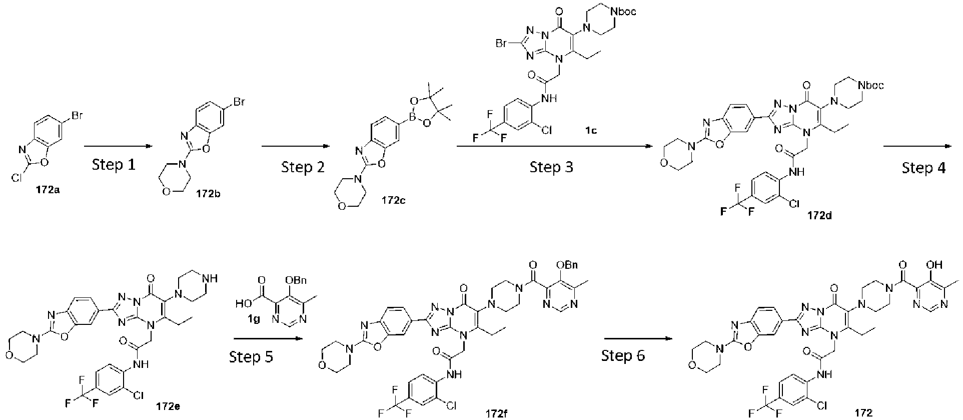

### Step 1

### 6-Bromo-2-morpholinobenzo[d]oxazole

Morpholine (1.50 g, 17.21 mmol) was added to a solution of 6-bromo-2-chlorobenzo[d]oxazole **172a** (0.4 g, 1.72 mmol, commercially available) in tetrahydrofuran (5 mL). The mixture was heated to 70 °C and reacted for 3 hours. The mixture was concentrated under reduced pressure, and the crude product was purified by silica gel column chromatography (eluent: System A) to obtain 6-bromo-2-morpholinobenzo[d]oxazole **172b** (0.35 g) with a yield of 71.84%.

MS m/z (ESI): 282.9 [M+H]

### Step 2

### 2-morpholino-6-(4,4,5,5-tetramethyl-1,3,2-dioxaborolan-2-yl)benzo[d]oxazole

6-Bromo-2-morpholinobenzo[d]oxazole **172b** (100 mg, 353.21 µmol), 4,4,5,5-tetramethyl-2-(4,4,5,5-tetramethyl-1,3,2-dioxaborolan-2-yl)-1,3,2-dioxaborolane (107.63 mg, 423.85 µmol), [1,1'-bis(diphenylphosphino)ferrocene]palladium(II) dichloride (26.20 mg, 35.32 µmol), and potassium acetate (52.00 mg, 529.81 µmol) were dissolved in 1,4-dioxane (3 mL). The mixture was subject to argon replacement three times, heated to 75 °C, and reacted for 4 h. Water (20 mL) was added to the reaction mixture, which was extracted with ethyl acetate (30 mL × 3). The combined organic phase was washed with saturated sodium chloride solution (30 mL × 2), dried over anhydrous sodium sulfate, filtered, and concentrated under reduced pressure. The resulting residue was purified by silica gel column chromatography (eluent: System A) to obtain 2-morpholino-6-(4,4,5,5-tetramethyl-1,3,2-dioxaborolan-2-yl)benzo[d]oxazole **172c** (80 mg) in a yield of 68.60%.

MS m/z (ESI): 331.2 [M+H]

### Step 3

### tert-butyl 4-(4-(2-((2-chloro-4-(trifluoromethyl)phenyl)amino)-2-oxoethyl)-5-ethyl-2-(2-morpholinobenzo[ d]oxazol-6-yl)-7-oxo-4,7-dihydro-[1,2,4]triazolo[1,5-a]pyrimidin-6-yl)piperazine-1-carboxylate

Tert-butyl 4-(2-bromo-4-(2-((2-chloro-4-(trifluoromethyl)phenyl)amino)-2-oxoethyl)-5-ethyl-7-oxo-4,7-dih ydro-[1,2,4]triazolo[1,5-a]pyrimidin-6-yl)piperazine-1-carboxylate **1c** (150 mg, 226.28 µmol), 2-morpholino-6-(4,4,5,5-tetramethyl-1,3,2-dioxaborolan-2-yl)benzo[d]oxazole **172c** (97.13 mg, 294.17 µmol), [1,1'-bis(diphenylphosphino)ferrocene]palladium(II) dichloride (16.79 mg, 22.63 µmol), and sodium carbonate (47.97 mg, 452.57 µmol) were dissolved in 1,4-dioxane (3 mL) and water (1 mL). The mixture was subject to argon replacement three times, heated to 80 °C, and reacted for 2 h. Water (20 mL) was added to the reaction mixture, which was extracted with dichloromethane (30 mL × 3). The combined organic phase was washed with saturated sodium chloride solution (30 mL × 2), dried over anhydrous sodium sulfate, filtered, and concentrated under reduced pressure. The resulting residue was purified by silica gel column chromatography (eluent: System B) to obtain tert-butyl 4-(4-(2-((2-chloro-4-(trifluoromethyl)phenyl)amino)-2-oxoethyl)-5-ethyl-2-(2-morpholinobenzo[ d]oxazol-6-yl)-7-oxo-4,7-dihydro-[1,2,4]triazolo[1,5-a]pyrimidin-6-yl)piperazine-1-carboxylate **172d** (120 mg) in a yield of 67.45%.

MS m/z (ESI): 786.3 [M+H]

### Step 4

### N-(2-chloro-4-(trifluoromethyl)phenyl)-2-(5-ethyl-2-(2-morpholinobenzo[d]oxazol-6-yl)-7-oxo-6 -(piperazin-1-yl)-[1,2,4]triazolo[1,5-a]pyrimidin-4(7H)-yl)acetamide

Tert-butyl 4-(4-(2-((2-chloro-4-(trifluoromethyl)phenyl)amino)-2-oxoethyl)-5-ethyl-2-(2-morpholinobenzo[ d]oxazol-6-yl)-7-oxo-4,7-dihydro-[1,2,4]triazolo[1,5-a]pyrimidin-6-yl)piperazine-1-carboxylate **172d** (120 mg, 152.63 µmol) was dissolved in dichloromethane (5 mL). Trifluoroacetic acid (1 g, 8.77 mmol) was added dropwise, and the reaction was performed at room temperature for 2 h. After the reaction was completed, the mixture was concentrated under reduced pressure to obtain N-(2-chloro-4-(trifluoromethyl)phenyl)-2-(5-ethyl-2-(2-morpholinobenzo[d] oxazol-6-yl)-7-oxo-6-(piperazin-1-yl)-[1,2,4]triazolo[1,5-a]pyrimidin-4(7H)-yl)acetamide **172e** (90 mg), which was used directly in the next step.

MS m/z (ESI): 686.2 [M+H]

### Step 5

### 2-(6-(4-(5-(benzyloxy)-6-methylpyrimidine-4-carbonyl)piperazin-1-yl)-5-ethyl-2-(2-morpholinob enzo[d]oxazol-6-yl)-7-oxo-[1,2,4]triazolo[1,5-a]pyrimidin-4(7H)-yl)-N-(2-chloro-4-(trifluoromet hyl)phenyl)acetamide

5-(Benzyloxy)-6-methylpyrimidine-4-carboxylic acid **1g** (48.06 mg, 196.77 µmol), N-(2-chloro-4-(trifluoromethyl)phenyl)-2-(5-ethyl-2-(2-morpholinobenzo[d]oxazol-6-yl)-7-oxo-6 -(piperazin-1-yl)-[1,2,4]triazolo[1,5-a]pyrimidin-4(7H)-yl)acetamide **172e** (90 mg, 131.18 µmol), N,N-diisopropylethylamine (33.91 mg, 262.36 µmol), 1-(3-dimethylaminopropyl)-3-ethylcarbodiimide hydrochloride (50.29 mg, 262.36 µmol), and 1-hydroxybenzotriazole (88.62 mg, 655.90 µmol) were sequentially added to N,N-dimethylformamide (3 mL), and the reaction was performed at room temperature for 18 h. Water (20 mL) was added to the reaction mixture, which was extracted with dichloromethane (30 mL × 3). The combined organic phase was washed with saturated sodium chloride solution (30 mL × 2), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-5-ethyl-2-(2-morpholinob enzo[d]oxazol-6-yl)-7-oxo-[1,2,4]triazolo[1,5-a]pyrimidin-4(7H)-yl)-N-(2-chloro-4-(trifluoromet hyl)phenyl)acetamide **172f** (70 mg) in a yield of 58.49%.

MS m/z (ESI): 912.3 [M+H]

### Step 6

### N-(2-chloro-4-(trifluoromethyl)phenyl)-2-(5-ethyl-6-(4-(5-hydroxy-6-methylpyrimidine-4-carbon yl)piperazin-1-yl)-2-(2-morpholinobenzo[d]oxazol-6-yl)-7-oxo-[1,2,4]triazolo[1,5-a]pyrimidin-4( 7H)-yl)acetamide

2-(6-(4-(5-(Benzyloxy)-6-methylpyrimidine-4-carbonyl)piperazin-1-yl)-5-ethyl-2-(2-m orpholinobenzo[d]oxazol-6-yl)-7-oxo-[1,2,4]triazolo[1,5-a]pyrimidin-4(7H)-yl)-N-(2-chloro-4-(tr ifluoromethyl)phenyl)acetamide **172f** (70 mg, 76.73 µmol) was dissolved in trifluoroacetic acid (4 mL), heated to 40 °C, and reacted for 3 h. The mixture was concentrated under reduced pressure, and the residue was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-2-(2-morpholinobenzo[d]oxazol-6-yl)-7-oxo-[1,2,4]triazolo[1,5-a]pyrimidin-4( 7H)-yl)acetamide **172** (15 mg) in a yield of 21.40%.

¹H NMR (400 MHz, DMSO-d6) δ 10.42 (s, 1H), 10.25 (s, 1H), 8.59 (s, 1H), 8.06 (d, J = 8.6 Hz, 1H), 7.96 (d, J = 2.1 Hz, 2H), 7.92 - 7.80 (m, 1H), 7.71 (dd, J = 8.7, 2.1 Hz, 1H), 7.52 (dd, J = 18.4, 8.3 Hz, 1H), 5.40 (s, 2H), 4.55 (d, J = 12.4 Hz, 1H), 3.74 (t, J = 4.6 Hz, 5H), 3.66 - 3.48 (m, 11H), 3.58 (s, 6H), 3.02 (p, J = 6.7 Hz, 3H), 2.85 (d, J = 11.2 Hz, 2H), 2.68 (d, J = 10.5 Hz, 1H), 2.46 (s, 3H), 1.22 (t, J = 7.4 Hz, 3H).

### Example 173

### N-(2-chloro-4-(trifluoromethyl)phenyl)-2-(2-(cyclopropylethynyl)-5-ethyl-6-(4-(5-hydroxy-6-me thylpyrimidine-4-carbonyl)piperazin-1-yl)-7-oxo-[1,2,4]triazolo[1,5-a]pyrimidin-4(7H)-yl)aceta mide

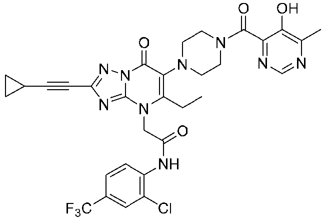

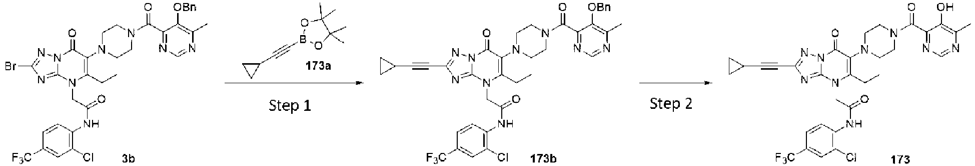

### Step 1 2-(6-(4-(5-(benzyloxy)-6-methylpyrimidine-4-carbonyl)piperazin-1-yl)-2-(cyclopropylethynyl)-5 -ethyl-7-oxo-[1,2,4]triazolo[1,5-a]pyrimidin-4(7H)-yl)-N-(2-chloro-4-(trifluoromethyl)phenyl)ac etamide

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (50.00 mg, 63.37 µmol), 2-(cyclopropylethynyl)-4,4,5,5-tetramethyl-1,3,2-dioxaborolane **173a** (14.61 mg, 76.05 µmol), [1,1'-bis(diphenylphosphino)ferrocene]palladium(II) dichloride (5.14 mg, 6.34 µmol), and sodium carbonate (20.15 mg, 190.11 µmol) were dissolved in 1,4-dioxane (1 mL) and water (0.3 mL). The mixture was subject to argon replacement four times and heated to 80 °C for reaction for 1.5 hours. The reaction mixture was extracted with ethyl acetate (40 mL × 3). The organic phase was washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-2-(cyclopropylethynyl)-5 -ethyl-7-oxo-[1,2,4]triazolo[1,5-a]pyrimidin-4(7H)-yl)-N-(2-chloro-4-(trifluoromethyl)phenyl)ac etamide **173b** (36 mg) with a yield of 73.38%.

MS m/z (ESI): 774.25 [M+1]

### Step 2

### N-(2-chloro-4-(trifluoromethyl)phenyl)-2-(2-(cyclopropylethynyl)-5-ethyl-6-(4-(5-hydroxy-6-me thylpyrimidine-4-carbonyl)piperazin-1-yl)-7-oxo-[1,2,4]triazolo[1,5-a]pyrimidin-4(7H)-yl)aceta mide

To a solution of 2-(6-(4-(5-(benzyloxy)-6-methylpyrimidine-4-carbonyl)piperazin-1-yl)-2-(cyclopropylethynyl)-5 -ethyl-7-oxo-[1,2,4]triazolo[1,5-a]pyrimidin-4(7H)-yl)-N-(2-chloro-4-(trifluoromethyl)phenyl)ac etamide **173b** (36 mg, 46.50 µmol) in dichloromethane (0.5 mL) was added trifluoroacetic acid (2 mL). The reaction was performed at room temperature for 18 hours. The mixture was concentrated under reduced pressure, and the residue was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(2-(cyclopropylethynyl)-5-ethyl-6-(4-(5-hydroxy-6-me thylpyrimidine-4-carbonyl)piperazin-1-yl)-7-oxo-[1,2,4]triazolo[1,5-a]pyrimidin-4(7H)-yl)aceta mide **173** (1.2 mg) with a yield of 3.58%.

MS m/z: 684.2 [M+1]
¹H NMR (400 MHz, DMSO) δ 10.29 (s, 1H), 10.20 (s, 1H), 8.57 (s, 1H), 8.09 (d, J = 8.6 Hz, 1H), 7.96 (s, 1H), 7.74 - 7.66 (m, 1H), 5.29 (s, 2H), 4.52 (d, J = 12.6 Hz, 1H), 3.50 (d, J = 13.1 Hz, 3H), 2.98 (s, 3H), 2.81 (d, J = 11.2 Hz, 1H), 2.63 (d, J = 10.9 Hz, 1H), 2.44 (s, 3H), 1.62 (td, J = 8.5, 4.3 Hz, 1H), 1.17 (t, J = 7.4 Hz, 3H), 0.99 - 0.91 (m, 2H), 0.84 - 0.78 (m, 2H).

### Example 174

### N-(2-chloro-4-(pentafluoro-λ⁶-sulfaneyl)phenyl)-2-(5-ethyl-6-(4-(5-hydroxy-6-methylpyrimidine -4-carbonyl)piperazin-1-yl)-7-oxo-2-(spiro[2.5]oct-5-en-6-yl)-[1,2,4]triazolo[1,5-a]pyrimidin-4(7 H)-yl)acetamide

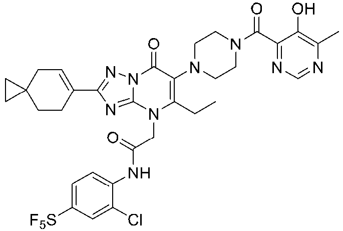

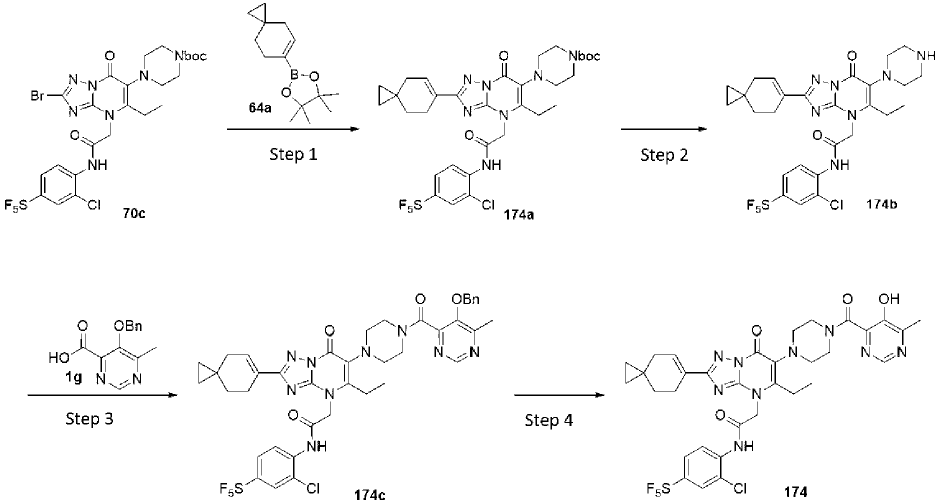

### Step 1

### tert-butyl 4-(4-(2-((2-chloro-4-(pentafluoro-λ⁶-sulfaneyl)phenyl)amino)-2-oxoethyl)-5-ethyl-7-oxo-2-(spiro [2.5]oct-5-en-6-yl)-4,7-dihydro-[1,2,4]triazolo[1,5-a]pyrimidin-6-yl)piperazine-1-carboxylate

At room temperature, tert-butyl 4-(2-bromo-4-(2-((2-chloro-4-(pentafluoro-λ⁶-sulfaneyl)phenyl)amino)-2-oxoethyl)-5-ethyl-7-ox o-4,7-dihydro-[1,2,4]triazolo[1,5-a]pyrimidin-6-yl)piperazine-1-carboxylate **70c** (100 mg, 138.71 µmol), 4,4,5,5-tetramethyl-2-(spiro[2.5]oct-5-en-6-yl)-1,3,2-dioxaborolane **64a** (38.97 mg, 166.45 µmol), [1,1'-bis(diphenylphosphino)ferrocene]palladium(II) dichloride (11.24 mg, 13.87 µmol), and sodium carbonate (44.11 mg, 416.13 µmol) were dissolved in 1,4-dioxane (1 mL) and water (0.3 mL). The mixture was subject to argon replacement four times and heated to 80 °C for reaction for 1.5 hours. Water (30 mL) was added to the reaction mixture, which was extracted with ethyl acetate (40 mL × 3). The organic phase was washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(4-(2-((2-chloro-4-(pentafluoro-λ⁶-sulfaneyl)phenyl)amino)-2-oxoethyl)-5-ethyl-7-oxo-2-(spiro [2.5] oct-5-en-6-yl)-4,7-dihydro-[1,2,4]triazolo[1,5-a]pyrimidin-6-yl)piperazine-1-carboxylate **174a** (100 mg) with a yield of 96.36%.

MS m/z (ESI): 748.2 [M+1]

### Step 2

### N-(2-chloro-4-(pentafluoro-λ⁶-sulfaneyl)phenyl)-2-(5-ethyl-7-oxo-6-(piperazin-l-yl)-2-(spiro[2.5 ]oct-5-en-6-yl)-[1,2,4]triazolo[1,5-a]pyrimidin-4(7H)-yl)acetamide

Trifluoroacetic acid (0.8 mL) was added to a solution of tert-butyl 4-(4-(2-((2-chloro-4-(pentafluoro-λ⁶-sulfaneyl)phenyl)amino)-2-oxoethyl)-5-ethyl-7-oxo-2-(spiro [2.5]oct-5-en-6-yl)-4,7-dihydro-[1,2,4]triazolo[1,5-a]pyrimidin-6-yl)piperazine-1-carboxylate **174a** (100 mg, 133.65 µmol) in dichloromethane (2.5 mL), and the reaction was performed at room temperature for 1 hour. The mixture was concentrated under reduced pressure to obtain N-(2-chloro-4-(pentafluoro-λ⁶-sulfaneyl)phenyl)-2-(5-ethyl-7-oxo-6-(piperazin-1-yl)-2-(spiro[2.5 ]oct-5-en-6-yl)-[1,2,4] triazolo[1,5-a]pyrimidin-4(7H)-yl)acetamide **174b** (80 mg) with a yield of 92.36%.

MS m/z (ESI): 648.2 [M+1]

### Step 3

### 2-(6-(4-(5-(benzyloxy)-6-methylpyrimidine-4-carbonyl)piperazin-1-yl)-5-ethyl-7-oxo-2-(spiro[2. 5]oct-5-en-6-yl)-[1,2,4]triazolo[1,5-a]pyrimidin-4(7H)-yl)-N-(2-chloro-4-(pentafluoro-λ⁶-sulfane yl)phenyl)acetamide

N-(2-chloro-4-(pentafluoro-λ⁶-sulfaneyl)phenyl)-2-(5-ethyl-7-oxo-6-(piperazin-1-yl)-2-(spiro[2.5]oct-5-en-6-yl)-[1,2,4]triazolo[1,5-a]pyrimidin-4(7H)-yl)acetamide **174b** (80 mg, 123.44 µmol), 5-(benzyloxy)-6-methylpyrimidine-4-carboxylic acid **1g** (39.19 mg, 160.47 µmol), 1-(3-dimethylaminopropyl)-3-ethylcarbodiimide hydrochloride (35.50 mg, 185.16 µmol), 1-hydroxybenzotriazole (25.02 mg, 185.16 µmol), and N,N-diisopropylethylamine (79.77 mg, 617.20 µmol) were added to N,N-dimethylformamide (3 mL), and the reaction was performed at room temperature for 4 hours. After the reaction was completed, water (30 mL) was added, and the mixture was extracted with ethyl acetate (30 mL × 2). The combined organic phases were washed with saturated sodium chloride solution (30 mL × 2), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-5-ethyl-7-oxo-2-(spiro[2. 5]oct-5-en-6-yl)-[1,2,4]triazolo[1,5-a]pyrimidin-4(7H)-yl)-N-(2-chloro-4-(pentafluoro-λ⁶-sulfane yl)phenyl)acetamide **174c** (83 mg) with a yield of 76.90%.

MS m/z: 874.2 [M+1]

### Step 4

### N-(2-chloro-4-(pentafluoro-λ⁶-sulfaneyl)phenyl)-2-(5-ethyl-6-(4-(5-hydroxy-6-methylpyrimidine -4-carbonyl)piperazin-1-yl)-7-oxo-2-(spiro[2.5]oct-5-en-6-yl)-[1,2,4]triazolo[1,5-a]pyrimidin-4(7 H)-yl)acetamide

2-(6-(4-(5-(benzyloxy)-6-methylpyrimidine-4-carbonyl)piperazin-1-yl)-5-ethyl-7-oxo-2 -(spiro[2.5]oct-5-en-6-yl)-[1,2,4]triazolo[1,5-a]pyrimidin-4(7H)-yl)-N-(2-chloro-4-(pentafluoro-λ ⁶-sulfaneyl)phenyl)acetamide **174c** (83 mg, 94.93 µmol) was added to dichloromethane (2.5 mL). Boron trichloride (0.8 mL) was added at 0 °C, and the reaction was performed at 0 °C for 1 hour. After the reaction was completed, methanol was added to quench the reaction. The mixture was concentrated under reduced pressure, and the residue was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(pentafluoro-λ⁶-sulfaneyl)phenyl)-2-(5-ethyl-6-(4-(5-hydroxy-6-methylpyrimidine -4-carbonyl) piperazin-1-yl)-7-oxo-2-(spiro[2.5]oct-5-en-6-yl)-[1,2,4]triazolo[1,5-a]pyrimidin-4(7H)-yl)aceta mide **174** (14.43 mg) with a yield of 19%.

MS m/z: 784.2 [M+1]
¹H NMR (400 MHz, DMSO) δ 10.39 (s, 1H), 10.22 (s, 1H), 8.58 (s, 1H), 8.15 (d, J = 2.6 Hz, 1H), 8.10 (d, J = 9.1 Hz, 1H), 7.90 (dd, J = 9.2, 2.7 Hz, 1H), 6.92 - 6.85 (m, 1H), 5.32 (s, 2H), 4.52 (d, J = 12.5 Hz, 1H), 3.49 (t, J = 11.3 Hz, 3H), 3.25 (s, 1H), 2.98 (d, J = 8.7 Hz, 3H), 2.82 (d, J = 11.3 Hz, 1H), 2.68 - 2.61 (m, 1H), 2.44 (s, 3H), 2.10 (d, J = 3.8 Hz, 2H), 1.49 (t, J = 6.1 Hz, 2H), 1.18 (t, J = 7.4 Hz, 3H), 0.39 - 0.29 (m, 4H).

### Example 175

### N-(2-chloro-4-(pentafluoro-λ6-sulfaneyl)phenyl)-2-(5-ethyl-6-(4-(5-hydroxy-6-methylpyrimidine -4-carbonyl)piperazin-1-yl)-7-oxo-2-(2-oxaspiro[3.5]non-6-en-7-yl)-[1,2,4]triazolo[1,5-a]pyrimid in-4(7H)-yl)acetamide

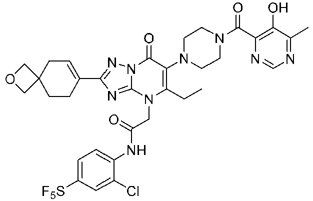

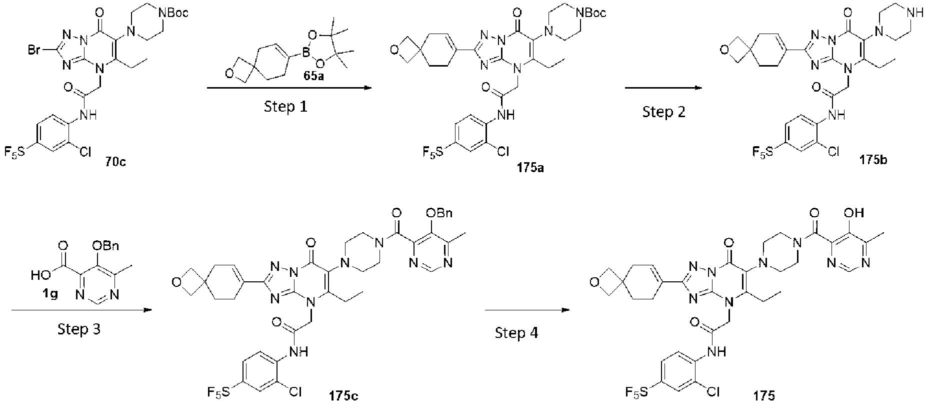

### Step 1

### tert-butyl 4-(4-(2-((2-chloro-4-(pentafluoro-λ⁶-sulfaneyl)phenyl)amino)-2-oxoethyl)-5-ethyl-7-oxo-2-(2-ox aspiro[3.5]non-6-en-7-yl)-4,7-dihydro-[1,2,4]triazolo[1,5-a]pyrimidin-6-yl)piperazine-1-carboxyl ate

At room temperature, tert-butyl 4-(2-bromo-4-(2-((2-chloro-4-(pentafluoro-λ⁶-sulfaneyl)phenyl)amino)-2-oxoethyl)-5-ethyl-7-ox o-4,7-dihydro-[1,2,4]triazolo[1,5-a]pyrimidin-6-yl)piperazine-1-carboxylate **70c** (60 mg, 87.40 µmol), 4,4,5,5-tetramethyl-2-(2-oxaspiro[3.5]non-6-en-7-yl)-1,3,2-dioxaborolane **65a** (26.24 mg, 104.88 µmol), [1,1-bis(diphenylphosphino)ferrocene]palladium(II) dichloride (7.08 mg, 8.74 µmol), and sodium carbonate (27.79 mg, 262.20 µmol) were dissolved in 1,4-dioxane (2 mL) and water (0.6 mL). The mixture was subject to argon replacement four times and was heated to 80 C for reaction for 1.5 hours. Water (30 mL) was added to the reaction mixture, and the mixture was extracted with ethyl acetate (40 mL × 3). The organic phase was washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(4-(2-((2-chloro-4-(pentafluoro-λ⁶-sulfaneyl)phenyl)amino)-2-oxoethyl)-5-ethyl-7-oxo-2-(2-ox aspiro[3.5]non-6-en-7-yl)-4,7-dihydro-[1,2,4]triazolo[1,5-a]pyrimidin-6-yl)piperazine-1-carboxyl ate **175a** (39 mg) with a yield of 61.15%.

MS m/z (ESI): 674.1 [M-56+1]

### Step 2

### N-(2-chloro-4-(pentafluoro-λ⁶-sulfaneyl)phenyl)-2-(5-ethyl-7-oxo-6-(piperazin-1-yl)-2-(2-oxaspi ro[3.5]non-6-en-7-yl)-[1,2,4]triazolo[1,5-a]pyrimidin-4(7H)-yl)acetamide

Trifluoroacetic acid (0.8 mL) was added to a solution of tert-butyl 4-(4-(2-((2-chloro-4-(pentafluoro-λ⁶-sulfaneyl)phenyl)amino)-2-oxoethyl)-5-ethyl-7-oxo-2-(2-ox aspiro[3.5]non-6-en-7-yl)-4,7-dihydro-[1,2,4]triazolo[1,5-a]pyrimidin-6-yl)piperazine-1-carboxyl ate **175a** (39 mg, 53.44 µmol) in dichloromethane (2.5 mL). The reaction was performed at room temperature for 1 hour. After the reaction was completed, the mixture was concentrated under reduced pressure to obtain N-(2-chloro-4-(pentafluoro-λ⁶-sulfaneyl)phenyl)-2-(5-ethyl-7-oxo-6-(piperazin-1-yl)-2-(2-oxaspi ro[3.5]non-6-en-7-yl)-[1,2,4]triazolo[1,5-a]pyrimidin-4(7H)-yl)acetamide **175b** (30 mg) with a yield of 89.15%.

MS m/z: 630.1 [M+1]

### Step 3

### 2-(6-(4-(5-(benzyloxy)-6-methylpyrimidine-4-carbonyl)piperazin-1-yl)-5-ethyl-7-oxo-2-(2-oxasp iro[3.5]non-6-en-7-yl)-[1,2,4]triazolo[1,5-a]pyrimidin-4(7H)-yl)-N-(2-chloro-4-(pentafluoro-λ⁶-s ulfaneyl)phenyl)acetamide

N-(2-chloro-4-(pentafluoro-λ⁶-sulfaneyl)phenyl)-2-(5-ethyl-7-oxo-6-(piperazin-1-yl)-2-(2-oxaspiro[3.5]non-6-en-7-yl)-[1,2,4]triazolo[1,5-a]pyrimidin-4(7H)-yl)acetamide **175b** (57 mg, 85.83 µmol), 5-(benzyloxy)-6-methylpyrimidine-4-carboxylic acid **1g** (27.25 mg, 111.58 µmol), 1-(3-dimethylaminopropyl)-3-ethylcarbodiimide hydrochloride (24.68 mg, 128.75 µmol), 1-hydroxybenzotriazole (17.40 mg, 128.75 µmol), and N,N-diisopropylethylamine (55.46 mg, 429.16 µmol) were added to N,N-dimethylformamide (3 mL). The reaction was performed at room temperature for 4 hours. Water (30 mL) was added to the reaction mixture, and the mixture was extracted with ethyl acetate (30 mL × 3). The combined organic phase was washed with saturated sodium chloride solution (30 mL × 2), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-5-ethyl-7-oxo-2-(2-oxasp iro[3.5]non-6-en-7-yl)-[1,2,4]triazolo[1,5-a]pyrimidin-4(7H)-yl)-N-(2-chloro-4-(pentafluoro-λ⁶-s ulfaneyl)phenyl)acetamide **175c** (38 mg) with a yield of 49.73%.

MS m/z: 890.2 [M+1]

### Step 4

### N-(2-chloro-4-(pentafluoro-λ⁶-sulfaneyl)phenyl)-2-(5-ethyl-6-(4-(5-hydroxy-6-methylpyrimidine -4-carbonyl)piperazin-1-yl)-7-oxo-2-(2-oxaspiro[3.5]non-6-en-7-yl)-[1,2,4]triazolo[1,5-a]pyrimid in-4(7H)-yl)acetamide

2-(6-(4-(5-(Benzyloxy)-6-methylpyrimidine-4-carbonyl)piperazin-1-yl)-5-ethyl-7-oxo-2 -(2-oxaspiro[3.5]non-6-en-7-yl)-[1,2,4]triazolo[1,5-a]pyrimidin-4(7H)-yl)-N-(2-chloro-4-(pentafl uoro-λ⁶-sulfaneyl)phenyl)acetamide **175c** (38 mg, 42.68 µmol) was added to dichloromethane (1 mL), and trifluoroacetic acid (4 mL) was added. The reaction was performed at room temperature for 18 hours. After the reaction was completed, methanol was added to quench the reaction, and the mixture was concentrated under reduced pressure. The residue was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(pentafluoro-λ⁶-sulfaneyl)phenyl)-2-(5-ethyl-6-(4-(5-hydroxy-6-methylpyrimidine -4-carbonyl)piperazin-1-yl)-7-oxo-2-(2-oxaspiro[3.5non-6-en-7-yl)-[1,2,4]triazolo[1,5-a]pyrimid in-4(7H)-yl)acetamide **175** (3.99 mg) with a yield of 11.45%.

MS m/z: 800.2 [M+1]
¹H NMR (400 MHz, DMSO) δ 10.38 (s, 1H), 10.21 (s, 1H), 8.58 (s, 1H), 8.19 - 8.07 (m, 2H), 7.90 (dd, J = 9.2, 2.6 Hz, 1H), 6.77 (s, 1H), 5.31 (s, 2H), 4.52 (d, J = 12.5 Hz, 1H), 4.37 (d, J = 5.7 Hz, 2H), 4.29 (d, J = 5.7 Hz, 2H), 3.50 (d, J = 11.0 Hz, 3H), 3.26 (d, J = 12.2 Hz, 1H), 2.97 (d, J = 8.8 Hz, 3H), 2.81 (d, J = 11.5 Hz, 1H), 2.63 (d, J = 10.7 Hz, 1H), 2.44 (s, 3H), 1.97 (d, J = 6.2 Hz, 2H), 1.18 (t, J = 7.4 Hz, 3H).

### Example 176

### 2-(5-ethyl-6-(4-(5-hydroxy-6-methylpyrimidine-4-carbonyl)piperazin-1-yl)-7-oxo-2-(2-oxaspiro[ 3.5]non-6-en-7-yl)-[1,2,4]triazolo[1,5-a]pyrimidin-4(7H)-yl)-N-(4-(pentafluoro-λ⁶-sulfaneyl)phe nyl)acetamide

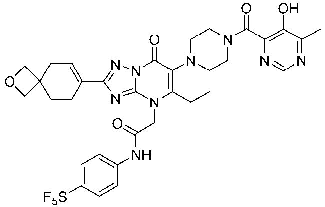

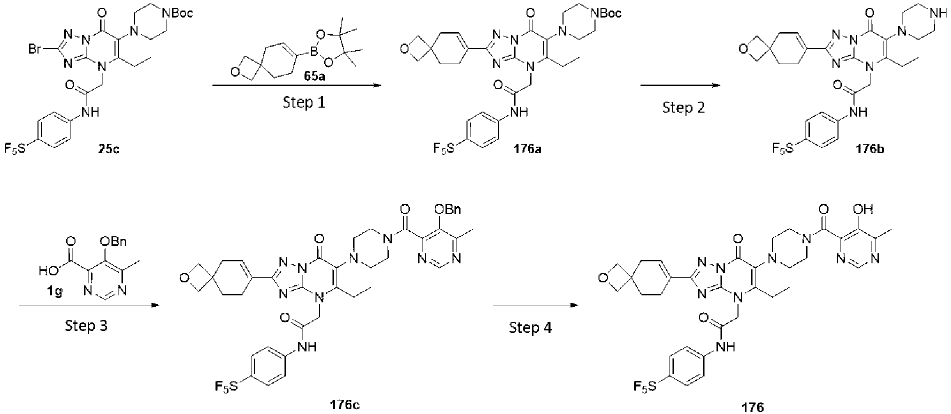

### Step 1

### tert-butyl 4-(5-ethyl-7-oxo-4-(2-oxo-2-((4-(pentafluoro-λ⁶-sulfaneyl)phenyl)amino)ethyl)-2-(2-oxaspiro[3.5 ]non-6-en-7-yl)-4,7-dihydro-[1,2,4]triazolo[1,5-a]pyrimidin-6-yl)piperazine-1-carboxylate

At room temperature, tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)piperazine-1-carboxylate **25c** (90 mg, 131.10 µmol), 4,4,5,5-tetramethyl-2-(2-oxaspiro[3.5]non-6-en-7-yl)-1,3,2-dioxaborolane **65a** (39.35 mg, 157.32 µmol), [1,1-bis(diphenylphosphino)ferrocene]palladium(II) dichloride (10.63 mg, 13.11 µmol), and sodium carbonate (41.69 mg, 393.30 µmol) were dissolved in 1,4-dioxane (2 mL) and water (0.6 mL). The mixture was subject to argon replacement four times, then heated to 80 °C and reacted for 1.5 hours. Water (30 mL) was added to the reaction mixture, which was extracted with ethyl acetate (40 mL×3). The organic phase was washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(5-ethyl-7-oxo-4-(2-oxo-2-((4-(pentafluoro-λ⁶-sulfaneyl)phenyl)amino)ethyl)-2-(2-oxaspiro[3.5 non-6-en-7-yl)-4,7-dihydro-[1,2,4]triazolo[1,5-a]pyrimidin-6-yl)piperazine-1-carboxylate **176a** (90 mg) in a yield of 94.07%.

MS m/z (ESI): 674.1 [M-56+1]

### Step 2

### 2-(5-ethyl-7-oxo-6-(piperazin-1-yl)-2-(2-oxaspiro[3.5]non-6-en-7-yl)-[1,2,4]triazolo[1,5-a]pyrimi din-4(7H)-yl)-N-(4-(pentafluoro-λ⁶-sulfaneyl)phenyl)acetamide

Trifluoroacetic acid (1 mL) was added to a solution of tert-butyl 4-(5-ethyl-7-oxo-4-(2-oxo-2-((4-(pentafluoro-λ⁶-sulfaneyl)phenyl)amino)ethyl)-2-(2-oxaspiro[3.5 ]non-6-en-7-yl)-4,7-dihydro-[1,2,4]triazolo[1,5-a]pyrimidin-6-yl)piperazine-1-carboxylate **176a** (90 mg, 123.33 µmol) in dichloromethane (4 mL), and the reaction was performed at room temperature for 1 hour. After completion of the reaction, the mixture was concentrated under reduced pressure to obtain 2-(5-ethyl-7-oxo-6-(piperazin-1-yl)-2-(2-oxaspiro[3.5]non-6-en-7-yl)-[1,2,4]triazolo[1,5-a]pyrimi din-4(7H)-yl)-N-(4-(pentafluoro-λ⁶-sulfaneyl)phenyl)acetamide **176b** (70 mg) in a yield of 90.14%, which was used directly in the next step.

MS m/z: 630.2 [M+1]

### Step 3

### 2-(6-(4-(5-(benzyloxy)-6-methylpyrimidine-4-carbonyl)piperazin-1-yl)-5-ethyl-7-oxo-2-(2-oxasp iro[3.5]non-6-en-7-yl)-[1,2,4]triazolo[1,5-a]pyrimidin-4(7H)-yl)-N-(4-(pentafluoro-λ⁶-sulfaneyl) phenyl)acetamide

2-(5-Ethyl-7-oxo-6-(piperazin-1-yl)-2-(2-oxaspiro[3.5]non-6-en-7-yl)-[1,2,4]triazolo [1,5-a]pyrimidin-4(7H)-yl)-N-(4-(pentafluoro-λ⁶-sulfaneyl)phenyl)acetamide **176b** (70 mg, 111.17 µmol), 5-(benzyloxy)-6-methylpyrimidine-4-carboxylic acid 1g (35.30 mg, 144.53 µmol), 1-(3-dimethylaminopropyl)-3-ethylcarbodiimide hydrochloride (31.97 mg, 166.76 µmol), 1-hydroxybenzotriazole (22.53 mg, 166.76 µmol), and N,N-diisopropylethylamine (71.84 mg, 555.87 µmol) were added to N,N-dimethylformamide (3 mL), and the reaction was performed at room temperature for 4 hours. Water (30 mL) was added to the reaction mixture, which was extracted with ethyl acetate (40 mL×3). The organic phase was washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-l-yl)-5-ethyl-7-oxo-2-(2-oxasp iro[3.5]non-6-en-7-yl)-[1,2,4]triazolo[1,5-a]pyrimidin-4(7H)-yl)-N-(4-(pentafluoro-λ⁶-sulfaneyl) phenyl)acetamide **176c** (69 mg) in a yield of 72.52%.

MS m/z: 856.3 [M+1]

### Step 4

### 2-(5-ethyl-6-(4-(5-hydroxy-6-methylpyrimidine-4-carbonyl)piperazin-1-yl)-7-oxo-2-(2-oxaspiro[ 3.5]non-6-en-7-yl)-[1,2,4]triazolo[1,5-a]pyrimidin-4(7H)-yl)-N-(4-(pentafluoro-λ⁶-sulfaneyl)phe nyl)acetamide

2-(6-(4-(5-(Benzyloxy)-6-methylpyrimidine-4-carbonyl)piperazin-1-yl)-5-ethyl-7-oxo-2 -(2-oxaspiro[3.5]non-6-en-7-yl)-[1,2,4]triazolo[1,5-a]pyrimidin-4(7H)-yl)-N-(4-(pentafluoro-λ⁶-s ulfaneyl)phenyl)acetamide **176c** (69 mg, 80.62 µmol) was added to dichloromethane (1 mL), followed by addition of trifluoroacetic acid (4 mL), and the reaction was performed at room temperature for 18 hours. After completion of the reaction, the mixture was concentrated under reduced pressure, and the residue was purified by preparative liquid chromatography (separation column: AKZONOBEL Kromasil; 250×21.2 mm I.D.; 5 µm, 20 mL/min; mobile phase A: 0.05% TFA + H₂O, mobile phase B: CH₃CN) to obtain 2-(5-ethyl-6-(4-(5-hydroxy-6-methylpyrimidine-4-carbonyl)piperazin-1-yl)-7-oxo-2-(2-oxaspiro [ 3.5]non-6-en-7-yl)-[1,2,4]triazolo[1,5-a]pyrimidin-4(7H)-yl)-N-(4-(pentafluoro-λ⁶-sulfaneyl)phe nyl)acetamide **176** (13.22 mg) in a yield of 20.13%. MS m/z: 766.2 [M+1]
¹H NMR (400 MHz, DMSO) δ 10.92 (s, 1H), 10.22 (s, 1H), 8.58 (s, 1H), 7.88 (d, J = 9.1 Hz, 2H), 7.76 (d, J = 8.9 Hz, 2H), 6.72 (s, 1H), 5.18 (s, 2H), 4.52 (d, J = 12.6 Hz, 1H), 4.36 (d, J = 5.7 Hz, 2H), 4.28 (d, J = 5.7 Hz, 2H), 3.49 (s, 3H), 3.26 (d, J = 12.8 Hz, 1H), 2.97 (d, J = 9.7 Hz, 3H), 2.81 (d, J = 11.6 Hz, 1H), 2.64 (d, J = 10.3 Hz, 1H), 2.44 (s, 3H), 1.95 (d, J = 6.1 Hz, 2H), 1.16 (t, J = 7.4 Hz, 3H).

### Example 177

### 2-(2-(bicyclo[3.1.0]hex-2-en-3-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(4-((trifluoromethyl)thio)phenyl)ac etamide

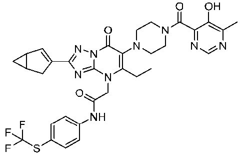

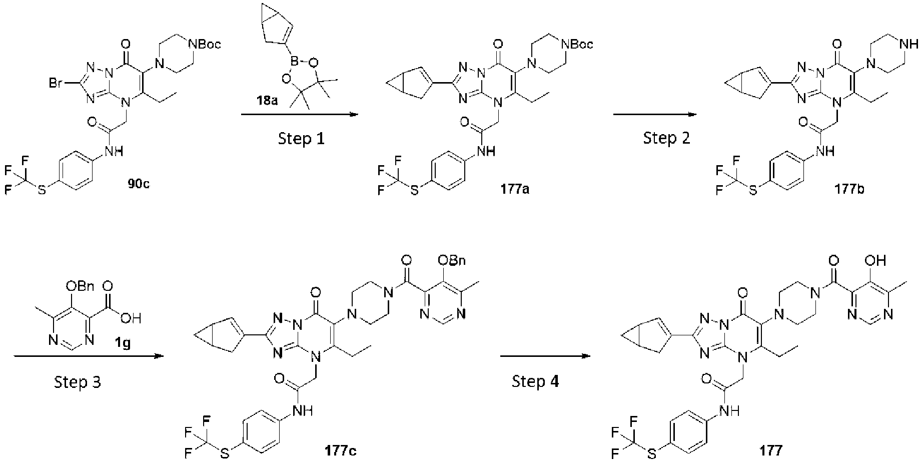

### Step 1

### tert-butyl 4-(2-(bicyclo[3. 1.0]hex-2-en-3-yl)-5-ethyl-7-oxo-4-(2-oxo-2-((4-((trifluoromethyl)thio)phenyl)a mino)ethyl)-4,7-dihydro-[1,2,4]triazolo[1,5-a]pyrimidin-6-yl)piperazine-1-carboxylate

At room temperature, tert-butyl 4-(2-bromo-5-ethyl-7-oxo-4-(2-oxo-2-((4-((trifluoromethyl)thio)phenyl)amino)ethyl)-4,7-dihydro -[1,2,4]triazolo[1,5-a]pyrimidin-6-yl)piperazine-1-carboxylate **90c** (60 mg, 90.84 µmol), 2-(bicyclo[3.1.0]hex-2-en-3-yl)-4,4,5,5-tetramethyl-1,3,2-dioxaborolane **18a** (22.47 mg, 109.01 µmol), [1,1'-bis(diphenylphosphino)ferrocene]palladium dichloride (7.36 mg, 9.08 µmol), and sodium carbonate (28.88 mg, 272.52 µmol) were added to 1,4-dioxane (1 mL) and water (0.3 mL). The mixture was subject to argon replacement four times, heated to 80 °C, and reacted for 1.5 hours. Water (20 mL) was added to the reaction mixture, which was extracted with ethyl acetate (40 mL × 3). The organic phase was washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(2-(bicyclo[3.1.0]hex-2-en-3-yl)-5-ethyl-7-oxo-4-(2-oxo-2-((4-((trifluoromethyl)thio)phenyl) amino)ethyl)-4,7-dihydro-[1,2,4]triazolo[1,5-a]pyrimidin-6-yl)piperazine-1-carboxylate **177a** (50 mg) with a yield of 83.43%.

MS m/z (ESI): 604.2 [M-56+1]

### Step 2

### 2-(2-(bicyclo[3.1.0]hex-2-en-3-yl)-5-ethyl-7-oxo-6-(piperazin-1-yl)-[1,2,4]triazolo[1,5-a]pyrimid in-4(7H)-yl)-N-(4-((trifluoromethyl)thio)phenyl)acetamide

To tert-butyl 4-(2-(bicyclo[3.1.0]hex-2-en-3-yl)-5-ethyl-7-oxo-4-(2-oxo-2-((4-((trifluoromethyl)thio)phenyl)a mino)ethyl)-4,7-dihydro-[1,2,4]triazolo[1,5-a]pyrimidin-6-yl)piperazine-1-carboxylate **177a** (50 mg, 75.79 µmol) in dichloromethane (2.5 mL) was added trifluoroacetic acid (0.8 mL). The mixture was reacted at room temperature for 2 hours. The reaction mixture was concentrated under reduced pressure to obtain 2-(2-(bicyclo[3.1.0]hex-2-en-3-yl)-5-ethyl-7-oxo-6-(piperazin-1-yl)-[1,2,4]triazolo[1,5-a]pyrimid in-4(7H)-yl)-N-(4-((trifluoromethyl)thio)phenyl)acetamide **177b** (40 mg) with a yield of 94.31 %.

MS m/z (ESI): 560.2 [M+1]

### Step 3

### 2-(6-(4-(5-(benzyloxy)-6-methylpyrimidine-4-carbonyl)piperazin-1-yl)-2-(bicyclo[3.1.0]hex-2-en -3-yl)-5-ethyl-7-oxo-[1,2,4]triazolo[1,5-a]pyrimidin-4(7H)-yl)-N-(4-((trifluoromethyl)thio)pheny l)acetamide

2-(2-(Bicyclo[3.1.0]hex-2-en-3-yl)-5-ethyl-7-oxo-6-(piperazin-1-yl)-[1,2,4]triazolo[1,5-a]pyrimidin-4(7H)-yl)-N-(4-((trifluoromethyl)thio)phenyl)acetamide **177b** (22.70 mg, 92.92 µmol), 1-(3-dimethylaminopropyl)-3-ethylcarbodiimide hydrochloride (20.55 mg, 107.22 µmol), 1-hydroxybenzotriazole (14.49 mg, 107.22 µmol), and N,N-diisopropylethylamine (46.19 mg, 357.39 µmol) were added to N,N-dimethylformamide (15 mL). The mixture was reacted at room temperature for 4 hours. Water (20 mL) was added to the reaction mixture, which was extracted with ethyl acetate (40 mL × 3). The organic phase was washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-2-(bicyclo[3.1.0]hex-2-en -3-yl)-5-ethyl-7-oxo-[1,2,4]triazolo[1,5-a]pyrimidin-4(7H)-yl)-N-(4-((trifluoromethyl)thio)pheny l)acetamide **177c** (40 mg) with a yield of 71.21%.

MS m/z: 786.3 [M+1]

### Step 4

### 2-(2-(bicyclo[3.1.0]hex-2-en-3-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(4-((trifluoromethyl)thio)phenyl)ac etamide

To a solution of 2-(6-(4-(5-(benzyloxy)-6-methylpyrimidine-4-carbonyl)piperazin-1-yl)-2-(bicyclo[3.1.0]hex-2-en -3-yl)-5-ethyl-7-oxo-[1,2,4]triazolo[1,5-a]pyrimidin-4(7H)-yl)-N-(4-((trifluoromethyl)thio)pheny l)acetamide **177c** (40 mg, 50.90 µmol) in dichloromethane (1 mL) was added trifluoroacetic acid (4 mL). The mixture was reacted at room temperature for 18 hours. After the reaction was complete, the mixture was concentrated under reduced pressure. The residue was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain 2-(2-(bicyclo[3.1.0]hex-2-en-3-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(4-((trifluoromethyl) thio)phenyl)acetamide **177** (3.01 mg) with a yield of 8.16%.

MS m/z: 696.3 [M+1]
¹H NMR (400 MHz, DMSO) δ 10.81 (s, 1H), 10.21 (s, 1H), 8.57 (s, 1H), 7.70 (q, J = 8.6 Hz, 4H), 6.77 (s, 1H), 5.16 (s, 2H), 4.52 (d, J = 12.7 Hz, 1H), 3.50 (d, J = 11.5 Hz, 3H), 2.94 (d, J = 17.7 Hz, 5H), 2.72 (dd, J = 42.7, 25.4 Hz, 5H), 2.44 (s, 4H), 1.96 (s, 1H), 1.78 (s, 1H), 1.16 (t, J = 7.3 Hz, 3H), 1.00 (s, 1H).

### Example 178

### 2-(5-ethyl-2-(5-hydroxy-1,3a,4,5,6,6a-hexahydropentalen-2-yl)-6-(4-(3-hydroxypicolinoyl)pipera zin-1-yl)-7-oxo-[1,2,4]triazolo[1,5-a]pyrimidin-4(7H)-yl)-N-(4-(pentafluoro-λ⁶-sulfaneyl)phenyl) acetamide

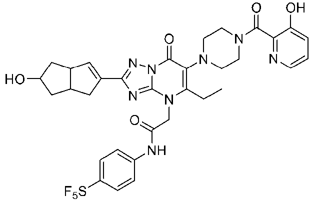

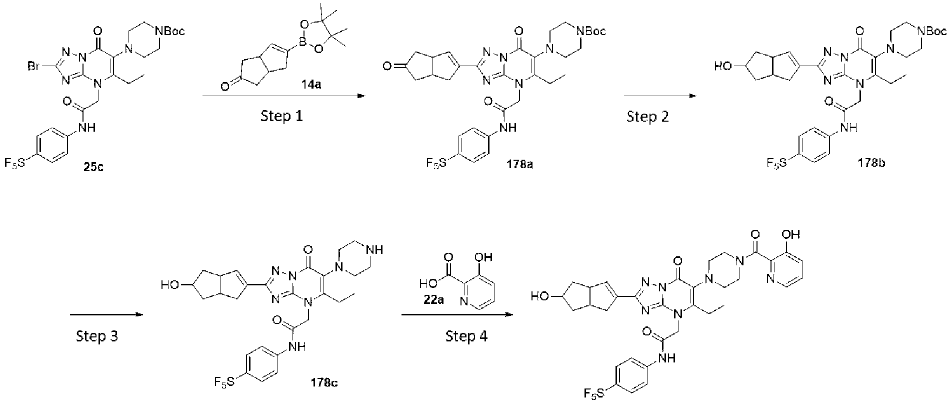

### Step 1

### tert-butyl 4-(5-ethyl-7-oxo-2-(5-oxo-1,3a,4,5,6,6a-hexahydropentalen-2-yl)-4-(2-oxo-2-((4-(pentafluoro-λ⁶-sulfaneyl)phenyl)amino)ethyl)-4,7-dihydro-[1,2,4]triazolo[1,5-a]pyrimidin-6-yl)piperazine-1-car boxylate

At room temperature, tert-butyl 4-(2-bromo-5-ethyl-7-oxo-4-(2-oxo-2-((4-(pentafluoro-λ⁶-sulfaneyl)phenyl)amino)ethyl)-4,7-dih ydro-[1,2,4]triazolo[1,5-a]pyrimidin-6-yl)piperazine-1-carboxylate **25c** (150 mg, 218.50 µmol), 5-(4,4,5,5-tetramethyl-1,3,2-dioxaborolan-2-yl)-3,3a,4,6a-tetrahydro-1H-cyclopenta[c]furan-2-on e **14a** (65.06 mg, 262.20 µmol), [1,1'-bis(diphenylphosphino)ferrocene]palladium(II) dichloride (17.71 mg, 21.85 µmol), and sodium carbonate (69.48 mg, 655.51 µmol) were dissolved in 1,4-dioxane (3 mL) and water (0.9 mL). The mixture was subject to argon replacement four times, heated to 80 °C, and reacted for 1.5 hours. Water (20 mL) was added to the reaction mixture, and the mixture was extracted with ethyl acetate (40 mL × 3) three times. The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(5-ethyl-7-oxo-2-(5-oxo-1,3a,4,5,6,6a-hexahydropentalen-2-yl)-4-(2-oxo-2-((4-(pentafluoro-λ⁶-sulfaneyl)phenyl)amino)ethyl)-4,7-dihydro-[1,2,4]triazolo[1,5-a]pyrimidin-6-yl)piperazine-1-car boxylate **178a** (110 mg) in a yield of 69.18%.

MS m/z (ESI): 672.2 [M+-56+1]

### Step 2

### tert-butyl 4-(5-ethyl-2-(5-hydroxy-1,3a,4,5,6,6a-hexahydropentalen-2-yl)-7-oxo-4-(2-oxo-2-((4-(pentafluor o-λ6-sulfaneyl)phenyl)amino)ethyl)-4,7-dihydro-[1,2,4]triazolo[1,5-a]pyrimidin-6-yl)piperazine-1-carboxylate

Tert-Butyl 4-(5-ethyl-7-oxo-2-(5-oxo-1,3a,4,5,6,6a-hexahydropentalen-2-yl)-4-(2-oxo-2-((4-(pentafluoro-λ⁶-sulfaneyl)phenyl)amino)ethyl)-4,7-dihydro-[1,2,4]triazolo[1,5-a]pyrimidin-6-yl)piperazine-1-car boxylate **178a** (110 mg, 151.15 µmol) was added to methanol (5 mL). Sodium borohydride (5.72 mg, 151.15 µmol) was added at 0 °C, and the mixture was heated to room temperature and reacted for 1 hour. Water (20 mL) was added at 0 °C, and the reaction mixture was extracted with ethyl acetate (30 mL × 3). The combined organic phases were washed with saturated sodium chloride solution (30 mL × 2) in sequence, dried over anhydrous sodium sulfate, filtered, and concentrated under reduced pressure. The resulting residue was purified by silica gel column chromatography (eluent: System B) to obtain tert-butyl 4-(5-ethyl-2-(5-hydroxy-1,3a,4,5,6,6a-hexahydropentalen-2-yl)-7-oxo-4-(2-oxo-2-((4-(pentafluor o-λ⁶-sulfaneyl)phenyl)amino)ethyl)-4,7-dihydro-[1,2,4]triazolo[1,5-a]pyrimidin-6-yl)piperazine-1-carboxylate **178b** (68 mg) in a yield of 61.65%.

MS m/z: 674.2 [M-56+1]

### Step 3

### 2-(5-ethyl-2-(5-hydroxy-1,3a,4,5,6,6a-hexahydropentalen-2-yl)-7-oxo-6-(piperazin-1-yl)-[1,2,4]tr iazolo[1,5-a]pyrimidin-4(7H)-yl)-N-(4-(pentafluoro-λ⁶-sulfaneyl)phenyl)acetamide

Trifluoroacetic acid (0.8 mL) was added to a solution of tert-butyl 4-(5-ethyl-2-(5-hydroxy-1,3a,4,5,6,6a-hexahydropentalen-2-yl)-7-oxo-4-(2-oxo-2-((4-(pentafluor o-λ⁶-sulfaneyl) phenyl)amino)ethyl)-4,7-dihydro-[1,2,4]triazolo[1,5-a]pyrimidin-6-yl)piperazine-1-carboxylate **178b** (68 mg, 93.18 µmol) in dichloromethane (2.5 mL), and the mixture was reacted at room temperature for 1 hour. The mixture was concentrated under reduced pressure to obtain 2-(5-ethyl-2-(5-hydroxy-1,3a,4,5,6,6a-hexahydropentalen-2-yl)-7-oxo-6-(piperazin-1-yl)-[1,2,4]tr iazolo[1,5-a]pyrimidin-4(7H)-yl)-N-(4-(pentafluoro-λ⁶-sulfaneyl)phenyl)acetamide **178c** (55 mg) in a yield of 93.74%.

MS m/z(ESI): 630.2 [M+1]

### Step 4

### 2-(5-ethyl-2-(5-hydroxy-1,3a,4,5,6,6a-hexahydropentalen-2-yl)-6-(4-(3-hydroxypicolinoyl)pipera zin-1-yl)-7-oxo-[1,2,4]triazolo[1,5-a]pyrimidin-4(7H)-yl)-N-(4-(pentafluoro-λ⁶-sulfaneyl)phenyl) acetamide

2-(5-Ethyl-2-(5-hydroxy-1,3a,4,5,6,6a-hexahydropentalen-2-yl)-7-oxo-6-(piperazin-1-yl )-[1,2,4]triazolo[1,5-a]pyrimidin-4(7H)-yl)-N-(4-(pentafluoro-λ⁶-sulfaneyl)phenyl)acetamide **178c** (55 mg, 87.35 µmol), 3-hydroxypicolinic acid **22a** (15.80 mg, 113.56 µmol), 1-(3-dimethylaminopropyl)-3-ethylcarbodiimide hydrochloride (25.12 mg, 131.03 µmol), 1-hydroxybenzotriazole (17.70 mg, 131.03 µmol), and N,N-diisopropylethylamine (56.45 mg, 436.75 µmol) were added to N,N-dimethylformamide (3 mL) and reacted at room temperature for 4 hours. After the reaction was complete, the reaction mixture was extracted with ethyl acetate (30 mL × 2). The aqueous layer was separated, and the combined organic phases were washed with saturated sodium chloride solution (30 mL × 2) in sequence, dried over anhydrous sodium sulfate, filtered, and concentrated under reduced pressure. The residue was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain 2-(5-ethyl-2-(5-hydroxy-1,3a,4,5,6,6a-hexahydropentalen-2-yl)-6-(4-(3-hydroxypicolinoyl)pipera zin-1-yl)-7-oxo-[1,2,4]triazolo[1,5-a]pyrimidin-4(7H)-yl)-N-(4-(pentafluoro-λ⁶-sulfaneyl)phenyl) acetamide **178** (2.93 mg) in a yield of 4.11%.

MS m/z: 751.2 [M+1]
¹H NMR (400 MHz, DMSO) δ 10.94 (s, 1H), 10.40 (s, 1H), 8.09 - 8.01 (m, 1H), 7.88 (d, J = 9.3 Hz, 2H), 7.77 (d, J = 8.8 Hz, 2H), 7.30 (d, J = 3.0 Hz, 2H), 6.49 (d, J = 2.2 Hz, 1H), 5.19 (s, 2H), 4.55 (d, J = 12.7 Hz, 1H), 3.98 (t, J = 6.7 Hz, 1H), 3.25 (s, 3H), 2.95 (s, 5H), 2.82 - 2.56 (m, 5H), 2.03 (s, 2H), 1.31 - 1.20 (m, 3H), 1.16 (t, J = 7.4 Hz, 3H).

### Example 179

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(5-(2,2,2-trifluoroethyl)-4,5,6,7-tetrahydrothieno[3,2-c]pyridin-2-yl)-[ 1,2,4]triazolo[1,5-a]pyrimidin-4(7H)-yl)acetamide

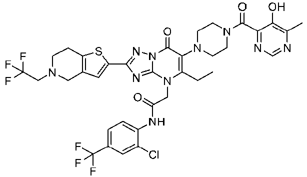

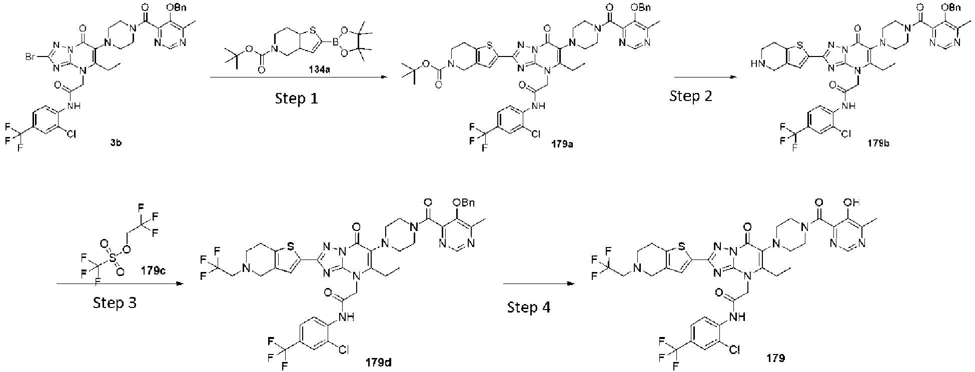

### Step 1

### tert-butyl 2-(6-(4-(5-(benzyloxy)-6-methylpyrimidine-4-carbonyl)piperazin-1-yl)-4-(2-((2-chloro-4-(trifluo romethyl)phenyl)amino)-2-oxoethyl)-5-ethyl-7-oxo-4,7-dihydro-[1,2,4]triazolo[1,5-a]pyrimidin-2-yl)-6,7-dihydrothieno[3,2-c]pyridine-5(4H)-carboxylate

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide 3b (80.00 mg, 101.39 µmol), tert-butyl 2-(4,4,5,5-tetramethyl-1,3,2-dioxaborolan-2-yl)-6,7-dihydro-4H-thieno[3,2-c]pyridine-5-carboxyl ate **134a** (44.45 mg, 121.67 µmol), [1,1'-bis(diphenylphosphino)ferrocene]palladium dichloride (8.22 mg, 10.14 µmol), and sodium carbonate (32.24 mg, 304.18 µmol) were added to 1,4-dioxane (2.5 mL) and water (0.3 mL). The mixture was subject to argon replacement four times, heated to 80 °C, and reacted for 1.5 hours. Water (20 mL) was added to the reaction mixture, and the mixture was extracted with ethyl acetate (40 mL × 3) three times. The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 2-(6-(4-(5-(benzyloxy)-6-methylpyrimidine-4-carbonyl)piperazin-1-yl)-4-(2-((2-chloro-4-(trifluo romethyl)phenyl)amino)-2-oxoethyl)-5-ethyl-7-oxo-4,7-dihydro-[1,2,4]triazolo[1,5-a]pyrimidin-2-yl)-6,7-dihydrothieno[3,2-c]pyridine-5(4H)-carboxylate **179a** (80 mg) in a yield of 83.28%.

MS m/z: 947.3 [M+1]

### Step 2

### 2-(6-(4-(5-(benzyloxy)-6-methylpyrimidine-4-carbonyl)piperazin-1-yl)-5-ethyl-7-oxo-2-(4,5,6,7-t etrahydrothieno[3,2-c]pyridin-2-yl)-[1,2,4]triazolo[1,5-a]pyrimidin-4(7H)-yl)-N-(2-chloro-4-(trifl uoromethyl)phenyl)acetamide

Tert-butyl 2-(6-(4-(5-(benzyloxy)-6-methylpyrimidine-4-carbonyl)piperazin-1-yl)-4-(2-((2-chloro-4-(trifluo romethyl)phenyl)amino)-2-oxoethyl)-5-ethyl-7-oxo-4,7-dihydro-[1,2,4] triazolo[1,5-a]pyrimidin-2-yl)-6,7-dihydrothieno[3,2-c]pyridine-5(4H)-carboxylate **179a** (80 mg, 84.44 µmol) was added to a solution of hydrogen chloride in 1,4-dioxane (3 mL, 4 M). The reaction mixture was stirred at room temperature for 1 hour. The mixture was concentrated under reduced pressure to obtain 2-(6-(4-(5-(benzyloxy)-6-methylpyrimidine-4-carbonyl)piperazin-1-yl)-5-ethyl-7-oxo-2-(4,5,6,7-t etrahydrothieno[3,2-c]pyridin-2-yl)-[1,2,4]triazolo[1,5-a]pyrimidin-4(7H)-yl)-N-(2-chloro-4-(trifl uoromethyl)phenyl)acetamide **179b** (70 mg) in a yield of 97.84%.

MS m/z (ESI): 847.2 [M+1]

### Step 3

### 2-(6-(4-(5-(benzyloxy)-6-methylpyrimidine-4-carbonyl)piperazin-1-yl)-5-ethyl-7-oxo-2-(5-(2,2,2 -trifluoroethyl)-4,5,6,7-tetrahydrothieno[3,2-c]pyridin-2-yl)-[1,2,4]triazolo[1,5-a]pyrimidin-4(7H )-yl)-N-(2-chloro-4-(trifluoromethyl)phenyl)acetamide

2-(6-(4-(5-(Benzyloxy)-6-methylpyrimidine-4-carbonyl)piperazin-1-yl)-5-ethyl-7-oxo-2 -(4,5,6,7-tetrahydrothieno[3,2-c]pyridin-2-yl)-[1,2,4]triazolo[1,5-a]pyrimidin-4(7H)-yl)-N-(2-chl oro-4-(trifluoromethyl)phenyl)acetamide **179b** (70 mg, 82.61 µmol), 2,2,2-trifluoroethyl trifluoromethanesulfonate **179c** (23.01 mg, 99.14 µmol, commercially available), and N,N-diisopropylethylamine (21.35 mg, 165.23 µmol) were added to N,N-dimethylformamide (2 mL). The reaction mixture was stirred at room temperature for 18 hours. Water (20 mL) was added to the reaction mixture, and the mixture was extracted with ethyl acetate (40 mL × 3) three times. The combined organic phases were washed with saturated sodium chloride solution (20 mL), dried over anhydrous sodium sulfate, filtered, and concentrated under reduced pressure. The resulting residue was purified by silica gel column chromatography (eluent: System A) to obtain 2-(6-(4-(5-(benzyloxy)-6-methylpyrimidine-4-carbonyl)piperazin-1-yl)-5-ethyl-7-oxo-2-(5-(2,2,2 -trifluoroethyl)-4,5,6,7-tetrahydrothieno[3,2-c]pyridin-2-yl)-[1,2,4]triazolo[1,5-a]pyrimidin-4(7H )-yl)-N-(2-chloro-4-(trifluoromethyl)phenyl)acetamide **179d** (40 mg) in a yield of 52.10%.

MS m/z (ESI): 929.2 [M+1]

### Step 4

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(5-(2,2,2-trifluoroethyl)-4,5,6,7-tetrahydrothieno[3,2-c]pyridin-2-yl)-[ 1,2,4]triazolo[1,5-a]pyrimidin-4(7H)-yl)acetamide

2-(6-(4-(5-(Benzyloxy)-6-methylpyrimidine-4-carbonyl)piperazin-1-yl)-5-ethyl-7-oxo-2 -(5-(2,2,2-trifluoroethyl)-4,5,6,7-tetrahydrothieno[3,2-c]pyridin-2-yl)-[1,2,4]triazolo[1,5-a]pyrimi din-4(7H)-yl)-N-(2-chloro-4-(trifluoromethyl)phenyl)acetamide **179d** (40 mg, 43.04 µmol) was added to dichloromethane (2.5 mL), followed by addition of boron trifluoride (0.8 mL, 1 M). The reaction mixture was stirred at 0 °C for 1 hour. After completion of the reaction, the mixture was concentrated under reduced pressure, and the residue was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin -1-yl)-7-oxo-2-(5-(2,2,2-trifluoroethyl)-4,5,6,7-tetrahydrothieno[3,2-c]pyridin-2-yl)-[1,2,4] triazolo[1,5-a]pyrimidin-4(7H)-yl)acetamide **179** (2.2 mg) in a yield of 5.04%.

MS m/z: 839.2 [M+1]
¹H NMR (400 MHz, DMSO) δ 10.37 (s, 1H), 10.22 (s, 1H), 8.58 (s, 1H), 8.05 (d, J = 8.5 Hz, 1H), 7.96 (d, J = 2.1 Hz, 1H), 7.72 (dd, J = 8.9, 2.1 Hz, 1H), 7.46 (s, 1H), 5.33 (s, 2H), 4.53 (d, J = 12.5 Hz, 1H), 3.77 (s, 2H), 3.39 (q, J = 10.0 Hz, 3H), 3.27 (d, J = 12.4 Hz, 2H), 3.05 - 2.95 (m, 5H), 2.86 (s, 3H), 2.64 (s, 1H), 2.45 (s, 3H), 1.20 (t, J = 7.5 Hz, 3H).

### Example 180

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(4-(2,2,2-trifluoroethyl)-2,3,4,5-tetrahydrobenzo[f][1,4]oxazepin-7-yl) -[1,2,4]triazolo[1,5-a]pyrimidin-4(7H)-yl)acetamide

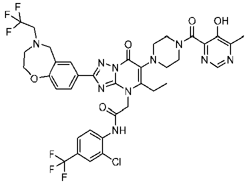

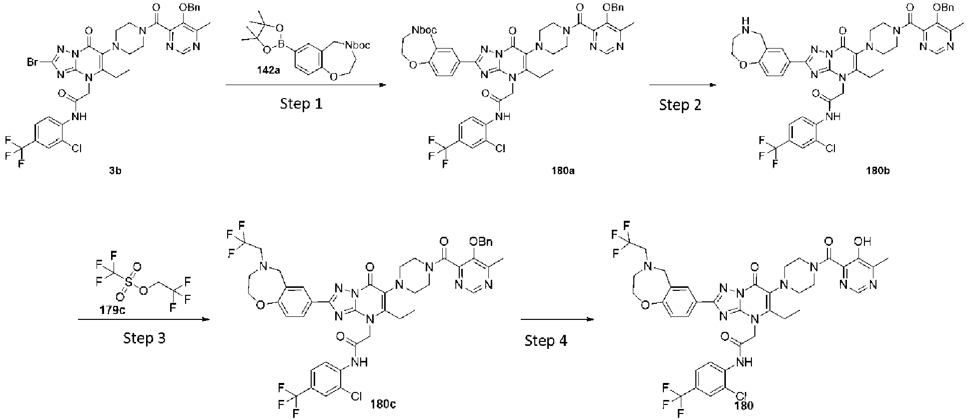

### Step 1

### tert-butyl 7-(6-(4-(5-(benzyloxy)-6-methylpyrimidine-4-carbonyl)piperazin-1-yl)-4-(2-((2-chloro-4-(trifluo romethyl)phenyl)amino)-2-oxoethyl)-5-ethyl-7-oxo-4,7-dihydro-[1,2,4]triazolo[1,5-a]pyrimidin-2-yl)-2,3-dihydrobenzo[f][1,4]oxazepine-4(5H)-carboxylate

At room temperature, 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (77 mg, 97.59 µmol), 7-(4,4,5,5-tetramethyl-1,3,2-dioxaborolan-2-yl)-2,3-dihydrobenzo[f][1,4]oxazepine-4(5H)-carbox ylate **142a** (43.95 mg, 117.11 µmol), [1,1'-bis(diphenylphosphino)ferrocene]palladium(II) dichloride (7.91 mg, 9.76 µmol), and sodium carbonate (31.03 mg, 292.78 µmol) were added to 1,4-dioxane (1 mL) and water (0.3 mL). The mixture was subject to argon replacement four times, then heated to 80 °C and reacted for 1.5 hours. Water (20 mL) was added to the reaction mixture, and the mixture was extracted with ethyl acetate (40 mL × 3) three times. The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 7-(6-(4-(5-(benzyloxy)-6-methylpyrimidine-4-carbonyl)piperazin-1-yl)-4-(2-((2-chloro-4-(trifluo romethyl)phenyl)amino)-2-oxoethyl)-5-ethyl-7-oxo-4,7-dihydro-[1,2,4]triazolo[1,5-a]pyrimidin-2-yl)-2,3-dihydrobenzo [f][1,4]oxazepine-4(5H)-carboxylate **180a** (48 mg) in a yield of51.37%.

MS m/z: 957.3 [M+1]

### Step 2

### 2-(6-(4-(5-(benzyloxy)-6-methylpyrimidine-4-carbonyl)piperazin-1-yl)-5-ethyl-7-oxo-2-(2,3,4,5-t etrahydrobenzo[f][1,4]oxazepin-7-yl)-[1,2,4]triazolo[1,5-a]pyrimidin-4(7H)-yl)-N-(2-chloro-4-(tr ifluoromethyl)phenyl)acetamide

Tert-butyl 7-(6-(4-(5-(benzyloxy)-6-methylpyrimidine-4-carbonyl)piperazin-1-yl)-4-(2-((2-chloro-4-(trifluo romethyl)phenyl)amino)-2-oxoethyl)-5-ethyl-7-oxo-4,7-dihydro-[1,2,4] triazolo[1,5-a]pyrimidin-2-yl)-2,3-dihydrobenzo[f][1,4]oxazepine-4(5H)-carboxylate **180a** (48 mg, 50.14 µmol) was added to a solution of hydrogen chloride in 1,4-dioxane (2 mL, 4 M). The mixture was reacted at room temperature for 1 hour. The mixture was concentrated under reduced pressure to obtain 2-(6-(4-(5-(benzyloxy)-6-methylpyrimidine-4-carbonyl)piperazin-1-yl)-5-ethyl-7-oxo-2-(2,3,4,5-t etrahydrobenzo[f][1,4]oxazepin-7-yl)-[1,2,4]triazolo[1,5-a]pyrimidin-4(7H)-yl)-N-(2-chloro-4-(tr ifluoromethyl)phenyl)acetamide **180b** (39 mg) in a yield of 90.74%.

MS m/z (ESI): 857.2 [M+1]

### Step 3

### 2-(6-(4-(5-(benzyloxy)-6-methylpyrimidine-4-carbonyl)piperazin-1-yl)-5-ethyl-7-oxo-2-(4-(2,2,2 -trifluoroethyl)-2,3,4,5-tetrahydrobenzo[f][1,4]oxazepin-7-yl)-[1,2,4]triazolo[1,5-a]pyrimidin-4(7 H)-yl)-N-(2-chloro-4-(trifluoromethyl)phenyl)acetamide

2-(6-(4-(5-(Benzyloxy)-6-methylpyrimidine-4-carbonyl)piperazin-1-yl)-5-ethyl-7-oxo-2 -(2,3,4,5-tetrahydrobenzo[f][1,4]oxazepin-7-yl)-[1,2,4]triazolo[1,5-a]pyrimidin-4(7H)-yl)-N-(2-c hloro-4-(trifluoromethyl)phenyl)acetamide **180b** (39 mg, 45.49 µmol), 2,2,2-trifluoroethyl trifluoromethanesulfonate **179c** (12.67 mg, 54.59 µmol), and N,N-diisopropylethylamine (11.76 mg, 90.99 µmol) were added to N,N-dimethylformamide (2 mL). The mixture was reacted at room temperature for 18 hours. Water (20 mL) was added to the reaction mixture, and the mixture was extracted with ethyl acetate (40 mL × 3) three times. The combined organic phases were washed with saturated sodium chloride solution (20 mL), dried over anhydrous sodium sulfate, filtered, and concentrated under reduced pressure. The resulting residue was purified by silica gel column chromatography (eluent: System A) to obtain 2-(6-(4-(5-(benzyloxy)-6-methylpyrimidine-4-carbonyl)piperazin-1-yl)-5-ethyl-7-oxo-2-(4-(2,2,2 -trifluoroethyl)-2,3,4,5-tetrahydrobenzo[f][1,4] oxazepin-7-yl)-[1,2,4]triazolo[1,5-a]pyrimidin-4(7H)-yl)-N-(2-chloro-4-(trifluoromethyl) phenyl)acetamide **180c** (33 mg) in a yield of 77.23%.

MS m/z (ESI): 939.2 [M+1]

### Step 4 N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(4-(2,2,2-trifluoroethyl)-2,3,4,5-tetrahydrobenzo[f][1,4]oxazepin-7-yl) -[1,2,4]triazolo[1,5-a]pyrimidin-4(7H)-yl)acetamide

2-(6-(4-(5-(Benzyloxy)-6-methylpyrimidine-4-carbonyl)piperazin-1-yl)-5-ethyl-7-oxo-2 -(4-(2,2,2-trifluoroethyl)-2,3,4,5-tetrahydrobenzo[f][1,4]oxazepin-7-yl)-[1,2,4]triazolo[1,5-a]pyri midin-4(7H)-yl)-N-(2-chloro-4-(trifluoromethyl)phenyl)acetamide **180c** (33 mg, 35.13 µmol) was added to dichloromethane (0.8 mL), followed by addition of trifluoroacetic acid (3 mL), and the reaction was performed at room temperature for 1 hour. After the reaction was complete, the mixture was concentrated under reduced pressure, and the residue was purified by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(4-(2,2,2-trifluoroethyl)-2,3,4,5-tetrahydrobenzo[f][1,4]oxazepin-7-yl) -[1,2,4] triazolo[1,5-a]pyrimidin-4(7H)-yl)acetamide **180** (3.60 mg) with a yield of 11.10%.

MS m/z: 849.2 [M+1]
¹H NMR (400 MHz, DMSO) δ 10.41 (s, 1H), 10.23 (s, 1H), 8.58 (s, 1H), 8.05 (d, J = 8.6 Hz, 1H), 7.98 - 7.92 (m, 3H), 7.71 (d, J = 8.3 Hz, 1H), 7.12 (d, J = 8.9 Hz, 1H), 5.38 (s, 2H), 4.54 (d, J = 12.4 Hz, 1H), 4.05 (d, J = 24.1 Hz, 5H), 3.53 (d, J = 11.9 Hz, 3H), 3.27 (q, J = 9.9 Hz, 3H), 3.19 (t, J = 4.3 Hz, 2H), 3.04 (s, 3H), 2.84 (d, J = 11.3 Hz, 1H), 2.67 (d, J = 10.0 Hz, 1H), 2.45 (s, 3H), 1.21 (t, J = 7.5 Hz, 3H).

### Example 181

### 2-(2-(Bicyclo[3.1.0]hex-2-en-3-yl)-5-ethyl-6-(4-(5-hydroxy-6-methylpyrimidine-4-carbonyl)pipe razin-1-yl)-7-oxo-[1,2,4]triazolo[1,5-a]pyrimidin-4(7H)-yl)-N-(4-((methyl-d3)thio)phenyl)aceta mide

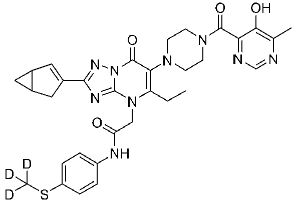

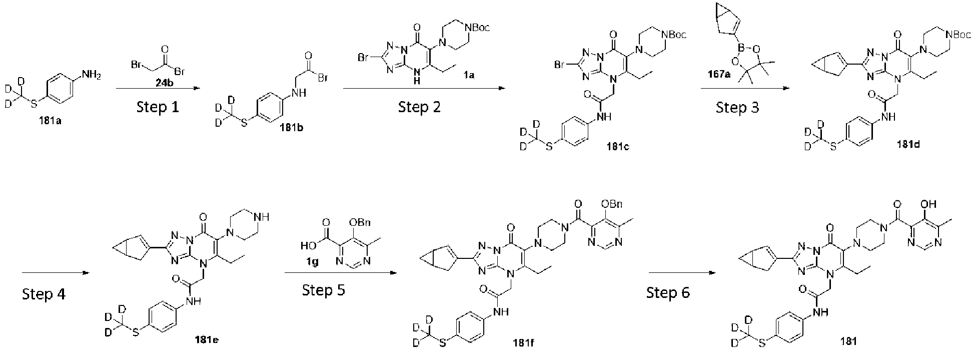

### Step 1

### (4-((Methyl-d3)thio)phenyl)glycinoyl bromide

Under an ice bath and argon atmosphere, 2-bromoacetyl bromide **24b** (468.30 mg, 2.32 mmol, 201.50 µL) was slowly added dropwise to a solution of 4-((methyl-d3)thio)aniline **181a** (300 mg, 2.11 mmol, prepared according to published patent CN115057803) and 4-dimethylaminopyridine (257.68 mg, 2.11 mmol) in dichloromethane (4.88 mL), and the reaction was performed at room temperature for 18 hours. Water (20 mL) was added to the reaction mixture, and the mixture was extracted with ethyl acetate (40 mL×3) three times. The combined organic phase was washed with saturated sodium chloride solution (20 mL), dried over anhydrous sodium sulfate, filtered, and concentrated under reduced pressure. The resulting residue was purified by silica gel column chromatography (eluent: System A) to obtain (4-((methyl-d3)thio)phenyl)glycinoyl bromide **181b** (330 mg) with a yield of 59.45%.

MS m/z (ESI): 262.9 [M+1]

### Step 2

### tert-butyl 4-(2-bromo-5-ethyl-4-(2-((4-((methyl-d3)thio)phenyl)amino)-2-oxoethyl)-7-oxo-4,7-dihydro-[1,2 ,4]triazolo[1,5-a]pyrimidin-6-yl)piperazine-1-carboxylate

Tert-butyl 4-(2-bromo-5-ethyl-7-oxo-4,7-dihydro-[1,2,4]triazolo[1,5-a]pyrimidin-6-yl)piperazine-1-carboxy late 1a (200 mg, 468.06 µmol) and (4-((methyl-d3)thio)phenyl)glycinoyl bromide **181b** (147.81 mg, 561.67 µmol) were added to N,N-dimethylformamide (5 mL), followed by addition of N,N-diisopropylethylamine (302.46 mg, 2.34 mmol). The temperature was raised to 70 °C, and the reaction was performed for 2 hours. Water (20 mL) was added to the reaction mixture, and the mixture was extracted with ethyl acetate (30 mL ×3) three times. The combined organic phase was washed with saturated sodium chloride solution (20 mL), dried over anhydrous sodium sulfate, filtered, and concentrated under reduced pressure. The resulting residue was purified by silica gel column chromatography (eluent: System A) to obtain tert-butyl 4-(2-bromo-5-ethyl-4-(2-((4-((methyl-d3)thio)phenyl)amino)-2-oxoethyl)-7-oxo-4,7-dihydro-[1,2 ,4]triazolo[1,5-a]pyrimidin-6-yl)piperazine-1-carboxylate **181c** (200 mg) with a yield of 70.10%.

MS m/z: 609.1 [M+1]

### Step 3

### tert-butyl 4-(2-(bicyclo[3.1.0]hex-2-en-3-yl)-5-ethyl-4-(2-((4-((methyl-d3)thio)phenyl)amino)-2-oxoethyl)-7-oxo-4,7-dihydro-[1,2,4]triazolo[1,5-a]pyrimidin-6-yl)piperazine-1-carboxylate

At room temperature, tert-butyl 4-(2-bromo-5-ethyl-4-(2-((4-((methyl-d3)thio)phenyl)amino)-2-oxoethyl)-7-oxo-4,7-dihydro-[1,2 ,4]triazolo[1,5-a]pyrimidin-6-yl)piperazine-1-carboxylate **181c** (200 mg, 328.11 µmol), 2-(bicyclo[3.1.0]hex-2-en-3-yl)-4,4,5,5-tetramethyl-1,3,2-dioxaborolane **167a** (67.62 mg, 328.11 µmol), [1,1'-bis(diphenylphosphino)ferrocene]palladium(II) dichloride (26.60 mg, 32.81 µmol), and sodium carbonate (104.33 mg, 984.33 µmol) were dissolved in 1,4-dioxane (2 mL) and water (0.6 mL). The mixture was subject to argon replacement four times, the temperature was raised to 80 °C, and the reaction was performed for 1.5 hours. Water (20 mL) was added to the reaction mixture, and the mixture was extracted with ethyl acetate (40 ml ×3) three times. The combined organic phase was washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl 4-(2-(bicyclo[3.1.0]hex-2-en-3-yl)-5-ethyl-4-(2-((4-((methyl-d3)thio)phenyl)amino)-2-oxoethyl)-7-oxo-4,7-dihydro-[1,2,4]triazolo [1,5-a]pyrimidin-6-yl)piperazine-1-carboxylate **181d** (113 mg) with a yield of 56.57%.

MS m/z (ESI): 553.1 [M-56+1]

### Step 4

### 2-(2-(Bicyclo[3.1.0]hex-2-en-3-yl)-5-ethyl-7-oxo-6-(piperazin-1-yl)-[1,2,4]triazolo[1,5-a]pyrimid in-4(7H)-yl)-N-(4-((methyl-d3)thio)phenyl)acetamide

Tert-butyl 4-(2-(bicyclo[3.1.0]hex-2-en-3-yl)-5-ethyl-4-(2-((4-((methyl-d3)thio)phenyl) amino)-2-oxoethyl)-7-oxo-4,7-dihydro-[1,2,4]triazolo[1,5-a]pyrimidin-6-yl)piperazine-1-carboxy late **181d** (113 mg, 185.62 µmol) was added to dichloromethane (2.5 mL), followed by addition of trifluoroacetic acid (0.8 mL), and the reaction was performed at room temperature for 1 hour. The reaction mixture was concentrated under reduced pressure to obtain 2-(2-(bicyclo[3.1.0]hex-2-en-3-yl)-5-ethyl-7-oxo-6-(piperazin-1-yl)-[1,2,4]triazolo[1,5-a]pyrimid in-4(7H)-yl)-N-(4-((methyl-d3)thio)phenyl)acetamide **181e** (90 mg) with a yield of 95.32%.

MS m/z(ESI): 509.3 [M+1]

### Step 5

### 2-(6-(4-(5-(benzyloxy)-6-methylpyrimidine-4-carbonyl)piperazin-1-yl)-2-(bicyclo[3.1.0]hex-2-en -3-yl)-5-ethyl-7-oxo-[1,2,4]triazolo[1,5-a]pyrimidin-4(7H)-yl)-N-(4-((methyl-d3)thio)phenyl)acet amide

2-(2-(Bicyclo[3.1.0]hex-2-en-3-yl)-5-ethyl-7-oxo-6-(piperazin-1-yl)-[1,2,4]triazolo[1,5-a]pyrimidin-4(7H)-yl)-N-(4-((methyl-d3)thio)phenyl)acetamide **181e** (90 mg, 176.94 µmol), 5-(benzyloxy)-6-methylpyrimidine-4-carboxylic acid **1g** (56.18 mg, 230.02 µmol), 1-(3-dimethylaminopropyl)-3-ethylcarbodiimide hydrochloride (50.88 mg, 265.41 µmol), 1-hydroxybenzotriazole (35.86 mg, 265.41 µmol), and N,N-diisopropylethylamine (114.34 mg, 884.69 µmol) were added to N,N-dimethylformamide (5 mL), and the reaction was performed at room temperature for 4 hours. Water (20 mL) was added to the reaction mixture, and the mixture was extracted with ethyl acetate (40 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain 2-(6-(4-(5-(benzyloxy)-6-methylpyrimidine-4-carbonyl)piperazin-1-yl)-2-(bicyclo[3.1.0]hex-2-en -3-yl)-5-ethyl-7-oxo-[1,2,4]triazolo[1,5-a]pyrimidin-4(7H)-yl)-N-(4-((methyl-d3)thio)phenyl)acet amide **181f** (100 mg) in a yield of 76.91%.

MS m/z: 735.2 [M+1]

### Step 6

### 2-(2-(bicyclo[3.1.0]hex-2-en-3-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(4-((methyl-d3)thio)phenyl)acetami de

Trifluoroacetic acid (3 mL) was added to a solution of 2-(6-(4-(5-(benzyloxy)-6-methylpyrimidine-4-carbonyl)piperazin-1-yl)-2-(bicyclo[3.1.0]hex-2-en -3-yl)-5-ethyl-7-oxo-[1,2,4]triazolo[1,5-a]pyrimidin-4(7H)-yl)-N-(4-((methyl-d3)thio)phenyl)acet amide **181f** (30 mg, 40.82 µmol) in dichloromethane (1 mL), and the reaction was performed at room temperature for 1 hour. After the reaction was complete, the mixture was concentrated under reduced pressure, and the residue was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain 2-(2-(bicyclo[3.1.0]hex-2-en-3-yl)-5-ethyl-6-(4-(5-hydroxy-6-methylpyrimidine-4-carbonyl)piper azin-1-yl)-7-oxo-[1,2,4]triazolo[1,5-a]pyrimidin-4(7H)-yl)-N-(4-((methyl-d3)thio)phenyl)acetami de **181** (3.55 mg) in a yield of 12.95%.

MS m/z: 645.3 [M+1]
¹H NMR (400 MHz, DMSO) δ 10.46 (d, J = 6.0 Hz, 1H), 10.23 (s, 1H), 8.58 (s, 1H), 7.56 - 7.44 (m, 2H), 7.24 (d, J = 8.5 Hz, 2H), 6.78 (q, J = 2.0 Hz, 1H), 5.12 (s, 2H), 4.52 (d, J = 12.6 Hz, 1H), 3.30 - 3.21 (m, 1H), 3.01 - 2.87 (m, 4H), 2.83 - 2.60 (m, 3H), 2.44 (s, 3H), 1.96 (tt, J = 6.4, 2.9 Hz, 1H), 1.78 (p, J = 6.8 Hz, 1H), 1.16 (t, J = 7.4 Hz, 3H), 1.00 (td, J = 7.7, 3.7 Hz, 1H).

### Example 182

### (E)-3-(4-(2-((2-chloro-4-(trifluoromethyl)phenyl)amino)-2-oxoethyl)-5-ethyl-6-(4-(5-hydroxy-6-methylpyrimidine-4-carbonyl)piperazin-1-yl)-7-oxo-4,7-dihydro-[1,2,4]triazolo[1,5-a]pyrimidin-2-yl)-N,N-dimethylacrylamide

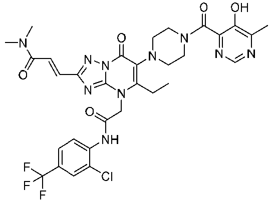

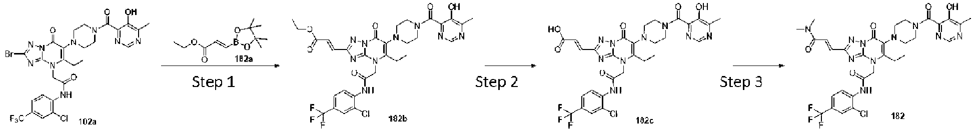

### Step 1

### ethyl (E)-3-(4-(2-((2-chloro-4-(trifluoromethyl)phenyl)amino)-2-oxoethyl)-5-ethyl-6-(4-(5-hydroxy-6-methylpyrimidine-4-carbonyl)piperazin-1-yl)-7-oxo-4,7-dihydro-[1,2,4]triazolo[1,5-a]pyrimidin-2-yl)acrylate

2-(2-Bromo-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamide **102a** (150 mg, 214.63 µmol), ethyl (E)-3-(4,4,5,5-tetramethyl-1,3,2-dioxaborolan-2-yl)acetate **182a** (58.23 mg, 257.56 µmol, commercially available), [1,1'-bis(diphenylphosphino)ferrocene]palladium(II) dichloride (17.40 mg, 21.46 µmol), and sodium carbonate (68.25 mg, 643.89 µmol) were dissolved in 1,4-dioxane (10 mL) and water (3 mL). The mixture was subject to argon replacement four times, then heated to 80 °C, and the reaction was performed for 1.5 hours. Water (20 mL) was added to the reaction mixture, and the mixture was extracted with ethyl acetate for 3 times (40 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System B) to obtain ethyl (E)-3-(4-(2-((2-chloro-4-(trifluoromethyl)phenyl)amino)-2-oxoethyl)-5-ethyl-6-(4-(5-hydroxy-6-methylpyrimidine-4-carbonyl)piperazin-1-yl)-7-oxo-4,7-dihydro-[1,2,4]triazolo[1,5-a]pyrimidin-2-yl)acrylate **182b** (87 mg) in a yield of 56.45%.

MS m/z (ESI): 718.2 [M+1]

### Step 2

### (E)-3-(4-(2-((2-chloro-4-(trifluoromethyl)phenyl)amino)-2-oxoethyl)-5-ethyl-6-(4-(5-hydroxy-6-methylpyrimidine-4-carbonyl)piperazin-1-yl)-7-oxo-4,7-dihydro-[1,2,4]triazolo[1,5-a]pyrimidin-2-yl)acrylic acid

Ethyl (E)-3-(4-(2-((2-chloro-4-(trifluoromethyl)phenyl)amino)-2-oxoethyl)-5-ethyl-6-(4-(5-hydroxy-6-methylpyrimidine-4-carbonyl)piperazin-1-yl)-7-oxo-4,7-dihydro-[1,2,4]triazolo [1,5-a] pyrimidin-2-yl)acrylate **182b** (87 mg, 121.16 µmol) was added to tetrahydrofuran (2.5 mL) and water (1.25 mL), lithium hydroxide monohydrate (10.17 mg, 242.31 µmol) was added, and the reaction was performed at room temperature for half an hour. Anhydrous sodium sulfate was added for drying, followed by filtration, and the mixture was concentrated under reduced pressure to obtain (E)-3-(4-(2-((2-chloro-4-(trifluoromethyl)phenyl)amino)-2-oxoethyl)-5-ethyl-6-(4-(5-hydroxy-6-methylpyrimidine-4-carbonyl)piperazin-1-yl)-7-oxo-4,7-dihydro-[1,2,4]triazolo[1,5-a]pyrimidin-2-yl)acrylic acid **182c** (35 mg) with a yield of 41.87%.

MS m/z: 690.2 [M+1]

### Step 3

### (E)-3-(4-(2-((2-chloro-4-(trifluoromethyl)phenyl)amino)-2-oxoethyl)-5-ethyl-6-(4-(5-hydroxy-6-methylpyrimidine-4-carbonyl)piperazin-1-yl)-7-oxo-4,7-dihydro-[1,2,4]triazolo[1,5-a]pyrimidin-2-yl)-N,N-dimethylacrylamide

(E)-3-(4-(2-((2-Chloro-4-(trifluoromethyl)phenyl)amino)-2-oxoethyl)-5-ethyl-6-(4-(5-h ydroxy-6-methylpyrimidine-4-carbonyl)piperazin-1-yl)-7-oxo-4,7-dihydro-[1,2,4]triazolo[1,5-a]p yrimidin-2-yl)acrylic acid **182c** (30 mg, 43.48 µmol) and N-methylmethanamine (2 M, 32.61 µL) were added to N,N-dimethylformamide (1.5 mL), 1-butylphosphoric anhydride (62.65 mg, 173.91 µmol) and N,N-diisopropylethylamine (16.86 mg, 130.43 µmol) were added, and the reaction was performed at room temperature for 18 hours. Water (20 mL) was added to the reaction mixture, the mixture was extracted with ethyl acetate for 3 times (40 mL×3), the combined organic phase was washed with saturated sodium chloride solution (20 mL), dried over anhydrous sodium sulfate, filtered, and concentrated under reduced pressure, and the residue was separated and purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (E)-3-(4-(2-((2-chloro-4-(trifluoromethyl)phenyl)amino)-2-oxoethyl)-5-ethyl-6-(4-(5-hydroxy-6-methylpyrimidine-4-carbonyl)piperazin-1-yl)-7-oxo-4,7-dihydro-[1,2,4]triazolo[1,5-a]pyrimidin-2-yl)-N,N-dimethylacrylamide **182** (2.74 mg) with a yield of 8.61%.

MS m/z (ESI): [M+1] 717.3
¹H NMR (400 MHz, DMSO) δ 10.37 (s, 1H), 10.23 (s, 1H), 8.58 (s, 1H), 8.05 (d, J = 8.6 Hz, 1H), 7.96 (d, J = 2.1 Hz, 1H), 7.72 (dd, J = 8.7, 2.1 Hz, 1H), 7.43 (d, J = 15.3 Hz, 1H), 7.27 (d, J = 15.3 Hz, 1H), 5.35 (s, 2H), 4.53 (d, J = 12.6 Hz, 1H), 3.28 (d, J = 12.7 Hz, 2H), 3.10 (s, 3H), 3.01 (q, J = 8.5 Hz, 4H), 2.94 (s, 3H), 2.83 (d, J = 11.4 Hz, 1H), 2.65 (d, J = 11.1 Hz, 1H), 2.45 (s, 3H), 1.20 (t, J = 7.4 Hz, 3H).

### Example 183

### N-(2-chloro-4-(trifluoromethyl)phenyl)-2-(5-ethyl-2-(1-ethyl-1H-indol-5-yl)-6-(4-(5-hydroxy-6-methylpyrimidine-4-carbonyl)piperazin-1-yl)-7-oxo-[1,2,4]triazolo[1,5-a]pyrimidin-4(7H)-yl)ace tamide

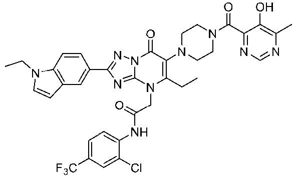

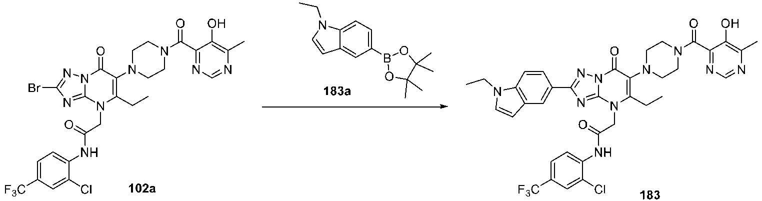

At room temperature, 2-(2-bromo-5-ethyl-6-(4-(5-hydroxy-6-methylpyrimidine-4 -carbonyl)piperazin-1-yl)-7-oxo-[1,2,4]triazolo[1,5-a]pyrimidin-4(7H)-yl)-N-(2-chloro-4-(trifluor omethyl)phenyl)acetamide **102a** (50 mg, 71.54 µmol), 1-ethyl-5-(4,4,5,5-tetramethyl-1,3,2-dioxaborolan-2-yl)-1H-indole **183a** (25.22 mg, 93.01 µmol, commercially available), [1,1'-bis(diphenylphosphino)ferrocene]palladium(II) dichloride (5.80 mg, 7.15 µmol), and sodium carbonate (22.75 mg, 214.63 µmol) were dissolved in 1,4-dioxane (5 mL) and water (0.5 mL), the mixture was subject to argon replacement three times, the temperature was raised to 85 °C, and the reaction was performed for 2 hours. Water (20 mL) was added to the reaction mixture, the mixture was extracted with ethyl acetate (40 mL ×3) for 3 times, the combined organic phase was washed with saturated sodium chloride solution (20 mL), dried over anhydrous sodium sulfate, filtered, and concentrated under reduced pressure, and the residue was separated and purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(5-ethyl-2-(1-ethyl-1H-indol-5-yl)-6-(4-(5-hydroxy-6-methylpyrimidine-4-carbonyl)piperazin-1-yl)-7-oxo-[1,2,4]triazolo[1,5-a]pyrimidin-4(7H)-yl)ace tamide **183** (14.61 mg) with a yield of 26.22%.

MS m/z (ESI): 763.2 [M+1]
¹H NMR (400 MHz, DMSO-d6) δ 10.42 (s, 1H), 10.25 (s, 1H), 8.59 (s, 1H), 8.35 (d, J = 1.5 Hz, 1H), 8.07 (d, J = 8.6 Hz, 1H), 7.97 (d, J = 2.1 Hz, 1H), 7.93 (dd, J = 8.6, 1.6 Hz, 1H), 7.71 (dd, J = 8.7, 2.1 Hz, 1H), 7.59 (d, J = 8.7 Hz, 1H), 7.47 (d, J = 3.1 Hz, 1H), 6.55 (d, J = 3.2 Hz, 1H), 5.41 (s, 2H), 4.55 (d, J = 12.6 Hz, 1H), 4.25 (t, J = 7.2 Hz, 2H), 3.55 (d, J = 9.3 Hz, 3H), 3.28 (t, J = 12.6 Hz, 1H), 3.03 (d, J = 8.0 Hz, 3H), 2.85 (d, J = 11.2 Hz, 1H), 2.68 (d, J = 10.6 Hz, 1H), 2.45 (s, 3H), 1.38 (t, J = 7.2 Hz, 3H), 1.22 (t, J = 7.4 Hz, 3H).

### Example 184

### N-(2-chloro-4-(trifluoromethyl)phenyl)-2-(5-ethyl-6-(4-(5-hydroxy-6-methylpyrimidine-4-carbon yl)piperazin-1-yl)-2-(1-isopropyl-1H-indol-5-yl)-7-oxo-[1,2,4]triazolo[1,5-a]pyrimidin-4(7H)-yl) acetamide

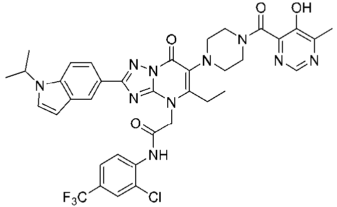

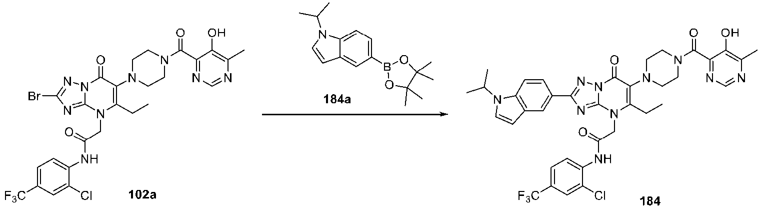

At room temperature, 2-(2-bromo-5-ethyl-6-(4-(5-hydroxy-6-methylpyrimidine -4-carbonyl)piperazin-1-yl)-7-oxo-[1,2,4]triazolo[1,5-a]pyrimidin-4(7H)-yl)-N-(2-chloro-4-(triflu oromethyl)phenyl)acetamide **102a** (160 mg, 228.94 µmol), 1-isopropyl-5-(4,4,5,5-tetramethyl-1,3,2-dioxaborolan-2-yl)-1H-indole **184a** (84.88 mg, 297.62 µmol, prepared according to published patent WO2017177836), [1,1"-bis(diphenylphosphino)ferrocene]palladium(II) dichloride (18.56 mg, 22.89 µmol), and sodium carbonate (72.80 mg, 686.82 µmol) were dissolved in 1,4-dioxane (5 mL) and water (0.5 mL). The mixture was subject to argon replacement three times, heated to 85 °C, and reacted for 2 hours. Water (20 mL) was added to the reaction mixture, and the mixture was extracted with ethyl acetate (40 mL × 3) three times. The combined organic phases were washed with saturated sodium chloride solution (20 mL), dried over anhydrous sodium sulfate, filtered, and concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-2-(1-isopropyl-1H-indol-5-yl)-7-oxo-[1,2,4]triazolo[1,5-a]pyrimidin-4(7H)-yl) acetamide **184** (13.06 mg) with a yield of 7.19%.

MS m/z (ESI): 777.3 [M+1]
¹H NMR (400 MHz, DMSO-d6) δ 10.44 (s, 1H), 10.26 (s, 1H), 8.60 (s, 1H), 8.35 (s, 1H), 8.08 (d, J = 8.6 Hz, 1H), 8.02 - 7.87 (m, 2H), 7.71 (dd, J = 8.7, 2.0 Hz, 1H), 7.60 (dd, J = 21.8, 6.0 Hz, 2H), 6.58 (d, J = 3.3 Hz, 1H), 5.42 (s, 2H), 4.80 (hept, J = 7.0 Hz, 1H), 4.55 (d, J = 12.5 Hz, 1H), 3.53 (s, 3H), 3.28 (s, 1H), 3.03 (q, J = 9.4, 8.2 Hz, 3H), 2.87 (s, 1H), 2.69 (s, 1H), 2.46 (s, 3H), 1.48 (d, J = 6.6 Hz, 6H), 1.23 (t, J = 7.4 Hz, 3H).

### Example 185

### N-(2-chloro-4-(trifluoromethyl)phenyl)-2-(5-ethyl-6-(4-(5-hydroxy-6-methylpyrimidine-4-carbon yl)piperazin-1-yl)-2-(1-(methyl-d3)-1H-indol-5-yl)-7-oxo-[1,2,4]triazolo[1,5-a]pyrimidin-4(7H)-yl)acetamide

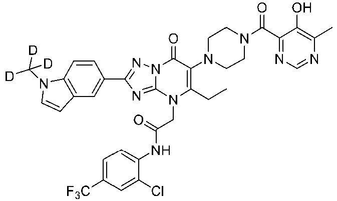

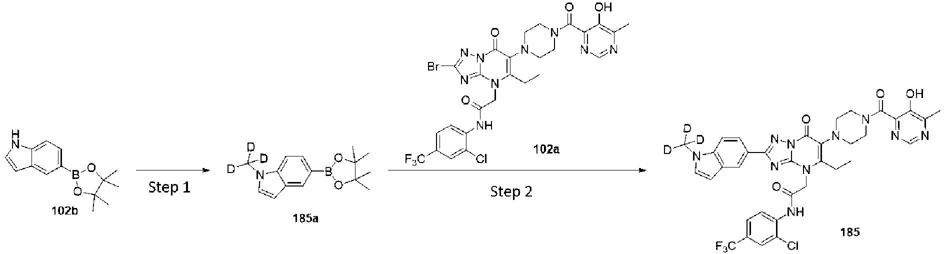

### Step 1

### 1-(methyl-d3)-5-(4,4,5,5-tetramethyl-1,3,2-dioxaborolan-2-yl)-1H-indole

Under ice bath and argon atmosphere, sodium hydride (65.81 mg, 1.65 mmol, 60% purity) was added to a solution of 5-(4,4,5,5-tetramethyl-1,3,2-dioxaborolan-2-yl)-1H-indole **102b** (200 mg, 822.68 µmol) in N,N-dimethylformamide (3 mL), followed by addition of iodomethane-d3 (178.88 mg, 1.23 mmol). The reaction was performed at room temperature for 18 hours. Water (20 mL) was added to the reaction mixture to quench the reaction, and the mixture was extracted with ethyl acetate (20 mL × 3). The combined organic phases were washed with saturated sodium chloride solution (20 mL), dried over anhydrous sodium sulfate, filtered, and concentrated under reduced pressure. The resulting residue was purified by silica gel column chromatography (eluent: System A) to obtain 1-(methyl-d3)-5-(4,4,5,5-tetramethyl-1,3,2 -dioxaborolan-2-yl)-1H-indole **185a** (140 mg) with a yield of 65.41%.

MS m/z (ESI): 261.2 [M+1]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-2-(1-(methyl-d3)-1H-indol-5-yl)-7-oxo-[1,2,4]triazolo[1,5-a]pyrimidin-4(7H)-yl)acetamide

At room temperature, 2-(2-bromo-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(triflu oromethyl)phenyl)acetamide **102a** (80 mg, 114.47 µmol), 1-(methyl-d3)-5-(4,4,5,5-tetramethyl-1,3,2-dioxaborolan-2-yl)-1H-indole **185a** (35.74 mg, 137.36 µmol), [1,1"-bis(diphenylphosphino)ferrocene]palladium(II) dichloride (9.28 mg, 11.45 µmol), and sodium carbonate (36.40 mg, 343.41 µmol) were added to 1,4-dioxane (5 mL) and water (1 mL). The mixture was subject to argon replacement three times, heated to 85 °C, and reacted for 2 hours. Water (20 mL) was added to the reaction mixture, and the mixture was extracted with ethyl acetate (40 mL × 3) three times. The combined organic phases were washed with saturated sodium chloride solution (20 mL), dried over anhydrous sodium sulfate, filtered, and concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(2-chloro-4-(trifluoromethyl)phenyl)-2-(5-ethyl-6-(4-(5-hydroxy-6-methylpyrimidine-4-carbon yl)piperazin-1-yl)-2-(1-(methyl-d3)-1H-indol-5-yl)-7-oxo-[1,2,4]triazolo[1,5-a]pyrimidin-4(7H)-yl)acetamide **185** (10.1 mg) with a yield of 11.61%.

MS m/z (ESI): 752.2 [M+1]
¹H NMR (400 MHz, DMSO-d6) δ 10.42 (s, 1H), 10.25 (s, 1H), 8.59 (s, 1H), 8.35 (d, J = 1.5 Hz, 1H), 8.08 (d, J = 8.6 Hz, 1H), 8.02 - 7.90 (m, 2H), 7.71 (dd, J = 8.7, 2.1 Hz, 1H), 7.55 (d, J = 8.6 Hz, 1H), 7.40 (d, J = 3.1 Hz, 1H), 6.55 (d, J = 3.1 Hz, 1H), 5.42 (s, 2H), 4.55 (d, J = 12.5 Hz, 1H), 3.70 - 3.50 (m, 3H), 3.28 (t, J = 12.6 Hz, 1H), 3.22 - 2.97 (m, 3H), 2.85 (d, J = 11.2 Hz, 1H), 2.68 (d, J = 10.7 Hz, 1H), 2.46 (s, 3H), 1.22 (t, J = 7.4 Hz, 3H).

### Example 186

### N-(2-chloro-4-(trifluoromethyl)phenyl)-2-(5-ethyl-6-(4-(3-hydroxypicolinoyl)piperazin-1-yl)-2-( 5-methoxy-1,3a,4,5,6,6a-hexahydropentalen-2-yl)-7-oxo-[1,2,4]triazolo[1,5-a]pyrimidin-4(7H)-y l)acetamide

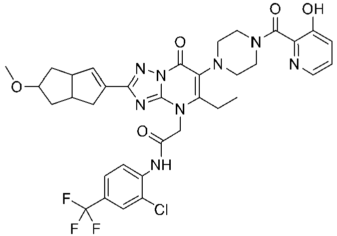

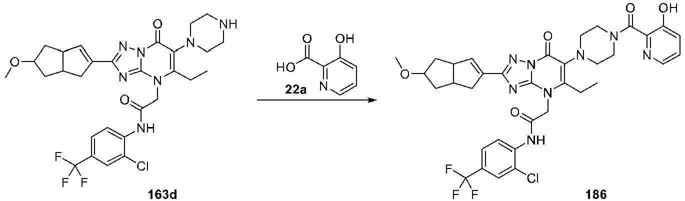

3-Hydroxypicolinic acid **22a** (5.05 mg, 36.29 µmol), 1-hydroxybenzotriazole (6.54 mg, 48.38 µmol), 1-(3-dimethylaminopropyl)-3-ethylcarbodiimide hydrochloride (9.27 mg, 48.38 µmol), and N,N-diisopropylethylamine (9.38 mg, 72.57 µmol) were dissolved in N,N-dimethylformamide (1 mL), and the mixture was stirred at 25 °C under nitrogen for 10 minutes. Then N-(2-chloro-4-(trifluoromethyl)phenyl)-2-(5-ethyl-2-(5-methoxy-1,3a,4,5,6,6a-hexahydropentale n-2-yl)-7-oxo-6-(piperazin-1-yl)-[1,2,4]triazolo[1,5-a]pyrimidin-4(7H)-yl)acetamide **163d** (15 mg, 24.19 µmol) was added, and the mixture was stirred at 30°C for 16 hours. After completion of the reaction, the reaction mixture was purified by preparative liquid chromatography (Waters 3767/QDA column: SunFire Sunfire C18, 19×250 mm, 10 µm; mobile phase A: 10 mmol/L A: 0.05% TFA/H2O, B: acetonitrile; flow rate: 20 mL/min; gradient: 55-65%; retention time: 9 min, 17 min) to obtain N-(2-chloro-4-(trifluoromethyl)phenyl)-2-(5-ethyl-6-(4-(3-hydroxypicolinoyl)piperazin-1-yl)-2-( 5-methoxy-1,3a,4,5,6,6a-hexahydropentalen-2-yl)-7-oxo-[1,2,4]triazolo[1,5-a]pyrimidin-4(7H)-y l)acetamide **186** (4.94 mg) with a yield of 27.55%.

MS m/z (ESI): 741.2 [M+1]
¹H NMR (400 MHz, DMSO) δ 10.36 (s, 2H), 8.07 - 8.05 (m, 2H), 7.96 (s, 1H), 7.70 (d, J = 8.0 Hz, 1H), 7.29 - 7.28 (m, 2H), 6.51 (s, 1H), 5.30 (s, 2H), 4.54 (d, J = 12.0 Hz, 1H), 3.73 - 3.70 (m, 1H), 3.51 - 3.42 (m, 2H), 3.25-3.22 (m, 2H), 3.13 (s, 3H), 2.98-2.90 (m, 4H), 2.79-2.77 (m, 2H), 2.67-2.62 (m, 1H), 2.58 (m, 2H), 2.13- 2.04 (m, 2H), 1.47-1.38 (m, 2H), 1.18 (t, J = 8.0 Hz, 3H).

### Example 187

### 2-(5-ethyl-6-(4-(3-hydroxypicolinoyl)piperazin-1-yl)-7-oxo-2-(3,3a,4,6a-tetrahydro-1H-cyclopen ta[c]furan-5-yl)-[1,2,4]triazolo[1,5-a]pyrimidin-4(7H)-yl)-N-(4-(pentafluoro-λ⁶-sulfaneyl)phenyl) acetamide

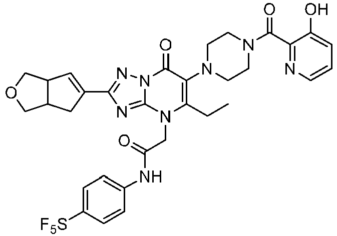

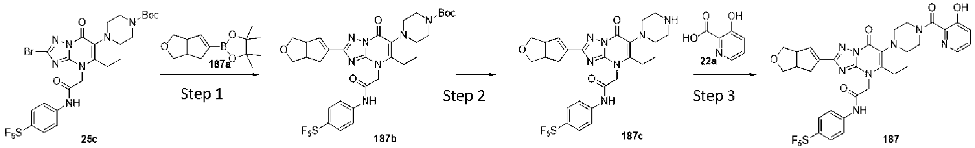

### Step 1

### tert-butyl 4-(5-ethyl-7-oxo-4-(2-oxo-2-((4-(pentafluoro-λ⁶-sulfaneyl)phenyl)amino)ethyl) -2-(3,3a,4,6a-tetrahydro-1H-cyclopenta[c]furan-5-yl)-4,7-dihydro-[1,2,4]triazolo[1,5-a]pyrimidin -6-yl)piperazine-1-carboxylate

Sodium carbonate (97.27 mg, 917.71 µmol), tert-butyl 4-(2-bromo-5-ethyl-7-oxo-4-(2-oxo-2-((4-(pentafluoro-λ⁶-su1faneyl)phenyl)amino)ethyl)-4,7-dih ydro-[1,2,4]triazolo[1,5-a]pyrimidin-6-yl)piperazine-1-carboxylate 25c (300 mg, 437.00 µmol), and [1,1'-bis(diphenylphosphino)ferrocene]palladium(II) dichloride (32.42 mg, 43.70 µmol) were added to a solution of 4,4,5,5-tetramethyl-2-(3,3a,4,6a-tetrahydro-1H-cyclopenta[c]furan-5-yl)-1,3,2-dioxaborolane **187a** (206.37 mg, 874.01 µmol) in 1,4-dioxane (3 mL) and water (0.3 mL). The mixture was stirred at 90 °C under nitrogen for 2 hours. After completion of the reaction, water (30 mL) was added to the reaction mixture, and the mixture was extracted with ethyl acetate (30 mL × 3). The combined organic phases were washed with saturated sodium chloride solution (30 mL × 2), dried over anhydrous sodium sulfate, filtered, and concentrated under reduced pressure. The resulting residue was purified by silica gel column chromatography (eluent: System A) to obtain tert-butyl 4-(5-ethyl-7-oxo-4-(2-oxo-2-((4-(pentafluoro-λ⁶ -sulfaneyl)phenyl)amino)ethyl)-2-(3,3a,4,6a-tetra hydro-1H-cyclopenta[c]furan-5-yl)-4,7-dihydro-[1,2,4]triazolo[1,5-a]pyrimidin-6-yl)piperazine-1 -carboxylate **187b** (186 mg) with a yield of 14.87%.

MS m/z (ESI): 616.1 [M+1-100]

### Step 2

### 2-(5-ethyl-7-oxo-6-(piperazin-1-yl)-2-(3,3a,4,6a-tetrahydro-1H-cyclopenta[c]furan-5-yl)-[1,2,4]tr iazolo[1,5-a]pyrimidin-4(7H)-yl)-N-(4-(pentafluoro-λ⁶-sulfaneyl)phenyl)acetamide

Tert-butyl 4-(5-ethyl-7-oxo-4-(2-oxo-2-((4-(pentafluoro-λ⁶-sulfaneyl)phenyl)amino) ethyl)-2-(3,3a,4,6a-tetrahydro-1H-cyclopenta[c]furan-5-yl)-4,7-dihydro-[1,2,4]triazolo[1,5-a]pyri midin-6-yl)piperazine-1-carboxylate **187b** (186 mg, 259.87 µmol) was dissolved in dichloromethane (10 mL), and trifluoroacetic acid (2 mL) was added at 0 °C. The reaction mixture was stirred at 25 °C for 0.5 hours. After completion of the reaction, the pH of the reaction mixture was adjusted to 8-9 with sodium bicarbonate solution, and the mixture was extracted with ethyl acetate (20 mL × 3). The combined organic phases were washed with saturated sodium chloride solution (30 mL × 2), dried over anhydrous sodium sulfate, filtered, and concentrated under reduced pressure to obtain 2-(5-ethyl-7-oxo-6-(piperazin-1-yl)-2-(3,3a,4,6a-tetrahydro-1H-cyclopenta[c]furan-5-yl)-[1,2,4]triazolo[1,5-a]pyrimidin-4(7H)-yl)-N-(4-(pentafluoro-λ⁶-sulfa neyl)phenyl)acetamide **187c** (140 mg) with a yield of 87.51%.

MS m/z (ESI): 616.6 [M+1]

### Step 3

### 2-(5-ethyl-6-(4-(3-hydroxypicolinoyl)piperazin-1-yl)-7-oxo-2-(3,3a,4,6a-tetrahydro-1H-cyclopen ta[c]furan-5-yl)-[1,2,4]triazolo[1,5-a]pyrimidin-4(7H)-yl)-N-(4-(pentafluoro-λ⁶-sulfaneyl)phenyl) acetamide

3-Hydroxypicolinic acid **22a** (44.06 mg, 316.76 µmol), 1-hydroxybenzotriazole (57.07 mg, 422.34 µmol), 1-(3-dimethylaminopropyl)-3-ethylcarbodiimide hydrochloride (80.96 mg, 422.34 µmol), and N,N-diisopropylethylamine (81.87 mg, 633.51 µmol) were dissolved in N,N-dimethylformamide (3 mL), and the mixture was stirred at 25 °C for 10 min. Then 2-(5-ethyl-7-oxo-6-(piperazin-1-yl)-2-(3,3a,4,6a-tetrahydro-1H-cyclopenta[c]furan-5-yl)-[1,2,4]tr iazolo[1,5-a]pyrimidin-4(7H)-yl)-N-(4-(pentafluoro-λ⁶-sulfaneyl)phenyl)acetamide **187c** (130 mg, 211.17 µmol) was added, and the mixture was stirred at 30 °C for 16 h. After the reaction was completed, the reaction mixture was purified by preparative liquid chromatography (Waters 3767/QDA column: SunFire Sunfire C18, 19×250 mm, 10 um; mobile phase A: 10 mmol/L A: 0.1% FA/H2O, B: acetonitrile; flow rate: 20 mL/min; gradient: 51-61% retention time: 8.3 min, 16 min) to obtain 2-(5-ethyl-6-(4-(3-hydroxypicolinoyl)piperazin-1-yl)-7-oxo-2-(3,3a,4,6a-tetrahydro-1H-cyclopenta[c]furan-5-yl)-[1,2,4]triazolo[1,5-a]pyrimidin-4(7H)-yl)-N-( 4-(pentafluoro-λ⁶-sulfaneyl)phenyl)acetamide **187** (53 mg) with a yield of 34.07%.

MS m/z (ESI): 737.1 [M+1]
¹H NMR (400 MHz, DMSO-d6) δ 10.97 (s, 1H), 10.43 (s, 1H), 8.05 (t, J = 4.0 Hz, 1H), 7.87 (d, J = 12.0 Hz, 2H), 7.76 (d, J = 12.0 Hz, 2H), 7.29 (m, 2H), 6.41 (s, 1H), 5.19 (s, 2H), 4.54 (d, J = 12.0 Hz, 1H), 3.74 (t, J = 8.0 Hz, 1H), 3.63-3.62 (m, 2H), 3.53 - 3.49 (m, 3H), 3.43-3.39 (m, 2H), 3.25-3.22 (m, 1H), 2.99-2.95 (m, 4H), 2.81-2.78 (m, 1H), 2.63 - 2.56 (m, 3H), 1.16 (t, J = 8.0 Hz, 3H).

### Example 188

### 2-(6-(4-(4-chloro-3-hydroxypicolinoyl)piperazin-1-yl)-5-ethyl-7-oxo-2-(3,3a,4,6a-tetrahydro-1H-cyclopenta[c]furan-5-yl)-[1,2,4]triazolo[1,5-a]pyrimidin-4(7H)-yl)-N-(2-chloro-4-(trifluoromethy l)phenyl)acetamide

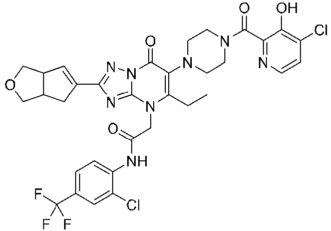

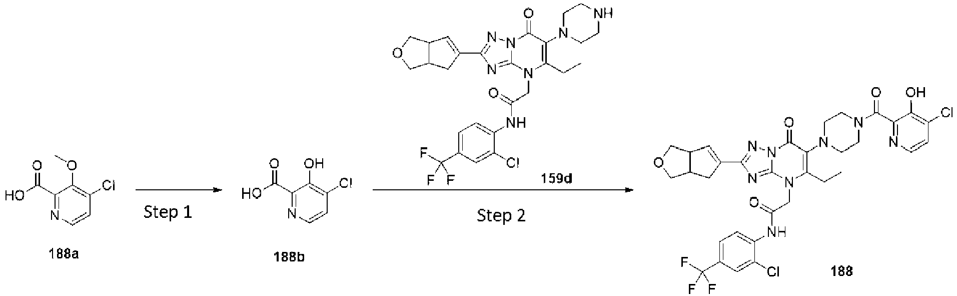

### Step 1

### 4-chloro-3-hydroxypicolinic acid

4-Chloro-3-methoxypicolinic acid **188a** (1.00 g, 5.33 mol) was dissolved in concentrated hydrochloric acid (3.5 mL), and then the reaction mixture was stirred at 100 °C for 12 h. After the reaction was completed, the reaction mixture was allowed to stand to cool to room temperature, and a large amount of solid precipitated. The mixture was filtered, and the filter residue was washed with water once and with acetone three times. The filter cake was dried to obtain 4-chloro-3-hydroxypicolinic acid **188b** (820 mg) with a yield of 88.6%.

¹H NMR (400 MHz, DMSO) δ 8.05 - 7.95 (m, 2H).

### Step 2

### 2-(6-(4-(4-chloro-3-hydroxypicolinoyl)piperazin-1-yl)-5-ethyl-7-oxo-2-(3,3a,4,6a-tetrahydro-1H-cyclopenta[c]furan-5-yl)-[1,2,4]triazolo[1,5-a]pyrimidin-4(7H)-yl)-N-(2-chloro-4-(trifluoromethy l)phenyl)acetamide

4-Chloro-3-hydroxypicolinic acid **188b** (131.17 mg, 760.12 umol), 1-hydroxybenzotriazole (136.95 mg, 1.01 mol), 1-(3-dimethylaminopropyl)-3-ethylcarbodiimide hydrochloride (194.3 mg, 1.01 mol), and N,N-diisopropylethylamine (196.5 mg, 1.52 mol) were dissolved in N,N-dimethylformamide (4 mL), and the mixture was stirred at 25 °C under nitrogen for 10 min. Then N-(2-chloro-4-(trifluoromethyl)phenyl)-2-(5-ethyl-7-oxo-6-(piperazin-1-yl)-2-(3,3a,4,6a-tetrahydro-1H-cyclopenta[c]furan-5-yl)-[1,2,4]triazolo[1,5-a]pyrimidin-4(7H)-yl)ace tamide **159d** (300 mg, 506.75 umol) was added, and stirring was continued at 25 °C for 16 h. After the reaction was completed, the reaction mixture was purified by preparative liquid chromatography (Waters 3767/QDA Column: SunFire Sunfire C18, 19×250 mm, 10 um; Mobile Phase A: 0.1% FA/H2O, B: ACN; flow rate: 20 mL/min; gradient: 59-69% Retention Time: 9 of 16 min) to obtain 2-(6-(4-(4-chloro-3-hydroxypicolinoyl)piperazin-1-yl)-5-ethyl-7-oxo-2-(3,3a,4,6a-tetrahydro-1H-cyclopenta[c]furan-5-yl)-[1,2,4]triazolo[1,5-a]pyrimidin-4(7H)-yl)-N-( 2-chloro-4-(trifluoromethyl)phenyl)acetamide **188** (17.15 mg) with a yield of 4.5%.

MS m/z (ESI): 749.0 [M+1]
¹H NMR (400 MHz, DMSO) δ 10.84 (s, 1H), 10.36 (s, 1H), 8.06 (d, J = 5.2 Hz, 2H), 7.97 (s, 1H), 7.72 (d, J = 8.4 Hz, 1H), 7.56 (d, J = 5.2 Hz, 1H), 6.45 (s, 1H), 5.32 (s, 2H), 4.59 - 4.49 (m, 1H), 3.78 - 3.73 (m, 1H), 3.66 - 3.62 (m, 2H), 3.56 - 3.50 (m, 5H), 3.06 - 2.93 (m, 6H), 2.85 - 2.77 (m, 1H), 2.64 - 2.59 (m, 2H), 1.21 - 1.16 (m, 3H).

### Example 189

### 2-(5-ethyl-6-(4-(5-hydroxy-6-methylpyrimidine-4-carbonyl)piperazin-1-yl)-7-oxo-2-(3,3a,4,6a-te trahydro-1H-cyclopenta[c]furan-5-yl)-[1,2,4]triazolo[1,5-a]pyrimidin-4(7H)-yl)-N-(4-(pentafluor o-λ⁶-sulfaneyl)phenyl)acetamide

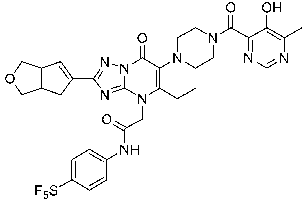

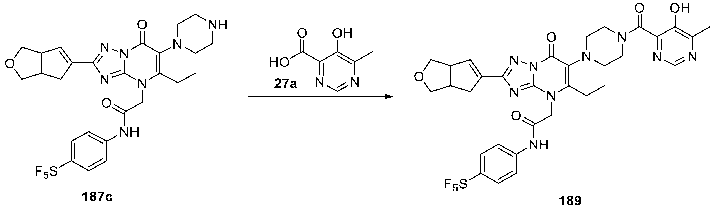

5-Hydroxy-6-methylpyrimidine-4-carboxylic acid **27a** (56.33 mg, 365.49 umol), 1-hydroxybenzotriazole (65.85 mg, 487.32 umol), 1-(3-dimethylaminopropyl)-3-ethylcarbodiimide hydrochloride (93.42 mg, 487.32 umol) and N,N-diisopropylethylamine (94.47 mg, 730.97 umol) were dissolved in N,N-dimethylformamide (5 mL), and the mixture was stirred at 25 °C for 10 minutes. Then 2-(5-ethyl-7-oxo-6-(piperazin-1-yl)-2-(3,3a,4,6a-tetrahydro-1H-cyclopenta[c]furan-5-yl)-[1,2,4]tr iazolo[1,5-a]pyrimidin-4(7H)-yl)-N-(4-(pentafluoro-λ⁶-sulfaneyl)phenyl)acetamide **187c** (150 mg, 243.66 umol) was added, and the mixture was stirred at 30 °C for 16 hours. After the reaction was complete, the reaction mixture was purified by preparative liquid chromatography (Waters 3767/QDA column: SunFire Sunfire C18, 19×250 mm, 10 um; mobile phase A: 10 mmol/L A: 0.1% FA/H2O, B: acetonitrile; flow rate: 20 mL/min; gradient: 51-61% retention time: 9 min, 16 min) to obtain 2-(5-ethyl-6-(4-(5-hydroxy-6-methylpyrimidine-4-carbonyl)piperazin-1-yl)-7-oxo-2-(3,3a,4,6a-tetrahydro-1H-cyclopenta[c]furan-5-yl)-[1,2,4]triazolo[1,5-a]pyrimidin-4(7H)-y l)-N-(4-(pentafluoro-λ⁶-sulfaneyl)phenyl)acetamide **189** (49.8 mg) in a yield of 27.19%.

MS m/z (ESI): 752.1 [M+1]
¹H NMR (400 MHz, DMSO-d6) δ 10.97 (s, 1H), 10.03 (m, 1H), 8.55 (s, 1H), 7.88 (d, J = 8.0 Hz, 2H), 7.77 (d, J = 8.0 Hz, 2H), 6.41 (s, 1H), 5.19 (s, 2H), 4.52 (d, J = 8.0 Hz, 1H), 3.74 (t, J = 8.0 Hz, 1H), 3.63 - 3.60 (m, 2H), 3.53-3.45 (m, 5H), 3.25 (m, 1H), 2.99-2.95 (m, 5H), 2.86 - 2.74 (m, 1H), 2.65-2.56 (m, 2H), 2.43 (s, 3H), 1.16 (t, J = 8.0 Hz, 3H).

### Example 190

### N-(2-chloro-4-(trifluoromethyl)phenyl)-2-(5-ethyl-6-(4-(5-hydroxy-2-methylpyrimidine-4-carbon yl)piperazin-1-yl)-7-oxo-2-(3,3a,4,6a-tetrahydro-1H-cyclopenta[c]furan-5-yl)-[1,2,4]triazolo[1,5-a]pyrimidin-4(7H)-yl)acetamide

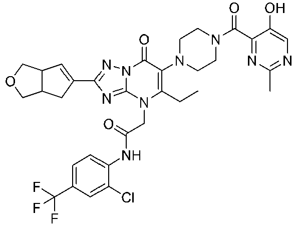

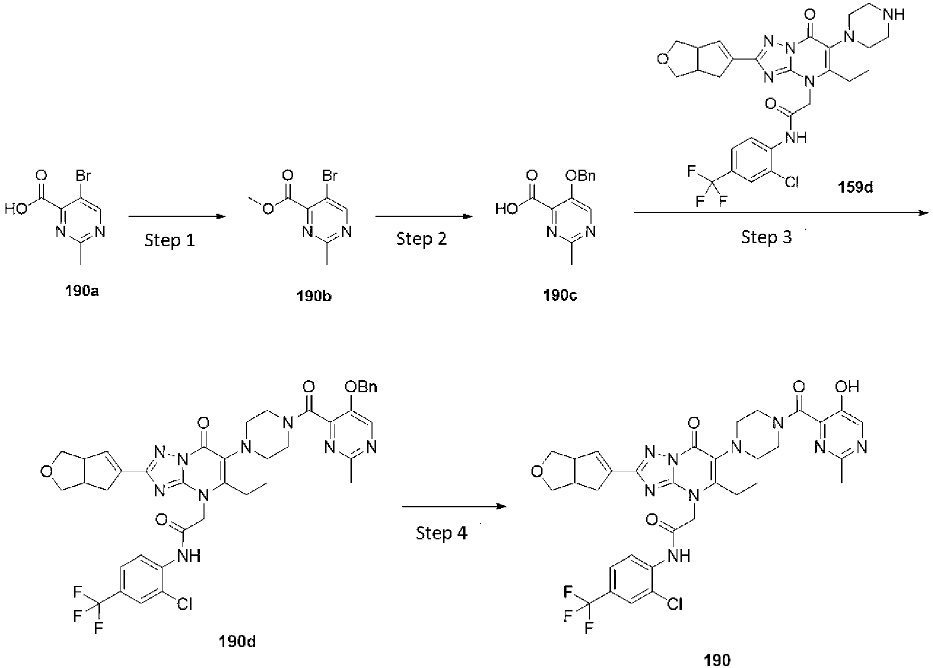

### Step 1

### methyl 5-bromo-2-methylpyrimidine-4-carboxylate

Oxalyl chloride (7.02 g, 55.29 mmol) was added to a solution of 5-bromo-2-methylpyrimidine-4-carboxylic acid **190a** (10.0 g, 46.08 mmol) in dichloromethane (100 mL), cooled to 0 °C, and N,N-dimethylformamide (336.81 mg, 4.61 mmol, 1 mL) was added dropwise. The mixture was stirred at room temperature for 1.5 hours, concentrated under reduced pressure, and methanol (60 mL) was added to the residue. The reaction mixture was stirred at room temperature under nitrogen for 30 minutes. After the reaction was complete, the reaction mixture was concentrated directly under reduced pressure, then 5% sodium bicarbonate solution (50 mL) was added to the residue, and the mixture was extracted with ethyl acetate (40 mL × 3). The combined organic phases were dried over anhydrous sodium sulfate, filtered, and concentrated under reduced pressure to obtain methyl 5-bromo-2-methylpyrimidine-4-carboxylate **190b** (10.1 g) in a yield of 93.93%.

¹H NMR (400 MHz, CDCl3) δ 8.89 (s, 1H), 4.03 (s, 3H), 2.76 (s, 3H).

### Step 2

### 5-(benzyloxy)-2-methylpyrimidine-4-carboxylic acid

Under nitrogen at 0 °C, sodium hydride (6.92 g, 173.13 mmol, 60% purity) was added to a solution of benzyl alcohol (18.7 g, 173.13 mmol) in tetrahydrofuran (100 mL), and the mixture was stirred at 0 °C for 0.5 hours. Then methyl 5-bromo-2-methylpyrimidine-4-carboxylate **190b** (10.0 g, 43.28 mmol) was added to the mixture. The reaction mixture was slowly heated to 25 °C and stirred for 12 hours. After the reaction was complete, saturated ammonium chloride (50 mL) was added to the reaction mixture to quench, and the mixture was extracted with ethyl acetate (50 mL × 3). The combined organic phases were washed with saturated sodium chloride solution (100 mL), dried over anhydrous sodium sulfate, filtered, and concentrated under reduced pressure. The residue was purified by reverse-phase column chromatography (C18) (eluent: D system) to obtain 5-(benzyloxy)-2-methylpyrimidine-4-carboxylic acid **190c** (1.0 g) in a yield of 9.5%.

MS m/z (ESI): 245.2 [M+1]

### Step 3

### 2-(6-(4-(5-(benzyloxy)-2-methylpyrimidine-4-carbonyl)piperazin-1-yl)-5-ethyl-7-oxo-2-(3,3a,4,6 a-tetrahydro-1H-cyclopenta[c]furan-5-yl)-[1,2,4]triazolo[1,5-a]pyrimidin-4(7H)-yl)-N-(2-chloro-4-(trifluoromethyl)phenyl)acetamide

5-(Benzyloxy)-2-methylpyrimidine-4-carboxylic acid **190c** (205.45 mg, 844.58 umol), 1-hydroxybenzotriazole (114.12 mg, 844.58 umol), 1-(3-dimethylaminopropyl)-3-ethylcarbodiimide hydrochloride (161.91 mg, 844.58 umol) and N,N-diisopropylethylamine (163.43 mg, 1.27 mmol) were dissolved in N,N-dimethylformamide (10 mL), and the mixture was stirred at 25 °C for 10 minutes. Then N-(2-chloro-4-(trifluoromethyl)phenyl)-2-(5-ethyl-7-oxo-6-(piperazin-1-yl)-2-(3,3a,4,6a-tetrahyd ro-1H-cyclopenta[c]furan-5-yl)-[1,2,4]triazolo[1,5-a]pyrimidin-4(7H)-yl)acetamide **159d** (250 mg, 422.29 umol) was added, and the mixture was stirred at 30 °C for 16 hours. After the reaction was complete, the reaction mixture was purified by preparative liquid chromatography (Waters 3767/QDA column: SunFire Sunfire C18, 19×250 mm, 10 um; mobile phase A: 10 mmol/L A: 0.1% TFA/H2O, B: acetonitrile; flow rate: 20 mL/min; gradient: 51-61% retention time: 8.3 min, 16 min) to obtain 2-(6-(4-(5-(benzyloxy)-2-methylpyrimidine-4-carbonyl)piperazin-1-yl)-5-ethyl-7-oxo-2-(3,3a,4,6a-tetrahydro-1H-cyclopenta[c]furan-5-yl)-[1,2,4]triazolo[1,5-a]pyri midin-4(7H)-yl)-N-(2-chloro-4-(trifluoromethyl)phenyl)acetamide **190d** (345 mg) in a yield of 98%.

MS m/z (ESI): 818.5 [M+1]

### Step 4

### N-(2-chloro-4-(trifluoromethyl)phenyl)-2-(5-ethyl-6-(4-(5-hydroxy-2-methylpyrimidine-4-carbon yl)piperazin-1-yl)-7-oxo-2-(3,3a,4,6a-tetrahydro-1H-cyclopenta[c]furan-5-yl)-[1,2,4]triazolo[1,5-a]pyrimidin-4(7H)-yl)acetamide

2-(6-(4-(5-(Benzyloxy)-2-methylpyrimidine-4-carbonyl)piperazin-1-yl)-5-ethyl-7-oxo-2 -(3,3a,4,6a-tetrahydro-1H-cyclopenta[c]furan-5-yl)-[1,2,4]triazolo[1,5-a]pyrimidin-4(7H)-yl)-N-( 2-chloro-4-(trifluoromethyl)phenyl)acetamide **190d** (345 mg, 635.51 µmol) was dissolved in trifluoroacetic acid (10 g, 87.70 mmol, 10 mL), and then the reaction mixture was stirred at 45 °C for 12 hours. After the reaction was completed, ethyl acetate was added to the reaction mixture to dilute, the pH was adjusted to 8-9 with saturated sodium bicarbonate solution, the mixture was extracted with ethyl acetate (30 mL×3), the combined organic phases were washed with saturated sodium chloride solution (50 mL), dried over anhydrous sodium sulfate, filtered, and concentrated under reduced pressure, and the resulting residue was purified by preparative high performance liquid chromatography (Waters 3767/Qda column: SunFire SunFire C18, 19×250 mm, 10 µm; mobile phase A: 0.1% FA/H2O, B: ACN; flow rate: 20 mL/min; retention time: 9 of 16 min) to obtain N-(2-chloro-4-(trifluoromethyl)phenyl)-2-(5-ethyl-6-(4-(5-hydroxy-2-methylpyrimidine-4-carbonyl)piperazin-1-yl)-7-oxo-2-(3,3a,4,6a-tetrahydro-1H-cyclopentene[c ]furan-5-yl)-[1,2,4]triazolo[1,5-a]pyrimidin-4(7H)-yl)acetamide **190** (22.07 mg) in a yield of 4.96%.

MS m/z (ESI): 728.1 [M+1]
¹H NMR (400 MHz, DMSO) δ 10.55 (s, 1H), 10.36 (s, 1H), 8.33 (s, 1H), 8.06 (d, J = 8.8 Hz, 1H), 7.97 (s, 1H), 7.71 (d, J = 8.4 Hz, 1H), 6.45 (s, 1H), 5.31 (s, 2H), 4.53-4.44 (m, 1H), 3.78-3.73 (m, 1H), 3.66-3.62 (m, 2H), 3.56-3.51 (m, 3H), 3.30-3.26 (m, 2H), 3.03-2.94 (m, 4H), 2.83-2.78 (m, 1H), 2.65-2.65 (m, 4H), 2.52 (s, 3H), 1.18 (t, J = 7.4 Hz, 3H).

### Example 191

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(3,3,3-trifluoroprop-1-en-2-yl)-[1,2,4]triazolo[1,5-a]pyrimidin-4(7H)-yl)acetamide

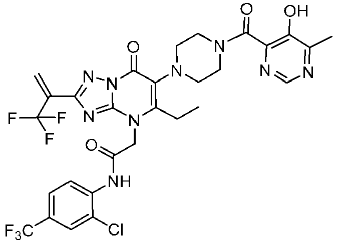

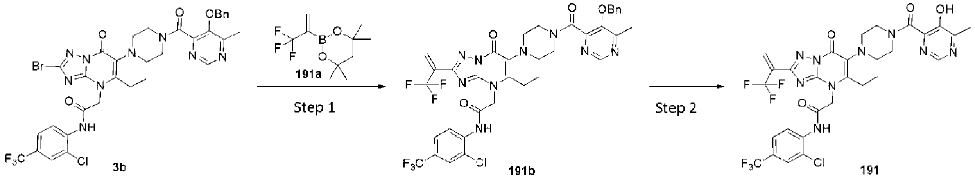

### Step 1

### 2-(6-(4-(5-(benzyloxy)-6-methylpyrimidine-4-carbonyl)piperazin-1-yl)-5-ethyl-7-oxo-2-(3,3,3-tri fluoroprop-1-en-2-yl)-[1,2,4]triazolo[1,5-a]pyrimidin-4(7H)-yl)-N-(2-chloro-4-(trifluoromethyl)p henyl)acetamide

At room temperature, to 1,4-dioxane (3 mL) of 2-(6-(4-(5-(benzyloxy)-6-methylpyrimidine-4-carbonyl)piperazin-1-yl)-2-bromo-5-ethyl-7-oxo-[ 1,2,4]triazolo[1,5-a]pyrimidin-4(7H)-yl)-N-(2-chloro-4-(trifluoromethyl)phenyl)acetamide **3b** (100 mg, 126.74 µmol) were added [1,1'-bis(diphenylphosphino)ferrocene]palladium(II) dichloride (10.27 mg, 12.67 µmol), sodium carbonate (26.87 mg, 253.49 µmol), 4,4,6,6-tetramethyl-2-(3,3,3-trifluoroprop-1-en-2-yl)-1,3,2-dioxaborolane **191a** (39.39 mg, 177.44 µmol, commercially available), and water (1 mL), and after replacing with argon, the temperature was raised to 80 °C and the reaction was performed for 4 hours. After the reaction was complete, water (50 mL) was added to dilute, the mixture was extracted with dichloromethane (30 mL ×3), the combined organic phases were washed with saturated sodium chloride solution (20 mL), dried over anhydrous sodium sulfate, filtered, and concentrated under reduced pressure, and purified by silica gel column chromatography (eluent: System B) to obtain 2-(6-(4-(5-(benzyloxy)-6-methylpyrimidine-4-carbonyl)piperazin-1-yl)-5-ethyl-7-oxo-2-(3,3,3-tri fluoroprop-1-en-2-yl)-[1,2,4]triazolo[1,5-a]pyrimidin-4(7H)-yl)-N-(2-chloro-4-(trifluoromethyl)p henyl)acetamide **191b** (30 mg) in a yield of 29.44%.

MS m/z (ESI): 804.2 [M+H]

### Step 2

### N-(2-chloro-4-(trifluoromethyl)phenyl)-2-(5-ethyl-6-(4-(5-hydroxy-6-methylpyrimidine-4-carbon yl)piperazin-1-yl)-7-oxo-2-(3,3,3-trifluoroprop-1-en-2-yl)-[1,2,4]triazolo[1,5-a]pyrimidin-4(7H)-yl)acetamide

2-(6-(4-(5-(Benzyloxy)-6-methylpyrimidine-4-carbonyl)piperazin-1-yl)-5-ethyl-7-oxo-2 -(3,3,3-trifluoroprop-1-en-2-yl)-[1,2,4]triazolo[1,5-a]pyrimidin-4(7H)-yl)-N-(2-chloro-4-(trifluor omethyl)phenyl)acetamide **191b** (30 mg, 37.31 µmol) was dissolved in trifluoroacetic acid (3 mL), the temperature was raised to 45 °C and the reaction was performed for 4 hours. After the reaction was completed, the mixture was concentrated under reduced pressure, and the residue was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(5-ethyl-6-(4-(5-hydroxy-6-methylpyrimidine-4-carbonyl)piperazin-1-yl)-7-oxo-2-(3,3,3-trifluoroprop-1-en-2-yl)-[1,2,4]triaz olo[1,5-a]pyrimidin-4(7H)-yl)acetamide **191** (8 mg) in a yield of 28.53%.

MS m/z (ESI): 714.2 [M+H]
¹H NMR (400 MHz, DMSO-d6) δ 10.38 (s, 1H), 10.23 (s, 1H), 8.58 (s, 1H), 8.03 (d, J = 8.5 Hz, 1H), 7.96 (d, J = 2.2 Hz, 1H), 7.72 (dd, J = 8.7, 2.2 Hz, 1H), 6.73 (d, J = 1.8 Hz, 1H), 6.44 (s, 1H), 5.34 (s, 2H), 4.53 (d, J = 12.5 Hz, 1H), 3.54-3.43 (m, 2H), 3.28 (d, J = 12.2 Hz, 1H), 3.01 (dt, J = 12.8, 6.4 Hz, 2H), 2.87-2.79 (m, 1H), 2.65 (d, J = 11.1 Hz, 1H), 2.45 (s, 3H), 1.20 (t, J = 7.4 Hz, 3H).

### Example 192

### N-(2-chloro-4-(trifluoromethyl)phenyl)-2-(2-(1-cyclopropyl-1H-indo1-5-yl)-5-ethyl-6-(4-(5-hydr oxy-6-methylpyrimidine-4-carbonyl)piperazin-1-yl)-7-oxo-[1,2,4]triazolo[1,5-a]pyrimidin-4(7H) -yl)acetamide

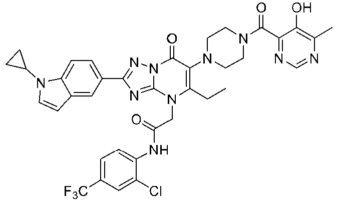

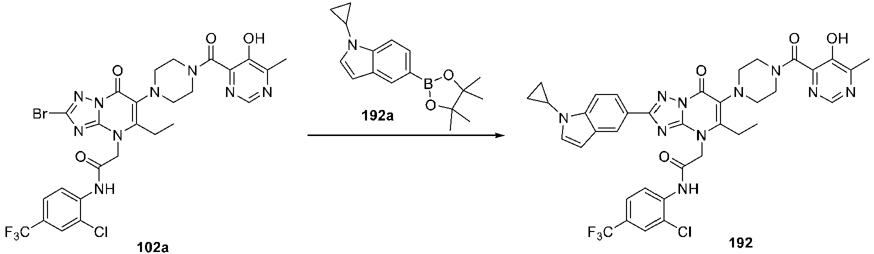

2-(2-Bromo-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluoromethyl)phenyl)acetamide 102a (100 mg, 143.09 µmol), 1-cyclopropyl-5-(4,4,5,5-tetramethyl-1,3,2-dioxaborolan-2-yl)-1H-indole **192a** (52.67 mg, 186.01 µmol, prepared according to the published literature "Journal of Medicinal Chemistry (2023), 66(1), 1063-1081"), [1,1'-bis(diphenylphosphino)ferrocene]palladium(II) dichloride dichloromethane complex (11.60 mg, 14.31 µmol), and sodium carbonate (45.50 mg, 429.26 µmol) were added to 1,4-dioxane (4 mL) and water (0.5 mL). The mixture was subject to argon replacement three times, heated to 85 °C, and reacted for 2 hours. After the reaction was complete, the mixture was concentrated under reduced pressure, and the resulting residue was purified by silica gel column chromatography (eluent: System B) and then separated and purified by preparative liquid chromatography (separation column: AKZONOBEL Kromasil; 250×21.2 mm I.D.; 5 µm, 20 mL/min; mobile phase A: 0.05% TFA+H₂O, mobile phase B: CH₃CN) to obtain N-(2-chloro-4-(trifluoromethyl)phenyl)-2-(2-(1-cyclopropyl-1H-indol-5-yl)-5-ethyl-6-(4-(5-hydr oxy-6-methylpyrimidine-4-carbonyl)piperazin-1-yl)-7-oxo-[1,2,4]triazolo[1,5-a]pyrimidin-4(7H) -yl)acetamide **192** (33.43 mg) with a yield of 29.84%.

MS m/z (ESI): 775.2 [M+1]
¹H NMR (400 MHz, DMSO-d6) δ 10.42 (s, 1H), 10.25 (s, 1H), 8.59 (s, 1H), 8.35 (d, J = 1.5 Hz, 1H), 8.08 (d, J = 8.6 Hz, 1H), 7.97 (td, J = 4.0, 3.6, 1.6 Hz, 2H), 7.71 (dd, J = 8.8, 2.1 Hz, 1H), 7.66 (d, J = 8.6 Hz, 1H), 7.40 (d, J = 3.2 Hz, 1H), 6.52 (d, J = 3.2 Hz, 1H), 5.42 (s, 2H), 4.55 (d, J = 12.5 Hz, 1H), 3.62-3.46 (m, 4H), 3.28 (t, J = 12.6 Hz, 1H), 3.03 (q, J = 9.4, 8.2 Hz, 3H), 2.85 (d, J = 11.2 Hz, 1H), 2.68 (d, J = 10.8 Hz, 1H), 2.46 (s, 3H), 1.23 (t, J = 7.4 Hz, 3H), 1.15-1.05 (m, 2H), 0.97 (dd, J = 4.8, 2.5 Hz, 2H).

### Example 193

### (E)-N-(2-chloro-4-(trifluoromethyl)phenyl)-2-(2-(2-ethoxyvinyl)-5-ethyl-6-(4-(5-hydroxy-6-meth ylpyrimidine-4-carbonyl)piperazin-1-yl)-7-oxo-[1,2,4]triazolo[1,5-a]pyrimidin-4(7H)-yl)acetami de

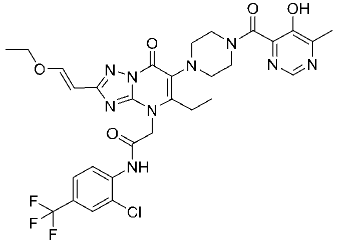

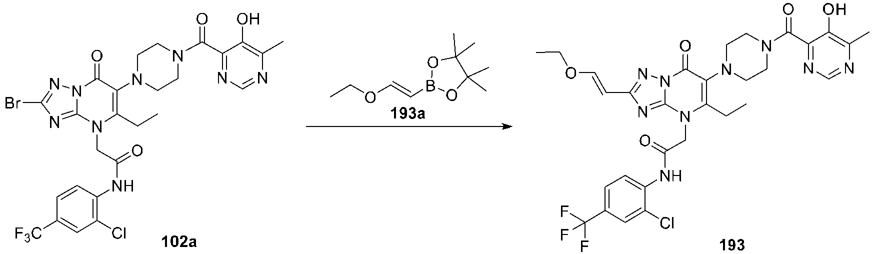

At room temperature, 2-(2-bromo-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluor omethyl)phenyl)acetamide **102a** (40 mg, 57.23 µmol), (E)-2-(2-ethoxyvinyl)-4,4,5,5-tetramethyl-1,3,2-dioxaborolane **193a** (10.20 mg, 51.51 µmol), [1,1'-bis(diphenylphosphino)ferrocene]palladium(II) dichloride dichloromethane complex (4.64 mg, 5.72 µmol), and sodium carbonate (18.20 mg, 171.70 µmol) were dissolved in 1,4-dioxane (10 mL) and water (3 mL). The mixture was subject to argon replacement four times, heated to 80 °C, and reacted for 1.5 hours. Water (30 mL) was added to the reaction mixture, and the reaction mixture was extracted with ethyl acetate (40 mL ×3). The organic phase was washed with saturated sodium chloride solution (20 mL), dried over anhydrous sodium sulfate, filtered, and concentrated under reduced pressure. The residue was purified by preparative liquid chromatography separation (separation column: AKZONOBEL Kromasil; 250×21.2 mm I.D.; 5 µm, 20 mL/min; mobile phase A: 0.05% TFA+H₂O, mobile phase B: CH₃CN) to obtain (E)-N-(2-chloro-4-(trifluoromethyl)phenyl)-2-(2-(2-ethoxyvinyl)-5-ethyl-6-(4-(5-hydroxy-6-meth ylpyrimidine-4-carbonyl)piperazin-1-yl)-7-oxo-[1,2,4]triazolo[1,5-a]pyrimidin-4(7H)-yl)acetami de **193** (3.17 mg) with a yield of 7.79%.

MS m/z (ESI): 690.2 [M+1]
¹H NMR (400 MHz, DMSO) δ 10.35 (s, 1H), 10.22 (s, 1H), 8.58 (s, 1H), 8.06 (d, J = 8.6 Hz, 1H), 7.97 (d, J = 2.1 Hz, 1H), 7.72 (dd, J = 8.7, 2.1 Hz, 1H), 7.54 (d, J = 12.7 Hz, 1H), 5.79 (d, J = 12.7 Hz, 1H), 5.29 (s, 2H), 4.52 (d, J = 12.6 Hz, 1H), 3.97 (q, J = 7.0 Hz, 2H), 3.24 (t, J = 12.8 Hz, 1H), 3.02-2.91 (m, 3H), 2.80 (d, J = 11.4 Hz, 1H), 2.63 (d, J = 11.0 Hz, 1H), 2.44 (s, 3H), 1.26 (t, J = 7.0 Hz, 3H), 1.18 (t, J = 7.4 Hz, 3H).

### Example 194

### (E)-2-(2-(3-(azetidin-1-yl)-3-oxoprop-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluor omethyl)phenyl)acetamide

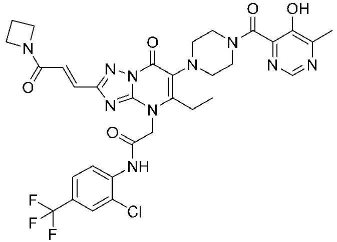

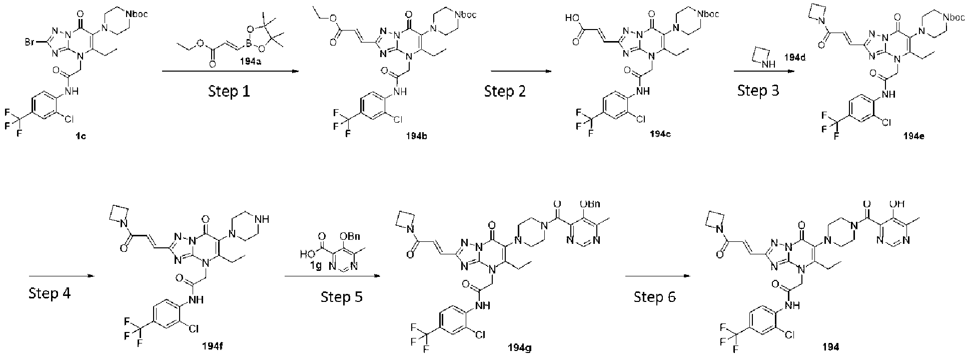

### Step 1

### tert-butyl (E)-4-(4-(2-((2-chloro-4-(trifluoromethyl)phenyl)amino)-2-oxoethyl)-2-(3-ethoxy-3-oxoprop-1-e n-1-yl)-5-ethyl-7-oxo-4,7-dihydro-[1,2,4]triazolo[1,5-a]pyrimidin-6-yl)piperazine-1-carboxylate

At room temperature, tert-butyl 4-(2-bromo-4-(2-((2-chloro-4-(trifluoromethyl)phenyl) amino)-2-oxoethyl)-5-ethyl-7-oxo-4,7-dihydro-[1,2,4]triazolo[1,5-a]pyrimidin-6-yl)piperazine-1-carboxylate 1c (450 mg, 678.85 µmol), (E)-3-(4,4,5,5-tetramethyl-1,3,2-dioxaborolan-2-yl)acrylate **194a** (184.17 mg, 814.62 µmol, commercially available), [1,1'-bis(diphenylphosphino)ferrocene]palladium(II) dichloride dichloromethane complex (55.03 mg, 67.89 µmol), and sodium carbonate (215.85 mg, 2.04 mmol) were dissolved in 1,4-dioxane (10 mL) and water (3 mL). The mixture was subject to argon replacement four times, heated to 80 °C, and reacted for 1.5 hours. Water (30 mL) was added to the reaction mixture, and the reaction mixture was extracted with ethyl acetate (40 mL ×3). The organic phase was washed with saturated sodium chloride solution (20 mL), dried over anhydrous sodium sulfate, filtered, and concentrated under reduced pressure. The resulting residue was purified by silica gel column chromatography (eluent: System B) to obtain tert-butyl (E)-4-(4-(2-((2-chloro-4-(trifluoromethyl) phenyl)amino)-2-oxoethyl)-2-(3-ethoxy-3-oxoprop-1-en-1-yl)-5-ethyl-7-oxo-4,7-dihydro-[1,2,4]t riazolo[1,5-a]pyrimidin-6-yl)piperazine-1-carboxylate **194b** (430 mg) with a yield of 92.87%.

MS m/z (ESI): 682.2 [M+1]

### Step 2

### (E)-3-(6-(4-(tert-butoxycarbonyl)piperazin-1-yl)-4-(2-((2-chloro-4-(trifluoromethyl)phenyl)amin o)-2-oxoethyl)-5-ethyl-7-oxo-4,7-dihydro-[1,2,4]triazolo[1,5-a]pyrimidin-2-yl)acrylic acid

Tert-butyl (E)-4-(4-(2-((2-chloro-4-(trifluoromethyl)phenyl)amino)-2-oxoethyl)-2-(3-ethoxy-3-oxoprop-1-en-1-yl)-5-ethyl-7-oxo-4,7-dihydro-[1,2,4]triazolo[1,5-a]pyrimidin-6-yl)pipe razine-1-carboxylate **194b** (480 mg, 703.72 µmol) was added to methanol (2.5 mL), tetrahydrofuran (2.5 mL), and water (1.25 mL), and lithium hydroxide monohydrate (59.06 mg, 1.41 mmol) was added, followed by reaction at room temperature for 30 minutes. 1 N hydrochloric acid was added to adjust to weak acidity, and the reaction mixture was extracted with ethyl acetate (30 mL × 3). The organic phase was washed with saturated sodium chloride aqueous solution (30 mL), dried over anhydrous sodium sulfate, filtered, and concentrated under reduced pressure. The resulting residue was purified by silica gel column chromatography (eluent: System B) to obtain (E)-3-(6-(4-(tert-butoxycarbonyl)piperazin-1-yl)-4-(2-((2-chloro-4-(trifluoromethyl)phenyl)amino)-2-oxoethyl)-5-ethyl-7-oxo-4,7-dihydro-[1,2,4]triazolo[1,5-a]py rimidin-2-yl)acrylic acid **194c** (80 mg) in a yield of 17.38%.

MS m/z: 598.1 [M-56+1]

### Step 3

### tert-butyl (E)-4-(2-(3-(azetidin-1-yl)-3-oxoprop-1-en-1-yl)-4-(2-((2-chloro-4-(trifluoromethyl) phenyl)amino)-2-oxoethyl)-5-ethyl-7-oxo-4,7-dihydro-[1,2,4]triazolo[1,5-a]pyrimidin-6-yl)piper azine-1-carboxylate

(E)-3-(6-(4-(tert-butoxycarbonyl)piperazin-1-yl)-4-(2-((2-chloro-4-(trifluoromethyl)phe nyl)amino)-2-oxoethyl)-5-ethyl-7-oxo-4,7-dihydro-[1,2,4]triazolo[1,5-a]pyrimidin-2-yl)acrylic acid **194c** (63 mg, 96.32 µmol) and azetidine **194d** (8.25 mg, 144.49 µmol) were added to N,N-dimethylformamide (1 mL), followed by addition of 1-butylphosphoric anhydride (138.81 mg, 385.30 µmol) and N,N-diisopropylethylamine (37.35 mg, 288.97 µmol), followed by reaction at room temperature for 18 hours. Water (30 mL) was added to the reaction mixture, which was extracted with ethyl acetate (30 mL × 2). The combined organic phase was washed with saturated sodium chloride aqueous solution (30 mL), dried over anhydrous sodium sulfate, filtered, and concentrated under reduced pressure. The resulting residue was purified by silica gel column chromatography (eluent: System A) to obtain tert-butyl (E)-4-(2-(3-(azetidin-1-yl)-3-oxoprop-1-en-1-yl)-4-(2-((2-chloro-4-(trifluoromethyl)phenyl)amin o)-2-oxoethyl)-5-ethyl-7-oxo-4,7-dihydro-[1,2,4]triazolo[1,5-a]pyrimidin-6-yl)piperazine-1-carb oxylate **194e** (60 mg) in a yield of 89.87%.

MS m/z (ESI): 637.2 [M-56+1]

### Step 4

### (E)-2-(2-(3-(azetidin-1-yl)-3-oxoprop-1-en-1-yl)-5-ethyl-7-oxo-6-(piperazin-1-yl)-[1,2,4]triazolo[ 1,5-a]pyrimidin-4(7H)-yl)-N-(2-chloro-4-(trifluoromethyl)phenyl)acetamide

Tert-butyl (E)-4-(2-(3-(azetidin-1-yl)-3-oxoprop-1-en-1-yl)-4-(2-((2-chloro-4-(trifluoromethyl)phenyl)amino)-2-oxoethyl)-5-ethyl-7-oxo-4,7-dihydro-[1,2,4]triazolo[1,5-a]pyri midin-6-yl)piperazine-1-carboxylate **194e** (60 mg, 86.57 µmol) was added to dichloromethane (2.5 mL), and trifluoroacetic acid (0.8 mL) was added, followed by reaction at room temperature for 1 hour. After the reaction was complete, the mixture was concentrated under reduced pressure to obtain (E)-2-(2-(3-(azetidin-1-yl)-3-oxoprop-1-en-1-yl)-5-ethyl-7-oxo-6-(piperazin-1-yl)-[1,2,4]triazolo[1,5-a]pyrimidin-4(7H)-yl)-N-(2-chloro-4-(trifluoromethyl)phenyl)acetamide **194f** (50 mg) in a yield of 97.40%.

MS m/z: 593.2 [M+1]

### Step 5

### (E)-2-(2-(3-(azetidin-1-yl)-3-oxoprop-1-en-1-yl)-6-(4-(5-(benzyloxy)-6-methylpyrimidine-4-carb onyl)piperazin-1-yl)-5-ethyl-7-oxo-[1,2,4]triazolo[1,5-a]pyrimidin-4(7H)-yl)-N-(2-chloro-4-(trifl uoromethyl)phenyl)acetamide

(E)-2-(2-(3-(azetidin-1-yl)-3-oxoprop-1-en-1-yl)-5-ethyl-7-oxo-6-(piperazin-1-yl)-[1,2, 4]triazolo[1,5-a]pyrimidin-4(7H)-yl)-N-(2-chloro-4-(trifluoromethyl)phenyl)acetamide **194f** (50 mg, 84.32 µmol), 5-(benzyloxy)-6-methylpyrimidine-4-carboxylic acid **1g** (26.77 mg, 109.61 µmol), 1-(3-dimethylaminopropyl)-3-ethylcarbodiimide hydrochloride (24.25 mg, 126.48 µmol), 1-hydroxybenzotriazole (17.09 mg, 126.48 µmol), and N,N-diisopropylethylamine (54.49 mg, 421.59 µmol) were added to N,N-dimethylformamide (3 mL), followed by reaction at room temperature for 4 hours. After the reaction was complete, water (30 mL) was added to the reaction mixture, which was extracted with ethyl acetate (30 mL × 2). The combined organic phase was washed with saturated sodium chloride aqueous solution (30 mL), dried over anhydrous sodium sulfate, filtered, and concentrated under reduced pressure. The resulting residue was purified by silica gel column chromatography (eluent: System B) to obtain (E)-2-(2-(3-(azetidin-1-yl)-3-oxoprop-1-en-1-yl)-6-(4-(5-(benzyloxy)-6-methylpyrimidine-4-carb onyl)piperazin-1-yl)-5-ethyl-7-oxo-[1,2,4]triazolo[1,5-a]pyrimidin-4(7H)-yl)-N-(2-chloro-4-(trifl uoromethyl)phenyl)acetamide **194g** (50 mg) in a yield of 72.38%.

MS m/z: 819.3 [M+1]

### Step 6

### (E)-2-(2-(3-(azetidin-1-yl)-3-oxoprop-1-en-1-yl)-5-ethyl-6-(4-(5-hydroxy-6-methylpyrimidine-4-carbonyl)piperazin-1-yl)-7-oxo-[1,2,4]triazolo[1,5-a]pyrimidin-4(7H)-yl)-N-(2-chloro-4-(trifluor omethyl)phenyl)acetamide

(E)-2-(2-(3-(azetidin-1-yl)-3-oxoprop-1-en-1-yl)-6-(4-(5-(benzyloxy)-6-methylpyrimidi ne-4-carbonyl)piperazin-1-yl)-5-ethyl-7-oxo-[1,2,4]triazolo[1,5-a]pyrimidin-4(7H)-yl)-N-(2-chlo ro-4-(trifluoromethyl)phenyl)acetamide **194g** (50 mg, 61.03 µmol) was added to dichloromethane (1 mL), followed by addition of trifluoroacetic acid (3 mL), and the reaction was stirred at room temperature for 1 hour. After the reaction was complete, the mixture was concentrated under reduced pressure, and the residue was purified by preparative liquid chromatography (separation column AKZONOBEL Kromasil; 250 ×21.2 mm I.D.; 5 µm, 20 mL/min; mobile phase A: 0.05% TFA+H₂O, mobile phase B: CH₃CN) to obtain (E)-2-(2-(3-(azetidin-1-yl)-3-oxoprop-1-en-1-yl)-5-ethyl-6-(4-(5-hydroxy-6-methylpyrimidine-4-carbonyl)piperazin-1-yl)-7-oxo-[1,2,4]triaz olo[1,5-a]pyrimidin-4(7H)-yl)-N-(2-chloro-4-(trifluoromethyl)phenyl)acetamide **194** (19.93 mg) with a yield of 44.34%.

MS m/z: 729.2 [M+1]
¹H NMR (400 MHz, DMSO) δ 10.38 (s, 1H), 10.22 (s, 1H), 8.58 (s, 1H), 8.05 (d, J = 8.6 Hz, 1H), 7.97 (d, J = 2.1 Hz, 1H), 7.72 (dd, J = 8.7, 2.1 Hz, 1H), 7.25 (d, J = 15.5 Hz, 1H), 6.95 (d, J = 15.5 Hz, 1H), 5.34 (s, 2H), 4.53 (d, J = 12.6 Hz, 1H), 4.28 (t, J = 7.7 Hz, 2H), 3.96 (t, J = 7.7 Hz, 2H), 3.49 (s, 3H), 3.26 (s, 2H), 3.00 (d, J = 7.8 Hz, 3H), 2.82 (d, J = 11.3 Hz, 1H), 2.63 (s, 1H), 2.44 (s, 3H), 2.24 (p, J = 7.7 Hz, 3H), 1.19 (t, J = 7.5 Hz, 3H).

### Example 195

### 2-(2-((1R,5R)-bicyclo[3.1.0]hex-2-en-3-yl)-5-ethyl-6-(4-(5-hydroxy-6-methylpyrimidine-4-carbo nyl)piperazin-1-yl)-7-oxo-[1,2,4]triazolo[1,5-a]pyrimidin-4(7H)-yl)-N-(4-(pentafluoro-λ6-sulfane yl)phenyl)acetamide

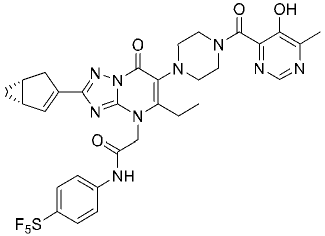

### Example 196

### 2-(2-((1S,5S)-bicyclo[3.1.0]hex-2-en-3-yl)-5-ethyl-6-(4-(5-hydroxy-6-methylpyrimidine-4-carbo nyl)piperazin-1-yl)-7-oxo-[1,2,4]triazolo[1,5-a]pyrimidin-4(7H)-yl)-N-(4-(pentafluoro-λ6-sulfane yl)phenyl)acetamide

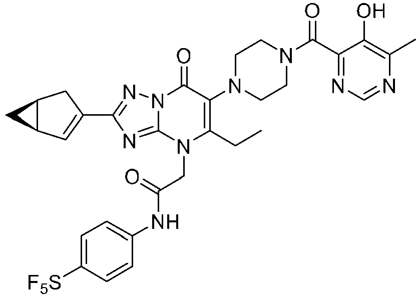

2-(2-(Bicyclo[3.1.0]hex-2-en-3-yl)-5-ethyl-6-(4-(5-hydroxy-6-methylpyrimidine-4-carb onyl)piperazin-1-yl)-7-oxo-[1,2,4]triazolo[1,5-a]pyrimidin-4(7H)-yl)-N-(4-(pentafluoro-λ⁶-thiol) phenyl)acetamide **66** (200 mg, 246.36 µmol) was separated by chiral preparative liquid chromatography (separation column Chiralpak IK, 2.1 cm I.D. × 25 cm Length, 5 µm, 30 mL/min; mobile phase CO2/MeOH/DEA (diethylamine) = 55/45/0.05 (v/v/v); detection wavelength: 214 nm; column temperature: 40 °C) to obtain 2-(2-((1R,5R)-bicyclo[3.1.0]hex-2-en-3-yl)-5-ethyl-6-(4-(5-hydroxy-6-methylpyrimidine-4-carbonyl)piperazin-1-yl)-7-oxo-[1,2,4]triazolo[1,5-a]pyrimi din-4(7H)-yl)-N-(4-(pentafluoro-A6-sulfaneyl)phenyl)acetamide **195** (77 mg) with a yield of 38.98%.

¹H NMR (400 MHz, DMSO) δ 10.92 (s, 1H), 10.21 (s, 1H), 8.58 (s, 1H), 7.88 (d, J = 9.2 Hz, 2H), 7.76 (d, J = 8.9 Hz, 2H), 6.77 (d, J = 2.2 Hz, 1H), 5.17 (s, 2H), 4.52 (d, J = 12.6 Hz, 1H), 3.50 (d, J = 10.4 Hz, 3H), 2.93 (d, J = 24.0 Hz, 4H), 2.85-2.61 (m, 4H), 2.44 (s, 3H), 1.96 (s, 1H), 1.78 (s, 1H), 1.16 (t, J = 7.4 Hz, 3H), 1.03-0.96 (m, 1H).

2-(2-((1S,5S)-bicyclo[3.1.0]hex-2-en-3-yl)-5-ethyl-6-(4-(5-hydroxy-6-methylpyrimidin e-4-carbonyl)piperazin-1-yl)-7-oxo-[1,2,4]triazolo[1,5-a]pyrimidin-4(7H)-yl)-N-(4-(pentafluoro-λ6-sulfaneyl)phenyl)acetamide **196** (82 mg) was obtained with a yield of 41.51%.

¹H NMR (400 MHz, DMSO) δ 10.94 (s, 1H), 8.52 (s, 1H), 7.88 (d, J = 9.2 Hz, 2H), 7.76 (d, J = 8.9 Hz, 2H), 6.77 (d, J = 2.2 Hz, 1H), 5.19 (d, J = 10.8 Hz, 2H), 4.52 (d, J = 12.6 Hz, 1H), 3.49 (d, J = 10.2 Hz, 3H), 2.93 (d, J = 25.4 Hz, 4H), 2.85-2.73 (m, 2H), 2.67 (d, J = 24.1 Hz, 2H), 2.42 (s, 3H), 1.96 (s, 1H), 1.78 (s, 1H), 1.16 (t, J = 7.4 Hz, 3H), 1.00 (d, J = 3.7 Hz, 1H).

### Biological Evaluation

### Test Example 1: Test for inhibition of WRN enzyme activity (ATPase) by the compounds of the present disclosure

The following method was used to determine the degree of inhibition of recombinant human WRN ATPase activity by the compounds of the present disclosure *in vitro.* In this method, ADP-Glo^{™} Kinase Assay kit (catalog No. V9102) from Promega was used. Detailed experimental procedures can be found in the kit manual.

The experimental procedure was briefly described as follows: test compounds were first dissolved in DMSO to prepare stock solutions, followed by serial dilution using reaction buffer (30 mM Tris, pH 7.5, 2 mM MgCl₂, 50 mM NaCl, 0.02% BSA, 0.1% Pluronic F127), with final concentrations of test compounds in the reaction system ranging from 1000 nM to 0.004 nM; WRN protein (purchased from BPS, catalog No. 101264), substrate 5-flap oligonucleotide (Flap26) (synthesized by Shanghai Generay Biotech Co., Ltd., sequence 5'-TTTTTTTTTTTTTTTTTTTTTCCAAGTAAAACGACGGCCAGTGC-3'), and ATP working solution (from ADP-Glo^{™} Kinase Assay kit component V915A) were prepared using reaction buffer. Reactions were performed in 384-well microplates. At first the compounds and recombinant human WRN protein (final concentration 5 nM) were added to the wells and the plate was incubated at room temperature for 15 minutes, then Flap solution (final concentration 0.1 nM) and ATP solution (final concentration 30 µM) were added to the reaction solution and the plate was incubated at room temperature for 10 minutes. Subsequently, 5 µL of ADP-Glo Reagent was added to the reaction system and incubated at room temperature for 40 minutes. Then 10 µL of Kinase Detection Reagent was added to the reaction system and incubated at room temperature for 30 minutes. After the incubation was completed, chemiluminescence intensity values of each well were measured using a microplate reader in luminescence mode. The percentage inhibition of the compounds at each concentration was calculated by comparison with the luminescence intensity values of the control group (0.1% DMSO), and IC₅₀ values of compounds were obtained by nonlinear regression analysis of compound concentration logarithm-inhibition rate using GraphPad Prism 9 software, as shown in Table 1.

The IC₅₀ value results of the compounds of the present disclosure are expressed as AA and A, respectively as follows:
"AA" indicates IC₅₀ ≤ 1 nM; "A" indicates 1 nM < IC₅₀ ≤ 100 nM.

**Table 1 IC₅₀ of the compounds of the present disclosure for inhibiting WRN enzyme activity (ATPase)**

| Example No. | IC₅₀(nM) | Example No. | IC₅₀(nM) | Example No. | IC₅₀(nM) |
|---|---|---|---|---|---|
| 1 | A | 68 | AA | 132 | A |
| 2 | A | 69 | AA | 133 | A |
| 3 | A | 70 | A | 134 | A |
| 4 | A | 71 | AA | 135 | A |
| 5 | A | 72 | A | 136 | A |
| 6 | AA | 73 | A | 137 | A |
| 7 | A | 74 | A | 138 | A |
| 8 | A | 75 | A | 139 | A |
| 9 | AA | 76 | A | 140 | A |
| 10 | A | 77 | A | 141 | A |
| 11 | A | 78 | A | 142 | A |
| 12 | A | 79 | A | 143 | A |
| 13 | A | 80 | A | 144 | A |
| 14 | A | 81 | A | 145 | A |
| *15* | A | 82 | A | 146 | A |
| 16 | A | 83 | A | 147 | A |
| 17 | A | 84 | A | 148 | A |
| 18 | A | 85 | A | 149 | A |
| 19 | AA | 86 | A | 150 | A |
| 20 | A | 87 | A | 151 | A |
| 21 | A | 88 | A | 152 | A |
| 22 | A | 89 | A | 153 | A |
| 23 | A | 90 | A | 154 | A |
| 24 | A | 91 | A | 155 | A |
| 25 | A | 92 | A | 156 | A |
| 26 | A | 94 | A | 157 | A |
| 27 | A | 95 | AA | 158 | A |
| 28 | A | 96 | A | 159 | A |
| 32 | A | 97 | A | 160 | A |
| 33 | AA | 98 | A | 161 | A |
| 35 | A | 99 | A | 162 | A |
| 36 | A | 100 | A | 163 | A |
| 37 | A | 101 | AA | 164 | A |
| 38 | AA | 102 | A | 165 | A |
| 41 | A | 103 | A | 166 | A |
| 42 | A | 104 | A | 167 | A |
| 43 | A | 105 | A | 168 | A |
| 44 | A | 106 | A | 169 | A |
| 45 | A | 107 | A | 170 | A |
| 46 | A | 108 | A | 171 | A |
| 47 | A | 110 | A | 173 | A |
| 48 | A | 111 | A | 174 | A |
| 49 | A | 112 | A | 175 | A |
| *50* | A | 113 | A | 176 | A |
| 51 | A | 114 | A | 177 | A |
| 52 | AA | 116 | A | 178 | AA |
| 53 | A | 117 | A | 179 | AA |
| 54 | A | 118 | A | 180 | A |
| 55 | A | 119 | A | 181 | A |
| 56 | A | 120 | A | 182 | A |
| 57 | A | 121 | A | 183 | A |
| 58 | A | 122 | A | 184 | A |
| 59 | A | 123 | A | 185 | A |
| 60 | A | 124 | A | 186 | A |
| 61 | A | 125 | AA | 187 | A |
| 62 | A | 126 | A | 188 | A |
| 63 | A | 127 | A | 189 | A |
| 64 | A | 128 | A | 193 | A |
| 65 | A | 129 | A | 194 | A |
| 66 | AA | 130 | A | 195 | A |
| 67 | AA | 131 | A | 196 | A |

Conclusion: The compounds of the present disclosure all exhibited good inhibitory effects on WRN enzymatic (ATPase) activity.

### Test Example 2: Inhibition assay of the compounds of the present disclosure on SW48 cell proliferation

The following method was used to determine the effect of the compounds of the present disclosure on SW48 cell proliferation. SW48 cells (MSI-H cells) were purchased from American ATCC cell bank and cultured in Leibovitz's L-15 medium (Gibco, catalog No. 11415064) containing 10% fetal bovine serum, 100 U penicillin, and 100 µg/mL streptomycin. Cell viability was determined by CellTiter-Glo^{®} Luminescent Cell Viability Assay kit (Promega, catalog No. G7573).

The experiment was performed according to the steps of the kit manual, which is briefly described as follows: test compounds were first dissolved in DMSO to prepare 10 mM stock solutions, and then diluted with medium to prepare test samples, with final compound concentrations ranging from 10000 nM to 1.52 nM. Cells in logarithmic growth phase were seeded into 96-well cell culture plates at a density of 500 cells per well and cultured overnight at 37 °C in an air incubator, then test compounds were added and cultivation continued for 120 hours. After the cultivation was completed, 40 µL of CellTiter-Glo detection reagent was added to each well, shaken for 5 minutes, and then left to stand for 10 minutes. Luminescence values of each well were read on a microplate reader using Luminescence mode. The percentage inhibition at each concentration point was calculated by comparison with the control group (0.1% DMSO), and then IC₅₀ values for the compounds for inhibition of cell proliferation were obtained using nonlinear regression analysis of compound concentration logarithm versus inhibition rate in GraphPad Prism 9 software, as shown in Table 2.

The IC₅₀ value results of the compounds of the present disclosure are represented by A, wherein "A" indicates 1 nM < IC₅₀ ≤ 100 nM.

**Table 2 IC₅₀ data for inhibition of SW48 cell proliferation by the compounds of the present disclosure**

| Example No. | IC₅₀(nM) | Example No. | IC₅₀(nM) | Example No. | IC₅₀(nM) |
|---|---|---|---|---|---|
| 6 | A | 102 | A | 153 | A |
| 17 | A | 103 | A | 154 | A |
| 18 | A | 104 | A | 155 | A |
| 19 | A | 105 | A | 156 | A |
| 22 | A | 106 | A | 157 | A |
| 27 | A | 112 | A | 158 | A |
| 33 | A | 113 | A | 159 | A |
| 47 | A | 118 | A | 161 | A |
| 49 | A | 120 | A | 162 | A |
| 52 | A | 121 | A | 163 | A |
| 53 | A | 123 | A | 164 | A |
| 54 | A | 125 | A | 169 | A |
| 55 | A | 126 | A | 170 | A |
| 84 | A | 129 | A | 174 | A |
| 85 | A | 130 | A | 175 | A |
| 86 | A | 132 | A | 176 | A |
| 87 | A | 133 | A | 178 | A |
| 88 | A | 134 | A | 183 | A |
| 91 | A | 140 | A | 184 | A |
| 96 | A | 142 | A | 185 | A |
| 97 | A | 147 | A | 186 | A |
| 98 | A | 149 | A | 188 | A |
| 99 | A | 150 | A | | |
| 101 | A | 151 | A | | |

Conclusion: The compounds of the present disclosure all exhibited good inhibitory effects on SW48 cell proliferation.

### Test Example 3: Inhibition assay of the compounds of the present disclosure on RL95-2 cell proliferation

The following method was used to determine the effect of the compounds of the present disclosure on RL95-2 cell proliferation. RL95-2 cells (MSI-H cells) were purchased from Cell Resource Center of Shanghai Institutes for Biological Sciences, Chinese Academy of Sciences, and cultured in DMEM/F12 medium (Gibco, catalog No. A4192001) containing 10% fetal bovine serum, 100 U penicillin, and 100 µg/mL streptomycin. Cell viability was determined by CellTiter-Glo^{®} Luminescent Cell Viability Assay kit (Promega, catalog No. G7573).

The experiment was performed according to the steps of the kit manual, which is briefly described as follows: test compounds were first dissolved in DMSO to prepare 10 mM stock solutions, and then diluted with medium to prepare test samples, with final compound concentrations ranging from 10000 nM to 1.52 nM. Cells in logarithmic growth phase were seeded into 96-well cell culture plates at a density of 500 cells per well and cultured overnight at 37 °C in a 5% CO₂ incubator, and then test compounds were added and cultivation continued for 120 hours. After the cultivation was completed, 50 µL of CellTiter-Glo detection reagent was added to each well, shaken for 5 minutes, and then left to stand for 10 minutes. Luminescence values of each well were read on a microplate reader using Luminescence mode. The percentage inhibition at each concentration point was calculated by comparison with the control group (0.1% DMSO), and then IC₅₀ values for the compounds for inhibition of cell proliferation were obtained using nonlinear regression analysis of compound concentration logarithm versus inhibition rate in GraphPad Prism 9 software, as shown in Table 3.

The IC₅₀ value results of the compounds of the present disclosure are represented by A, wherein "A" indicates 1 nM < IC₅₀ ≤ 100 nM.

**Table 3 IC₅₀ data for inhibition of RL95-2 cell proliferation by the compounds of the present disclosure**

| Example No. | IC₅₀(nM) | Example No. | IC₅₀(nM) | Example No. | IC₅₀(nM) |
|---|---|---|---|---|---|
| 6 | A | 84 | A | 140 | A |
| 17 | A | 85 | A | 142 | A |
| 18 | A | 86 | A | 147 | A |
| 19 | A | 88 | A | 149 | A |
| 22 | A | 89 | A | 150 | A |
| 27 | A | 97 | A | 152 | A |
| 47 | A | 98 | A | 153 | A |
| 49 | A | 99 | A | 154 | A |
| 53 | A | 101 | A | 156 | A |
| 54 | A | 102 | A | 157 | A |
| 55 | A | 103 | A | 158 | A |
| 62 | A | 106 | A | 159 | A |
| 63 | A | 112 | A | 161 | A |
| 65 | A | 113 | A | 162 | A |
| 66 | A | 118 | A | 163 | A |
| 67 | A | 120 | A | 164 | A |
| 68 | A | 121 | A | 167 | A |
| 69 | A | 123 | A | 178 | A |
| 70 | A | 125 | A | 183 | A |
| 71 | A | 126 | A | 184 | A |
| 73 | A | 129 | A | 185 | A |
| 74 | A | 130 | A | 186 | A |
| 76 | A | 132 | A | 188 | A |
| 77 | A | 133 | A | | |
| 80 | A | 134 | A | | |

Conclusion: The compounds of the present disclosure all exhibited good inhibitory effects on RL95-2 cell proliferation.

### Test Example 4: Permeability assay of the compounds of the present disclosure

The bidirectional permeability and efflux ratio of the example compounds and control compound were determined in a Caco-2 monolayer cell model.

Caco-2 cells (cultured at Shanghai Medicilon Inc.) were recovered in the experiment. After the cells were in good condition, they were digested, and the cell density was adjusted (1 × 10⁵ cells/cm²). Caco-2 cells were seeded into Transwell chambers and placed in 24-well plates for cultivation. After continuous cultivation for about 21 days, TEER values were measured one day before the experiment to ensure formation of a confluent cell monolayer for transport experiments (TEER > 200 Ω·cm²). Test compounds were diluted from 10 mM stock solutions to 10 µM concentration using transport buffer (HBSS containing BSA) (metoprolol and atenolol were set as positive compounds). For efflux determination, 0.6 mL of compound dilution solution was added to the outer well (side B), and 0.4 mL of blank transport buffer was added to the Transwell chamber. For absorption determination, 0.4 mL of transport buffer containing compound was added to the Transwell chamber, and 0.6 mL of blank transport buffer was added to the outer side. After setup, the 24-well plates were cultivated at 37 °C and 5% CO₂ with 95% relative humidity for 120 minutes, and permeability of test compounds from A to B direction or B to A direction was measured, respectively, with two duplicate wells set for each direction. Liquid in Transwell chambers and 24-well plates was collected separately, 60 µL each, and 180 µL of acetonitrile (containing internal standard) was added, vortexed for 1 min, then centrifuged at 4 °C, 10000 rpm for 10 min. The supernatant was taken, filtered through a 0.22 µm membrane, and placed in vials for testing. The content of the compounds in each Transwell chamber and 24-well plate was determined by liquid chromatography-tandem mass spectrometry (LC-MS/MS). Permeability data for some of the compounds of the present disclosure are shown in Table 4.

**Table 4 Permeability data of compounds of the present disclosure**

| Example | Caco-2 permeability | | |
|---|---|---|---|
| | A to B | B to A | Efflux ratio |
| | Pₐₚₚ (10⁻⁶ cm/s) | Pₐₚₚ (10⁻⁶ cm/s) | Pₐₚₚ(B-A)/ Pₐₚₚ(A-B) |
| 68 | 4.64 | 5.09 | 1.10 |
| 80 | 3.67 | 2.29 | 0.62 |
| Control compound HRO761 | 3.96 | 14.47 | 3.65 |

Conclusion: Examples 68 and 80 both exhibited moderate permeability with no significant efflux; control compound HRO761 exhibited moderate permeability but had apparent potential efflux properties.

### Test Example 5: Metabolic stability study of the compounds of the present disclosure in human/dog liver microsomes

### 1. Experimental Objective

The purpose of this study was to investigate the metabolic stability of the compounds of the present disclosure in human and dog liver microsomes.

### 2. Reagent Information (see Table 5)

**Table 5 Information on reagents used in the experiment**

| Name | Catalog No. | Supplier |
|---|---|---|
| Mixed human liver microsomes | 0121D1.03 | Iphase |
| Dog liver microsomes | 032C13.13 | Iphase |
| Midazolam maleate | 171265-201402 | National Institutes for Food and Drug Control |
| NADPH | 10107824001 | Roche, Switzerland |
| Potassium dihydrogen phosphate | 20150326 | Sinopharm Chemical Reagent Co., Ltd. |
| Dipotassium hydrogen phosphate | 20211202 | Sinopharm Chemical Reagent Co., Ltd. |
| Magnesium chloride (MgCl₂) | 1001553880 | Merck, USA |
| Loratadine | 100615-202005 | National Institutes for Food and Drug Control |
| Glyburide | BD103656 | Bide Pharmatech |
| DMSO | D8418-1L | Merck, USA |
| Methanol | 1.06007.4008 | Merck, USA |
| Acetonitrile | 1.00030.4008 | Merck, USA |
| Formic acid | FA-3116U | Anaqua Chemicals Supply |

### 3. Experimental Protocol

The compounds were co-incubated with human (or dog) liver microsomes, and the reaction was initiated by adding coenzyme NADPH (1 mM). At 0, 5, 15, 30, and 60 minutes, 60 µL of incubation solution was removed, and 180 µL of acetonitrile containing internal standard (clopidogrel) was added to quench the reaction, the mixture was shaken for 10 seconds, centrifuged at 10000 rpm for 10 minutes, and the supernatant was taken for analysis by LC-MS/MS. The in vitro intrinsic clearance was calculated based on the elimination half-life of the test compound in the incubation system. Midazolam was used as an internal reference compound, with two parallel incubations for each. Incubation conditions are summarized in Table 6 below:

**Table 6 Incubation conditions for the example compounds of the present disclosure**

| | |
|---|---|
| Liver microsomes | 0.5 mg mL⁻¹ (test compound); 0.2 mg mL⁻¹ (midazolam) |
| Incubation buffer | Phosphate buffer (100 mM, pH 7.4) |
| Initial concentration of the test compounds | 1M |
| Final volume of the incubation system | 0.1 mL |
| Incubation time | 0, 5, 15, 30, 60 min (compounds of the present disclosure) 0, 5, 20 min (midazolam) |
| Magnesium chloride | 3 mM |
| NADPH | 1 mM |
| Parallel reactions | Duplicate |

### 4. Data Analysis

The ratio of the peak area of analyte to internal standard (A_{analyte}/A_{IS}) was obtained from the instrument, and the remaining percentage (%Control) was calculated from the ratio of A_{analyte}/A_{IS} in non-zero time point samples to zero time samples. Ln (%Control) was plotted against incubation time and linearly fitted. The test compound clearance constant (k, min⁻¹) and clearance half-life (T_{1/2}, min) were calculated using the following equations. $k = - slope$ ${T}_{1 / 2} = 0.693 / k .$

### 5. Experimental Results (see Table 7)

**Table 7 Human/dog liver microsome stability results for the example compounds of the present disclosure and control compound HRO761**

| Example | Liver microsomal stability t_{1/2} (min) | |
|---|---|---|
| | Dog | Human |
| 67 | 318 | 657 |
| 76 | 200 | 999 |
| 80 | 637 | 1304 |
| 125 | 182 | 705 |
| HRO761 | 175 | 483 |

Conclusion: Compared with the control compound HRO761, the half-life of the compounds of the present disclosure was significantly prolonged, and the liver microsome stability in human and dog was significantly improved.

### Test Example 6: Pharmacokinetic test of the compounds of the present disclosure in mice

### 1. Experimental Objective

ICR mice were used as test animals, the plasma concentrations of the control compound HRO761 and the compounds of the present disclosure at different time points after intragastric administration were determined by LC/MS/MS method, and the pharmacokinetic characteristics of the compounds of the present disclosure in mice were studied.

### 2. Experimental Protocol

### 6.1 Experimental drugs and animals;

Control compound HRO761, Compounds 6, 27, 67, 68, 80, 97, 103, and 106.

ICR mice, male, 30.8 to 38.0 g, purchased from Vital River Laboratory Animal Technology Co., Ltd.

### 6.2 Drug preparation

An appropriate amount of the test compound was weighed, and an appropriate amount of DMA (N,N-dimethylacetamide), 30% Solutol HS15 (30% polyethylene glycol-15 hydroxystearate), and Saline (saline) were added sequentially, the mixture was ultrasonicated and vortex mixed to prepare a 1 mg/mL dosing formulation. The ratio of DMA:30% Solutol HS15:Saline was 5:5:90 (v/v/v).

### 2.3 Administration

After overnight fasting, ICR mice in each test compound injection group (9 mice per group) were administered intragastrically (PO, compound dose was 10 mg/kg, administration volume was 10 mL/kg), and food was given 4 hours after administration.

### 3. Procedures

About 0.1 mL of blood was collected via the orbital cavity before administration and at 0.25 h, 0.5 h, 1 h, 2 h, 4 h, 8 h, 10 h, and 24 h after administration, and whole blood samples were placed in anticoagulant tubes containing EDTA-K2. The samples were placed on ice after collection, and plasma was separated by centrifugation (centrifugation conditions: 1500 g, 10 minutes). The collected plasma was stored at -40 to -20 °C before analysis.

The content of the test compound in mouse plasma after intragastric administration was determined by LC-MS/MS.

### 4. Pharmacokinetic Parameter Results

The pharmacokinetic parameters of the test compounds are shown in Table 8 below.

**Table 8 Pharmacokinetic parameters of test compounds in mice**

| Example No. | Pharmacokinetic experiment | | | |
|---|---|---|---|---|
| | Administration (Dosage) | Plasma concentration Cmax (ng/mL) | Area under curve AUC₀₋ₜ(ng h/mL) | Half-life T_{1/2} (h) |
| 6 | Oral (10 mg/kg) | 15880 | 39740 | 1.26 |
| 27 | Oral (10 mg/kg) | 18124 | 66598 | 2.27 |
| 67 | Oral (10 mg/kg) | 31390 | 83446 | 0.66 |
| 68 | Oral (10 mg/kg) | 17251 | 98867 | 2.41 |
| 80 | Oral (10 mg/kg) | 19753 | 103688 | 2.11 |
| 97 | Oral (10 mg/kg) | 9924 | 28481 | 2.30 |
| 103 | Oral (10 mg/kg) | 7125 | 24282 | 2.75 |
| 106 | Oral (10 mg/kg) | 44999 | 330355 | 4.85 |
| HRO761 | Oral (10 mg/kg) | 7013 | 15563 | 2.81 |

Conclusion: Compared with the control compound HRO761, each compound of the present disclosure had higher blood drug concentration and area under the curve, and exhibited significantly improved pharmacokinetic properties.

Note: The structure of HRO761 (prepared according to Example 42 of patent publication WO2022249060) is as follows:

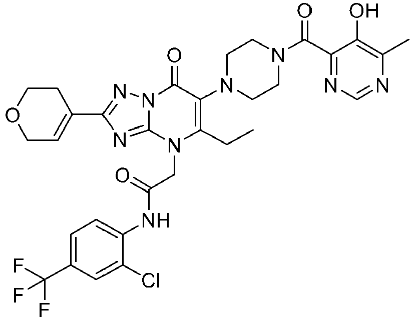

## Claims

1. A compound of formula (I): 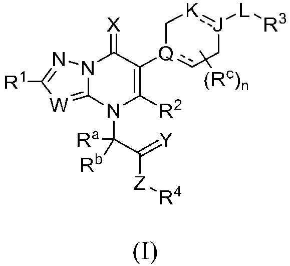
or a stereoisomer, tautomer, or pharmaceutically acceptable salt thereof,
wherein:
W is selected from the group consisting of N and CH;
X is selected from the group consisting of O and S;
Y is selected from the group consisting of CH₂, O, and S;
Z is selected from the group consisting of NH, CH₂, and CD₂;
L is selected from the group consisting of -C(=O)-, -C(=S)-, -S(=O)-, -S(=O)₂-, 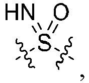 and -NR^{g}C(=O)-;
R⁸ is selected from the group consisting of hydrogen and C₁₋₆ alkyl;
- - - - - in Q - - - - - is selected from the group consisting of a single bond and a double bond;
Q is selected from the group consisting of C, N, and CR^{d};
R^{d} is selected from the group consisting of hydrogen, hydroxy, cyano, halogen, and methoxy;
- - - - - in K - - - - - is selected from the group consisting of a single bond and a double bond, or K - - - - - is absent, wherein when K - - - - - is absent, a 5-membered ring 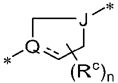 is formed;
provided that:
when - - - - - in K - - - - - is a single bond, K is selected from the group consisting of -(CH₂)ₚ- and -NH-, and J is N; p is selected from the group consisting of 1 and 2;
when - - - - - in K - - - - - is a double bond, K is CH, and J is C;
R^{a} and R^{b} are each independently selected from the group consisting of hydrogen, deuterium, halogen, C₁₋₆ alkyl, and C₁₋₆ alkoxy, wherein the halogen is preferably fluorine; alternatively, R^{a} and R^{b}, together with the carbon atom to which they are attached, form a C₃₋₆ cycloalkyl, wherein the C₃₋₆ cycloalkyl is optionally further substituted with one or more substituents selected from the group consisting of halogen, hydroxy, cyano, carbonyl, and -NR⁶R⁷;
R^{c}, being the same or different, is each independently selected from the group consisting of hydrogen, deuterium, C₁₋₆ alkyl, and C₃₋₆ cycloalkyl, wherein the C₁₋₆ alkyl or C₃₋₆ cycloalkyl is optionally further substituted with one or more substituents selected from the group consisting of halogen, hydroxy, cyano, carbonyl, and -NR⁶R⁷;
alternatively, two R^{c}, together with the same carbon atom to which they are attached, form a -C(=O)-;
alternatively, two R^{c}, together with the same carbon atom to which they are attached, form a C₃₋₆ cycloalkyl or a 3- to 8- membered heterocyclyl, wherein the 3- to 8- membered heterocyclyl contains one or more N, O, or S(=O)ᵣ;
alternatively, two R^{c}, together with the different carbon atoms to which they are respectively attached, form a C₄₋₆ cycloalkyl or a 4- to 8- membered heterocyclyl, wherein the 4- to 8-membered heterocyclyl contains one or more N, O, or S(=O)ᵣ;
R¹ is selected from the group consisting of alkyl, alkenyl, alkynyl, -NR¹⁵R¹⁶, alkoxy, alkylthio, 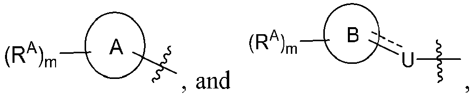 wherein the alkyl, alkoxy, or alkylthio is optionally further substituted with one or more substituents selected from R^{A}, and the alkenyl or alkynyl is substituted with one or more substituents selected from R^{AA};
R^{AA} is selected from the group consisting of halogen, alkoxy, aryl, heteroaryl, cycloalkyl, heterocyclyl, fused ring, and -C(=O)NR⁶R⁷, wherein the aryl, heteroaryl, cycloalkyl, heterocyclyl, or fused ring is optionally further substituted with one or more substituents selected from the group consisting of C₁₋₆ alkyl, C₁₋₆ haloalkyl, C₁₋₆ alkoxy, C₁₋₆ haloalkoxy, halogen, hydroxy, and cyano, provided that the heterocyclyl is not morpholinyl;
R¹⁵ and R¹⁶ are each independently selected from the group consisting of hydrogen, deuterium, C₁₋₆ alkyl, and aryl, wherein the C₁₋₆ alkyl or aryl is optionally further substituted with one or more substituents selected from the group consisting of halogen, hydroxy, and cyano;
ring A is selected from the group consisting of:
4) C₃₋₈ saturated monocyclic cycloalkyl, C₇₋₈ partially unsaturated monocyclic cycloalkyl, spirocycloalkyl, fused cycloalkyl, or bridged cycloalkyl, wherein the spirocycloalkyl, fused cycloalkyl, or bridged cycloalkyl comprises 0 or 1 double bond;
5) 4-membered monocyclic heterocyclyl, 7- to 8- membered monocyclic heterocyclyl, spiroheterocyclyl, or fused heterocyclyl, wherein the monocyclic heterocyclyl, spiroheterocyclyl, or fused heterocyclyl comprises 0 or 1 double bond, provided that any ring constituting the spiroheterocyclyl is not cyclopropane, wherein the monocyclic heterocyclyl is optionally further formed into a bridged heterocyclyl by connecting two non-directly connected atoms on the ring via C₁₋₂ alkylene; and
6) bicyclic aryl, bicyclic heteroaryl, or bicyclic fused ring;
- - - - - in - - - - - U is selected from the group consisting of a single bond and a double bond;
provided that: when - - - - - in - - - - - U is selected from a double bond, U is CR^{bb}, and ring B is selected from the group consisting of cycloalkyl, heterocyclyl, and fused cyclyl;
when - - - - - in - - - - -U is selected from a single bond, U is selected from the group consisting of -O-, -N(R^{e})-, and -S-, and ring B is selected from the group consisting of cycloalkyl, heterocyclyl, aryl, heteroaryl, and fused cyclyl;
R^{bb} is selected from the group consisting of hydrogen and C₁₋₆ alkyl;
R^{e} is selected from the group consisting of hydrogen and C₁₋₆ alkyl;
R^{A}, being the same or different, is each independently selected from the group consisting of deuterium, hydroxy, halogen, nitro, cyano, alkyl, alkenyl, alkynyl, cycloalkyl, heterocyclyl, aryl, heteroaryl, -SF₅, -OR⁵, -OC(=O)R⁵, -C(=O)R⁵, -C(=O)OR⁵, -N(R⁶)C(=O)R⁷, -N(R⁶)C(=O)OR⁷, -NR⁶R⁷, -C(=O)NR⁶R⁷, -S(=O)ᵣNR⁶R⁷, and -S(=O)ᵣR⁵, wherein the alkyl, alkenyl, alkynyl, cycloalkyl, heterocyclyl, aryl, or heteroaryl is optionally further substituted with one or more substituents selected from the group consisting of deuterium, hydroxy, halogen, nitro, cyano, alkyl, alkoxy, haloalkyl, haloalkoxy, hydroxyalkyl, cycloalkyl, heterocyclyl, aryl, heteroaryl, -OR⁸, =O, -C(=O)R⁸, -C(=O)OR⁸, -OC(=O)R⁸, -NR⁹R¹⁰, -C(=O)NR⁹R¹⁰, -S(=O)₂NR⁹R¹⁰, -N(R⁹)C(=O)R¹⁰, and -N(R⁹)C(=O)OR¹⁰;
alternatively, two R^{A}, together with the same carbon atom to which they are attached, form a -C(=O), -C(=S), or -C(=NR^{cc});
R^{cc} is selected from the group consisting of hydrogen, hydroxy, and C₁₋₆ alkoxy;
R² is selected from the group consisting of hydrogen, deuterium, alkyl, hydroxyalkyl, haloalkyl, cycloalkyl, -OR⁵, -S(=O)ᵣR⁵, and -NR⁶R⁷;
R³ is selected from the group consisting of hydrogen, C₁₋₆ alkyl, amino, hydroxylamino, aryl, heteroaryl, 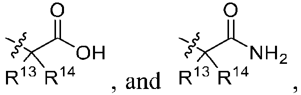 wherein the C₁₋₆ alkyl, aryl, or heteroaryl is optionally further substituted with one or more R^{B};
R^{B}, being the same or different, is each independently selected from the group consisting of deuterium, alkyl, hydroxy, halogen, nitro, cyano, alkenyl, alkynyl, cycloalkyl, heterocyclyl, aryl, heteroaryl, -SF₅, -OR⁵, -OC(=O)R⁵, -C(=O)R⁵, -C(=O)OR⁵, -N(R⁶)C(=O)R⁷, -N(R⁶)C(=O)OR⁷, -NR⁶R⁷, -C(=O)NR⁶R⁷, -S(=O)ᵣNR⁶R⁷, and -S(=O)ᵣR⁵, wherein the alkenyl, alkynyl, cycloalkyl, heterocyclyl, aryl, or heteroaryl is optionally further substituted with one or more substituents selected from the group consisting of hydroxy, halogen, nitro, cyano, alkyl, alkoxy, haloalkyl, haloalkoxy, hydroxyalkyl, cycloalkyl, heterocyclyl, aryl, heteroaryl, -OR⁸, =O, -C(=O)R⁸, -C(=O)OR⁸, -OC(=O)R⁸, -NR⁹R¹⁰, -C(=O)NR⁹R¹⁰, -S(=O)₂NR⁹R¹⁰, -N(R⁹)C(=O)R¹⁰, and -N(R⁹)C(=O)OR¹⁰;
R¹³ and R¹⁴ are each independently selected from the group consisting of hydrogen, deuterium, C₁₋₆ alkyl, and C₁₋₆ alkoxy; alternatively, R¹³ and R¹⁴, together with the carbon atom to which they are commonly attached, form a C₃₋₆ cycloalkyl, wherein the C₃₋₆ cycloalkyl is optionally further substituted with one or more substituents selected from the group consisting of halogen, hydroxy, and cyano;
R⁴ is selected from the group consisting of aryl, heteroaryl, and bicyclic fused ring, wherein the bicyclic fused ring is preferably a fused ring formed from a monocyclic aryl or monocyclic heteroaryl and a monocyclic heterocyclyl or monocyclic cycloalkyl, wherein the aryl, heteroaryl, or bicyclic fused ring is optionally further substituted with one or more substituents selected from the group consisting of halogen, hydroxy, cyano, -SF₅, C₁₋₆ alkyl, C₁₋₆ cycloalkyl, C₁₋₆ haloalkyl, C₁₋₆ halocycloalkyl, C₁₋₆ hydroxyalkyl, C₁₋₆ alkoxy, C₁₋₆ haloalkoxy, C₁₋₆ alkenyl, C₁₋₆ alkynyl, -NR⁶R⁷, -C(=O)R⁵, and -S(=O)ᵣR⁵;
R⁵ is each independently selected from the group consisting of hydrogen, alkyl, cycloalkyl, heterocyclyl, aryl, and heteroaryl, wherein the alkyl, cycloalkyl, heterocyclyl, aryl, or heteroaryl is optionally further substituted with one or more substituents selected from the group consisting of deuterium, hydroxy, halogen, nitro, cyano, alkyl, alkoxy, haloalkyl, haloalkoxy, cycloalkyl, heterocyclyl, aryl, heteroaryl, =O, -C(=O)R⁸, -C(=O)OR⁸, -OC(=O)R⁸, -NR⁹R¹⁰, -C(=O)NR⁹R¹⁰, -S(=O)₂NR⁹R¹⁰, and -N(R⁹)C(=O)R¹⁰;
R⁶ and R⁷ are each independently selected from the group consisting of hydrogen, hydroxy, alkyl, cycloalkyl, heterocyclyl, aryl, and heteroaryl, wherein the alkyl, cycloalkyl, heterocyclyl, aryl, or heteroaryl may be optionally further substituted with one or more substituents selected from the group consisting of hydroxy, halogen, nitro, cyano, alkyl, alkoxy, cycloalkyl, heterocyclyl, aryl, heteroaryl, =O, -C(=O)R⁸, -C(=O)OR⁸, -OC(=O)R⁸, -NR⁹R¹⁰, -C(=O)NR⁹R¹⁰, -S(=O)₂NR⁹R¹⁰, and -N(R⁹)C(=O)R¹⁰;
alternatively, R⁶ and R⁷, together with the atom to which they are attached, form a 4- to 8-membered heterocyclyl containing one or more N, O, or S(=O)ᵣ, wherein the 4- to 8- membered heterocyclyl is optionally further substituted with one or more substituents selected from the group consisting of hydroxy, halogen, nitro, cyano, alkyl, alkoxy, cycloalkyl, heterocyclyl, aryl, heteroaryl, =O, -C(=O)R⁸, -C(=O)OR⁸, -OC(=O)R⁸, -NR⁹R¹⁰, -C(=O)NR⁹R¹⁰, -S(=O)₂NR⁹R¹⁰, and -N(R⁹)C(=O)R¹⁰;
R⁸, R⁹, and R¹⁰ are each independently selected from the group consisting of hydrogen, alkyl, amino, cycloalkyl, heterocyclyl, aryl, and heteroaryl, wherein the alkyl, cycloalkyl, heterocyclyl, aryl, or heteroaryl may be optionally further substituted with one or more substituents selected from the group consisting of hydroxy, halogen, nitro, amino, cyano, alkyl, alkoxy, cycloalkyl, heterocyclyl, aryl, heteroaryl, carboxy, and carboxylate;
n is 0, 1, 2, 3, 4, 5, or 6;
m is 0, 1, 2, 3, 4, or 5; and
r is each independently 0, 1, or 2.

2. The compound or stereoisomer, tautomer, or pharmaceutically acceptable salt thereof according to claim 1, wherein W is N.

3. The compound or stereoisomer, tautomer, or pharmaceutically acceptable salt thereof according to claim 1 or 2, wherein W is CH.

4. The compound or stereoisomer, tautomer, or pharmaceutically acceptable salt thereof according to any one of claims 1 to 3, wherein X and Y are O.

5. The compound or stereoisomer, tautomer, or pharmaceutically acceptable salt thereof according to any one of claims 1 to 4, wherein Z is NH and L is -C(=O)-.

6. The compound or stereoisomer, tautomer, or pharmaceutically acceptable salt thereof according to any one of claims 1 to 5, wherein - - - - - in Q - - - - - is a single bond; Q is selected from the group consisting of N and CH;
- - - - - in K - - - - - is a single bond, K is selected from the group consisting of CH₂ and CH₂CH₂, and J is N; and
R^{c} is hydrogen.

7. The compound or stereoisomer, tautomer, or pharmaceutically acceptable salt thereof according to any one of claims 1 to 6, wherein R^{a} and R^{b} are hydrogen.

8. The compound or stereoisomer, tautomer, or pharmaceutically acceptable salt thereof according to any one of claims 1 to 2 and 4 to 7, being a compound of formula (II), or a stereoisomer, tautomer, or pharmaceutically acceptable salt thereof: 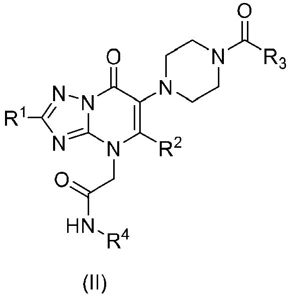 wherein: R¹, R², R³, and R⁴ are as defined in claim 1.

9. The compound or stereoisomer, tautomer, or pharmaceutically acceptable salt thereof according to any one of claims 1 and 3 to 7, being a compound of formula (III), or a stereoisomer, tautomer, or pharmaceutically acceptable salt thereof: 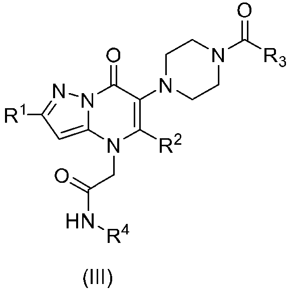 wherein: R¹, R², R³, and R⁴ are as defined in claim 1.

10. The compound or stereoisomer, tautomer, or pharmaceutically acceptable salt thereof according to claim 1, wherein - - - - - in Q - - - - - is a single bond; Q is N;
- - - - - in K - - - - - is a single bond, K is CH₂, and J is CH;
R^{c} is hydrogen; and
L is -NHC(=O)-.

11. The compound or stereoisomer, tautomer, or pharmaceutically acceptable salt thereof according to any one of claims 1 to 10, wherein R¹ is selected from the group consisting of C₁₋₆ alkyl, -NR¹⁵R¹⁶, C₂₋₆ alkenyl, and C₂₋₆ alkynyl, wherein the C₁₋₆ alkyl is optionally further substituted with one or more halogens, wherein the C₂₋₆ alkenyl or C₂₋₆ alkynyl is substituted with at least one or more substituents selected from R^{AA};
R^{AA} is selected from the group consisting of halogen, alkoxy, aryl, heteroaryl, cycloalkyl, heterocyclyl, fused ring, and -C(=O)NR⁶R⁷; wherein the alkoxy, aryl, heteroaryl, cycloalkyl, heterocyclyl, or fused ring is optionally further substituted with one or more substituents selected from the group consisting of methyl, trifluoromethyl, methoxy, trifluoromethoxy, halogen, hydroxy, and cyano; provided that the heterocyclyl is not morpholinyl;
R⁶ and R⁷ are each independently selected from the group consisting of hydrogen and alkyl, or, R⁶ and R⁷, together with the N atom to which they are attached, form a 4- to 8- membered heterocyclyl;
R^{1S} is selected from the group consisting of hydrogen;
R¹⁶ is selected from the group consisting of C₁₋₆ alkyl and aryl, wherein the C₁₋₆ alkyl or aryl is optionally further substituted with one or more halogens.

12. The compound or stereoisomer, tautomer, or pharmaceutically acceptable salt thereof according to any one of claims 1 to 10, wherein R¹ is selected from 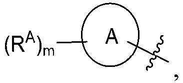 wherein ring A is selected from the group consisting of C₃₋₈ saturated monocyclic cycloalkyl, C₇₋₈ partially unsaturated monocyclic cycloalkyl, spirocycloalkyl, fused cycloalkyl, bridged cycloalkyl, 4-membered monocyclic heterocyclyl, 7- to 8- membered monocyclic heterocyclyl, spiroheterocyclyl, fused heterocyclyl, bicyclic heteroaryl, and bicyclic fused ring, wherein the spirocycloalkyl, fused cycloalkyl, bridged cycloalkyl, 4-membered monocyclic heterocyclyl, 7-to 8- membered monocyclic heterocyclyl, or spiroheterocyclyl contains 0 or 1 double bond, provided that any ring constituting the spiroheterocyclyl is not cyclopropane;
R^{A} is selected from the group consisting of deuterium, halogen, hydroxy, methoxy, -NR⁶R⁷, -C(=O)NR⁶R⁷, C₁₋₆ alkyl, C₃₋₈ cycloalkyl, and 5- to 6- membered heterocyclyl, wherein the C₁₋₆ alkyl or 5- to 6- membered heterocyclyl is optionally further substituted with one or more substituents selected from the group consisting of deuterium, hydroxy, halogen, cyano, alkyl, alkoxy, haloalkyl, and haloalkoxy, alternatively, two R^{A}, together with the same carbon atom to which they are attached, form a -C(=O) or -C(=NR^{cc});
R⁶ and R⁷ are selected from the group consisting of hydrogen;
R^{cc} is selected from the group consisting of hydroxy and methoxy;
m is 0, 1, 2, 3, 4, or 5.

13. The compound or stereoisomer, tautomer, or pharmaceutically acceptable salt thereof according to any one of claims 1 to 10, wherein R¹ is selected from 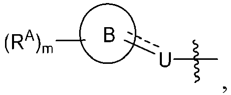 wherein - - - - - in - - - - -U is selected from a double bond, U is CR^{bb}, and ring B is selected from the group consisting of cycloalkyl and heterocyclyl;
R^{bb} is selected from the group consisting of hydrogen and methyl;
R^{A} is selected from the group consisting of deuterium, halogen, hydroxy, methyl, methoxy, -NR⁶R⁷, and -C(=O)NR⁶R⁷; R⁶ and R⁷ are each independently hydrogen;
m is 0, 1, 2, 3, 4, or 5.

14. The compound or stereoisomer, tautomer, or pharmaceutically acceptable salt thereof according to any one of claims 1 to 10, wherein R¹ is selected from 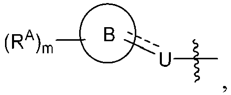 wherein - - - - - in - - - - - U is selected from a single bond, U is O, and ring B is selected from the group consisting of aryl and heteroaryl; and
m is 0.

15. The compound or stereoisomer, tautomer, or pharmaceutically acceptable salt thereof according to any one of claims 1 to 10, wherein R¹ is selected from the group consisting of:

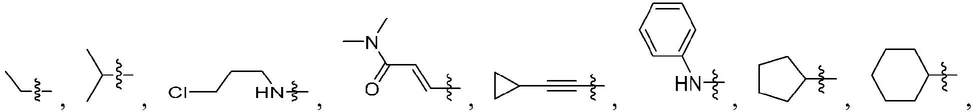

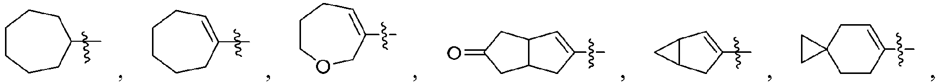

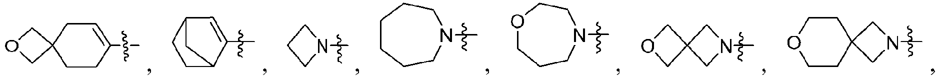

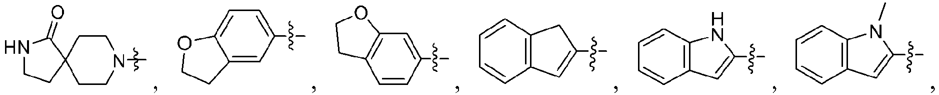

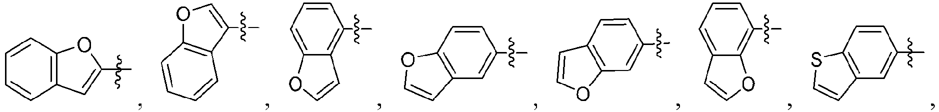

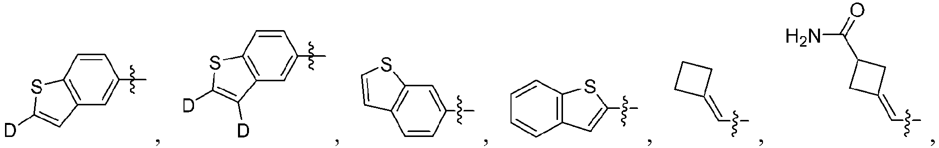

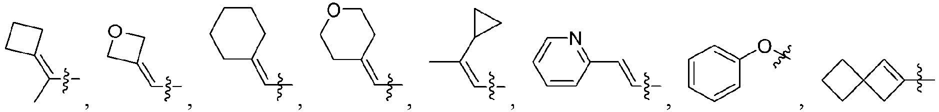

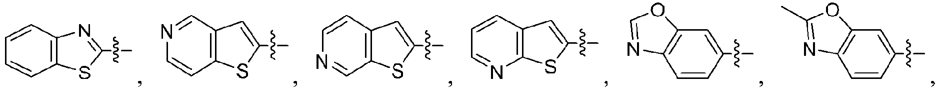

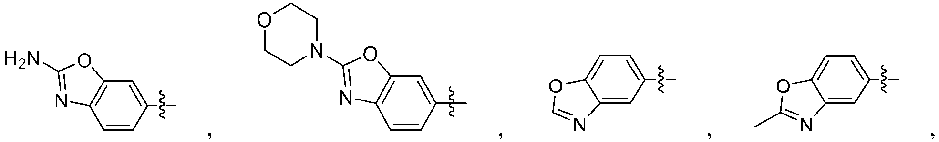

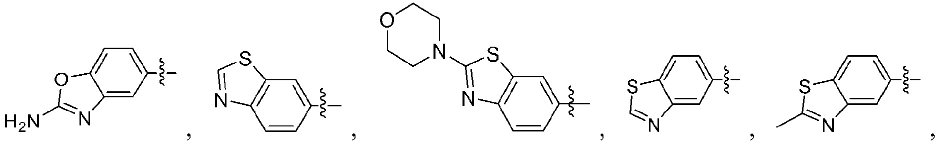

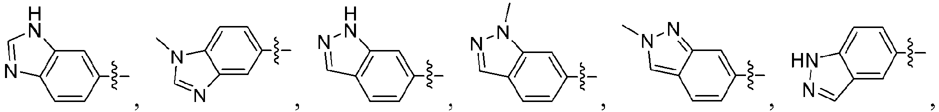

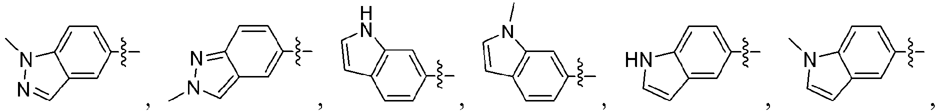

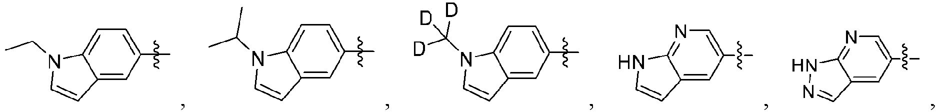

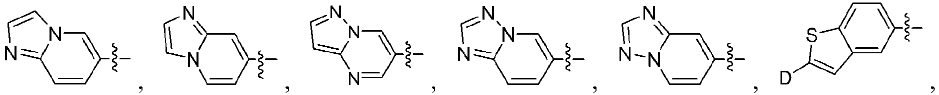

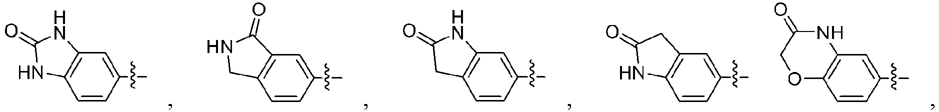

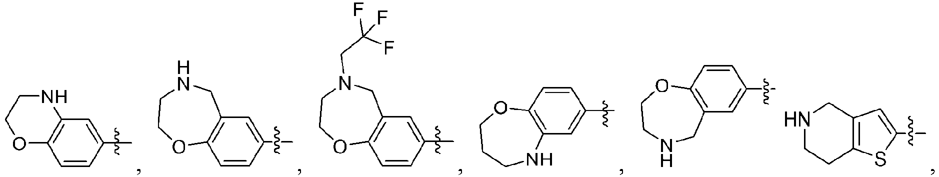

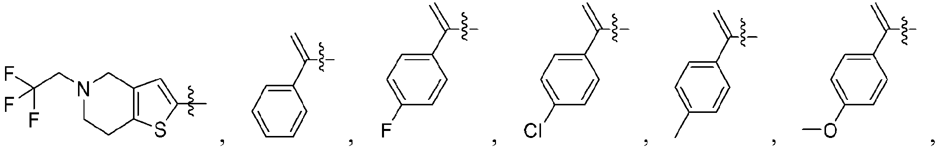

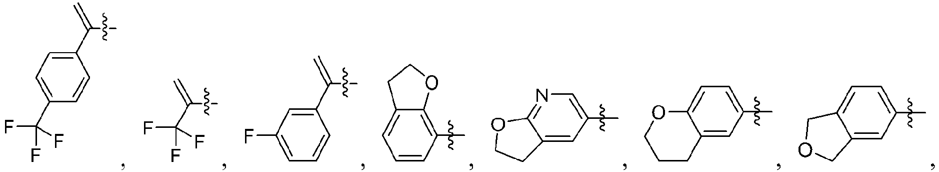

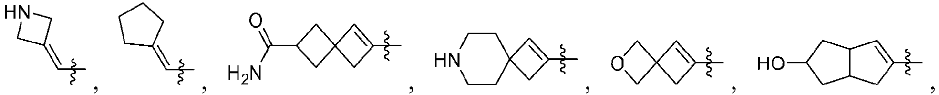

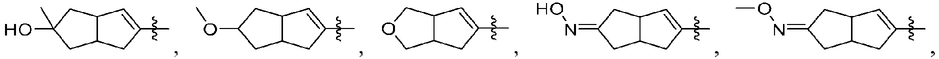

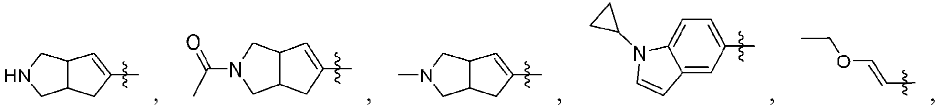

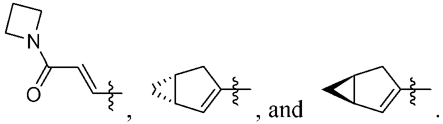

16. The compound or stereoisomer, tautomer, or pharmaceutically acceptable salt thereof according to any one of claims 1 to 15, wherein R² is selected from the group consisting of hydrogen, deuterium, C₁₋₃ alkyl, 3- to 6- membered cycloalkyl, C₁₋₃ hydroxyalkyl, and C₁₋₃ haloalkyl, preferably methyl, ethyl, isopropyl, cyclopropyl, or cyclobutyl.

17. The compound or stereoisomer, tautomer, or pharmaceutically acceptable salt thereof according to any one of claims 1 to 16, wherein R³ is selected from the group consisting of 5- or 6- membered heteroaryl and phenyl, wherein the 5- or 6- membered heteroaryl or phenyl is optionally further substituted with one or more substituents selected from the group consisting of deuterium, hydroxy, halogen, alkyl, alkylthio, alkoxy, haloalkyl, hydroxyalkyl, and haloalkoxy.

18. The compound or stereoisomer, tautomer, or pharmaceutically acceptable salt thereof according to claim 17, wherein R³ is selected from the group consisting of:

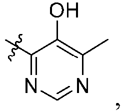

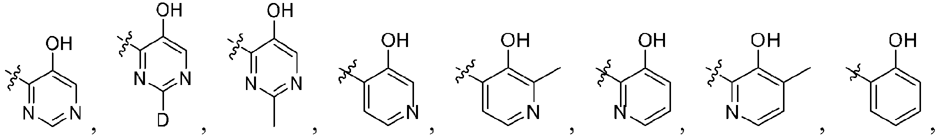

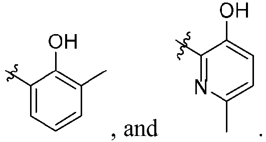

19. The compound or stereoisomer, tautomer, or pharmaceutically acceptable salt thereof according to any one of claims 1 to 18, wherein R⁴ is selected from the group consisting of phenyl and heteroaryl, wherein the phenyl or heteroaryl is optionally further substituted with one or more substituents selected from the group consisting of halogen, hydroxy, cyano, -SF₅, C₁₋₆ alkyl, C₁₋₆ haloalkyl, C₁₋₆ alkoxy, C₁₋₆ haloalkoxy, C₁₋₆ alkenyl, C₁₋₆ alkynyl, -NR⁶R⁷, -C(=O)R⁵, and -S(=O)ᵣR⁵
R⁵, R⁶, and R⁷ are each independently selected from the group consisting of hydrogen and C₁₋₆ alkyl, wherein the C₁₋₆ alkyl is optionally further substituted with one or more deuterium or halogen;
alternatively, R⁶ and R⁷, together with the atom to which they are attached, form a 4- to 8-membered heterocyclyl containing one or more N, O, or S(=O)ᵣ, wherein the 4- to 8- membered heterocyclyl is optionally further substituted with one or more substituents selected from the group consisting of hydroxy, halogen, C₁₋₆ alkyl, C₁₋₆ alkoxy, amino, and =O; and
r is 0, 1, or 2.

20. The compound or stereoisomer, tautomer, or pharmaceutically acceptable salt thereof according to claim 19, wherein R⁴ is selected from the group consisting of:

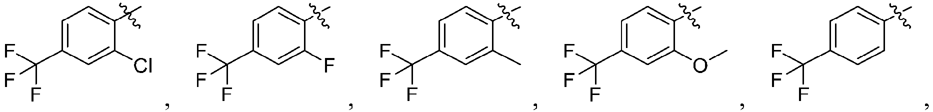

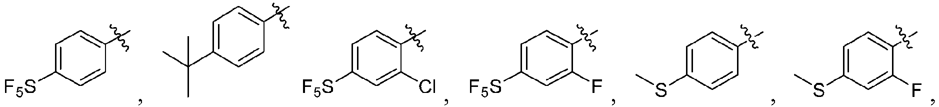

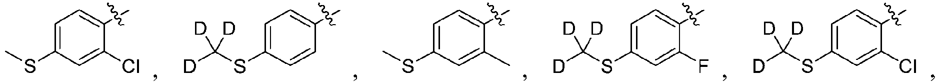

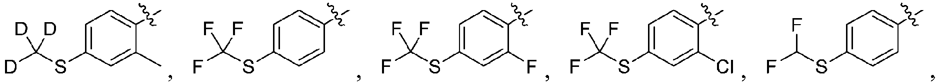

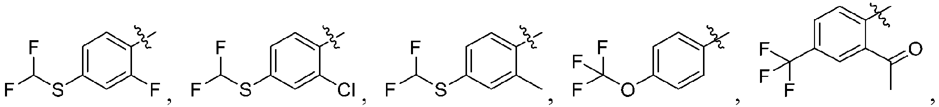

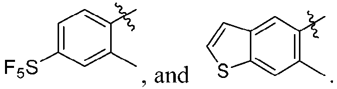

21. The compound or stereoisomer, tautomer, or pharmaceutically acceptable salt thereof according to any one of claims 1 to 20, wherein the compound is selected from the group consisting of: 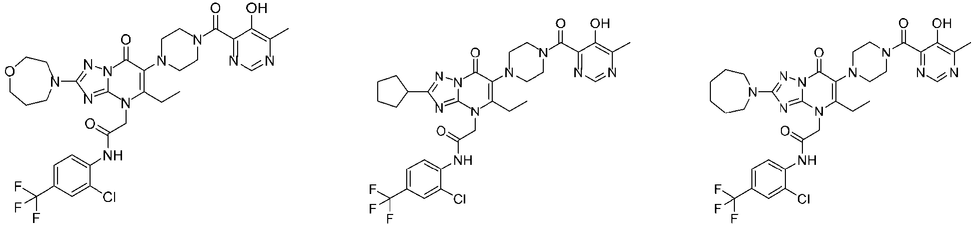 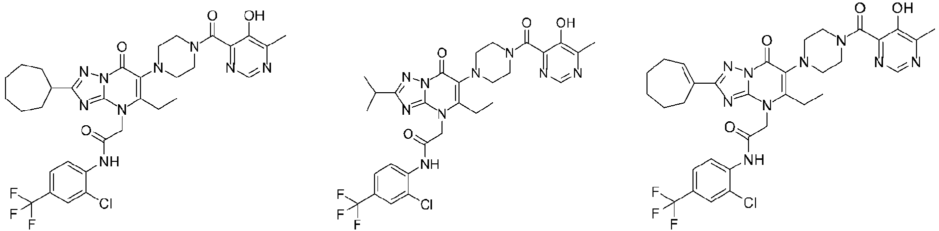 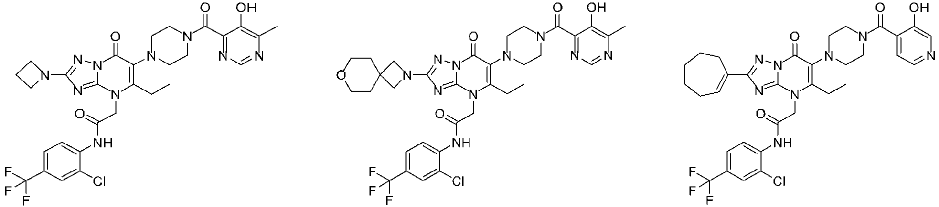 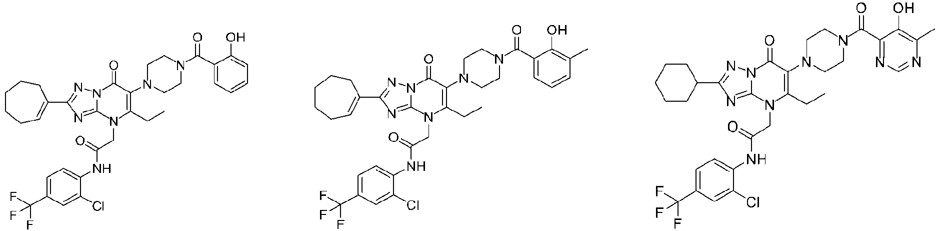 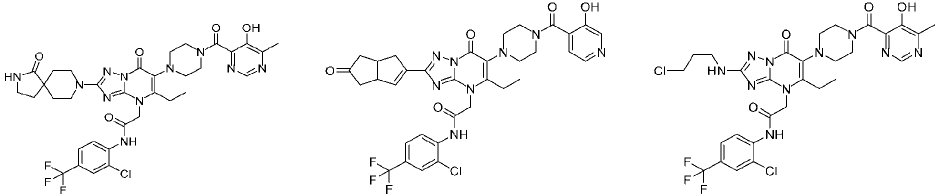 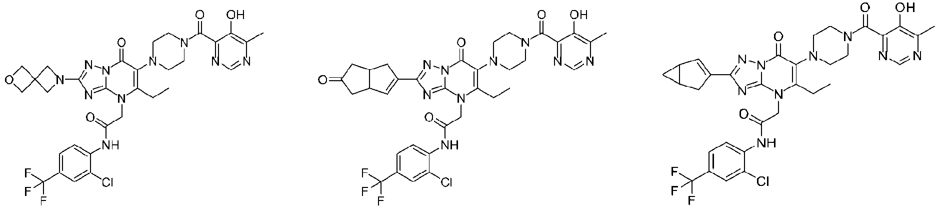 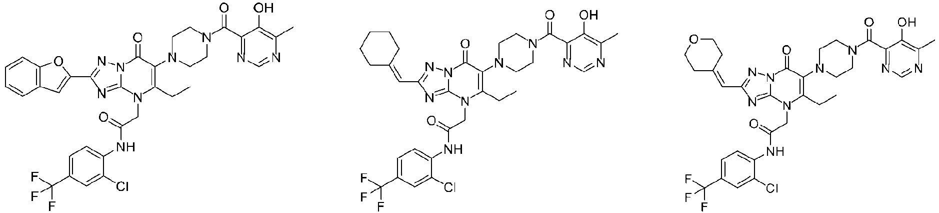 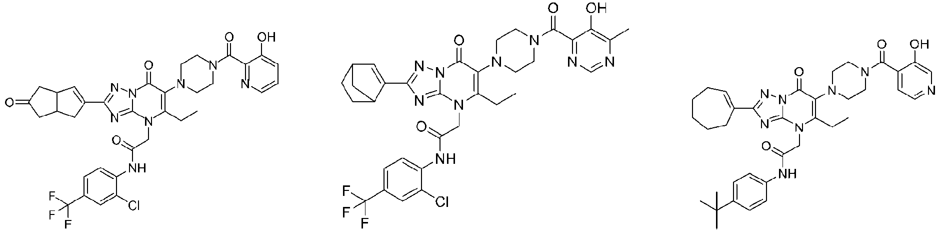 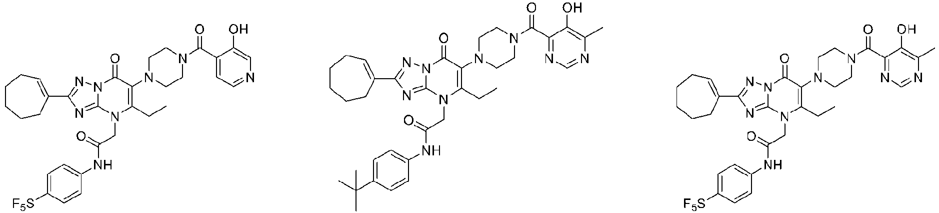 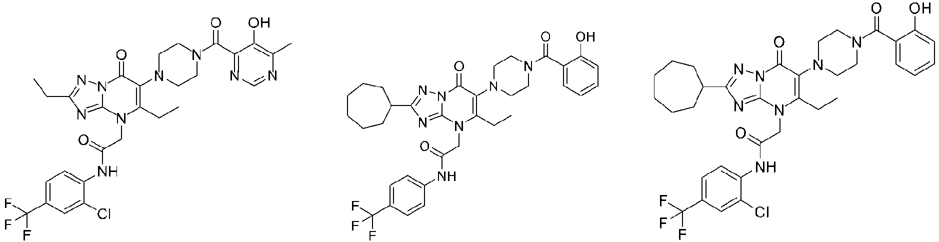 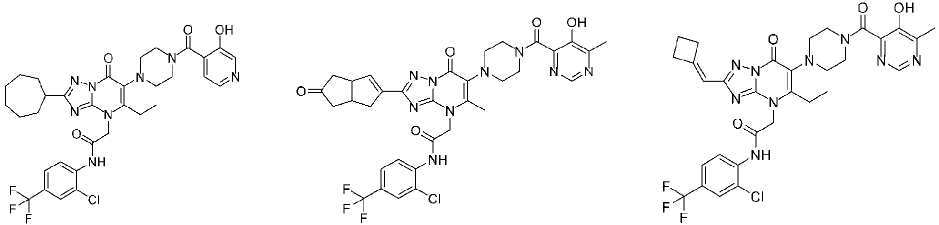 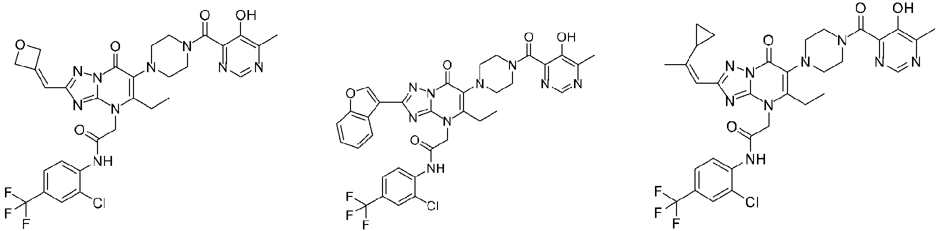 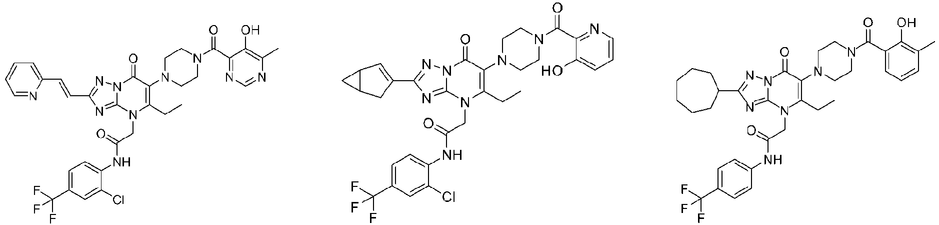 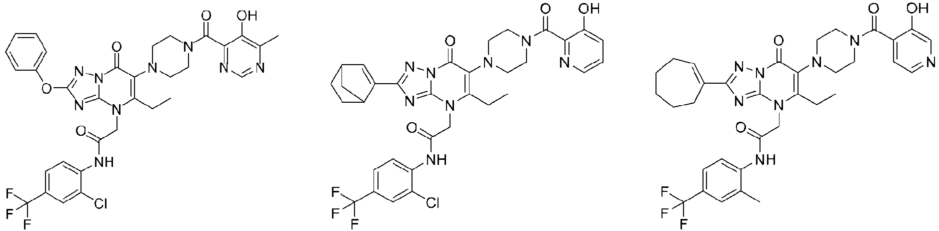 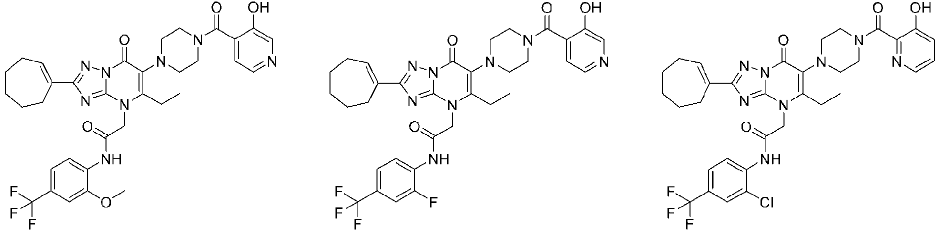 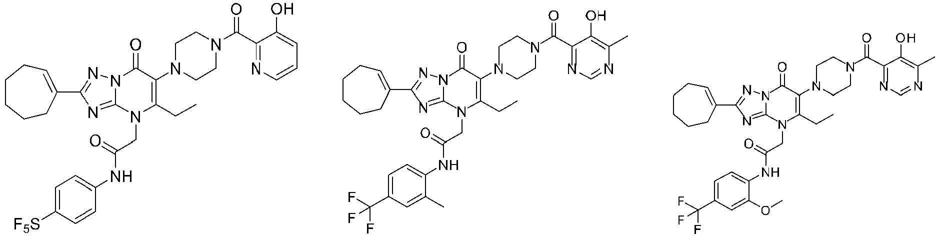 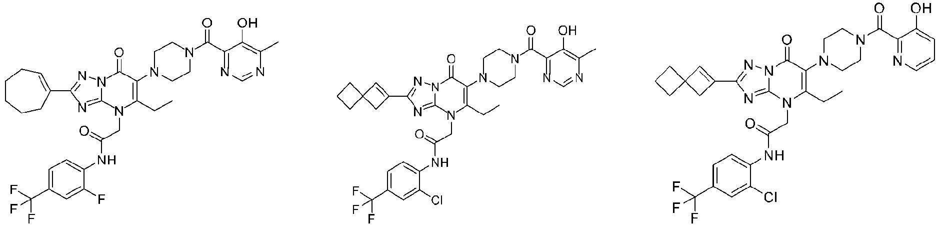 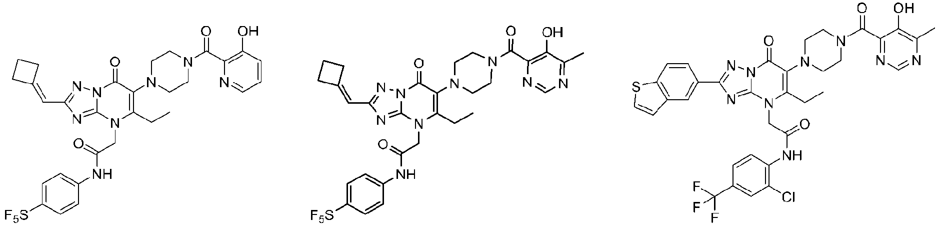 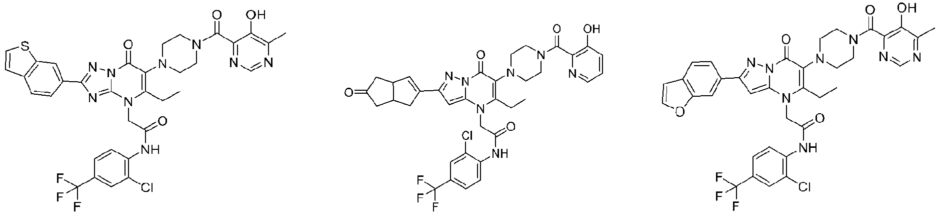 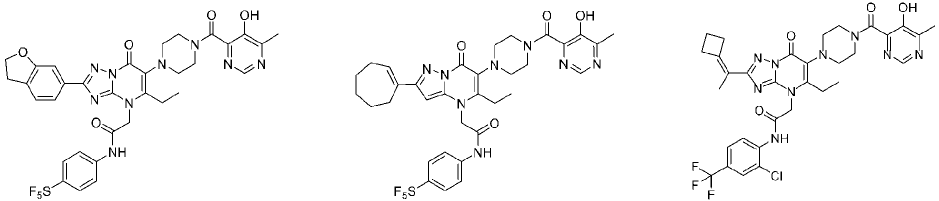 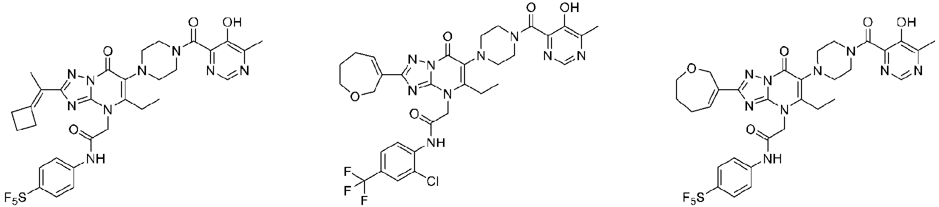 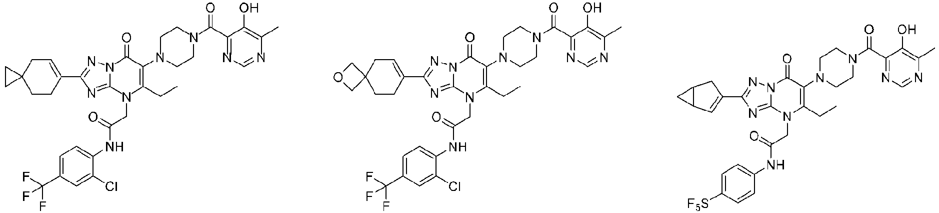 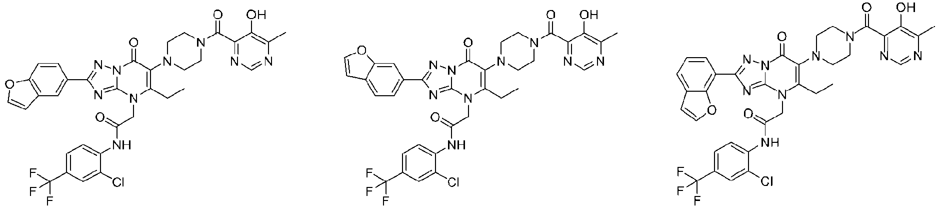 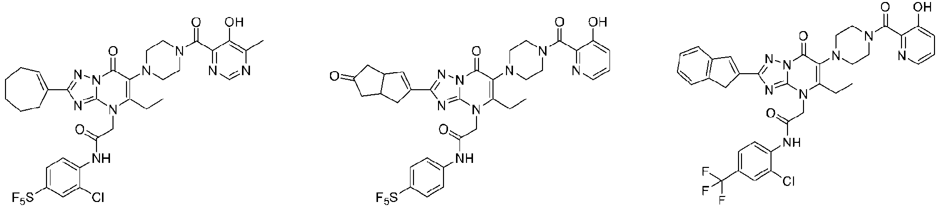 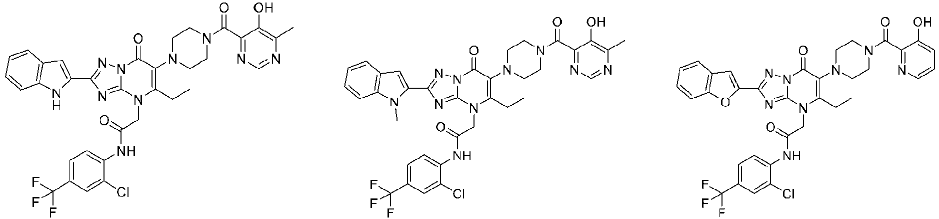 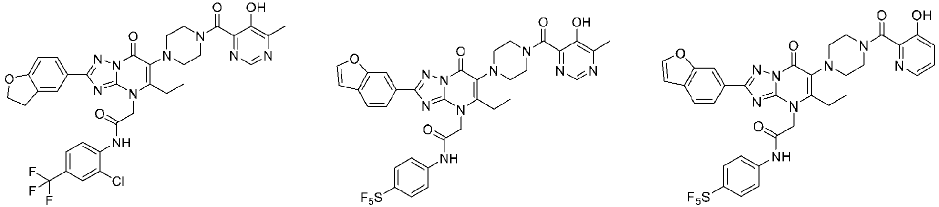 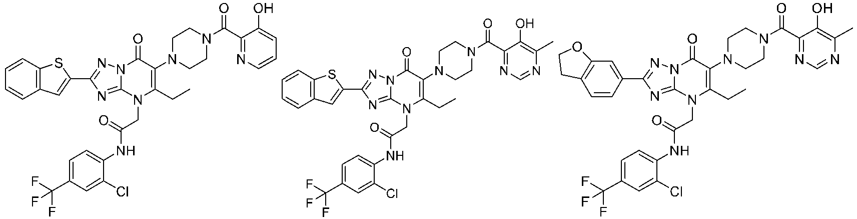 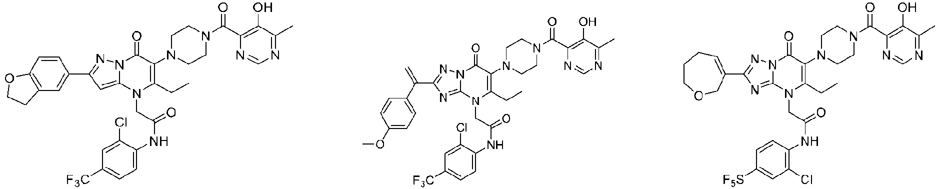 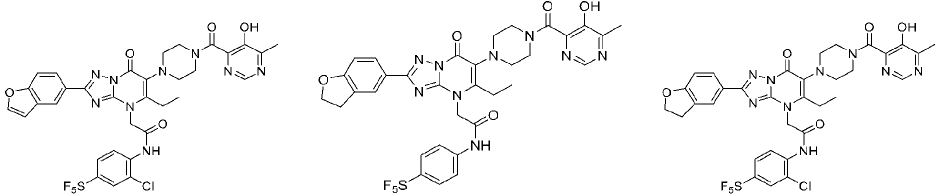 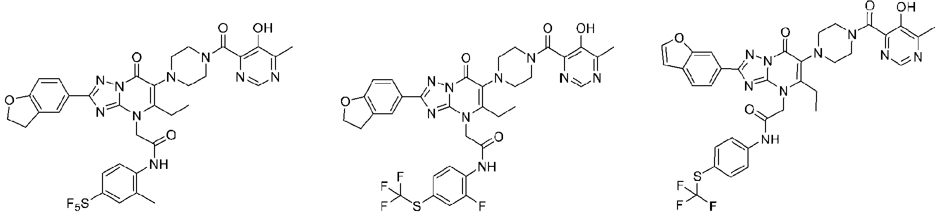 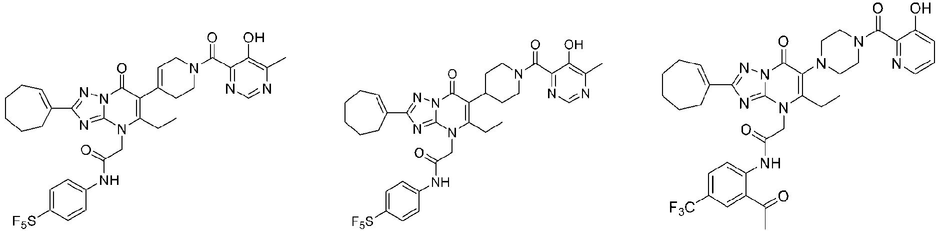 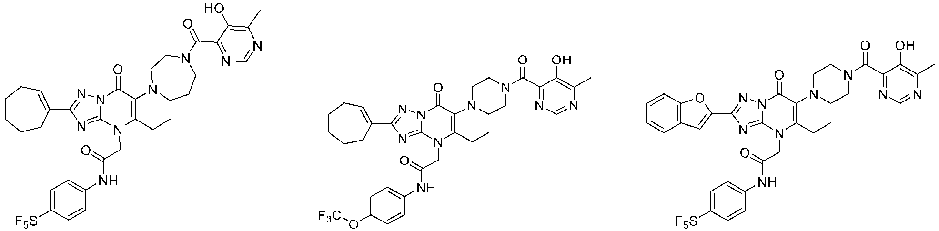 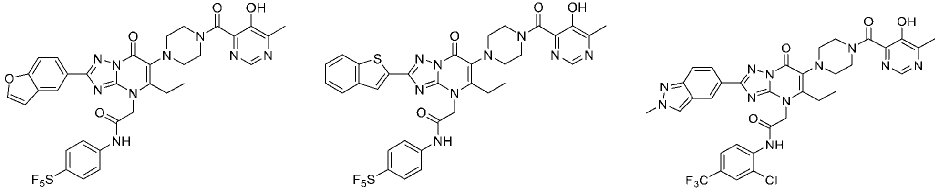 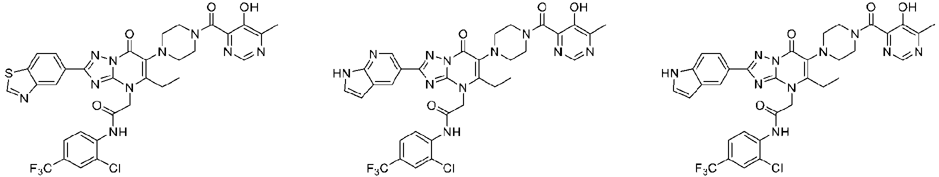 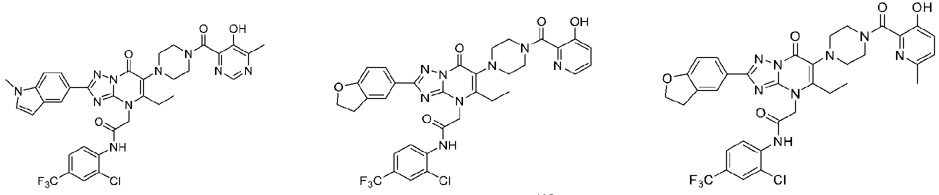 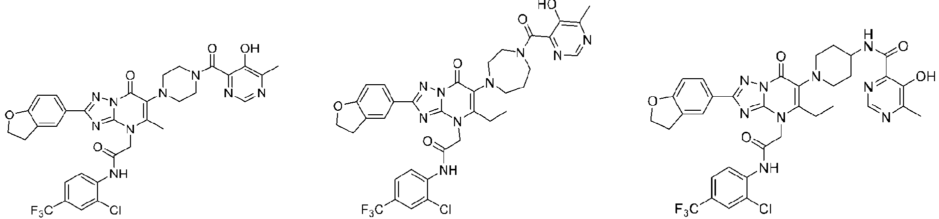 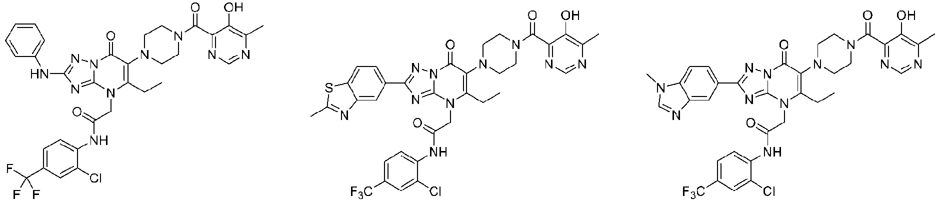 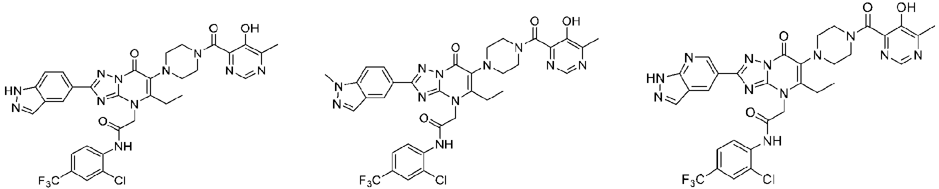 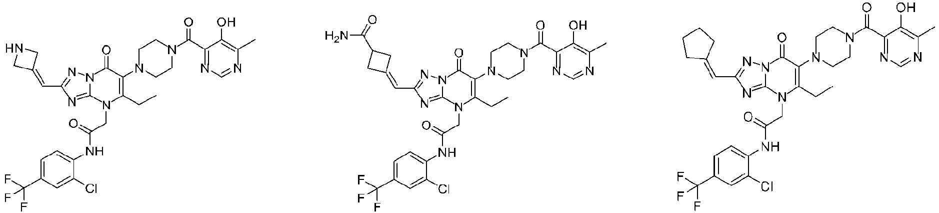 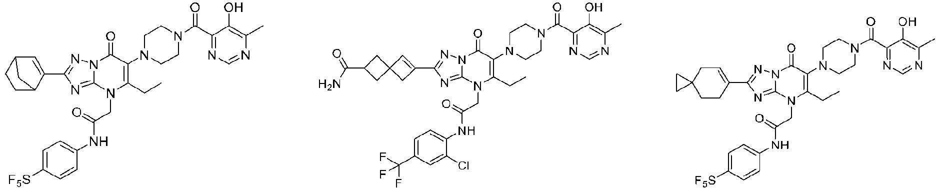 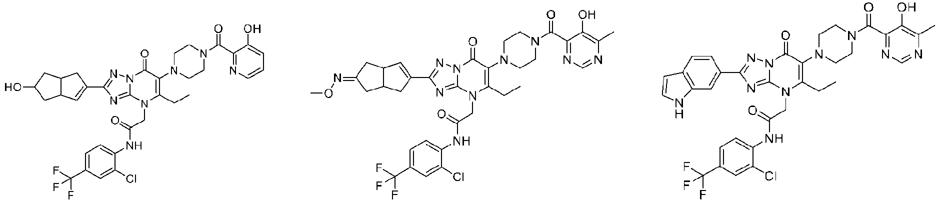 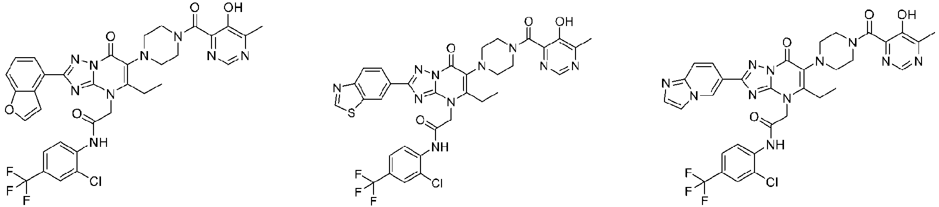 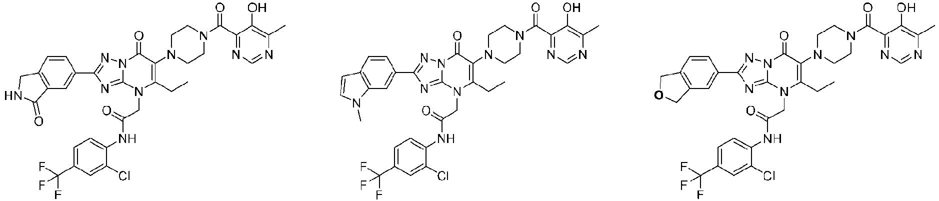 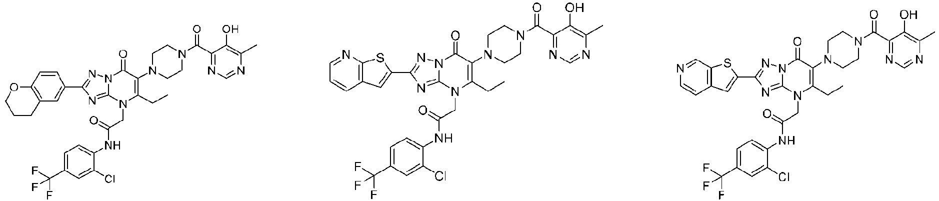 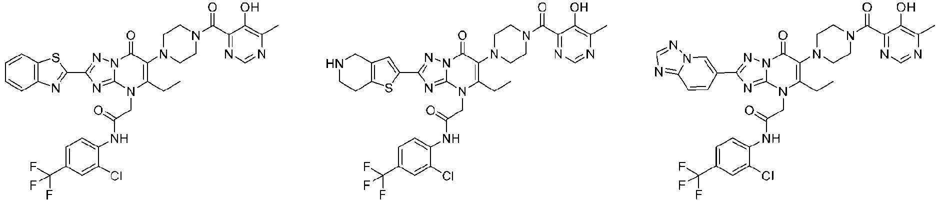 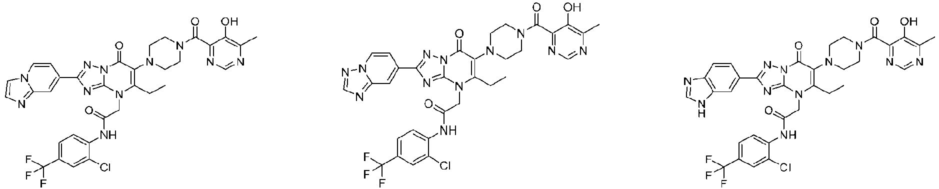 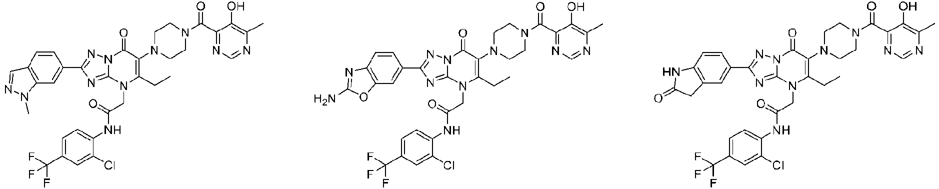 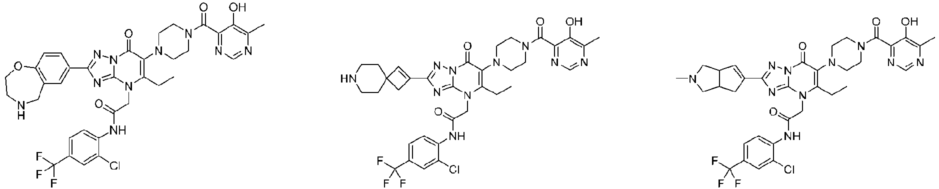 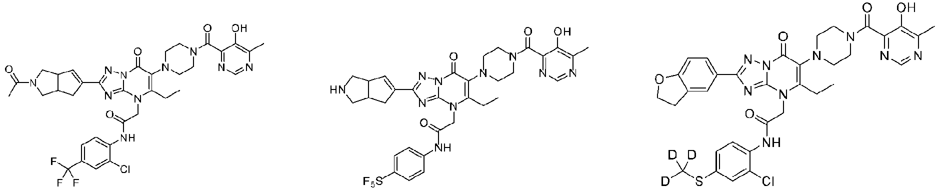 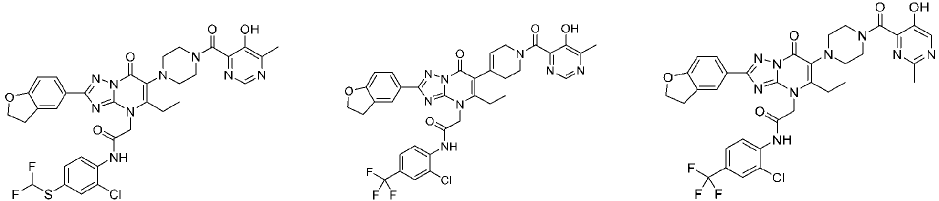 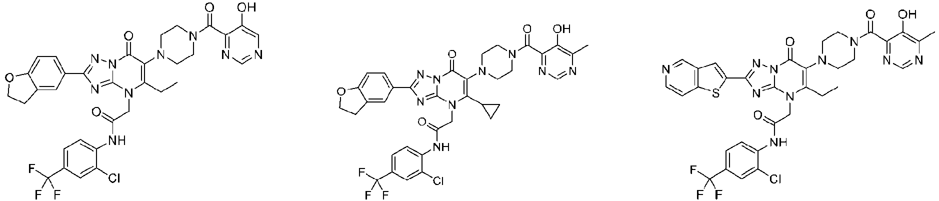 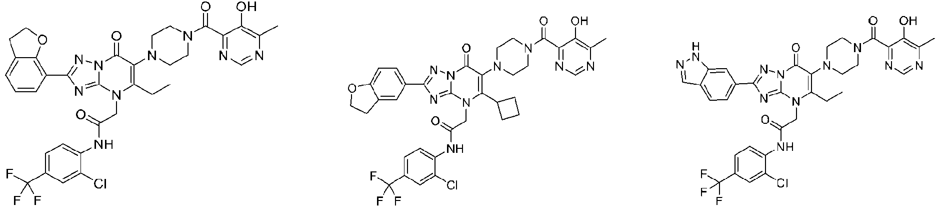 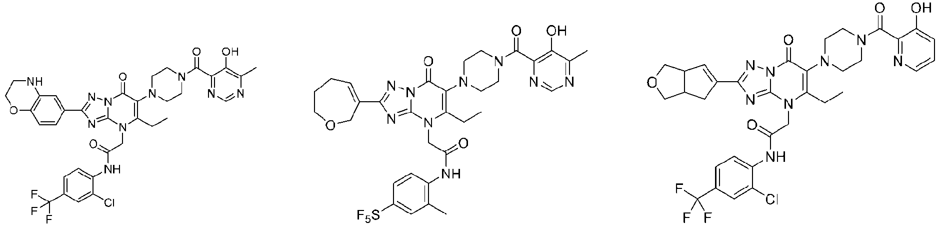 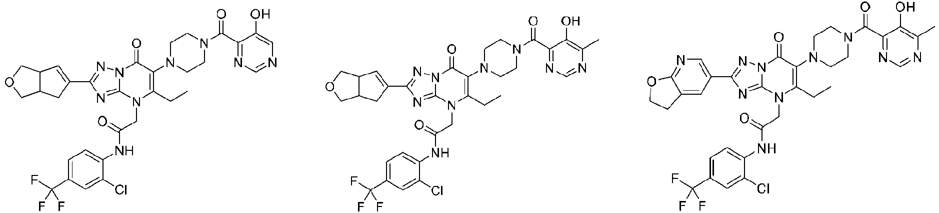 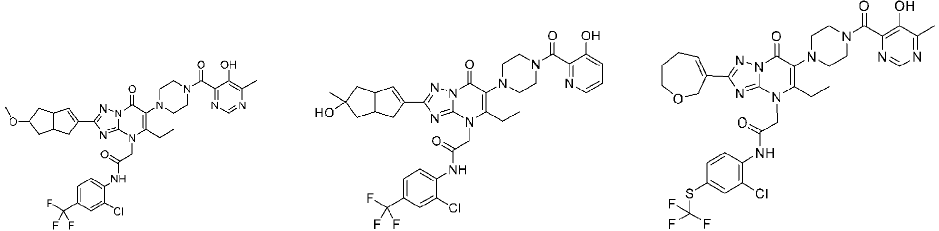 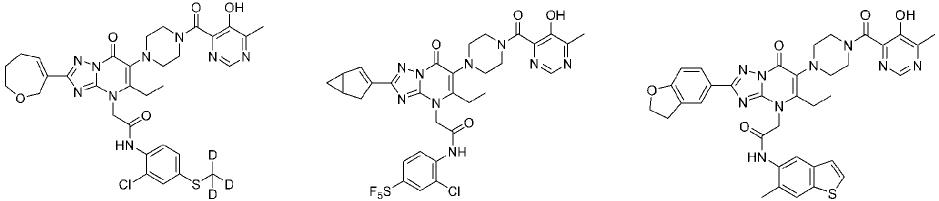 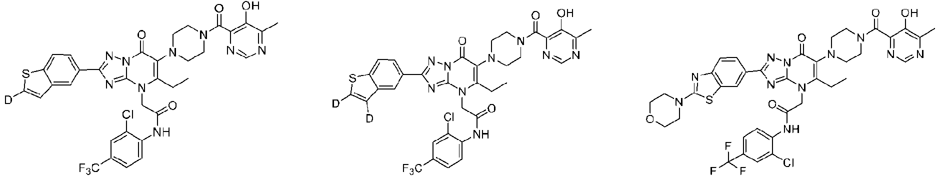 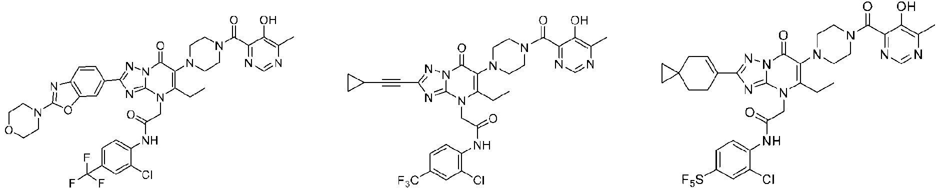 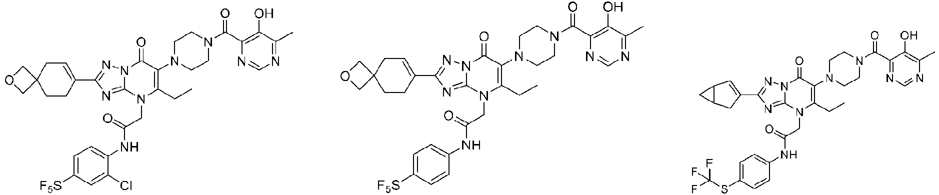 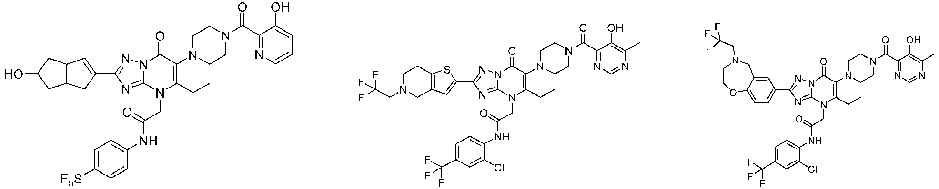 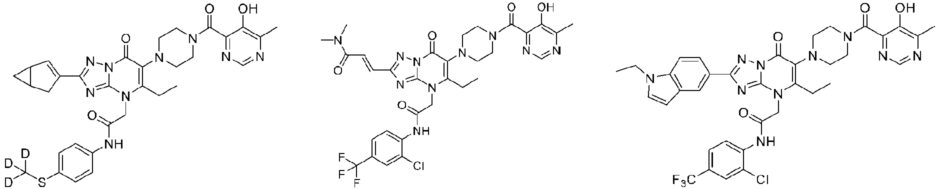 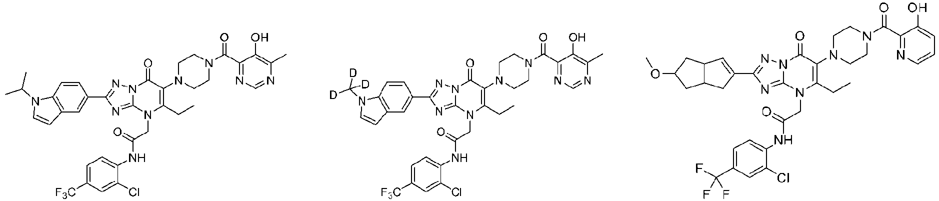 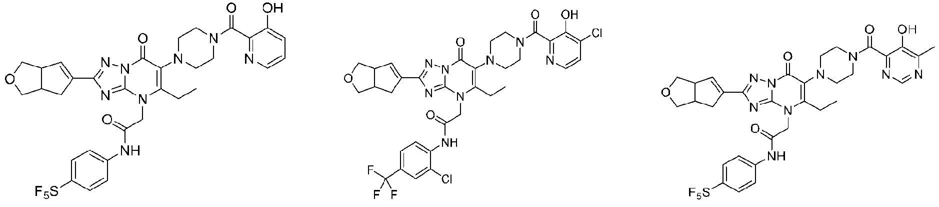 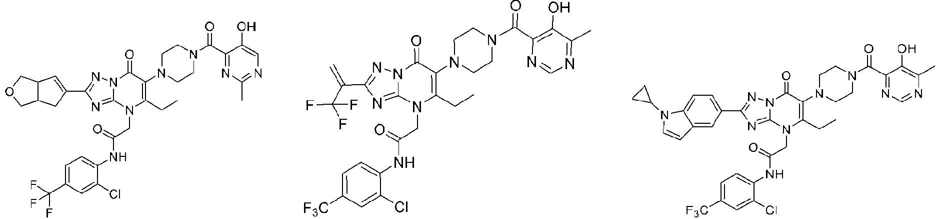 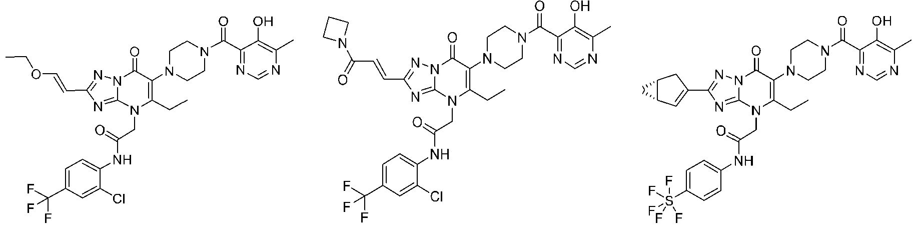 and

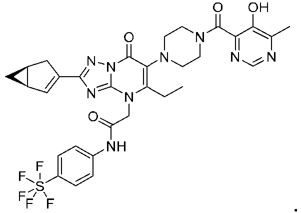

22. A pharmaceutical composition comprising the compound or stereoisomer, tautomer, or pharmaceutically acceptable salt thereof according to any one of claims 1 to 21, and a pharmaceutically acceptable carrier.

23. Use of the compound or stereoisomer, tautomer, or pharmaceutically acceptable salt thereof according to any one of claims 1 to 21, or the pharmaceutical composition according to claim 22, in the manufacture of a WRN inhibitor.

24. Use of the compound or stereoisomer, tautomer, or pharmaceutically acceptable salt thereof according to any one of claims 1 to 21, or the pharmaceutical composition according to claim 22, in the manufacture of a medicament for treating a WRN-mediated disease; preferably, the WRN-mediated disease is a high microsatellite instability cancer.

25. The use according to claim 24, wherein the high microsatellite instability cancer is selected from the group consisting of colorectal cancer, gastric cancer, endometrial cancer, rectal adenocarcinoma, adrenocortical carcinoma, uterine sarcoma, cervical cancer, nephroblastoma, mesothelioma, esophageal cancer, breast cancer, renal clear cell carcinoma, ovarian serous cystadenocarcinoma, cholangiocarcinoma, thymoma, liver cancer, head and neck squamous cell carcinoma, sarcoma, cutaneous melanoma, lung squamous cell carcinoma, prostate cancer, lung adenocarcinoma, bladder transitional cell carcinoma, pediatric neuroblastoma, chronic lymphocytic leukemia, and glioma, and is preferably colorectal cancer, gastric cancer, or endometrial cancer.

26. Use of the compound or stereoisomer, tautomer, or a pharmaceutically acceptable salt thereof according to any one of claims 1 to 21, or the pharmaceutical composition according to claim 22, in the manufacture of a medicament for treating a high microsatellite instability cancer.

27. The use according to claim 26, wherein the high microsatellite instability cancer is selected from the group consisting of colorectal cancer, gastric cancer, endometrial cancer, rectal adenocarcinoma, adrenocortical carcinoma, uterine sarcoma, cervical cancer, nephroblastoma, mesothelioma, esophageal cancer, breast cancer, renal clear cell carcinoma, ovarian serous cystadenocarcinoma, cholangiocarcinoma, thymoma, liver cancer, head and neck squamous cell carcinoma, sarcoma, cutaneous melanoma, lung squamous cell carcinoma, prostate cancer, lung adenocarcinoma, bladder transitional cell carcinoma, pediatric neuroblastoma, chronic lymphocytic leukemia, and glioma, and is preferably colorectal cancer, gastric cancer, or endometrial cancer.
